# EUROPEAN PATENT APPLICATION

(11) **EP 4 371 982 A2**
(43) Date of publication of application: **22.05.2024**
(21) Application number: 24161427.0
(22) Date of filing: 22.02.2018
(51) Int. Cl.: C07D 401/12

(54) **MACROCYCLIC BROAD SPECTRUM ANTIBIOTICS**

(30) Priority: 15.02.2017 WO PCT/CN2017/073575; 19.05.2017 WO PCT/CN2017/085075
(62) Divisional of application: 18754727.8
(71) Applicant: RQX Pharmaceuticals, Inc., La Jolla, CA 92037 (US); Genentech, Inc., South San Francisco, CA 94080 (US)
(72) Inventor: SMITH, Peter Andrew, San Francisco, 94107 (US); ROBERTS, Tucker Curran, San Diego, 92103 (US); HIGUCHI, Robert I., Solana Beach, 92075 (US); PARASELLI, Prasuna, San Diego, 92130 (US); KOEHLER, Michael F. T., Palo Alto, 94306 (US); SCHWARZ, Jacob Bradley, San Ramon, 94582 (US); CRAWFORD, James John, San Francisco, 94131 (US); LY, Cuong Q., Burlingame, 94010 (US); HANAN, Emily J., Redwood City, 94061 (US); HU, Huiyong, Fremont, 94555 (US); CHEN, Yongsheng, Shanghai, 200131 (CN); YU, Zhiyong, Shanghai, 200131 (CN); WINSHIP, Paul Colin Michael, Harlow, CM19 5TR (GB); McCLEOD, Calum, Harlow, CM19 5TR (GB); BLENCH, Toby, Harlow, CM19 5TR (GB)
(74) Representative: Mewburn Ellis LLP

(57) **Abstract**

Provided herein are antibacterial compounds, wherein the compounds in some embodiments have broad spectrum bioactivity. In various embodiments, the compounds act by inhibition of bacterial type 1 signal peptidase (SpsB), an essential protein in bacteria. Pharmaceutical compositions and methods for treatment using the compounds described herein are also provided.

## Description

### CROSS-REFERENCE

[0001] This patent application claims the benefit of PCT Application No. PCT/CN2017/073575, filed February 15, 2017; and PCT Application No. PCT/CN2017/085075, filed May 19, 2017; each of which is incorporated herein by reference in their entirety.

### BACKGROUND OF THE INVENTION

Antibiotic resistance is a serious and growing phenomenon in contemporary medicine and has emerged as a major public health concern of the 21st century. Therefore, novel classes of broad-spectrum antibiotics, especially those that target novel mechanisms of action, are needed to treat multidrug-resistant pathogens.

### SUMMARY OF THE INVENTION

Described herein are novel macrocyclic compounds for the treatment of microbial infections, such as for the treatment of bacterial infections. In various embodiments, the present disclosure provides lipopeptide macrocyclic compounds for the treatment of bacterial infections. In various embodiments, the present disclosure provides classes and subclasses of chemical compounds structurally related to arylomycin for the treatment of bacterial infections. In various embodiments, the macrocyclic compounds act by inhibition of bacterial type 1 signal peptidase (SpsB), an essential protein in bacteria. In some embodiments, the signal peptidase is a Gram-negative signal peptidase. In some embodiments, the signal peptidase is LepB.

In one aspect described herein is a compound of Formula (I): 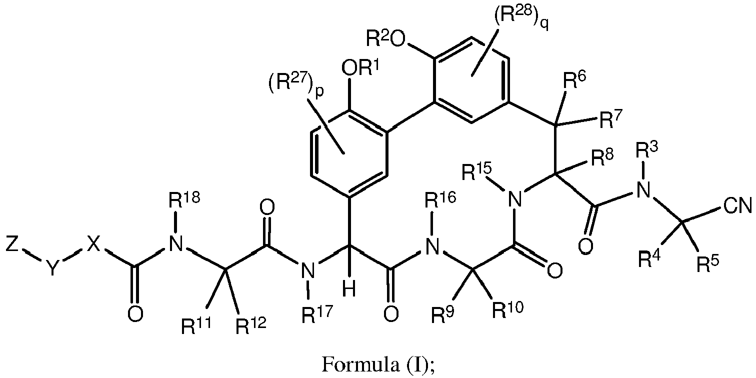 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring; R³ is H or -(C₁-C₆)alkyl;
R⁴ is H, -(C₁-C₆)alkyl, -(C₁-C₆)alkyl-OH, -(C₃-C₆)cycloalkyl, or -C(O)NH₂;
or R³ and R⁴ are combined to form a heterocycloalkyl ring;
R⁵ is H or -(C₁-C₆)alkyl;
or R⁴ and R⁵ and the carbon atom to which they are attached form a cyclopropyl ring;
R⁶, R⁷, and R⁸ are each independently H, fluoro, hydroxyl, amino, optionally substituted alkyl,optionally substituted heteroalkyl, or -(C₁-C₆)alkyl;
R⁹ is H, -(C₁-C₆)alkyl, -(C₁-C₆)haloalkyl, or -(C₃-C₆)cycloalkyl;
R¹⁰ is H, -(C₁-C₆)alkyl, -(C₁-C₆)haloalkyl, or -(C₃-C₆)cycloalkyl;
or R⁹ and R¹⁰ are combined to form a heterocycloalkyl or cycloalkyl ring
R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
or R¹¹ and R¹⁸ are combined to form an optionally substituted heterocycloalkyl ring; and R¹² is H;
R¹⁵, R¹⁶, R¹⁷, and R¹⁸ are each independently H, -(C₁-C₆)alkyl, -(C₃-C₆)cycloalkyl, -(C₁-C₆)alkyl-OR²³, - (C₁-C₆)alkyl-C(O)OR²³, or -(C₁-C₆)alkyl-NR²¹R²².
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -Oaryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted-(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO_{Z}H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring;
each R²⁷ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R¹ and R²⁷ and the atoms to which they are attached form an optionally substituted 5- or 6-membered heterocycloalkyl ring;
each R²⁸ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R² and R²⁸ and the atoms to which they are attached form an optionally substituted 5- or 6-membered heterocycloalkyl ring;
p is 0, 1, or 2; and
q is 0, 1, or 2;
or a pharmaceutically acceptable salt, solvate, or prodrug thereof.

In another embodiment is a compound of Formula (I) having the structure of Formula (Ia):

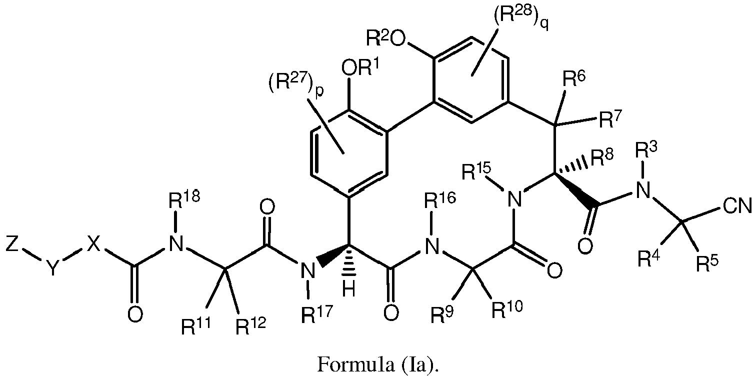

In another embodiment is a compound of Formula (I) or (Ia) wherein R⁶, R⁷, and R⁸ are H. In another embodiment is a compound of Formula (I) or (Ia) wherein R¹⁵ and R¹⁶ are H. In another embodiment is a compound of Formula (I) or (Ia) having the structure of Formula (Ib):

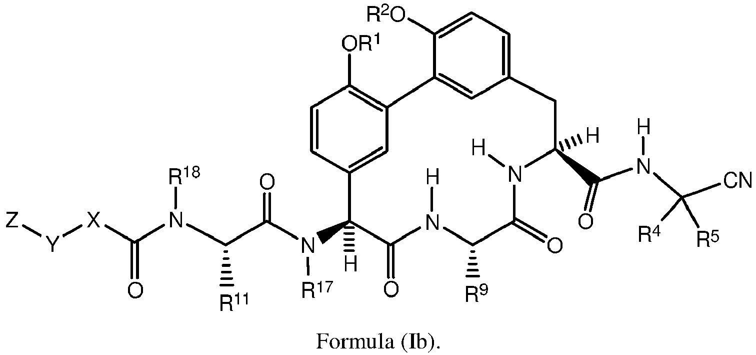

In another embodiment is a compound of Formula (I), (Ia), or (Ib) wherein R¹⁷ is -CH₃. In another embodiment is a compound of Formula (I), (Ia), or (Ib) wherein R¹⁸ is H. In another embodiment is a compound of Formula (I), (Ia), or (Ib) wherein R⁵ is H. In another embodiment is a compound of Formula (I), (Ia), or (Ib) wherein R⁴ is H. In another embodiment is a compound of Formula (I), (Ia), or (Ib) wherein R⁴ is -(C₁-C₆)alkyl. In another embodiment is a compound of Formula (I), (Ia), or (Ib) wherein R⁴ is -(C₃-C₆)cycloalkyl. In another embodiment is a compound of Formula (I), (Ia), or (Ib) wherein R⁴ and R⁵ and the carbon atom to which they are attached form a cyclopropyl ring. In another embodiment is a compound of Formula (I), (Ia), or (Ib) wherein R⁹ is -(C₁-C₆)alkyl. In another embodiment is a compound of Formula (I), (Ia), or (Ib) wherein R⁹ is -CH₃. In another embodiment is a compound of Formula (I), (Ia), or (Ib) having the structure of Formula (Ic): 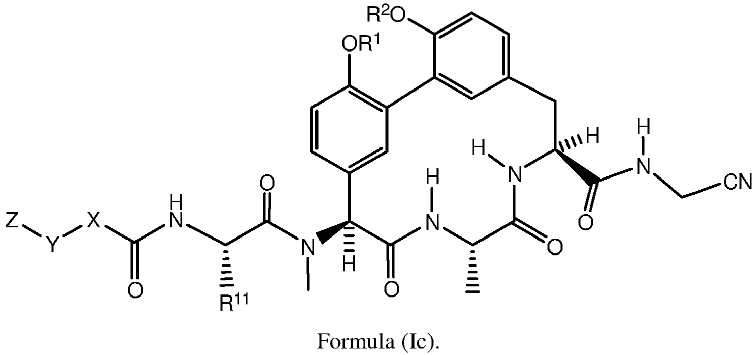 [0007] In another embodiment is a compound of Formula (I) or (Ia)-(Ic) wherein R¹¹ is -(C₁-C₆)alkyl-OR²³. In another embodiment is a compound of Formula (I) or (Ia)-(Ic) wherein R¹¹ is -CH₂CH₂OH. In another embodiment is a compound of Formula (I) or (Ia)-(Ic) wherein R¹¹ is -(C₁-C₆)alkyl. In another embodiment is a compound of Formula (I) or (Ia)-(Ic) wherein R¹¹ is -(C₁-C₆)alkyl-NR²¹R²². In another embodiment is a compound of Formula (I) or (Ia)-(Ic) wherein R¹¹ is -(C₁-C₆)alkyl-NH₂. In another embodiment is a compound of Formula (I) or (Ia)-(Ic) wherein R¹¹ is -CH₂NH₂. In another embodiment is a compound of Formula (I) or (Ia)-(Ic) wherein R¹¹ is -CH₂CH₂NH₂. In another embodiment is a compound of Formula (I) or (Ia)-(Ic) wherein R¹¹ is -CH₂CH₂CH₂NH₂. In another embodiment is a compound of Formula (I) or (Ia)-(Ic) wherein R¹¹ is -CH₂CH₂CH₂CH₂NH₂. In another embodiment is a compound of Formula (I) or (Ia)-(Ic) wherein R¹ and R² are each independently H or -(C₁-C₆)alkyl-NR²¹R²². In another embodiment is a compound of Formula (I) or (Ia)-(Ic) wherein R¹ and R² are each independently -(C₁-C₆)alkyl-NR²¹R²². In another embodiment is a compound of Formula (I) or (Ia)-(Ic) wherein R¹ and R² are each -CH₂CH₂NH₂. In another embodiment is a compound of Formula (I) or (Ia)-(Ic) wherein R¹ is -(C₁-C₆)alkyl-NR²¹R²² and R² is H. In another embodiment is a compound of Formula (I) or (Ia)-(Ic) wherein R¹ is -CH₂CH₂NH₂ and R² is H. In another embodiment is a compound of Formula (I) or (Ia)-(Ic) wherein R¹ is H and R² is -(C₁-C₆)alkyl-NR²¹R²². In another embodiment is a compound of Formula (I) or (Ia)-(Ic) wherein R¹ is H and R² is -CH₂CH₂NH₂. In another embodiment is a compound of Formula (I) or (Ia)-(Ic) wherein R¹ is H and R² is H. In another embodiment is a compound of Formula (I) or (Ia)-(Ic) having the structure of Formula (Id): 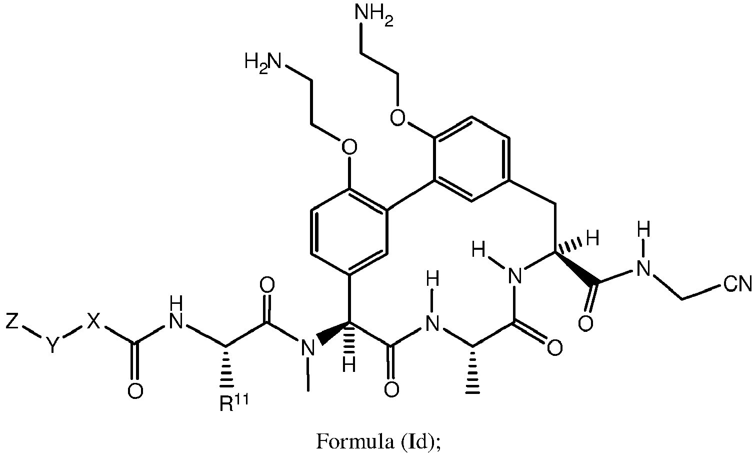 wherein R¹¹ is -CH₂CH₂NH₂ or -CH₂CH₂CH₂NH₂.

In one aspect described herein is a compound of Formula (II): 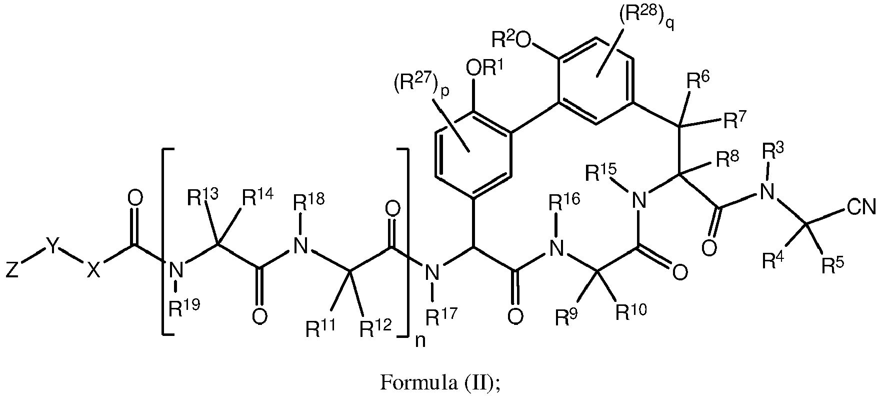 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring;
R³ is H or -(C₁-C₆)alkyl;
R⁴ is H, -(C₁-C₆)alkyl, -(C₁-C₆)alkyl-OH, -(C₃-C₆)cycloalkyl, or -C(O)NH₂;
or R³ and R⁴ are combined to form a heterocycloalkyl ring;
R⁵ is H or -(C₁-C₆)alkyl;
or R⁴ and R⁵ and the carbon atom to which they are attached form a cyclopropyl ring;
R⁶, R⁷, and R⁸ are each independently H or -(C₁-C₆)alkyl;
R⁹ is H, -(C₁-C₆)alkyl, -(C₁-C₆)haloalkyl, or -(C₃-C₆)cycloalkyl;
R¹⁰ is H or -(C₁-C₆)alkyl;
R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
R¹³ and R¹⁴ are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-CN, -(C₁-C₆)alkyl-C(O)NR²⁵R²⁶, -(C₁-C₆)alkyl-N(H)C(NH)NH₂, -(C₁-C₆)alkyl-heterocycloalkyl, or -(C₁-C₆)alkyl-heteroaryl;
or R¹³ and R¹⁹ are combined to form an optionally substituted heterocycloalkyl ring; and R¹⁴ is H;
R¹⁵, R¹⁶, R¹⁷, R¹⁸, and R¹⁹ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, -C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring;
each R²⁷ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R¹ and R²⁷ and the atoms to which they are attached form an optionally substituted 5- or 6-membered heterocycloalkyl ring;
each R²⁸ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R² and R²⁸ and the atoms to which they are attached form an optionally substituted 5- or 6-membered heterocycloalkyl ring;
n is 0 or 1;
p is 0, 1, or 2; and
q is 0, 1, or 2;
or a pharmaceutically acceptable salt, solvate, or prodrug thereof.

In another embodiment is a compound of Formula (II) having the structure of Formula (IIa):

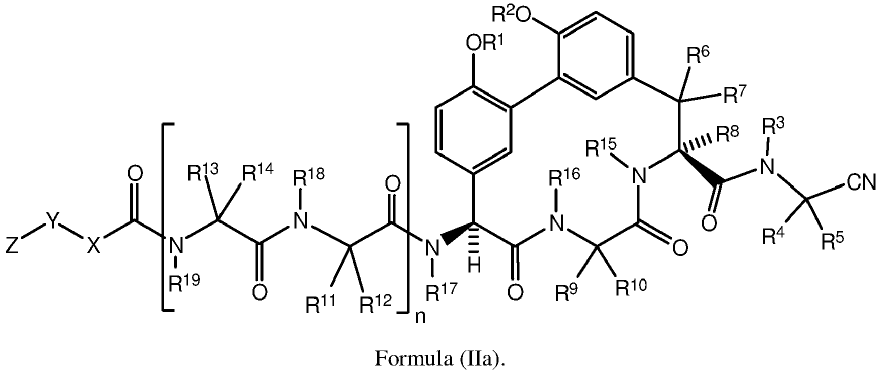

In another embodiment is a compound of Formula (II) or (IIa) wherein R⁶, R⁷, and R⁸ are H. In another embodiment is a compound of Formula (II) or (IIa) wherein R¹⁵ and R¹⁶ are H. In another embodiment is a compound of Formula (II) or (IIa) having the structure of Formula (IIb):

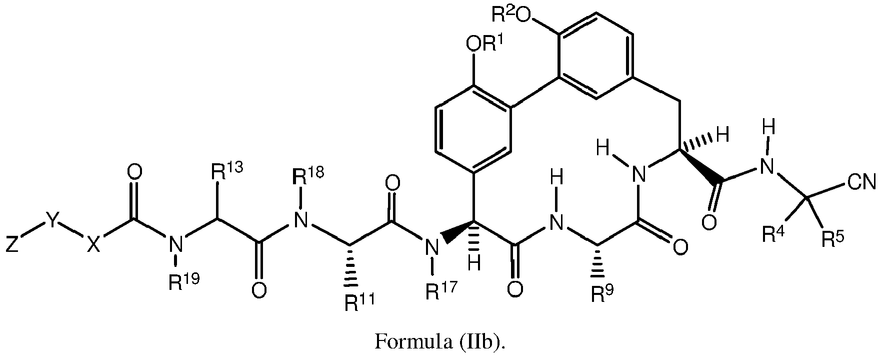

In another embodiment is a compound of Formula (II), (IIa), or (IIb) wherein R¹⁸ is H. In another embodiment is a compound of Formula (II), (IIa), or (IIb) wherein R¹⁹ is H. In another embodiment is a compound of Formula (II), (IIa), or (IIb) wherein R¹⁷ is -CH₃. In another embodiment is a compound of Formula (II), (IIa), or (IIb) wherein R⁵ is H. In another embodiment is a compound of Formula (II), (IIa), or (IIb) wherein R⁴ is H. In another embodiment is a compound of Formula (II), (IIa), or (IIb) wherein R⁴ is -(C₁-C₆)alkyl. In another embodiment is a compound of Formula (II), (IIa), or (IIb) wherein R⁴ is -(C₃-C₆)cycloalkyl. In another embodiment is a compound of Formula (II), (IIa), or (IIb) wherein R⁴ and R⁵ and the carbon atom to which they are attached form a cyclopropyl ring. In another embodiment is a compound of Formula (II), (IIa), or (IIb) wherein R⁹ is -(C₁-C₆)alkyl. In another embodiment is a compound of Formula (II), (IIa), or (IIb) wherein R⁹ is -CH₃. In another embodiment is a compound of Formula (II), (IIa), or (IIb) having the structure of Formula (IIc): 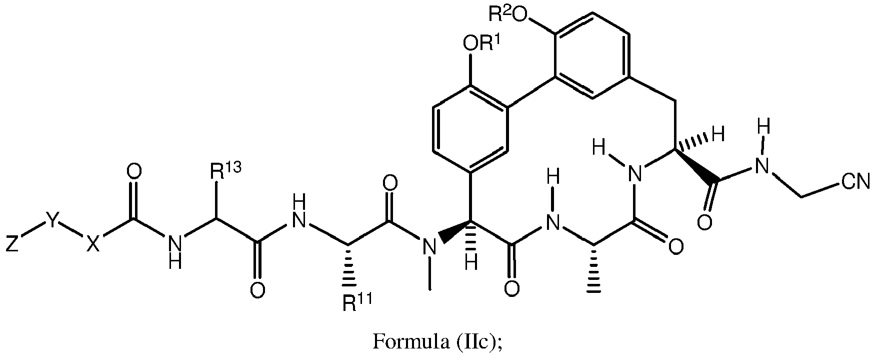 wherein R¹ and R² are each independently H or -CH₂CH₂NH₂.

In another embodiment is a compound of Formula (II) or (IIa)-(IIc) wherein R¹¹ is -(C₁-C₆)alkyl-OR²³. In another embodiment is a compound of Formula (II) or (IIa)-(IIc) wherein R¹¹ is -CH₂CH₂OH. In another embodiment is a compound of Formula (II) or (IIa)-(IIc) wherein R¹¹ is -(C₁-C₆)alkyl. In another embodiment is a compound of Formula (II) or (IIa)-(IIc) wherein R¹¹ is -(C₁-C₆)alkyl-NR²¹R²². In another embodiment is a compound of Formula (II) or (IIa)-(IIc) wherein R¹¹ is -(C₁-C₆)alkyl-NH₂. In another embodiment is a compound of Formula (II) or (IIa)-(IIc) wherein R¹¹ is -CH₂NH₂. In another embodiment is a compound of Formula (II) or (IIa)-(IIc) wherein R¹¹ is -CH₂CH₂NH₂. In another embodiment is a compound of Formula (II) or (IIa)-(IIc) wherein R¹¹ is -CH₂CH₂CH₂NH₂. In another embodiment is a compound of Formula (II) or (IIa)-(IIc) wherein R¹¹ is -CH₂CH₂CH₂CH₂NH₂. In another embodiment is a compound of Formula (II) or (IIa)-(IIc) wherein R¹³ is -(C₁**-**C₆)alkyl-OR²³. In another embodiment is a compound of Formula (II) or (IIa)-(IIc) wherein R¹³ is -CH₂OH. In another embodiment is a compound of Formula (II) or (IIa)-(IIc) wherein R¹³ is -CH₂CH₂OH. In another embodiment is a compound of Formula (II) or (IIa)-(IIc) having the structure of Formula (IId):

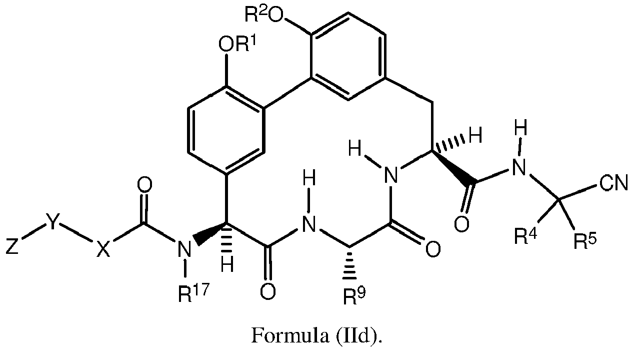

In another embodiment is a compound of Formula (II) or (IIa)-(IId) wherein R¹⁷ is -CH₃. In another embodiment is a compound of Formula (II) or (IIa)-(IId) wherein R⁵ is H. In another embodiment is a compound of Formula (II) or (IIa)-(IId) wherein R⁴ is H. In another embodiment is a compound of Formula (II) or (IIa)-(IId) wherein R⁴ is -(C₁-C₆)alkyl. In another embodiment is a compound of Formula (II) or (IIa)-(IId) wherein R⁴ is -(C₃-C₆)cycloalkyl. In another embodiment is a compound of Formula (II) or (IIa)-(IId) wherein R⁴ and R⁵ and the carbon atom to which they are attached form a cyclopropyl ring. In another embodiment is a compound of Formula (II) or (IIa)-(IId) wherein R⁹ is -(C₁-C₆)alkyl. In another embodiment is a compound of Formula (II) or (IIa)-(IId) wherein R⁹ is -CH₃. In another embodiment is a compound of Formula (II) or (IIa)-(IId) wherein R¹ and R² are each independently H or -(C₁-C₆)alkyl-NR²¹R²². In another embodiment is a compound of Formula (II) or (IIa)-(IId) wherein R¹ and R² are each independently -(C₁-C₆)alkyl-NR²¹R²². In another embodiment is a compound of Formula (II) or (IIa)-(IId) wherein R¹ and R² are each -CH₂CH₂NH₂. In another embodiment is a compound of Formula (II) or (IIa)-(IId) wherein R¹ is -(C₁-C₆)alkyl-NR²¹R²² and R² is H. In another embodiment is a compound of Formula (II) or (IIa)-(IId) wherein R¹ is -CH₂CH₂NH₂ and R² is H. In another embodiment is a compound of Formula (II) or (IIa)-(IId) wherein R¹ is H and R² is -(C₁-C₆)alkyl-NR²¹R²². In another embodiment is a compound of Formula (II) or (IIa)-(IId) wherein R¹ is H and R² is -CH₂CH₂NH₂. In another embodiment is a compound of Formula (II) or (IIa)-(IId) having the structure of Formula (IIe): 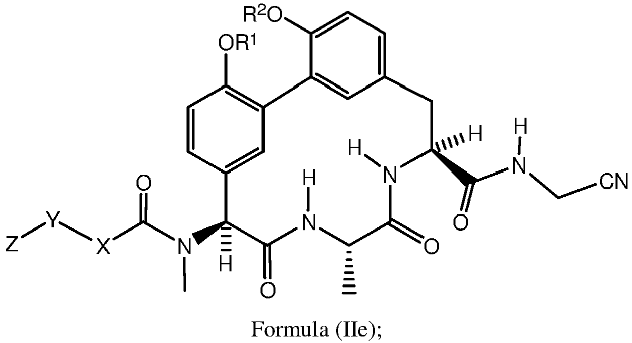 wherein R¹ and R² are each independently H or -CH₂CH₂NH₂.

In another embodiment is a compound of Formula (I), (Ia)-(Id), (II), or (IIa)-(IIe) wherein X is optionally substituted aryl. In another embodiment is a compound of Formula (I), (Ia)-(Id), (II), or (IIa)-(IIe) wherein X is optionally substituted phenyl. In another embodiment is a compound of Formula (I), (Ia)-(Id), (II), or (IIa)-(IIe) wherein X is optionally substituted heteroaryl. In another embodiment is a compound of Formula (I), (Ia)-(Id), (II), or (IIa)-(IIe) wherein X is disubstituted heteroaryl. In another embodiment is a compound of Formula (I), (Ia)-(Id), (II), or (IIa)-(IIe) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In another embodiment is a compound of Formula (I), (Ia)-(Id), (II), or (IIa)-(IIe) wherein X is heteroaryl disubstituted with substituents each independently selected from -(C₁-C₆)alkyl. In another embodiment is a compound of Formula (I), (Ia)-(Id), (II), or (IIa)-(IIe) wherein X is heteroaryl disubstituted with methyl. In another embodiment is a compound of Formula (I), (Ia)-(Id), (II), or (IIa)-(IIe) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In another embodiment is a compound of Formula (I), (Ia)-(Id), (II), or (IIa)-(IIe) wherein X is pyridinyl disubstituted with substituents each independently selected from -(C₁-C₆)alkyl. In another embodiment is a compound of Formula (I), (Ia)-(Id), (II), or (IIa)-(IIe) wherein X is pyridinyl disubstituted with methyl. In another embodiment is a compound of Formula (I), (Ia)-(Id), (II), or (IIa)-(IIe) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In another embodiment is a compound of Formula (I), (Ia)-(Id), (II), or (IIa)-(IIe) wherein X is pyrimidinyl disubstituted with substituents each independently selected from -(C₁-C₆)alkyl. In another embodiment is a compound of Formula (I), (Ia)-(Id), (II), or (IIa)-(IIe) wherein X is pyrimidinyl disubstituted with methyl. In another embodiment is a compound of Formula (I), (Ia)-(Id), (II), or (IIa)-(IIe) wherein X is optionally substituted -(C₁-C₆)alkyl-. In another embodiment is a compound of Formula (I), (Ia)-(Id), (II), or (IIa)-(IIe) wherein Y is optionally substituted aryl. In another embodiment is a compound of Formula (I), (Ia)-(Id), (II), or (IIa)-(IIe) wherein Y is optionally substituted phenyl. In another embodiment is a compound of Formula (I), (Ia)-(Id), (II), or (IIa)-(IIe) wherein Y is optionally substituted heteroaryl. In another embodiment is a compound of Formula (I), (Ia)-(Id), (II), or (IIa)-(IIe) wherein Y is optionally substituted -(C₁-C₆)alkyl-. In another embodiment is a compound of Formula (I), (Ia)-(Id), (II), or (IIa)-(IIe) wherein Y is optionally substituted (C₃-C₇)cycloalkyl-. In another embodiment is a compound of Formula (I), (Ia)-(Id), (II), or (IIa)-(IIe) wherein Y is optionally substituted heterocycloalkyl. In another embodiment is a compound of Formula (I), (Ia)-(Id), (II), or (IIa)-(IIe) wherein Y is -O-. In another embodiment is a compound of Formula (I), (Ia)-(Id), (II), or (IIa)-(IIe) wherein Y is -(C₂-C₆)alkynyl. In another embodiment is a compound of Formula (I), (Ia)-(Id), (II), or (IIa)-(IIe) wherein Y is -O-(C₁-C₆)alkyl-. In another embodiment is a compound of Formula (I), (Ia)-(Id), (II), or (IIa)-(IIe) wherein Y is a bond. In another embodiment is a compound of Formula (I), (Ia)-(Id), (II), or (IIa)-(IIe) wherein Z is -(C₁-C₁₂)alkyl. In another embodiment is a compound of Formula (I), (Ia)-(Id), (II), or (IIa)-(IIe) wherein Z is n-butyl, isobutyl, or tert-butyl. In another embodiment is a compound of Formula (I), (Ia)-(Id), (II), or (IIa)-(IIe) wherein Z is -O-(C₁-C₁₂)alkyl. In another embodiment is a compound of Formula (I), (Ia)-(Id), (II), or (IIa)-(IIe) wherein Z is -O-(C₃-C₇)cycloalkyl. In another embodiment is a compound of Formula (I), (Ia)-(Id), (II), or (IIa)-(IIe) wherein Z is -(C₂-C₁₂)alkenyl. In another embodiment is a compound of Formula (I), (Ia)-(Id), (II), or (IIa)-(IIe) wherein Z is optionally substituted aryl. In another embodiment is a compound of Formula (I), (Ia)-(Id), (II), or (IIa)-(IIe) wherein Z is optionally substituted phenyl. In another embodiment is a compound of Formula (I), (Ia)-(Id), (II), or (IIa)-(IIe) wherein Z is phenyl monsubstituted or disubstituted with a substituent independently selected from -(C₁-C₈)alkyl. In another embodiment is a compound of Formula (I), (Ia)-(Id), (II), or (IIa)-(IIe) wherein Z is phenyl monosubstituted with n-butyl, isobutyl, or tert-butyl. In another embodiment is a compound of Formula (I), (Ia)-(Id), (II), or (IIa)-(IIe) wherein Z is phenyl monosubstituted with n-butyl. In another embodiment is a compound of Formula (I), (Ia)-(Id), (II), or (IIa)-(IIe) wherein Z is phenyl monosubstituted with isobutyl. In another embodiment is a compound of Formula (I), (Ia)-(Id), (II), or (IIa)-(IIe) wherein Z is phenyl monosubstituted with tert-butyl. In another embodiment is a compound of Formula (I), (Ia)-(Id), (II), or (IIa)-(IIe) wherein Z is optionally substituted heteroaryl. In another embodiment is a compound of Formula (I), (Ia)-(Id), (II), or (IIa)-(IIe) wherein Z is optionally substituted - (C₃-C₇)cycloalkyl. In another embodiment is a compound of Formula (I), (Ia)-(Id), (II), or (IIa)-(IIe) wherein Z is optionally substituted heterocycloalkyl. In another embodiment is a compound of Formula (I), (Ia)-(Id), (II), or (IIa)-(IIe) wherein Z is halogen. In another embodiment is a compound of Formula (I), (Ia)-(Id), (II), or (IIa)-(IIe) wherein Z-Y-X- is not 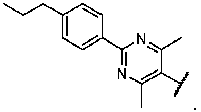 In another embodiment is a compound of Formula (I), (Ia)-(Id), (II), or (IIa)-(IIe) wherein the compound is selected from any of the compounds in table 1 or a pharmaceutically acceptable salt, solvate, or prodrug thereof.

In one aspect described herein is a pharmaceutical composition comprising the compound disclosed herein, or a pharmaceutically acceptable salt, pharmaceutically acceptable solvate, or pharmaceutically acceptable prodrug thereof, and a pharmaceutically acceptable excipient.

In one aspect described herein is a use of a compound disclosed herein, or a pharmaceutically acceptable salt, pharmaceutically acceptable solvate, or pharmaceutically acceptable prodrug thereof, for preparation of a medicament for treatment of a bacterial infection in a patient.

In one aspect described herein is a method of treatment of a bacterial infection in a mammal, comprising administering to the mammal an effective amount of a compound disclosed herein, or a pharmaceutically acceptable salt, pharmaceutically acceptable solvate, or pharmaceutically acceptable prodrug thereof, to the mammal at a frequency and for a duration sufficient to provide a beneficial effect to the mammal.

In one aspect described herein is a method of treatment of a lepB-mediated infection in a mammal, comprising administering to the mammal an effective amount of a compound disclosed herein, or a pharmaceutically acceptable salt, pharmaceutically acceptable solvate, or pharmaceutically acceptable prodrug thereof, to the mammal at a frequency and for a duration sufficient to provide a beneficial effect to the mammal.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### DETAILED DESCRIPTION OF THE INVENTION

### Definitions

As used in the specification and the appended claims, the singular forms "a," "an" and "the" include plural referents unless the context clearly dictates otherwise.

The term "about" as used herein, when referring to a numerical value or range, allows for a degree of variability in the value or range, for example, within 10%, or within 5% of a stated value or of a stated limit of a range.

All percent compositions are given as weight-percentages, unless otherwise stated.

All average molecular weights of polymers are weight-average molecular weights, unless otherwise specified.

As used herein, "individual" (as in the subject of the treatment) means both mammals and non-mammals. Mammals include, for example, humans; non-human primates, e.g. apes and monkeys; and non-primates, e.g. dogs, cats, cattle, horses, sheep, and goats. Non-mammals include, for example, fish and birds.

The term "disease" or "disorder" or "malcondition" are used interchangeably, and are used to refer to diseases or conditions wherein a bacterial SPase plays a role in the biochemical mechanisms involved in the disease or malcondition such that a therapeutically beneficial effect can be achieved by acting on the enzyme. "Acting on" SPase can include binding to SPase and/or inhibiting the bioactivity of an SPase.

The expression "effective amount", when used to describe therapy to an individual suffering from a disorder, refers to the amount of a compound described herein that is effective to inhibit or otherwise act on SPase in the individual's tissues wherein SPase involved in the disorder is active, wherein such inhibition or other action occurs to an extent sufficient to produce a beneficial therapeutic effect.

"Substantially" as the term is used herein means completely or almost completely; for example, a composition that is "substantially free" of a component either has none of the component or contains such a trace amount that any relevant functional property of the composition is unaffected by the presence of the trace amount, or a compound is "substantially pure" is there are only negligible traces of impurities present.

"Treating" or "treatment" within the meaning herein refers to an alleviation of symptoms associated with a disorder or disease, or inhibition of further progression or worsening of those symptoms, or prevention or prophylaxis of the disease or disorder, or curing the disease or disorder. Similarly, as used herein, an "effective amount" or a "therapeutically effective amount" of a compound refers to an amount of the compound that alleviates, in whole or in part, symptoms associated with the disorder or condition, or halts or slows further progression or worsening of those symptoms, or prevents or provides prophylaxis for the disorder or condition. In particular, a "therapeutically effective amount" refers to an amount effective, at dosages and for periods of time necessary, to achieve the desired therapeutic result. A therapeutically effective amount is also one in which any toxic or detrimental effects of compounds described herein are outweighed by the therapeutically beneficial effects.

By "chemically feasible" is meant a bonding arrangement or a compound where the generally understood rules of organic structure are not violated; for example a structure within a definition of a claim that would contain in certain situations a pentavalent carbon atom that would not exist in nature would be understood to not be within the claim. The structures disclosed herein, in all of their embodiments are intended to include only "chemically feasible" structures, and any recited structures that are not chemically feasible, for example in a structure shown with variable atoms or groups, are not intended to be disclosed or claimed herein.

When a substituent is specified to be an atom or atoms of specified identity, "or a bond", a configuration is referred to when the substituent is "a bond" that the groups that are immediately adjacent to the specified substituent are directly connected to each other in a chemically feasible bonding configuration.

All chiral, diastereomeric, racemic forms of a structure are intended, unless a particular stereochemistry or isomeric form is specifically indicated. Compounds described herein can include enriched or resolved optical isomers at any or all asymmetric atoms as are apparent from the depictions, at any degree of enrichment. Both racemic and diastereomeric mixtures, as well as the individual optical isomers can be isolated or synthesized so as to be substantially free of their enantiomeric or diastereomeric partners, and these are all within the scope of the invention.

The inclusion of an isotopic form of one or more atoms in a molecule that is different from the naturally occurring isotopic distribution of the atom in nature is referred to as an "isotopically labeled form" of the molecule. All isotopic forms of atoms are included as options in the composition of any molecule, unless a specific isotopic form of an atom is indicated. For example, any hydrogen atom or set thereof in a molecule can be any of the isotopic forms of hydrogen, i.e., protium (¹H), deuterium (²H), or tritium (³H) in any combination. Similarly, any carbon atom or set thereof in a molecule can be any of the isotopic form of carbons, such as ¹¹C, ¹²C, ¹³C, or ¹⁴C, or any nitrogen atom or set thereof in a molecule can be any of the isotopic forms of nitrogen, such as ¹³N, ¹⁴N, or ¹⁵N. A molecule can include any combination of isotopic forms in the component atoms making up the molecule, the isotopic form of every atom forming the molecule being independently selected. In a multi-molecular sample of a compound, not every individual molecule necessarily has the same isotopic composition. For example, a sample of a compound can include molecules containing various different isotopic compositions, such as in a tritium or ¹⁴C radiolabeled sample where only some fraction of the set of molecules making up the macroscopic sample contains a radioactive atom. It is also understood that many elements that are not artificially isotopically enriched themselves are mixtures of naturally occurring isotopic forms, such as ¹⁴N and ¹⁵N, ³²S and ³⁴S, and so forth. A molecule as recited herein is defined as including isotopic forms of all its constituent elements at each position in the molecule. As is well known in the art, isotopically labeled compounds can be prepared by the usual methods of chemical synthesis, except substituting an isotopically labeled precursor molecule. The isotopes, radiolabeled or stable, can be obtained by any method known in the art, such as generation by neutron absorption of a precursor nuclide in a nuclear reactor, by cyclotron reactions, or by isotopic separation such as by mass spectrometry. The isotopic forms are incorporated into precursors as required for use in any particular synthetic route. For example, ¹⁴C and ³H can be prepared using neutrons generated in a nuclear reactor. Following nuclear transformation, ¹⁴C and ³H are incorporated into precursor molecules, followed by further elaboration as needed.

The term "amino protecting group" or "N-protected" as used herein refers to those groups intended to protect an amino group against undesirable reactions during synthetic procedures and which can later be removed to reveal the amine. Commonly used amino protecting groups are disclosed in Protective Groups in Organic Synthesis, Greene, T.W.; Wuts, P. G. M., John Wiley & Sons, New York, NY, (3rd Edition, 1999). Amino protecting groups include acyl groups such as formyl, acetyl, propionyl, pivaloyl, t-butylacetyl, 2-chloroacetyl, 2-bromoacetyl, trifluoroacetyl, trichloroacetyl, o-nitrophenoxyacetyl, α-chlorobutyryl, benzoyl, 4-chlorobenzoyl, 4-bromobenzoyl, 4-nitrobenzoyl, and the like; sulfonyl groups such as benzenesulfonyl, p-toluenesulfonyl and the like; alkoxy- or aryloxy-carbonyl groups (which form urethanes with the protected amine) such as benzyloxycarbonyl (Cbz), p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxycarbonyl, t-butyloxycarbonyl (Boc), diisopropylmethoxycarbonyl, isopropyloxycarbonyl, ethoxycarbonyl, methoxycarbonyl, allyloxycarbonyl (Alloc), 2,2,2-trichloroethoxycarbonyl, 2-trimethylsilylethyloxycarbonyl (Teoc), phenoxycarbonyl, 4-nitrophenoxycarbonyl, fluorenyl-9-methoxycarbonyl (Fmoc), cyclopentyloxycarbonyl, adamantyloxycarbonyl, cyclohexyloxycarbonyl, phenylthiocarbonyl and the like; aralkyl groups such as benzyl, triphenylmethyl, benzyloxymethyl and the like; and silyl groups such as trimethylsilyl and the like. Amine protecting groups also include cyclic amino protecting groups such as phthaloyl and dithiosuccinimidyl, which incorporate the amino nitrogen into a heterocycle. Typically, amino protecting groups include formyl, acetyl, benzoyl, pivaloyl, t-butylacetyl, phenylsulfonyl, Alloc, Teoc, benzyl, Fmoc, Boc and Cbz. It is well within the skill of the ordinary artisan to select and use the appropriate amino protecting group for the synthetic task at hand.

The term "hydroxyl protecting group" or "O-protected" as used herein refers to those groups intended to protect an OH group against undesirable reactions during synthetic procedures and which can later be removed to reveal the amine. Commonly used hydroxyl protecting groups are disclosed in Protective Groups in Organic Synthesis, Greene, T.W.; Wuts, P. G. M., John Wiley & Sons, New York, NY, (3rd Edition, 1999). Hydroxyl protecting groups include acyl groups such as formyl, acetyl, propionyl, pivaloyl, t-butylacetyl, 2-chloroacetyl, 2-bromoacetyl, trifluoroacetyl, trichloroacetyl, o-nitrophenoxyacetyl, α-chlorobutyryl, benzoyl, 4-chlorobenzoyl, 4-bromobenzoyl, 4-nitrobenzoyl, and the like; sulfonyl groups such as benzenesulfonyl, p-toluenesulfonyl and the like; acyloxy groups (which form urethanes with the protected amine) such as benzyloxycarbonyl (Cbz), p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxycarbonyl, t-butyloxycarbonyl (Boc), diisopropylmethoxycarbonyl, isopropyloxycarbonyl, ethoxycarbonyl, methoxycarbonyl, allyloxycarbonyl (Alloc), 2,2,2-trichloroethoxycarbonyl, 2-trimethylsilylethyloxycarbonyl (Teoc), phenoxycarbonyl, 4-nitrophenoxycarbonyl, fluorenyl-9-methoxycarbonyl (Fmoc), cyclopentyloxycarbonyl, adamantyloxycarbonyl, cyclohexyloxycarbonyl, phenylthiocarbonyl and the like; aralkyl groups such as benzyl, triphenylmethyl, benzyloxymethyl and the like; and silyl groups such as trimethylsilyl and the like. It is well within the skill of the ordinary artisan to select and use the appropriate hydroxyl protecting group for the synthetic task at hand.

In general, "substituted" refers to an organic group as defined herein in which one or more bonds to a hydrogen atom contained therein are replaced by one or more bonds to a non-hydrogen atom such as, but not limited to, a halogen (i.e., F, Cl, Br, and I); an oxygen atom in groups such as hydroxyl groups, alkoxy groups, aryloxy groups, aralkyloxy groups, oxo(carbonyl) groups, carboxyl groups including carboxylic acids, carboxylates, and carboxylate esters; a sulfur atom in groups such as thiol groups, alkyl and aryl sulfide groups, sulfoxide groups, sulfone groups, sulfonyl groups, and sulfonamide groups; a nitrogen atom in groups such as amines, hydroxylamines, nitriles, nitro groups, N-oxides, hydrazides, azides, and enamines; and other heteroatoms in various other groups. Non-limiting examples of substituents that can be bonded to a substituted carbon (or other) atom include F, Cl, Br, I, OR', OC(O)N(R')₂, CN, NO, NO₂, ONO₂, azido, CF₃, OCF₃, R', O (oxo), S (thiono), C(O), S(O), methylenedioxy, ethylenedioxy, N(R')₂, SR', SOR', SO₂R', SO₂N(R')₂, SO₃R', C(O)R', C(O)C(O)R', C(O)CH₂C(O)R', C(S)R', C(O)OR', OC(O)R', C(O)N(R')₂, OC(O)N(R')₂, C(S)N(R')₂, (CH₂)_{0- 2}N(R')C(O)R', (CH₂)₀₋₂N(R')N(R')₂, N(R')N(R')C(O)R', N(R')N(R')C(O)OR', N(R')N(R')CON(R')₂, N(R')SO₂R', N(R')SO₂N(R')₂, N(R')C(O)OR', N(R')C(O)R', N(R')C(S)R', N(R')C(O)N(R')₂, N(R')C(S)N(R')₂, N(COR')COR', N(OR')R', C(=NH)N(R')₂, C(O)N(OR')R', or C(=NOR')R' wherein R' can be hydrogen or a carbon-based moiety, and wherein the carbon-based moiety can itself be further substituted.

When a substituent is monovalent, such as, for example, For Cl, it is bonded to the atom it is substituting by a single bond. When a substituent is more than monovalent, such as O, which is divalent, it can be bonded to the atom it is substituting by more than one bond, i.e., a divalent substituent is bonded by a double bond; for example, a C substituted with O forms a carbonyl group, C=O, which can also be written as "CO", "C(O)", or "C(=O)", wherein the C and the O are double bonded. When a carbon atom is substituted with a double-bonded oxygen (=O) group, the oxygen substituent is termed an "oxo" group. When a divalent substituent such as NR is double-bonded to a carbon atom, the resulting C(=NR) group is termed an "imino" group. When a divalent substituent such as S is double-bonded to a carbon atom, the results C(=S) group is termed a "thiocarbonyl" group.

Alternatively, a divalent substituent such as O, S, C(O), S(O), or S(O)₂ can be connected by two single bonds to two different carbon atoms. For example, O, a divalent substituent, can be bonded to each of two adjacent carbon atoms to provide an epoxide group, or the O can form a bridging ether group, termed an "oxy" group, between adjacent or non-adjacent carbon atoms, for example bridging the 1,4-carbons of a cyclohexyl group to form a [2.2.1]-oxabicyclo system. Further, any substituent can be bonded to a carbon or other atom by a linker, such as (CH₂)ₙ or (CR'₂)ₙ wherein n is 1, 2, 3, or more, and each R' is independently selected.

C(O) and S(O)₂ groups can be bound to one or two heteroatoms, such as nitrogen, rather than to a carbon atom. For example, when a C(O) group is bound to one carbon and one nitrogen atom, the resulting group is called an "amide" or "carboxamide." When a C(O) group is bound to two nitrogen atoms, the functional group is termed a urea. When a S(O)₂ group is bound to one carbon and one nitrogen atom, the resulting unit is termed a "sulfonamide." When a S(O)₂ group is bound to two nitrogen atoms, the resulting unit is termed a "sulfamate."

Substituted alkyl, alkenyl, alkynyl, cycloalkyl, and cycloalkenyl groups as well as other substituted groups also include groups in which one or more bonds to a hydrogen atom are replaced by one or more bonds, including double or triple bonds, to a carbon atom, or to a heteroatom such as, but not limited to, oxygen in carbonyl (oxo), carboxyl, ester, amide, imide, urethane, and urea groups; and nitrogen in imines, hydroxyimines, oximes, hydrazones, amidines, guanidines, and nitriles.

Substituted ring groups such as substituted cycloalkyl, aryl, heterocyclyl and heteroaryl groups also include rings and fused ring systems in which a bond to a hydrogen atom is replaced with a bond to a carbon atom. Therefore, substituted cycloalkyl, aryl, heterocyclyl and heteroaryl groups can also be substituted with alkyl, alkenyl, and alkynyl groups as defined herein.

By a "ring system" as the term is used herein is meant a moiety comprising one, two, three or more rings, which can be substituted with non-ring groups or with other ring systems, or both, which can be fully saturated, partially unsaturated, fully unsaturated, or aromatic, and when the ring system includes more than a single ring, the rings can be fused, bridging, or spirocyclic. By "spirocyclic" is meant the class of structures wherein two rings are fused at a single tetrahedral carbon atom, as is well known in the art.

As to any of the groups described herein, which contain one or more substituents, it is understood, of course, that such groups do not contain any substitution or substitution patterns which are sterically impractical and/or synthetically non-feasible. In addition, the compounds of this disclosed subject matter include all stereochemical isomers arising from the substitution of these compounds.

Selected substituents within the compounds described herein are present to a recursive degree. In this context, "recursive substituent" means that a substituent may recite another instance of itself or of another substituent that itself recites the first substituent. Because of the recursive nature of such substituents, theoretically, a large number may be present in any given claim. One of ordinary skill in the art of medicinal chemistry and organic chemistry understands that the total number of such substituents is reasonably limited by the desired properties of the compound intended. Such properties include, by of example and not limitation, physical properties such as molecular weight, solubility or log P, application properties such as activity against the intended target, and practical properties such as ease of synthesis.

Recursive substituents are an intended aspect of the disclosed subject matter. One of ordinary skill in the art of medicinal and organic chemistry understands the versatility of such substituents. To the degree that recursive substituents are present in a claim of the disclosed subject matter, the total number should be determined as set forth above.

Alkyl groups include straight chain and branched alkyl groups and cycloalkyl groups having from 1 to about 20 carbon atoms, and typically from 1 to 12 carbons or, in some embodiments, from 1 to 8 carbon atoms. Examples of straight chain alkyl groups include those with from 1 to 8 carbon atoms such as methyl, ethyl, n-propyl, n-butyl, n-pentyl, n-hexyl, n-heptyl, and n-octyl groups. Examples of branched alkyl groups include, but are not limited to, isopropyl, iso-butyl, sec-butyl, t-butyl, neopentyl, isopentyl, and 2,2-dimethylpropyl groups. As used herein, the term "alkyl" encompasses n-alkyl, isoalkyl, and anteisoalkyl groups as well as other branched chain forms of alkyl. Representative substituted alkyl groups can be substituted one or more times with any of the groups listed above, for example, amino, hydroxy, cyano, carboxy, nitro, thio, alkoxy, and halogen groups.

The term "alkylene" means a linear saturated divalent hydrocarbon radical of one to six carbon atoms or a branched saturated divalent hydrocarbon radical of one to six carbon atoms unless otherwise stated, such as methylene, ethylene, propylene, 1-methylpropylene, 2-methylpropylene, butylene, pentylene, and the like.

The term "carbonyl" means C=O.

The terms "carboxy" and "hydroxycarbonyl" mean COOH.

Cycloalkyl groups are cyclic alkyl groups such as, but not limited to, cyclopropyl, cyclobutyl, cyclopentyl, cyclohexyl, cycloheptyl, and cyclooctyl groups. In some embodiments, the cycloalkyl group can have 3 to about 8-12 ring members, whereas in other embodiments the number of ring carbon atoms range from 3 to 4, 5, 6, or 7. Cycloalkyl groups further include polycyclic cycloalkyl groups such as, but not limited to, norbornyl, adamantyl, bornyl, camphenyl, isocamphenyl, and carenyl groups, and fused rings such as, but not limited to, decalinyl, and the like. Cycloalkyl groups also include rings that are substituted with straight or branched chain alkyl groups as defined above. Representative substituted cycloalkyl groups can be mono-substituted or substituted more than once, such as, but not limited to, 2,2-, 2,3-, 2,4- 2,5- or 2,6-disubstituted cyclohexyl groups or mono-, di- or tri-substituted norbornyl or cycloheptyl groups, which can be substituted with, for example, amino, hydroxy, cyano, carboxy, nitro, thio, alkoxy, and halogen groups. The term "cycloalkenyl" alone or in combination denotes a cyclic alkenyl group.

The terms "carbocyclic," "carbocyclyl," and "carbocycle" denote a ring structure wherein the atoms of the ring are carbon, such as a cycloalkyl group or an aryl group. In some embodiments, the carbocycle has 3 to 8 ring members, whereas in other embodiments the number of ring carbon atoms is 4, 5, 6, or 7. Unless specifically indicated to the contrary, the carbocyclic ring can be substituted with as many as N-1 substituents wherein N is the size of the carbocyclic ring with, for example, alkyl, alkenyl, alkynyl, amino, aryl, hydroxy, cyano, carboxy, heteroaryl, heterocyclyl, nitro, thio, alkoxy, and halogen groups, or other groups as are listed above. A carbocyclyl ring can be a cycloalkyl ring, a cycloalkenyl ring, or an aryl ring. A carbocyclyl can be monocyclic or polycyclic, and if polycyclic each ring can be independently be a cycloalkyl ring, a cycloalkenyl ring, or an aryl ring.

(Cycloalkyl)alkyl groups, also denoted cycloalkylalkyl, are alkyl groups as defined above in which a hydrogen or carbon bond of the alkyl group is replaced with a bond to a cycloalkyl group as defined above.

Alkenyl groups include straight and branched chain and cyclic alkyl groups as defined above, except that at least one double bond exists between two carbon atoms. Thus, alkenyl groups have from 2 to about 20 carbon atoms, and typically from 2 to 12 carbons or, in some embodiments, from 2 to 8 carbon atoms. Examples include, but are not limited to
vinyl, -CH=CH(CH₃), -CH=C(CH₃)₂, -C(CH₃)=CH₂, -C(CH₃)=CH(CH₃), -C(CH₂CH₃)=CH₂, cyclohexenyl, cyclopentenyl, cyclohexadienyl, butadienyl, pentadienyl, and hexadienyl among others.

Cycloalkenyl groups include cycloalkyl groups having at least one double bond between 2 carbons. Thus for example, cycloalkenyl groups include but are not limited to cyclohexenyl, cyclopentenyl, and cyclohexadienyl groups. Cycloalkenyl groups can have from 3 to about 8-12 ring members, whereas in other embodiments the number of ring carbon atoms range from 3 to 5, 6, or 7. Cycloalkyl groups further include polycyclic cycloalkyl groups such as, but not limited to, norbornyl, adamantyl, bornyl, camphenyl, isocamphenyl, and carenyl groups, and fused rings such as, but not limited to, decalinyl, and the like, provided they include at least one double bond within a ring. Cycloalkenyl groups also include rings that are substituted with straight or branched chain alkyl groups as defined above.

(Cycloalkenyl)alkyl groups are alkyl groups as defined above in which a hydrogen or carbon bond of the alkyl group is replaced with a bond to a cycloalkenyl group as defined above.

Alkynyl groups include straight and branched chain alkyl groups, except that at least one triple bond exists between two carbon atoms. Thus, alkynyl groups have from 2 to about 20 carbon atoms, and typically from 2 to 12 carbons or, in some embodiments, from 2 to 8 carbon atoms. Examples include, but are not limited to -C≡CH, -C≡C(CH₃), -C≡C(CH₂CH₃), -CH₂C≡CH, -CH₂C≡C(CH₃), and -CH₂C≡C(CH₂CH₃) among others.

The term "heteroalkyl" by itself or in combination with another term means, unless otherwise stated, a stable straight or branched chain alkyl group consisting of the stated number of carbon atoms and one or two heteroatoms selected from the group consisting of O, N, and S, and wherein the nitrogen and sulfur atoms may be optionally oxidized and the nitrogen heteroatom may be optionally quaternized. The heteroatom(s) may be placed at any position of the heteroalkyl group, including between the rest of the heteroalkyl group and the fragment to which it is attached, as well as attached to the most distal carbon atom in the heteroalkyl group. Examples
include: -O-CH₂-CH₂-CH₃, -CH₂-CH₂CH₂-OH, -CH₂-CH₂-NH-CH₃, -CH₂-S-CH₂-CH₃, -CH₂CH₂-S(=O)-C H₃, and -CH₂CH₂-O-CH₂CH₂-O-CH₃. Up to two heteroatoms may be consecutive, such as, for
example, -CH₂-NH-OCH₃, or -CH₂-CH₂-S-S-CH₃.

A "heterocycloalkyl" ring is a cycloalkyl ring containing at least one heteroatom. A heterocycloalkyl ring can also be termed a "heterocyclyl," described below.

The term "heteroalkenyl" by itself or in combination with another term means, unless otherwise stated, a stable straight or branched chain monounsaturated or di-unsaturated hydrocarbon group consisting of the stated number of carbon atoms and one or two heteroatoms selected from the group consisting of O, N, and S, and wherein the nitrogen and sulfur atoms may optionally be oxidized and the nitrogen heteroatom may optionally be quaternized. Up to two heteroatoms may be placed consecutively. Examples include -CH=CH-O-CH₃, -CH=CH-CH₂-OH, -CH₂-CH=N-OCH₃, -CH=CH-N(CH₃)-CH₃, -CH₂-CH=CH-CH₂-SH, and and -CH=CH-O-CH₂CH₂-O-CH₃.

Aryl groups are cyclic aromatic hydrocarbons that do not contain heteroatoms in the ring. Thus aryl groups include, but are not limited to, phenyl, azulenyl, heptalenyl, biphenyl, indacenyl, fluorenyl, phenanthrenyl, triphenylenyl, pyrenyl, naphthacenyl, chrysenyl, biphenylenyl, anthracenyl, and naphthyl groups. In some embodiments, aryl groups contain about 6 to about 14 carbons in the ring portions of the groups. Aryl groups can be unsubstituted or substituted, as defined above. Representative substituted aryl groups can be mono-substituted or substituted more than once, such as, but not limited to, 2-, 3-, 4-, 5-, or 6-substituted phenyl or 2-8 substituted naphthyl groups, which can be substituted with carbon or non-carbon groups such as those listed above.

Aralkyl groups are alkyl groups as defined above in which a hydrogen or carbon bond of an alkyl group is replaced with a bond to an aryl group as defined above. Representative aralkyl groups include benzyl and phenylethyl groups and fused (cycloalkylaryl)alkyl groups such as 4-ethyl-indanyl. Aralkenyl group are alkenyl groups as defined above in which a hydrogen or carbon bond of an alkyl group is replaced with a bond to an aryl group as defined above.

Heterocyclyl groups or the term "heterocyclyl" includes aromatic and non-aromatic ring compounds containing 3 or more ring members, of which, one or more is a heteroatom such as, but not limited to, N, O, and S. Thus a heterocyclyl can be a heterocycloalkyl, or a heteroaryl, or if polycyclic, any combination thereof. In some embodiments, heterocyclyl groups include 3 to about 20 ring members, whereas other such groups have 3 to about 15 ring members. A heterocyclyl group designated as a C₂-heterocyclyl can be a 5-ring with two carbon atoms and three heteroatoms, a 6-ring with two carbon atoms and four heteroatoms and so forth. Likewise a C₄-heterocyclyl can be a 5-ring with one heteroatom, a 6-ring with two heteroatoms, and so forth. The number of carbon atoms plus the number of heteroatoms sums up to equal the total number of ring atoms. A heterocyclyl ring can also include one or more double bonds. A heteroaryl ring is an embodiment of a heterocyclyl group. The phrase "heterocyclyl group" includes fused ring species including those comprising fused aromatic and non-aromatic groups. For example, a dioxolanyl ring and a benzdioxolanyl ring system (methylenedioxyphenyl ring system) are both heterocyclyl groups within the meaning herein. The phrase also includes polycyclic ring systems containing a heteroatom such as, but not limited to, quinuclidyl. Heterocyclyl groups can be unsubstituted, or can be substituted as discussed above. Heterocyclyl groups include, but are not limited to, pyrrolidinyl, piperidinyl, piperazinyl, morpholinyl, pyrrolyl, pyrazolyl, triazolyl, tetrazolyl, oxazolyl, isoxazolyl, thiazolyl, pyridinyl, thiophenyl, benzothiophenyl, benzofuranyl, dihydrobenzofuranyl, indolyl, dihydroindolyl, azaindolyl, indazolyl, benzimidazolyl, azabenzimidazolyl, benzoxazolyl, benzothiazolyl, benzothiadiazolyl, imidazopyridinyl, isoxazolopyridinyl, thianaphthalenyl, purinyl, xanthinyl, adeninyl, guaninyl, quinolinyl, isoquinolinyl, tetrahydroquinolinyl, quinoxalinyl, and quinazolinyl groups. Representative substituted heterocyclyl groups can be mono-substituted or substituted more than once, such as, but not limited to, piperidinyl or quinolinyl groups, which are 2-, 3-, 4-, 5-, or 6-substituted, or disubstituted with groups such as those listed above.

Heteroaryl groups are aromatic ring compounds containing 5 or more ring members, of which, one or more is a heteroatom such as, but not limited to, N, O, and S; for instance, heteroaryl rings can have 5 to about 8-12 ring members. A heteroaryl group is a variety of a heterocyclyl group that possesses an aromatic electronic structure. A heteroaryl group designated as a C₂-heteroaryl can be a 5-ring with two carbon atoms and three heteroatoms, a 6-ring with two carbon atoms and four heteroatoms and so forth. Likewise a C₄-heteroaryl can be a 5-ring with one heteroatom, a 6-ring with two heteroatoms, and so forth. The number of carbon atoms plus the number of heteroatoms sums up to equal the total number of ring atoms. Heteroaryl groups include, but are not limited to, groups such as pyrrolyl, pyrazolyl, triazolyl, tetrazolyl, oxazolyl, isoxazolyl, thiazolyl, pyridinyl, thiophenyl, benzothiophenyl, benzofuranyl, indolyl, azaindolyl, indazolyl, benzimidazolyl, azabenzimidazolyl, benzoxazolyl, benzothiazolyl, benzothiadiazolyl, imidazopyridinyl, isoxazolopyridinyl, thianaphthalenyl, purinyl, xanthinyl, adeninyl, guaninyl, quinolinyl, isoquinolinyl, tetrahydroquinolinyl, quinoxalinyl, and quinazolinyl groups. Heteroaryl groups can be unsubstituted, or can be substituted with groups as is discussed above. Representative substituted heteroaryl groups can be substituted one or more times with groups such as those listed above.

Additional examples of aryl and heteroaryl groups include but are not limited to phenyl, biphenyl, indenyl, naphthyl (1-naphthyl, 2-naphthyl), N-hydroxytetrazolyl, N-hydroxytriazolyl, N-hydroxyimidazolyl, anthracenyl (1-anthracenyl, 2-anthracenyl, 3-anthracenyl), thiophenyl (2-thienyl, 3-thienyl), furyl (2-furyl, 3-furyl), indolyl, oxadiazolyl, isoxazolyl, quinazolinyl, fluorenyl, xanthenyl, isoindanyl, benzhydryl, acridinyl, thiazolyl, pyrrolyl (2-pyrrolyl), pyrazolyl (3-pyrazolyl), imidazolyl (1-imidazolyl, 2-imidazolyl, 4-imidazolyl, 5-imidazolyl), triazolyl (1,2,3-triazol-1-yl, 1,2,3-triazol-2-yl 1,2,3-triazol-4-yl, 1,2,4-triazol-3-yl), oxazolyl (2-oxazolyl, 4-oxazolyl, 5-oxazolyl), thiazolyl (2-thiazolyl, 4-thiazolyl, 5-thiazolyl), pyridyl (2-pyridyl, 3-pyridyl, 4-pyridyl), pyrimidinyl (2-pyrimidinyl, 4-pyrimidinyl, 5-pyrimidinyl, 6-pyrimidinyl), pyrazinyl, pyridazinyl (3- pyridazinyl, 4-pyridazinyl, 5-pyridazinyl), quinolyl (2-quinolyl, 3-quinolyl, 4-quinolyl, 5-quinolyl, 6-quinolyl, 7-quinolyl, 8-quinolyl), isoquinolyl (1-isoquinolyl, 3-isoquinolyl, 4-isoquinolyl, 5-isoquinolyl, 6-isoquinolyl, 7-isoquinolyl, 8-isoquinolyl), benzo[b]furanyl (2-benzo[b]furanyl, 3-benzo[b]furanyl, 4-benzo[b]furanyl, 5-benzo[b]furanyl, 6-benzo[b]furanyl, 7-benzo[b]furanyl), 2,3-dihydro-benzo[b]furanyl (2-(2,3-dihydro-benzo[b]furanyl), 3-(2,3-dihydro-benzo[b]furanyl), 4-(2,3-dihydro-benzo[b]furanyl), 5-(2,3-dihydro-benzo[b]furanyl), 6-(2,3-dihydro-benzo[b]furanyl), 7-(2,3-dihydro-benzo[b]furanyl), benzo[b]thiophenyl (2-benzo[b]thiophenyl, 3-benzo[b]thiophenyl, 4-benzo[b]thiophenyl, 5-benzo[b]thiophenyl, 6-benzo[b]thiophenyl, 7-benzo[b]thiophenyl), 2,3-dihydro-benzo[b]thiophenyl, (2-(2,3-dihydro-benzo[b]thiophenyl), 3-(2,3-dihydro-benzo[b]thiophenyl), 4-(2,3-dihydro-benzo[b]thiophenyl), 5-(2,3-dihydro-benzo[b]thiophenyl), 6-(2,3-dihydro-benzo[b]thiophenyl), 7-(2,3-dihydro-benzo[b]thiophenyl), indolyl (1-indolyl, 2-indolyl, 3-indolyl, 4-indolyl, 5-indolyl, 6-indolyl, 7-indolyl), indazole (1-indazolyl, 3-indazolyl, 4-indazolyl, 5-indazolyl, 6-indazolyl, 7-indazolyl), benzimidazolyl (1-benzimidazolyl, 2-benzimidazolyl, 4-benzimidazolyl, 5-benzimidazolyl, 6-benzimidazolyl, 7-benzimidazolyl, 8-benzimidazolyl), benzoxazolyl (1-benzoxazolyl, 2-benzoxazolyl), benzothiazolyl (1-benzothiazolyl, 2-benzothiazolyl, 4-benzothiazolyl, 5-benzothiazolyl, 6-benzothiazolyl, 7-benzothiazolyl), carbazolyl (1-carbazolyl, 2-carbazolyl, 3-carbazolyl, 4-carbazolyl), 5H-dibenz[b,f]azepine (5H-dibenz[b,f]azepin-1-yl, 5H-dibenz[b,f]azepine-2-yl, 5H-dibenz[b,f]azepine-3-yl, 5H-dibenz[b,f]azepine-4-yl, 5H-dibenz[b,f]azepine-5-yl), 10,11-dihydro-5H-dibenz[b,f]azepine (10,11-dihydro-5H-dibenz[b,f]azepine-1-yl, 10,11-dihydro-5H-dibenz[b,f]azepine-2-yl, 10,11-dihydro-5H-dibenz[b,f]azepine-3-yl, 10,11-dihydro-5H-dibenz[b,f]azepine-4-yl, 10,11-dihydro-5H-dibenz[b,f]azepine-5-yl), and the like.

Heterocyclylalkyl groups are alkyl groups as defined above in which a hydrogen or carbon bond of an alkyl group as defined above is replaced with a bond to a heterocyclyl group as defined above. Representative heterocyclyl alkyl groups include, but are not limited to, furan-2-yl methyl, furan-3-yl methyl, pyridine-3-yl methyl, tetrahydrofuran-2-yl ethyl, and indol-2-yl propyl.

Heteroarylalkyl groups are alkyl groups as defined above in which a hydrogen or carbon bond of an alkyl group is replaced with a bond to a heteroaryl group as defined above.

The term "alkoxy" refers to an oxygen atom connected to an alkyl group, including a cycloalkyl group, as are defined above. Examples of linear alkoxy groups include but are not limited to methoxy, ethoxy, propoxy, butoxy, pentyloxy, hexyloxy, and the like. Examples of branched alkoxy include but are not limited to isopropoxy, sec-butoxy, tert-butoxy, isopentyloxy, isohexyloxy, and the like. Examples of cyclic alkoxy include but are not limited to cyclopropyloxy, cyclobutyloxy, cyclopentyloxy, cyclohexyloxy, and the like. An alkoxy group can include one to about 12-20 carbon atoms bonded to the oxygen atom, and can further include double or triple bonds, and can also include heteroatoms. For example, an allyloxy group is an alkoxy group within the meaning herein. A methoxyethoxy group is also an alkoxy group within the meaning herein, as is a methylenedioxy group in a context where two adjacent atoms of a structures are substituted therewith.

The term "thioalkoxy" refers to an alkyl group previously defined attached to the parent molecular moiety through a sulfur atom.

The term "glycosyloxyoxy" refers to a glycoside attached to the parent molecular moiety through an oxygen atom.

The term "alkoxycarbonyl" represents as ester group; i.e. an alkoxy group, attached to the parent molecular moiety through a carbonyl group such as methoxycarbonyl, ethoxycarbonyl, and the like.

The terms "halo" or "halogen" or "halide" by themselves or as part of another substituent mean, unless otherwise stated, a fluorine, chlorine, bromine, or iodine atom, preferably, fluorine, chlorine, or bromine.

A "haloalkyl" group includes mono-halo alkyl groups, poly-halo alkyl groups wherein all halo atoms can be the same or different, and per-halo alkyl groups, wherein all hydrogen atoms are replaced by halogen atoms, such as fluoro. Examples of haloalkyl include trifluoromethyl, 1,1-dichloroethyl, 1,2-dichloroethyl, 1,3-dibromo-3,3-difluoropropyl, perfluorobutyl, and the like.

A "haloalkoxy" group includes mono-halo alkoxy groups, poly-halo alkoxy groups wherein all halo atoms can be the same or different, and per-halo alkoxy groups, wherein all hydrogen atoms are replaced by halogen atoms, such as fluoro. Examples of haloalkoxy include trifluoromethoxy, 1,1-dichloroethoxy, 1,2-dichloroethoxy, 1,3-dibromo-3,3-difluoropropoxy, perfluorobutoxy, and the like.

The term "(Cₓ-C_{y})perfluoroalkyl," wherein x < y, means an alkyl group with a minimum of x carbon atoms and a maximum of y carbon atoms, wherein all hydrogen atoms are replaced by fluorine atoms. Preferred is -(C₁-C₆)perfluoroalkyl, more preferred is -(C₁-C₃)perfluoroalkyl, most preferred is - CF₃.

The term "(Cₓ-C_{y})perfluoroalkylene," wherein x < y, means an alkyl group with a minimum of x carbon atoms and a maximum of y carbon atoms, wherein all hydrogen atoms are replaced by fluorine atoms. Preferred is -(C₁-C₆)perfluoroalkylene, more preferred is -(C₁-C₃)perfluoroalkylene, most preferred is -CF₂-.

The terms "aryloxy" and "arylalkoxy" refer to, respectively, an aryl group bonded to an oxygen atom and an aralkyl group bonded to the oxygen atom at the alkyl moiety. Examples include but are not limited to phenoxy, naphthyloxy, and benzyloxy.

An "acyl" group as the term is used herein refers to a group containing a carbonyl moiety wherein the group is bonded via the carbonyl carbon atom. The carbonyl carbon atom is also bonded to another carbon atom, which can be part of an alkyl, aryl, aralkyl cycloalkyl, cycloalkylalkyl, heterocyclyl, heterocyclylalkyl, heteroaryl, heteroarylalkyl group or the like. In the special case wherein the carbonyl carbon atom is bonded to a hydrogen, the group is a "formyl" group, an acyl group as the term is defined herein. An acyl group can include 0 to about 12-20 additional carbon atoms bonded to the carbonyl group. An acyl group can include double or triple bonds within the meaning herein. An acryloyl group is an example of an acyl group. An acyl group can also include heteroatoms within the meaning here. A nicotinoyl group (pyridyl-3-carbonyl) group is an example of an acyl group within the meaning herein. Other examples include acetyl, benzoyl, phenylacetyl, pyridylacetyl, cinnamoyl, and acryloyl groups and the like. When the group containing the carbon atom that is bonded to the carbonyl carbon atom contains a halogen, the group is termed a "haloacyl" group. An example is a trifluoroacetyl group.

The term "amine" includes primary, secondary, and tertiary amines having, e.g., the formula N(group)₃ wherein each group can independently be H or non-H, such as alkyl, aryl, and the like. Amines include but are not limited to R-NH₂, for example, alkylamines, arylamines, alkylarylamines; R₂NH wherein each R is independently selected, such as dialkylamines, diarylamines, aralkylamines, heterocyclylamines and the like; and R₃N wherein each R is independently selected, such as trialkylamines, dialkylarylamines, alkyldiarylamines, triarylamines, and the like. The term "amine" also includes ammonium ions as used herein.

An "amino" group is a substituent of the form -NH₂, -NHR, -NR₂, -NR₃⁺, wherein each R is independently selected, and protonated forms of each, except for -NR₃⁺, which cannot be protonated. Accordingly, any compound substituted with an amino group can be viewed as an amine. An "amino group" within the meaning herein can be a primary, secondary, tertiary or quaternary amino group. An "alkylamino" group includes a monoalkylamino, dialkylamino, and trialkylamino group.

An "ammonium" ion includes the unsubstituted ammonium ion NH₄⁺, but unless otherwise specified, it also includes any protonated or quaternarized forms of amines. Thus, trimethylammonium hydrochloride and tetramethylammonium chloride are both ammonium ions, and amines, within the meaning herein.

The term "amide" (or "amido") includes C- and N-amide groups, i.e., -C(O)NR₂, and -NRC(O)R groups, respectively. Amide groups therefore include but are not limited to primary carboxamide groups (-C(O)NH₂) and formamide groups (-NHC(O)H). A "carboxamido" or "aminocarbonyl" group is a group of the formula C(O)NR₂, wherein R can be H, alkyl, aryl, etc.

The term "azido" refers to an N₃ group. An "azide" can be an organic azide or can be a salt of the azide (N₃⁻) anion. The term "nitro" refers to an NO₂ group bonded to an organic moiety. The term "nitroso" refers to an NO group bonded to an organic moiety. The term nitrate refers to an ONO₂ group bonded to an organic moiety or to a salt of the nitrate (NO₃⁻) anion.

The term "urethane" ("carbamoyl" or "carbamyl") includes N- and O-urethane groups, i.e., -NRC(O)OR and -OC(O)NR₂ groups, respectively.

The term "sulfonamide" (or "sulfonamido") includes S- and N-sulfonamide groups, i.e., -SO₂NR₂ and -NRSO₂R groups, respectively. Sulfonamide groups therefore include but are not limited to sulfamoyl groups (-SO₂NH₂). An organosulfur structure represented by the formula -S(O)(NR)- is understood to refer to a sulfoximine, wherein both the oxygen and the nitrogen atoms are bonded to the sulfur atom, which is also bonded to two carbon atoms.

The term "amidine" or "amidino" includes groups of the formula -C(NR)NR₂. Typically, an amidino group is -C(NH)NH₂.

The term "guanidine" or "guanidino" includes groups of the formula -NRC(NR)NR₂. Typically, a guanidino group is -NHC(NH)NH₂.

The term "ring derived from a sugar" refers to a compound that forms a ring by removing the hydrogen atoms from two hydroxyl groups of any sugar.

A "salt" as is well known in the art includes an organic compound such as a carboxylic acid, a sulfonic acid, or an amine, in ionic form, in combination with a counterion. For example, acids in their anionic form can form salts with cations such as metal cations, for example sodium, potassium, and the like; with ammonium salts such as NH₄⁺ or the cations of various amines, including tetraalkyl ammonium salts such as tetramethylammonium, or other cations such as trimethylsulfonium, and the like. A "pharmaceutically acceptable" or "pharmacologically acceptable" salt is a salt formed from an ion that has been approved for human consumption and is generally non-toxic, such as a chloride salt or a sodium salt. A "zwitterion" is an internal salt such as can be formed in a molecule that has at least two ionizable groups, one forming an anion and the other a cation, which serve to balance each other. For example, amino acids such as glycine can exist in a zwitterionic form. A "zwitterion" is a salt within the meaning herein. The compounds described herein may take the form of salts. The term "salts" embraces addition salts of free acids or free bases which are compounds described herein. Salts can be "pharmaceutically-acceptable salts." The term "pharmaceutically-acceptable salt" refers to salts which possess toxicity profiles within a range that affords utility in pharmaceutical applications. Pharmaceutically unacceptable salts may nonetheless possess properties such as high crystallinity, which have utility in the practice of the present disclosure, such as for example utility in process of synthesis, purification or formulation of compounds of the present disclosure.

Suitable pharmaceutically-acceptable acid addition salts may be prepared from an inorganic acid or from an organic acid. Examples of inorganic acids include hydrochloric, hydrobromic, hydriodic, nitric, carbonic, sulfuric, and phosphoric acids. Appropriate organic acids may be selected from aliphatic, cycloaliphatic, aromatic, araliphatic, heterocyclic, carboxylic and sulfonic classes of organic acids, examples of which include formic, acetic, propionic, succinic, glycolic, gluconic, lactic, malic, tartaric, citric, ascorbic, glucuronic, maleic, fumaric, pyruvic, aspartic, glutamic, benzoic, anthranilic, 4-hydroxybenzoic, phenylacetic, mandelic, embonic (pamoic), methanesulfonic, ethanesulfonic, benzenesulfonic, pantothenic, trifluoromethanesulfonic, 2-hydroxyethanesulfonic, p-toluenesulfonic, sulfanilic, cyclohexylaminosulfonic, stearic, alginic, β-hydroxybutyric, salicylic, galactaric and galacturonic acid. Examples of pharmaceutically unacceptable acid addition salts include, for example, perchlorates and tetrafluoroborates.

Suitable pharmaceutically acceptable base addition salts of compounds of the present disclosure include, for example, metallic salts including alkali metal, alkaline earth metal and transition metal salts such as, for example, calcium, magnesium, potassium, sodium and zinc salts. Pharmaceutically acceptable base addition salts also include organic salts made from basic amines such as, for example, N,N'-dibenzylethylenediamine, chloroprocaine, choline, diethanolamine, ethylenediamine, meglumine (N-methylglucamine) and procaine. Examples of pharmaceutically unacceptable base addition salts include lithium salts and cyanate salts. Although pharmaceutically unacceptable salts are not generally useful as medicaments, such salts may be useful, for example as intermediates in the synthesis of Formula (I), (Ia)-(If), (II), (IIa)-(IIe), (III), (IIIa)-(IIIc), (IV), (IVa)-(IVc), (V), or (Va)-(Vc) compounds, for example in their purification by recrystallization. All of these salts may be prepared by conventional means from the corresponding compound according to Formula (I), (1a)-(If), (II), (IIa)-(IIe), (III), (IIIa)-(IIIc), (IV), (IVa)-(IVc), (V), or (Va)-(Vc) by reacting, for example, the appropriate acid or base with the compound according to Formula (I), (1a)-(If), (II), (IIa)-(IIe), (III), (IIIa)-(IIIc), (IV), (IVa)-(IVc), (V), or (Va)-(Vc). The term "pharmaceutically acceptable salts" refers to nontoxic inorganic or organic acid and/or base addition salts, see, for example, Lit et al., Salt Selection for Basic Drugs (1986), Int J. Pharm., 33, 201-217, incorporated by reference herein.

A "hydrate" is a compound that exists in a composition with water molecules. The composition can include water in stoichiometic quantities, such as a monohydrate or a dihydrate, or can include water in random amounts. As the term is used herein a "hydrate" refers to a solid form, i.e., a compound in water solution, while it may be hydrated, is not a hydrate as the term is used herein.

A "solvate" is a similar composition except that a solvent other that water replaces the water. For example, methanol or ethanol can form an "alcoholate", which can again be stoichiometic or non-stoichiometric. As the term is used herein a "solvate" refers to a solid form, i.e., a compound in solution in a solvent, while it may be solvated, is not a solvate as the term is used herein.

A "prodrug" as is well known in the art is a substance that can be administered to a patient where the substance is converted in vivo by the action of biochemicals within the patients body, such as enzymes, to the active pharmaceutical ingredient. Examples of prodrugs include esters of carboxylic acid groups, which can be hydrolyzed by endogenous esterases as are found in the bloodstream of humans and other mammals. Further examples examples of prodrugs include boronate esters which can be hydrolyzed under physiological conditions to afford the corresponding boronic acid. Conventional procedures for the selection and preparation of suitable prodrug derivatives are described, for example, in "Design of Prodrugs", ed. H. Bundgaard, Elsevier, 1985.

In addition, where features or aspects of the present disclosure are described in terms of Markush groups, those skilled in the art will recognize that the presently described compounds is also thereby described in terms of any individual member or subgroup of members of the Markush group. For example, if X is described as selected from the group consisting of bromine, chlorine, and iodine, claims for X being bromine and claims for X being bromine and chlorine are fully described. Moreover, where features or aspects of the present disclosure are described in terms of Markush groups, those skilled in the art will recognize that the present disclosure is also thereby described in terms of any combination of individual members or subgroups of members of Markush groups. Thus, for example, if X is described as selected from the group consisting of bromine, chlorine, and iodine, and Y is described as selected from the group consisting of methyl, ethyl, and propyl, claims for X being bromine and Y being methyl are fully described.

If a value of a variable that is necessarily an integer, e.g., the number of carbon atoms in an alkyl group or the number of substituents on a ring, is described as a range, e.g., 0-4, what is meant is that the value can be any integer between 0 and 4 inclusive, i.e., 0, 1, 2, 3, or 4.

In various embodiments, the compound or set of compounds, such as are used in the inventive methods, can be any one of any of the combinations and/or sub-combinations of the above-listed embodiments.

In various embodiments, a compound as shown in any of the Examples, or among the exemplary compounds, is provided.

Provisos may apply to any of the disclosed categories or embodiments wherein any one or more of the other above disclosed embodiments or species may be excluded from such categories or embodiments.

The present disclosure further embraces isolated compounds according to Formula (I), (1a)-(If), (II), (IIa)-(IIe), (III), (IIIa)-(IIIc), (IV), (IVa)-(IVc), (V), or (Va)-(Vc). The expression "isolated compound" refers to a preparation of a compound of Formula (I), (1a)-(If), (II), (IIa)-(IIe), (III), (IIIa)-(IIIc), (IV), (IVa)-(IVc), (V), or (Va)-(Vc), or a mixture of compounds according to Formula (I), (Ia)-(If), (II), (IIa)-(IIe), (III), (IIIa)-(IIIc), (IV), (IVa)-(IVc), (V), or (Va)-(Vc), wherein the isolated compound has been separated from the reagents used, and/or byproducts formed, in the synthesis of the compound or compounds. "Isolated" does not mean that the preparation is technically pure (homogeneous), but it is sufficiently pure to compound in a form in which it can be used therapeutically. Preferably an "isolated compound" refers to a preparation of a compound of Formula (I), (1a)-(If), (II), (IIa)-(IIe), (III), (IIIa)-(IIIc), (IV), (IVa)-(IVc), (V), or (Va)-(Vc) or a mixture of compounds according to Formula (I), (Ia)-(If), (II), (IIa)-(IIe), (III), (IIIa)-(IIIc), (IV), (IVa)-(IVc), (V), or (Va)-(Vc), which contains the named compound or mixture of compounds according to Formula (I), (1a)-(If), (II), (IIa)-(IIe), (III), (IIIa)-(IIIc), (IV), (IVa)-(IVc), (V), or (Va)-(Vc) in an amount of at least 10 percent by weight of the total weight. Preferably the preparation contains the named compound or mixture of compounds in an amount of at least 50 percent by weight of the total weight; more preferably at least 80 percent by weight of the total weight; and most preferably at least 90 percent, at least 95 percent or at least 98 percent by weight of the total weight of the preparation.

The compounds described herein and intermediates may be isolated from their reaction mixtures and purified by standard techniques such as filtration, liquid-liquid extraction, solid phase extraction, distillation, recrystallization or chromatography, including flash column chromatography, or HPLC.

### Isomerism and Tautomerism in Compounds Described Herein

### Tautomerism

Within the present disclosure it is to be understood that a compound of Formula (I), (1a)-(If), (II), (IIa)-(IIe), (III), (IIIa)-(IIIc), (IV), (IVa)-(IVc), (V), or (Va)-(Vc) or a salt thereof may exhibit the phenomenon of tautomerism whereby two chemical compounds that are capable of facile interconversion by exchanging a hydrogen atom between two atoms, to either of which it forms a covalent bond. Since the tautomeric compounds exist in mobile equilibrium with each other they may be regarded as different isomeric forms of the same compound. It is to be understood that the formulae drawings within this specification can represent only one of the possible tautomeric forms. However, it is also to be understood that the present disclosure encompasses any tautomeric form, and is not to be limited merely to any one tautomeric form utilized within the formulae drawings. The formulae drawings within this specification can represent only one of the possible tautomeric forms and it is to be understood that the specification encompasses all possible tautomeric forms of the compounds drawn not just those forms which it has been convenient to show graphically herein. For example, tautomerism may be exhibited by a pyrazolyl group bonded as indicated by the wavy line. While both substituents would be termed a 4-pyrazolyl group, it is evident that a different nitrogen atom bears the hydrogen atom in each structure.

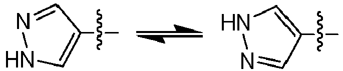

Such tautomerism can also occur with substituted pyrazoles such as 3-methyl, 5-methyl, or 3,5-dimethylpyrazoles, and the like. Another example of tautomerism is amido-imido (lactam-lactim when cyclic) tautomerism, such as is seen in heterocyclic compounds bearing a ring oxygen atom adjacent to a ring nitrogen atom. For example, the equilibrium: 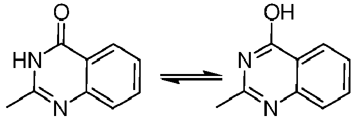 is an example of tautomerism. Accordingly, a structure depicted herein as one tautomer is intended to also include the other tautomer.

### Optical Isomerism

It will be understood that when compounds of the present disclosure contain one or more chiral centers, the compounds may exist in, and may be isolated as pure enantiomeric or diastereomeric forms or as racemic mixtures. The present disclosure therefore includes any possible enantiomers, diastereomers, racemates or mixtures thereof of the compounds described herein.

The isomers resulting from the presence of a chiral center comprise a pair of non-superimposable isomers that are called "enantiomers." Single enantiomers of a pure compound are optically active, *i.e*., they are capable of rotating the plane of plane polarized light. Single enantiomers are designated according to the *Cahn-Ingold-Prelog* system. The priority of substituents is ranked based on atomic weights, a higher atomic weight, as determined by the systematic procedure, having a higher priority ranking. Once the priority ranking of the four groups is determined, the molecule is oriented so that the lowest ranking group is pointed away from the viewer. Then, if the descending rank order of the other groups proceeds clockwise, the molecule is designated (*R*) and if the descending rank of the other groups proceeds counterclockwise, the molecule is designated (*S*). In the example below, the *Cahn-Ingold-Prelog* ranking is A > B > C > D. The lowest ranking atom, D is oriented away from the viewer.

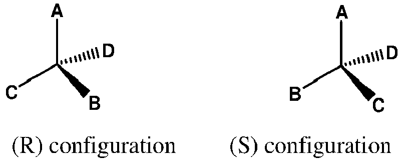

The present disclosure is meant to encompass diastereomers as well as their racemic and resolved, diastereomerically and enantiomerically pure forms and salts thereof. Diastereomeric pairs may be resolved by known separation techniques including normal and reverse phase chromatography, and crystallization.

"Isolated optical isomer" means a compound which has been substantially purified from the corresponding optical isomer(s) of the same formula. Preferably, the isolated isomer is at least about 80%, more preferably at least 90% pure, even more preferably at least 98% pure, most preferably at least about 99% pure, by weight.

Isolated optical isomers may be purified from racemic mixtures by well-known chiral separation techniques. According to one such method, a racemic mixture of a compound described herein, or a chiral intermediate thereof, is separated into 99% wt.% pure optical isomers by HPLC using a suitable chiral column, such as a member of the series of DAICEL^{®} CHIRALPAK^{®} family of columns (Daicel Chemical Industries, Ltd., Tokyo, Japan). The column is operated according to the manufacturer's instructions.

### Rotational Isomerism

It is understood that due to chemical properties (*i.e*., resonance lending some double bond character to the C-N bond) of restricted rotation about the amide bond linkage (as illustrated below) it is possible to observe separate rotamer species and even, under some circumstances, to isolate such species (see below). It is further understood that certain structural elements, including steric bulk or substituents on the amide nitrogen, may enhance the stability of a rotamer to the extent that a compound may be isolated as, and exist indefinitely, as a single stable rotamer. The present disclosure therefore includes any possible stable rotamers of formula (I) which are biologically active in the treatment of cancer or other proliferative disease states.

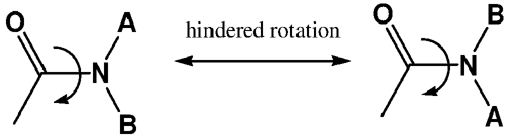

### Regioisomerism

In some embodiments, the compounds described herein have a particular spatial arrangement of substituents on the aromatic rings, which is related to the structure activity relationship demonstrated by the compound class. Often such substitution arrangement is denoted by a numbering system; however, numbering systems are often not consistent between different ring systems. In six-membered aromatic systems, the spatial arrangements are specified by the common nomenclature "para" for 1,4-substitution, "meta" for 1,3-substitution and "ortho" for 1,2-substitution as shown below.

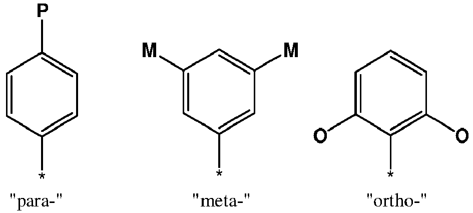

In various embodiments, the compound or set of compounds, such as are among the inventive compounds or are used in the inventive methods, can be any one of any of the combinations and/or subcombinations of the above-listed embodiments.

### Compounds

In one aspect described herein are compounds of Formula (I): 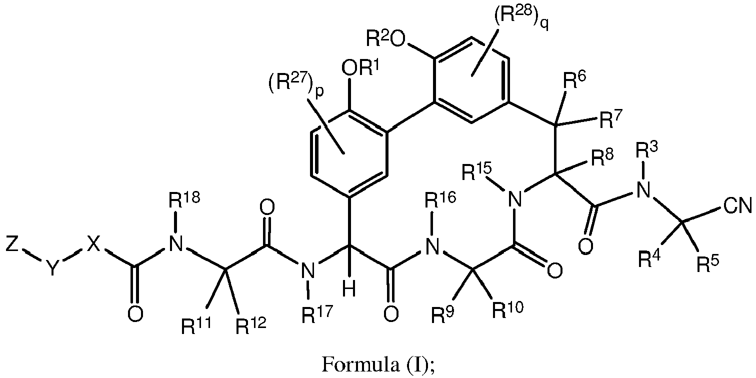 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring;
R³ is H or -(C₁-C₆)alkyl;
R⁴ is H, -(C₁-C₆)alkyl, -(C₁-C₆)alkyl-OH, -(C₃-C₆)cycloalkyl, or -C(O)NH₂;
or R³ and R⁴ are combined to form a heterocycloalkyl ring;
R⁵ is H or -(C₁-C₆)alkyl;
or R⁴ and R⁵ and the carbon atom to which they are attached form a cyclopropyl ring;
R⁶, R⁷, and R⁸ are each independently H, fluoro, hydroxyl, amino, optionally substituted alkyl, optionally substituted heteroalkyl, or -(C₁-C₆)alkyl;
R⁹ is H, -(C₁-C₆)alkyl, -(C₁-C₆)haloalkyl, or -(C₃-C₆)cycloalkyl;
R¹⁰ is H, -(C₁-C₆)alkyl, -(C₁-C₆)haloalkyl, or -(C₃-C₆)cycloalkyl;
or R⁹ and R¹⁰ are combined to form a heterocycloalkyl or cycloalkyl ring
R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl- N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
or R¹¹ and R¹⁸ are combined to form an optionally substituted heterocycloalkyl ring; and R¹² is H;
R¹⁵, R¹⁶, R¹⁷, and R¹⁸ are each independently H, -(C₁-C₆)alkyl, -(C₃-C₆)cycloalkyl, -(C₁-C₆)alkyl-OR²³, - (C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₁-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring;
each R²⁷ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R¹ and R²⁷ and the atoms to which they are attached form an optionally substituted 5- or 6-membered heterocycloalkyl ring;
each R²⁸ is independently halogen, -NR²³R²⁴, -NHC(O)R²³, -NC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R² and R²⁸ and the atoms to which they are attached form an optionally substituted 5- or 6-membered heterocycloalkyl ring;
p is 0, 1, or 2; and
q is 0, 1, or 2;
or a pharmaceutically acceptable salt, solvate, or prodrug thereof.

In one embodiment is a compound of Formula (I) wherein R⁶, R⁷, and R⁸ are H.

In another embodiment is a compound of Formula (I) wherein R¹⁵ and R¹⁶ are H.

In one embodiment is a compound of Formula (I) wherein R¹⁷ is -(C₁-C₆)alkyl. In another embodiment is a compound of Formula (I) wherein R¹⁷ is -CH₃. In another embodiment is a compound of Formula (I) wherein R¹⁷ is -CH₂CH₃. In another embodiment is a compound of Formula (I) wherein R¹⁷ is -(C₃-C₆)cycloalkyl. In another embodiment is a compound of Formula (I) wherein R¹⁷ is cyclopropyl. In another embodiment is a compound of Formula (I) wherein R¹⁷ is -(C₁-C₆)alkyl-C(O)OR²³. In another embodiment is a compound of Formula (I) wherein R¹⁷ is -CH₂CH₂OH. In another embodiment is a compound of Formula (I) wherein R¹⁷ is -(C₁-C₆)alkyl-NR²¹R²². In another embodiment is a compound of Formula (I) wherein R¹⁷ is -CH₂CH₂NH₂. In another embodiment is a compound of Formula (I) wherein R¹⁷ is H.

In another embodiment is a compound of Formula (I) wherein R¹⁸ is H.

In another embodiment is a compound of Formula (I) wherein R³ is H.

In another embodiment is a compound of Formula (I) wherein R⁵ is H.

In another embodiment is a compound of Formula (I) wherein R⁴ is H. In another embodiment is a compound of Formula (I) wherein R⁴ is -(C₁-C₆)alkyl. In another embodiment is a compound of Formula (I) wherein R⁴ is -CH₃. In another embodiment is a compound of Formula (I) wherein R⁴ is -CH₂CH₃. In another embodiment is a compound of Formula (I) wherein R⁴ is -(C₁-C₆)alkyl-OH. In another embodiment is a compound of Formula (I) wherein R⁴ is -CH₂OH. In another embodiment is a compound of Formula (I) wherein R⁴ is -(C₃-C₆)cycloalkyl. In another embodiment is a compound of Formula (I) wherein R⁴ is cyclopropyl. In another embodiment is a compound of Formula (I) wherein R⁴ is -C(O)NH₂.

In another embodiment is a compound of Formula (I) wherein R³, R⁴, and R⁵ are H.

In another embodiment is a compound of Formula (I) wherein R⁴ and R⁵ and the carbon atom to which they are attached form a cyclopropyl ring.

In another embodiment is a compound of Formula (I) wherein R¹⁰ is H.

In another embodiment is a compound of Formula (I) wherein R¹⁰ is H and R⁹ is -(C₁-C₆)alkyl. In another embodiment is a compound of Formula (I) wherein R¹⁰ is H and R⁹ is -CH₃. In another embodiment is a compound of Formula (I) wherein R¹⁰ is H and R⁹ is -CH₂CH₃. In another embodiment is a compound of Formula (I) wherein R¹⁰ is H and R⁹ is -(C₁-C₆)haloalkyl. In another embodiment is a compound of Formula (I) wherein R¹⁰ is H and R⁹ is -CH₂F. In another embodiment is a compound of Formula (I) wherein R¹⁰ is H and R⁹ is -CHF₂. In another embodiment is a compound of Formula (I) wherein R¹⁰ is H and R⁹ is -(C₃-C₆)cycloalkyl. In another embodiment is a compound of Formula (I) wherein R¹⁰ is H and R⁹ is cyclopropyl. In another embodiment is a compound of Formula (I) wherein R¹⁰ is H and R⁹ is H.

In another embodiment is a compound of Formula (I) wherein R¹² is H.

In another embodiment is a compound of Formula (I) wherein R¹² is H and R¹¹ is -(C₁-C₆)alkyl. In another embodiment is a compound of Formula (I) wherein R¹² is H and R¹¹ is -CH₃. In another embodiment is a compound of Formula (I) wherein R¹² is H and R¹¹ is -(C₁-C₆)alkyl-OR²³. In another embodiment is a compound of Formula (I) wherein R¹² is H and R¹¹ is -CH₂OH. In another embodiment is a compound of Formula (I) wherein R¹² is H and R¹¹ is -CH₂CH₂OH. In another embodiment is a compound of Formula (I) wherein R¹² is H and R¹¹ is -(C₁-C₆)alkyl. In another embodiment is a compound of Formula (I) wherein R¹² is H and R¹¹ is -(C₁-C₆)alkyl-NR²¹R²². In another embodiment is a compound of Formula (I) wherein R¹² is H and R¹¹ is -(C₁-C₆)alkyl-NH₂. In another embodiment is a compound of Formula (I) wherein R¹² is H and R¹¹ is -CH₂NH₂. In another embodiment is a compound of Formula (I) wherein R¹² is H and R¹¹ is -CH₂CH₂NH₂. In another embodiment is a compound of Formula (I) wherein R¹² is H and R¹¹ is -CH₂CH₂CH₂NH₂. In another embodiment is a compound of Formula (I) wherein R¹² is H and R¹¹ is -CH₂CH₂CH₂CH₂NH₂. In another embodiment is a compound of Formula (I) wherein R¹² is H and R¹¹ is -(C₁-C₆)alkyl-CN. In another embodiment is a compound of Formula (I) wherein R¹² is H and R¹¹ is -CH₂CN. In another embodiment is a compound of Formula (I) wherein R¹² is H and R¹¹ is -(C₁-C₆)alkyl-C(O)NR²⁵R²⁶. In another embodiment is a compound of Formula (I) wherein R¹² is H and R¹¹ is - CH₂C(O)NH₂. In another embodiment is a compound of Formula (I) wherein R¹² is H and R¹¹ is - CH₂CH₂C(O)NH₂. In another embodiment is a compound of Formula (I) wherein R¹² is H and R¹¹ is -(C₁-C₆)alkyl-heteroaryl. In another embodiment is a compound of Formula (I) wherein R¹² is H and R¹¹ is -(C₁-C₆)alkyl-N(H)S(O)₂NR²⁵R²⁶. In another embodiment is a compound of Formula (I) wherein R¹² is H and R¹¹ is -CH₂N(H)S(O)₂NH₂. In another embodiment is a compound of Formula (I) wherein R¹² is H and R¹¹ is H.

In another embodiment is a compound of Formula (I) wherein R¹¹ and R¹⁸ are combined to form an optionally substituted heterocycloalkyl ring and R¹² is H.

In another embodiment is a compound of Formula (I) wherein p is 1 and R²⁷ is halogen. In another embodiment is a compound of Formula (I) wherein p is 1 and R²⁷ is optionally substituted -(C₁-C₆)alkyl. In another embodiment is a compound of Formula (I) wherein q is 0, p is 1 and R²⁷ is halogen. In another embodiment is a compound of Formula (I) wherein q is 0, p is 1 and R²⁷ is optionally substituted - (C₁-C₆)alkyl. In another embodiment is a compound of Formula (I) wherein q is 1 and R²⁸ is halogen. In another embodiment is a compound of Formula (I) wherein q is 1 and R²⁸ is optionally substituted -(C₁-C₆)alkyl. In another embodiment is a compound of Formula (I) wherein p is 0, q is 1 and R²⁸ is halogen. In another embodiment is a compound of Formula (I) wherein p is 0, q is 1 and R²⁸ is optionally substituted - (C₁-C₆)alkyl.

In another embodiment is a compound of Formula (I) wherein p is 0, and q is 0.

In another embodiment is a compound of Formula (I) wherein R¹ and R² are each independently H or -(C₁-C₆)alkyl-NR²¹R²². In another embodiment is a compound of Formula (I) wherein R¹ and R² are each H. In another embodiment is a compound of Formula (I) wherein R¹ and R² are each independently - (C₁-C₆)alkyl-NR²¹R²². In another embodiment is a compound of Formula (I) wherein R¹ is H, and R² is - (C₁-C₆)alkyl-NR²¹R²². In another embodiment is a compound of Formula (I) wherein R¹ is -(C₁-C₆)alkyl-NR²¹R²², and R² is H. In another embodiment is a compound of Formula (I) wherein R¹ is H, and R² is - CH₂CH₂NH₂. In another embodiment is a compound of Formula (I) wherein R¹ is -CH₂CH₂NH₂, and R² is H. In another embodiment is a compound of Formula (I) wherein R¹ and R² are each -CH₂CH₂NH₂. In a further embodiment is a compound of Formula (I) wherein R¹ is -(C₁-C₆)alkyl-NR²¹R²² and R² is H. In a further embodiment is a compound of Formula (I) wherein R¹ is -CH₂CH₂NH₂ and R² is H. In a further embodiment is a compound of Formula (I) wherein R¹ is H and R² is -(C₁-C₆)alkyl-NR²¹R²². In a further embodiment is a compound of Formula (I) wherein R¹ is H and R² is -CH₂CH₂NH₂. In a further embodiment is a compound of Formula (I) wherein R¹ and R² and the atoms to which they are attached form an optionally substituted heterocycloalkyl ring. In another embodiment is a compound of Formula (I) wherein R¹ and R² are each independently H, -(C₁-C₆)alkyl-NR²¹R²², or -CH₂CH(OH)CH₂NH₂. In another embodiment is a compound of Formula (I) wherein R¹ and R² are each independently - CH₂CH(OH)CH₂NH₂. In another embodiment is a compound of Formula (I) wherein R¹ is H, and R² is - CH₂CH(OH)CH₂NH₂. In another embodiment is a compound of Formula (I) wherein R¹ is - CH₂CH(OH)CH₂NH₂, and R² is H.

In another embodiment is a compound of Formula (I) wherein X is optionally substituted aryl. In a further embodiment is a compound of Formula (I) wherein X is optionally substituted phenyl. In another embodiment is a compound of Formula (I) wherein X is optionally substituted heteroaryl. In a further embodiment is a compound of Formula (I) wherein X is disubstituted heteroaryl. In a further embodiment is a compound of Formula (I) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (I) wherein X is heteroaryl disubstituted with substituents each independently selected from -(C₁-C₆)alkyl. In a further embodiment is a compound of Formula (I) wherein X is heteroaryl disubstituted with methyl. In a further embodiment is a compound of Formula (I) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (I) wherein X is pyridinyl disubstituted with substituents each independently selected from -(C₁-C₆)alkyl. In a further embodiment is a compound of Formula (I) wherein X is pyridinyl disubstituted with methyl. In a further embodiment is a compound of Formula (I) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (I) wherein X is pyrimidinyl disubstituted with substituents each independently selected from -(C₁-C₆)alkyl. In a further embodiment is a compound of Formula (I) wherein X is pyrimidinyl disubstituted with methyl. In another embodiment is a compound of Formula (I) wherein X is optionally substituted -(C₁-C₆)alkyl-.

In another embodiment is a compound of Formula (I) wherein Y is optionally substituted aryl. In a further embodiment is a compound of Formula (I) wherein Y is optionally substituted phenyl. In another embodiment is a compound of Formula (I) wherein Y is optionally substituted heteroaryl. In another embodiment is a compound of Formula (I) wherein Y is optionally substituted -(C₁-C₆)alkyl-. In another embodiment is a compound of Formula (I) wherein Y is optionally substituted (C₃-C₇)cycloalkyl-. In another embodiment is a compound of Formula (I) wherein Y is optionally substituted heterocycloalkyl. In another embodiment is a compound of Formula (I) wherein Y is -O-. In another embodiment is a compound of Formula (I) wherein Y is -(C₂-C₆)alkynyl. In another embodiment is a compound of Formula (I) wherein Y is -O-(C₁-C₆)alkyl-. In another embodiment is a compound of Formula (I) wherein Y is a bond.

In another embodiment is a compound of Formula (I) wherein Z is -(C₁-C₁₂)alkyl. In a further embodiment is a compound of Formula (I) wherein Z is n-butyl, isobutyl, or tert-butyl. In another embodiment is a compound of Formula (I) wherein Z is -O-(C₁-C₁₂)alkyl. In another embodiment is a compound of Formula (I) wherein Z is -O-(C₃-C₇)cycloalkyl. In another embodiment is a compound of Formula (I) wherein Z is -(C₂-C₁₂)alkenyl. In another embodiment is a compound of Formula (I) wherein Z is optionally substituted aryl. In a further embodiment is a compound of Formula (I) wherein Z is optionally substituted phenyl. In a further embodiment is a compound of Formula (I) wherein Z is phenyl monsubstituted or disubstituted with a substituent independently selected from -(C₁-C₈)alkyl. In a further embodiment is a compound of Formula (I) wherein Z is phenyl monosubstituted with n-butyl, isobutyl, or tert-butyl. In a further embodiment is a compound of Formula (I) wherein Z is phenyl monosubstituted with n-butyl. In a further embodiment is a compound of Formula (I) wherein Z is phenyl monosubstituted with isobutyl. In a further embodiment is a compound of Formula (I) wherein Z is phenyl monosubstituted with tert-butyl. In another embodiment is a compound of Formula (I) wherein Z is optionally substituted heteroaryl. In another embodiment is a compound of Formula (I) wherein Z is optionally substituted -(C₃-C₇)cycloalkyl. In another embodiment is a compound of Formula (I) wherein Z is optionally substituted heterocycloalkyl. In another embodiment is a compound of Formula (I) wherein Z is halogen.

In another embodiment is a compound of Formula (I) wherein Z-Y-X- is not

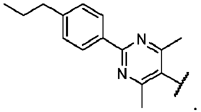

In another embodiment is a compound of Formula (I) having the structure of Formula (Ia): 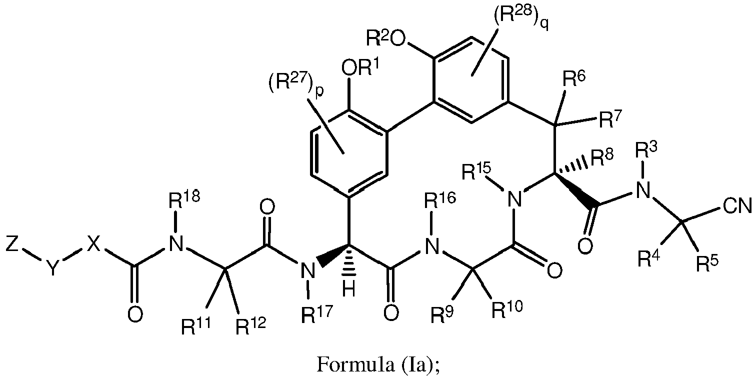 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring;
R³ is H or -(C₁-C₆)alkyl;
R⁴ is H, -(C₁-C₆)alkyl, -(C₁-C₆)alkyl-OH, -(C₃-C₆)cycloalkyl, or -C(O)NH₂;
or R³ and R⁴ are combined to form a heterocycloalkyl ring;
R⁵ is H or -(C₁-C₆)alkyl;
or R⁴ and R⁵ and the carbon atom to which they are attached form a cyclopropyl ring;
R⁶, R⁷, and R⁸ are each independently H, fluoro, hydroxyl, amino, optionally substituted alkyl, heteroalkyl, or -(C₁-C₆)alkyl;
R⁹ is H, -(C₁-C₆)alkyl, -(C₁-C₆)haloalkyl, or -(C₃-C₆)cycloalkyl;
R¹⁰ is H, -(C₁-C₆)alkyl, -(C₁-C₆)haloalkyl, or -(C₃-C₆)cycloalkyl;
or R⁹ and R¹⁰ are combined to form a heterocycloalkyl or cycloalkyl ring
R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
or R¹¹ and R¹⁸ are combined to form an optionally substituted heterocycloalkyl ring; and R¹² is H;
R¹⁵, R¹⁶, R¹⁷, and R¹⁸ are each independently H, -(C₁-C₆)alkyl, -(C₃-C₆)cycloalkyl, -(C₁-C₆)alkyl-OR²³, - (C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring;
each R²⁷ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R¹ and R²⁷ and the atoms to which they are attached form an optionally substituted 5- or 6-membered heterocycloalkyl ring;
each R²⁸ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R² and R²⁸ and the atoms to which they are attached form an optionally substituted 5- or 6-membered heterocycloalkyl ring;
p is 0, 1, or 2; and
q is 0, 1, or 2;

or a pharmaceutically acceptable salt, solvate, or prodrug thereof.In one embodiment is a compound of Formula (Ia) wherein R⁶, R⁷, and R⁸ are H.

In another embodiment is a compound of Formula (Ia) wherein R¹⁵ and R¹⁶ are H.

In one embodiment is a compound of Formula (Ia) wherein R¹⁷ is -(C₁-C₆)alkyl. In another embodiment is a compound of Formula (Ia) wherein R¹⁷ is -CH₃. In another embodiment is a compound of Formula (Ia) wherein R¹⁷ is -CH₂CH₃. In another embodiment is a compound of Formula (Ia) wherein R¹⁷ is -(C₃-C₆)cycloalkyl. In another embodiment is a compound of Formula (Ia) wherein R¹⁷ is cyclopropyl. In another embodiment is a compound of Formula (Ia) wherein R¹⁷ is -(C₁-C₆)alkyl-C(O)OR²³. In another embodiment is a compound of Formula (Ia) wherein R¹⁷ is -CH₂CH₂OH. In another embodiment is a compound of Formula (Ia) wherein R¹⁷ is -(C₁-C₆)alkyl-NR²¹R²². In another embodiment is a compound of Formula (Ia) wherein R¹⁷ is -CH₂CH₂NH₂. In another embodiment is a compound of Formula (Ia) wherein R¹⁷ is H.

In another embodiment is a compound of Formula (Ia) wherein R¹⁸ is H.

In another embodiment is a compound of Formula (Ia) wherein R³ is H.

In another embodiment is a compound of Formula (Ia) wherein R⁵ is H.

In another embodiment is a compound of Formula (Ia) wherein R⁴ is H. In another embodiment is a compound of Formula (Ia) wherein R⁴ is -(C₁-C₆)alkyl. In another embodiment is a compound of Formula (Ia) wherein R⁴ is -CH₃. In another embodiment is a compound of Formula (Ia) wherein R⁴ is - CH₂CH₃. In another embodiment is a compound of Formula (Ia) wherein R⁴ is -(C₁-C₆)alkyl-OH. In another embodiment is a compound of Formula (Ia) wherein R⁴ is -CH₂OH. In another embodiment is a compound of Formula (Ia) wherein R⁴ is -(C₃-C₆)cycloalkyl. In another embodiment is a compound of Formula (Ia) wherein R⁴ is cyclopropyl. In another embodiment is a compound of Formula (Ia) wherein R⁴ is -C(O)NH₂.

In another embodiment is a compound of Formula (Ia) wherein R⁴ and R⁵ and the carbon atom to which they are attached form a cyclopropyl ring.

In another embodiment is a compound of Formula (Ia) wherein R³, R⁴, and R⁵ are H.

In another embodiment is a compound of Formula (Ia) wherein R¹⁰ is H.

In another embodiment is a compound of Formula (Ia) wherein R¹⁰ is H and R⁹ is -(C₁-C₆)alkyl. In another embodiment is a compound of Formula (Ia) wherein R¹⁰ is H and R⁹ is -CH₃. In another embodiment is a compound of Formula (Ia) wherein R¹⁰ is H and R⁹ is -CH₂CH₃. In another embodiment is a compound of Formula (Ia) wherein R¹⁰ is H and R⁹ is -(C₁-C₆)haloalkyl. In another embodiment is a compound of Formula (Ia) wherein R¹⁰ is H and R⁹ is -CH₂F. In another embodiment is a compound of Formula (Ia) wherein R¹⁰ is H and R⁹ is -CHF₂. In another embodiment is a compound of Formula (Ia) wherein R¹⁰ is H and R⁹ is -(C₃-C₆)cycloalkyl. In another embodiment is a compound of Formula (Ia) wherein R¹⁰ is H and R⁹ is cyclopropyl. In another embodiment is a compound of Formula (Ia) wherein R¹⁰ is H and R⁹ is H.

In another embodiment is a compound of Formula (Ia) wherein R¹² is H.

In another embodiment is a compound of Formula (Ia) wherein R¹² is H and R¹¹ is -(C₁-C₆)alkyl. In another embodiment is a compound of Formula (Ia) wherein R¹² is H and R¹¹ is -CH₃. In another embodiment is a compound of Formula (Ia) wherein R¹² is H and R¹¹ is -(C₁-C₆)alkyl-OR²³. In another embodiment is a compound of Formula (Ia) wherein R¹² is H and R¹¹ is -CH₂OH. In another embodiment is a compound of Formula (Ia) wherein R¹² is H and R¹¹ is -CH₂CH₂OH. In another embodiment is a compound of Formula (Ia) wherein R¹² is H and R¹¹ is -(C₁-C₆)alkyl. In another embodiment is a compound of Formula (Ia) wherein R¹² is H and R¹¹ is -(C₁-C₆)alkyl-NR²¹R²². In another embodiment is a compound of Formula (Ia) wherein R¹² is H and R¹¹ is -(C₁-C₆)alkyl-NH₂. In another embodiment is a compound of Formula (Ia) wherein R¹² is H and R¹¹ is -CH₂NH₂. In another embodiment is a compound of Formula (Ia) wherein R¹² is H and R¹¹ is -CH₂CH₂NH₂. In another embodiment is a compound of Formula (Ia) wherein R¹² is H and R¹¹ is -CH₂CH₂CH₂NH₂. In another embodiment is a compound of Formula (Ia) wherein R¹² is H and R¹¹ is -CH₂CH₂CH₂CH₂NH₂. In another embodiment is a compound of Formula (Ia) wherein R¹² is H and R¹¹ is -(C₁-C₆)alkyl-CN. In another embodiment is a compound of Formula (Ia) wherein R¹² is H and R¹¹ is -CH₂CN. In another embodiment is a compound of Formula (Ia) wherein R¹² is H and R¹¹ is -(C₁-C₆)alkyl-C(O)NR²⁵R²⁶. In another embodiment is a compound of Formula (Ia) wherein R¹² is H and R¹¹ is -CH₂C(O)NH₂. In another embodiment is a compound of Formula (Ia) wherein R¹² is H and R¹¹ is -CH₂CH₂C(O)NH₂. In another embodiment is a compound of Formula (Ia) wherein R¹² is H and R¹¹ is -(C₁-C₆)alkyl-heteroaryl. In another embodiment is a compound of Formula (Ia) wherein R¹² is H and R¹¹ is -(C₁-C₆)alkyl-N(H)S(O)₂NR²⁵R²⁶. In another embodiment is a compound of Formula (Ia) wherein R¹² is H and R¹¹ is -CH₂N(H)S(O)₂NH₂. In another embodiment is a compound of Formula (Ia) wherein R¹² is H and R¹¹ is H.

In another embodiment is a compound of Formula (Ia) wherein R¹¹ and R¹⁸ are combined to form an optionally substituted heterocycloalkyl ring and R¹² is H.

In another embodiment is a compound of Formula (Ia) wherein p is 1 and R²⁷ is halogen. In another embodiment is a compound of Formula (Ia) wherein p is 1 and R²⁷ is optionally substituted -(C₁-C₆)alkyl. In another embodiment is a compound of Formula (Ia) wherein q is 0, p is 1 and R²⁷ is halogen. In another embodiment is a compound of Formula (Ia) wherein q is 0, p is 1 and R²⁷ is optionally substituted -(C₁-C₆)alkyl. In another embodiment is a compound of Formula (Ia) wherein q is 1 and R²⁸ is halogen. In another embodiment is a compound of Formula (Ia) wherein q is 1 and R²⁸ is optionally substituted -(C₁-C₆)alkyl. In another embodiment is a compound of Formula (Ia) wherein p is 0, q is 1 and R²⁸ is halogen. In another embodiment is a compound of Formula (Ia) wherein p is 0, q is 1 and R²⁸ is optionally substituted -(C₁-C₆)alkyl.

In another embodiment is a compound of Formula (Ia) wherein p is 0, and q is 0.

In another embodiment is a compound of Formula (Ia) wherein R¹ and R² are each independently H or -(C₁-C₆)alkyl-NR²¹R²². In another embodiment is a compound of Formula (Ia) wherein R¹ and R² are each H. In another embodiment is a compound of Formula (Ia) wherein R¹ and R² are each independently -(C₁-C₆)alkyl-NR²¹R²². In another embodiment is a compound of Formula (Ia) wherein R¹ is H, and R² is -(C₁-C₆)alkyl-NR²¹R²². In another embodiment is a compound of Formula (Ia) wherein R¹ is - (C₁-C₆)alkyl-NR²¹R²², and R² is H. In another embodiment is a compound of Formula (Ia) wherein R¹ is H, and R² is -CH₂CH₂NH₂. In another embodiment is a compound of Formula (Ia) wherein R¹ is - CH₂CH₂NH₂, and R² is H. In another embodiment is a compound of Formula (Ia) wherein R¹ and R² are each -CH₂CH₂NH₂. In a further embodiment is a compound of Formula (Ia) wherein R¹ is -(C₁-C₆)alkyl-NR²¹R²² and R² is H. In a further embodiment is a compound of Formula (Ia) wherein R¹ is -CH₂CH₂NH₂ and R² is H. In a further embodiment is a compound of Formula (Ia) wherein R¹ is H and R² is -(C₁-C₆)alkyl-NR²¹R²². In a further embodiment is a compound of Formula (Ia) wherein R¹ is H and R² is - CH₂CH₂NH₂. In another embodiment is a compound of Formula (Ia) wherein R¹ and R² are each independently H, -(C₁-C₆)alkyl-NR²¹R²², or -CH₂CH(OH)CH₂NH₂. In another embodiment is a compound of Formula (Ia) wherein R¹ and R² are each independently -CH₂CH(OH)CH₂NH₂. In another embodiment is a compound of Formula (Ia) wherein R¹ is H, and R² is -CH₂CH(OH)CH₂NH₂. In another embodiment is a compound of Formula (Ia) wherein R¹ is -CH₂CH(OH)CH₂NH₂, and R² is H.

In a further embodiment is a compound of Formula (Ia) wherein R¹ and R² and the atoms to which they are attached form an optionally substituted heterocycloalkyl ring.

In another embodiment is a compound of Formula (Ia) wherein X is optionally substituted aryl. In a further embodiment is a compound of Formula (Ia) wherein X is optionally substituted phenyl. In another embodiment is a compound of Formula (Ia) wherein X is optionally substituted heteroaryl. In a further embodiment is a compound of Formula (Ia) wherein X is disubstituted heteroaryl. In a further embodiment is a compound of Formula (Ia) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (Ia) wherein X is heteroaryl disubstituted with substituents each independently selected from -(C₁-C₆)alkyl. In a further embodiment is a compound of Formula (Ia) wherein X is heteroaryl disubstituted with methyl. In a further embodiment is a compound of Formula (Ia) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (Ia) wherein X is pyridinyl disubstituted with substituents each independently selected from -(C₁-C₆)alkyl. In a further embodiment is a compound of Formula (Ia) wherein X is pyridinyl disubstituted with methyl. In a further embodiment is a compound of Formula (Ia) wherein X is pyrimidinyl disubstituted with substituents each independently selected from halogen, -CN, optionally substituted -(C₁-C₆)alkyl, optionally substituted -O- (C₁-C₆)alkyl, OR²³, -NR²⁵R²⁶, or -NO₂. In a further embodiment is a compound of Formula (Ia) wherein X is pyrimidinyl disubstituted with substituents each independently selected from -(C₁-C₆)alkyl. In a further embodiment is a compound of Formula (Ia) wherein X is pyrimidinyl disubstituted with methyl. In another embodiment is a compound of Formula (Ia) wherein X is optionally substituted -(C₁-C₆)alkyl-.

In another embodiment is a compound of Formula (Ia) wherein Y is optionally substituted aryl. In a further embodiment is a compound of Formula (Ia) wherein Y is optionally substituted phenyl. In another embodiment is a compound of Formula (Ia) wherein Y is optionally substituted heteroaryl. In another embodiment is a compound of Formula (Ia) wherein Y is optionally substituted -(C₁-C₆)alkyl-. In another embodiment is a compound of Formula (Ia) wherein Y is optionally substituted (C₃-C₇)cycloalkyl-. In another embodiment is a compound of Formula (Ia) wherein Y is optionally substituted heterocycloalkyl. In another embodiment is a compound of Formula (Ia) wherein Y is -O-. In another embodiment is a compound of Formula (Ia) wherein Y is -(C₂-C₆)alkynyl. In another embodiment is a compound of Formula (Ia) wherein Y is -O-(C₁-C₆)alkyl-. In another embodiment is a compound of Formula (Ia) wherein Y is a bond.

In another embodiment is a compound of Formula (Ia) wherein Z is -(C₁-C₁₂)alkyl. In a further embodiment is a compound of Formula (Ia) wherein Z is n-butyl, isobutyl, or tert-butyl. In another embodiment is a compound of Formula (Ia) wherein Z is -O-(C₁-C₁₂)alkyl. In another embodiment is a compound of Formula (Ia) wherein Z is -O-(C₃-C₇)cycloalkyl. In another embodiment is a compound of Formula (Ia) wherein Z is -(C₂-C₁₂)alkenyl. In another embodiment is a compound of Formula (Ia) wherein Z is optionally substituted aryl. In a further embodiment is a compound of Formula (Ia) wherein Z is optionally substituted phenyl. In a further embodiment is a compound of Formula (Ia) wherein Z is phenyl monsubstituted or disubstituted with a substituent independently selected from -(C₁-C₈)alkyl. In a further embodiment is a compound of Formula (Ia) wherein Z is phenyl monosubstituted with n-butyl, isobutyl, or tert-butyl. In a further embodiment is a compound of Formula (Ia) wherein Z is phenyl monosubstituted with n-butyl. In a further embodiment is a compound of Formula (Ia) wherein Z is phenyl monosubstituted with isobutyl. In a further embodiment is a compound of Formula (Ia) wherein Z is phenyl monosubstituted with tert-butyl. In another embodiment is a compound of Formula (Ia) wherein Z is optionally substituted heteroaryl. In another embodiment is a compound of Formula (Ia) wherein Z is optionally substituted -(C₃-C₇)cycloalkyl. In another embodiment is a compound of Formula (Ia) wherein Z is optionally substituted heterocycloalkyl. In another embodiment is a compound of Formula (Ia) wherein Z is halogen.

In another embodiment is a compound of Formula (Ia) wherein Z-Y-X- is not

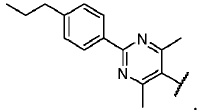

In another embodiment is a compound of Formula (I) having the structure of Formula (Ib): 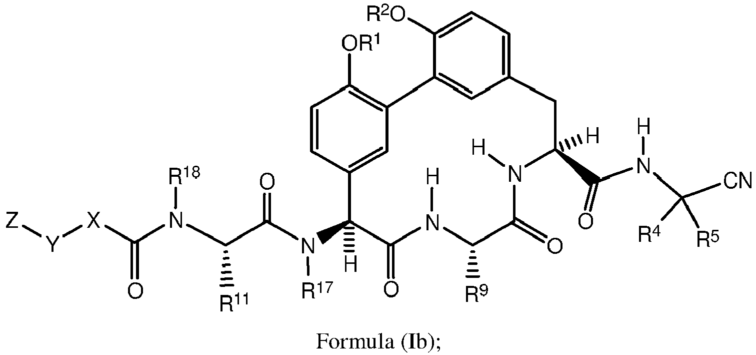 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring; R⁴ is H, -(C₁-C₆)alkyl, -(C₁-C₆)alkyl-OH, -(C₃-C₆)cycloalkyl, or -C(O)NH₂;
R⁵ is H or -(C₁-C₆)alkyl;
or R⁴ and R⁵ and the carbon atom to which they are attached form a cyclopropyl ring;
R⁹ is H, -(C₁-C₆)alkyl, -(C₁-C₆)haloalkyl, or -(C₃-C₆)cycloalkyl;
R¹¹ is H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
or R¹¹ and R¹⁸ are combined to form an optionally substituted heterocycloalkyl ring; and R¹² is H;
R¹⁷ and R¹⁸ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring; and

or a pharmaceutically acceptable salt, solvate, or prodrug thereof.In one embodiment is a compound of Formula (Ib) wherein R¹⁷ is -(C₁-C₆)alkyl. In another embodiment is a compound of Formula (Ib) wherein R¹⁷ is -CH₃. In another embodiment is a compound of Formula (Ib) wherein R¹⁷ is -CH₂CH₃. In another embodiment is a compound of Formula (Ib) wherein R¹⁷ is -(C₃-C₆)cycloalkyl. In another embodiment is a compound of Formula (Ib) wherein R¹⁷ is cyclopropyl. In another embodiment is a compound of Formula (Ib) wherein R¹⁷ is -(C₁-C₆)alkyl-C(O)OR²³. In another embodiment is a compound of Formula (Ib) wherein R¹⁷ is -CH₂CH₂OH. In another embodiment is a compound of Formula (Ib) wherein R¹⁷ is -(C₁-C₆)alkyl-NR²¹R²². In another embodiment is a compound of Formula (Ib) wherein R¹⁷ is -CH₂CH₂NH₂. In another embodiment is a compound of Formula (Ib) wherein R¹⁷ is H.

In another embodiment is a compound of Formula (Ib) wherein R¹⁸ is H.

In another embodiment is a compound of Formula (Ib) wherein R⁵ is H.

In another embodiment is a compound of Formula (Ib) wherein R⁴ is H. In another embodiment is a compound of Formula (Ib) wherein R⁴ is -(C₁-C₆)alkyl. In another embodiment is a compound of Formula (Ib) wherein R⁴ is -CH₃. In another embodiment is a compound of Formula (Ib) wherein R⁴ is - CH₂CH₃. In another embodiment is a compound of Formula (Ib) wherein R⁴ is -(C₁-C₆)alkyl-OH. In another embodiment is a compound of Formula (Ib) wherein R⁴ is -CH₂OH. In another embodiment is a compound of Formula (Ib) wherein R⁴ is -(C₃-C₆)cycloalkyl. In another embodiment is a compound of Formula (Ib) wherein R⁴ is cyclopropyl. In another embodiment is a compound of Formula (Ib) wherein R⁴ is -C(O)NH₂.

In another embodiment is a compound of Formula (Ib) wherein R⁴ and R⁵ and the carbon atom to which they are attached form a cyclopropyl ring.

In another embodiment is a compound of Formula (Ib) wherein R⁹ is -(C₁-C₆)alkyl. In another embodiment is a compound of Formula (Ib) wherein R⁹ is -CH₃. In another embodiment is a compound of Formula (Ib) wherein R⁹ is -CH₂CH₃. In another embodiment is a compound of Formula (Ib) wherein R⁹ is -(C₁-C₆)haloalkyl. In another embodiment is a compound of Formula (Ib) wherein R⁹ is -CH₂F. In another embodiment is a compound of Formula (Ib) wherein R⁹ is -CHF₂. In another embodiment is a compound of Formula (Ib) wherein R⁹ is -(C₃-C₆)cycloalkyl. In another embodiment is a compound of Formula (Ib) wherein R⁹ is cyclopropyl. In another embodiment is a compound of Formula (Ib) wherein R⁹ is H.

In another embodiment is a compound of Formula (Ib) wherein R¹¹ is -(C₁-C₆)alkyl. In another embodiment is a compound of Formula (Ib) wherein R¹¹ is -CH₃. In another embodiment is a compound of Formula (Ib) wherein R¹¹ is -(C₁-C₆)alkyl-OR²³. In another embodiment is a compound of Formula (Ib) wherein R¹¹ is -CH₂OH. In another embodiment is a compound of Formula (Ib) wherein R¹¹ is - CH₂CH₂OH. In another embodiment is a compound of Formula (Ib) wherein R¹¹ is -(C₁-C₆)alkyl. In another embodiment is a compound of Formula (Ib) wherein R¹¹ is -(C₁-C₆)alkyl-NR²¹R²². In another embodiment is a compound of Formula (Ib) wherein R¹¹ is -(C₁-C₆)alkyl-NH₂. In another embodiment is a compound of Formula (Ib) wherein R¹¹ is -CH₂NH₂. In another embodiment is a compound of Formula (Ib) wherein R¹¹ is -CH₂CH₂NH₂. In another embodiment is a compound of Formula (Ib) wherein R¹¹ is - CH₂CH₂CH₂NH₂. In another embodiment is a compound of Formula (Ib) wherein R¹¹ is - CH₂CH₂CH₂CH₂NH₂. In another embodiment is a compound of Formula (Ib) wherein R¹¹ is -(C₁-C₆)alkyl-CN. In another embodiment is a compound of Formula (Ib) wherein R¹¹ is -CH₂CN. In another embodiment is a compound of Formula (Ib) wherein R¹¹ is -(C₁-C₆)alkyl-C(O)NR²⁵R²⁶. In another embodiment is a compound of Formula (Ib) wherein R¹¹ is -CH₂C(O)NH₂. In another embodiment is a compound of Formula (Ib) wherein R¹¹ is -CH₂CH₂C(O)NH₂. In another embodiment is a compound of Formula (Ib) wherein R¹¹ is -(C₁-C₆)alkyl-heteroaryl. In another embodiment is a compound of Formula (Ib) wherein R¹¹ is -(C₁-C₆)alkyl-N(H)S(O)₂NR²⁵R²⁶. In another embodiment is a compound of Formula (Ib) wherein R¹¹ is -CH₂N(H)S(O)₂NH₂. In another embodiment is a compound of Formula (Ib) wherein R¹¹ is H.

In another embodiment is a compound of Formula (Ib) wherein R¹¹ and R¹⁸ are combined to form an optionally substituted heterocycloalkyl ring.

In another embodiment is a compound of Formula (Ib) wherein R¹ and R² are each independently H or -(C₁-C₆)alkyl-NR²¹R²². In another embodiment is a compound of Formula (Ib) wherein R¹ and R² are each H. In another embodiment is a compound of Formula (Ib) wherein R¹ and R² are each independently -(C₁-C₆)alkyl-NR²¹R²². In another embodiment is a compound of Formula (Ib) wherein R¹ is H, and R² is -(C₁-C₆)alkyl-NR²¹R²². In another embodiment is a compound of Formula (Ib) wherein R¹ is - (C₁-C₆)alkyl-NR²¹R²², and R² is H. In another embodiment is a compound of Formula (Ib) wherein R¹ is H, and R² is -CH₂CH₂NH₂. In another embodiment is a compound of Formula (Ib) wherein R¹ is - CH₂CH₂NH₂, and R² is H. In another embodiment is a compound of Formula (Ib) wherein R¹ and R² are each -CH₂CH₂NH₂. In a further embodiment is a compound of Formula (Ib) wherein R¹ is -(C₁-C₆)alkyl-NR²¹R²² and R² is H. In a further embodiment is a compound of Formula (Ib) wherein R¹ is -CH₂CH₂NH₂ and R² is H. In a further embodiment is a compound of Formula (Ib) wherein R¹ is H and R² is -(C₁-C₆)alkyl-NR²¹R²². In a further embodiment is a compound of Formula (Ib) wherein R¹ is H and R² is - CH₂CH₂NH₂. In another embodiment is a compound of Formula (Ib) wherein R¹ and R² are each independently H, -(C₁-C₆)alkyl-NR²¹R²², or -CH₂CH(OH)CH₂NH₂. In another embodiment is a compound of Formula (Ib) wherein R¹ and R² are each independently -CH₂CH(OH)CH₂NH₂. In another embodiment is a compound of Formula (Ib) wherein R¹ is H, and R² is -CH₂CH(OH)CH₂NH₂. In another embodiment is a compound of Formula (Ib) wherein R¹ is -CH₂CH(OH)CH₂NH₂, and R² is H.

In a further embodiment is a compound of Formula (Ib) wherein R¹ and R² and the atoms to which they are attached form an optionally substituted heterocycloalkyl ring.

In another embodiment is a compound of Formula (Ib) wherein X is optionally substituted aryl. In a further embodiment is a compound of Formula (Ib) wherein X is optionally substituted phenyl. In another embodiment is a compound of Formula (Ib) wherein X is optionally substituted heteroaryl. In a further embodiment is a compound of Formula (Ib) wherein X is disubstituted heteroaryl. In a further embodiment is a compound of Formula (Ib) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (Ib) wherein X is heteroaryl disubstituted with substituents each independently selected from -(C₁-C₆)alkyl. In a further embodiment is a compound of Formula (Ib) wherein X is heteroaryl disubstituted with methyl. In a further embodiment is a compound of Formula (Ib) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (Ib) wherein X is pyridinyl disubstituted with substituents each independently selected from -(C₁-C₆)alkyl. In a further embodiment is a compound of Formula (Ib) wherein X is pyridinyl disubstituted with methyl. In a further embodiment is a compound of Formula (Ib) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (Ib) wherein X is pyrimidinyl disubstituted with substituents each independently selected from -(C₁-C₆)alkyl. In a further embodiment is a compound of Formula (Ib) wherein X is pyrimidinyl disubstituted with methyl. In another embodiment is a compound of Formula (Ib) wherein X is optionally substituted -(C₁-C₆)alkyl-.

In another embodiment is a compound of Formula (Ib) wherein Y is optionally substituted aryl. In a further embodiment is a compound of Formula (Ib) wherein Y is optionally substituted phenyl. In another embodiment is a compound of Formula (Ib) wherein Y is optionally substituted heteroaryl. In another embodiment is a compound of Formula (Ib) wherein Y is optionally substituted -(C₁-C₆)alkyl-. In another embodiment is a compound of Formula (Ib) wherein Y is optionally substituted (C₃-C₇)cycloalkyl-. In another embodiment is a compound of Formula (Ib) wherein Y is optionally substituted heterocycloalkyl. In another embodiment is a compound of Formula (Ib) wherein Y is -O-. In another embodiment is a compound of Formula (Ib) wherein Y is -(C₂-C₆)alkynyl. In another embodiment is a compound of Formula (Ib) wherein Y is -O-(C₁-C₆)alkyl-. In another embodiment is a compound of Formula (Ib) wherein Y is a bond.

In another embodiment is a compound of Formula (Ib) wherein Z is -(C₁-C₁₂)alkyl. In a further embodiment is a compound of Formula (Ib) wherein Z is n-butyl, isobutyl, or tert-butyl. In another embodiment is a compound of Formula (Ib) wherein Z is -O-(C₁-C₁₂)alkyl. In another embodiment is a compound of Formula (Ib) wherein Z is -O-(C₃-C₇)cycloalkyl. In another embodiment is a compound of Formula (Ib) wherein Z is -(C₂-C₁₂)alkenyl. In another embodiment is a compound of Formula (Ib) wherein Z is optionally substituted aryl. In a further embodiment is a compound of Formula (Ib) wherein Z is optionally substituted phenyl. In a further embodiment is a compound of Formula (Ib) wherein Z is phenyl monsubstituted or disubstituted with a substituent independently selected from -(C₁-C₈)alkyl. In a further embodiment is a compound of Formula (Ib) wherein Z is phenyl monosubstituted with n-butyl, isobutyl, or tert-butyl. In a further embodiment is a compound of Formula (Ib) wherein Z is phenyl monosubstituted with n-butyl. In a further embodiment is a compound of Formula (Ib) wherein Z is phenyl monosubstituted with isobutyl. In a further embodiment is a compound of Formula (Ib) wherein Z is phenyl monosubstituted with tert-butyl. In another embodiment is a compound of Formula (Ib) wherein Z is optionally substituted heteroaryl. In another embodiment is a compound of Formula (Ib) wherein Z is optionally substituted -(C₃-C₇)cycloalkyl. In another embodiment is a compound of Formula (Ib) wherein Z is optionally substituted heterocycloalkyl. In another embodiment is a compound of Formula (Ib) wherein Z is halogen.

In another embodiment is a compound of Formula (Ib) wherein Z-Y-X- is not

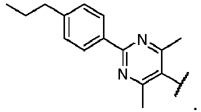

In another embodiment is a compound of Formula (I) having the structure of Formula (Ic): 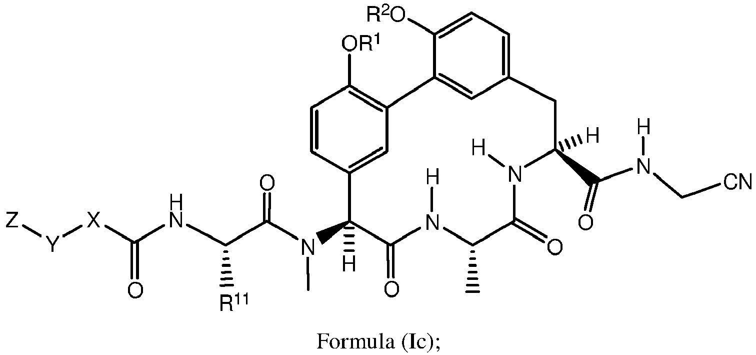 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R^{22]}₂, -(C₁-C₆)heteroalkyl, or optionally substituted heterocycloalkyl;
or R¹ and R² and the atoms to which they are attached form an optionally substituted heterocycloalkyl ring;
R¹¹ is H, -NH₂, -(C₁-C₆)alkyl, -(C₁-C₆)alkyl-OR²³, -(C₁-C₆)alkyl-SR²³, -(C₁-C₆)alkyl-C(0)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-Cdalkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring; and

or a pharmaceutically acceptable salt, solvate, or prodrug thereof.In another embodiment is a compound of Formula (Ic) wherein R¹¹ is -(C₁-C₆)alkyl. In another embodiment is a compound of Formula (Ic) wherein R¹¹ is -CH₃. In another embodiment is a compound of Formula (Ic) wherein R¹¹ is -(C₁-C₆)alkyl-OR²³. In another embodiment is a compound of Formula (Ic) wherein R¹¹ is -CH₂OH. In another embodiment is a compound of Formula (Ic) wherein R¹¹ is -CH₂CH₂OH. In another embodiment is a compound of Formula (Ic) wherein R¹¹ is -(C₁-C₆)alkyl. In another embodiment is a compound of Formula (Ic) wherein R¹¹ is -(C₁-C₆)alkyl-NR²¹R²². In another embodiment is a compound of Formula (Ic) wherein R¹¹ is -(C₁-C₆)alkyl-NH₂. In another embodiment is a compound of Formula (Ic) wherein R¹¹ is -CH₂NH₂. In another embodiment is a compound of Formula (Ic) wherein R¹¹ is -CH₂CH₂NH₂. In another embodiment is a compound of Formula (Ic) wherein R¹¹ is -CH₂CH₂CH₂NH₂. In another embodiment is a compound of Formula (Ic) wherein R¹¹ is -CH₂CH₂CH₂CH₂NH₂. In another embodiment is a compound of Formula (Ic) wherein R¹¹ is -(C₁-C₆)alkyl-CN. In another embodiment is a compound of Formula (Ic) wherein R¹¹ is -CH₂CN. In another embodiment is a compound of Formula (Ic) wherein R¹¹ is -(C₁-C₆)alkyl-C(O)NR²⁵R²⁶. In another embodiment is a compound of Formula (Ic) wherein R¹¹ is - CH₂C(O)NH₂. In another embodiment is a compound of Formula (Ic) wherein R¹¹ is -CH₂CH₂C(O)NH₂. In another embodiment is a compound of Formula (Ic) wherein R¹¹ is -(C₁-C₆)alkyl-heteroaryl. In another embodiment is a compound of Formula (Ic) wherein R¹¹ is -(C₁-C₆)alkyl-N(H)S(O)₂NR²⁵R²⁶. In another embodiment is a compound of Formula (Ic) wherein R¹¹ is -CH₂N(H)S(O)₂NH₂. In another embodiment is a compound of Formula (Ic) wherein R¹¹ is H.

In another embodiment is a compound of Formula (Ic) wherein R¹ and R² are each independently H or -(C₁-C₆)alkyl-NR²¹R²². In another embodiment is a compound of Formula (Ic) wherein R¹ and R² are each H. In another embodiment is a compound of Formula (Ic) wherein R¹ and R² are each independently -(C₁-C₆)alkyl-NR²¹R²². In another embodiment is a compound of Formula (Ic) wherein R¹ is H, and R² is -(C₁-C₆)alkyl-NR²¹R²². In another embodiment is a compound of Formula (Ic) wherein R¹ is - (C₁-C₆)alkyl-NR²¹R²², and R² is H. In another embodiment is a compound of Formula (Ic) wherein R¹ is H, and R² is -CH₂CH₂NH₂. In another embodiment is a compound of Formula (Ic) wherein R¹ is - CH₂CH₂NH₂, and R² is H. In another embodiment is a compound of Formula (Ic) wherein R¹ and R² are each -CH₂CH₂NH₂. In a further embodiment is a compound of Formula (Ic) wherein R¹ is -(C₁-C₆)alkyl-NR²¹R²² and R² is H. In a further embodiment is a compound of Formula (Ic) wherein R¹ is -CH₂CH₂NH₂ and R² is H. In a further embodiment is a compound of Formula (Ic) wherein R¹ is H and R² is -(C₁-C₆)alkyl-NR²²R²². In a further embodiment is a compound of Formula (Ic) wherein R¹ is H and R² is - CH₂CH₂NH₂. In another embodiment is a compound of Formula (Ic) wherein R¹ and R² are each independently H, -(C₁-C₆)alkyl-NR²¹R²², or -CH₂CH(OH)CH₂NH₂. In another embodiment is a compound of Formula (Ic) wherein R¹ and R² are each independently -CH₂CH(OH)CH₂NH₂. In another embodiment is a compound of Formula (Ic) wherein R¹ is H, and R² is -CH₂CH(OH)CH₂NH₂. In another embodiment is a compound of Formula (Ic) wherein R¹ is -CH₂CH(OH)CH₂NH₂, and R² is H.

In a further embodiment is a compound of Formula (Ic) wherein R¹ and R² and the atoms to which they are attached form an optionally substituted heterocycloalkyl ring.

In another embodiment is a compound of Formula (Ic) wherein X is optionally substituted aryl. In a further embodiment is a compound of Formula (Ic) wherein X is optionally substituted phenyl. In another embodiment is a compound of Formula (Ic) wherein X is optionally substituted heteroaryl. In a further embodiment is a compound of Formula (Ic) wherein X is disubstituted heteroaryl. In a further embodiment is a compound of Formula (Ic) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (Ic) wherein X is heteroaryl disubstituted with substituents each independently selected from -(C₁-C₆)alkyl. In a further embodiment is a compound of Formula (Ic) wherein X is heteroaryl disubstituted with methyl. In a further embodiment is a compound of Formula (Ic) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (Ic) wherein X is pyridinyl disubstituted with substituents each independently selected from -(C₁-C₆)alkyl. In a further embodiment is a compound of Formula (Ic) wherein X is pyridinyl disubstituted with methyl. In a further embodiment is a compound of Formula (Ic) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (Ic) wherein X is pyrimidinyl disubstituted with substituents each independently selected from -(C₁-C₆)alkyl. In a further embodiment is a compound of Formula (Ic) wherein X is pyrimidinyl disubstituted with methyl. In another embodiment is a compound of Formula (Ic) wherein X is optionally substituted -(C₁-C₆)alkyl-.

In another embodiment is a compound of Formula (Ic) wherein Y is optionally substituted aryl. In a further embodiment is a compound of Formula (Ic) wherein Y is optionally substituted phenyl. In another embodiment is a compound of Formula (Ic) wherein Y is optionally substituted heteroaryl. In another embodiment is a compound of Formula (Ic) wherein Y is optionally substituted -(C₁-C₆)alkyl-. In another embodiment is a compound of Formula (Ic) wherein Y is optionally substituted (C₃-C₇)cycloalkyl-. In another embodiment is a compound of Formula (Ic) wherein Y is optionally substituted heterocycloalkyl. In another embodiment is a compound of Formula (Ic) wherein Y is -O-. In another embodiment is a compound of Formula (Ic) wherein Y is -(C₂-C₆)alkynyl. In another embodiment is a compound of Formula (Ic) wherein Y is -O-(C₁-C₆)alkyl-. In another embodiment is a compound of Formula (Ic) wherein Y is a bond.

In another embodiment is a compound of Formula (Ic) wherein Z is -(C₁-C₁₂)alkyl. In a further embodiment is a compound of Formula (Ic) wherein Z is n-butyl, isobutyl, or tert-butyl. In another embodiment is a compound of Formula (Ic) wherein Z is -O-(C₁-C₁₂)alkyl. In another embodiment is a compound of Formula (Ic) wherein Z is -O-(C₃-C₇)cycloalkyl. In another embodiment is a compound of Formula (Ic) wherein Z is -(C₂-C₁₂)alkenyl. In another embodiment is a compound of Formula (Ic) wherein Z is optionally substituted aryl. In a further embodiment is a compound of Formula (Ic) wherein Z is optionally substituted phenyl. In a further embodiment is a compound of Formula (Ic) wherein Z is phenyl monsubstituted or disubstituted with a substituent independently selected from -(C₁-C₈)alkyl. In a further embodiment is a compound of Formula (Ic) wherein Z is phenyl monosubstituted with n-butyl, isobutyl, or tert-butyl. In a further embodiment is a compound of Formula (Ic) wherein Z is phenyl monosubstituted with n-butyl. In a further embodiment is a compound of Formula (Ic) wherein Z is phenyl monosubstituted with isobutyl. In a further embodiment is a compound of Formula (Ic) wherein Z is phenyl monosubstituted with tert-butyl. In another embodiment is a compound of Formula (Ic) wherein Z is optionally substituted heteroaryl. In another embodiment is a compound of Formula (Ic) wherein Z is optionally substituted -(C₃-C₇)cycloalkyl. In another embodiment is a compound of Formula (Ic) wherein Z is optionally substituted heterocycloalkyl. In another embodiment is a compound of Formula (Ic) wherein Z is halogen.

In another embodiment is a compound of Formula (Ic) wherein Z-Y-X- is not

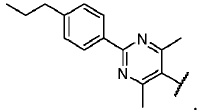

In another embodiment is a compound of Formula (I) having the structure of Formula (Id): 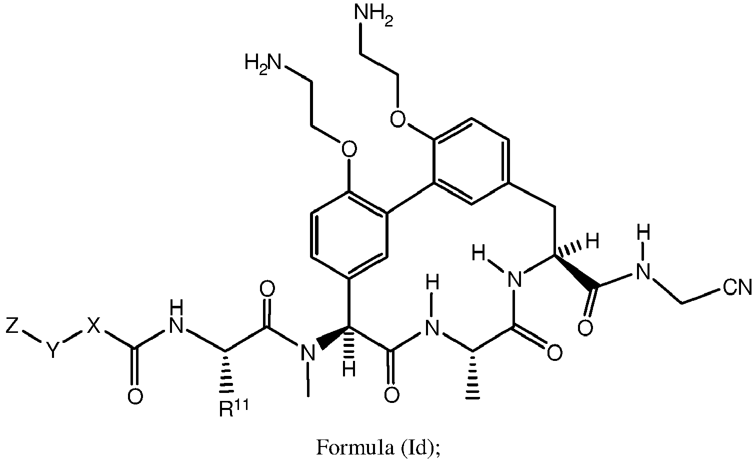 wherein:
R¹¹ is -CH₂NH₂, -CH₂CH₂NH, or -CH₂CH₂CH₂NH₂;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring; and

or a pharmaceutically acceptable salt, solvate, or prodrug thereof.In one embodiment is a compound of Formula (Id) wherein R¹¹ is -CH₂NH₂. In another embodiment is a compound of Formula (Id) wherein R¹¹ is -CH₂CH₂NH₂. In another embodiment is a compound of Formula (Id) wherein R¹¹ is - CH₂CH₂CH₂NH₂.

In another embodiment is a compound of Formula (Id) wherein X is optionally substituted aryl. In a further embodiment is a compound of Formula (Id) wherein X is optionally substituted phenyl. In another embodiment is a compound of Formula (Id) wherein X is optionally substituted heteroaryl. In a further embodiment is a compound of Formula (Id) wherein X is disubstituted heteroaryl. In a further embodiment is a compound of Formula (Id) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (Id) wherein X is heteroaryl disubstituted with substituents each independently selected from -(C₁-C₆)alkyl. In a further embodiment is a compound of Formula (Id) wherein X is heteroaryl disubstituted with methyl. In a further embodiment is a compound of Formula (Id) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (Id) wherein X is pyridinyl disubstituted with substituents each independently selected from -(C₁-C₆)alkyl. In a further embodiment is a compound of Formula (Id) wherein X is pyridinyl disubstituted with methyl. In a further embodiment is a compound of Formula (Id) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (Id) wherein X is pyrimidinyl disubstituted with substituents each independently selected from -(C₁-C₆)alkyl. In a further embodiment is a compound of Formula (Id) wherein X is pyrimidinyl disubstituted with methyl. In another embodiment is a compound of Formula (Id) wherein X is optionally substituted -(C₁-C₆)alkyl-.

In another embodiment is a compound of Formula (Id) wherein Y is optionally substituted aryl. In a further embodiment is a compound of Formula (Id) wherein Y is optionally substituted phenyl. In another embodiment is a compound of Formula (Id) wherein Y is optionally substituted heteroaryl. In another embodiment is a compound of Formula (Id) wherein Y is optionally substituted -(C₁-C₆)alkyl-. In another embodiment is a compound of Formula (Id) wherein Y is optionally substituted (C₃-C₇)cycloalkyl-. In another embodiment is a compound of Formula (Id) wherein Y is optionally substituted heterocycloalkyl. In another embodiment is a compound of Formula (Id) wherein Y is -O-. In another embodiment is a compound of Formula (Id) wherein Y is -(C₂-C₆)alkynyl. In another embodiment is a compound of Formula (Id) wherein Y is -O-(C₁-C₆)alkyl-. In another embodiment is a compound of Formula (Id) wherein Y is a bond.

In another embodiment is a compound of Formula (Id) wherein Z is -(C₁-C₁₂)alkyl. In a further embodiment is a compound of Formula (Id) wherein Z is n-butyl, isobutyl, or tert-butyl. In another embodiment is a compound of Formula (Id) wherein Z is -O-(C₁-C₁₂)alkyl. In another embodiment is a compound of Formula (Id) wherein Z is -O-(C₃-C₇)cycloalkyl. In another embodiment is a compound of Formula (Id) wherein Z is -(C₂-C₁₂)alkenyl. In another embodiment is a compound of Formula (Id) wherein Z is optionally substituted aryl. In a further embodiment is a compound of Formula (Id) wherein Z is optionally substituted phenyl. In a further embodiment is a compound of Formula (Id) wherein Z is phenyl monsubstituted or disubstituted with a substituent independently selected from -(C₁-C₈)alkyl. In a further embodiment is a compound of Formula (Id) wherein Z is phenyl monosubstituted with n-butyl, isobutyl, or tert-butyl. In a further embodiment is a compound of Formula (Id) wherein Z is phenyl monosubstituted with n-butyl. In a further embodiment is a compound of Formula (Id) wherein Z is phenyl monosubstituted with isobutyl. In a further embodiment is a compound of Formula (Id) wherein Z is phenyl monosubstituted with tert-butyl. In another embodiment is a compound of Formula (Id) wherein Z is optionally substituted heteroaryl. In another embodiment is a compound of Formula (Id) wherein Z is optionally substituted -(C₃-C₇)cycloalkyl. In another embodiment is a compound of Formula (Id) wherein Z is optionally substituted heterocycloalkyl. In another embodiment is a compound of Formula (Id) wherein Z is halogen.

In another embodiment is a compound of Formula (Id) wherein Z-Y-X- is not

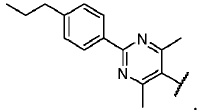

In another embodiment is a compound of Formula (I) having the structure of Formula (Ie): 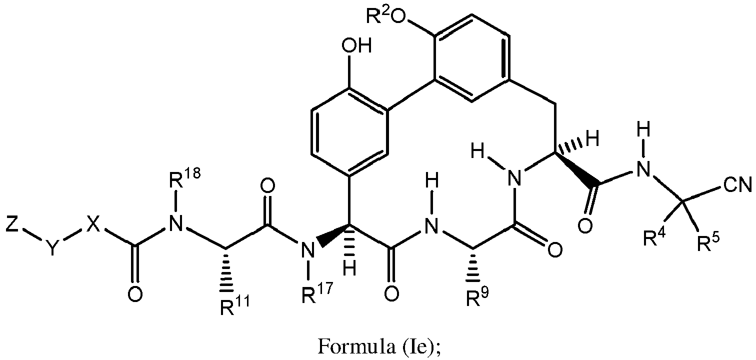 wherein:
R² is H, -(C₁-C₆)alkyl, -(C₁-C₆)alkyl-OR²³, -CH₂CH(OH)CH₂NH₂, -CH₂CH(heterocycloalkyl)CH₂NH₂, - CH₂C(O)NH₂, -CH₂C(O)N(H)CH₂CN, -(C₁-C₆)alkyl-C(O)OR²³, -(C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²², -(C₁-C₆)alkyl-N(R²³)C(O)(C₁-C₆)alkyl, - (C₁-C₆)alkyl-C(O)N(R²³)(C₁-C₆)alkyl, -(C₁-C₆)alkyl-C(O)N(R²³)(C₁-C₆)alkyl-heterocycloalkyl, (C₁-C₆)heteroalkyl, or optionally substituted heterocycloalkyl;
R⁴ is H, -(C₁-C₆)alkyl, -(C₁-C₆)alkyl-OH, -(C₃-C₆)cycloalkyl, or -C(O)NH₂;
R⁵ is H or -(C₁-C₆)alkyl;
or R⁴ and R⁵ and the carbon atom to which they are attached form a cyclopropyl ring;
R⁹ is H, -(C₁-C₆)alkyl, -(C₁-C₆)haloalkyl, or -(C₃-C₆)cycloalkyl;
R¹¹ is H, -NH₂, -(C₁-C₆)alkyl, -(C₁-C₆)alkyl-OR²³, -(C₁-C₆)alkyl-SR²³, -(C₁-C₆)alkyl-C(0)OR²³, -(C₁-C₆)alkyl-NR²¹R²², -(C₁-C₆)alkyl-NR²³OR²³, -(C₁-C₆)alkyl-NHC(O)NR²³0R²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
or R¹¹ and R¹⁸ are combined to form an optionally substituted heterocycloalkyl ring; and R¹² is H;
R¹⁷ and R¹⁸ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-,
optionally substituted heterocycloalkyl, optionally substituted aryl, or optionally substituted heteroaryl; Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring; and

or a pharmaceutically acceptable salt, solvate, or prodrug thereof.In one embodiment is a compound of Formula (Ie) wherein R¹⁷ is -(C₁-C₆)alkyl. In another embodiment is a compound of Formula (Ie) wherein R¹⁷ is -CH₃. In another embodiment is a compound of Formula (Ie) wherein R¹⁷ is -CH₂CH₃. In another embodiment is a compound of Formula (Ie) wherein R¹⁷ is -(C₃-C₆)cycloalkyl. In another embodiment is a compound of Formula (Ie) wherein R¹⁷ is cyclopropyl. In another embodiment is a compound of Formula (Ie) wherein R¹⁷ is -(C₁-C₆)alkyl-C(O)OR²³. In another embodiment is a compound of Formula (Ie) wherein R¹⁷ is -CH₂CH₂OH. In another embodiment is a compound of Formula (Ie) wherein R¹⁷ is -(C₁-C₆)alkyl-NR²¹R²². In another embodiment is a compound of Formula (Ie) wherein R¹⁷ is -CH₂CH₂NH₂. In another embodiment is a compound of Formula (Ie) wherein R¹⁷ is H.

In another embodiment is a compound of Formula (Ie) wherein R¹⁸ is H.

In another embodiment is a compound of Formula (Ie) wherein R⁵ is H.

In another embodiment is a compound of Formula (Ie) wherein R⁴ is H. In another embodiment is a compound of Formula (Ie) wherein R⁴ is -(C₁-C₆)alkyl. In another embodiment is a compound of Formula (Ie) wherein R⁴ is -CH₃. In another embodiment is a compound of Formula (Ie) wherein R⁴ is - CH₂CH₃. In another embodiment is a compound of Formula (Ie) wherein R⁴ is -(C₁-C₆)alkyl-OH. In another embodiment is a compound of Formula (Ie) wherein R⁴ is -CH_{z}OH. In another embodiment is a compound of Formula (Ie) wherein R⁴ is -(C₃-C₆)cycloalkyl. In another embodiment is a compound of Formula (Ie) wherein R⁴ is cyclopropyl. In another embodiment is a compound of Formula (Ie) wherein R⁴ is -C(O)NH₂.

In another embodiment is a compound of Formula (Ie) wherein R⁴ and R⁵ and the carbon atom to which they are attached form a cyclopropyl ring.

In another embodiment is a compound of Formula (Ie) wherein R⁹ is -(C₁-C₆)alkyl. In another embodiment is a compound of Formula (Ie) wherein R⁹ is -CH₃. In another embodiment is a compound of Formula (Ie) wherein R⁹ is -CH₂CH₃. In another embodiment is a compound of Formula (Ie) wherein R⁹ is -(C₁-C₆)haloalkyl. In another embodiment is a compound of Formula (Ie) wherein R⁹ is -CH₂F. In another embodiment is a compound of Formula (Ie) wherein R⁹ is -CHF₂. In another embodiment is a compound of Formula (Ie) wherein R⁹ is -(C₃-C₆)cycloalkyl. In another embodiment is a compound of Formula (Ie) wherein R⁹ is cyclopropyl. In another embodiment is a compound of Formula (Ie) wherein R⁹ is H.

In another embodiment is a compound of Formula (Ie) wherein R¹¹ is -(C₁-C₆)alkyl. In another embodiment is a compound of Formula (Ie) wherein R¹¹ is -CH₃. In another embodiment is a compound of Formula (Ie) wherein R¹¹ is -(C₁-C₆)alkyl-OR²³. In another embodiment is a compound of Formula (Ie) wherein R¹¹ is -CH₂OH. In another embodiment is a compound of Formula (Ie) wherein R¹¹ is - CH₂CH₂OH. In another embodiment is a compound of Formula (Ie) wherein R¹¹ is -(C₁-C₆)alkyl. In another embodiment is a compound of Formula (Ie) wherein R¹¹ is -(C₁-C₆)alkyl-NR²¹R²². In another embodiment is a compound of Formula (Ie) wherein R¹¹ is -(C₁-C₆)alkyl-NH₂. In another embodiment is a compound of Formula (Ie) wherein R¹¹ is -CH₂NH₂. In another embodiment is a compound of Formula (Ie) wherein R¹¹ is -CH₂CH₂NH₂. In another embodiment is a compound of Formula (Ie) wherein R¹¹ is-CH₂CH₂CH₂NH₂. In another embodiment is a compound of Formula (Ie) wherein R¹¹ is - CH₂CH₂CH₂CH₂NH₂. In another embodiment is a compound of Formula (Ie) wherein R¹¹ is -(C₁-C₆)alkyl-CN. In another embodiment is a compound of Formula (Ie) wherein R¹¹ is -CH₂CN. In another embodiment is a compound of Formula (Ie) wherein R¹¹ is -(C₁-C₆)alkyl-C(O)NR²⁵R²⁶. In another embodiment is a compound of Formula (Ie) wherein R¹¹ is -CH₂C(O)NH₂. In another embodiment is a compound of Formula (Ie) wherein R¹¹ is -CH₂CH₂C(O)NH₂. In another embodiment is a compound of Formula (Ie) wherein R¹¹ is -(C₁-C₆)alkyl-heteroaryl. In another embodiment is a compound of Formula (Ie) wherein R¹¹ is -(C₁-C₆)alkyl-N(H)S(O)₂NR²⁵R²⁶. In another embodiment is a compound of Formula (Ie) wherein R¹¹ is -CH₂N(H)S(O)₂NH₂. In another embodiment is a compound of Formula (Ie) wherein R¹¹ is H.

In another embodiment is a compound of Formula (Ie) wherein R¹¹ and R¹⁸ are combined to form an optionally substituted heterocycloalkyl ring.

In another embodiment is a compound of Formula (Ie) wherein R² is H or -(C₁-C₆)alkyl-NR²¹R²². In another embodiment is a compound of Formula (Ie) wherein R² is H. In another embodiment is a compound of Formula (Ie) wherein R² is -(C₁-C₆)alkyl-NR²¹R²². In another embodiment is a compound of Formula (Ie) wherein R² is -CH₂CH₂NH₂. In another embodiment is a compound of Formula (Ie) wherein R² is -CH₂CH₂CH₂NH₂. In another embodiment is a compound of Formula (Ie) wherein R² is -CH₂NH₂. In another embodiment is a compound of Formula (Ie) wherein R² is H, -(C₁-C₆)alkyl-NR²¹R²², or -CH₂CH(OH)CH₂NH₂. In another embodiment is a compound of Formula (Ie) wherein R² is - CH₂CH(OH)CH₂NH₂.

In another embodiment is a compound of Formula (Ie) wherein X is optionally substituted aryl. In a further embodiment is a compound of Formula (Ie) wherein X is optionally substituted phenyl. In another embodiment is a compound of Formula (Ie) wherein X is optionally substituted heteroaryl. In a further embodiment is a compound of Formula (Ie) wherein X is monosubstituted or disubstituted heteroaryl. In a further embodiment is a compound of Formula (Ie) wherein X is disubstituted heteroaryl. In a further embodiment is a compound of Formula (Ie) wherein X is heteroaryl monosubstituted or disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (Ie) wherein X is heteroaryl monosubstituted or disubstituted with substituents each independently selected from -(C₁-C₆)alkyl. In a further embodiment is a compound of Formula (Ie) wherein X is heteroaryl monosubstituted or disubstituted with methyl. In a further embodiment is a compound of Formula (Ie) wherein X is pyridinyl monosubstituted or disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (Ie) wherein X is pyridinyl monosubstituted or disubstituted with substituents each independently selected from -(C₁-C₆)alkyl. In a further embodiment is a compound of Formula (Ie) wherein X is pyridinyl monosubstituted or disubstituted with methyl. In a further embodiment is a compound of Formula (Ie) wherein X is pyrimidinyl monosubstituted or disubstituted with substituents each independently selected from halogen, - CN, optionally substituted -(C₁-C₆)alkyl, optionally substituted -O-(C₁-C₆)alkyl, OR²³, -NR²⁵R²⁶, or -NO₂. In a further embodiment is a compound of Formula (Ie) wherein X is pyrimidinyl monosubstituted or disubstituted with substituents each independently selected from -(C₁-C₆)alkyl. In a further embodiment is a compound of Formula (Ie) wherein X is pyrimidinyl monosubstituted or disubstituted with methyl. In another embodiment is a compound of Formula (Ie) wherein X is optionally substituted -(C₁-C₆)alkyl-.

In another embodiment is a compound of Formula (Ie) wherein Y is optionally substituted aryl. In a further embodiment is a compound of Formula (Ie) wherein Y is optionally substituted phenyl. In another embodiment is a compound of Formula (Ie) wherein Y is optionally substituted heteroaryl. In another embodiment is a compound of Formula (Ie) wherein Y is optionally substituted -(C₁-C₆)alkyl-. In another embodiment is a compound of Formula (Ie) wherein Y is optionally substituted (C₃-C₇)cycloalkyl-. In another embodiment is a compound of Formula (Ie) wherein Y is optionally substituted heterocycloalkyl. In another embodiment is a compound of Formula (Ie) wherein Y is -O-. In another embodiment is a compound of Formula (Ie) wherein Y is -(C₂-C₆)alkynyl. In another embodiment is a compound of Formula (Ie) wherein Y is -O-(C₁-C₆)alkyl-. In another embodiment is a compound of Formula (Ie) wherein Y is a bond.

In another embodiment is a compound of Formula (Ie) wherein Z is -(C₁-C₁₂)alkyl. In a further embodiment is a compound of Formula (Ie) wherein Z is n-butyl, isobutyl, or tert-butyl. In another embodiment is a compound of Formula (Ie) wherein Z is -O-(C₁-C₁₂)alkyl. In another embodiment is a compound of Formula (Ie) wherein Z is -O-(C₃-C₇)cycloalkyl. In another embodiment is a compound of Formula (Ie) wherein Z is -(C₂-C₁₂)alkenyl. In another embodiment is a compound of Formula (Ie) wherein Z is optionally substituted aryl. In a further embodiment is a compound of Formula (Ie) wherein Z is optionally substituted phenyl. In a further embodiment is a compound of Formula (Ie) wherein Z is phenyl monsubstituted or disubstituted with a substituent independently selected from -(C₁-C₈)alkyl. In a further embodiment is a compound of Formula (Ie) wherein Z is phenyl monosubstituted with n-butyl, isobutyl, or tert-butyl. In a further embodiment is a compound of Formula (Ie) wherein Z is phenyl monosubstituted with n-butyl. In a further embodiment is a compound of Formula (Ie) wherein Z is phenyl monosubstituted with isobutyl. In a further embodiment is a compound of Formula (Ie) wherein Z is phenyl monosubstituted with tert-butyl. In another embodiment is a compound of Formula (Ie) wherein Z is optionally substituted heteroaryl. In another embodiment is a compound of Formula (Ie) wherein Z is optionally substituted -(C₃-C₇)cycloalkyl. In another embodiment is a compound of Formula (Ie) wherein Z is optionally substituted heterocycloalkyl. In another embodiment is a compound of Formula (Ie) wherein Z is halogen.

In another embodiment is a compound of Formula (I) having the structure of Formula (If): 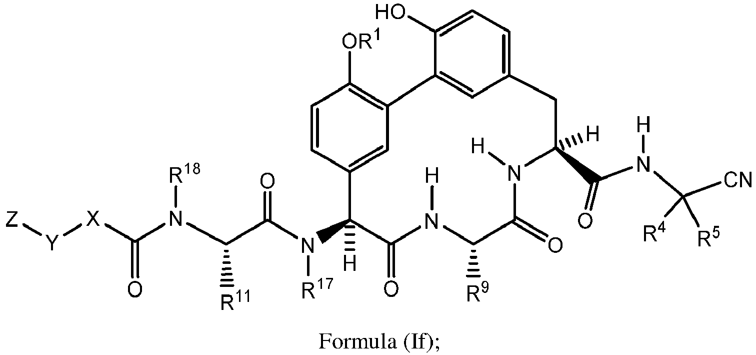 wherein:
R¹ is H, -(C₁-C₆)alkyl, -(C₁-C₆)alkyl-OR²³, -CH₂CH(OH)CH₂NH₂, -CH₂CH(heterocycloalkyl)CH₂NH₂, - CH₂C(O)NH₂, -CH₂C(O)N(H)CH₂CN, -(C₁-C₆)alkyl-C(O)OR²³, -(C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²², -(C₁-C₆)alkyl-N(R²³)C(O)(C₁-C₆)alkyl, (C₁-C₆)alkyl-C(O)N(R²³)(C₁-C₆)alkyl, -(C₁-C₆)alkyl-C(O)N(R²³)(C₁-C₆)alkyl-heterocycloalkyl, (C₁-C₆)heteroalkyl, or optionally substituted heterocycloalkyl;
R⁴ is H, -(C₁-C₆)alkyl, -(C₁-C₆)alkyl-OH, -(C₃-C₆)cycloalkyl, or -C(O)NH₂;
R⁵ is H or -(C₁-C₆)alkyl;
or R⁴ and R⁵ and the carbon atom to which they are attached form a cyclopropyl ring;
R⁹ is H, -(C₁-C₆)alkyl, -(C₁-C₆)haloalkyl, or -(C₃-C₆)cycloalkyl;
R¹¹ is H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
or R¹¹ and R¹⁸ are combined to form an optionally substituted heterocycloalkyl ring; and R¹² is H;
R¹⁷ and R¹⁸ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring; and

or a pharmaceutically acceptable salt, solvate, or prodrug thereof.In one embodiment is a compound of Formula (If) wherein R¹⁷ is -(C₁-C₆)alkyl. In another embodiment is a compound of Formula (If) wherein R¹⁷ is -CH₃. In another embodiment is a compound of Formula (If) wherein R¹⁷ is -CH₂CH₃. In another embodiment is a compound of Formula (If) wherein R¹⁷ is -(C₃-C₆)cycloalkyl. In another embodiment is a compound of Formula (If) wherein R¹⁷ is cyclopropyl. In another embodiment is a compound of Formula (If) wherein R¹⁷ is -(C₁-C₆)alkyl-C(O)OR²³. In another embodiment is a compound of Formula (If) wherein R¹⁷ is -CH₂CH₂OH. In another embodiment is a compound of Formula (If) wherein R¹⁷ is -(C₁-C₆)alkyl-NR²¹R²². In another embodiment is a compound of Formula (If) wherein R¹⁷ is -CH₂CH₂NH₂. In another embodiment is a compound of Formula (If) wherein R¹⁷ is H.

In another embodiment is a compound of Formula (If) wherein R¹⁸ is H.

In another embodiment is a compound of Formula (If) wherein R⁵ is H.

In another embodiment is a compound of Formula (If) wherein R⁴ is H. In another embodiment is a compound of Formula (If) wherein R⁴ is -(C₁-C₆)alkyl. In another embodiment is a compound of Formula (If) wherein R⁴ is -CH₃. In another embodiment is a compound of Formula (If) wherein R⁴ is - CH₂CH₃. In another embodiment is a compound of Formula (If) wherein R⁴ is -(C₁-C₆)alkyl-OH. In another embodiment is a compound of Formula (If) wherein R⁴ is -CH₂OH. In another embodiment is a compound of Formula (If) wherein R⁴ is -(C₃-C₆)cycloalkyl. In another embodiment is a compound of Formula (If) wherein R⁴ is cyclopropyl. In another embodiment is a compound of Formula (If) wherein R⁴ is -C(O)NH₂.

In another embodiment is a compound of Formula (If) wherein R⁴ and R⁵ and the carbon atom to which they are attached form a cyclopropyl ring.

In another embodiment is a compound of Formula (If) wherein R⁹ is -(C₁-C₆)alkyl. In another embodiment is a compound of Formula (If) wherein R⁹ is -CH₃. In another embodiment is a compound of Formula (If) wherein R⁹ is -CH₂CH₃. In another embodiment is a compound of Formula (If) wherein R⁹ is -(C₁-C₆)haloalkyl. In another embodiment is a compound of Formula (If) wherein R⁹ is -CH₂F. In another embodiment is a compound of Formula (If) wherein R⁹ is -CHF₂. In another embodiment is a compound of Formula (If) wherein R⁹ is -(C₃-C₆)cycloalkyl. In another embodiment is a compound of Formula (If) wherein R⁹ is cyclopropyl. In another embodiment is a compound of Formula (If) wherein R⁹ is H.

In another embodiment is a compound of Formula (If) wherein R¹¹ is -(C₁-C₆)alkyl. In another embodiment is a compound of Formula (If) wherein R¹¹ is -CH₃. In another embodiment is a compound of Formula (If) wherein R¹¹ is -(C₁-C₆)alkyl-OR²³. In another embodiment is a compound of Formula (If) wherein R¹¹ is -CH₂OH. In another embodiment is a compound of Formula (If) wherein R¹¹ is - CH₂CH₂OH. In another embodiment is a compound of Formula (If) wherein R¹¹ is -(C₁-C₆)alkyl. In another embodiment is a compound of Formula (If) wherein R¹¹ is -(C₁-C₆)alkyl-NR²¹R²². In another embodiment is a compound of Formula (If) wherein R¹¹ is -(C₁-C₆)alkyl-NH₂. In another embodiment is a compound of Formula (If) wherein R¹¹ is -CH₂NH₂. In another embodiment is a compound of Formula (If) wherein R¹¹ is -CH₂CH₂NH₂. In another embodiment is a compound of Formula (If) wherein R¹¹ is - CH₂CH₂CH₂NH₂. In another embodiment is a compound of Formula (If) wherein R¹¹ is - CH₂CH₂CH₂CH₂NH₂. In another embodiment is a compound of Formula (If) wherein R¹¹ is -(C₁-C₆)alkyl-CN. In another embodiment is a compound of Formula (If) wherein R¹¹ is -CH₂CN. In another embodiment is a compound of Formula (If) wherein R¹¹ is -(C₁-C₆)alkyl-C(O)NR²⁵R²⁶. In another embodiment is a compound of Formula (If) wherein R¹¹ is -CH₂C(O)NH₂. In another embodiment is a compound of Formula (If) wherein R¹¹ is -CH₂CH₂C(O)NH₂. In another embodiment is a compound of Formula (If) wherein R¹¹ is -(C₁-C₆)alkyl-heteroaryl. In another embodiment is a compound of Formula (If) wherein R¹¹ is -(C₁-C₆)alkyl-N(H)S(O)₂NR²⁵R²⁶. In another embodiment is a compound of Formula (If) wherein R¹¹ is -CH₂N(H)S(O)₂NH₂. In another embodiment is a compound of Formula (If) wherein R¹¹ is H.

In another embodiment is a compound of Formula (If) wherein R¹¹ and R¹⁸ are combined to form an optionally substituted heterocycloalkyl ring.

In another embodiment is a compound of Formula (If) wherein R¹ is H or -(C₁-C₆)alkyl-NR²¹R²². In another embodiment is a compound of Formula (If) wherein R¹ is H. In another embodiment is a compound of Formula (If) wherein R¹ is -(C₁-C₆)alkyl-NR²¹R²². In another embodiment is a compound of Formula (If) wherein R¹ is -CH₂CH₂NH₂. In another embodiment is a compound of Formula (If) wherein R¹ is -CH₂CH₂CH₂NH₂. In another embodiment is a compound of Formula (If) wherein R¹ is - CH₂NH₂. In another embodiment is a compound of Formula (If) wherein R¹ is H, -(C₁-C₆)alkyl-NR²¹R²², or -CH₂CH(OH)CH₂NH₂. In another embodiment is a compound of Formula (If) wherein R¹ is - CH₂CH(OH)CH₂NH₂.

In another embodiment is a compound of Formula (If) wherein X is optionally substituted aryl. In a further embodiment is a compound of Formula (If) wherein X is optionally substituted phenyl. In another embodiment is a compound of Formula (If) wherein X is optionally substituted heteroaryl. In a further embodiment is a compound of Formula (If) wherein X is monosubstituted or disubstituted heteroaryl. In a further embodiment is a compound of Formula (If) wherein X is disubstituted heteroaryl. In a further embodiment is a compound of Formula (If) wherein X is heteroaryl monosubstituted or disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (If) wherein X is heteroaryl monosubstituted or disubstituted with substituents each independently selected from -(C₁-C₆)alkyl. In a further embodiment is a compound of Formula (If) wherein X is heteroaryl monosubstituted or disubstituted with methyl. In a further embodiment is a compound of Formula (If) wherein X is pyridinyl monosubstituted or disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (If) wherein X is pyridinyl monosubstituted or disubstituted with substituents each independently selected from -(C₁-C₆)alkyl. In a further embodiment is a compound of Formula (If) wherein X is pyridinyl monosubstituted or disubstituted with methyl. In a further embodiment is a compound of Formula (If) wherein X is pyrimidinyl monosubstituted or disubstituted with substituents each independently selected from halogen, - CN, optionally substituted -(C₁-C₆)alkyl, optionally substituted -O-(C₁-C₆)alkyl, OR²³, -NR²⁵R²⁶, or -NO₂. In a further embodiment is a compound of Formula (If) wherein X is pyrimidinyl monosubstituted or disubstituted with substituents each independently selected from -(C₁-C₆)alkyl. In a further embodiment is a compound of Formula (If) wherein X is pyrimidinyl monosubstituted or disubstituted with methyl. In another embodiment is a compound of Formula (If) wherein X is optionally substituted -(C₁-C₆)alkyl-.

In another embodiment is a compound of Formula (If) wherein Y is optionally substituted aryl. In a further embodiment is a compound of Formula (If) wherein Y is optionally substituted phenyl. In another embodiment is a compound of Formula (If) wherein Y is optionally substituted heteroaryl. In another embodiment is a compound of Formula (If) wherein Y is optionally substituted -(C₁-C₆)alkyl-. In another embodiment is a compound of Formula (If) wherein Y is optionally substituted (C₃-C₇)cycloalkyl-. In another embodiment is a compound of Formula (If) wherein Y is optionally substituted heterocycloalkyl. In another embodiment is a compound of Formula (If) wherein Y is -O-. In another embodiment is a compound of Formula (If) wherein Y is -(C₂-C₆)alkynyl. In another embodiment is a compound of Formula (If) wherein Y is -O-(C₁-C₆)alkyl-. In another embodiment is a compound of Formula (If) wherein Y is a bond.

In another embodiment is a compound of Formula (If) wherein Z is -(C₁-C₁₂)alkyl. In a further embodiment is a compound of Formula (If) wherein Z is n-butyl, isobutyl, or tert-butyl. In another embodiment is a compound of Formula (If) wherein Z is -O-(C₁-C₁₂)alkyl. In another embodiment is a compound of Formula (If) wherein Z is -O-(C₃-C₇)cycloalkyl. In another embodiment is a compound of Formula (If) wherein Z is -(C₂-C₁₂)alkenyl. In another embodiment is a compound of Formula (If) wherein Z is optionally substituted aryl. In a further embodiment is a compound of Formula (If) wherein Z is optionally substituted phenyl. In a further embodiment is a compound of Formula (If) wherein Z is phenyl monsubstituted or disubstituted with a substituent independently selected from -(C₁-C₈)alkyl. In a further embodiment is a compound of Formula (If) wherein Z is phenyl monosubstituted with n-butyl, isobutyl, or tert-butyl. In a further embodiment is a compound of Formula (If) wherein Z is phenyl monosubstituted with n-butyl. In a further embodiment is a compound of Formula (If) wherein Z is phenyl monosubstituted with isobutyl. In a further embodiment is a compound of Formula (If) wherein Z is phenyl monosubstituted with tert-butyl. In another embodiment is a compound of Formula (If) wherein Z is optionally substituted heteroaryl. In another embodiment is a compound of Formula (If) wherein Z is optionally substituted -(C₃-C₇)cycloalkyl. In another embodiment is a compound of Formula (If) wherein Z is optionally substituted heterocycloalkyl. In another embodiment is a compound of Formula (If) wherein Z is halogen.

In another aspect described herein is a compound of Formula (II): 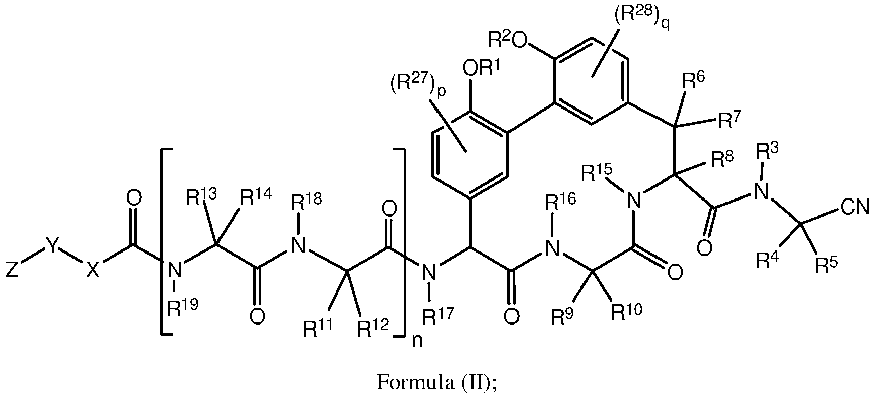 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring;
R³ is H or -(C₁-C₆)alkyl;
R⁴ is H, -(C₁-C₆)alkyl, -(C₁-C₆)alkyl-OH, -(C₃-C₆)cycloalkyl, or -C(O)NH₂;
or R³ and R⁴ are combined to form a heterocycloalkyl ring;
R⁵ is H or -(C₁-C₆)alkyl;
or R⁴ and R⁵ and the carbon atom to which they are attached form a cyclopropyl ring;
R⁶, R⁷, and R⁸ are each independently H or -(C₁-C₆)alkyl;
R⁹ is H, -(C₁-C₆)alkyl, -(C₁-C₆)haloalkyl, or -(C₃-C₆)cycloalkyl;
R¹⁰ is H or -(C₁-C₆)alkyl;
R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
R¹³ and R¹⁴ are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-CN, -(C₁-C₆)alkyl-C(O)NR²⁵R²⁶, -(C₁-C₆)alkyl-N(H)C(NH)NH₂, -(C₁-C₆)alkyl-heterocycloalkyl, or -(C₁-C₆)alkyl-heteroaryl;
or R¹³ and R¹⁹ are combined to form an optionally substituted heterocycloalkyl ring; and R¹⁴ is H;
R¹⁵, R¹⁶, R¹⁷, R¹⁸, and R¹⁹ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, -C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring;
each R²⁷ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R¹ and R²⁷ and the atoms to which they are attached form an optionally substituted 5- or 6-membered heterocycloalkyl ring;
each R²⁸ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R² and R²⁸ and the atoms to which they are attached form an optionally substituted 5- or 6-membered heterocycloalkyl ring;
n is 0 or 1;
p is 0, 1, or 2; and
q is 0, 1, or 2;
or a pharmaceutically acceptable salt, solvate, or prodrug thereof.

In one embodiment is a compound of Formula (II) wherein n is 0. In another embodiment is a compound of Formula (II) wherein n is 1.

In another embodiment is a compound of Formula (II) wherein R⁶, R⁷, and R⁸ are H.

In another embodiment is a compound of Formula (II) wherein R¹⁵ and R¹⁶ are H.

In one embodiment is a compound of Formula (II) wherein R¹⁷ is -(C₁-C₆)alkyl. In another embodiment is a compound of Formula (II) wherein R¹⁷ is -CH₃. In another embodiment is a compound of Formula (II) wherein R¹⁷ is -CH₂CH₃. In another embodiment is a compound of Formula (II) wherein R¹⁷ is -(C₃-C₆)cycloalkyl. In another embodiment is a compound of Formula (II) wherein R¹⁷ is cyclopropyl. In another embodiment is a compound of Formula (II) wherein R¹⁷ is -(C₁-C₆)alkyl-C(O)OR²³. In another embodiment is a compound of Formula (II) wherein R¹⁷ is -CH₂CH₂OH. In another embodiment is a compound of Formula (II) wherein R¹⁷ is -(C₁-C₆)alkyl-NR²¹R²². In another embodiment is a compound of Formula (II) wherein R¹⁷ is -CH₂CH₂NH₂. In another embodiment is a compound of Formula (II) wherein R¹⁷ is H.

In another embodiment is a compound of Formula (II) wherein R¹⁸ is H.

In another embodiment is a compound of Formula (II) wherein R¹⁹ is H.

In another embodiment is a compound of Formula (II) wherein R³ is H.

In another embodiment is a compound of Formula (II) wherein R⁵ is H.

In another embodiment is a compound of Formula (II) wherein R⁴ is H. In another embodiment is a compound of Formula (II) wherein R⁴ is -(C₁-C₆)alkyl. In another embodiment is a compound of Formula (II) wherein R⁴ is -CH₃. In another embodiment is a compound of Formula (II) wherein R⁴ is - CH₂CH₃. In another embodiment is a compound of Formula (II) wherein R⁴ is -(C₁-C₆)alkyl-OH. In another embodiment is a compound of Formula (II) wherein R⁴ is -CH₂OH. In another embodiment is a compound of Formula (II) wherein R⁴ is -(C₃-C₆)cycloalkyl. In another embodiment is a compound of Formula (II) wherein R⁴ is cyclopropyl. In another embodiment is a compound of Formula (II) wherein R⁴ is -C(O)NH₂.

In another embodiment is a compound of Formula (II) wherein R³, R⁴, and R⁵ are H.

In another embodiment is a compound of Formula (II) wherein R⁴ and R⁵ and the carbon atom to which they are attached form a cyclopropyl ring.

In another embodiment is a compound of Formula (II) wherein R¹⁰ is H.

In another embodiment is a compound of Formula (II) wherein R¹⁰ is H and R⁹ is -(C₁-C₆)alkyl. In another embodiment is a compound of Formula (II) wherein R¹⁰ is H and R⁹ is -CH₃. In another embodiment is a compound of Formula (II) wherein R¹⁰ is H and R⁹ is -CH₂CH₃. In another embodiment is a compound of Formula (II) wherein R¹⁰ is H and R⁹ is -(C₁-C₆)haloalkyl. In another embodiment is a compound of Formula (II) wherein R¹⁰ is H and R⁹ is -CH₂F. In another embodiment is a compound of Formula (II) wherein R¹⁰ is H and R⁹ is -CHF₂. In another embodiment is a compound of Formula (II) wherein R¹⁰ is H and R⁹ is -(C₃-C₆)cycloalkyl. In another embodiment is a compound of Formula (II) wherein R¹⁰ is H and R⁹ is cyclopropyl. In another embodiment is a compound of Formula (II) wherein R¹⁰ is H and R⁹ is H.

In another embodiment is a compound of Formula (II) wherein R¹² is H.

In another embodiment is a compound of Formula (II) wherein R¹² is H and R¹¹ is -(C₁-C₆)alkyl. In another embodiment is a compound of Formula (II) wherein R¹² is H and R¹¹ is -CH₃. In another embodiment is a compound of Formula (II) wherein R¹² is H and R¹¹ is -(C₁-C₆)alkyl-OR²³. In another embodiment is a compound of Formula (II) wherein R¹¹ is -CH₂OH. In another embodiment is a compound of Formula (II) wherein R¹² is H and R¹¹ is -CH₂CH₂OH. In another embodiment is a compound of Formula (II) wherein R¹² is H and R¹¹ is -(C₁-C₆)alkyl. In another embodiment is a compound of Formula (II) wherein R¹² is H and R¹¹ is -(C₁-C₆)alkyl-NR²¹R²². In another embodiment is a compound of Formula (II) wherein R¹² is H and R¹¹ is -(C₁-C₆)alkyl-NH₂. In another embodiment is a compound of Formula (II) wherein R¹² is H and R¹¹ is -CH₂NH₂. In another embodiment is a compound of Formula (II) wherein R¹² is H and R¹¹ is -CH₂CH₂NH₂. In another embodiment is a compound of Formula (II) wherein R¹² is H and R¹¹ is -CH₂CH₂CH₂NH₂. In another embodiment is a compound of Formula (II) wherein R¹² is H and R¹¹ is -CH₂CH₂CH₂CH₂NH₂. In another embodiment is a compound of Formula (II) wherein R¹² is H and R¹¹ is -(C₁-C₆)alkyl-CN. In another embodiment is a compound of Formula (II) wherein R¹² is H and R¹¹ is -CH₂CN. In another embodiment is a compound of Formula (II) wherein R¹² is H and R¹¹ is -(C₁-C₆)alkyl-C(O)NR²⁵R²⁶. In another embodiment is a compound of Formula (II) wherein R¹² is H and R¹¹ is -CH₂C(O)NH₂. In another embodiment is a compound of Formula (II) wherein R¹² is H and R¹¹ is -CH₂CH₂C(O)NH₂. In another embodiment is a compound of Formula (II) wherein R¹² is H and R¹¹ is -(C₁-C₆)alkyl-heteroaryl. In another embodiment is a compound of Formula (II) wherein R¹² is H and R¹¹ is -(C₁-C₆)alkyl-N(H)S(O)₂NR²⁵R²⁶. In another embodiment is a compound of Formula (II) wherein R¹² is H and R¹¹ is -CH₂N(H)S(O)₂NH₂. In another embodiment is a compound of Formula (II) wherein R¹² is H and R¹¹ is H.

In another embodiment is a compound of Formula (II) wherein R¹¹ and R¹⁸ are combined to form an optionally substituted heterocycloalkyl ring and R¹² is H.

In another embodiment is a compound of Formula (II) wherein R¹⁴ is H.

In another embodiment is a compound of Formula (II) wherein R¹⁴ is H and R¹³ is -(C₁-C₆)alkyl. In another embodiment is a compound of Formula (II) wherein R¹⁴ is H and R¹³ is -CH₃. In another embodiment is a compound of Formula (II) wherein R¹⁴ is H and R¹³ is -(C₁-C₆)alkyl-OR²³. In another embodiment is a compound of Formula (II) wherein R¹⁴ is H and R¹³ is -CH₂OH. In another embodiment is a compound of Formula (II) wherein R¹⁴ is H and R¹³ is -CH₂CH₂OH. In another embodiment is a compound of Formula (II) wherein R¹⁴ is H and R¹³ is -(C₁-C₆)alkyl. In another embodiment is a compound of Formula (II) wherein R¹⁴ is H and R¹³ is -(C₁-C₆)alkyl-NR²¹R²². In another embodiment is a compound of Formula (II) wherein R¹⁴ is H and R¹³ is -(C₁-C₆)alkyl-NH₂. In another embodiment is a compound of Formula (II) wherein R¹⁴ is H and R¹³ is -CH₂NH₂. In another embodiment is a compound of Formula (II) wherein R¹⁴ is H and R¹³ is -CH₂CH₂NH₂. In another embodiment is a compound of Formula (II) wherein R¹⁴ is H and R¹³ is -CH₂CH₂CH₂NH₂. In another embodiment is a compound of Formula (II) wherein R¹⁴ is H and R¹³ is -CH₂CH₂CH₂CH₂NH₂. In another embodiment is a compound of Formula (II) wherein R¹⁴ is H and R¹³ is -(C₁-C₆)alkyl-CN. In another embodiment is a compound of Formula (II) wherein R¹⁴ is H and R¹³ is -CH₂CN. In another embodiment is a compound of Formula (II) wherein R¹⁴ is H and R¹³ is -(C₁-C₆)alkyl-C(O)NR²⁵R²⁶. In another embodiment is a compound of Formula (II) wherein R¹⁴ is H and R¹³ is -CH₂C(O)NH₂. In another embodiment is a compound of Formula (II) wherein R¹⁴ is H and R¹³ is -CH₂CH₂C(O)NH₂. In another embodiment is a compound of Formula (II) wherein R¹⁴ is H and R¹³ is -(C₁-C₆)alkyl-heteroaryl. In another embodiment is a compound of Formula (II) wherein R¹⁴ is H and R¹³ is H.

In another embodiment is a compound of Formula (II) wherein R¹³ and R¹⁹ are combined to form an optionally substituted heterocycloalkyl ring and R¹⁴ is H.

In another embodiment is a compound of Formula (II) wherein p is 1 and R²⁷ is halogen. In another embodiment is a compound of Formula (II) wherein p is 1 and R²⁷ is optionally substituted -(C₁-C₆)alkyl. In another embodiment is a compound of Formula (II) wherein q is 0, p is 1 and R²⁷ is halogen. In another embodiment is a compound of Formula (II) wherein q is 0, p is 1 and R²⁷ is optionally substituted -(C₁-C₆)alkyl. In another embodiment is a compound of Formula (II) wherein q is 1 and R²⁸ is halogen. In another embodiment is a compound of Formula (II) wherein q is 1 and R²⁸ is optionally substituted -(C₁-C₆)alkyl. In another embodiment is a compound of Formula (II) wherein p is 0, q is 1 and R²⁸ is halogen. In another embodiment is a compound of Formula (II) wherein p is 0, q is 1 and R²⁸ is optionally substituted -(C₁-C₆)alkyl.

In another embodiment is a compound of Formula (II) wherein p is 0, and q is 0.

In another embodiment is a compound of Formula (II) wherein R¹ and R² are each independently H or -(C₁-C₆)alkyl-NR²¹R²². In another embodiment is a compound of Formula (II) wherein R¹ and R² are each H. In another embodiment is a compound of Formula (II) wherein R¹ and R² are each independently - (C₁-C₆)alkyl-NR²¹R²². In another embodiment is a compound of Formula (II) wherein R¹ is H, and R² is - (C₁-C₆)alkyl-NR²¹R²². In another embodiment is a compound of Formula (II) wherein R¹ is -(C₁-C₆)alkyl-NR²¹R²², and R² is H. In another embodiment is a compound of Formula (II) wherein R¹ is H, and R² is - CH₂CH₂NH₂. In another embodiment is a compound of Formula (II) wherein R¹ is -CH₂CH₂NH₂, and R² is H. In another embodiment is a compound of Formula (II) wherein R¹ and R² are each -CH₂CH₂NH₂. In another embodiment is a compound of Formula (II) wherein R¹ and R² are each independently H, -(C₁-C₆)alkyl-NR²¹R²², or -CH₂CH(OH)CH₂NH₂. In another embodiment is a compound of Formula (II) wherein R¹ and R² are each independently -CH₂CH(OH)CH₂NH₂. In another embodiment is a compound of Formula (II) wherein R¹ is H, and R² is -CH₂CH(OH)CH₂NH₂. In another embodiment is a compound of Formula (II) wherein R¹ is -CH₂CH(OH)CH₂NH₂, and R² is H. In a further embodiment is a compound of Formula (II) wherein R¹ and R² and the atoms to which they are attached form an optionally substituted heterocycloalkyl ring.

In another embodiment is a compound of Formula (II) wherein X is optionally substituted aryl. In a further embodiment is a compound of Formula (II) wherein X is optionally substituted phenyl. In another embodiment is a compound of Formula (II) wherein X is optionally substituted heteroaryl. In a further embodiment is a compound of Formula (II) wherein X is disubstituted heteroaryl. In a further embodiment is a compound of Formula (II) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (II) wherein X is heteroaryl disubstituted with substituents each independently selected from -(C₁-C₆)alkyl. In a further embodiment is a compound of Formula (II) wherein X is heteroaryl disubstituted with methyl. In a further embodiment is a compound of Formula (II) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (II) wherein X is pyridinyl disubstituted with substituents each independently selected from -(C₁-C₆)alkyl. In a further embodiment is a compound of Formula (II) wherein X is pyridinyl disubstituted with methyl. In a further embodiment is a compound of Formula (II) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (II) wherein X is pyrimidinyl disubstituted with substituents each independently selected from -(C₁-C₆)alkyl. In a further embodiment is a compound of Formula (II) wherein X is pyrimidinyl disubstituted with methyl. In another embodiment is a compound of Formula (II) wherein X is optionally substituted -(C₁-C₆)alkyl-.

In another embodiment is a compound of Formula (II) wherein Y is optionally substituted aryl. In a further embodiment is a compound of Formula (II) wherein Y is optionally substituted phenyl. In another embodiment is a compound of Formula (II) wherein Y is optionally substituted heteroaryl. In another embodiment is a compound of Formula (II) wherein Y is optionally substituted -(C₁-C₆)alkyl-. In another embodiment is a compound of Formula (II) wherein Y is optionally substituted (C₃-C₇)cycloalkyl-. In another embodiment is a compound of Formula (II) wherein Y is optionally substituted heterocycloalkyl. In another embodiment is a compound of Formula (II) wherein Y is -O-. In another embodiment is a compound of Formula (II) wherein Y is -(C₂-C₆)alkynyl. In another embodiment is a compound of Formula (II) wherein Y is -O-(C₁-C₆)alkyl-. In another embodiment is a compound of Formula (II) wherein Y is a bond.

In another embodiment is a compound of Formula (II) wherein Z is -(C₁-C₁₂)alkyl. In a further embodiment is a compound of Formula (II) wherein Z is n-butyl, isobutyl, or tert-butyl. In another embodiment is a compound of Formula (II) wherein Z is -O-(C₁-C₁₂)alkyl. In another embodiment is a compound of Formula (II) wherein Z is -O-(C₃-C₇)cycloalkyl. In another embodiment is a compound of Formula (II) wherein Z is -(C₂-C₁₂)alkenyl. In another embodiment is a compound of Formula (II) wherein Z is optionally substituted aryl. In a further embodiment is a compound of Formula (II) wherein Z is optionally substituted phenyl. In a further embodiment is a compound of Formula (II) wherein Z is phenyl monsubstituted or disubstituted with a substituent independently selected from -(C₁-C₈)alkyl. In a further embodiment is a compound of Formula (II) wherein Z is phenyl monosubstituted with n-butyl, isobutyl, or tert-butyl. In a further embodiment is a compound of Formula (II) wherein Z is phenyl monosubstituted with n-butyl. In a further embodiment is a compound of Formula (II) wherein Z is phenyl monosubstituted with isobutyl. In a further embodiment is a compound of Formula (II) wherein Z is phenyl monosubstituted with tert-butyl. In another embodiment is a compound of Formula (II) wherein Z is optionally substituted heteroaryl. In another embodiment is a compound of Formula (II) wherein Z is optionally substituted -(C₃-C₇)cycloalkyl. In another embodiment is a compound of Formula (II) wherein Z is optionally substituted heterocycloalkyl. In another embodiment is a compound of Formula (II) wherein Z is halogen.

In another embodiment described herein is a compound of Formula (II) having the structure of Formula (IIa): 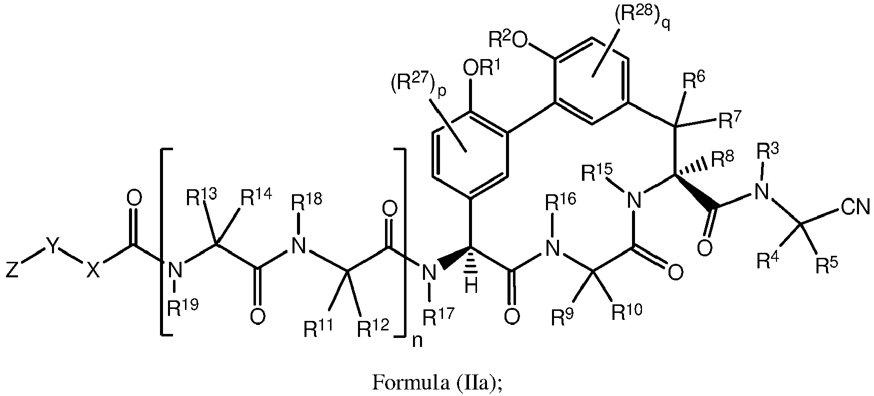 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring;
R³ is H or -(C₁-C₆)alkyl;
R⁴ is H, -(C₁-C₆)alkyl, -(C₁-C₆)alkyl-OH, -(C₃-C₆)cycloalkyl, or -C(O)NH₂;
or R³ and R⁴ are combined to form a heterocycloalkyl ring;
R⁵ is H or -(C₁-C₆)alkyl;
or R⁴ and R⁵ and the carbon atom to which they are attached form a cyclopropyl ring;
R⁶, R⁷, and R⁸ are each independently H or -(C₁-C₆)alkyl;
R⁹ is H, -(C₁-C₆)alkyl, -(C₁-C₆)haloalkyl, or -(C₃-C₆)cycloalkyl;
R¹⁰ is H or -(C₁-C₆)alkyl;
R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
R¹³ and R¹⁴ are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-CN, -(C₁-C₆)alkyl-C(O)NR²⁵R²⁶, -(C₁-C₆)alkyl-N(H)C(NH)NH₂, -(C₁-C₆)alkyl-heterocycloalkyl, or -(C₁-C₆)alkyl-heteroaryl;
or R¹³ and R¹⁹ are combined to form an optionally substituted heterocycloalkyl ring; and R¹⁴ is H;
R¹⁵, R¹⁶, R¹⁷, R¹⁸, and R¹⁹ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, -C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂alkyl-OR²³, -(C₁-C₁₂)alkyl-CN, -S-(C₁-C₁₂)alkyl, - N(R²⁴)(C₁C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring;
each R²⁷ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R¹ and R²⁷ and the atoms to which they are attached form an optionally substituted 5- or 6-membered heterocycloalkyl ring;
each R²⁸ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R² and R²⁸ and the atoms to which they are attached form an optionally substituted 5- or 6-membered heterocycloalkyl ring;
n is 0 or 1;
p is 0, 1, or 2; and
q is 0, 1, or 2;

or a pharmaceutically acceptable salt, solvate, or prodrug thereof.In one embodiment is a compound of Formula (IIa) wherein n is 0. In another embodiment is a compound of Formula (IIa) wherein n is 1.

In another embodiment is a compound of Formula (IIa) wherein R⁶, R⁷, and R⁸ are H.

In another embodiment is a compound of Formula (IIa) wherein R¹⁵ and R¹⁶ are H.

In one embodiment is a compound of Formula (IIa) wherein R¹⁷ is -(C₁-C₆)alkyl. In another embodiment is a compound of Formula (IIa) wherein R¹⁷ is -CH₃. In another embodiment is a compound of Formula (IIa) wherein R¹⁷ is -CH₂CH₃. In another embodiment is a compound of Formula (IIa) wherein R¹⁷ is -(C₃-C₆)cycloalkyl. In another embodiment is a compound of Formula (IIa) wherein R¹⁷ is cyclopropyl. In another embodiment is a compound of Formula (IIa) wherein R¹⁷ is -(C₁-C₆)alkyl-C(O)OR²³. In another embodiment is a compound of Formula (IIa) wherein R¹⁷ is -CH₂CH₂OH. In another embodiment is a compound of Formula (IIa) wherein R¹⁷ is -(C₁-C₆)alkyl-NR²¹R²². In another embodiment is a compound of Formula (IIa) wherein R¹⁷ is -CH₂CH₂NH₂. In another embodiment is a compound of Formula (IIa) wherein R¹⁷ is H.

In another embodiment is a compound of Formula (IIa) wherein R¹⁸ is H.

In another embodiment is a compound of Formula (IIa) wherein R¹⁹ is H.

In another embodiment is a compound of Formula (IIa) wherein R³ is H.

In another embodiment is a compound of Formula (IIa) wherein R⁵ is H.

In another embodiment is a compound of Formula (IIa) wherein R⁴ is H. In another embodiment is a compound of Formula (IIa) wherein R⁴ is -(C₁-C₆)alkyl. In another embodiment is a compound of Formula (IIa) wherein R⁴ is -CH₃. In another embodiment is a compound of Formula (IIa) wherein R⁴ is - CH₂CH₃. In another embodiment is a compound of Formula (IIa) wherein R⁴ is -(C₁-C₆)alkyl-OH. In another embodiment is a compound of Formula (IIa) wherein R⁴ is -CH₂OH. In another embodiment is a compound of Formula (IIa) wherein R⁴ is -(C₃-C₆)cycloalkyl. In another embodiment is a compound of Formula (IIa) wherein R⁴ is cyclopropyl. In another embodiment is a compound of Formula (IIa) wherein R⁴ is -C(O)NH₂.

In another embodiment is a compound of Formula (IIa) wherein R³, R⁴, and R⁵ are H.

In another embodiment is a compound of Formula (IIa) wherein R⁴ and R⁵ and the carbon atom to which they are attached form a cyclopropyl ring.

In another embodiment is a compound of Formula (IIa) wherein R¹⁰ is H.

In another embodiment is a compound of Formula (IIa) wherein R¹⁰ is H and R⁹ is -(C₁-C₆)alkyl. In another embodiment is a compound of Formula (IIa) wherein R¹⁰ is H and R⁹ is -CH₃. In another embodiment is a compound of Formula (IIa) wherein R¹⁰ is H and R⁹ is -CH₂CH₃. In another embodiment is a compound of Formula (IIa) wherein R¹⁰ is H and R⁹ is -(C₁-C₆)haloalkyl. In another embodiment is a compound of Formula (IIa) wherein R¹⁰ is H and R⁹ is -CH₂F. In another embodiment is a compound of Formula (IIa) wherein R¹⁰ is H and R⁹ is -CHF₂. In another embodiment is a compound of Formula (IIa) wherein R¹⁰ is H and R⁹ is -(C₃-C₆)cycloalkyl. In another embodiment is a compound of Formula (IIa) wherein R¹⁰ is H and R⁹ is cyclopropyl. In another embodiment is a compound of Formula (IIa) wherein R¹⁰ is H and R⁹ is H.

In another embodiment is a compound of Formula (IIa) wherein R¹² is H.

In another embodiment is a compound of Formula (IIa) wherein R¹² is H and R¹¹ is -(C₁-C₆)alkyl. In another embodiment is a compound of Formula (IIa) wherein R¹² is H and R¹¹ is -CH₃. In another embodiment is a compound of Formula (IIa) wherein R¹² is H and R¹¹ is -(C₁-C₆)alkyl-OR²³. In another embodiment is a compound of Formula (IIa) wherein R¹¹ is -CH₂OH. In another embodiment is a compound of Formula (IIa) wherein R¹² is H and R¹¹ is -CH₂CH₂OH. In another embodiment is a compound of Formula (IIa) wherein R¹² is H and R¹¹ is -(C₁-C₆)alkyl. In another embodiment is a compound of Formula (IIa) wherein R¹² is H and R¹¹ is -(C₁-C₆)alkyl-NR²¹R²². In another embodiment is a compound of Formula (IIa) wherein R¹² is H and R¹¹ is -(C₁-C₆)alkyl-NH₂. In another embodiment is a compound of Formula (IIa) wherein R¹² is H and R¹¹ is -CH₂NH₂. In another embodiment is a compound of Formula (IIa) wherein R¹² is H and R¹¹ is -CH₂CH₂NH₂. In another embodiment is a compound of Formula (IIa) wherein R¹² is H and R¹¹ is -CH₂CH₂CH₂NH₂. In another embodiment is a compound of Formula (IIa) wherein R¹² is H and R¹¹ is -CH₂CH₂CH₂CH₂NH₂. In another embodiment is a compound of Formula (IIa) wherein R¹² is H and R¹¹ is -(C₁-C₆)alkyl-CN. In another embodiment is a compound of Formula (IIa) wherein R¹² is H and R¹¹ is -CH₂CN. In another embodiment is a compound of Formula (IIa) wherein R¹² is H and R¹¹ is -(C₁-C₆)alkyl-C(O)NR²⁵R²⁶. In another embodiment is a compound of Formula (IIa) wherein R¹² is H and R¹¹ is -CH₂C(O)NH₂. In another embodiment is a compound of Formula (IIa) wherein R¹² is H and R¹¹ is -CH₂CH₂C(O)NH₂. In another embodiment is a compound of Formula (IIa) wherein R¹² is H and R¹¹ is -(C₁-C₆)alkyl-heteroaryl. In another embodiment is a compound of Formula (IIa) wherein R¹² is H and R¹¹ is -(C₁-C₆)alkyl-N(H)S(O)₂NR²⁵R²⁶. In another embodiment is a compound of Formula (IIa) wherein R¹² is H and R¹¹ is -CH₂N(H)S(O)₂NH₂. In another embodiment is a compound of Formula (IIa) wherein R¹² is H and R¹¹ is H.

In another embodiment is a compound of Formula (IIa) wherein R¹¹ and R¹⁸ are combined to form an optionally substituted heterocycloalkyl ring and R¹² is H.

In another embodiment is a compound of Formula (IIa) wherein R¹⁴ is H.

In another embodiment is a compound of Formula (IIa) wherein R¹⁴ is H and R¹³ is -(C₁-C₆)alkyl. In another embodiment is a compound of Formula (IIa) wherein R¹⁴ is H and R¹³ is -CH₃. In another embodiment is a compound of Formula (IIa) wherein R¹⁴ is H and R¹³ is -(C₁-C₆)alkyl-OR²³. In another embodiment is a compound of Formula (IIa) wherein R¹⁴ is H and R¹³ is -CH₂OH. In another embodiment is a compound of Formula (IIa) wherein R¹⁴ is H and R¹³ is -CH₂CH₂OH. In another embodiment is a compound of Formula (IIa) wherein R¹⁴ is H and R¹³ is -(C₁-C₆)alkyl. In another embodiment is a compound of Formula (IIa) wherein R¹⁴ is H and R¹³ is -(C₁-C₆)alkyl-NR²¹R²². In another embodiment is a compound of Formula (IIa) wherein R¹⁴ is H and R¹³ is -(C₁-C₆)alkyl-NH₂. In another embodiment is a compound of Formula (IIa) wherein R¹⁴ is H and R¹³ is -CH₂NH₂. In another embodiment is a compound of Formula (IIa) wherein R¹⁴ is H and R¹³ is -CH₂CH₂NH₂. In another embodiment is a compound of Formula (IIa) wherein R¹⁴ is H and R¹³ is -CH₂CH₂CH₂NH₂. In another embodiment is a compound of Formula (IIa) wherein R¹⁴ is H and R¹³ is -CH₂CH₂CH₂CH₂NH₂. In another embodiment is a compound of Formula (IIa) wherein R¹⁴ is H and R¹³ is -(C₁-C₆)alkyl-CN. In another embodiment is a compound of Formula (IIa) wherein R¹⁴ is H and R¹³ is -CH₂CN. In another embodiment is a compound of Formula (IIa) wherein R¹⁴ is H and R¹³ is -(C₁-C₆)alkyl-C(O)NR²⁵R²⁶. In another embodiment is a compound of Formula (IIa) wherein R¹⁴ is H and R¹³ is -CH₂C(O)NH₂. In another embodiment is a compound of Formula (IIa) wherein R¹⁴ is H and R¹³ is -CH₂CH₂C(O)NH₂. In another embodiment is a compound of Formula (IIa) wherein R¹⁴ is H and R¹³ is -(C₁-C₆)alkyl-heteroaryl. In another embodiment is a compound of Formula (IIa) wherein R¹⁴ is H and R¹³ is H.

In another embodiment is a compound of Formula (IIa) wherein R¹³ and R¹⁹ are combined to form an optionally substituted heterocycloalkyl ring and R¹⁴ is H.

In another embodiment is a compound of Formula (IIa) wherein p is 1 and R²⁷ is halogen. In another embodiment is a compound of Formula (IIa) wherein p is 1 and R²⁷ is optionally substituted -(C₁-C₆)alkyl. In another embodiment is a compound of Formula (IIa) wherein q is 0, p is 1 and R²⁷ is halogen. In another embodiment is a compound of Formula (IIa) wherein q is 0, p is 1 and R²⁷ is optionally substituted -(C₁-C₆)alkyl. In another embodiment is a compound of Formula (IIa) wherein q is 1 and R²⁸ is halogen. In another embodiment is a compound of Formula (IIa) wherein q is 1 and R²⁸ is optionally substituted -(C₁-C₆)alkyl. In another embodiment is a compound of Formula (IIa) wherein p is 0, q is 1 and R²⁸ is halogen. In another embodiment is a compound of Formula (IIa) wherein p is 0, q is 1 and R²⁸ is optionally substituted -(C₁-C₆)alkyl.

In another embodiment is a compound of Formula (IIa) wherein p is 0, and q is 0.

In another embodiment is a compound of Formula (IIa) wherein R¹ and R² are each independently H or -(C₁-C₆)alkyl-NR²¹R²². In another embodiment is a compound of Formula (IIa) wherein R¹ and R² are each H. In another embodiment is a compound of Formula (IIa) wherein R¹ and R² are each independently -(C₁-C₆)alkyl-NR²¹R²². In another embodiment is a compound of Formula (IIa) wherein R¹ is H, and R² is -(C₁-C₆)alkyl-NR²¹R²². In another embodiment is a compound of Formula (IIa) wherein R¹ is -(C₁-C₆)alkyl-NR²¹R²², and R² is H. In another embodiment is a compound of Formula (IIa) wherein R¹ is H, and R² is -CH₂CH₂NH₂. In another embodiment is a compound of Formula (IIa) wherein R¹ is -CH₂CH₂NH₂, and R² is H. In another embodiment is a compound of Formula (IIa) wherein R¹ and R² are each -CH₂CH₂NH₂. In another embodiment is a compound of Formula (IIa) wherein R¹ and R² are each independently H, -(C₁-C₆)alkyl-NR²¹R²², or -CH₂CH(OH)CH₂NH₂. In another embodiment is a compound of Formula (IIa) wherein R¹ and R² are each independently -CH₂CH(OH)CH₂NH₂. In another embodiment is a compound of Formula (IIa) wherein R¹ is H, and R² is -CH₂CH(OH)CH₂NH₂. In another embodiment is a compound of Formula (IIa) wherein R¹ is -CH₂CH(OH)CH₂NH₂, and R² is H. In a further embodiment is a compound of Formula (IIa) wherein R¹ and R² and the atoms to which they are attached form an optionally substituted heterocycloalkyl ring.

In another embodiment is a compound of Formula (IIa) wherein X is optionally substituted aryl. In a further embodiment is a compound of Formula (IIa) wherein X is optionally substituted phenyl. In another embodiment is a compound of Formula (IIa) wherein X is optionally substituted heteroaryl. In a further embodiment is a compound of Formula (IIa) wherein X is disubstituted heteroaryl. In a further embodiment is a compound of Formula (IIa) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (IIa) wherein X is heteroaryl disubstituted with substituents each independently selected from -(C₁-C₆)alkyl. In a further embodiment is a compound of Formula (IIa) wherein X is heteroaryl disubstituted with methyl. In a further embodiment is a compound of Formula (IIa) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IIa) wherein X is pyridinyl disubstituted with substituents each independently selected from -(C₁-C₆)alkyl. In a further embodiment is a compound of Formula (IIa) wherein X is pyridinyl disubstituted with methyl. In a further embodiment is a compound of Formula (IIa) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IIa) wherein X is pyrimidinyl disubstituted with substituents each independently selected from -(C₁-C₆)alkyl. In a further embodiment is a compound of Formula (IIa) wherein X is pyrimidinyl disubstituted with methyl. In another embodiment is a compound of Formula (IIa) wherein X is optionally substituted -(C₁-C₆)alkyl-.

In another embodiment is a compound of Formula (IIa) wherein Y is optionally substituted aryl. In a further embodiment is a compound of Formula (IIa) wherein Y is optionally substituted phenyl. In another embodiment is a compound of Formula (IIa) wherein Y is optionally substituted heteroaryl. In another embodiment is a compound of Formula (IIa) wherein Y is optionally substituted -(C₁-C₆)alkyl-. In another embodiment is a compound of Formula (IIa) wherein Y is optionally substituted (C₃-C₇)cycloalkyl-. In another embodiment is a compound of Formula (IIa) wherein Y is optionally substituted heterocycloalkyl. In another embodiment is a compound of Formula (IIa) wherein Y is -O-. In another embodiment is a compound of Formula (IIa) wherein Y is -(C₂-C₆)alkynyl. In another embodiment is a compound of Formula (IIa) wherein Y is -O-(C₁-C₆)alkyl-. In another embodiment is a compound of Formula (IIa) wherein Y is a bond.

In another embodiment is a compound of Formula (IIa) wherein Z is -(C₁-C₁₂)alkyl. In a further embodiment is a compound of Formula (IIa) wherein Z is n-butyl, isobutyl, or tert-butyl. In another embodiment is a compound of Formula (IIa) wherein Z is -O-(C₁-C₁₂)alkyl. In another embodiment is a compound of Formula (IIa) wherein Z is -O-(C₃-C₇)cycloalkyl. In another embodiment is a compound of Formula (IIa) wherein Z is -(C₂-C₁₂)alkenyl. In another embodiment is a compound of Formula (IIa) wherein Z is optionally substituted aryl. In a further embodiment is a compound of Formula (IIa) wherein Z is optionally substituted phenyl. In a further embodiment is a compound of Formula (IIa) wherein Z is phenyl monsubstituted or disubstituted with a substituent independently selected from -(C₁-C₈)alkyl. In a further embodiment is a compound of Formula (IIa) wherein Z is phenyl monosubstituted with n-butyl, isobutyl, or tert-butyl. In a further embodiment is a compound of Formula (IIa) wherein Z is phenyl monosubstituted with n-butyl. In a further embodiment is a compound of Formula (IIa) wherein Z is phenyl monosubstituted with isobutyl. In a further embodiment is a compound of Formula (IIa) wherein Z is phenyl monosubstituted with tert-butyl. In another embodiment is a compound of Formula (IIa) wherein Z is optionally substituted heteroaryl. In another embodiment is a compound of Formula (IIa) wherein Z is optionally substituted -(C₃-C₇)cycloalkyl. In another embodiment is a compound of Formula (IIa) wherein Z is optionally substituted heterocycloalkyl. In another embodiment is a compound of Formula (IIa) wherein Z is halogen.

In another embodiment described herein is a compound of Formula (II) having the structure of Formula (IIb): 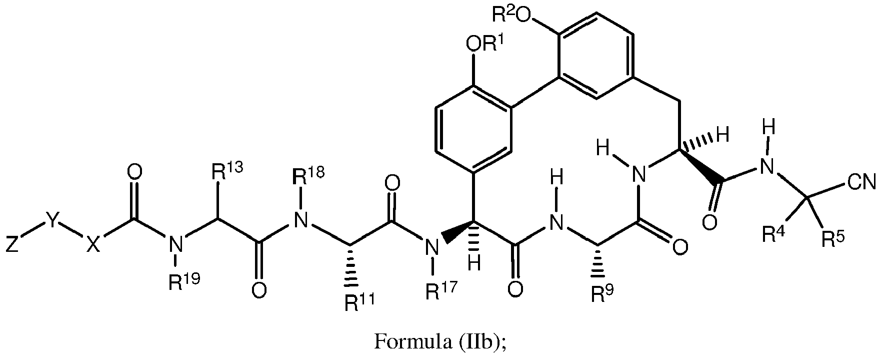 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring; R⁴ is H, -(C₁-C₆)alkyl, -(C₁-C₆)alkyl-OH, -(C₃-C₆)cycloalkyl, or -C(O)NH₂;
R⁵ is H or -(C₁-C₆)alkyl;
or R⁴ and R⁵ and the carbon atom to which they are attached form a cyclopropyl ring;
R⁹ is H, -(C₁-C₆)alkyl, -(C₁-C₆)haloalkyl, or -(C₃-C₆)cycloalkyl;
R¹¹ is H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
R¹³ is H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-CN, -(C₁-C₆)alkyl-C(O)NR²⁵R²⁶, -(C₁-C₆)alkyl-N(H)C(NH)NH₂, -(C₁-C₆)alkyl-heterocycloalkyl, or -(C₁-C₆)alkyl-heteroaryl;
or R¹³ and R¹⁹ are combined to form an optionally substituted heterocycloalkyl ring; and R¹⁴ is H;
R¹⁷, R¹⁸, and R¹⁹ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, -C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl,optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂alkyl-OR²³, -(C₁-C₁₂)alkyl-CN, -S-(C₁-C₁₂)alkyl, - N(R²⁴)(C₁C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl; and or R²⁵ and R²⁶ and the nitrogen atom to which they are attached form a heterocycloalkyl ring;

or a pharmaceutically acceptable salt, solvate, or prodrug thereof.In one embodiment is a compound of Formula (IIb) wherein R¹⁷ is -(C₁-C₆)alkyl. In another embodiment is a compound of Formula (IIb) wherein R¹⁷ is -CH₃. In another embodiment is a compound of Formula (IIb) wherein R¹⁷ is -CH₂CH₃. In another embodiment is a compound of Formula (IIb) wherein R¹⁷ is -(C₃-C₆)cycloalkyl. In another embodiment is a compound of Formula (IIb) wherein R¹⁷ is cyclopropyl. In another embodiment is a compound of Formula (IIb) wherein R¹⁷ is -(C₁-C₆)alkyl-C(O)OR²³. In another embodiment is a compound of Formula (IIb) wherein R¹⁷ is -CH₂CH₂OH. In another embodiment is a compound of Formula (IIb) wherein R¹⁷ is -(C₁-C₆)alkyl-NR²¹R²². In another embodiment is a compound of Formula (IIb) wherein R¹⁷ is -CH₂CH₂NH₂. In another embodiment is a compound of Formula (IIb) wherein R¹⁷ is H.

In another embodiment is a compound of Formula (IIb) wherein R¹⁸ is H.

In another embodiment is a compound of Formula (IIb) wherein R¹⁹ is H.

In another embodiment is a compound of Formula (IIb) wherein R⁵ is H.

In another embodiment is a compound of Formula (IIb) wherein R⁴ is H. In another embodiment is a compound of Formula (IIb) wherein R⁴ is -(C₁-C₆)alkyl. In another embodiment is a compound of Formula (IIb) wherein R⁴ is -CH₃. In another embodiment is a compound of Formula (IIb) wherein R⁴ is - CH₂CH₃. In another embodiment is a compound of Formula (IIb) wherein R⁴ is -(C₁-C₆)alkyl-OH. In another embodiment is a compound of Formula (IIb) wherein R⁴ is -CH₂OH. In another embodiment is a compound of Formula (IIb) wherein R⁴ is -(C₃-C₆)cycloalkyl. In another embodiment is a compound of Formula (IIb) wherein R⁴ is cyclopropyl. In another embodiment is a compound of Formula (IIb) wherein R⁴ is -C(O)NH₂.

In another embodiment is a compound of Formula (IIb) wherein R⁴ and R⁵ are H.

In another embodiment is a compound of Formula (IIb) wherein R⁴ and R⁵ and the carbon atom to which they are attached form a cyclopropyl ring.

In another embodiment is a compound of Formula (IIb) wherein R⁹ is -(C₁-C₆)alkyl. In another embodiment is a compound of Formula (IIb) wherein R⁹ is -CH₃. In another embodiment is a compound of Formula (IIb) wherein R⁹ is -CH₂CH₃. In another embodiment is a compound of Formula (IIb) wherein R⁹ is -(C₁-C₆)haloalkyl. In another embodiment is a compound of Formula (IIb) wherein R⁹ is -CH₂F. In another embodiment is a compound of Formula (IIb) wherein R⁹ is -CHF₂. In another embodiment is a compound of Formula (IIb) wherein R⁹ is -(C₃-C₆)cycloalkyl. In another embodiment is a compound of Formula (IIb) wherein R⁹ is cyclopropyl. In another embodiment is a compound of Formula (IIb) wherein R⁹ is H.

In another embodiment is a compound of Formula (IIb) wherein R¹¹ is -(C₁-C₆)alkyl. In another embodiment is a compound of Formula (IIb) wherein R¹¹ is -CH₃. In another embodiment is a compound of Formula (IIb) wherein R¹¹ is -(C₁-C₆)alkyl-OR²³. In another embodiment is a compound of Formula (IIb) wherein R¹¹ is -CH₂OH. In another embodiment is a compound of Formula (IIb) wherein R¹¹ is - CH₂CH₂OH. In another embodiment is a compound of Formula (IIb) wherein R¹¹ is -(C₁-C₆)alkyl. In another embodiment is a compound of Formula (IIb) wherein R¹¹ is -(C₁-C₆)alkyl-NR²¹R²². In another embodiment is a compound of Formula (IIb) wherein R¹¹ is -(C₁-C₆)alkyl-NH₂. In another embodiment is a compound of Formula (IIb) wherein R¹¹ is -CH₂NH₂. In another embodiment is a compound of Formula (IIb) wherein R¹¹ is -CH₂CH₂NH₂. In another embodiment is a compound of Formula (IIb) wherein R¹¹ is - CH₂CH₂CH₂NH₂. In another embodiment is a compound of Formula (IIb) wherein R¹¹ is - CH₂CH₂CH₂CH₂NH₂. In another embodiment is a compound of Formula (IIb) wherein R¹¹ is -(C₁-C₆)alkyl-CN. In another embodiment is a compound of Formula (IIb) wherein R¹¹ is -CH₂CN. In another embodiment is a compound of Formula (IIb) wherein R¹¹ is -(C₁-C₆)alkyl-C(O)NR²⁵R²⁶. In another embodiment is a compound of Formula (IIb) wherein R¹¹ is -CH₂C(O)NH₂. In another embodiment is a compound of Formula (IIb) wherein R¹¹ is -CH₂CH₂C(O)NH₂. In another embodiment is a compound of Formula (IIb) wherein R¹¹ is -(C₁-C₆)alkyl-heteroaryl. In another embodiment is a compound of Formula (IIb) wherein R¹¹ is -(C₁-C₆)alkyl-N(H)S(O)₂NR²⁵R²⁶. In another embodiment is a compound of Formula (IIb) wherein R¹¹ is -CH₂N(H)S(O)₂NH₂. In another embodiment is a compound of Formula (IIb) wherein R¹¹ is H.

In another embodiment is a compound of Formula (IIb) wherein R¹¹ and R¹⁸ are combined to form an optionally substituted heterocycloalkyl ring and R¹² is H.

In another embodiment is a compound of Formula (IIb) wherein R¹³ is -(C₁-C₆)alkyl. In another embodiment is a compound of Formula (IIb) wherein R¹³ is -CH₃. In another embodiment is a compound of Formula (IIb) wherein R¹³ is -(C₁-C₆)alkyl-OR²³. In another embodiment is a compound of Formula (IIb) wherein R¹³ is -CH₂OH. In another embodiment is a compound of Formula (IIb) wherein R¹³ is - CH₂CH₂OH. In another embodiment is a compound of Formula (IIb) wherein R¹³ is -(C₁-C₆)alkyl. In another embodiment is a compound of Formula (IIb) wherein R¹³ is -(C₁-C₆)alkyl-NR²¹R²². In another embodiment is a compound of Formula (IIb) wherein R¹³ is -(C₁-C₆)alkyl-NH₂. In another embodiment is a compound of Formula (IIb) wherein R¹³ is -CH₂NH₂. In another embodiment is a compound of Formula (IIb) wherein R¹³ is -CH₂CH₂NH₂. In another embodiment is a compound of Formula (IIb) wherein R¹³ is - CH₂CH₂CH₂NH₂. In another embodiment is a compound of Formula (IIb) wherein R¹³ is - CH₂CH₂CH₂CH₂NH₂. In another embodiment is a compound of Formula (IIb) wherein R¹³ is -(C₁-C₆)alkyl-CN. In another embodiment is a compound of Formula (IIb) wherein R¹³ is -CH₂CN. In another embodiment is a compound of Formula (IIb) wherein R¹³ is -(C₁-C₆)alkyl-C(O)NR²⁵R²⁶. In another embodiment is a compound of Formula (IIb) wherein R¹³ is -CH₂C(O)NH₂. In another embodiment is a compound of Formula (IIb) wherein R¹³ is -CH₂CH₂C(O)NH₂. In another embodiment is a compound of Formula (IIb) wherein R¹³ is -(C₁-C₆)alkyl-heteroaryl. In another embodiment is a compound of Formula (IIb) wherein R¹³ is H.

In another embodiment is a compound of Formula (IIb) wherein R¹³ and R¹⁹ are combined to form an optionally substituted heterocycloalkyl ring and R¹⁴ is H.

In another embodiment is a compound of Formula (IIb) wherein R¹ and R² are each independently H, or -(C₁-C₆)alkyl-NR²¹R²². In another embodiment is a compound of Formula (IIb) wherein R¹ and R² are each H. In another embodiment is a compound of Formula (IIb) wherein R¹ and R² are each independently -(C₁-C₆)alkyl-NR²¹R²². In another embodiment is a compound of Formula (IIb) wherein R¹ is H, and R² is -(C₁-C₆)alkyl-NR²¹R²². In another embodiment is a compound of Formula (IIb) wherein R¹ is -(C₁-C₆)alkyl-NR²¹R²², and R² is H. In another embodiment is a compound of Formula (IIb) wherein R¹ is H, and R² is -CH₂CH₂NH₂. In another embodiment is a compound of Formula (IIb) wherein R¹ is -CH₂CH₂NH₂, and R² is H. In another embodiment is a compound of Formula (IIb) wherein R¹ and R² are each -CH₂CH₂NH₂. In another embodiment is a compound of Formula (IIb) wherein R¹ and R² are each independently H, -(C₁-C₆)alkyl-NR²¹R²², or -CH₂CH(OH)CH₂NH₂. In another embodiment is a compound of Formula (IIb) wherein R¹ and R² are each independently -CH₂CH(OH)CH₂NH₂. In another embodiment is a compound of Formula (IIb) wherein R¹ is H, and R² is -CH₂CH(OH)CH₂NH₂. In another embodiment is a compound of Formula (IIb) wherein R¹ is -CH₂CH(OH)CH₂NH₂, and R² is H. In a further embodiment is a compound of Formula (IIb) wherein R¹ and R² and the atoms to which they are attached form an optionally substituted heterocycloalkyl ring.

In another embodiment is a compound of Formula (IIb) wherein X is optionally substituted aryl. In a further embodiment is a compound of Formula (IIb) wherein X is optionally substituted phenyl. In another embodiment is a compound of Formula (IIb) wherein X is optionally substituted heteroaryl. In a further embodiment is a compound of Formula (IIb) wherein X is disubstituted heteroaryl. In a further embodiment is a compound of Formula (IIb) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (IIb) wherein X is heteroaryl disubstituted with substituents each independently selected from -(C₁-C₆)alkyl. In a further embodiment is a compound of Formula (IIb) wherein X is heteroaryl disubstituted with methyl. In a further embodiment is a compound of Formula (IIb) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IIb) wherein X is pyridinyl disubstituted with substituents each independently selected from -(C₁-C₆)alkyl. In a further embodiment is a compound of Formula (IIb) wherein X is pyridinyl disubstituted with methyl. In a further embodiment is a compound of Formula (IIb) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IIb) wherein X is pyrimidinyl disubstituted with substituents each independently selected from -(C₁-C₆)alkyl. In a further embodiment is a compound of Formula (IIb) wherein X is pyrimidinyl disubstituted with methyl. In another embodiment is a compound of Formula (IIb) wherein X is optionally substituted -(C₁-C₆)alkyl-.

In another embodiment is a compound of Formula (IIb) wherein Y is optionally substituted aryl. In a further embodiment is a compound of Formula (IIb) wherein Y is optionally substituted phenyl. In another embodiment is a compound of Formula (IIb) wherein Y is optionally substituted heteroaryl. In another embodiment is a compound of Formula (IIb) wherein Y is optionally substituted -(C₁-C₆)alkyl-. In another embodiment is a compound of Formula (IIb) wherein Y is optionally substituted (C₃-C₇)cycloalkyl-. In another embodiment is a compound of Formula (IIb) wherein Y is optionally substituted heterocycloalkyl. In another embodiment is a compound of Formula (IIb) wherein Y is -O-. In another embodiment is a compound of Formula (IIb) wherein Y is -(C₂-C₆)alkynyl. In another embodiment is a compound of Formula (IIb) wherein Y is -O-(C₁-C₆)alkyl-. In another embodiment is a compound of Formula (IIb) wherein Y is a bond.

In another embodiment is a compound of Formula (IIb) wherein Z is -(C₁-C₁₂)alkyl. In a further embodiment is a compound of Formula (IIb) wherein Z is n-butyl, isobutyl, or tert-butyl. In another embodiment is a compound of Formula (IIb) wherein Z is -O-(C₁-C₁₂)alkyl. In another embodiment is a compound of Formula (IIb) wherein Z is -O-(C₃-C₇)cycloalkyl. In another embodiment is a compound of Formula (IIb) wherein Z is -(C₂-C₁₂)alkenyl. In another embodiment is a compound of Formula (IIb) wherein Z is optionally substituted aryl. In a further embodiment is a compound of Formula (IIb) wherein Z is optionally substituted phenyl. In a further embodiment is a compound of Formula (IIb) wherein Z is phenyl monsubstituted or disubstituted with a substituent independently selected from -(C₁-C₈)alkyl. In a further embodiment is a compound of Formula (IIb) wherein Z is phenyl monosubstituted with n-butyl, isobutyl, or tert-butyl. In a further embodiment is a compound of Formula (IIb) wherein Z is phenyl monosubstituted with n-butyl. In a further embodiment is a compound of Formula (IIb) wherein Z is phenyl monosubstituted with isobutyl. In a further embodiment is a compound of Formula (IIb) wherein Z is phenyl monosubstituted with tert-butyl. In another embodiment is a compound of Formula (IIb) wherein Z is optionally substituted heteroaryl. In another embodiment is a compound of Formula (IIb) wherein Z is optionally substituted -(C₃-C₇)cycloalkyl. In another embodiment is a compound of Formula (IIb) wherein Z is optionally substituted heterocycloalkyl. In another embodiment is a compound of Formula (IIb) wherein Z is halogen.

In another embodiment described herein is a compound of Formula (II) having the structure of Formula (IIc): 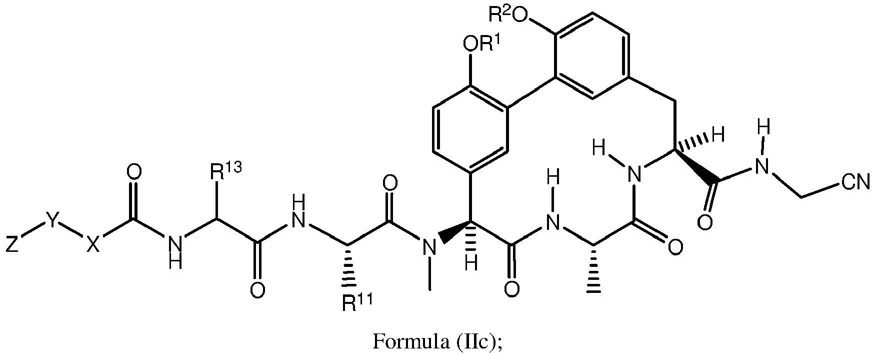 wherein:
R¹ and R² are each independently H or -CH₂CH₂NH₂;
R¹¹ is H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶,-(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryll;
R¹³ is H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-CN, -(C₁-C₆)alkyl-C(O)NR²⁵R²⁶, -(C₁-C₆)alkyl-N(H)C(NH)NH₂, -(C₁-C₆)alkyl-heterocycloalkyl, or -(C₁-C₆)alkyl-heteroaryl;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, -C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl; and or R²⁵ and R²⁶ and the nitrogen atom to which they are attached form a heterocycloalkyl ring;

or a pharmaceutically acceptable salt, solvate, or prodrug thereof.In another embodiment is a compound of Formula (IIc) wherein R¹¹ is -(C₁-C₆)alkyl. In another embodiment is a compound of Formula (IIc) wherein R¹¹ is -CH₃. In another embodiment is a compound of Formula (IIc) wherein R¹¹ is - (C₁-C₆)alkyl-OR²³. In another embodiment is a compound of Formula (IIc) wherein R¹¹ is -CH₂OH. In another embodiment is a compound of Formula (IIc) wherein R¹¹ is -CH₂CH₂OH. In another embodiment is a compound of Formula (IIc) wherein R¹¹ is -(C₁-C₆)alkyl. In another embodiment is a compound of Formula (IIc) wherein R¹¹ is -(C₁-C₆)alkyl-NR²¹R²². In another embodiment is a compound of Formula (IIc) wherein R¹¹ is -(C₁-C₆)alkyl-NH₂. In another embodiment is a compound of Formula (IIc) wherein R¹¹ is -CH₂NH₂. In another embodiment is a compound of Formula (IIc) wherein R¹¹ is -CH₂CH₂NH₂. In another embodiment is a compound of Formula (IIc) wherein R¹¹ is -CH₂CH₂CH₂NH₂. In another embodiment is a compound of Formula (IIc) wherein R¹¹ is -CH₂CH₂CH₂CH₂NH₂. In another embodiment is a compound of Formula (IIc) wherein R¹¹ is -(C₁-C₆)alkyl-CN. In another embodiment is a compound of Formula (IIc) wherein R¹¹ is -CH₂CN. In another embodiment is a compound of Formula (IIc) wherein R¹¹ is -(C₁-C₆)alkyl-C(O)NR²⁵R²⁶. In another embodiment is a compound of Formula (IIc) wherein R¹¹ is - CH₂C(O)NH₂. In another embodiment is a compound of Formula (IIc) wherein R¹¹ is -CH₂CH₂C(O)NH₂. In another embodiment is a compound of Formula (IIc) wherein R¹¹ is -(C₁-C₆)alkyl-heteroaryl. In another embodiment is a compound of Formula (IIc) wherein R¹¹ is -(C₁-C₆)alkyl-N(H)S(O)₂NR²⁵R²⁶. In another embodiment is a compound of Formula (IIc) wherein R¹¹ is -CH₂N(H)S(O)₂NH₂. In another embodiment is a compound of Formula (IIc) wherein R¹¹ is H.

In another embodiment is a compound of Formula (IIc) wherein R¹³ is -(C₁-C₆)alkyl. In another embodiment is a compound of Formula (IIc) wherein R¹³ is -CH₃. In another embodiment is a compound of Formula (IIc) wherein R¹³ is -(C₁-C₆)alkyl-OR²³. In another embodiment is a compound of Formula (IIc) wherein R¹³ is -CH₂OH. In another embodiment is a compound of Formula (IIc) wherein R¹³ is - CH₂CH₂OH. In another embodiment is a compound of Formula (IIc) wherein R¹³ is -(C₁-C₆)alkyl. In another embodiment is a compound of Formula (IIc) wherein R¹³ is -(C₁-C₆)alkyl-NR²¹R²². In another embodiment is a compound of Formula (IIc) wherein R¹³ is -(C₁-C₆)alkyl-NH₂. In another embodiment is a compound of Formula (IIc) wherein R¹³ is -CH₂NH₂. In another embodiment is a compound of Formula (IIc) wherein R¹³ is -CH₂CH₂NH₂. In another embodiment is a compound of Formula (IIc) wherein R¹³ is - CH₂CH₂CH₂NH₂. In another embodiment is a compound of Formula (IIc) wherein R¹³ is - CH₂CH₂CH₂CH₂NH₂. In another embodiment is a compound of Formula (IIc) wherein R¹³ is -(C₁-C₆)alkyl-CN. In another embodiment is a compound of Formula (IIc) wherein R¹³ is -CH₂CN. In another embodiment is a compound of Formula (IIc) wherein R¹³ is -(C₁-C₆)alkyl-C(O)NR²⁵R²⁶. In another embodiment is a compound of Formula (IIc) wherein R¹³ is -CH₂C(O)NH₂. In another embodiment is a compound of Formula (IIc) wherein R¹³ is -CH₂CH₂C(O)NH₂. In another embodiment is a compound of Formula (IIc) wherein R¹³ is -(C₁-C₆)alkyl-heteroaryl. In another embodiment is a compound of Formula (IIc) wherein R¹³ is H.

In another embodiment is a compound of Formula (IIc) wherein R¹ and R² are each H. In another embodiment is a compound of Formula (IIc) wherein R¹ is H, and R² is -CH₂CH₂NH₂. In another embodiment is a compound of Formula (IIc) wherein R¹ is -CH₂CH₂NH₂, and R² is H. In another embodiment is a compound of Formula (IIc) wherein R¹ and R² are each -CH₂CH₂NH₂. In a further embodiment is a compound of Formula (IIc) wherein R¹ and R² and the atoms to which they are attached form an optionally substituted heterocycloalkyl ring.

In another embodiment is a compound of Formula (IIc) wherein X is optionally substituted aryl. In a further embodiment is a compound of Formula (IIc) wherein X is optionally substituted phenyl. In another embodiment is a compound of Formula (IIc) wherein X is optionally substituted heteroaryl. In a further embodiment is a compound of Formula (IIc) wherein X is disubstituted heteroaryl. In a further embodiment is a compound of Formula (IIc) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (IIc) wherein X is heteroaryl disubstituted with substituents each independently selected from -(C₁-C₆)alkyl. In a further embodiment is a compound of Formula (IIc) wherein X is heteroaryl disubstituted with methyl. In a further embodiment is a compound of Formula (IIc) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IIc) wherein X is pyridinyl disubstituted with substituents each independently selected from -(C₁-C₆)alkyl. In a further embodiment is a compound of Formula (IIc) wherein X is pyridinyl disubstituted with methyl. In a further embodiment is a compound of Formula (IIc) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IIc) wherein X is pyrimidinyl disubstituted with substituents each independently selected from -(C₁-C₆)alkyl. In a further embodiment is a compound of Formula (IIc) wherein X is pyrimidinyl disubstituted with methyl. In another embodiment is a compound of Formula (IIc) wherein X is optionally substituted -(C₁-C₆)alkyl-.

In another embodiment is a compound of Formula (IIc) wherein Y is optionally substituted aryl. In a further embodiment is a compound of Formula (IIc) wherein Y is optionally substituted phenyl. In another embodiment is a compound of Formula (IIc) wherein Y is optionally substituted heteroaryl. In another embodiment is a compound of Formula (IIc) wherein Y is optionally substituted -(C₁-C₆)alkyl-. In another embodiment is a compound of Formula (IIc) wherein Y is optionally substituted (C₃-C₇)cycloalkyl-. In another embodiment is a compound of Formula (IIc) wherein Y is optionally substituted heterocycloalkyl. In another embodiment is a compound of Formula (IIc) wherein Y is -O-. In another embodiment is a compound of Formula (IIc) wherein Y is -(C₂-C₆)alkynyl. In another embodiment is a compound of Formula (IIc) wherein Y is -O-(C₁-C₆)alkyl-. In another embodiment is a compound of Formula (IIc) wherein Y is a bond.

In another embodiment is a compound of Formula (IIc) wherein Z is -(C₁-C₁₂)alkyl. In a further embodiment is a compound of Formula (IIc) wherein Z is n-butyl, isobutyl, or tert-butyl. In another embodiment is a compound of Formula (IIc) wherein Z is -O-(C₁-C₁₂)alkyl. In another embodiment is a compound of Formula (IIc) wherein Z is -O-(C₃-C₇)cycloalkyl. In another embodiment is a compound of Formula (IIc) wherein Z is -(C₂-C₁₂)alkenyl. In another embodiment is a compound of Formula (IIc) wherein Z is optionally substituted aryl. In a further embodiment is a compound of Formula (IIc) wherein Z is optionally substituted phenyl. In a further embodiment is a compound of Formula (IIc) wherein Z is phenyl monsubstituted or disubstituted with a substituent independently selected from -(C₁-C₈)alkyl. In a further embodiment is a compound of Formula (IIc) wherein Z is phenyl monosubstituted with n-butyl, isobutyl, or tert-butyl. In a further embodiment is a compound of Formula (IIc) wherein Z is phenyl monosubstituted with n-butyl. In a further embodiment is a compound of Formula (IIc) wherein Z is phenyl monosubstituted with isobutyl. In a further embodiment is a compound of Formula (IIc) wherein Z is phenyl monosubstituted with tert-butyl. In another embodiment is a compound of Formula (IIc) wherein Z is optionally substituted heteroaryl. In another embodiment is a compound of Formula (IIc) wherein Z is optionally substituted -(C₃-C₇)cycloalkyl. In another embodiment is a compound of Formula (IIc) wherein Z is optionally substituted heterocycloalkyl. In another embodiment is a compound of Formula (IIc) wherein Z is halogen.

In another embodiment described herein are compounds of Formula (II) having the structure of Formula (IId): 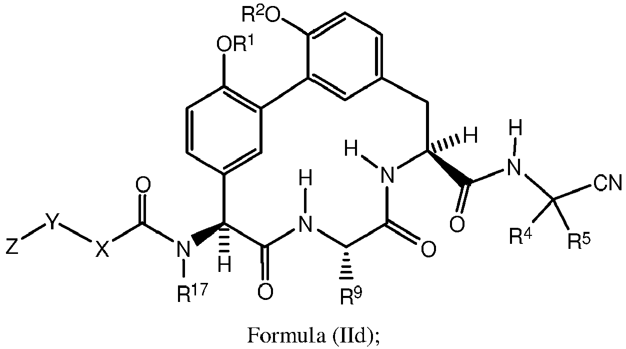 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring; R⁴ is H, -(C₁-C₆)alkyl, -(C₁-C₆)alkyl-OH, -(C₃-C₆)cycloalkyl, or -C(O)NH₂;
R⁵ is H or -(C₁-C₆)alkyl;
or R⁴ and R⁵ and the carbon atom to which they are attached form a cyclopropyl ring;
R⁹ is H, -(C₁-C₆)alkyl, -(C₁-C₆)haloalkyl, or -(C₃-C₆)cycloalkyl;
R¹⁷ is H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, -C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-,
optionally substituted heterocycloalkyl, optionally substituted aryl, or optionally substituted heteroaryl; Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl; and
or R²⁵ and R²⁶ and the nitrogen atom to which they are attached form a heterocycloalkyl ring;

or a pharmaceutically acceptable salt, solvate, or prodrug thereof.In one embodiment is a compound of Formula (IId) wherein R¹⁷ is -(C₁-C₆)alkyl. In another embodiment is a compound of Formula (IId) wherein R¹⁷ is -CH₃. In another embodiment is a compound of Formula (IId) wherein R¹⁷ is -CH₂CH₃. In another embodiment is a compound of Formula (IId) wherein R¹⁷ is -(C₃-C₆)cycloalkyl. In another embodiment is a compound of Formula (IId) wherein R¹⁷ is cyclopropyl. In another embodiment is a compound of Formula (IId) wherein R¹⁷ is -(C₁-C₆)alkyl-C(O)OR²³. In another embodiment is a compound of Formula (IId) wherein R¹⁷ is -CH₂CH₂OH. In another embodiment is a compound of Formula (IId) wherein R¹⁷ is -(C₁-C₆)alkyl-NR²¹R²². In another embodiment is a compound of Formula (IId) wherein R¹⁷ is -CH₂CH₂NH₂. In another embodiment is a compound of Formula (IId) wherein R¹⁷ is H.

In another embodiment is a compound of Formula (IId) wherein R⁵ is H.

In another embodiment is a compound of Formula (IId) wherein R⁴ is H. In another embodiment is a compound of Formula (IId) wherein R⁴ is -(C₁-C₆)alkyl. In another embodiment is a compound of Formula (IId) wherein R⁴ is -CH₃. In another embodiment is a compound of Formula (IId) wherein R⁴ is - CH₂CH₃. In another embodiment is a compound of Formula (IId) wherein R⁴ is -(C₁-C₆)alkyl-OH. In another embodiment is a compound of Formula (IId) wherein R⁴ is -CH₂OH. In another embodiment is a compound of Formula (IId) wherein R⁴ is -(C₃-C₆)cycloalkyl. In another embodiment is a compound of Formula (IId) wherein R⁴ is cyclopropyl. In another embodiment is a compound of Formula (IId) wherein R⁴ is -C(O)NH₂.

In another embodiment is a compound of Formula (IId) wherein R⁴ and R⁵ are H.

In another embodiment is a compound of Formula (IId) wherein R⁴ and R⁵ and the carbon atom to which they are attached form a cyclopropyl ring.

In another embodiment is a compound of Formula (IId) wherein R⁹ is -(C₁-C₆)alkyl. In another embodiment is a compound of Formula (IId) wherein R⁹ is -CH₃. In another embodiment is a compound of Formula (IId) wherein R⁹ is -CH₂CH₃. In another embodiment is a compound of Formula (IId) wherein R⁹ is -(C₁-C₆)haloalkyl. In another embodiment is a compound of Formula (IId) wherein R⁹ is -CH₂F. In another embodiment is a compound of Formula (IId) wherein R⁹ is -CHF₂. In another embodiment is a compound of Formula (IId) wherein R⁹ is -(C₃-C₆)cycloalkyl. In another embodiment is a compound of Formula (IId) wherein R⁹ is cyclopropyl. In another embodiment is a compound of Formula (IId) wherein R⁹ is H.

In another embodiment is a compound of Formula (IId) wherein R¹ and R² are each independently H or -(C₁-C₆)alkyl-NR²¹R²². In another embodiment is a compound of Formula (IId) wherein R¹ and R² are each H. In another embodiment is a compound of Formula (IId) wherein R¹ and R² are each independently -(C₁-C₆)alkyl-NR²¹R²². In another embodiment is a compound of Formula (IId) wherein R¹ is H, and R² is -(C₁-C₆)alkyl-NR²¹R²². In another embodiment is a compound of Formula (IId) wherein R¹ is -(C₁-C₆)alkyl-NR²¹R²², and R² is H. In another embodiment is a compound of Formula (IId) wherein R¹ is H, and R² is -CH₂CH₂NH₂. In another embodiment is a compound of Formula (IId) wherein R¹ is -CH₂CH₂NH₂, and R² is H. In another embodiment is a compound of Formula (IId) wherein R¹ and R² are each -CH₂CH₂NH₂. In another embodiment is a compound of Formula (IId) wherein R¹ and R² are each independently H, -(C₁-C₆)alkyl-NR²¹R²², or -CH₂CH(OH)CH₂NH₂. In another embodiment is a compound of Formula (IId) wherein R¹ and R² are each independently -CH₂CH(OH)CH₂NH₂. In another embodiment is a compound of Formula (IId) wherein R¹ is H, and R² is -CH₂CH(OH)CH₂NH₂. In another embodiment is a compound of Formula (IId) wherein R¹ is -CH₂CH(OH)CH₂NH₂, and R² is H. In a further embodiment is a compound of Formula (IId) wherein R¹ and R² and the atoms to which they are attached form an optionally substituted heterocycloalkyl ring.

In another embodiment is a compound of Formula (IId) wherein X is optionally substituted aryl. In a further embodiment is a compound of Formula (IId) wherein X is optionally substituted phenyl. In another embodiment is a compound of Formula (IId) wherein X is optionally substituted heteroaryl. In a further embodiment is a compound of Formula (IId) wherein X is disubstituted heteroaryl. In a further embodiment is a compound of Formula (IId) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (IId) wherein X is heteroaryl disubstituted with substituents each independently selected from -(C₁-C₆)alkyl. In a further embodiment is a compound of Formula (IId) wherein X is heteroaryl disubstituted with methyl. In a further embodiment is a compound of Formula (IId) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IId) wherein X is pyridinyl disubstituted with substituents each independently selected from -(C₁-C₆)alkyl. In a further embodiment is a compound of Formula (IId) wherein X is pyridinyl disubstituted with methyl. In a further embodiment is a compound of Formula (IId) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IId) wherein X is pyrimidinyl disubstituted with substituents each independently selected from -(C₁-C₆)alkyl. In a further embodiment is a compound of Formula (IId) wherein X is pyrimidinyl disubstituted with methyl. In another embodiment is a compound of Formula (IId) wherein X is optionally substituted -(C₁-C₆)alkyl-.

In another embodiment is a compound of Formula (IId) wherein Y is optionally substituted aryl. In a further embodiment is a compound of Formula (IId) wherein Y is optionally substituted phenyl. In another embodiment is a compound of Formula (IId) wherein Y is optionally substituted heteroaryl. In another embodiment is a compound of Formula (IId) wherein Y is optionally substituted -(C₁-C₆)alkyl-. In another embodiment is a compound of Formula (IId) wherein Y is optionally substituted (C₃-C₇)cycloalkyl-. In another embodiment is a compound of Formula (IId) wherein Y is optionally substituted heterocycloalkyl. In another embodiment is a compound of Formula (IId) wherein Y is -O-. In another embodiment is a compound of Formula (IId) wherein Y is -(C₂-C₆)alkynyl. In another embodiment is a compound of Formula (IId) wherein Y is -O-(C₁-C₆)alkyl-. In another embodiment is a compound of Formula (IId) wherein Y is a bond.

In another embodiment is a compound of Formula (IId) wherein Z is -(C₁-C₁₂)alkyl. In a further embodiment is a compound of Formula (IId) wherein Z is n-butyl, isobutyl, or tert-butyl. In another embodiment is a compound of Formula (IId) wherein Z is -O-(C₁-C₁₂)alkyl. In another embodiment is a compound of Formula (IId) wherein Z is -O-(C₃-C₇)cycloalkyl. In another embodiment is a compound of Formula (IId) wherein Z is -(C₂-C₁₂)alkenyl. In another embodiment is a compound of Formula (IId) wherein Z is optionally substituted aryl. In a further embodiment is a compound of Formula (IId) wherein Z is optionally substituted phenyl. In a further embodiment is a compound of Formula (IId) wherein Z is phenyl monsubstituted or disubstituted with a substituent independently selected from -(C₁-C₈)alkyl. In a further embodiment is a compound of Formula (IId) wherein Z is phenyl monosubstituted with n-butyl, isobutyl, or tert-butyl. In a further embodiment is a compound of Formula (IId) wherein Z is phenyl monosubstituted with n-butyl. In a further embodiment is a compound of Formula (IId) wherein Z is phenyl monosubstituted with isobutyl. In a further embodiment is a compound of Formula (IId) wherein Z is phenyl monosubstituted with tert-butyl. In another embodiment is a compound of Formula (IId) wherein Z is optionally substituted heteroaryl. In another embodiment is a compound of Formula (IId) wherein Z is optionally substituted -(C₃-C₇)cycloalkyl. In another embodiment is a compound of Formula (IId) wherein Z is optionally substituted heterocycloalkyl. In another embodiment is a compound of Formula (IId) wherein Z is halogen.

In another embodiment described herein are compounds of Formula (II) having the structure of Formula (IIe): 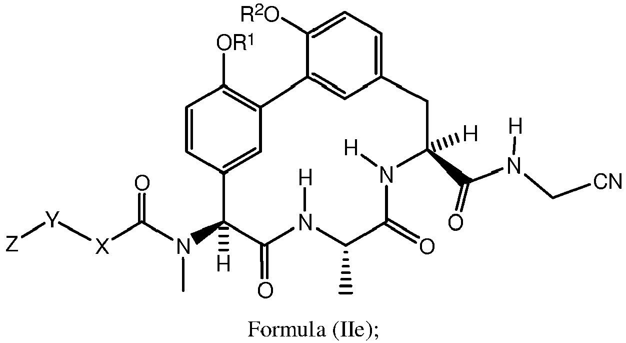 wherein:
R¹ and R² are each independently H or -CH₂CH₂NH₂;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, -C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl; and
or R²⁵ and R²⁶ and the nitrogen atom to which they are attached form a heterocycloalkyl ring;
or a pharmaceutically acceptable salt, solvate, or prodrug thereof.

In one embodiment is a compound of Formula (IIe) wherein R¹ and R² are each H. In another embodiment is a compound of Formula (IIe) wherein R¹ is H, and R² is -CH₂CH₂NH₂. In another embodiment is a compound of Formula (IIe) wherein R¹ is -CH₂CH₂NH₂, and R² is H. In another embodiment is a compound of Formula (IIe) wherein R¹ and R² are each -CH₂CH₂NH₂.

In another embodiment is a compound of Formula (IIe) wherein X is optionally substituted aryl. In a further embodiment is a compound of Formula (IIe) wherein X is optionally substituted phenyl. In another embodiment is a compound of Formula (IIe) wherein X is optionally substituted heteroaryl. In a further embodiment is a compound of Formula (IIe) wherein X is disubstituted heteroaryl. In a further embodiment is a compound of Formula (IIe) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (IIe) wherein X is heteroaryl disubstituted with substituents each independently selected from -(C₁-C₆)alkyl. In a further embodiment is a compound of Formula (IIe) wherein X is heteroaryl disubstituted with methyl. In a further embodiment is a compound of Formula (IIe) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IIe) wherein X is pyridinyl disubstituted with substituents each independently selected from -(C₁-C₆)alkyl. In a further embodiment is a compound of Formula (IIe) wherein X is pyridinyl disubstituted with methyl. In a further embodiment is a compound of Formula (IIe) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IIe) wherein X is pyrimidinyl disubstituted with substituents each independently selected from -(C₁-C₆)alkyl. In a further embodiment is a compound of Formula (IIe) wherein X is pyrimidinyl disubstituted with methyl. In another embodiment is a compound of Formula (IIe) wherein X is optionally substituted -(C₁-C₆)alkyl-.

In another embodiment is a compound of Formula (IIe) wherein Y is optionally substituted aryl. In a further embodiment is a compound of Formula (IIe) wherein Y is optionally substituted phenyl. In another embodiment is a compound of Formula (IIe) wherein Y is optionally substituted heteroaryl. In another embodiment is a compound of Formula (IIe) wherein Y is optionally substituted -(C₁-C₆)alkyl-. In another embodiment is a compound of Formula (IIe) wherein Y is optionally substituted (C₃-C₇)cycloalkyl-. In another embodiment is a compound of Formula (IIe) wherein Y is optionally substituted heterocycloalkyl. In another embodiment is a compound of Formula (IIe) wherein Y is -O-. In another embodiment is a compound of Formula (IIe) wherein Y is -(C₂-C₆)alkynyl. In another embodiment is a compound of Formula (IIe) wherein Y is -O-(C₁-C₆)alkyl-. In another embodiment is a compound of Formula (IIe) wherein Y is a bond.

In another embodiment is a compound of Formula (IIe) wherein Z is -(C₁-C₁₂)alkyl. In a further embodiment is a compound of Formula (IIe) wherein Z is n-butyl, isobutyl, or tert-butyl. In another embodiment is a compound of Formula (IIe) wherein Z is -O-(C₁-C₁₂)alkyl. In another embodiment is a compound of Formula (IIe) wherein Z is -O-(C₃-C₇)cycloalkyl. In another embodiment is a compound of Formula (IIe) wherein Z is -(C₂-C₁₂)alkenyl. In another embodiment is a compound of Formula (IIe) wherein Z is optionally substituted aryl. In a further embodiment is a compound of Formula (IIe) wherein Z is optionally substituted phenyl. In a further embodiment is a compound of Formula (IIe) wherein Z is phenyl monsubstituted or disubstituted with a substituent independently selected from -(C₁-C₈)alkyl. In a further embodiment is a compound of Formula (IIe) wherein Z is phenyl monosubstituted with n-butyl, isobutyl, or tert-butyl. In a further embodiment is a compound of Formula (IIe) wherein Z is phenyl monosubstituted with n-butyl. In a further embodiment is a compound of Formula (IIe) wherein Z is phenyl monosubstituted with isobutyl. In a further embodiment is a compound of Formula (IIe) wherein Z is phenyl monosubstituted with tert-butyl. In another embodiment is a compound of Formula (IIe) wherein Z is optionally substituted heteroaryl. In another embodiment is a compound of Formula (IIe) wherein Z is optionally substituted -(C₃-C₇)cycloalkyl. In another embodiment is a compound of Formula (IIe) wherein Z is optionally substituted heterocycloalkyl. In another embodiment is a compound of Formula (IIe) wherein Z is halogen.

In one aspect described herein are compounds of Formula (III): 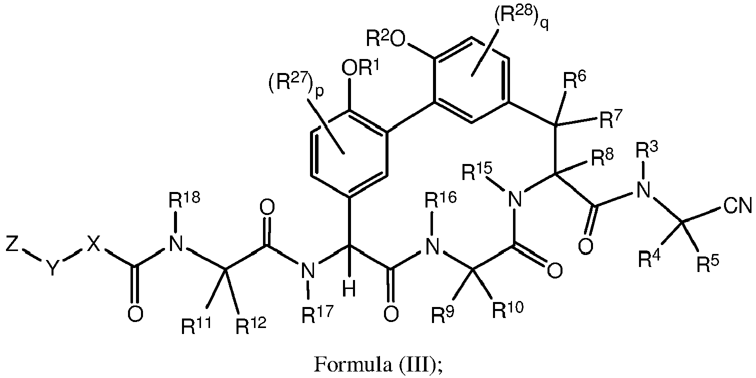 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring;
R³ is H or -(C₁-C₆)alkyl;
R⁴ is H, -(C₁-C₆)alkyl, -(C₁-C₆)alkyl-OH, -(C₃-C₆)cycloalkyl, or -C(O)NH₂;
or R³ and R⁴ are combined to form a heterocycloalkyl ring;
R⁵ is H or -(C₁-C₆)alkyl;
or R⁴ and R⁵ and the carbon atom to which they are attached form a cyclopropyl ring;
R⁶, R⁷, and R⁸ are each independently H, fluoro, hydroxyl, amino, optionally substituted alkyl, heteroalkyl, or -(C₁-C₆)alkyl;
R⁹ is H, -(C₁-C₆)alkyl, -(C₁-C₆)haloalkyl, or -(C₃-C₆)cycloalkyl;
R¹⁰ is H, -(C₁-C₆)alkyl, -(C₁-C₆)haloalkyl, or -(C₃-C₆)cycloalkyl;
or R⁹ and R¹⁰ are combined to form a heterocycloalkyl or cycloalkyl ring
R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
or R¹¹ and R¹⁸ are combined to form an optionally substituted heterocycloalkyl ring; and R¹² is H;
R¹⁵, R¹⁶, R¹⁷, and R¹⁸ are each independently H, -(C₁-C₆)alkyl, -(C₃-C₆)cycloalkyl, -(C₁-C₆)alkyl-OR²³, - (C₁-C₆)alkyl-C(O)OR²³, or -(C₁-C₆)alkyl-NR²¹R²²;
X is disubstituted heteroaryl;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring;
each R²⁷ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R¹ and R²⁷ and the atoms to which they are attached form an optionally substituted 5- or 6-membered heterocycloalkyl ring;
each R²⁸ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R² and R²⁸ and the atoms to which they are attached form an optionally substituted 5- or 6-membered heterocycloalkyl ring;
p is 0, 1, or 2; and
q is 0, 1, or 2;
or a pharmaceutically acceptable salt, solvate, or prodrug thereof.

In one embodiment is a compound of Formula (III) wherein R⁶, R⁷, and R⁸ are H.

In another embodiment is a compound of Formula (III) wherein R¹⁵ and R¹⁶ are H.

In one embodiment is a compound of Formula (III) wherein R¹⁷ is -(C₁-C₆)alkyl. In another embodiment is a compound of Formula (III) wherein R¹⁷ is -CH₃. In another embodiment is a compound of Formula (III) wherein R¹⁷ is -CH₂CH₃. In another embodiment is a compound of Formula (III) wherein R¹⁷ is -(C₃-C₆)cycloalkyl. In another embodiment is a compound of Formula (III) wherein R¹⁷ is cyclopropyl. In another embodiment is a compound of Formula (III) wherein R¹⁷ is -(C₁-C₆)alkyl-C(O)OR²³. In another embodiment is a compound of Formula (III) wherein R¹⁷ is -CH₂CH₂OH. In another embodiment is a compound of Formula (III) wherein R¹⁷ is -(C₁-C₆)alkyl-NR²¹R²². In another embodiment is a compound of Formula (III) wherein R¹⁷ is -CH₂CH₂NH₂. In another embodiment is a compound of Formula (III) wherein R¹⁷ is H.

In another embodiment is a compound of Formula (III) wherein R¹⁸ is H.

In another embodiment is a compound of Formula (III) wherein R³ is H.

In another embodiment is a compound of Formula (III) wherein R⁵ is H.

In another embodiment is a compound of Formula (III) wherein R⁴ is H. In another embodiment is a compound of Formula (III) wherein R⁴ is -(C₁-C₆)alkyl. In another embodiment is a compound of Formula (III) wherein R⁴ is -CH₃. In another embodiment is a compound of Formula (III) wherein R⁴ is - CH₂CH₃. In another embodiment is a compound of Formula (III) wherein R⁴ is -(C₁-C₆)alkyl-OH. In another embodiment is a compound of Formula (III) wherein R⁴ is -CH₂OH. In another embodiment is a compound of Formula (III) wherein R⁴ is -(C₃-C₆)cycloalkyl. In another embodiment is a compound of Formula (III) wherein R⁴ is cyclopropyl. In another embodiment is a compound of Formula (III) wherein R⁴ is -C(O)NH₂.

In another embodiment is a compound of Formula (III) wherein R³, R⁴, and R⁵ are H.

In another embodiment is a compound of Formula (III) wherein R⁴ and R⁵ and the carbon atom to which they are attached form a cyclopropyl ring.

In another embodiment is a compound of Formula (III) wherein R¹⁰ is H.

In another embodiment is a compound of Formula (III) wherein R¹⁰ is H and R⁹ is -(C₁-C₆)alkyl. In another embodiment is a compound of Formula (III) wherein R¹⁰ is H and R⁹ is -CH₃. In another embodiment is a compound of Formula (III) wherein R¹⁰ is H and R⁹ is -CH₂CH₃. In another embodiment is a compound of Formula (III) wherein R¹⁰ is H and R⁹ is -(C₁-C₆)haloalkyl. In another embodiment is a compound of Formula (III) wherein R¹⁰ is H and R⁹ is -CH₂F. In another embodiment is a compound of Formula (III) wherein R¹⁰ is H and R⁹ is -CHF₂. In another embodiment is a compound of Formula (III) wherein R¹⁰ is H and R⁹ is -(C₃-C₆)cycloalkyl. In another embodiment is a compound of Formula (III) wherein R¹⁰ is H and R⁹ is cyclopropyl. In another embodiment is a compound of Formula (III) wherein R¹⁰ is H and R⁹ is H.

In another embodiment is a compound of Formula (III) wherein R¹² is H.

In another embodiment is a compound of Formula (III) wherein R¹² is H and R¹¹ is - (C₁-C₆)alkyl. In another embodiment is a compound of Formula (III) wherein R¹² is H and R¹¹ is -CH₃. In another embodiment is a compound of Formula (III) wherein R¹² is H and R¹¹ is -(C₁-C₆)alkyl-OR²³. In another embodiment is a compound of Formula (III) wherein R¹² is H and R¹¹ is -CH₂OH. In another embodiment is a compound of Formula (III) wherein R¹² is H and R¹¹ is -CH₂CH₂OH. In another embodiment is a compound of Formula (III) wherein R¹² is H and R¹¹ is -(C₁-C₆)alkyl. In another embodiment is a compound of Formula (III) wherein R¹² is H and R¹¹ is -(C₁-C₆)alkyl-NR²¹R²². In another embodiment is a compound of Formula (III) wherein R¹² is H and R¹¹ is -(C₁-C₆)alkyl-NH₂. In another embodiment is a compound of Formula (III) wherein R¹² is H and R¹¹ is -CH₂NH₂. In another embodiment is a compound of Formula (III) wherein R¹² is H and R¹¹ is -CH₂CH₂NH₂. In another embodiment is a compound of Formula (III) wherein R¹² is H and R¹¹ is -CH₂CH₂CH₂NH₂. In another embodiment is a compound of Formula (III) wherein R¹² is H and R¹¹ is -CH₂CH₂CH₂CH₂NH₂. In another embodiment is a compound of Formula (III) wherein R¹² is H and R¹¹ is -(C₁-C₆)alkyl-CN. In another embodiment is a compound of Formula (III) wherein R¹² is H and R¹¹ is -CH₂CN. In another embodiment is a compound of Formula (III) wherein R¹² is H and R¹¹ is -(C₁-C₆)alkyl-C(O)NR²⁵R²⁶. In another embodiment is a compound of Formula (III) wherein R¹² is H and R¹¹ is -CH₂C(O)NH₂. In another embodiment is a compound of Formula (III) wherein R¹² is H and R¹¹ is -CH₂CH₂C(O)NH₂. In another embodiment is a compound of Formula (III) wherein R¹² is H and R¹¹ is -(C₁-C₆)alkyl-heteroaryl. In another embodiment is a compound of Formula (III) wherein R¹² is H and R¹¹ is -(C₁-C₆)alkyl-N(H)S(O)₂NR²⁵R²⁶. In another embodiment is a compound of Formula (III) wherein R¹² is H and R¹¹ is -CH₂N(H)S(O)₂NH₂. In another embodiment is a compound of Formula (III) wherein R¹² is H and R¹¹ is H.

In another embodiment is a compound of Formula (III) wherein R¹¹ and R¹⁸ are combined to form an optionally substituted heterocycloalkyl ring and R¹² is H.

In another embodiment is a compound of Formula (III) wherein p is 1 and R²⁷ is halogen. In another embodiment is a compound of Formula (III) wherein p is 1 and R²⁷ is optionally substituted -(C₁-C₆)alkyl. In another embodiment is a compound of Formula (III) wherein q is 0, p is 1 and R²⁷ is halogen. In another embodiment is a compound of Formula (III) wherein q is 0, p is 1 and R²⁷ is optionally substituted -(C₁-C₆)alkyl. In another embodiment is a compound of Formula (III) wherein q is 1 and R²⁸ is halogen. In another embodiment is a compound of Formula (III) wherein q is 1 and R²⁸ is optionally substituted -(C₁-C₆)alkyl. In another embodiment is a compound of Formula (III) wherein p is 0, q is 1 and R²⁸ is halogen. In another embodiment is a compound of Formula (III) wherein p is 0, q is 1 and R²⁸ is optionally substituted -(C₁-C₆)alkyl.

In another embodiment is a compound of Formula (III) wherein p is 0, and q is 0.

In another embodiment is a compound of Formula (III) wherein R¹ and R² are each independently H or -(C₁-C₆)alkyl-NR²¹R²². In another embodiment is a compound of Formula (III) wherein R¹ and R² are each H. In another embodiment is a compound of Formula (III) wherein R¹ and R² are each independently -(C₁-C₆)alkyl-NR²¹R²². In another embodiment is a compound of Formula (III) wherein R¹ is H, and R² is -(C₁-C₆)alkyl-NR²¹R²². In another embodiment is a compound of Formula (III) wherein R¹ is -(C₁-C₆)alkyl-NR²¹R²², and R² is H. In another embodiment is a compound of Formula (III) wherein R¹ is H, and R² is -CH₂CH₂NH₂. In another embodiment is a compound of Formula (III) wherein R¹ is -CH₂CH₂NH₂, and R² is H. In another embodiment is a compound of Formula (III) wherein R¹ and R² are each -CH₂CH₂NH₂. In a further embodiment is a compound of Formula (III) wherein R¹ is -(C₁-C₆)alkyl-NR²¹R²² and R² is H. In a further embodiment is a compound of Formula (III) wherein R¹ is - CH₂CH₂NH₂ and R² is H. In a further embodiment is a compound of Formula (III) wherein R¹ is H and R² is -(C₁-C₆)alkyl-NR²¹R²². In a further embodiment is a compound of Formula (III) wherein R¹ is H and R² is -CH₂CH₂NH₂. In another embodiment is a compound of Formula (III) wherein R¹ and R² are each independently H, -(C₁-C₆)alkyl-NR²¹R²², or -CH₂CH(OH)CH₂NH₂. In another embodiment is a compound of Formula (III) wherein R¹ and R² are each independently -CH₂CH(OH)CH₂NH₂. In another embodiment is a compound of Formula (III) wherein R¹ is H, and R² is -CH₂CH(OH)CH₂NH₂. In another embodiment is a compound of Formula (III) wherein R¹ is -CH₂CH(OH)CH₂NH₂, and R² is H. In a further embodiment is a compound of Formula (III) wherein R¹ and R² and the atoms to which they are attached form an optionally substituted heterocycloalkyl ring.

In another embodiment is a compound of Formula (III) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (III) wherein X is heteroaryl disubstituted with substituents each independently selected from - (C₁-C₆)alkyl. In a further embodiment is a compound of Formula (III) wherein X is heteroaryl disubstituted with methyl. In a further embodiment is a compound of Formula (III) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (III) wherein X is pyridinyl disubstituted with substituents each independently selected from -(C₁-C₆)alkyl. In a further embodiment is a compound of Formula (III) wherein X is pyridinyl disubstituted with methyl. In a further embodiment is a compound of Formula (III) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (III) wherein X is pyrimidinyl disubstituted with substituents each independently selected from -(C₁-C₆)alkyl. In a further embodiment is a compound of Formula (III) wherein X is pyrimidinyl disubstituted with methyl.

In another embodiment is a compound of Formula (III) wherein Y is optionally substituted aryl. In a further embodiment is a compound of Formula (III) wherein Y is optionally substituted phenyl. In another embodiment is a compound of Formula (III) wherein Y is optionally substituted heteroaryl. In another embodiment is a compound of Formula (III) wherein Y is optionally substituted -(C₁-C₆)alkyl-. In another embodiment is a compound of Formula (III) wherein Y is optionally substituted (C₃-C₇)cycloalkyl-. In another embodiment is a compound of Formula (III) wherein Y is optionally substituted heterocycloalkyl. In another embodiment is a compound of Formula (III) wherein Y is -O-. In another embodiment is a compound of Formula (III) wherein Y is -(C₂-C₆)alkynyl. In another embodiment is a compound of Formula (III) wherein Y is -O-(C₁-C₆)alkyl-. In another embodiment is a compound of Formula (III) wherein Y is a bond.

In another embodiment is a compound of Formula (III) wherein Z is -(C₁₋C₁₂)alkyl. In a further embodiment is a compound of Formula (III) wherein Z is n-butyl, isobutyl, or tert-butyl. In another embodiment is a compound of Formula (III) wherein Z is -O-(C₁-C₁₂)alkyl. In another embodiment is a compound of Formula (III) wherein Z is -O-(C₃-C₇)cycloalkyl. In another embodiment is a compound of Formula (III) wherein Z is -(C₂-C₁₂)alkenyl. In another embodiment is a compound of Formula (III) wherein Z is optionally substituted aryl. In a further embodiment is a compound of Formula (III) wherein Z is optionally substituted phenyl. In a further embodiment is a compound of Formula (III) wherein Z is phenyl monsubstituted or disubstituted with a substituent independently selected from -(C₁-C₈)alkyl. In a further embodiment is a compound of Formula (III) wherein Z is phenyl monosubstituted with n-butyl, isobutyl, or tert-butyl. In a further embodiment is a compound of Formula (III) wherein Z is phenyl monosubstituted with n-butyl. In a further embodiment is a compound of Formula (III) wherein Z is phenyl monosubstituted with isobutyl. In a further embodiment is a compound of Formula (III) wherein Z is phenyl monosubstituted with tert-butyl. In another embodiment is a compound of Formula (III) wherein Z is optionally substituted heteroaryl. In another embodiment is a compound of Formula (III) wherein Z is optionally substituted -(C₃-C₇)cycloalkyl. In another embodiment is a compound of Formula (III) wherein Z is optionally substituted heterocycloalkyl. In another embodiment is a compound of Formula (III) wherein Z is halogen.

In another embodiment is a compound of Formula (III) wherein Z-Y-X- is not

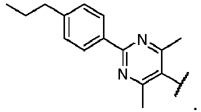

In another embodiment is a compound of Formula (III) having the structure of Formula (IIIa): 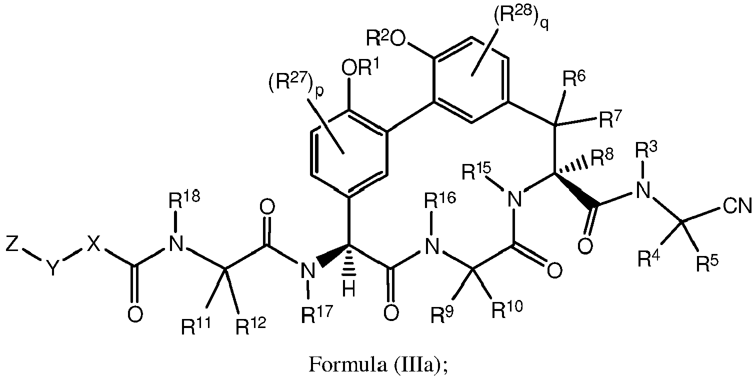 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring;
R³ is H or -(C₁-C₆)alkyl;
R⁴ is H, -(C₁-C₆)alkyl, -(C₁-C₆)alkyl-OH, -(C₃-C₆)cycloalkyl, or -C(O)NH₂;
or R³ and R⁴ are combined to form a heterocycloalkyl ring;
R⁵ is H or -(C₁-C₆)alkyl;
or R⁴ and R⁵ and the carbon atom to which they are attached form a cyclopropyl ring;
R⁶, R⁷, and R⁸ are each independently H, fluoro, hydroxyl, amino, optionally substituted alkyl, heteroalkyl, or -(C₁-C₆)alkyl;
R⁹ is H, -(C₁-C₆)alkyl, -(C₁-C₆)haloalkyl, or -(C₃-C₆)cycloalkyl;
R¹⁰ is H, -(C₁-C₆)alkyl, -(C₁-C₆)haloalkyl, or -(C₃-C₆)cycloalkyl;
or R⁹ and R¹⁰ are combined to form a heterocycloalkyl or cycloalkyl ring
R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
or R¹¹ and R¹⁸ are combined to form an optionally substituted heterocycloalkyl ring; and R¹² is H;
R¹⁵, R¹⁶, R¹⁷, and R¹⁸ are each independently H, -(C₁-C₆)alkyl, -(C₃-C₆)cycloalkyl, -(C₁-C₆)alkyl-OR²³, - (C₁-C₆)alkyl-C(O)OR²³, or -(C₁-C₆)alkyl-NR²¹R²²;
X is disubstituted heteroaryl;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring;
each R²⁷ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R¹ and R²⁷ and the atoms to which they are attached form an optionally substituted 5- or 6-membered heterocycloalkyl ring;
each R²⁸ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R² and R²⁸ and the atoms to which they are attached form an optionally substituted 5- or 6-membered heterocycloalkyl ring;
p is 0, 1, or 2; and
q is 0, 1, or 2;

or a pharmaceutically acceptable salt, solvate, or prodrug thereof.In one embodiment is a compound of Formula (IIIa) wherein R⁶, R⁷, and R⁸ are H.

In another embodiment is a compound of Formula (IIIa) wherein R¹⁵ and R¹⁶ are H.

In one embodiment is a compound of Formula (IIIa) wherein R¹⁷ is -(C₁-C₆)alkyl. In another embodiment is a compound of Formula (IIIa) wherein R¹⁷ is -CH₃. In another embodiment is a compound of Formula (IIIa) wherein R¹⁷ is -CH₂CH₃. In another embodiment is a compound of Formula (IIIa) wherein R¹⁷ is -(C₃-C₆)cycloalkyl. In another embodiment is a compound of Formula (IIIa) wherein R¹⁷ is cyclopropyl. In another embodiment is a compound of Formula (IIIa) wherein R¹⁷ is -(C₁-C₆)alkyl-C(O)OR²³. In another embodiment is a compound of Formula (IIIa) wherein R¹⁷ is -CH₂CH₂OH. In another embodiment is a compound of Formula (IIIa) wherein R¹⁷ is -(C₁-C₆)alkyl-NR²¹R²². In another embodiment is a compound of Formula (IIIa) wherein R¹⁷ is -CH₂CH₂NH₂. In another embodiment is a compound of Formula (IIIa) wherein R¹⁷ is H.

In another embodiment is a compound of Formula (IIIa) wherein R¹⁸ is H.

In another embodiment is a compound of Formula (IIIa) wherein R³ is H.

In another embodiment is a compound of Formula (IIIa) wherein R⁵ is H.

In another embodiment is a compound of Formula (IIIa) wherein R⁴ is H. In another embodiment is a compound of Formula (IIIa) wherein R⁴ is -(C₁-C₆)alkyl. In another embodiment is a compound of Formula (IIIa) wherein R⁴ is -CH₃. In another embodiment is a compound of Formula (IIIa) wherein R⁴ is -CH₂CH₃. In another embodiment is a compound of Formula (IIIa) wherein R⁴ is -(C₁-C₆)alkyl-OH. In another embodiment is a compound of Formula (IIIa) wherein R⁴ is -CH₂OH. In another embodiment is a compound of Formula (IIIa) wherein R⁴ is -(C₃-C₆)cycloalkyl. In another embodiment is a compound of Formula (IIIa) wherein R⁴ is cyclopropyl. In another embodiment is a compound of Formula (IIIa) wherein R⁴ is -C(O)NH₂.

In another embodiment is a compound of Formula (IIIa) wherein R⁴ and R⁵ and the carbon atom to which they are attached form a cyclopropyl ring.

In another embodiment is a compound of Formula (IIIa) wherein R³, R⁴, and R⁵ are H.

In another embodiment is a compound of Formula (IIIa) wherein R¹⁰ is H.

In another embodiment is a compound of Formula (IIIa) wherein R¹⁰ is H and R⁹ is -(C₁-C₆)alkyl. In another embodiment is a compound of Formula (IIIa) wherein R¹⁰ is H and R⁹ is -CH₃. In another embodiment is a compound of Formula (IIIa) wherein R¹⁰ is H and R⁹ is -CH₂CH₃. In another embodiment is a compound of Formula (IIIa) wherein R¹⁰ is H and R⁹ is -(C₁-C₆)haloalkyl. In another embodiment is a compound of Formula (IIIa) wherein R¹⁰ is H and R⁹ is -CH₂F. In another embodiment is a compound of Formula (IIIa) wherein R¹⁰ is H and R⁹ is -CHF₂. In another embodiment is a compound of Formula (IIIa) wherein R¹⁰ is H and R⁹ is -(C₃-C₆)cycloalkyl. In another embodiment is a compound of Formula (IIIa) wherein R¹⁰ is H and R⁹ is cyclopropyl. In another embodiment is a compound of Formula (IIIa) wherein R¹⁰ is H and R⁹ is H.

In another embodiment is a compound of Formula (IIIa) wherein R¹² is H.

In another embodiment is a compound of Formula (IIIa) wherein R¹² is H and R¹¹ is -(C₁-C₆)alkyl. In another embodiment is a compound of Formula (IIIa) wherein R¹² is H and R¹¹ is -CH₃. In another embodiment is a compound of Formula (IIIa) wherein R¹² is H and R¹¹ is -(C₁-C₆)alkyl-OR²³. In another embodiment is a compound of Formula (IIIa) wherein R¹² is H and R¹¹ is -CH₂OH. In another embodiment is a compound of Formula (IIIa) wherein R¹² is H and R¹¹ is -CH₂CH₂OH. In another embodiment is a compound of Formula (IIIa) wherein R¹² is H and R¹¹ is -(C₁-C₆)alkyl. In another embodiment is a compound of Formula (IIIa) wherein R¹² is H and R¹¹ is -(C₁-C₆)alkyl-NR²¹R²². In another embodiment is a compound of Formula (IIIa) wherein R¹² is H and R¹¹ is -(C₁-C₆)alkyl-NH₂. In another embodiment is a compound of Formula (IIIa) wherein R¹² is H and R¹¹ is -CH₂NH₂. In another embodiment is a compound of Formula (IIIa) wherein R¹² is H and R¹¹ is -CH₂CH₂NH₂. In another embodiment is a compound of Formula (IIIa) wherein R¹² is H and R¹¹ is -CH₂CH₂CH₂NH₂. In another embodiment is a compound of Formula (IIIa) wherein R¹² is H and R¹¹ is -CH₂CH₂CH₂CH₂NH₂. In another embodiment is a compound of Formula (IIIa) wherein R¹² is H and R¹¹ is -(C₁-C₆)alkyl-CN. In another embodiment is a compound of Formula (IIIa) wherein R¹² is H and R¹¹ is -CH₂CN. In another embodiment is a compound of Formula (IIIa) wherein R¹² is H and R¹¹ is -(C₁-C₆)alkyl-C(O)NR²⁵R²⁶. In another embodiment is a compound of Formula (IIIa) wherein R¹² is H and R¹¹ is -CH₂C(O)NH₂. In another embodiment is a compound of Formula (IIIa) wherein R¹² is H and R¹¹ is -CH₂CH₂C(O)NH₂. In another embodiment is a compound of Formula (IIIa) wherein R¹² is H and R¹¹ is -(C₁-C₆)alkyl-heteroaryl. In another embodiment is a compound of Formula (IIIa) wherein R¹² is H and R¹¹ is -(C₁-C₆)alkyl-N(H)S(O)₂NR²⁵R²⁶. In another embodiment is a compound of Formula (IIIa) wherein R¹² is H and R¹¹ is - CH₂N(H)S(O)₂NH₂. In another embodiment is a compound of Formula (IIIa) wherein R¹² is H and R¹¹ is H.

In another embodiment is a compound of Formula (IIIa) wherein R¹¹ and R¹⁸ are combined to form an optionally substituted heterocycloalkyl ring and R¹² is H.

In another embodiment is a compound of Formula (IIIa) wherein p is 1 and R²⁷ is halogen. In another embodiment is a compound of Formula (IIIa) wherein p is 1 and R²⁷ is optionally substituted -(C₁-C₆)alkyl. In another embodiment is a compound of Formula (IIIa) wherein q is 0, p is 1 and R²⁷ is halogen. In another embodiment is a compound of Formula (IIIa) wherein q is 0, p is 1 and R²⁷ is optionally substituted -(C₁-C₆)alkyl. In another embodiment is a compound of Formula (IIIa) wherein q is 1 and R²⁸ is halogen. In another embodiment is a compound of Formula (IIIa) wherein q is 1 and R²⁸ is optionally substituted -(C₁-C₆)alkyl. In another embodiment is a compound of Formula (IIIa) wherein p is 0, q is 1 and R²⁸ is halogen. In another embodiment is a compound of Formula (IIIa) wherein p is 0, q is 1 and R²⁸ is optionally substituted -(C₁-C₆)alkyl.

In another embodiment is a compound of Formula (IIIa) wherein p is 0, and q is 0.

In another embodiment is a compound of Formula (IIIa) wherein R¹ and R² are each independently H or -(C₁-C₆)alkyl-NR²¹R²². In another embodiment is a compound of Formula (IIIa) wherein R¹ and R² are each H. In another embodiment is a compound of Formula (IIIa) wherein R¹ and R² are each independently -(C₁-C₆)alkyl-NR²¹R²². In another embodiment is a compound of Formula (IIIa) wherein R¹ is H, and R² is -(C₁-C₆)alkyl-NR²¹R²². In another embodiment is a compound of Formula (IIIa) wherein R¹ is -(C₁-C₆)alkyl-NR²¹R²², and R² is H. In another embodiment is a compound of Formula (IIIa) wherein R¹ is H, and R² is -CH₂CH₂NH₂. In another embodiment is a compound of Formula (IIIa) wherein R¹ is -CH₂CH₂NH₂, and R² is H. In another embodiment is a compound of Formula (IIIa) wherein R¹ and R² are each -CH₂CH₂NH₂. In a further embodiment is a compound of Formula (IIIa) wherein R¹ is -(C₁-C₆)alkyl-NR²¹R²² and R² is H. In a further embodiment is a compound of Formula (IIIa) wherein R¹ is - CH₂CH₂NH₂ and R² is H. In a further embodiment is a compound of Formula (IIIa) wherein R¹ is H and R² is -(C₁-C₆)alkyl-NR²¹R²². In a further embodiment is a compound of Formula (IIIa) wherein R¹ is H and R² is -CH₂CH₂NH₂. In another embodiment is a compound of Formula (IIIa) wherein R¹ and R² are each independently H, -(C₁-C₆)alkyl-NR²¹R²², or -CH₂CH(OH)CH₂NH₂. In another embodiment is a compound of Formula (IIIa) wherein R¹ and R² are each independently -CH₂CH(OH)CH₂NH₂. In another embodiment is a compound of Formula (IIIa) wherein R¹ is H, and R² is -CH₂CH(OH)CH₂NH₂. In another embodiment is a compound of Formula (IIIa) wherein R¹ is -CH₂CH(OH)CH₂NH₂, and R² is H. In a further embodiment is a compound of Formula (IIIa) wherein R¹ and R² and the atoms to which they are attached form an optionally substituted heterocycloalkyl ring.

In another embodiment is a compound of Formula (IIIa) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (IIIa) wherein X is heteroaryl disubstituted with substituents each independently selected from -(C₁-C₆)alkyl. In a further embodiment is a compound of Formula (IIIa) wherein X is heteroaryl disubstituted with methyl. In a further embodiment is a compound of Formula (IIIa) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IIIa) wherein X is pyridinyl disubstituted with substituents each independently selected from -(C₁-C₆)alkyl. In a further embodiment is a compound of Formula (IIIa) wherein X is pyridinyl disubstituted with methyl. In a further embodiment is a compound of Formula (IIIa) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IIIa) wherein X is pyrimidinyl disubstituted with substituents each independently selected from -(C₁-C₆)alkyl. In a further embodiment is a compound of Formula (IIIa) wherein X is pyrimidinyl disubstituted with methyl.

In another embodiment is a compound of Formula (IIIa) wherein Y is optionally substituted aryl. In a further embodiment is a compound of Formula (IIIa) wherein Y is optionally substituted phenyl. In another embodiment is a compound of Formula (IIIa) wherein Y is optionally substituted heteroaryl. In another embodiment is a compound of Formula (IIIa) wherein Y is optionally substituted -(C₁-C₆)alkyl-. In another embodiment is a compound of Formula (IIIa) wherein Y is optionally substituted (C₃-C₇)cycloalkyl-. In another embodiment is a compound of Formula (IIIa) wherein Y is optionally substituted heterocycloalkyl. In another embodiment is a compound of Formula (IIIa) wherein Y is -O-. In another embodiment is a compound of Formula (IIIa) wherein Y is -(C₂-C₆)alkynyl. In another embodiment is a compound of Formula (IIIa) wherein Y is -O-(C₁-C₆)alkyl-. In another embodiment is a compound of Formula (IIIa) wherein Y is a bond.

In another embodiment is a compound of Formula (IIIa) wherein Z is -(C₁₋C₁₂)alkyl. In a further embodiment is a compound of Formula (IIIa) wherein Z is n-butyl, isobutyl, or tert-butyl. In another embodiment is a compound of Formula (IIIa) wherein Z is -O-(C₁-C₁₂)alkyl. In another embodiment is a compound of Formula (IIIa) wherein Z is -O-(C₃-C₇)cycloalkyl. In another embodiment is a compound of Formula (IIIa) wherein Z is -(C₂-C₁₂)alkenyl. In another embodiment is a compound of Formula (IIIa) wherein Z is optionally substituted aryl. In a further embodiment is a compound of Formula (IIIa) wherein Z is optionally substituted phenyl. In a further embodiment is a compound of Formula (IIIa) wherein Z is phenyl monsubstituted or disubstituted with a substituent independently selected from -(C₁-C₈)alkyl. In a further embodiment is a compound of Formula (IIIa) wherein Z is phenyl monosubstituted with n-butyl, isobutyl, or tert-butyl. In a further embodiment is a compound of Formula (IIIa) wherein Z is phenyl monosubstituted with n-butyl. In a further embodiment is a compound of Formula (IIIa) wherein Z is phenyl monosubstituted with isobutyl. In a further embodiment is a compound of Formula (IIIa) wherein Z is phenyl monosubstituted with tert-butyl. In another embodiment is a compound of Formula (IIIa) wherein Z is optionally substituted heteroaryl. In another embodiment is a compound of Formula (IIIa) wherein Z is optionally substituted -(C₃-C₇)cycloalkyl. In another embodiment is a compound of Formula (IIIa) wherein Z is optionally substituted heterocycloalkyl. In another embodiment is a compound of Formula (IIIa) wherein Z is halogen.

In another embodiment is a compound of Formula (IIIa) wherein Z-Y-X- is not

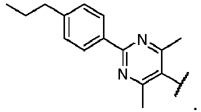

In another embodiment is a compound of Formula (III) having the structure of Formula (IIIb): 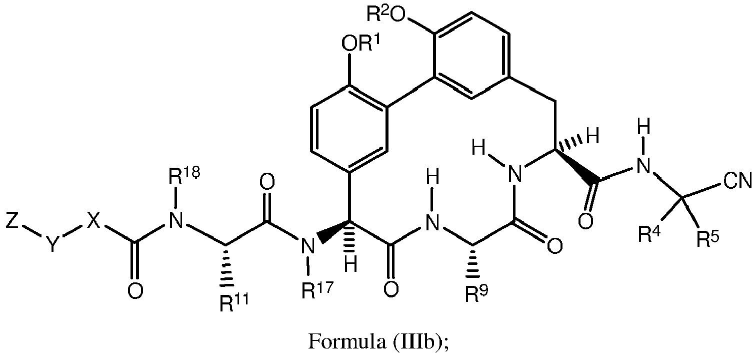 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²², - (C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring;
R⁴ is H, -(C₁-C₆)alkyl, -(C₁-C₆)alkyl-OH, -(C₃-C₆)cycloalkyl, or -C(O)NH₂;
R⁵ is H or -(C₁-C₆)alkyl;
or R⁴ and R⁵ and the carbon atom to which they are attached form a cyclopropyl ring;
R⁹ is H, -(C₁-C₆)alkyl, -(C₁-C₆)haloalkyl, or -(C₃-C₆)cycloalkyl;
R¹¹ is H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
or R¹¹ and R¹⁸ are combined to form an optionally substituted heterocycloalkyl ring; and R¹² is H;
R¹⁷ and R¹⁸ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is disubstituted heteroaryl;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring; and

or a pharmaceutically acceptable salt, solvate, or prodrug thereof.In one embodiment is a compound of Formula (IIIb) wherein R¹⁷ is -(C₁-C₆)alkyl. In another embodiment is a compound of Formula (IIIb) wherein R¹⁷ is -CH₃. In another embodiment is a compound of Formula (IIIb) wherein R¹⁷ is -CH₂CH₃. In another embodiment is a compound of Formula (IIIb) wherein R¹⁷ is -(C₃-C₆)cycloalkyl. In another embodiment is a compound of Formula (IIIb) wherein R¹⁷ is cyclopropyl. In another embodiment is a compound of Formula (IIIb) wherein R¹⁷ is -(C₁-C₆)alkyl-C(O)OR²³. In another embodiment is a compound of Formula (IIIb) wherein R¹⁷ is -CH₂CH₂OH. In another embodiment is a compound of Formula (IIIb) wherein R¹⁷ is -(C₁-C₆)alkyl-NR²¹R²². In another embodiment is a compound of Formula (IIIb) wherein R¹⁷ is -CH₂CH₂NH₂. In another embodiment is a compound of Formula (IIIb) wherein R¹⁷ is H.

In another embodiment is a compound of Formula (IIIb) wherein R¹⁸ is H.

In another embodiment is a compound of Formula (IIIb) wherein R⁵ is H.

In another embodiment is a compound of Formula (IIIb) wherein R⁴ is H. In another embodiment is a compound of Formula (IIIb) wherein R⁴ is -(C₁-C₆)alkyl. In another embodiment is a compound of Formula (IIIb) wherein R⁴ is -CH₃. In another embodiment is a compound of Formula (IIIb) wherein R⁴ is -CH₂CH₃. In another embodiment is a compound of Formula (IIIb) wherein R⁴ is -(C₁-C₆)alkyl-OH. In another embodiment is a compound of Formula (IIIb) wherein R⁴ is -CH₂OH. In another embodiment is a compound of Formula (IIIb) wherein R⁴ is -(C₃-C₆)cycloalkyl. In another embodiment is a compound of Formula (IIIb) wherein R⁴ is cyclopropyl. In another embodiment is a compound of Formula (IIIb) wherein R⁴ is -C(O)NH₂.

In another embodiment is a compound of Formula (IIIb) wherein R⁴ and R⁵ and the carbon atom to which they are attached form a cyclopropyl ring.

In another embodiment is a compound of Formula (IIIb) wherein R⁹ is -(C₁-C₆)alkyl. In another embodiment is a compound of Formula (IIIb) wherein R⁹ is -CH₃. In another embodiment is a compound of Formula (IIIb) wherein R⁹ is -CH₂CH₃. In another embodiment is a compound of Formula (IIIb) wherein R⁹ is -(C₁-C₆)haloalkyl. In another embodiment is a compound of Formula (IIIb) wherein R⁹ is - CH₂F. In another embodiment is a compound of Formula (IIIb) wherein R⁹ is -CHF₂. In another embodiment is a compound of Formula (IIIb) wherein R⁹ is -(C₃-C₆)cycloalkyl. In another embodiment is a compound of Formula (IIIb) wherein R⁹ is cyclopropyl. In another embodiment is a compound of Formula (IIIb) wherein R⁹ is H.

In another embodiment is a compound of Formula (IIIb) wherein R¹¹ is -(C₁-C₆)alkyl. In another embodiment is a compound of Formula (IIIb) wherein R¹¹ is -CH₃. In another embodiment is a compound of Formula (IIIb) wherein R¹¹ is -(C₁-C₆)alkyl-OR²³. In another embodiment is a compound of Formula (IIIb) wherein R¹¹ is -CH₂OH. In another embodiment is a compound of Formula (IIIb) wherein R¹¹ is - CH₂CH₂OH. In another embodiment is a compound of Formula (IIIb) wherein R¹¹ is -(C₁-C₆)alkyl. In another embodiment is a compound of Formula (IIIb) wherein R¹¹ is -(C₁-C₆)alkyl-NR²¹R²². In another embodiment is a compound of Formula (IIIb) wherein R¹¹ is -(C₁-C₆)alkyl-NH₂. In another embodiment is a compound of Formula (IIIb) wherein R¹¹ is -CH₂NH₂. In another embodiment is a compound of Formula (IIIb) wherein R¹¹ is -CH₂CH₂NH₂. In another embodiment is a compound of Formula (IIIb) wherein R¹¹ is -CH₂CH₂CH₂NH₂. In another embodiment is a compound of Formula (IIIb) wherein R¹¹ is - CH₂CH₂CH₂CH₂NH₂. In another embodiment is a compound of Formula (IIIb) wherein R¹¹ is -(C₁-C₆)alkyl-CN. In another embodiment is a compound of Formula (IIIb) wherein R¹¹ is -CH₂CN. In another embodiment is a compound of Formula (IIIb) wherein R¹¹ is -(C₁-C₆)alkyl-C(O)NR²⁵R²⁶. In another embodiment is a compound of Formula (IIIb) wherein R¹¹ is -CH₂C(O)NH₂. In another embodiment is a compound of Formula (IIIb) wherein R¹¹ is -CH₂CH₂C(O)NH₂. In another embodiment is a compound of Formula (IIIb) wherein R¹¹ is -(C₁-C₆)alkyl-heteroaryl. In another embodiment is a compound of Formula (IIIb) wherein R¹¹ is -(C₁-C₆)alkyl-N(H)S(O)₂NR²⁵R²⁶. In another embodiment is a compound of Formula (IIIb) wherein R¹¹ is -CH₂N(H)S(O)₂NH₂. In another embodiment is a compound of Formula (IIIb) wherein R¹¹ is H.

In another embodiment is a compound of Formula (IIIb) wherein R¹¹ and R¹⁸ are combined to form an optionally substituted heterocycloalkyl ring.

In another embodiment is a compound of Formula (IIIb) wherein R¹ and R² are each independently H or -(C₁-C₆)alkyl-NR²¹R²². In another embodiment is a compound of Formula (IIIb) wherein R¹ and R² are each H. In another embodiment is a compound of Formula (IIIb) wherein R¹ and R² are each independently -(C₁-C₆)alkyl-NR²¹R²². In another embodiment is a compound of Formula (IIIb) wherein R¹ is H, and R² is -(C₁-C₆)alkyl-NR²¹R²². In another embodiment is a compound of Formula (IIIb) wherein R¹ is -(C₁-C₆)alkyl-NR²¹R²², and R² is H. In another embodiment is a compound of Formula (IIIb) wherein R¹ is H, and R² is -CH₂CH₂NH₂. In another embodiment is a compound of Formula (IIIb) wherein R¹ is -CH₂CH₂NH₂, and R² is H. In another embodiment is a compound of Formula (IIIb) wherein R¹ and R² are each -CH₂CH₂NH₂. In a further embodiment is a compound of Formula (IIIb) wherein R¹ is -(C₁-C₆)alkyl-NR²¹R²² and R² is H. In a further embodiment is a compound of Formula (IIIb) wherein R¹ is - CH₂CH₂NH₂ and R² is H. In a further embodiment is a compound of Formula (IIIb) wherein R¹ is H and R² is -(C₁-C₆)alkyl-NR²¹R²². In a further embodiment is a compound of Formula (IIIb) wherein R¹ is H and R² is -CH₂CH₂NH₂. In another embodiment is a compound of Formula (IIIb) wherein R¹ and R² are each independently H, -(C₁-C₆)alkyl-NR²¹R²², or -CH₂CH(OH)CH₂NH₂. In another embodiment is a compound of Formula (IIIb) wherein R¹ and R² are each independently -CH₂CH(OH)CH₂NH₂. In another embodiment is a compound of Formula (IIIb) wherein R¹ is H, and R² is -CH₂CH(OH)CH₂NH₂. In another embodiment is a compound of Formula (IIIb) wherein R¹ is -CH₂CH(OH)CH₂NH₂, and R² is H. In a further embodiment is a compound of Formula (IIIb) wherein R¹ and R² and the atoms to which they are attached form an optionally substituted heterocycloalkyl ring.

In another embodiment is a compound of Formula (IIIb) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (IIIb) wherein X is heteroaryl disubstituted with substituents each independently selected from -(C₁-C₆)alkyl. In a further embodiment is a compound of Formula (IIIb) wherein X is heteroaryl disubstituted with methyl. In a further embodiment is a compound of Formula (IIIb) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IIIb) wherein X is pyridinyl disubstituted with substituents each independently selected from -(C₁-C₆)alkyl. In a further embodiment is a compound of Formula (IIIb) wherein X is pyridinyl disubstituted with methyl. In a further embodiment is a compound of Formula (IIIb) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IIIb) wherein X is pyrimidinyl disubstituted with substituents each independently selected from -(C₁-C₆)alkyl. In a further embodiment is a compound of Formula (IIIb) wherein X is pyrimidinyl disubstituted with methyl.

In another embodiment is a compound of Formula (IIIb) wherein Y is optionally substituted aryl. In a further embodiment is a compound of Formula (IIIb) wherein Y is optionally substituted phenyl. In another embodiment is a compound of Formula (IIIb) wherein Y is optionally substituted heteroaryl. In another embodiment is a compound of Formula (IIIb) wherein Y is optionally substituted -(C₁-C₆)alkyl-. In another embodiment is a compound of Formula (IIIb) wherein Y is optionally substituted (C₃-C₇)cycloalkyl-. In another embodiment is a compound of Formula (IIIb) wherein Y is optionally substituted heterocycloalkyl. In another embodiment is a compound of Formula (IIIb) wherein Y is -O-. In another embodiment is a compound of Formula (IIIb) wherein Y is -(C₂-C₆)alkynyl. In another embodiment is a compound of Formula (IIIb) wherein Y is -O-(C₁-C₆)alkyl-. In another embodiment is a compound of Formula (IIIb) wherein Y is a bond.

In another embodiment is a compound of Formula (IIIb) wherein Z is -(C₁₋C₁₂)alkyl. In a further embodiment is a compound of Formula (IIIb) wherein Z is n-butyl, isobutyl, or tert-butyl. In another embodiment is a compound of Formula (IIIb) wherein Z is -O-(C₁-C₁₂)alkyl. In another embodiment is a compound of Formula (IIIb) wherein Z is -O-(C₃-C₇)cycloalkyl. In another embodiment is a compound of Formula (IIIb) wherein Z is -(C₂-C₁₂)alkenyl. In another embodiment is a compound of Formula (IIIb) wherein Z is optionally substituted aryl. In a further embodiment is a compound of Formula (IIIb) wherein Z is optionally substituted phenyl. In a further embodiment is a compound of Formula (IIIb) wherein Z is phenyl monsubstituted or disubstituted with a substituent independently selected from -(C₁-C₈)alkyl. In a further embodiment is a compound of Formula (IIIb) wherein Z is phenyl monosubstituted with n-butyl, isobutyl, or tert-butyl. In a further embodiment is a compound of Formula (IIIb) wherein Z is phenyl monosubstituted with n-butyl. In a further embodiment is a compound of Formula (IIIb) wherein Z is phenyl monosubstituted with isobutyl. In a further embodiment is a compound of Formula (IIIb) wherein Z is phenyl monosubstituted with tert-butyl. In another embodiment is a compound of Formula (IIIb) wherein Z is optionally substituted heteroaryl. In another embodiment is a compound of Formula (IIIb) wherein Z is optionally substituted -(C₃-C₇)cycloalkyl. In another embodiment is a compound of Formula (IIIb) wherein Z is optionally substituted heterocycloalkyl. In another embodiment is a compound of Formula (IIIb) wherein Z is halogen.

In another embodiment is a compound of Formula (IIIb) wherein Z-Y-X- is not

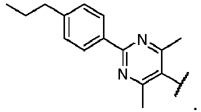

In another embodiment is a compound of Formula (III) having the structure of Formula (IIIc): 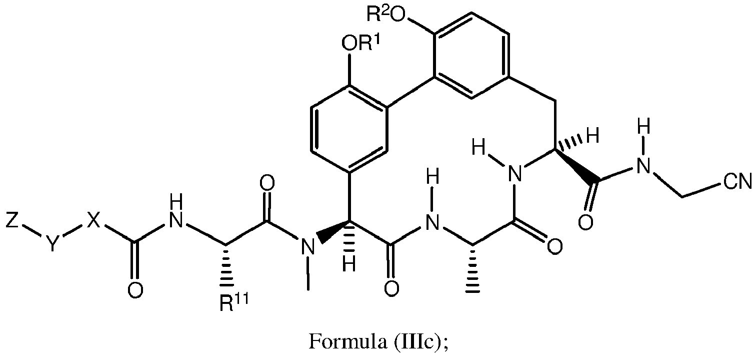 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²², - (C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring;
R¹¹ is H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
X is disubstituted heteroaryl;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring; and

or a pharmaceutically acceptable salt, solvate, or prodrug thereof.In another embodiment is a compound of Formula (IIIc) wherein R¹¹ is -(C₁-C₆)alkyl. In another embodiment is a compound of Formula (IIIc) wherein R¹¹ is -CH₃. In another embodiment is a compound of Formula (IIIc) wherein R¹¹ is -(C₁-C₆)alkyl-OR²³. In another embodiment is a compound of Formula (IIIc) wherein R¹¹ is -CH₂OH. In another embodiment is a compound of Formula (IIIc) wherein R¹¹ is -CH₂CH₂OH. In another embodiment is a compound of Formula (IIIc) wherein R¹¹ is -(C₁-C₆)alkyl. In another embodiment is a compound of Formula (IIIc) wherein R¹¹ is -(C₁-C₆)alkyl-NR²¹R²². In another embodiment is a compound of Formula (IIIc) wherein R¹¹ is -(C₁-C₆)alkyl-NH₂. In another embodiment is a compound of Formula (IIIc) wherein R¹¹ is -CH₂NH₂. In another embodiment is a compound of Formula (IIIc) wherein R¹¹ is -CH₂CH₂NH₂. In another embodiment is a compound of Formula (IIIc) wherein R¹¹ is -CH₂CH₂CH₂NH₂. In another embodiment is a compound of Formula (IIIc) wherein R¹¹ is -CH₂CH₂CH₂CH₂NH₂. In another embodiment is a compound of Formula (IIIc) wherein R¹¹ is -(C₁-C₆)alkyl-CN. In another embodiment is a compound of Formula (IIIc) wherein R¹¹ is -CH₂CN. In another embodiment is a compound of Formula (IIIc) wherein R¹¹ is -(C₁-C₆)alkyl-C(O)NR²⁵R²⁶. In another embodiment is a compound of Formula (IIIc) wherein R¹¹ is -CH₂C(O)NH₂. In another embodiment is a compound of Formula (IIIc) wherein R¹¹ is - CH₂CH₂C(O)NH₂. In another embodiment is a compound of Formula (IIIc) wherein R¹¹ is -(C₁-C₆)alkyl-heteroaryl. In another embodiment is a compound of Formula (IIIc) wherein R¹¹ is -(C₁-C₆)alkyl-N(H)S(O)₂NR²⁵R²⁶. In another embodiment is a compound of Formula (IIIc) wherein R¹¹ is - CH₂N(H)S(O)₂NH₂. In another embodiment is a compound of Formula (IIIc) wherein R¹¹ is H.

In another embodiment is a compound of Formula (IIIc) wherein R¹ and R² are each independently H or -(C₁-C₆)alkyl-NR²¹R²². In another embodiment is a compound of Formula (IIIc) wherein R¹ and R² are each H. In another embodiment is a compound of Formula (IIIc) wherein R¹ and R² are each independently -(C₁-C₆)alkyl-NR²¹R²². In another embodiment is a compound of Formula (IIIc) wherein R¹ is H, and R² is -(C₁-C₆)alkyl-NR²¹R²². In another embodiment is a compound of Formula (IIIc) wherein R¹ is -(C₁-C₆)alkyl-NR²¹R²², and R² is H. In another embodiment is a compound of Formula (IIIc) wherein R¹ is H, and R² is -CH₂CH₂NH₂. In another embodiment is a compound of Formula (IIIc) wherein R¹ is -CH₂CH₂NH₂, and R² is H. In another embodiment is a compound of Formula (IIIc) wherein R¹ and R² are each -CH₂CH₂NH₂. In a further embodiment is a compound of Formula (IIIc) wherein R¹ is -(C₁-C₆)alkyl-NR²¹R²² and R² is H. In a further embodiment is a compound of Formula (IIIc) wherein R¹ is - CH₂CH₂NH₂ and R² is H. In a further embodiment is a compound of Formula (IIIc) wherein R¹ is H and R² is -(C₁-C₆)alkyl-NR²¹R²². In a further embodiment is a compound of Formula (IIIc) wherein R¹ is H and R² is -CH₂CH₂NH₂. In another embodiment is a compound of Formula (IIIc) wherein R¹ and R² are each independently H, -(C₁-C₆)alkyl-NR²¹R²², or -CH₂CH(OH)CH₂NH₂. In another embodiment is a compound of Formula (IIIc) wherein R¹ and R² are each independently -CH₂CH(OH)CH₂NH₂. In another embodiment is a compound of Formula (IIIc) wherein R¹ is H, and R² is -CH₂CH(OH)CH₂NH₂. In another embodiment is a compound of Formula (IIIc) wherein R¹ is -CH₂CH(OH)CH₂NH₂, and R² is H. In a further embodiment is a compound of Formula (IIIc) wherein R¹ and R² and the atoms to which they are attached form an optionally substituted heterocycloalkyl ring.

In another embodiment is a compound of Formula (IIIc) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (IIIc) wherein X is heteroaryl disubstituted with substituents each independently selected from -(C₁-C₆)alkyl. In a further embodiment is a compound of Formula (IIIc) wherein X is heteroaryl disubstituted with methyl. In a further embodiment is a compound of Formula (IIIc) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IIIc) wherein X is pyridinyl disubstituted with substituents each independently selected from -(C₁-C₆)alkyl. In a further embodiment is a compound of Formula (IIIc) wherein X is pyridinyl disubstituted with methyl. In a further embodiment is a compound of Formula (IIIc) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IIIc) wherein X is pyrimidinyl disubstituted with substituents each independently selected from -(C₁-C₆)alkyl. In a further embodiment is a compound of Formula (IIIc) wherein X is pyrimidinyl disubstituted with methyl.

In another embodiment is a compound of Formula (IIIc) wherein Y is optionally substituted aryl. In a further embodiment is a compound of Formula (IIIc) wherein Y is optionally substituted phenyl. In another embodiment is a compound of Formula (IIIc) wherein Y is optionally substituted heteroaryl. In another embodiment is a compound of Formula (IIIc) wherein Y is optionally substituted -(C₁-C₆)alkyl-. In another embodiment is a compound of Formula (IIIc) wherein Y is optionally substituted (C₃-C₇)cycloalkyl-. In another embodiment is a compound of Formula (IIIc) wherein Y is optionally substituted heterocycloalkyl. In another embodiment is a compound of Formula (IIIc) wherein Y is -O-. In another embodiment is a compound of Formula (IIIc) wherein Y is -(C₂-C₆)alkynyl. In another embodiment is a compound of Formula (IIIc) wherein Y is -O-(C₁-C₆)alkyl-. In another embodiment is a compound of Formula (IIIc) wherein Y is a bond.

In another embodiment is a compound of Formula (IIIc) wherein Z is -(C₁-C₁₂)alkyl. In a further embodiment is a compound of Formula (IIIc) wherein Z is n-butyl, isobutyl, or tert-butyl. In another embodiment is a compound of Formula (IIIc) wherein Z is -O-(C₁-C₁₂)alkyl. In another embodiment is a compound of Formula (IIIc) wherein Z is -O-(C₃-C₇)cycloalkyl. In another embodiment is a compound of Formula (IIIc) wherein Z is -(C₂-C₁₂)alkenyl. In another embodiment is a compound of Formula (IIIc) wherein Z is optionally substituted aryl. In a further embodiment is a compound of Formula (IIIc) wherein Z is optionally substituted phenyl. In a further embodiment is a compound of Formula (IIIc) wherein Z is phenyl monsubstituted or disubstituted with a substituent independently selected from -(C₁-C₈)alkyl. In a further embodiment is a compound of Formula (IIIc) wherein Z is phenyl monosubstituted with n-butyl, isobutyl, or tert-butyl. In a further embodiment is a compound of Formula (IIIc) wherein Z is phenyl monosubstituted with n-butyl. In a further embodiment is a compound of Formula (IIIc) wherein Z is phenyl monosubstituted with isobutyl. In a further embodiment is a compound of Formula (IIIc) wherein Z is phenyl monosubstituted with tert-butyl. In another embodiment is a compound of Formula (IIIc) wherein Z is optionally substituted heteroaryl. In another embodiment is a compound of Formula (IIIc) wherein Z is optionally substituted -(C₃-C₇)cycloalkyl. In another embodiment is a compound of Formula (IIIc) wherein Z is optionally substituted heterocycloalkyl. In another embodiment is a compound of Formula (IIIc) wherein Z is halogen.

In another embodiment is a compound of Formula (IIIc) wherein Z-Y-X- is not

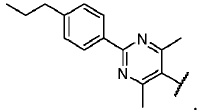

In one aspect described herein are compounds of Formula (IV): 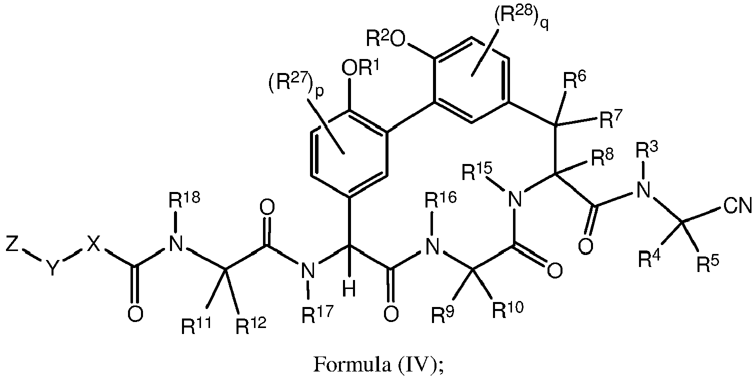 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²², - (C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²₂, -(C₁-C₆)heteroalkyl, or optionally substituted heterocycloalkyl;
or R¹ and R² and the atoms to which they are attached form an optionally substituted heterocycloalkyl ring;
R³ is H or -(C₁-C₆)alkyl;
R⁴ is H, -(C₁-C₆)alkyl, -(C₁-C₆)alkyl-OH, -(C₃-C₆)cycloalkyl, or -C(O)NH₂;
or R³ and R⁴ are combined to form a heterocycloalkyl ring;
R⁵ is H or -(C₁-C₆)alkyl;
or R⁴ and R⁵ and the carbon atom to which they are attached form a cyclopropyl ring;
R⁶, R⁷, and R⁸ are each independently H, fluoro, hydroxyl, amino, optionally substituted alkyl, optionally substituted heteroalkyl, or -(C₁-C₆)alkyl;
R⁹ is H, -(C₁-C₆)alkyl, -(C₁-C₆)haloalkyl, or -(C₃-C₆)cycloalkyl;
R¹⁰ is H, -(C₁-C₆)alkyl, -(C₁-C₆)haloalkyl, or -(C₃-C₆)cycloalkyl;
or R⁹ and R¹⁰ are combined to form a heterocycloalkyl or cycloalkyl ring
R¹¹ and R¹² are each independently H or -(C₁-C₆)alkyl-N(H)S(O)₂NR²⁵R²⁶;
R¹⁵, R¹⁶, R¹⁷, and R¹⁸ are each independently H, -(C₁-C₆)alkyl, -(C₃-C₆)cycloalkyl, -(C₁-C₆)alkyl-OR²³, - (C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring;
each R²⁷ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R¹ and R²⁷ and the atoms to which they are attached form an optionally substituted 5- or 6-membered heterocycloalkyl ring;
each R²⁸ is independently halogen, -NR²³R²⁴, -NHC(O)R²³, -NC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R² and R²⁸ and the atoms to which they are attached form an optionally substituted 5- or 6-membered heterocycloalkyl ring;
p is 0, 1, or 2; and
q is 0, 1, or 2;
or a pharmaceutically acceptable salt, solvate, or prodrug thereof.

In one embodiment is a compound of Formula (IV) wherein R⁶, R⁷, and R⁸ are H.

In another embodiment is a compound of Formula (IV) wherein R¹⁵ and R¹⁶ are H.

In one embodiment is a compound of Formula (IV) wherein R¹⁷ is -(C₁-C₆)alkyl. In another embodiment is a compound of Formula (IV) wherein R¹⁷ is -CH₃. In another embodiment is a compound of Formula (IV) wherein R¹⁷ is -CH₂CH₃. In another embodiment is a compound of Formula (IV) wherein R¹⁷ is -(C₃-C₆)cycloalkyl. In another embodiment is a compound of Formula (IV) wherein R¹⁷ is cyclopropyl. In another embodiment is a compound of Formula (IV) wherein R¹⁷ is -(C₁-C₆)alkyl-C(O)OR²³. In another embodiment is a compound of Formula (IV) wherein R¹⁷ is -CH₂CH₂OH. In another embodiment is a compound of Formula (IV) wherein R¹⁷ is -(C₁-C₆)alkyl-NR²¹R²². In another embodiment is a compound of Formula (IV) wherein R¹⁷ is -CH₂CH₂NH₂. In another embodiment is a compound of Formula (IV) wherein R¹⁷ is H.

In another embodiment is a compound of Formula (IV) wherein R¹⁸ is H.

In another embodiment is a compound of Formula (IV) wherein R³ is H.

In another embodiment is a compound of Formula (IV) wherein R⁵ is H.

In another embodiment is a compound of Formula (IV) wherein R⁴ is H. In another embodiment is a compound of Formula (IV) wherein R⁴ is -(C₁-C₆)alkyl. In another embodiment is a compound of Formula (IV) wherein R⁴ is -CH₃. In another embodiment is a compound of Formula (IV) wherein R⁴ is - CH₂CH₃. In another embodiment is a compound of Formula (IV) wherein R⁴ is -(C₁-C₆)alkyl-OH. In another embodiment is a compound of Formula (IV) wherein R⁴ is -CH₂OH. In another embodiment is a compound of Formula (IV) wherein R⁴ is -(C₃-C₆)cycloalkyl. In another embodiment is a compound of Formula (IV) wherein R⁴ is cyclopropyl. In another embodiment is a compound of Formula (IV) wherein R⁴ is -C(O)NH₂.

In another embodiment is a compound of Formula (IV) wherein R³, R⁴, and R⁵ are H.

In another embodiment is a compound of Formula (IV) wherein R⁴ and R⁵ and the carbon atom to which they are attached form a cyclopropyl ring.

In another embodiment is a compound of Formula (IV) wherein R¹⁰ is H.

In another embodiment is a compound of Formula (IV) wherein R¹⁰ is H and R⁹ is -(C₁-C₆)alkyl. In another embodiment is a compound of Formula (IV) wherein R¹⁰ is H and R⁹ is -CH₃. In another embodiment is a compound of Formula (IV) wherein R¹⁰ is H and R⁹ is -CH₂CH₃. In another embodiment is a compound of Formula (IV) wherein R¹⁰ is H and R⁹ is -(C₁-C₆)haloalkyl. In another embodiment is a compound of Formula (IV) wherein R¹⁰ is H and R⁹ is -CH₂F. In another embodiment is a compound of Formula (IV) wherein R¹⁰ is H and R⁹ is -CHF₂. In another embodiment is a compound of Formula (IV) wherein R¹⁰ is H and R⁹ is -(C₃-C₆)cycloalkyl. In another embodiment is a compound of Formula (IV) wherein R¹⁰ is H and R⁹ is cyclopropyl. In another embodiment is a compound of Formula (IV) wherein R¹⁰ is H and R⁹ is H.

In another embodiment is a compound of Formula (IV) wherein R¹² is H.

In another embodiment is a compound of Formula (IV) wherein R¹² is H and R¹¹ is -(C₁-C₆)alkyl-N(H)S(O)₂NR²⁵R²⁶. In another embodiment is a compound of Formula (IV) wherein R¹² is H and R¹¹ is -CH₂N(H)S(O)₂NH₂.

In another embodiment is a compound of Formula (IV) wherein p is 1 and R²⁷ is halogen. In another embodiment is a compound of Formula (IV) wherein p is 1 and R²⁷ is optionally substituted -(C₁-C₆)alkyl. In another embodiment is a compound of Formula (IV) wherein q is 0, p is 1 and R²⁷ is halogen. In another embodiment is a compound of Formula (IV) wherein q is 0, p is 1 and R²⁷ is optionally substituted -(C₁-C₆)alkyl. In another embodiment is a compound of Formula (IV) wherein q is 1 and R²⁸ is halogen. In another embodiment is a compound of Formula (IV) wherein q is 1 and R²⁸ is optionally substituted -(C₁-C₆)alkyl. In another embodiment is a compound of Formula (IV) wherein p is 0, q is 1 and R²⁸ is halogen. In another embodiment is a compound of Formula (IV) wherein p is 0, q is 1 and R²⁸ is optionally substituted -(C₁-C₆)alkyl.

In another embodiment is a compound of Formula (IV) wherein p is 0, and q is 0.

In another embodiment is a compound of Formula (IV) wherein R¹ and R² are each independently H or -(C₁-C₆)alkyl-NR²¹R²². In another embodiment is a compound of Formula (IV) wherein R¹ and R² are each H. In another embodiment is a compound of Formula (IV) wherein R¹ and R² are each independently -(C₁-C₆)alkyl-NR²¹R²². In another embodiment is a compound of Formula (IV) wherein R¹ is H, and R² is -(C₁-C₆)alkyl-NR²¹R²². In another embodiment is a compound of Formula (IV) wherein R¹ is -(C₁-C₆)alkyl-NR²¹R²², and R² is H. In another embodiment is a compound of Formula (IV) wherein R¹ is H, and R² is -CH₂CH₂NH₂. In another embodiment is a compound of Formula (IV) wherein R¹ is -CH₂CH₂NH₂, and R² is H. In another embodiment is a compound of Formula (IV) wherein R¹ and R² are each -CH₂CH₂NH₂. In a further embodiment is a compound of Formula (IV) wherein R¹ is -(C₁-C₆)alkyl-NR²¹R²² and R² is H. In a further embodiment is a compound of Formula (IV) wherein R¹ is - CH₂CH₂NH₂ and R² is H. In a further embodiment is a compound of Formula (IV) wherein R¹ is H and R² is -(C₁-C₆)alkyl-NR²¹R²². In a further embodiment is a compound of Formula (IV) wherein R¹ is H and R² is -CH₂CH₂NH₂. In another embodiment is a compound of Formula (IV) wherein R¹ and R² are each independently H, -(C₁-C₆)alkyl-NR²¹R²², or -CH₂CH(OH)CH₂NH₂. In another embodiment is a compound of Formula (IV) wherein R¹ and R² are each independently -CH₂CH(OH)CH₂NH₂. In another embodiment is a compound of Formula (IV) wherein R¹ is H, and R² is -CH₂CH(OH)CH₂NH₂. In another embodiment is a compound of Formula (IV) wherein R¹ is -CH₂CH(OH)CH₂NH₂, and R² is H. In a further embodiment is a compound of Formula (IV) wherein R¹ and R² and the atoms to which they are attached form an optionally substituted heterocycloalkyl ring.

In another embodiment is a compound of Formula (IV) wherein X is optionally substituted aryl. In a further embodiment is a compound of Formula (IV) wherein X is optionally substituted phenyl. In another embodiment is a compound of Formula (IV) wherein X is optionally substituted heteroaryl. In a further embodiment is a compound of Formula (IV) wherein X is disubstituted heteroaryl. In a further embodiment is a compound of Formula (IV) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (IV) wherein X is heteroaryl disubstituted with substituents each independently selected from -(C₁-C₆)alkyl. In a further embodiment is a compound of Formula (IV) wherein X is heteroaryl disubstituted with methyl. In a further embodiment is a compound of Formula (IV) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IV) wherein X is pyridinyl disubstituted with substituents each independently selected from -(C₁-C₆)alkyl. In a further embodiment is a compound of Formula (IV) wherein X is pyridinyl disubstituted with methyl. In a further embodiment is a compound of Formula (IV) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IV) wherein X is pyrimidinyl disubstituted with substituents each independently selected from -(C₁-C₆)alkyl. In a further embodiment is a compound of Formula (IV) wherein X is pyrimidinyl disubstituted with methyl. In another embodiment is a compound of Formula (IV) wherein X is optionally substituted -(C₁-C₆)alkyl-.

In another embodiment is a compound of Formula (IV) wherein Y is optionally substituted aryl. In a further embodiment is a compound of Formula (IV) wherein Y is optionally substituted phenyl. In another embodiment is a compound of Formula (IV) wherein Y is optionally substituted heteroaryl. In another embodiment is a compound of Formula (IV) wherein Y is optionally substituted -(C₁-C₆)alkyl-. In another embodiment is a compound of Formula (IV) wherein Y is optionally substituted (C₃-C₇)cycloalkyl-. In another embodiment is a compound of Formula (IV) wherein Y is optionally substituted heterocycloalkyl. In another embodiment is a compound of Formula (IV) wherein Y is -O-. In another embodiment is a compound of Formula (IV) wherein Y is -(C₂-C₆)alkynyl. In another embodiment is a compound of Formula (IV) wherein Y is -O-(C₁-C₆)alkyl-. In another embodiment is a compound of Formula (IV) wherein Y is a bond.

In another embodiment is a compound of Formula (IV) wherein Z is -(C₁-C₁₂)alkyl. In a further embodiment is a compound of Formula (IV) wherein Z is n-butyl, isobutyl, or tert-butyl. In another embodiment is a compound of Formula (IV) wherein Z is -O-(C₁-C₁₂)alkyl. In another embodiment is a compound of Formula (IV) wherein Z is -O-(C₃-C₇)cycloalkyl. In another embodiment is a compound of Formula (IV) wherein Z is -(C₂-C₁₂)alkenyl. In another embodiment is a compound of Formula (IV) wherein Z is optionally substituted aryl. In a further embodiment is a compound of Formula (IV) wherein Z is optionally substituted phenyl. In a further embodiment is a compound of Formula (IV) wherein Z is phenyl monsubstituted or disubstituted with a substituent independently selected from -(C₁-C₈)alkyl. In a further embodiment is a compound of Formula (IV) wherein Z is phenyl monosubstituted with n-butyl, isobutyl, or tert-butyl. In a further embodiment is a compound of Formula (IV) wherein Z is phenyl monosubstituted with n-butyl. In a further embodiment is a compound of Formula (IV) wherein Z is phenyl monosubstituted with isobutyl. In a further embodiment is a compound of Formula (IV) wherein Z is phenyl monosubstituted with tert-butyl. In another embodiment is a compound of Formula (IV) wherein Z is optionally substituted heteroaryl. In another embodiment is a compound of Formula (IV) wherein Z is optionally substituted -(C₃-C₇)cycloalkyl. In another embodiment is a compound of Formula (IV) wherein Z is optionally substituted heterocycloalkyl. In another embodiment is a compound of Formula (IV) wherein Z is halogen.

In another embodiment is a compound of Formula (IV) having the structure of Formula (IVa): 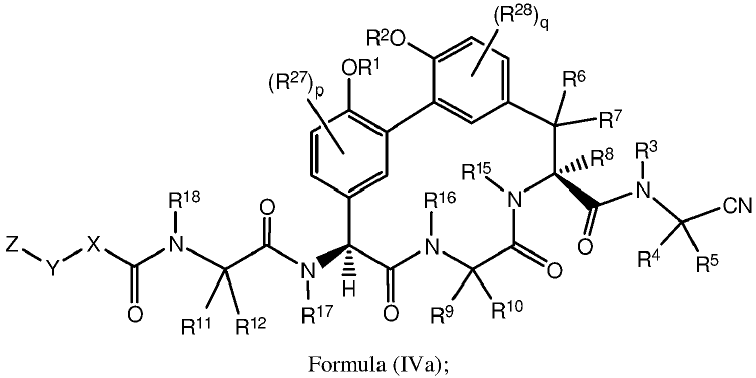 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-CO0)NR²⁵R²⁶, -(C₁-C₆)alkyl-N(R²³)C(O)(C₁-C₆)alkylNR²¹R²², - (C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring;
R³ is H or -(C₁-C₆)alkyl;
R⁴ is H, -(C₁-C₆)alkyl, -(C₁-C₆)alkyl-OH, -(C₃-C₆)cycloalkyl, or -C(O)NH₂;
or R³ and R⁴ are combined to form a heterocycloalkyl ring;
R⁵ is H or -(C₁-C₆)alkyl;
or R⁴ and R⁵ and the carbon atom to which they are attached form a cyclopropyl ring;
R⁶, R⁷, and R⁸ are each independently H, fluoro, hydroxyl, amino, optionally substituted alkyl, heteroalkyl, or -(C₁-C₆)alkyl;
R⁹ is H, -(C₁-C₆)alkyl, -(C₁-C₆)haloalkyl, or -(C₃-C₆)cycloalkyl;
R¹⁰ is H, -(C₁-C₆)alkyl, -(C₁-C₆)haloalkyl, or -(C₃-C₆)cycloalkyl;
or R⁹ and R¹⁰ are combined to form a heterocycloalkyl or cycloalkyl ring
R¹¹ and R¹² are each independently H and -(C₁-C₆)alkyl-N(H)S(O)₂NR²⁵R²⁶;
R¹⁵, R¹⁶, R¹⁷, and R¹⁸ are each independently H, -(C₁-C₆)alkyl, -(C₃-C₆)cycloalkyl, -(C₁-C₆)alkyl-OR²³, - (C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring;
each R²⁷ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R¹ and R²⁷ and the atoms to which they are attached form an optionally substituted 5- or 6-membered heterocycloalkyl ring;
each R²⁸ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R² and R²⁸ and the atoms to which they are attached form an optionally substituted 5- or 6-membered heterocycloalkyl ring;
p is 0, 1, or 2; and
q is 0, 1, or 2;

or a pharmaceutically acceptable salt, solvate, or prodrug thereof.In one embodiment is a compound of Formula (IVa) wherein R⁶, R⁷, and R⁸ are H.

In another embodiment is a compound of Formula (IVa) wherein R¹⁵ and R¹⁶ are H.

In one embodiment is a compound of Formula (IVa) wherein R¹⁷ is -(C₁-C₆)alkyl. In another embodiment is a compound of Formula (IVa) wherein R¹⁷ is -CH₃. In another embodiment is a compound of Formula (IVa) wherein R¹⁷ is -CH₂CH₃. In another embodiment is a compound of Formula (IVa) wherein R¹⁷ is -(C₃-C₆)cycloalkyl. In another embodiment is a compound of Formula (IVa) wherein R¹⁷ is cyclopropyl. In another embodiment is a compound of Formula (IVa) wherein R¹⁷ is -(C₁-C₆)alkyl-C(O)OR²³. In another embodiment is a compound of Formula (IVa) wherein R¹⁷ is -CH₂CH₂OH. In another embodiment is a compound of Formula (IVa) wherein R¹⁷ is -(C₁-C₆)alkyl-NR²¹R²². In another embodiment is a compound of Formula (IVa) wherein R¹⁷ is -CH₂CH₂NH₂. In another embodiment is a compound of Formula (IVa) wherein R¹⁷ is H.

In another embodiment is a compound of Formula (IVa) wherein R¹⁸ is H.

In another embodiment is a compound of Formula (IVa) wherein R³ is H.

In another embodiment is a compound of Formula (IVa) wherein R⁵ is H.

In another embodiment is a compound of Formula (IVa) wherein R⁴ is H. In another embodiment is a compound of Formula (IVa) wherein R⁴ is -(C₁-C₆)alkyl. In another embodiment is a compound of Formula (IVa) wherein R⁴ is -CH₃. In another embodiment is a compound of Formula (IVa) wherein R⁴ is -CH₂CH₃. In another embodiment is a compound of Formula (IVa) wherein R⁴ is -(C₁-C₆)alkyl-OH. In another embodiment is a compound of Formula (IVa) wherein R⁴ is -CH₂OH. In another embodiment is a compound of Formula (IVa) wherein R⁴ is -(C₃-C₆)cycloalkyl. In another embodiment is a compound of Formula (IVa) wherein R⁴ is cyclopropyl. In another embodiment is a compound of Formula (IVa) wherein R⁴ is -C(O)NH₂.

In another embodiment is a compound of Formula (IVa) wherein R⁴ and R⁵ and the carbon atom to which they are attached form a cyclopropyl ring.

In another embodiment is a compound of Formula (IVa) wherein R³, R⁴, and R⁵ are H.

In another embodiment is a compound of Formula (IVa) wherein R¹⁰ is H.

In another embodiment is a compound of Formula (IVa) wherein R¹⁰ is H and R⁹ is -(C₁-C₆)alkyl. In another embodiment is a compound of Formula (IVa) wherein R¹⁰ is H and R⁹ is -CH₃. In another embodiment is a compound of Formula (IVa) wherein R¹⁰ is H and R⁹ is -CH₂CH₃. In another embodiment is a compound of Formula (IVa) wherein R¹⁰ is H and R⁹ is -(C₁-C₆)haloalkyl. In another embodiment is a compound of Formula (IVa) wherein R¹⁰ is H and R⁹ is -CH₂F. In another embodiment is a compound of Formula (IVa) wherein R¹⁰ is H and R⁹ is -CHF₂. In another embodiment is a compound of Formula (IVa) wherein R¹⁰ is H and R⁹ is -(C₃-C₆)cycloalkyl. In another embodiment is a compound of Formula (IVa) wherein R¹⁰ is H and R⁹ is cyclopropyl. In another embodiment is a compound of Formula (IVa) wherein R¹⁰ is H and R⁹ is H.

In another embodiment is a compound of Formula (IVa) wherein R¹² is H.

In another embodiment is a compound of Formula (IVa) wherein R¹² is H and R¹¹ is -(C₁-C₆)alkyl-N(H)S(O)₂NR²⁵R²⁶. In another embodiment is a compound of Formula (IVa) wherein R¹² is H and R¹¹ is -CH₂N(H)S(O)₂NH₂.

In another embodiment is a compound of Formula (IVa) wherein p is 1 and R²⁷ is halogen. In another embodiment is a compound of Formula (IVa) wherein p is 1 and R²⁷ is optionally substituted -(C₁-C₆)alkyl. In another embodiment is a compound of Formula (IVa) wherein q is 0, p is 1 and R²⁷ is halogen. In another embodiment is a compound of Formula (IVa) wherein q is 0, p is 1 and R²⁷ is optionally substituted -(C₁-C₆)alkyl. In another embodiment is a compound of Formula (IVa) wherein q is 1 and R²⁸ is halogen. In another embodiment is a compound of Formula (IVa) wherein q is 1 and R²⁸ is optionally substituted -(C₁-C₆)alkyl. In another embodiment is a compound of Formula (IVa) wherein p is 0, q is 1 and R²⁸ is halogen. In another embodiment is a compound of Formula (IVa) wherein p is 0, q is 1 and R²⁸ is optionally substituted -(C₁-C₆)alkyl.

In another embodiment is a compound of Formula (IVa) wherein p is 0, and q is 0.

In another embodiment is a compound of Formula (IVa) wherein R¹ and R² are each independently H or -(C₁-C₆)alkyl-NR²¹R²². In another embodiment is a compound of Formula (IVa) wherein R¹ and R² are each H. In another embodiment is a compound of Formula (IVa) wherein R¹ and R² are each independently -(C₁-C₆)alkyl-NR²¹R²². In another embodiment is a compound of Formula (IVa) wherein R¹ is H, and R² is -(C₁-C₆)alkyl-NR²¹R²². In another embodiment is a compound of Formula (IVa) wherein R¹ is -(C₁-C₆)alkyl-NR²¹R²², and R² is H. In another embodiment is a compound of Formula (IVa) wherein R¹ is H, and R² is -CH₂CH₂NH₂. In another embodiment is a compound of Formula (IVa) wherein R¹ is -CH₂CH₂NH₂, and R² is H. In another embodiment is a compound of Formula (IVa) wherein R¹ and R² are each -CH₂CH₂NH₂. In a further embodiment is a compound of Formula (IVa) wherein R¹ is -(C₁-C₆)alkyl-NR²¹R²² and R² is H. In a further embodiment is a compound of Formula (IVa) wherein R¹ is - CH₂CH₂NH₂ and R² is H. In a further embodiment is a compound of Formula (IVa) wherein R¹ is H and R² is -(C₁-C₆)alkyl-NR²¹R²². In a further embodiment is a compound of Formula (IVa) wherein R¹ is H and R² is -CH₂CH₂NH₂. In another embodiment is a compound of Formula (IVa) wherein R¹ and R² are each independently H, -(C₁-C₆)alkyl-NR²¹R²², or -CH₂CH(OH)CH₂NH₂. In another embodiment is a compound of Formula (IVa) wherein R¹ and R² are each independently -CH₂CH(OH)CH₂NH₂. In another embodiment is a compound of Formula (IVa) wherein R¹ is H, and R² is -CH₂CH(OH)CH₂NH₂. In another embodiment is a compound of Formula (IVa) wherein R¹ is -CH₂CH(OH)CH₂NH₂, and R² is H. In a further embodiment is a compound of Formula (IVa) wherein R¹ and R² and the atoms to which they are attached form an optionally substituted heterocycloalkyl ring.

In another embodiment is a compound of Formula (IVa) wherein X is optionally substituted aryl. In a further embodiment is a compound of Formula (IVa) wherein X is optionally substituted phenyl. In another embodiment is a compound of Formula (IVa) wherein X is optionally substituted heteroaryl. In a further embodiment is a compound of Formula (IVa) wherein X is disubstituted heteroaryl. In a further embodiment is a compound of Formula (IVa) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (IVa) wherein X is heteroaryl disubstituted with substituents each independently selected from -(C₁-C₆)alkyl. In a further embodiment is a compound of Formula (IVa) wherein X is heteroaryl disubstituted with methyl. In a further embodiment is a compound of Formula (IVa) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IVa) wherein X is pyridinyl disubstituted with substituents each independently selected from -(C₁-C₆)alkyl. In a further embodiment is a compound of Formula (IVa) wherein X is pyridinyl disubstituted with methyl. In a further embodiment is a compound of Formula (IVa) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IVa) wherein X is pyrimidinyl disubstituted with substituents each independently selected from -(C₁-C₆)alkyl. In a further embodiment is a compound of Formula (IVa) wherein X is pyrimidinyl disubstituted with methyl. In another embodiment is a compound of Formula (IVa) wherein X is optionally substituted -(C₁-C₆)alkyl-.

In another embodiment is a compound of Formula (IVa) wherein Y is optionally substituted aryl. In a further embodiment is a compound of Formula (IVa) wherein Y is optionally substituted phenyl. In another embodiment is a compound of Formula (IVa) wherein Y is optionally substituted heteroaryl. In another embodiment is a compound of Formula (IVa) wherein Y is optionally substituted -(C₁-C₆)alkyl-. In another embodiment is a compound of Formula (IVa) wherein Y is optionally substituted (C₃-C₇)cycloalkyl-. In another embodiment is a compound of Formula (IVa) wherein Y is optionally substituted heterocycloalkyl. In another embodiment is a compound of Formula (IVa) wherein Y is -O-. In another embodiment is a compound of Formula (IVa) wherein Y is -(C₂-C₆)alkynyl. In another embodiment is a compound of Formula (IVa) wherein Y is -O-(C₁-C₆)alkyl-. In another embodiment is a compound of Formula (IVa) wherein Y is a bond.

In another embodiment is a compound of Formula (IVa) wherein Z is -(C₁-C₁₂)alkyl. In a further embodiment is a compound of Formula (IVa) wherein Z is n-butyl, isobutyl, or tert-butyl. In another embodiment is a compound of Formula (IVa) wherein Z is -O-(C₁-C₁₂)alkyl. In another embodiment is a compound of Formula (IVa) wherein Z is -O-(C₃-C₇)cycloalkyl. In another embodiment is a compound of Formula (IVa) wherein Z is -(C₂-C₁₂)alkenyl. In another embodiment is a compound of Formula (IVa) wherein Z is optionally substituted aryl. In a further embodiment is a compound of Formula (IVa) wherein Z is optionally substituted phenyl. In a further embodiment is a compound of Formula (IVa) wherein Z is phenyl monsubstituted or disubstituted with a substituent independently selected from -(C₁-C₈)alkyl. In a further embodiment is a compound of Formula (IVa) wherein Z is phenyl monosubstituted with n-butyl, isobutyl, or tert-butyl. In a further embodiment is a compound of Formula (IVa) wherein Z is phenyl monosubstituted with n-butyl. In a further embodiment is a compound of Formula (IVa) wherein Z is phenyl monosubstituted with isobutyl. In a further embodiment is a compound of Formula (IVa) wherein Z is phenyl monosubstituted with tert-butyl. In another embodiment is a compound of Formula (IVa) wherein Z is optionally substituted heteroaryl. In another embodiment is a compound of Formula (IVa) wherein Z is optionally substituted -(C₃-C₇)cycloalkyl. In another embodiment is a compound of Formula (IVa) wherein Z is optionally substituted heterocycloalkyl. In another embodiment is a compound of Formula (IVa) wherein Z is halogen.

In another embodiment is a compound of Formula (IV) having the structure of Formula (IVb): 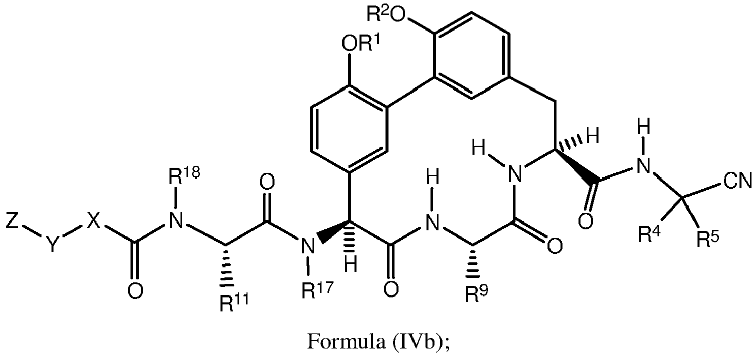 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring;
R⁴ is H, -(C₁-C₆)alkyl, -(C₁-C₆)alkyl-OH, -(C₃-C₆)cycloalkyl, or -C(O)NH₂;
R⁵ is H or -(C₁-C₆)alkyl;
or R⁴ and R⁵ and the carbon atom to which they are attached form a cyclopropyl ring;
R⁹ is H, -(C₁-C₆)alkyl, -(C₁-C₆)haloalkyl, or -(C₃-C₆)cycloalkyl;
R¹¹ is -(C₁-C₆)alkyl-N(H)S(O)₂NR²⁵R²⁶;
R¹⁷ and R¹⁸ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-Cₑ)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring; and

or a pharmaceutically acceptable salt, solvate, or prodrug thereof.In one embodiment is a compound of Formula (IVb) wherein R¹⁷ is -(C₁-C₆)alkyl. In another embodiment is a compound of Formula (IVb) wherein R¹⁷ is -CH₃. In another embodiment is a compound of Formula (IVb) wherein R¹⁷ is -CH₂CH₃. In another embodiment is a compound of Formula (IVb) wherein R¹⁷ is -(C₃-C₆)cycloalkyl. In another embodiment is a compound of Formula (IVb) wherein R¹⁷ is cyclopropyl. In another embodiment is a compound of Formula (IVb) wherein R¹⁷ is -(C₁-C₆)alkyl-C(O)OR²³. In another embodiment is a compound of Formula (IVb) wherein R¹⁷ is -CH₂CH₂OH. In another embodiment is a compound of Formula (IVb) wherein R¹⁷ is -(C₁-C₆)alkyl-NR²¹R²². In another embodiment is a compound of Formula (IVb) wherein R¹⁷ is -CH₂CH₂NH₂. In another embodiment is a compound of Formula (IVb) wherein R¹⁷ is H.

In another embodiment is a compound of Formula (IVb) wherein R¹⁸ is H.

In another embodiment is a compound of Formula (IVb) wherein R⁵ is H.

In another embodiment is a compound of Formula (IVb) wherein R⁴ is H. In another embodiment is a compound of Formula (IVb) wherein R⁴ is -(C₁-C₆)alkyl. In another embodiment is a compound of Formula (IVb) wherein R⁴ is -CH₃. In another embodiment is a compound of Formula (IVb) wherein R⁴ is -CH₂CH₃. In another embodiment is a compound of Formula (IVb) wherein R⁴ is -(C₁-C₆)alkyl-OH. In another embodiment is a compound of Formula (IVb) wherein R⁴ is -CH₂OH. In another embodiment is a compound of Formula (IVb) wherein R⁴ is -(C₃-C₆)cycloalkyl. In another embodiment is a compound of Formula (IVb) wherein R⁴ is cyclopropyl. In another embodiment is a compound of Formula (IVb) wherein R⁴ is -C(O)NH₂.

In another embodiment is a compound of Formula (IVb) wherein R⁴ and R⁵ and the carbon atom to which they are attached form a cyclopropyl ring.

In another embodiment is a compound of Formula (IVb) wherein R⁹ is -(C₁-C₆)alkyl. In another embodiment is a compound of Formula (IVb) wherein R⁹ is -CH₃. In another embodiment is a compound of Formula (IVb) wherein R⁹ is -CH₂CH₃. In another embodiment is a compound of Formula (IVb) wherein R⁹ is -(C₁-C₆)haloalkyl. In another embodiment is a compound of Formula (IVb) wherein R⁹ is - CH₂F. In another embodiment is a compound of Formula (IVb) wherein R⁹ is -CHF₂. In another embodiment is a compound of Formula (IVb) wherein R⁹ is -(C₃-C₆)cycloalkyl. In another embodiment is a compound of Formula (IVb) wherein R⁹ is cyclopropyl. In another embodiment is a compound of Formula (IVb) wherein R⁹ is H.

In another embodiment is a compound of Formula (IVb) wherein R¹¹ is -(C₁-C₆)alkyl-N(H)S(O)₂NR²⁵R²⁶. In another embodiment is a compound of Formula (IVb) wherein R¹¹ is - CH₂N(H)S(O)₂NH₂.

In another embodiment is a compound of Formula (IVb) wherein R¹ and R² are each independently H or -(C₁-C₆)alkyl-NR²¹R²². In another embodiment is a compound of Formula (IVb) wherein R¹ and R² are each H. In another embodiment is a compound of Formula (IVb) wherein R¹ and R² are each independently -(C₁-C₆)alkyl-NR²¹R²². In another embodiment is a compound of Formula (IVb) wherein R¹ is H, and R² is -(C₁-C₆)alkyl-NR²¹R²². In another embodiment is a compound of Formula (IVb) wherein R1is -(C₁-C₆)alkyl-NR²¹R²², and R² is H. In another embodiment is a compound of Formula (IVb) wherein R¹ is H, and R² is -CH₂CH₂NH₂. In another embodiment is a compound of Formula (IVb) wherein R¹ is -CH₂CH₂NH₂, and R² is H. In another embodiment is a compound of Formula (IVb) wherein R¹ and R² are each -CH₂CH₂NH₂. In a further embodiment is a compound of Formula (IVb) wherein R¹ is -(C₁-C₆)alkyl-NR²¹R²² and R² is H. In a further embodiment is a compound of Formula (IVb) wherein R¹ is - CH₂CH₂NH₂ and R² is H. In a further embodiment is a compound of Formula (IVb) wherein R¹ is H and R² is -(C₁-C₆)alkyl-NR²¹R²². In a further embodiment is a compound of Formula (IVb) wherein R¹ is H and R² is -CH₂CH₂NH₂. In another embodiment is a compound of Formula (IVb) wherein R¹ and R² are each independently H, -(C₁-C₆)alkyl-NR²¹R²², or -CH₂CH(OH)CH₂NH₂. In another embodiment is a compound of Formula (IVb) wherein R¹ and R² are each independently -CH₂CH(OH)CH₂NH₂. In another embodiment is a compound of Formula (IVb) wherein R¹ is H, and R² is -CH₂CH(OH)CH₂NH₂. In another embodiment is a compound of Formula (IVb) wherein R¹ is -CH₂CH(OH)CH₂NH₂, and R² is H. In a further embodiment is a compound of Formula (IVb) wherein R¹ and R² and the atoms to which they are attached form an optionally substituted heterocycloalkyl ring.

In another embodiment is a compound of Formula (IVb) wherein X is optionally substituted aryl. In a further embodiment is a compound of Formula (IVb) wherein X is optionally substituted phenyl. In another embodiment is a compound of Formula (IVb) wherein X is optionally substituted heteroaryl. In a further embodiment is a compound of Formula (IVb) wherein X is disubstituted heteroaryl. In a further embodiment is a compound of Formula (IVb) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (IVb) wherein X is heteroaryl disubstituted with substituents each independently selected from -(C₁-C₆)alkyl. In a further embodiment is a compound of Formula (IVb) wherein X is heteroaryl disubstituted with methyl. In a further embodiment is a compound of Formula (IVb) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IVb) wherein X is pyridinyl disubstituted with substituents each independently selected from -(C₁-C₆)alkyl. In a further embodiment is a compound of Formula (IVb) wherein X is pyridinyl disubstituted with methyl. In a further embodiment is a compound of Formula (IVb) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IVb) wherein X is pyrimidinyl disubstituted with substituents each independently selected from -(C₁-C₆)alkyl. In a further embodiment is a compound of Formula (IVb) wherein X is pyrimidinyl disubstituted with methyl. In another embodiment is a compound of Formula (IVb) wherein X is optionally substituted -(C₁-C₆)alkyl-.

In another embodiment is a compound of Formula (IVb) wherein Y is optionally substituted aryl. In a further embodiment is a compound of Formula (IVb) wherein Y is optionally substituted phenyl. In another embodiment is a compound of Formula (IVb) wherein Y is optionally substituted heteroaryl. In another embodiment is a compound of Formula (IVb) wherein Y is optionally substituted -(C₁-C₆)alkyl-. In another embodiment is a compound of Formula (IVb) wherein Y is optionally substituted (C₃-C₇)cycloalkyl-. In another embodiment is a compound of Formula (IVb) wherein Y is optionally substituted heterocycloalkyl. In another embodiment is a compound of Formula (IVb) wherein Y is -O-. In another embodiment is a compound of Formula (IVb) wherein Y is -(C₂-C₆)alkynyl. In another embodiment is a compound of Formula (IVb) wherein Y is -O-(C₁-C₆)alkyl-. In another embodiment is a compound of Formula (IVb) wherein Y is a bond.

In another embodiment is a compound of Formula (IVb) wherein Z is -(C₁-C₁₂)alkyl. In a further embodiment is a compound of Formula (IVb) wherein Z is n-butyl, isobutyl, or tert-butyl. In another embodiment is a compound of Formula (IVb) wherein Z is -O-(C₁-C₁₂)alkyl. In another embodiment is a compound of Formula (IVb) wherein Z is -O-(C₃-C₇)cycloalkyl. In another embodiment is a compound of Formula (IVb) wherein Z is -(C₂-C₁₂)alkenyl. In another embodiment is a compound of Formula (IVb) wherein Z is optionally substituted aryl. In a further embodiment is a compound of Formula (IVb) wherein Z is optionally substituted phenyl. In a further embodiment is a compound of Formula (IVb) wherein Z is phenyl monsubstituted or disubstituted with a substituent independently selected from -(C₁-C₈)alkyl. In a further embodiment is a compound of Formula (IVb) wherein Z is phenyl monosubstituted with n-butyl, isobutyl, or tert-butyl. In a further embodiment is a compound of Formula (IVb) wherein Z is phenyl monosubstituted with n-butyl. In a further embodiment is a compound of Formula (IVb) wherein Z is phenyl monosubstituted with isobutyl. In a further embodiment is a compound of Formula (IVb) wherein Z is phenyl monosubstituted with tert-butyl. In another embodiment is a compound of Formula (IVb) wherein Z is optionally substituted heteroaryl. In another embodiment is a compound of Formula (IVb) wherein Z is optionally substituted -(C₃-C₇)cycloalkyl. In another embodiment is a compound of Formula (IVb) wherein Z is optionally substituted heterocycloalkyl. In another embodiment is a compound of Formula (IVb) wherein Z is halogen.

In another embodiment is a compound of Formula (IV) having the structure of Formula (IVc): 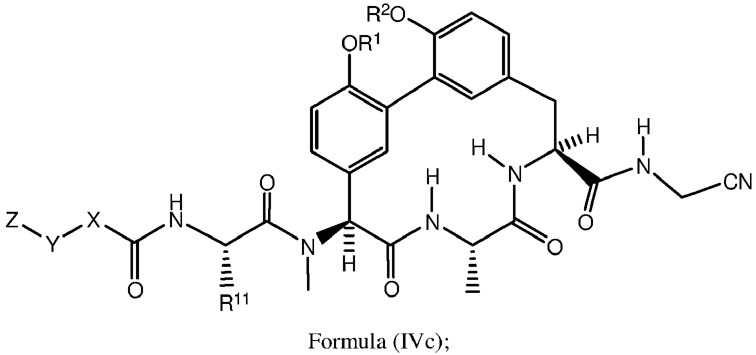 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring;
R¹¹ is -(C₁-C₆)alkyl-N(H)S(O)₂NR²⁵R²⁶;
X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(CrC₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring; and or a pharmaceutically acceptable salt, solvate, or prodrug thereof.

In another embodiment is a compound of Formula (IVc) wherein R¹¹ is -(C₁-C₆)alkyl-N(H)S(O)₂NR²⁵R²⁶. In another embodiment is a compound of Formula (IVc) wherein R¹¹ is - CH₂N(H)S(O)₂NH₂.

In another embodiment is a compound of Formula (IVc) wherein R¹ and R² are each independently H or -(C₁-C₆)alkyl-NR²¹R²². In another embodiment is a compound of Formula (IVc) wherein R¹ and R² are each H. In another embodiment is a compound of Formula (IVc) wherein R¹ and R² are each independently -(C₁-C₆)alkyl-NR²¹R²². In another embodiment is a compound of Formula (IVc) wherein R¹ is H, and R² is -(C₁-C₆)alkyl-NR²¹R²². In another embodiment is a compound of Formula (IVc) wherein R¹ is -(C₁-C₆)alkyl-NR²¹R²², and R² is H. In another embodiment is a compound of Formula (IVc) wherein R¹ is H, and R² is -CH₂CH₂NH₂. In another embodiment is a compound of Formula (IVc) wherein R¹ is -CH₂CH₂NH₂, and R² is H. In another embodiment is a compound of Formula (IVc) wherein R¹ and R² are each -CH₂CH₂NH₂. In a further embodiment is a compound of Formula (IVc) wherein R¹ is -(C₁-C₆)alkyl-NR²¹R²² and R² is H. In a further embodiment is a compound of Formula (IVc) wherein R¹ is - CH₂CH₂NH₂ and R² is H. In a further embodiment is a compound of Formula (IVc) wherein R¹ is H and R² is -(C₁-C₆)alkyl-NR²¹R²². In a further embodiment is a compound of Formula (IVc) wherein R¹ is H and R² is -CH₂CH₂NH₂. In another embodiment is a compound of Formula (IVc) wherein R¹ and R² are each independently H, -(C₁-C₆)alkyl-NR²¹R²², or -CH₂CH(OH)CH₂NH₂. In another embodiment is a compound of Formula (IVc) wherein R¹ and R² are each independently -CH₂CH(OH)CH₂NH₂. In another embodiment is a compound of Formula (IVc) wherein R¹ is H, and R² is -CH₂CH(OH)CH₂NH₂. In another embodiment is a compound of Formula (IVc) wherein R¹ is -CH₂CH(OH)CH₂NH₂, and R² is H. In a further embodiment is a compound of Formula (IVc) wherein R¹ and R² and the atoms to which they are attached form an optionally substituted heterocycloalkyl ring.

In another embodiment is a compound of Formula (IVc) wherein X is optionally substituted aryl. In a further embodiment is a compound of Formula (IVc) wherein X is optionally substituted phenyl. In another embodiment is a compound of Formula (IVc) wherein X is optionally substituted heteroaryl. In a further embodiment is a compound of Formula (IVc) wherein X is disubstituted heteroaryl. In a further embodiment is a compound of Formula (IVc) wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (IVc) wherein X is heteroaryl disubstituted with substituents each independently selected from -(C₁-C₆)alkyl. In a further embodiment is a compound of Formula (IVc) wherein X is heteroaryl disubstituted with methyl. In a further embodiment is a compound of Formula (IVc) wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IVc) wherein X is pyridinyl disubstituted with substituents each independently selected from -(C₁-C₆)alkyl. In a further embodiment is a compound of Formula (IVc) wherein X is pyridinyl disubstituted with methyl. In a further embodiment is a compound of Formula (IVc) wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (IVc) wherein X is pyrimidinyl disubstituted with substituents each independently selected from -(C₁-C₆)alkyl. In a further embodiment is a compound of Formula (IVc) wherein X is pyrimidinyl disubstituted with methyl. In another embodiment is a compound of Formula (IVc) wherein X is optionally substituted -(C₁-C₆)alkyl-.

In another embodiment is a compound of Formula (IVc) wherein Y is optionally substituted aryl. In a further embodiment is a compound of Formula (IVc) wherein Y is optionally substituted phenyl. In another embodiment is a compound of Formula (IVc) wherein Y is optionally substituted heteroaryl. In another embodiment is a compound of Formula (IVc) wherein Y is optionally substituted -(C₁-C₆)alkyl-. In another embodiment is a compound of Formula (IVc) wherein Y is optionally substituted (C₃-C₇)cycloalkyl-. In another embodiment is a compound of Formula (IVc) wherein Y is optionally substituted heterocycloalkyl. In another embodiment is a compound of Formula (IVc) wherein Y is -O-. In another embodiment is a compound of Formula (IVc) wherein Y is -(C₂-C₆)alkynyl. In another embodiment is a compound of Formula (IVc) wherein Y is -O-(C₁-C₆)alkyl-. In another embodiment is a compound of Formula (IVc) wherein Y is a bond.

In another embodiment is a compound of Formula (IVc) wherein Z is -(C₁-C₁₂)alkyl. In a further embodiment is a compound of Formula (IVc) wherein Z is n-butyl, isobutyl, or tert-butyl. In another embodiment is a compound of Formula (IVc) wherein Z is -O-(C₁-C₁₂)alkyl. In another embodiment is a compound of Formula (IVc) wherein Z is -O-(C₃-C₇)cycloalkyl. In another embodiment is a compound of Formula (IVc) wherein Z is -(C₂-C₁₂)alkenyl. In another embodiment is a compound of Formula (IVc) wherein Z is optionally substituted aryl. In a further embodiment is a compound of Formula (IVc) wherein Z is optionally substituted phenyl. In a further embodiment is a compound of Formula (IVc) wherein Z is phenyl monsubstituted or disubstituted with a substituent independently selected from -(C₁-C₈)alkyl. In a further embodiment is a compound of Formula (IVc) wherein Z is phenyl monosubstituted with n-butyl, isobutyl, or tert-butyl. In a further embodiment is a compound of Formula (IVc) wherein Z is phenyl monosubstituted with n-butyl. In a further embodiment is a compound of Formula (IVc) wherein Z is phenyl monosubstituted with isobutyl. In a further embodiment is a compound of Formula (IVc) wherein Z is phenyl monosubstituted with tert-butyl. In another embodiment is a compound of Formula (IVc) wherein Z is optionally substituted heteroaryl. In another embodiment is a compound of Formula (IVc) wherein Z is optionally substituted -(C₃-C₇)cycloalkyl. In another embodiment is a compound of Formula (IVc) wherein Z is optionally substituted heterocycloalkyl. In another embodiment is a compound of Formula (IVc) wherein Z is halogen.

In some embodiments, the compound of Formula (I), (Ia), (Ib), (Ic), (Id), (Ie), (If), (II), (IIa), (IIb), (IIc), (IId), (IIe), (III), (IIIa), (IIIb), (IIIc), (IV), (IVa), (IVb), and (IVc) is selected from a compound in table 1 or a pharmaceutically acceptable salt, solvate, or prodrug thereof.

**Table 1**

| **Cp. #** | **Name** | **Structure** |
|---|---|---|
| **201** | rac-(8S,11S,14S)-3,18-bis(2-aminoethoxy)-N-(cyanomethyl)-11-methyl-14-[methyl-[rac-(2S)-4-amino-2-[[4-[3-(4-butylphenyl)pyrazol-1-yl]-2-methylbenzoyl]amino]butanoyl]amino]-10,13-dioxo-9,12-diazatricyclo[13.3.1.12,6]icosa-1(18),2(20),3,5,15(19),16-hexaene-8-carboxamide | 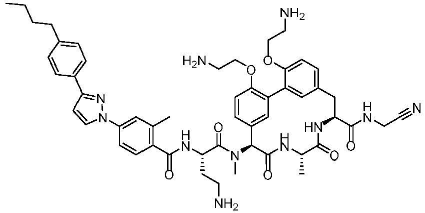 |
| **202** | rac-(8S,11S,14S)-3,18-bis(2-aminoethoxy)-N-(cyanomethyl)-11-methyl-14- [methyl- [rac-(2S)-4-amino-2-[[2-methyl-4- [4-(pentafluoro-lambda6-sulfanyl)phenyl] benzoyl] amino] buta noyl]amino]-10,13-dioxo-9,12-diazatricyclo[13.3.1.12,6]icosa-1(18),2(20),3,5,15(19),16-hexaene-8-carboxamide | 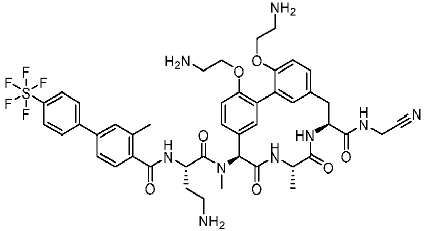 |
| **203** | rac-(8S,11S,14S)-3,18-bis(2-aminoethoxy)-N-(cyanomethyl)-11-methyl-14-[methyl-[rac-(2S)-4-amino-2-[[4-(4-tert-butylphenyl)-2-methylbenzoyl]amino]butanoyl]amino]-10,13-dioxo-9,12-diazatricyclo[13.3.1.12,6]icosa-1(18),2(20),3,5,15(19),16-hexaene-8-carboxamide | 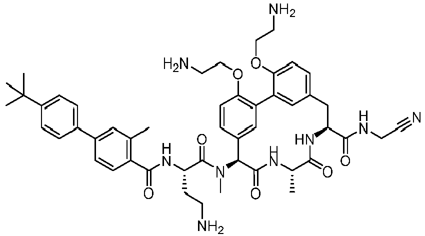 |
| **204** | rac-(8S,11S,14S)-3,18-bis(2-aminoethoxy)-N-(cyanomethyl)-11-methyl-14-[methyl-[rac-(2S)-4-amino-2-[[4-(2-hexylpyrimidin-5-yl)-2-methylbenzoyl]amino]butanoyl]amino]-10,13-dioxo-9,12-diazatricyclo[13.3.1.12,6]icosa-1(18),2(20),3,5,15(19),16-hexaene-8-carboxamide | 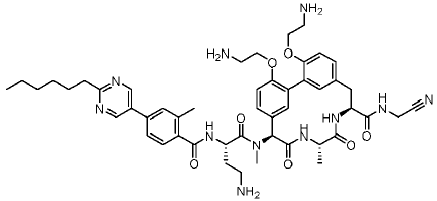 |
| **205** | rac-(8S,11S,14S)-3,18-bis(2-aminoethoxy)-N-(cyanomethyl)-11-methyl-14-[methyl-[rac-(2S)-4-amino-2-[2-methyl-6-(4-pentylphenyl)pyridine-3-carbonyl] amino butanoyl] amino- 10,13-dioxo-9,12-diazatricyclo[13.3.1.12,6]icosa-1(18),2(20),3,5,15(19),16-hexaene-8-carboxamide | 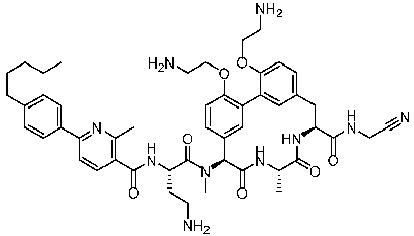 |
| **206** | rac-(8S,11S,14S)-3,18-bis(2-aminoethoxy)-N-(cyanomethyl)-11-methyl-14-[methyl-[rac-(2S)-4-amino-2-[[6-(4-tert-butylphenyl)-2-methyl-pyridine-3-carbonyl] amino butanoyl]amino- 10,13-dioxo-9,12-diazatricyclo[13.3.1.12,6]icosa-1(18),2(20),3,5,15(19),16-hexaene-8-carboxamide | 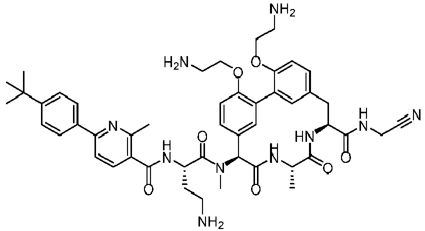 |
| **207** | rac-(8S,11S,14S)-3,18-bis(2-aminoethoxy)-N-(cyanomethyl)-11-methyl-14-[methyl-[rac-(2S)-4-amino-2-[[6-(4-butylphenyl)-2-methyl-pyridine-3-carbonyl] amino butanoyl] amino- 10,13-dioxo-9,12-diazatricyclo[13.3.1.12,6]icosa-1(18),2(20),3,5,15(19),16-hexaene-8-carboxamide | 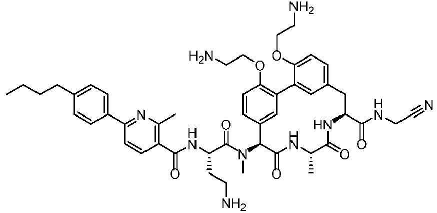 |
| **208** | rac-(8S,11S,14S)-3,18-bis(2-aminoethoxy)-N-(cyanomethyl)-11-methyl-14-[methyl-[rac-(2S)-4-amino-2-[[2-methyl-6-(4-propylphenyl)pyridine-3- carbonyl] amino butanoyl] amino- 10,13-dioxo-9,12-diazatricyclo[13.3.1.12,6]icosa-1(18),2(20),3,5,15(19),16-hexaene-8-carboxamide | 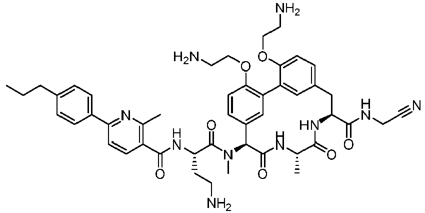 |
| **209** | rac-(8S,11S,14S)-3,18-bis(2-aminoethoxy)-N-(cyanomethyl)-11-methyl-14-[methyl-[rac-(2S)-4-amino-2-[[2-methyl-6-(2-pentylpyrimidin-5-yl)pyridine-3-carbonyl] amino butanoyl] amino- 10,13-dioxo-9,12-diazatricyclo[13.3.1.12,6]icosa-1(18),2(20),3,5,15(19),16-hexaene-8-carboxamide | 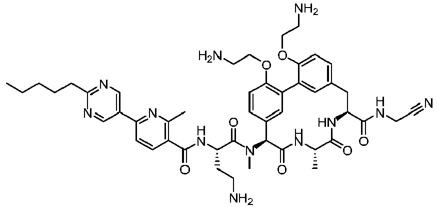 |
| **210** | rac-(8S,11S,14S)-3,18-bis(2-aminoethoxy)-N-(cyanomethyl)-11-methyl-14-[methyl-[rac-(2S)-4-amino-2-[[6-(4-tert-butyl-2-methylphenyl)-2-methyl-pyridine-3-carbonyl] amino butanoyl] amino- 10,13-dioxo-9,12-diazatricyclo[13.3.1.12,6]icosa-1(18),2(20),3,5,15(19),16-hexaene-8-carboxamide | 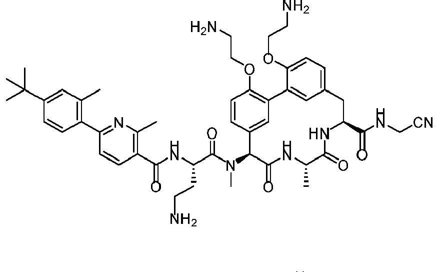 |
| **211** | rac-(8S,11S,14S)-3,18-bis(2-aminoethoxy)-N-(cyanomethyl)-11-methyl-14-[methyl-[rac-(2S)-4-amino-2-[[2-methyl-6-[4-[1-(trifluoromethyl)cyclopropyl]phenyl ]pyridine-3-carbonyl]amino]butanoyl]amino- 10,13-dioxo-9,12-diazatricyclo[13.3.1.12,6]icosa-1(18),2(20),3,5,15(19),16-hexaene-8-carboxamide | 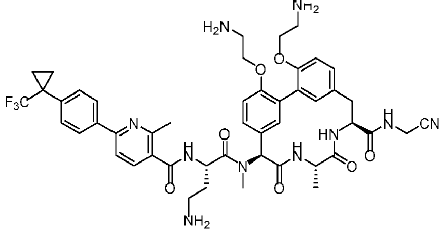 |
| **212** | rac-(8S,11S,14S)-3,18-bis(2-aminoethoxy)-N-(cyanomethyl)-11-methyl-14-[methyl-[rac-(2S)-4-amino-2-[[2-methyl-6-[4-(1-methylcyclopropyl)phenyl]pyridine-3-carbonyl]amino]butanoyl]amino]- 10,13-dioxo-9,12-diazatricyclo[13.3.1.12,6]icosa-1(18),2(20),3,5,15(19),16-hexaene-8-carboxamide | 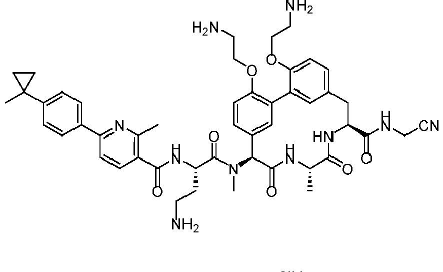 |
| **213** | rac-(8S,11S,14S)-3,18-bis(2-aminoethoxy)-N-(cyanomethyl)-11-methyl-14-[methyl-[rac-(2S)-4-amino-2-[[6-(3,3-dimethylbut-1-ynyl)-2-methyl-pyridine-3-carbonyl]amino]butanoyl]amino- 10,13-dioxo-9,12-diazatricyclo[13.3.1.12,6]icosa-1(18),2(20),3,5,15(19),16-hexaene-8-carboxamide | 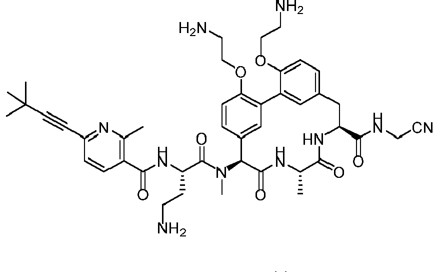 |
| **214** | rac-(8S,11S,14S)-3,18-bis(2-aminoethoxy)-N-(cyanomethyl)-11-methyl-14-[methyl-[rac-(2S)-4-amino-2-[[6-(2-cyclohexylethynyl)-2-methyl-pyridine-3 - carbonyl]amino]butanoyl]amino- 10,13-dioxo-9,12-diazatricyclo[13.3.1.12,6]icosa-1(18),2(20),3,5,15(19),16-hexaene-8-carboxamide | 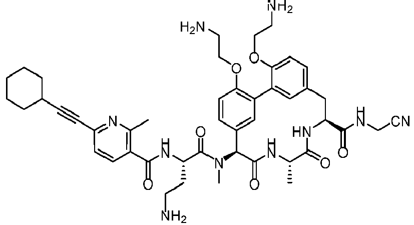 |
| **215** | rac-(8S,11S,14S)-3,18-bis(2-aminoethoxy)-N-(cyanomethyl)-11-methyl-14-[methyl-[rac-(2S)-4-amino-2-[[6-(4-tert-butylphenyl)-4-methyl-pyridine-3-carbonyl]amino]butanoyl]amino- 10,13-dioxo-9,12-diazatricyclo[13.3.1.12,6]icosa-1(18),2(20),3,5,15(19),16-hexaene-8-carboxamide | 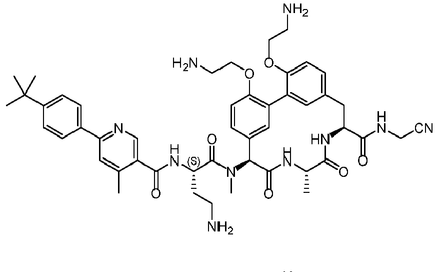 |
| **216** | rac-(8S,11S,14S)-3,18-bis(2-aminoethoxy)-N-(cyanomethyl)-11-methyl-14-[methyl-[rac-(2S)-4-amino-2-[(4-methyl-2-tetralin-6-yl-pyrimidine-5-carbonyl)amino]butanoyl]amino]-10,13-dioxo-9,12-diazatricyclo[13.3.1.12,6]icosa-1(18),2(20),3,5,15(19),16-hexaene-8-carboxamide | 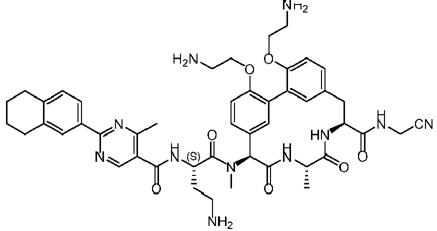 |
| **217** | rac-(8S,11S,14S)-3,18-bis(2-aminoethoxy)-N-(cyanomethyl)-11-methyl-14-[methyl-[rac-(2S)-4-amino-2-[[2-[4-(cyclobutylmethyl)phenyl] -4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 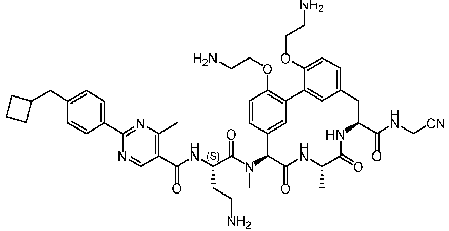 |
| **218** | rac-(8S,11S,14S)-3,18-bis(2-aminoethoxy)-N-(cyanomethyl)-11-methyl-14-[methyl-[rac-(2S)-4-amino-2-[[2-[4-(cyclopentylmethyl)phenyl]-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 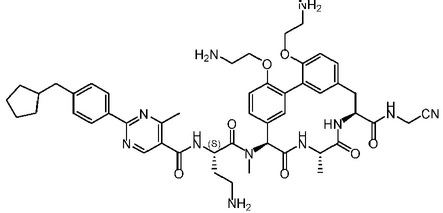 |
| **219** | rac-(8S,11S,14S)-3,18-bis(2-aminoethoxy)-N-(cyanomethyl)-11-methyl-14-[methyl-[rac-(2S)-4-amino-2-[[4-methyl-2-[4-[1-(trifluoromethyl)cyclopropyl]phenyl ]pyrimidine-5-carbonyl] amino butanoyl] amino]]-10,13-dioxo-9,12-diazatricyclo[13.3.1.12,6]icosa-1(18),2(20),3,5,15(19),16-hexaene-8-carboxamide | 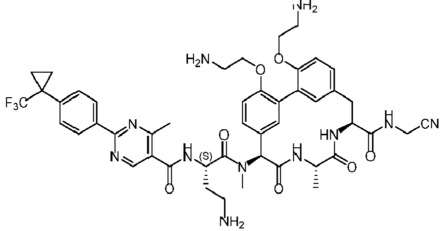 |
| **220** | rac-(8S,11S,14S)-3,18-bis(2-aminoethoxy)-N-(cyanomethyl)-11-methyl-14-[methyl-[rac-(2S)-4-amino-2-[[2-(4-isopentylphenyl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 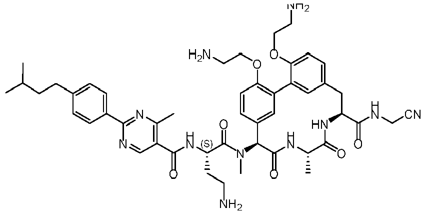 |
| **221** | rac-(8S,11S,14S)-3,18-bis(2-aminoethoxy)-N-(cyanomethyl)-11-methyl-14-[methyl-[rac-(2S)-4-amino-2-[[2-(4-ethyl-1-naphthyl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 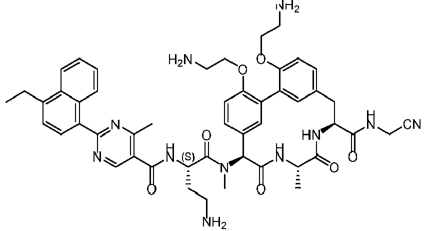 |
| **222** | rac-(8S,11S,14S)-3,18-bis(2-aminoethoxy)-N-(cyanomethyl)-11-methyl-14-[methyl-[rac-(2S)-4-amino-2-[[4-methyl-2-(2-methyl-4-propyl-phenyl)pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 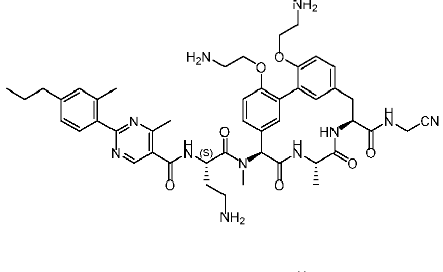 |
| **223** | rac-(8S,11S,14S)-3,18-bis(2-aminoethoxy)-N-(cyanomethyl)-11-methyl-14-[methyl-[rac-(2S)-4-amino-2-[[2-(4-butyl-2-methylphenyl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 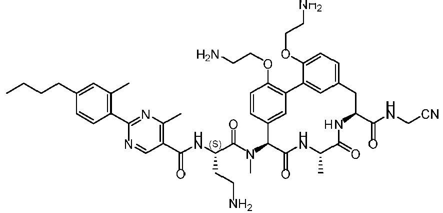 |
| **224** | rac-(8S,11S,14S)-3,18-bis(2-aminoethoxy)-N-(cyanomethyl)-11-methyl-14-[methyl-[rac-(2S)-4-amino-2-[[4-methyl-2-[4-(1-methylcyclopropyl)phenyl]pyrimidin e-5-carbonyl]amino]butanoyl]amino- 10,13-dioxo-9,12-diazatricyclo[13.3.1.12,6]icosa-1(18),2(20),3,5,15(19),16-hexaene-8-carboxamide | 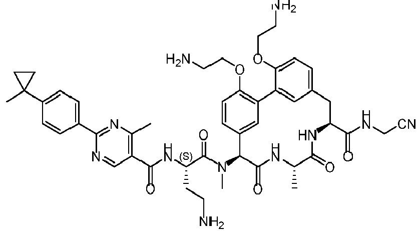 |
| **225** | rac-(8S,11S,14S)-3,18-bis(2-aminoethoxy)-N-(cyanomethyl)-11-methyl-14-[methyl-[rac-(2S)-4-amino-2-[[4-methyl-2-[4-(pentafluoro-lambda6-sulfanyl)phenyl]pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 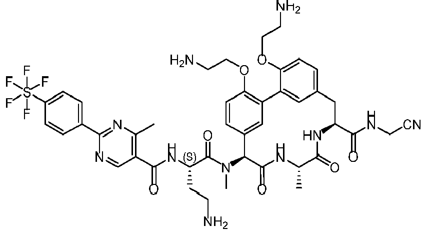 |
| **226** | rac-(8S,11S,14S)-3,18-bis(2-aminoethoxy)-N-(cyanomethyl)-11-methyl-14-[methyl-[rac-(2S)-4-amino-2-[[2-[4-(2,2-dimethylpropyl)phenyl]-4-methylpyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 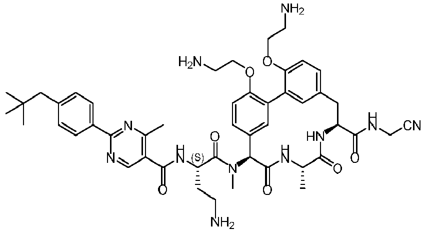 |
| **227** | rac-(8S,11S,14S)-3,18-bis(2-aminoethoxy)-N-(cyanomethyl)-11-methyl-14-[methyl-[rac-(2S)-4-amino-2-[[4-methyl-2-(4-pent-1-ynylphenyl)pyrimidine-5-carbonyl]amino]butanoyl]amino-10,13-dioxo-9,12-diazatricyclo[13.3.1.12,6]icosa-1(18),2(20),3,5,15(19),16-hexaene-8-carboxamide | 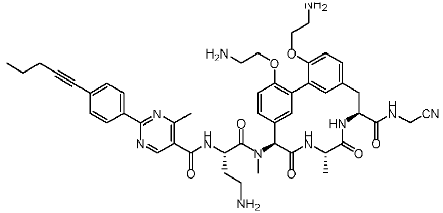 |
| **228** | rac-(8S,11S,14S)-3,18-bis(2-aminoethoxy)-N-(cyanomethyl)-11-methyl-14-[methyl-[rac-(2S)-4-amino-2-[[2-(3,3-dimethylbut-1-ynyl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 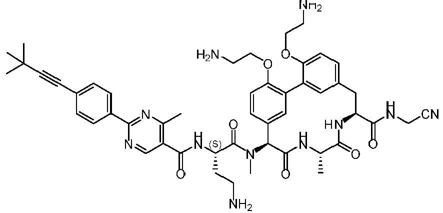 |
| **229** | rac-(8S,11S,14S)-3,18-bis(2-aminoethoxy)-N-(cyanomethyl)-11-methyl-14-[methyl-[rac-(2S)-4-amino-2-[[4-methyl-2-(3-methylbut-1-ynyl)pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 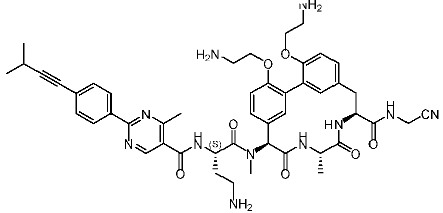 |
| **230** | rac-(8S,11S,14S)-3,18-bis(2-aminoethoxy)-N-(cyanomethyl)-11-methyl-14-[methyl-[rac-(2S)-4-amino-2-[[2-(4-cyclopentylphenyl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 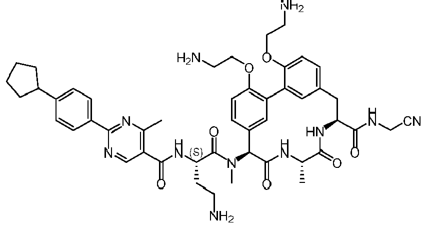 |
| **231** | rac-(8S,11S,14S)-3,18-bis(2-aminoethoxy)-N-(cyanomethyl)-11-methyl-14-[methyl-[rac-(2S)-4-amino-2-[[4-methyl-2-(4-tetrahydropyran-4-ylphenyl)pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 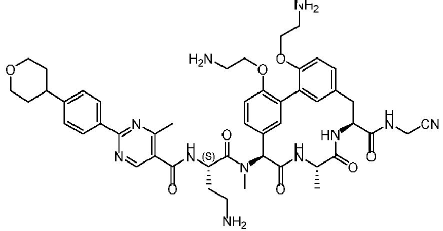 |
| **232** | rac-(8S,11S,14S)-3,18-bis(2-aminoethoxy)-N-(cyanomethyl)-11-methyl-14-[methyl-[rac-(2S)-4-amino-2-[[2-[2-(4-tert-butylphenyl)pyrimidin-5-yl]-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 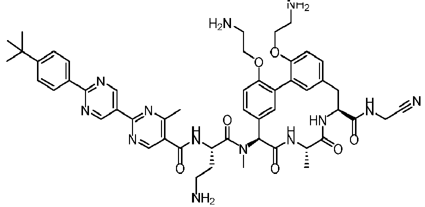 |
| **233** | rac-(8S,11S,14S)-3,18-bis(2-aminoethoxy)-N-(cyanomethyl)-11-methyl-14-[methyl-[rac-(2S)-4-amino-2-[[2-(4-cyclohexylphenyl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 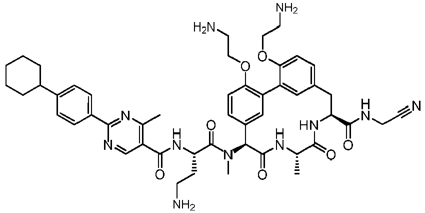 |
| **234** | rac-(8S,11S,14S)-3,18-bis(2-aminoethoxy)-N-(cyanomethyl)-11-methyl-14-[methyl-[rac-(2S)-4-amino-2-[[2-(1,1-dimethylindan-5-yl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 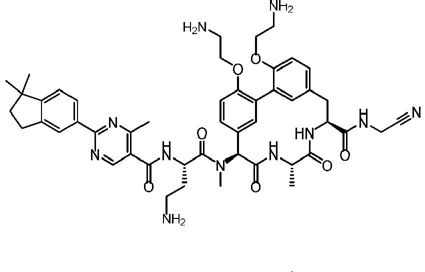 |
| **235** | rac-(8S,11S,14S)-3,18-bis(2-aminoethoxy)-N-(cyanomethyl)-11-methyl-14-[methyl-[rac-(2S)-4-amino-2-[[2-(4-butyl-2-chlorophenyl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 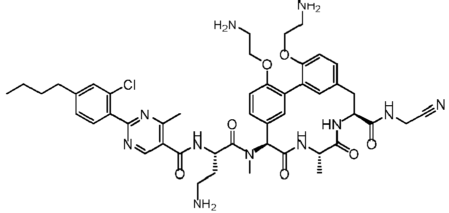 |
| **236** | rac-(8S,11S,14S)-3,18-bis(2-aminoethoxy)-N-(cyanomethyl)-11-methyl-14-[methyl-[rac-(2S)-4-amino-2-[[4-methyl-2-[4-(1-methyl-1-phenyl-ethyl)phenyl]pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 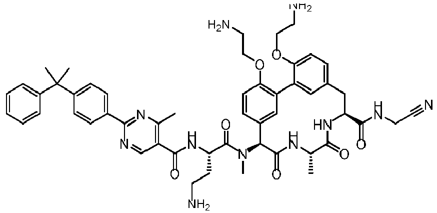 |
| **237** | rac-(8S,11S,14S)-3,18-bis(2-aminoethoxy)-N-(cyanomethyl)-11-methyl-14-[methyl-[rac-(2S)-4-amino-2-[[2-(3-tert-butylphenyl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 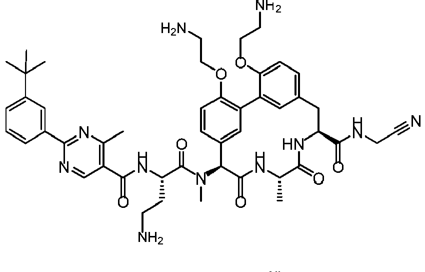 |
| **238** | rac-(8S,11S,14S)-3,18-bis(2-aminoethoxy)-N-(cyanomethyl)-11-methyl-14-[methyl-[rac-(2S)-4-amino-2-[[4-methyl-2-[4-(3-methyloxetan-3-yl)phenyl]pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 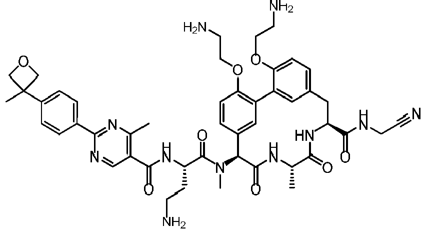 |
| **239** | rac-(8S,11S,14S)-3,18-bis(2-aminoethoxy)-N-(cyanomethyl)-11-methyl-14-[methyl-[rac-(2S)-2-[[2-[4-(2-adamantyl)phenyl]-4-methylpyrimidine-5-carbonyl]amino]]-4-amino-butanoyl]amino]-10,13-dioxo-9,12-diazatricyclo[13.3.1.12,6]icosa-l(18),2(20),3,5,15(19),16-hexaene-8-carboxamide | 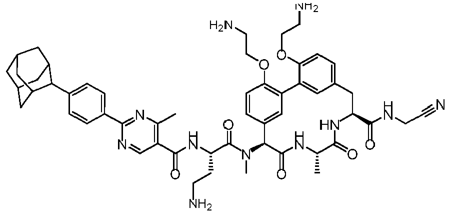 |
| **240** | rac-(8S,11S,14S)-3,18-bis(2-aminoethoxy)-N-(cyanomethyl)-11-methyl-14-[methyl-[rac-(2S)-4-amino-2-[[2-(4-heptylphenyl)-4-methyl-pyrimidine-5-carbonyl]amino]butanoyl]amino- 10,13-dioxo-9,12-diazatricyclo[13.3.1.12,6]icosa-l(18),2(20),3,5,15(19),16-hexaene-8-carboxamide | 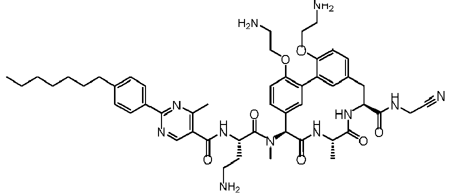 |
| **241** | rac-(8S,11S,14S)-3,18-bis(2-aminoethoxy)-N-(cyanomethyl)-11-methyl-14-[methyl-[rac-(2S)-4-amino-2-[[4-methyl-2-(4-pentoxyphenyl)pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 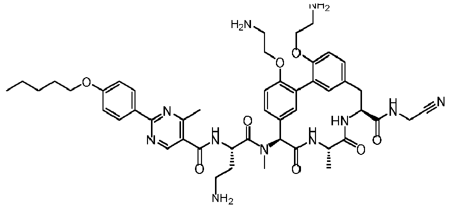 |
| **242** | rac-(8S,11S,14S)-3,18-bis(2-aminoethoxy)-N-(cyanomethyl)-11-methyl-14-[methyl-[rac-(2S)-4-amino-2-[[2-(4-butoxy-2-methyl-phenyl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 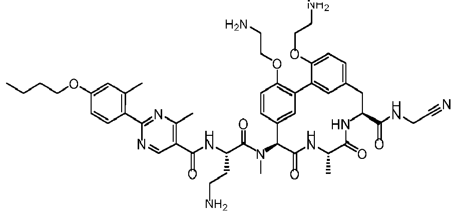 |
| **243** | rac-(8S,11S,14S)-3,18-bis(2-aminoethoxy)-N-(cyanomethyl)-11-methyl-14-[methyl-[rac-(2S)-4-amino-2-[[2-(4-butoxy-3,5-dimethyl-phenyl)-4-methylpyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 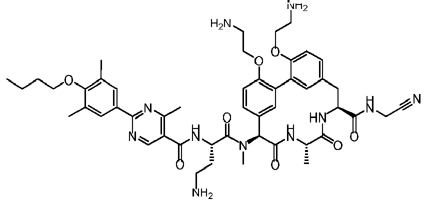 |
| **244** | rac-(8S,11S,14S)-3,18-bis(2-aminoethoxy)-N-(cyanomethyl)-11-methyl-14-[methyl-[rac-(2S)-4-amino-2-[[2-(4-butoxy-3-fluorophenyl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 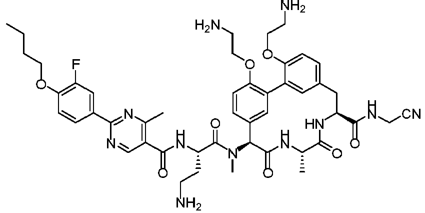 |
| **245** | rac-(8S,11S,14S)-3,18-bis(2-aminoethoxy)-N-(cyanomethyl)-11-methyl-14-[methyl-[rac-(2S)-4-amino-2-[[2-(4-isopentyloxyphenyl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 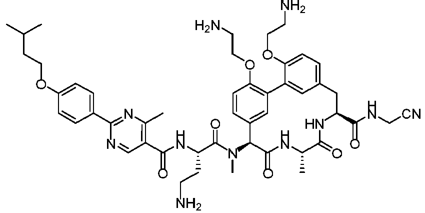 |
| **246** | rac-(8S,11S,14S)-3,18-bis(2-aminoethoxy)-N-(cyanomethyl)-11-methyl-14-[methyl-[rac-(2S)-4-amino-2-[[2-(4-isopropoxyphenyl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 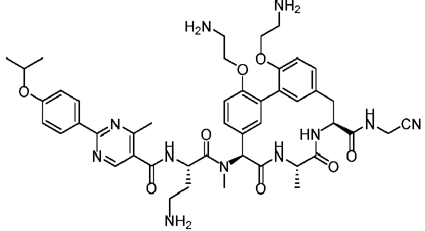 |
| **247** | rac-(8S,11S,14S)-3,18-bis(2-aminoethoxy)-N-(cyanomethyl)-11-methyl-14-methyl-[rac-(2S)-4-amino-2-[[2-(4-butoxy-3-chlorophenyl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 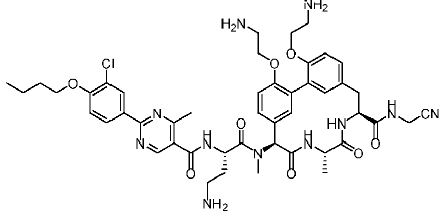 |
| **248** | rac-(8S,11S,14S)-3,18-bis(2-aminoethoxy)-N-(cyanomethyl)-11-methyl-14-[methyl-[rac-(2S)-4-amino-2-[[2-(4-hexoxyphenyl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 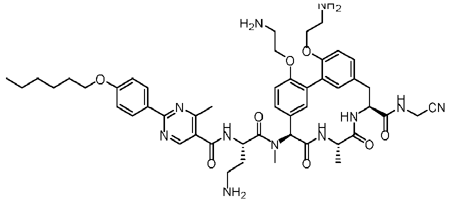 |
| **249** | rac-(8S,11S,14S)-3,18-bis(2-aminoethoxy)-N-(cyanomethyl)-11-methyl-14-[methyl-[rac-(2S)-4-amino-2-[[2-(2,2-dimethylchroman-6-yl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 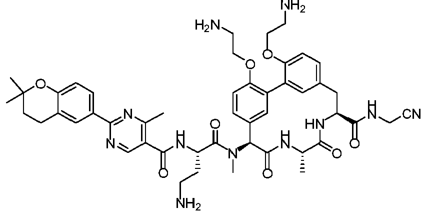 |
| **250** | rac-(8S,11S,14S)-3,18-bis(2-aminoethoxy)-N-(cyanomethyl)-11-methyl-14-[methyl-[rac-(2S)-4-amino-2-[[2-(4-tert-butoxyphenyl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 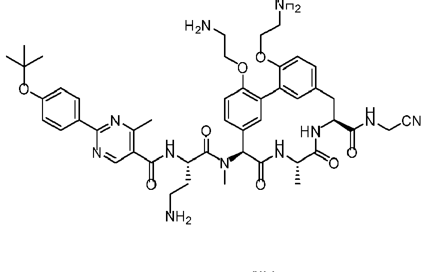 |
| **251** | rac-(8S,11S,14S)-3,18-bis(2-aminoethoxy)-N-(cyanomethyl)-11-methyl-14-[methyl-[rac-(2S)-4-amino-2-[[2-(3-isopropoxyphenyl)-4-methyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 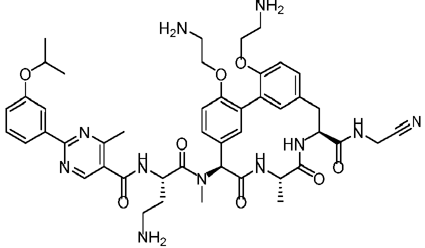 |
| **252** | rac-(8S,11S,14S)-3,18-bis(2-aminoethoxy)-N-(cyanomethyl)-11-methyl-14-methyl-[rac-(2S)-4-amino-2-[[2-[4-(2,2-dimethylpropoxy)phenyl]-4-methylpyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 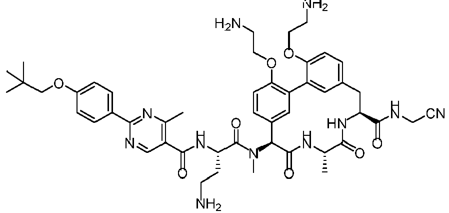 |
| **253** | rac-(8S,11S,14S)-3,18-bis(2-aminoethoxy)-N-(cyanomethyl)-11-methyl-14-[methyl-[rac-(2S)-4-amino-2-[[2-(4-tert-butylphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 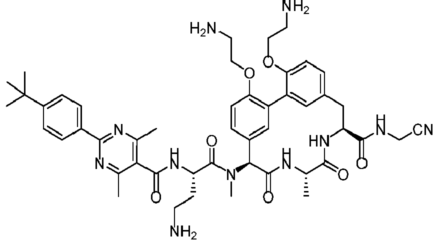 |
| **254** | rac-(8S,11S,14S)-18-(2-aminoethoxy)-N-(cyanomethyl)-3-hydroxy-11-methyl-14-[methyl-[rac-(2S)-4-amino-2-[[2-(4-tert-butylphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 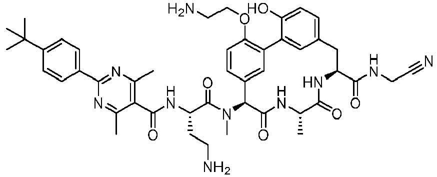 |
| **255** | rac-(8S,11S,14S)-3,18-bis(2-aminoethoxy)-N-(cyanomethyl)-11-methyl-14-[methyl-[rac-(2S)-4-amino-2-[[2-(4-butylphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 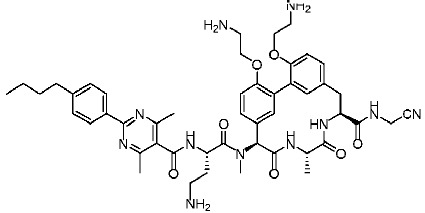 |
| **256** | rac-(8S,11S,14S)-3,18-bis(2-aminoethoxy)-N-(cyanomethyl)-11-methyl-14-[methyl-[rac-(2S)-4-amino-2-[[2-(4-heptylphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 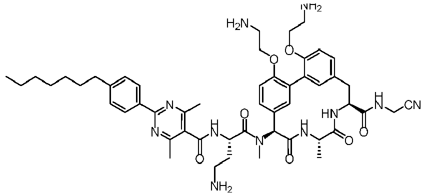 |
| **257** | rac-(8S,11S,14S)-3,18-bis(2-aminoethoxy)-N-(cyanomethyl)-11-methyl-14-[methyl-[rac-(2S)-4-amino-2-[[4,6-dimethyl-2-(4-trimethylsilylphenyl)pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 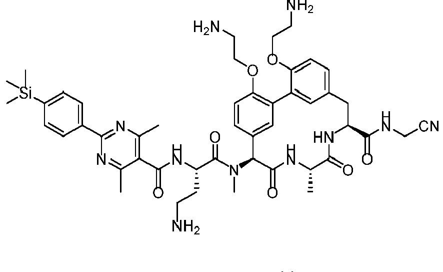 |
| **258** | rac-(8S,11S,14S)-3,18-bis(2-aminoethoxy)-N-(cyanomethyl)-11-methyl-14-[methyl-[rac-(2S)-4-amino-2-[[2-(4-isobutylphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 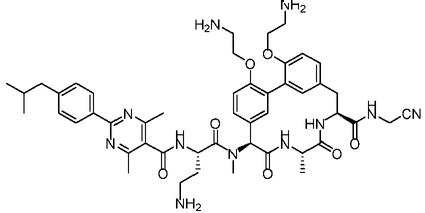 |
| **259** | rac-(8S,11S,14S)-3,18-bis(2-aminoethoxy)-N-(cyanomethyl)-11-methyl-14-[methyl-[rac-(2S)-4-amino-2-[(4,6-dimethyl-2-tetralin-6-yl-pyrimidine-5-carbonyl)amino]butanoyl]amino]]-10,13-dioxo-9,12-diazatricyclo[13.3.1.12,6]icosa-1(18),2(20),3,5,15(19),16-hexaene-8-carboxamide | 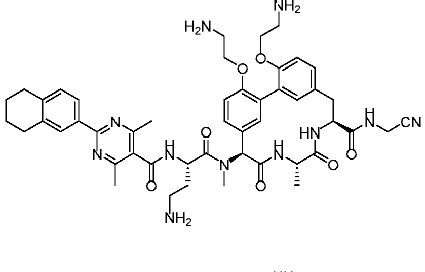 |
| **260** | rac-(8S,11S,14S)-3,18-bis(2-aminoethoxy)-N-(cyanomethyl)-11-methyl-14-[methyl-[rac-(2S)-4-amino-2-[[2-(4-ethylphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 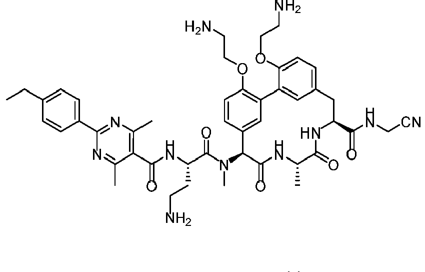 |
| **261** | rac-(8S,11S,14S)-3,18-bis(2-aminoethoxy)-N-(cyanomethyl)-11-methyl-14-[methyl-[rac-(2S)-4-amino-2-[[2-[4-(1,1-dimethylpropyl)phenyl]-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 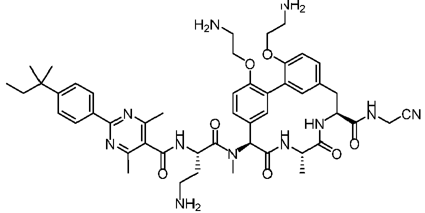 |
| **262** | rac-(8S,11S,14S)-3,18-bis(2-aminoethoxy)-N-(cyanomethyl)-11-methyl-14-[methyl-[rac-(2S)-4-amino-2-[[2-(4-isopentylphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 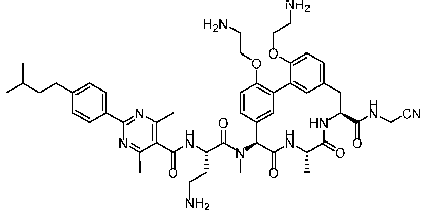 |
| **263** | rac-(8S,11S,14S)-3,18-bis(2-aminoethoxy)-N-(cyanomethyl)-11-methyl-14-[methyl-[rac-(2S)-4-amino-2-[[2-(3-tert-butylphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 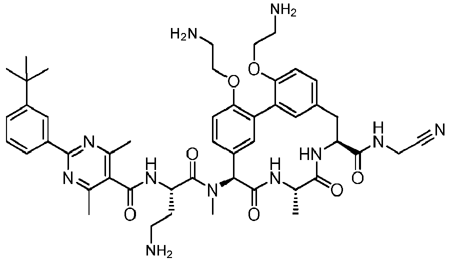 |
| **264** | rac-(8S,11S,14S)-3,18-bis(2-aminoethoxy)-N-(cyanomethyl)-11-methyl-14-[methyl-[rac-(2S)-4-amino-2-[[2-[4-(1-cyano-1-methyl-ethyl)phenyl]-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 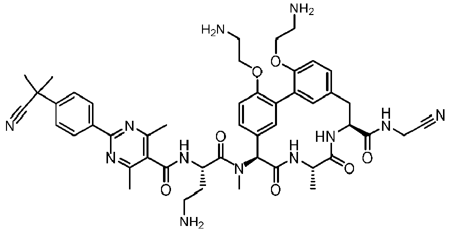 |
| **265** | rac-(8S,11S,14S)-3,18-bis(2-aminoethoxy)-N-(cyanomethyl)-11-methyl-14-[methyl-[rac-(2S)-4-amino-2-[[4,6-dimethyl-2-(4-vinylphenyl)pyrimidine-5- carbonyl]amino]butanoyl]amino- 10,13-dioxo-9,12-diazatricyclo[13.3.1.12,6]icosa-1(18),2(20),3,5,15(19),16-hexaene-8-carboxamide | 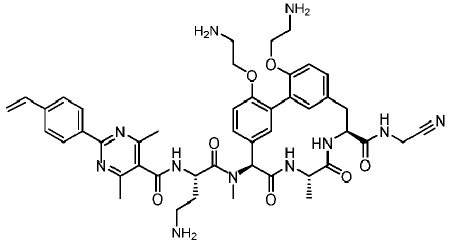 |
| **266** | rac-(8S,11S,14S)-3,18-bis(2-aminoethoxy)-N-(cyanomethyl)-11-methyl-14-[methyl-[rac-(2S)-4-amino-2-[[2-(4-isopropenylphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 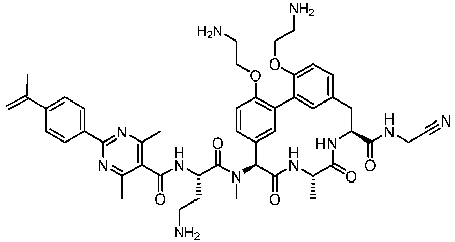 |
| **267** | rac-(8S,11S,14S)-3,18-bis(2-aminoethoxy)-N-(cyanomethyl)-11-methyl-14-[methyl-[rac-(2S)-4-amino-2-[[2-[4-(1-methoxy-1- methyl-ethyl)phenyl]-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 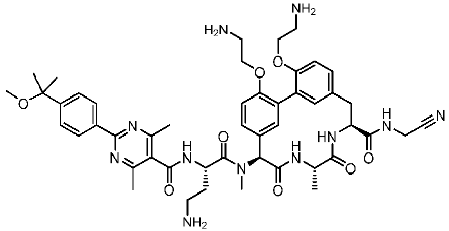 |
| **268** | rac-(8S,11S,14S)-3,18-bis(2-aminoethoxy)-N-(cyanomethyl)-11-methyl-14-[methyl-[rac-(2S)-4-amino-2-[[2-(3,4-dibutylphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 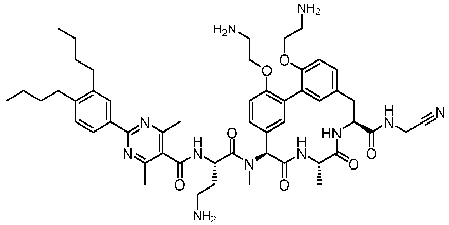 |
| **269** | rac-(8S,11S,14S)-3,18-bis(2-aminoethoxy)-N-(cyanomethyl)-11-methyl-14-[methyl-[rac-(2S)-4-amino-2-[[2-(1,1-dimethylindan-5-yl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 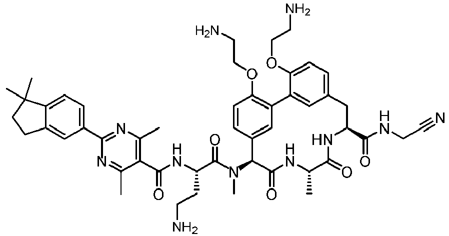 |
| **270** | rac-(8S,11S,14S)-3,18-bis(2-aminoethoxy)-N-(cyanomethyl)-11-methyl-14-[methyl-[rac-(2S)-4-amino-2-[[2-(1,1-dimethyltetralin-6-yl)-4,6-dimethyl-pyrimidine-5-carbonyl]amino]butanoyl]amino- 10,13-dioxo-9,12-diazatricyclo[13.3.1.12,6]icosa-l(18),2(20),3,5,15(19),16-hexaene-8-carboxamide | 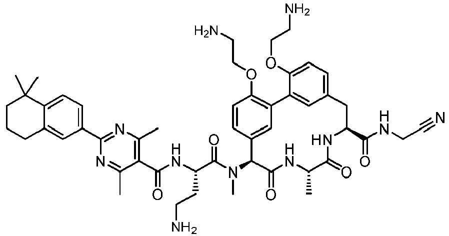 |
| **271** | rac-(8S,11S,14S)-3,18-bis(2-aminoethoxy)-N-(cyanomethyl)-11-methyl-14-[methyl-[rac-(2S)-4-amino-2-[[2-(4,4-dimethylchroman-7-yl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 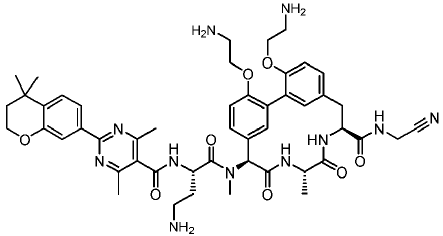 |
| **272** | rac-(8S,11S,14S)-3,18-bis(2-aminoethoxy)-N-(cyanomethyl)-11-methyl-14-[methyl-[rac-(2S)-4-amino-2-[[2-[4-(1,2-dimethylprop-1-enyl)phenyl]-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 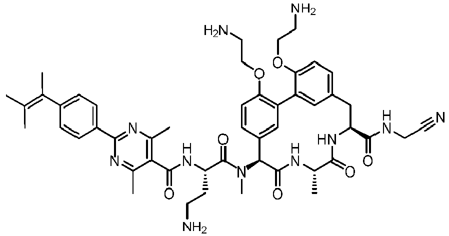 |
| **273** | rac-(8S,11S,14S)-3,18-bis(2-aminoethoxy)-N-(cyanomethyl)-11-methyl-14-[methyl-[rac-(2S)-4-amino-2-[[4,6-dimethyl-2-[4-(2-methylprop-1-enyl)phenyl]pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 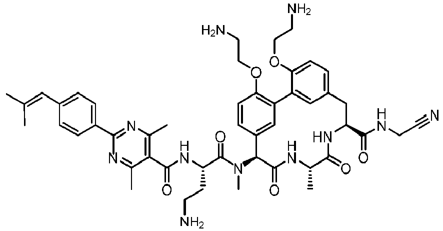 |
| **274** | rac-(8S,11S,14S)-3,18-bis(2-aminoethoxy)-N-(cyanomethyl)-11-methyl-14-[methyl-[rac-(2S)-4-amino-2-[[2-[4-(cyclopentylidenemethyl)phenyl]-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 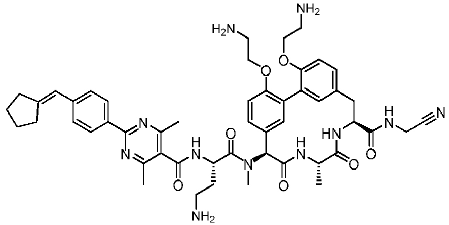 |
| **275** | rac-(8S,11S,14S)-3,18-bis(2-aminoethoxy)-N-(cyanomethyl)-11-methyl-14-[methyl-[rac-(2S)-4-amino-2-[[2-[4-(cyclopentylmethyl)phenyl]-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 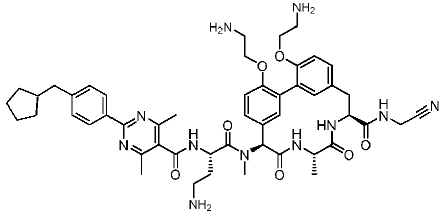 |
| **276** | rac-(8S,11S,14S)-3,18-bis(2-aminoethoxy)-N-(cyanomethyl)-11-methyl-14-[methyl-[rac-(2S)-4-amino-2-[[2-[4-(2-ethylbut-1-enyl)phenyl]-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 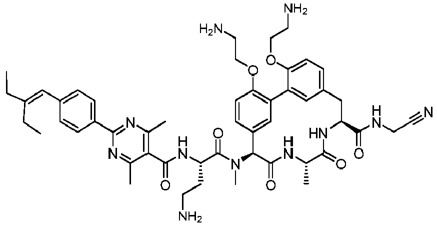 |
| **277** | rac-(8S,11S,14S)-3,18-bis(2-aminoethoxy)-N-(cyanomethyl)-11-methyl-14-methyl-[rac-(2S)-4-amino-2-[[2-[4-(2-ethylbutyl)phenyl]-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 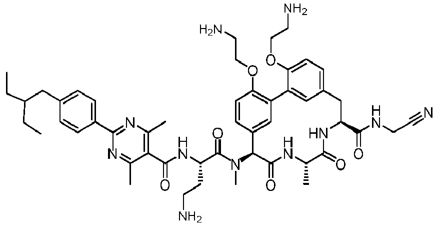 |
| **278** | rac-(8S,11S,14S)-3,18-bis(2-aminoethoxy)-N-(cyanomethyl)-11-methyl-14-[methyl-[rac-(2S)-4-amino-2-[[4,6-dimethyl-2-[4-[rac-(E)-pent-1-enyl]phenyl]pyrimidine-5-carbonyl]amino]butanoyl]amino]- 10,13-dioxo-9,12-diazatricyclo[13.3.1.12,6]icosa-1(18),2(20),3,5,15(19),16-hexaene-8-carboxamide | 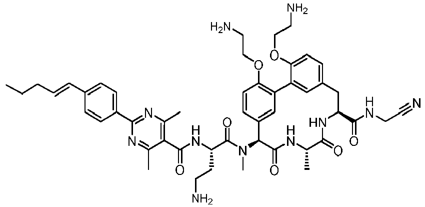 |
| **279** | rac-(8S,11S,14S)-3,18-bis(2-aminoethoxy)-N-(cyanomethyl)-11-methyl-14-[methyl-[rac-(2S)-4-amino-2-[[4,6-dimethyl-2-[4-[rac-(E)-but-1-enyl]phenyl]pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 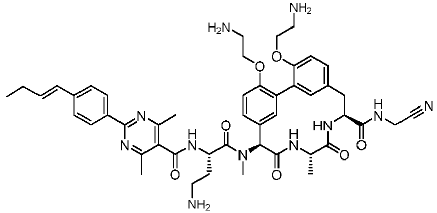 |
| **280** | rac-(8S,11S,14S)-3,18-bis(2-aminoethoxy)-N-(cyanomethyl)-11-methyl-14-[methyl-[rac-(2S)-4-amino-2-[[4,6-dimethyl-2-[4-(1-methylcyclopropyl)phenyl]pyrimidin e-5-carbonyl]amino]butanoyl]amino]]-10,13-dioxo-9,12-diazatricyclo[13.3.1.12,6]icosa-1(18),2(20),3,5,15(19),16-hexaene-8-carboxamide | 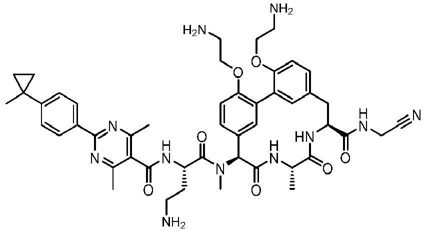 |
| **281** | rac-(8S,11S,14S)-3,18-bis(2-aminoethoxy)-N-(cyanomethyl)-11-methyl-14-[methyl-[rac-(2S)-4-amino-2-[[2-[4-(1-ethylcyclopropyl)phenyl]-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 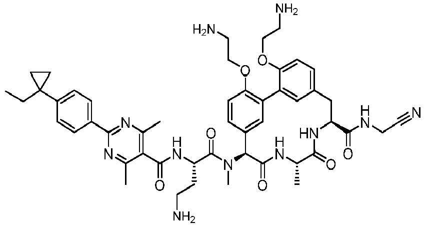 |
| **282** | rac-(8S,11S,14S)-3,18-bis(2-aminoethoxy)-N-(cyanomethyl)-11-methyl-14-[methyl-[rac-(2S)-4-amino-2-[[4,6-dimethyl-2-[4-(1-propylcyclopropyl)phenyl]pyrimidin e-5-carbonyl]amino]butanoyl]amino- 10,13-dioxo-9,12-diazatricyclo[13.3.1.12,6]icosa-l(18),2(20),3,5,15(19),16-hexaene-8-carboxamide | 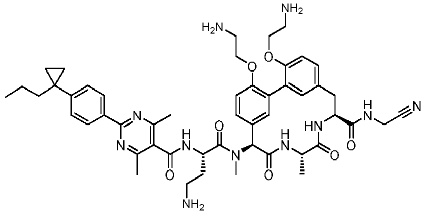 |
| **283** | rac-(8S,11S,14S)-3,18-bis(2-aminoethoxy)-N-(cyanomethyl)-11-methyl-14-[methyl-[rac-(2S)-4-amino-2-[[2-[4-(1-butylcyclopropyl)phenyl]-4,6-dimethyl-pyrimidine-5-carbonyl]amino]butanoyl]amino- 10,13-dioxo-9,12-diazatricyclo[13.3.1.12,6]icosa-l(18),2(20),3,5,15(19),16-hexaene-8-carboxamide | 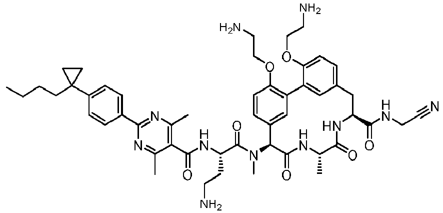 |
| **284** | rac-(8S,11S,14S)-3,18-bis(2-aminoethoxy)-N-(cyanomethyl)-11-methyl-14-[methyl-[rac-(2S)-4-amino-2-[(4,6-dimethyl-2-spiro[cyclopropane-1,1'-indane]-5'-yl-pyrimidine-5-carbonyl)amino]butanoyl]amino]]-10,13-dioxo-9,12-diazatricyclo[13.3.1.12,6]icosa-1(18),2(20),3,5,15(19),16-hexaene-8-carboxamide | 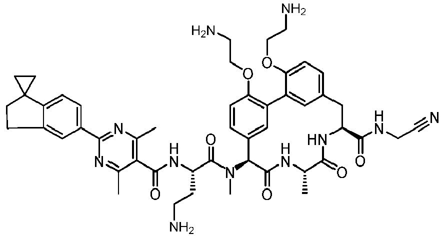 |
| **285** | rac-(8S,11S,14S)-3,18-bis(2-aminoethoxy)-N-(cyanomethyl)-11-methyl-14-[methyl-[rac-(2S)-4-amino-2-[(4,6-dimethyl-2-spiro[chromane-4,1'-cyclopropane]-7-yl-pyrimidine-5-carbonyl)amino]butanoyl]amino]]-10,13-dioxo-9,12-diazatricyclo[13.3.1.12,6]icosa-1(18),2(20),3,5,15(19),16-hexaene-8-carboxamide | 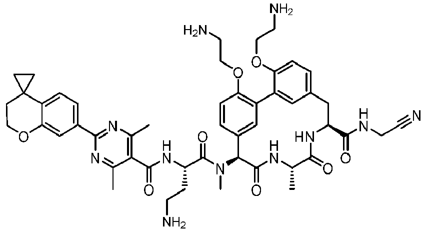 |
| **286** | rac-(8S,11S,14S)-3,18-bis(2-aminoethoxy)-N-(cyanomethyl)-11-methyl-14-[methyl-[rac-(2S)-4-amino-2-[[2-[4-(1-cyclopentylcyclopropyl)phenyl]-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 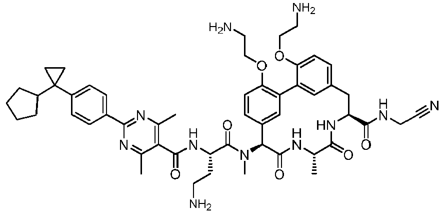 |
| **287** | rac-(8S,11S,14S)-3,18-bis(2-aminoethoxy)-N-(cyanomethyl)-11-methyl-14-[methyl-[rac-(2S)-4-amino-2-[[2-[(4-tert-butylphenyl)methyl]-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 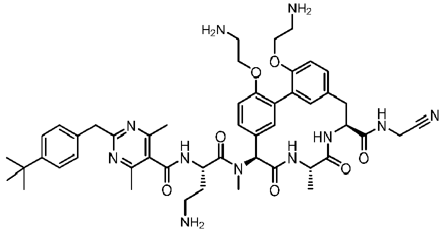 |
| **288** | rac-(8S,11S,14S)-3,18-bis(2-aminoethoxy)-N-(cyanomethyl)-11-methyl-14-[methyl-[rac-(2S)-4-amino-2-[[2-[1-(4-tert-butylphenyl)-1-methyl-ethyl]-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 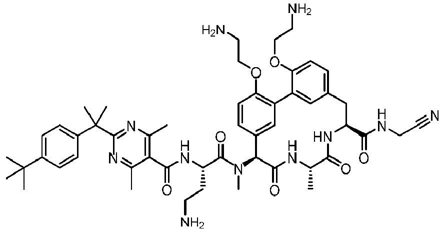 |
| **289** | rac-(8S,11S,14S)-3,18-bis(2-aminoethoxy)-N-(cyanomethyl)-11-methyl-14-[methyl-[rac-(2S)-4-amino-2-[[2-[(4-tert-butylphenyl)-difluoro-methyl]-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 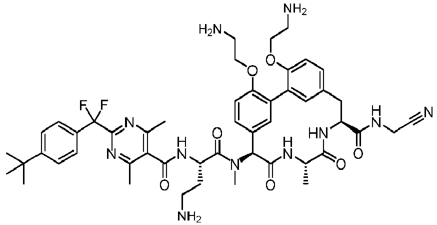 |
| **290** | rac-(8S,11S,14S)-3,18-bis(2-aminoethoxy)-N-(cyanomethyl)-11-methyl-14-[methyl-[rac-(2S)-4-amino-2-[[2-(1,1-dimethylisochroman-6-yl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 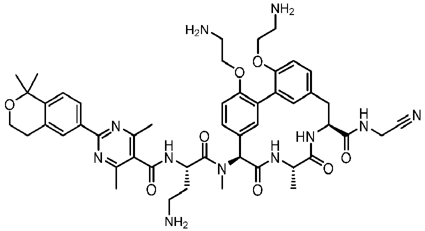 |
| **291** | rac-(8S,11S,14S)-3,18-bis(2-aminoethoxy)-N-(cyanomethyl)-11-methyl-14-[methyl-[rac-(2S)-4-amino-2-[[2-(2,2-dimethyl-3H-benzofuran-5-yl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 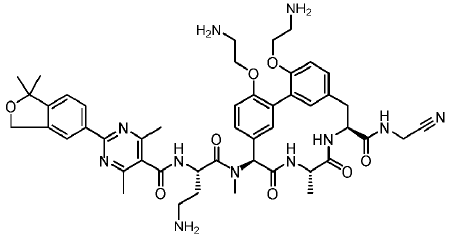 |
| **292** | rac-(8S,11S,14S)-3,18-bis(2-aminoethoxy)-N-(cyanomethyl)-11-methyl-14-[methyl-[rac-(2S)-4-amino-2-[[2-(3,3-dimethyl-2H-benzofuran-6-yl)-4,6-dimethyl-pyrimidine-5-carbonyl]amino]butanoyl]amino- 10,13-dioxo-9,12-diazatricyclo[13.3.1.12,6]icosa-l(18),2(20),3,5,15(19),16-hexaene-8-carboxamide | 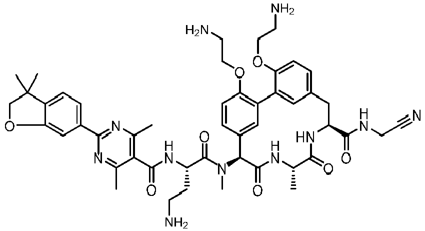 |
| **293** | rac-(8S,11S,14S)-3,18-bis(2-aminoethoxy)-N-(cyanomethyl)-11-methyl-14-[methyl-[rac-(2S)-4-amino-2-[[2-(5-butyl-2-pyridyl)-4,6-dimethyl-pyrimidine-5-carbonyl]amino]butanoyl]amino- 10,13-dioxo-9,12-diazatricyclo[13.3.1.12,6]icosa-l(18),2(20),3,5,15(19),16-hexaene-8-carboxamide | 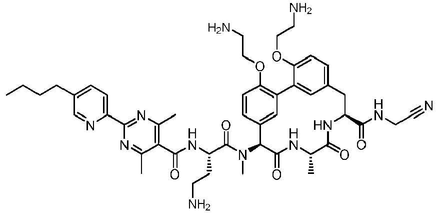 |
| **294** | rac-(8S,11S,14S)-3,18-bis(2-aminoethoxy)-N-(cyanomethyl)-11-methyl-14-[methyl-[rac-(2S)-4-amino-2-[[2-[4-(l,l-difluorobutyl)phenyl]-4,6-dimethyl-pyrimidine-5-carbonyl]amino]butanoyl]amino- 10,13-dioxo-9,12-diazatricyclo[13.3.1.12,6]icosa-l(18),2(20),3,5,15(19),16-hexaene-8-carboxamide | 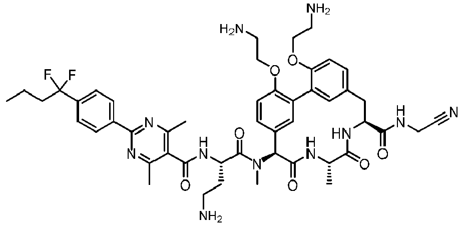 |
| **295** | rac-(8S,11S,14S)-3,18-bis(2-aminoethoxy)-N-(cyanomethyl)-11-methyl-14-[methyl-[rac-(2S)-4-amino-2-[[2-(4-tert-butylcyclohexen-1-yl)-4,6-dimethyl-pyrimidine-5-carbonyl]amino]butanoyl]amino]]-10,13-dioxo-9,12-diazatricyclo[13.3.1.12,6]icosa-1(18),2(20),3,5,15(19),16-hexaene-8-carboxamide | 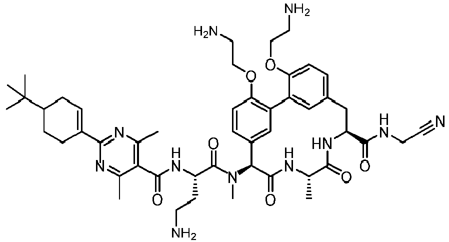 |
| **296** | rac-(8S,11S,14S)-3,18-bis(2-aminoethoxy)-N-(cyanomethyl)-11-methyl-14-[methyl-[rac-(2S)-4-amino-2-[[2-(4-tert-butylcyclohexyl)-4,6-dimethyl-pyrimidine-5-carbonyl]amino]butanoyl]amino]]-10,13-dioxo-9,12-diazatricyclo[13.3.1.12,6]icosa-1(18),2(20),3,5,15(19),16-hexaene-8-carboxamide | 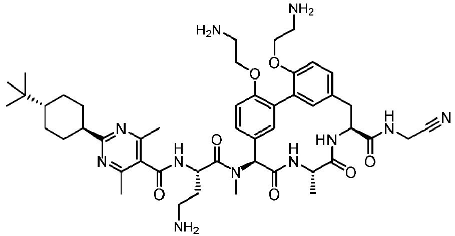 |
| **297** | rac-(8S,11S,14S)-3,18-bis(2-aminoethoxy)-N-(cyanomethyl)-11-methyl-14-[methyl-[rac-(2S)-4-amino-2-[[2-(4-tert-butylcyclohexyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 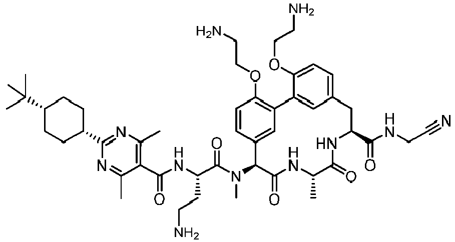 |
| **298** | rac-(8S,11S,14S)-3,18-bis(2-aminoethoxy)-N-(cyanomethyl)-11-methyl-14-[methyl-[rac-(2S)-4-amino-2-[[2-(4-tert-butyl-2-hydroxyphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 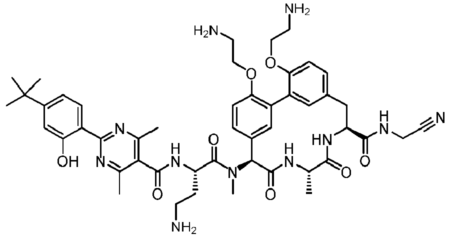 |
| **299** | rac-(8S,11S,14S)-3,18-bis(2-aminoethoxy)-N-(cyanomethyl)-11-methyl-14-[methyl-[rac-(2S)-4-amino-2-[[2-(4-tert-butyl-2-fluorophenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 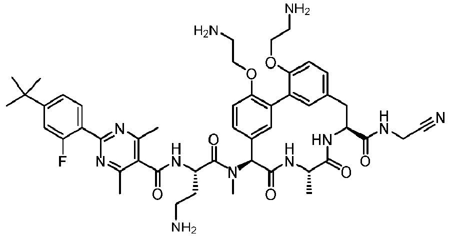 |
| **300** | rac-(8S,11S,14S)-3,18-bis(2-aminoethoxy)-N-(cyanomethyl)-11-methyl-14-[methyl-[rac-(2S)-4-amino-2-[[2-(4-cyclopropylphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 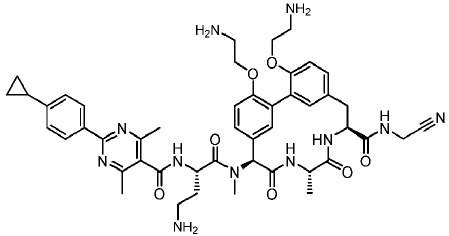 |
| **301** | rac-(8S,11S,14S)-3,18-bis(2-aminoethoxy)-N-(cyanomethyl)-11-methyl-14-[methyl-[rac-(2S)-4-amino-2-[[2-(4-cyclobutylphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 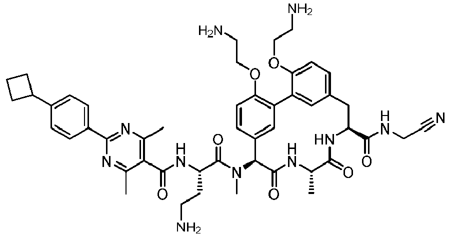 |
| **302** | rac-(8S,11S,14S)-3,18-bis(2-aminoethoxy)-N-(cyanomethyl)-11-methyl-14-[methyl-[rac-(2S)-4-amino-2-[[2-(4-cyclopentylphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 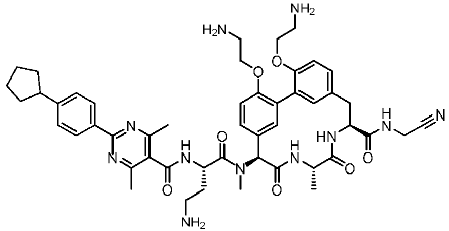 |
| **303** | rac-(8S,11S,14S)-3,18-bis(2-aminoethoxy)-N-(cyanomethyl)-11-methyl-14-[methyl-[rac-(2S)-4-amino-2-[[2-[4-(cyclohexen-1- yl)phenyl]-4,6-dimethyl-pyrimidine-5-carbonyl]amino]butanoyl]amino]- 10,13-dioxo-9,12-diazatricyclo[13.3.1.12,6]icosa-1(18),2(20),3,5,15(19),16-hexaene-8-carboxamide | 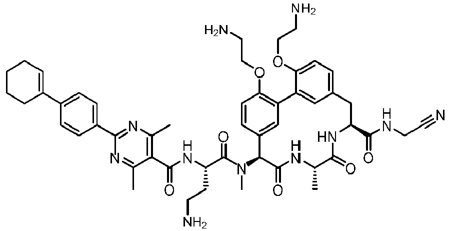 |
| **304** | rac-(8S,11S,14S)-3,18-bis(2-aminoethoxy)-N-(cyanomethyl)-11-methyl-14-[methyl-[rac-(2S)-4-amino-2-[[2-(4-cyclohexylphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 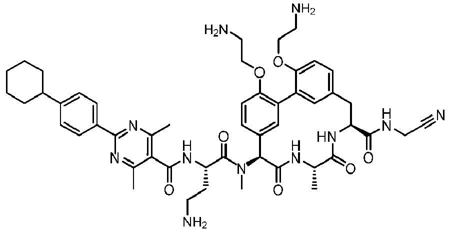 |
| **305** | rac-(8S,11S,14S)-3,18-bis(2-aminoethoxy)-N-(cyanomethyl)-11-methyl-14-[methyl-[rac-(2S)-4-amino-2-[[2-(4-cycloheptylphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 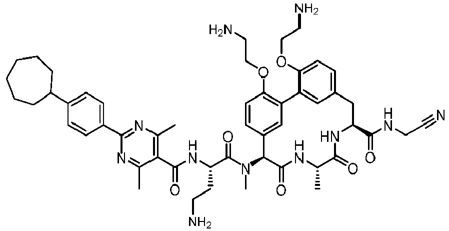 |
| **306** | rac-(8S,11S,14S)-3,18-bis(2-aminoethoxy)-N-(cyanomethyl)-11-methyl-14-[methyl-[rac-(2S)-4-amino-2-[[2-(3,3-dimethylbut-1-ynyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 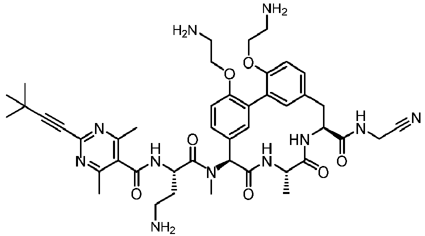 |
| **307** | rac-(8S,11S,14S)-3,18-bis(2-aminoethoxy)-N-(cyanomethyl)-11-methyl-14-[methyl-[rac-(2S)-4-amino-2-[[2-[2-(4-ethylphenyl)ethynyl]-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 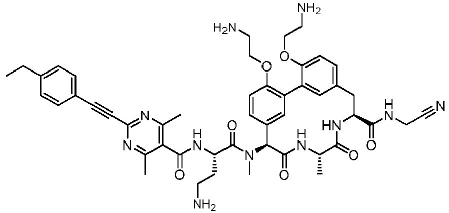 |
| **308** | rac-(8S,11S,14S)-3,18-bis(2-aminoethoxy)-N-(cyanomethyl)-11-methyl-14-[methyl-[rac-(2S)-4-amino-2-[[2-[2-(4-butylphenyl)ethynyl]-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 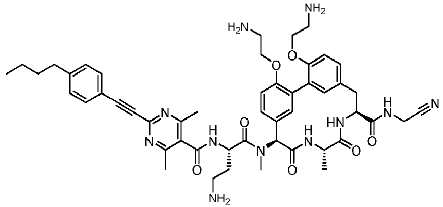 |
| **309** | rac-(8S,11S,14S)-3,18-bis(2-aminoethoxy)-N-(cyanomethyl)-11-methyl-14-[methyl-[rac-(2S)-4-amino-2-[[4,6-dimethyl-2-(2-phenylethynyl)pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 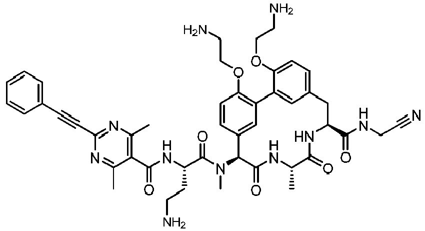 |
| **310** | rac-(8S,11S,14S)-3,18-bis(2-aminoethoxy)-N-(cyanomethyl)-11-methyl-14-[methyl-[rac-(2S)-4-amino-2-[[4,6-dimethyl-2-[2-(4-vinylphenyl)ethynyl]pyrimidine-5- carbonyl]amino]butanoyl]amino- 10,13-dioxo-9,12-diazatricyclo[13.3.1.12,6]icosa-1(18),2(20),3,5,15(19),16-hexaene-8-carboxamide | 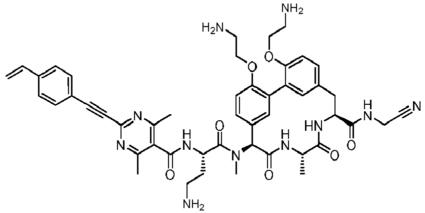 |
| **311** | rac-(8S,11S,14S)-3,18-bis(2-aminoethoxy)-N-(cyanomethyl)-11-methyl-14-[methyl-[rac-(2S)-4-amino-2-[[2-[2-(4-tert-butylphenyl)ethynyl]-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 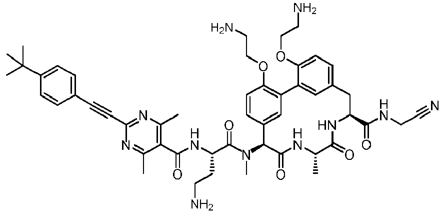 |
| **312** | rac-(8S,11S,14S)-3,18-bis(2-aminoethoxy)-N-(cyanomethyl)-11-methyl-14-[methyl-[rac-(2S)-4-amino-2-[[2-[2-[4-(1,2-dimethylprop-1-enyl)phenyl]ethynyl]-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 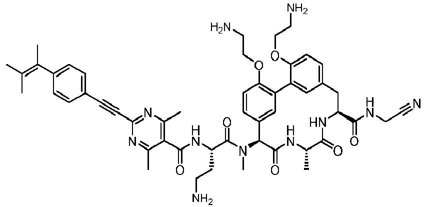 |
| **313** | rac-(8S,11S,14S)-3,18-bis(2-aminoethoxy)-N-(cyanomethyl)-11-methyl-14-[methyl-[rac-(2S)-4-amino-2-[[4,6-dimethyl-2-[2-[4-(2-methylprop-1-enyl)phenyl]ethynyl]pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 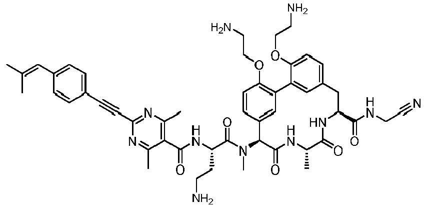 |
| **314** | rac-(8S,11S,14S)-3,18-bis(2-aminoethoxy)-N-(cyanomethyl)-11-methyl-14-[methyl-[rac-(2S)-4-amino-2-[[2-[2-(4-isopropenylphenyl)ethynyl]-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 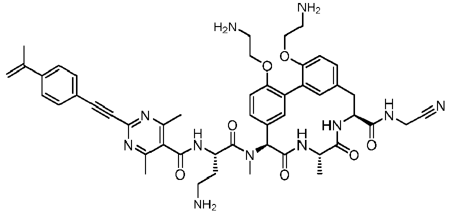 |
| **315** | rac-(8S,11S,14S)-3,18-bis(2-aminoethoxy)-N-(cyanomethyl)-11-methyl-14-[methyl-[rac-(2S)-4-amino-2-[[2-(4-tert-butyl-1- piperidyl)-4,6-dimethyl-pyrimidine-5-carbonyl]amino]butanoyl]amino]- 10,13-dioxo-9,12-diazatricyclo[13.3.1.12,6]icosa-1(18),2(20),3,5,15(19),16-hexaene-8-carboxamide | 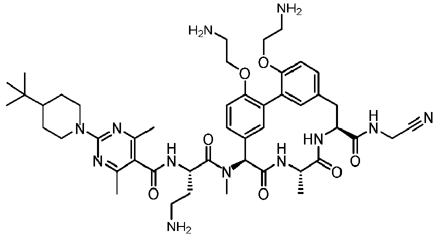 |
| **316** | rac-(8S,11S,14S)-3,18-bis(2-aminoethoxy)-N-(cyanomethyl)-11-methyl-14-[methyl-[rac-(2S)-4-amino-2-[[2-(3-tert-butylazetidin-1-yl)-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 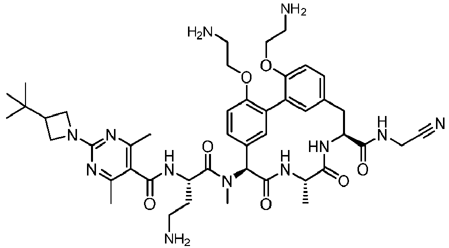 |
| **317** | rac-(8S,11S,14S)-3,18-bis(2-aminoethoxy)-N-(cyanomethyl)-11-methyl-14-[methyl-[rac-(2S)-4-amino-2-[[4,6-dimethyl-2-(4-pentoxy-1-piperidyl)pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 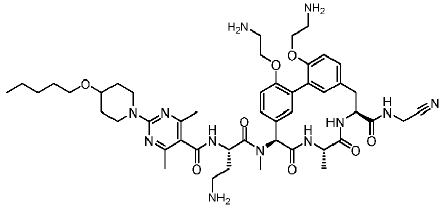 |
| **318** | rac-(8S,11S,14S)-3,18-bis(2-aminoethoxy)-N-(cyanomethyl)-11-methyl-14-[methyl-[rac-(2S)-4-amino-2-[[2-[4-(4-tert-butylphenyl)piperazin-1-yl]-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 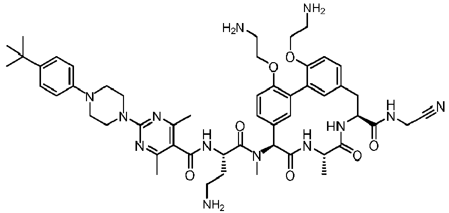 |
| **319** | rac-(8S,11S,14S)-3,18-bis(2-aminoethoxy)-N-(cyanomethyl)-11-methyl-14-[methyl-[rac-(2S)-4-amino-2-[[2-(4-tert-butyl-2-oxo-1-pyridyl)-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 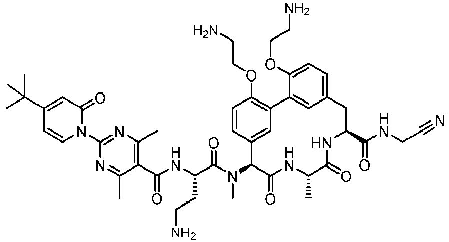 |
| **320** | rac-(8S,11S,14S)-3,18-bis(2-aminoethoxy)-N-(cyanomethyl)-11-methyl-14-[methyl-[rac-(2S)-4-amino-2-[[2-[4-(4-tert-butyl-1-piperidyl)phenyl]-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 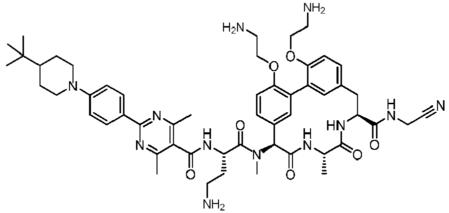 |
| **321** | rac-(8S,11S,14S)-3,18-bis(2-aminoethoxy)-N-(cyanomethyl)-11-methyl-14-[methyl-[rac-(2S)-4-amino-2-[[4,6-dimethyl-2-(4-pyrrolidin-1-ylphenyl)pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 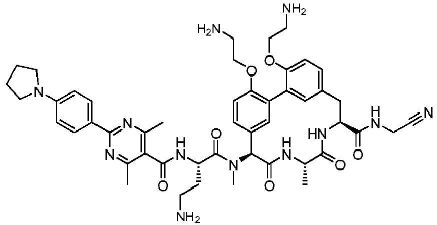 |
| **322** | rac-(8S,11S,14S)-3,18-bis(2-aminoethoxy)-N-(cyanomethyl)-11-methyl-14-[methyl-[rac-(2S)-4-amino-2-[[2-[4-(4,4-difluoro-1- piperidyl)phenyl]-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 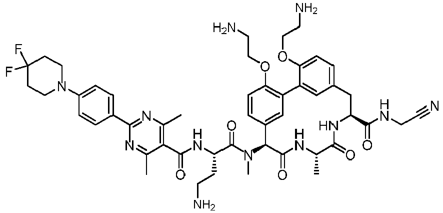 |
| **323** | rac-(8S,11S,14S)-3,18-bis(2-aminoethoxy)-N-(cyanomethyl)-11-methyl-14-[methyl-[rac-(2S)-4-amino-2-[[2-[4-(azetidin-1- yl)phenyl]-4,6-dimethyl-pyrimidine-5-carbonyl]amino]butanoyl]amino]- 10,13-dioxo-9,12-diazatricyclo[13.3.1.12,6]icosa-1(18),2(20),3,5,15(19),16-hexaene-8-carboxamide | 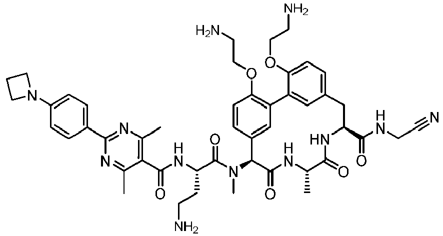 |
| **324** | rac-(8S,11S,14S)-3,18-bis(2-aminoethoxy)-N-(cyanomethyl)-11-methyl-14-[methyl-[rac-(2S)-4-amino-2-[[4,6-dimethyl-2-[4-(1-piperidyl)phenyl]pyrimidine-5- carbonyl]amino]butanoyl]amino]]-10,13-dioxo-9,12-diazatricyclo[13.3.1.12,6]icosa-1(18),2(20),3,5,15(19),16-hexaene-8-carboxamide | 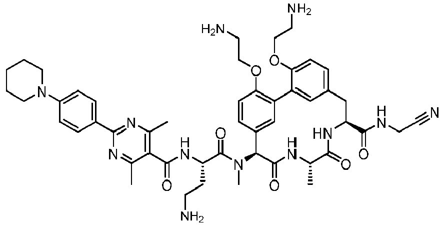 |
| **325** | rac-(8S,11S,14S)-3,18-bis(2-aminoethoxy)-N-(cyanomethyl)-11-methyl-14-[methyl-[rac-(2S)-4-amino-2-[[2-[4-(azepan-1- yl)phenyl]-4,6-dimethyl-pyrimidine-5-carbonyl]amino]butanoyl]amino]- 10,13-dioxo-9,12-diazatricyclo[13.3.1.12,6]icosa-1(18),2(20),3,5,15(19),16-hexaene-8-carboxamide | 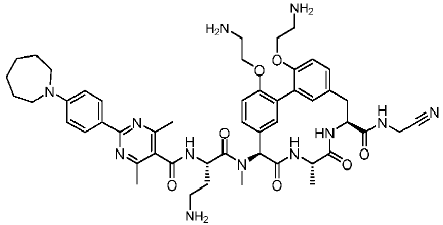 |
| **326** | rac-(8S,11S,14S)-3,18-bis(2-aminoethoxy)-N-(cyanomethyl)-11-methyl-14-[methyl-[rac-(2S)-4-amino-2-[[2-[4-(azocan-1-yl)phenyl]-4,6-dimethyl-pyrimidine-5-carbonyl]amino]butanoyl]amino]- 10,13-dioxo-9,12-diazatricyclo[13.3.1.12,6]icosa-1(18),2(20),3,5,15(19),16-hexaene-8-carboxamide | 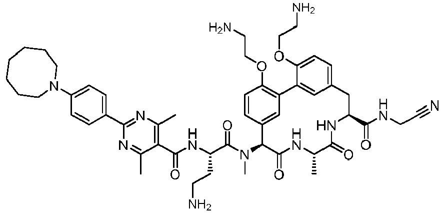 |
| **327** | rac-(8S,11S,14S)-3,18-bis(2-aminoethoxy)-N-(cyanomethyl)-11-methyl-14-[methyl-[rac-(2S)-4-amino-2-[[4,6-dimethyl-2-[4-[rac-(3S,SR)-3,5-dimethyl-1-piperidyl]phenyl]pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 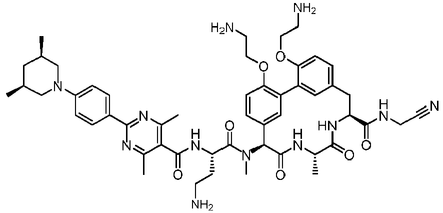 |
| **328** | rac-(8S,11S,14S)-3,18-bis(2-aminoethoxy)-N-(cyanomethyl)-11-methyl-14-[methyl-[rac-(2S)-4-amino-2-[[4,6-dimethyl-2-(4-pentoxyphenyl)pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 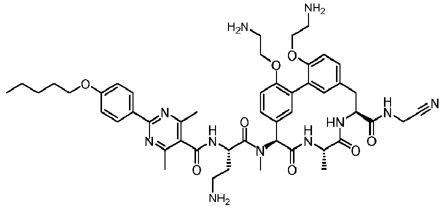 |
| **329** | rac-(8S,11S,14S)-3,18-bis(2-aminoethoxy)-N-(cyanomethyl)-11-methyl-14-[methyl-[rac-(2S)-4-amino-2-[[2-(4-isopentyloxyphenyl)-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 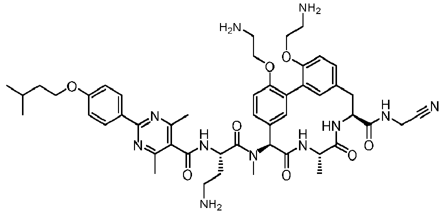 |
| **330** | rac-(8S,11S,14S)-3,18-bis(2-aminoethoxy)-N-(cyanomethyl)-11-methyl-14-[methyl-[rac-(2S)-4-amino-2-[[4,6-dimethyl-2-(4-propoxyphenyl)pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 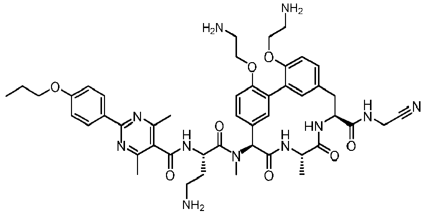 |
| **331** | rac-(8S,11S,14S)-3,18-bis(2-aminoethoxy)-N-(cyanomethyl)-11-methyl-14-[methyl-[rac-(2S)-4-amino-2-[[2-(4-butoxyphenyl)-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 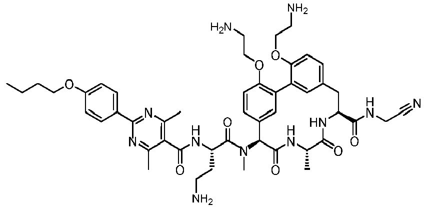 |
| **332** | rac-(8S,11S,14S)-3,18-bis(2-aminoethoxy)-N-(cyanomethyl)-11-methyl-14-[methyl-[rac-(2S)-4-amino-2-[[2-(4-hexoxyphenyl)-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 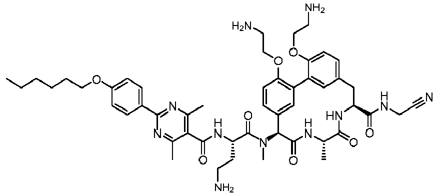 |
| **333** | rac-(8S,11S,14S)-3,18-bis(2-aminoethoxy)-N-(cyanomethyl)-11-methyl-14-[methyl-[rac-(2S)-4-amino-2-[[2-[4-(2-methoxyethoxy)phenyl]-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 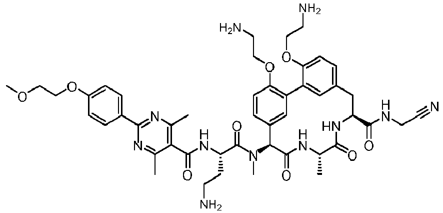 |
| **334** | rac-(8S,11S,14S)-3,18-bis(2-aminoethoxy)-N-(cyanomethyl)-11-methyl-14-[methyl-[rac-(2S)-4-amino-2-[[2-(2,3-dihydro-1,4-benzodioxin-6-yl)-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 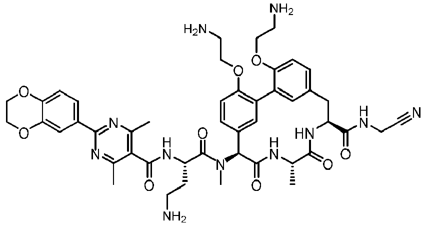 |
| **335** | rac-(8S,11S,14S)-3,18-bis(2-aminoethoxy)-N-(cyanomethyl)-11-methyl-14-[methyl-[rac-(2S)-4-amino-2-[[2-[4-(cyclobutoxy)phenyl]-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 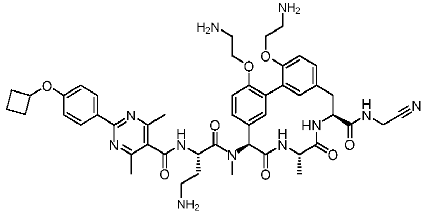 |
| **336** | rac-(8S,11S,14S)-3,18-bis(2-aminoethoxy)-N-(cyanomethyl)-11-methyl-14-[methyl-[rac-(2S)-4-amino-2-[[2-[4-(cyclopentoxy)phenyl]-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 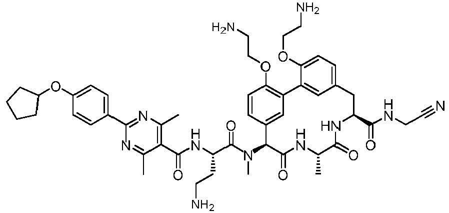 |
| **337** | rac-(8S,11S,14S)-3,18-bis(2-aminoethoxy)-N-(cyanomethyl)-11-methyl-14-[methyl-[rac-(2S)-4-amino-2-[[2-[4-(cyclohexoxy)phenyl]-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 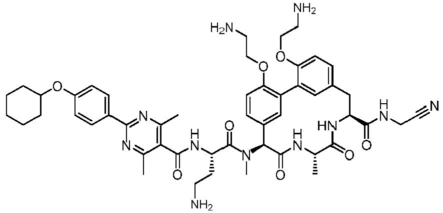 |
| **338** | rac-(8S,11S,14S)-3,18-bis(2-aminoethoxy)-N-(cyanomethyl)-11-methyl-14-[methyl-[rac-(2S)-4-amino-2-[[2-[4-(3,3-dimethylbutoxy)phenyl]-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 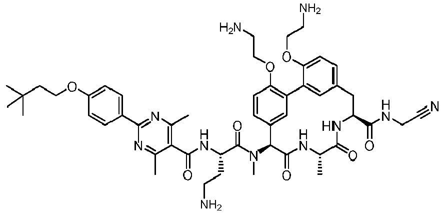 |
| **339** | rac-(8S,11S,14S)-3,18-bis(2-aminoethoxy)-N-(cyanomethyl)-11-methyl-14-[methyl-[rac-(2S)-4-amino-2-[[2-[4-(1-ethylpropoxy)phenyl]-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 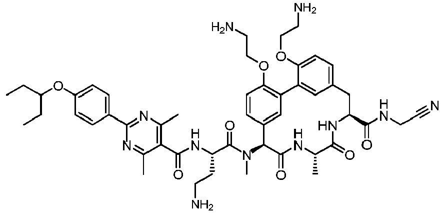 |
| **340** | rac-(8S,11S,14S)-3,18-bis(2-aminoethoxy)-N-(cyanomethyl)-11-methyl-14-[methyl-[rac-(2S)-4-amino-2-[[2-[4-(cycloheptoxy)phenyl]-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 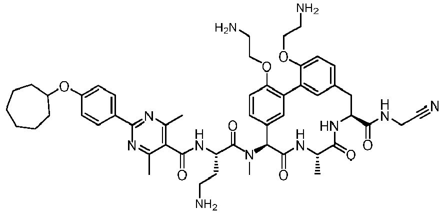 |
| **341** | rac-(8S,11S,14S)-3,18-bis(2-aminoethoxy)-N-(cyanomethyl)-11-methyl-14-[methyl-[rac-(2S)-4-amino-2-[[4,6-dimethyl-2-[4-(1-propylbutoxy)phenyl]pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 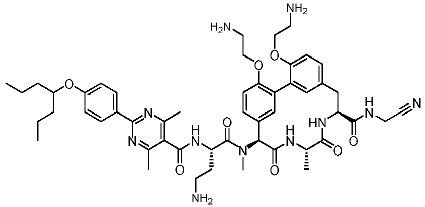 |
| **342** | rac-(8S,11S,14S)-3,18-bis(2-aminoethoxy)-N-(cyanomethyl)-11-methyl-14-[methyl-[rac-(2S)-4-amino-2-[[2-(4-tert-butoxyphenyl)-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 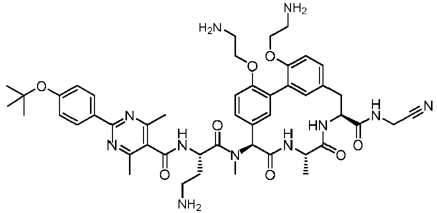 |
| **343** | rac-(8S,11S,14S)-3,18-bis(2-aminoethoxy)-N-(cyanomethyl)-11-methyl-14-[methyl-[rac-(2S)-4-amino-2-[[2-[4-(tert-butoxymethyl)phenyl]-4,6-dimethyl-pyrimidine-5-carbonyl]amino]butanoyl]amino]-10,13-dioxo-9,12-diazatricyclo[13.3.1.12,6]icosa-l(18),2(20),3,5,15(19),16-hexaene-8-carboxamide | 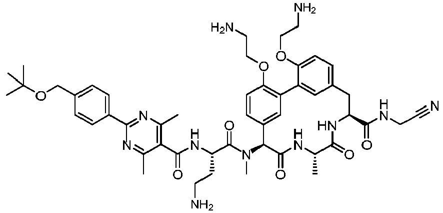 |
| **344** | rac-(8S,11S,14S)-3,18-bis(2-aminoethoxy)-N-(cyanomethyl)-11-methyl-14-[methyl-[rac-(2S)-4-amino-2-[[4,6-dimethyl-2-[4-(1-methylcyclopropoxy)phenyl]pyrimid ine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 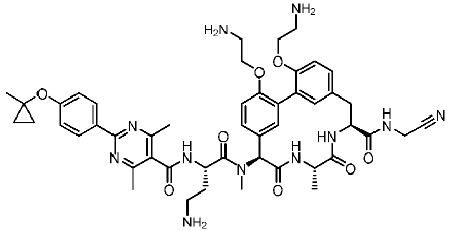 |
| **345** | rac-(8S,11S,14S)-3,18-bis(2-aminoethoxy)-N-(cyanomethyl)-11-methyl-14-[methyl-[rac-(2S)-4-amino-2-[[2-[4-(1-ethylcyclopropoxy)phenyl]-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 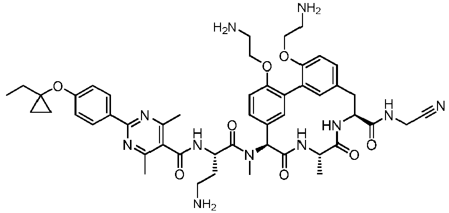 |
| **346** | rac-(8S,11S,14S)-3,18-bis(2-aminoethoxy)-N-(cyanomethyl)-11-methyl-14-[methyl-[rac-(2S)-4-amino-2-[[4,6-dimethyl-2-[4-(1-propylcyclopropoxy)phenyl]pyrimid ine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 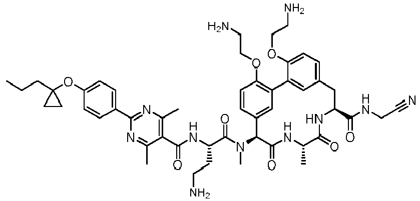 |
| **347** | rac-(8S,11S,14S)-3,18-bis(2-aminoethoxy)-N-(cyanomethyl)-11-methyl-14-[methyl-[rac-(2S)-4-amino-2-[[2-[4-(1-butylcyclopropoxy)phenyl]-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 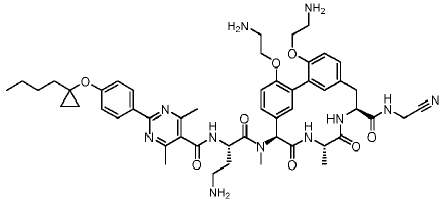 |
| **348** | rac-(8S,11S,14S)-3,18-bis(2-aminoethoxy)-N-(cyanomethyl)-11-methyl-14-[methyl-[rac-(2S)-4-amino-2-[[2-[4-(4,4-dimethylcyclohexoxy)phenyl]-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 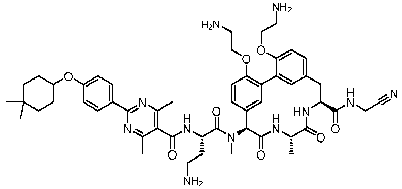 |
| **349** | rac-(8S,11S,14S)-3,18-bis(2-aminoethoxy)-N-(cyanomethyl)-11-methyl-14-[methyl-[rac-(2S)-4-amino-2-[[4,6-dimethyl-2-[4-[[rac-(3aR,7aS)-2,3,3a,4,5,6,7,7a-octahydro-1H-inden-2-yl]oxy]phenyl]pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 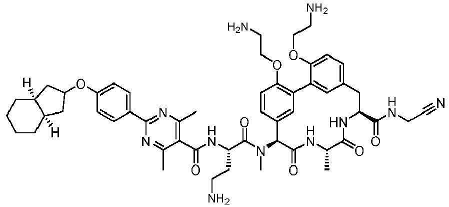 |
| **350** | rac-(8S,11S,14S)-3,18-bis(2-aminoethoxy)-N-(cyanomethyl)-11-methyl-14-[methyl-[rac-(2S)-4-amino-2-[[4,6-dimethyl-2-[4-[rac-(1S,4R)-norbornan-2-yl]oxyphenyl]pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 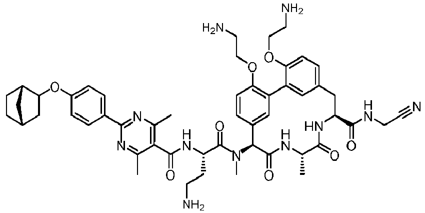 |
| **351** | rac-(8S,11S,14S)-3,18-bis(2-aminoethoxy)-N-(cyanomethyl)-11-methyl-14-[methyl-[rac-(2S)-4-amino-2-[[2-(4-butoxy-2,3,5,6-tetrafluoro-phenyl)-4,6-dimethy-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 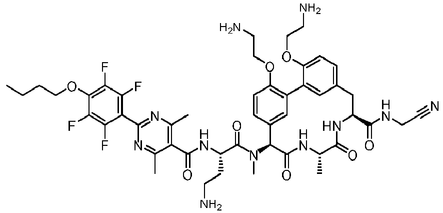 |
| **352** | rac-(8S,11S,14S)-3,18-bis(2-aminoethoxy)-N-(cyanomethyl)-11-methyl-14-[methyl-[rac-(2S)-4-amino-2-[[4,6-dimethyl-2-(4-pentylsulfanylphenyl)pyrimidine-5-carbonyl]amino]butanoyl]amino]-10,13-dioxo-9,12-diazatricyclo[13.3.1.12,6]icosa-l(18),2(20),3,5,15(19),16-hexaene-8-carboxamide | 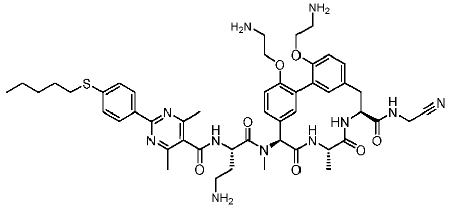 |
| **353** | rac-(8S,11S,14S)-3,18-bis(2-aminoethoxy)-N-(cyanomethyl)-11-methyl-14-[methyl-[rac-(2S)-4-amino-2-[[2-(4-tert-butylphenoxy)-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 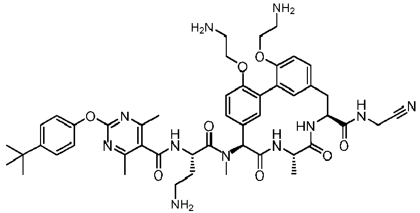 |
| **354** | rac-(8S,11S,14S)-3,18-bis(2-aminoethoxy)-N-(cyanomethyl)-11-methyl-14-[methyl-[rac-(2S)-4-amino-2-[[2-(4-butylphenoxy)-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 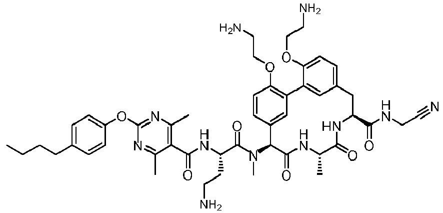 |
| **355** | rac-(8S,11S,14S)-3,18-bis(2-aminoethoxy)-N-(cyanomethyl)-11-methyl-14-[methyl-[rac-(2S)-4-amino-2-[[2-(3,4-dibutylphenoxy)-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 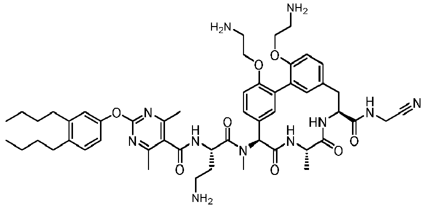 |
| **356** | rac-(8S,11S,14S)-3,18-bis(2-aminoethoxy)-N-(cyanomethyl)-11-methyl-14-[methyl-[rac-(2S)-4-amino-2-[[2-[4-(4,4-dimethylpentoxy)phenyl]-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 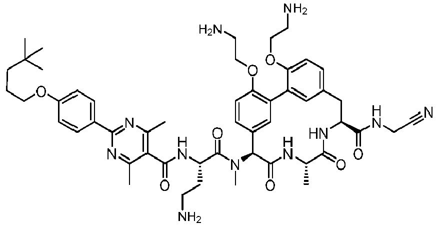 |
| **357** | rac-(8S,11S,14S)-3,18-bis(2-aminoethoxy)-N-(cyanomethyl)-11-methyl-14-[methyl-[rac-(2S)-4-amino-2-[[2-[4-(5,5-dimethylhexoxy)phenyl]-4,6-dimethyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 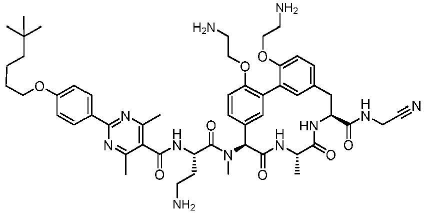 |
| **358** | rac-(8S,11S,14S)-3,18-bis(2-aminoethoxy)-N-(cyanomethyl)-11-methyl-14-[methyl-[rac-(2S)-4-amino-2-[[6-(4-tert-butylphenyl)-2,4-dimethyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 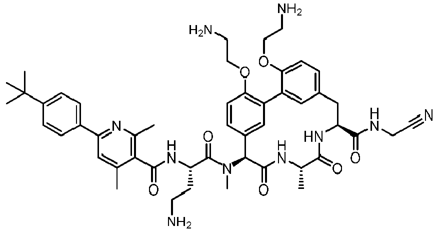 |
| **359** | rac-(8S,11S,14S)-3,18-bis(2-aminoethoxy)-N-(cyanomethyl)-11-methyl-14-[methyl-[rac-(2S)-4-amino-2-[[6-[4-(2,2-dimethylpropoxy)phenyl]-2,4-dimethyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 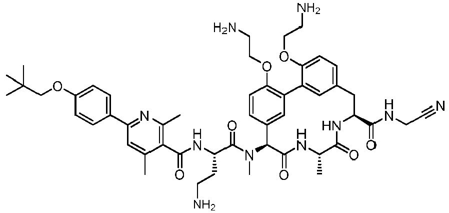 |
| **360** | rac-(8S,11S,14S)-3,18-bis(2-aminoethoxy)-N-(cyanomethyl)-11-methyl-14-[methyl-[rac-(2S)-4-amino-2-[[6-(4-isopropoxyphenyl)-2,4-dimethyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 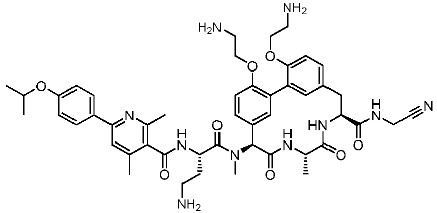 |
| **361** | rac-(8S,11S,14S)-3,18-bis(2-aminoethoxy)-N-(cyanomethyl)-11-methyl-14-[methyl-[rac-(2S)-4-amino-2-[[6-(1,1-dimethylindan-5-yl)-2,4-dimethyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 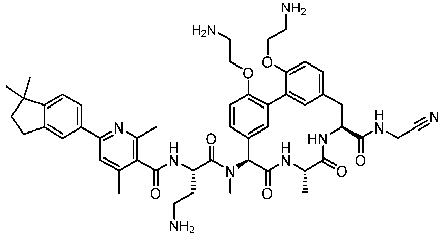 |
| **362** | rac-(8S,11S,14S)-3,18-bis(2-aminoethoxy)-N-(cyanomethyl)-11-methyl-14-[methyl-[rac-(2S)-4-amino-2-[[2,4-dimethyl-6-(4-pentoxyphenyl)pyridine-3- carbonyl]amino]butanoyl]amino]-10,13-dioxo-9,12-diazatricyclo[13.3.1.12,6]icosa-1(18),2(20),3,5,15(19),16-hexaene-8-carboxamide | 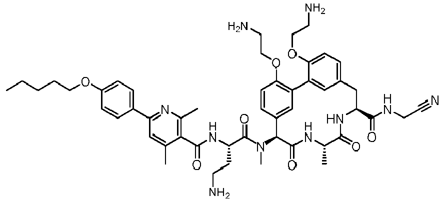 |
| **363** | rac-(8S,11S,14S)-3,18-bis(2-aminoethoxy)-N-(cyanomethyl)-11-methyl-14-[methyl-[rac-(2S)-4-amino-2-[[6-(4-tert-butyl-2-fluorophenyl)-2,4-dimethyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 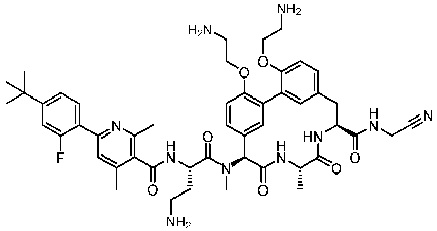 |
| **364** | rac-(8S,11S,14S)-3,18-bis(2-aminoethoxy)-N-(cyanomethyl)-11-methyl-14-[methyl-[rac-(2S)-4-amino-2-[[6-[4-(cyclohexoxy)phenyl]-2,4-dimethylpyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 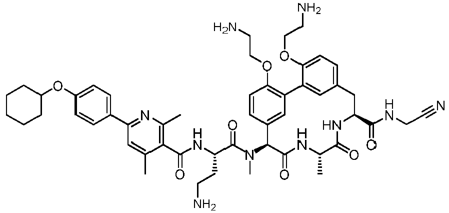 |
| **365** | rac-(8S,11S,14S)-3,18-bis(2-aminoethoxy)-N-(cyanomethyl)-11-methyl-14-[methyl-[rac-(2S)-4-amino-2-[[6-(5-butyl-2-pyridyl)-2,4-dimethyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 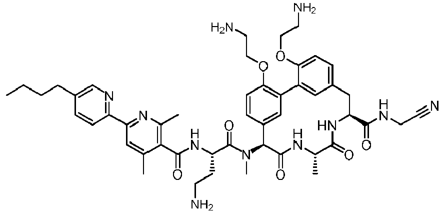 |
| **366** | rac-(8S,11S,14S)-3,18-bis(2-aminoethoxy)-N-(cyanomethyl)-11-methyl-14-[methyl-[rac-(2S)-4-amino-2-[[6-(4-butylphenyl)-2,4-dimethyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 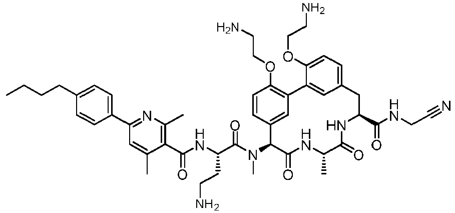 |
| **367** | rac-(8S,11S,14S)-3,18-bis(2-aminoethoxy)-N-(cyanomethyl)-11-methyl-14-[methyl-[rac-(2S)-4-amino-2-[[6-(4-butoxy-2,3,5,6-tetrafluoro-phenyl)-2,4-dimethylpyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 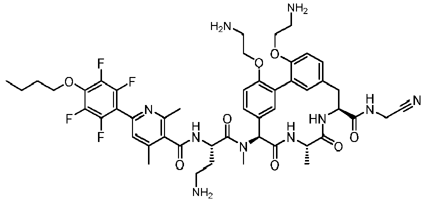 |
| **368** | rac-(8S,11S,14S)-3,18-bis(2-aminoethoxy)-N-(cyanomethyl)-11-methyl-14-[methyl-[rac-(2S)-4-amino-2-[[6-(4-tert-butyl-2-nitrophenyl)-2,4-dimethyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 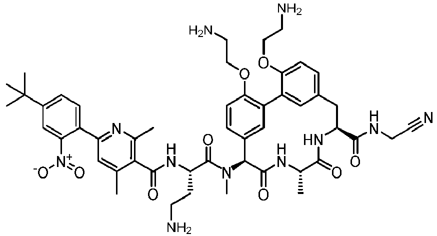 |
| **369** | rac-(8S,11S,14S)-3,18-bis(2-aminoethoxy)-N-(cyanomethyl)-11-methyl-14-[methyl-[rac-(2S)-4-amino-2-[[6-(2-amino-4-tert-butylphenyl)-2,4-dimethyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 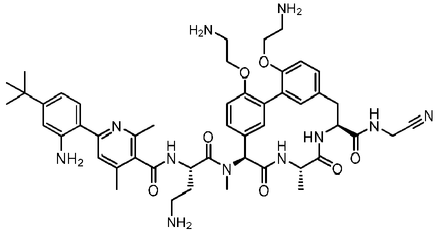 |
| **370** | rac-(8S,11S,14S)-3,18-bis(2-aminoethoxy)-N-(cyanomethyl)-11-methyl-14-[methyl-[rac-(2S)-4-amino-2-[[4-(4-tert-butylphenyl)-2,6-dimethylbenzoyl]amino]butanoyl]amino]]-10,13-dioxo-9,12-diazatricyclo[13.3.1.12,6]icosa-1(18),2(20),3,5,15(19),16-hexaene-8-carboxamide | 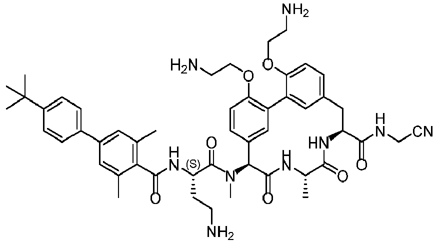 |
| **371** | rac-(8S,11S,14S)-3,18-bis(2-aminoethoxy)-N-(cyanomethyl)-11-methyl-14-[methyl-[rac-(2S)-4-amino-2-[[4-(4-tert-butylphenyl)-2-chlorobenzoyl]amino]butanoyl]amino]]-10,13-dioxo-9,12-diazatricyclo[13.3.1.12,6]icosa-1(18),2(20),3,5,15(19),16-hexaene-8-carboxamide | 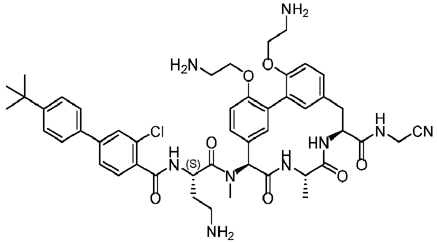 |
| **372** | rac-(8S,11S,14S)-3,18-bis(2-aminoethoxy)-N-(cyanomethyl)-11-methyl-14-[methyl-[rac-(2S)-4-amino-2-[[2-amino-4-(4-tert-butylphenyl)benzoyl]amino]butanoy 1]amino]-10,13-dioxo-9,12-diazatricyclo[13.3.1.12,6]icosa-1(18),2(20),3,5,15(19),16-hexaene-8-carboxamide | 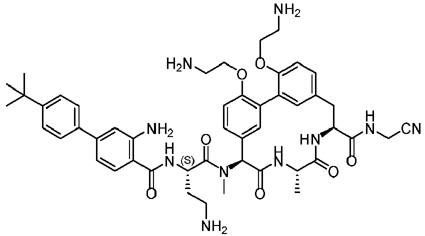 |
| **373** | rac-(8S,11S,14S)-3,18-bis(2-aminoethoxy)-N-(cyanomethyl)-11-methyl-14-[methyl-[rac-(2S)-4-amino-2-[[2-amino-4-(4-tert-butylphenyl)-6-methylbenzoyl]amino]butanoyl]amino]]-10,13-dioxo-9,12-diazatricyclo[13.3.1.12,6]icosa-1(18),2(20),3,5,15(19),16-hexaene-8-carboxamide | 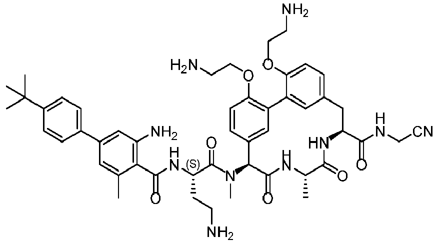 |
| **374** | rac-(8S,11S,14S)-3,18-bis(2-aminoethoxy)-N-(cyanomethyl)-11-methyl-14-[methyl-[rac-(2S)-4-amino-2-[[4-(4-tert-butylphenoxy)-2,3-dimethylbenzoyl]amino]butanoyl]amino]]-10,13-dioxo-9,12-diazatricyclo[13.3.1.12,6]icosa-1(18),2(20),3,5,15(19),16-hexaene-8-carboxamide | 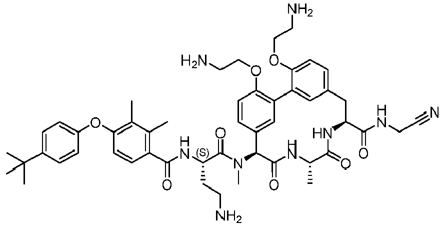 |
| **375** | rac-(8S,11S,14S)-3,18-bis(2-aminoethoxy)-N-(cyanomethyl)-11-methyl-14-[methyl-[rac-(2S)-4-amino-2-[[4-(4-tert-butylphenoxy)-2,6-dimethylbenzoyl]amino]butanoyl]amino]]-10,13-dioxo-9,12-diazatricyclo[13.3.1.12,6]icosa-1(18),2(20),3,5,15(19),16-hexaene-8-carboxamide | 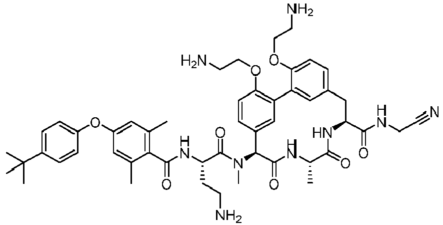 |
| **376** | rac-(8S,11S,14S)-3,18-bis(2-aminoethoxy)-N-(cyanomethyl)-11-methyl-14-[methyl-[rac-(2S)-4-amino-2-[[4-(4-tert-butylphenoxy)-2-chlorobenzoyl]amino]butanoyl]amino]]-10,13-dioxo-9,12-diazatricyclo[13.3.1.12,6]icosa-1(18),2(20),3,5,15(19),16-hexaene-8-carboxamide | 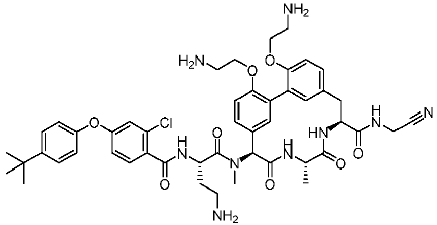 |
| **377** | rac-(8S,11S,14S)-3,18-bis(2-aminoethoxy)-N-(cyanomethyl)-11-methyl-14-[methyl-[rac-(2S)-4-amino-2-[[4-(4-butylphenoxy)-2-chlorobenzoyl]amino]butanoyl]amino]]-10,13-dioxo-9,12-diazatricyclo[13.3.1.12,6]icosa-1(18),2(20),3,5,15(19),16-hexaene-8-carboxamide | 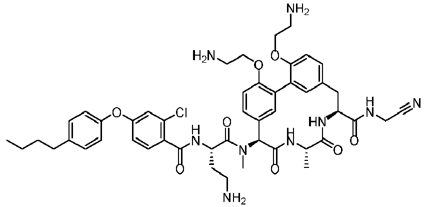 |
| **378** | rac-(8S,11S,14S)-3,18-bis(2-aminoethoxy)-N-(cyanomethyl)-11-methyl-14-[methyl-[rac-(2S)-4-amino-2-[[2-chloro-4-(3,4-dibutylphenoxy)benzoyl]amino]]buta noyl]amino]-10,13-dioxo-9,12-diazatricyclo[13.3.1.12,6]icosa-1(18),2(20),3,5,15(19),16-hexaene-8-carboxamide | 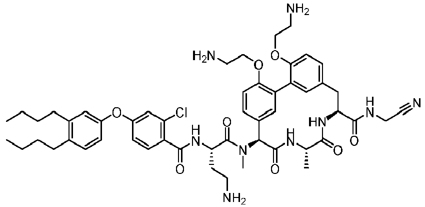 |
| **379** | 2-[5-heptyl-2-[[rac-(1S)-3-amino-1-[methyl-[rac-(7S,10S,13S)-3,18-bis(2-aminoethoxy)-13-(cyanomethylcarbamoyl)-10-methyl-8,11-dioxo-9,12-diazatricyclo[13.3.1.12,6]icosa-1(19),2,4,6(20),15,17-hexaen-7-yl]carbamoyl]propyl]carbamoyl]phe noxy]aceticacid | 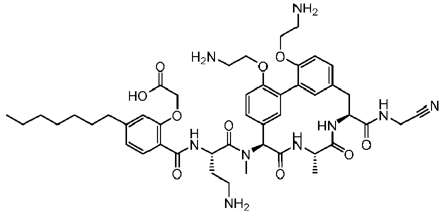 |
| **380** | rac-(8S,11S,14S)-3,18-bis(2-aminoethoxy)-N-(cyanomethyl)-11-methyl-14-[methyl-[rac-(2S)-4-amino-2-[[4-[5-(4-hexoxyphenyl)isoxazol-3-yl]benzoyl]amino]butanoyl]amino]- 10,13-dioxo-9,12-diazatricyclo[13.3.1.12,6]icosa-1(18),2(20),3,5,15(19),16-hexaene-8-carboxamide | 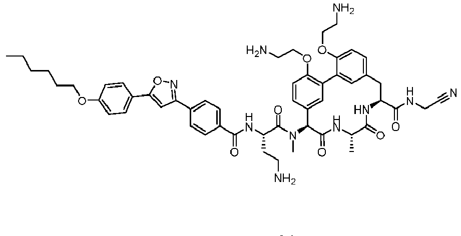 |
| **381** | 4-methyl-2-(4-pentylphenyl)-N-[rac-(1S)-3-amino-1-[methyl-[rac-(7S,10S,13S)-3,18-bis(2-aminoethoxy)-13-(cyanomethylcarbamoyl)-10-methyl-8,11-dioxo-9,12-diazatricyclo[13.3.1.12,6]icosa-1(19),2,4,6(20),15,17-hexaen-7-yl]carbamoyl]propyl]oxazole-5-carboxamide | 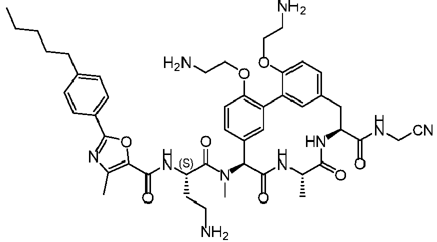 |
| **382** | 4-methyl-2-(4-pentylphenyl)-N-[rac-(1R)-3-amino-1-[methyl-[rac-(7S,10S,13S)-3,18-bis(2-aminoethoxy)-13-(cyanomethylcarbamoyl)-10-methyl-8,11-dioxo-9,12-diazatricyclo[13.3.1.12,6]icosa-1(19),2,4,6(20),15,17-hexaen-7-yl]carbamoyl]propyl]oxazole-5-carboxamide | 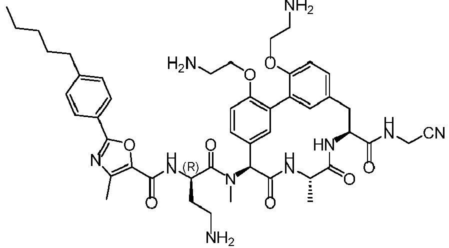 |
| **383** | rac-(8S,11S,14S)-3,18-bis(2-aminoethoxy)-N-(cyanomethyl)-11-methyl-14-[methyl-[rac-(2S)-4-amino-2-[[1-(4-tert-butylphenyl)-2-oxo-pyridine-4-carbonyl]amino]butanoyl]amino]-10,13-dioxo-9,12-diazatricyclo[13.3.1.12,6]icosa-1(18),2(20),3,5,15(19),16-hexaene-8-carboxamide | 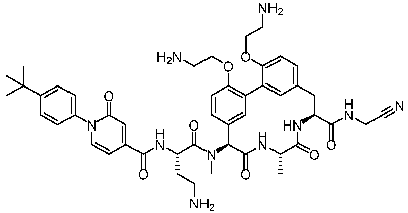 |
| **384** | rac-(8S,11S,14S)-3,18-bis(2-aminoethoxy)-N-(cyanomethyl)-11-methyl-14-[methyl-[rac-(2S)-4-amino-2-[[l-(4-tert-butylphenyl)-6-oxo-pyridazine-4-carbonyl]amino]butanoyl]amino]-10,13-dioxo-9,12-diazatricyclo[13.3.1.12,6]icosa-1(18),2(20),3,5,15(19),16-hexaene-8-carboxamide | 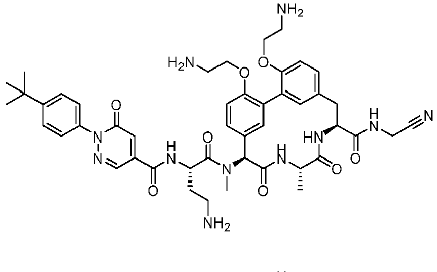 |
| **385** | rac-(8S,11S,14S)-3,18-bis(2-aminoethoxy)-N-(cyanomethyl)-11-methyl-14-[methyl-[rac-(2S)-4-amino-2-[[3-(4-tert-butylphenyl)-5-methyl-1,2,4-triazine-6-carbonyl]amino]butanoyl]amino]-10,13-dioxo-9,12-diazatricyclo[13.3.1.12,6]icosa-1(18),2(20),3,5,15(19),16-hexaene-8-carboxamide | 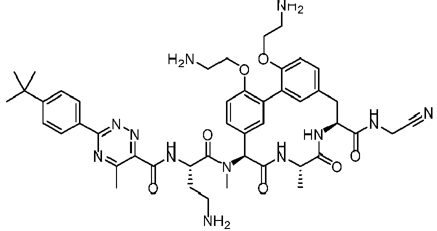 |
| **386** | rac-(8S,11S,14S)-3,18-bis(2-aminoethoxy)-N-(cyanomethyl)-11-methyl-14-[methyl-[rac-(2S)-4-amino-2-[[5-(4-tert-butylphenyl)-1-methyl-6-oxo-pyrazine-2-carbonyl]amino]butanoyl]amino]]-10,13-dioxo-9,12-diazatricyclo[13.3.1.12,6]icosa-1(18),2(20),3,5,15(19),16-hexaene-8-carboxamide | 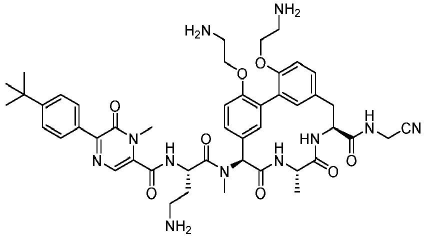 |
| **387** | rac-(8S,11S,14S)-3,18-bis(2-aminoethoxy)-N-(cyanomethyl)-11-methyl-14-[methyl-[rac-(2S)-4-amino-2-[[1-(4-tert-butylphenyl)-5-methyl-6-oxo-pyridazine-4-carbonyl]amino]butanoyl]amino]-10,13-dioxo-9,12-diazatricyclo[13.3.1.12,6]icosa-1(18),2(20),3,5,15(19),16-hexaene-8-carboxamide | 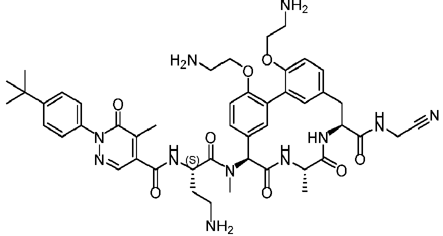 |
| **388** | rac-(8S,11S,14S)-3,18-bis(2-aminoethoxy)-N-(cyanomethyl)-11-methyl-14-[methyl-[rac-(2S)-4-amino-2-[[2-(difluoromethyl)-4-(2-hexylpyrimidin-5-yl)benzoyl]amino]butanoyl]amino]- 10,13-dioxo-9,12-diazatricyclo[13.3.1.12,6]icosa-1(18),2(20),3,5,15(19),16-hexaene-8-carboxamide | 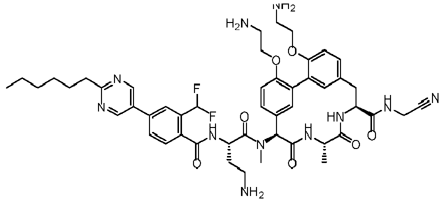 |
| **389** | rac-(8S,11S,14S)-3,18-bis(2-aminoethoxy)-N-(cyanomethyl)-11-methyl-14-[methyl-[rac-(2S)-4-amino-2-[[1-(4-tert-butylphenyl)piperidine-4-carbonyl]amino]butanoyl]amino]-10,13-dioxo-9,12-diazatricyclo[13.3.1.12,6]icosa-1(18),2(20),3,5,15(19),16-hexaene-8-carboxamide | 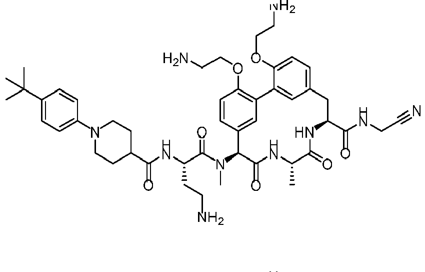 |
| **390** | rac-(8S,11S,14S)-3,18-bis(2-aminoethoxy)-N-(cyanomethyl)-11-methyl-14-[methyl-[rac-(2S)-4-amino-2-[[4-(4-tert-butylphenyl)piperidine-1-carbonyl]amino]butanoyl]amino]-10,13-dioxo-9,12-diazatricyclo[13.3.1.12,6]icosa-1(18),2(20),3,5,15(19),16-hexaene-8-carboxamide | 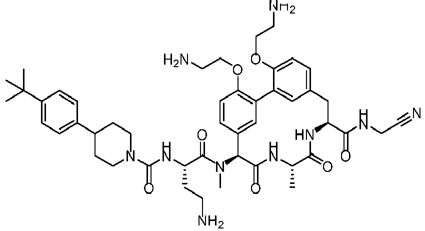 |
| **391** | rac-(8S,11S,14S)-3,18-bis(2-aminoethoxy)-N-(cyanomethyl)-11-methyl-14-[methyl-[rac-(2S)-4-amino-2-[[4-(4-tert-butylphenyl)cyclohexanecarbonyl]a mino]butanoyl]amino]-10,13-dioxo-9,12-diazatricyclo[13.3.1.12,6]icosa-1(18),2(20),3,5,15(19),16-hexaene-8-carboxamide | 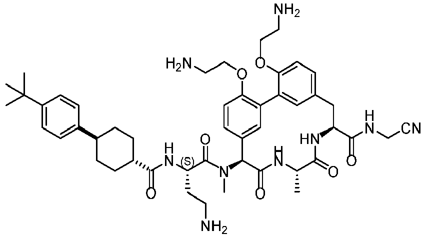 |
| **392** | rac-(8S,11S,14S)-3,18-bis(2-aminoethoxy)-N-(cyanomethyl)-11-methyl-14-[methyl-[rac-(2S)-4-amino-2-[[4-(4-tert-butylphenyl)cyclohexanecarbonyl]a mino]butanoyl]amino]-10,13-dioxo-9,12-diazatricyclo[13.3.1.12,6]icosa-1(18),2(20),3,5,15(19),16-hexaene-8-carboxamide | 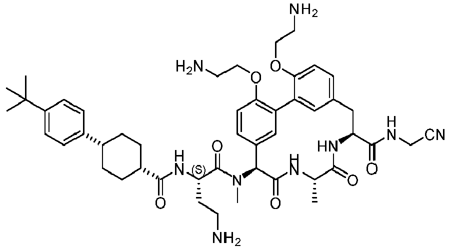 |
| **393** | rac-(8S,11S,14S)-3,18-bis(2-aminoethoxy)-N-(cyanomethyl)-11-methyl-14-[methyl-[rac-(2S)-4-amino-2-[[7-(4-tert-butylphenyl)-2-methyl-imidazo[1,2-a]pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 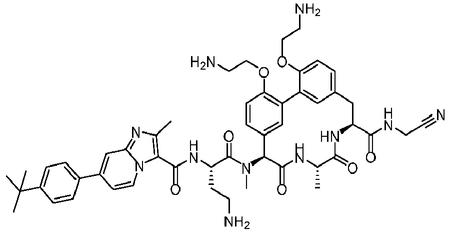 |
| **394** | rac-(8S,11S,14S)-3,18-bis(2-aminoethoxy)-N-(cyanomethyl)-11-methyl-14-[methyl-[rac-(2S)-4-amino-2-[(7-hexyl-2-methylquinoline-3-carbonyl)amino]butanoyl]amino]]-10,13-dioxo-9,12-diazatricyclo[13.3.1.12,6]icosa-1(18),2(20),3,5,15(19),16-hexaene-8-carboxamide | 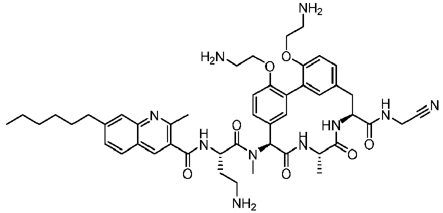 |
| **395** | rac-(8S,11S,14S)-3,18-bis(2-aminoethoxy)-N-(cyanomethyl)-11-methyl-14-[methyl-[rac-(2S)-4-amino-2-[(6-hexyl-2-methylquinoline-3-carbonyl)amino]butanoyl]amino]]-10,13-dioxo-9,12-diazatricyclo[13.3.1.12,6]icosa-1(18),2(20),3,5,15(19),16-hexaene-8-carboxamide | 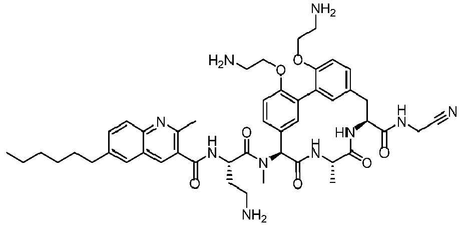 |
| **396** | rac-(8S,11S,14S)-3,18-bis(2-aminoethoxy)-N-(cyanomethyl)-11-methyl-14-[methyl-[rac-(2S)-4-amino-2-[(6-hexoxy-2-methylquinoline-3-carbonyl)amino]butanoyl]amino]-10,13-dioxo-9,12-diazatricyclo[13.3.1.12,6]icosa-1(18),2(20),3,5,15(19),16-hexaene-8-carboxamide | 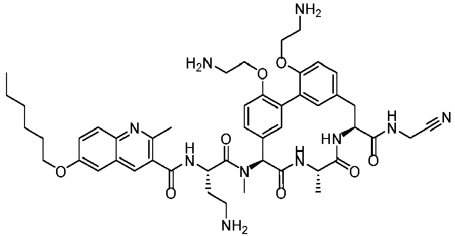 |
| **397** | rac-(8S,11S,14S)-3,18-bis(2-aminoethoxy)-N-(cyanomethyl)-11-methyl-14-[methyl-[rac-(2S)-4-amino-2-[[6-(4-tert-butylphenoxy)-2-methyl-quinoline-3-carbonyl]amino]butanoyl]amino]-10,13-dioxo-9,12-diazatricyclo[13.3.1.12,6]icosa-1(18),2(20),3,5,15(19),16-hexaene-8-carboxamide | 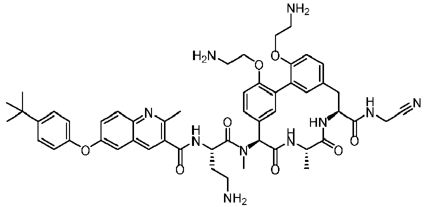 |
| **398** | rac-(8S,11S,14S)-3,18-bis(2-aminoethoxy)-N-(cyanomethyl)-11-methyl-14-[methyl-[rac-(2S)-4-amino-2-[(7-hexyl-2-methyl-1,5- naphthyridine-3- carbonyl)amino]butanoyl]amino]]-10,13-dioxo-9,12-diazatricyclo[13.3.1.12,6]icosa-1(18),2(20),3,5,15(19),16-hexaene-8-carboxamide | 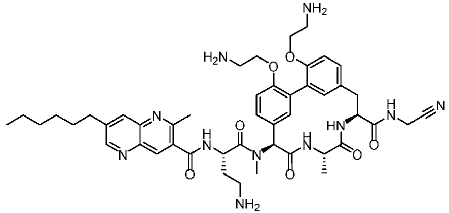 |
| **399** | rac-(8S,11S,14S)-3,18-bis(2-aminoethoxy)-N-(cyanomethyl)-11-methyl-14-[methyl-[rac-(2S)-4-amino-2-[(6-hexyl-2,4-dimethyl-quinoline-3-carbonyl)amino]butanoyl]amino]]-10,13-dioxo-9,12-diazatricyclo[13.3.1.12,6]icosa-1(18),2(20),3,5,15(19),16-hexaene-8-carboxamide | 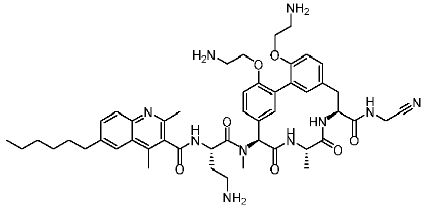 |
| **400** | rac-(8S,11S,14S)-3,18-bis(2-aminoethoxy)-N-(cyanomethyl)-11-methyl-14-[methyl-[rac-(2S)-4-amino-2-[(7-hexyl-3-methylnaphthalene-2-carbonyl)amino]butanoyl]amino]-10,13-dioxo-9,12-diazatricyclo[13.3.1.12,6]icosa-1(18),2(20),3,5,15(19),16-hexaene-8-carboxamide | 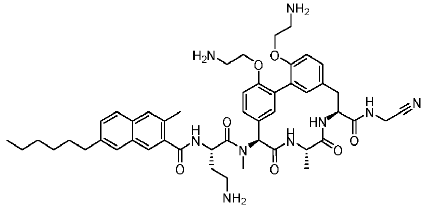 |
| **401** | rac-(8S,11S,14S)-3,18-bis(2-aminoethoxy)-N-(cyanomethyl)-11-methyl-14-[methyl-[rac-(2S)-4-amino-2-[[5-bromo-6-(4-tert-butylphenyl)-2-methyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 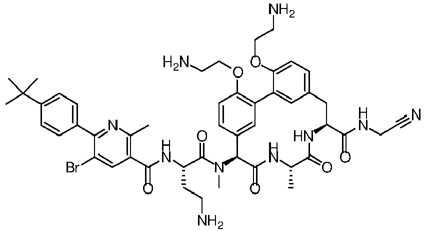 |
| **402** | rac-(8S,11S,14S)-3,18-bis(2-aminoethoxy)-N-(cyanomethyl)-11-methyl-14-[methyl-[rac-(2S)-4-amino-2-[[6-(4-tert-butylphenyl)-2,5-dimethyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 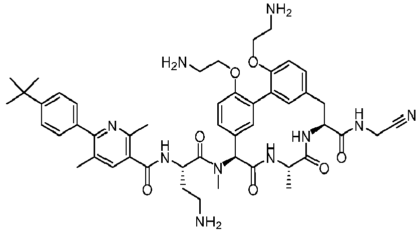 |
| **403** | rac-(8S,11S,14S)-3,18-bis(2-aminoethoxy)-N-(cyanomethyl)-11-methyl-14-[methyl-[rac-(2S)-4-amino-2-[[6-(4-tert-butylphenyl)-5-methoxy-2-methyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 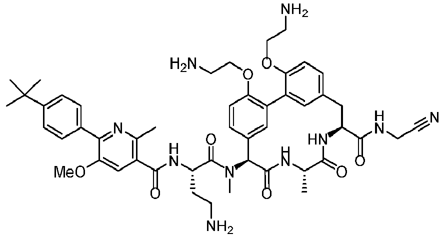 |
| **404** | rac-(8S,11S,14S)-3,18-bis(2-aminoethoxy)-N-(cyanomethyl)-11-methyl-14-[methyl-[rac-(2S)-4-amino-2-[[6-(4-tert-butylphenyl)-5-hydroxy-2-methyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 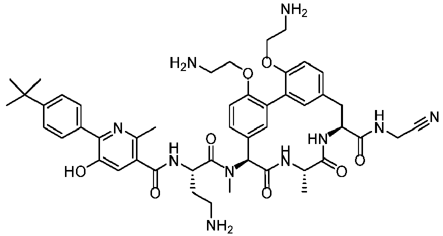 |
| **405** | rac-(8S,11S,14S)-3,18-bis(2-aminoethoxy)-N-(cyanomethyl)-11-methyl-14-[methyl-[rac-(2S)-4-amino-2-[[5-amino-6-(4-tert-butylphenyl)-2-methyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 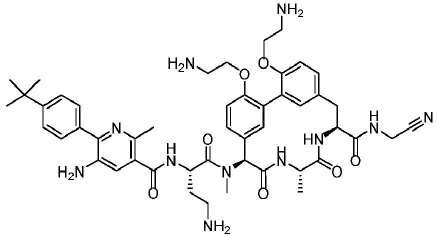 |
| **406** | rac-(8S,11S,14S)-3,18-bis(2-aminoethoxy)-N-(cyanomethyl)-11-methyl-14-[methyl-[rac-(2S)-4-amino-2-[[6-(4-tert-butylphenyl)-2-methyl-5-nitro-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 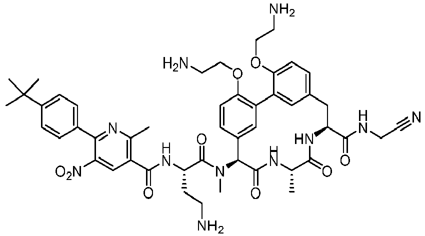 |
| **407** | rac-(8S,11S,14S)-3,18-bis(2-aminoethoxy)-N-(cyanomethyl)-11-methyl-14-[methyl-[rac-(2S)-4-amino-2-[[6-(4-tert-butylphenyl)-5-chloro-2-methyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 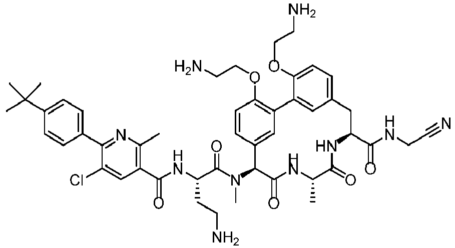 |
| **408** | rac-(8S,11S,14S)-3,18-bis(2-aminoethoxy)-N-(cyanomethyl)-11-methyl-14-[methyl-[rac-(2S)-4-amino-2-[[6-(4-tert-butylphenyl)-5-(dimethylamino)-2-methyl-pyridine-3-carbonyl]amino]butanoyl]amino]- 10,13-dioxo-9,12-diazatricyclo[13.3.1.12,6]icosa-1(18),2(20),3,5,15(19),16-hexaene-8-carboxamide | 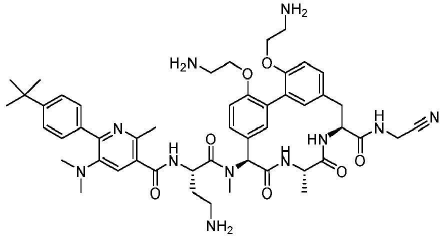 |
| **409** | rac-(8S,11S,14S)-3,18-bis(2-aminoethoxy)-N-(cyanomethyl)-11-methyl-14-[methyl-[rac-(2S)-4-amino-2-[[6-(4-tert-butylphenyl)-2-methyl-5-(methylamino)pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 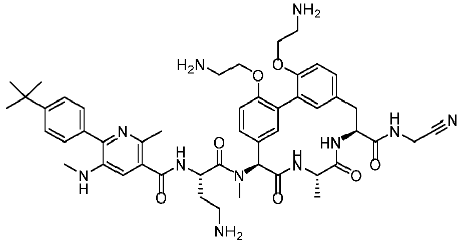 |
| **410** | rac-(8S,11S,14S)-3,18-bis(2-aminoethoxy)-N-(cyanomethyl)-11-methyl-14-[methyl-[rac-(2S)-4-amino-2-[[6-(4-tert-butylphenyl)-5-cyano-2-methyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 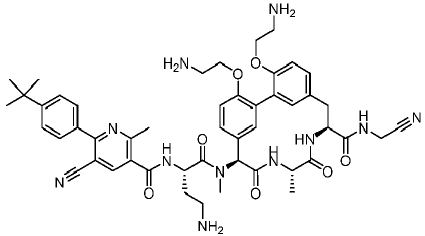 |
| **411** | rac-(8S,11S,14S)-3,18-bis(2-aminoethoxy)-N-(cyanomethyl)-11-methyl-14-[methyl-[rac-(2S)-4-amino-2-[[4-amino-6-(4-tert-butylphenyl)pyridine-3-carbonyl]amino]butanoyl]amino]]-10,13-dioxo-9,12-diazatricyclo[13.3.1.12,6]icosa-1(18),2(20),3,5,15(19),16-hexaene-8-carboxamide | 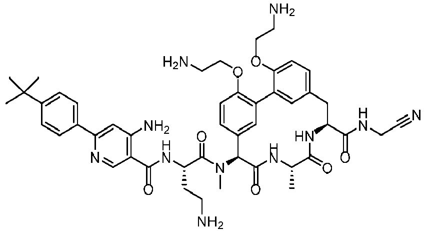 |
| **412** | rac-(8S,11S,14S)-3,18-bis(2-aminoethoxy)-N-(cyanomethyl)-11-methyl-14-[methyl-[rac-(2S)-4-amino-2-[[2-amino-6-(4-tert-butylphenyl)pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 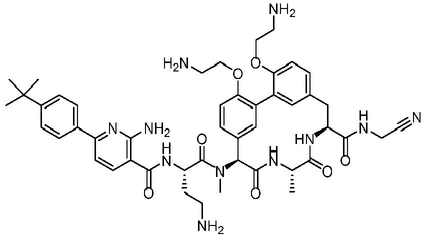 |
| **413** | rac-(8S,11S,14S)-3,18-bis(2-aminoethoxy)-N-(cyanomethyl)-11-methyl-14-[methyl-[rac-(2S)-4-amino-2-[[4-amino-6-(4-tert-butylphenyl)-2-methyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 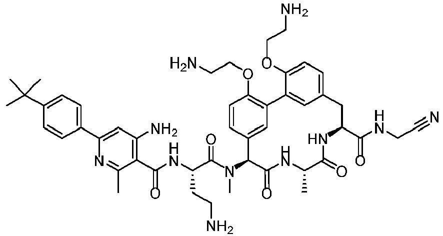 |
| **414** | rac-(8S,11S,14S)-3,18-bis(2-aminoethoxy)-N-(cyanomethyl)-11-methyl-14-[methyl-[rac-(2S)-4-amino-2-[[2-amino-6-(4-tert-butylphenyl)-4-methyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 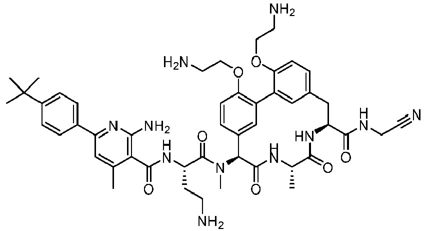 |
| **415** | rac-(8S,11S,14S)-3,18-bis(2-aminoethoxy)-N-(cyanomethyl)-11-methyl-14-[methyl-[rac-(2S)-4-amino-2-[[2-amino-6-(4-tert-butylphenyl)-5-nitro-pyridine-3-carbonyl]amino]butanoyl]amino]]-10,13-dioxo-9,12-diazatricyclo[13.3.1.12,6]icosa-1(18),2(20),3,5,15(19),16-hexaene-8-carboxamide | 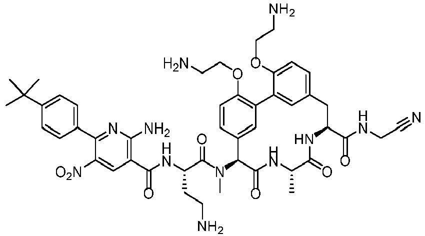 |
| **416** | rac-(8S,11S,14S)-3,18-bis(2-aminoethoxy)-N-(cyanomethyl)-11-methyl-14-[methyl-[rac-(2S)-4-amino-2-[[2,5-diamino-6-(4-tert-butylphenyl)pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 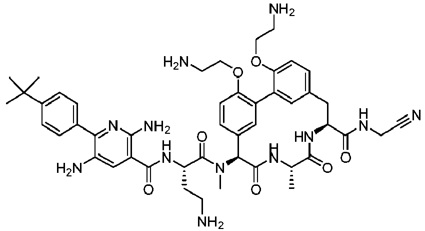 |
| **417** | rac-(8S,11S,14S)-3,18-bis(2-aminoethoxy)-N-(cyanomethyl)-11-methyl-14-[methyl-[rac-(2S)-4-amino-2-[[4-ethyl-2-(4-isobutylphenyl)pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 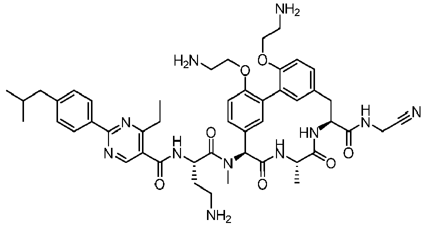 |
| **418** | rac-(8S,11S,14S)-3,18-bis(2-aminoethoxy)-N-(cyanomethyl)-11-methyl-14-[methyl-[rac-(2S)-4-amino-2-[[4-(difluoromethyl)-2-(4-isobutylphenyl)pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 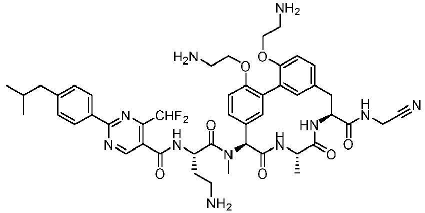 |
| **419** | rac-(8S,11S,14S)-3,18-bis(2-aminoethoxy)-N-(cyanomethyl)-11-methyl-14-[methyl-[rac-(2S)-4-amino-2-[[2-(4-tert-butylphenyl)-4-hydroxy-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 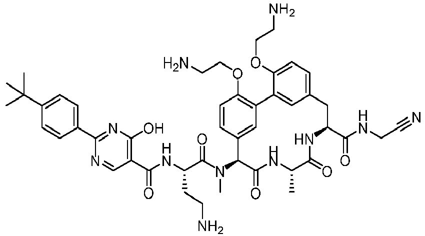 |
| **420** | 2-[2-(4-tert-butylphenyl)-5-[[rac-(1S)-3-amino-1-[methyl-[rac-(7S,10S,13S)-3,18-bis(2-aminoethoxy)-13-(cyanomethylcarbamoyl)-10-methyl-8,11-dioxo-9,12-diazatricyclo[13.3.1.12,6]icosa-1(19),2,4,6(20),15,17-hexaen-7-yl]carbamoyl]propyl]carbamoyl]pyri midin-4-yl]oxyaceticacid | 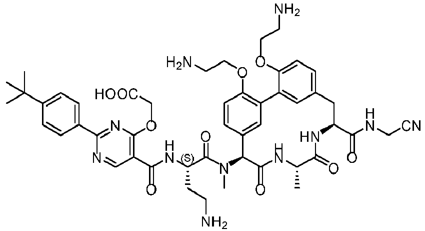 |
| **421** | rac-(8S,11S,14S)-3,18-bis(2-aminoethoxy)-N-(cyanomethyl)-11-methyl-14-[methyl-[rac-(2S)-4-amino-2-[[2-(4-tert-butylphenyl)-4-chloro-6-methyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 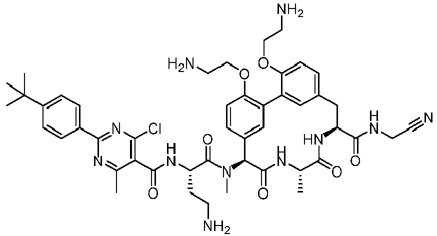 |
| **422** | rac-(8S,11S,14S)-3,18-bis(2-aminoethoxy)-N-(cyanomethyl)-11-methyl-14-[methyl-[rac-(2S)-4-amino-2-[[2-(4-tert-butylphenyl)-4-methoxy-6-methyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 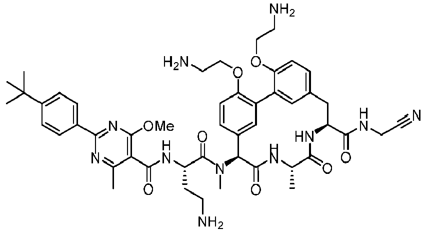 |
| **423** | rac-(8S,11S,14S)-3,18-bis(2-aminoethoxy)-N-(cyanomethyl)-11-methyl-14-[methyl-[rac-(2S)-4-amino-2-[[2-(4-tert-butylphenyl)-4-hydroxy-6-methyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 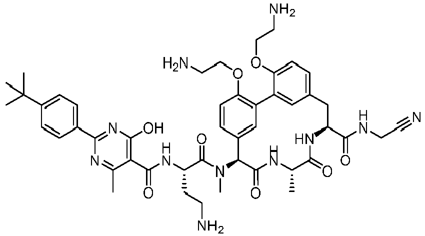 |
| **424** | rac-(8S,11S,14S)-3,18-bis(2-aminoethoxy)-N-(cyanomethyl)-11-methyl-14-[methyl-[rac-(2S)-4-amino-2-[[2-(4-tert-butylphenyl)-4-(dimethylamino)-6-methylpyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 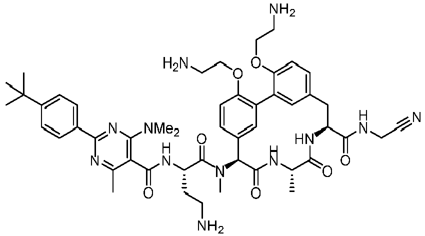 |
| **425** | rac-(8S,11S,14S)-3,18-bis(2-aminoethoxy)-N-(cyanomethyl)-11-methyl-14-[methyl-[rac-(2S)-4-amino-2-[[2-(4-tert-butylphenyl)-4-methyl-6-(methylamino)pyrimidine-5-carbonyl]amino]butanoyl]amino]- 10,13-dioxo-9,12-diazatricyclo[13.3.1.12,6]icosa-1(18),2(20),3,5,15(19),16-hexaene-8-carboxamide | 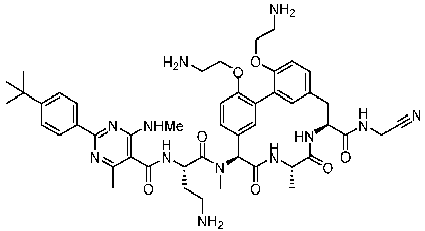 |
| **426** | rac-(8S,11S,14S)-3,18-bis(2-aminoethoxy)-N-(cyanomethyl)-11-methyl-14-[methyl-[rac-(2S)-4-amino-2-[[4-amino-2-(4-tert-butylphenyl)-6-methyl-pyrimidine-5-carbonyl]amino]butanoyl]amino]- 10,13-dioxo-9,12-diazatricyclo[13.3.1.12,6]icosa-1(18),2(20),3,5,15(19),16-hexaene-8-carboxamide | 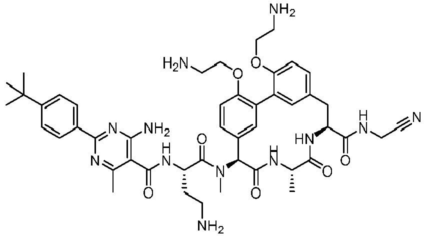 |
| **427** | rac-(8S,11S,14S)-3,18-bis(2-aminoethoxy)-N-(cyanomethyl)-11-methyl-14-[methyl-[rac-(2S)-4-amino-2-[[4-amino-2-[4-(3,3-dimethylbutoxy)phenyl] -6-methylpyrimidine-5-carbonyl]aminobutanoyl]amino-10,13-dioxo-9,12-diazatricyclo[13.3.1.12,6]icosa-1(18),2(20),3,5,15(19),16-hexaene-8-carboxamide | 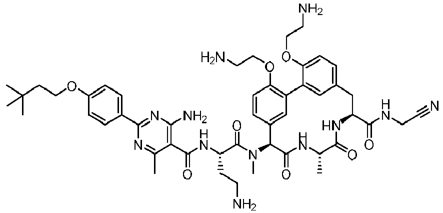 |
| **428** | rac-(8S,11S,14S)-3,18-bis(2-aminoethoxy)-N-(cyanomethyl)-11-methyl-14-[methyl-[rac-(2S)-4-amino-2-[[2-(4-tert-butylphenyl)-4-methyl-6-(trifluoromethyl)pyrimidine-5-carbonyl]aminobutanoyl]amino-10,13-dioxo-9,12-diazatricyclo[13.3.1.12,6]icosa-l(18),2(20),3,5,15(19),16-hexaene-8-carboxamide | 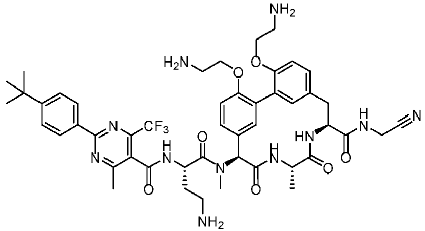 |
| **429** | rac-(8S,11S,14S)-3,18-bis(2-aminoethoxy)-N-(cyanomethyl)-11-methyl-14-[methyl-[rac-(2S)-4-amino-2-[[4,6-diamino-2-(4-tert-butylphenyl)pyrimidine-5-carbonyl]aminobutanoyl]amino-10,13-dioxo-9,12-diazatricyclo[13.3.1.12,6]icosa-1(18),2(20),3,5,15(19),16-hexaene-8-carboxamide | 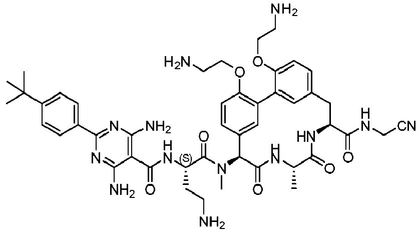 |
| **430** | rac-(8S,11S,14S)-N-(cyanomethyl)-3,18-dihydroxy-11-methyl-14-[methyl-[rac-(2S)-4-amino-2-[[6-(4-isobutylphenyl)-2-methyl-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 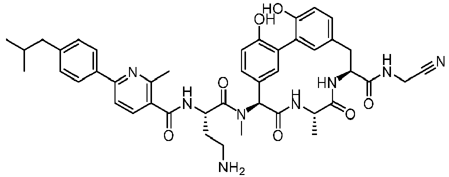 |
| **431** | rac-(8S,11S,14S)-N-(cyanomethyl)-3,18-dihydroxy-11-methyl-14-[methyl-[rac-(2S)-4-amino-2-[[2-(4-tert-butylphenyl)-4-methylpyrimidine-5-carbonyl]aminobutanoyl]amino-10,13-dioxo-9,12-diazatricyclo[13.3.1.12,6]icosa-1(18),2(20),3,5,15(19),16-hexaene-8-carboxamide | 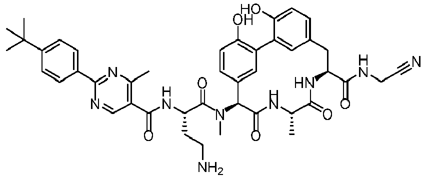 |
| **432** | rac-(8S,11S,14S)-N-(cyanomethyl)-3,18-dihydroxy-11-methyl-14-[methyl-[rac-(2S)-4-amino-2-[[4-methyl-2-(4-pentylphenyl)pyrimidine-5- carbonyl]aminobutanoyl]amino-10,13-dioxo-9,12-diazatricyclo[13.3.1.12,6]icosa-1(18),2(20),3,5,15(19),16-hexaene-8-carboxamide | 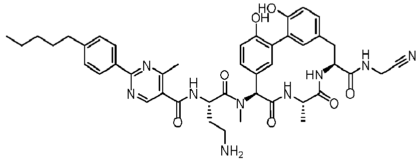 |
| **433** | rac-(8S,11S,14S)-3,18-bis(3-aminopropoxy)-N-(cyanomethyl)-11-methyl-14-[methyl-[rac-(2S)-4-amino-2-[[2-(4-tert-butylphenyl)-4-methyl-pyrimidine-5-carbonyl]aminobutanoyl]amino-10,13-dioxo-9,12-diazatricyclo[13.3.1.12,6]icosa-l(18),2(20),3,5,15(19),16-hexaene-8-carboxamide | 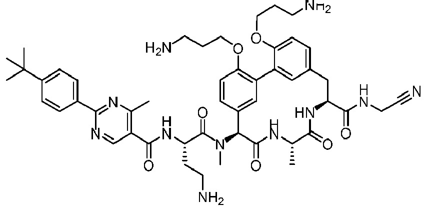 |
| **434** | rac-(8S,11S,14S)-3,18-bis(3-aminopropoxy)-N-(cyanomethyl)-11-methyl-14-[methyl-[rac-(2S)-4-amino-2-[[2-(4-tert-butylphenyl)-4,6-dimethyl-pyrimidine-5-carbonyl]aminobutanoyl]amino-10,13-dioxo-9,12-diazatricyclo[13.3.1.12,6]icosa-l(18),2(20),3,5,15(19),16-hexaene-8-carboxamide | 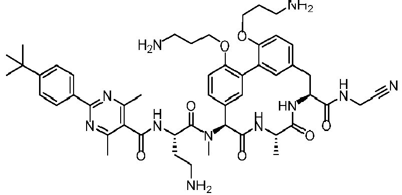 |
| **435** | rac-(8S,11S,14S)-3,18-bis(3-aminopropoxy)-N-(cyanomethyl)-11-methyl-14-[methyl-[rac-(2S)-4-amino-2-[[4,6-dimethyl-2-(4-pentoxyphenyl)pyrimidine-5-carbonyl]aminobutanoyl]amino-10,13-dioxo-9,12-diazatricyclo[13.3.1.12,6]icosa-1(18),2(20),3,5,15(19),16-hexaene-8-carboxamide | 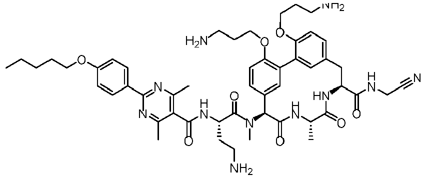 |
| **436** | rac-(8S,11S,14S)-3,18-bis(3-aminopropoxy)-N-(cyanomethyl)-11-methyl-14-[methyl-[rac-(2S)-4-amino-2-[[4-amino-2-[4-(3,3-dimethylbutoxy)phenyl]-6-methylpyrimidine-5-carbonyl]aminobutanoyl]amino-10,13-dioxo-9,12-diazatricyclo[13.3.1.12,6]icosa-1(18),2(20),3,5,15(19),16-hexaene-8-carboxamide | 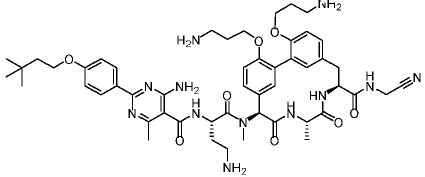 |
| **437** | rac-(8S,11S,14S)-3,18-bis(4-aminobutoxy)-N-(cyanomethyl)-11-methyl-14-[methyl-[rac-(2S)-4-amino-2-[[2-(4-tert-butylphenyl)-4,6-dimethyl-pyrimidine-5-carbonyl]aminobutanoyl]amino-10,13-dioxo-9,12-diazatricyclo[13.3.1.12,6]icosa-1(18),2(20),3,5,15(19),16-hexaene-8-carboxamide | 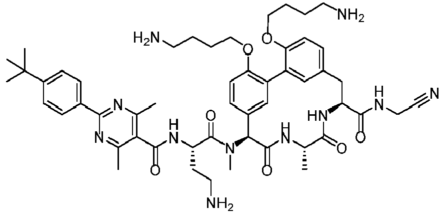 |
| **438** | rac-(8S,11S,14S)-N-(cyanomethyl)-11-methyl-14-[methyl-[rac-(2S)-4-amino-2-[[2-(4-tert-butylphenyl)-4-methyl-pyrimidine-5-carbonyl]aminobutanoyl]amino-10,13-dioxo-3,18-bis [rac-(2S)-2-aminopropoxy]-9,12-diazatricyclo[13.3.1.12,6]icosa-l(18),2(20),3,5,15(19),16-hexaene-8-carboxamide | 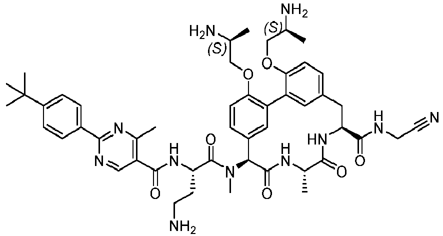 |
| **439** | rac-(8S,11S,14S)-3,18-bis(3-amino-2-hydroxy-propoxy)-N-(cyanomethyl)-11-methyl-14-[methyl-[rac-(2S)-4-amino-2-[[2-(4-tert-butylphenyl)-4,6-dimethyl-pyrimidine-5-carbonyl]aminobutanoyl]amino-10,13-dioxo-9,12-diazatricyclo[13.3.1.12,6]icosa-1(18),2(20),3,5,15(19),16-hexaene-8-carboxamide | 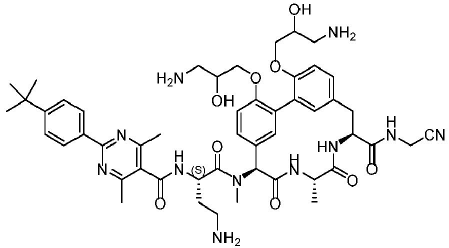 |
| **440** | | 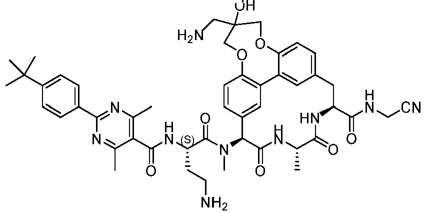 |
| **441** | rac-(8S,11S,14S)-N-(cyanomethyl)-11-methyl-3,18-bis[2-[(N-methylcarbamimidoyl)amino]ethoxy ]-14-[methyl-[rac-(2S)-4-amino-2-[[2-(4-tert-butylphenyl)-4,6-dimethyl-pyrimidine-5-carbonyl]aminobutanoyl]amino-10,13-dioxo-9,12-diazatricyclo[13.3.1.12,6]icosa-1(18),2(20),3,5,15(19),16-hexaene-8-carboxamide | 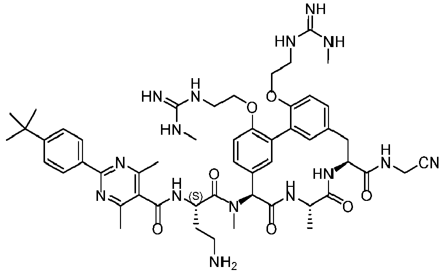 |
| **442** | rac-(8S,11S,14S)-N-(cyanomethyl)-3,18-bis[2-(dimethylamino)ethoxy]- 11-methyl-14-[methyl-[rac-(2S)-4-amino-2-[[2-(4-tert-butylphenyl)-4-methyl-pyrimidine-5-carbonyl]aminobutanoyl]amino-10,13-dioxo-9,12-diazatricyclo[13.3.1.12,6]icosa-1(18),2(20),3,5,15(19),16-hexaene-8-carboxamide | 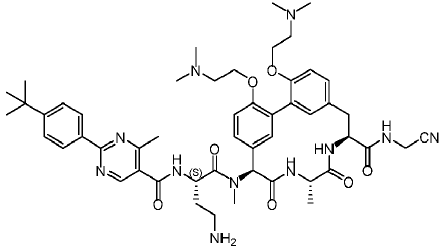 |
| **443** | rac-(2S)-N,N'-dimethyl-2-[(2-methyl-4-octyl-benzoyl)amino-N-[rac-(7S,10S,13S)-3,18-bis(2-aminoethoxy)-13-(cyanomethylcarbamoyl)-10-methyl-8,11-dioxo-9,12-diazatricyclo[13.3.1.12,6]icosa-1(19),2,4,6(20),15,17-hexaen-7-yl]butanediamide | 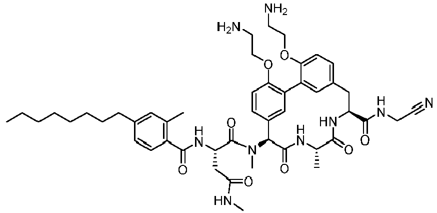 |
| **444** | rac-(2S)-N,N',N'-trimethyl-2-[(2-methyl-4-octyl-benzoyl)amino-N-[rac-(7S,10S,13S)-3,18-bis(2-aminoethoxy)-13-(cyanomethylcarbamoyl)-10-methyl-8,11-dioxo-9,12-diazatricyclo[13.3.1.12,6]icosa-1(19),2,4,6(20),15,17-hexaen-7-yl]pentanediamide | 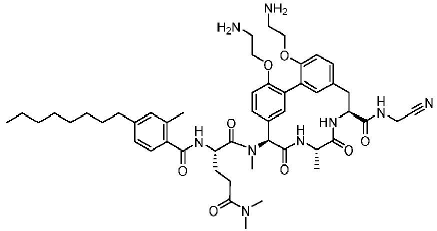 |
| **445** | rac-(2S)-N'-ethyl-N-methyl-2-[(2-methyl-4-octyl-benzoyl)amino-N-[rac-(7S,10S,13S)-3,18-bis(2-aminoethoxy)-13-(cyanomethylcarbamoyl)-10-methyl-8,11-dioxo-9,12-diazatricyclo[13.3.1.12,6]icosa-1(19),2,4,6(20),15,17-hexaen-7-yl]pentanediamide | 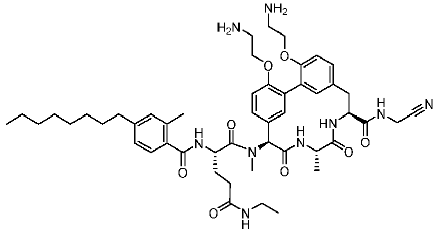 |
| **446** | rac-(2S)-N'-(2-hydroxyethyl)-N-methyl-2-[(2-methyl-4-octylbenzoyl)amino] -N- [rac-(7S,10S,13S)-3,18-bis(2-aminoethoxy)-13-(cyanomethylcarbamoyl)-10-methyl-8,11-dioxo-9,12-diazatricyclo[13.3.1.12,6]icosa-1(19),2,4,6(20),15,17-hexaen-7-yl]pentanediamide | 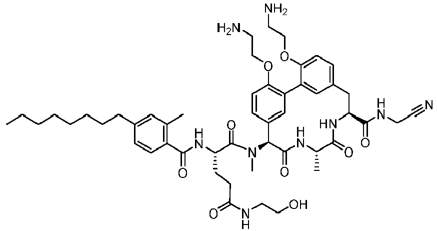 |
| **447** | rac-(2S)-N-methyl-2-[(2-methyl-4-octyl-benzoyl)amino]-N'-(oxetan-3-yl)-N-[rac-(7S,10S,13S)-3,18-bis(2-aminoethoxy)-13-(cyanomethylcarbamoyl)-10-methyl-8,11-dioxo-9,12-diazatricyclo[13.3.1.12,6]icosa-1(19),2,4,6(20),15,17-hexaen-7-yl]pentanediamide | 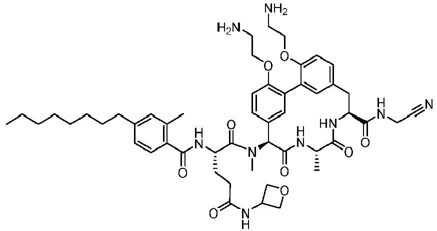 |
| **448** | rac-(2S)-N'-[2-hydroxy-1-(hydroxymethyl)ethyl]-N-methyl-2-[(2-methyl-4-octyl-benzoyl)amino]-N-[rac-(7S,10S,13S)-3,18-bis(2-aminoethoxy)-13-(cyanomethylcarbamoyl)-10-methyl-8,11-dioxo-9,12-diazatricyclo[13.3.1.12,6]icosa-1(19),2,4,6(20),15,17-hexaen-7-yl]pentanediamide | 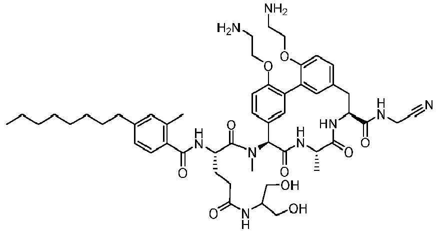 |
| **449** | rac-(8S,11S,14S)-3,18-bis(2-aminoethoxy)-N-(cyanomethyl)-11-methyl-14-[methyl-[rac-(2S)-2-[(2-methyl-4-octyl-benzoyl)amino]-4-(sulfamoylamino)butanoyl]amino]-10,13-dioxo-9,12-diazatricyclo[13.3.1.12,6]icosa-1(18),2(20),3,5,15(19),16-hexaene-8-carboxamide | 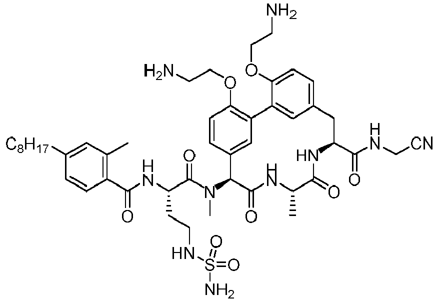 |
| **450** | rac-(8S,11S,14S)-3,18-bis(2-aminoethoxy)-N-(cyanomethyl)-11-methyl-14-[methyl-[rac-(2S)-2-[(2-methyl-4-octyl-benzoyl)amino]-5- ureido-pentanoyl]amino-10,13-dioxo-9,12-diazatricyclo[13.3.1.12,6]icosa-l(18),2(20),3,5,15(19),16-hexaene-8-carboxamide | 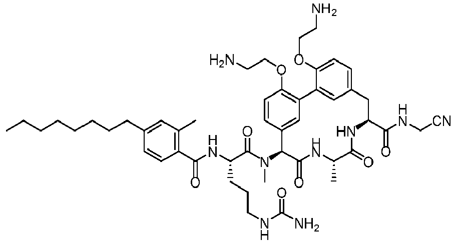 |
| **451** | rac-(8S,11S,14S)-3,18-bis(2-aminoethoxy)-N-(cyanomethyl)-11-methyl-14-[methyl-[rac-(2S)-2-[[4-methyl-2-(4-pentylphenyl)pyrimidine-5- carbonyl]amino]-4-ureido-butanoyl]amino-10,13-dioxo-9,12-diazatricyclo[13.3.1.12,6]icosa-1(18),2(20),3,5,15(19),16-hexaene-8-carboxamide | 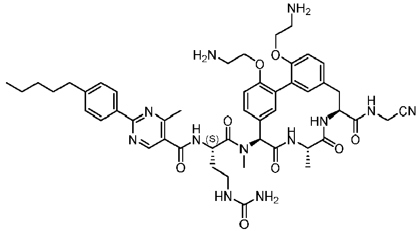 |
| **452** | rac-(8S,11S,14S)-3,18-bis(2-aminoethoxy)-N-(cyanomethyl)-11-methyl-14-[methyl-[rac-(2S)-2-[[2-(4-tert-butylphenyl)-4-methylpyrimidine-5-carbonyl]amino]-4-ureido-butanoyl]amino-10,13-dioxo-9,12-diazatricyclo[13.3.1.12,6]icosa-l(18),2(20),3,5,15(19),16-hexaene-8-carboxamide | 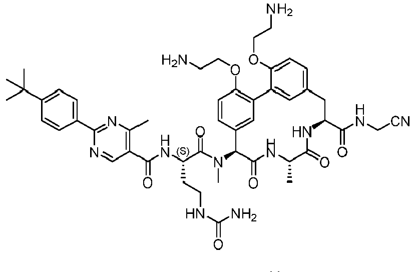 |
| **453** | rac-(8S,11S,14S)-3,18-bis(2-aminoethoxy)-N-(cyanomethyl)-11-methyl-14-[methyl-[rac-(2S)-2-[(4-heptyl-2-methyl-benzoyl)amino]-3-(methylamino)propanoyl]amino]- 10,13-dioxo-9,12-diazatricyclo[13.3.1.12,6]icosa-l(18),2(20),3,5,15(19),16-hexaene-8-carboxamide | 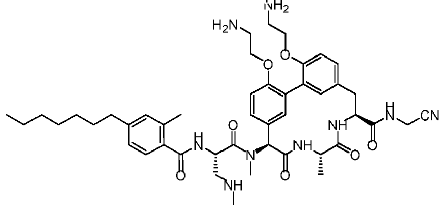 |
| **454** | rac-(8S,11S,14S)-3,18-bis(2-aminoethoxy)-N-(cyanomethyl)-11-methyl-14-[methyl-[rac-(2R)-2-[(4-heptyl-2-methyl-benzoyl)amino]-3-sulfanyl-propanoyl]amino-10,13-dioxo-9,12-diazatricyclo[13.3.1.12,6]icosa-l(18),2(20),3,5,15(19),16-hexaene-8-carboxamide | 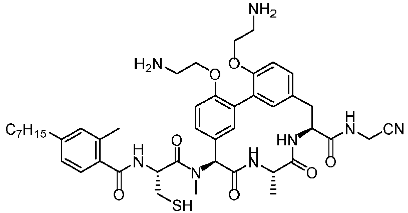 |
| **455** | | 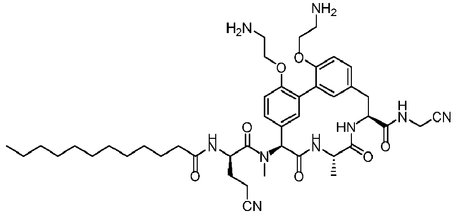 |
| **456** | rac-(8S,11S,14S)-3,18-bis(2-aminoethoxy)-N-(cyanomethyl)-11-methyl-14- [methyl- [rac-(2R)-4-amino-2-[(4-heptyl-2-methylbenzoyl)amino]butanoyl]amino]-10,13-dioxo-9,12-diazatricyclo[13.3.1.12,6]icosa-1(18),2(20),3,5,15(19),16-hexaene-8-carboxamide | 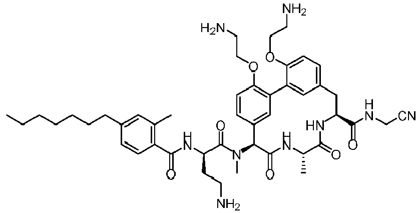 |
| **457** | rac-(8S,11S,14S)-3,18-bis(2-aminoethoxy)-N-(cyanomethyl)-11-methyl-14-[methyl-[rac-(2S)-3-amino-2-[[2-(4-tert-butylphenyl)-4-methyl-pyrimidine-5-carbonyl]amino]propanoyl]amino]- 10,13-dioxo-9,12-diazatricyclo[13.3.1.12,6]icosa-l(18),2(20),3,5,15(19),16-hexaene-8-carboxamide | 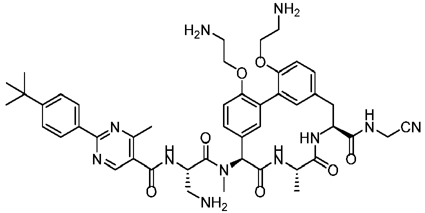 |
| **458** | rac-(8S,11S,14S)-3,18-bis(2-aminoethoxy)-N-(cyanomethyl)-11-methyl-14-[methyl-[rac-(2S)-4-hydroxy-2-[[2-(4-isobutylphenyl)-4-methyl-pyrimidine-5-carbonyl]aminobutanoyl]amino-10,13-dioxo-9,12-diazatricyclo[13.3.1.12,6]icosa-l(18),2(20),3,5,15(19),16-hexaene-8-carboxamide | 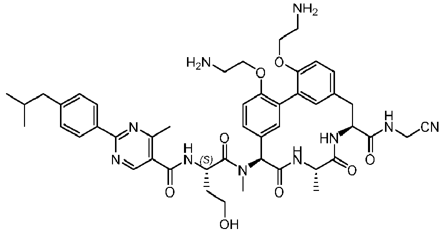 |
| **459** | rac-(8S,11S,14S)-3,18-bis(2-aminoethoxy)-N-(cyanomethyl)-11-methyl-14-[methyl-[rac-(2S)-2-[[2-(4-tert-butylphenyl)-4-methylpyrimidine-5-carbonyl]amino]-4-hydroxy-butanoyl]amino-10,13-dioxo-9,12-diazatricyclo[13.3.1.12,6]icosa-l(18),2(20),3,5,15(19),16-hexaene-8-carboxamide | 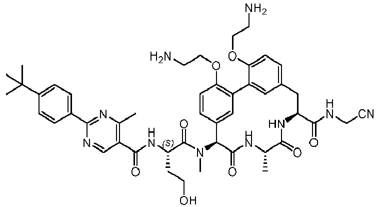 |
| **460** | rac-(8S,11S,14S)-3,18-bis(2-aminoethoxy)-N-(cyanomethyl)-11-methyl-14-[methyl-[rac-(2S)-2-[[2-(4-tert-butylphenyl)-4-methylpyrimidine-5-carbonyl]amino]-4-(methoxyamino)butanoyl]amino]- 10,13-dioxo-9,12-diazatricyclo[13.3.1.12,6]icosa-l(18),2(20),3,5,15(19),16-hexaene-8-carboxamide | 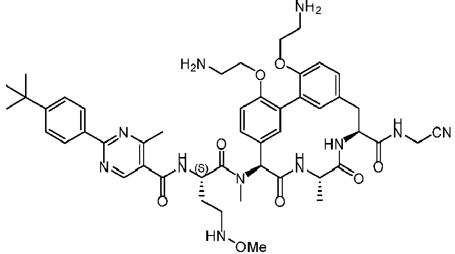 |
| **461** | rac-(8S,11S,14S)-3,18-bis(2-aminoethoxy)-N-(cyanomethyl)-11-methyl-14-[methyl-[rac-(2S,4R)-4-(aminomethyl)-1-(4-hexyl-2-methylbenzoyl)pyrrolidine-2-carbonyl]amino-10,13-dioxo-9,12-diazatricyclo[13.3.1.12,6]icosa-1(18),2(20),3,5,15(19),16-hexaene-8-carboxamide | 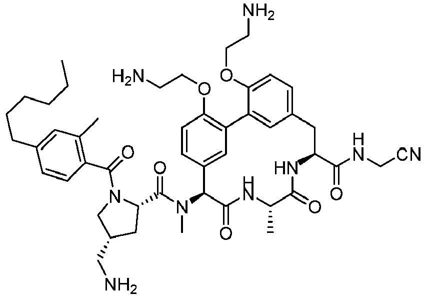 |
| **462** | rac-(8S,11S,14S)-3,18-bis(2-aminoethoxy)-N-(cyanomethyl)-11-methyl-14-[methyl-[rac-(2S)-2-[[2-(4-tert-butylphenyl)-4-methylpyrimidine-5-carbonyl]amino]-3-guanidino-propanoyl]amino]-10,13-dioxo-9,12-diazatricyclo[13.3.1.12,6]icosa-1(18),2(20),3,5,15(19),16-hexaene-8-carboxamide | 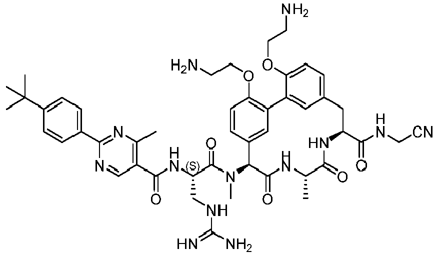 |
| **463** | | 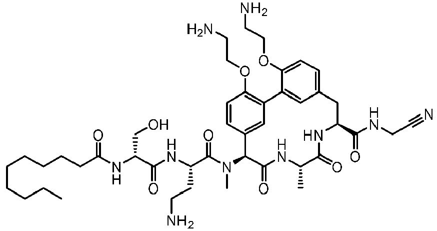 |
| **464** | rac-(8S,11S,14S)-3,18-bis(2-aminoethoxy)-N-(cyanomethyl)-11-methyl-14-[methyl-[rac-(2S)-4-amino-2-[[rac-(2S)-2-[[2-(4-tert-butylphenyl)-4,6-dimethyl-pyrimidine-5-carbonyl]amino]propanoyl]amino]bu tanoyl]amino-10,13-dioxo-9,12-diazatricyclo[13.3.1.12,6]icosa-l(18),2(20),3,5,15(19),16-hexaene-8-carboxamide | 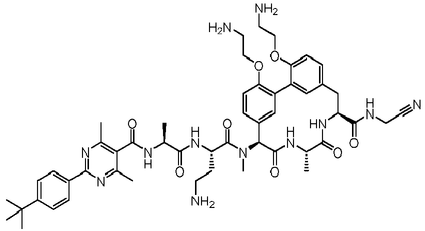 |
| **465** | rac-(8S,11S,14S)-3,18-bis(2-aminoethoxy)-N-(cyanomethyl)-11-methyl-14-[methyl-[rac-(2S)-4-amino-2-[[rac-(2S)-2-[[2-(4-tert-butylphenyl)-4,6-dimethyl-pyrimidine-5-carbonyl]amino]-3-hydroxy-propanoyl] amino] butanoyl] amino]- 10,13-dioxo-9,12-diazatricyclo[13.3.1.12,6]icosa-1(18),2(20),3,5,15(19),16-hexaene-8-carboxamide | 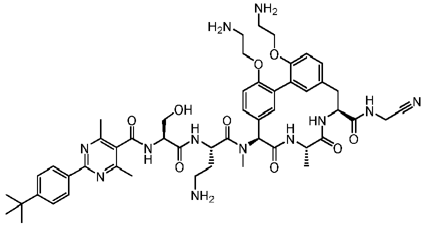 |
| **466** | rac-(8S,11S,14S)-3,18-bis(2-aminoethoxy)-N-(cyanomethyl)-11-methyl-14-[methyl-[rac-(2S)-4-amino-2-[[rac-(2S)-3-tert-butoxy-2-[[2-(4-tert-butylphenyl)-4,6-dimethyl-pyrimidine-5-carbonyl]amino]propanoyl]amino]bu tanoyl]amino-10,13-dioxo-9,12-diazatricyclo[13.3.1.12,6]icosa-l(18),2(20),3,5,15(19),16-hexaene-8-carboxamide | 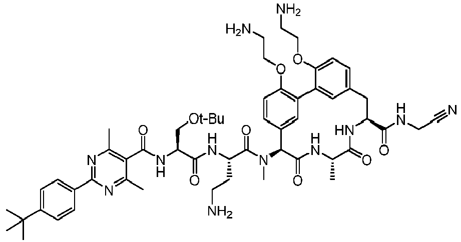 |
| **467** | rac-(8S,11S,14S)-3,18-bis(2-aminoethoxy)-N-(cyanomethyl)-11-methyl-14- [methyl- [rac-(2S)-4-amino-2-[[rac-(2R)-2-[[2-(4-tert-butylphenyl)-4,6-dimethyl-pyrimidine-5-carbonyl]amino]-3-hydroxy-propanoyl] amino] butanoyl] amino]- 10,13-dioxo-9,12-diazatricyclo[13.3.1.12,6]icosa-1(18),2(20),3,5,15(19),16-hexaene-8-carboxamide | 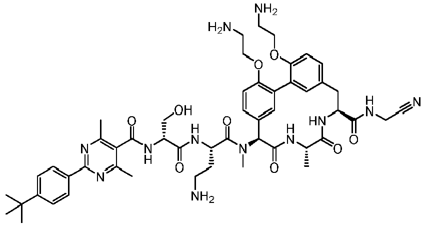 |
| **468** | rac-(8S,11S,14S)-3,18-bis(2-aminoethoxy)-N-(cyanomethyl)-11-methyl-14-[methyl-[rac-(2S)-4-amino-2-[[rac-(2R)-3-tert-butoxy-2-[[2-(4-tert-butylphenyl)-4,6-dimethyl-pyrimidine-5-carbonyl]amino]propanoyl]amino]bu tanoyl]amino-10,13-dioxo-9,12-diazatricyclo[13.3.1.12,6]icosa-l(18),2(20),3,5,15(19),16-hexaene-8-carboxamide | 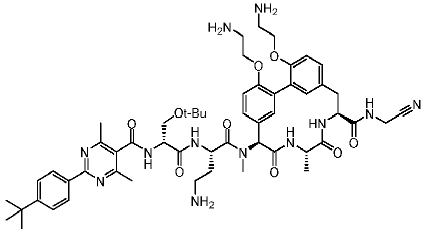 |
| **469** | rac-(8S,11S,14S)-3,18-bis(2-aminoethoxy)-N-(cyanomethyl)-11-methyl-14-[methyl-[rac-(2S)-4-amino-2-[[rac-(2S)-2-[[2-(4-tert-butylphenyl)-4,6-dimethyl-pyrimidine-5-carbonyl]amino]pentanoyl]amino]bu tanoyl]amino-10,13-dioxo-9,12-diazatricyclo[13.3.1.12,6]icosa-1(18),2(20),3,5,15(19),16-hexaene-8-carboxamide | 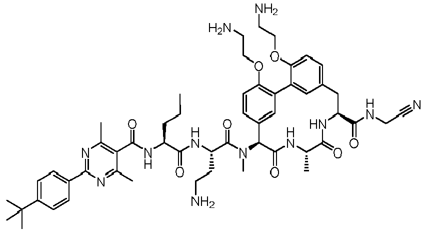 |
| **470** | rac-(8S,11S,14S)-3,18-bis(2-aminoethoxy)-N-(cyanomethyl)-11-methyl-14- [methyl- [rac-(2S)-4-amino-2-[[2-[[2-(4-tert-butylphenyl)-4,6-dimethyl-pyrimidine-5-carbonyl]amino]acetyl]amino]butan oyl]amino]-10,13-dioxo-9,12-diazatricyclo[13.3.1.12,6]icosa-1(18),2(20),3,5,15(19),16-hexaene-8-carboxamide | 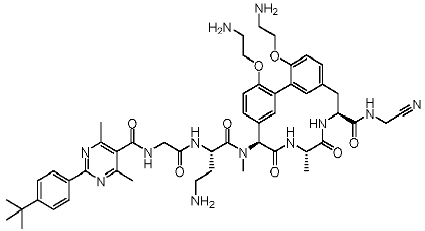 |
| **471** | rac-(8S,11S,14S)-N-(cyanomethyl)-3,18-dihydroxy-11-methyl-10,13-dioxo-14-[[rac-(2S)-4-amino-2-[(4-hexyl-2-methylbenzoyl)amino]butanoyl]amino]-9,12-diazatricyclo[13.3.1.12,6]icosa-l(18),2(20),3,5,15(19),16-hexaene-8-carboxamide | 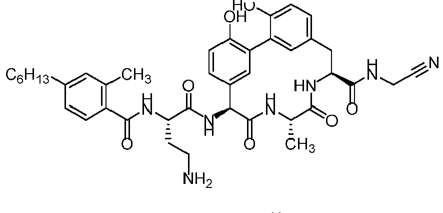 |
| **472** | rac-(8S,11S,14S)-3,18-bis(2-aminoethoxy)-N-(cyanomethyl)-11-methyl-10,13-dioxo-14-[[rac-(2S)-4-amino-2-[[2-(4-tert-butylphenyl)-4-methyl-pyrimidine-5-carbonyl]aminobutanoyl]amino-9,12-diazatricyclo[13.3.1.12,6]icosa-l(18),2(20),3,5,15(19),16-hexaene-8-carboxamide | 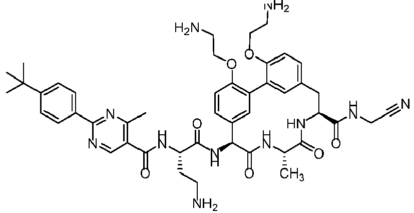 |
| **473** | rac-(8S,11S,14S)-N-(cyanomethyl)-14-[ethyl-[rac-(2S)-4-amino-2-[(4-hexyl-2-methyl-benzoyl)amino]butanoyl]amino]-3,18-dihydroxy-11-methyl-10,13-dioxo-9,12-diazatricyclo[13.3.1.12,6]icosa-1(18),2(20),3,5,15(19),16-hexaene-8-carboxamide | 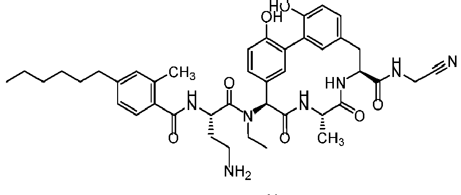 |
| **474** | rac-(8S,11S,14S)-14-[2-aminoethyl-[rac-(2S)-4-amino-2- [(4-hexyl-2-methylbenzoyl)amino]butanoyl]amino]-N-(cyanomethyl)-3,18-dihydroxy-11-methyl-10,13-dioxo-9,12-diazatricyclo[13.3.1.12,6]icosa-1(18),2(20),3,5,15(19),16-hexaene-8-carboxamide | 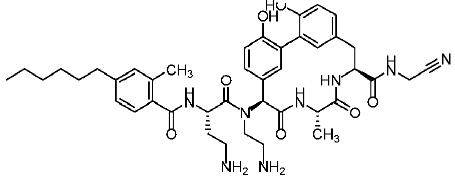 |
| **475** | rac-(8S,11S,14S)-N-(cyanomethyl)-3,18-dihydroxy-14-[2-hydroxyethyl-[rac-(2S)-4-amino-2- [(4-hexyl-2-methylbenzoyl)amino]butanoyl]amino]-11-methyl-10,13-dioxo-9,12-diazatricyclo[13.3.1.12,6]icosa-1(18),2(20),3,5,15(19),16-hexaene-8-carboxamide | 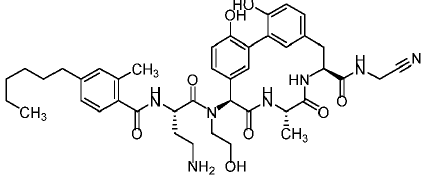 |
| **476** | rac-(8S,14S)-3,18-bis(2-aminoethoxy)-N-(cyanomethyl)-14-[methyl-[rac-(2S)-4-amino-2-[[2-(4-tert-butylphenyl)-4-methylpyrimidine-5-carbonyl]aminobutanoyl]amino-10,13-dioxo-9,12-diazatricyclo[13.3.1.12,6]icosa-l(18),2(20),3,5,15(19),16-hexaene-8-carboxamide | 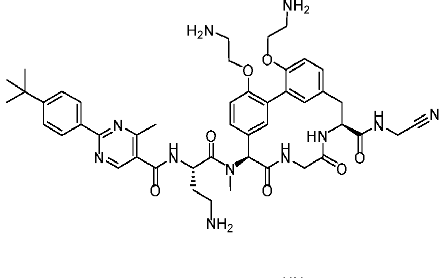 |
| **477** | rac-(8S,11S,14S)-3,18-bis(2-aminoethoxy)-N-(cyanomethyl)-11-(hydroxymethyl)-14-[methyl-[rac-(2S)-4-amino-2-[[2-(4-tert-butylphenyl)-4-methyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 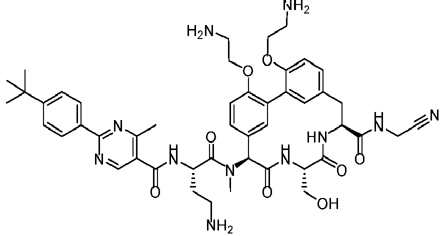 |
| **478** | rac-(8S,11S,14S)-3,18-bis(2-aminoethoxy)-N-(cyanomethyl)-11-cyclopropyl- 14-[methyl-[rac-(2S)-4-amino-2-[(4-heptyl-2-methylbenzoyl)amino]butanoyl]amino]-10,13-dioxo-9,12-diazatricyclo[13.3.1.12,6]icosa-1(18),2(20),3,5,15(19),16-hexaene-8-carboxamide | 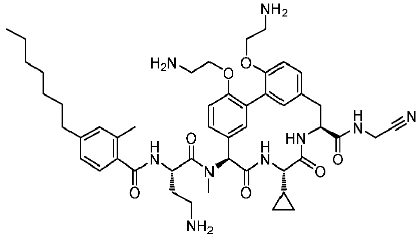 |
| **479** | rac-(8S,11S,14S)-3,18-bis(2-aminoethoxy)-N-(cyanomethyl)-11-ethyl-14-[methyl-[rac-(2S)-4-amino-2-[(4-heptyl-2-methylbenzoyl)amino]butanoyl]amino]-10,13-dioxo-9,12-diazatricyclo[13.3.1.12,6]icosa-1(18),2(20),3,5,15(19),16-hexaene-8-carboxamide | 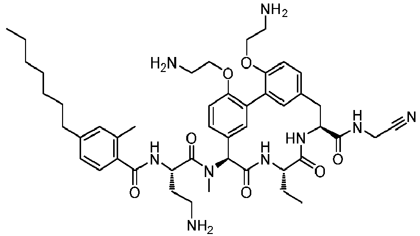 |
| **480** | rac-(8S,11S,14S)-3,18-bis(2-aminoethoxy)-N-(cyanomethyl)-11-ethyl-10,13-dioxo-14-[[rac-(2S)-4-amino-2-[(4-heptyl-2-methylbenzoyl)amino]butanoyl]amino]-9,12-diazatricyclo[13.3.1.12,6]icosa-1(18),2(20),3,5,15(19),16-hexaene-8-carboxamide | 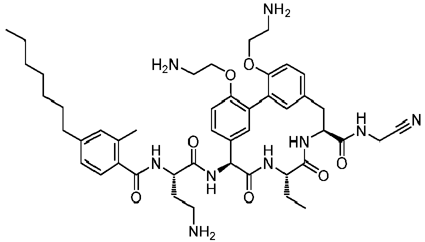 |
| **481** | rac-(8S,11S,14S)-3,18-bis(2-aminoethoxy)-14-[[4-amino-1-(4-hexyl-2-methyl-benzoyl)pyrrolidine-2-carbonyl]-methyl-amino]-N-(cyanomethyl)-11-methyl-10,13-dioxo-9,12-diazatricyclo[13.3.1.12,6]icosa-l(18),2(20),3,5,15(19),16-hexaene-8-carboxamide | 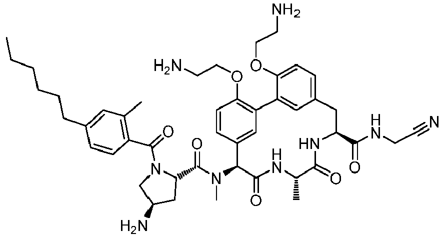 |
| **482** | rac-(8S,11S,14S)-3,18-bis(2-aminoethoxy)-14-[(4-amino-1-tetradecanoyl-pyrrolidine-2-carbonyl)-methyl-amino]-N-(cyanomethyl)-11-methyl-10,13-dioxo-9,12-diazatricyclo[13.3.1.12,6]icosa-1(18),2(20),3,5,15(19),16-hexaene-8-carboxamide | 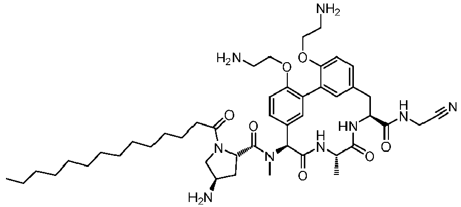 |
| **483** | rac-(8S,11S,14S)-3,18-bis(2-aminoethoxy)-14-[(4-amino-1-tetradecanoyl-pyrrolidine-2-carbonyl)-methyl-amino]-N-(cyanomethyl)-11-methyl-10,13-dioxo-9,12-diazatricyclo[13.3.1.12,6]icosa-1(18),2(20),3,5,15(19),16-hexaene-8-carboxamide | 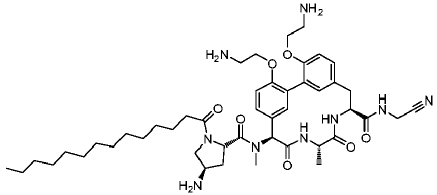 |
| **484** | | 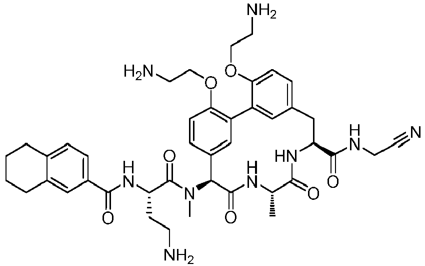 |
| **485** | | 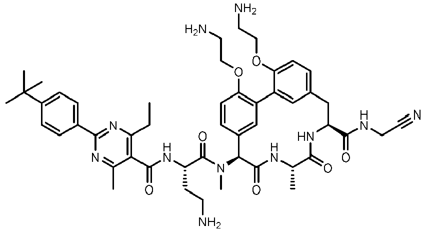 |
| **486** | | 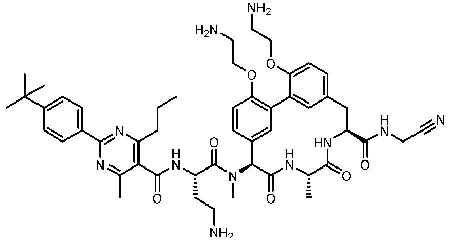 |
| **487** | | 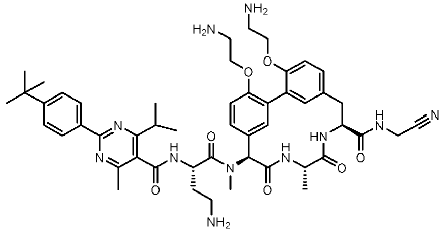 |
| **488** | | 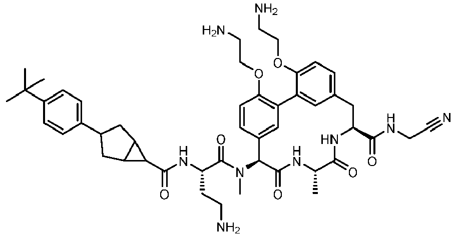 |
| **489** | | 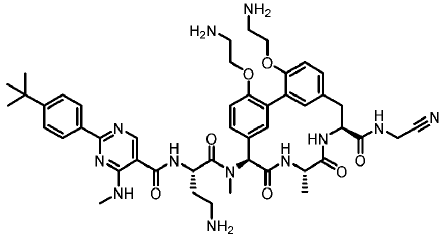 |
| **490** | | 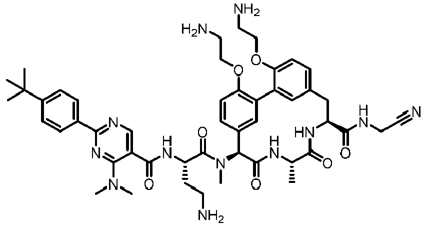 |
| **491** | rac-(8S,11S,14S)-3,18-bis(2-aminoethoxy)-N-(cyanomethyl)-11-methyl-14-[methyl-[rac-(2S)-3-[(2-aminoacetyl)amino]-2-[[2-(4-tert-butylphenyl)-4-methyl-pyrimidine-5-carbonyl] amino]propanoyl] amino] - 10,13-dioxo-9,12-diazatricyclo[13.3.1.12,6]icosa-l(18),2(20),3,5,15(19),16-hexaene-8-carboxamide | 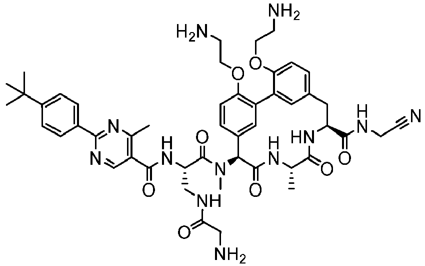 |
| **492** | (8S,11S,14S)-14-[[(2S)-4-amino-2-[[2-(4-tert-butylphenyl)-4,6-dimethyl-pyrimidine-5-carbonyl]amino]-4-methyl-pentanoyl-methyl-amino]-3,18-bis(2-aminoethoxy)-N-(cyanomethyl)-11-methyl-10,13-dioxo-9,12-diazatricyclo[13.3.1.12,6]icosa-l(18),2(20),3,5,15(19),16-hexaene-8-carboxamide | 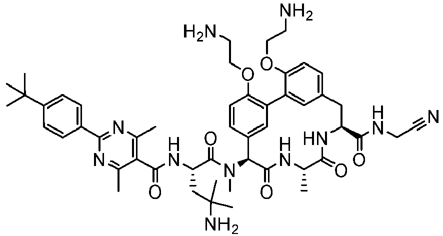 |
| **493** | rac-(8S,11S,14S)-3,18-bis(2-aminoethoxy)-N-(cyanomethyl)-11-methyl-14-[methyl-[rac-(2S)-4-[(2-amino-2-oxo-ethyl)amino]-2-[[2-(4-tert-butylphenyl)-4-methylpyrimidine-5-carbonyl]aminobutanoyl]amino-10,13-dioxo-9,12-diazatricyclo[13.3.1.12,6]icosa-1(18),2(20),3,5,15(19),16-hexaene-8-carboxamide | 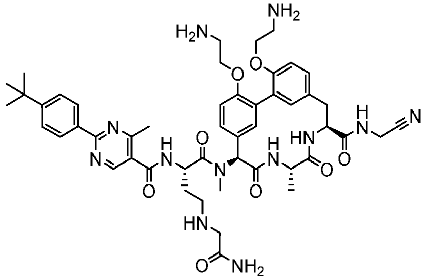 |
| **494** | rac-(8S,11S,14S)-3,18-bis(2-aminoethoxy)-N-(cyanomethyl)-11-methyl-14-[methyl-[rac-(2S)-2-[[2-(4-tert-butylphenyl)-4-methylpyrimidine-5-carbonyl]amino]-4-formamido-butanoyl]amino]-10,13-dioxo-9,12-diazatricyclo[13.3.1.12,6]icosa-l(18),2(20),3,5,15(19),16-hexaene-8-carboxamide | 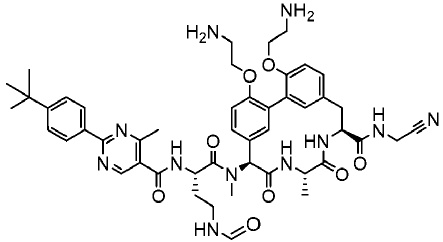 |
| **495** | rac-(8S,11S,14S)-3,18-bis(2-aminoethoxy)-N-(cyanomethyl)-11-methyl-14-[methyl-[rac-(2S)-3-(2-aminoethoxy)-2-[[2-(4-tert-butylphenyl)-4,6-dimethyl-pyrimidine-5-carbonyl] amino]propanoyl]amino]- 10,13-dioxo-9,12-diazatricyclo[13.3.1.12,6]icosa-l(18),2(20),3,5,15(19),16-hexaene-8-carboxamide | 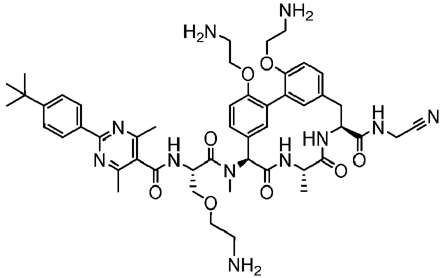 |
| **496** | (8S,11S,14S)-14-[[(2S)-4-acetamido-2-[[2-[4-(cyclohexoxy)phenyl]-4,6-dimethyl-pyrimidine-5-carbonyl]aminobutanoyl]-methyl-amino]-3,18-bis(2-aminoethoxy)-N-(cyanomethyl)-11-methyl-10,13-dioxo-9,12-diazatricyclo[13.3.1.12,6]icosa-l(18),2(20),3,5,15(19),16-hexaene-8-carboxamide | 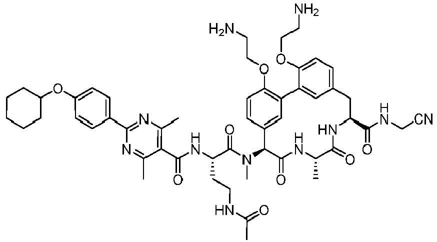 |
| **497** | (8S,11S,14S)-3,18-bis(2-aminoethoxy)-N-(cyanomethyl)-14-[[(2S)-2-[[2-[4-(cyclohexoxy)phenyl]-4,6-dimethyl-pyrimidine-5-carbonyl]amino]-4-(methanesulfonamido)butanoyl]-methyl-amino]-11-methyl-10,13-dioxo-9,12-diazatricyclo[13.3.1.12,6]icosa-l(18),2(20),3,5,15(19),16-hexaene-8-carboxamide | 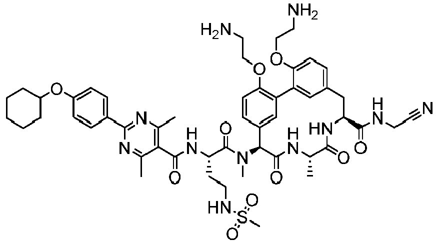 |
| **498** | (8S,11S,14S)-3,18-bis(2-aminoethoxy)-N-(cyanomethyl)-14-[[(2S)-2-[[2-[4-(cyclohexoxy)phenyl]-4,6-dimethyl-pyrimidine-5-carbonyl]amino]-4-ureido-butanoyl]-methyl-amino]-11-methyl-10,13-dioxo-9,12-diazatricyclo[13.3.1.12,6]icosa-l(18),2(20),3,5,15(19),16-hexaene-8-carboxamide | 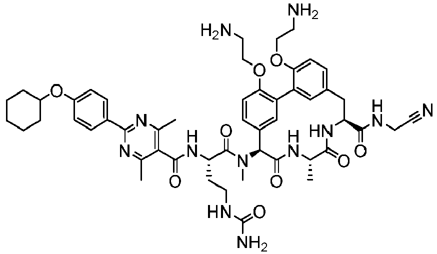 |
| **499** | (8S,11S,14S)-3,18-bis(2-aminoethoxy)-N-(cyanomethyl)-14-[[(2S)-2-[[2-[4-(cyclohexoxy)phenyl]-4,6-dimethyl-pyrimidine-5-carbonyl]amino]-3-ureido-propanoyl]-methyl-amino]- 11-methyl-10,13-dioxo-9,12-diazatricyclo[13.3.1.12,6]icosa-1(18),2(20),3,5,15(19),16-hexaene-8-carboxamide | 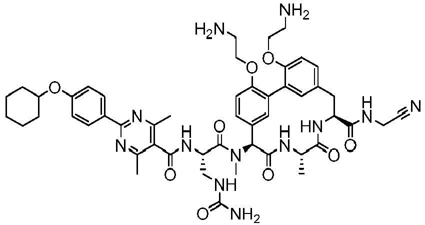 |
| **500** | (8S,11S,14S)-3,18-bis(2-aminoethoxy)-N-(cyanomethyl)-14-[[(2S)-2-[[2-[4-(cyclohexoxy)phenyl]-4,6-dimethyl-pyrimidine-5-carbonyl]amino]-4-(sulfamoylamino)butanoyl]-methyl-amino]-11-methyl-10,13-dioxo-9,12-diazatricyclo[13.3.1.12,6]icosa-1(18),2(20),3,5,15(19),16-hexaene-8-carboxamide | 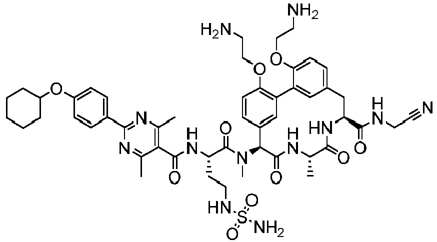 |
| **501** | (8S,11S,14S)-3,18-bis(2-aminoethoxy)-N-(cyanomethyl)-14-[[(2S)-2-[[2-[4-(cyclohexoxy)phenyl]-4,6-dimethyl-pyrimidine-5-carbonyl]amino]-5-ureido-pentanoyl]-methyl-amino]- 11-methyl-10,13-dioxo-9,12-diazatricyclo[13.3.1.12,6]icosa-1(18),2(20),3,5,15(19),16-hexaene-8-carboxamide | 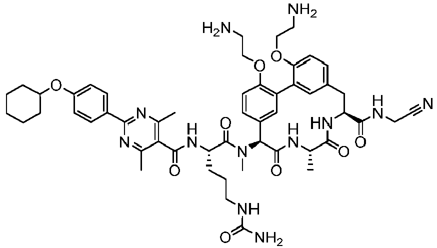 |
| **502** | (8S,11S,14S)-3,18-bis(2-aminoethoxy)-N-(cyanomethyl)-14-[[(2S)-2-[[2-[4-(cyclohexoxy)phenyl]-4,6-dimethyl-pyrimidine-5-carbonyl]amino]-3-(sulfamoylamino)propanoyl]- methyl-amino]-11-methyl-10,13-dioxo-9,12-diazatricyclo[13.3.1.12,6]icosa-l(18),2(20),3,5,15(19),16-hexaene-8-carboxamide | 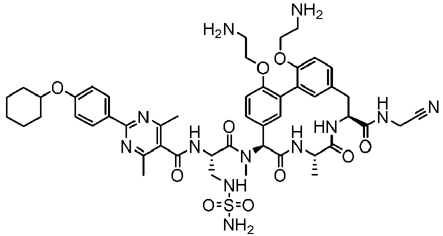 |
| **503** | (8S,11S,14S)-3,18-bis(2-aminoethoxy)-N-(cyanomethyl)-14-[[(2S)-2-[[2-[4-(cyclohexoxy)phenyl]-4,6-dimethyl-pyrimidine-5-carbonyl]amino]-5-(sulfamoylamino)pentanoyl]-methyl-amino]-11-methyl-10,13-dioxo-9,12-diazatricyclo[13.3.1.12,6]icosa-1(18),2(20),3,5,15(19),16-hexaene-8-carboxamide | 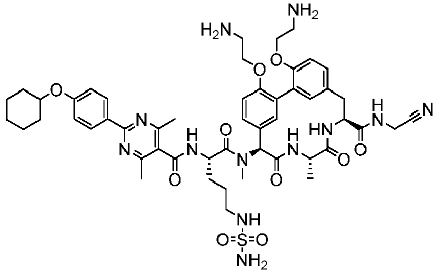 |
| **504** | rac-(8S,11S,14S)-3,18-bis(2-aminoethoxy)-N-(cyanomethyl)-11-methyl-14-[methyl-[rac-(2S)-2-[[2-[4-(cyclohexoxy)phenyl]-4,6-dimethyl-pyrimidine-5-carbonyl]amino]-4-formamido-butanoyl]amino-10,13-dioxo-9,12-diazatricyclo[13.3.1.12,6]icosa-l(18),2(20),3,5,15(19),16-hexaene-8-carboxamide | 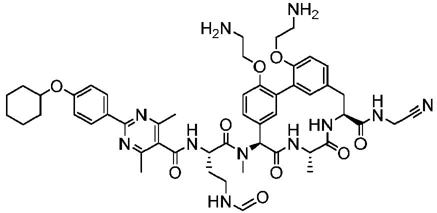 |
| **505** | rac-(2S)-2-[[2-[4-(cyclohexoxy)phenyl]-4,6-dimethyl-pyrimidine-5-carbonyl]amino-N-methyl-N-[rac-(7S,10S,13S)-3,18-bis(2-aminoethoxy)-13-(cyanomethylcarbamoyl)-10-methyl-8,11-dioxo-9,12-diazatricyclo[13.3.1.12,6]icosa-1(19),2,4,6(20),15,17-hexaen-7-yl]pentanediamide | 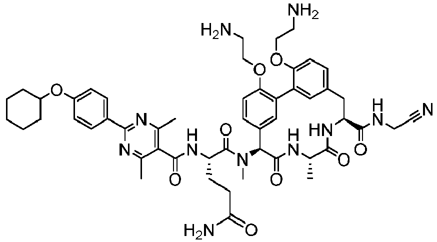 |
| **506** | (2S)-N-[(7S,10S,13S)-3,18-bis(2-aminoethoxy)-13-(cyanomethylcarbamoyl)-10-methyl-8,11-dioxo-9,12-diazatricyclo[13.3.1.12,6]icosa-1(19),2,4,6(20),15,17-hexaen-7-yl]-2-[[2-[4-(cyclohexoxy)phenyl]-4,6-dimethyl-pyrimidine-5-carbonyl]amino-N-methyl-butanediamide | 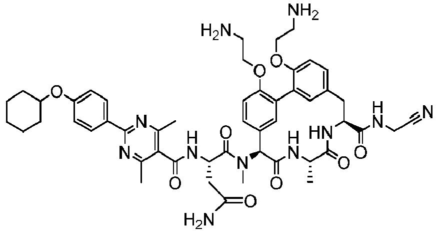 |
| **507** | (8S,11S,14S)-3,18-bis(2-aminoethoxy)-N-(cyanomethyl)-14-[[(2S)-2-[[2-[4-(cyclohexoxy)phenyl]-4,6-dimethyl-pyrimidine-5-carbonyl]amino]-3-hydroxy-propanoyl]-methyl-amino]- 11-methyl-10,13-dioxo-9,12-diazatricyclo[13.3.1.12,6]icosa-1(18),2(20),3,5,15(19),16-hexaene-8-carboxamide | 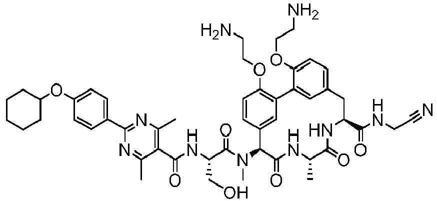 |
| **508** | (8S,11S,14S)-3,18-bis(2-aminoethoxy)-N-(cyanomethyl)-14-[[(2R)-2-[[2-[4-(cyclohexoxy)phenyl]-4,6-dimethyl-pyrimidine-5-carbonyl]amino]-3-hydroxy-propanoyl]-methyl-amino]- 11-methyl-10,13-dioxo-9,12-diazatricyclo[13.3.1.12,6]icosa-l(18),2(20),3,5,15(19),16-hexaene-8-carboxamide | 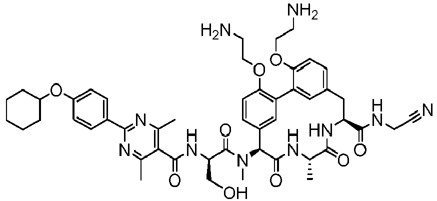 |
| **509** | (8S,11S,14S)-3,18-bis(2-aminoethoxy)-N-(cyanomethyl)-14-[[(2S)-2-[[2-[4-(cyclohexoxy)phenyl]-4,6-dimethyl-pyrimidine-5-carbonyl]amino]-4-hydroxy-butanoyl]-methyl-amino]- 11-methyl-10,13-dioxo-9,12-diazatricyclo[13.3.1.12,6]icosa-1(18),2(20),3,5,15(19),16-hexaene-8-carboxamide | 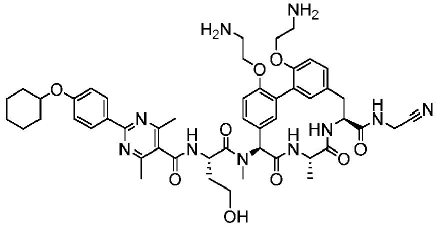 |
| **510** | (8S,11S,14S)-14-[[(2S)-4-amino-2-[[(2S)-2-[[2-[4-(cyclohexoxy)phenyl]-4,6-dimethyl-pyrimidine-5-carbonyl]aminobutanoyl]aminobut anoyl]-methyl-amino]-3,18-bis(2-aminoethoxy)-N-(cyanomethyl)-11-methyl-10,13-dioxo-9,12-diazatricyclo[13.3.1.12,6]icosa-1(18),2(20),3,5,15(19),16-hexaene-8-carboxamide | 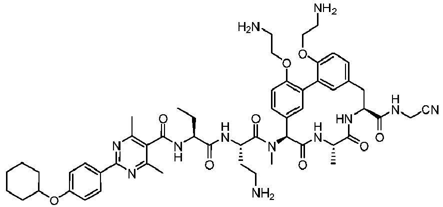 |
| **511** | (8S,11S,14S)-3,18-bis(2-aminoethoxy)-N-(cyanomethyl)-14-[[(2S)-2-[[2-[4-(cyclohexoxy)phenyl]-4,6-dimethyl-pyrimidine-5-carbonyl]amino-4-[(2-hydroxyacetyl)amino]butanoyl]- methyl-amino]-11-methyl-10,13-dioxo-9,12-diazatricyclo[13.3.1.12,6]icosa-l(18),2(20),3,5,15(19),16-hexaene-8-carboxamide | 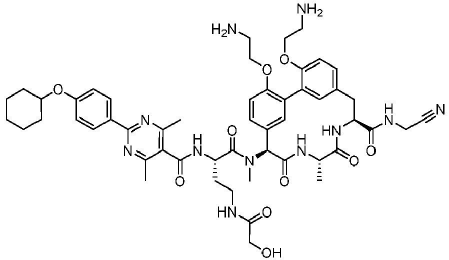 |
| **512** | (8S,11S,14S)-3,18-bis(2-aminoethoxy)-N-(cyanomethyl)-14-[[(2S)-2-[[2-[4-(cyclohexoxy)phenyl]-4,6-dimethyl-pyrimidine-5-carbonyl]amino]-4-(hydroxycarbamoylamino)butanoyl]-methyl-amino]-11-methyl-10,13-dioxo-9,12-diazatricyclo[13.3.1.12,6]icosa-l(18),2(20),3,5,15(19),16-hexaene-8-carboxamide | 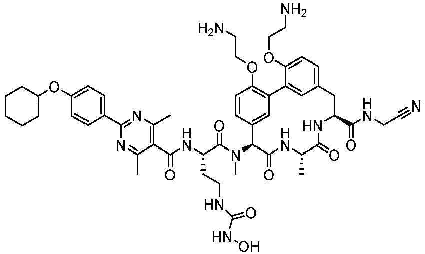 |
| **513** | rac-(8S,11S,14S)-3,18-bis(2-aminoethoxy)-N-(cyanomethyl)-11-methyl-14-[methyl-[rac-(2S)-4-amino-2-[[4,6-dimethyl-2-(4-pentylphenyl)pyrimidine-5- carbonyl]aminobutanoyl]amino-10,13-dioxo-9,12-diazatricyclo[13.3.1.12,6]icosa-1(18),2(20),3,5,15(19),16-hexaene-8-carboxamide | 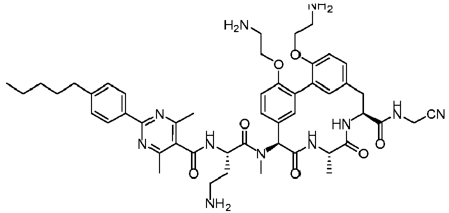 |
| **514** | rac-(8S,11S,14S)-18-(2-aminoethoxy)-N-(cyanomethyl)-3-hydroxy-11-methyl-14-[methyl-[rac-(2S)-4-amino-2-[[4,6-dimethyl-2-(4-trimethylsilylphenyl)pyrimidine-5-carbonyl]aminobutanoyl]amino-10,13-dioxo-9,12-diazatricyclo[13.3.1.12,6]icosa-1(18),2(20),3,5,15(19),16-hexaene-8-carboxamide | 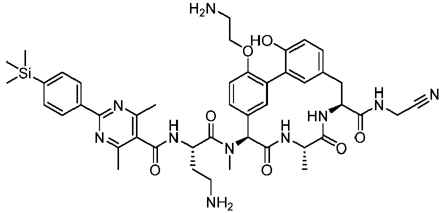 |
| **516** | rac-(8S,11S,14S)-18-(2-aminoethoxy)-N-(cyanomethyl)-3-hydroxy-11-methyl-14-[methyl-[rac-(2S)-4-amino-2-[[2-(4-ethylphenyl)-4,6-dimethyl-pyrimidine-5-carbonyl]aminobutanoyl]amino-10,13-dioxo-9,12-diazatricyclo[13.3.1.12,6]icosa-1(18),2(20),3,5,15(19),16-hexaene-8-carboxamide | 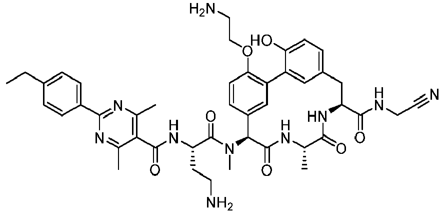 |
| **517** | rac-(8S,11S,14S)-3-(2-acetamidoethoxy)-18-(2-aminoethoxy)-N-(cyanomethyl)-11-methyl-14-[methyl-[rac-(2S)-4-amino-2-[[2-(4-tert-butylphenyl)-4,6-dimethyl-pyrimidine-5-carbonyl]aminobutanoyl]amino-10,13-dioxo-9,12-diazatricyclo[13.3.1.12,6]icosa-1(18),2(20),3,5,15(19),16-hexaene-8-carboxamide | 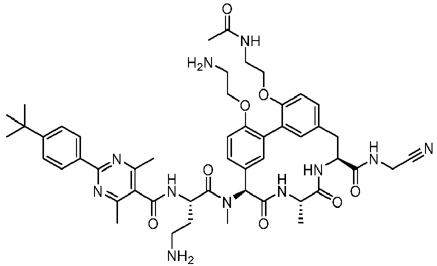 |
| **518** | rac-(8S,11S,14S)-18-(2-aminoethoxy)-N-(cyanomethyl)-3-hydroxy-11-methyl-14-[methyl-[rac-(2S)-4-amino-2-[[4,6-dimethyl-2-(4-pentoxyphenyl)pyrimidine-5-carbonyl]aminobutanoyl]amino-10,13-dioxo-9,12-diazatricyclo[13.3.1.12,6]icosa-1(18),2(20),3,5,15(19),16-hexaene-8-carboxamide | 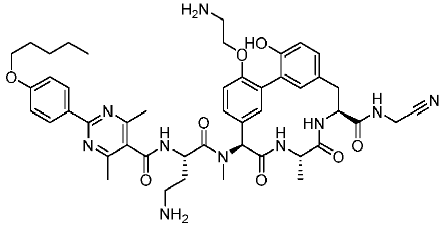 |
| **519** | rac-(8S,11S,14S)-3,18-bis(2-aminoethoxy)-N-(cyanomethyl)-11-methyl-10,13-dioxo-14-[[rac-(2S)-4-amino-2-[[2-[4-(cyclohexoxy)phenyl]-4,6-dimethyl-pyrimidine-5-carbonyl]aminobutanoyl]amino-9,12-diazatricyclo[13.3.1.12,6]icosa-1(18),2(20),3,5,15(19),16-hexaene-8-carboxamide | 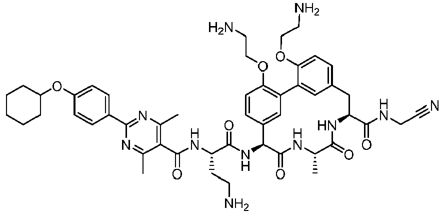 |
| **520** | rac-(8S,11S,14S)-3,18-bis(2-aminoethoxy)-N-(cyanomethyl)-11-methyl-14-[methyl-[rac-(2S)-4-amino-2-[[2,4-diamino-6-(4-tert-butylphenyl)pyridine-3-carbonyl]aminobutanoyl]amino-10,13-dioxo-9,12-diazatricyclo[13.3.1.12,6]icosa-l(18),2(20),3,5,15(19),16-hexaene-8-carboxamide | 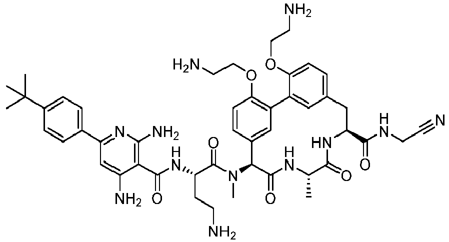 |
| **521** | rac-(8S,11S,14S)-18-(2-aminoethoxy)-N-(cyanomethyl)-3-hydroxy-11-methyl-14-[methyl-[rac-(2S)-4-amino-2-[[4,6-dimethyl-2-(4-pentylphenyl)pyrimidine-5- carbonyl]aminobutanoyl]amino-10,13-dioxo-9,12-diazatricyclo[13.3.1.12,6]icosa-l(18),2(20),3,5,15(19),16-hexaene-8-carboxamide | 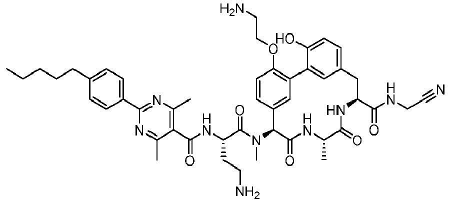 |
| **522** | rac-(8S,11S,14S)-3-(2-aminoethoxy)-N-(cyanomethyl)-18-hydroxy-11-methyl-14-[methyl-[rac-(2S)-4-amino-2-[[2-[4-(cyclohexoxy)phenyl]-4,6-dimethyl-pyrimidine-5-carbonyl]aminobutanoyl]amino-10,13-dioxo-9,12-diazatricyclo[13.3.1.12,6]icosa-l(18),2(20),3,5,15(19),16-hexaene-8-carboxamide | 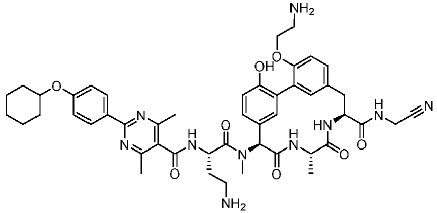 |
| **523** | rac-(8S,11S,14S)-18-(2-aminoethoxy)-N-(cyanomethyl)-3-hydroxy-11-methyl-14-[methyl-[rac-(2S)-4-amino-2-[[2-(4-tert-butylphenyl)-4-methyl-pyrimidine-5-carbonyl]amino]butanoyl]amino]- 10,13-dioxo-9,12-diazatricyclo[13.3.1.12,6]icosa-l(18),2(20),3,5,15(19),16-hexaene-8-carboxamide | 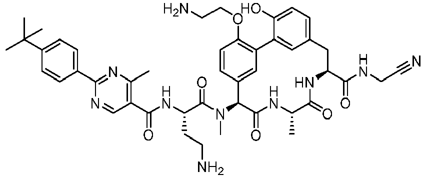 |
| **524** | rac-(8S,11S,14S)-3,18-bis(2-aminoethoxy)-N-(cyanomethyl)-11-methyl-14-[methyl-[rac-(2S)-4-amino-2-[[2-(4-hydroxyphenyl)-4-methyl-pyrimidine-5-carbonyl]aminobutanoyl]amino-10,13-dioxo-9,12-diazatricyclo[13.3.1.12,6]icosa-1(18),2(20),3,5,15(19),16-hexaene-8-carboxamide | 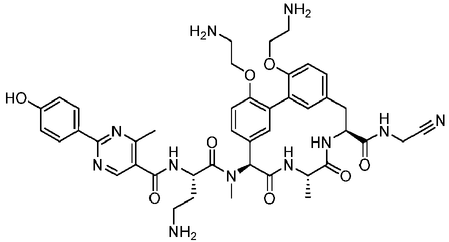 |
| **525** | rac-(8S,11S,14S)-18-(2-aminoethoxy)-N-(cyanomethyl)-3-hydroxy-11-methyl-14-[methyl-[rac-(2S)-4-amino-2-[[6-(4-isobutylphenyl)-4-methyl-pyridine-3-carbonyl]amino]butanoyl]amino]- 10,13-dioxo-9,12-diazatricyclo[13.3.1.12,6]icosa-1(18),2(20),3,5,15(19),16-hexaene-8-carboxamide | 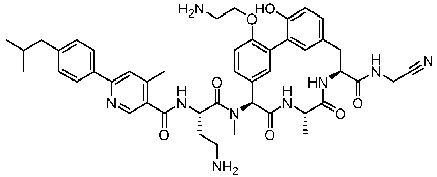 |
| **526** | rac-(8S,11S,14S)-18-(2-aminoethoxy)-N-(cyanomethyl)-3-hydroxy-11-methyl-14-[methyl-[rac-(2S)-4-amino-2-[[6-(4-tert-butylphenyl)-2-methyl-pyridine-3-carbonyl]amino]butanoyl]amino-10,13-dioxo-9,12-diazatricyclo[13.3.1.12,6]icosa-1(18),2(20),3,5,15(19),16-hexaene-8-carboxamide | 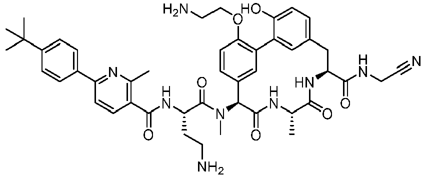 |
| **527** | rac-(8S,11S,14S)-3,18-bis(2-aminoethoxy)-N-(cyanomethyl)-11-methyl-14-[methyl-[rac-(2S)-4-amino-2-[[2-[4-(3,3-dimethylbutoxy)-1-piperidyl]-4,6-dimethyl-pyrimidine-5-carbonyl]aminobutanoyl]amino-10,13-dioxo-9,12-diazatricyclo[13.3.1.12,6]icosa-1(18),2(20),3,5,15(19),16-hexaene-8-carboxamide | 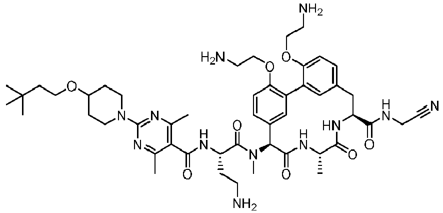 |
| **528** | rac-(8S,11S,14S)-3-(2-aminoethoxy)-N-(cyanomethyl)-18-hydroxy-11-methyl-14-[methyl-[rac-(2S)-4-amino-2-[[2-[4-(3,3-dimethylbutoxy)phenyl]-4,6-dimethyl-pyrimidine-5-carbonyl]amino]butanoyl]amino-10,13-dioxo-9,12-diazatricyclo[13.3.1.12,6]icosa-1(18),2(20),3,5,15(19),16-hexaene-8-carboxamide | 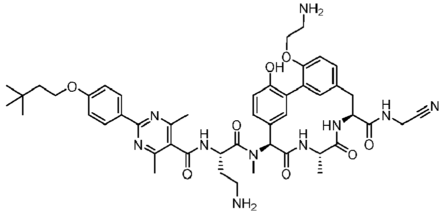 |
| **529** | rac-(8S,11S,14S)-3,18-bis(2-aminoethoxy)-N-(cyanomethyl)-11-methyl-14-[methyl-[rac-(2S)-4-amino-2-[[4-amino-6-(5 -butyl-2-pyridyl)-2-methyl-pyridine-3-carbonyl]amino]butanoyl]amino-10,13-dioxo-9,12-diazatricyclo[13.3.1.12,6]icosa-1(18),2(20),3,5,15(19),16-hexaene-8-carboxamide | 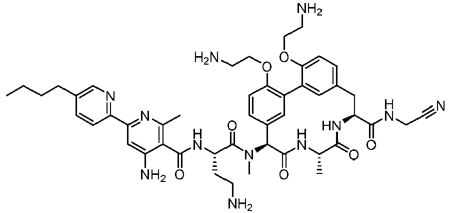 |
| **530** | rac-(8S,11S,14S)-18-(2-aminoethoxy)-N-(cyanomethyl)-3-hydroxy-11-methyl-14-[methyl-[rac-(2S)-4-amino-2-[[6-(4-tert-butylphenyl)-2,4-dimethyl-pyridine-3-carbonyl]amino]butanoyl]amino]- 10,13-dioxo-9,12-diazatricyclo[13.3.1.12,6]icosa-l(18),2(20),3,5,15(19),16-hexaene-8-carboxamide | 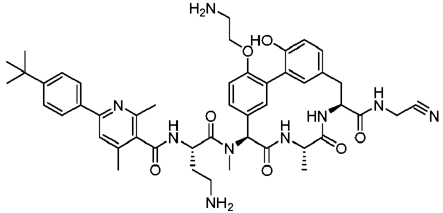 |
| **531** | rac-(8S,11S,14S)-18-(2-aminoethoxy)-N-(cyanomethyl)-3-hydroxy-11-methyl-14-[methyl-[rac-(2S)-4-amino-2-[[2-(l,l-dimethylindan-5-yl)-4,6-dimethyl-pyrimidine-5-carbonyl]aminobutanoyl]amino-10,13-dioxo-9,12-diazatricyclo[13.3.1.12,6]icosa-1(18),2(20),3,5,15(19),16-hexaene-8-carboxamide | 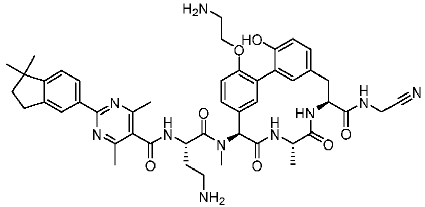 |
| **532** | rac-(8S,11S,14S)-3,18-bis(2-aminoethoxy)-N-(cyanomethyl)-11-methyl-14-[methyl-[rac-(2S)-4-amino-2-[[4-amino-2-[4-(cycloheptoxy)phenyl]-6-methylpyrimidine-5-carbonyl]amino]butanoyl]amino-10,13-dioxo-9,12-diazatricyclo[13.3.1.12,6]icosa-l(18),2(20),3,5,15(19),16-hexaene-8-carboxamide | 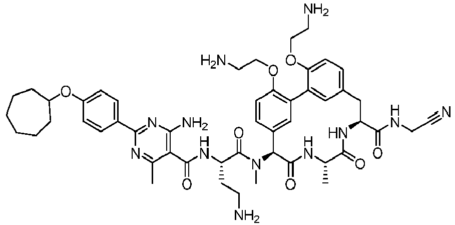 |
| **533** | rac-(8S,11S,14S)-3,18-bis(2-aminoethoxy)-N-(cyanomethyl)-11-methyl-14-[methyl-[rac-(2S)-2-[[5-acetamido-6-(4-tert-butylphenyl)-2-methyl-pyridine-3-carbonyl]amino]- 4-amino-butanoyl]amino]-10,13-dioxo-9,12-diazatricyclo[13.3.1.12,6]icosa-l(18),2(20),3,5,15(19),16-hexaene-8-carboxamide | 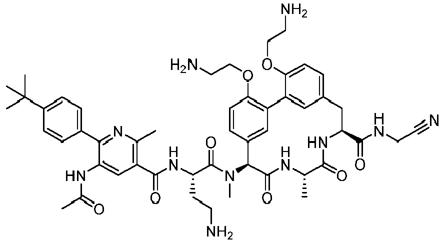 |
| **534** | rac-(8S,11S,14S)-3,18-bis(2-aminoethoxy)-N-(cyanomethyl)-11-methyl-14-[methyl-[rac-(2S)-4-amino-2-[[4-amino-2-[5-(3,3-dimethylbutoxy)-2-pyridyl]-6-methyl-pyrimidine-5-carbonyl]amino]butanoyl]amino-10,13-dioxo-9,12-diazatricyclo[13.3.1.12,6]icosa-1(18),2(20),3,5,15(19),16-hexaene-8-carboxamide | 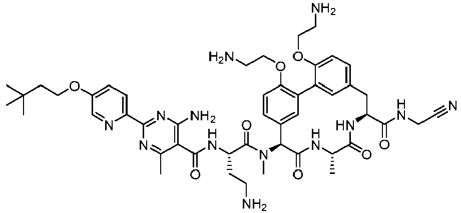 |
| **535** | rac-(8S,11S,14S)-18-(2-aminoethoxy)-N-(cyanomethyl)-3-hydroxy-11-methyl-14-[methyl-[rac-(2S)-4-amino-2-[[2-[4-(1,1-dimethylpropyl)phenyl]-4-methylpyrimidine-5-carbonyl]amino]butanoyl]amino-10,13-dioxo-9,12-diazatricyclo[13.3.1.12,6]icosa-1(18),2(20),3,5,15(19),16-hexaene-8-carboxamide | 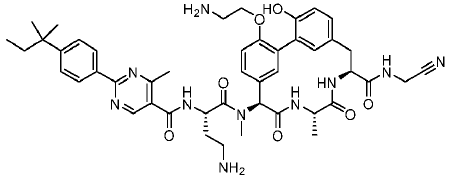 |
| **536** | rac-(8S,11S,14S)-3-(2-aminoethoxy)-N-(cyanomethyl)-18-hydroxy-11-methyl-14-[methyl-[rac-(2S)-4-amino-2-[[4,6-dimethyl-2-(4-pentoxyphenyl)pyrimidine-5-carbonyl]amino]butanoyl]amino-10,13-dioxo-9,12-diazatricyclo[13.3.1.12,6]icosa-1(18),2(20),3,5,15(19),16-hexaene-8-carboxamide | 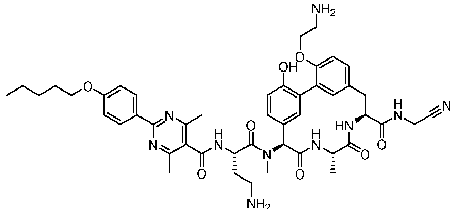 |
| **537** | rac-(8S,11S,14S)-3,18-bis(2-aminoethoxy)-N-(cyanomethyl)-11-methyl-14- [methyl-[rac-(2S)-4-amino-2-[[6-(4-tert-butylphenyl)-2-methyl-5-(trifluoromethyl)pyridine-3-carbonyl]amino]butanoyl]amino]- 10,13-dioxo-9,12-diazatricyclo[13.3.1.12,6]icosa-1(18),2(20),3,5,15(19),16-hexaene-8-carboxamide | 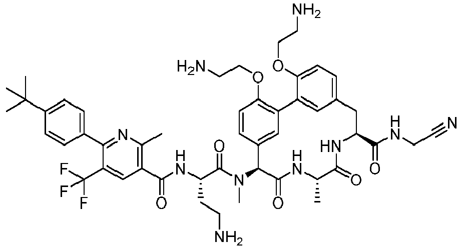 |
| **538** | rac-(8S,11S,14S)-3,18-bis(2-aminoethoxy)-N-(cyanomethyl)-11-methyl-14-[methyl-[rac-(2S)-4-amino-2-[[4-amino-6-[4-(3,3-dimethylbutoxy)phenyl]-2-methyl-pyridine-3-carbonyl]amino]butanoyl]amino-10,13-dioxo-9,12-diazatricyclo[13.3.1.12,6]icosa-1(18),2(20),3,5,15(19),16-hexaene-8-carboxamide | 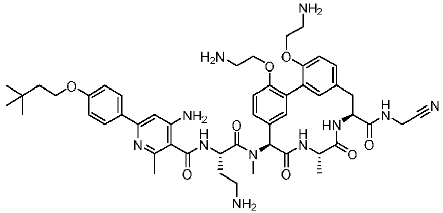 |
| **539** | rac-(8S,11S,14S)-18-(2-aminoethoxy)-N-(cyanomethyl)-3-hydroxy-11-methyl-14-[methyl-[rac-(2S)-4-amino-2-[[2-[4-(3,3-dimethylbutoxy)phenyl]-4,6-dimethyl-pyrimidine-5-carbonyl]amino]butanoyl]amino-10,13-dioxo-9,12-diazatricyclo[13.3.1.12,6]icosa-1(18),2(20),3,5,15(19),16-hexaene-8-carboxamide | 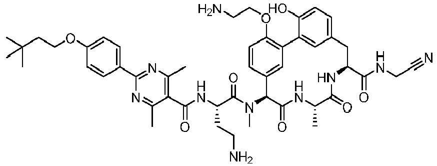 |
| **540** | rac-(8S,11S,14S)-3,18-bis(2-aminoethoxy)-N-(cyanomethyl)-11-methyl-14-[methyl-[rac-(2S)-4-amino-2-[[2-(2-tert-butylpyrimidin-5-yl)-4,6-dimethyl-pyrimidine-5-carbonyl]amino]butanoyl]amino-10,13-dioxo-9,12-diazatricyclo[13.3.1.12,6]icosa-l(18),2(20),3,5,15(19),16-hexaene-8-carboxamide | 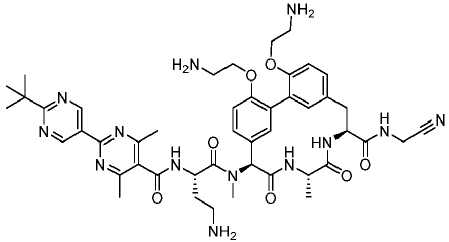 |
| **541** | rac-(8S,11S,14S)-18-(2-aminoethoxy)-N-(cyanomethyl)-3-hydroxy-11-methyl-14-[methyl-[rac-(2S)-4-amino-2-[[4-(4-tert-butylphenyl)-2,6-dimethylbenzoyl]amino]butanoyl]amino]-10,13-dioxo-9,12-diazatricyclo[13.3.1.12,6]icosa-l(18),2(20),3,5,15(19),16-hexaene-8-carboxamide | 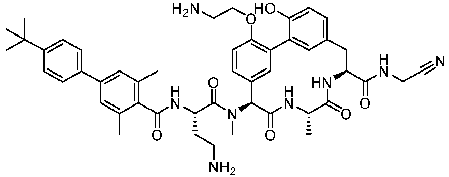 |
| **542** | rac-(8S,11S,14S)-3,18-bis(2-aminoethoxy)-N-(cyanomethyl)-11-methyl-14-[methyl-[rac-(2S)-4-amino-2-[[2-(4-hydroxyphenyl)-4,6-dimethyl-pyrimidine-5-carbonyl]amino]butanoyl]amino-10,13-dioxo-9,12-diazatricyclo[13.3.1.12,6]icosa-l(18),2(20),3,5,15(19),16-hexaene-8-carboxamide | 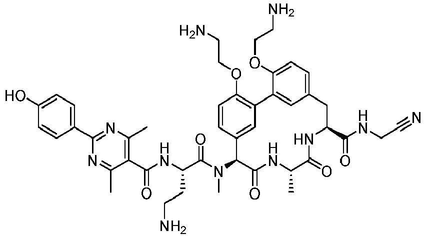 |
| **543** | rac-(8S,11S,14S)-18-(2-aminoethoxy)-N-(cyanomethyl)-3-hydroxy-11-methyl-14-[methyl-[rac-(2S)-4-amino-2-[[4,6-dimethyl-2-[4-(1-methylcyclopropyl)phenyl]pyrimidin e-5-carbonyl]amino]butanoyl]amino-10,13-dioxo-9,12-diazatricyclo[13.3.1.12,6]icosa-l(18),2(20),3,5,15(19),16-hexaene-8-carboxamide | 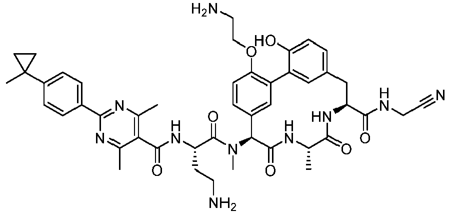 |
| **544** | rac-(8S,11S,14S)-3,18-bis(2-aminoethoxy)-N-(cyanomethyl)-11-methyl-14-[methyl-[rac-(2S)-4-amino-2-[[4-amino-6-[4-(cyclohexoxy)phenyl]-2-methyl-pyridine-3-carbonyl]amino]butanoyl]amino-10,13-dioxo-9,12-diazatricyclo[13.3.1.12,6]icosa-1(18),2(20),3,5,15(19),16-hexaene-8-carboxamide | 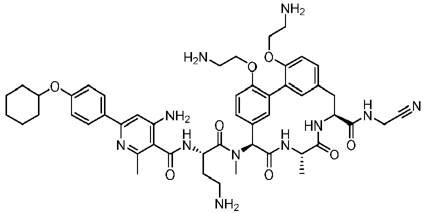 |
| **545** | rac-(8S,11S,14S)-3,18-bis(2-aminoethoxy)-N-(cyanomethyl)-11-methyl-14-[methyl-[rac-(2S)-4-amino-2-[[4-amino-6-[4-(cycloheptoxy)phenyl]-2-methyl-pyridine-3-carbonyl]amino]butanoyl]amino-10,13-dioxo-9,12-diazatricyclo[13.3.1.12,6]icosa-l(18),2(20),3,5,15(19),16-hexaene-8-carboxamide | 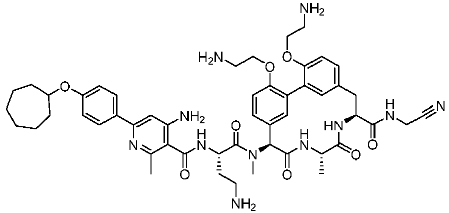 |
| **546** | rac-(8S,11S,14S)-3,18-bis(2-aminoethoxy)-N-(cyanomethyl)-11-methyl-14-[methyl-[rac-(2S)-4-amino-2-[[4-amino-6-(4-tert-butylphenyl)-2-ethyl-pyridine-3-carbonyl]amino]butanoyl]amino]-10,13-dioxo-9,12-diazatricyclo[13.3.1.12,6]icosa-l(18),2(20),3,5,15(19),16-hexaene-8-carboxamide | 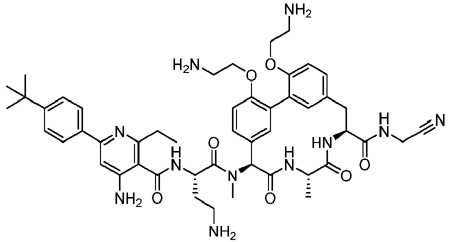 |
| **547** | rac-(8S,11S,14S)-3,18-bis(2-aminoethoxy)-N-(cyanomethyl)-11-methyl-14-[methyl-[rac-(2S)-4-amino-2-[[4-amino-2- [4-(cyclohexoxy)phenyl] -6-methylpyrimidine-5-carbonyl]amino]butanoyl]amino-10,13-dioxo-9,12-diazatricyclo[13.3.1.12,6]icosa-1(18),2(20),3,5,15(19),16-hexaene-8-carboxamide | 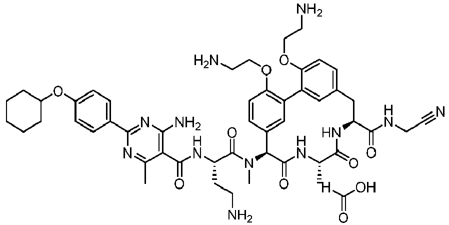 |
| **548** | rac-(8S,11S,14S)-18-(2-aminoethoxy)-N-(cyanomethyl)-3-hydroxy-11-methyl-14-[methyl-[rac-(2S)-4-amino-2-[[4-amino-2-(4-tert-butylphenyl)-6-methyl-pyrimidine-5-carbonyl]amino]butanoyl] amino] - 10,13-dioxo-9,12-diazatricyclo[13.3.1.12,6]icosa-l(18),2(20),3,5,15(19),16-hexaene-8-carboxamide | 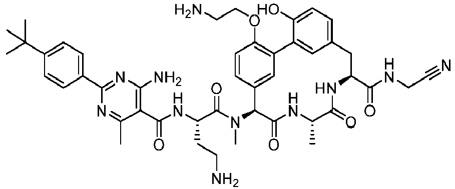 |
| **549** | rac-(8S,11S,14S)-3-(2-aminoethoxy)-N-(cyanomethyl)-18-hydroxy-11-methyl-14-[methyl-[rac-(2S)-4-amino-2-[[2-(4-tert-butylphenyl)-4-methyl-pyrimidine-5-carbonyl]amino]butanoyl]amino]-10,13-dioxo-9,12-diazatricyclo[13.3.1.12,6]icosa-1(18),2(20),3,5,15(19),16-hexaene-8-carboxamide | 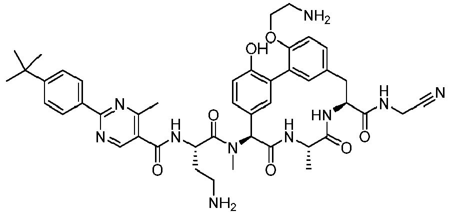 |
| **550** | rac-(8S,11S,14S)-3,18-bis(2-aminoethoxy)-N-(cyanomethyl)-11-methyl-14-[methyl-[rac-(2S)-4-amino-2-[[2-[2-(3,3-dimethylbutoxy)pyrimidin-5-yl]-4,6-dimethyl-pyrimidine-5-carbonyl]amino]butanoyl]amino-10,13-dioxo-9,12-diazatricyclo[13.3.1.12,6]icosa-l(18),2(20),3,5,15(19),16-hexaene-8-carboxamide | 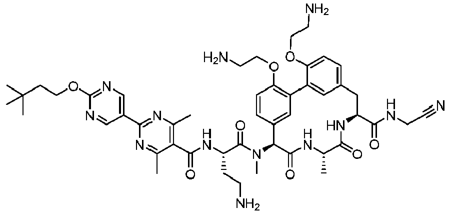 |
| **551** | rac-(8S,11S,14S)-3,18-bis(2-aminoethoxy)-N-(cyanomethyl)-11-methyl-14-[methyl-[rac-(2S)-4-amino-2-[[4-(2-amino-2-oxo-ethoxy)-6-(4-tert-butylphenyl)-2-methyl-pyridine-3-carbonyl]amino]butanoyl]amino-10,13-dioxo-9,12-diazatricyclo[13.3.1.12,6]icosa-1(18),2(20),3,5,15(19),16-hexaene-8-carboxamide | 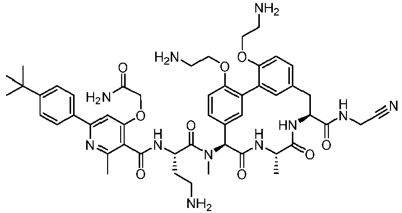 |
| **552** | rac-(8S,11S,14S)-18-(2-aminoethoxy)-N-(cyanomethyl)-3-hydroxy-11-methyl-14-[methyl-[rac-(2S)-4-amino-2-[[2-(4-isopropoxyphenyl)-4,6-dimethyl-pyrimidine-5-carbonyl]amino]butanoyl]amino-10,13-dioxo-9,12-diazatricyclo[13.3.1.12,6]icosa-l(18),2(20),3,5,15(19),16-hexaene-8-carboxamide | 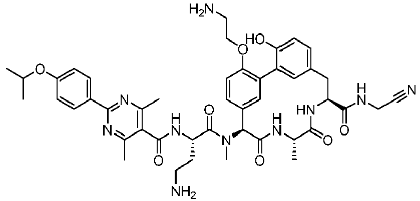 |
| **553** | rac-(8S,11S,14S)-3-(2-aminoethoxy)-N-(cyanomethyl)-18-hydroxy-11-methyl-14-[methyl-[rac-(2S)-4-amino-2-[[2-(4-tert-butylphenyl)-4,6-dimethyl-pyrimidine-5-carbonyl]amino]butanoyl]amino-10,13-dioxo-9,12-diazatricyclo[13.3.1.12,6]icosa-l(18),2(20),3,5,15(19),16-hexaene-8-carboxamide | 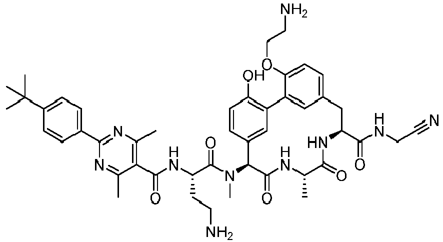 |
| **554** | rac-(8S,11S,14S)-3,18-bis(2-aminoethoxy)-N-(cyanomethyl)-11-methyl-14-[methyl-[rac-(2S)-4-amino-2-[[4-amino-6-(4-tert-butylphenyl)-2-chloro-pyridine-3-carbonyl]amino]butanoyl]amino]-10,13-dioxo-9,12-diazatricyclo[13.3.1.12,6]icosa-1(18),2(20),3,5,15(19),16-hexaene-8-carboxamide | 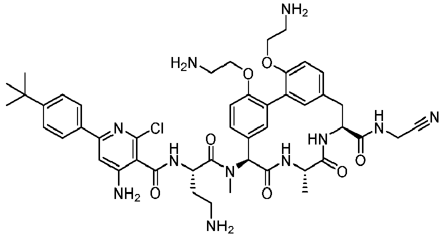 |
| **555** | rac-(8S,11S,14S)-3,18-bis(2-aminoethoxy)-N-(cyanomethyl)-11-methyl-14-[methyl-[rac-(2S)-4-amino-2-[[4,6-dimethyl-2-[4-[rac-(2R,6S)-2,6-dimethyl-1-piperidyl]phenyl]pyrimidine-5-carbonyl]amino]butanoyl]amino-10,13-dioxo-9,12-diazatricyclo[13.3.1.12,6]icosa-1(18),2(20),3,5,15(19),16-hexaene-8-carboxamide | 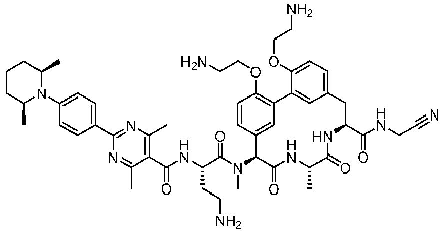 |
| **556** | rac-(8S,11S,14S)-18-(2-aminoethoxy)-N-(cyanomethyl)-3-hydroxy-11-methyl-14-[methyl-[rac-(2S)-4-amino-2-[[4-amino-6-(4-tert-butylphenyl)-2-methyl-pyridine-3-carbonyl]amino]butanoyl]amino-10,13-dioxo-9,12-diazatricyclo[13.3.1.12,6]icosa-1(18),2(20),3,5,15(19),16-hexaene-8-carboxamide | 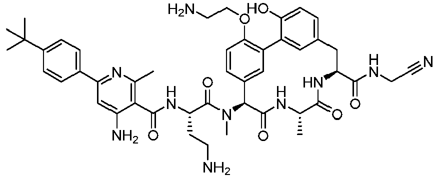 |
| **557** | rac-(8S,11S,14S)-3-(2-aminoethoxy)-N-(cyanomethyl)-18-hydroxy-11-methyl-14-[methyl-[rac-(2S)-4-amino-2-[[4-amino-6-(4-tert-butylphenyl)-2-methyl-pyridine-3-carbonyl]amino]butanoyl]amino-10,13-dioxo-9,12-diazatricyclo[13.3.1.12,6]icosa-1(18),2(20),3,5,15(19),16-hexaene-8-carboxamide | 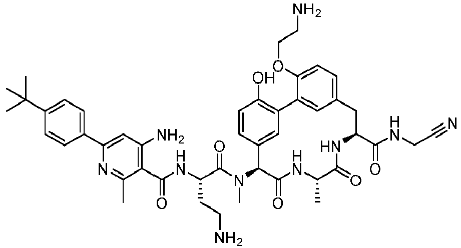 |
| **558** | rac-(8S,11S,14S)-3-(2-aminoethoxy)-N-(cyanomethyl)-18-methoxy-11-methyl-14-[methyl-[rac-(2S)-4-amino-2-[[2-(4-tert-butylphenyl)-4-methyl-pyrimidine-5-carbonyl]amino]butanoyl]amino]- 10,13-dioxo-9,12-diazatricyclo[13.3.1.12,6]icosa-1(18),2(20),3,5,15(19),16-hexaene-8-carboxamide | 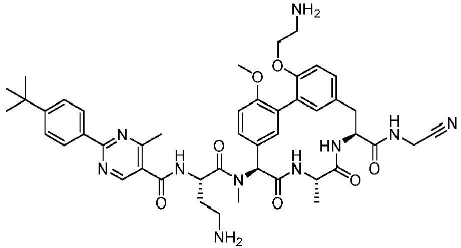 |
| **559** | rac-(8S,11S,14S)-4-acetamido-18-(2-aminoethoxy)-N-(cyanomethyl)-3-hydroxy-11-methyl-14-[methyl-[rac-(2S)-4-amino-2-[[2-(4-tert-butylphenyl)-4,6-dimethyl-pyrimidine-5-carbonyl]amino]butanoyl]amino-10,13-dioxo-9,12-diazatricyclo[13.3.1.12,6]icosa-1(18),2(20),3,5,15(19),16-hexaene-8-carboxamide | 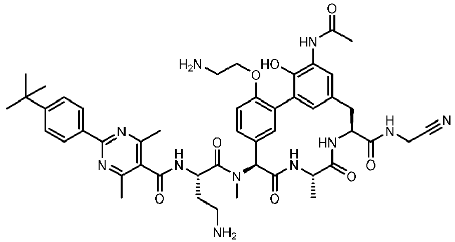 |
| **560** | rac-(8S,11S,14S)-18-(2-aminoethoxy)-N-(cyanomethyl)-4-fluoro-3-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 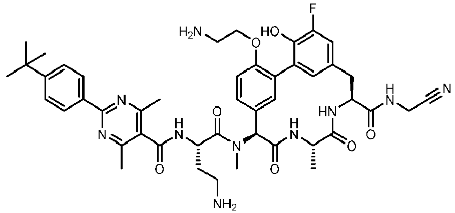 |
| **561** | rac-(8S,11S,14S)-4-(2-aminoethoxy)-N-(cyanomethyl)-3,18-dihydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2,4,6(20),15(19),16-hexaene-8-carboxamide | 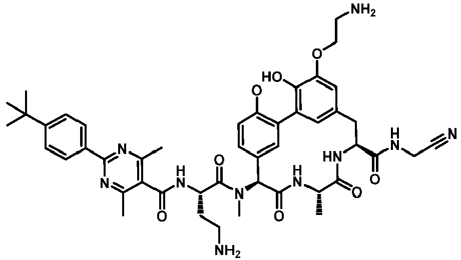 |
| **562** | rac-(8S,11S,14S)-4,18-bis(2-aminoethoxy)-N-(cyanomethyl)-3-hydroxy-11-methyl-14-[methyl-[rac-(2S)-4-amino-2-[[2-(4-tert-butylphenyl)-4,6-dimethyl-pyrimidine-5-carbonyl] amino butanoyl]amino- 10,13-dioxo-9,12-diazatricyclo[13.3.1.12,6]icosa-1(18),2,4,6(20),15(19),16-hexaene-8-carboxamide | 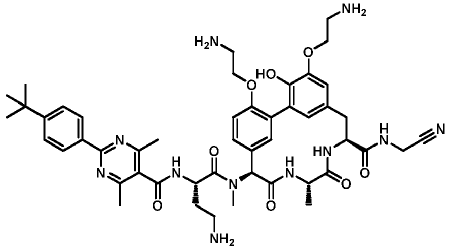 |
| **564** | rac-(8S,11S,14S)-3,18-bis(3-aminopropoxy) -N-(cyanomethyl) - 11-methyl-14-[methyl-[rac-(2S)-4-amino-2-[[4-amino-6-[4-(cycloheptoxy)phenyl]-2-methyl-pyridine-3-carbonyl]aminobutanoyl]amino- 10,13-dioxo-9,12-diazatricyclo[13.3.1.12,6]icosa-1(18),2(20),3,5,15(19),16-hexaene-8-carboxamide | 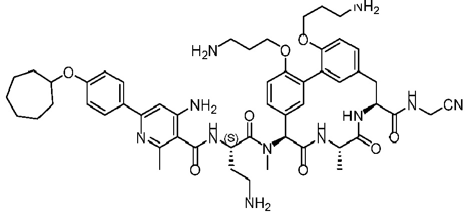 |
| **565** | rac-(8S,11S,14S)-3,18-bis(3-aminopropoxy)-N-(cyanomethyl)- 11-methyl-14-[methyl-[rac-(2S)-4-amino-2-[[4-amino-6-[4-(cyclohexoxy)phenyl]-2-methyl-pyridine-3-carbonyl]aminobutanoyl]amino- 10,13-dioxo-9,12-diazatricyclo[13.3.1.12,6]icosa-1(18),2(20),3,5,15(19),16-hexaene-8-carboxamide | 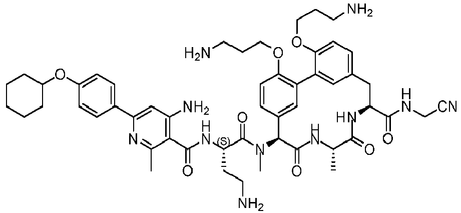 |
| **566** | rac-(8S,11S,14S)-3,18-bis(3-aminopropoxy)-N-(cyanomethyl)-11-methyl-14-[methyl-[rac-(2S)-4-amino-2-[[4-amino-2-[4-(cycloheptoxy)phenyl]-6-methylpyrimidine-5-carbonyl]amino]butanoyl]amino-10,13-dioxo-9,12-diazatricyclo[13.3.1.12,6]icosa-1(18),2(20),3,5,15(19),16-hexaene-8-carboxamide | 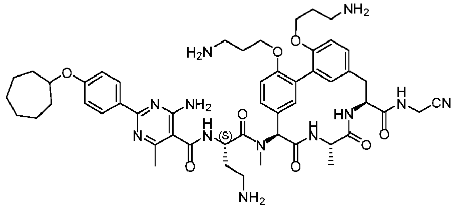 |
| **567** | rac-(8S,11S,14S)-N-(cyanomethyl)-11-methyl-14-[methyl-[rac-(2S)-4-amino-2-[[2-(4-tert-butylphenyl)-4,6-dimethyl-pyrimidine-5-carbonyl]amino]butanoyl]amino]-10,13-dioxo-3,18-bis[rac-(2R)-3-amino-2-hydroxy-propoxy]-9,12-diazatricyclo[13.3.1.12,6]icosa-1(18),2(20),3,5,15(19),16-hexaene-8-carboxamide | 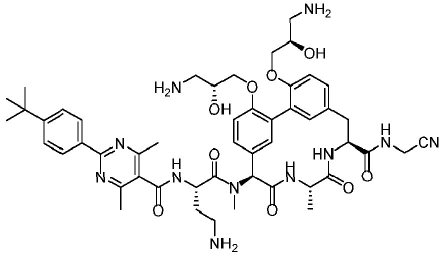 |
| **568** | rac-(8S,11S,14S)-N-(cyanomethyl)-11-methyl-14-[methyl-[rac-(2S)-4-amino-2-[[2-[4-(cyclohexoxy)phenyl]-4,6-dimethyl-pyrimidine-5-carbonyl]amino]butanoyl]amino]-10,13-dioxo-3,18-bis[rac-(2R)-3-amino-2-hydroxy-propoxy]-9,12-diazatricyclo[13.3.1.12,6]icosa-1(18),2(20),3,5,15(19),16-hexaene-8-carboxamide | 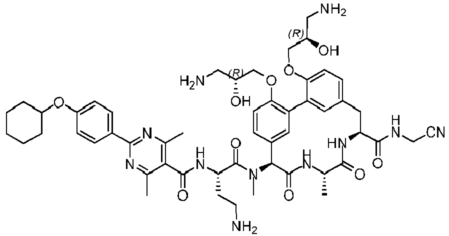 |
| **569** | rac-(8S,11S,14S)-N-(cyanomethyl)-11-methyl-14-[methyl-[rac-(2S)-4-amino-2-[[2-(4-isopropoxyphenyl)-4,6-dimethyl-pyrimidine-5-carbonyl]amino]butanoyl]amino]-10,13-dioxo-3,18-bis [rac-(2R)-3-amino-2-hydroxy-propoxy]-9,12-diazatricyclo[13.3.1.12,6]icosa-1(18),2(20),3,5,15(19),16-hexaene-8-carboxamide | 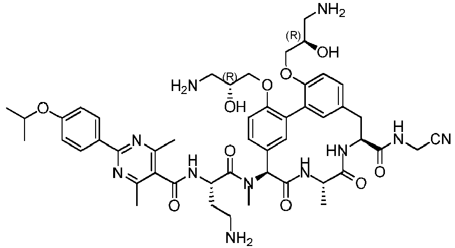 |
| **570** | rac-(8S,11S,14S)-N-(cyanomethyl)-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-3,18-bis[rac-(2S)-3-amino-2-hydroxy-propoxy]-9,12-diazatricyclo[13.3.1.12,6]icosa-1(18),2(20),3,5,15(19),16-hexaene-8-carboxamide | 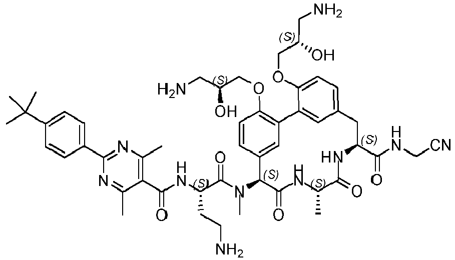 |
| **571** | rac-(8S,11S,14S)-3-(2-aminoethoxy)-N-(cyanomethyl)-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-18-[rac-(2R)-3-amino-2-hydroxy-propoxy]-9,12-diazatricyclo[13.3.1.12,6]icosa-1(18),2(20),3,5,15(19),16-hexaene-8-carboxamide | 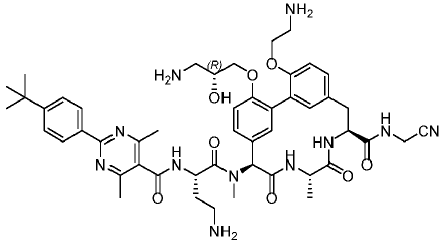 |
| **572** | rac-(8S,11S,14S)-3,18-bis(2-aminoethoxy)-N-(cyanomethyl)-11-methyl-14-[methyl-[rac-(2S)-2-[[2-(4-tert-butylphenyl)-4,6-dimethyl-pyrimidine-5-carbonyl]amino]-3-(sulfamoylamino)propanoyl]amino]- 10,13-dioxo-9,12-diazatricyclo[13.3.1.12,6]icosa-1(18),2(20),3,5,15(19),16-hexaene-8-carboxamide | 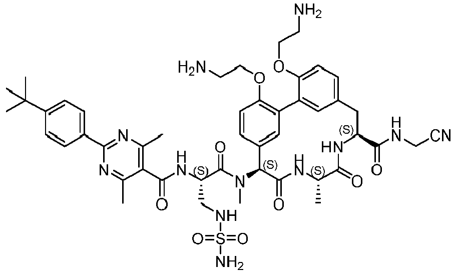 |
| **573** | rac-(8S,11S,14S)-3,18-bis(2-aminoethoxy)-N-(cyanomethyl)-11-methyl-10,13-dioxo-14-[[rac-(2S)-2-[[2-[4-(cyclohexoxy)phenyl]-4,6-dimethyl-pyrimidine-5-carbonyl]amino]-3- (sulfamoylamino)propanoyl] amino] - 9,12-diazatricyclo[13.3.1.12,6]icosa-1(18),2(20),3,5,15(19),16-hexaene-8-carboxamide | 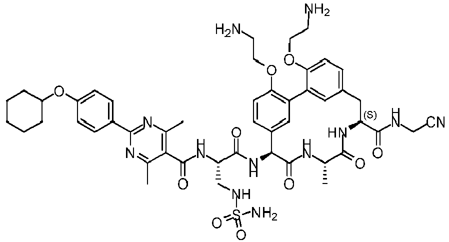 |
| **574** | rac-(8S,11S,14S)-3,18-bis(2-aminoethoxy)-N-(cyanomethyl)-11-methyl-14-[methyl-[rac-(2S)-2-[[2-(4-tert-butylphenyl)-4,6-dimethyl-pyrimidine-5-carbonyl]amino]-4-(sulfamoylamino)butanoyl]amino]-10,13-dioxo-9,12-diazatricyclo[13.3.1.12,6]icosa-1(18),2(20),3,5,15(19),16-hexaene-8-carboxamide | 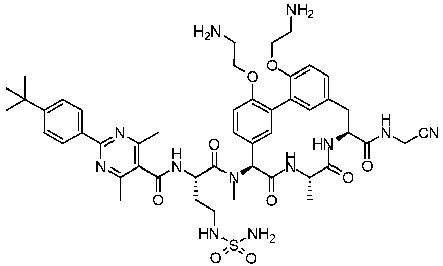 |
| **575** | rac-(8S,11S,14S)-3,18-bis(3-aminopropoxy)-N-(cyanomethyl)-11-methyl-14-[methyl-[rac-(2S)-2-[[2-(4-tert-butylphenyl)-4,6-dimethyl-pyrimidine-5-carbonyl]amino]-3- (sulfamoylamino)propanoyl]amino]- 10,13-dioxo-9,12-diazatricyclo[13.3.1.12,6]icosa-1(18),2(20),3,5,15(19),16-hexaene-8-carboxamide | 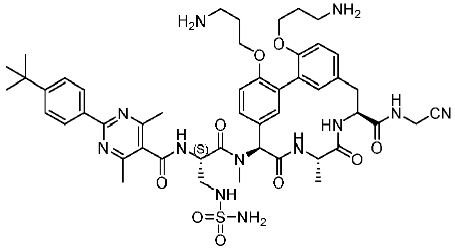 |
| **576** | rac-(8S,11S,14S)-3,18-bis(3-aminopropoxy)-N-(cyanomethyl)-11-methyl-14-[methyl-[rac-(2S)-2-[[2-[4-(cyclohexoxy)phenyl]-4,6-dimethyl-pyrimidine-5-carbonyl]amino]-3- (sulfamoylamino)propanoyl]amino]- 10,13-dioxo-9,12-diazatricyclo[13.3.1.12,6]icosa-1(18),2(20),3,5,15(19),16-hexaene-8-carboxamide | 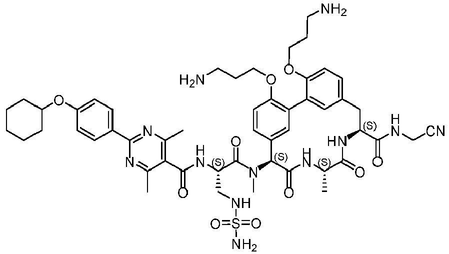 |
| **577** | rac-(8S,11S,14S)-N-(cyanomethyl)-11-methyl-14-[methyl-[rac-(2S)-2-[[2-(4-tert-butylphenyl)-4,6-dimethyl-pyrimidine-5-carbonyl]amino]-3- (sulfamoylamino)propanoyl]amino]- 10,13-dioxo-3,18-bis[rac-(2R)-3-amino-2-hydroxy-propoxy] -9,12-diazatricyclo[13.3.1.12,6]icosa-1(18),2(20),3,5,15(19),16-hexaene-8-carboxamide | 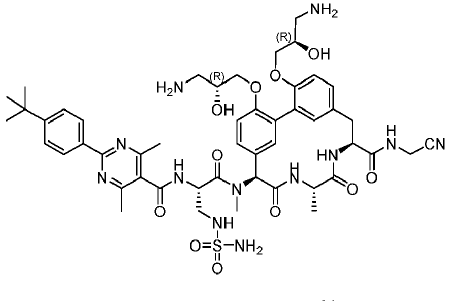 |
| **578** | rac-(8S,11S,14S)-N-(cyanomethyl)-11-methyl-14-[methyl-[rac-(2S)-2-[[2-[4-(cyclohexoxy)phenyl]-4,6-dimethyl-pyrimidine-5-carbonyl]amino]-3- (sulfamoylamino)propanoyl]amino]- 10,13-dioxo-3,18-bis [rac-(2R)-3-amino-2-hydroxy-propoxy] -9,12-diazatricyclo[13.3.1.12,6]icosa-1(18),2(20),3,5,15(19),16-hexaene-8-carboxamide | 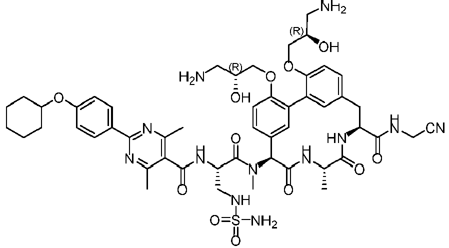 |
| **579** | rac-(8S,11S,14S)-N-(cyanomethyl)-11-methyl-14-[methyl-[rac-(2S)-2-[[2-(4-isopropoxyphenyl)-4,6-dimethyl-pyrimidine-5-carbonyl]amino]-3- (sulfamoylamino)propanoyl]amino]- 10,13-dioxo-3,18-bis[rac-(2R)-3-amino-2-hydroxy-propoxy] -9,12-diazatricyclo[13.3.1.12,6]icosa-1(18),2(20),3,5,15(19),16-hexaene-8-carboxamide | 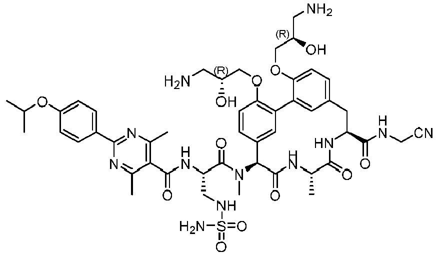 |
| **580** | rac-(8S,11S,14S)-N-(cyanomethyl)-11-methyl-14-[methyl-[rac-(2S)-2-[[2-(4-tert-butylphenyl)-4,6-dimethyl-pyrimidine-5-carbonyl]amino]-4-(sulfamoylamino)butanoyl]amino]-10,13-dioxo-3,18-bis[rac-(2R)-3-amino-2-hydroxy-propoxy]-9,12-diazatricyclo[13.3.1.12,6]icosa-1(18),2(20),3,5,15(19),16-hexaene-8-carboxamide | 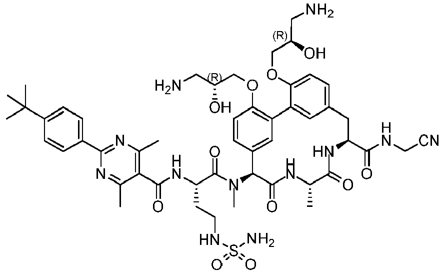 |
| **581** | rac-(8S,11S,14S)-N-(cyanomethyl)-11-methyl-14-[methyl-[rac-(2S)-2-[[2-(4-tert-butylphenyl)-4,6-dimethyl-pyrimidine-5-carbonyl]amino]-3- (sulfamoylamino)propanoyl]amino]- 10,13-dioxo-3,18-bis[rac-(2S)-3-amino-2-hydroxy-propoxy]-9,12-diazatricyclo[13.3.1.12,6]icosa-1(18),2(20),3,5,15(19),16-hexaene-8-carboxamide | 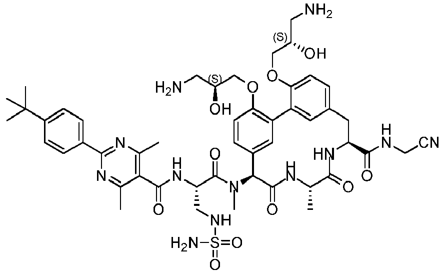 |
| **582** | rac-(8S,11S,14S)-N-(cyanomethyl)-18-hydroxy-11-methyl-14-[methyl-[rac-(2S)-2-[[2-(4-tert-butylphenyl)-4,6-dimethyl-pyrimidine-5-carbonyl]amino]-3- (sulfamoylamino)propanoyl]amino]- 10,13-dioxo-3,17-bis[rac-(2R)-3-amino-2-hydroxy-propoxy]-9,12-diazatricyclo[13.3.1.12,6]icosa-1(18),2(20),3,5,15(19),16-hexaene-8-carboxamide | 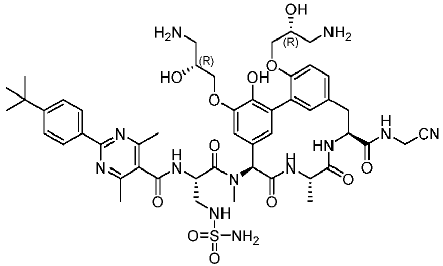 |
| **583** | (8S,11S,14S)-14-[[(2S)-4-amino-2-[[2-(4-tert-butylphenyl)-4,6-dimethyl-pyrimidine-5-carbonyl]aminobutanoyl]-methyl-amino]-3,17-bis(2-aminoethoxy)-N-(cyanomethyl)-18-hydroxy-11-methyl-10,13-dioxo-9,12-diazatricyclo[13.3.1.12,6]icosa-1(18),2(20),3,5,15(19),16-hexaene-8-carboxamide | 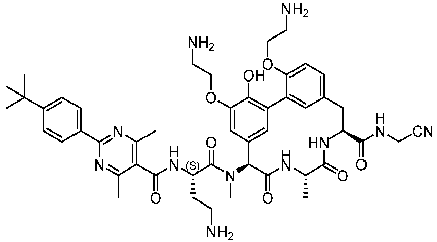 |
| **584** | rac-(8S,11S,14S)-3,17-bis(2-aminoethoxy)-N-(cyanomethyl)-18-hydroxy-11-methyl-14-[methyl-[rac-(2S)-2-[[2-(4-tert-butylphenyl)-4,6-dimethyl-pyrimidine-5-carbonyl]amino]-3- (sulfamoylamino)propanoyl]amino]- 10,13-dioxo-9,12-diazatricyclo[13.3.1.12,6]icosa-1(18),2(20),3,5,15(19),16-hexaene-8-carboxamide | 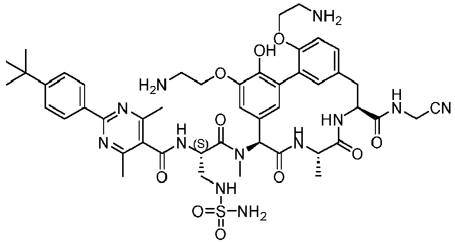 |
| **585** | rac-(8S,11S,14S)-3,17-bis(2-aminoethoxy)-N-(cyanomethyl)-18-methoxy-11-methyl-14-[methyl-[rac-(2S)-2-[[2-(4-tert-butylphenyl)-4,6-dimethyl-pyrimidine-5-carbonyl]amino]-3- (sulfamoylamino)propanoyl]amino]- 10,13-dioxo-9,12-diazatricyclo[13.3.1.12,6]icosa-1(18),2(20),3,5,15(19),16-hexaene-8-carboxamide | 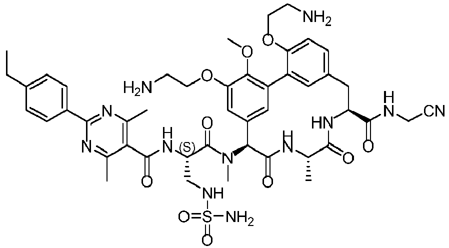 |
| **586** | rac-(8S,11S,14S)-N-(cyanomethyl)-18-methoxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-3,17-bis[rac-(2R)-3-amino-2-hydroxy-propoxy]-9,12-diazatricyclo[13.3.1.12,6]icosa-1(18),2(20),3,5,15(19),16-hexaene-8-carboxamide | 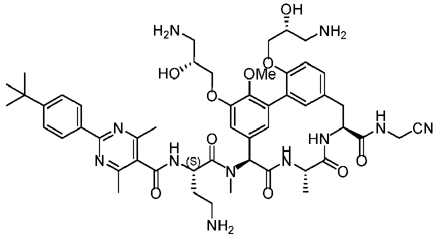 |
| **587** | rac-(8S,11S,14S)-17-(2-aminoethoxy)-N-(cyanomethyl)-18-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-3-[rac-(2R)-3-amino-2-hydroxy-propoxy] -9,12-diazatricyclo[13.3.1.12,6]icosa-1(18),2(20),3,5,15(19),16-hexaene-8-carboxamide | 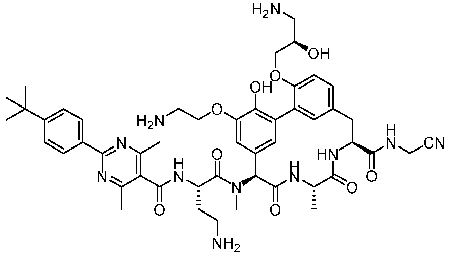 |
| **588** | rac-(8S,11S,14S)-3-(2-aminoethoxy)-N-(cyanomethyl)-18-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-17-[rac-(2R)-3-amino-2-hydroxy-propoxy] -9,12-diazatricyclo[13.3.1.12,6]icosa-1(18),2(20),3,5,15(19),16-hexaene-8-carboxamide | 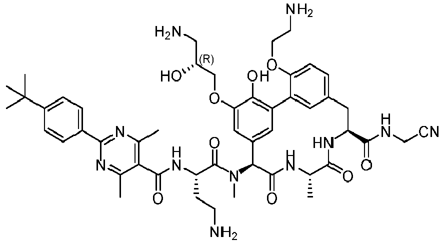 |
| **589** | rac-(8S,11S,14S)-3-(2-aminoethoxy)-17-(2-aminoethylamino)-N-(cyanomethyl)-18-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 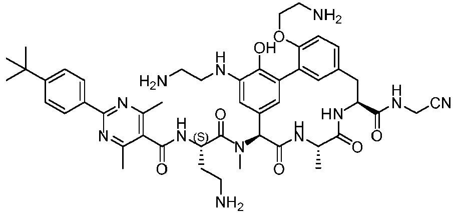 |
| **590** | rac-(8S,11S,14S)-3-(2-aminoethoxy)-N-(cyanomethyl)-17-(2-guanidinoethoxy)-18-hydroxy-11-methyl-14-[methyl-[rac-(2S)-2-[[2-(4-tert-butylphenyl)-4,6-dimethyl-pyrimidine-5-carbonyl]amino]-3-(sulfamoylamino)propanoyl]amino]- 10,13-dioxo-9,12-diazatricyclo[13.3.1.12,6]icosa-1(18),2(20),3,5,15(19),16-hexaene-8-carboxamide | 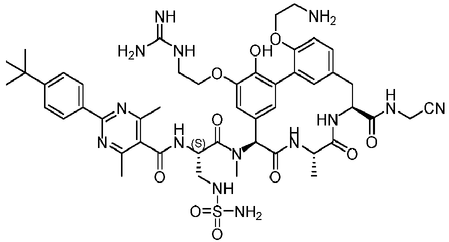 |
| **591** | rac-(8S,11S,14S)-N-(cyanomethyl)-3,18-dimethoxy-11-methyl-14-[methyl-[rac-(2S)-4-amino-2-[[2-(4-tert-butylphenyl)-4,6-dimethyl-pyrimidine-5-carbonyl]aminobutanoyl]amino]-10,13-dioxo-4,17-bis[rac-(2R)-3-amino-2-hydroxy-propoxy]-9,12-diazatricyclo[13.3.1.12,6]icosa-1(18),2(20),3,5,15(19),16-hexaene-8-carboxamide | 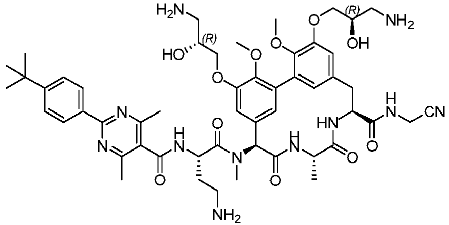 |
| **592** | rac-(8S,11S,14S)-4,17-bis(2-aminoethoxy)-N-(cyanomethyl)-3,18-dimethoxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 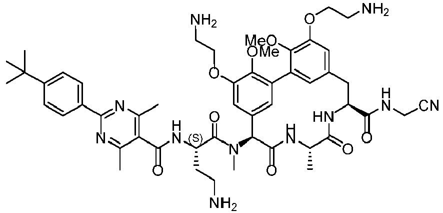 |
| **593** | rac-(8S,11S,14S)-N-(cyanomethyl)-3,18-dimethoxy-11-methyl-14-[methyl-[rac-(2S)-2-[[2-(4-tert-butylphenyl)-4,6-dimethyl-pyrimidine-5-carbonyl]amino]-3-(sulfamoylamino)propanoyl]amino]- 10,13-dioxo-4,17-bis[rac-(2R)-3-amino-2-hydroxy-propoxy]-9,12-diazatricyclo[13.3.1.12,6]icosa-1(18),2(20),3,5,15(19),16-hexaene-8-carboxamide | 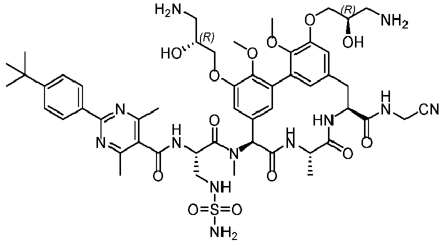 |
| **594** | rac-(8S,11S,14S)-4-(2-aminoethoxy)-N-(cyanomethyl)-3-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-18-[rac-(2R)-3-amino-2-hydroxy-propoxy]-9,12-diazatricyclo[13.3.1.12,6]icosa-1(18),2(20),3,5,15(19),16-hexaene-8-carboxamide | 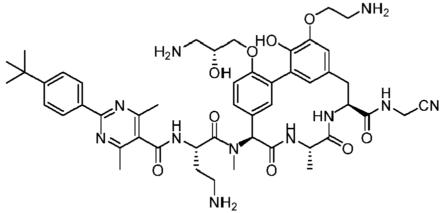 |
| **595** | rac-(8S,11S,14S)-N-(cyanomethyl)-18-(2-guanidinoethoxy)-3-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 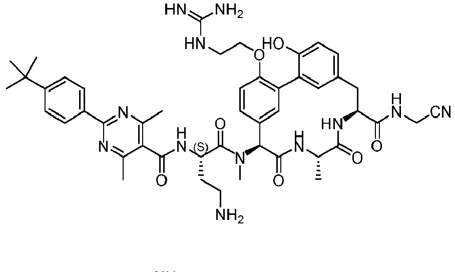 |
| **596** | rac-(8S,11S,14S)-N-(cyanomethyl)-18-(3-guanidinopropoxy)-3-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 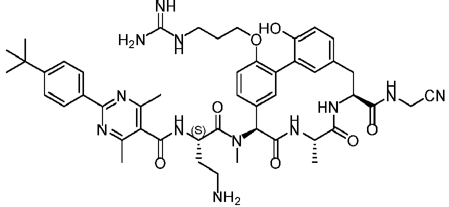 |
| **597** | rac-(8S,11S,14S)-3-(3-aminopropoxy)-N-(cyanomethyl)-18-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 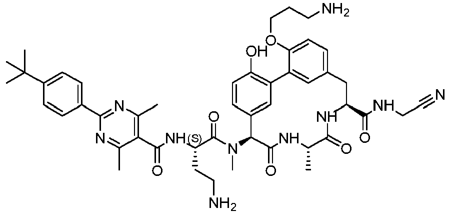 |
| **598** | rac-(8S,11S,14S)-18-(3-aminopropoxy)-N-(cyanomethyl)-3-hydroxy-11-methyl-14-[methyl-[rac-(2S)-4-amino-2-[[4,6-dimethyl-2-(4-pentoxyphenyl)pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 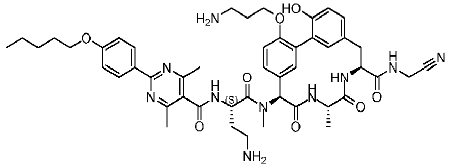 |
| **599** | rac-(8S,11S,14S)-18-[2-[carbamimidoyl(methyl)amino]ethox y]-N-(cyanomethyl)-3-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 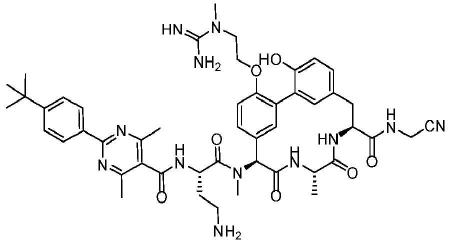 |
| **600** | rac-(8S,11S,14S)-N-(cyanomethyl)-3-hydroxy-11-methyl-18-[2-(methylamino)ethoxy]-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 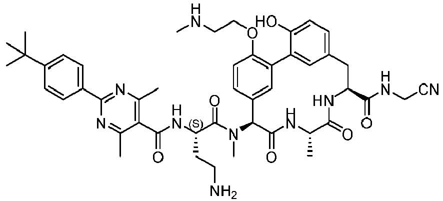 |
| **601** | rac-(8S,11S,14S)-18-[2-(2-aminoethylamino)ethoxy]-N-(cyanomethyl)-3-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 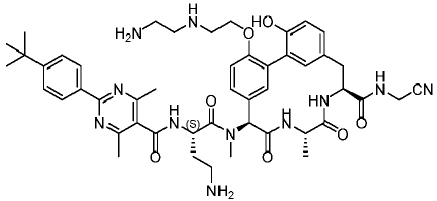 |
| **602** | rac-(8S,11S,14S)-18-[2-(3-aminopropylamino)ethoxy]-N-(cyanomethyl)-3-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 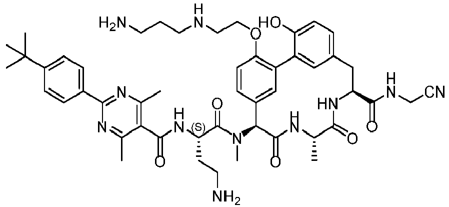 |
| **603** | rac-(8S,11S,14S)-N-(cyanomethyl)-18-[2-(ethanimidoylamino)ethoxy]- 3-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 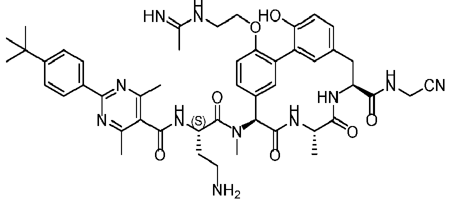 |
| **604** | rac-(8S,11S,14S)-18-(azetidin-3-yloxy)-N-(cyanomethyl)-3-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 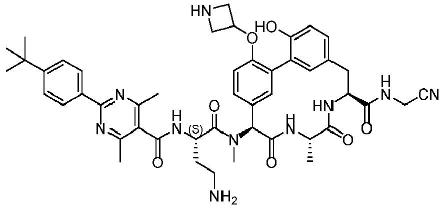 |
| **605** | rac-(8S,11S,14S)-3-(azetidin-3-yloxy)-N-(cyanomethyl)-18-hydroxy-11-methyl-14-[methyl-[rac-(2S)-4-amino-2-[[2-(4-tert-butylphenyl)-4,6-dimethyl-pyrimidine-5-carbonyl]amino]butanoyl]amino]- 10,13-dioxo-9,12-diazatricyclo[13.3.1.12,6]icosa-1(18),2(20),3,5,15(19),16-hexaene-8-carboxamide | 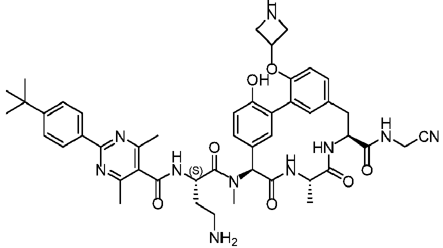 |
| **606** | rac-(8S,11S,14S)-3-(2-amino-1-methyl-ethoxy)-N-(cyanomethyl)-18-hydroxy-11-methyl-14-[methyl-[rac-(2S)-4-amino-2-[[2-(4-tert-butylphenyl)-4,6-dimethyl-pyrimidine-5-carbonyl]amino]butanoyl]amino]- 10,13-dioxo-9,12-diazatricyclo[13.3.1.12,6]icosa-1(18),2(20),3,5,15(19),16-hexaene-8-carboxamide | 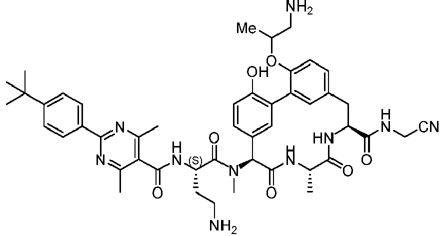 |
| **607** | rac-(8S,11S,14S)-18-(2-amino-1-methyl-ethoxy)-N-(cyanomethyl)-3-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 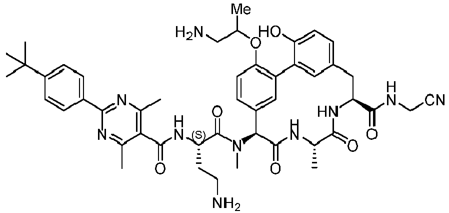 |
| **608** | rac-(8S,11S,14S)-N-(cyanomethyl)-3-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-18-(4-piperidyloxy)-9,12-diazatricyclo[13.3.1.12,6]icosa-1(18),2(20),3,5,15(19),16-hexaene-8-carboxamide | 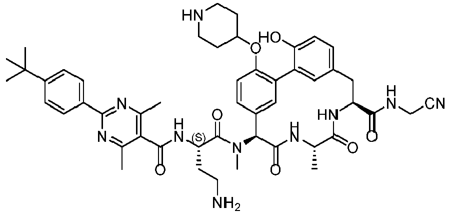 |
| **609** | rac-(8S,11S,14S)-N-(cyanomethyl)-18-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-3-(4-piperidyloxy)-9,12-diazatricyclo[13.3.1.12,6]icosa-1(18),2(20),3,5,15(19),16-hexaene-8-carboxamide | 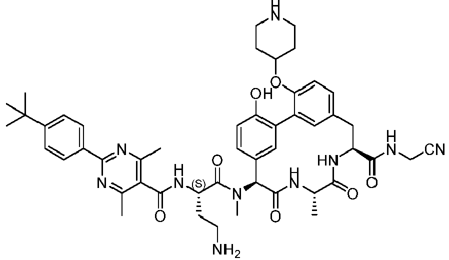 |
| **610** | rac-(8S,11S,14S)-3-[3-amino-2-(aminomethyl)propoxy]-N-(cyanomethyl)-18-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 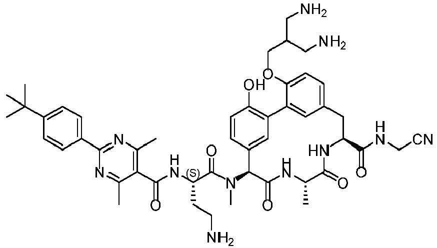 |
| **611** | rac-(8S,11S,14S)-18-[3-amino-2-(aminomethyl)propoxy]-N-(cyanomethyl)-3-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 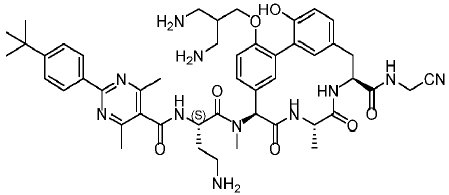 |
| **612** | rac-(8S,11S,14S)-N-(cyanomethyl)-18-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-3-[rac-(2R)-2-amino-3-hydroxy-propoxy]-9,12-diazatricyclo[13.3.1.12,6]icosa-1(18),2(20),3,5,15(19),16-hexaene-8-carboxamide | 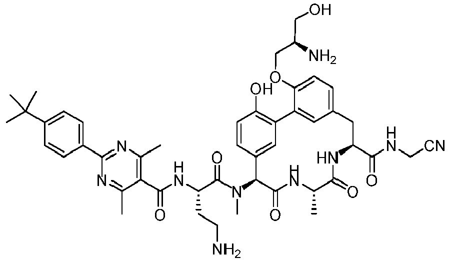 |
| **613** | rac-(8S,11S,14S)-N-(cyanomethyl)-18-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-3-[rac-(2S)-2-amino-3-hydroxy-propoxy]-9,12-diazatricyclo[13.3.1.12,6]icosa-1(18),2(20),3,5,15(19),16-hexaene-8-carboxamide | 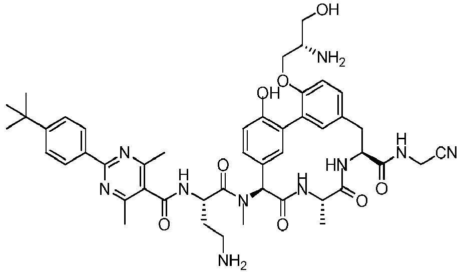 |
| **614** | rac-(8S,11S,14S)-N-(cyanomethyl)-18-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-3-[rac-(2R)-3-amino-2-hydroxy-propoxy]-9,12-diazatricyclo[13.3.1.12,6]icosa-1(18),2(20),3,5,15(19),16-hexaene-8-carboxamide | 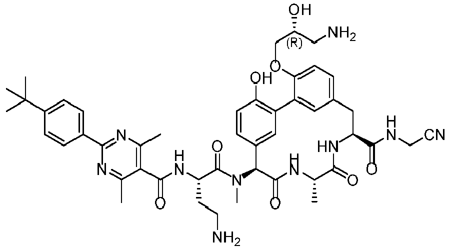 |
| **615** | rac-(8S,11S,14S)-18-(2-aminoethoxy)-N-(cyanomethyl)-3-hydroxy-11-methyl-14-[methyl-[rac-(2S)-2-[[2-[4-(cyclohexoxy)phenyl]-4,6-dimethyl-pyrimidine-5-carbonyl]amino]-4-(sulfamoylamino)butanoyl]amino]-10,13-dioxo-9,12-diazatricyclo[13.3.1.12,6]icosa-1(18),2(20),3,5,15(19),16-hexaene-8-carboxamide | 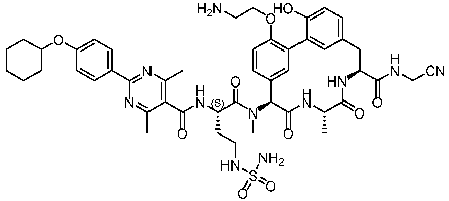 |
| **616** | rac-(8S,11S,14S)-3-(2-aminoethoxy)-N-(cyanomethyl)-18-hydroxy-11-methyl-14-[methyl-[rac-(2S)-2-[[2-[4-(cyclohexoxy)phenyl]-4,6-dimethyl-pyrimidine-5-carbonyl] amino] -3 - (sulfamoylamino)propanoyl] amino] - 10,13-dioxo-9,12-diazatricyclo[13.3.1.12,6]icosa-1(18),2(20),3,5,15(19),16-hexaene-8-carboxamide | 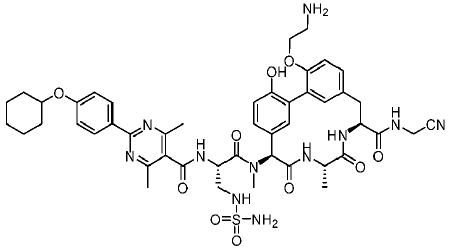 |
| **617** | rac-(8S,11S,14S)-N-(cyanomethyl)-3-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-18-[rac-(2R)-3-amino-2-hydroxy-propoxy]-9,12-diazatricyclo[13.3.1.12,6]icosa-1(18),2(20),3,5,15(19),16-hexaene-8-carboxamide | 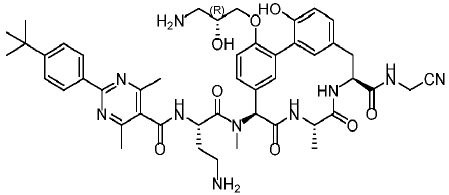 |
| **618** | rac-(8S,11S,14S)-18-(2-aminoethoxy)-N-(cyanomethyl)-3-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-4-ureido-9,12-diazatricyclo[13.3.1.12,6]icosa-1(18),2(20),3,5,15(19),16-hexaene-8-carboxamide | 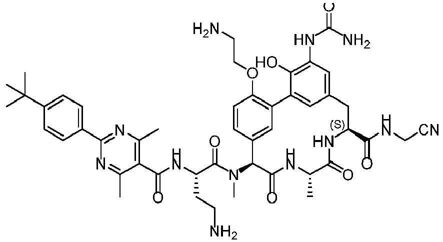 |
| **619** | rac-(8S,11S,14S)-3,18-bis(2-aminoethoxy)-N-(cyanomethyl)-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-4-ureido-9,12-diazatricyclo[13.3.1.12,6]icosa-1(18),2(20),3,5,15(19),16-hexaene-8-carboxamide | 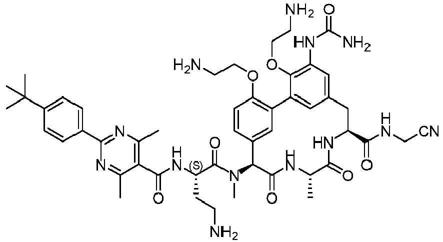 |
| **620** | rac-(8S,11S,14S)-17-acetamido-3-(2-aminoethoxy)-N-(cyanomethyl)-18-hydroxy-11-methyl-14-[methyl-[rac-(2S)-4-amino-2-[[2-(4-tert-butylphenyl)-4,6-dimethyl-pyrimidine-5-carbonyl]amino]butanoyl]amino]- 10,13-dioxo-9,12-diazatricyclo[13.3.1.12,6]icosa-1(18),2(20),3,5,15(19),16-hexaene-8-carboxamide | 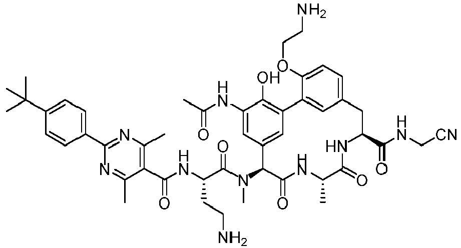 |
| **621** | rac-(8S,11S,14S)-3-(2-aminoethoxy)-N-(cyanomethyl)-18-hydroxy-11-methyl-14-[methyl-[rac-(2S)-4-amino-2-[[2-(4-tert-butylphenyl)-4,6-dimethyl-pyrimidine-5-carbonyl]amino]butanoyl]amino]- 10,13-dioxo-17-ureido-9,12-diazatricyclo[13.3.1.12,6]icosa-1(18),2(20),3,5,15(19),16-hexaene-8-carboxamide | 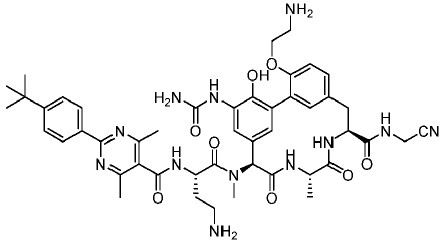 |
| **622** | rac-(8S,11S,14S)-3,18-bis(2-aminoethoxy)-N-(cyanomethyl)-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-17-ureido-9,12-diazatricyclo[13.3.1.12,6]icosa-1(18),2(20),3,5,15(19),16-hexaene-8-carboxamide | 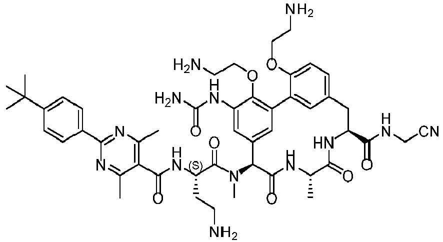 |
| **623** | | 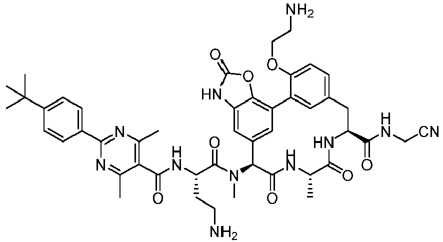 |
| **624** | rac-(8S,11S,14S)-3-amino-18-(2-aminoethoxy)-N-(cyanomethyl)-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 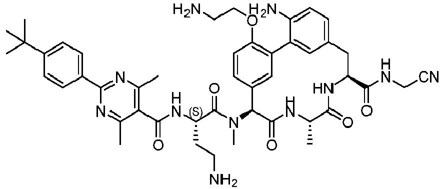 |
| **625** | rac-(8S,11S,14S)-18-(2-aminoethoxy)-N-(cyanomethyl)-5-hydroxy-3-methoxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 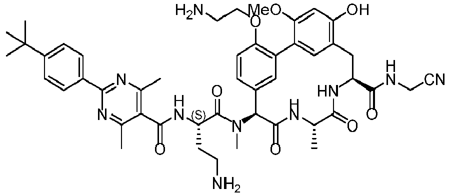 |
| **626** | rac-(8S,11S,14S)-18-(2-aminoethoxy)-5-(aminomethyl)-N-(cyanomethyl)-3-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxamide | 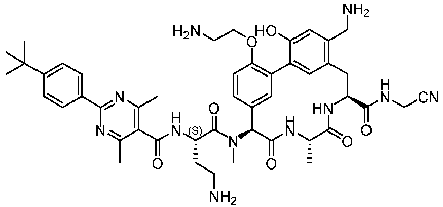 |
| **627** | rac-(8S,11S,14S)-5,18-bis(2-aminoethoxy)-N-(cyanomethyl)-3-methoxy-11-methyl-14-[methyl-[rac-(2S)-2-[[2-(4-tert-butylphenyl)-4,6-dimethyl-pyrimidine-5-carbonyl]amino]-3- (sulfamoylamino)propanoyl]amino]- 10,13-dioxo-9,12-diazatricyclo[13.3.1.12,6]icosa-1(18),2(20),3,5,15(19),16-hexaene-8-carboxamide | 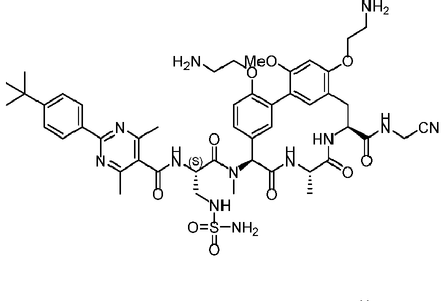 |
| **628** | rac-(8S,11S,14S)-5,18-bis(2-aminoethoxy)-N-(cyanomethyl)-3-hydroxy-11-methyl-14-[methyl-[rac-(2S)-2-[[2-(4-tert-butylphenyl)-4,6-dimethyl-pyrimidine-5-carbonyl]amino]-3- (sulfamoylamino)propanoyl]amino]- 10,13-dioxo-9,12-diazatricyclo[13.3.1.12,6]icosa-1(18),2(20),3,5,15(19),16-hexaene-8-carboxamide | 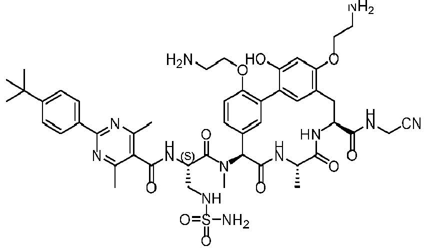 |
| **629** | rac-(8S,11S,14S)-3-(2-aminoethoxy)-17-(aminomethyl)-N-(cyanomethyl)-18-hydroxy-11-methyl-14-[methyl-[rac-(2S)-2-[[2-(4-tert-butylphenyl)-4,6-dimethyl-pyrimidine-5-carbonyl]amino]-3-(sulfamoylamino)propanoyl]amino]- 10,13-dioxo-9,12-diazatricyclo[13.3.1.12,6]icosa-1(18),2(20),3,5,15(19),16-hexaene-8-carboxamide | 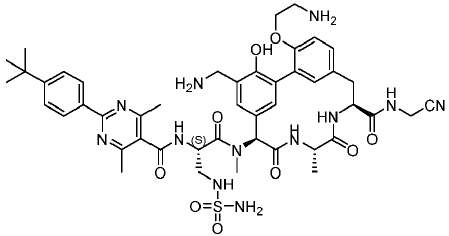 |
| **630** | (8S,11S,14S)-14-[[(2S)-4-amino-2-[[2-(4-tert-butylphenyl)-4,6-dimethyl-pyrimidine-5-carbonyl]amino]butanoyl]-methyl-amino]-17-(2-aminoethylamino)-N-(cyanomethyl)-3-hydroxy-18-methoxy-11-methyl-10,13-dioxo-9,12-diazatricyclo[13.3.1.12,6]icosa-1(18),2(20),3,5,15(19),16-hexaene-8-carboxamide | 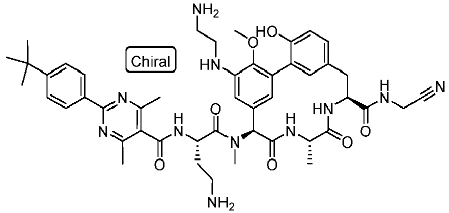 |
| **631** | (8S,11S,14S)-17-amino-14-[[(2S)-4-amino-2-[[2-(4-tert-butylphenyl)-4,6-dimethyl-pyrimidine-5-carbonyl]aminobutanoyl]-methyl-amino]-18-(2-aminoethoxy)-N-(cyanomethyl)-3-hydroxy-11-methyl-10,13-dioxo-9,12-diazatricyclo[13.3.1.12,6]icosa-1(18),2(20),3,5,15(19),16-hexaene-8-carboxamide | 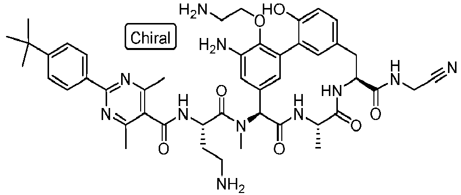 |
| **632** | (SS,11S,14S)-14-[[(2S)-4-amino-2-[[2-(4-tert-butylphenyl)-4,6-dimethyl-pyrimidine-5-carbonyl]amino]butanoyl]-methyl-amino]-17-(2-aminoethoxy)-N-(cyanomethyl)-3,18-dihydroxy-11-methyl-10,13-dioxo-9,12-diazatricyclo[13.3.1.12,6]icosa-1(18),2(20),3,5,15(19),16-hexaene-8-carboxamide | 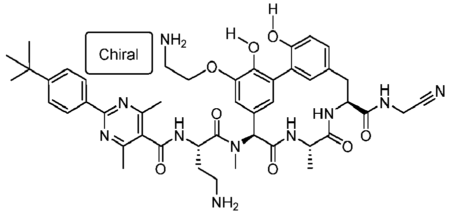 |
| **633** | (8S,11S,14S)-14-[[(2S)-4-amino-2-[[2-(4-tert-butylphenyl)-4,6-dimethyl-pyrimidine-5-carbonyl]amino]butanoyl]-methyl-amino]-17,18-bis(2-aminoethoxy)-N-(cyanomethyl)-3-hydroxy-11-methyl-10,13-dioxo-9,12-diazatricyclo[13.3.1.12,6]icosa-1(18),2(20),3,5,15(19),16-hexaene-8-carboxamide | 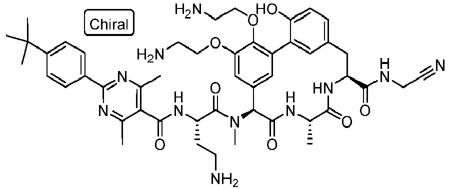 |
| **634** | rac-(8S,11S,14S)-3-(2-aminoethoxy)-18-(aminomethyl)-N-(cyanomethyl)-11-methyl-14-[methyl-[rac-(2S)-4-amino-2-[[2-(4-tert-butylphenyl)-4,6-dimethyl-pyrimidine-5-carbonyl]amino]butanoyl]amino]- 10,13-dioxo-9,12-diazatricyclo[13.3.1.12,6]icosa-1(18),2,4,6(20),15(19),16-hexaene-8-carboxamide | 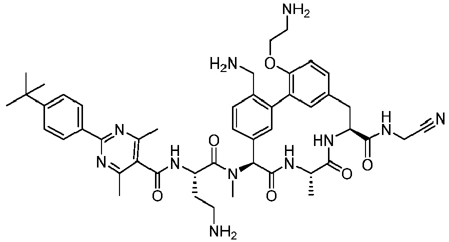 |
| **635** | rac-(8S,11S,14S)-3-(2-aminoethoxy)-17-(aminomethyl)-N-(cyanomethyl)-18-hydroxy-11-methyl-14-[methyl-[rac-(2S)-4-amino-2-[[2-(4-tert-butylphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 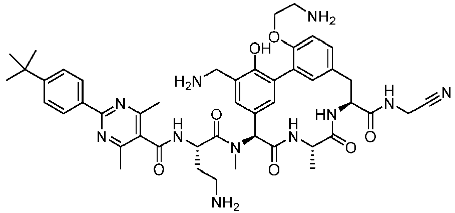 |
| **636** | rac-(8S,11S,14S)-3-(2-aminoethoxy)-17-(3-aminopropyl)-N-(cyanomethyl)-18-hydroxy-11-methyl-14-[methyl-[rac-(2S)-4-amino-2-[[2-(4-tert-butylphenyl)-4,6-dimethyl-pyrimidine-5-carbonyl]amino]butanoyl]amino- 10,13-dioxo-9,12-diazatricyclo[13.3.1.12,6]icosa-1(18),2(20),3,5,15(19),16-hexaene-8-carboxamide | 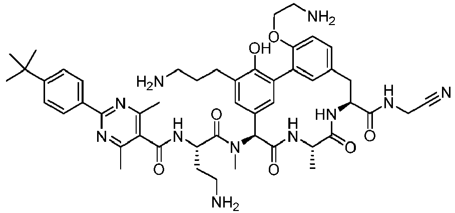 |
| **637** | rac-(8S,11S,14S)-3-(2-aminoethoxy)-17-(3-aminopropyl)-N-(cyanomethyl)-18-hydroxy-11-methyl-14-[methyl-[rac-(2S)-2-[[2-(4-tert-butylphenyl)-4,6-dimethyl-pyrimidine-5-carbonyl]amino]-3-(sulfamoylamino)propanoyl]amino]- 10,13-dioxo-9,12-diazatricyclo[13.3.1.12,6]icosa-1(18),2(20),3,5,15(19),16-hexaene-8-carboxamide | 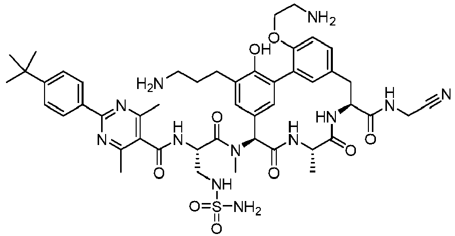 |

In one aspect described herein are compounds of Formula (V): 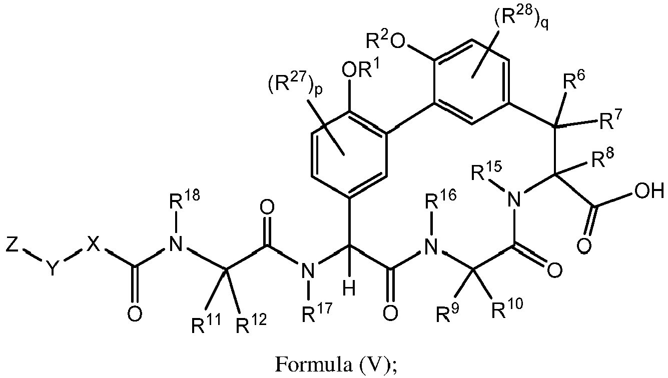 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring;
R⁶, R⁷, and R⁸ are each independently H, fluoro, hydroxyl, amino, optionally substituted alkyl,heteroalkyl, or -(C₁-C₆)alkyl;
R⁹ is H, -(C₁-C₆)alkyl, -(C₁-C₆)haloalkyl, or -(C₃-C₆)cycloalkyl;
R¹⁰ is H, -(C₁-C₆)alkyl, -(C₁-C₆)haloalkyl, or -(C₃-C₆)cycloalkyl;
or R⁹ and R¹⁰ are combined to form a heterocycloalkyl or cycloalkyl ring
R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
or R¹¹ and R¹⁸ are combined to form an optionally substituted heterocycloalkyl ring; and R¹² is H;
R¹⁵, R¹⁶, R¹⁷, and R¹⁸ are each independently H, -(C₁-C₆)alkyl, -(C₃-C₆)cycloalkyl, -(C₁-C₆)alkyl-OR²³, - (C₁-C₆)alkyl-C(O)OR²³, or -(C₁-C₆)alkyl-NR²¹R²²;
X is optionally substituted heteroaryl;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring;
each R²⁷ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R¹ and R²⁷ and the atoms to which they are attached form an optionally substituted 5- or 6-membered heterocycloalkyl ring;
each R²⁸ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R² and R²⁸ and the atoms to which they are attached form an optionally substituted 5- or 6-membered heterocycloalkyl ring;
p is 0, 1, or 2; and
q is 0, 1, or 2;
or a pharmaceutically acceptable salt, solvate, or prodrug thereof.

In one embodiment is a compound of Formula (V) wherein R⁶, R⁷, and R⁸ are H.

In another embodiment is a compound of Formula (V) wherein R¹⁵ and R¹⁶ are H.

In one embodiment is a compound of Formula (V) wherein R¹⁷ is -(C₁-C₆)alkyl. In another embodiment is a compound of Formula (V) wherein R¹⁷ is -CH₃. In another embodiment is a compound of Formula (V) wherein R¹⁷ is -CH₂CH₃. In another embodiment is a compound of Formula (V) wherein R¹⁷ is -(C₃-C₆)cycloalkyl. In another embodiment is a compound of Formula (V) wherein R¹⁷ is cyclopropyl. In another embodiment is a compound of Formula (V) wherein R¹⁷ is -(C₁-C₆)alkyl-C(O)OR²³. In another embodiment is a compound of Formula (V) wherein R¹⁷ is -CH₂CH₂OH. In another embodiment is a compound of Formula (V) wherein R¹⁷ is -(C₁-C₆)alkyl-NR²¹R²². In another embodiment is a compound of Formula (V) wherein R¹⁷ is -CH₂CH₂NH₂. In another embodiment is a compound of Formula (V) wherein R¹⁷ is H.

In another embodiment is a compound of Formula (V) wherein R¹⁸ is H.

In another embodiment is a compound of Formula (V) wherein R¹⁰ is H.

In another embodiment is a compound of Formula (V) wherein R¹⁰ is H and R⁹ is -(C₁-C₆)alkyl. In another embodiment is a compound of Formula (V) wherein R¹⁰ is H and R⁹ is -CH₃. In another embodiment is a compound of Formula (V) wherein R¹⁰ is H and R⁹ is -CH₂CH₃. In another embodiment is a compound of Formula (V) wherein R¹⁰ is H and R⁹ is -(C₁-C₆)haloalkyl. In another embodiment is a compound of Formula (V) wherein R¹⁰ is H and R⁹ is -CH₂F. In another embodiment is a compound of Formula (V) wherein R¹⁰ is H and R⁹ is -CHF₂. In another embodiment is a compound of Formula (V) wherein R¹⁰ is H and R⁹ is -(C₃-C₆)cycloalkyl. In another embodiment is a compound of Formula (V) wherein R¹⁰ is H and R⁹ is cyclopropyl. In another embodiment is a compound of Formula (V) wherein R¹⁰ is H and R⁹ is H.

In another embodiment is a compound of Formula (V) wherein R¹² is H.

In another embodiment is a compound of Formula (V) wherein R¹² is H and R¹¹ is -(C₁-C₆)alkyl. In another embodiment is a compound of Formula (V) wherein R¹² is H and R¹¹ is -CH₃. In another embodiment is a compound of Formula (V) wherein R¹² is H and R¹¹ is -(C₁-C₆)alkyl-OR²³. In another embodiment is a compound of Formula (V) wherein R¹² is H and R¹¹ is -CH₂OH. In another embodiment is a compound of Formula (V) wherein R¹² is H and R¹¹ is -CH₂CH₂OH. In another embodiment is a compound of Formula (V) wherein R¹² is H and R¹¹ is -(C₁-C₆)alkyl. In another embodiment is a compound of Formula (V) wherein R¹² is H and R¹¹ is -(C₁-C₆)alkyl-NR²¹R²². In another embodiment is a compound of Formula (V) wherein R¹² is H and R¹¹ is -(C₁-C₆)alkyl-NH₂. In another embodiment is a compound of Formula (V) wherein R¹² is H and R¹¹ is -CH₂NH₂. In another embodiment is a compound of Formula (V) wherein R¹² is H and R¹¹ is -CH₂CH₂NH₂. In another embodiment is a compound of Formula (V) wherein R¹² is H and R¹¹ is -CH₂CH₂CH₂NH₂. In another embodiment is a compound of Formula (V) wherein R¹² is H and R¹¹ is -CH₂CH₂CH₂CH₂NH₂. In another embodiment is a compound of Formula (V) wherein R¹² is H and R¹¹ is -(C₁-C₆)alkyl-CN. In another embodiment is a compound of Formula (V) wherein R¹² is H and R¹¹ is -CH₂CN. In another embodiment is a compound of Formula (V) wherein R¹² is H and R¹¹ is -(C₁-C₆)alkyl-C(O)NR²⁵R²⁶. In another embodiment is a compound of Formula (V) wherein R¹² is H and R¹¹ is -CH₂C(O)NH₂. In another embodiment is a compound of Formula (V) wherein R¹² is H and R¹¹ is -CH₂CH₂C(O)NH₂. In another embodiment is a compound of Formula (V) wherein R¹² is H and R¹¹ is -(C₁-C₆)alkyl-N(H)S(O)₂NR²⁵R²⁶. In another embodiment is a compound of Formula (V) wherein R¹² is H and R¹¹ is -CH₂N(H)S(O)₂NH₂. In another embodiment is a compound of Formula (V) wherein R¹² is H and R¹¹ is -(C₁-C₆)alkyl-heteroaryl.

In another embodiment is a compound of Formula (V) wherein R¹² is H and R¹¹ is H.

In another embodiment is a compound of Formula (V) wherein R¹¹ and R¹⁸ are combined to form an optionally substituted heterocycloalkyl ring and R¹² is H.

In another embodiment is a compound of Formula (V) wherein p is 1 and R²⁷ is halogen. In another embodiment is a compound of Formula (V) wherein p is 1 and R²⁷ is optionally substituted -(C₁-C₆)alkyl. In another embodiment is a compound of Formula (V) wherein q is 0, p is 1 and R²⁷ is halogen. In another embodiment is a compound of Formula (V) wherein q is 0, p is 1 and R²⁷ is optionally substituted -(C₁-C₆)alkyl. In another embodiment is a compound of Formula (V) wherein q is 1 and R²⁸ is halogen. In another embodiment is a compound of Formula (V) wherein q is 1 and R²⁸ is optionally substituted -(C₁-C₆)alkyl. In another embodiment is a compound of Formula (V) wherein p is 0, q is 1 and R²⁸ is halogen. In another embodiment is a compound of Formula (V) wherein p is 0, q is 1 and R²⁸ is optionally substituted -(C₁-C₆)alkyl.

In another embodiment is a compound of Formula (V) wherein p is 0, and q is 0.

In another embodiment is a compound of Formula (V) wherein R¹ and R² are each independently H or -(C₁-C₆)alkyl-NR²¹R²². In another embodiment is a compound of Formula (V) wherein R¹ and R² are each H. In another embodiment is a compound of Formula (V) wherein R¹ and R² are each independently -(C₁-C₆)alkyl-NR²¹R²². In another embodiment is a compound of Formula (V) wherein R¹ is H, and R² is -(C₁-C₆)alkyl-NR²¹R²². In another embodiment is a compound of Formula (V) wherein R¹ is - (C₁-C₆)alkyl-NR²¹R²², and R² is H. In another embodiment is a compound of Formula (V) wherein R¹ is H, and R² is -CH₂CH₂NH₂. In another embodiment is a compound of Formula (V) wherein R¹ is - CH₂CH₂NH₂, and R² is H. In another embodiment is a compound of Formula (V) wherein R¹ and R² are each -CH₂CH₂NH₂. In a further embodiment is a compound of Formula (V) wherein R¹ is -(C₁-C₆)alkyl-NR²¹R²² and R² is H. In a further embodiment is a compound of Formula (V) wherein R¹ is -CH₂CH₂NH₂ and R² is H. In a further embodiment is a compound of Formula (V) wherein R¹ is H and R² is -(C₁-C₆)alkyl-NR²¹R²². In a further embodiment is a compound of Formula (V) wherein R¹ is H and R² is - CH₂CH₂NH₂. In another embodiment is a compound of Formula (V) wherein R¹ and R² are each independently H, -(C₁-C₆)alkyl-NR²¹R²², or -CH₂CH(OH)CH₂NH₂. In another embodiment is a compound of Formula (V) wherein R¹ and R² are each independently -CH₂CH(OH)CH₂NH₂. In another embodiment is a compound of Formula (V) wherein R¹ is H, and R² is -CH₂CH(OH)CH₂NH₂. In another embodiment is a compound of Formula (V) wherein R¹ is -CH₂CH(OH)CH₂NH₂, and R² is H. In a further embodiment is a compound of Formula (V) wherein R¹ and R² and the atoms to which they are attached form an optionally substituted heterocycloalkyl ring.

In another embodiment is a compound of Formula (V) wherein X is optionally substituted heteroaryl. In a further embodiment is a compound of Formula (V) wherein X is monosubstituted or disubstituted heteroaryl. In a further embodiment is a compound of Formula (V) wherein X is heteroaryl monosubstituted or disubstituted with a substituent independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (V) wherein X is heteroaryl monosubstituted or disubstituted with a substituent independently selected from -(C₁-C₆)alkyl.In a further embodiment is a compound of Formula (V) wherein X is heteroaryl monosubstituted or disubstituted with methyl. In a further embodiment is a compound of Formula (V) wherein X is pyridinyl monosubstituted or disubstituted with a substituent independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (V) wherein X is pyridinyl monosubstituted or disubstituted with a substituent independently selected from -(C₁-C₆)alkyl. In a further embodiment is a compound of Formula (V) wherein X is pyridinyl monosubstituted or disubstituted with methyl. In a further embodiment is a compound of Formula (V) X is pyrimidinyl monosubstituted or disubstituted with a substituent independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (V) X is pyrimidinyl monosubstituted or disubstituted with a substituent independently selected from -(C₁-C₆)alkyl. In a further embodiment is a compound of Formula (V) X is pyrimidinyl monosubstituted or disubstituted with methyl.

In another embodiment is a compound of Formula (V) wherein Y is optionally substituted aryl. In a further embodiment is a compound of Formula (V) wherein Y is optionally substituted phenyl. In another embodiment is a compound of Formula (V) wherein Y is optionally substituted heteroaryl. In another embodiment is a compound of Formula (V) wherein Y is optionally substituted -(C₁-C₆)alkyl-. In another embodiment is a compound of Formula (V) wherein Y is optionally substituted (C₃-C₇)cycloalkyl-. In another embodiment is a compound of Formula (V) wherein Y is optionally substituted heterocycloalkyl. In another embodiment is a compound of Formula (V) wherein Y is -O-. In another embodiment is a compound of Formula (V) wherein Y is -(C₂-C₆)alkynyl. In another embodiment is a compound of Formula (V) wherein Y is -O-(C₁-C₆)alkyl-. In another embodiment is a compound of Formula (V) wherein Y is a bond.

In another embodiment is a compound of Formula (V) wherein Z is -(C₁-C₁₂)alkyl. In a further embodiment is a compound of Formula (V) wherein Z is n-butyl, isobutyl, or tert-butyl. In another embodiment is a compound of Formula (V) wherein Z is -O-(C₁-C₁₂)alkyl. In another embodiment is a compound of Formula (V) wherein Z is -O-(C₃-C₇)cycloalkyl. In another embodiment is a compound of Formula (V) wherein Z is -(C₂-C₁₂)alkenyl. In another embodiment is a compound of Formula (V) wherein Z is optionally substituted aryl. In a further embodiment is a compound of Formula (V) wherein Z is optionally substituted phenyl. In a further embodiment is a compound of Formula (V) wherein Z is phenyl monsubstituted or disubstituted with a substituent independently selected from -(C₁-C₈)alkyl. In a further embodiment is a compound of Formula (V) wherein Z is phenyl monosubstituted with n-butyl, isobutyl, or tert-butyl. In a further embodiment is a compound of Formula (V) wherein Z is phenyl monosubstituted with n-butyl. In a further embodiment is a compound of Formula (V) wherein Z is phenyl monosubstituted with isobutyl. In a further embodiment is a compound of Formula (V) wherein Z is phenyl monosubstituted with tert-butyl. In another embodiment is a compound of Formula (V) wherein Z is optionally substituted heteroaryl. In another embodiment is a compound of Formula (V) wherein Z is optionally substituted -(C₃-C₇)cycloalkyl. In another embodiment is a compound of Formula (V) wherein Z is optionally substituted heterocycloalkyl. In another embodiment is a compound of Formula (V) wherein Z is halogen.

In another embodiment is a compound of Formula (V) having the structure of Formula (Va): 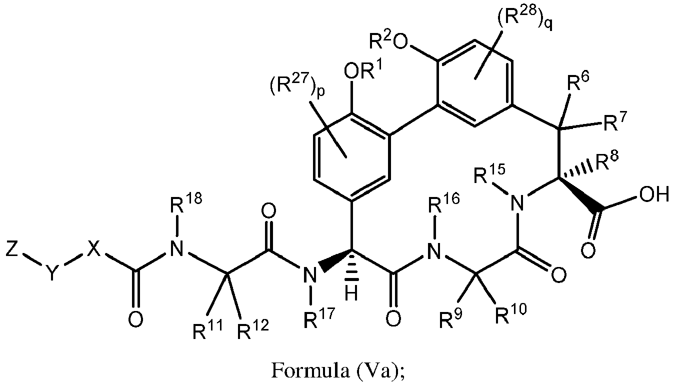 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring;
R⁶, R⁷, and R⁸ are each independently H, fluoro, hydroxyl, amino, optionally substituted alkyl, optionally substituted heteroalkyl, or -(C₁-C₆)alkyl;
R⁹ is H, -(C₁-C₆)alkyl, -(C₁-C₆)haloalkyl, or -(C₃-C₆)cycloalkyl;
R¹⁰ is H, -(C₁-C₆)alkyl, -(C₁-C₆)haloalkyl, or -(C₃-C₆)cycloalkyl;
or R⁹ and R¹⁰ are combined to form a heterocycloalkyl or cycloalkyl ring
R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alₖyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
or R¹¹ and R¹⁸ are combined to form an optionally substituted heterocycloalkyl ring; and R¹² is H;
R¹⁵, R¹⁶, R¹⁷, and R¹⁸ are each independently H, -(C₁-C₆)alkyl, -(C₃-C₆)cycloalkyl, -(C₁-C₆)alkyl-OR²³, - (C₁-C₆)alkyl-C(O)OR²³, or -(C₁-C₆)alkyl-NR²¹R²²;
X is optionally substituted heteroaryl;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-,
optionally substituted heterocycloalkyl, optionally substituted aryl, or optionally substituted heteroaryl; Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring;
each R²⁷ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R¹ and R²⁷ and the atoms to which they are attached form an optionally substituted 5- or 6-membered heterocycloalkyl ring;
each R²⁸ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R² and R²⁸ and the atoms to which they are attached form an optionally substituted 5- or 6-membered heterocycloalkyl ring;
p is 0, 1, or 2; and
q is 0, 1, or 2;
or a pharmaceutically acceptable salt, solvate, or prodrug thereof.In one embodiment is a compound of Formula (Va) wherein R⁶, R⁷, and R⁸ are H.

In another embodiment is a compound of Formula (Va) wherein R¹⁵ and R¹⁶ are H.

In one embodiment is a compound of Formula (Va) wherein R¹⁷ is -(C₁-C₆)alkyl. In another embodiment is a compound of Formula (Va) wherein R¹⁷ is -CH₃. In another embodiment is a compound of Formula (Va) wherein R¹⁷ is -CH₂CH₃. In another embodiment is a compound of Formula (Va) wherein R¹⁷ is -(C₃-C₆)cycloalkyl. In another embodiment is a compound of Formula (Va) wherein R¹⁷ is cyclopropyl. In another embodiment is a compound of Formula (Va) wherein R¹⁷ is -(C₁-C₆)alkyl-C(O)OR²³. In another embodiment is a compound of Formula (Va) wherein R¹⁷ is -CH₂CH₂OH. In another embodiment is a compound of Formula (Va) wherein R¹⁷ is -(C₁-C₆)alkyl-NR²¹R²². In another embodiment is a compound of Formula (Va) wherein R¹⁷ is -CH₂CH₂NH₂. In another embodiment is a compound of Formula (Va) wherein R¹⁷ is H.

In another embodiment is a compound of Formula (Va) wherein R¹⁸ is H.

In another embodiment is a compound of Formula (Va) wherein R¹⁰ is H.

In another embodiment is a compound of Formula (Va) wherein R¹⁰ is H and R⁹ is -(C₁-C₆)alkyl. In another embodiment is a compound of Formula (Va) wherein R¹⁰ is H and R⁹ is -CH₃. In another embodiment is a compound of Formula (Va) wherein R¹⁰ is H and R⁹ is -CH₂CH₃. In another embodiment is a compound of Formula (Va) wherein R¹⁰ is H and R⁹ is -(C₁-C₆)haloalkyl. In another embodiment is a compound of Formula (Va) wherein R¹⁰ is H and R⁹ is -CH₂F. In another embodiment is a compound of Formula (Va) wherein R¹⁰ is H and R⁹ is -CHF₂. In another embodiment is a compound of Formula (Va) wherein R¹⁰ is H and R⁹ is -(C₃-C₆)cycloalkyl. In another embodiment is a compound of Formula (Va) wherein R¹⁰ is H and R⁹ is cyclopropyl. In another embodiment is a compound of Formula (Va) wherein R¹⁰ is H and R⁹ is H.

In another embodiment is a compound of Formula (Va) wherein R¹² is H.

In another embodiment is a compound of Formula (Va) wherein R¹² is H and R¹¹ is -(C₁-C₆)alkyl. In another embodiment is a compound of Formula (Va) wherein R¹² is H and R¹¹ is -CH₃. In another embodiment is a compound of Formula (Va) wherein R¹² is H and R¹¹ is -(C₁-C₆)alkyl-OR²³. In another embodiment is a compound of Formula (Va) wherein R¹² is H and R¹¹ is -CH₂OH. In another embodiment is a compound of Formula (Va) wherein R¹² is H and R¹¹ is -CH₂CH₂OH. In another embodiment is a compound of Formula (Va) wherein R¹² is H and R¹¹ is -(C₁-C₆)alkyl. In another embodiment is a compound of Formula (Va) wherein R¹² is H and R¹¹ is -(C₁-C₆)alkyl-NR²¹R²². In another embodiment is a compound of Formula (Va) wherein R¹² is H and R¹¹ is -(C₁-C₆)alkyl-NH₂. In another embodiment is a compound of Formula (Va) wherein R¹² is H and R¹¹ is -CH₂NH₂. In another embodiment is a compound of Formula (Va) wherein R¹² is H and R¹¹ is -CH₂CH₂NH₂. In another embodiment is a compound of Formula (Va) wherein R¹² is H and R¹¹ is -CH₂CH₂CH₂NH₂. In another embodiment is a compound of Formula (Va) wherein R¹² is H and R¹¹ is -CH₂CH₂CH₂CH₂NH₂. In another embodiment is a compound of Formula (Va) wherein R¹² is H and R¹¹ is -(C₁-C₆)alkyl-CN. In another embodiment is a compound of Formula (Va) wherein R¹² is H and R¹¹ is -CH₂CN. In another embodiment is a compound of Formula (Va) wherein R¹² is H and R¹¹ is -(C₁-C₆)alkyl-C(O)NR²⁵R²⁶. In another embodiment is a compound of Formula (Va) wherein R¹² is H and R¹¹ is -CH₂C(O)NH₂. In another embodiment is a compound of Formula (Va) wherein R¹² is H and R¹¹ is -CH₂CH₂C(O)NH₂. In another embodiment is a compound of Formula (Va) wherein R¹² is H and R¹¹ is -(C₁-C₆)alkyl-heteroaryl. In another embodiment is a compound of Formula (Va) wherein R¹² is H and R¹¹ is -(C₁-C₆)alkyl-N(H)S(O)₂NR²⁵R²⁶. In another embodiment is a compound of Formula (Va) wherein R¹² is H and R¹¹ is -CH₂N(H)S(O)₂NH₂. In another embodiment is a compound of Formula (Va) wherein R¹² is H and R¹¹ is H.

In another embodiment is a compound of Formula (Va) wherein R¹¹ and R¹⁸ are combined to form an optionally substituted heterocycloalkyl ring and R¹² is H.

In another embodiment is a compound of Formula (Va) wherein p is 1 and R²⁷ is halogen. In another embodiment is a compound of Formula (Va) wherein p is 1 and R²⁷ is optionally substituted -(C₁-C₆)alkyl. In another embodiment is a compound of Formula (Va) wherein q is 0, p is 1 and R²⁷ is halogen. In another embodiment is a compound of Formula (Va) wherein q is 0, p is 1 and R²⁷ is optionally substituted -(C₁-C₆)alkyl. In another embodiment is a compound of Formula (Va) wherein q is 1 and R²⁸ is halogen. In another embodiment is a compound of Formula (Va) wherein q is 1 and R²⁸ is optionally substituted -(C₁-C₆)alkyl. In another embodiment is a compound of Formula (Va) wherein p is 0, q is 1 and R²¹ is halogen. In another embodiment is a compound of Formula (Va) wherein p is 0, q is 1 and R²⁸ is optionally substituted -(C₁-C₆)alkyl.

In another embodiment is a compound of Formula (Va) wherein p is 0, and q is 0.

In another embodiment is a compound of Formula (Va) wherein R¹ and R² are each independently H or -(C₁-C₆)alkyl-NR²¹R²². In another embodiment is a compound of Formula (Va) wherein R¹ and R² are each H. In another embodiment is a compound of Formula (Va) wherein R¹ and R² are each independently -(C₁-C₆)alkyl-NR²¹R²². In another embodiment is a compound of Formula (Va) wherein R¹ is H, and R² is -(C₁-C₆)alkyl-NR²¹R²². In another embodiment is a compound of Formula (Va) wherein R¹ is -(C₁-C₆)alkyl-NR²¹R²², and R² is H. In another embodiment is a compound of Formula (Va) wherein R¹ is H, and R² is -CH₂CH₂NH₂. In another embodiment is a compound of Formula (Va) wherein R¹ is -CH₂CH₂NH₂, and R² is H. In another embodiment is a compound of Formula (Va) wherein R¹ and R² are each -CH₂CH₂NH₂. In a further embodiment is a compound of Formula (Va) wherein R¹ is -(C₁-C₆)alkyl-NR²¹R²² and R² is H. In a further embodiment is a compound of Formula (Va) wherein R¹ is - CH₂CH₂NH₂ and R² is H. In a further embodiment is a compound of Formula (Va) wherein R¹ is H and R² is -(C₁-C₆)alkyl-NR²¹R²². In a further embodiment is a compound of Formula (Va) wherein R¹ is H and R² is -CH₂CH₂NH₂. In another embodiment is a compound of Formula (Va) wherein R¹ and R² are each independently H, -(C₁-C₆)alkyl-NR²¹R²², or -CH₂CH(OH)CH₂NH₂. In another embodiment is a compound of Formula (Va) wherein R¹ and R² are each independently -CH₂CH(OH)CH₂NH₂. In another embodiment is a compound of Formula (Va) wherein R¹ is H, and R² is -CH₂CH(OH)CH₂NH₂. In another embodiment is a compound of Formula (Va) wherein R¹ is -CH₂CH(OH)CH₂NH₂, and R² is H. In a further embodiment is a compound of Formula (Va) wherein R¹ and R² and the atoms to which they are attached form an optionally substituted heterocycloalkyl ring.

In another embodiment is a compound of Formula (Va) wherein X is optionally substituted heteroaryl. In a further embodiment is a compound of Formula (Va) wherein X is monosubstituted or disubstituted heteroaryl. In a further embodiment is a compound of Formula (Va) wherein X is heteroaryl monosubstituted or disubstituted with a substituent independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (Va) wherein X is heteroaryl monosubstituted or disubstituted with a substituent independently selected from -(C₁-C₆)alkyl.In a further embodiment is a compound of Formula (Va) wherein X is heteroaryl monosubstituted or disubstituted with methyl. In a further embodiment is a compound of Formula (Va) wherein X is pyridinyl monosubstituted or disubstituted with a substituent independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (Va) wherein X is pyridinyl monosubstituted or disubstituted with a substituent independently selected from -(C₁-C₆)alkyl. In a further embodiment is a compound of Formula (Va) wherein X is pyridinyl monosubstituted or disubstituted with methyl. In a further embodiment is a compound of Formula (Va) X is pyrimidinyl monosubstituted or disubstituted with a substituent independently selected from halogen, - CN, optionally substituted -(C₁-C₆)alkyl, optionally substituted -O-(C₁-C₆)alkyl, OR²³, -NR²⁵R²⁶, or -NO₂. In a further embodiment is a compound of Formula (Va) X is pyrimidinyl monosubstituted or disubstituted with a substituent independently selected from -(C₁-C₆)alkyl. In a further embodiment is a compound of Formula (Va) X is pyrimidinyl monosubstituted or disubstituted with methyl.

In another embodiment is a compound of Formula (Va) wherein Y is optionally substituted aryl. In a further embodiment is a compound of Formula (Va) wherein Y is optionally substituted phenyl. In another embodiment is a compound of Formula (Va) wherein Y is optionally substituted heteroaryl. In another embodiment is a compound of Formula (Va) wherein Y is optionally substituted -(C₁-C₆)alkyl-. In another embodiment is a compound of Formula (Va) wherein Y is optionally substituted (C₃-C₇)cycloalkyl-. In another embodiment is a compound of Formula (Va) wherein Y is optionally substituted heterocycloalkyl. In another embodiment is a compound of Formula (Va) wherein Y is -O-. In another embodiment is a compound of Formula (Va) wherein Y is -(C₂-C₆)alkynyl. In another embodiment is a compound of Formula (Va) wherein Y is -O-(C₁-C₆)alkyl-. In another embodiment is a compound of Formula (Va) wherein Y is a bond.

In another embodiment is a compound of Formula (Va) wherein Z is -(C₁-C₁₂)alkyl. In a further embodiment is a compound of Formula (Va) wherein Z is n-butyl, isobutyl, or tert-butyl. In another embodiment is a compound of Formula (Va) wherein Z is -O-(C₁-C₁₂)alkyl. In another embodiment is a compound of Formula (Va) wherein Z is -O-(C₃-C₇)cycloalkyl. In another embodiment is a compound of Formula (Va) wherein Z is -(C₂-C₁₂)alkenyl. In another embodiment is a compound of Formula (Va) wherein Z is optionally substituted aryl. In a further embodiment is a compound of Formula (Va) wherein Z is optionally substituted phenyl. In a further embodiment is a compound of Formula (Va) wherein Z is phenyl monsubstituted or disubstituted with a substituent independently selected from -(C₁-C₈)alkyl. In a further embodiment is a compound of Formula (Va) wherein Z is phenyl monosubstituted with n-butyl, isobutyl, or tert-butyl. In a further embodiment is a compound of Formula (Va) wherein Z is phenyl monosubstituted with n-butyl. In a further embodiment is a compound of Formula (Va) wherein Z is phenyl monosubstituted with isobutyl. In a further embodiment is a compound of Formula (Va) wherein Z is phenyl monosubstituted with tert-butyl. In another embodiment is a compound of Formula (Va) wherein Z is optionally substituted heteroaryl. In another embodiment is a compound of Formula (Va) wherein Z is optionally substituted -(C₃-C₇)cycloalkyl. In another embodiment is a compound of Formula (Va) wherein Z is optionally substituted heterocycloalkyl. In another embodiment is a compound of Formula (Va) wherein Z is halogen.

In another embodiment is a compound of Formula (V) having the structure of Formula (Vb): 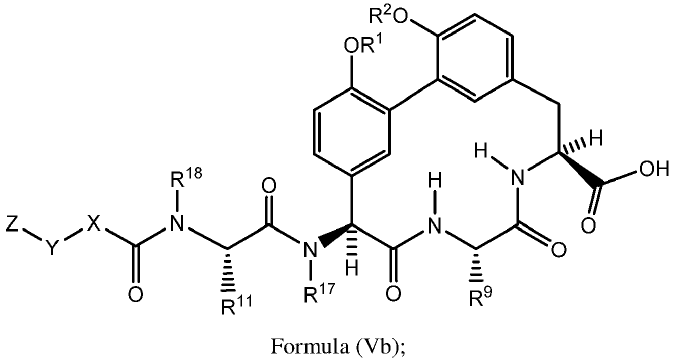 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring;
R⁹ is H, -(C₁-C₆)alkyl, -(C₁-C₆)haloalkyl, or -(C₃-C₆)cycloalkyl;
R¹¹ is H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
or R¹¹ and R¹⁸ are combined to form an optionally substituted heterocycloalkyl ring; and R¹² is H;
R¹⁷ and R¹⁸ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted heteroaryl;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-,
optionally substituted heterocycloalkyl, optionally substituted aryl, or optionally substituted heteroaryl; Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring; and
or a pharmaceutically acceptable salt, solvate, or prodrug thereof.In one embodiment is a compound of Formula (Vb) wherein R¹⁷ is -(C₁-C₆)alkyl. In another embodiment is a compound of Formula (Vb) wherein R¹⁷ is -CH₃. In another embodiment is a compound of Formula (Vb) wherein R¹⁷ is -CH₂CH₃. In another embodiment is a compound of Formula (Vb) wherein R¹⁷ is -(C₃-C₆)cycloalkyl. In another embodiment is a compound of Formula (Vb) wherein R¹⁷ is cyclopropyl. In another embodiment is a compound of Formula (Vb) wherein R¹⁷ is -(C₁-C₆)alkyl-C(O)OR²³. In another embodiment is a compound of Formula (Vb) wherein R¹⁷ is -CH₂CH₂OH. In another embodiment is a compound of Formula (Vb) wherein R¹⁷ is -(C₁-C₆)alkyl-NR²¹R²². In another embodiment is a compound of Formula (Vb) wherein R¹⁷ is -CH₂CH₂NH₂. In another embodiment is a compound of Formula (Vb) wherein R¹⁷ is H.

In another embodiment is a compound of Formula (Vb) wherein R¹⁸ is H.

In another embodiment is a compound of Formula (Vb) wherein R⁹ is -(C₁-C₆)alkyl. In another embodiment is a compound of Formula (Vb) wherein R⁹ is -CH₃. In another embodiment is a compound of Formula (Vb) wherein R⁹ is -CH₂CH₃. In another embodiment is a compound of Formula (Vb) wherein R⁹ is -(C₁-C₆)haloalkyl. In another embodiment is a compound of Formula (Vb) wherein R⁹ is -CH₂F. In another embodiment is a compound of Formula (Vb) wherein R⁹ is -CHF₂. In another embodiment is a compound of Formula (Vb) wherein R⁹ is -(C₃-C₆)cycloalkyl. In another embodiment is a compound of Formula (Vb) wherein R⁹ is cyclopropyl. In another embodiment is a compound of Formula (Vb) wherein R⁹ is H.

In another embodiment is a compound of Formula (Vb) wherein R¹¹ is -(C₁-C₆)alkyl. In another embodiment is a compound of Formula (Vb) wherein R¹¹ is -CH₃. In another embodiment is a compound of Formula (Vb) wherein R¹¹ is -(C₁-C₆)alkyl-OR²³. In another embodiment is a compound of Formula (Vb) wherein R¹¹ is -CH₂OH. In another embodiment is a compound of Formula (Vb) wherein R¹¹ is - CH₂CH₂OH. In another embodiment is a compound of Formula (Vb) wherein R¹¹ is -(C₁-C₆)alkyl. In another embodiment is a compound of Formula (Vb) wherein R¹¹ is -(C₁-C₆)alkyl-NR²¹R²². In another embodiment is a compound of Formula (Vb) wherein R¹¹ is -(C₁-C₆)alkyl-NH₂. In another embodiment is a compound of Formula (Vb) wherein R¹¹ is -CH₂NH₂. In another embodiment is a compound of Formula (Vb) wherein R¹¹ is -CH₂CH₂NH₂. In another embodiment is a compound of Formula (Vb) wherein R¹¹ is - CH₂CH₂CH₂NH₂. In another embodiment is a compound of Formula (Vb) wherein R¹¹ is - CH₂CH₂CH₂CH₂NH₂. In another embodiment is a compound of Formula (Vb) wherein R¹¹ is -(C₁-C₆)alkyl-CN. In another embodiment is a compound of Formula (Vb) wherein R¹¹ is -CH₂CN. In another embodiment is a compound of Formula (Vb) wherein R¹¹ is -(C₁-C₆)alkyl-C(O)NR²⁵R²⁶. In another embodiment is a compound of Formula (Vb) wherein R¹¹ is -CH₂C(O)NH₂. In another embodiment is a compound of Formula (Vb) wherein R¹¹ is -CH₂CH₂C(O)NH₂. In another embodiment is a compound of Formula (Vb) wherein R¹¹ is -(C₁-C₆)alkyl-heteroaryl. In another embodiment is a compound of Formula (Vb) wherein R¹¹ is -(C₁-C₆)alkyl-N(H)S(O)₂NR²⁵R²⁶. In another embodiment is a compound of Formula (Vb) wherein R¹¹ is -CH₂N(H)S(O)₂NH₂. In another embodiment is a compound of Formula (Vb) wherein R¹¹ is H.

In another embodiment is a compound of Formula (Vb) wherein R¹¹ and R¹⁸ are combined to form an optionally substituted heterocycloalkyl ring.

In another embodiment is a compound of Formula (Vb) wherein R¹ and R² are each independently H or -(C₁-C₆)alkyl-NR²¹R²². In another embodiment is a compound of Formula (Vb) wherein R¹ and R² are each H. In another embodiment is a compound of Formula (Vb) wherein R¹ and R² are each independently -(C₁-C₆)alkyl-NR²¹R²². In another embodiment is a compound of Formula (Vb) wherein R¹ is H, and R² is -(C₁-C₆)alkyl-NR²¹R²². In another embodiment is a compound of Formula (Vb) wherein R¹ is -(C₁-C₆)alkyl-NR²¹R²², and R² is H. In another embodiment is a compound of Formula (Vb) wherein R¹ is H, and R² is -CH₂CH₂NH₂. In another embodiment is a compound of Formula (Vb) wherein R¹ is -CH₂CH₂NH₂, and R² is H. In another embodiment is a compound of Formula (Vb) wherein R¹ and R² are each -CH₂CH₂NH₂. In a further embodiment is a compound of Formula (Vb) wherein R¹ is -(C₁-C₆)alkyl-NR²¹R²² and R² is H. In a further embodiment is a compound of Formula (Vb) wherein R¹ is - CH₂CH₂NH₂ and R² is H. In a further embodiment is a compound of Formula (Vb) wherein R¹ is H and R² is -(C₁-C₆)alkyl-NR²¹R²². In a further embodiment is a compound of Formula (Vb) wherein R¹ is H and R² is -CH₂CH₂NH₂. In another embodiment is a compound of Formula (Vb) wherein R¹ and R² are each independently H, -(C₁-C₆)alkyl-NR²¹R²², or -CH₂CH(OH)CH₂NH₂. In another embodiment is a compound of Formula (Vb) wherein R¹ and R² are each independently -CH₂CH(OH)CH₂NH₂. In another embodiment is a compound of Formula (Vb) wherein R¹ is H, and R² is -CH₂CH(OH)CH₂NH₂. In another embodiment is a compound of Formula (Vb) wherein R¹ is -CH₂CH(OH)CH₂NH₂, and R² is H. In a further embodiment is a compound of Formula (Vb) wherein R¹ and R² and the atoms to which they are attached form an optionally substituted heterocycloalkyl ring.

In another embodiment is a compound of Formula (Vb) wherein X is optionally substituted heteroaryl. In a further embodiment is a compound of Formula (Vb) wherein X is monosubstituted or disubstituted heteroaryl. In a further embodiment is a compound of Formula (Vb) wherein X is heteroaryl monosubstituted or disubstituted with a substituent independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (Vb) wherein X is heteroaryl monosubstituted or disubstituted with a substituent independently selected from -(C₁-C₆)alkyl.In a further embodiment is a compound of Formula (Vb) wherein X is heteroaryl monosubstituted or disubstituted with methyl. In a further embodiment is a compound of Formula (Vb) wherein X is pyridinyl monosubstituted or disubstituted with a substituent independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (Vb) wherein X is pyridinyl monosubstituted or disubstituted with a substituent independently selected from -(C₁-C₆)alkyl. In a further embodiment is a compound of Formula (Vb) wherein X is pyridinyl monosubstituted or disubstituted with methyl. In a further embodiment is a compound of Formula (Vb) X is pyrimidinyl monosubstituted or disubstituted with a substituent independently selected from halogen, - CN, optionally substituted -(C₁-C₆)alkyl, optionally substituted -O-(C₁-C₆)alkyl, OR²³, -NR²⁵R²⁶, or -NO₂. In a further embodiment is a compound of Formula (Vb) X is pyrimidinyl monosubstituted or disubstituted with a substituent independently selected from -(C₁-C₆)alkyl. In a further embodiment is a compound of Formula (Vb) X is pyrimidinyl monosubstituted or disubstituted with methyl.

In another embodiment is a compound of Formula (Vb) wherein Y is optionally substituted aryl. In a further embodiment is a compound of Formula (Vb) wherein Y is optionally substituted phenyl. In another embodiment is a compound of Formula (Vb) wherein Y is optionally substituted heteroaryl. In another embodiment is a compound of Formula (Vb) wherein Y is optionally substituted -(C₁-C₆)alkyl-. In another embodiment is a compound of Formula (Vb) wherein Y is optionally substituted (C₃-C₇)cycloalkyl-. In another embodiment is a compound of Formula (Vb) wherein Y is optionally substituted heterocycloalkyl. In another embodiment is a compound of Formula (Vb) wherein Y is -O-. In another embodiment is a compound of Formula (Vb) wherein Y is -(C₂-C₆)alkynyl. In another embodiment is a compound of Formula (Vb) wherein Y is -O-(C₁-C₆)alkyl-. In another embodiment is a compound of Formula (Vb) wherein Y is a bond.

In another embodiment is a compound of Formula (Vb) wherein Z is -(C₁-C₁₂)alkyl. In a further embodiment is a compound of Formula (Vb) wherein Z is n-butyl, isobutyl, or tert-butyl. In another embodiment is a compound of Formula (Vb) wherein Z is -O-(C₁-C₁₂)alkyl. In another embodiment is a compound of Formula (Vb) wherein Z is -O-(C₃-C₇)cycloalkyl. In another embodiment is a compound of Formula (Vb) wherein Z is -(C₂-C₁₂)alkenyl. In another embodiment is a compound of Formula (Vb) wherein Z is optionally substituted aryl. In a further embodiment is a compound of Formula (Vb) wherein Z is optionally substituted phenyl. In a further embodiment is a compound of Formula (Vb) wherein Z is phenyl monsubstituted or disubstituted with a substituent independently selected from -(C₁-C₈)alkyl. In a further embodiment is a compound of Formula (Vb) wherein Z is phenyl monosubstituted with n-butyl, isobutyl, or tert-butyl. In a further embodiment is a compound of Formula (Vb) wherein Z is phenyl monosubstituted with n-butyl. In a further embodiment is a compound of Formula (Vb) wherein Z is phenyl monosubstituted with isobutyl. In a further embodiment is a compound of Formula (Vb) wherein Z is phenyl monosubstituted with tert-butyl. In another embodiment is a compound of Formula (Vb) wherein Z is optionally substituted heteroaryl. In another embodiment is a compound of Formula (Vb) wherein Z is optionally substituted -(C₃-C₇)cycloalkyl. In another embodiment is a compound of Formula (Vb) wherein Z is optionally substituted heterocycloalkyl. In another embodiment is a compound of Formula (Vb) wherein Z is halogen.

In another embodiment is a compound of Formula (V) having the structure of Formula (Vc): 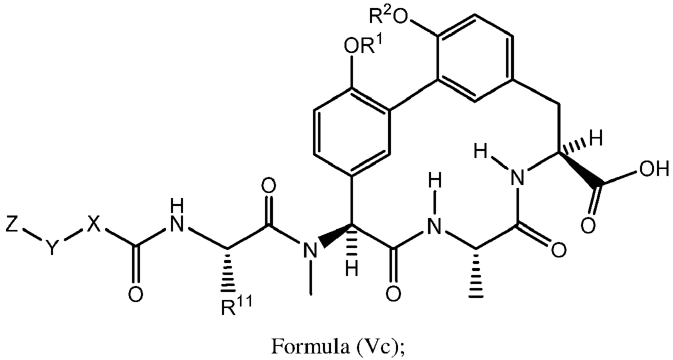 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring;
R¹¹ is H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²¹R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
X is optionally substituted heteroaryl;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring; and
or a pharmaceutically acceptable salt, solvate, or prodrug thereof.In another embodiment is a compound of Formula (Vc) wherein R¹¹ is -(C₁-C₆)alkyl. In another embodiment is a compound of Formula (Vc) wherein R¹¹ is -CH₃. In another embodiment is a compound of Formula (Vc) wherein R¹¹ is -(C₁-C₆)alkyl-OR²³. In another embodiment is a compound of Formula (Vc) wherein R¹¹ is -CH₂OH. In another embodiment is a compound of Formula (Vc) wherein R¹¹ is -CH₂CH₂OH. In another embodiment is a compound of Formula (Vc) wherein R¹¹ is -(C₁-C₆)alkyl. In another embodiment is a compound of Formula (Vc) wherein R¹¹ is -(C₁-C₆)alkyl-NR²¹R²². In another embodiment is a compound of Formula (Vc) wherein R¹¹ is -(C₁-C₆)alkyl-NH₂. In another embodiment is a compound of Formula (Vc) wherein R¹¹ is -CH₂NH₂. In another embodiment is a compound of Formula (Vc) wherein R¹¹ is -CH₂CH₂NH₂. In another embodiment is a compound of Formula (Vc) wherein R¹¹ is -CH₂CH₂CH₂NH₂. In another embodiment is a compound of Formula (Vc) wherein R¹¹ is -CH₂CH₂CH₂CH₂NH₂. In another embodiment is a compound of Formula (Vc) wherein R¹¹ is -(C₁-C₆)alkyl-CN. In another embodiment is a compound of Formula (Vc) wherein R¹¹ is -CH₂CN. In another embodiment is a compound of Formula (Vc) wherein R¹¹ is -(C₁-C₆)alkyl-C(O)NR²⁵R²⁶. In another embodiment is a compound of Formula (Vc) wherein R¹¹ is - CH₂C(O)NH₂. In another embodiment is a compound of Formula (Vc) wherein R¹¹ is -CH₂CH₂C(O)NH₂. In another embodiment is a compound of Formula (Vc) wherein R¹¹ is -(C₁-C₆)alkyl-heteroaryl. In another embodiment is a compound of Formula (Vc) wherein R¹¹ is -(C₁-C₆)alkyl-N(H)S(O)₂NR²⁵R²⁶. In another embodiment is a compound of Formula (Vc) wherein R¹¹ is -CH₂N(H)S(O)₂NH₂. In another embodiment is a compound of Formula (Vc) wherein R¹¹ is H.

In another embodiment is a compound of Formula (Vc) wherein R¹ and R² are each independently H or -(C₁-C₆)alkyl-NR²¹R²². In another embodiment is a compound of Formula (Vc) wherein R¹ and R² are each H. In another embodiment is a compound of Formula (Vc) wherein R¹ and R² are each independently -(C₁-C₆)alkyl-NR²¹R²². In another embodiment is a compound of Formula (Vc) wherein R¹ is H, and R² is -(C₁-C₆)alkyl-NR²¹R²². In another embodiment is a compound of Formula (Vc) wherein R¹ is -(C₁-C₆)alkyl-NR²¹R²², and R² is H. In another embodiment is a compound of Formula (Vc) wherein R¹ is H, and R² is -CH₂CH₂NH₂. In another embodiment is a compound of Formula (Vc) wherein R¹ is -CH₂CH₂NH₂, and R² is H. In another embodiment is a compound of Formula (Vc) wherein R¹ and R² are each -CH₂CH₂NH₂. In a further embodiment is a compound of Formula (Vc) wherein R¹ is -(C₁-C₆)alkyl-NR²¹R²² and R² is H. In a further embodiment is a compound of Formula (Vc) wherein R¹ is - CH₂CH₂NH₂ and R² is H. In a further embodiment is a compound of Formula (Vc) wherein R¹ is H and R² is -(C₁-C₆)alkyl-NR²¹R²². In a further embodiment is a compound of Formula (Vc) wherein R¹ is H and R² is -CH₂CH₂NH₂. In another embodiment is a compound of Formula (Vc) wherein R¹ and R² are each independently H, -(C₁-C₆)alkyl-NR²¹R²², or -CH₂CH(OH)CH₂NH₂. In another embodiment is a compound of Formula (Vc) wherein R¹ and R² are each independently -CH₂CH(OH)CH₂NH₂. In another embodiment is a compound of Formula (Vc) wherein R¹ is H, and R² is -CH₂CH(OH)CH₂NH₂. In another embodiment is a compound of Formula (Vc) wherein R¹ is -CH₂CH(OH)CH₂NH₂, and R² is H. In a further embodiment is a compound of Formula (Vc) wherein R¹ and R² and the atoms to which they are attached form an optionally substituted heterocycloalkyl ring.

In another embodiment is a compound of Formula (Vc) wherein X is optionally substituted heteroaryl. In a further embodiment is a compound of Formula (Vc) wherein X is monosubstituted or disubstituted heteroaryl. In a further embodiment is a compound of Formula (Vc) wherein X is heteroaryl monosubstituted or disubstituted with a substituent independently selected from halogen, -CN, optionally substituted -(C₁-C₆)alkyl, optionally substituted -O-(C₁-C₆)alkyl, OR²³, -NR²⁵R²⁶, and -NO₂. In a further embodiment is a compound of Formula (Vc) wherein X is heteroaryl monosubstituted or disubstituted with a substituent independently selected from -(C₁-C₆)alkyl.In a further embodiment is a compound of Formula (Vc) wherein X is heteroaryl monosubstituted or disubstituted with methyl. In a further embodiment is a compound of Formula (Vc) wherein X is pyridinyl monosubstituted or disubstituted with a substituent independently selected from halogen, -CN, optionally substituted -(C₁-C₆)alkyl, optionally substituted -O-(C₁-C₆)alkyl, OR²³, -NR²⁵R²⁶, or -NO₂. In a further embodiment is a compound of Formula (Vc) wherein X is pyridinyl monosubstituted or disubstituted with a substituent independently selected from -(C₁-C₆)alkyl. In a further embodiment is a compound of Formula (Vc) wherein X is pyridinyl monosubstituted or disubstituted with methyl. In a further embodiment is a compound of Formula (Vc) X is pyrimidinyl monosubstituted or disubstituted with a substituent independently selected from halogen, - CN, optionally substituted -(C₁-C₆)alkyl, optionally substituted -O-(C₁-C₆)alkyl, OR²³, -NR²⁵R²⁶, or -NO₂. In a further embodiment is a compound of Formula (Vc) X is pyrimidinyl monosubstituted or disubstituted with a substituent independently selected from -(C₁-C₆)alkyl. In a further embodiment is a compound of Formula (Vc) X is pyrimidinyl monosubstituted or disubstituted with methyl.

In another embodiment is a compound of Formula (Vc) wherein Y is optionally substituted aryl. In a further embodiment is a compound of Formula (Vc) wherein Y is optionally substituted phenyl. In another embodiment is a compound of Formula (Vc) wherein Y is optionally substituted heteroaryl. In another embodiment is a compound of Formula (Vc) wherein Y is optionally substituted -(C₁-C₆)alkyl-. In another embodiment is a compound of Formula (Vc) wherein Y is optionally substituted (C₃-C₇)cycloalkyl-. In another embodiment is a compound of Formula (Vc) wherein Y is optionally substituted heterocycloalkyl. In another embodiment is a compound of Formula (Vc) wherein Y is -O-. In another embodiment is a compound of Formula (Vc) wherein Y is -(C₂-C₆)alkynyl. In another embodiment is a compound of Formula (Vc) wherein Y is -O-(C₁-C₆)alkyl-. In another embodiment is a compound of Formula (Vc) wherein Y is a bond.

In another embodiment is a compound of Formula (Vc) wherein Z is -(C₁-C₁₂)alkyl. In a further embodiment is a compound of Formula (Vc) wherein Z is n-butyl, isobutyl, or tert-butyl. In another embodiment is a compound of Formula (Vc) wherein Z is -O-(C₁-C₁₂)alkyl. In another embodiment is a compound of Formula (Vc) wherein Z is -O-(C₃-C₇)cycloalkyl. In another embodiment is a compound of Formula (Vc) wherein Z is -(C₂-C₁₂)alkenyl. In another embodiment is a compound of Formula (Vc) wherein Z is optionally substituted aryl. In a further embodiment is a compound of Formula (Vc) wherein Z is optionally substituted phenyl. In a further embodiment is a compound of Formula (Vc) wherein Z is phenyl monsubstituted or disubstituted with a substituent independently selected from -(C₁-C₈)alkyl. In a further embodiment is a compound of Formula (Vc) wherein Z is phenyl monosubstituted with n-butyl, isobutyl, or tert-butyl. In a further embodiment is a compound of Formula (Vc) wherein Z is phenyl monosubstituted with n-butyl. In a further embodiment is a compound of Formula (Vc) wherein Z is phenyl monosubstituted with isobutyl. In a further embodiment is a compound of Formula (Vc) wherein Z is phenyl monosubstituted with tert-butyl. In another embodiment is a compound of Formula (Vc) wherein Z is optionally substituted heteroaryl. In another embodiment is a compound of Formula (Vc) wherein Z is optionally substituted -(C₃-C₇)cycloalkyl. In another embodiment is a compound of Formula (Vc) wherein Z is optionally substituted heterocycloalkyl. In another embodiment is a compound of Formula (Vc) wherein Z is halogen.

In some embodiments, the compound of Formula (V), (Va), (Vb) and (Vc) is selected from a compound in table 2 or a pharmaceutically acceptable salt, solvate, or prodrug thereof.

**Table 2**

| **Cp. #** | **Name** | **Structure** |
|---|---|---|
| **563** | rac-(8S,11S,14S)-4,18-bis(2-aminoethoxy)-3-hydroxy-11-methyl-14-[methyl-[rac-(2S)-4-amino-2-[[2-(4-tert-butylphenyl)-4,6-dimethyl-pyrimidine-5-carbonyl]amino]butanoyl]amino-10,13-dioxo-9,12-diazatricyclo[13.3.1.12,6]icosa-1(1S),2,4,6(20),15(19),16-hexaene-8-carboxylic acid | 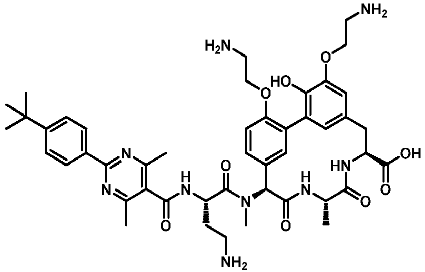 |
| **638** | rac-(8S,11S,14S)-3,18-bis(2-aminoethoxy)-11-methyl-14-[methyl-[rac-(2S)-4-amino-2-[[4,6-dimethyl-2-(4-pentoxyphenyl)pyrimidine-5-carbonyl]aminobutanoyl]amino-10,13-dioxo-9,12-diazatricyclo[13.3.1.12,6]icosa-1(1S),2(20),3,5,15(19),16-hexaene-8-carboxylic acid | 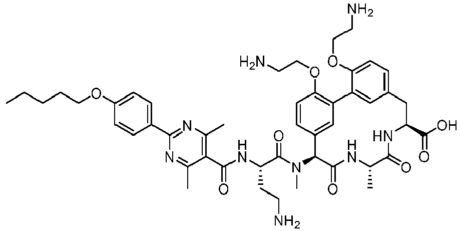 |
| **639** | rac-(8S,11S,14S)-3,18-bis(2-aminoethoxy)-11-methyl-14-[methyl-[rac-(2S)-4-amino-2-[[2-[4-(1-ethylcyclopropoxy)phenyl]-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxylic acid | 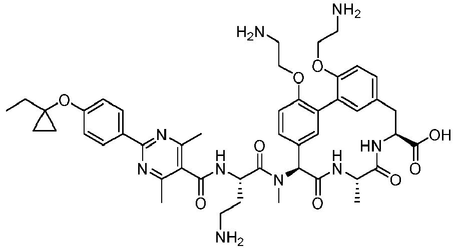 |
| **640** | rac-(8S,11S,14S)-3,18-bis(2-aminoethoxy)-11-methyl-14-[methyl-[rac-(2S)-4-amino-2-[[2-(4-tert-butylphenyl)-4-methylpyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxylic acid | 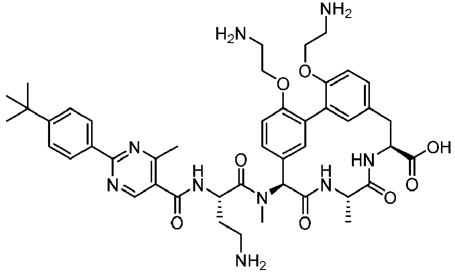 |
| **641** | rac-(8S,11S,14S)-3,18-bis(2-aminoethoxy)-11-methyl-14-[methyl-[rac-(2S)-4-amino-2-[[2-(4-hexoxyphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxylic acid | 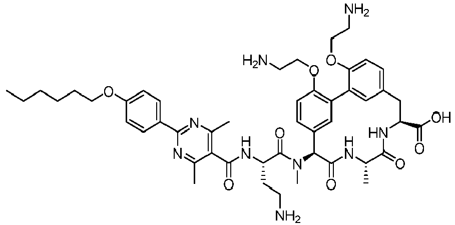 |
| **642** | rac-(8S,11S,14S)-3-(2-aminoethoxy) -18 -hydroxy-11-methyl-14-[methyl-[rac-(2S)-4-amino-2-[[4,6-dimethyl-2-(4-pentoxyphenyl)pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxylic acid | 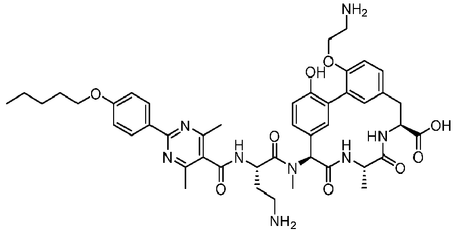 |
| **643** | rac-(8S,11S,14S)-18-(2-aminoethoxy)-3-hydroxy-11-methyl-14-[methyl-[rac-(2S)-4-amino-2-[[2-(4-isopropoxyphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxylic acid | 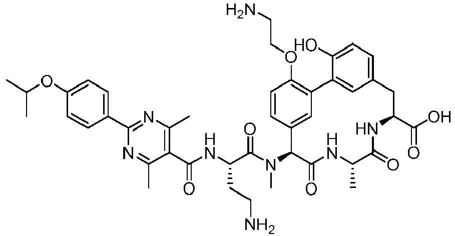 |
| **644** | rac-(8S,11S,14S)-18-(2-aminoethoxy)-3-hydroxy-11-methyl-14-[methyl-[rac-(2S)-4-amino-2-[[6-(4-tert-butylphenyl)-2,4-dimethylpyridine-3-carbonyl]aminobutanoyl]amino- 10,13-dioxo-9,12-diazatricyclo[13.3.1.12,6]icosa-1(18),2(20),3,5,15(19),16-hexaene-8-carboxylic acid | 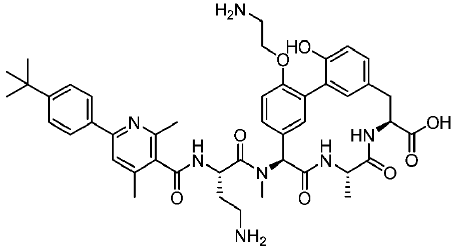 |
| **645** | rac-(8S,11S,14S)-3,18-bis(2-aminoethoxy)-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxylic acid | 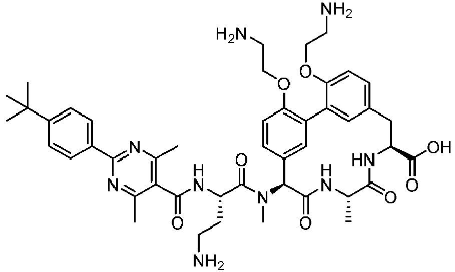 |
| **646** | rac-(8S,11S,14S)-18-(2-aminoethoxy)-3-hydroxy-11-methyl-14-[methyl-[rac-(2S)-4-amino-2-[[2-(4-tert-butylphenyl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxylic acid | 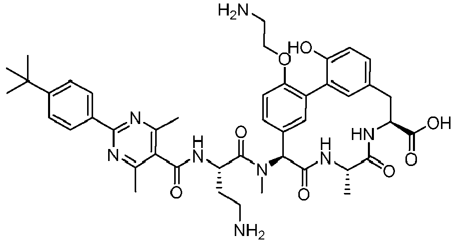 |
| **647** | rac-(85,11S,14S)-3,18-bis(2-aminoethoxy)-11-methyl-14-[methyl-[rac-(2S)-4-amino-2-[[2-(1,1-dimethylindan-5-yl)-4,6-dimethyl-pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxylic acid | 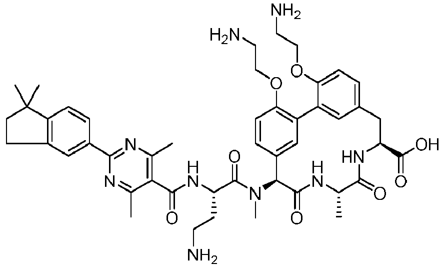 |
| **648** | rac-(8S,11S,14S)-18-(2-aminoethoxy)-3-hydroxy-11-methyl-14-[methyl-[rac-(2S)-4-amino-2-[[4,6-dimethyl-2-(4-pentoxyphenyl)pyrimidine-5-carbonyl]aminobutanoyl]amino- 10,13-dioxo-9,12-diazatricyclo[13.3.1.12,6]icosa-1(18),2(20),3,5,15(19),16-hexaene-8-carboxylic acid | 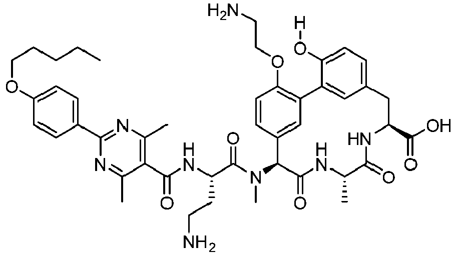 |
| **649** | | 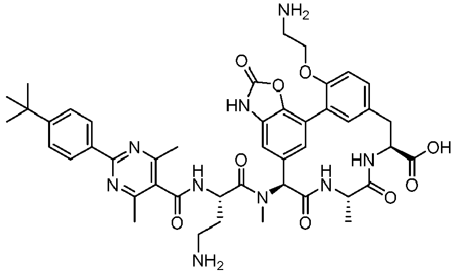 |

In another aspect are hydrates or metabolites of any of the aforementioned compounds.

In another aspect are pharmaceutical compositions comprising any of the aforementioned compounds together with a pharmaceutically acceptable excipient.

In another aspect described herein is the use of a compound described herein in the manufacture of a medicament for treatment of a bacterial infection in a patient.

In another aspect are methods of treating a mammal in need of such treatment comprising administering to the mammal an antibacterial effective amount of any of the aforementioned compounds at a frequency and for a duration sufficient to provide a beneficial effect to the mammal. In one embodiment, the mammal has a bacteria-related infection that is resistant to treatment with arylomycin A2. In a further embodiment, the causative bacteria species of the bacteria infection is an infection involoving Pseudomonas aeruginosa, Pseudomonas fluorescens, Pseudomonas acidovorans, Pseudomonas alcaligenes, Pseudomonas putida, Stenotrophomonas maltophilia, Burkholderia cepacia, Aeromonas hydrophilia, Escherichia coli, Citrobacter freundii, Salmonella typhimurium, Salmonella typhi, Salmonella paratyphi, Salmonella enteritidis, Shigella dysenteriae, Shigella flexneri, Shigella sonnei, Enterobacter cloacae, Enterobacter aerogenes, Klebsiella pneumoniae, Klebsiella oxytoca, Serratia marcescens, Francisella tularensis, Morganella morganii, Proteus mirabilis, Proteus vulgaris, Providencia alcalifaciens, Providencia rettgeri, Providencia stuartii, Acinetobacter baumannii, Acinetobacter calcoaceticus, Acinetobacter haemolyticus, Yersinia enterocolitica, Yersinia pestis, Yersinia pseudotuberculosis, Yersinia intermedia, Bordetella pertussis, Bordetella parapertussis, Bordetella bronchiseptica, Haemophilus influenzae, Haemophilus parainfluenzae, Haemophilus haemolyticus, Haemophilus parahaemolyticus, Haemophilus ducreyi, Pasteurella multocida, Pasteurella haemolytica, Branhamella catarrhalis, Helicobacter pylori, Campylobacter fetus, Campylobacter jejuni, Campylobacter coli, Borrelia burgdorferi, Vibrio cholerae, Vibrio parahaemolyticus, Legionella pneumophila, Listeria monocytogenes, Neisseria gonorrhoeae, Neisseria meningitidis, Kingella, Moraxella, Gardnerella vaginalis, Bacteroides fragilis, Bacteroides distasonis, Bacteroides 3452A homology group, Bacteroides vulgatus, Bacteroides ovalus, Bacteroides thetaiotaomicron, Bacteroides uniformis, Bacteroides eggerthii, Bacteroides splanchnicus, Clostridium difficile, Mycobacterium tuberculosis, Mycobacterium avium, Mycobacterium intracellulare, Mycobacterium leprae, Corynebacterium diphtheriae, Corynebacterium ulcerans, Streptococcus pneumoniae, Streptococcus agalactiae, Streptococcus pyogenes, Enterococcus faecalis, Enterococcus faecium, Staphylococcus aureus, Staphylococcus epidermidis, Staphylococcus saprophyticus, Staphylococcus intermedius, Staphylococcus hyicus subsp. hyicus, Staphylococcus haemolyticus, Staphylococcus hominis, or Staphylococcus saccharolyticus. In another embodiment the bacterial infection is an infection involving a Gram-negative bacteria. In another embodiment the bacterial infection is a lepB-mediated infection. In a further embodiment, the bacterial infection is an infection involving a Gram-positive bacteria.

In a further embodiment are methods of treating a mammal in need of such treatment comprising administering to the mammal a second therapeutic agent to any of the aforementioned methods of treatment. In another embodiment, the second therapeutic agent is a not an SpsB inhibitor. In another embodiment, the second therapeutic agent is an aminoglycoside antibiotic, fluoroquinolone antibiotic, β-lactam antibiotic, macrolide antibiotic, glycopeptide antibiotic, rifampicin, chloramphenicol, fluoramphenicol, colistin, mupirocin, bacitracin, daptomycin, or linezolid.

In some embodiments is a method for treating a bacterial infection in a patient, preferably a human, where the treatment includes administering a therapeutically or pharmacologically effective amount of a combination of 1) a β-lactam antibiotic; and 2) a compound of Formula (I), (Ia)-(If), (II), (IIa)-(IIe), (III), (IIIa)-(IIIc), (IV), (IVa)-(IVc), (V), or (Va)-(Vc), or a pharmaceutically acceptable salt thereof; and 3) a pharmaceutically acceptable carrier. In embodiments where a β-lactam antibiotic is used in combination with a compound of Formula (I), (Ia)-(If), (II), (IIa)-(IIe), (III), (IIIa)-(IIIc), (IV), (IVa)-(IVc), (V), or (Va)-(Vc), the β-lactam antibiotic may be a carbapenem, cephalosporin, cephamycin, monobactam or penicillin. Exemplary carbapenem antibiotics useful in the methods of the invention include ertapenem, imipenem, biapenem, and meropenem. Exemplary cephalosporin antibiotics useful in the methods of the invention include, ceftobiprole, ceftaroline, Cefiprome, Cefozopran, cefepime, Cefotaxime, and ceftriazone. Exemplary penicillin antibiotics useful in the methods of the invention include ampicillin, amoxacillin, piperacillin, oxacillin, cloxacillin, methicillin, and nafcillin. In some embodiments of the invention, the β-lactam may be administered with a β-lactamase inhibitor. In some embodiments of the invention, the carbapenem may be administered with a DHP inhibitor, e.g., cilastatin.

In various embodiments of the invention where a compound of Formula (I), (1a)-(If), (II), (IIa)-(IIe), (III), (IIIa)-(IIIc), (IV), (IVa)-(IVc), (V), or (Va)-(Vc) and a β-lactam antibiotic are used in combination, the β-lactam antibiotic and compound of Formula (I), (1a)-(If), (II), (IIa)-(IIe), (III), (IIIa)-(IIIc), (IV), (IVa)-(IVc), (V), or (Va)-(Vc) can be administered sequentially or concurrently. Preferably, the β-lactam antibiotic and compound of Formula (I), (1a)-(If), (II), (IIa)-(IIe), (III), (IIIa)-(IIIc), (IV), (IVa)-(IVc), (V), or (Va)-(Vc)are administered together. When administered concurrently, the β-lactam antibiotic and compound of Formula (I), (Ia)-(If), (II), (IIa)-(IIe), (III), (IIIa)-(IIIc), (IV), (IVa)-(IVc), (V), or (Va)-(Vc) may be administered in the same formulation or in separate formulations. When administered sequentially, either the β-lactam or compound of Formula (I), (Ia)-(If), (II), (IIa)-(IIe), (III), (IIIa)-(IIIc), (IV), (IVa)-(IVc), (V), or (Va)-(Vc) may be administered first. After administration of the first compound, the other compound is administered, for example, within from 1 to 60 minutes, e.g., within 1, 2, 3, 4, 5, 10, 15, 30, or 60 minutes. In one aspect of the invention, when a β-lactamase inhibitor is used, it may be administered separately, or in a formulation with the compound of Formula (I), (1a)-(If), (II), (IIa)-(IIe), (III), (IIIa)-(IIIc), (IV), (IVa)-(IVc), (V), or (Va)-(Vc) and/or β-lactam antibiotic. In one aspect of the invention, when a DHP inhibitor is used to improve the stability of a carbapenem, it may be administered separately, or in a formulation with the compound of Formula (I), (1a)-(If), (II), (IIa)-(IIe), (III), (IIIa)-(IIIc), (IV), (IVa)-(IVc), (V), or (Va)-(Vc) and/or carbapenem.

Further described herein are pharmaceutical compositions comprising a compound of Formula (I), (Ia)-(If), (II), (IIa)-(IIe), (III), (IIIa)-(IIIc), (IV), (IVa)-(IVc), (V), or (Va)-(Vc), a pharmaceutically acceptable carrier, and optionally a β-lactam antibiotic. In embodiments where a combination is used, the β-lactam antibiotic and the compound of Formula (I), (1a)-(If), (II), (IIa)-(IIe), (III), (IIIa)-(IIIc), (IV), (IVa)-(IVc), (V), or (Va)-(Vc), are present in such amounts that their combination constitutes a therapeutically effective amount. Due to the potentiating effects of the compound of Formula (I), (Ia)-(If), (II), (IIa)-(IIe), (III), (IIIa)-(IIIc), (IV), (IVa)-(IVc), (V), or (Va)-(Vc), the amount of β-lactam antibiotic present in a combination may be less that of a β-lactam antibiotic used alone. In certain embodiments, the composition further comprises a β-lactamase antibiotic.

In further embodiments where the β-lactam antibiotic is a carbapenem, is provided a pharmaceutical composition comprising a carbapenem antibiotic, a DHP inhibitor, a compound of Formula (I), (Ia)-(If), (II), (IIa)-(IIe), (III), (IIIa)-(IIIc), (IV), (IVa)-(IVc), (V), or (Va)-(Vc), and a pharmaceutically acceptable carrier. In some embodiments where the β-lactara antibiotic is a carbepenem, the carbapenem antibiotic is preferably selected from the group consisting of ertapenem, imipenem, and meropenem.

In some embodiments is a compound of Formula (I), (1a)-(If), (II), (IIa)-(IIe), (III), (IIIa)-(IIIc), (IV), (IVa)-(IVc), (V), or (Va)-(Vc) for use in treating a bacterial infection. In some embodiments is a compound of Formula (I), (1a)-(If), (II), (IIa)-(IIe), (III), (IIIa)-(IIIc), (IV), (IVa)-(IVc), (V), or (Va)-(Vc), in combination with one or more additional therapeutical agents including a β-lactam antibiotic, for use in treating a bacterial infection. In some embodiments is a compound of Formula (I), (Ia)-(If), (II), (IIa)-(IIe), (III), (IIIa)-(IIIc), (IV), (IVa)-(IVc), (V), or (Va)-(Vc) for use as a medicament for treating a bacterial infection. In some embodiments is a compound of Formula (I), (1a)-(If), (II), (IIa)-(IIe), (III), (IIIa)-(IIIc), (IV), (IVa)-(IVc), (V), or (Va)-(Vc), in combination with one or more additional therapeutical agents including a β-lactam antibiotic, for use as a medicament for treating a bacterial infection. In some embodiments is a compound of Formula (I), (Ia)-(If), (II), (IIa)-(IIe), (III), (IIIa)-(IIIc), (IV), (IVa)-(IVc), (V), or (Va)-(Vc) for use in the preparation of a medicament for treating a bacterial infection. In some embodiments is a compound of Formula (I), (Ia)-(If), (II), (IIa)-(IIe), (III), (IIIa)-(IIIc), (IV), (IVa)-(IVc), (V), or (Va)-(Vc), in combination with one or more additional therapeutical agents including a β-lactam antibiotic, for use in the preparation of a medicament for treating a bacterial infection.

In some embodiments described herein, a compound of Formula (I), (Ia)-(If), (II), (IIa)-(IIe), (III), (IIIa)-(IIIc), (IV), (IVa)-(IVc), (V), or (Va)-(Vc) can enhance the activity of a β-lactam antibacterial agent by inducing susceptibility to the antibacterial agent in a drug- resistant strain such as MRSA. In some embodiments, a compound of Formula (I), (1a)-(If), (II), (IIa)-(IIe), (III), (IIIa)-(IIIc), (IV), (IVa)-(IVc), (V), or (Va)-(Vc) can enhance the activity of a β-lactam antibacterial agent by reducing the dosage of the antibacterial agent need for a therapeutic effect in a drug-sensitive strain. For example, if a compound of Formula (I), (1a)-(If), (II), (IIa)-(IIe), (III), (IIIa)-(IIIc), (IV), (IVa)-(IVc), (V), or (Va)-(Vc) reduces the Minimum Inhibitory Concentration (MIC) of an antibacterial agent (where the MIC is the minimum concentration of antibacterial agent which will completely inhibit growth) in a susceptible strain, then such treatment may be advantageous to enable a reduction in the amount of antibacterial agent administered (could reduce side effects of an antibiotic), or to decrease the frequency of administration. In some embodiments, compounds of Formula (I), (1a)-(If), (II), (IIa)-(IIe), (III), (IIIa)-(IIIc), (IV), (IVa)-(IVc), (V), or (Va)-(Vc) can enhance the activity of an antibacterial agent such as a carbapenem to prevent the emergence of a resistant sub-population in a heterogeneous bacterial population with a resistant sub-population.

Potentiators can be used to enhance the activity of antibacterial agents whose clinical efficacy has been limited by the increasing prevalence of resistant strains. In some embodiments described herein, a compound of Formula (I), (1a)-(If), (II), (IIa)-(IIe), (III), (IIIa)-(IIIc), (IV), (IVa)-(IVc), (V), or (Va)-(Vc) is used as a potentiator wherein a compound of Formula (I), (1a)-(If), (II), (IIa)-(IIe), (III), (IIIa)-(IIIc), (IV), (IVa)-(IVc), (V), or (Va)-(Vc) can be administered together with a β-lactam antibiotic (either concurrently or sequentially) to allow effective treatment of an infection involving a resistant bacterium, or to reduce the amount of antibacterial agent necessary to treat an infection.

In one embodiment, is a compound described herein which displays antibiotic activity useful in the treatment of bacterial infections, such as by way of example only, various strains of S. aureus, S. pneumoniae, E. faecalis, E. faecium, B. subtilis and E. coli including species that are resistant to many known antibiotics such as methicillin-resistant S. aureus (MRSA), vancomycin-resistant Enterococcus sp. (VRE), multidrug-resistant E. faecium, macrolide-resistant S. aureus and S. epidermidis, and linezolide-resistant S. aureus and E. faecium.

### Methicillin-Resistant Staphylococcus aureus

Staphylococcus aureus (S. aureus), a spherical bacterium, is the most common cause of staph infections. S. aureus has been known to cause a range of illnesses from minor skin infections, such as pimples, impetigo, boils, cellulitis folliculitis, furuncles, carbuncles, scalded skin syndrome, abscesses, to life-threatening diseases such as pneumonia, meningitis, osteomyelitis endocarditis, toxic shock syndrome, and septicemia. Further, S. aureus is one of the most common causes of nosocomial infections, often causing postsurgical wound infections.

Methicillin was introduced in the late 1950s to treat infections caused by penicillin-resistant S. aureus. It has been reported previously that *S*. *aureus* isolates had acquired resistance to methicillin (methicillin-resistant *S*. *aureus,* MRSA). The methicillin resistance gene (*mecA*) encodes a methicillin-resistant penicillin-binding protein that is not present in susceptible strains. *mecA* is carried on a mobile genetic element, the staphylococcal cassette chromosome *mec* (SCC*mec*), of which four forms have been described that differ in size and genetic composition. The methicillin-resistant penicillin-binding protein allows for resistance to β-lactam antibiotics and obviates their clinical use during MRSA infections.

In one aspect is a method for treating a subject having a resistant bacterium comprising administering to the subject a compound of Formula (I), (Ia)-(If), (II), (IIa)-(IIe), (III), (IIIa)-(IIIc), (IV), (IVa)-(IVc), (V), or (Va)-(Vc) or a pharmaceutically acceptable salt, solvate, or prodrug thereof. In one embodiment, the bacterium is a Gram-positive bacteria. In another embodiment, the Gram-positive bacterium is S. aureus. In further embodiment, the S. aureus is resistant or refractory to a beta-lactam antibiotic. In yet a further embodiment, the beta-lactam antibiotic belongs to the class of penicillins. In a further embodiment, the beta-lactam antibiotic is methicillin. In yet another embodiment, the subject has a methicillin-resistant S. aureus bacteria. In one embodiment the beta-lactam antibiotic is flucloxacillin. In another embodiment is a method for treating a subject having a dicloxacillin-resistant bacteria comprising administering to the subject a compound of Formula (I), (Ia)-(If), (II), (IIa)-(IIe), (III), (IIIa)-(IIIc), (IV), (IVa)-(IVc), (V), or (Va)-(Vc) or a pharmaceutically acceptable salt, solvate, or prodrug thereof wherein the subject is refractory to dicloxacillin. Also disclosed herein is a method for treating a subject having a methicillin-resistant bacteria comprising administering a compound of Formula (I), (1a)-(If), (II), (IIa)-(IIe), (III), (IIIa)-(IIIc), (IV), (IVa)-(IVc), (V), or (Va)-(Vc) or a pharmaceutically acceptable salt, solvate, or prodrug thereof wherein the subject has been determined to have a methicillin-resistant bacteria. In one embodiment the subject is screened for methicillin-resistant bacteria. In another embodiment, the subject screening is performed through a nasal culture. In a further embodiment the methicillin-resistant bacteria is detected by swabbing the nostril(s) of the subject and isolating the bacteria. In another embodiment, Real-time PCR and/or Quantitative PCR is employed to determine whether the subject has a methicillin-resistant bacteria.

In one embodiment is a method for treating a subject having a first-generation cephalosporin-resistant bacteria comprising administering a compound of Formula (I), (1a)-(If), (II), (IIa)-(IIe), (III), (IIIa)-(IIIc), (IV), (IVa)-(IVc), (V), or (Va)-(Vc) or a pharmaceutically acceptable salt, solvate, or prodrug thereof wherein the subject is refractory to a first-generation cephalosporin. In one embodiment, the bacteria is resistant to a first-generation cephalosporin. In a further embodiment, the bacteria is resistant to cefacetrile. In another embodiment, the bacteria is resistant to cefadroxil. In yet another embodiment, the bacteria is resistant to cefalexin. In one embodiment, the bacteria is resistant to cefaloglycin. In another embodiment, the bacteria is resistant to cefalonium. In another embodiment, the bacteria is resistant to cefaloridine. In yet another embodiment, the bacteria is resistant to cefalotin. In a further embodiment, the bacteria is resistant to cefapirin. In yet a further embodiment, the bacteria is resistant to cefatrizine. In one embodiment, the bacteria is resistant to cefazaflur. In another embodiment, the bacteria is resistant to cefazedone. In yet another embodiment, the bacteria is resistant to cefazolin. In a further embodiment, the bacteria is resistant to cefradine. In yet a further embodiment, the bacteria is resistant to cefroxadine. In one embodiment, the bacteria is resistant to ceftezole.

In one embodiment is a method for treating a subject having a second-generation cephalosporin-resistant bacteria comprising administering a compound of Formula (I), (1a)-(If), (II), (IIa)-(IIe), (III), (IIIa)-(IIIc), (IV), (IVa)-(IVc), (V), or (Va)-(Vc) or a pharmaceutically acceptable salt, solvate, or prodrug thereof wherein the subject is refractory to a second-generation cephalosporin. In another embodiment, the bacteria is resistant to a second-generation cephalosporin. In a further embodiment, the bacteria is resistant to cefaclor. In another embodiment, the bacteria is resistant to cefonicid. In yet another embodiment, the bacteria is resistant to cefprozil. In one embodiment, the bacteria is resistant to cefuroxime. In another embodiment, the bacteria is resistant to cefuzonam. In another embodiment, the bacteria is resistant to cefmetazole. In yet another embodiment, the bacteria is resistant to cefotetan. In a further embodiment, the bacteria is resistant to cefoxitin.

In one embodiment is a method for treating a subject having a third-generation cephalosporin-resistant bacteria comprising administering a compound of Formula (I), (1a)-(If), (II), (IIa)-(IIe), (III), (IIIa)-(IIIc), (IV), (IVa)-(IVc), (V), or (Va)-(Vc) or a pharmaceutically acceptable salt, solvate, or prodrug thereof wherein the subject is refractory to a third-generation cephalosporin. In another embodiment, the bacteria is resistant to a third-generation cephalosporin. In a further embodiment, the bacteria is resistant to cefcapene. In another embodiment, the bacteria is resistant to cefdaloxime. In yet another embodiment, the bacteria is resistant to cefdinir. In one embodiment, the bacteria is resistant to cefditoren. In another embodiment, the bacteria is resistant to cefixime. In another embodiment, the bacteria is resistant to cefmenoxime. In yet another embodiment, the bacteria is resistant to cefodizime. In a further embodiment, the bacteria is resistant to cefotaxime. In yet a further embodiment, the bacteria is resistant to cefpimizole. In one embodiment, the bacteria is resistant to cefpodoxime. In another embodiment, the bacteria is resistant to cefteram. In yet another embodiment, the bacteria is resistant to ceftibuten. In a further embodiment, the bacteria is resistant to ceftiofur. In yet a further embodiment, the bacteria is resistant to ceftiolene. In one embodiment, the bacteria is resistant to ceftizoxime. In another embodiment, the bacteria is resistant to ceftriaxone. In yet another embodiment, the bacteria is resistant to cefoperazone. In yet a further embodiment, the bacteria is resistant to ceftazidime.

In one embodiment is a method for treating a subject having a fourth-generation cephalosporin-resistant bacteria comprising administering a compound of Formula (I), (1a)-(If), (II), (IIa)-(IIe), (III), (IIIa)-(IIIc), (IV), (IVa)-(IVc), (V), or (Va)-(Vc) or a pharmaceutically acceptable salt, solvate, or prodrug thereof wherein the subject is refractory to a fourth-generation cephalosporin. In another embodiment, the bacteria is resistant to a fourth-generation cephalosporin. In a further embodiment, the bacteria is resistant to cefclidine. In another embodiment, the bacteria is resistant to cefepime. In yet another embodiment, the bacteria is resistant to cefluprenam. In one embodiment, the bacteria is resistant to cefoselis. In another embodiment, the bacteria is resistant to cefozopran. In another embodiment, the bacteria is resistant to cefpirome. In yet another embodiment, the bacteria is refractory to cefquinome.

In one embodiment is a method for treating a subject having a carbapenem-resistant bacteria comprising administering a compound of Formula (I), (1a)-(If), (II), (IIa)-(IIe), (III), (IIIa)-(IIIc), (IV), (IVa)-(IVc), (V), or (Va)-(Vc) or a pharmaceutically acceptable salt, solvate, or prodrug thereof wherein the subject is refractory to a carbapenem. In another embodiment, the bacteria is resistant to a carbapenem. In a further embodiment, the bacteria is resistant to imipenem. In another embodiment, the bacteria is resistant to meropenem. In yet another embodiment, the bacteria is resistant to ertapenem. In one embodiment, the bacteria is resistant to faropenem. In another embodiment, the bacteria is resistant to doripenem. In another embodiment, the bacteria is resistant to panipenem. In yet another embodiment, the bacteria is resistant to biapenem,

### Vancomycin-Intermediate and Vancomycin-Resistant Staphylococcus aureus

Vancomycin-intermediate Staphylococcus aureus and vancomycin-resistant staphylococcus aureus are specific types of antimicrobial-resistant Staph bacteria that are refractory to vancomycin treatment. S. aureus isolates for which vancomycin MICs are 4-8 µg/mL are classified as vancomycin-intermediate and isolates for which vancomycin MICs are ≥16 µg/mL are classified as vancomycin-resistant (Clinical and Laboratory Standards Institute/NCCLS. Performance Standards for Antimicrobial Susceptibility Testing. Sixteenth informational supplement. M100-S16. Wayne, PA: CLSI, 2006).

As used herein, the term "minimum inhibitory concentration" (MIC) refers to the lowest concentration of an antibiotic that is needed to inhibit growth of a bacterial isolate in vitro. A common method for determining the MIC of an antibiotic is to prepare several tubes containing serial dilutions of the antibiotic, that are then inoculated with the bacterial isolate of interest. The MIC of an antibiotic is determined from the tube with the lowest concentration that shows no turbidity (no growth).

In one aspect is a method of treating a subject having a bacterial infection comprising administering to the subject a compound of Formula (I), (Ia)-(If), (II), (IIa)-(IIe), (III), (IIIa)-(IIIc), (IV), (IVa)-(IVc), (V), or (Va)-(Vc) or a pharmaceutically acceptable salt, solvate, or prodrug thereof wherein the bacterial infection comprises a vancomycin-intermediate Staphylococcus aureus bacterium. In one embodiment, the vancomycin-intermediate Staphylococcus aureus bacterium has a MIC of between about 4 to about 8 µg/mL. In another embodiment, the vancomycin-intermediate Staphylococcus aureus bacterium has a MIC of about 4 µg/mL. In yet another embodiment, the vancomycin-intermediate Staphylococcus aureus bacterium has a MIC of about 5 µg/mL. In a further embodiment, the vancomycin-intermediate Staphylococcus aureus bacterium has a MIC of about 6 µg/mL. In yet a further embodiment, the vancomycin-intermediate Staphylococcus aureus bacterium has a MIC of about 7 µg/mL. In one embodiment, the vancomycin-intermediate Staphylococcus aureus bacterium has a MIC of about 8 µg/mL.

In another aspect is a method of treating a subject having a bacterial infection comprising administering to the subject a compound of Formula (I), (Ia)-(If), (II), (IIa)-(IIe), (III), (IIIa)-(IIIc), (IV), (IVa)-(IVc), (V), or (Va)-(Vc) or a pharmaceutically acceptable salt, solvate, or prodrug thereof wherein the bacterial infection comprises a vancomycin-resistant Staphylococcus aureus bacterium. In one embodiment, the vancomycin-resistant Staphylococcus aureus bacterium has a MIC of between about 16 µg/mL. In another embodiment, the vancomycin-resistant Staphylococcus aureus bacterium has a MIC of about ≥ 16 µg/mL. In yet another embodiment, the vancomycin-resistant Staphylococcus aureus bacterium has a MIC of about 20 µg/mL. In a further embodiment, the vancomycin-resistant Staphylococcus aureus bacterium has a MIC of about 25 µg/mL.

In one embodiment, conditions treated by the compounds described herein include, but are not limited to, endocarditis, osteomyelitis, neningitis, skin and skin structure infections, genitourinary tract infections, abscesses, and necrotizing infections. In another embodiment, the compounds disclosed herein are used to treat conditions, such as, but not limited to, diabetic foot infections, decubitus ulcers, burn infections, animal or human bite wound infections, synergistic-necrotizing gangrene, necrotizing fascilitis, intra-abdominal infection associated with breeching of the intestinal barrier, pelvic infection associated with breeching of the intestinal barrier, aspiration pneumonia, and post-operative wound infections. In another embodiment, the conditions listed herein are caused by, contain, or result in the presence of VISA and/or VRSA.

### Vancomycin-Resistant Enterococci

Enterococci are bacteria that are normally present in the human intestines and in the female genital tract and are often found in the environment. These bacteria sometimes cause infections. In some cases, enterococci have become resistant to vancomycin (also known as vancomycin-resistant enterococci or VRE.) Common forms of resistance to vancomycin occur in enterococcal strains that involve the acquisition of a set of genes endoding proteins that direct peptidoglycan precursors to incorporate D-Ala-D-Lac instead of D-Ala-D-Ala. The six different types of vancomycin resistance shown by enterococcus are: Van-A, Van-B, Van-C, Van-D, Van-E and Van-F. In some cases, Van-A VRE is resistant to both vancomycin and teicoplanin, while in other cases, Van-B VRE is resistant to vancomycin but sensitive to teicoplanin; in further cases Van-C is partly resistant to vancomycin, and sensitive to teicoplanin.

In one aspect, is a method of treating a subject having a vancomycin-resistant enterococci comprising administering to the subject a compound of Formula (I), (Ia)-(If), (II), (IIa)-(IIe), (III), (IIIa)-(IIIc), (IV), (IVa)-(IVc), (V), or (Va)-(Vc) or a pharmaceutically acceptable salt, solvate, or prodrug thereof wherein the enterococci has developed resistance to vancomycin. In one embodiment, the subject has been previously treated with vancomycin for a sustained period of time. In another embodiment, the subject has been hospitalized. In yet another embodiment, the subject has a weakened immune system such as patients in Intensive Care Units or in cancer or transplant wards. In a further embodiment, the subject has undergone surgical procedures such as, for example, abdominal or chest surgery. In yet a further embodiment, the subject has been colonized vith VRE. In one embodiment, the subject has a medical device such that an infection has developed. In another embodiment, the medical device is a urinary catheter or central intravenous (IV) catheter.

In another embodiment, is a method of treating a subject having a vancomycin-resistant enterococci comprising administering to the subject a compound of Formula (I), (Ia)-(If), (II), (IIa)-(IIe), (III), (IIIa)-(IIIc), (IV), (IVa)-(IVc), (V), or (Va)-(Vc) or a pharmaceutically acceptable salt, solvate, or prodrug thereof wherein the enterococcus has Van-A resistance.

In another embodiment, is a method of treating a subject having a vancomycin-resistant enterococci comprising administering to the subject a compound of Formula (I), (Ia)-(If), (II), (IIa)-(IIe), (III), (IIIa)-(IIIc), (IV), (IVa)-(IVc), (V), or (Va)-(Vc) or a pharmaceutically acceptable salt, solvate, or prodrug thereof wherein the enterococcus has Van-B resistance.

In another embodiment, is a method of treating a subject having a vancomycin-resistant enterococci comprising administering to the subject a compound of Formula (I), (Ia)-(If), (II), (IIa)-(IIe), (III), (IIIa)-(IIIc), (IV), (IVa)-(IVc), (V), or (Va)-(Vc) or a pharmaceutically acceptable salt, solvate, or prodrug thereof wherein the enterococcus has Van-C resistance.

### Administration and Pharmaceutical Composition

Pharmaceutical compositions described herein comprise a therapeutically effective amount of a compound described herein (i.e., a compound of any of Formula (I), (Ia)-(If), (II), (IIa)-(IIe), (III), (IIIa)-(IIIc), (IV), (IVa)-(IVc), (V), or (Va)-(Vc)) formulated together with one or more pharmaceutically acceptable carriers. As used herein, the term "pharmaceutically acceptable carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator. The pharmaceutical compositions described herein can be administered to humans and other animals orally, rectally, parenterally, intracisternally, intravaginally, intraperitoneally, topically (as by powders, ointments, or drops), bucally, or as an oral or nasal spray, or a liquid aerosol or dry powder formulation for inhalation.

Liquid dosage forms for oral administration include pharmaceutically acceptable emulsions, microemulsions, solutions, suspensions, syrups and elixirs. In addition to the active compounds, the liquid dosage forms optionally contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions are optionally formulated according to the known art using suitable dispersing or wetting agents and suspending agents. The sterile injectable preparation is optionally a sterile injectable solution, suspension or emulsion in a nontoxic parenterally acceptable diluent or solvent, for example, as a solution in 1,3-butanediol. Among the acceptable vehicles and solvents that are optionally employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This is optionally accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is optionally accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are optionally prepared by entrapping the drug in liposomes or microemulsions which are compatible with body tissues.

Compositions for rectal or vaginal administration are preferably suppositories which can be prepared by mixing the compound described herein (i.e., a compound of any of Formula (I), (Ia)-(If), (II), (IIa)-(IIe), (III), (IIIa)-(IIIc), (IV), (IVa)-(IVc), (V), or (Va)-(Vc)) with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, acetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form optionally comprise buffering agents.

Solid compositions of a similar type are optionally employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings known in the pharmaceutical formulating art. They optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Solid compositions of a similar type are optionally employed as fillers in soft and hard-filled gelatin capsules using such excipients as lactose or milk sugar as well as high molecular weight polyethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings known in the pharmaceutical formulating art. In such solid dosage forms the active compound is optionally admixed with at least one inert diluent such as sucrose, lactose or starch. Such dosage forms optionally comprise, as is normal practice, additional substances other than inert diluents, e.g., tableting lubricants and other tableting aids such a magnesium stearate and microcrystalline cellulose. In the case of capsules, tablets and pills, the dosage forms optionally comprise buffering agents. They optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Dosage forms for topical or transdermal administration of a compound described herein include ointments, pastes, creams, lotions, gels, powders, solutions, sprays, inhalants or patches. The active component is admixed under sterile conditions with a pharmaceutically acceptable carrier and any needed preservatives or buffers as are optionally required. Ophthalmic formulations, ear drops, and the like are also contemplated.

The ointments, pastes, creams and gels may contain, in addition to an active compound described herein, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Compositions described herein are optionally formulated for delivery as a liquid aerosol or inhalable dry powder. Liquid aerosol formulations are optionally nebulized predominantly into particle sizes that can be delivered to the terminal and respiratory bronchioles where bacteria reside in patients with bronchial infections, such as chronic bronchitis and pneumonia. Pathogenic bacteria are commonly present throughout airways down to bronchi, bronchioli and lung parenchema, particularly in terminal and respiratory bronchioles. During exacerbation of infection, bacteria can also be present in alveoli. Liquid aerosol and inhalable dry powder formulations are preferably delivered throughout the endobronchial tree to the terminal bronchioles and eventually to the parenchymal tissue.

Aerosolized formulations described herein are optionally delivered using an aerosol forming device, such as a jet, vibrating porous plate or ultrasonic nebulizer, preferably selected to allow the formation of a aerosol particles having with a mass medium average diameter predominantly between 1 to 5 □. Further, the formulation preferably has balanced osmolarity ionic strength and chloride concentration, and the smallest aerosolizable volume able to deliver effective dose of the compounds described herein compound described herein (i.e., a compound of any of Formula (I), (Ia)-(If), (II), (IIa)-(IIe), (III), (IIIa)-(IIIc), (IV), (IVa)-(IVc), (V), or (Va)-(Vc)) to the site of the infection. Additionally, the aerosolized formulation preferably does not impair negatively the functionality of the airways and does not cause undesirable side effects.

Aerosolization devices suitable for administration of aerosol formulations described herein include, for example, jet, vibrating porous plate, ultrasonic nebulizers and energized dry powder inhalers, that are able to nebulize the formulation into aerosol particle size predominantly in the size range from 1-5µ. Predominantly in this application means that at least 70% but preferably more than 90% of all generated aerosol particles are within 1-5µ range. A jet nebulizer works by air pressure to break a liquid solution into aerosol droplets. Vibrating porous plate nebulizers work by using a sonic vacuum produced by a rapidly vibrating porous plate to extrude a solvent droplet through a porous plate. An ultrasonic nebulizer works by a piezoelectric crystal that shears a liquid into small aerosol droplets. A variety of suitable devices are available, including, for example, AeroNebTM and AeroDoseTM vibrating porous plate nebulizers (AeroGen, Inc., Sunnyvale, California), Sidestream^{®} nebulizers (Medic-Aid Ltd., West Sussex, England), Pari LC^{®} and Pari LC Star^{®} jet nebulizers (Pari Respiratory Equipment, Inc., Richmond, Virginia), and AerosonicTM (DeVilbiss Medizinische Produkte (Deutschland) GmbH, Heiden, Germany) and UltraAire^{®} (Omron Healthcare, Inc., Vernon Hills, Illinois) ultrasonic nebulizers.

In some embodiments, compounds described herein compound described herein (i.e., a compound of any of Formula (I), (Ia)-(If), (II), (IIa)-(IIe), (III), (IIIa)-(IIIc), (IV), (IVa)-(IVc), (V), or (Va)-(Vc)) are formulated for use as topical powders and sprays that contain, in addition to the compounds described herein, excipients such as lactose, talc, silicic acid, aluminum hydroxide, calcium silicates and polyamide powder, or mixtures of these substances. Sprays optionally contain customary propellants such as chlorofluorohydrocarbons.

Transdermal patches have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

According to the methods of treatment described herein, bacterial infections are treated or prevented in a patient such as a human or lower mammal by administering to the patient a therapeutically effective amount of a compound described herein, in such amounts and for such time as is necessary to achieve the desired result. By a "therapeutically effective amount" of a compound described herein is meant a sufficient amount of the compound to treat bacterial infections, at a reasonable benefit/risk ratio applicable to any medical treatment. It will be understood, however, that the total daily usage of the compounds and compositions described herein will be decided by the attending physician within the scope of sound medical judgment. The specific therapeutically effective dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors known in the medical arts.

The total daily dose of the compounds described herein compound described herein (i.e., a compound of any of Formula (I), (Ia)-(If), (II), (IIa)-(IIe), (III), (IIIa)-(IIIc), (IV), (IVa)-(IVc), (V), or (Va)-(Vc)) administered to a human or other mammal in single or in divided doses can be in amounts, for example, from 0.01 to 50 mg/kg body weight or more usually from 0.1 to 25 mg/kg body weight. Single dose compositions may contain such amounts or submultiples thereof to make up the daily dose. In general, treatment regimens described herein comprise administration to a patient in need of such treatment from about 10 mg to about 2000 mg of the compound(s) described herein per day in single or multiple doses.

### Examples

Compounds disclosed herein are made by the methods depicted in the reaction schemes shown below. Procedures are provided herein that, in combination with the knowledge of the synthetic organic chemist of ordinary skill in the art, are in some embodiments used to prepare the full range of compounds as disclosed and claimed herein.

The starting materials and reagents used in preparing these compounds are either available from commercial suppliers such as Aldrich Chemical Co., (Milwaukee, Wis.), Bachem (Torrance, Calif.), or Sigma (St. Louis, Mo.) or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991), March's Advanced Organic Chemistry, (John Wiley and Sons, 4th Edition) and Larock's Comprehensive Organic Transformations (VCH Publishers Inc., 1989). These schemes are merely illustrative of some methods by which the compounds disclosed herein are in some embodiments synthesized, and various modifications to these schemes can be made and will be suggested to one skilled in the art having referred to this disclosure. The starting materials and the intermediates, and the final products of the reaction may be isolated and purified if desired using conventional techniques, including but not limited to filtration, distillation, crystallization, chromatography and the like. Such materials may be characterized using conventional means, including physical constants and spectral data. Compounds are typically isolated as formic acid salts by reverse phase HPLC using AcCN/H₂0 with formic acid as an additive. In some instances, purifications are conducted without formic acid, and the compounds are isolated as the free base.

The methods of LCMS analysis are as follows:
LCMS (Method 5-95 AB, ESI): ESI, 5% AcCN/H₂O, 0.7 min; to 95% AcCN/H₂O, 0.4 min; 1.5 mL/min, Merck RP-18e, 2 × 25 mm.
LCMS (Method 10-80AB, 2 min, ESI): ESI, 10% AcCN/H₂O (0.04% TFA), 0.9 min to 80% AcCN/H₂O (0.04% TFA), then held for 0.6 min; 1.2 mL/min, Xtimate C18, 3 µm, 2.1 × 30 mm).
LCMS (Method 10-80AB, 7 min, ESI): ESI, 10% AcCN/H₂O (0.04% TFA), 6 min to 80% AcCN/H₂O (0.04% TFA), then held for 0.9 min; 0.8 mL/min, Xtimate C18, 3 µm, 2.1 × 30 mm).

### Example A: Synthesis of (S)-methyl 2-amino-3-(4-methoxy-3-(4,4,5,5-tetramethyl-1,3,2-dioxaborolan-2-yl)phenyl)propanoate

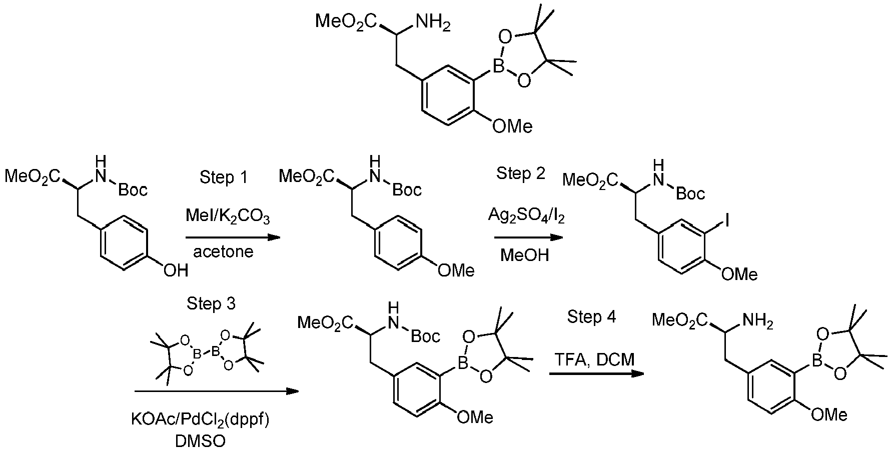

Step 1: To a solution of (S)-methyl 2-((tert-butoxycarbonyl)amino)-3-(4-hydroxyphenyl)propanoate (100 g, 0.323 mol) in acetone (2.0 L) was added K₂CO₃ (37 g, 0.34 mol). After the addition, MeI (32 mL, 0.97 mol) was added dropwise, and the reaction mixture was stirred at room temperature for 72 h and monitored by TLC. The reaction had not yet gone to completion, so NaOH (0.1 eq) was added to the reaction mixture. And after 2 h, the reaction was completed. The solid was filtered and the solvent was removed. The residue was taken up in ethyl acetate and washed with H₂O, extracted with ethyl acetate (300 mL × 3). The combined organic layers were washed with brine, dried over Na₂SO₄ and concentrated to give (S)-methyl 2-((tert-butoxycarbonyl)amino)-3-(4-methoxyphenyl)propanoate (100 g, 95.4%).

Step 2: To a solution of (S)-methyl 2-((tert-butoxycarbonyl)amino)-3-(4-methoxyphenyl)propanoate (80 g, 40 g each × 2, run in two separate batches, 259 mmol overall) in methanol (1.5 L in each of the two flasks) was added sequentially Ag₂SO₄ (85 g, 272 mmol, ½-added to each flask) and I₂ (72 g, 283 mmol, ½-added to each flask). The reaction mixture was stirred at room temperature for 2 h. The reaction was monitored by LCMS. When all (S)-methyl 2-((tert-butoxycarbonyl)amino)-3-(4-methoxyphenyl)propanoate had been consumed, then a solution of 10% (w/w) sodium thiosulfate was added until the reaction turned pale yellow. The solid was filtered and most of the methanol was evaporated by rotary evaporation. Water and ethyl acetate were added to each batch. The aqueous layer was extracted with ethyl acetate (3 × 200 mL). The combined organic layers were washed with brine, dried over sodium sulfate and concentrated. The crude material was combined for the two batches and they were purified together by flash column chromatography on silica gel (25% then 35% then 40% ethyl acetate in hexanes) to give (S)-methyl 2-((tert-butoxycarbonyl)amino)-3-(3-iodo-4-methoxyphenyl)propanoate (97 g, 89%).

Step 3: (S)-Methyl 2-((tert-butoxycarbonyl)amino)-3-(3-iodo-4-methoxyphenyl)propanoate (92 g, 46 g each run in two separate batches, 211 mmol) was dissolved in anhydrous DMSO (1.5 L, ½-added for each batch) under argon and to the solution was added bis(pinacolato) diboron (80.5 g, 317 mmol, ½-added for each batch) and KOAc (103 g, 1.05 mol, ½-added for each batch). This mixture was degassed with argon for twenty minutes, then Pd(dppf)Cl₂ (4.6 g, 6 mmol, ½-added for each batch) was added. The mixture was degassed with argon five times, then kept under argon and heated to 80°C for 3 h. TLC showed that the reaction was complete, and the reaction mixture was cooled to room temperature and filtered. The reaction mixture was dissolved in EA and washed with H₂O. The aqueous layer was extracted ethyl acetate (3 × 200 mL). The combined organic layers were dried over sodium sulfate, filtered and concentrated to give the crude product. The batches were then combined and purified together by flash column chromatography on silica gel (3% ethyl acetate in hexanes, then 20% to 25% ethyl acetate in hexanes to give (S)-methyl 2-((tert-butoxycarbonyl)amino)-3-(4-methoxy-3-(4,4,5,5-tetramethyl-1,3,2-dioxaborolan-2-yl)phenyl)propanoate (70 g, 76%).

Step 4: (S)-Methyl 2-((tert-butoxycarbonyl)amino)-3-(4-methoxy-3-(4,4,5,5-tetramethyl-1,3,2-dioxaborolan-2-yl)phenyl)propanoate (22 g, 50.6 mmol) was dissolved in dichloromethane (150 mL) and treated with trifluoroacetic acid (50 mL). The reaction mixture was stirred at room temperature and the reaction was monitored by HPLC. When all of the starting material had been consumed, the solvents were evaporated, DCM was added and Na₂CO₃ was added to neutralize the TFA. The mixture was filtered, and the solution was concentrated. DCM was added to the concentrated oil, and the mixture was cooled at 0°C for 1hr, whereupon the solid precipitates that formed were filtered. The filtrate was concentrated to give (S)-methyl 2-amino-3-(4-methoxy-3-(4,4,5,5-tetramethyl-1,3,2-dioxaborolan-2-yl)phenyl)propanoate. The material was used without further purification.

### Example B: Synthesis of (S)-2-((tert-butoxycarbonyl)amino)-2-(4-hydroxyphenyl)acetic acid

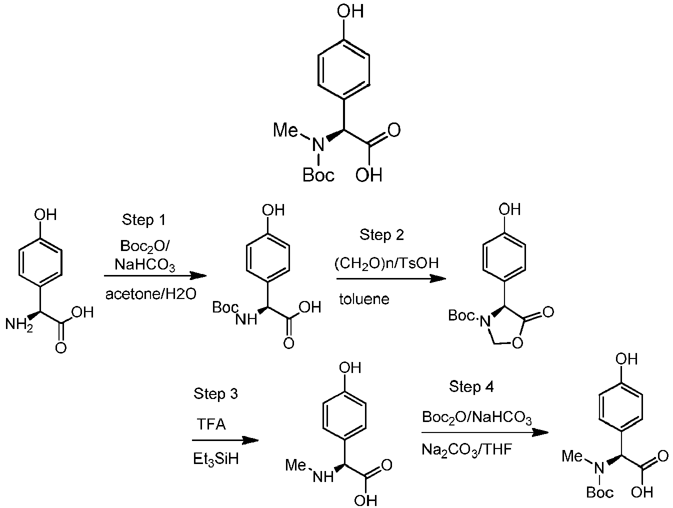

Step 1: To a stirred mixture of (S)-2-amino-2-(4-hydroxyphenyl)acetic acid (100 g, 0.6 mol, 1 eq) in a mixture of acetone (400 mL) and water (400 mL) was added di-*tert*-butyl dicarbonate (130.5 g, 0.6 mol, 1 eq) and NaHCO₃ (75.4 g, 0.9 mol, 1.5 eq). The mixture was allowed to stir at 25°C overnight. After HPLC showed the reaction was complete, the mixture was acidified with 5% citric acid (pH ~ 3). The mixture was filtered and the filter cake was washed with water, then dried to give (S)-2-((tert-butoxycarbonyl)amino)-2-(4-hydroxyphenyl)acetic acid (140 g, 87.5%). The crude product was used directly without further purification.

Step 2: To a solution of (S)-2-((tert-butoxycarbonyl)amino)-2-(4-hydroxyphenyl)acetic acid (45 g, 0.17 mol) in dry benzene (500 mL) was added paraformaldehyde (75.6 g, 0.84 mol, 5 eq) and *p-*toluenesulfonic acid (1.6 g, 8.5 mmol, 0.05 eq). A Dean-Stark apparatus with an attached condenser was then fit to the top of the flask and the mixture was heated at approximately 120°C until LC-MS showed the reaction was complete. The reaction was then cooled and the benzene was evaporated. The residue was taken up in ethyl acetate, washed with saturated NaHCO₃ (2 × 150 mL), then dried over sodium sulfate, and filtered. The solvent was removed to give (S)-tert-butyl 4-(4-hydroxyphenyl)-5-oxooxazolidine-3-carboxylate (36 g, 76.5%).

Step 3: (S)-tert-Butyl 4-(4-hydroxyphenyl)-5-oxooxazolidine-3-carboxylate (36 g, 0.13 mol, 1 eq) was dissolved in trifluoroacetic acid (75 mL) at 0°C then treated with triethylsilane (80 mL, 4 eq). The mixture was stirred at room temperature overnight. After LC-MS showed the reaction was complete, TFA was then evaporated to afford (S)-2-(4-hydroxyphenyl)-2-(methylamino)acetic acid, which was used without further purification.

Step 4: The resultant (S)-2-(4-hydroxyphenyl)-2-(methylamino)acetic acid was dissolved in water (85 mL), and to this solution was added solid NaHCO₃ until the pH reached 7. The solution was cooled to 0°C, then Na₂CO₃ was added until pH reached 9. A solution of di-*tert*-butyldicarbonate (28.3 g, 1.0 eq) in THF (75 mL) was added to the mixture. The mixture was allowed to warm to room temperature then stirred overnight. After HPLC showed the reaction was complete, THF was then evaporated. The aqueous solution was extracted 2x with hexanes and then acidified with citric acid to pH ~3-4. The acidified solution was then extracted with ethyl acetate (200 mL × 3). The combined organic layers were washed with brine, dried over sodium sulfate, filtered and concentrated to give (S)-2-((tert-butoxycarbonyl)(methyl)amino)-2-(4-hydroxyphenyl)acetic acid (35 g, 97% via 2 steps).

### Example C: Synthesis of Compound 101-B

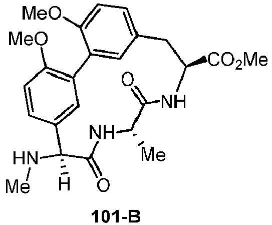

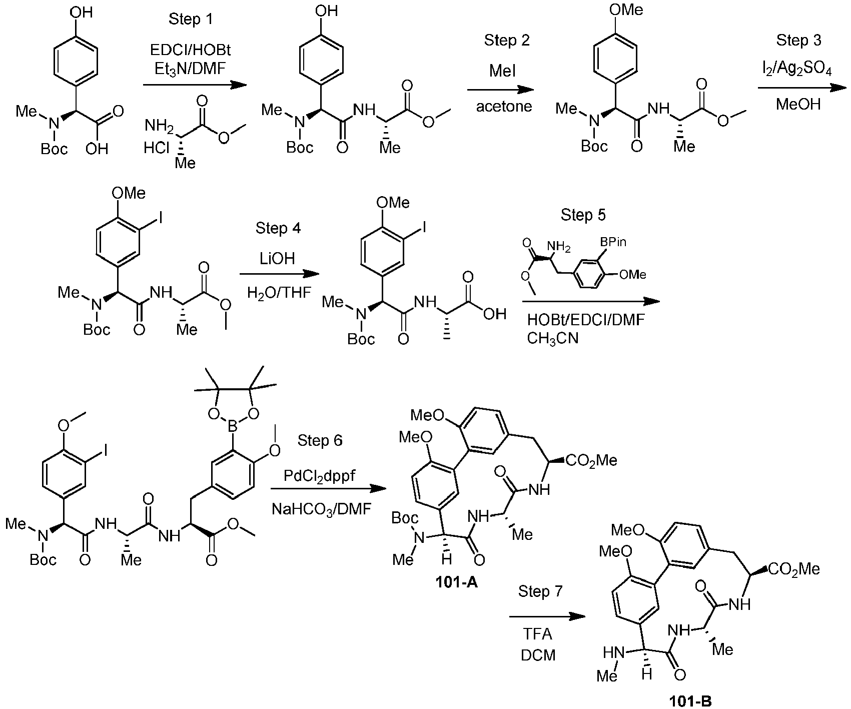

Step 1: To a solution of (S)-2-((tert-butoxycarbonyl)(methyl)amino)-2-(4-hydroxyphenyl)acetic acid (35 g, 0.12 mol) in DMF (300 mL) was added triethylamine (18.4 mL, 0.14 mol, 1.1 eq), HOBt (16.2 g, 0.12 mol, 1 eq), Ala-OMe HCl (19.5 g, 0.14 mol, 1.1 eq) and EDC (26.7 g, 0.14 mol, 1.1 eq) and the reaction was stirred overnight. After LC-MS showed the reaction was complete, water and EtOAc were added. The aqueous layer was extracted with EtOAc (3×150 mL), and the combined organic layers were washed with 5% citric acid (pH - 3), saturated NaHCO₃ (aq), water and brine. The combined organic layers were then dried over sodium sulfate, filtered and concentrated to give (S)-methyl 2-((S)-2-((tert-butoxycarbonyl)(methyl)amino)-2-(4-hydroxyphenyl)acetamido)propanoate (30 g, 65.8%) as a white foam. The crude product was taken on to the next step directly without further purification.

Step 2: To a solution of (S)-methyl 2-((S)-2-((tert-butoxycarbonyl)(methyl)amino)-2-(4-hydroxyphenyl)acetamido)propanoate (30 g, 82 mmol) in acetone (400 mL) was added K₂CO₃ (56.6 g, 0.41 mol, 5 eq) and iodomethane (20.8 mL, 0.41 mol, 5 eq) and the reaction was stirred at reflux overnight. After LC-MS showed the reaction was complete, the reaction was then cooled to room temperature and the mixture was filtered. The filtrate was concentrated and the residue was taken up in water and ethyl acetate. The aqueous phase was extracted with EtOAc (3 × 150 mL). The combined organic layers were dried over sodium sulfate, filtered and concentrated to give (S)-methyl 2-((S)-2-((tert-butoxycarbonyl)(methyl)amino)-2-(4-methoxyphenyl)acetamido)propanoate (28 g, 90%), as a white foam.

Step 3: To a solution of (S)-methyl 2-((S)-2-((tert-butoxycarbonyl)(methyl)amino)-2-(4-methoxyphenyl)acetamido)propanoate (85 g, 0.22 mol, 1 eq) in methanol (1000 mL) was added sequentially Ag₂SO₄ (72.6 g, 0.23 mol, 1.05 eq) and I₂ (59.6 g, 1.05 eq). After LC-MS showed the reaction was complete, a solution of 10% (w/w) sodium thiosulfate was added until the reaction turned pale yellow. Most of the methanol was evaporated by rotary evaporation and then water and ethyl acetate were added. The aqueous layer was extracted with ethyl acetate (3 × 300 mL). The combined organic layers were washed with brine, dried over sodium sulfate and concentrated to give (S)-methyl 2-((S)-2-((tert-butoxycarbonyl)(methyl)amino)-2-(3-iodo-4-methoxyphenyl)acetamido)propanoate (100 g, 88.5%).

Step 4: To (S)-methyl 2-((S)-2-((tert-butoxycarbonyl)(methyl)amino)-2-(3-iodo-4-methoxyphenyl)acetamido)propanoate (25 g, 49.4 mmol, 1 eq) in THF (300 mL) was added 0.2 M LiOH (500 mL, 98.8 mmol, 2 eq). The solution was stirred until TLC showed all starting material had been consumed. 5% citric acid (pH - 3) was added to pH - 3 and then the THF was evaporated by rotary evaporation. The aqueous layer was extracted with EtOAc (3 × 100 mL). The combined organic layers were washed with brine, dried over sodium sulfate, filtered and concentrated to give (S)-2-((S)-2-((tert-butoxycarbonyl)(methyl)amino)-2-(3-iodo-4-methoxyphenyl)acetamido)propanoic acid (23 g, 94.6%), which was used directly without further purification.

Step 5: To a solution of (S)-methyl 2-amino-3-(4-methoxy-3-(4,4,5,5-tetramethyl-1,3,2-dioxaborolan-2-yl)phenyl)propanoate (6.5 g, 19.4 mmol, 1 eq) and (S)-2-((S)-2-((tert-butoxycarbonyl)(methyl)amino)-2-(3-iodo-4-methoxyphenyl)acetamido)propanoic acid (10 g, 20.3 mmol, 1.05 eq) in acetonitrile:DMF (2.2:1, 168 mL) was added HOBt (6.5 g, 48.5 mmol, 2.5 eq) and EDC (8.1 g, 42.7 mmol, 2.2 eq). The reaction was stirred at room temperature overnight. After LC-MS showed the reaction was complete, diluted citric acid (pH- 3) was added and the aqueous was extracted with EtOAc (3 × 150 mL). The combined organic layers were then washed with saturated NaHCO₃ solution, brine and dried over sodium sulfate. The mixture was filtered and the filtrate was concentrated to give the crude product (6S,9S,12S)-methyl 6-(3-iodo-4-methoxyphenyl)-12-(4-methoxy-3-(4,4,5,5-tetramethyl-1,3,2-dioxaborolan-2-yl)benzyl)-2,2,5,9-tetramethyl-4,7,10-trioxo-3-oxa-5,8,11-triazatridecan-13-oate, which was used directly without further purification.

Step 6: (6S,9S,12S)-Methyl 6-(3-iodo-4-methoxyphenyl)-12-(4-methoxy-3-(4,4,5,5-tetramethyl-1,3,2-dioxaborolan-2-yl)benzyl)-2,2,5,9-tetramethyl-4,7,10-trioxo-3-oxa-5,8,11 -triazatridecan-13-oate (16 g, 19.4 mmol, 1 eq) and NaHCO₃ (16.3 g, 0.19 mol) were sealed in a flask with a condenser and put under an atmosphere of argon. DMF (600 mL) in a round bottle flask was purged several times via cycling with vacuum and Ar. PdCl₂(dppf) (3.3 g, 4.5 mmol) was then added to the DMF. The DMF solution was then degassed with Ar for 15 minutes. The solution of PdCl₂(dppf) dissolved in DMF was then transferred via syringe to the flask containing the substrate and NaHCO₃. The resulting mixture was submitted to several more cycles of vacuum and Ar then heated to 120°C overnight. After LCMS showed the reaction was completed, DMF was evaporated under vacuum. The crude material was subjected to abbreviated column chromatography (40% EA in PE) to remove most of the Pd species and then purified by prep HPLC to give Compound 101-A (2.1 g, 19.5% over two steps).

Step 7: To a stirred solution of Compound 101-A (2.1 g, 3.78 mmol) in DCM (25 mL) was added TFA (2 mL). The reaction was monitored via TLC and when starting material was consumed, the solvent was evaporated under vacuum. The residue was then dissolved in EtOAc and the organic layer was washed with saturated NaHCO₃ (10 mL), dried over sodium sulfate and concentrated to give Compound 101-B (1.7 g, 98.8%). MS (ESI) m/z 456.2 (M + H)⁺.

### Example D: Synthesis of Compound 101-G

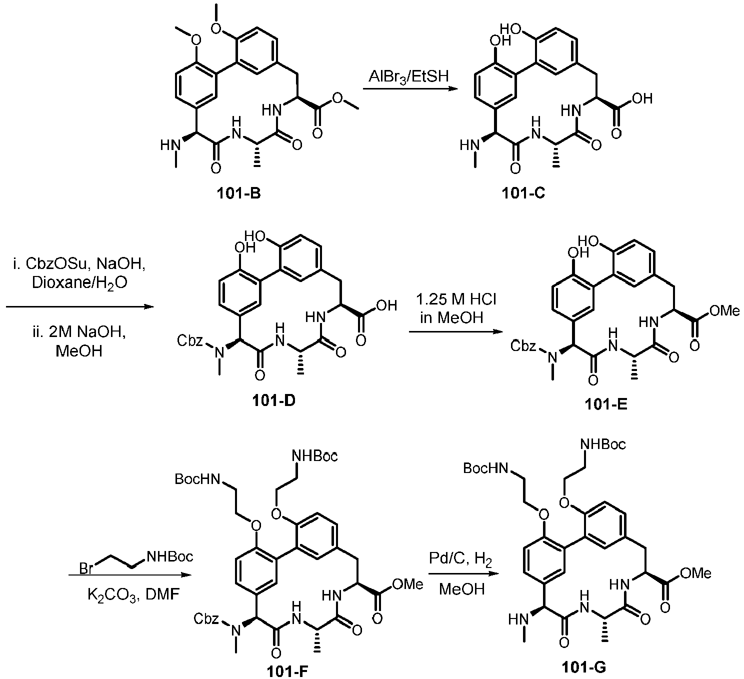

Step 1: To a solution of Compound 101-B (5.0 g, 11.0 mmol) in EtSH (116 mL, 1.61 mol), AlBr₃ (165 mL, 165 mmol) was added slowly at 0°C under N₂. The mixture was stirred for 18 h. The volatiles were removed under reduced pressure and the residue was quenched by water (50 mL), which was further washed by DCM (20 mL × 3). The aqueous layer was purified by prep-HPLC (acetonitrile 1-20% / 0.1% TFA in water) to give Compound 101-C (4.5g, 99.2% yield) as a white solid.

Step 2: To a solution of Compound 101-C (4.7 g, 8.9 mmol) in 1,4-dioxane/H₂O (9:1, 165 mL) was added 1 N NaOH dropwise until pH~11. A solution of Cbz-OSu (6.66 g, 26.7 mmol) dissolved in 1,4-dioxane (50 mL) was then added. After stirring for 1 h, NaOH (1.07 g, 26.7 mmol) was then added to the reaction followed by MeOH (60 mL). This resulting mixture was allowed to stir for 20 mins. To the reaction was then added dilute citric acid (10% v/v, 50 mL), the aqueous layer was extracted with EtOAc (3 × 150 mL) and the combined organic layers were washed with brine (3 × 100 mL), dried over Na₂SO₄ and concentrated to give the crude product. The residue was diluted with DCM (50 mL) and the suspension was filtered to give desired compound (3.2 g). The DCM phase was concentrated and the residue was purified by silica gel column (eluting 10-20% methanol in EtOAc) to give the desired compound (1.0 g). The combined batches gave Compound 101-D (4.2 g, 86.1% yield) as a white solid.

Step 3: To Compound 101-D (4.3 g, 7.85 mmol) was added a solution of 1.25M HCl in MeOH (128 mL) and the reaction was stirred at 0°C. The volatiles were removed to afford Compound 101-E (4.15 g, 94.1 % yield) as a white solid, which was used directly in the next step.

Step 4: To a solution of Compound 101-E (3.9 g, 6.94 mmol) and K₂CO₃ (14.4 g, 104 mmol) in DMF (50 mL) was added tert-butyl 2-bromoethylcarbamate (15.6 g, 69.5 mmol) at 0°C. The mixture was stirred at room temperature for 48 h. The mixture was filtered and the filtrate was diluted with EtOAc (500 mL). The EtOAc layer was washed with brine (2 × 400 mL), dried over Na₂SO₄, concentrated and purified by chromatography on silica (solvent gradient: 0-60% EtOAc in petroleum ether) to afford Compound 101-F (4.8 g, 81.5% yield) as a white solid.

Step 5: To a solution of Compound 101-F (4.8 g, 5.7 mmol) in MeOH (100 mL), 10% Pd/C (1.26 g, 1.18 mmol) on carbon was added at room temperature. The reaction mixture was stirred for 1 h at the same temperature under hydrogen atmosphere (15 psi). The filtrate was then concentrated to afford Compound 101-G (4.0 g, 99% yield) as a white solid.

### Example E: Synthesis of Compounds 101-1, 101-J, 101-K, and 101-L

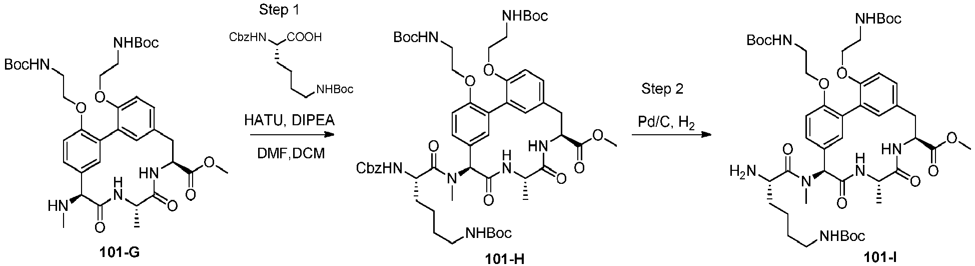

Step 1: To a solution of Compound 101-G (3.5 g, 4.9 mmol) and (S)-2-(((benzyloxy)carbonyl)amino)-6-((tert-butoxycarbonyl)amino)hexanoic acid (2.4 g, 6.4 mmol) in DCM (30 mL) at 0 °C, HATU (3.7 g, 9.8 mmol) and DIPEA (1.9 g, 14.7 mmol) was added. The resulting mixture was allowed to gradually warm up to room temperature and stirred for 2 h. The reaction mixture was diluted with DCM (100 mL), which was washed with brine (100 mL × 3). The organic layer was dried over Na₂SO₄, concentrated and the residue was purified by silica column chromatography to afford Compound 101-H (5.3 g, 99% yield) as a white solid.

Step 2: The hydrogenation step was performed using Example D using Compound 101-H (1.5 g, 1.4 mmol) to afford Compound 101-I (1.2 g, 93% yield) as a white solid. LCMS (Method 5-95 AB, ESI): t_{R} = 0.711, [M + H]⁺ = 942.6.

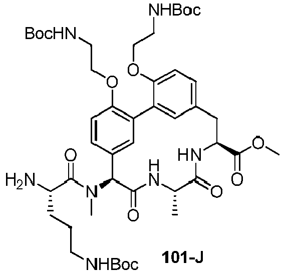

Compound 101-J was prepared from Compound 101-G and (S)-2-(((benzyloxy)carbonyl)amino)-5-((tert-butoxycarbonyl)amino)pentanoic acid as described above. LCMS (Method 5-95 AB, ESI): t_{R} = 0.841, [M + H]⁺ = 928.4.

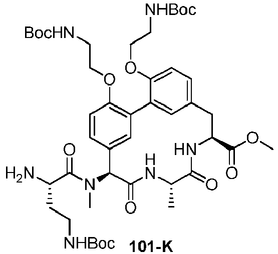

Compound 101-K was prepared from Compound 101G and (S)-2-(((benzyloxy)carbonyl)amino)-4-((tert-butoxycarbonyl)amino)butanoic acid as described above. LCMS (Method 5-95 AB, ESI): t_{R} = 0.838, [M + H]⁺ = 914.5.

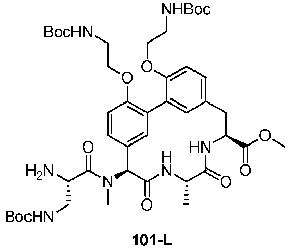

Compound 101-L was prepared from Compound 101G and (S)-2-(((benzyloxy)carbonyl)amino)-3-((tert-butoxycarbonyl)amino)propanoic acid as described above. LCMS (Method 5-95 AB, ESI): t_{R} = 0.833, [M + H]⁺ = 900.5.

### Example F: Synthesis of 3-((tert-butoxycarbonyl)(decyl)amino)propanoic acid

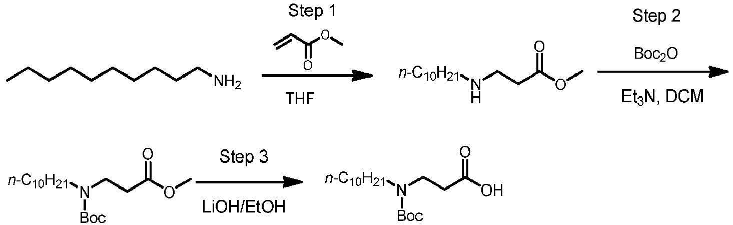

To a solution of methyl acrylate (2.2 g, 26 mmol) in THF (20 mL) was added a solution of decan-1-amine (6 g, 38 mmol) in THF (20 mL) at 0°C. The reaction mixture was stirred at 30°C for 48 h. The resulting solution was concentrated to obtain methyl 3-(decylamino)propanoate (6.4 g).

Step 2: To a solution of crude methyl 3-(decylamino)propanoate (6.4 g, 15 mmol) and Et₃N (4 g, 40 mmol) in DCM (30 mL) was added dropwise a solution of Boc₂O (5.7 g, 26 mmol) in DCM (20 mL) at 0°C. The reaction mixture was then allowed to warm to 30°C gradually and stirred for 18 h. After the reaction was completed, H₂O (50 mL) was added and the resulting aqueous layer was further extracted with DCM (50 mL*2). The combined organic layers were concentrated and the residue was purified by silica gel column (PE/EtOAc=50/1~20/1) to give methyl 3-((tert-butoxycarbonyl)(decyl)amino)propanoate (6.5 g, 73%) as a colorless oil.

Step 3: To a solution of methyl 3-((tert-butoxycarbonyl)(decyl)amino)propanoate (8.2 g, 23.9 mmol, crude) in EtOH (40 mL) was added a solution of LiOH (1.15 g, 48 mmol) in H₂O (20 mL) at 0°C. The reaction mixture was then allowed to warm to 30°C gradually and stirred for 18 h. After the reaction was complete, EtOH was removed under reduced pressure. The remaining aqueous solution was then adjusted to pH=2~3 with 6 N HCl, followed by the extraction with EtOAc (50 mL *3). The combined EtOAc layers were dried over Na₂SO₄, and concentrated to give 3-((tert-butoxycarbonyl)(decyl)amino)propanoic acid (7 g, 88.6%) as a colorless oil. ¹H NMR (400 MHz, CDCl₃) δ 3.47-3.43 (t, *J*=6.8Hz, 2H), 3.19-3.15 (t, *J*=7.2Hz, 2H), 2.61 (brs, 2H), 1.51-1.39 (m, 11H), 1.24-1.22 (m, 14H), 0.88-0.84 (t, *J*=6.8Hz, 3H).

### Example G: Synthesis of Compound 101

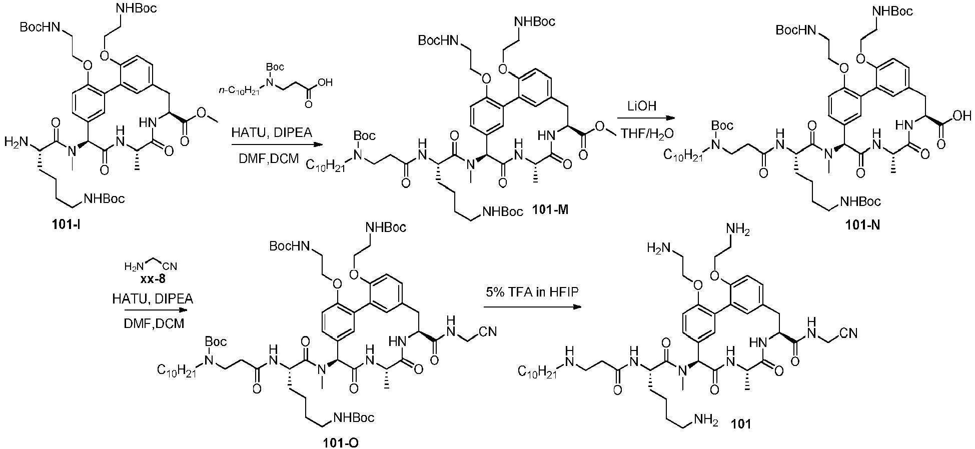

Step 1: Example E was applied to Compound 101-I (1.0 g, 1.27 mmol) and 3-((tert-butoxycarbonyl)(decyl)amino)propanoic acid (504 mg, 1.53 mmol) to afford Compound 101-M (1.3 g, 82% yield) as a white solid.

Step 2: To a solution of Compound 101-M (1.3 g, 1.04 mmol) in THF/H₂O (40 mL, 1:1) was added LiOH monohydrate (87 mg, 2.07 mmol) at 0°C. The mixture was allowed to gradually warm up to room temperature and stirred for 1 h. Most THF was removed under reduced pressure and the resulting mixture was adjusted pH=5 with saturated citric acid, which was further extracted by EtOAc (30 mL × 3). The combined organic layers were washed with brine (100 mL), dried over Na₂SO₄ and concentrated to give Compound 101-N (1.1 g, 86% yield) as a white solid.

Steps 3 and 4: To a solution of Compound 101-N (180 mg, 0.15 mmol), aminoacetonitrile hydrochloride (31 mg, 0.33 mmol) and DIPEA (38 mg, 0.29 mmol) in DCM/DMF (3 mL, 2:1) at 0°C, HATU (56 mg, 0.15 mmol) was added while stirring. The resulting mixture was stirred at room temperature for 1h. Most DCM was removed under reduced pressure and the residue was poured into water (10 mL), which was extracted with EtOAc (20 mL × 3). The combined organic layers were washed with brine (50 mL), dried over Na₂SO₄, concentrated to the residue, which was purified by flash chromatography column to afford Compound 101-O (140 mg, 76%) as a white solid.

Compound 101-O (130 mg, 0.10 mmol) was added to 5% TFA in HFIP (6.5 mL) and the mixture was stirred at room temperature for 2 h. Volatiles were removed under reduced pressure and the resulting crude was re-dissolved with DMF (5mL), which was neutralized with solid NaHCO₃. The filtrate was then purified by HPLC to afford Compound 101 (54 mg, 60% yield) as a white solid. LCMS (Method 5-95 AB, ESI): t_{R} = 0.710, M+H⁺ = 877.6; ¹HNMR (400 MHz, MeOH-*d*4) δ 8.51 (brs, 2H, HCOOH), 7.28-7.25 (m, 2H), 7.20 (d, *J*=8 Hz, 1H), 7.18 (d, *J*=8 Hz, 1H), 6.90 (brs, 1H), 6.84 (brs, 1H), 6.37 (s, 1H), 4.82-4.79 (m, 3H), 4.28-4.20 (m, 4H), 4.21 (s, 2H), 3.33-3.26 (m, 2H), 3.26-3.16 (m, 5H), 3.16-3.12 (m, 1H), 3.11-2.95 (m, 2H), 2.95-2.91 (m, 2H), 2.90 (s, 3H), 2.73-2.66 (m, 2H), 1.75-1.65 (m, 6H), 1.64-1.51 (m, 1H), 1.50-1.16 (m, 18H), 0.92 (t, *J*=6.8 Hz, 3H).

### Example H: Synthesis of 4'-(tert-butyl)-3-methyl-[1,1'-biphenyl]-4-carboxylic acid

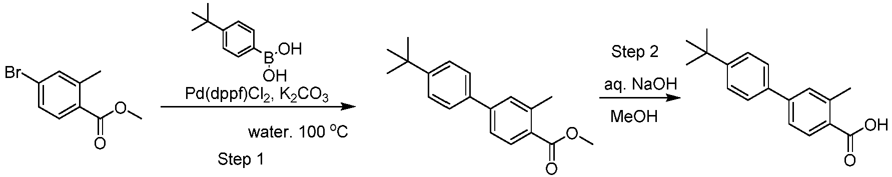

Step 1: To a solution of 4-t-butylbenzeneboronic acid (151.6 mg, 0.85 mmol) in 1,4-dioxane (5 mL) and water (1 mL) were added potassium carbonate (181.0 mg, 1.31 mmol), 1,1'-bis(diphenylphosphino)ferrocene palladium dichloride (47.9 mg, 0.07 mmol), and methyl 4-bromo-2-methylbenzoate (150.0 mg, 0.65 mmol). The reaction mixture was stirred at 100 °C for 2 h under N₂ and concentrated. The residue was taken up in EtOAc (20 mL) and washed with water (20 mL × 2) and brine (20 mL). The organic layer was dried over MgSO₄ and concentrated. The residue was purified by flash column chromatography (5% EtOAc in petroleum ether, Rf = 0.7) to afford methyl 4-(4-tert-butylphenyl)-2-methyl-benzoate (120 mg, 64.9% yield) as a colorless oil. LCMS (5-95AB_1.5min): t_{R} = 0.972 min, [M + H]⁺ = 281.9.

Step 2: Methyl 4-(4-tert-butylphenyl)-2-methyl-benzoate (120.0 mg, 0.430 mmol) was hydrolyzed to give 4'-(tert-butyl)-3-methyl-[1,1'-biphenyl]-4-carboxylic acid (100 mg, 0.3726 mmol, 87.7% yield) as a white solid.

### Example I: Synthesis of methyl 4-(5-isobutylpyrazin-2-yl)-2-methylbenzoate

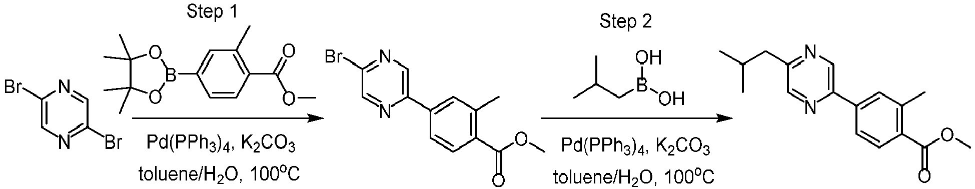

To a solution of 2,5-dibromopyrazine (200.0 mg, 0.84 mmol) in toluene (5 mL) and water (1 mL) were added potassium carbonate (348.6 mg, 2.52 mmol), methyl 2-methyl-4-(4,4,5,5-tetramethyl-1,3,2-dioxaborolan-2-yl)benzoate (232.2 mg, 0.84 mmol) and tetrakis(triphenylphosphine)palladium(0) (97.2 mg, 0.08 mmol). The reaction mixture was stirred at 80 °C for 16 h and filtered. The filtrate was diluted with H₂O (20 mL) and extracted with EtOAc (40 mL × 2). The combined organic layers were washed with water (80 mL × 3) and brine (80 mL), dried over Na₂SO₄ and concentrated. The residue was purified by prep-TLC (7.5% EtOAc in petroleum ether) to obtain methyl 4-(5-bromopyrazin-2-yl)-2-methylbenzoate (150 mg, 58.1% yield) as a white solid. ¹H NMR (400 MHz, CDCl₃): δ 8.81 (s, 1H), 8.75 (d, J = 1.2 Hz, 1H), 8.04 (d, J = 8.4 Hz, 1H), 7.88 (s, 1H), 7.85 (d, J = 8.4 Hz, 1H), 3.93 (s, 3H), 2.70 (s, 3H).

To a solution of isobutylboronic acid (99.6 mg, 0.98 mmol) in toluene (3 mL) and water (0.3 mL) were added tetrakis(triphenylphosphine)palladium(0) (56.4 mg, 0.05 mmol), potassium carbonate (202.5 mg, 1.47 mmol) and methyl 4-(5-bromopyrazin-2-yl)-2-methylbenzoate (150.0 mg, 0.49 mmol). The reaction mixture was stirred at 100 °C for 16 h and filtered. The filtrate was diluted with H₂O (20 mL) and extracted with EtOAc (40 mL × 2). The combined organic layers were washed with water (80 mL × 3) and brine (80 mL), dried over Na₂SO₄ and concentrated. The residue was purified by prep-TLC (9.5% EtOAc in petroleum ether, Rf = 0.4) to obtain methyl 4-(5-isobutylpyrazin-2-yl)-2-methylbenzoate (52 mg, 37.4% yield) as a yellow oil. LCMS (Method 5-95 AB, ESI): t_{R} = 0.956 min, [M+H]⁺ = 284.9.

### Example J: Synthesis of ethyl 2-bromo-4-methylpyrimidine-5-carboxylate

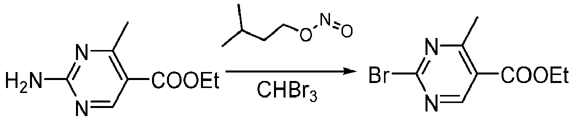

To a solution of ethyl 2-amino-4-methylpyrimidine-5-carboxylate (4.0 g, 22 mmol) in CHBr₃ (66 mL) was added isopentyl nitrite (44 mL) and the mixture was stirred at 85°C for 4 h. The volatiles were removed and the residue was taken up by EtOAc (100 mL), which was washed by brine (100 mL × 2). The organic layer was dried over Na₂SO₄, concentrated and the residue was purified by silica gel flash column to give ethyl 2-bromo-4-methylpyrimidine-5-carboxylate (3.0 g, 55.5% yield) as a white solid. ¹H NMR (400 MHz, CDCl₃): δ 8.93 (s, 1H), 4.41 (q, *J* = 7.2 Hz, 2H), 2.82 (s, 3H), 1.41 (t, *J* = 7.0 Hz, 3H).

### Example K: Synthesis of 2-fluoro-4-octylbenzoic acid

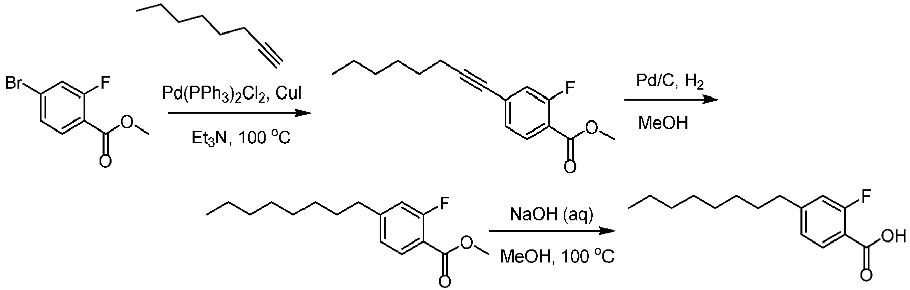

A mixture of methyl 4-bromo-2-fluorobenzoate (500.0 mg, 2.15 mmol), oct-1-yne (702.9 mg, 6.44 mmol), bis(triphenylphosphine)palladium(II)dichloride (75.3 mg, 0.11 mmol) and copper(I) iodide (20.4 mg, 0.11 mmol) in triethylamine (9.83 mL, 70.9 mmol) was stirred at 100 °C for 2 h under nitrogen atmosphere. LCMS (5-95AB/1.5min): t_{R} = 1.006 min, [M + H]⁺ 262.9 showed 60% of DP. The reaction was quenched with water (15 mL) and extracted with dichloromethane (3 × 25 mL). The combined organic extracts were washed with brine (2 × 25 mL), dried over anhydrous Na₂SO₄ and filtered. The filtrate was concentrated and the residue was purified by column chromatography on silica gel (eluting with 5% ethyl acetate in petroleum ether, Rf = 0.5) to afford methyl 2-fluoro-4-(oct-1-yn-1-yl)benzoate (550 mg, 97.7% yield) as a brown solid. LCMS (5-95AB_1.5 min): t_{R} = 1.006 min, [M + H]⁺ 262.9.

To a solution of methyl 2-fluoro-4-(oct-1-yn-1-yl)benzoate (550.0 mg, 2.1 mmol) in methanol (25 mL) was added 10% Palladium on carbon (111.56 mg, 0.10 mmol). The mixture was stirred at 30 °C under hydrogen (40 psi) for 16h. The reaction was filtered over a pad of Celite and concentrated. The residue was purified by column chromatography on silica gel (eluting with petroleum ether / ethyl acetate from 100:1 to 10:1) to afford methyl 2-fluoro-4-octylbenzoate (500 mg, 89.5% yield) as a yellow solid. LCMS (5-95AB_1.5min): t_{R} = 1.033 min, [M + H]⁺ 266.

To a solution of methyl 2-fluoro-4-octylbenzoate (500.0 mg, 1.88 mmol) in methanol (5 mL) was added NaOH (1000.0 mg, 25 mmol) in water (5 mL). The mixture was stirred at 100 °C for 2h, cooled to RT and hydrochloric acid (1.0 M) was added until pH = 3-4. The mixture was extracted with ethyl acetate (3 × 30 mL). The combined organic extracts were washed with brine (2 × 30 mL), dried over sodium sulfate and filtered. The filtrate was concentrated to give 2-fluoro-4-octylbenzoic acid (450 mg, 95% yield) as a yellow solid. ¹H NMR (400 MHz, CDCl₃): δ 7.94 (t, *J* = 8.0 Hz, 1H), 7.06 (d, *J* = 8.4 Hz, 1H), 6.99 (d, *J* = 12.0 Hz, 1H), 2.66 (t, *J* = 7.4 Hz, 2H), 1.65 - 1.62 (m, 2H), 1.31 - 1.28 (m, 10H), 0.89 (t, *J* = 6.8 Hz, 3H).

### Example L: Synthesis of 4-(tert-butyl)-2-(difluoromethyl)benzoic acid

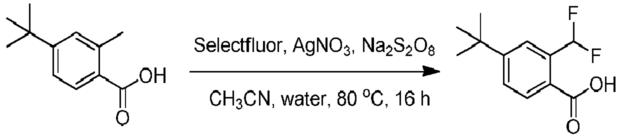

To a degassed mixture of 4-tert-butyl-2-methyl-benzoic acid (192.0 mg, 1 mmol), sodium persulfate (1.19 g, 4.99 mmol) and Selectfluor (1.77 g, 4.99 mmol) in acetonitrile (4 mL) and water (4 mL) in dry-ice acetone bath was added silvernitrate (17.0 mg, 0.10 mmol). The mixture was degassed by three freeze-pump-thaw cycles and heated at 80 °C for 16 h. Water (10 mL) was added and the mixture was extracted with EtOAc (15 mL × 2). The combined organic layers were concentrated and the residue was purified by prep-TLC (petroleum ether/EtOAc/HOAc 2/1/0.01, Rf = 0.3) to give 4-(tert-butyl)-2-(difluoromethyl)benzoic acid (75 mg, 32.9% yield) as a white solid.

### Example M: Synthesis of 3-butyl-2-methylbenzoic acid

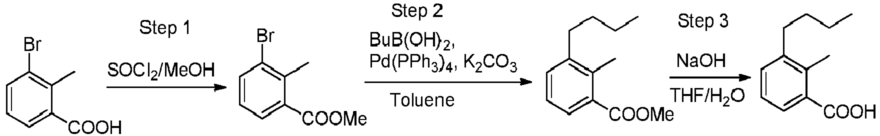

Step 1: To a mixture of 3-bromo-2-methylbenzoic acid (5.0 g, 23 mmol) in MeOH (80 mL) was added SOCl₂ (11.0 g, 93 mmol) at 20°C. The mixture was stirred for 1.5h at 70°C. The volatiles were removed and the residue was taken up by EtOAc (100 mL), which was washed sequentially with saturated NaHCO₃ and brine (each 100 mL). The EtOAc layer was dried over Na₂SO₄, concentrated and the residue was purified by flash column chromatography to give methyl 3-bromo-2-methylbenzoate (5.3g, 99% yield) as a red solid.

Step 2: A solution of methyl 3-bromo-2-methylbenzoate (500 mg, 2.2 mmol), n-butyl boronic acid (890 mg, 8.7 mmol), Pd(PPh₃)₄ (252 mg, 0.22 mmol) and K₂CO₃ (905 mg, 6.6 mmol) in toluene (20 mL) was stirred at 100°C for 4 h. After filtration, the filtrate was washed with brine (20 mL × 3), dried over Na₂SO₄ and concentrated. The residue was purified by HPLC to give methyl 3-butyl-2-methylbenzoate (120 mg, 27% yield) as colorless oil. LCMS (Method 5-95 AB, ESI): t_{R} = 0.871, [M + H]⁺ = 206.9.

Step 3: The ester hydrolysis method with NaOH as previously described (Example H) was applied to methyl 3-butyl-2-methylbenzoate (120 mg, 0.58 mmol) to afford 3-butyl-2-methylbenzoic acid (110 mg, 98% yield) as a white solid.

### Example N: Synthesis of methyl 2-[(E)-3-tert-butoxy-3-oxo-prop-1-enyl]-4-octyl-benzoate

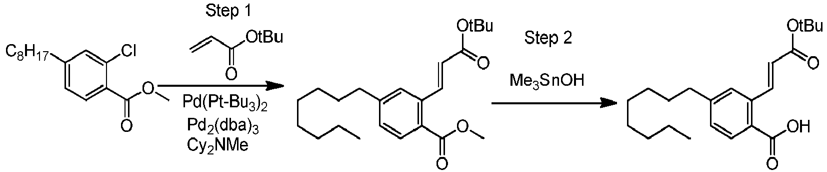

Step 1: To a solution of methyl 2-chloro-4-octyl-benzoate (350.0 mg, 1.24 mmol) in 1,4-dioxane (2 mL) were added t-butyl acrylate (174.5 mg, 1.36 mmol), bis(tri-t-butylphosphine)palladium(0) (9.5 mg, 0.02 mmol), tris(dibenzylideneacetone)dipalladium(0) (34.0 mg, 0.04 mmol) and N,N-dicyclohexylmethylamine (265.9 mg, 1.36 mmol). The mixture was stirred at 25 °C for 16 h under N₂ and filtered. The filtrate was diluted with water (10 mL) and extracted with EtOAc (10 mL × 3). The combined organic layers were dried over Na₂SO₄, filtered and concentrated. The residue was purified by prep-TLC (10% EtOAc in petroleum ether, Rf = 0.7) to afford methyl 2-[(E)-3-tert-butoxy-3-oxo-prop-1-enyl]-4-octyl-benzoate (290 mg, 62.6% yield) as a yellow oil. ¹H NMR (400 MHz, CDCl₃): δ 8.35 (d, J = 16.0 Hz, 1H), 7.86 (d, J = 8.0 Hz, 1H), 7.39 (s, 1H), 7.22 (d, J = 8.0 Hz, 1H), 6.23 (d, J = 15.9 Hz, 1H), 3.91 (s, 3H), 2.64 (t, J = 7.8 Hz, 2H), 1.65-1.55 (m, 2H), 1.54 (s, 9H), 1.33 - 1.22 (m, 10H), 0.88 (t, J = 6.6 Hz, 3H).

Step 2: To a solution of methyl 2-[(E)-3-tert-butoxy-3-oxo-prop-1-enyl]-4-octyl-benzoate (290.0 mg, 0.77 mmol) in 1,2-dichloroethane (2 mL) was added trimethyltin hydroxide (1400.2 mg, 7.74 mmol) and the mixture was stirred at 80 °C for 16 h. LCMS (5-95AB/1.5min): t_{R} = 1.158 min, [M+Na]+383.1 showed 36% of DP and 51% of SM. The mixture was diluted with 0.1N KHSO₄ (5 mL) and extracted with EtOAc (10 mL × 3). The combined organic layers were washed with brine, dried over Na₂SO₄, filtered and concentrated. The residue was purified by prep-TLC (50% EtOAc in petroleum ether, Rf = 0.2) to afford 2-[(E)-3-tert-butoxy-3-oxo-prop-1-enyl]-4-octyl-benzoic acid (80 mg, 28.7% yield) as a yellow solid.

### Example O: Synthesis of Compound 102

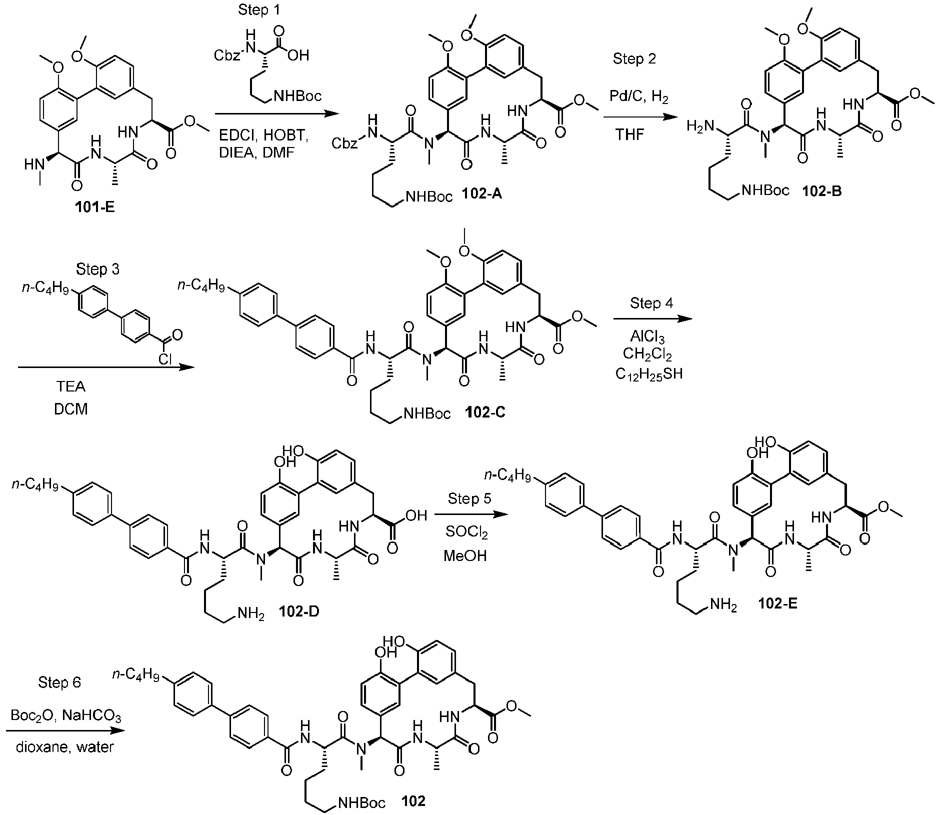

Step 1: To a solution of Compound 101-E (800 mg, 1.76 mmol) in DMF (15 mL) was added (S)-2-(((benzyloxy)carbonyl)amino)-6-((tert-butoxycarbonyl)amino)hexanoic acid (735 mg, 1.93 mmol), 3-[(E)-ethylazo]-N,N-dimethyl-propan-1-amine hydrochloride (946.77 mg, 5.27 mmol), 1-hydroxybenzotriazole (711.94 mg, 5.27 mmol), and N,N-diisopropylethylamine (681 mg, 5.27 mmol). The mixture was stirred at 30 °C for 16 h. TLC showed the start material was consumed (50% ethyl acetate in petroleum ether, R_{f} = 0.5). The mixture was poured into water (30 mL). The precipitate was filtered, washed with water, re-dissolved in methanol, and concentrated to give Compound 102-A (1200 mg, 1.45 mmol, 83.5% yield) as a yellow solid.

Step 2: To a solution of Compound 102-A (1200 mg, 1.47 mmol) in methanol (15 mL) was added Pd/C (200.0 mg, 1.47 mmol), and the mixture was stirred at 30°C under hydrogen (50 psi) for 16 h. The catalyst was filtered off and the filtrate was concentrated to give Compound 102-B (900 mg, 1.12 mmol, 81.6% yield) as a white solid. LCMS (5-95AB_1.5min_1500): t_{R} = 0.782 min, [M + H]⁺ 684.4.

Step 3: A mixture of 4-(4-butylphenyl)benzoic acid (200 mg, 0.79 mmol) in thionyl chloride (5.0 mL) was stirred at 60 °C for 16 h. The solution was concentrated and dissolved in dichloromethane (2 mL). To the solution of Compound 102-B (500 mg, 0.73 mmol) and triethylamine (74 mg, 0.73 mmol) in dichloromethane (15 mL) was added the above solution of 4-(4-butylphenyl)benzoyl chloride in dichloromethane. The reaction mixture was stirred at 25 °C for 3 h. LCMS showed that all of start material was consumed completely. TLC (10% dichloromethane in methanol, Rf = 0.4). The reaction was concentrated to dryness and the residue was purified by flash column chromatography (eluted with 5% dichloromethane in methanol). The desired fractions were concentrated to afford Compound 102-C (650 mg, 0.71 mmol, 96.6% yield) as a white solid. LCMS (5-95AB/1.5 min): t_{R} = 0.951 min, [M + H]⁺ 921.4. Alternatively, this coupling reaction was performed using 4'-butyl-[1,1'-biphenyl]-4-carboxylic acid using conditions in Example 4.

Step 4: A mixture of aluminium chloride (2.8 g, 21.19 mmol) and 1-dodecanethiol (4.3 g, 21.19 mmol) in dichloromethane (12 mL) was stirred at 26 °C for 5 min, and then cooled to 0 °C. Then Compound 102-C (650 mg, 0.71 mmol) was added slowly. The solution was stirred at 26 °C for 2 h. LCMS showed that all of start material was consumed completely. The solution was quenched by 1N hydrochloride acid, and filtered. The filter cake was dried to afford crude Compound 102-D as a white solid. LCMS (5-95AB/1.5 min): t_{R} = 0.828 min, [M + H]⁺ =778.4.

Step 5: A solution of Compound 331-D (500 mg, 0.64 mmol) and thionyl chloride (229 mg, 1.93 mmol) in methanol (10 mL) was stirred at 60 °C for 1 h. LCMS showed that all of start material was consumed completely. The solution was concentrated to afford Compound 102-E (500 mg, 0.63 mmol, 98.2% yield) as a yellow solid. LCMS (5-95AB/1.5 min): t_{R} = 0.856 min, [M + H]⁺ = 792.8.

Step 6: To the solution of Compound 102-E (500 mg, 0.63 mmol) and sodium bicarbonate (10.6 mg, 0.13 mmol) in 1,4-dioxane (6 mL) and water (2 mL) was added di-tert-butyl dicarbonate (138 mg, 0.63 mmol). LCMS showed that all of start material was consumed completely. TLC (5% dichloromethane in methanol, Rf = 0.2). The reaction was concentrated to dryness and the residue was taken up in ethyl acetate (50 mL). It was washed with water (20 mL × 2) and brine (10 mL), dried (sodium sulfate) and concentrated. The crude was purified by flash column chromatography (eluted with 5% dichloromethane in methanol). The desired fractions were concentrated in vacuo afford Compound 102-F (500 mg, 0.56 mmol, 88.8% yield) as a white solid. LCMS (5-95AB/1.5 min): t_{R} = 1.048 min, [M + H]⁺ = 892.4.

### Example P: Synthesis of Compound 103

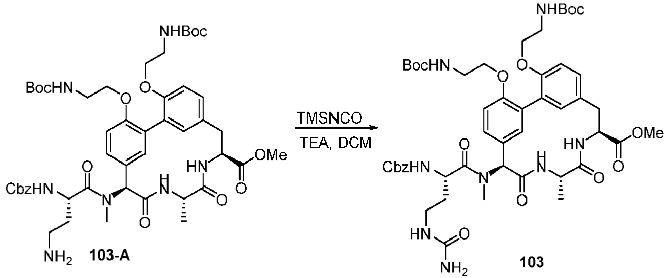

To a solution of Compound 103-A (120 mg, 0.13 mmol) and Et₃N (53 µL, 0.38 mmol) in DCM (5 mL) was added trimethylsilyl isocyanate (44 mg, 0.38 mmol) at 0°C. The resulting mixture was warmed up to room temperature while stirring and stirred at the same temperature for 2 h. The volatiles were removed and the residue was purified by prep-TLC to afford Compound 103 (90 mg, 72% yield) as a white solid. LCMS (Method 5-95 AB, ESI): RT = 0.759, [M + H]⁺ = 991.7.

### Example Q: Synthesis of (S)-2-(((benzyloxy)carbonyl)amino)-4-(N-methyl-2-nitrophenylsulfonamido)butanoic acid

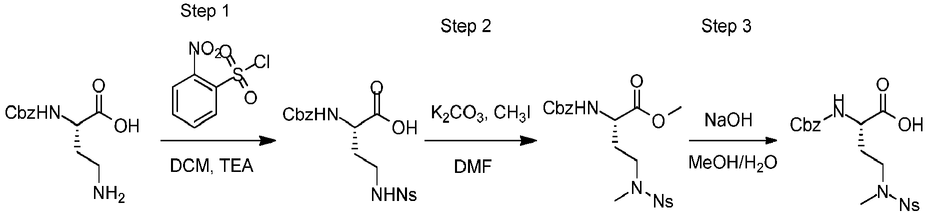

Step 1: To a solution of (S)-4-amino-2-(((benzyloxy)carbonyl)amino)butanoic acid (2000.0 mg, 7.93 mmol) in N,N-dimethylformamide (50 mL) were added 2-nitrobenzenesulfonylchloride (0.46 mL, 23.78 mmol) and triethylamine (4.42 mL, 31.71 mmol) dropwise. The reaction mixture was stirred at 25 °C for 12 h and filtered. To the filtrate was added water (50 mL) and the resulting precipitate was collected to obtain (S)-2-(((benzyloxy)carbonyl)amino)-4-(2-nitrophenylsulfonamido)butanoic acid (2000 mg, 4.5723 mmol, 57.7% yield) as a white solid. LCMS (Method 5-95 AB, ESI): RT = 0.790 min, [M + H]⁺ = 437.0.

Step 2: To a solution of (S)-2-(((benzyloxy)carbonyl)amino)-4-(2-nitrophenylsulfonamido)butanoic acid (800.0 mg, 1.83 mmol) in N,N-dimethylformamide (5 mL) were added iodomethane (4.59 mL, 73.43 mmol) and potassium carbonate (758.3 mg, 5.49 mmol). The mixture was stirred at 25°C for 14 h, diluted with H₂O (20 mL) and extracted with EtOAc (35 mL × 3). The combined organic layers were washed with water (30 mL ×4) and brine (40 mL), dried over Na₂SO₄ and concentrated. The residue was purified by flash column chromatography (30% EtOAc in petroleum ether, Rf = 0.3) to obtain (S)-methyl 2-(((benzyloxy)carbonyl)amino)-4-(N-methyl-2-nitrophenylsulfonamido)butanoate (610 mg, 71.7% yield) as a yellow oil. LCMS (Method 5-95 AB, ESI): RT = 0.813 min, [M + H]⁺ = 466.1.

Step 3: NaOH hydrolysis of the ester afforded (S)-2-(((benzyloxy)carbonyl)amino)-4-(N-methyl-2-nitrophenylsulfonamido)butanoic acid.

### Example R: Synthesis of (S)-2-(((benzyloxy)carbonyl)amino)-4-((tertbutyldimethylsilyl)oxy)butanoic acid

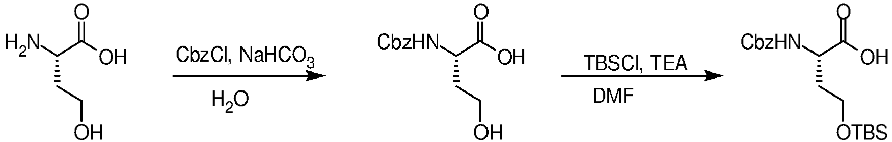

To a mixture of benzyl chloroformate (930.84 mg, 5.46 mmol) and sodium bicarbonate (705.25 mg, 8.39 mmol) in water (10 mL) was added (2S)-2-amino-4-hydroxy-butanoic acid (500.0 mg, 4.2 mmol), and stirred at 15 °C for 3 hours under nitrogen. The reaction mixture was washed with ethyl acetate (20 mL × 3), acidified to pH 4 using 2N HCl (about 20 mL) at 0°C, and extracted with ethyl acetate (30 mL × 3). The combined organic layers were dried over sodium sulfate and concentrated to afford (2S)-2-(benzyloxycarbonylamino)-4-hydroxy-butanoic acid (450 mg, 1.7769 mmol, 42.3% yield) as a colorless oil. It was used in the next step without further purification.

To a mixture of (2S)-2-(benzyloxycarbonylamino)-4-hydroxy-butanoic acid (450.0 mg, 1.78 mmol) and triethylamine (395.57 mg, 3.91 mmol) in N,N-dimethylformamide (8 mL) was added tert-butyldimethylchlorosilane (401.72 mg, 2.67 mmol) at 0°C and stirred at 15 °C for 1 hour. The reaction mixture was diluted with water (30 mL) and sodium carbonate (5 g) was added. The resulting mixture was washed with ethyl acetate (15 mL × 3). The aqueous phase was acidified to pH 4 using 2 N HCl (about 20 mL) at 0°C and extracted with ethyl acetate (30 mL × 3). The combined organic layers were dried over sodium sulfate and concentrated to afford the (S)-2-(((benzyloxy)carbonyl)amino)-4-((tertbutyldimethylsilyl)oxy)butanoic acid (450 mg, 1.2245 mmol, 68.9% yield) as a colorless oil. It was used in the next step without further purification. LCMS (Method 5-95 AB, ESI): t_{R} = 0.833 min, [M+Na]⁺ = 389.9.

### Example S: Synthesis of (S)-2-decanamidopentanoic acid

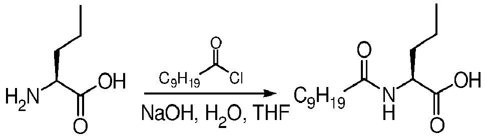

To a stirred solution of decanoyl chloride (500 mg, 2.6 mmol) in THF (5 mL) was added (S)-2-aminopentanoic acid (461 mg, 3.9 mmol) and 2N NaOH (5.0 mL) at 0°C and the resulting mixture was stirred at 0°C for 1 h. The pH of the mixture was adjusted to pH=2 using 1N HCl, which was extracted with EtOAc (20 mL × 3). The combined organic layers were washed with brine (50 mL × 2), dried over Na₂SO₄ and concentrated to afford (S)-2-decanamidopentanoic acid (630 mg, 88.5% yield) as a white solid, which was used directly in the next step. LCMS (5-95 AB, ESI): t_{R} = 0.904, [M + H]⁺ = 272.0.

### Example T: Synthesis of Compound 104

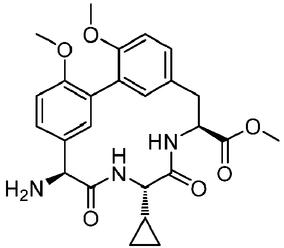

Compound 104 was synthesized following procedures analogous to those described for Example C (Compound 101-B), in which (S)-2-((tert-butoxycarbonyl)amino)-2-(4-hydroxyphenyl)acetic acid and methyl (S)-2-amino-2-cyclopropylacetate were used in Step 1, to afford the title compound as brown solid. LCMS (ESI): [M+H]⁺ = 354.

### Example U: Synthesis of Compound 105

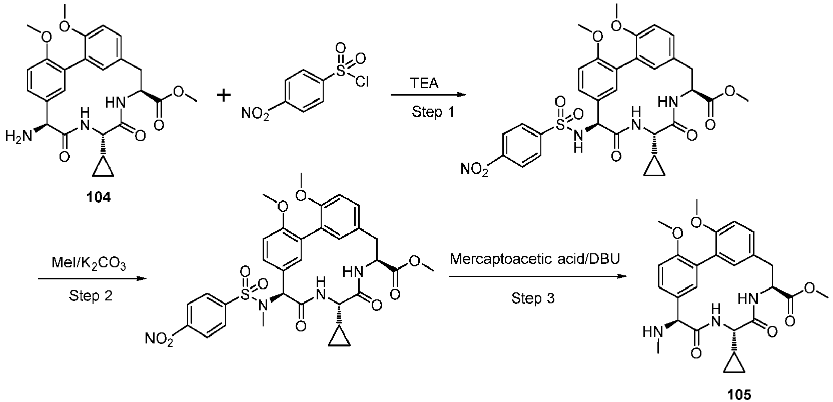

Step 1: To a solution of Compound **104** (1.16 g, 2.48 mmol) and triethylamine (0.86 mL, 6.20 mmol) in acetonitrile (25 mL) was added 4-nitrobenzenesulfonyl chloride (660 mg, 2.98 mmol) in portions, and the resulting reaction mixture was stirred at room temperature for 4 h. The precipitate was collected by filtration, washed with small amount of acetonitrile, and dried under vacuum overnight to give 1.14 g (70%) of methyl (4*S*,7*S*,10*S*)-7-cyclopropyl-1⁶,2⁶-dimethoxy-10-((4-nitrophenyl)sulfonamido)-6,9-dioxo-5,8-diaza-1,2(1,3)-dibenzenacyclodecaphane-4-carboxylate as an off white solid, which was carried forward without further purification. LCMS (ESI): [M+H]⁺ = 653.

Step 2: To a mixture of (4*S*,7*S*,10*S*)-7-cyclopropyl-1⁶,2⁶-dimethoxy-10-((4-nitrophenyl)sulfonamido)-6,9-dioxo-5,8-diaza-1,2(1,3)-dibenzenacyclodecaphane-4-carboxylate (1.14 g, 1.75 mmol) and K₂CO₃ (1.93 g, 14.0 mmol,) in acetone (20 mL) was added iodomethane (0.870 mL, 14.0 mmol). The resulting reaction mixture was stirred at room temperature overnight. The mixture was filtered and evaporated *in vacuo.* The residue was diluted with water, extracted with isopropyl acetate (2 × 100 ml), dried over Mg₂SO₄, filtered, evaporated *in vacuo,* and dried under vacuum to give 1.21 g (100%) of methyl (4*S*,7*S*,10*S*)-7-cyclopropyl-1⁶,2⁶-dimethoxy-10-((*N*-methyl-4-nitrophenyl)sulfonamido)-6,9-dioxo-5,8-diaza-1,2(1,3)-dibenzenacyclodecaphane-4-carboxylate as off white solid, which was carried forward without purification. LCMS (ESI): [M+H]⁺ = 667.

Step 3: To a solution methyl (4*S*,7*S*,10*S*)-7-cyclopropyl-1⁶,2⁶-dimethoxy-10-((*N*-methyl-4-nitrophenyl)sulfonamido)-6,9-dioxo-5,8-diaza-1,2(1,3)-dibenzenacyclodecaphane-4-carboxylate (1.11 g, 1.66 mmol) in acetonitrile (22 mL) was added mercaptoacetic acid (6.6 equiv., 1.01 g, 11.0 mmol) and DBU (2.50 mL, 16.6 mmol), and the resulting mixture stirred at room temperature for 3 h. The reaction mixture was evaporated *in vacuo,* diluted with isopropyl acetate (50 mL) and washed with saturated aqueous NaHCO₃ (50 mL). The aqueous layer was again extracted with iPrOAc (50 mL). The combined organics were washed with water and brine, dried over Mg₂SO₄, filtered, evaporated *in vacuo,* and dried under vacuum to give 776 mg (96.8%) of Compound **105** as an off white solid. LCMS (ESI) [M + H]⁺ = 482.

### Example V: Synthesis of Compounds 106-B1 and 106-B2

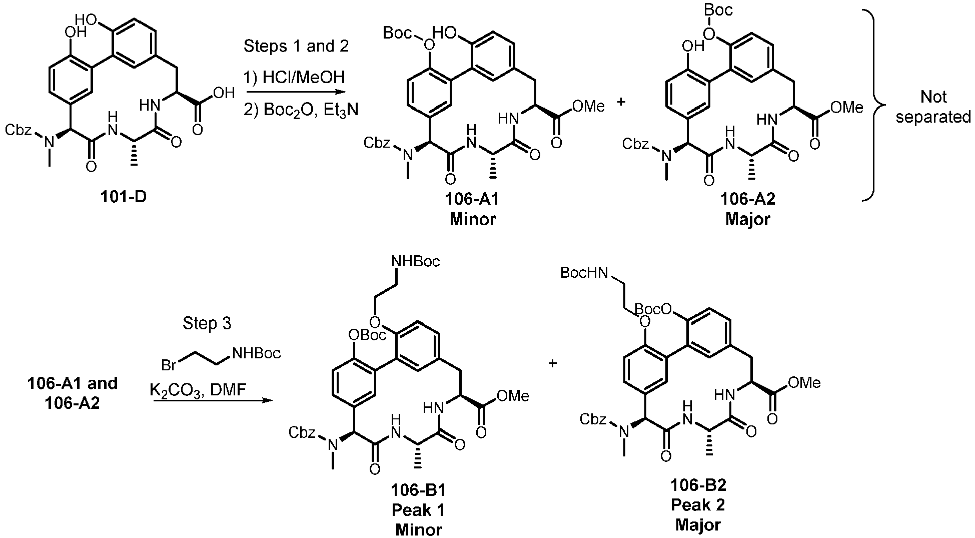

Step 1: Compound 101-D (2.0 g, 3.65 mmol) was added to a solution of 1.25N HCl/MeOH (150 mL) and the mixture was stirred at 0°C for 4 h. The volatiles were removed to give the crude as a white solid.

Step 2: The above crude was dissolved in DCM (5 mL) and the mixture was added Boc₂O (0.93 g, 4.27 mmol) and TEA (1.08 g, 10.7 mmol). The resulting mixture was stirred for at room temperature for 16 h. The volatiles were removed and the residue was purified by silica gel flash column to obtain Compound 106-A1 and 106-A2 as a mixture of regioisomers (1.8 g, 76.4% yield) as a white solid. LCMS (5-95 AB, ESI): t_{R} = 0.880, [M + H]⁺ = 684.6.

To a mixture of Compound 106-A1 and 106-A2 (1.8 g, 2.72 mmol) and *t*-butyl (2-bromoethyl)carbamate (3.0 g, 13.6 mmol) in DMF (5 mL) was added K₂CO₃ (3.8 g, 27.2 mmol) and the reaction mixture was stirred at room temperature for 3 h. The reaction mixture was added with DCM (50 mL), which was washed with 2N HCl, saturated NaHCO₃ and brine (20 mL each). The organic layer was then dried over Na₂SO₄, concentrated and the residue was purified on silica gel flash column to afford the mixture of regioisomers, which was further purified by SFC (OD, 250mm × 30mm, 5um) to afford Compound 106-B1(80 mg, 3.6% yield) and Compound 106-B2 (1.6 g, 73.2% yield) as a white solid.

### Example 1: Synthesis of Compound 201

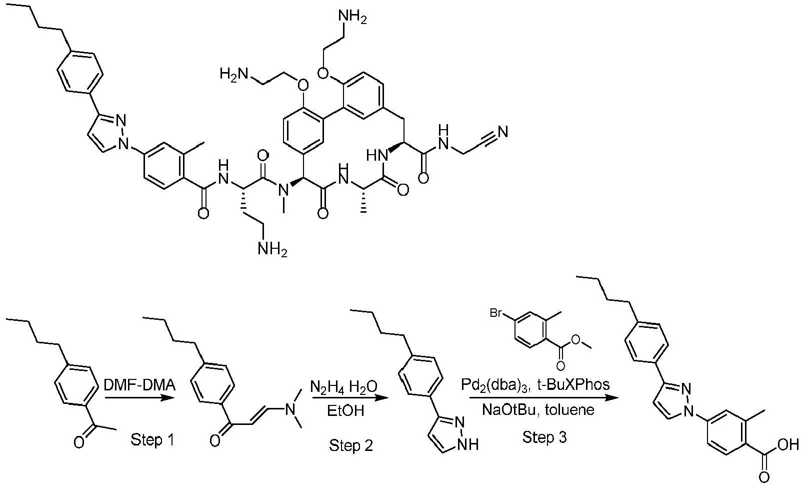

Step 1: A solution of 1-(4-butylphenyl)ethan-1-one (500 mg, 2.8 mmol), DMF-DMA (406 mg, 3.4 mmol) and proline (32.66mg, 0.2800mmol) was stirred at 80 °C for 3 h. The reaction was quenched with water (15 mL), which was extracted with EtOAc (3 × 20 mL). The combined organic layers were washed with brine (50 mL), dried over Na₂SO₄, concentrated and the crude was purified by silica gel chromatography, eluting with 0-20% EtOAc in petroleum ether, to give 1-(4-butylphenyl)-3-(dimethylamino)prop-2-en-1-one (550 mg, 84% yield) as a yellow solid.

Step 2: A solution of 1-(4-butylphenyl)-3-(dimethylamino)prop-2-en-1-one (400 mg, 1.73 mmol) and hydrazine monohydrate (90 µL, 8.65 mmol) in EtOH (5 mL) was stirred at 90 °C for 2 h. The reaction was partitioned between water and EtOAc (50 mL each). The organic layer was dried over Na₂SO₄, concentrated to give 3-(4-butylphenyl)-1*H*-pyrazole (300 mg, 87% yield) as yellow oil, which was used directly in the next step.

Step 3: A solution of 3-(4-butylphenyl)-1*H*-pyrazole (240 mg, 1.2 mmol), methyl 4-bromo-2-methylbenzoate (412 mg, 1.8 mmol), Pd₂(dba)₃ (27 mg, 0.03 mmol), t-BuXPhos (51 mg, 0.12 mmol) and NaOt-Bu (173 mg, 1.8 mmol) in toluene (3 mL) was stirred at 80 °C under N₂ for 7 hr. The reaction was portioned with water and EtOAc (50 mL each). The organic layer was dried over Na₂SO₄, concentrated and the residue was purified by HPLC to give 4-(3-(4-butylphenyl)-1*H*-pyrazol-1-yl)-2-methylbenzoic acid (90 mg, 22.5% yield) as a white solid. ¹H NMR (400 MHz, DMSO-d6): δ 8.65 (d, *J*=5.6 Hz, 1H), 7.98 (d, *J*=8.0 Hz, 1H), 7.89-7.80 (m, 4H), 7.28 (d, *J*=8.0 Hz, 2H), 7.05 (d, *J*=5.6 Hz, 1H), 2.63 (s, 3H), 2.60 (t, *J*=7.2 Hz, 2H), 1.62-1.55 (m, 2H), 1.37-1.28 (m, 2H), 0.91 (t, *J*=7.2 Hz, 3H).

Compound 201 (formic acid salt) was prepared as a white solid from Compound **101-K** and 4-(3-(4-butylphenyl)-1*H*-pyrazol-1-yl)-2-methylbenzoic acid by utilizing methods analogous to those described in Example G. LCMS (Method 5-95 AB, ESI): t_{R} = 0.656 min, [M + H]⁺ = 954.5; ¹H NMR (400 MHz, MeOH-*d*₄) δ 8.50 (br s, 2H), 8.28 (br s, 1H), 7.82-7.74 (m, 4H), 7.54 (d, *J*=8.0 Hz, 1H), 7.34 (d, *J*=8.0 Hz, 1H), 7.27 (d, *J*=8 Hz, 2H), 7.24-7.16 (m, 2H), 7.10-7.05 (m, 1H), 6.93-6.85 (m, 2H), 6.77 (s, 1H), 6.42 (s, 1H), 5.21-5.14 (m, 1H), 4.82-4.77 (m, 2H), 4.28-4.10 (m, 4H), 4.20 (s, 2H), 3.30-3.07 (m, 8H), 2.95 (s, 3H), 2.67 (t, *J*=8 Hz, 2H), 2.51(s, 3H), 2.36-2.24 (m, 1H), 2.23-2.10 (m, 1H), 1.70-1.60 (m, 2H), 1.46-1.33(m, 5H), 0.97 (t, *J*=7.2 Hz, 3H).

### Example 2: Synthesis of Compound 202

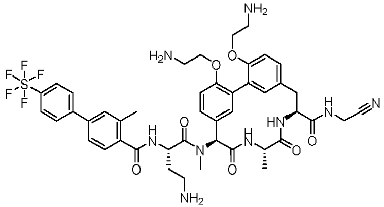

Compound 202 (formic acid salt) was prepared as a white solid from Compound **101-K** by utilizing methods analogous to those described in Example H. LCMS (Method 5-95 AB, ESI): t_{R} = 0.608 min, [M + H]⁺ = 958.5; ¹H NMR (400 MHz, MeOH-*d*₄) δ 8.48 (br s, 2H), 7.94 (d, *J*=8.4 Hz, 2H), 7.86 (d, *J*=8.0 Hz, 2H), 7.66-7.57 (m, 3H), 7.36 (d, *J*=8.0 Hz, 1H), 7.20 (d, *J*=8.4 Hz, 1H), 7.11 (d, *J*=8.4 Hz, 1H), 6.93 (s, 1H), 6.85 (s, 1H), 6.38 (s, 1H), 5.21-5.18 (m, 1H), 4.95-4.78 (m, 2H), 4.30-4.10 (m, 4H), 4.20 (s, 2H), 3.50-3.18 (m, 8H), 2.98 (s, 3H), 2.55 (s, 3H), 2.33-2.25 (m, 1H), 2.25-2.16 (m, 1H), 1.38 (d, *J*=6.8, 3H).

### Example 3: Synthesis of Compound 203

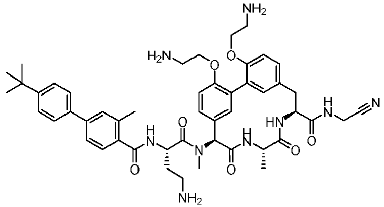

Compound 203 (formic acid salt) was prepared as a white solid from Compound **101-K** by utilizing methods analogous to those described in Example H. LCMS (Method 5-95 AB, ESI): t_{R} = 0.735 min, [M + H]⁺ = 888.4; ¹H NMR (400 MHz, MeOH-*d*₄) δ 8.51 (br s, 2H), 7.58-7.35 (m, 7H), 7.35-7.33 (m, 1H), 7.23-7.18 (m, 2H), 7.11-7.08 (m, 2H), 6.88 (br s, 1H), 6.38 (s, 1H), 5.17-5.10 (m, 1H), 4.85-4.80 (m, 2H), 4.25-4.20 (m, 4H), 4.22 (s, 2H), 3.27-3.00 (m, 8H), 2.95 (s, 3H), 2.52 (s, 3H), 2.29-2.16 (m, 2H), 1.42 (t, *J*=7.2 Hz, 3H), 1.39 (s, 9H).

### Example 4: Synthesis of Compound 204

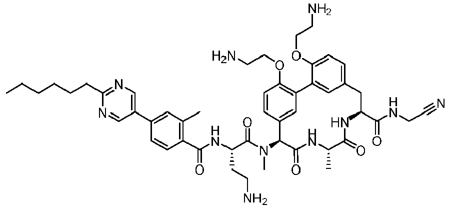

Compound 204 (formic acid salt) was prepared as a white solid from Compound **101-K** by utilizing methods analogous to those described in Example I. LCMS (Method 5-95 AB, ESI): t_{R} = 0.710 min, [M + H]⁺ = 918.6; ¹H NMR (400 MHz, MeOH-*d*₄) δ 8.95 (s, 2H), 8.50 (br s, 1H), 7.65-7.58 (m, 2H), 7.50 (d, *J*=8.0 Hz, 1H), 7.33 (d, *J*=8.0 Hz, 1H), 7.24-7.16 (m, 2H), 7.07 (d, *J*=8.0 Hz, 1H), 6.90 (d, *J*=2.4 Hz, 1H), 6.69 (s, 1H), 6.45 (s, 1H), 5.22-5.16 (m, 1H), 4.80-4.76 (m, 2H), 4.30-4.18 (m, 4H), 4.20 (s, 2H), 3.27-3.06 (m, 8H), 3.00 (t, *J*=7.6 Hz, 2H), 2.98 (s, 3H), 2.48 (s, 3H), 2.35-2.24 (m, 1H), 2.22-2.11 (m, 1H), 1.91-1.81 (m, 2H), 1.42-1.33 (m, 9H), 0.93 (t, *J*=6.8 Hz, 3H).

### Example 5: Synthesis of Compound 205

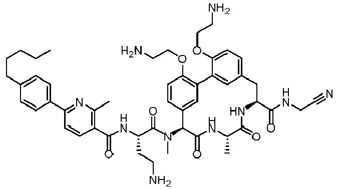

Step 1: A solution of 6-bromo-2-methylnicotinic acid (140 mg, 0.65 mmol), 4-n-pentyl-benzene boronic acid (187 mg, 0.97 mmol), Pd(dppf)Cl₂ (95 mg, 0.13 mmol) and Cs₂CO₃ (634 mg, 1.94 mmol) in toluene/H₂O (11 mL, v/v=10/1) was stirred at 110 °C for 16 h under N₂. After filtration, the filtrate was partitioned with EtOAc and H₂O (each 50 mL). The organic layer was washed with brine (2 × 30 mL), dried over Na₂SO₄, concentrated and the residue was purified by preparatory-TLC to obtain 2-methyl-6-(4-pentylphenyl)nicotinic acid (120 mg, 65% yield) as a colorless oil. ¹H NMR (400 MHz, CD₃Cl) δ 8.36 (d, *J*=8.4 Hz, 1H), 8.01 (d, *J*=8.4 Hz, 2H), 7.66 (d, *J*=8.4 Hz, 1H), 7.31 (d, *J*=8.4 Hz, 2H), 2.96 (s, 3H), 2.68 (t, *J*=8.0 Hz, 2H), 1.66-1.34 (m, 6H), 0.91 (t, *J*=6.0 Hz, 3H).

Compound 205 (formic acid salt) was prepared as a white solid from Compound **101-K** and 2-methyl-6-(4-pentylphenyl)nicotinic acid by utilizing methods analogous to those described in Example G. LCMS (Method 5-95 AB, ESI): t_{R} = 0.643 min, [M + H]⁺ = 903.5; ¹H NMR (400 MHz, MeOH-*d*₄) δ 8.50 (s, 1H), 7.80-7.65 (m, 3H), 7.31 (d, *J*=8.4 Hz, 1H), 7.31-7.21 (m, 2H), 7.19-7.12 (m, 2H), 6.92 (s, 1H), 6.82 (s, 1H), 6.39 (s, 1H), 5.19-5.16 (m, 1H), 4.90-4.79 (m, 2H), 4.30-4.15 (m, 4H), 4.20 (s, 2H), 3.30-3.15 (m, 8H), 2.96 (s, 3H), 2.68 (t, J=7.6 Hz, 2H), 2.65 (s, 3H), 2.32-2.27 (m, 1H), 2.20-2.13 (m, 1H), 1.38-1.25 (m, 6H), 0.93 (t, J=6.4 Hz, 3H).

### Example 6: Synthesis of Compound 206

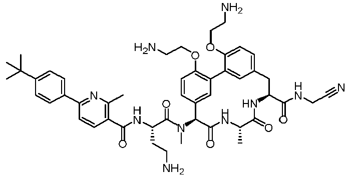

Compound 206 (formic acid salt) was prepared as a white solid from Compound **101-K** by utilizing methods analogous those described in Example 5. LCMS (Method 5-95 AB, ESI): t_{R} = 0.706 min, [M + H]⁺ = 889.6; ¹H NMR (400 MHz, MeOH-*d*₄) δ 8.50 (s, 2H), 7.92 (d, *J*=8.4 Hz, 2H), 7.84 (d, *J*=8.0 Hz, 1H), 7.73 (d, *J*=8.0 Hz, 1H), 7.54 (d, *J*=8.4 Hz, 2H), 7.36-7.28 (m, 1H), 7.25- 7.16 (m, 2H), 7.10 (d, *J*=8.4 Hz, 1H), 6.90 (d, *J*=2.0 Hz, 1H), 6.77 (s, 1H), 6.41 (s, 1H), 5.19-5.16 (m, 1H), 4.85-4.79 (m, 2H), 4.24-4.15 (m, 4H), 4.20 (s, 2H), 3.17-3.08 (m, 8H), 2.96 (s, 3H), 2.68 (s, 3H), 2.30-2.26 (m, 1H), 2.18-2.12 (m, 1H), 1.37 (s, 9H), 1.36 (t, J=6.4 Hz, 3H).

### Example 7: Synthesis of Compound 207

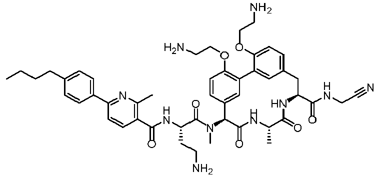

Compound 207 (formic acid salt) was prepared as a white solid from Compound **101-K** by utilizing methods analogous to those described in Example 5. LCMS (Method 5-95 AB, ESI): t_{R} = 0.707 min, [M + H]⁺ = 889.6; ¹H NMR (400 MHz, MeOH-*d*₄) δ 8.49 (br s, 3H), 7.88-7.82 (m, 3H), 7.72 (d, *J*=8.4 Hz, 1H), 7.31-7.29 (m, 3H), 7.20-7.18 (m, 2H), 7.11 (d, *J*=8.4 Hz, 1H), 6.89 (d, *J*=2.0 Hz, 1H), 6.74 (s, 1H), 6.43 (s, 1H), 5.17-5.15 (m, 1H), 4.79-4.75 (m, 2H), 4.30-4.15 (m, 4H), 4.20 (s, 2H), 3.34-3.10 (m, 8H), 2.96 (s, 3H), 2.69 (t, J=8.0 Hz, 2H), 2.66 (s, 3H), 2.40-2.25 (m, 1H), 2.20-2.05 (m, 1H), 1.55-1.50 (m, 2H), 1.42-1.34 (m, 4H), 0.96 (t, J=7.2 Hz, 3H).

### Example 8: Synthesis of Compound 208

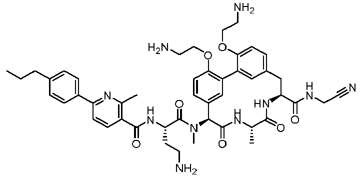

Compound 208 (formic acid salt) was prepared as a white solid from Compound **101-K** by utilizing methods analogous to those described in Example 5. LCMS (Method 5-95 AB, ESI): t_{R} = 0.690 min, [M + H]⁺ = 875.5; ¹H NMR (400 MHz, MeOH-*d*₄) δ 8.42 (s, 1H), 7.93 (d, *J*=8.0 Hz, 2H), 7.86 (d, *J*=8.0 Hz, 1H), 7.74 (d, *J*=8.0 Hz, 1H), 7.33 (d, *J*=8.4 Hz, 2H), 7.24 (d, *J*=8.4 Hz, 1H), 7.18 (d, *J*=8.4 Hz, 1H), 7.09 (d, *J*=8.4 Hz, 1H), 6.91 (s, 1H), 6.82 (s, 1H), 6.37 (s, 1H), 5.19-5.17 (m, 1H), 4.98-4.78 (m, 2H), 4.23-4.15 (m, 4H), 4.19 (s, 2H), 3.55-3.13 (m, 8H), 2.95 (s, 3H), 2.69 (s, 3H), 2.67 (t, J=8.0 Hz, 2H), 2.33-2.24 (m, 1H), 2.24- 2.08 (m, 1H), 1.72-1.67 (m, 2H), 1.39-1.33 (m, 2H), 0.97 (t, *J*=7.6 Hz, 3H).

### Example 9: Synthesis of Compound 209

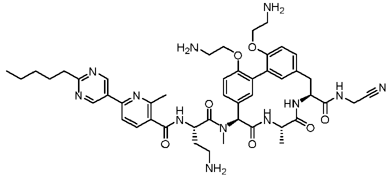

Compound 209 (formic acid salt) was prepared as a white solid from Compound **101-K** by utilizing methods analogous to those described in Example 5 and Example I. LCMS (Method 5-95 AB, ESI): t_{R} = 0.679 min, [M + H]⁺ = 905.5; ¹H NMR (400 MHz, MeOH-*d*₄) δ 9.29 (s, 2H), 7.89 (s, 2H), 7.32 (d, *J*=7.6 Hz, 1H), 7.25-7.11 (m, 2H), 7.10-7.00 (m, 1H), 6.88 (s, 1H), 6.67 (br s, 1H), 6.44 (s, 1H), 5.20-5.16 (m, 1H), 4.76-4.64 (m, 2H), 4.40-4.11 (m, 6H), 3.29-2.89 (m, 13H), 2.64 (s, 3H), 2.36-2.09 (m, 2H), 1.90-1.84 (m, 2H), 1.43-1.32 (m, 6H), 0.93 (t, *J*=6.4 Hz, 3H).

### Example 10: Synthesis of Compound 210

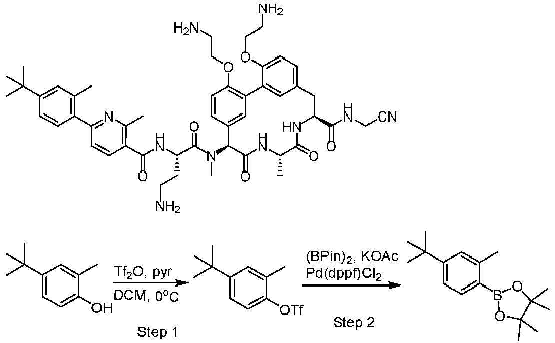

Step 1: A mixture of 4-(*tert*-butyl)-2-methylphenol (1.0 g, 6.1 mmol), pyridine (0.96 g, 12.2 mmol) and trifluoromethanesulfonic anhydride (2.1 g, 12.2 mmol) in DCM (10 mL) was stirred at 20 °C for 2 h. The reaction mixture was diluted with water (30 mL), which was extracted by EtOAc (3 × 30 mL). The combined organic layers were washed with brine (2 × 50 mL), dried over MgSO₄ and concentrated to give 4-(*tert*-butyl)-2-methylphenyl trifluoromethanesulfonate (1.5 g, 83% yield) as a colorless oil.

Step 2: A mixture of 4-(*tert*-butyl)-2-methylphenyl trifluoromethanesulfonate (1.0 g, 3.4 mmol), bis(pinacolato)diboron (2.6 g, 10.2 mmol), Pd(dppf)Cl₂ (247 mg, 0.34 mmol), and potassium acetate (1.7 g, 17.0 mmol) in 1,4-dioxane (10 mL) was stirred at 80 °C under nitrogen for 12 h. The reaction mixture was diluted with water (30 mL), which was extracted by EtOAc (3 × 30 mL). The combined organic layers were washed with brine (2 × 50 mL), dried over MgSO₄ and concentrated and the residue was purified by prep-TLC (5% EtOAc in petroleum ether) to give 2-(4-(*tert*-butyl)-2-methylphenyl)-4,4,5,5-tetramethyl-1,3,2-dioxaborolane (700 mg, 76% yield) as a colorless oil. ¹H NMR (400 MHz, CDCl₃) δ 7.72 (d, *J* = 8.4 Hz, 1H), 7.23-7.18 (m, 2H), 2.50 (s, 3H), 1.32 (s, 12H), 1.30 (s, 9H).

Compound 210 (formic acid salt) was prepared as a white solid from Compound **101-K** and 2-(4-(tert-butyl)-2-methylphenyl)-4,4,5,5-tetramethyl-1,3,2-dioxaborolane by utilizing methods analogous to those described in Example 5. LCMS (Method 5-95 AB, ESI): t_{R} = 0.706 min, [M + H]⁺ = 904.2; ¹H NMR (400 MHz, MeOH-*d*₄) δ 8.49 (br s, 4H), 7.91 (d, *J*=8.0 Hz, 1H), 7.41 (d, *J*=8.0 Hz, 1H), 7.36-7.10 (m, 7H), 6.90 (s, 1H), 6.82 (s, 1H), 6.37 (s, 1H), 5.19-5.15 (m, 1H), 4.90-4.78 (m, 2H), 4.24-4.17 (m, 4H), 4.19 (s, 2H), 3.34-3.05 (m, 8H), 2.96 (s, 3H), 2.40-2.20 (m, 1H), 2.32 (s, 3H), 2.20-2.05 (m, 1H), 1.36 (s, 9H), 1.35 (d, *J*=7.2 Hz, 3H).

### Example 11: Synthesis of Compound 211

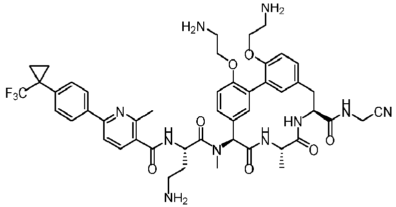

Compound 211 (formic acid salt) was prepared as a white solid from Compound **101-K** by utilizing methods analogous those described in Example 5. LCMS (Method 5-95 AB, ESI): t_{R} = 0.705 min, [M + H]⁺ = 941.4; ¹H NMR (400 MHz, MeOH-*d*₄) δ 8.04 (d, *J*=8.0 Hz, 2H), 7.89 (d, *J*=8.0 Hz, 1H), 7.79 (d, *J*=8.4 Hz, 1H), 7.61 (d, *J*=8.0 Hz, 2H), 7.37-7.28 (m, 1H), 7.26-7.17 (m, 2H), 7.10 (d, *J*=8.4 Hz, 1H), 6.91 (s, 1H), 6.81 (s, 1H), 6.39 (s, 1H), 5.20-5.15 (m, 1H), 4.83-4.78 (m, 2H), 4.28-4.17 (m, 4H), 4.20 (s, 2H), 3.37-3.08 (m, 8H), 2.96 (s, 3H), 2.70 (s, 3H), 2.34-2.25 (m, 1H), 2.20-2.12 (m, 1H), 1.43 (br s, 2H), 1.36 (d, *J*=6.8 Hz, 3H), 1.17-1.11 (br s, 2H).

### Example 12: Synthesis of Compound 212

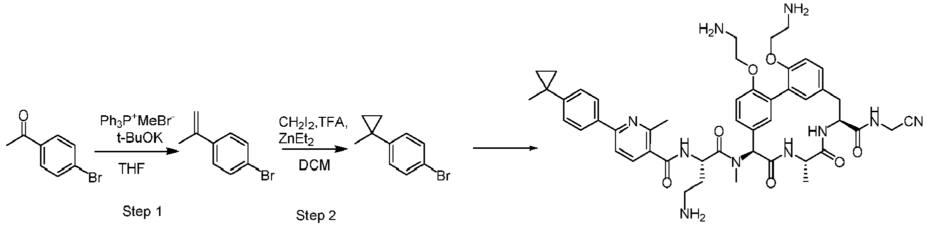

Step 1: A mixture of 1-(4-bromophenyl)ethan-1-one (1.0 g, 5.0 mmol), methyl(triphenyl)phosphonium chloride (4.1 g, 13 mmol), and t-BuOK (1.5 g, 13 mmol) in THF (50 mL) was stirred at 20 °C for 3 h. The volatiles were removed and the residue was re-dissolved in EtOAc (50 mL), which was washed with brine (2 × 50 mL). The organic layer was dried over MgSO₄, concentrated and the residue was purified by flash column chromatography, eluting with 5% EtOAc in petroleum ether, to give 1-bromo-4-(prop-1-en-2-yl)benzene (800 mg, 81% yield) as a colorless oil.

Step 2: To a solution of Et₂Zn (1N in toluene, 3.5 mL) in DCM (10 mL) was added a solution of trifluoroacetic acid (1.54 mL, 20.0 mmol) in DCM (10 mL) dropwise via syringe at 0 °C under N₂ and the mixture was stirred for 20 min at the same temperature, followed by the addition of a solution of 1-bromo-4-(prop-1-en-2-yl)benzene (346 mg, 1.76 mmol) in DCM (10 mL). After an additional 20 min of stirring, CH₂I₂ (0.28 mL, 3.5 mmol) was added and the resulting mixture was stirred at 25 °C for 16 h. The reaction was diluted with petroleum ether (30 mL), which was washed with 1N aq HCl, saturated NaHCO₃ and brine (each 20 mL). The organic layer was dried over MgSO₄, concentrated and the residue was purified by HPLC to give 1-bromo-4-(1-methylcyclopropyl)benzene (150 mg, 40% yield) as a colorless oil.

Compound 212 (formic acid salt) was prepared as a white solid from **101-K** and 1-bromo-4-(1-methylcyclopropyl)benzene by utilizing methods analogous those described in Example 10. LCMS (Method 5-95 AB, ESI): t_{R} = 0.689 min, [M + H]⁺ = 887.4; ¹H NMR (400 MHz, MeOH-*d*₄) δ 8.46 (br s, 3H), 7.92 (d, *J*=8.4 Hz, 2H), 7.85 (d, *J* =8.4 Hz, 1H), 7.73 (d, *J* =8.0 Hz, 1H), 7.37 (d, *J* =8.0 Hz, 2H), 7.31 (d, *J* =8.0 Hz, 1H), 7.23 (d, *J* =8.0 Hz, 1H), 7.18 (d, *J* =8.4 Hz, 1H), 7.09 (d, *J* =8.4 Hz, 1H), 6.90 (s, 1H), 6.80 (s, 1H), 6.38 (s, 1H), 5.20-5.13 (m, 1H), 4.85-4.79 (m, 2H), 4.26-4.16 (m, 4H), 4.19 (s, 2H), 3.28-3.07 (m, 8H), 2.95 (s, 3H), 2.68 (s, 3H), 2.33-2.23 (m, 1H), 2.21-2.09 (m, 1H), 1.45 (s, 3H), 1.35 (d, *J* =7.2 Hz, 3H), 0.92 (s, 2H), 0.83 (s, 2H).

### Example 13: Synthesis of Compound 213

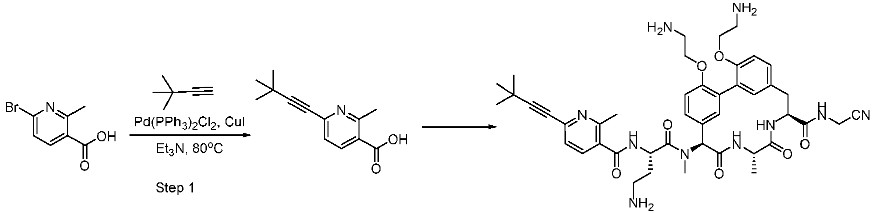

Step 1: A mixture of compound 6-bromo-2-methylnicotinic acid (100 mg, 0.46 mmol), 3,3-dimethyl-1-butyne (380 mg, 4.6 mmol), CuI (17.6 mg, 0.09 mmol), Pd(dppf)Cl₂ (32.5 mg, 0.05 mmol) in Et₃N (10 mL) was stirred at 60 °C under N₂ for 16 h. The mixture was diluted with water (20 mL), which was extracted by EtOAc (2 × 20 mL). The combined organic layers were washed with brine (2 × 40 mL). The organic layer was dried over Na₂SO₄, concentrated and the residue was purified by preparatory-TLC (eluting with 10% MeOH in DCM, R_{f}= 0.4) to give 6-(3,3-dimethylbut-1-yn-1-yl)-2-methylnicotinic acid (60 mg, 60% yield) as a yellow solid. LCMS (Method 5-95 AB, ESI): t_{R} = 0.642 min, [M + H]⁺ = 217.8.

Compound 213 (formic acid salt) was prepared as a white solid from Compound **101-K** and 6-(3,3-dimethylbut-1-yn-1-yl)-2-methylnicotinic acid by utilizing methods analogous to those described in Example G. LCMS (Method 5-95 AB, ESI): t_{R} = 0.616 min, [M + H]⁺ = 837.4; ¹H NMR (400 MHz, MeOH-*d*₄) δ 8.50 (br s, 2H), 7.78 (d, *J*=8.0 Hz, 1H), 7.36 (d, *J*=8.0 Hz, 1H), 7.28 (d, *J*=8.0 Hz, 1H), 7.23 (d, *J*=8.0 Hz, 1H), 7.18 (d, *J*=8.0 Hz, 1H), 7.10 (d, *J*=8.0 Hz, 1H), 6.89 (s, 1H), 6.78 (s, 1H), 6.40 (s, 1H), 5.15-5.13 (m, 1H), 4.85-4.78 (m, 2H), 4.28-4.18 (m, 4H), 4.20 (s, 2H), 3.24-3.10 (m, 8H), 2.94 (s, 3H), 2.57 (s, 3H), 1.36 (s, 9H), 1.35 (d, J=6.8 Hz, 3H).

### Example 14: Synthesis of Compound 214

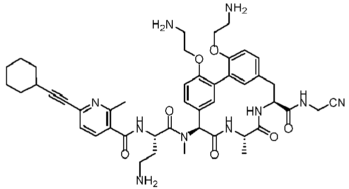

Compound 214 (formic acid salt) was prepared as a white solid from Compound **101-K** by utilizing methods analogous to those described in Example 13. LCMS (Method 5-95 AB, ESI): t_{R} = 0.689 min, [M + H]⁺ = 863.9; ¹H NMR (400 MHz, MeOH-*d*₄) δ 8.46 (br s, 2H), 7.78 (d, *J*=7.6 Hz, 1H), 7.37 (d, *J*=7.6 Hz, 1H), 7.33-7.27 (m, 1H), 7.24 (d, *J*=4.8 Hz, 1H), 7.17 (d, *J*=8.4 Hz, 1H), 7.09 (d, *J*=8.4 Hz, 1H), 6.89 (d, *J* =2.2 Hz, 1H), 6.81 (s, 1H), 6.36 (s, 1H), 5.16-5.09 (m, 1H), 4.80-4.71 (m, 2H), 4.25-4.16 (m, 4H), 4.19 (s, 2H), 3.34-3.08 (m, 8H), 2.93 (s, 3H), 2.74-2.66 (m, 1H), 2.57 (s, 3H), 2.33-2.20 (m, 1H), 2.18-2.06 (m, 1H), 1.95-1.89 (m, 2H), 1.79-1.76 (m, 2H), 1.59-1.55 (m, 3H), 1.45 - 1.37 (m, 3H), 1.34 (d, *J* =7.2 Hz, 3H).

### Example 15: Synthesis of Compound 215

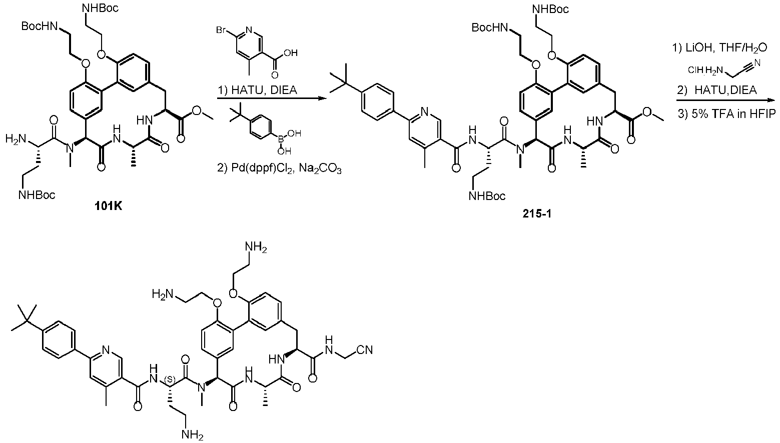

Starting from Compound **101-K**, typical amide coupling (HATU/DIEA), Suzuki coupling ester hydrolysis (LiOH, THF/H₂O), amide coupling (HATU/DIEA) and Boc removal (TFA/HFIP) conditions, analogous to those described in Examples G and H, were applied to afford Compound 215 (formic acid salt) as a white solid. LCMS (Method 5-95 AB, ESI): t_{R} = 0.702 min, [M + H]⁺ = 889.5; ¹H NMR (400 MHz, MeOH-*d*₄) δ 8.58 (s, 1H), 8.49 (br s, 3H), 7.92-7.87 (m, 2H), 7.78 (s, 1H), 7.57-7.51 (m, 3H), 7.32 (d, *J*=8.0Hz, 1H), 7.20 (d, *J*=8.0Hz, 2H), 7.08 (d, *J*=8.0Hz, 1H), 6.89 (s, 1H), 6.43 (s, 1H), 5.20-5.17 (m, 1H), 4.82-4.79 (m, 2H), 4.24-4.18 (m, 4H), 4.20 (s, 2H), 3.16-3.12 (m, 8H), 2.95 (s, 3H), 2.52 (s, 3H), 2.29-2.17 (m, 2H), 1.38 (s, 9H), 1.36 (d, *J*=7.2 Hz, 3H).

### Example 16: Synthesis of Compound 216

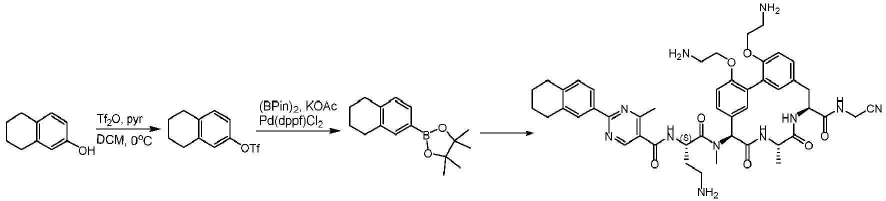

Compound 216 (formic acid salt) was prepared as a white solid starting from 5,6,7,8-tetrahydronaphthalen-2-ol and Compound **101-K** by utilizing methods analogous to those described in Example 10 and Example J. LCMS (Method 5-95 AB, ESI): t_{R} = 0.709 min, [M + H]⁺ = 888.5; ¹H NMR (400 MHz, MeOH-*d*₄) δ 8.77 (s, 1H), 8.48 (br s, 2H), 8.20-8.05 (m, 2H), 7.40-7.30 (m, 1H), 7.30-7.15 (m, 3H), 7.15-7.05 (m, 1H), 6.91 (s, 1H), 6.80 (s, 1H), 6.40 (s, 1H), 5.25-5.15 (m, 1H), 4.75-4.70 (m, 2H), 4.25-4.15 (m, 4H), 4.19 (s, 2H), 3.25-3.05 (m, 8H), 2.96 (s, 3H), 2.87 (br s, 4H), 2.75 (s, 3H), 2.35-2.25 (m, 1H), 2.25-2.10 (m, 1H), 1.86 (br s, 4H), 1.36 (d, J=6.8 Hz, 3H).

### Example 17: Synthesis of Compound 217

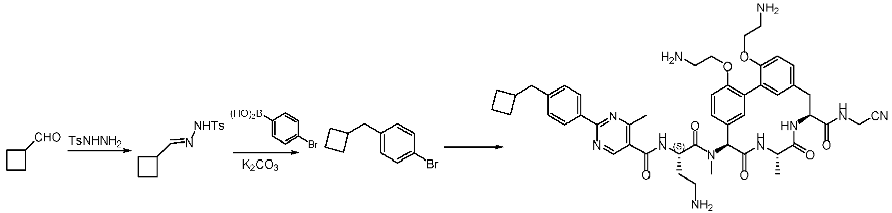

Step 1: A mixture of cyclobutane carbaldehyde (202 mg, 2.4 mmol) and 4-methylbenzenesulfonohydrazide (448 mg, 2.4 mmol) in 1,4-dioxane (1 mL) was stirred at 50 °C for 1 h. The volatiles were removed under reduced pressure to give N'-(cyclobutylmethylene)-4-methylbenzenesulfonohydrazide (607 mg), which was used directly in the next step.

Step 2: A mixture of N'-(cyclobutylmethylene)-4-methylbenzenesulfonohydrazide (600 mg, 2.4 mmol), 4-bromophenyl boronic acid (716 mg, 3.6 mmol), and K₂CO₃ (657 mg, 4.8 mmol) in 1,4-dioxane (20 mL) was stirred at 110 °C for 16 h. The reaction was diluted with water (10 mL), which was extracted with EtOAc (3 × 20 mL). The combined organic layers were washed with brine (50 mL), dried over MgSO₄, concentrated and the residue was purified by silica-gel column chromatography, eluting with petroleum ether, to give 1-bromo-4-(cyclobutylmethyl)benzene (378 mg, 71% yield) as colorless oil.

Compound 217 (formic acid salt) was prepared as a white solid from 1-bromo-4-(cyclobutylmethyl)benzene and Compound **101-K** by utilizing methods analogous to those described in Example 10 and Example J. LCMS (Method 5-95 AB, ESI): t_{R} = 0.640 min, [M + H]⁺ = 902.5; ¹H NMR (400 MHz, MeOH-*d*₄) δ 8.77 (s, 2H), 8.55 (br s, 2H), 8.31 (d, *J*=8.4 Hz, 1H), 7.32-7.28 (m, 3H), 7.20-7.17 (m, 2H), 7.07 (d, *J*=8.4 Hz, 1H), 6.88 (s, 1H), 6.76 (s, 1H), 6.74 (s, 1H), 5.18 (t, *J*=5.2 Hz, 1H), 4.85-4.78 (m, 2H), 4.21-4.15 (m, 4H), 4.19 (s, 2H), 3.14-3.00 (m, 8H), 2.95 (s, 3H), 2.78 (d, *J*=7.6 Hz, 2H), 2.66 (s, 3H), 2.20-2.10 (m, 1H), 2.07-2.00 (m, 3H), 1.95-1.78 (m, 4H), 1.35 (d, *J*=6.8 Hz, 3H).

### Example 18: Synthesis of Compound 218

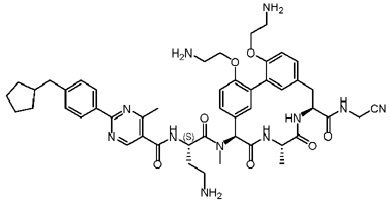

Compound 218 (formic acid salt) was prepared as a white solid from Compound **101-K** by utilizing methods analogous to those described in Example 17. LCMS (Method 5-95 AB, ESI): t_{R} = 0.757 min, [M + H]⁺ = 916.5; ¹H NMR (400 MHz, MeOH-*d*₄) δ 8.79 (s, 1H), 8.48 (br s, 2H), 8.36 (d, *J*=8.4 Hz, 2H), 7.34-7.30 (m, 3H), 7.25-7.17 (m, 2H), 7.10 (d, *J*=8.4 Hz, 1H), 6.91 (d, *J*=2.0 Hz, 1H), 6.82 (s, 1H), 6.39 (s, 1H), 5.21-5.16 (m, 1H), 4.72-4.68 (m, 2H), 4.26-4.16 (m, 4H), 4.20 (s, 2H), 3.40-3.04 (m, 8H), 2.96 (s, 3H), 2.70-2.50 (m, 2H), 2.40 (s, 6H), 2.33-2.27 (m, 1H), 2.20-2.12 (m, 2H), 1.80-1.65 (m, 4H), 1.61-1.53 (m, 2H), 1.36 (d, *J*=6.8 Hz, 3H), 1.30-1.22 (m, 2H).

### Example 19: Synthesis of Compound 219

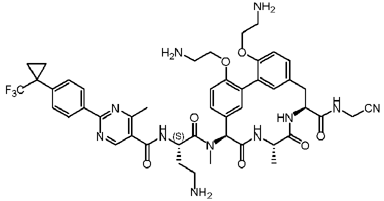

Compound 219 (formic acid salt) was prepared as a white solid from Compound **101-K** by utilizing methods analogous to those described in Example 10 and Example J. LCMS (Method 5-95 AB, ESI): t_{R} = 0.714 min, [M + H]⁺ = 942.6; ¹H NMR (400 MHz, MeOH-*d*₄) δ 8.82 (s, 1H), 8.46 (d, *J*=8.0 Hz, 2H), 7.63 (d, *J*=8.0 Hz, 2H), 7.33 (d, *J*=8.4 Hz, 1H), 7.22 (m, 2H), 7.10 (d, *J*=8.4 Hz, 1H), 6.91 (d, *J*=1.6 Hz, 1H), 6.81 (s, 1H), 6.40 (s, 1H), 5.22-5.17 (m, 1H), 4.75-4.71 (m, 2H), 4.28-4.15 (m, 4H), 4.20 (s, 2H), 3.35-3.07 (m, 8H), 2.96 (s, 3H), 2.72 (s, 3H), 2.33-2.27 (m, 1H), 2.21-2.14 (m, 1H), 1.43 (br s, 2H), 1.36 (d, *J*=6.8 Hz, 3H), 1.16 (br s, 2H).

### Example 20: Synthesis of Compound 220

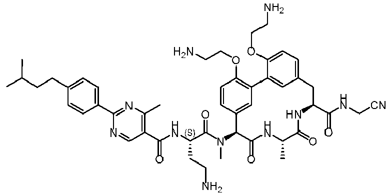

Compound 220 (formic acid salt) was prepared as a white solid from Compound **101-K** by utilizing methods analogous to those described in Example 17. LCMS (Method 5-95 AB, ESI): t_{R} = 0.632 min, [M + H]⁺ = 904.6; ¹H NMR (400 MHz, MeOH-*d*₄) δ 8.77 (s, 1H), 8.48 (br s, 2H), 8.32 (d, *J*=8.0 Hz, 2H), 7.40-7.30 (m, 3H), 7.21 (d, *J*=8.0 Hz, 2H), 7.09 (d, *J*=8.0 Hz, 1H), 6.89 (s, 1H), 6.75 (s, 1H), 6.44 (s, 1H), 5.20-5.15 (m, 1H), 4.85-4.75 (m, 2H), 4.35-4.15 (m, 4H), 4.20 (s, 2H), 3.40-3.05 (m, 8H), 2.96 (s, 3H), 2.72 (t, J=7.6 Hz, 2H), 2.69 (s, 3H), 2.35-2.25 (m, 1H), 2.20-2.10 (m, 1H), 1.70-1.50 (m, 3H), 1.36 (d, *J*=6.4 Hz, 3H), 0.98 (d, J=6.0 Hz, 6H).

### Example 21: Synthesis of Compound 221

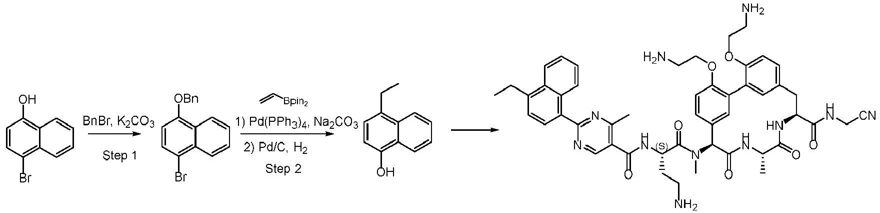

Step 1: A mixture of 4-bromonaphthalen-1-ol (2.0 g, 9.0 mmol), benzyl bromide (2.3 g, 13.5 mmol), and K₂CO₃ (3.7 g, 27 mmol) in DMF (1 mL) was stirred at 20 °C for 16 h. The reaction was poured into water (50 mL), which was extracted with EtOAc (3 × 50 mL). The combined organic layers were washed with brine (2 × 100 mL), dried over Na₂SO₄, concentrated and the residue was purified by silica-gel chromatography to give 1-(benzyloxy)-4-bromonaphthalene (2.0 g, 71.2% yield) as a yellow oil. Step 2: Typical Suzuki (Example H) and hydrogenation conditions (Example D) were applied to 1-(benzyloxy)-4-bromonaphthalene to give 4-ethylnaphthalen-1-ol as a white solid.

Compound 221 (formic acid salt) was prepared as a white solid from Compound **101-K** and 4-ethylnaphthalen-1-ol by utilizing methods analogous to those described in Example 10 and Example J. LCMS (Method 5-95 AB, ESI): t_{R} = 0.701 min, [M + H]⁺ = 912.5; ¹H NMR (400 MHz, MeOH-*d*₄) δ 8.93 (s, 1H), 8.50 (d, J=8.4 Hz, 1H), 8.23 (d, J=8.4 Hz, 1H), 7.89 (d, J=8.0 Hz, 1H), 7.63-7.53 (m, 3H), 7.37-7.32 (m, 1H), 7.25-7.16 (m, 2H), 7.11 (d, J=8.0 Hz, 1H), 6.92 (s, 1H), 6.84 (s, 1H), 6.42 (s, 1H), 5.24-5.20 (m, 1H), 4.83-4.80 (m, 2H), 4.27-4.13 (m, 4H), 4.19 (s, 2H), 3.50-3.46 (m, 1H), 3.29-3.07 (m, 9H), 2.98 (s, 3H), 2.77 (s, 3H), 2.35-2.29 (m, 1H), 2.21-2.16 (m, 1H), 1.43 (t, J=7.2 Hz, 3H), 1.36 (d, J=6.8 Hz, 3H).

### Example 22: Synthesis of Compound 222

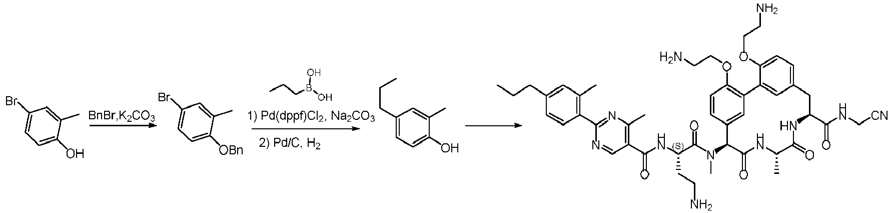

Alkylation (example 21), Suzuki (Example H) and hydrogenation (Pd/C, H₂, Example D) conditions were applied to 4-bromo-2-methylphenol to give 2-methyl-4-propylphenol as a colorless oil. Compound 222 (formic acid salt) was prepared as a white solid from Compound **101-K** by utilizing methods analogous to those described in Example 21 and Example J. LCMS (Method 5-95 AB, ESI): t_{R} = 0.680 min, [M + H]⁺ = 890.5; ¹H NMR (400 MHz, MeOH-*d*₄) δ 8.82 (s, 1H), 8.45 (br s, 3H), 7.64 (d, *J*=7.2 Hz, 1H), 7.32-7.25 (m, 1H), 7.20-7.09 (m, 5H), 6.89 (s, 1H), 6.77 (s, 1H), 6.42 (s, 1H), 5.20-5.15 (m, 1H), 4.85-4.78 (m, 2H), 4.27-4.15 (m, 4H), 4.20 (s, 2H), 3.30-3.13 (m, 8H), 2.96 (s, 3H), 2.63 (s, 3H), 2.62 (t, J=7.2 Hz, 2H), 2.49 (s, 3H), 2.40-2.25 (m, 1H), 2.20-2.05 (m, 1H), 1.71-1.66 (m, 2H), 1.36 (d, *J*=6.8 Hz, 3H), 0.97 (t, J=6.8 Hz, 3H).

### Example 23: Synthesis of Compound 223

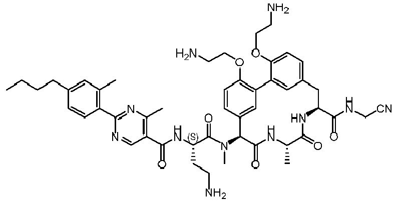

Compound 223 (formic acid salt) was prepared as a white solid from Compound **101-K** by utilizing methods analogous to those described in Example 22. LCMS (Method 5-95 AB, ESI): t_{R} = 0.714 min, [M + H]⁺ = 904.4; ¹H NMR (400 MHz, MeOH-*d*₄) δ 8.82 (s, 1H), 8.46 (br s, 2H), 7.68 (d, *J*=7.6 Hz, 1H), 7.32-7.25 (m, 1H), 7.22-7.10 (m, 5H), 6.90 (s, 1H), 6.80 (s, 1H), 6.39 (s, 1H), 5.20-5.15 (m, 1H), 4.85-4.78 (m, 2H), 4.27-4.15 (m, 4H), 4.20 (s, 2H), 3.30-3.13 (m, 8H), 2.95 (s, 3H), 2.75-2.61 (m, 2H), 2.69 (s, 3H), 2.50 (s, 3H), 2.40-2.25 (m, 1H), 2.20-2.05 (m, 1H), 1.66-1.62 (m, 2H), 1.39-1.34 (m, 5H), 0.96 (t, J=7.6 Hz, 3H).

### Example 24: Synthesis of Compound 224

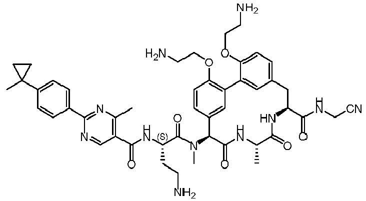

Compound 224 (formic acid salt) was prepared as a white solid from Compound **101-K** by utilizing methods analogous to those described in Example 12 and Example J. LCMS (Method 5-95 AB, ESI): t_{R} = 0.599 min, [M + H]⁺ = 888.5; ¹H NMR (400 MHz, MeOH-*d*₄) δ 8.75 (s, 1H), 8.50 (br s, 2H), 8.28 (d, *J*=8.0 Hz, 2H), 7.37-7.30 (m, 3H), 7.21-7.19 (m, 2H), 7.07 (d, J=8.0 Hz, 1H), 6.88 (s, 1H), 6.70 (s,1H), 6.48 (s, 1H), 5.20-5.16 (m, 1H), 4.85-4.78 (m, 2H), 4.28-4.21 (m, 6H), 3.31-3.13 (m, 8H), 2.96 (s, 3H), 2.67 (s, 3H), 2.31-2.27 (m, 1H), 2.19-2.16 (m, 1H), 1.47 (s, 3H), 1.35 (d, *J*=7.2 Hz, 1H), 0.97-0.94 (m, 2H), 0.86-0.83 (m, 2H).

### Example 25: Synthesis of Compound 225

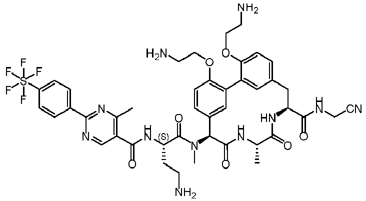

Compound 225 (formic acid salt) was prepared as a white solid from Compound **101-K** by utilizing methods analogous to those described in Example 2 and Example J. LCMS (Method 5-95 AB, ESI): t_{R} = 0.607 min, [M + H]⁺ = 960.5; ¹H NMR (400 MHz, MeOH-*d*₄) δ 8.87 (s, 1H), 8.65 (d, *J*=8.4 Hz, 2H), 8.50 (br s, 2H), 7.98 (d, *J*=8.4 Hz, 2H), 7.33-7.31 (m, 1H), 7.24-7.18 (m, 2H), 7.10 (d, J=8.4 Hz, 1H), 6.91 (s, 1H), 6.79 (s, 1H), 6.41 (s, 1H), 5.20-5.18 (m, 1H), 4.80-4.78 (m,2H), 4.25-4.20 (m, 6H), 3.48-3.44 (m, 1H), 3.17-3.07 (m, 7H), 2.97 (s, 3H), 2.73 (s, 3H), 2.30-2.28 (m, 1H), 2.18-2.17 (m, 1H), 1.36 (d, *J*=6.4 Hz, 3H).

### Example 26: Synthesis of Compound 226

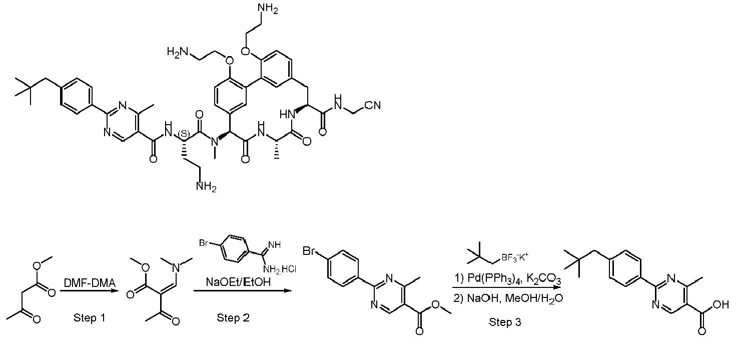

Step 1: A mixture of DMF-DMA (2.5 g, 21 mmol) and methyl acetoacetate (2.0 g, 17 mmol) was stirred at 100 °C for 2 h. The reaction was concentrated to give methyl 2-((dimethylamino)methylene)-3-oxobutanoate (2.8 g), which was used directly in the next step.

Step 2: A mixture of methyl 2-((dimethylamino)methylene)-3-oxobutanoate (800 mg, 4.7 mmol), 4-bromobenzamidine hydrochloride (1.0 g, 4.3 mmol) and sodium ethoxide (293 mg, 4.3 mmol) in ethanol (15 mL) was stirred at 70 °C for 2 h. The volatiles were removed and the residue was extracted with EtOAc (50 mL), which was washed with brine (2 × 50 mL). The organic layer was dried over Na₂SO₄, concentrated and the residue was purified on silica-gel column, eluting with 0-2% EtOAc in petroleum ether, to afford methyl 2-(4-bromophenyl)-4-methylpyrimidine-5-carboxylate (700 mg, 47% yield) as a white solid.

Step 3: Starting from methyl 2-(4-bromophenyl)-4-methylpyrimidine-5-carboxylate, typical Suzuki and ester hydrolysis (NaOH, MeOH) conditions were applied to give 4-methyl-2-(4-neopentylphenyl)pyrimidine-5-carboxylic acid as a white solid.

Compound 226 (formic acid salt) was prepared as a white solid from Compound **101-K** and 4-methyl-2-(4-neopentylphenyl)pyrimidine-5-carboxylic acid by utilizing methods analogous to those described in Example G. LCMS (Method 5-95 AB, ESI): t_{R} = 0.617 min, [M + H]⁺ = 904.5; ¹H NMR (400 MHz, MeOH-*d*₄) δ 8.77 (s, 1H), 8.47 (br s, 1H), 8.30 (d, *J*=8.0 Hz, 2H), 7.35-7.27 (m, 3H), 7.24-7.17 (m, 2H), 7.09 (d, *J*=8.4 Hz, 1H), 6.89 (d, *J*=1.6 Hz, 1H), 6.72 (s, 1H), 6.47 (s, 1H), 5.22-5.16 (m, 1H), 4.82-4.75 (m, 2H), 4.32-4.17 (m, 4H), 4.19 (s, 2H), 3.29-3.06 (m, 8H), 2.97 (s, 3H), 2.69 (s, 3H), 2.61 (s, 2H), 2.36-2.27 (m, 1H), 2.23-2.14 (m, 1H), 1.37 (d, *J*=6.8 Hz, 3H), 0.97 (s, 9H).

### Example 27: Synthesis of Compound 227

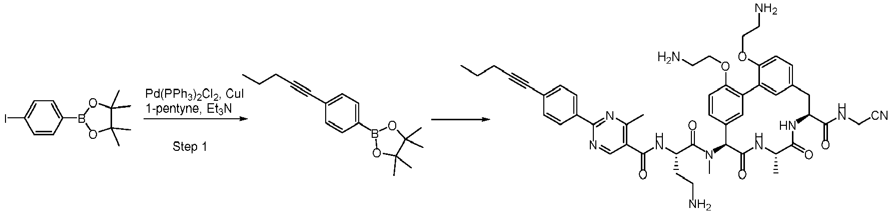

Step 1: Typical Sonogashira condition (Example K) was applied to 2-(4-iodophenyl)-4,4,5,5-tetramethyl-1,3,2-dioxaborolane to give 4,4,5,5-tetramethyl-2-(4-(pent-1-yn-1-yl)phenyl)-1,3,2-dioxaborolane as a yellow oil.

Compound 227 (formic acid salt) was prepared as a white solid from Compound **101-K** and 4,4,5,5-tetramethyl-2-(4-(pent-1-yn-1-yl)phenyl)-1,3,2-dioxaborolane by utilizing methods analogous to those described in Example J. LCMS (Method 5-95 AB, ESI): t_{R} = 0.613 min, [M + H]⁺ = 900.6; ¹H NMR (400 MHz, MeOH-*d*₄) δ 8.78 (s, 1H), 8.48 (br s, 2H), 8.36 (d, *J*=8.0 Hz, 2H), 7.48 (d, *J*=8.0 Hz, 2H), 7.32 (d, *J*=8.4 Hz, 1H), 7.20 (d, *J*=8.4 Hz, 2H), 7.09 (d, *J*=8.4 Hz, 1H), 6.89 (s, 1H), 6.74 (s, 1H), 6.45 (s, 1H), 5.25-5.15 (m, 1H), 4.80-4.75 (m, 2H), 4.30-4.15 (m, 6H), 3.40-3.35 (m, 1H), 3.30-3.10 (m, 7H), 2.96 (s, 3H), 2.69 (s, 3H), 2.44 (t, *J*=7.2 Hz, 2H), 2.35-2.25 (m, 1H), 2.25-2.15 (m, 1H), 1.66 (q, *J*=6.8 Hz, 2H), 1.36 (d, *J*=6.4 Hz, 3H), 1.09 (t, *J*=7.6 Hz, 3H).

### Example 28: Synthesis of Compound 228

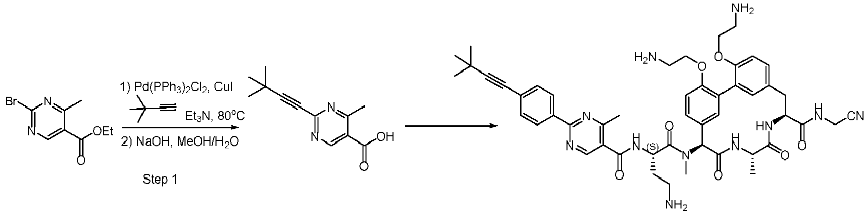

Step 1: Starting from ethyl 2-bromo-4-methylpyrimidine-5-carboxylate (described in Example 26), typical Sonogashira (Example K) and ester hydrolysis (NaOH, MeOH/H₂O, described in Example H) conditions were applied to give 2-(3,3-dimethylbut-1-yn-1-yl)-4-methylpyrimidine-5-carboxylic acid as a yellow solid.

Compound 228 (formic acid salt) was prepared as a white solid from Compound **101-K** and 2-(3,3-dimethylbut-1-yn-1-yl)-4-methylpyrimidine-5-carboxylic acid by utilizing methods analogous to those described in Example G. LCMS (Method 5-95 AB, ESI): t_{R} = 0.542 min, [M + H]⁺ = 838.3; ¹H NMR (400 MHz, MeOH-*d*₄) δ 8.68 (s, 1H), 8.47 (br s, 3H), 7.30 (d, *J*=8.4 Hz, 1H), 7.24 (d, *J*=8.4 Hz, 1H), 7.18 (d, *J*=8.4 Hz, 1H), 7.09 (d, *J*=8.4 Hz, 1H), 6.90 (s, 1H), 6.81 (s, 1H), 6.37 (s, 1H), 5.16-5.12 (m, 1H), 4.81-4.77 (m, 2H), 4.26-4.16 (m, 4H), 4.19 (s, 2H), 3.48 (br s, 1H), 3.21-3.09 (m, 7H), 2.93 (s, 3H), 2.61 (s, 3H), 2.29-2.24 (m, 1H), 2.16-2.11 (m, 1H), 1.38 (s, 9H), 1.36 (t, *J*=6.4 Hz, 3H).

### Example 29: Synthesis of Compound 229

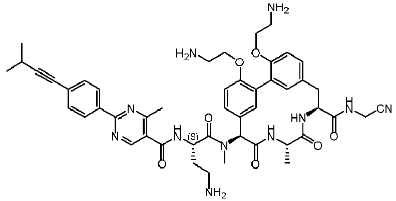

Compound 229 (formic acid salt) was prepared as a white solid from Compound **101-K** by utilizing methods analogous to those described in Example 28. LCMS (Method 0-30 AB, ESI): t_{R} = 0.999 min, [M + H]⁺ = 824.3; ¹H NMR (400 MHz, MeOH-*d*₄) δ 8.68 (s, 1H), 8.49 (br. s., 1H), 7.29 (d, *J*=8.4 Hz, 1H), 7.24 (d, *J*=8.4 Hz, 1H), 7.17 (d, *J*=8.4 Hz, 1H), 7.08 (d, *J*=8.4 Hz, 1H), 6.89 (s, 1H), 6.80 (s, 1H), 6.37 (s, 1H), 5.14 (t, *J*=6.8 Hz, 1H), 4.87-4.76 (m, 2H), 4.25-4.16 (m, 4H), 4.19 (s, 2H), 3.40-3.30 (m, 1H), 3.20-3.08 (m, 7H), 2.93 (s, 3H), 2.90-2.83 (m, 1H), 2.60 (s, 3H), 2.32-2.21 (m, 1H), 2.18-2.08 (m, 1H), 1.34 (d, *J*=6.8 Hz, 3H), 1.31 (d, *J*=7.2 Hz, 6H).

### Example 30: Synthesis of Compound 230

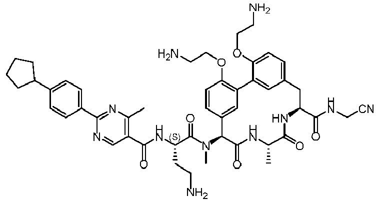

Compound 230 (formic acid salt) was prepared as a white solid from Compound **101-K** by utilizing methods analogous to those described in Example J. LCMS (Method 5-95 AB, ESI): t_{R} = 0.727 min, [M + H]⁺ = 902.4; ¹H NMR (400 MHz, MeOH-*d*₄) δ 8.78 (s, 1H), 8.40-8.33 (m, 2H), 7.40 (d, *J*=8.0 Hz, 1H), 7.37-7.31 (m, 1H), 7.23-7.17 (m, 2H), 7.14-7.08 (m, 2H), 6.93 (s, 1H), 6.84 (s, 1H), 6.38 (s, 1H), 5.20-5.16 (m, 1H), 4.83-4.78 (m, 2H), 4.38-4.16 (m, 6H), 3.48-3.18 (m, 8H), 2.96 (s, 3H), 2.71 (s, 3H), 2.40-2.30 (m, 1H), 2.25-2.07 (m, 4H), 1.94-1.61 (m, 8H), 1.37 (d, J=6.8 Hz, 3H).

### Example 31: Synthesis of Compound 231

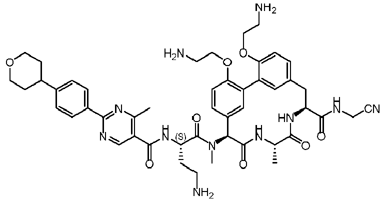

Compound 231 (formic acid salt) was prepared as a white solid from Compound **101-K** by utilizing methods analogous to those described in Example 17. LCMS (Method 5-95 AB, ESI): t_{R} = 0.649 min, [M + H]⁺ = 918.4; ¹H NMR (400 MHz, MeOH-*d*₄) δ 8.81 (s, 1H), 8.45 (br s, 1H), 8.42 (d, *J*=8.0 Hz, 2H), 7.43 (d, *J*=8.0 Hz, 2H), 7.38-7.32 (m, 1H), 7.28-7.19 (m, 2H), 7.11 (d, *J*=8.4 Hz, 1H), 6.93 (s, 1H), 6.83 (s, 1H), 6.41 (s, 1H), 5.22-5.20 (m, 1H), 4.84-4.80 (m, 2H), 4.30-4.21 (m, 6H), 4.11-4.08 (m, 2H), 3.65-3.58 (m, 2H), 3.25-3.11 (m, 8H), 2.97 (s, 3H), 2.73 (s, 3H), 2.34-2.28 (m, 1H), 2.23-2.14 (m, 1H), 1.88-1.81(m, 5H), 1.39 (t, *J*=6.4 Hz, 3H).

### Example 32: Synthesis of Compound 232

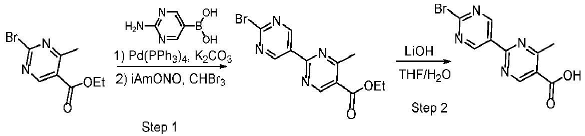

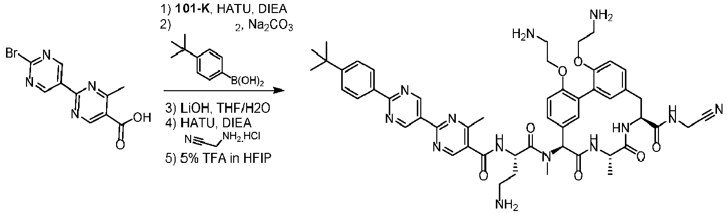

Step 1: Starting from ethyl 2-bromo-4-methylpyrimidine-5-carboxylate (described in Example 26), typical Suzuki (Example H) and Sandmeyer conditions (Example J) were followed to give ethyl 2'-bromo-4-methyl-[2,5'-bipyrimidine]-5-carboxylate as a white solid.

Step 2: Typical ester hydrolysis condition (LiOH, THF/H₂O, Example G) was applied to ethyl 2'-bromo-4-methyl-[2,5'-bipyrimidine]-5-carboxylate to give 2'-bromo-4-methyl-[2,5'-bipyrimidine]-5-carboxylic acid as a white solid. LCMS (Method 5-95 AB, ESI): t_{R} = 0.671, [M + H]⁺ = 295.0.

Step 3: Starting from Compound **101-K** and 2'-bromo-4-methyl-[2,5'-bipyrimidine]-5-carboxylic acid, typical amide coupling (HATU/DIEA), Suzuki, ester hydrolysis (LiOH, THF/H₂O), amide coupling (HATU/DIEA), and Boc removal (TFA/HFIP) conditions, analogous to those described in Examples G and H, were followed to give Compound 232 (formic acid salt) as a white solid. LCMS (Method 5-95 AB, ESI): t_{R} = 0.753 min, [M + H]⁺ = 968.6; ¹H NMR (400 MHz, MeOH-*d*₄) δ 9.66 (s, 2H), 8.80 (s, 1H), 8.54-8.36 (m, 5H), 7.60 (d, *J*=7.6 Hz, 2H), 7.33-7.22 (m, 2H), 7.09-6.97 (m, 2H), 6.85 (s, 1H), 6.61 (s, 1H), 6.48 (s, 1H), 5.22-5.18 (m, 1H), 4.85-4.74 (m, 2H), 4.39-4.16 (m, 6H), 3.28-3.05 (m, 8H), 2.97 (s, 3H), 2.68 (s, 3H), 2.33-2.15 (m, 2H), 1.39 (s, 9H), 1.36 (d, J=6.8 Hz, 3H).

### Example 33: Synthesis of Compound 233

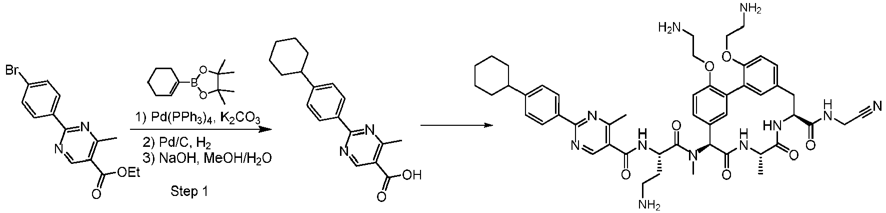

Step 1: Starting from ethyl 2-(4-bromophenyl)-4-methylpyrimidine-5-carboxylate (described in Example 26), typical Suzuki, hydrogenation (Pd/C, H₂) and ester hydrolysis (NaOH, MeOH/H₂O) conditions, analogous to those described in Examples G and H, were applied to give 2-(4-cyclohexylphenyl)-4-methylpyrimidine-5-carboxylic acid as a white solid. LCMS (Method 5-95 AB, ESI): t_{R} = 0.901 min, [M + H]⁺ = 296.9.

Compound 233 (formic acid salt) was prepared as a white solid from Compound **101-K** and 2-(4-cyclohexylphenyl)-4-methylpyrimidine-5-carboxylic acid by utilizing methods analogous to those described in Example G. LCMS (Method 5-95 AB, ESI): t_{R} = 0.746 min, [M + H]⁺ = 916.4; ¹H NMR (400 MHz, MeOH-*d*₄) δ 8.78 (s, 1H), 8.37-8.30 (m, 3H), 7.40-7.31 (m, 2H), 7.25-7.20 (m, 2H), 7.10 (d, *J*=8.4 Hz, 1H), 6.92 (s, 1H), 6.83 (s, 1H), 6.38 (s, 1H), 5.21-5.16 (m, 1H), 4.84-4.80 (m, 2H), 4.33-4.17 (m, 4H), 4.20 (s, 2H), 3.50-3.46 (m, 1H), 3.27-3.10 (m, 7H), 2.95 (s, 3H), 2.71 (s, 3H), 2.60-2.50 (m, 1H), 2.34-2.29 (m, 1H), 2.19-2.15 (m, 1H), 1.92-1.87 (m, 4H), 1.82-1.77 (m, 2H), 1.55-1.45 (m, 4H), 1.36 (d, J=7.2 Hz, 3H).

### Example 34: Synthesis of Compound 234

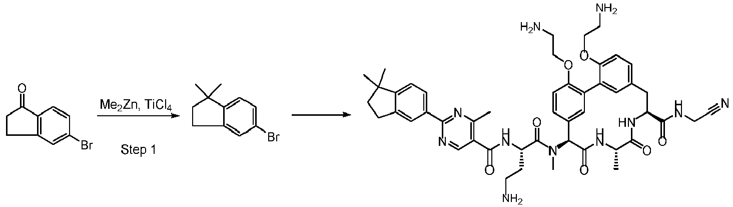

Step 1: To a solution of TiCl₄ (3.2 mL, 28.4 mmol) in DCM (10 mL) was added Me₂Zn (1N in toluene, 28.4 mL) at -78 °C and the resulting orange-brown solution was stirred vigorously at the same temperature for 1 h, followed by the dropwise addition of a solution of 5-bromo-2,3-dihydro-1 -inden-1-one (1.0g, 4.74 mmol) in DCM (20 mL). The mixture was stirred at -78 °C for 2 h; then allowed to reach -10 °C before quenching with ice-cold saturated aqueous NH₄Cl solution. The organic layer was separated and the aqueous layer was extracted with DCM (2 × 40 mL). The combined organic layers were dried over Na₂SO₄, concentrated and the residue was purified by silica-gel column, eluting with petroleum ether, to give 5-bromo-1,1-dimethyl-2,3-dihydro-1*H*-indene (450 mg, 42% yield) as a yellow oil. 1H NMR (400 MHz, CDCl₃) δ 7.35 (s, 1H), 7.20 (d, *J*=8.0 Hz, 1H), 7.02 (d, *J*=8.0 Hz, 1H), 2.90 (t, *J*=7.2 Hz, 2H), 1.95 (t, *J*=7.2 Hz, 2H), 1.27 (s, 6H).

Compound 234 (formic acid salt) was prepared as a white solid from Compound **101-K** and 5-bromo-1,1-dimethyl-2,3-dihydro-1*H*-indene by utilizing methods analogous to those described in Example 10 and Example J. LCMS (Method 5-95 AB, ESI): t_{R} = 0.725 min, [M + H]⁺ = 902.6; ¹H NMR (400 MHz, MeOH-*d*₄) δ 8.74 (s, 1H), 8.52 (br s, 1H), 8.18 (d, *J*=8.0 Hz, 1H), 8.13 (s, 1H), 7.31-7.21(m, 3H), 7.12-7.05 (m, 2H), 6.85 (s, 1H), 6.60 (s, 1H), 6.54 (s, 1H), 5.20-5.15 (m, 1H),4.82-4.69 (m, 2H), 4.34-4.18 (m, 6H), 3.23-3.12 (m, 8H), 2.95 (br s, 5H), 2.65 (s, 3H), 2.20-2.17 (m, 2H), 2.01 (t, *J*=6.8 Hz, 2H), 1.36 (d, *J*=6.8 Hz, 3H), 1.32 (s, 6H).

### Example 35: Synthesis of Compound 235

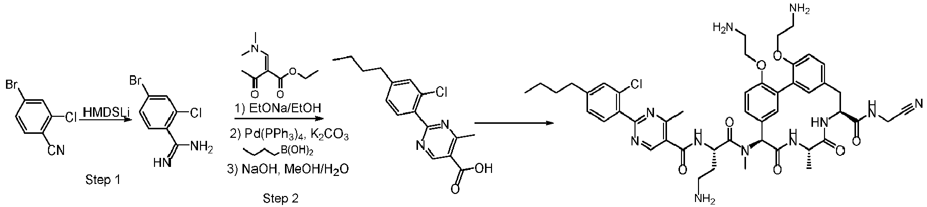

Step 1: To a solution of 4-bromo-2-chlorobenzonitrile (2.0 g, 9.2 mmol) in THF (20 mL) at 0 °C was added HMDSLi (1N solution in THF, 13.9 mL) dropwise and the reaction was stirred at 0 °C for 16 h, followed by the addition of aqueous HCl (1N, 10 mL). The resulting precipitate was collected, washed with EtOAc and dried under vacuum to give 4-bromo-2-chlorobenzimidamide (4.0 g, 93% yield).

Step 2: 2-(4-Butyl-2-chlorophenyl)-4-methylpyrimidine-5-carboxylic acid was prepared as a white solid from 4-bromo-2-chlorobenzimidamide by utilizing methods analogous to those described in Example 26.

Compound 235 (formic acid salt) was prepared as a white solid from Compound **101-K** and 2-(4-butyl-2-chlorophenyl)-4-methylpyrimidine-5-carboxylic acid by utilizing methods analogous to those described in Example G. LCMS (Method 5-95 AB, ESI): t_{R} = 0.713 min, [M + H]⁺ = 924.4; ¹H NMR (400 MHz, MeOH-*d*₄) δ 8.86 (s, 1H), 8.50 (br s, 1H), 7.62 (d, *J*=8.4 Hz, 1H), 7.40 (s, 1H), 7.35-7.28 (m, 2H), 7.25 (d, *J*=8.4 Hz, 1H), 7.20 (d, *J*=8.8 Hz, 1H), 7.10 (d, *J*=8.8 Hz, 1H), 6.92 (s, 1H), 6.82 (s, 1H), 6.40 (s, 1H), 5.23-5.16 (m, 1H), 4.81-4.78 (m, 2H), 4.27-4.17 (m, 4H), 4.20 (s, 2H), 3.36-3.13 (m, 8H), 2.96 (s, 3H), 2.74-2.69 (m, 2H), 2.71 (s, 3H), 2.34-2.27 (m, 1H), 2.21-2.15 (m, 1H), 1.71-1.62 (m, 2H), 1.46-1.35 (m, 5H), 0.98 (t, *J*=7.2 Hz, 3H).

### Example 36: Synthesis of Compound 236

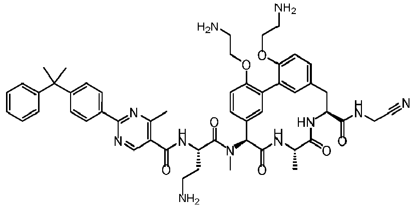

Compound 236 (formic acid salt) was prepared as a white solid from (4-bromophenyl)(phenyl)methanone and Compound **101-K** by utilizing methods analogous to those described in Example 34. LCMS (Method 5-95 AB, ESI): t_{R} = 0.632 min, [M + H]⁺ = 952.4; ¹H NMR (400 MHz, MeOH-*d*₄) δ 8.74 (s, 1H), 8.49 (br s, 2H), 8.27 (d, *J*=7.6 Hz, 2H), 7.36 (d, *J*=8.4 Hz, 2H), 7.32-7.10 (m, 8H), 7.03 (d, *J*=8.4 Hz, 1H), 6.86 (s, 1H), 6.67 (s, 1H), 6.47 (s, 1H), 5.21-5.14 (m, 1H), 4.79-4.75 (m, 2H), 4.32-4.13 (m, 6H), 3.25-3.07 (m, 8H), 2.95 (s, 3H), 2.66 (s, 3H), 2.37-2.23 (m, 1H), 2.21-2.08 (m, 1H), 1.73 (s, 6H), 1.35 (d, *J*=6.8 Hz, 3H).

### Example 37: Synthesis of Compound 237

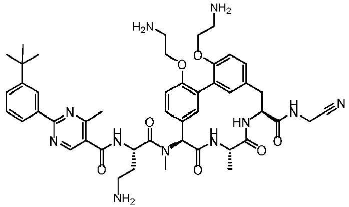

Compound 237 (formic acid salt) was prepared as a white solid from Compound **101-K** by utilizing methods analogous to those described in Example J. LCMS (Method 5-95 AB, ESI): t_{R} = 0.706 min, [M + H]⁺ = 890.7; ¹H NMR (400 MHz, MeOH-*d*₄) δ 8.81 (s, 1H), 8.50 (s, 1H), 8.45 (br s, 1H), 8.26 (d, *J*=8.0 Hz, 1H), 7.61 (d, *J*=8.0 Hz, 1H), 7.44 (t, *J*=8.0 Hz, 1H), 7.37-7.30 (m, 1H), 7.26-7.17 (m, 2H), 7.09 (d, *J*=8.0 Hz, 1H), 6.91 (d, *J*=2.0 Hz, 1H), 6.82 (s, 1H), 6.39 (s, 1H), 5.23-5.16 (m, 1H), 4.80-4.76 (m, 2H), 4.29-4.15 (m, 6H), 3.27-3.09 (m, 8H), 2.96 (s, 3H), 2.72 (s, 3H), 2.35-2.26 (m, 1H), 2.22-2.13 (m, 1H), 1.40 (s, 9H), 1.36 (d, *J*=6.5 Hz,3H).

### Example 38: Synthesis of Compound 238

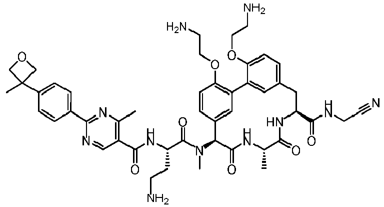

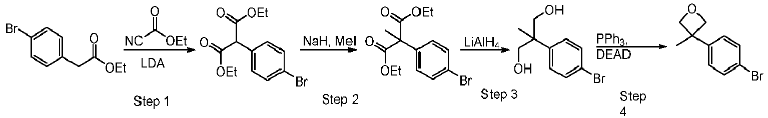

Step 1: A mixture of ethyl 2-(4-bromophenyl)acetate (10.0 g, 41 mmol) and lithium diisopropylamide (2N in THF, 41 mL) in dry THF (60 mL) was stirred at -78 °C for 0.5 h, followed by the addition of ethyl cyano-formate (4.14 mL, 45 mmol). The resulting mixture was gradually warmed up to 20 °C while stirring and was stirred at the same temperature for 18 h. The reaction was quenched with water (30 mL), which was partitioned between 1N aqueous HCl (150 mL) and DCM (150 mL). The organic layer was dried over Na₂SO₄ and concentrated and the residue was purified by silica-gel chromatography, eluting with 0-20% EtOAc in petroleum ether, to give diethyl 2-(4-bromophenyl)malonate (8.5 g, 66% yield) as colorless oil.

Step 2: A mixture of diethyl 2-(4-bromophenyl)malonate (10.0 g, 32 mmol) and NaH (60% dispersion in oil, 2.5 g, 64 mmol) in dry THF (80 mL) was stirred at 0 °C for 0.5 h, followed by the addition of iodomethane (6.0 mL, 96 mmol). The resulting mixture was gradually warmed up to 20 °C while stirring and was stirred at the same temperature for 16 h. The reaction was partitioned between 1N aqueous HCl (150 mL) and DCM (150 mL). The organic layer was dried over Na₂SO₄ and concentrated and the residue was purified by silica-gel chromatography, eluting with 0-20% EtOAc in petroleum ether, to give diethyl 2-(4-bromophenyl)-2-methylmalonate (5.2 g, 50% yield) as colorless oil.

Step 3: A mixture of diethyl 2-(4-bromophenyl)-2-methylmalonate (5.2 g, 15.8 mmol) and LiAlH₄ (3.0 g, 79 mmol) was stirred at 0 °C for 5 h. The mixture was quenched by water (20 mL), which was partitioned between 1N aqueous HCl (100 mL) and DCM (150 mL). The organic layer was dried over Na₂SO₄ and concentrated and the residue was purified by silica-gel chromatography, eluting with EtOAc/petroleum ether (1:1) to give 2-(4-bromophenyl)-2-methylpropane-1,3-diol (2.3 g, 59% yield) as a white solid.

Step 4: A mixture of 2-(4-bromophenyl)-2-methylpropane-1,3-diol (2.0 g, 8.2 mmol), triphenylphosphine (4.3 g, 16.4 mmol) and diisopropyl azodicarboxylate (3.2 mL, 16.4 mmol) in toluene (20 mL) was heated under microwave irradiation at 140 °C for 1 h. The volatiles were removed and the residue was purified by silica-gel column, eluting with 10% EtOAc/petroleum ether to give 3-(4-bromophenyl)-3-methyloxetane (0.58 g, 31% yield) as colorless oil. ¹H NMR (400MHz, CD₃Cl) δ 7.48 (d, *J*=8.4 Hz, 2H), 7.09 (d, *J*=8.4 Hz, 2H), 4.92 (d, *J*=5.2 Hz, 2H), 4.63 (d, *J*=5.2 Hz, 2H), 1.71 (s, 3H).

Compound 238 (formic acid salt) was prepared as a white solid from Compound **101-K** and 3-(4-bromophenyl)-3-methyloxetane by utilizing methods analogous to those described in Example 10 and Example J. LCMS (Method 5-95 AB, ESI): t_{R} = 0.618 min, [M + H]⁺ = 904.7; ¹H NMR (400 MHz, MeOH-*d*₄) δ 8.80 (s, 1H), 8.46 (br s, 3H), 8.43 (s, 1H), 7.38 (d, *J*=8.0 Hz, 2H), 7.33 (d, *J*=8.4 Hz,1H), 7.23-7.19 (m, 2H), 7.09 (d, *J*=8.4 Hz), 6.91 (s, 1H), 6.77 (s, 1H),6.42 (s,1H), 5.20-5.16 (m, 1H), 5.03-5.00 (m, 2H), 4.83-4.80 (m, 2H), 4.73-4.70 (m, 2H), 4.30-4.20 (m, 6H), 3.25-3.12 (m, 8H), 2.96 (s, 3H), 2.71 (s, 3H), 2.30-2.16 (m, 2H), 1.76 (s, 3H), 1.36 (d, *J*=6.8 Hz, 3H).

### Example 39: Synthesis of Compound 239

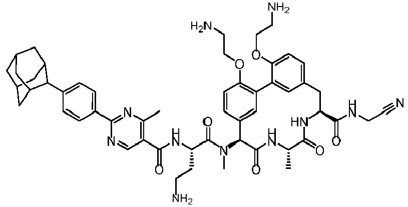

Compound 239 (formic acid salt) was prepared as a white solid from Compound **101-K** by utilizing methods analogous to those described in Example 17. LCMS (Method 5-95 AB, ESI): t_{R} = 0.790 min, [M + H]⁺ = 968.6; ¹H NMR (400 MHz, MeOH-*d*₄) δ 8.78 (s, 1H), 8.51 (br s, 3H), 8.36 (d, *J*=8.0 Hz, 2H), 7.52 (d, *J*=8.4 Hz, 2H), 7.33 (d, *J*=8.0 Hz, 1H), 7.23-7.17 (m, 2H), 7.08 (d, *J*=8.4 Hz, 1H), 6.89 (s, 1H), 6.73 (s, 1H), 6.46 (s, 1H), 5.17-5.21 (m, 1H), 4.81-4.79 (m, 2H), 4.31-4.19 (m, 6H), 3.10-3.22 (m, 8H), 2.97 (s, 3H), 2.69 (s, 3H), 2.56 (br s, 2H), 2.27-2.32 (m, 1H), 2.19-2.16 (m, 1H), 2.00-2.10 (m, 6H), 1.78-1.94 (m, 6H), 1.70-1.62 (m, 2H), 1.36 (d, *J*=7.2 Hz, 3H).

### Example 40: Synthesis of Compound 240

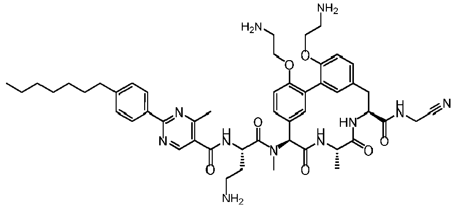

Compound 240 (formic acid salt) was prepared as a white solid from Compound **101-K** by utilizing methods analogous to those described in Example 26. LCMS (Method 5-95 AB, ESI): t_{R} = 0.774 min, [M + H]⁺ = 932.7; ¹H NMR (400 MHz, MeOH-*d*₄) δ 8.75 (s, 1H), 8.48 (br s, 2H), 8.29 (d, *J*=8.0 Hz, 2H), 8.31 (d, *J*=8.0 Hz, 2H), 7.24-7.13 (m, 2H), 7.08 (d, *J*=8.0 Hz, 2H), 6.88 (s, 1H), 6.71 (s, 1H), 6.47 (s, 1H), 5.21-5.16 (m, 1H), 4.82-4.75 (m, 2H), 4.35-4.16 (m, 4H), 4.21 (s, 2H), 3.27-3.05 (m, 8H), 2.96 (s, 3H), 2.76-2.64 (m, 2H), 2.67 (s, 3H), 2.32-2.27 (m, 1H), 2.21-2.14 (m, 1H), 1.71-1.64 (m, 2H), 1.45-1.25 (m, 11H), 0.90 (t, *J*=6.8 Hz 3H).

### Example 41: Synthesis of Compound 241

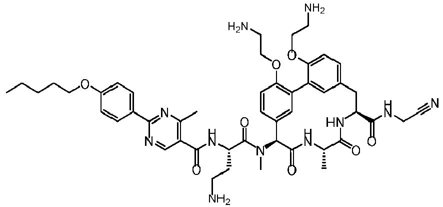

Compound 241 (formic acid salt) was prepared as a white solid from Compound **101-K** by utilizing methods analogous to those described in Example J. LCMS (Method 5-95 AB, ESI): t_{R} = 0.731 min, [M + H]⁺ = 920.4; ¹H NMR (400 MHz, MeOH-*d*₄) δ 8.72 (s, 1H), 8.51 (br s, 2H), 8.33 (d, *J*=8.4 Hz, 2H), 7.31 (d, *J*=8.0 Hz, 1H), 7.23-7.16 (m, 2H), 7.08 (d, *J*=8.0 Hz, 1H), 7.01 (d, *J*=8.4 Hz, 2H), 6.88 (s, 1H), 6.71 (s, 1H), 6.48 (s, 1H), 5.22-5.15 (m, 1H), 4.85-4.79 (m, 2H), 4.35-4.13 (m, 6H), 4.07 (t, *J*=6.4 Hz, 2H), 3.29-3.01 (m, 8H), 2.96 (s, 3H), 2.66 (s, 3H), 2.35-2.24 (m, 1H), 2.22-2.15 (m, 1H), 1.88 - 1.78 (m, 2H), 1.50-1.38 (m, 6H), 1.35 (d, *J*=6.8 Hz, 3H), 0.98 (t, *J*=7.2 Hz, 3H).

### Example 42: Synthesis of Compound 242

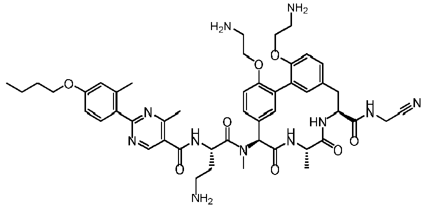

Compound 242 (formic acid salt) was prepared as a white solid from Compound **101-K** by utilizing methods analogous to those described in Example J. LCMS (Method 5-95 AB, ESI): t_{R} = 0.713 min, [M + H]⁺ = 920.5; ¹H NMR (400 MHz, MeOH-*d*₄) δ 8.80 (s, 1H), 7.82 (d, *J*=8.8 Hz, 1H), 7.36 (d, *J*=8.4 Hz, 1H), 7.26 (d, *J*=8.4 Hz, 1H), 7.20 (d, *J*=8.8 Hz, 1H), 7.11 (d, *J*=8.8 Hz, 1H), 6.92 (s, 1H), 6.89-6.85 (m, 2H), 6.83 (s, 1H), 6.39 (s, 1H), 5.21-5.16 (m, 1H), 4.84-4.79 (m, 2H), 4.34-4.18 (m, 6H), 4.05 (t, *J*=6.4 Hz, 2H), 3.37-3.11 (m, 8H), 2.96 (s, 3H), 2.70 (s, 3H), 2.56 (s, 3H), 2.32-2.27 (m, 1H), 2.21-2.15 (m, 1H), 1.82-1.75 (m, 2H), 1.61 - 1.48 (m, 4H), 1.36 (d, *J*=7.2 Hz, 3H), 1.01 (t, *J*=7.6 Hz, 3H).

### Example 43: Synthesis of Compound 243

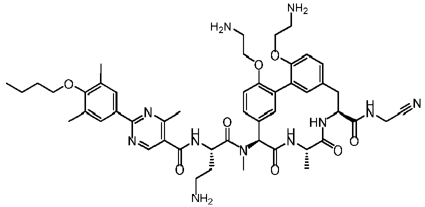

Compound 243 (formic acid salt) was prepared as a white solid from Compound **101-K** by utilizing methods analogous to those described in Example J. LCMS (Method 5-95 AB, ESI): t_{R} = 0.731 min, [M + H]⁺ = 934.5; ¹H NMR (400 MHz, MeOH-*d*₄) δ 8.75 (s, 1H), 8.08 (s, 2H), 7.33-7.28 (m, 1H), 7.23-7.17 (m, 2H), 7.11-7.05 (m, 1H), 6.88 (s, 1H), 6.73 (s, 1H), 6.45 (s, 1H), 5.20-5.14 (m, 1H), 4.82-4.75 (m, 2H), 4.29-4.17 (m, 6H), 3.89-3.83 (m, 2H), 3.25-3.10 (m, 8H), 2.96 (s, 3H), 2.67 (s, 3H), 2.43 (s, 6H), 2.33-2.10 (m, 2H), 1.89- 1.77 (m, 2H), 1.66-1.54 (m, 2H), 1.36 (d, J=6.8 Hz, 3H), 1.04 (t, J=7.2 Hz, 3H).

### Example 44: Synthesis of Compound 244

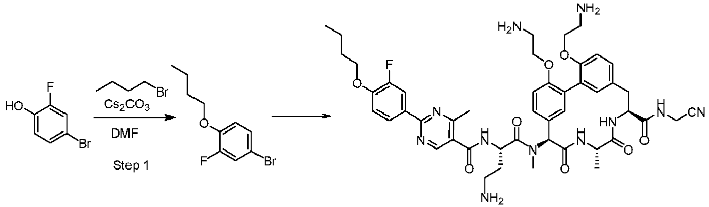

Step 1: A mixture of 4-bromo-2-fluorophenol (1.0 g, 5.2 mmol), 1-bromobutane (1.1 g, 7.8 mmol), and Cs₂CO₃ (5.1 g, 15.7 mmol) in DMF (20 mL) was stirred at 20 °C for 16 h under N₂. The volatiles were removed and the residue was re-dissolved with EtOAc (100 mL), which was washed by brine (2 × 100 mL). The organic layer was dried over Na₂SO₄, concentrated and the residue was purified by silica-gel column, eluting with petroleum ether, to give 4-bromo-1-butoxy-2-fluorobenzene (1.1 g, 85% yield) as a yellow oil.

Compound 244 (formic acis salt) was prepared as a white solid from Compound **101-K** and 4-bromo-1-butoxy-2-fluorobenzene by utilizing methods analogous to those described in Example 10 and Example J. LCMS (Method 5-95 AB, ESI): t_{R} = 0.720 min, [M + H]⁺ = 924.4; ¹H NMR (400 MHz, MeOH-*d*₄) δ 8.72 (s, 1H), 8.50 (br s, 1H), 8.22 (d, *J*=8.4 Hz, 1H), 8.07 (d, *J*=8.4 Hz, 1H), 7.32 (d, *J*=8.4 Hz, 1H), 7.25-7.15 (m, 3H), 7.08 (d, *J*=8.4 Hz, 1H), 6.88 (s, 1H), 6.69 (s, 1H), 6.47 (s, 1H), 5.22-5.15 (m, 1H), 4.82-4.77 (m, 2H), 4.39-4.19 (m, 6H), 4.16 (t, *J*=6.0 Hz, 2H), 3.29-3.03 (m, 8H), 2.96 (s, 3H), 2.66 (s, 3H), 2.31-2.28 (m, 1H), 2.19-2.16 (m, 1H), 1.90-1.79 (m, 2H), 1.60-1.52 (m, 2H), 1.36 (d, *J*=7.2 Hz, 3H), 1.03 (t, *J*=7.2 Hz, 3H).

### Example 45: Synthesis of Compound 245

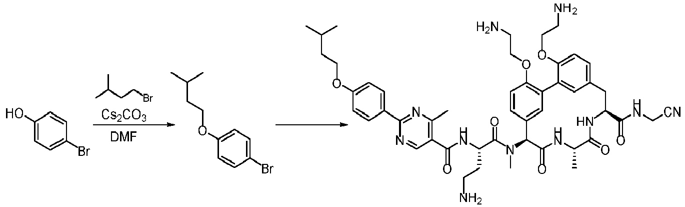

Compound 245 (formic acid salt) was prepared as a white solid from Compound **101-K** and 1-bromo-4-(isopentyloxy)benzene by utilizing methods analogous to those described in Example 244. LCMS (Method 5-95 AB, ESI): t_{R} = 0.623 min, [M + H]⁺ = 920.6; ¹H NMR (400 MHz, MeOH-*d*₄) δ 8.71 (s, 1H), 8.51 (br s, 1H), 8.28 (br s, 2H), 7.32-7.30 (m, 1H), 7.22-7.15 (m, 2H), 7.09-7.07 (m, 1H), 7.05-7.01 (m, 2H), 6.87 (s, 1H), 6.66 (br s, 1H), 6.48 (br s, 1H), 5.19-5.18 (m, 1H), 4.85-4.78 (m, 2H), 4.29-4.18 (m, 6H), 4.11 (t, *J*=6.4 Hz, 2H), 3.31-3.13 (m, 8H), 2.96 (s, 3H), 2.65 (s, 3H), 2.28-2.17 (m, 1H), 2.17-2.15 (m, 1H), 1.90-1.86 (m, 1H), 1.74-1.69 (m, 2H), 1.36 (d, *J*=6.4 Hz, 3H), 1.01 (t, *J*=6.4 Hz, 6H).

### Example 46: Synthesis of Compound 246

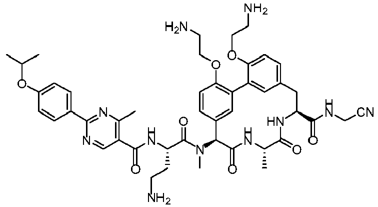

Compound 246 (formic acid salt) was prepared as a white solid from Compound **101-K** by utilizing methods analogous to those described in Example J. LCMS (Method 5-95 AB, ESI): t_{R} = 0.556 min, [M + H]⁺ = 892.4; ¹H NMR (400 MHz, MeOH-*d*₄) δ 8.70 (s, 1H), 8.49 (br s, 1H), 8.29 (d, *J*=8.8 Hz, 2H), 7.35-7.28 (m, 1H), 7.24-7.13 (m, 2H), 7.08 (d, *J*=8.4 Hz, 1H), 6.98 (d, *J*=8.8 Hz, 2H), 6.87 (d, *J*=2.2 Hz, 1H), 6.68 (s, 1H), 6.48 (s, 1H), 5.22-5.13 (m, 1H), 4.80-4.71 (m, 2H), 4.36-4.12 (m, 7H), 3.26-3.07 (m, 8H), 2.96 (s, 3H), 2.65 (s, 3H), 2.32-2.24 (m, 1H), 2.22-2.13 (m, 1H), 1.38-1.3 (m, 9H).

### Example 47: Synthesis of Compound 247

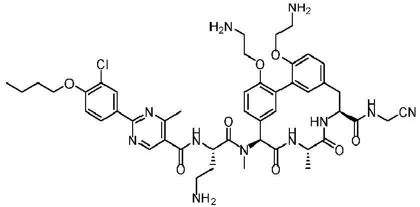

Compound 247 (formic acid salt) was prepared as a white solid from Compound **101-K** by utilizing methods analogous to those described in Example J. LCMS (Method 5-95 AB, ESI): t_{R} = 0.632 min, [M + H]⁺ = 940.8; ¹H NMR (400 MHz, MeOH-*d*₄) δ 8.72 (s, 1H), 8.50 (br s, 2H), 8.40-8.30 (m, 2H), 7.31 (d, *J*=8.8 Hz, 1H), 7.25-7.10 (m, 3H), 7.07 (d, *J*=8.8 Hz, 1H), 6.86 (s, 1H), 6.63 (s, 1H), 6.51 (s, 1H), 5.25-5.15 (m, 1H), 4.80-4.75 (m, 2H), 4.40-4.20 (m, 6H), 4.17 (t, *J*=6.4 Hz, 2Hz), 3.40-3.05 (m, 8H), 2.96 (s, 3H), 2.66 (s, 3H), 2.35-2.25 (m, 1H), 2.20-2.15 (m, 1H), 1.90-1.80 (m, 2H), 1.62-1.55 (m, 2H), 1.36 (d, *J*=6.8 Hz, 3H), 1.04 (t, *J*=7.6 Hz, 3H).

### Example 48: Synthesis of Compound 248

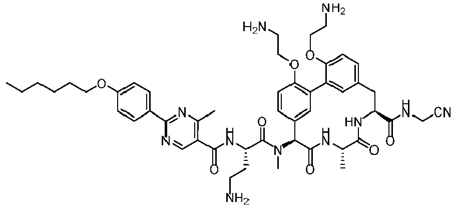

Compound 248 (formic acid salt) was prepared as a white solid from Compound **101-K** by utilizing methods analogous to those described in Example J. LCMS (Method 5-95 AB, ESI): t_{R} = 0.634 min, [M + H]⁺ = 934.7; ¹H NMR (400 MHz, MeOH-*d*₄) δ 8.74 (s, 1H), 8.49 (br s, 2H), 8.38 (br s, 2H), 7.32 (d, *J*=8.4 Hz, 1H), 7.23-7.17 (m, 2H), 7.09 (d, *J*=8.4 Hz, 1H), 7.05-6.99 (m, 2H), 6.91 (s, 1H), 6.78 (br s, 1H), 6.42 (br s, 1H), 5.20-5.14 (m, 1H), 4.81-4.75 (m, 2H), 4.25-4.16(m, 6H), 4.07 (t, *J*=6.4 Hz, 2H), 3.48 (br s, 1H), 3.27-3.07 (m, 7H), 2.95 (s, 3H), 2.68 (s, 3H), 2.37-2.20 (m, 1H), 2.20-2.07 (m, 1H), 1.88-1.75 (m, 3H), 1.51- 1.49 (m, 2H), 1.44-1.29 (m, 6H), 0.94 (t, *J*=7.2 Hz, 3H).

### Example 49: Synthesis of Compound 249

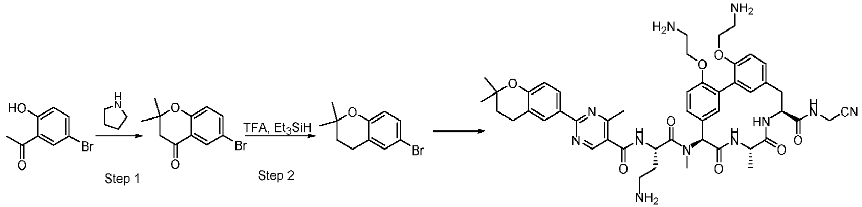

Step 1: A mixture of 1-(5-bromo-2-hydroxyphenyl)ethan-1-one (1.0 g, 4.65 mmol) and pyrrolidine (0.78 mL, 9.3 mmol) in toluene (8 mL) was stirred at 25 °C for 10 min, followed by the addition of acetone (3 mL). The resulting mixture was stirred at the same temperature for 16 h. The volatiles were removed and the residue was taken up by EtOAc (50 mL), which was washed with brine (2 × 50 mL). The organic layer was dried over Na₂SO₄, concentrated and the residue was purified by silica-gel column, eluting with 0-5% EtOAc in petroleum ether, to give 6-bromo-2,2-dimethylchroman-4-one (850 mg, 72% yield) as yellow oil.

Step 2: A mixture of 6-bromo-2,2-dimethylchroman-4-one (650 mg, 2.55 mmol) and triethylsilane (1.45 g, 12.8 mmol) in TFA (6 mL) was stirred at 0 °C for 12 h. The volatiles were removed and the residue was taken up by EtOAc (50 mL), which was washed with saturated aqueous NaHCO₃ and brine (each 50 mL). The organic layer was dried over Na₂SO₄, concentrated and the residue was purified by silica-gel column, eluting with 1-5% EtOAc in petroleum ether, to give 6-bromo-2,2-dimethylchromane (490 mg, 80% yield) as a colorless oil. ¹H NMR (400 MHz, CDCl₃) δ 7.19-7.16 (m, 2H), 6.66 (d, *J*=8.4 Hz, 1H), 2.76 (t, *J*=6.8 Hz, 1H), 1.79 (t, *J*=6.8 Hz, 1H), 1.33 (s, 6H).

Compound 249 (formic acid salt) was prepared as a white solid from Compound **101-K** and 6-bromo-2,2-dimethylchromane by utilizing methods analogous to those described in Example 10 and Example J. LCMS (Method 5-95 AB, ESI): t_{R} = 0.709 min, [M + H]⁺ = 918.5; ¹H NMR (400 MHz, MeOH-*d*₄) δ 8.68 (s, 1H), 8.46 (br s, 1H), 8.12-8.06 (m, 2H), 7.31 (d, *J*=8.0 Hz, 1H), 7.22 (d, *J*=8.4 Hz, 1H), 7.16 (d, *J*=8.0 Hz, 1H), 7.08 (d, *J*=8.4 Hz, 1H), 6.86 (s, 1H), 6.79 (d, *J*=8.4 Hz, 1H), 6.63 (s, 1H), 6.52 (s, 1H), 5.21-5.15 (m, 1H), 4.81-4.70 (m, 2H), 4.36-4.21 (m, 6H), 3.29-3.15 (m, 8H), 2.97 (s, 3H), 2.88 (d, *J*=6.4 Hz, 2H), 2.64 (s, 3H), 2.35-2.25 (m, 1H), 2.22-2.12 (m, 1H), 1.89 (t, *J*=6.4 Hz, 2H), 1.38 (s, 6H), 1.36 (d, *J*=7.2 Hz, 3H).

### Example 50: Synthesis of Compound 250

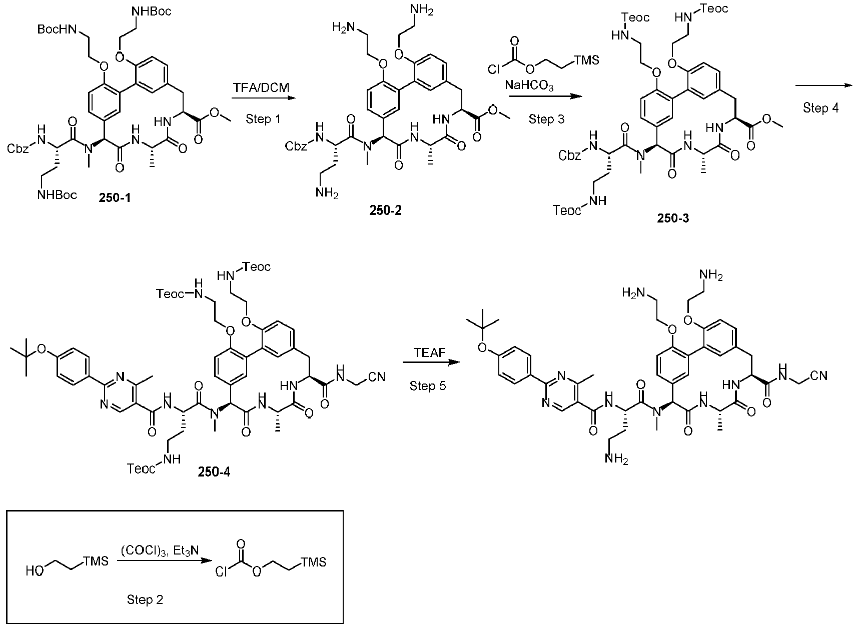

Step 1: Typical Boc removal (TFA/DCM) condition was applied to compound **250-1** (prepared following procedures analogous to those described for Compound 101-H) to give compound **250-2.**

Step 2: To a solution of (COCl)₃ (916 mg, 2.85 mmol) in dry diethyl ether (6 mL) was added 2-(triethylsilyl)-ethanol (1.0 g, 8.46 mmol) and pyridine (535 mg, 6.77 mmol) and the mixture was stirred at -30 °C for 4 h. After filtration, the filtrate was evaporated under reduced pressure and the residue was distilled to give 2-(trimethylsilyl)ethyl carbonochloridate (1.0 g, 65% yield).

Step 3: A solution of 2-(trimethylsilyl)ethyl carbonochloridate (580 mg, 3.21 mmol) in 1,4-dioxane (10 mL) was treated with saturated aqueous NaHCO₃ solution until pH~7-8, followed by the addition of compound **250-2** (400 mg, 0.53 mmol). The resulting mixture was stirred at 20 °C for 2 h. The volatiles were removed and the residue was taken up by EtOAc (30 mL), which was washed with saturated brine (30 mL). The organic layer was dried over Na₂SO₄, concentrated and the residue was purified by Prep-TLC (DCM/MeOH=10:1, Rf=0.4) to give compound **250-3** (500 mg, 79% yield) as a yellow solid. LCMS (Method 5-95 AB, ESI): t_{R} = 1.007 min, [M + H]⁺ = 1180.7.

Step 4: Compound **250-4** was prepared as a white solid from compound **250-3** by utilizing methods analogous to those described in Example 10 and Example J. LCMS (Method 5-95 AB, ESI): t_{R} = 1.048 min, [M + H]⁺ = 1339.7.

Step 5: A mixture of compound **250-4** (70 mg, 0.05 mmol) and tetraethylammonium fluoride (1N in THF, 0.26 mL) in THF (3 mL) was stirred at 60 °C for 16 h. The volatiles were removed and the residue was purified by HPLC, eluting with 14-45% acetonitrile (0.225% formic acid) in water, to give Compound 250 (formic acid salt) (31.7 mg, 67% yield) as a white solid. LCMS (Method 5-95 AB, ESI): t_{R} = 0.694 min, [M + Na]⁺ = 928.5; ¹H NMR (400 MHz, MeOH-*d*₄) δ 8.76 (s, 1H), 8.43 (br s, 3H), 8.35 (d, *J*=8.4 Hz, 2H), 7.32 (d, *J*=8.4 Hz, 1H), 7.23-7.17 (m, 2H), 7.13-7.07 (m, 3H), 6.89 (s, 1H), 6.76 (s, 1H), 6.42 (s, 1H), 5.20-5.15 (m, 1H), 4.85-4.75 (m, 2H), 4.30-4.18 (m, 6H), 3.28-3.11 (m, 8H), 2.95 (s, 3H), 2.68 (s, 3H), 2.40-2.30 (m, 1H), 2.20-2.14 (m, 1H), 1.43 (s, 9H), 1.36 (d, *J*=7.2 Hz, 3H).

### Example 51: Synthesis of Compound 251

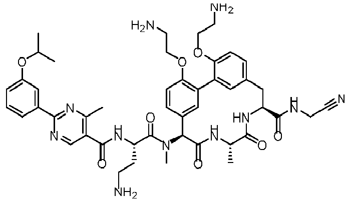

Compound 251 (formic acid salt) was prepared as a white solid from Compound **101-K** by utilizing methods analogous to those described in Example J. LCMS (Method 5-95 AB, ESI): t_{R} = 0.663 min, [M + H]⁺ = 892.5; ¹H NMR (400 MHz, MeOH-*d*₄) δ 8.78 (s, 1H), 8.48 (br s, 1H), 7.97-7.93 (m, 2H), 7.38 (d, *J*=8.0 Hz, 1H), 7.32 (d, *J*=8.0 Hz, 1H), 7.20 (d, *J*=8.4 Hz, 2H), 7.08 (d, *J*=8.4 Hz, 2H), 6.89 (s, 1H), 6.72 (s, 1H), 6.46 (s, 1H), 5.21-5.17 (m, 1H), 4.83-4.80 (m, 2H), 4.72-4.68 (m, 1H), 4.31-4.20 (m, 6H), 3.25-3.13 (m, 8H), 2.96 (s, 3H), 2.69 (s, 3H), 2.21-2.18 (m, 1H), 2.17-2.13 (m, 1H), 1.38-1.35 (m, 9H).

### Example 52: Synthesis of Compound 252

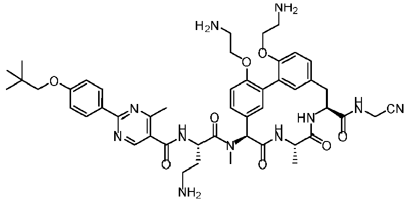

Compound 252 (formic acid salt) was prepared as a white solid from Compound **101-K** by utilizing methods analogous to those described in Example 10 and Example J. LCMS (Method 5-95 AB, ESI): t_{R} = 0.619 min, [M + H]⁺ = 920.3; ¹H NMR (400 MHz, MeOH-*d*₄) δ 8.75 (s, 1H), 8.52 (br s, 2H), 8.37 (d, *J*=8.4 Hz, 2H), 7.34 (d, *J*=8.0 Hz, 1H), 7.22 (d, *J*=8.4 Hz, 2H), 7.11 (d, *J*=8.0 Hz, 1H), 7.05 (d, *J*=8.0 Hz, 2H), 6.91 (d, *J*=1.8 Hz, 1H), 6.76 (s, 1H), 6.46 (s, 1H), 5.23-5.16 (m, 1H), 4.85-4.78 (m, 2H), 4.32-4.19 (m, 6H), 3.75 (s, 2H), 3.28-3.12 (m, 8H), 2.97 (s, 3H), 2.69 (s, 3H), 2.36-2.27 (m, 1H), 2.23-2.11 (m, 1H), 1.38 (d, *J*=6.8 Hz, 3H), 1.10 (s, 9H).

### Example 53: Synthesis of Compound 253

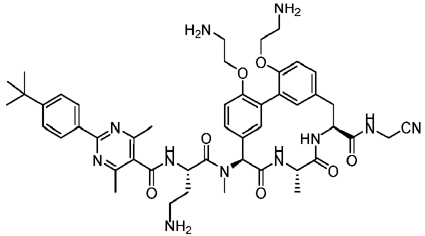

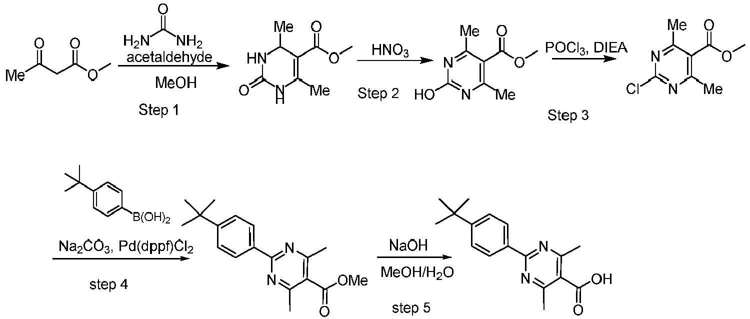

Step 1: A mixture of urea (0.52 g, 8.6 mmol), acetaldehyde (0.49 mL, 8.6 mmol), methyl 3-oxobutanoate (1.0 g, 8.6 mmol) and glacial acetic acid (1 drop) in methanol (2 mL) was stirred at 90 °C for 16 h. To the reaction mixture was added water (10 mL), followed by the filtration; the cake was then washed with water and dried in air to obtain methyl 4,6-dimethyl-2-oxo-1,2,3,4-tetrahydropyrimidine-5-carboxylate (900 mg, 56.7% yield) as a pale yellow solid.

Step 2: Methyl 4,6-dimethyl-2-oxo-1,2,3,4-tetrahydropyrimidine-5-carboxylate (900 mg, 4.9 mmol) was added in portions to an ice-cooled solution of 50% HNO₃ (4.0 mL) over 3 min. The reaction mixture was stirred at 0 °C for 10 min. The mixture was poured into ice water (20 mL), neutralized with solid K₂CO₃ and the resulting mixture was extracted with ethyl acetate (10 mL). The aqueous layer was extracted again with CHCl₃ (40 mL × 2). The organic layers were combined, dried over Na₂SO₄ and concentrated to obtain methyl 2-hydroxy-4,6-dimethylpyrimidine-5-carboxylate (500 mg, 2.7 mmol, 56.1% yield) as a pale yellow solid.

Step 3: A mixture of methyl 2-hydroxy-4,6-dimethylpyrimidine-5-carboxylate, POCl₃ (6.2 mL, 66.5 mmol), and DIPEA (1.28 g, 9.9 mmol) was stirred at 110 °C for 3 h. The mixture was evaporated *in vacuo,* diluted with ethyl acetate (40 mL), washed with saturated aqueous NaHCO₃ solution (25 mL), brine (30 mL), dried over Na₂SO₄ and evaporated *in vacuo.* The residue was purified via silica gel chromatography (0-30% ethyl acetate in petroleum ether) to give methyl 2-chloro-4,6-dimethylpyrimidine-5-carboxylate (350 mg, 1.7 mmol, 63.6% yield) as a white solid. ¹H NMR (400 MHz, CDCl3) δ 3.98 (s, 3H), 2.55 (s, 6H).

Step 4: A mixture of methyl 2-chloro-4,6-dimethylpyrimidine-5-carboxylate (4.5 g, 22.4 mmol), 4-*tert*-butylbenzene boronic acid (4.8 g, 26.9 mmol), Pd(dppf)Cl₂ (1.6 g, 2.24 mmol) and Na₂CO₃ (4.8 g, 44.9 mmol) in dioxane/water (110 mL, v/v=10/1) was stirred at 100 °C under N₂ for 16 h. The mixture was diluted with water (150 mL), and extracted with EtOAc (200 mL × 3). The combined organic layers were washed with brine (300 mL × 2), dried over Na₂SO₄, concentrated and purified by silica-gel chromatography, eluting with 0-5% EtOAc in petroleum ether, to give methyl 2-(4-(*tert*-butyl)phenyl)-4,6-dimethylpyrimidine-5-carboxylate (6.2 g, 93% yield) as a white solid. LCMS (Method 5-95 AB, ESI): t_{R} = 1.075 min, [M + H]⁺ = 299.1.

Step 5: A mixture of methyl 2-(4-(*tert*-butyl)phenyl)-4,6-dimethylpyrimidine-5-carboxylate (6.2 g, 20.8 mmol) and NaOH (1.7 g, 41.6 mmol) in MeOH/water (80 mL, v/v=1:1) was stirred at 90 °C for 4 h. The volatiles were removed under reduced pressure and the residue was acidified with 1N HCl to pH=4~5, followed by the extration with EtOAc (100 mL × 2). The combined organic layers were washed with brine (100 mL × 2), dried over Na₂SO₄ and concentrated to give 2-(4-(*tert*-butyl)phenyl)-4,6-dimethylpyrimidine-5-carboxylic acid (5.8 g, 98% yield) as a white solid. LCMS (Method 5-95 AB, ESI): t_{R} = 0.936 min, [M + H]⁺ = 285.0; ¹H NMR (400 MHz, MeOH-*d*₄) δ 8.39 (d, *J*=8.4 Hz, 2H), 7.52 (d, *J*=8.4 Hz, 2H), 2.75 (s, 6H), 1.37 (s, 9H).

Compound 253 (formic acid salt) was prepared as a white solid from Compound **101-K** and 2-(4-(*tert*-butyl)phenyl)-4,6-dimethylpyrimidine-5-carboxylic acidby utilizing methods analogous to those described in Example J. LCMS (Method 5-95 AB, ESI): t_{R} = 0.715 min, [M + H]⁺ = 904.6; ¹H NMR (400 MHz, MeOH-*d*₄) δ 8.48 (br s, 1H), 8.34 (d, *J*=8.0 Hz, 2H), 7.55 (d, *J*=8.0 Hz, 2H), 7.24-7.15 (m, 2H), 7.04 (d, *J*=8.0 Hz, 1H), 6.95-6.90 (m, 1H), 6.83 (s, 1H), 6.73 (s, 1H), 6.47 (s, 1H), 5.28-5.23 (m, 1H), 4.83-4.80 (m, 2H), 4.54-4.45 (m, 2H), 4.32-4.23 (m, 4H), 3.42-3.38 (m, 1H), 3.27-3.12 (m, 7H), 3.05 (s, 3H), 2.57 (s, 6H), 2.38-2.26 (m, 1H), 2.25-2.13 (m, 1H), 1.39 (s, 3H), 1.35 (t, *J*=7.2 Hz, 3H).

### Example 54: Synthesis of Compound 254

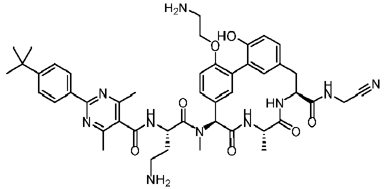

Compound 254 (formic acid salt) was prepared as a white solid from Compound **106-B2** by utilizing methods analogous to those described in Example 53 and V. LCMS (Method 5-95 AB, ESI): t_{R} = 0.776 min, [M + H]⁺ = 861.5; ¹H NMR (500 MHz, DMSO-d6) δ 9.17 (d, J = 7.3 Hz, 1H), 8.98 (d, J = 7.7 Hz, 1H), 8.71 (t, J = 5.5 Hz, 1H), 8.44 (d, J = 9.0 Hz, 1H), 8.36 - 8.29 (m, 2H), 7.59 - 7.52 (m, 2H), 7.19 - 7.04 (m, 3H), 6.90 - 6.80 (m, 2H), 6.71 (s, 1H), 6.42 (s, 1H), 5.09 - 5.01 (m, 1H), 4.80 - 4.66 (m, 2H), 4.29 - 4.15 (m, 4H), 3.19 - 3.09 (m, 3H), 3.02 - 2.88 (m, 6H), 2.50 (s, 6H), 2.14 - 2.03 (m, 1H), 2.02 - 1.91 (m, 1H), 1.35 (s, 9H), 1.21 (d, J = 6.6 Hz, 3H).

### Example 55: Synthesis of Compound 255

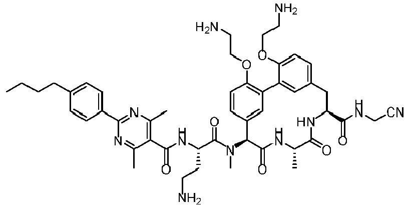

Compound 255 (formic acid salt) was prepared as a white solid from Compound **101-K** by utilizing methods analogous to those described in Example 53. LCMS (Method 5-95 AB, ESI): t_{R} = 0.715 min, [M + H]⁺ = 904.5; ¹H NMR (400 MHz, MeOH-*d*₄) δ 8.44 (br s, 2H), 8.33 (d, *J*=7.6 Hz, 2H), 7.35-7.27 (m, 3H), 7.26-7.18 (m, 2H), 7.10 (d, *J*=8.4 Hz, 1H), 6.92 (s, 1H), 6.83 (s, 1H), 6.42 (s, 1H), 5.24-5.20 (m, 1H), 4.85-4.75(m, 2H), 4.30-4.16 (m, 6H), 3.49-3.47 (m, 1H), 3.26-3.10 (m, 6H), 3.01 (s, 3H), 2.69 (t, J=7.6 Hz, 2H), 2.57 (s, 6H), 2.32-2.24 (m, 1H), 2.20-2.12 (m, 1H), 1.69-1.63 (m, 2H), 1.43-1.38 (m, 2H), 1.36 (d, *J*=6.8 Hz, 3H), 0.97 (t, *J*=7.6 Hz, 3H).

### Example 56: Synthesis of Compound 256

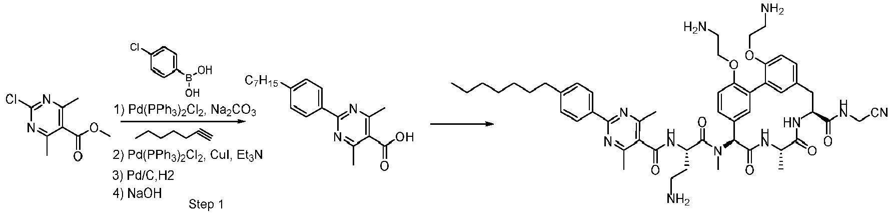

Step 1: Starting from methyl 2-chloro-4,6-dimethylpyrimidine-5-carboxylate (described in Example 53), typical Suzuki, Sonogashira, hydrogenation (Pd/C, H₂) and ester hydrolysis (NaOH, MeOH/H₂O) conditions, analogous to those described in Examples D, H, and K, were applied to give 2-(4-heptylphenyl)-4,6-dimethylpyrimidine-5-carboxylic acid as a white solid.

Compound 256 (formic acid salt) was prepared as a white solid from Compound **101-K** and 2-(4-heptylphenyl)-4,6-dimethylpyrimidine-5-carboxylic acid by utilizing methods analogous to those described in Example G. LCMS (Method 5-95 AB, ESI): t_{R} = 0.652 min, [M + H]⁺ = 946.4; ¹H NMR (400 MHz, MeOH-*d*₄) δ 8.48 (br s, 2H), 8.24-8.18 (m, 2H), 7.31-7.22 (m, 4H), 7.12-7.06 (m, 2H), 6.88 (s, 1H), 6.66 (s, 1H), 6.53 (s, 1H), 5.25-5.21 (m, 1H), 4.85-4.77 (m, 2H), 4.34 (s, 2H), 4.30-4.22 (m, 4H), 3.27-3.16 (m, 4H), 3.18-3.05 (m, 4H), 3.02 (s, 3H), 2.69 (t, *J*=7.6 Hz, 2H), 2.50 (s, 6H), 2.32-2.26 (m, 1H), 2.20-2.14 (m, 1H), 1.70-1.61 (m, 2H), 1.42-1.28 (m, 11H), 0.91 (t, J=6.8 Hz, 3H).

### Example 57: Synthesis of Compound 257

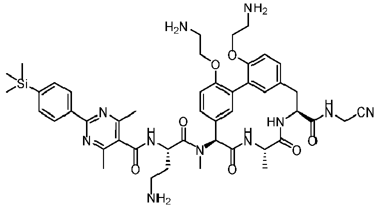

Compound 257 (formic acid salt) was prepared as a white solid from Compound **101-K** by utilizing methods analogous to those described in Example 53. LCMS (Method 5-95 AB, ESI): t_{R} = 0.737 min, [M + Na]⁺ = 942.6; ¹H NMR (400 MHz, MeOH-*d*₄) δ 8.50 (br s, 1H), 8.40 (d, *J*=8.0 Hz, 2H), 7.68 (d, *J*=8.0 Hz, 2H), 7.31 (d, *J*=8.0 Hz, 1H), 7.30-7.15 (m, 2H), 7.11 (d, *J*=8.0 Hz, 1H), 6.94 (d, *J*=2.0 Hz, 1H), 6.83 (s, 1H), 6.45 (s, 1H), 5.30-5.20 (m, 1H), 4.80-4.70 (m, 2H), 4.30-4.10 (m, 6H), 3.25-3.10 (m, 8H), 3.03 (s, 3H), 2.59 (s, 6H), 2.35-2.25 (m, 1H), 2.25-2.10 (m, 1H), 1.38 (d, J=7.2 Hz, 3H), 0.33 (s, 9H).

### Example 58: Synthesis of Compound 258

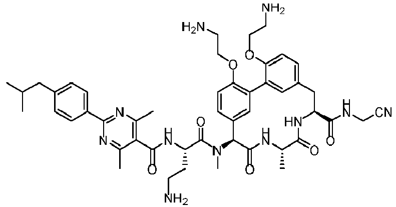

Compound 258 (formic acid salt) was prepared as a white solid from Compound **101-K** by utilizing methods analogous to those described in Example 53. LCMS (Method 5-95 AB, ESI): t_{R} = 0.733 min, [M + H]⁺ = 905.0; ¹H NMR (400 MHz, MeOH-*d*₄) δ 8.50 (br s, 1H), 8.28 (d, *J*=8.0 Hz, 2H), 7.35-7.25 (m, 3H), 7.25-7.15 (m, 2H), 7.09 (d, *J*=8.4 Hz, 1H), 6.90 (s, 1H), 6.75 (brs, 1H), 6.48 (s, 1H), 5.35-5.20 (m, 1H), 4.80-4.70 (m, 2H), 4.40-4.10 (m, 6H), 3.30-3.05 (m, 8H), 3.01 (s, 3H), 2.60-2.45 (m, 8H), 2.35-2.25 (m, 1H), 2.25-2.10 (m, 1H), 2.00-1.90 (m, 1H), 1.36 (d, *J*=7.2 Hz, 3H), 0.96 (t, J=6.8 Hz, 6H).

### Example 59: Synthesis of Compound 259

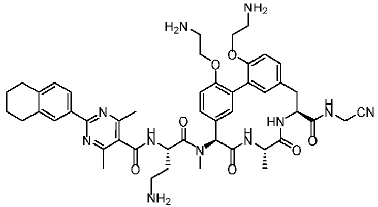

Compound 259 (formic acid salt) was prepared as a white solid from Compound **101-K** by utilizing methods analogous to those described in Example 53. LCMS (Method 5-95 AB, ESI): t_{R} = 0.621 min, [M + H]⁺ = 902.4; ¹H NMR (400 MHz, MeOH-*d*₄) δ 8.51 (brs, 2H), 8.04 (brs, 2H), 7.45-7.35 (m, 1H), 7.30-7.05 (m, 5H), 6.89 (s, 1H), 6.72 (s, 1H), 6.49 (s, 1H), 5.30-5.20 (m, 1H), 4.85-4.75 (m, 2H), 4.35-4.10 (m, 6H), 3.35-3.05 (m, 8H), 3.01 (s, 3H), 2.84 (brs, 4H), 2.52 (s, 6H), 2.30-2.20 (m, 1H), 2.20-2.10 (m, 1H), 1.86 (brs, 4H), 1.35 (d, *J*=7.2 Hz, 3H).

### Example 60: Synthesis of Compound 260

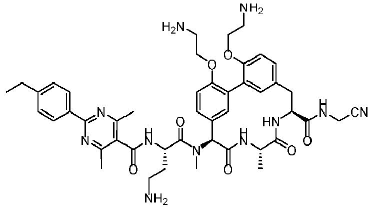

Compound 260 (formic acid salt) was prepared as a white solid from Compound **101-K** by utilizing methods analogous to those described in Example 53. LCMS (Method 5-95 AB, ESI): t_{R} = 0.682 min, [M + H]⁺ = 876.7; ¹H NMR (400 MHz, MeOH-*d*₄) δ 8.49 (br s, 2H), 8.35-8.15 (m, 2H), 7.40-7.30 (m, 3H), 7.30-7.15 (m, 2H), 7.09 (d, J=8.4 Hz, 1H), 6.89 (s, 1H), 6.73 (s, 1H), 6.48 (s, 1H), 5.35-5.20 (m, 1H), 4.85-4.75 (m, 2H), 4.35-4.10 (m, 6H), 3.30-3.05 (m, 8H), 3.01 (s, 3H), 2.74 (q, J=7.2 Hz, 2H), 2.53 (s, 6H), 2.35-2.25 (m, 1H), 2.25-2.05 (m, 1H), 1.36 (d, J=6.4 Hz, 3H), 1.30 (t, J=7.2 Hz, 3H).

### Example 61: Synthesis of Compound 261

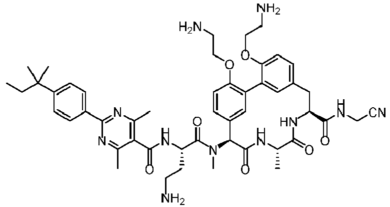

Compound 261 (formic acid salt) was prepared as a white solid from Compound **101-K** by utilizing methods analogous to those described in Examples 53 and 10. LCMS (Method 5-95 AB, ESI): t_{R} = 0.735 min, [M + H]⁺ = 918.6; ¹H NMR (400 MHz, MeOH-*d*₄) δ 8.42 (br s, 2H), 8.33 (d, *J*=8.4 Hz, 2H), 7.48 (d, *J*=8.4 Hz, 2H), 7.30 (d, *J*=8.0 Hz, 1H), 7.20 (d, *J*=8.0 Hz, 2H), 7.10 (d, *J*=8.0 Hz, 1H), 6.92 (s, 1H), 6.79 (s, 1H), 6.44 (s, 1H), 5.30-5.20 (m, 1H), 4.85-4.75 (m, 2H), 4.35-4.15 (m, 6H), 3.30-3.05 (m, 8H), 3.01 (s, 3H), 2.56 (s, 6H), 2.35-2.25 (m, 1H), 2.25-2.10 (m, 1H), 1.78 (q, *J*=7.2 Hz, 2H), 1.40-1.35 (m, 9H), 0.72 (t, *J*=6.8 Hz, 3H).

### Example 62: Synthesis of Compound 262

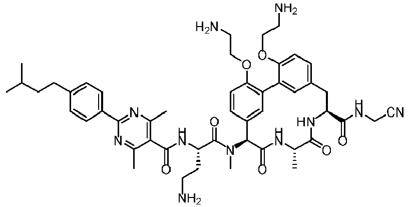

Compound 262 (formic acid salt) was prepared as a white solid from Compound **101-K** by utilizing methods analogous to those described in Examples 53 and 17. LCMS (Method 5-95 AB, ESI): t_{R} = 0.775 min, [M + H]⁺ = 918.4; ¹H NMR (400 MHz, MeOH-*d*₄) δ 8.45 (br s, 1H), 8.22 (d, *J*=8.0 Hz, 2H), 7.35-7.15 (m, 4H), 7.15-7.05 (m, 2H), 6.88 (s, 1H), 6.66 (s, 1H), 6.52 (s, 1H), 5.30-5.20 (m, 1H), 4.85-4.70 (m, 2H), 4.40-4.15 (m, 6H), 3.40-3.35 (m, 1H), 3.30-3.20 (m, 3H), 3.15 - 2.95 (m, 7H), 2.71 (t, *J*=8.0 Hz, 2H), 2.50 (s, 6H), 2.35-2.25 (m, 1H), 2.25-2.15 (m, 1H), 1.65-1.50 (m, 3H), 1.36 (d, *J*=6.8 Hz, 3H), 0.98 (t, *J*=5.6 Hz, 6H).

### Example 63: Synthesis of Compound 263

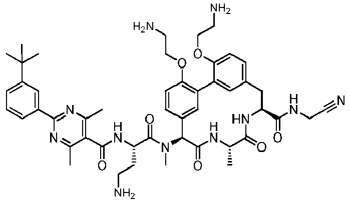

Compound 263 (formic acid salt) was prepared as a white solid from Compound 101-K by utilizing methods analogous to those described in Examples 10 and 53. LCMS (Method 5-95 AB, ESI): t_{R} = 0.711 min, [M + H]⁺ = 904.4; ¹H NMR (400 MHz, MeOH-*d*₄) δ 8.48 (br s, 1H), 8.14 (d, *J*=7.6 Hz, 1H), 7.58 (d, *J*=8.0 Hz, 1H), 7.42-7.28 (m, 3H), 7.25-7.16 (m, 2H), 7.10 (d, *J*=8.4 Hz, 1H), 6.90 (s, 1H), 6.73 (s, 1H), 6.48 (s, 1H), 5.27-5.23 (m, 1H), 4.82-4.76 (m, 2H), 4.36-4.17 (m, 6H), 3.29-3.09 (m, 8H), 3.02 (s, 3H), 2.55 (s, 6H), 2.34-2.26 (m, 1H), 2.22-2.14 (m, 1H), 1.40 (s, 9H), 1.36 (d, *J*=6.4 Hz, 3H).

### Example 64: Synthesis of Compound 264

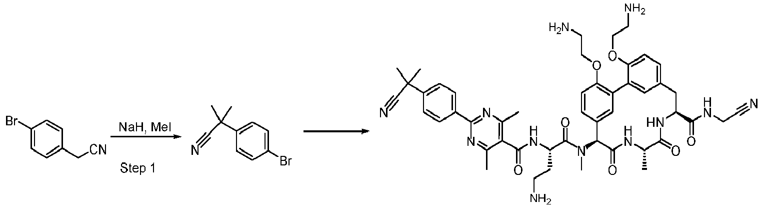

Step 1: A mixture of 2-(4-bromophenyl)acetonitrile (3.0 g, 15.3 mmol) and NaH (60% in oil, 1.84 g, 45.9 mmol) in THF (100 mL) was stirred at 0 °C for 1 h, followed by the dropwise addition of iodomethane (9.5 g, 67.2 mmol). The resulting mixture was stirred at 20 °C for 16 h. The reaction mixture was quenched with saturated aqueous NH₄Cl solution (50 mL), which was extracted with EtOAc (3 × 50ml). The combined organic layers were washed with brine (2 × 100 mL), dried over Na₂SO₄, concentrated in vacuo and the residue was purified by silica gel chromatography, eluting with 0-5% EtOAc in petroleum ether, to give 2-(4-bromophenyl)-2-methylpropanenitrile (3.3 g, 96% yield) as pale yellow oil. ¹H NMR (400 MHz, CDCl₃) δ 7.23 (d, *J*=8.0 Hz, 2H), 7.36 (d, *J*=8.0 Hz, 2H), 1.72 (s, 6H).

Compound 264 (formic acid salt) was prepared as a white solid from Compound **101-K** and 2-(4-bromophenyl)-2-methylpropanenitrile by utilizing methods analogous to those described in Examples 10 and 53. LCMS (Method 0-30 AB, ESI): t_{R} = 1.153 min, [M + H]⁺ = 915.5; ¹H NMR (400 MHz, MeOH-*d*₄) δ 8.50 (br s, 2H), 8.32 (d, *J*=8.0 Hz, 2H), 7.58 (d, *J*=8.0 Hz, 2H), 7.27 (brs, 2H), 7.10-7.01 (m, 2H), 6.84 (s, 1H), 6.60 (brs, 1H), 6.49 (brs, 1H), 5.35-5.29 (m, 1H), 4.82-4.72 (m, 2H), 4.40 (s, 2H), 4.30-4.22 (m, 4H), 3.29-3.21 (m, 4H), 3.14 (t, *J*=7.7 Hz, 2H), 3.02 (s, 3H), 3.01-2.90 (m, 2H), 2.46 (s, 6H), 2.32-2.26 (m, 1H), 2.20-2.13 (m, 1H), 1.79 (s, 6H), 1.35 (d, *J*=6.6 Hz, 3H).

### Example 65: Synthesis of Compound 265

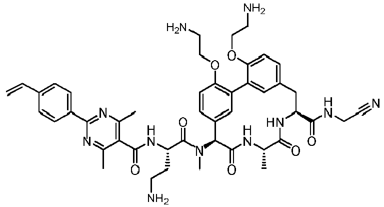

Compound 265 (formic acid salt) was prepared as a white solid from Compound **101-K** by utilizing methods analogous to those described in Examples 10 and 53. LCMS (Method 5-95 AB, ESI): t_{R} = 0.671 min, [M + Na]⁺ = 896.8; ¹H NMR (400 MHz, MeOH-*d*₄) δ 8.58 (br s, 2H), 8.31 (d, *J*=8.0 Hz, 2H), 7.53 (d, *J*=8.0 Hz, 2H), 7.31-7.21 (m, 2H), 7.16-7.08 (m, 2H), 6.90-6.78 (m, 2H), 6.67 (s, 1H), 6.51 (s, 1H), 5.92 (d, *J*=17.6 Hz, 1H), 5.36 (d, *J*=10.6 Hz, 1H), 5.00-4.70 (m, 3H), 4.34-4.26 (m, 4H), 4.22 (s, 2H), 3.27-3.23 (m, 4H), 3.18-3.05 (m, 4H), 3.02 (s, 3H), 2.52 (s, 6H), 2.30-2.28 (m, 1H), 2.20-2.13 (m, 1H), 1.36 (d, *J*=6.0 Hz, 3H).

### Example 66: Synthesis of Compound 266

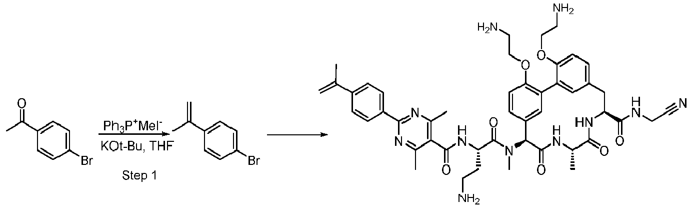

Step 1: Conditions analogous to those described in Example 12 were applied to 1-(4-bromophenyl)ethan-1-one to afford 1-bromo-4-(prop-1-en-2-yl)benzene as a colorless oil. ¹H NMR (400 MHz, CDCl₃) δ= 7.45 (d, *J*=8.4 Hz, 1H), 7.34 (d, *J*=8.4 Hz, 1H), 5.38 (s, 1H), 5.12 (s, 1H), 2.14 (s, 3H).

Compound 266 (formic acid salt) was prepared as a white solid from Compound **101-K** and 1-bromo-4-(prop-1-en-2-yl)benzene by utilizing methods analogous to those described in Examples 10 and 53. LCMS (Method 5-95 AB, ESI): t_{R} = 0.704 min, [M + H]⁺ = 888.8; ¹H NMR (400 MHz, MeOH-*d*₄) δ 8.47 (br s, 1H), 8.37 (d, *J*=8.0 Hz, 2H), 7.61 (d, *J*=8.0 Hz, 2H), 7.32-7.27 (m, 1H), 7.23-7.16 (m, 2H), 7.09 (d, *J*=8.0 Hz, 1H), 6.90 (brs, 1H), 6.76 (brs, 1H), 6.44 (s, 1H), 5.51 (s, 1H), 5.25-5.20 (m, 1H), 4.95-4.76 (m, 3H), 4.30-4.16 (m, 6H), 3.27-3.05 (m, 8H), 3.00 (s, 3H), 2.55 (s, 6H), 2.29-2.24 (m, 1H), 2.20 (s, 3H), 2.18-2.12 (m, 1H), 1.35 (d, *J*=7.2 Hz, 3H).

### Example 67: Synthesis of Compound 267

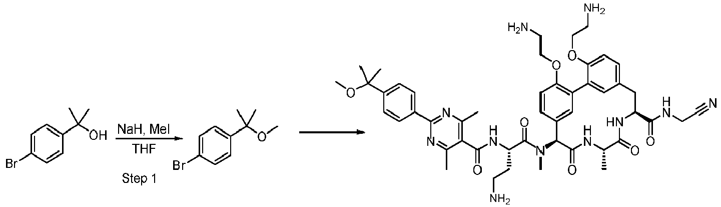

Step 1: A mixture of 2-(4-bromophenyl)propan-2-ol (300 mg, 1.39 mmol) and NaH (60% in oil, 62 mg, 1.53 mmol) in THF (5 mL) was stirred at 0 °C for 1 h, followed by the addition of iodomethane (3.8 g, 27 mmol). The resulting mixture was stirred at 15 °C for 5 h. The reaction was diluted with water (30 mL), which was extracted by EtOAc (3 × 30 mL). The combined organic layers were washed with brine (50 mL), dried over Na₂SO₄ and concentrated to afford 1-bromo-4-(2-methoxypropan-2-yl)benzene (300 mg) as a colorless oil, which was used directly in the next step without further purification. ¹H NMR (400 MHz, CDCl₃) δ 7.47 (d, J = 8.4 Hz, 2H), 7.28 (d, J =8.4 Hz, 2H), 3.05 (s, 3H), 1.50 (s, 6H).

Compound 267 (formic acid salt) was prepared as a white solid from Compound **101-K** and 1-bromo-4-(2-methoxypropan-2-yl)benzene by utilizing methods analogous to those described in Examples 10 and 53. LCMS (Method 5-95 AB, ESI): t_{R} = 0.664 min, [M + H]⁺ = 920.5; ¹H NMR (400 MHz, MeOH-*d*₄) δ 8.46 (br s, 2H), 8.37 (d, *J*=8.0 Hz, 2H), 7.53 (d, *J*=8.0 Hz, 2H), 7.30 (d, *J*=8.4 Hz, 1H), 7.22-7.17 (m, 2H), 7.10 (d, *J*=8.4 Hz, 1H), 6.90 (s, 1H), 6.74 (s, 1H), 6.46 (s, 1H), 5.25-5.23 (m, 1H), 4.80-4.76 (m, 1H), 4.45-4.15 (m, 7H), 3.26-3.05 (m, 11H), 3.01 (s, 3H), 2.54 (s, 6H), 2.40-2.20 (m, 1H), 2.15-2.05 (m, 1H), 1.56 (s, 6H), 1.35 (d, *J*=6.4 Hz, 3H).

### Example 68: Synthesis of Compound 268

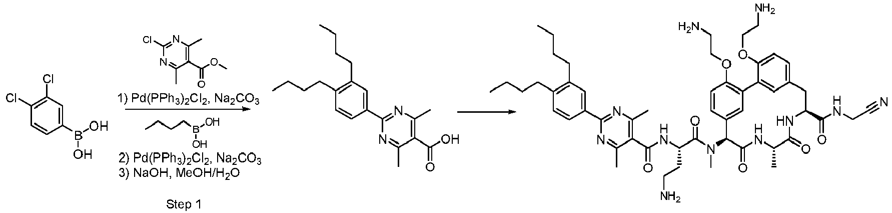

Step 1: Starting from (3,4-dichlorophenyl)boronic acid, sequential Suzuki coupling and ester hydrolysis (NaOH, MeOH/H₂O) conditions, analogous to those described in Example H, were followed to give 2-(3,4-dibutylphenyl)-4,6-dimethylpyrimidine-5-carboxylic acid as a white solid.

Compound 268 (formic acid salt) was prepared as a white solid from Compound **101-K** and 2-(3,4-dibutylphenyl)-4,6-dimethylpyrimidine-5-carboxylic acid by utilizing methods analogous to those described in Example G. LCMS (Method 5-95 AB, ESI): t_{R} = 0.687 min, [M + H]⁺ = 960.5; ¹H NMR (400 MHz, MeOH-*d*₄) δ 8.47 (br s, 3H), 8.19 (s, 1H), 8.09 (d, *J*=8.4 Hz, 1H), 7.32-7.16 (m, 4H), 7.09 (d, *J*=8.4 Hz, 1H), 6.90 (s, 1H), 6.77 (s, 1H), 6.45 (s, 1H), 5.26-5.20 (m, 1H), 4.83-4.77 (m, 2H), 4.33-4.15 (m, 6H), 3.27-3.06 (m, 8H), 3.00 (s, 3H), 2.78-2.66 (m, 4H), 2.54 (s, 6H), 2.31-2.25 (m, 1H), 2.19-2.13 (m, 1H), 1.68-1.56 (m, 4H), 1.52-1.41 (m, 4H), 1.35 (d, *J*=7.2 Hz, 3H), 0.99 (t, J=7.6 Hz, 6H).

### Example 69: Synthesis of Compound 269

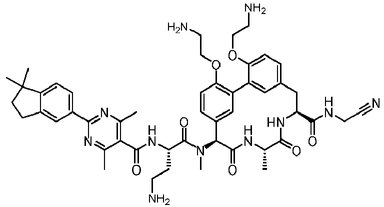

Compound 269 (formic acid salt) was prepared as a white solid from Compound **101-K** by utilizing methods analogous to those described in Examples 53 and 34. LCMS (Method 5-95 AB, ESI): t_{R} = 0.723 min, [M + H]⁺ = 916.4; ¹H NMR (400 MHz, MeOH-*d*₄) δ 8.44 (br s, 3H), 8.13-8.02 (m, 2H),7.27-7.18 (m, 4H),7.08 (b rs, 2H),6.85 (s, 1H),6.60 (br s, 1H), 5.32-5.28 (m, 1H), 4.80-4.76 (m, 2H), 4.40 (s, 2H), 4.35-4.24 (m, 4H), 3.40-3.33 (m, 1H),3.28-3.23 (m, 2H),3.14 (t, *J*=7.6 Hz, 3H), 3.06-2.89 (m, 8H), 2.46 (s, 6H), 2.34-2.24 (m, 1H), 2.21-2.11 (m,1H), 2.00 (t, *J*=7.6 Hz, 3H),1.35 (d, *J*=6.4 Hz, 3H), 1.31 (s, 6H).

### Example 70: Synthesis of Compound 270

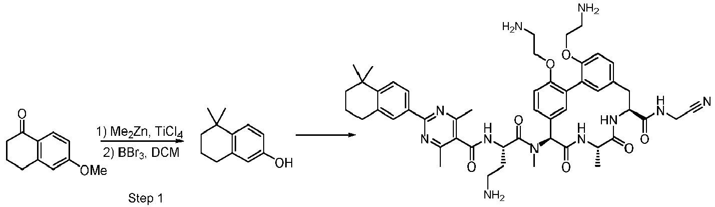

Step 1: Starting from 6-methoxy-3,4-dihydronaphthalen-1(2*H*)-one, di-methylation and de-methylation conditions (as described in Examples 34 and 98) were followed to give 5,5-dimethyl-5,6,7,8-tetrahydronaphthalen-2-ol as a yellow oil.

Compound 270 (formic acid salt) was prepared as a white solid from Compound **101-K** and 5,5-dimethyl-5,6,7,8-tetrahydronaphthalen-2-ol by utilizing methods analogous to those described in Examples 10 and 53. LCMS (Method 5-95 AB, ESI): t_{R} = 0.741 min, [M + H]⁺ = 930.5; ¹H NMR (400 MHz, MeOH-*d*₄) δ 8.51 (br s, 3H), 8.03-7.95 (m, 2H), 7.42 (d, *J*=8.0 Hz, 1H), 7.28 (br s, 2H), 7.10-7.02 (m, 2H), 6.86 (s, 1H), 6.63 (br s, 1H), 5.31 (br s, 1H), 4.80-4.78 (m, 2H), 4.39 (br s, 2H), 4.27 (br s, 4H), 3.25-3.14 (m, 6H), 3.10-2.95 (m, 2H), 3.04 (s, 3H), 2.87-2.79 (m, 2H), 2.47 (s, 6H), 2.32-2.17 (m, 2H), 2.31-2.17 (m, 2H), 1.89-1.88 (m, 2H), 1.77-1.76 (m, 2H), 1.40-1.35 (m, 9H).

### Example 71: Synthesis of Compound 271

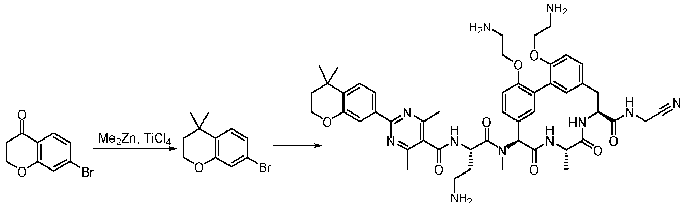

Step 1: Following di-methylation conditions analogous to those described in Example 34, 7-bromochroman-4-one was converted to 7-bromo-4,4-dimethylchromane. ¹H NMR (400 MHz, CDCl₃) δ 7.11 (d, *J*=8.0 Hz, 1H), 7.00-6.95 (m, 2H), 4.18 (t, *J*=5.2 Hz, 2H), 1.82 (t, *J*=5.2 Hz, 2H), 1.31 (s, 6H).

Compound 271 (formic acid salt) was prepared as a white solid from Compound **101-K** and 7-bromo-4,4-dimethylchromane by utilizing methods analogous to those described in Examples 10 and 53. LCMS (Method 5-95 AB, ESI): t_{R} = 0.690 min, [M + H]⁺ = 932.4; ¹H NMR (400 MHz, MeOH-*d*₄) δ 8.50 (br s, 2H), 7.84 (d, *J*=8.0 Hz, 1H), 7.40-7.22 (m, 4H), 7.02 (d, *J*=8.0 Hz, 1H), 6.86-6.70 (m, 3H), 5.37-5.33 (m, 1H), 4.85-4.72 (m, 2H), 4.54-4.45 (m, 2H), 4.25-4.15 (m, 6H), 3.45-3.41 (m, 3H), 3.28-3.24 (m, 3H), 3.16-3.12 (m, 2H), 3.02 (s, 3H), 2.85-2.77 (m, 1H), 2.37-2.31 (m, 5H), 2.17-2.12 (m, 2H), 1.93-1.89 (m, 2H), 1.43-1.34 (m, 9H).

### Example 72: Synthesis of Compound 272

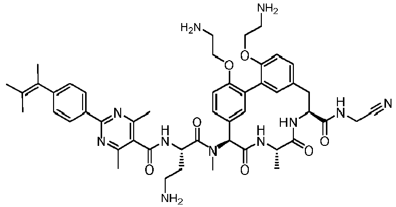

Compound 272 (formic acid salt) was prepared as a white solid from Compound **101-K** by utilizing methods analogous to those described in Example 66. LCMS (Method 5-95 AB, ESI): t_{R} = 0.739 min, [M + H]⁺ = 916.5; ¹H NMR (400 MHz, MeOH-*d*₄) δ 8.45 (br s, 2H), 8.28 (d, *J*=8.0 Hz, 2H), 7.29 (d, *J*=8.0 Hz, 1H), 7.24-7.15 (m, 4H), 7.09 (d, *J*=8.4 Hz, 1H), 6.89 (s, 1H), 6.69 (s, 1H), 6.50 (s, 1H), 5.26-5.23 (m, 1H), 4.81-4.76 (m, 2H), 4.32-4.17 (m, 6H), 3.31-3.12 (m, 8H), 3.01 (s, 3H), 2.52 (s, 6H), 2.32-2.13 (m, 2H), 2.00 (s, 3H), 1.86 (s, 3H), 1.63 (s, 3H), 1.36 (d, *J*=6.4 Hz, 3H).

### Example 73: Synthesis of Compound 273

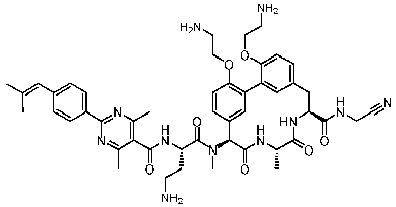

Compound 273 (formic acid salt) was prepared as a white solid from Compound **101-K** by utilizing methods analogous to those described in Example 66. LCMS (Method 5-95 AB, ESI): t_{R} = 0.724 min, [M + H]⁺ = 902.4; ¹H NMR (400 MHz, MeOH-*d*₄) δ 8.49 (br s, 3H), 8.19 (d, *J*=7.2 Hz, 2H), 7.30-7.20 (m, 4H), 7.08 (brs, 2H), 6.86 (s, 1H), 6.61 (s, 1H), 6.55 (s, 1H), 6.35 (s, 1H), 5.35-5.25 (m, 1H), 4.85-4.75 (m, 2H), 4.40-4.20 (m, 8H), 3.28-3.24 (m, 2H), 3.17-3.13 (m, 2H), 3.05-2.95 (m, 2H), 3.01 (s, 3H), 2.46 (s, 6H), 2.35-2.25 (m, 1H), 2.20-2.10 (m, 1H), 1.96 (s, 3H), 1.94 (s, 3H), 1.35 (d, *J*=6.8 Hz, 3H).

### Example 74: Synthesis of Compound 274

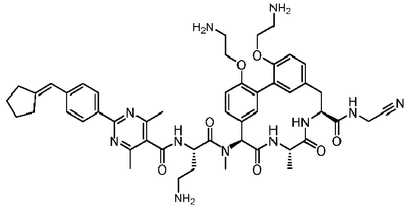

Compound 274 (formic acid salt) was prepared as a white solid from Compound **101-K** by utilizing methods analogous to those described in Example 66. LCMS (Method 5-95 AB, ESI): t_{R} = 0.682 min, [M + H]⁺ = 928.6; ¹H NMR (400 MHz, MeOH-*d*₄) δ 8.49 (br s, 3H), 8.26 (d, *J*=8.0 Hz, 2H), 7.39 (d, *J*=8.0 Hz, 2H), 7.29 (d, *J*=8.0 Hz, 1H), 7.24 (d, *J*=8.0 Hz, 1H), 7.14-7.09 (m, 2H), 6.89 (s, 1H), 6.66 (s, 1H), 6.54 (s, 1H), 6.45 (s, 1H), 5.29-5.26 (m, 1H), 4.79-4.60 (m, 2H), 4.34-4.23 (m, 6H), 3.27-3.00 (m, 8H), 3.02 (s, 3H), 2.67-2.61 (m, 2H), 2.56-2.49 (m, 6H), 2.52 (s, 2H), 2.33-2.28 (m, 1H), 2.20-2.16 (m, 1H), 1.90-1.83 (m, 2H), 1.75-1.71 (m, 2H), 1.36 (d, *J*=7.2 Hz, 3H).

### Example 75: Synthesis of Compound 275

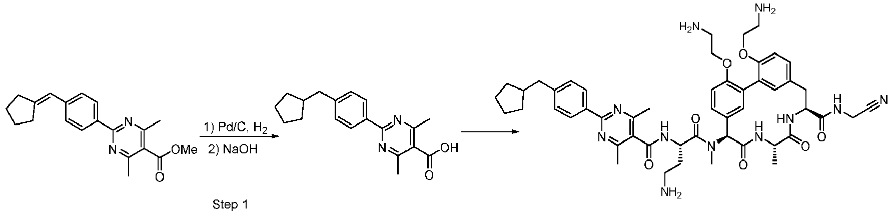

Step 1: Starting from methyl 2-(4-(cyclopentylidenemethyl)phenyl)-4,6-dimethylpyrimidine-5-carboxylate (as described in Example 74), typical hydrogenation (Pd/C, H₂, Example D) and ester hydrolysis (NaOH, MeOH/H₂O, Example H) conditions were applied to give 2-(4-(cyclopentylmethyl)phenyl)-4,6-dimethylpyrimidine-5-carboxylic acid as a white solid. LCMS (Method 5-95 AB, ESI): t_{R} = 1.015 min, [M + H]⁺ = 311.0.

Compound 275 (formic acid salt) was prepared as a white solid from Compound **101-K** and 2-(4-(cyclopentylmethyl)phenyl)-4,6-dimethylpyrimidine-5-carboxylic acid by utilizing methods analogous to those described in Example G. LCMS (Method 5-95 AB, ESI): t_{R} = 0.747 min, [M + H]⁺ = 930.5; ¹H NMR (400 MHz, MeOH-*d*₄) δ 8.53 (br s, 3H), 8.17 (d, *J*=8.0 Hz, 2H), 7.30-7.22 (m, 4H), 7.13-7.07 (m, 2H), 6.87 (s, 1H), 6.64 (s, 1H), 6.55 (br s, 1H), 5.34-5.30 (m, 1H), 4.82-4.77 (m, 2H), 4.38-4.26 (m, 6H), 3.29-3.14 (m, 8H), 3.04 (s, 3H), 2.71 (d, *J*=7.6 Hz, 2H), 2.47 (s, 6H), 2.34-2.29 (m, 1H), 2.19-2.18 (m, 1H), 1.76-1.60 (m, 7H), 1.37 (d, *J*=7.2 Hz, 3H), 1.33-1.26 (m, 2H).

### Example 76: Synthesis of Compound 276

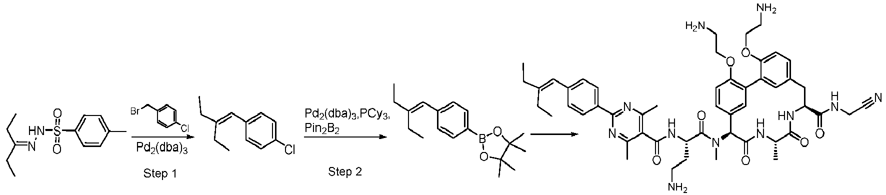

Step 1: A mixture of 4-methyl-N'-(pentan-3-ylidene)benzenesulfonohydrazide (1.0 g, 3.9 mmol), 1-(bromomethyl)-4-chlorobenzene (88 mg, 4.3 mmol), Pd₂(dba)₃ (90 mg, 0.10 mmol), tri(2-furyl)phosphine (183 mg, 0.79 mmol) and t-BuOLi (944 mg, 11.8 mmol) in toluene (40 mL) was stirred at 80 °C for 16 h under nitrogen. The volatiles were removed under reduced pressure and the residue was taken up by EtOAc (100 mL), which was washed by brine (100 mL). The organic layer was dried over MgSO₄, concentrated and the residue was purified by silica gel chromatography, eluting with 5% EtOAc in petroleum ether, to give 1-chloro-4-(2-ethylbut-1-en-1-yl)benzene (200 mg, 26% yield) as a colorless oil. ¹H NMR (CDCl₃, 400MHz): 7.28 (d, *J*=8.4 Hz, 2H), 7.14 (d, *J*=8.4 Hz, 2H), 6.18 (s, 1H), 2.24-2.14 (m, 4H), 1.14-1.05 (m, 6H).

Step 2: A mixture of 1-chloro-4-(2-ethylbut-1-en-1-yl)benzene (500 mg, 2.6 mmol), bis(pinacolato)diboron (783 mg, 3.1 mmol), Pd₂(dba)₃ (118 mg, 0.13 mmol), tricyclohexylphosphine (86 mg, 0.31 mmol) and potassium acetate (755 mg, 7.7 mmol) in 1,4-dioxane (20 mL) was stirred at 100 °C for 16 h under nitrogen. The volatiles were removed under reduced pressure and the residue was purified by silica gel chromatography, eluting with 0-1% EtOAc in petroleum ether, to give 2-(4-(2-ethylbut-1-en-1-yl)phenyl)-4,4,5,5-tetramethyl-1,3,2-dioxaborolane (400 mg, 54% yield) as a greenish oil.

Compound 276 (formic acid salt) was prepared as a white solid from Compound **101-K** and 2-(4-(2-ethylbut-1-en-1-yl)phenyl)-4,4,5,5-tetramethyl-1,3,2-dioxaborolane by utilizing methods analogous to those described in Example 53. LCMS (Method 5-95 AB, ESI): t_{R} = 0.641 min, [M + H]⁺ = 930.5; ¹H NMR (400 MHz, MeOH-*d*₄) δ 8.51 (s, 2H), 8.20 (d, *J*=8.0 Hz, 2H), 7.31-7.22 (m, 4H), 7.08 (br s, 2H), 6.86 (s, 1H), 6.66-6.51 (m, 2H), 6.32 (s, 1H), 5.33-5.26 (m, 1H), 4.87-4.72 (m, 2H), 4.32 (s, 2H), 4.29-4.20 (m, 4H), 3.29-3.20 (m, 4H), 3.18-3.12 (s, 2H), 3.08-2.98 (m, 5H), 2.47 (s, 6H), 2.40-2.24 (m, 5H), 2.22-2.12 (m, 1H), 1.35 (d, *J*=6.4 Hz, 3H), 1.20-1.10 (m, 6H).

### Example 77: Synthesis of Compound 277

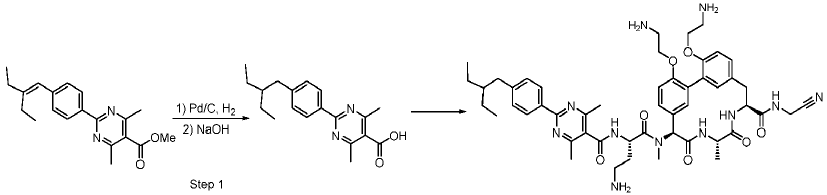

Step 1: Starting from methyl 2-(4-(2-ethylbut-1-en-1-yl)phenyl)-4,6-dimethylpyrimidine-5-carboxylate (prepared as described in Example 76), typical hydrogenation (Pd/C, H₂, Example D) and ester hydrolysis (NaOH, MeOH/H₂O, Example H) conditions were applied to give 2-(4-(2-ethylbutyl)phenyl)-4,6-dimethylpyrimidine-5-carboxylic acid as a white solid.

Compound 277 (formic acid salt) was prepared as a white solid from Compound **101-K** and 2-(4-(2-ethylbutyl)phenyl)-4,6-dimethylpyrimidine-5-carboxylic acid by utilizing methods analogous to those described in Example G. LCMS (Method 5-95 AB, ESI): t_{R} = 0.767 min, [M + H]⁺ = 932.4; ¹H NMR (400 MHz, MeOH-*d*₄) δ 8.49 (br s, 2H), 8.20 (d, *J*=8.0 Hz, 2H), 7.32-7.18 (m, 4H), 7.15-7.04 (m, 2H), 6.86 (s, 1H), 6.62 (s, 1H), 6.53 (s, 1H), 5.30-5.23 (m, 1H), 4.78-4.55 (m, 2H), 4.34 (s, 2H), 4.27-4.16 (m, 4H), 3.28-3.19 (m, 4H), 3.13-3.05 (m, 4H), 3.01 (s, 3H), 2.62 (d, *J*=7.2 Hz, 2H), 2.49 (s, 6H), 2.35-2.23 (m, 1H), 2.20-2.12 (m, 1H), 1.64-1.53 (m, 1H), 1.39-1.31 (m, 7H), 0.93 (t, J=7.2 Hz, 6H).

### Example 78: Synthesis of Compound 278

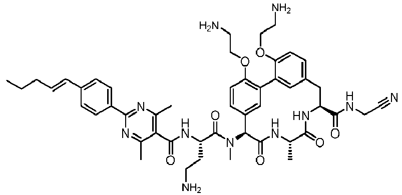

Compound 278 (formic acid salt) was prepared as a white solid from Compound **101-K** by utilizing methods analogous to those described in Example 56. LCMS (Method 5-95 AB, ESI): t_{R} = 0.631 min, [M + H]⁺ = 916.4; ¹H NMR (400 MHz, MeOH-*d*₄) δ 8.14-8.10 (m, 2H), 7.48-7.40 (m, 2H), 7.26 (br s, 2H),7.07-7.00 (m, 2H), 6.83 (s, 1H), 6.66 (s, 1H), 6.50-6.38 (m, 3H), 5.34-5.30 (m, 1H), 4.79-4.74 (m, 2H), 4.30-4.20 (m, 6H), 3.31 - 3.14 (m, 6H), 2.91(s, 3H), 2.90-2.80 (m, 2H), 2.45 (s, 6H), 2.28-2.21(m, 4H), 1.60-1.51 (m, 2H), 1.36 (d, *J*=6.8 Hz, 3H), 1.01 (t, *J*=7.6 Hz, 3H).

### Example 79: Synthesis of Compound 279

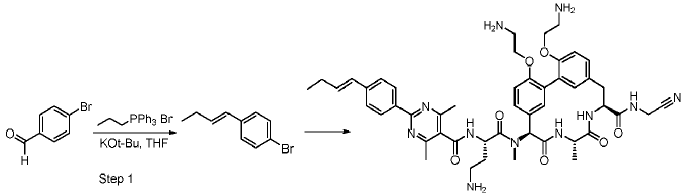

Step 1: Typical Wittig reaction condition (as described in Example 12) was applied to 4-bromobenzaldehyde to give (*E*)-1-bromo-4-(but-1-en-1-yl)benzene as a colorless oil.

Compound 279 (formic acid salt) was prepared as a white solid from Compound **101-K** and (*E*)-1-bromo-4-(but-1-en-1-yl)benzene by utilizing methods analogous to those described in Example 53. LCMS (Method 5-95 AB, ESI): t_{R} = 0.714 min, [M + H]⁺ = 902.5; ¹H NMR (400 MHz, MeOH-*d*₄) δ 8.46 (br s, 1H), 8.25-8.21 (m, 2H), 7.45-7.05 (m, 7H), 6.88 (brs, 1H), 6.50-6.40 (m, 3H), 5.79-5.73 (m, 1H), 5.27-5.22 (m, 1H), 4.81-4.75 (m, 2H), 4.32-4.20 (m, 6H), 3.24-3.11 (m, 6H), 3.05-2.96 (m, 5H), 2.52 (s, 6H), 2.41-2.27 (m, 3H), 2.20-2.13 (m, 1H), 1.36 (d, *J*=6.8 Hz, 3H), 1.10 (t, *J*=7.6 Hz, 3H)

### Example 80: Synthesis of Compound 280

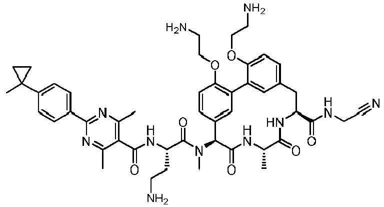

Compound 280 (formic acid salt) was prepared as a white solid from Compound **101-K** by utilizing methods analogous to those described in Examples 12 and 53. LCMS (Method 5-95 AB, ESI): t_{R} = 0.586 min, [M + H]⁺ = 902.3; ¹H NMR (400 MHz, MeOH-*d*₄) δ 8.50 (br s, 1H), 8.35-8.20 (m, 2H), 7.36-7.28 (m, 3H), 7.21 (d, *J*=8.4 Hz, 2H), 7.09 (d, *J*=8.4 Hz, 1H), 6.90 (br s, 1H), 6.76 (br s, 1H), 6.46 (s, 1H), 5.26-5.20 (m, 1H), 4.79-4.70 (m, 2H), 4.35-4.19 (m, 4H), 4.21 (s, 2H), 3.26-3.18 (m, 4H), 3.16-3.09 (m, 4H), 3.01 (s, 3H), 2.54 (s, 6H), 2.32-2.25 (m, 1H), 2.20-2.13 (m, 1H), 1.47 (s, 3H), 1.36 (d, *J*=6.6 Hz, 3H), 0.94 (br s, 2H), 0.84 (br s, 2H).

### Example 81: Synthesis of Compound 281

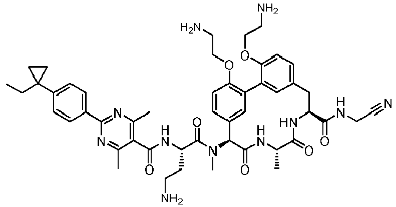

Compound 281 (formic acid salt) was prepared as a white solid from Compound **101-K** by utilizing methods analogous to those described in Examples 12 and 53. LCMS (Method 5-95 AB, ESI): t_{R} = 0.721 min, [M + H]⁺ = 916.5; ¹H NMR (400 MHz, MeOH-*d*₄) δ 8.50 (br s, 2H), 8.20 (d, *J*=8.0 Hz, 2H), 7.35 (d, *J*=8.0 Hz, 2H), 7.30-7.20 (m, 2H), 7.08 (br s, 2H), 6.86 (s, 1H), 6.57 (br s, 1H), 5.35-5.25 (m, 1H), 4.85-4.75 (m, 2H), 4.40-4.20 (m, 6H), 3.30-3.20 (m, 4H), 3.16-3.12 (m, 2H), 3.05-2.95 (m, 5H), 2.48 (s, 6H), 2.35-2.25 (m, 1H), 2.20-2.10 (m, 1H), 1.68 (q, *J*=7.2 Hz, 2H), 1.35 (d, *J*=6.8 Hz, 3H), 0.92 (t, *J*=6.8 Hz, 3H), 0.84 (br s, 2H), 0.78 (br s, 2H).

### Example 82: Synthesis of Compound 282

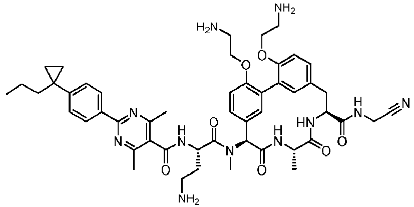

Compound 282 (formic acid salt) was prepared as a white solid from Compound **101-K** by utilizing methods analogous to those described in Examples 12 and 53. LCMS (Method 5-95 AB, ESI): t_{R} = 0.753 min, [M + H]⁺ = 930.4; ¹H NMR (400 MHz, MeOH-*d*₄) δ 8.51 (br s, 2H), 8.22 (d, *J*=8.0 Hz, 2H), 7.38 (d, *J*=8.0 Hz, 2H), 7.31-7.25 (m, 2H), 7.10 (br s, 2H), 6.88 (s, 1H), 6.62 (s, 1H), 6.58 (s, 1H), 5.32-5.28 (m, 1H), 4.82-4.77 (m, 3H), 4.36 (s, 2H), 4.30-4.21 (m, 4H), 3.32-3.10 (m, 8H), 3.04 (s, 3H), 2.50 (s, 6H), 2.35-2.15 (m, 2H), 1.67-1.63 (m, 2H), 1.41-1.31 (m, 5H), 0.92 (t, *J* = 6.8 Hz, 3H), 0.88-0.84 (m, 2H), 0.80-0.76 (m, 2H).

### Example 83: Synthesis of Compound 283

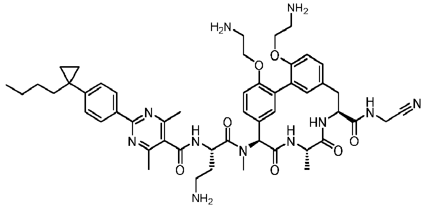

Compound 283 (formic acid salt) was prepared as a white solid from Compound **101-K** by utilizing methods analogous to those described in Examples 12 and 53. LCMS (Method 5-95 AB, ESI): t_{R} = 0.775 min, [M + H]⁺ = 944.6; ¹H NMR (400 MHz, MeOH-*d*₄) δ8.46 (brs, 3H), 8.31 (d, *J*=8.0 Hz, 2H), 7.40 (d, *J*=8.0 Hz, 2H), 7.30 (d, *J*=8.0 Hz, 1H), 7.20 (d, *J*=8.0 Hz, 2H), 7.10 (d, *J*=8.4 Hz, 1H), 6.91 (s, 1H), 6.80 (brs, 1H), 6.44 (s, 1H), 5.25-5.21 (m, 1H), 4.89-4.80 (m, 2H), 4.30-4.18 (m, 6H), 3.25-3.09 (m, 8H), 3.01 (s, 3H), 2.56 (s, 6H), 2.31-2.26 (m, 1H), 2.19-2.14 (m, 1H), 1.65 (brs, 3H), 1.39-1.26 (m, 7H), 0.91-0.80 (m, 5H), 0.79-0.72 (m., 2H).

### Example 84: Synthesis of Compound 284

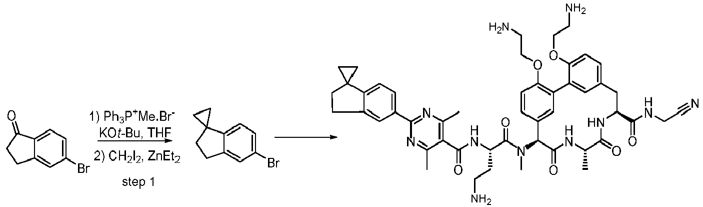

Step 1: Starting from 5-bromo-2,3-dihydro-1H-inden-1-one, Wittig and cyclo-proponation conditions (as described in Example 12) were followed to give 5'-bromo-2',3'-dihydrospiro[cyclopropane-1,1'-indene] as a yellow oil. ¹H NMR (400 MHz, CDCl₃) δ 7.32 (d, *J*=1.6 Hz, 1H), 7.23 (dd, *J*=8.0, 1.6 Hz, 1H), 6.53(d, *J*=8.0 Hz, 1H), 3.03 (t, *J*=7.2 Hz, 2H), 2.13 (t, *J*=7.2 Hz, 2H), 0.97-0.93 (m, 2H), 0.89-0.87 (m, 2H).

Compound 284 (formic acid salt) was prepared as a white solid from Compound **101-K** and 5'-bromo-2',3'-dihydrospiro[cyclopropane-1,1'-indene] by utilizing methods analogous to those described in Example 53. LCMS (Method 5-95 AB, ESI): t_{R} = 0.711 min, [M + Na]⁺ = 936.5; ¹H NMR (400 MHz, MeOH-*d*₄) δ 8.49 (brs, 2H), 8.15-8.02 (m, 2H), 7.31-7.21 (m, 2H), 7.16-7.07 (m, 2H), 6.90-6.81 (m, 1H), 6.77 (d, *J*=8.0 Hz, 1H), 6.70-6.47 (m, 2H), 5.28-5.25 (m, 1H), 4.84-4.75 (m, 2H), 4.35 (s, 2H), 4.29-4.19 (m, 4H), 3.29-3.19 (m, 4H), 3.18-2.97 (m, 9H), 2.48 (s, 6H), 2.34-2.25 (m, 1H), 2.28-2.11 (m, 3H), 1.36 (d, *J*=6.8 Hz, 3 H), 1.04-0.95 (m, 4H).

### Example 85: Synthesis of Compound 285

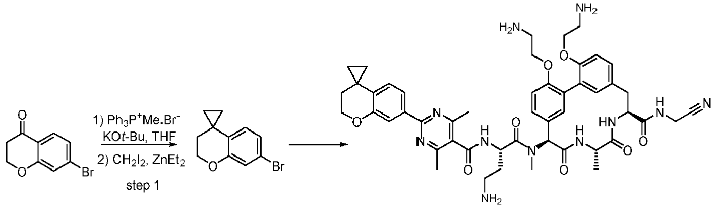

Step 1: Starting from 7-bromochroman-4-one, Wittig and cyclo-propanation conditions (as described in Example 12) were followed to give 7-bromospiro[chromane-4,1'-cyclopropane] as a yellow oil. ¹H NMR (400 MHz, CDCl₃) δ 6.96 (d, *J*=8.0 Hz, 1H), 6.95 (s, 1H), 6.51(d, *J*=8.0 Hz, 2H), 4.28 (t, *J*=5.2 Hz, 2H), 1.85(t, *J*=5.2 Hz, 2H), 1.03(t, *J*=4.4 Hz, 2H), 0.86(t, *J*=4.4 Hz, 2H).

Compound 285 (formic acid salt) was prepared as a white solid from Compound **101-K** and 7-bromospiro[chromane-4,1'-cyclopropane] by utilizing methods analogous to those described in Example 53. LCMS (Method 5-95 AB, ESI): t_{R} = 0.683 min, [M + H]⁺ = 930.4; ¹H NMR (400 MHz, MeOH-*d*₄) δ8.51 (br s, 2H), 7.80 (br s, 1H), 7.53-7.41 (m, 1H), 7.31-7.20 (m, 2H), 7.15-6.89 (m, 3H), 6.85-6.72 (m, 2H), 6.67 (s, 1H), 5.32-5.21 (m, 1H), 4.80-4.70 (m, 2H), 4.37-4.25 (m, 8H), 3.46-3.35 (m, 2H), 3.27-3.20 (m, 4H), 3.18-3.10 (m, 2H), 3.02 (s, 3H), 2.96-2.85 (m, 2H), 2.38 (s, 6H), 2.30-2.25 (m, 1H), 2.19-2.10 (m, 1H), 1.96-1.87 (m, 2H), 1.35 (d, J=6.2 Hz, 3H), 1.20-1.12 (m, 2H), 1.00-0.91 (m, 2H).

### Example 86: Synthesis of Compound 286

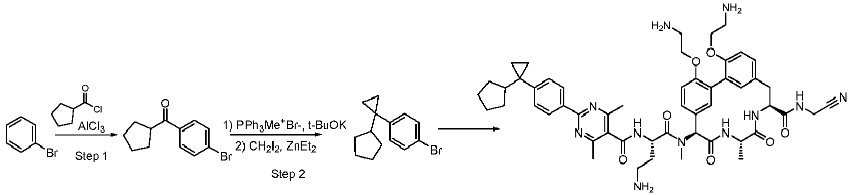

Step 1: To a mixture of cyclopentane carbonyl chloride (2.0 g, 15 mmol) and bromo- benzene (7.1 g, 45 mmol) was added AlCl₃ (3.0 g, 22.5 mmol) slowly at 0 °C and the mixture was stirred for at 20 °C for 3 h. The mixture was quenched with a saturated aqueous NH₄Cl solution (20 mL), which was extracted with EtOAc (3 × 30 mL). The combined organic layers were washed with brine (100 mL), dried over Na₂SO₄, concentrated and the residue was purified by silica gel chromatography, eluting with 0-5% EtOAc in petroleum ether, to give (4-bromophenyl)(cyclopentyl)methanone (1.56 g, 41% yield) as a yellow oil.

Step 2: Starting from (4-bromophenyl)(cyclopentyl)methanone, Wittig and cyclo-proponation conditions (as described in Example 12) were followed to give 1-bromo-4-(1-cyclopentylcyclopropyl)benzene as a yellow oil. ¹H NMR (400 MHz, CDCl₃) δ 7.39 (d, *J*=8.0 Hz, 2H), 7.19 (d, *J*=8.0 Hz, 2H), 1.80-1.75 (m, 1H), 1.63-1.60 (m, 2H), 1.50-1.45 (m, 4H), 1.12-1.07 (m, 2H), 0.71 (br s, 2H), 0.67 (br s, 2H).

Compound 286 (formic acid salt) was prepared as a white solid from Compound **101-K** and 1-bromo-4-(1-cyclopentylcyclopropyl)benzene by utilizing methods analogous to those described in Example 53. LCMS (Method 5-95 AB, ESI): t_{R} = 0.769 min, [M + H]⁺ = 956.5; ¹H NMR (400 MHz, MeOH-*d*₄) δ 8.47 (br s, 2H), 8.20 (br s, 2H), 7.39 (d, *J*=7.6 Hz, 2H), 7.26 (brs, 2H), 7.09 (brs, 2H), 6.87 (s, 1H), 6.64 (s, 1H), 6.50 (s, 1H), 5.32-5.24 (m, 1H), 4.80-4.72 (m, 2H), 4.36 (s, 2H), 4.29-4.17 (m, 4H), 3.29-3.08 (m, 8H), 3.02 (s, 3H), 2.49 (s, 6H), 2.33-2.22 (m, 1H), 2.21-2.12 (m, 1H),1.97-1.88 (m, 1H),1.74-1.62 (m, 2H), 1.51 (brs, 4H), 1.36 (d, J=6 Hz, 3 H), 1.22 (br s, 2H), 0.81 (br s, 2H), 0.75 (br s, 2H).

### Example 87: Synthesis of Compound 287

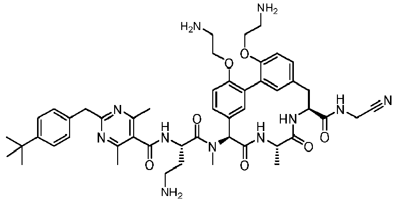

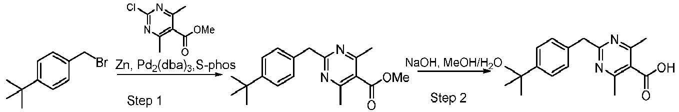

Step 1: A mixture of Zn (684 mg, 10.5 mmol), 1-(bromomethyl)-4-(tert-butyl)benzene (792 mg, 3.5 mmol) and iodine (100 mg) in DMF (3 mL) was stirred at 25 °C for 1 h under nitrogen, followed by the addition of methyl 2-chloro-4,6-dimethylpyrimidine-5-carboxylate (described in Example 53) (350 mg, 1.75 mmol), 2-dicyclohexylphosphino-2',6'-dimethoxybiphenyl (36 mg, 0.09 mmol) and Pd₂(dba)₃ (40 mg, 0.04 mmol). The resulting mixture was stirred for another 3 h at 60 °C under nitrogen. The reaction mixture was diluted with EtOAc (50 mL), which was washed with brine (50 mL). The organic layer was dried over Na₂SO₄, concentrated and the residue was purified by silica gel chromatography, eluting with 20% EtOAc in petroleum ether, to give methyl 2-(4-(tert-butyl)benzyl)-4,6-dimethylpyrimidine-5-carboxylate (350 mg, 51% yield) as colorless oil. ¹H NMR (400 MHz, CDCl₃) δ 7.32 (br s, 4H), 4.18 (s, 2H), 3.94 (s, 3H), 2.51(s, 6H), 1.30 (s, 9H).

Step 2: Typical ester hydrolysis condition (NaOH, MeOH/H₂O) as described in Example H were applied to methyl 2-(4-(*tert*-butyl)benzyl)-4,6-dimethylpyrimidine-5-carboxylate to give 2-(4-(*tert-*butyl)benzyl)-4,6-dimethylpyrimidine-5-carboxylic acid as a white solid.

Compound 287 (formic acid salt) was prepared as a white solid from Compound **101-K** and 2-(4-(*tert*-butyl)benzyl)-4,6-dimethylpyrimidine-5-carboxylic acid by utilizing methods analogous to those described in Example G. LCMS (Method 5-95 AB, ESI): t_{R} = 0.694 min, [M + H]⁺ = 918.5; ¹H NMR (400 MHz, MeOH-*d*₄) δ 8.46 (brs, 3H), 7.31-7.18 (m, 7H), 7.11 (d, *J*=8.8 Hz, 1H), 6.90 (d, *J*=2.0 Hz, 1H), 6.80 (s, 1H), 6.38 (s, 1H), 5.17-5.15 (m, 1H), 4.83-4.77 (m, 2H), 4.26-4.13 (m, 8H), 3.34-3.05 (m, 8H), 2.97 (s, 3H), 2.49 (s, 6H), 2.25-2.15 (m, 1H), 2.15-2.01 (m, 1H), 1.34 (d, J=6.4 Hz, 3H), 1.29 (s, 9H).

### Example 88: Synthesis of Compound 288

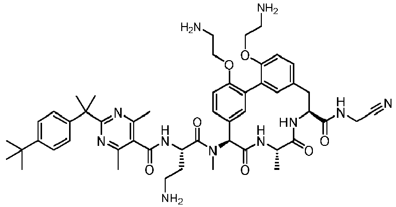

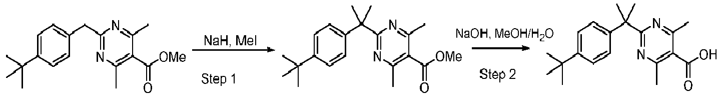

Step 1: A mixture of methyl 2-[(4-tert-butylphenyl)methyl]-4,6-dimethyl-pyrimidine-5-carboxylate (described in Example 87) (150.0 mg, 0.4800 mmol) and NaH (28.8 mg, 1.2 mmol) in DMF (5 mL) was stirred at 0 °C for 2 h, followed by the addition of iodomethane (2.6 g, 18.3 mmol). The resulting mixture was stirred at 20 °C for 5 h. The reaction was diluted with water (30 mL), which was extracted by EtOAc (30 mL × 3). The combined organic layers were washed with brine (50 mL × 2), dried over MgSO₄, concentrated and the residue was purified by preparatory-TLC (eluent: 10% EtOAc in petroleum, Rf =0.5) to give methyl 2-[1-(4-*tert*-butylphenyl)-1-methyl-ethyl]-4,6-dimethyl-pyrimidine-5-carboxylate (40 mg, 24.5% yield) as a colorless oil. LCMS (Method 5-95 AB, ESI): t_{R} = 1.050 min, [M + H]⁺ = 341.3.

Step 2: Starting from methyl 2-[1-(4-*tert*-butylphenyl)-1-methyl-ethyl]-4,6-dimethyl-pyrimidine-5-carboxylate (40 mg, 0.12 mmol), ester hydrolysis conditions (NaOH, MeOH/H₂O, described in Example H) were followed to give 2-(2-(4-(*tert-*butyl)phenyl)propan-2-yl)-4,6-dimethylpyrimidine-5-carboxylic acid (38 mg, 99% yield) as a white solid.

Compound 288 (formic acid salt) was prepared as a white solid from Compound **101-K** and 2-(2-(4-(*tert*-butyl)phenyl)propan-2-yl)-4,6-dimethylpyrimidine-5-carboxylic acid by utilizing methods analogous to those described in Example G. LCMS (Method 5-95 AB, ESI): t_{R} = 0.755 min, [M + H]⁺ = 946.5; ¹H NMR (400 MHz, MeOH-*d*₄) δ 8.47 (brs, 4H), 7.40-7.20 (m, 6H), 7.17 (d, *J*=8.4 Hz, 1H), 7.10 (d, *J*=8.4 Hz, 1H), 6.90 (d, *J*=2.0 Hz, 1H), 6.82 (s, 1H), 6.37 (s, 1H), 5.17-5.15 (m, 1H), 4.80-4.77 (m, 1H), 4.40-4.10 (m, 7H), 3.34-3.00 (m, 8H), 2.97 (s, 3H), 2.80-2.70 (m, 3H), 2.48 (s, 3H), 2.25-2.15 (m, 1H), 2.15-2.00 (m, 1H), 1.70 (d, *J*=7.2Hz, 3H), 1.34-1.25 (m, 15H).

### Example 89: Synthesis of Compound 289

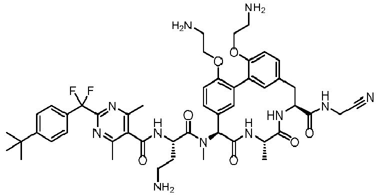

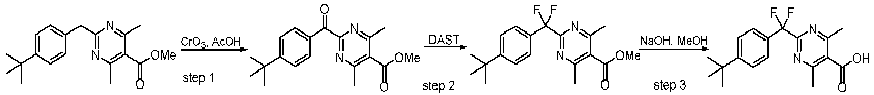

Step 1: A mixture of methyl 2-(4-(*tert*-butyl)benzyl)-4,6-dimethylpyrimidine-5-carboxylate (prepared as described in Example 88) (250 mg, 0.80 mmol) and CrO₃ (240 mg, 2.4 mmol) in acetic acid (5 mL) was stirred at 30 °C for 6 h. The reaction mixture was diluted with water (30 mL), which was extracted by EtOAc (3 × 30 mL). The combined organic layers were washed with brine (2 × 50 mL), dried over MgSO₄, concentrated and the residue was purified by prep-TLC (eluting with 20% EtOAc in petroleum ether) to give methyl 2-(4-(*tert*-butyl)benzoyl)-4,6-dimethylpyrimidine-5-carboxylate (150 mg, 57% yield) as a white solid. LCMS (Method 5-95 AB, ESI): t_{R} = 0.932 min, [M + H]⁺ = 326.9.

Step 2: A mixture of methyl 2-(4-(*tert*-butyl)benzoyl)-4,6-dimethylpyrimidine-5-carboxylate (120 mg, 0.37 mmol) and diethylaminosulfur trifluoride (2 mL) was stirred at 50 °C for 2 h. The reaction was diluted with water (30 mL), which was extracted by EtOAc (3 × 30 mL). The combined organic layers were washed with brine (2 × 50 mL), dried over MgSO₄, concentrated and the residue was purified by prep-TLC (eluting with 20% EtOAc in petroleum ether) to give methyl 2-((4-(*tert-*butyl)phenyl)difluoromethyl)-4,6-dimethylpyrimidine-5-carboxylate (80 mg, 62% yield) as a white solid. Step 3: Typical ester hydrolysis conditions (NaOH, MeOH/H₂O, as described in Example H) were applied to methyl 2-((4-(*tert*-butyl)phenyl)difluoromethyl)-4,6-dimethylpyrimidine-5-carboxylate to give 2-((4-(*tert*-butyl)phenyl)difluoromethyl)-4,6-dimethylpyrimidine-5-carboxylic acid as a white solid. LCMS (Method 5-95 AB, ESI): t_{R} = 0.899 min, [M + H]⁺ = 334.9.

Compound 289 (formic acid salt) was prepared as a white solid from Compound **101-K** and 2-((4-(*tert*-butyl)phenyl)difluoromethyl)-4,6-dimethylpyrimidine-5-carboxylic acid by utilizing methods analogous to those described in Example G. LCMS (Method 5-95 AB, ESI): t_{R} = 0.748 min, [M + H]⁺ = 954.6; ¹H NMR (400 MHz, MeOH-*d*₄) δ 8.51 (brs, 2H), 7.54 (d, *J*=8.0 Hz, 2H), 7.48 (d, *J*=8.0 Hz, 2H), 7.30-7.20 (m, 2H), 7.17 (d, *J*=8.0 Hz, 1H), 7.09 (d, *J*=8.0 Hz, 1H), 6.90 (s, 1H), 6.83 (s,1H), 6.38 (s, 1H), 5.20-5.15 (m, 1H), 4.79-4.75 (m, 1H), 4.25-4.15 (m, 7H), 3.35-3.32 (m, 1H), 3.20-3.05 (m, 7H), 2.97 (s, 3H), 2.56 (s, 6H), 2.30-2.20 (m, 1H), 2.15-2.05 (m, 1H), 1.36 (d, *J*=7.2 Hz, 3H), 1.32 (s, 9H).

### Example 90: Synthesis of Compound 290

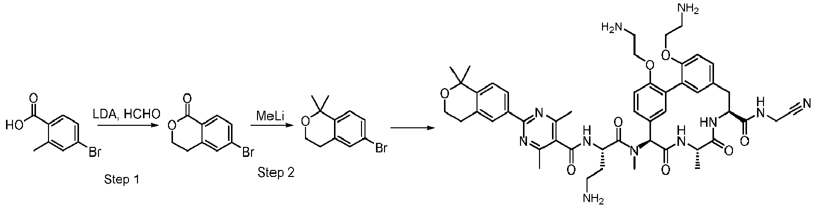

Step 1: To a solution of LDA (2N in THF, 35 mL) was added 4-bromo-2-methylbenzoic acid (5.0 g, 23.2 mmol) over 15 min and the reaction was stirred at -40 °C for 30 min. After warming to 15 °C, HCHO (2.7 g, 93 mmol) was added while keeping the internal temperature (ice water bath) below 18 °C. The resulting mixture was stirred at 15 °C for 2 h, and then cooled to 0 °C followed by the addition of aqueous 3N HCl until pH<3. The above mixture was then extracted with EtOAc (2 × 100 mL) and the combined organic layers were then concentrated to roughly 50 mL, to which Amberlyst^{®} 15 ion exchange resin (1.5 g) was added and the mixture was stirred at 48 °C for 14 h. The volatiles were removed and the residue was purified by silica-gel column, eluting with 20-30% EtOAc in petroleum ether, to give 6-bromoisochroman-1-one (1.5 g, 28% yield) as a light yellow solid. ¹H NMR (400MHz, CDCl₃) δ 7.97 (d, *J*=8.5 Hz, 1H), 7.55 (d, *J*=8.5 Hz, 1H), 7.46 (s, 1H), 4.55 (t, *J*=6.0 Hz, 2H), 3.06 (t, *J*=6.0 Hz, 2H).

Step 2: To a solution of 6-bromoisochroman-1-one (800 mg, 3.5 mmol) in THF (10 mL) was added MeMgBr (3N solution in Et₂O, 8.2 mL) at -78 °C. The mixture was stirred at the same temperature for 0.5 h; then warmed up to 20 °C while stirring and was stirred for additional 1 h. The reaction was poured into cold saturated aqueous NH₄Cl (50 mL), which was extracted with EtOAc (2 × 50 mL). The combined organic layers were dried over Na₂SO₄, evaporated *in vacuo* and the residue was purified by silica gel chromatography, eluting with 50-60% EtOAc in petroleum ether, to give 6-bromo-1,1-dimethylisochromane (600 mg, 71% yield) as a white solid.

Compound 290 (formic acid salt) was prepared as a white solid from Compound **101-K** and 6-bromo-1,1-dimethylisochromane by utilizing methods analogous to those described in Example 53. LCMS (Method 5-95 AB, ESI): t_{R} = 0.532 min, [M + H]⁺ = 932.3; ¹H NMR (400 MHz, MeOH-*d*₄) δ 8.50 (br s, 1H), 8.10-7.95 (m, 2H), 7.33-7.18 (m, 3H), 7.13-6.95 (m, 2H), 6.83 (s, 1H), 6.63 (s, 1H), 6.48 (s, 1H), 5.35-5.25 (m, 1H), 4.82-4.70 (m, 2H), 4.48-4.30 (m, 4H), 4.25 (s, 2H), 3.98 (t, *J*=5.6 Hz, 2H), 3.30-3.28 (m, 2H), 3.24 (brs, 2H), 3.14 (t, *J*=8.0 Hz, 2H), 3.02 (s, 3H), 2.97-2.62 (m, 4H), 2.44 (s, 6H), 2.33-2.16 (m, 2H), 1.57 (s, 6H), 1.35 (d, *J*=6.8 Hz, 3H).

### Example 91: Synthesis of Compound 291

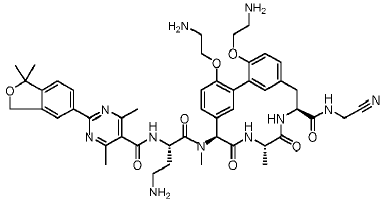

Compound 291 (formic acid salt) was prepared as a white solid from Compound **101-K** by utilizing methods analogous to those described in Example 53. LCMS (Method 5-95 AB, ESI): t_{R} = 0.674 min, [M + H]⁺ = 918.5; ¹H NMR (400 MHz, MeOH-*d*₄) δ 8.53 (brs, 2H), 8.10-8.04 (m, 3H), 7.25-7.21 (m, 2H), 7.06 (d, *J*=7.6 Hz, 2H), 6.82 (s, 1H), 6.68 (brs, 2H), 5.31-5.27 (m, 2H), 4.80-4.75 (m, 3H), 4.26-4.17 (m, 6H), 3.31-3.25 (m, 1H), 3.17-3.13 (m, 7H), 3.09 (s, 3H), 2.39 (s, 6H), 2.23-2.15 (m, 1H), 2.12-2.05 (m, 1H), 1.51 (s, 6H),1.34 (d, *J*=6.8 Hz, 3H).

### Example 92: Synthesis of Compound 292

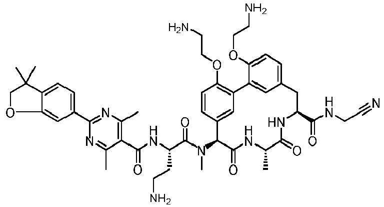

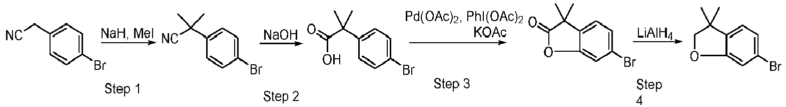

Step 1: Typical alkylation (NaH, MeI) conditions (as described in Example 88) were applied to 1-bromo-4-(isocyanomethyl)benzene to give 1-bromo-4-(2-isocyanopropan-2-yl)benzene as a yellow oil.

Step 2: A mixture of 1-bromo-4-(2-isocyanopropan-2-yl)benzene (1.77 g, 7.9 mmol) and NaOH (948 mg, 23.7 mmol) in EtOH/H₂O (40 mL, v/v=1/1) was stirred at 100 °C for 16 h. The volatiles were removed and the residue was treated with aq 1N HCl until pH=4, which was then extracted with EtOAc (3x 30 mL). The combined organic layers were washed with brine (2 × 80 mL), dried over Na₂SO₄ and evaporated *in vacuo* to give 2-(4-bromophenyl)-2-methylpropanoic acid (1.9 g, 99% yield) as a yellow solid.

Step 3: A mixture of 2-(4-bromophenyl)-2-methylpropanoic acid (1.0 g, 4.1 mmol), PhI(OAc)₂ (2.0 g, 6.2 mmol), Pd(OAc)₂ (46 mg, 0.21 mmol), 2-acetamidoacetic acid (144 mg, 1.2 mmol) and K₂CO₃ (807 mg, 8.2 mmol) in *tert*-butanol (10 mL) was stirred at 100 °C for 20 h under nitrogen. The volatiles were removed and the residue was purified by silica gel chromatography, eluting with 0-10% EtOAc in petroleum ether, to give 6-bromo-3,3-dimethylbenzofuran-2(3*H*)-one (260 mg, 26% yield) as a off-white solid. ¹H NMR (400 MHz, MeOH-*d*4) δ 7.37 (s, 1H), 7.36 (d, *J*=7.6 Hz, 1H), 7.28 (d, *J*=7.6 Hz, 1H), 1.47 (s, 6H).

Step 4: To a solution of lithium aluminum hydride (49 mg, 1.29 mmol) in THF (10 mL) was added 6-bromo-3,3-dimethylbenzofuran-2(3*H*)-one (260 mg, 1.1 mmol) at 0 °C and the mixture was stirred for at 25 °C for 2 h. The reaction was quenched with saturated aqueous NH₄Cl (20 mL), which was extracted with EtOAc (2 × 25 mL). The combined organic layers were washed with brine (50 mL), concentrated and the residue was purified by preparatory TLC (eluting with 30% ethyl acetate in petroleum ether, R_{f}=0.5) to give 6-bromo-3,3-dimethyl-2,3-dihydrobenzofuran (180 mg, 74% yield) as a off-white solid.

Compound 292 (formic acid salt) was prepared as a white solid from Compound **101-K** and 6-bromo-3,3-dimethyl-2,3-dihydrobenzofuran by utilizing methods analogous to those described in Example 53. LCMS (Method 5-95 AB, ESI): t_{R} = 0.688 min, [M + H]⁺ = 918.5; ¹H NMR (400 MHz, MeOH-*d*₄) δ 8.53 (br s, 2H), 7.92 (d, *J*=8.0 Hz, 1H), 7.49-7.38 (m, 1H), 7.32-7.26 (m, 1H), 7.24-7.19 (m, 1H), 7.17 (d, *J*=8.0 Hz, 1H), 7.00-6.93 (m, 1H), 6.89-6.66 (m, 3H), 6.13 (brs, 1H), 5.39-5.31 (m, 1H), 4.80-4.70 (m, 2H), 4.51-4.33 (m, 2H), 4.33-4.16 (m, 6H), 3.40-3.33 (m, 2H), 3.20-3.05 (m, 4H), 3.02 (s, 3H), 2.88-2.67 (m, 2H), 2.31 (s, 6H), 2.23-1.98 (m, 2H), 1.43-1.30 (m, 9H).

### Example 93: Synthesis of Compound 293

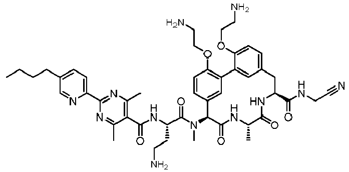

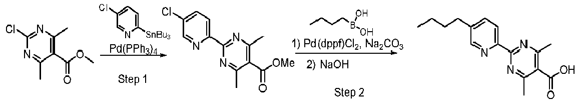

Step 1: A mixture of methyl 2-chloro-4,6-dimethylpyrimidine-5-carboxylate (described in Example 53) (200 mg, 1.00 mmol), 5-chloro-2-(tributylstannyl)pyridine (400 mg, 0.99 mmol), and Pd(PPh₃)₄ (115 mg, 0.10 mmol) in toluene (6 mL) was stirred at 110 °C for 16 h under nitrogen. The volatiles were removed and the residue was purified by silica gel chromatography, eluting with 0-50% EtOAc in petroleum ether, to give methyl 2-(5-chloropyridin-2-yl)-4,6-dimethylpyrimidine-5-carboxylate (140 mg, 51% yield) as a white solid.

Step 2: Starting from methyl 2-(5-chloropyridin-2-yl)-4,6-dimethylpyrimidine-5-carboxylate, typical Suzuki coupling and ester hydrolysis conditions, as described in Example H, were followed to give 2-(5-butylpyridin-2-yl)-4,6-dimethylpyrimidine-5-carboxylic acid as a white solid.

Compound 293 (formic acid salt) was prepared as a white solid from Compound **101-K** and 2-(5-butylpyridin-2-yl)-4,6-dimethylpyrimidine-5-carboxylic acid by utilizing methods analogous to those described in Example G. LCMS (Method 5-95 AB, ESI): t_{R} = 0.636 min, [M + H]⁺ = 905.8; ¹H NMR (400 MHz, MeOH-*d*₄) δ 8.85 (br s, 1H), 8.42 (d, *J*=8.0 Hz, 2H), 7.76-7.72 (m, 1H), 7.29 (d, *J*=8.0 Hz, 1H), 7.24-7.20 (m, 1H), 6.86-6.59 (m, 4H), 5.45-5.36 (m, 1H), 4.79-4.70 (m, 2H), 4.67-4.41 (m, 2H), 4.31-4.15 (m, 4H), 3.59-3.34 (m, 4H), 3.30-3.03 (m, 4H), 3.01 (s, 3H), 2.76 (t, *J*=7.6 Hz, 2H), 2.37 (s, 6H), 2.27-2.00 (m, 2H), 1.79-1.67 (m, 2H), 1.55-1.44 (m, 2H), 1.36 (d, *J*=6.8 Hz, 3H), 1.04 (t, *J*=7.2 Hz, 3H).

### Example 94: Synthesis of Compound 294

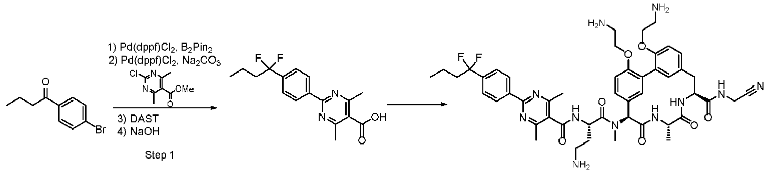

Step 1: Starting with 1-(4-bromophenyl)butan-1-one, typical Suzuki borylation, Suzuki, di-fluoronation (described in Example 89) and ester hydrolysis (NaOH, MeOH/H₂O) conditions were applied to give 2-(4-(1,1-difluorobutyl)phenyl)-4,6-dimethylpyrimidine-5-carboxylic acid as a white solid. LCMS (Method 5-95 AB, ESI): t_{R} = 0.844 min, [M + H]⁺ = 320.9

Compound 294 (formic acid salt) was prepared as a white solid from Compound **101-K** and 2-(4-(1,1-difluorobutyl)phenyl)-4,6-dimethylpyrimidine-5-carboxylic acid by utilizing methods analogous to those described in Example G. LCMS (Method 5-95 AB, ESI): t_{R} = 0.725 min, [M + H]⁺ = 940.5; ¹H NMR (400 MHz, MeOH-*d*₄) δ 8.37 (d, *J*=8.0 Hz, 2H), 7.55 (d, *J*=8.0 Hz, 2H), 7.32-7.22 (m, 2H), 7.08 (brs, 2H), 6.86 (s, 1H), 6.56 (brs, 2H), 5.31-5.25 (m, 1H), 4.82-4.75 (m, 2H), 4.44-4.20 (m, 6H), 3.38 - 3.32 (m, 3H), 3.18-3.13 (m, 3H), 3.07-2.96 (m, 5H), 2.49 (s, 6H), 2.35-2.09 (m, 4H), 1.52-1.42 (m, 2H), 1.35 (d, *J*=6.8 Hz, 3H), 0.98 (t, *J*=7.2 Hz, 3H).

### Example 95: Synthesis of Compound 295

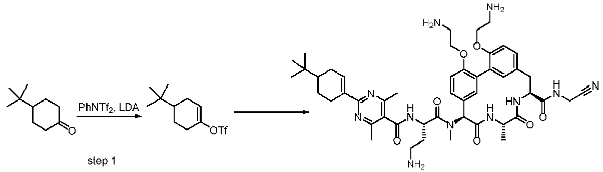

Step 1: To a solution of 4-(tert-butyl)cyclohexan-1-one (1.54 g, 10 mmol) in THF (20 mL) was added LDA (2N in THF, 5.5 mL) dropwise at -78 °C and the mixture was stirred at the same temperature for 2 h, followed by the addition of PhNTf₂ (3.9 g, 11 mmol). The resulting mixture was stirred at 20 °C for 12 h. The reaction was diluted with EtOAc (60 mL), which was washed with brine (2 × 50 mL). The organic layer was dried over Na₂SO₄, concentrated and the residue was purified by silica gel chromatography, eluting with 5% EtOAc in petroleum ether, to give 4-(*tert*-butyl)cyclohex-1-en-1-yl trifluoromethanesulfonate (800 mg, 28% yield) as a colorless oil. ¹H NMR (400 MHz, MeOH-d4): δ 5.74 (t, *J*=2.8 Hz, 1H), 2.40-2.10 (m, 3H), 2.00-1.85 (m, 2H), 1.40-1.20 (m, 2H), 0.87 (s, 9H).

Compound 295 (formic acid salt) was prepared as a white solid from Compound **101-K** and 4-(*tert*-butyl)cyclohex-1-en-1-yl trifluoromethanesulfonate by utilizing methods analogous to those described in Examples 53 and 10. LCMS (Method 5-95 AB, ESI): t_{R} = 0.740 min, [M + H]⁺ = 908.5; ¹H NMR (400 MHz, MeOH-*d*₄) δ 8.56 (br s, 3H), 7.30-7.15 (m, 4H), 7.12-7.05 (m, 1H), 6.85 (brs, 1H), 6.68 (brs, 1H), 6.45 (brs, 1H), 5.20-5.10 (m, 1H), 4.79-4.75 (m, 2H), 4.40-4.10 (m, 6H), 3.33-3.05 (m, 8H), 2.98 (s, 3H), 2.90-2.60 (m, 1H), 2.44 (s, 6H), 2.40-1.90 (m, 7H), 1.36 (d, *J*=6.8 Hz, 3H), 1.23-1.05 (m, 1H), 0.98 (s, 9H).

### Example 96: Synthesis of Compound 296

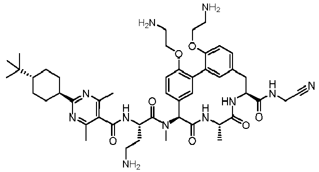

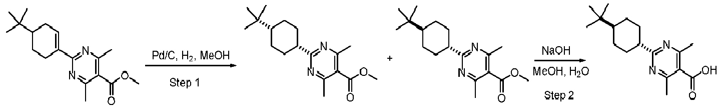

Step 1: A mixture of 2-(4-*tert*-butylcyclohexen-1-yl)-4,6-dimethyl-pyrimidine-5-carboxylate (as described in Example 95) (250 mg, 0.83 mmol) and 10% Pd/C (88 mg, 0.08 mmol) in MeOH (100 mL) was stirred at 15 °C for 5 h under H₂. The volatiles were removed and the residue was purified and the *cis* and *trans* stereoisomers separated by preparatory-TLC (10% EtOAc in petroleum ether, Rf=0.7) to give (*cis*)-methyl 2-(4-*tert*-butylcyclohexyl)-4,6-dimethyl-pyrimidine-5-carboxylate (70 mg, 28% yield) and (*trans*)-methyl 2-(4-*tert*-butylcyclohexyl)-4,6-dimethyl-pyrimidine-5-carboxylate (100 mg, 40% yield) as a white solid.

Step 2: Starting from (*trans*)-methyl 2-(4-*tert*-butylcyclohexyl)-4,6-dimethyl-pyrimidine-5-carboxylate (70 mg, 0.23 mmol), typical ester hydrolysis conditions (NaOH, MeOH/H₂O) were followed to give (*trans*)-4-*tert*-butylcyclohexyl)-4,6-dimethylpyrimidine-5-carboxylic acid (65 mg, 97% yield) as a white solid.

Compound 296 (formic acid salt) was prepared as a white solid from Compound **101-K** and (*trans*)-4-*tert*-butylcyclohexyl)-4,6-dimethylpyrimidine-5-carboxylic acid by utilizing methods analogous to those described in Example 53. LCMS (Method 5-95 AB, ESI): t_{R} = 0.740 min, [M + H]⁺ = 910.6; ¹H NMR (400 MHz, MeOH-*d*₄) δ 8.56 (br s, 3H), 7.29-7.26 (m, 2H), 7.19 (d, *J*= 8.4 Hz, 1H), 7.10 (d, *J*=8.4 Hz, 1H), 6.90 (d, *J*=2.0 Hz, 1H), 6.82 (s, 1H), 6.38 (s, 1H), 5.20-5.15 (m, 1H), 4.79-4.75 (m, 2H), 4.40-4.10 (m, 6H), 3.33-3.05 (m, 8H), 2.97 (s, 3H), 2.75-2.65 (m, 1H), 2.49 (s, 6H), 2.30-2.05 (m, 2H), 2.00-1.85 (m, 4H), 1.70-1.55 (m, 2H), 1.35 (d, *J*=6.8 Hz, 3H), 1.25-1.05 (m, 3H), 0.91 (s, 9H).

### Example 97: Synthesis of Compound 297

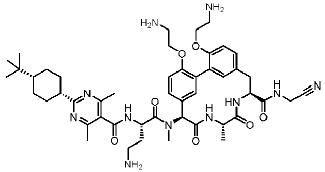

Compound 297 (formic acid salt) was prepared as a white solid from Compound **101-K** and (*cis*)-methyl 2-(4-*tert*-butylcyclohexyl)-4,6-dimethyl-pyrimidine-5-carboxylate by utilizing methods analogous to those described in Example 96. LCMS (Method 5-95 AB, ESI): t_{R} = 0.740 min, [M + H]⁺ = 910.6; ¹H NMR (400 MHz, MeOH-*d*₄) δ 8.50 (br s, 2H), 7.28-7.26 (m, 2H), 7.18 (d, *J*=8.4 Hz, 1H), 7.09 (d, *J*=8.8 Hz, 1H),, 6.90 (d, *J*=2.0 Hz, 1H), 6.82 (d, *J*=2.0 Hz, 1H), 6.39 (s, 1H), 5.20-5.15 (m, 1H), 4.79-4.75 (m, 2H), 4.40-4.10 (m, 6H), 3.25-3.05 (m, 8H), 2.98 (s, 3H), 2.55 (s, 6H), 2.55-2.40 (m, 2H), 2.30-2.00 (m, 2H), 1.75-1.60 (m, 2H), 1.60-1.50 (m, 2H), 1.40-1.20 (m, 3H), 1.38 (d, *J*=6.8 Hz, 3H), 1.20-1.05 (m, 1H), 0.81 (s, 9H).

### Example 98: Synthesis of Compound 298

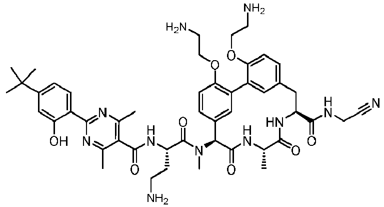

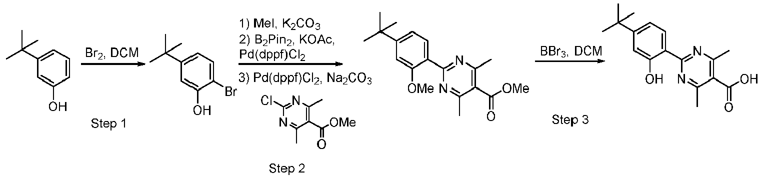

Step1: To a solution of 3-(*tert*-butyl)phenol (1.5 g, 10 mmol) in DCM (20 mL) was added a solution of bromine (0.51 mL, 10 mmol) in DCM (5 mL) over 15 min while keeping the temperature of the reaction below 35 °C. After that, the reaction was quenched with 5% aqueous Na₂S₂SO₃ (15 mL) while stirring. The organic layer was separated, washed with brine (50 mL), dried over Na₂SO₄, concentrated and the residue was purified by flash chromatography on silica, eluting with 0-5% EtOAc in petroleum ether, to give 2-bromo-5-(*tert*-butyl)phenol (1.6 g, 70% yield) as a colorless oil. ¹H NMR (400 MHz, CDCl₃) δ 7.37 (d, *J*=8.0 Hz, 1H), 7.07 (s, 1H), 6.85 (d, *J*= 8.0 Hz, 1H), 5.44 (s, 1H), 1.30 (s, 9H).

Step 2: Starting from 2-bromo-5-(tert-butyl)phenol, typical alkylation (as described in Example 21), Suzuki borylation and Suzuki coupling conditions (as described in Example 10) were applied to give methyl 2-(4-(*tert*-butyl)-2-methoxyphenyl)-4,6-dimethylpyrimidine-5-carboxylate as a yellow oil. LCMS (ESI): [M + H]⁺ = 329.0.

Step 3: To a solution of 2-(4-(*tert*-butyl)-2-methoxyphenyl)-4,6-dimethylpyrimidine-5-carboxylate (150 mg, 0.46 mmol) in DCM (10 mL) was added BBr₃ (87 µL, 0.91 mmol) and the mixture was stirred at 0 °C for 12 h. The reaction was quenched with 5% aqueous Na₂S₂SO₃ (10 mL), the organic layer was separated, washed with brine (50 mL), dried over Na₂SO₄, concentrated and the residue was purified by HPLC to give 2-(4-(*tert*-butyl)-2-hydroxyphenyl)-4,6-dimethylpyrimidine-5-carboxylic acid (50 mg, 36.4% yield) as a yellow solid. LCMS (ESI): [M + H]⁺ = 300.9.

Compound 298 (formic acid salt) was prepared as a white solid from Compound **101-K** and 2-(4-(*tert*-butyl)-2-hydroxyphenyl)-4,6-dimethylpyrimidine-5-carboxylic acid by utilizing methods analogous to those described in Example G. LCMS (Method 5-95 AB, ESI): t_{R} = 0.730 min, [M + H]⁺ = 920.5; ¹H NMR (400 MHz, MeOH-*d*₄) δ 8.50 (br s, 3H), 8.34 (d, *J*=8.4 Hz, 2H), 7.30-7.25 (m, 2H), 7.02-6.98 (m, 2H), 6.82 (s, 1H), 6.69 (brs, 1H), 5.35-5.31 (m, 1H), 4.85-4.78 (m, 2H), 4.32-4.12 (m, 6H), 3.38-3.34 (m, 2H), 3.16-3.12 (m, 4H), 3.02 (s, 3H), 3.00-2.90 (m, 2H), 2.43 (s, 6H), 2.31-2.10 (m, 2H), 1.40-1.35 (m, 12H).

### Example 99: Synthesis of Compound 299

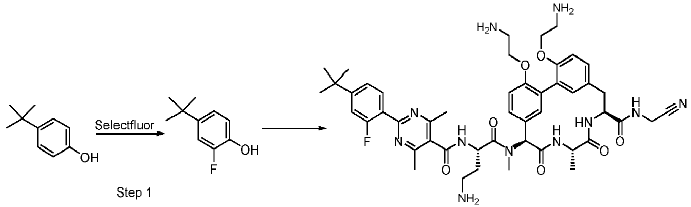

Step 1: A mixture of 4-(*tert*-butyl)phenol (2.0 g, 13 mmol) and Selectfluor (5.2 g, 14.6 mmol) in MeOH (25 mL) was stirred at 85 °C for 4 h. The volatiles were removed and the residue was taken up by EtOAc (50 mL), which was washed with brine (2 × 50 mL). The organic layer was dried over Na₂SO₄, concentrated and the residue was purified by silica gel chromatography, eluting with 0-10% EtOAc in petroleum ether, to give 4-(*tert*-butyl)-2-fluorophenol (900 mg, 40% yield). ¹H NMR (400 MHz, CDCl₃) δ 7.12-7.00 (m, 2H), 6.96-6.89 (m, 1H), 5.04(s, 1H), 1.29 (s, 9H).

Compound 299 (formic acid salt) was prepared as a white solid from Compound **101-K** and 4-(tert-butyl)-2-fluorophenol by utilizing methods analogous to those described in Examples 10 and 53. LCMS (Method 5-95 AB, ESI): t_{R} = 0.711 min, [M + H]⁺ = 922.5; ¹H NMR (400 MHz, MeOH-*d*₄) δ 8.51(br s, 3H), 7.85-7.82 (m, 1H), 7.36-7.05 (m, 6H), 6.88 (s, 1H), 6.68(s, 1H), 6.51 (s, 1H), 5.30-5.24 (m, 1H), 4.80-4.70 (m, 2H), 4.30-4.20 (m, 6H), 3.32-3.00 (m, 8H), 2.95 (s, 3H), 2.56 (s, 6H), 2.35-2.25 (m, 1H), 2.25-2.15 (m, 1H), 1.38 (s, 9H), 1.36 (d, J=6.4 Hz, 3H).

### Example 100: Synthesis of Compound 300

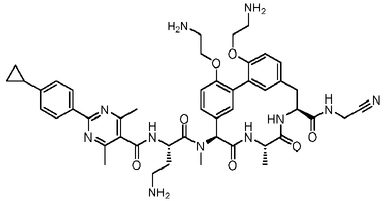

Compound 300 (formic acid salt) was prepared as a white solid from Compound **101-K** by utilizing methods analogous to those described in Example 56. LCMS (Method 5-95 AB, ESI): t_{R} = 0.683 min, [M + H]⁺ = 888.4; ¹H NMR (400 MHz, MeOH-*d*₄) δ 8.52 (br s, 1H), 8.10 (d, *J*=7.6Hz, 2H), 7.26 (brs, 2H), 7.15-7.01 (m, 4H), 6.84 (s, 1H), 6.62 (s, 1H), 6.51 (s, 1H), 5.33-5.27 (m, 1H), 4.80-4.72 (m, 1H), 4.36 (brs, 3H), 4.27-4.23 (m, 4H), 3.29-3.26 (m, 2H), 3.22-3.06 (m, 6H), 3.02 (s, 3H), 2.99-2.90 (m, 2H), 2.43 (s, 6H), 2.33-2.23 (m, 1H), 2.21-2.10 (m, 1H), 2.03-1.93 (m, 1H), 1.35 (d, *J*=6.8 Hz, 3H), 1.10-1.03 (m, 2H), 0.81-0.75 (m, 2H).

### Example 101: Synthesis of Compound 301

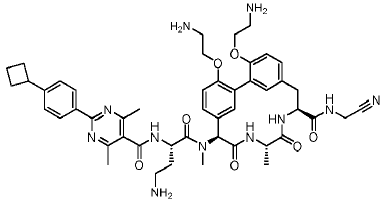

Compound 301 (formic acid salt) was prepared as a white solid from Compound **101-K** by utilizing methods analogous to those described in Example 56. LCMS (Method 5-95 AB, ESI): t_{R} = 0.709 min, [M + H]⁺ = 902.4; ¹H NMR (400 MHz, MeOH-*d*₄) δ 8.53 (br s, 1H), 8.15 (d, *J*=7.8 Hz, 2H), 7.28-7.23 (m, 4H), 7.07-6.95 (m, 2H), 6.83 (s, 1H), 6.67 (s, 1H), 6.46 (s, 1H), 5.33-5.29 (m, 1H), 4.79-4.74 (m, 2H), 4.32-4.19 (m, 6H), 3.68-3.58 (m, 1H), 3.22-3.06 (m, 7H), 3.02 (s, 3H), 2.47-2.38 (m, 2H), 2.42 (s, 6H), 2.32-2.04 (m, 6H), 1.97-1.88 (m, 1H), 1.34 (d, *J*=6.8 Hz, 3H).

### Example 102: Synthesis of Compound 302

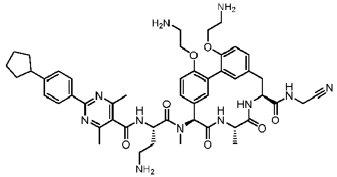

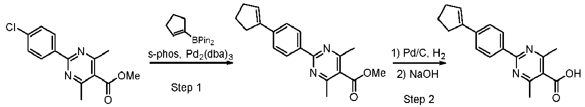

Step 1: A solution of methyl 2-(4-chlorophenyl)-4,6-dimethylpyrimidine-5-carboxylate (100mg, 0.36 mmol, described in Example 56), 2-(cyclopent-1-en-1-yl)-4,4,5,5-tetramethyl-1,3,2-dioxaborolane (91 mg, 0.47 mmol), Pd₂(dba)₃ (8.3 mg, 0.01 mmol), S-phos(7.4 mg, 0.02 mmol) and K₃PO₄ (230 mg, 1.1 mmol) in 1,4-dioxane/H₂O (6 mL, v/v=5/1) was stirred at 110 °C for 16 h under N₂. After filtration, the volatiles were removed under reduced pressure and the residue was purified by preparatory-TLC (eluent: EtOAc: petroleum ether=1:10) to obtain methyl 2-(4-(cyclopent-1-en-1-yl)phenyl)-4,6-dimethylpyrimidine-5-carboxylate (110 mg, 99% yield) as a white solid. LCMS (Method 5-95 AB, ESI): t_{R} = 0.969 min, [M + H]⁺ = 309.3.

Step 2: Starting from methyl 2-(4-(cyclopent-1-en-1-yl)phenyl)-4,6-dimethylpyrimidine-5-carboxylate, hydrogenation (as described in Example D) and ester hydrolysis (as described in Example H) conditions were followed to give 2-(4-cyclopentylphenyl)-4,6-dimethylpyrimidine-5-carboxylic acid as a white solid. LCMS (Method 5-95 AB, ESI): t_{R} = 0.986 min, [M + H]⁺ = 297.0

Compound 302 (formic acid salt) was prepared as a white solid from Compound **101-K** and 2-(4-cyclopentylphenyl)-4,6-dimethylpyrimidine-5-carboxylic acid by utilizing methods analogous to those described in Example G. LCMS (Method 5-95 AB, ESI): t_{R} = 0.727 min, [M + H]⁺ = 916.4; ¹H NMR (400 MHz, MeOH-*d*₄) δ 8.50 (br s, 2H), 8.34-8.30 (m, 2H), 7.40 (d, *J*=8.8 Hz, 2H), 7.37-7.29 (m, 2H), 7.22 (d, *J*=8.8 Hz, 2H), 7.13-7.08 (m, 2H), 6.51 (brs, 1H), 5.28-5.22 (m, 1H), 4.85-4.75 (m, 2H), 4.35-4.23 (m, 6H), 3.26-3.00 (m, 8H), 3.01 (s, 3H), 2.57 (s, 6H), 2.33-2.13 (m, 4H), 1.95-1.62 (m, 7H), 1.36 (d, J=6.8 Hz, 3H).

### Example 103: Synthesis of Compound 303

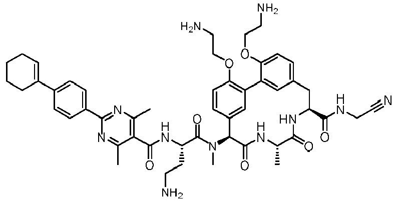

Compound 303 (formic acid salt) was prepared as a white solid from Compound **101-K** by utilizing methods analogous to those described in Example 302. LCMS (Method 5-95 AB, ESI): t_{R} = 0.610 min, [M + H]⁺ = 928.5; ¹H NMR (400 MHz, MeOH-*d*₄) δ 8.47 (b rs, 2H), 8.37-8.31 (m, 2H), 7.52 (d, J=8.0 Hz, 2H), 7.33-7.17 (m, 3H), 7.16-7.01 (m, 2H), 6.79 (s, 1H), 6.50 (s, 1H), 6.29 (s, 1H), 5.29-5.18 (m, 1H), 4.83-4.75 (m, 2H), 4.25-4.10 (m, 4H), 4.20 (s, 2H), 3.56-3.36 (m, 1H), 3.29-2.99 (m, 10H), 2.55 (s, 6H), 2.51-2.45 (m, 2H), 2.36-2.24 (m, 3H), 2.22-2.11(m, 1H), 1.90-1.80 (m, 2H), 1.75-1.65 (m, 2H), 1.44-1.35 (m, 3H).

### Example 104 : Synthesis of Compound 304

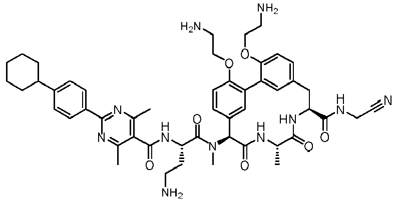

Compound 304 (formic acid salt) was prepared as a white solid from Compound **101-K** by utilizing methods analogous to those described in Example 302. LCMS (Method 5-95 AB, ESI): t_{R} = 0.610 min, [M + H]⁺ = 928.5; ¹H NMR (400 MHz, MeOH-*d*₄) δ 8.54 (br s, 1H), 8.34-8.30 (m, 2H), 7.38-7.15 (m, 5H), 7.14-7.03 (m, 2H), 6.92 (s, 0.5H), 6.79(s, 0.5H), 6.50 (s, 0.5 H), 6.41(s, 0.5H), 5.26-5.19 (m, 1H), 4.85-4.78 (m, 2H), 4.25-4.17 (m, 4H), 4.20 (s, 2H), 3.30-2.97(m, 11H), 2.66-2.60 (m, 1H), 2.57(s, 6H), 2.36-2.24 (m, 1H), 2.21-2.09 (m, 1H), 1.96-1.77 (m, 5H), 1.55-1.42 (m, 5H), 1.36 (d, J=6.8 Hz, 3H).

### Example 105: Synthesis of Compound 305

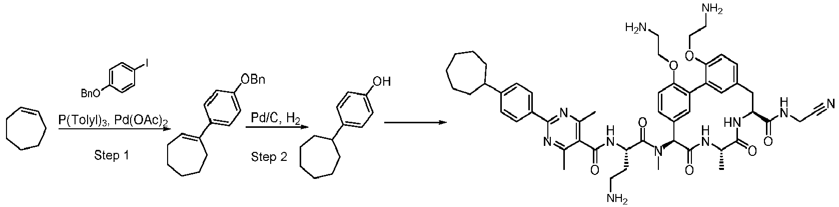

Step 1: A mixture of cycloheptene (155 mg, 1.6 mmol), 1-(benzyloxy)-4-iodobenzene (200 mg, 0.64 mmol), Pd(OAc)₂ (3.6 mg, 0.02 mmol), P(tolyl)₃ (9.8 mg, 0.03 mmol), (*t*-Bu)₄NBr (208 mg, 0.64 mmol) and K₂CO₃ (224 mg, 1.6 mmol) in DMF (10 mL) was stirred at 110 °C for 16 h. The volatiles were removed and the residue was taken up in EtOAc (50 mL), which was washed with brine (2 × 50 mL). The organic layer was dried over MgSO₄, concentrated and the residue was purified by preparatory-TLC (eluting with 1% EtOAc in petroleum ether) to give 1-(4-(benzyloxy)phenyl)cyclohept-1-ene (100 mg, 56% yield) as a colorless oil.

Step 2: Typical hydrogenation conditions (Pd/C, H₂, as described in Example D) were applied to 1-(4-(benzyloxy)phenyl)cyclohept-1-ene to give 4-cycloheptylphenol as a white solid.

Compound 305 (formic acid salt) was prepared as a white solid from Compound **101-K** and 4-cycloheptylphenol by utilizing methods analogous to those described in Examples 53 and 10. LCMS (Method 5-95 AB, ESI): t_{R} = 0.765 min, [M + H]⁺ = 944.5; ¹H NMR (400 MHz, MeOH-*d*₄) δ 8.54 (brs, 2H), 8.21-8.17 (m, 2H), 7.30-7.18 (m, 4H), 7.05 (brs, 2H), 6.84 (s, 1H), 6.65-6.55 (m, 2H), 5.30-5.22 (m, 1H), 4.83-4.79 (m, 2H), 4.33-4.11 (m, 6H), 3.17-3.04 (m, 6H), 3.04-2.85 (m, 5H), 2.80-2.70 (m, 1H), 2.46 (s, 6H), 2.30-2.20 (m, 1H), 2.16-2.03 (m, 1H), 1.98-1.82 (m, 4H), 1.80-1.60 (m, 8H), 1.35 (d, J=6.8 Hz, 3H).

### Example 106: Synthesis of Compound 306

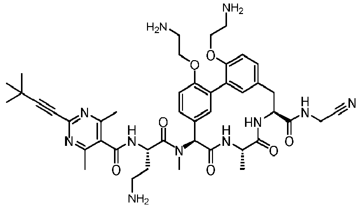

Compound 306 (formic acid salt) was prepared as a white solid from Compound **101-K** by utilizing methods analogous to those described in Examples 53 and 27. LCMS (Method 5-95 AB, ESI): t_{R} = 0.535 min, [M + H]⁺ = 852.5; ¹H NMR (400 MHz, MeOH-*d*₄) δ 8.50 (brs, 1H), 7.30-7.22 (m, 2H), 7.20 (d, *J*=8.0 Hz, 2H), 7.11 (d, *J*=8.0 Hz, 1H), 6.89 (d, *J*=1.6 Hz, 1H), 6.76 (s, 1H), 6.44 (s, 1H), 5.25-5.15 (m, 1H), 4.85-4.70 (m, 2H), 4.35-4.15 (m, 6H), 3.40-3.12 (m, 8H), 2.99 (s, 3H), 2.47 (s, 6H), 2.35-2.15 (m, 2H), 1.41 (s, 9H), 1.36 (d, *J*=7.2 Hz, 3H).

### Example 107: Synthesis of Compound 307

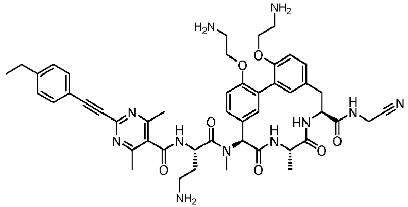

Compound 307 (formic acid salt) was prepared as a white solid from Compound **101-K** by utilizing methods analogous to those described in Examples 53 and 27. LCMS (Method 5-95 AB, ESI): t_{R} = 0.701 min, [M + H]⁺ = 900.5; ¹H NMR (400 MHz, MeOH-*d*₄) δ 8.45 (brs, 2H), 7.56 (d, *J*=8.0 Hz, 2H), 7.32-7.19 (m, 5H), 7.08 (d, *J*=8.0 Hz, 1H), 6.89 (d, *J*=2.4 Hz, 1H), 6.75(s, 1H), 6.45 (s, 1H), 5.23-5.20 (m, 1H), 4.80-4.78 (m, 2H), 4.29 (s, 2H), 4.24-4.20 (brs, 4H), 3.36-3.34 (m, 1H), 3.25-3.09 (m, 7H), 2.99 (s, 3H), 2.70 (q, J=7.6 Hz, 2H), 2.50 (s, 6H), 2.29-2.14 (m, 2H), 1.35 (d, *J*=6.8 Hz, 3H), 1.26 (t, *J*=7.6 Hz, 3H).

### Example 108: Synthesis of Compound 308

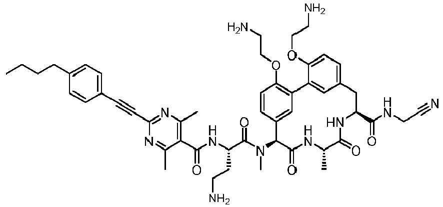

Compound 308 (formic acid salt) was prepared as a white solid from Compound **101-K** by utilizing methods analogous to those described in Examples 53 and 27. LCMS (Method 5-95 AB, ESI): t_{R} = 0.747 min, [M + H]⁺ = 928.5; ¹H NMR (400 MHz, MeOH-*d*₄) δ 8.47 (brs, 3H),7.56 (d, *J*=8.0 Hz, 2H), 7.31-7.27 (m, 3H), 7.24-7.19 (m, 2H), 7.08 (d, *J*=8.0 Hz, 1H), 6.89 (s, 1H), 6.75 (s, 1H), 6.46 (s, 1H), 5.24-5.20 (m, 1H), 4.81-4.77 (m, 2H), 4.30 (s, 2H), 4.21 (brs, 4H), 3.36-3.21 (m, 8H), 3.13 (s, 3H), 2.69 (t, *J*=7.2 Hz, 2H), 2.50 (s, 6H), 2.31-2.11 (m, 2H),1.68-1.60 (m, 2H), 1.43-1.35 (m, 5H), 0.97 (t, *J*=7.2 Hz, 3H).

### Example 109: Synthesis of Compound 309

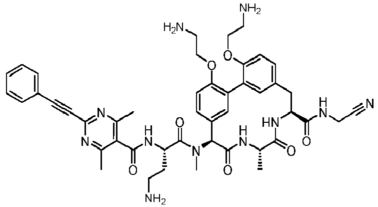

Compound 309 (formic acid salt) was prepared as a white solid from Compound **101-K** by utilizing methods analogous to those described in Examples 53 and 27. LCMS (Method 5-95 AB, ESI): t_{R} = 0.638 min, [M + H]⁺ = 872.7; ¹H NMR (400 MHz, MeOH-*d*₄) δ 8.48 (brs, 2H), 7.63 (d, *J*=8.0 Hz, 2H), 7.53-7.45 (m, 3H), 7.28-7.18 (m, 3H), 7.05 (d, *J*=8.0 Hz, 1H), 6.86 (s, 1H), 6.64 (s, 1H), 6.51(s, 1H), 5.26-5.23(m, 1H), 4.81-4.76 (m, 2H), 4.35 (s, 2H), 4.22 (brs, 4H), 3.38-3.22(m, 5H), 3.14-3.07 (m,3H), 2.99 (s, 3H), 2.47 (s, 6H), 2.31-2.12 (m, 2H), 1.35 (d, J=6.8 Hz, 3H).

### Example 110: Synthesis of Compound 310

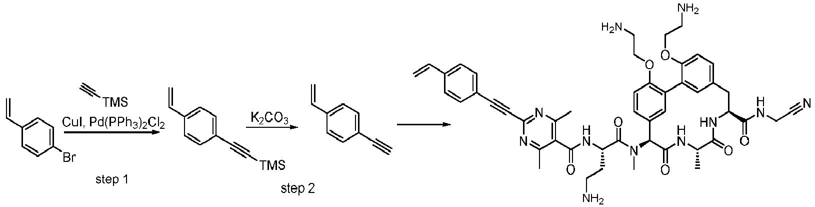

Step 1: Typical Sonogoshira condition (as described in Example 13) was applied to 1-bromo-4-vinylbenzene to give trimethyl((4-vinylphenyl)ethynyl)silane as a yellow oil.

Step 2: A mixture of trimethyl((4-vinylphenyl)ethynyl)silane (200 mg, 1.0 mmol) and K₂CO₃ (345 mg, 2.5 mmol) in MeOH (10 mL) was stirred at 25 °C for 3 h. The volatiles were removed and the residue was purified by silica gel chromatography, eluting with 0-1% EtOAc in petroleum ether, to give 1-ethynyl-4-vinylbenzene (60 mg, 47% yield) as a yellow oil.

Compound 310 (formic acid salt) was prepared as a white solid from Compound **101-K** and 1-ethynyl-4-vinylbenzene by utilizing methods analogous to those described in Examples 53 and 27. LCMS (Method 5-95 AB, ESI): t_{R} = 0.692 min, [M + H]⁺ = 898.6; ¹H NMR (400 MHz, MeOH-*d*₄) δ 8.14 (brs, 3H), 7.63 (d, *J*=8.4 Hz, 2H), 7.54 (d, *J*=8.4 Hz, 2H), 7.30-7.09 (m, 4H), 6.93 (s, 1H), 6.84-6.75 (m, 2H), 6.42 (s, 1H), 5.93 (d, J=17.6 Hz, 1H), 5.38 (d, J=11.2 Hz, 1H), 5.23-5.19 (m, 1H), 4.81-4.77 (m, 2H), 4.34-4.22 (m, 4H), 4.20 (s, 2H), 3.49-3.46 (m, 1H), 3.20-3.09 (m, 7H), 3.01 (s, 3H), 2.54 (s, 6H), 2.30-2.24 (m, 1H), 2.22-2.13 (m, 1H), 1.36 (d, *J*=7.2 Hz, 3H).

### Example 111: Synthesis of Compound 311

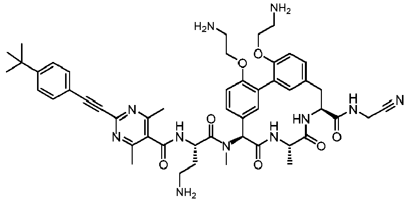

Compound 311 (formic acid salt) was prepared as a white solid from Compound **101-K** by utilizing methods analogous to those described in Example 110. LCMS (Method 5-95 AB, ESI): t_{R} = 0.741 min, [M + H]⁺ = 928.5; ¹H NMR (400 MHz, MeOH-*d*₄) δ 8.49 (brs, 2H), 7.59 (d, *J*=8.0 Hz, 2H), 7.50 (d, *J*=8.0 Hz, 2H), 7.32-7.17 (m, 3H), 7.10 (d, *J*=8.4 Hz, 1H), 6.92 (brs, 1H), 6.84-6.80 (m, 1H), 6.24 (s, 1H), 5.38-5.34 (m, 1H), 4.83-4.76 (m, 2H), 4.28-4.18 (m, 4H), 4.20 (s, 2H), 3.38-3.08 (m, 8H), 3.01 (s, 3H), 2.52 (s, 6H), 2.28-2.23 (s, 1H), 2.20-2.16 (m, 2H), 1.35 (s, 9H), 1.33 (d, J=6.8 Hz, 3H).

### Example 112: Synthesis of Compound 312

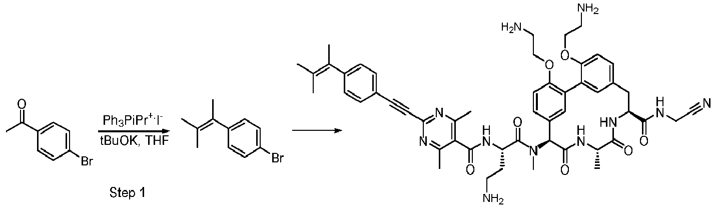

Step 1: Typical Wittig condition (as described in Example 12) was applied to 1-(4-bromophenyl)ethan-1-one to give 1-bromo-4-(3-methylbut-2-en-2-yl)benzene as a colorless oil.

Compound 312 (formic acid salt) was prepared as a white solid from Compound **101-K** and 1-bromo-4-(3-methylbut-2-en-2-yl)benzene by utilizing methods analogous to those described in Example 110. LCMS (Method 5-95 AB, ESI): t_{R} = 0.752 min, [M + H]⁺ = 940.6; ¹H NMR (400 MHz, MeOH-*d*₄) δ 8.49 (brs, 2H), 7.61 (d, *J*=8.0 Hz, 2H), 7.31-7.18 (m, 5H), 7.09 (d, *J*=8.4 Hz, 1H), 6.91 (d, *J*=8.4 Hz, 1H), 6.83 (s, 1H), 6.42 (s, 1H), 5.23-5.19 (m, 1H), 4.83-4.78 (m, 2H), 4.25-4.19 (m, 4H), 4.20 (s, 2H), 3.48-3.45 (m, 1H), 3.20-3.08 (m, 7H), 3.00 (s, 3H), 2.54 (s, 6H), 2.29-2.24 (m, 1H), 2.20-2.12 (m, 1H), 1.98 (s, 3H), 1.85 (s, 3H), 1.61 (s, 3H), 1.36 (d, *J*=6.4 Hz, 3H).

### Example 113: Synthesis of Compound 313

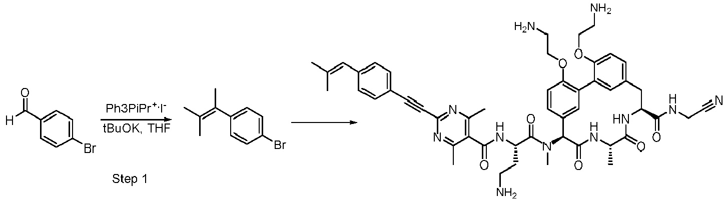

Step 1: Typical Wittig condition (as described in Example 12) was applied to 4-bromobenzaldehyde to give 1-bromo-4-(3-methylbut-2-en-2-yl)benzene as a colorless oil. ¹H NMR (400 MHz, CDCl₃) δ 7.44 (d, *J*=8.0 Hz, 2H), 7.10 (d, *J*=8.0 Hz, 2H), 1.90 (s, 3H), 1.85 (s, 3H).

Compound 313 (formic acid salt) was prepared as a white solid from Compound **101-K** and 1-bromo-4-(3-methylbut-2-en-2-yl)benzene by utilizing methods analogous to those described in Example 110. LCMS (Method 5-95 AB, ESI): t_{R} = 0.728 min, [M + H]⁺ = 926.6; ¹H NMR (400 MHz, MeOH-*d*₄) δ 8.45 (brs, 1H), 7.60 (d, *J*=8.0 Hz, 2H), 7.36-7.17 (m, 5H), 7.09 (d, *J*=8.4 Hz, 1H), 6.91 (brs, 1H), 6.79 (brs, 1H), 6.43 (s, 1H), 6.32 (s, 1H), 5.24-5.18 (m, 1H), 4.83-4.78 (m, 2H), 4.34-4.17 (m, 6H), 3.49-3.46 (m, 1H), 3.26-3.07 (m, 7H), 3.00 (s, 3H), 2.52 (s, 6H), 2.32-2.24 (m, 1H), 2.19-2.14 (m, 1H), 1.95 (s, 3H), 1.91 (s, 3H), 1.36 (d, *J*=6.8 Hz, 3H).

### Example 114: Synthesis of Compound 314

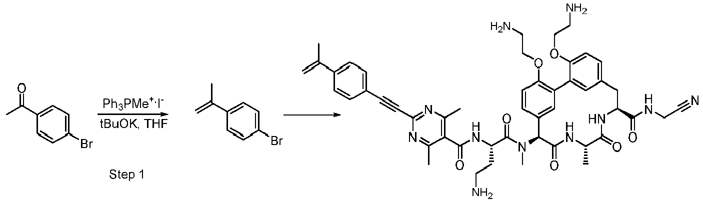

Step 1: Typical Wittig condition (as described in Example 12) was applied to 1-(4-bromophenyl)ethan-1-one to give 1-bromo-4-(prop-1-en-2-yl)benzene as a colorless oil. ¹H NMR (400 MHz, CDCl₃) δ 7.45 (d, *J*=8.4 Hz, 2H), 7.33 (d, *J*=8.4 Hz, 2H), 5.36 (s, 1H), 5.11 (s, 1H), 2.13 (s, 3H).

Compound 314 (formic acid salt) was prepared as a white solid from Compound **101-K** and 1-bromo-4-(prop-1-en-2-yl)benzene by utilizing methods analogous to those described in Example 110. LCMS (Method 5-95 AB, ESI): t_{R} = 0.730 min, [M + H]⁺ = 912.4; ¹H NMR (400 MHz, MeOH-*d*₄) δ 7.65-7.57 (m, 3H), 7.39-7.05 (m, 5H), 6.93 (brs, 1H), 6.84 (brs, 1H), 6.42 (brs, 1H), 5.51 (s, 1H), 5.23-5.19 (m, 1H), 4.82-4.77 (m, 2H), 4.35-4.18 (s, 6H), 3.48-3.35 (m, 3H), 3.28-3.07 (m, 5H), 3.00 (s, 3H), 2.54 (s, 3H), 2.23-2.12 (m, 9H), 1.36 (d, *J*=6.4 Hz, 3H).

### Example 115: Synthesis of Compound 315

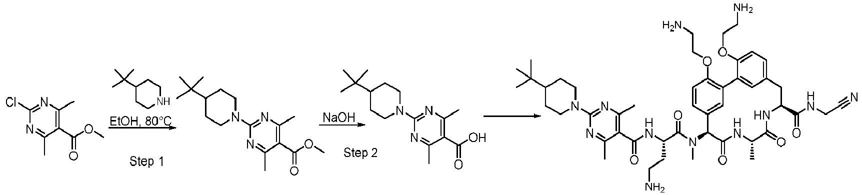

Step 1: A mixture of methyl 2-chloro-4,6-dimethylpyrimidine-5-carboxylate (described in Example 53) (150 mg, 0.75 mmol) and 4-(*tert*-butyl)piperidine (158 mg, 1.1 mmol) in EtOH (10 mL) was stirred at 80 °C for 2 h. The volatiles were removed and the residue was purified by preparatory-TLC (20% EtOAc in petroleum ether, R_{f}= 0.6) to give methyl 2-(4-(*tert*-butyl)piperidin-1-yl)-4,6-dimethylpyrimidine-5-carboxylate (140 mg, 61% yield) as a yellow oil. LCMS (Method 5-95 AB, ESI): t_{R} = 0.987 min, [M + H]⁺ = 306.0.

Step 2: Typical ester hydrolysis conditions (NaOH, MeOH/H₂O, described in Example H) were applied to methyl 2-(4-(*tert*-butyl)piperidin-1-yl)-4,6-dimethylpyrimidine-5-carboxylate to give 2-(4-(*tert-*butyl)piperidin-1-yl)-4,6-dimethylpyrimidine-5-carboxylic acid as a white solid.

Compound 315 (formic acid salt) was prepared as a white solid from Compound **101-K** and 2-(4-(tert-butyl)piperidin-1-yl)-4,6-dimethylpyrimidine-5-carboxylic acid by utilizing methods analogous to those described in Example G. LCMS (Method 5-95 AB, ESI): t_{R} = 0.713 min, [M + H]⁺ = 911.8; ¹H NMR (400 MHz, MeOH-*d*₄) δ 8.49 (brs, 4H), 7.25 (d, *J*=8.4 Hz, 2H), 7.20 (d, *J*=8.4 Hz, 1H), 7.11 (d, *J*=8.4 Hz, 1H), 6.88 (s, 1H), 6.72 (s, 1H), 6.49 (s, 1H), 5.20-5.15 (m, 1H), 4.85-4.75 (m, 2H), 4.35-4.20 (m, 6H), 3.30-3.26 (m, 1H), 3.26-3.20 (m, 4H), 3.18-3.08 (m, 3H), 2.98 (s, 3H), 2.80-2.60 (m, 4H), 2.27 (s, 6H), 2.26-2.20 (m, 1H), 2.18-2.10 (m, 1H), 1.64-1.56 (m, 2H),1.36 (d, *J*=6.8 Hz, 3H), 1.32-1.05 (m, 3H), 0.89 (s, 9H).

### Example 116: Synthesis of Compound 316

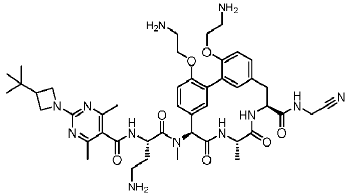

Compound 316 (formic acid salt) was prepared as a white solid from Compound **101-K** by utilizing methods analogous to those described in Example 315. LCMS (Method 5-95 AB, ESI): t_{R} = 0.532 min, [M + H]⁺ = 883.4; ¹H NMR (400 MHz, MeOH-*d*₄) δ 8.45 (brs, 2H), 7.26 (d, *J*=8.4 Hz, 2H), 7.19 (d, *J*=8.4 Hz, 1H), 7.11 (d, *J*=8.4 Hz, 1H), 6.89 (s, 1H), 6.77 (s, 1H), 6.43 (s, 1H), 5.17-5.10 (m, 1H), 4.80-5.75 (m, 2H), 4.30-4.19 (m, 4H), 4.21 (s, 2H), 4.07-4.04 (m, 2H), 3.90-3.88 (m, 2H), 3.25-3.20 (m, 4H), 3.19-3.07 (m, 4H), 2.97 (s, 3H), 2.62-2.60 (m, 1H), 2.31 (s, 6H), 2.27-2.22 (m, 1H), 2.16-2.10 (m, 1H), 1.35 (d, *J*=7.2 Hz, 3H), 0.94 (s, 9H).

### Example 117: Synthesis of Compound 317

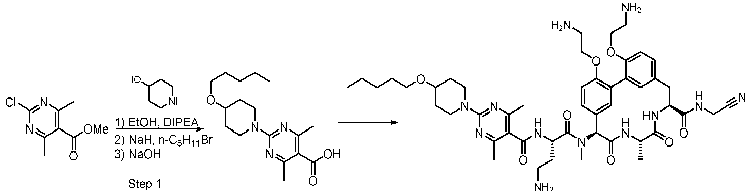

Step 1: Starting from methyl 2-chloro-4,6-dimethylpyrimidine-5-carboxylate, described in Example 53, typical S_{N}Ar (as described in Example 315), alkylation (as described in Example 38) and ester hydrolysis (as described in Example H) conditions were followed to give 4,6-dimethyl-2-(4-(pentyloxy)piperidin-1-yl)pyrimidine-5-carboxylic acid as a white solid.

Compound 317 (formic acid salt) was prepared as a white solid from Compound **101-K** and 4,6-dimethyl-2-(4-(pentyloxy)piperidin-1-yl)pyrimidine-5-carboxylic acid by utilizing methods analogous to those described in Example G. LCMS (Method 5-95 AB, ESI): t_{R} = 0.592 min, [M + H]⁺ = 941.4; ¹H NMR (400 MHz, MeOH-*d*₄) δ 8.36 (brs, 4H), 7.26-7.20 (m, 3H), 7.11 (d, *J*=8.0, 1H), 6.88 (brs, 1H), 6.73 (s, 1H), 6.47 (s, 1H), 5.19-5.16 (m, 1H), 4.85-4.78 (m, 2H), 4.31-4.21 (m, 8H), 3.55-3.50 (m, 3H), 3.25-3.09 (m, 8H), 2.97 (s, 3H), 2.26 (s, 6H), 2.24-2.11 (m, 3H), 1.88-1.85 (m, 2H), 1.58-1.56 (m, 2H), 1.43-1.34 (m,11H), 0.93 (s, 3H).

### Example 118: Synthesis of Compound 318

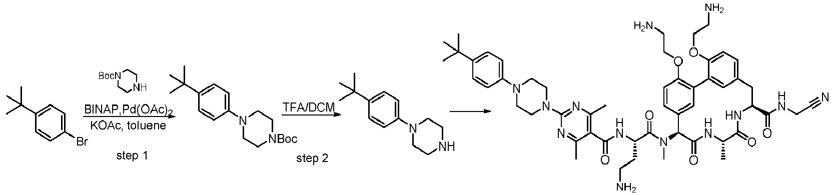

Step 1: A mixture of 1-bromo-4-(*tert*-butyl)benzene (550 mg, 2.6 mmol), *tert*-butyl piperazine-1-carboxylate (577 mg, 3.1 mmol), Pd(OAc)₂ (23 mg, 0.10 mmol), BINAP (1.6 g, 2.6 mmol) and Cs₂CO₃ (1.23 g, 3.9 mmol) in toluene (10 mL) was stirred at 90 °C for 16 h. The volatiles were removed and the residue was re-dissolved with EtOAc (50 mL), which was washed with brine (50 mL). The organic layer was dried over Na₂SO₄, concentrated and the residue was purified by silica gel chromatography, eluting with 5% EtOAc in petroleum ether, to give *tert*-butyl 4-(4-(*tert*-butyl)phenyl)piperazine-1-carboxylate (300 mg, 37% yield) as a yellow solid. LCMS (Method 5-95 AB, ESI): t_{R} = 0.872 min, [M + H]⁺ = 318.9.

Step 2: A solution of *tert*-butyl 4-(4-*tert*-butylphenyl)piperazine-1-carboxylate (300 mg, 0.94 mmol) in 20% TFA in DCM (15 mL) was stirred at 20 °C for 16 h. The volatiles were removed under reduced pressure and the residue was treated with saturated aqueous NaHCO₃ until pH>7, which was then extracted with EtOAc (20 mL × 3). The combined organic layers were washed with brine (50 mL), dried over Na₂SO₄ and concentrated to give 1-(4-*tert*-butylphenyl)piperazine (200 mg, 97% yield) as a yellow solid.

Compound 318 (formic acid salt) was prepared as a white solid from Compound **101-K** and 1-(4-(*tert*-butyl)phenyl)piperazine by utilizing methods analogous to those described in Example 315. LCMS (Method 5-95 AB, ESI): t_{R} = 0.724 min, [M + H]⁺ = 988.6; ¹H NMR (400 MHz, MeOH-*d*₄) δ 8.49 (br s, 2H), 7.31 (d, *J*=8.4 Hz, 2H), 7.28-7.18 (m, 3H), 7.10 (d, *J*=8.4 Hz, 1H), 6.96 (d, J=8.4 Hz, 2H), 6.88 (s, 1H), 6.71 (s, 1H), 6.48 (s, 1H), 5.20-5.16 (m, 1H), 4.81-4.77 (m, 2H), 4.30 (s, 2H), 4.22 (brs, 4H), 3.93 (brs, 4H), 3.28-3.05 (m, 8H), 2.98 (s, 3H), 2.29 (s, 6H), 2.20-2.12 (m, 1H), 2.09-2.02 (m, 1H), 1.36 (d, *J*=6.8 Hz, 3H), 1.30 (s, 9H).

### Example 119: Synthesis of Compound 319

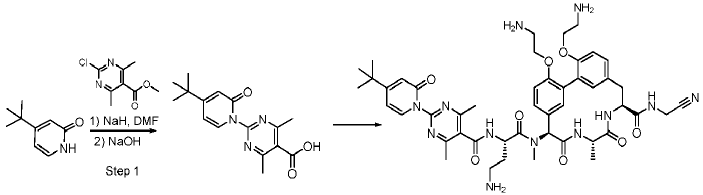

Step 1: Starting from 4-(*tert*-butyl)pyridin-2(1*H*)-one and methyl 2-chloro-4,6-dimethylpyrimidine-5-carboxylate (described in Example 53), typical alkylation (as described in Example 315) and ester hydrolysis (described in Example H) conditions were applied to give 2-(4-(*tert*-butyl)-2-oxopyridin-1(2*H*)-yl)-4,6-dimethylpyrimidine-5-carboxylic acid as a white solid. LCMS (Method 5-95 AB, ESI): t_{R} = 0.378 min, [M + H]⁺ = 302.1.

Compound 319 (formic acid salt) was prepared as a white solid from Compound **101-K** and 2-(4-(*tert*-butyl)-2-oxopyridin-1(2*H*)-yl)-4,6-dimethylpyrimidine-5-carboxylic acid by utilizing methods analogous to those described in Example G. LCMS (Method 5-95 AB, ESI): t_{R} = 0.644 min, [M + H]⁺ = 921.6; ¹H NMR (400 MHz, MeOH-*d*₄) δ 8.48 (brs, 3H), 7.68 (d, *J*=8.0 Hz, 1H), 7.29 (d, *J*=8.0 Hz, 1H), 7.26-7.17 (m, 2H), 7.10 (d, *J*=8.0 Hz, 1H), 6.92 (s, 1H), 6.78 (s, 1H), 6.63 (d, *J*=8.0 Hz, 1H), 6.57 (s, 1H), 6.45 (s, 1H), 5.26-5.23 (m, 1H), 4.81-4.74 (m, 2H), 4.30-4.19 (m, 6H), 3.22-3.10 (m, 8H), 3.01 (s, 3H), 2.55 (s, 6H), 2.35-2.25 (m, 1H), 2.22-2.12 (m, 1H), 1.36 (d, *J*=6.8 Hz, 3H), 1.33 (s, 9H).

### Example 120: Synthesis of Compound 320

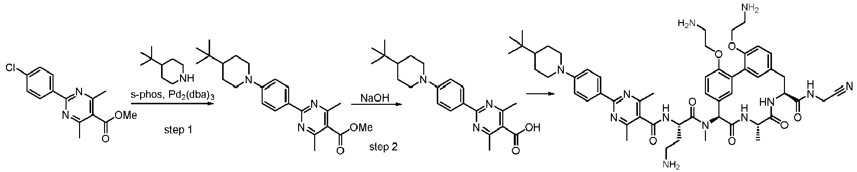

Step 1: A mixture of methyl 2-(4-chlorophenyl)-4,6-dimethylpyrimidine-5-carboxylate (120 mg, 0.43 mmol) and 4-(*tert*-butyl)piperidine (780 mg, 0.56 mmol), Pd₂(dba)₃ (20 mg, 0.02 mmol), 2-dicyclohexylphosphino-2',6'-dimethoxybiphenyl (18 mg, 0.04 mmol) and *t*-BuONa (62.5 mg, 0.65 mmol) in toluene (8 mL) was stirred for at 100 °C for 24 h under N₂. The volatiles were removed under reduced pressure and the residue was purified by preparatory-TLC (eluting with 20% EtOAc in petroleum ether) to give methyl 2-(4-(4-(*tert*-butyl)piperidin-1-yl)phenyl)-4,6-dimethylpyrimidine-5-carboxylate (80 mg, 48% yield) as a yellow solid.

Step 2: Typical ester hydrolysis conditions (NaOH, MeOH/H₂O) as described in Example H were applied to methyl 2-(4-(4-(*tert*-butyl)piperidin-1-yl)phenyl)-4,6-dimethylpyrimidine-5-carboxylate to give 2-(4-(4-(tert-butyl)piperidin-1-yl)phenyl)-4,6-dimethylpyrimidine-5-carboxylic acid as a white solid.

Compound 320 (formic acid salt) was prepared as a white solid from Compound **101-K** and 2-(4-(4-(*tert*-butyl)piperidin-1-yl)phenyl)-4,6-dimethylpyrimidine-5-carboxylic acid by utilizing methods analogous to those described in Example G. LCMS (Method 5-95 AB, ESI): t_{R} = 0.924 min, [M + H]⁺ = 987.6; ¹H NMR (400 MHz, MeOH-*d*₄) δ 8.50 (brs, 2H), 8.18-8.14 (m, 2H), 7.31-7.26 (m, 1H), 7.25-7.20 (m, 1H), 7.18-7.07 (m, 2H), 6.98 (d, *J*=8.4 Hz, 2H), 6.88 (brs, 1H), 6.66 (brs, 1H), 6.54 (s, 1H), 5.28-5.21 (m, 1H), 4.83-4.75 (m, 2H), 4.35-4.19 (m, 4H), 4.23(s, 2H), 4.03-3.93 (m, 2H), 3.29-3.07 (m, 8H), 3.01 (s, 3H), 2.80-2.69 (m, 2H), 2.47 (s, 6H), 2.32-2.21 (m, 1H), 2.19-2.10 (m, 1H), 1.89-1.81 (m,, 2H), 1.50-1.40 (m, 2H), 1.35 (d, *J*=6.8 Hz, 3H), 1.30-1.25 (m, 1H), 0.93 (s, 9H).

### Example 121: Synthesis of Compound 321

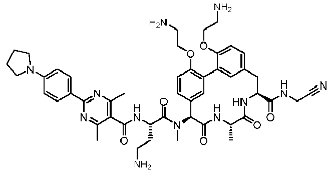

Compound 321 (formic acid salt) was prepared as a white solid from Compound **101-K** by utilizing methods analogous to those described in Example 120. LCMS (Method 5-95 AB, ESI): t_{R} = 0.690 min, [M + H]⁺ = 917.5; ¹H NMR (400 MHz, MeOH-*d*₄) δ 8.51 (brs, 3H), 8.20-8.10 (m, 2H), 7.30-7.15 (m, 3H), 7.09 (d, *J*=8.4 Hz, 1H), 6.88 (s, 1H), 6.69 (s, 1H), 6.60 (d, J=8.4 Hz, 2H), 6.52 (s, 1H), 5.30-5.20 (m, 1H), 4.85-4.75 (m, 2H), 4.30-4.15 (m, 6H), 3.45-3.35 (m, 5H), 3.26-3.05 (m, 7H), 3.01 (s, 3H), 2.46 (s, 6H), 2.30-2.25 (m, 1H), 2.20-2.05 (m, 5H), 1.35 (d, J=6.4 Hz, 3H).

### Example 122: Synthesis of Compound 322

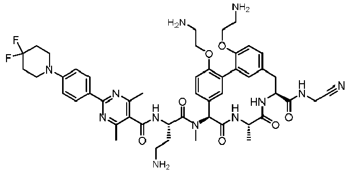

Compound 322 (formic acid salt) was prepared as a white solid from Compound 101-K by utilizing methods analogous to those described in Example 120. LCMS (Method 5-95 AB, ESI): t_{R} = 0.693 min, [M + H]⁺ = 967.6; ¹H NMR (400 MHz, MeOH-*d*₄) δ 8.49 (brs, 1H), 8.24 (d, *J*=8.4 Hz, 2H), 7.31-7.25 (m, 1H), 7.24-7.13 (m, 2H), 7.11-7.02 (m, 3H), 6.88 (s, 1H), 6.70 (brs, 1H), 6.49 (s, 1H), 5.26-5.21 (m, 1H), 4.80-4.77 (m, 2H), 4.32-4.16 (m, 4H), 4.22 (s, 2H), 3.56-3.51 (m, 4H), 3.30-3.04 (m, 8H), 3.00 (s, 3H), 2.48 (s, 6H), 2.35-2.02 (m, 6H), 1.35 (d, *J*=6.4 Hz, 3H).

### Example 123: Synthesis of Compound 323

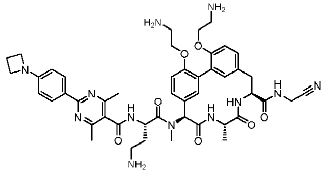

Compound 323 (formic acid salt) was prepared as a white solid from Compound **101-K** by utilizing methods analogous to those described in Example 120. LCMS (Method 5-95 AB, ESI): t_{R} = 0.651 min, [M + H]⁺ = 903.3; ¹H NMR (400 MHz, MeOH-*d*₄) δ 8.09 (brs, 2H), 7.34-7.06 (m, 5H), 6.87 (s, 1H), 6.65-6.38 (m, 5H), 5.30-5.24 (m, 1H), 4.84-4.81 (m, 2H), 4.43-4.23 (m, 6H), 4.04-3.94 (m, 4H), 3.33-3.10 (m, 5H), 3.02 (brs, 6H), 2.43 (s, 6H), 2.40-2.35 (m, 2H), 2.32-2.11 (m, 2H), 1.35 (d, *J*=6.8 Hz, 3H).

### Example 124: Synthesis of Compound 324

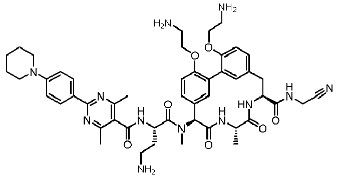

Compound 324 (formic acid salt) was prepared as a white solid from Compound **101-K** by utilizing methods analogous to those described in Example 120. LCMS (Method 5-95 AB, ESI): t_{R} = 0.950 min, [M + H]⁺ = 931.5; ¹H NMR (400 MHz, MeOH-*d*₄) δ 8.51 (brs, 2H), 8.10 (d, *J*=8.4 Hz, 2H), 7.26 (brs, 2H), 7.09 (brs, 2H), 6.94 (d, *J*=8.4 Hz, 2H), 6.86 (s, 1H), 6.61 (brs, 1H), 6.57 (brs, 1H), 5.31-5.27 (m, 1H) 4.85-4.78 (m, 2H), 4.35 (s, 2H), 4.25 (brs, 4H), 3.37-3.34 (m,4H), 3.28-3.10 (m, 6H), 3.05-2.93 (m, 2H), 3.02 (s, 3H), 2.42 (s, 6H), 2.34-2.23 (m, 1H), 2.20-2.10 (m, 1H), 1.76-1.66 (m, 6H), 1.35 (d, *J*=6.8 Hz, 3H).

### Example 125: Synthesis of Compound 325

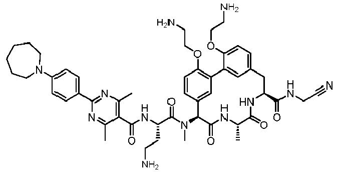

Compound 325 (formic acid salt) was prepared as a white solid from Compound **101-K** by utilizing methods analogous to those described in Example 120. LCMS (Method 5-95 AB, ESI): t_{R} = 0.719 min, [M + H]⁺ = 945.5; ¹H NMR (400 MHz, MeOH-*d*₄) δ 8.50 (brs, 1H), 8.02-7.98 (m, 2H), 7.28-7.24 (m, 2H), 7.13- 6.99 (m, 2H), 6.83 (brs, 1H), 6.75-6.60 (m, 3H), 6.50 (brs, 1H), 5.35-5.25 (m, 1H), 4.78-4.75 (m, 2H), 4.27 (s, 2H), 4.25- 4.18 (m, 4H), 3.62-3.58 (m, 4H), 3.31-3.26 (m, 4H), 3.25-3.21 (m, 2H), 3.15-3.11 (m, 2H), 3.02 (s, 3H), 2.37 (s, 6H), 2.30-2.25 (m, 1H), 2.21-2.09 (m, 1H), 1.86 (brs, 4H), 1.61 (brs, 4H), 1.34 (d, *J*=6.8 Hz, 3H).

### Example 126: Synthesis of Compound 326

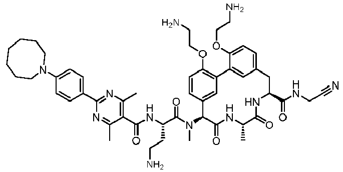

Compound 326 (formic acid salt) was prepared as a white solid from Compound **101-K** by utilizing methods analogous to those described in Example 120. LCMS (Method 5-95 AB, ESI): t_{R} = 0.615 min, [M + H]⁺ = 959.6; ¹H NMR (400 MHz, MeOH-*d*₄) δ 8.23 (brs, 2H), 8.22-8.18 (m, 2H), 7.39-6.86 (m, 6H), 6.79-6.59 (m, 2H), 6.49 (s, 1H), 5.29-5.11 (m, 1H), 4.75-4.67 (m, 2H), 4.39-4.04 (m, 6H), 3.64-3.55 (m, 4H), 3.30-2.83 (m,11H), 2.50 (s, 6H), 2.35-2.06 (m, 2H), 1.81 (brs, 4H), 1.55 (brs, 4H), 1.45-1.39 (m, 2H), 1.36 (t, *J*=7.2 Hz, 3H).

### Example 127: Synthesis of Compound 327

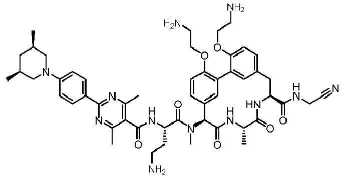

Compound 327 (formic acid salt) was prepared as a white solid from Compound **101-K** by utilizing methods analogous to those described in Example 120. LCMS (Method 5-95 AB, ESI): t_{R} = 0.681 min, [M + H]⁺ = 959.7; ¹H NMR (400 MHz, MeOH-*d*₄) δ 8.41 (brs, 2H), 8.25 (d, *J*=8.0 Hz, 2H), 7.30-7.10 (m, 4H), 7.00 (d, *J*=8.4 Hz, 2H), 6.91 (s, 1H), 6.79 (s, 1H), 6.44 (s, 1H), 5.24-5.20 (m, 1H), 4.85-4.75 (m, 2H), 4.36-4.18 (m, 8H), 3.90-3.82 (m, 2H), 3.29-3.22 (m, 6H), 3.16-3.11 (m, 2H), 3.01 (s, 3H), 2.52 (s, 6H), 2.40-2.25 (m, 3H), 2.20-2.10 (m, 1H), 1.90-1.83 (m, 1H), 1.80-1.72 (m, 2H), 1.36 (d, *J*=6.4 Hz, 3H), 0.98 (d, *J*=6.4 Hz, 6H), 0.87-0.74 (m, 1H).

### Example 128: Synthesis of Compound 328

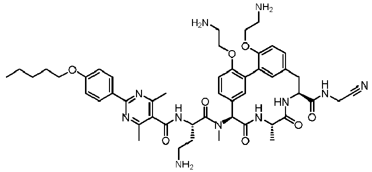

Compound 328 (formic acid salt) was prepared as a white solid from Compound **101-K** by utilizing methods analogous to those described in Example 53. LCMS (Method 5-95 AB, ESI): t_{R} = 0.667 min, [M + H]⁺ = 934.6; ¹H NMR (400 MHz, MeOH-*d*₄) δ 8.15 (d, *J*=8.0 Hz, 2H), 7.26 (brs, 2H), 7.15-7.05 (m, 2H), 6.94 (d, *J*=8.4 Hz, 2H), 6.85 (s, 1H), 6.62 (s, 1H), 6.51 (s, 1H), 5.35-5.25 (m, 1H), 4.80-4.70 (m, 2H), 4.27 (s, 2H), 4.25-4.15 (m, 4H), 4.06 (t, *J*=6.8 Hz, 2H), 3.40-3.35 (m, 1H), 3.30-2.85 (m, 10H), 2.42 (s, 6H), 2.35-2.25 (m, 1H), 2.25-2.15 (m, 1H), 1.85-1.75 (m, 2H), 1.55-1.40 (m, 4H), 1.35 (d, *J*=6.4 Hz, 3H), 0.98 (t, *J*=7.2 Hz, 3H).

### Example 129: Synthesis of Compound 329

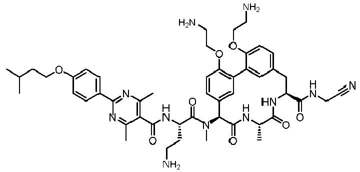

Compound 329 (formic acid salt) was prepared as a white solid from Compound **101-K** by utilizing methods analogous to those described in Example 53. LCMS (Method 5-95 AB, ESI): t_{R} = 0.740 min, [M + H]⁺ = 934.5; ¹H NMR (400 MHz, MeOH-*d*₄) δ 8.47 (brs, 1H), 8.25-8.18 (m, 2H), 7.28-7.22 (m, 2H), 7.10 (brs, 2H), 6.95 (d, *J*=8.4 Hz, 2H), 6.86 (brs, 1H), 6.57 (brs, 1H), 5.29-5.25 (m, 1H), 4.85-4.75 (m, 2H), 4.38-4.22 (m, 6H), 4.10 (t, *J*=6.8 Hz, 2H), 3.27-3.05 (m, 8H), 3.01 (s, 3H), 2.48 (s, 6H), 2.34-2.24 (m, 1H), 2.21-2.10 (m, 1H), 1.95-1.82 (m, 1H), 1.75-1.63 (m, 2H), 1.35 (d, *J*=6.8 Hz, 3H), 1.00 (d, *J*=6.4 Hz, 6H).

### Example 130: Synthesis of Compound 330

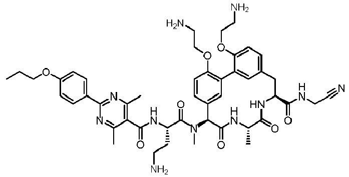

Compound 330 (formic acid salt) was prepared as a white solid from Compound **101-K** by utilizing methods analogous to those described in Example 53. LCMS (Method 5-95 AB, ESI): t_{R} = 0.698 min, [M + H]⁺ = 906.4; ¹H NMR (400 MHz, MeOH-*d*₄) δ 8.49 (brs, 2H), 8.18 (d, *J*=8.0 Hz, 2H), 7.30-7.22 (m, 2H), 7.09 (brs, 2H), 6.95 (d, J=8.4 Hz, 2H), 6.86 (s, 1H), 6.60 (brs, 1H), 6.56 (brs, 1H), 5.35-5.25 (m, 1H), 4.85-4.70 (m, 2H), 4.37 (s, 2H), 4.30-4.15 (m, 4H), 4.03 (t, *J*=6.4 Hz, 2H), 3.25-2.90 (m, 8H), 3.02 (s, 3H), 2.44 (s, 6H), 2.35-2.20 (m, 1H), 2.20-2.10 (m, 1H), 1.90-1.80 (m, 2H), 1.35 (d, *J*=6.8 Hz, 3H), 1.09 (t, J=7.6 Hz, 3H).

### Example 131: Synthesis of Compound 331

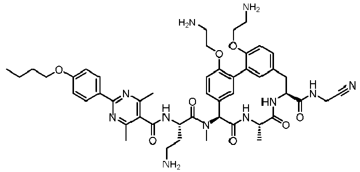

Compound 331 (formic acid salt) was prepared as a white solid from Compound 101-K by utilizing methods analogous to those described in Example 53. LCMS (Method 5-95 AB, ESI): t_{R} = 0.724 min, [M + H]⁺ = 920.4; ¹H NMR (400 MHz, MeOH-*d*₄) δ 8.50 (brs, 3H), 8.13 (d, J=7.6 Hz, 2H), 7.26 (brs, 2H), 7.15-7.00 (m, 2H), 6.93 (d, J=8.4 Hz, 2H), 6.84 (s, 1H), 6.65 (s, 1H), 6.48 (s, 1H), 5.35-5.25 (m, 1H), 4.85-4.70 (m, 2H), 4.28 (s, 3H), 4.30-4.15 (m, 4H), 4.07 (t, J=6.4 Hz, 2H), 3.40-2.90 (m, 8H), 3.02 (s, 3H), 2.41 (s, 6H), 2.35-2.25 (m, 1H), 2.20-2.10 (m, 1H), 1.85-1.75 (m, 2H), 1.60-1.50 (m, 2H), 1.35 (d, J=6.8 Hz, 3H), 1.03 (t, *J*=6.8 Hz, 3H).

### Example 132: Synthesis of Compound 332

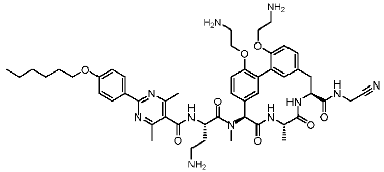

Compound 332 (formic acid salt) was prepared as a white solid from Compound **101-K** by utilizing methods analogous to those described in Example 53. LCMS (Method 5-95 AB, ESI): t_{R} = 0.780 min, [M + H]⁺ = 948.7; ¹H NMR (400 MHz, MeOH-*d*₄) δ 8.50 (brs, 3H), 8.19 (d, *J*=8.0 Hz, 2H), 7.26 (brs, 2H), 7.09 (brs, 2H), 6.95 (d, *J*=8.8 Hz, 2H), 6.86 (s, 1H), 6.60 (brs, 1H), 6.56 (brs, 1H), 5.32-5.25 (m, 1H), 4.81-4.71 (m, 2H), 4.37 (s, 2H), 4.30-4.21 (m, 4H), 4.06 (t, *J*=6.4 Hz, 2H), 3.25-2.95 (m, 8H), 3.02 (s, 3H), 2.44 (s, 6H), 2.30-2.15 (m, 2H), 1.87-1.79 (m, 2H), 1.57-1.48 (m, 2H), 1.47-1.24 (m, 7H), 0.95 (t, *J*=6.4 Hz, 3H).

### Example 133: Synthesis of Compound 333

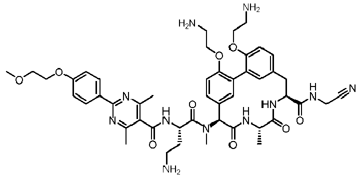

Compound 333 (formic acid salt) was prepared as a white solid from Compound **101-K** by utilizing methods analogous to those described in Example 53. LCMS (Method 5-95 AB, ESI): t_{R} = 0.502 min, [M + H]⁺ = 922.4; ¹H NMR (400 MHz, MeOH-*d*₄) δ 8.24 (d, J=8.0 Hz, 2H), 7.29-7.20 (m, 2H), 7.16-7.05 (m, 2H), 6.99 (d, J=8.0 Hz, 2H), 6.87 (s, 1H), 6.62 (s, 1H), 6.55 (s, 1H), 5.31-5.24 (m, 1H), 4.85-4.76 (m, 2H), 4.40-4.17 (m, 8H), 3.79 (t, J=4.0 Hz, 2H), 3.46 (s, 3H), 3.28-2.95 (m, 8H), 3.01 (s, 3H), 2.47 (s, 6H), 2.34-2.25 (m, 1H), 2.19-2.10 (m, 1H), 1.35 (t, *J*=6.8 Hz, 2H).

### Example 134: Synthesis of Compound 334

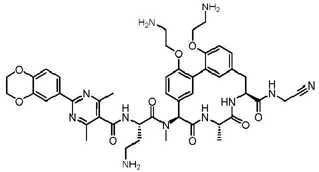

Compound 334 (formic acid salt) was prepared as a white solid from Compound **101-K** by utilizing methods analogous to those described in Example 53. LCMS (Method 5-95 AB, ESI): t_{R} = 0.640 min, [M + H]⁺ = 906.6; ¹H NMR (400 MHz, MeOH-*d*₄) δ 8.49 (brs, 2H), 7.90-7.75 (m, 2H), 7.40-7.02 (m, 4H), 6.95-6.80 (m, 2H), 6.67 (brs, 1H), 6.49 (brs, 1H), 5.30-5.20 (m, 1H), 4.79-4.75 (m, 1H), 4.40-4.10 (m, 11H), 3.25-3.10 (m, 8H), 3.00 (s, 3H), 2.47 (s, 6H), 2.35-2.15 (m, 1H), 2.15-2.05 (m, 1H), 1.35 (d, J=6.8 Hz, 3H).

### Example 135: Synthesis of Compound 335

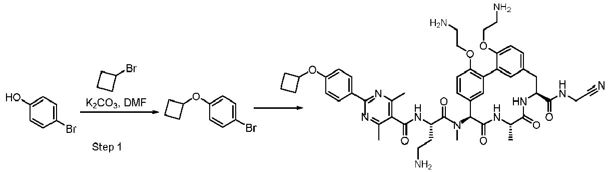

Step 1: A mixture of 4-bromophenol (500 mg, 2.9 mmol), bromocyclobutane (585 mg, 4.4 mmol) and K₂CO₃ (800 mg, 5.8 mmol) in DMF (2 mL) was stirred at 80 °C for 16 h. The volatiles were removed under reduced pressure and the residue was taken up in EtOAc (50 mL), which was washed with brine (50 mL). The organic layer was dried over MgSO₄, concentrated and the residue was purified by preparatory-TLC (eluting with 5% EtOAc in petroleum ether, R_{f}=0.3) to afford 1-bromo-4-cyclobutoxybenzene (400 mg, 61% yield) as a colorless oil. ¹H NMR (400 MHz, MeOH-*d*₄) δ 7.35 (d, *J*=8.0 Hz, 2H), 6.70 (d, *J*=8.0 Hz, 2H), 4.65-4.55 (m, 1H), 2.47-2.43 (m, 2H), 2.19-2.13 (m, 2H), 1.85-1.75 (m, 1H), 1.70-1.60 (m, 1H).

Compound 335 (formic acid salt) was prepared as a white solid from Compound **101-K** and 1-bromo-4-cyclobutoxybenzene by utilizing methods analogous to those described in Example 53. LCMS (Method 5-95 AB, ESI): t_{R} = 0.687 min, [M + H]⁺ = 918.7; ¹H NMR (400 MHz, MeOH-*d*₄) δ 8.49 (brs, 2H), 8.25 (brs, 2H), 7.30-7.20 (m, 2H), 7.17-7.06 (m., 2H), 6.88 (brs, 3H), 6.67 (brs, 1H), 6.52 (s, 1H), 5.27-5.23 (m, 1H), 4.84-4.73 (m, 3H), 4.35-4.19 (m, 6H), 3.25-3.00 (m, 11H), 2.58- 2.45 (m, 2H), 2.49 (s, 6H), 2.36-2.09 (m, 5H), 1.96-1.86 (m, 1H), 1.82-1.73 (m, 1H), 1.35 (d, *J*=6.8 Hz, 3H).

### Example 136: Synthesis of Compound 336

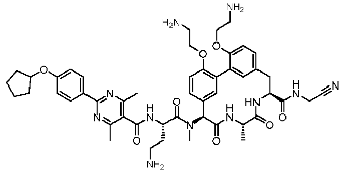

Compound 336 (formic acid salt) was prepared as a white solid from Compound **101-K** by utilizing methods analogous to those described in Example 135. LCMS (Method 5-95 AB, ESI): t_{R} = 0.714 min, [M + H]⁺ = 932.3; ¹H NMR (400 MHz, MeOH-*d*₄) δ 8.50 (brs, 3H), 8.14 (d, *J*=8.0 Hz, 2H), 7.26 (brs, 2H), 7.15-7.00 (m, 2H), 6.90 (d, *J*=8.4 Hz, 2H), 6.84 (s, 1H), 6.63 (s, 1H), 6.51 (s, 1H), 5.35-5.25 (m, 1H), 4.85-4.70 (m, 3H), 4.39 (s, 2H), 4.25-4.15 (m, 4H), 3.40-3.10 (m, 5H), 3.02 (s, 3H), 3.00-2.85 (m, 2H), 2.42 (s, 6H), 2.35-2.20 (m, 1H), 2.20-2.10 (m, 1H), 2.10-1.95 (m, 2H), 1.95-1.80 (m, 4H), 1.80-1.65 (m, 2H), 1.35 (d, *J*=6.8 Hz, 3H).

### Example 137: Synthesis of Compound 337

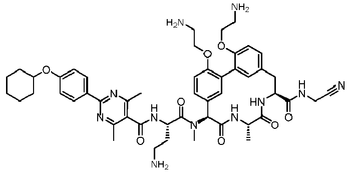

Compound 337 (formic acid salt) was prepared as a white solid from Compound **101-K** by utilizing methods analogous to those described in Example 135. LCMS (Method 5-95 AB, ESI): t_{R} = 0.730 min, [M + H]⁺ = 946.6; ¹H NMR (400 MHz, MeOH-*d*₄) δ 8.50 (brs, 3H), 8.15 (d, *J*=8.0 Hz, 2H), 7.26 (brs, 2H), 7.10-7.00 (m, 2H), 6.93 (d, *J*=8.4 Hz, 2H), 6.84 (s, 1H), 6.62 (s, 1H), 6.52 (s, 1H), 5.35-5.25 (m, 1H), 4.85-4.70 (m, 2H), 4.50-4.30 (m, 3H), 4.25 (brs, 4H), 3.40-3.10 (m, 5H), 3.02 (s, 3H), 3.00-2.85 (m, 2H), 2.42 (s, 6H), 2.35-2.20 (m, 1H), 2.20-2.10 (m, 1H), 2.10-2.00 (m, 2H), 1.90-1.80 (m, 2H), 1.70-1.40 (m, 6H), 1.35 (d, *J*=6.8 Hz, 3H).

### Example 138: Synthesis of Compound 338

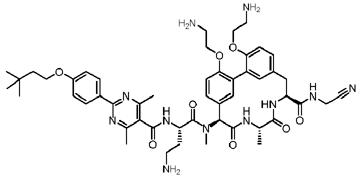

Compound 338 (formic acid salt) was prepared as a white solid from Compound **101-K** by utilizing methods analogous to those described in Example 135. LCMS (Method 5-95 AB, ESI): t_{R} = 0.767 min, [M + H]⁺ = 948.5; ¹H NMR (400 MHz, MeOH-*d*₄) δ 8.49 (brs, 2H), 8.29 (d, J=8.0 Hz, 2H), 7.29 (d, J=8.0 Hz, 1H), 7.24-7.13 (m, 2H), 7.09 (d,J=8.0 Hz, 1H), 6.99 (d, J=8.0 Hz, 2H), 6.89 (s, 1H), 6.70 (s, 1H), 6.50 (s, 1H), 5.27-5.23 (m, 1H), 4.81-4.75 (m, 2H), 4.29 (s, 2H), 4.25-4.20 (m, 4H), 4.14 (t, J=7.2 Hz, 2H), 3.24-3.11 (m, 8H), 3.01 (s, 3H), 2.50 (s, 6H), 2.34-2.22 (m,1H), 2.21-2.09 (m,1H), 1.77 (t, *J*=7.2 Hz, 2H), 1.35(d, *J*=6.4 Hz, 3H), 1.04 (s, 9H).

### Example 139: Synthesis of Compound 339

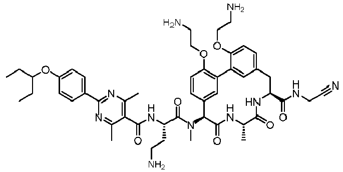

Compound 339 (formic acid salt) was prepared as a white solid from Compound **101-K** by utilizing methods analogous to those described in Example 135. LCMS (Method 5-95 AB, ESI): t_{R} = 0.612 min, [M + H]⁺ = 934.4; ¹H NMR (400 MHz, MeOH-*d*₄) δ 8.51 (brs, 3H), 8.24 (d, *J*=8.0 Hz, 1H), 8.14 (d, *J*=8.0 Hz, 1H), 7.28-7.24 (m, 2H), 7.11-6.88 (m, 6H), 6.67 (s, 0.5H), 6.61 (0.5H), 5.34-5.29 (m, 1H), 4.80-4.77 (m, 2H), 4.37-4.22 (m, 7H), 3.36-3.00 (m, 11H), 2.50 (s, 3H), 2.43 (s, 3H), 2.34-2.18 (m, 2H), 1.77-1.72 (m, 4H) 1.45-1.35 (m, 3H), 1.04-1.00 (m, 6H).

### Example 140: Synthesis of Compound 340

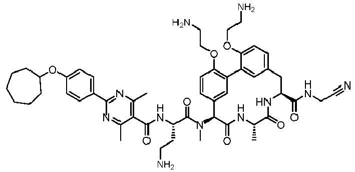

Compound 340 (formic acid salt) was prepared as a white solid from Compound 101-K by utilizing methods analogous to those described in Example 135. LCMS (Method 5-95 AB, ESI): t_{R} = 0.618 min, [M + H]⁺ = 960.3; ¹H NMR (400 MHz, MeOH-*d*₄) δ 8.52 (brs, 2H), 8.11 (d, J=8.0 Hz, 2H), 7.26 (brs, 2H), 7.07 (d, J=8.0 Hz, 1H), 7.02-6.96 (m, 1H), 6.86 (d, J=8.4 Hz, 2H), 6.82 (s,1H), 6.67 (s, 1H), 6.44 (s, 1H), 5.33-5.30 (m, 1H), 4.79-4.74 (m, 2H), 4.63-4.56 (m, 1H), 4.37 (s, 2H), 4.26-4.23 (m, 4H), 3.27-3.00 (m, 8H), 3.02 (s, 3H), 2.38 (s, 6H), 2.32-2.26 (m, 1H), 2.11-2.03 (m, 3H), 1.88-1.77 (m, 5H), 1.68-1.65 (m, 5H), 1.57-1.53 (m, 2H), 1.34 (d, *J*=6.4 Hz, 3H).

### Example 141: Synthesis of Compound 341

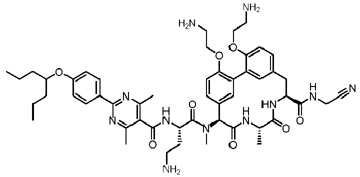

Compound 341 (formic acid salt) was prepared as a white solid from Compound **101-K** by utilizing methods analogous to those described in Example 135. LCMS (Method 5-95 AB, ESI): t_{R} = 0.789 min, [M + H]⁺ = 962.6; ¹H NMR (400 MHz, MeOH-*d*₄) δ 8.51 (brs, 2H), 8.22 (d, *J*=8.0 Hz, 1H), 7.30-7.20 (m, 2H), 7.13-7.04 (m, 2H), 6.94 (d, *J*=8.4 Hz, 2H), 6.87 (s, 1H), 6.61 (brs, 1H), 6.56 (brs, 1H), 5.30-5.24 (m, 1H), 4.85-4.73 (m, 2H), 4.49-4.44 (m, 1H), 4.31 (s, 2H), 4.26-4.21 (m, 4H), 3.31-2.95 (m, 8H), 3.01 (s, 3H), 2.46 (s, 6H), 2.31-2.26 (m, 1H), 2.15 -2.05 (m, 1H), 1.77-1.64 (m, 4H), 1.56-1.40 (m, 4H), 1.35 (d, *J*=6.4 Hz, 3H), 0.96 (t, *J*=7.6 Hz, 6H).

### Example 142: Synthesis of Compound 342

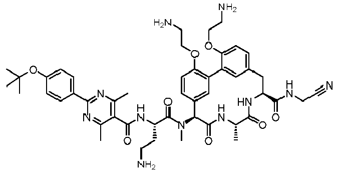

Compound 342 (formic acid salt) was prepared as a white solid from Compound **101-K** by utilizing methods analogous to those described in Example 50. LCMS (Method 5-95 AB, ESI): t_{R} = 0.707 min, [M + H]⁺ = 920.5; ¹H NMR (400 MHz, MeOH-*d*₄) δ 8.17 (d, *J*=8.0 Hz, 2H), 7.25 (brs, 2H), 7.11-6.99 (m, 4H), 6.84 (s, 1H), 6.64 (s, 1H), 6.52 (s, 1H), 5.36-5.23 (m, 1H), 4.84-4.74 (m, 2H), 4.36 (s, 2H), 4.28-4.15 (m, 4H), 3.26-3.10 (m, 6H), 3.10-2.89 (m, 2H), 3.02 (s, 3H), 2.44 (s, 6H), 2.34-2.22 (m, 1H), 2.18-2.09 (m, 1H), 1.43 (s, 9H), 1.35 (d, *J*=7.2Hz, 3H).

### Example 143: Synthesis of Compound 343

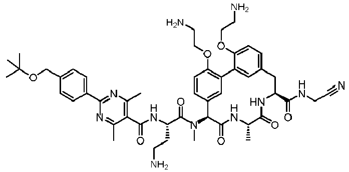

Compound 343 (formic acid salt) was prepared as a white solid from Compound **101-K** by utilizing methods analogous to those described in Example 50. LCMS (Method 5-95 AB, ESI): t_{R} = 0.706 min, [M + H]⁺ = 934.4; ¹H NMR (400 MHz, MeOH-*d*₄) δ 8.52 (brs, 3H), 8.35-8.29 (m, 3H), 7.49-7.43 (m, 4H), 7.30-7.27 (m, 1H), 7.11 (brs, 1H), 6.91 (s, 1H), 6.54 (s, 1H), 4.82-4.80 (m, 2H), 4.58 (brs, 4H), 4.24 (brs, 5H), 3.21-3.13 (m, 8H), 3.04 (s, 3H), 2.54 (s, 6H), 2.30-2.10 (m, 2H), 1.37 (d, J=6.8 Hz, 3H), 1.35 (s, 9H).

### Example 144: Synthesis of Compound 344

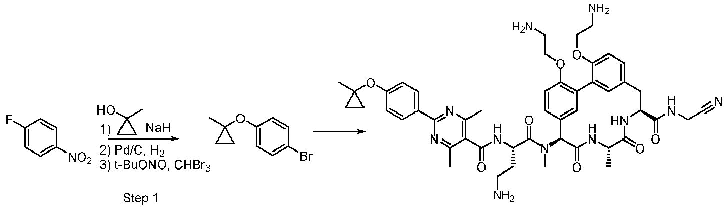

Step 1: Starting from 1-fluoro-4-nitrobenzene, typical alkylation (using NaH as described in Example 38), hydrogenation (using Pd/C and H₂ as described in Example E) and Sandmyer (as described in Example J) conditions were followed to give 1-bromo-4-(1-methylcyclopropoxy)benzene as a yellow solid.

Compound 344 (formic acid salt) was prepared as a white solid from Compound **101-K** and 1-bromo-4-(1-methylcyclopropoxy)benzene by utilizing methods analogous to those described in Example 53. LCMS (Method 5-95 AB, ESI): t_{R} = 0.709 min, [M + H]⁺ = 918.5; ¹H NMR (400 MHz, MeOH-*d*₄) δ 8.46 (brs, 2H), 8.27 (d, J=7.6 Hz, 2H), 7.31-7.21 (m, 2H), 7.20-7.12 (m, 1H), 7.11-7.04 (m, 3H), 6.88 (s, 1H), 6.68 (s, 1H), 6.52 (s, 1H), 5.30-6.22 (m, 1H), 4.81-4.74 (m, 2H), 4.37-4.17 (m, 6H), 3.29-3.20 (m, 5H), 3.17-3.08 (m, 3H), 3.02 (s, 3H), 2.49(s, 6H), 2.34-2.24 (m, 1H), 2.21-2.11 (m, 1H), 1.59 (s, 3H), 1.36 (d, J=6.8 Hz, 3 H), 1.02-0.95 (m, 2H), 0.84-0.76 (m, 2H).

### Example 145: Synthesis of Compound 345

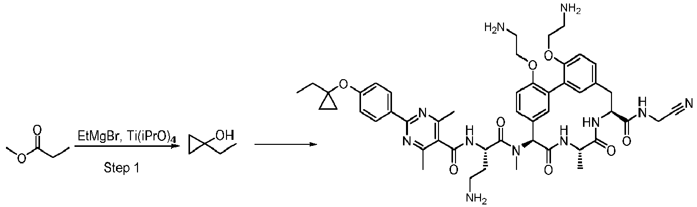

Step 1: To a stirred solution of methyl propionate (5.0 g, 56.8 mmol) and Ti(*i*-PrO)₄ (1.6 g, 5.7 mmol) in Et₂O (80 mL) was added EtMgBr (3N in Et₂O, 41.6 mL) over a period of 1 h, and the mixture was stirred for another 10 min. The mixture was then poured into cold 10% aqueous H₂SO₄ (100 mL) while maintaining the temperature below 5 °C. The resulting mixture was extracted with Et₂O (3 × 100 mL). The combined organic layers were washed with brine (50 mL), dried over Na₂SO₄, and concentrated to give 1-ethylcyclopropan-1-ol (4.5 g, 92% yield) as a yellow oil. ¹H NMR (400 MHz, CDCl₃) δ 1.56 (q, *J*=7.2 Hz, 2H), 1.00 (t, *J*=7.2 Hz, 3H), 0.70 (t, *J*=5.6 Hz, 2H), 0.42 (t, *J*=5.6 Hz 2H).

Compound 345 (formic acid salt) was prepared as a white solid from Compound **101-K** and 1-ethylcyclopropan-1-ol by utilizing methods analogous to those described in Example 144. LCMS (Method 5-95 AB, ESI): t_{R} = 0.602 min, [M + H]⁺ = 932.4; ¹H NMR (400 MHz, MeOH-*d*₄) δ 8.45 (brs, 2H), 8.23 (d, *J*= 8.0 Hz, 2H), 7.28 (brs, 2H), 7.16-7.03 (m, 4H), 6.89 (s, 1H), 6.62 (brs, 1H), 6.58 (brs, 1H), 5.30-5.28 (m, 1H), 4.84-4.79 (m, 2H), 4.40 (s, 2H), 4.35-4.23 (m, 4H), 3.32-3.28 (m, 4H), 3.04 (brs, 7H), 2.49 (s, 6H), 2.34-2.25 (m, 1H), 2.24-2.13 (m, 1H), 1.87 (q, *J*=6.8 Hz, 2H), 1.37 (d, *J*=6.4 Hz, 3H), 1.07 (t, *J*=6.8 Hz, 3H), 1.01-0.95 (m, 2H), 0.86-0.81 (m, 2H).

### Example 146: Synthesis of Compound 346

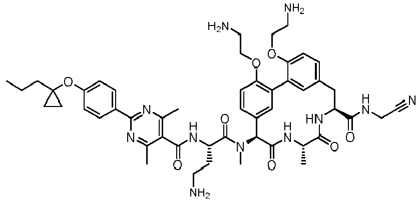

Compound 346 (formic acid salt) was prepared as a white solid from Compound **101-K** by utilizing methods analogous to those described in Example 145. LCMS (Method 5-95 AB, ESI): t_{R} = 0.731 min, [M + H]⁺ = 946.5; ¹H NMR (400 MHz, MeOH-*d*₄) δ 8.52 (brs, 2H), 8.17 (d, *J*=7.6 Hz, 2H), 7.25 (brs, 2H), 7.12-6.97 (m, 4H), 6.83 (s, 1H), 6.64 (s, 1H), 6.50 (s, 1H), 5.29-5.26 (m, 1H), 4.84-4.75 (m, 2H), 4.37-4.17 (m, 6H), 3.25-2.95 (m, 8H), 3.02 (s, 3H), 2.42 (s, 6H), 2.30-2.23 (m, 1H), 2.14-2.11 (m, 1H), 1.86-1.74 (m, 2H), 1.60-1.50 (m, 2H), 1.35 (d, *J*=6.8 Hz, 3H), 1.03-0.88 (m, 5H), 0.84-0.80 (m, 2H).

### Example 147: Synthesis of Compound 347

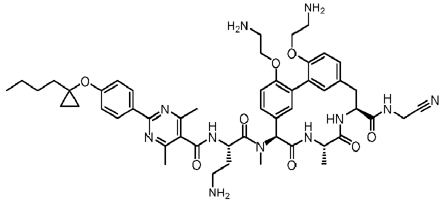

Compound 347 (formic acid salt) was prepared as a white solid from Compound **101-K** by utilizing methods analogous to those described in Example 145. LCMS (Method 5-95 AB, ESI): t_{R} = 0.745 min, [M + H]⁺ = 960.5; ¹H NMR (400 MHz, MeOH-*d*₄) δ 8.51 (brs, 1H), 8.19 (d, *J*=7.6 Hz, 2H), 7.23 (d, *J*=8.0 Hz, 2H), 7.11-6.97 (m, 4H), 6.85 (s, 1H), 6.58 (brs, 2H), 5.30-5.26 (m, 1H), 4.83-4.72 (m, 2H), 4.24 (s, 2H), 4.20-4.10 (m, 4H), 3.30-3.08 (m, 8H), 3.01 (s, 3H), 2.43 (s, 6H), 2.32-2.22 (m, 1H), 2.20-2.10 (m, 1H), 1.89-1.76 (m, 2H), 1.51-1.45 (m, 2H), 1.43-1.30 (m, 5H), 1.00-0.85 (m, 5H), 0.85-0.78 (m, 2H).

### Example 148: Synthesis of Compound 348

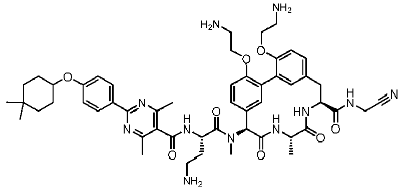

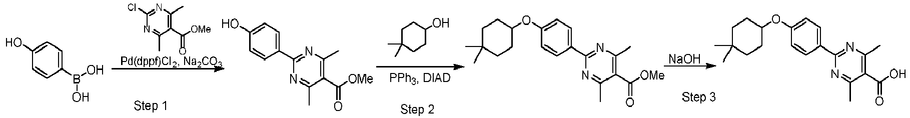

Step 1: Typical Suzuki conditions, as described in Example H, were applied to (4-hydroxyphenyl)boronic acid and methyl 2-chloro-4,6-dimethylpyrimidine-5-carboxylate (described in Example 53) to give methyl 2-(4-hydroxyphenyl)-4,6-dimethylpyrimidine-5-carboxylate as a white solid. ¹H NMR (400 MHz, CDCl₃) δ8.39 (d, J = 8.8 Hz, 2H), 6.91 (d, J =8.8 Hz, 2H), 5.11 (s, 1H), 3.95 (s, 3H), 2.58 (s, 6H).

Step 2: To a solution of methyl 2-(4-hydroxyphenyl)-4,6-dimethylpyrimidine-5-carboxylate (50 mg, 0.19 mmol), 4,4-dimethylcyclohexan-1-ol (62 mg, 0.48 mmol) and PPh₃ (127 mg, 0.48 mmol) in toluene (5 mL) was added diisopropyl azodicarboxylate (98 mg, 0.48 mmol) dropwise at 0 °C and the resulting mixture was stirred at 80 °C for 2 h. The volatiles were removed under reduced pressure and the residue was purified by preparatory-TLC (eluting with 30% EtOAc in petroleum ether) to give methyl 2-(4-((4,4-dimethylcyclohexyl)oxy)phenyl)-4,6-dimethylpyrimidine-5-carboxylate (50 mg, 70% yield) as a white solid.

Step 3: Typical ester hydrolysis condition (NaOH, MeOH/H₂O, as described in Example H) was applied to methyl 2-(4-((4,4-dimethylcyclohexyl)oxy)phenyl)-4,6-dimethylpyrimidine-5-carboxylate to give 2-(4-((4,4-dimethylcyclohexyl)oxy)phenyl)-4,6-dimethylpyrimidine-5-carboxylic acid as a white solid. LCMS (Method 5-95 AB, ESI): t_{R} = 1.022 min, [M + H]⁺ = 355.0.

Compound 348 (formic acid salt) was prepared as a white solid from Compound **101-K** and 2-(4-((4,4-dimethylcyclohexyl)oxy)phenyl)-4,6-dimethylpyrimidine-5-carboxylic acid by utilizing methods analogous to those described in Example G. LCMS (Method 5-95 AB, ESI): t_{R} = 0.740 min, [M + H]⁺ = 974.6; ¹H NMR (400 MHz, MeOH-*d*₄) δ 8.51 (brs, 1H), 8.25 (d, *J*= 8.0 Hz, 2H), 7.30-7.15 (m, 2H), 7.14-7.05 (m, 2H), 6.96 (d, *J*=8.4 Hz, 2H), 6.87 (s, 1H), 6.64 (s, 1H), 6.52 (s, 1H), 5.26-5.23 (m, 1H), 4.85-4.75 (m, 2H), 4.45-4.40 (m, 1H), 4.30-4.10 (m, 6H), 3.30-2.95 (m, 8H), 3.00 (s, 3H), 2.47 (s, 6H), 2.25-2.15 (m, 1H), 2.15-2.00 (m, 1H), 1.95-1.85 (m, 2H), 1.75-1.60 (m, 2H), 1.59-1.45 (m, 2H), 1.40-1.25 (m, 5H), 1.01 (s, 3H), 0.99 (s, 3H).

### Example 149: Synthesis of Compound 349

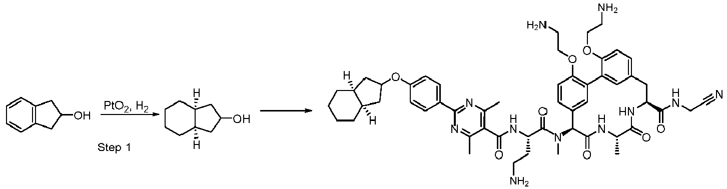

Step 1: A mixture of 2,3-dihydro-1*H*-inden-2-ol (100 mg, 0.75 mmol) and PtO₂ (30.0 mg, 0.75 mmol) was stirred in MeOH/AcOH (11 mL, v/v=10/1) under H₂ (15 psi) for 16 h. After filtration, the volatiles were removed under reduced pressure and the residue was purified by silica gel chromatography, eluting with 15% EtOAc in petroleum ether, to give (*cis*)-octahydro-1*H*-inden-2-ol (20 mg, 19% yield) as a colorless oil. ¹H NMR (400 MHz, CDCl₃) δ 4.41-4.35 (m, 1H), 2.10-1.98 (m, 2H), 1.95-1.80 (m, 2H), 1.55-1.45 (m, 6H), 1.38-1.22 (m, 3H), 0.90-0.80 (m, 1H).

Compound 349 (formic acid salt, mixture of diastereomers) was prepared as a white solid using **101-K** and (*cis*)-octahydro-1*H*-inden-2-ol by utilizing methods analogous to those described in Example 148. LCMS (Method 5-95 AB, ESI): t_{R} = 0.750 min, [M + H]⁺ = 986.6; ¹H NMR (400 MHz, MeOH-*d*₄) δ 8.51 (brs, 3H), 8.30 (d, *J*=8.4 Hz, 2H), 7.27 (d, *J*=8.4 Hz, 1H), 7.19 (d, *J*=8.4 Hz, 2H), 7.08 (d, *J*=8.4 Hz, 1H), 7.00-6.80 (m, 3H), 6.74 (s, 1H), 6.46 (s, 1H), 5.26-5.20 (m, 1H), 5.00-4.75 (m, 3H), 4.21 (s, 2H), 4.20-4.16 (m, 4H), 3.34-3.05 (m, 8H), 3.00 (s, 3H), 2.51 (s, 6H), 2.40-2.05 (m, 7H), 1.85-1.75 (m, 2H), 1.70-1.45 (m, 4H), 1.40-1.30 (m, 6H).

### Example 150: Synthesis of Compound 350

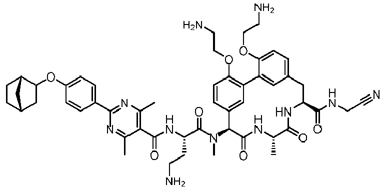

Compound 350 (formic acid salt) was prepared as a white solid from Compound **101-K** by utilizing methods analogous to those described in Example 148. LCMS (Method 5-95 AB, ESI): t_{R} = 0.750 min, [M + H]⁺ = 958.5; ¹H NMR (400 MHz, MeOH-*d*₄) δ 8.49 (brs, 3H), 8.20 (d, *J*=8.0 Hz, 2H), 7.28-7.22 (m, 2H), 7.09-7.07 (m, 2H), 6.90-6.86 (m, 3H), 6.60 (brs, 1H), 6.55 (brs, 1H), 5.28-5.25 (m, 1H), 4.85-4.77 (m, 2H), 4.32-4.30 (m, 3H), 4.25-4.19 (m, 4H), 3.25-3.05 (m, 8H), 3.01 (s, 1H), 2.45 (s, 6H), 2.31-2.12 (m, 2H), 1.89-1.84 (m, 1H), 1.70-1.57 (m, 5H), 1.35 (d, *J*=6.4 Hz, 3H), 1.26-1.22 (m, 4H).

### Example 151: Synthesis of Compound 351

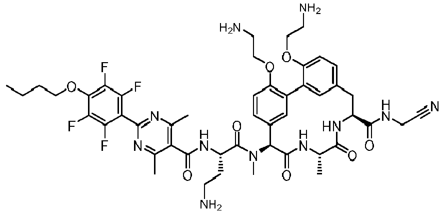

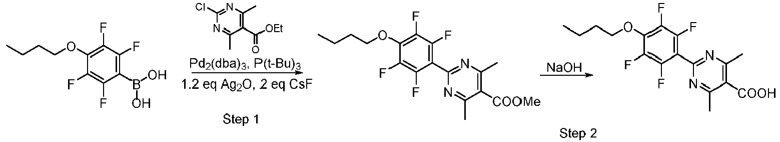

Step 1: A mixture of (4-butoxy-2,3,5,6-tetrafluorophenyl)boronic acid (146 mg, 0.55 mmol), ethyl 2-chloro-4,6-dimethylpyrimidine-5-carboxylate (100 mg, 0.50 mmol), Pd₂(dba)₃ (23 mg, 0.02 mmol), P(*t*-Bu)₃ (15 mg, 0.07 mmol), Ag₂O (139 mg, 0.6 mmol) and CsF (189 mg, 1.25 mmol) in toluene (5 mL) was stirred at 100 °C for 20 h under N₂. The volatiles were removed under reduced pressure and the residue was purified by preparatory-TLC (eluting with 10% EtOAc in petroleum ether, R_{f} = 0.4) to give methyl 2-(4-butoxy-2,3,5,6-tetrafluorophenyl)-4,6-dimethylpyrimidine-5-carboxylate (50 mg, 26% yield) as a colorless oil. LCMS (ESI): [M + H]⁺ = 387.1.

Step 2: Typical ester hydrolysis conditions (NaOH, MeOH/H₂O, Example H) were applied to methyl 2-(4-butoxy-2,3,5,6-tetrafluorophenyl)-4,6-dimethylpyrimidine-5-carboxylate to give 2-(4-butoxy-2,3,5,6-tetrafluorophenyl)-4,6-dimethylpyrimidine-5-carboxylic acid as a white solid.

Compound 351 (formic acid salt) was prepared as a white solid from Compound **101-K** and 2-(4-butoxy-2,3,5,6-tetrafluorophenyl)-4,6-dimethylpyrimidine-5-carboxylic acid by utilizing methods analogous to those described in Example G. LCMS (Method 5-95 AB, ESI): t_{R} = 0.619 min, [M + H]⁺ = 992.5; ¹H NMR (400 MHz, MeOH-*d*₄) δ 8.50 (brs, 1H), 7.34-7.28 (m, 1H), 7.27-7.22 (m, 1H), 7.19 (d, J=8.4 Hz, 1H), 7.10 (d, *J*=8.4 Hz, 1H), 6.92 (d, *J*=2.4 Hz, 1H), 6.84 (d, *J*=2.4 Hz, 1H), 6.41 (s, 1H), 5.24-5.18 (m, 1H), 4.82-4.80 (m, 2H), 4.36 (t, *J*=6.4 Hz, 2H), 4.26-4.17 (m, 6H), 3.36-3.37 (m, 1H), 3.21-3.10 (m, 7H), 3.01 (s, 3H), 2.61 (s, 6H), 2.34-2.24 (m, 1H), 2.21-2.11 (m, 1H), 1.85-1.76 (m, 2H), 1.61-1.51 (m, 2H), 1.36 (d, *J*=7.0 Hz, 3H),1.02 (t, *J*=7.4 Hz, 3H).

### Example 152: Synthesis of Compound 352

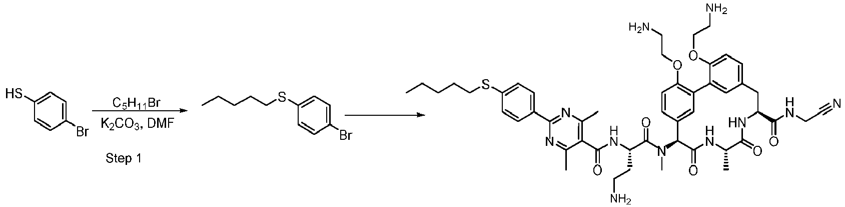

Step 1: A mixture of 4-bromobenzenethiol (300 mg, 1.6 mmol), 1-bromopentane (1.2 g, 8.0 mmol) and K₂CO₃ (658 mg, 4.8 mmol) in DMF (7 mL) was stirred at 80 °C for 16 h. The reaction was poured into water (20 mL), which was extracted with EtOAc (3 x 30 mL). The combined organic layers were washed with brine (2 x 50 mL), dried over Na₂SO₄, concentrated and the residue was purified via silica gel chromatography, eluting with 5% EtOAc in petroleum ether, to give (4-bromophenyl)(pentyl)sulfane (300 mg, 73% yield) as a colorless oil.

Compound 352 (formic acid salt) was prepared as a white solid from Compound **101-K** and (4-bromophenyl)(pentyl)sulfane by utilizing methods analogous to those described in Example 53. LCMS (Method 5-95 AB, ESI): t_{R} = 0.760 min, [M + H]⁺ = 950.4; ¹H NMR (400 MHz, MeOH-*d*₄) δ 8.50 (brs, 3H), 8.31 (d, *J*=8.0 Hz, 2H), 7.38 (d, *J*=8.4 Hz, 2H), 7.27 (d, J=2.0 Hz, 1H), 7.20 (d, *J*=8.4 Hz, 2H), 7.09 (d, *J*=8.4 Hz, 1H), 6.90 (s, 1H), 6.89 (s, 1H), 6.45 (s, 1H), 5.24-5.20 (m, 1H), 4.86-4.75 (m, 1H), 4.24-4.15 (m, 7H), 3.20-2.95 (m, 13H), 2.53 (s, 6H), 2.25-2.15 (m, 1H), 2.15-2.05 (m, 1H), 1.75-1.60 (m, 2H), 1.50-1.25 (m, 7H), 0.93 (t, *J*=7.2 Hz, 3H).

### Example 153: Synthesis of Compound 353

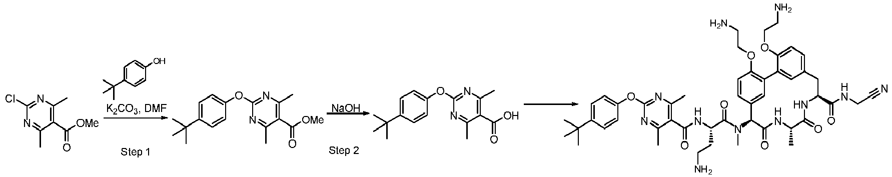

Step 1: A mixture of methyl 2-chloro-4,6-dimethylpyrimidine-5-carboxylate (described in Example 53) (100 mg, 0.50 mmol), 4-*tert*-butyl phenol (97 mg, 0.65 mmol) and K₂CO₃ (207 mg, 1.5 mmol) in DMF (3 mL) was stirred at 100 °C for 4 h. The volatiles were removed under reduced pressure and the residue was taken up by EtOAc (30 mL), which was washed with brine (2 × 30 mL). The organic layer was dried over Na₂SO₄, concentrated and the residue was purified by reverse-phase HPLC to give methyl 2-(4-(*tert*-butyl)phenoxy)-4,6-dimethylpyrimidine-5-carboxylate (73 mg, 47% yield) as white solid. LCMS (Method 5-95 AB, ESI): t_{R} = 0.953 min, [M + H]⁺ = 314.9.

Step 2: Typical ester hydrolysis conditions (NaOH, MeOH/H₂O, described in Example H) were applied to methyl 2-(4-(*tert*-butyl)phenoxy)-4,6-dimethylpyrimidine-5-carboxylate to give 2-(4-(*tert-*butyl)phenoxy)-4,6-dimethylpyrimidine-5-carboxylic acid as a white solid.

Compound 353 (formic acid salt) was prepared as a white solid from Compound **101-K** and 2-(4-(tert-butyl)phenoxy)-4,6-dimethylpyrimidine-5-carboxylic acid by utilizing methods analogous to those described in Example G. LCMS (Method 5-95 AB, ESI): t_{R} = 0.721 min, [M + H]⁺ = 920.5; ¹H NMR (400 MHz, MeOH-*d*₄) δ 8.48 (brs, 2H),7.46 (d, J=8.8 Hz, 2H), 7.26-7.14 (m, 3H), 7.11-7.03 (m, 3H), 6.89 (s, 1H), 6.79 (s, 1H), 6.40 (s, 1H), 5.20-5.14 (m, 1H), 4.79-4.62 (m, 2H), 4.29-4.18 (m, 6H), 3.40-3.35 (m, 1H), 3.21-3.08 (m, 7H), 2.97 (s, 3H), 2.41 (s, 6H), 2.30-2.10 (m, 2H), 1.36 (s, 9H), 1.35 (d, J=6.8 Hz, 3H).

### Example 154: Synthesis of Compound 354

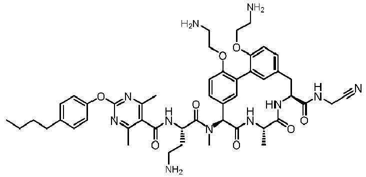

Compound 354 (formic acid salt) was prepared as a white solid from Compound **101-K** by utilizing methods analogous to those described in Example 153. LCMS (Method 5-95 AB, ESI): t_{R} = 0.738 min, [M + H]⁺ = 920.4; ¹H NMR (400 MHz, MeOH-*d*₄) δ 8.51 (brs, 2H), 7.26-7.21 (m, 4H), 7.18 (d, J=8.4 Hz, 1H), 7.10 (d, *J*=8.4 Hz, 1H), 7.01 (d, *J*=8.4 Hz, 2H), 6.88 (d, *J*=2.4 Hz, 1H), 6.75 (s, 1H), 6.44 (s, 1H), 5.20-5.17 (m, 1H), 4.82-4.77 (m, 2H), 4.25-4.18 (m, 6H), 3.40-3.35 (m, 1H), 3.18-3.00 (m, 7H), 2.98 (s, 3H), 2.66 (t, *J*=8.4 Hz, 2H), 2.40 (s, 6H), 2.30-2.21 (m, 1H), 2.18-2.08 (m, 1H), 1.67-1.61 (m, 2H), 1.49-1.30 (m, 5H), 0.97 (t, *J*=7.5 Hz, 3H).

### Example 155: Synthesis of Compound 355

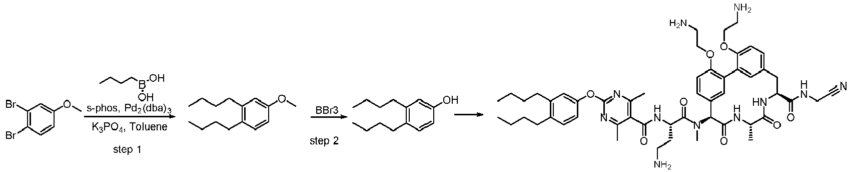

Step 1: Typical Suzuki conditions, as described in Example H, were applied to 1,2-dibromo-4-methoxybenzene to give 1,2-dibutyl-4-methoxybenzene as a colorless oil.

Step 2: To a solution of 1,2-dibutyl-4-methoxybenzene (410 mg, 1.9 mmol) in DCM (20 mL) added BBr₃ (0.54 mL, 5.6 mmol) at 0 °C and the mixture was stirred at 15 °C for 16 h. The reaction was quenched with MeOH (20 mL), the volatiles were removed under reduced pressure, and the residue was purified by silica gel chromatography, eluting with 0-20% EtOAc in petroleum ether, to give 3,4-dibutylphenol (370 mg, 96% yield) as a brown oil. ¹H NMR (400 MHz, MeOH-*d*4): δ 6.91 (d, *J*=8.4 Hz, 1H), 6.56 (d, *J*=2.8 Hz, 1H), 6.55-6.50 (m, 1H), 2.60-2.45 (m, 4H), 1.60-1.45 (m, 4H), 1.45-1.30 (m, 4H), 1.00-0.92 (m, 6H).

Compound 355 (formic acid salt) was prepared as a white solid from Compound **101-K** and 3,4-dibutylphenol by utilizing methods analogous to those described in Example 153. LCMS (Method 5-95 AB, ESI): t_{R} = 0.791 min, [M + H]⁺ = 976.4; ¹H NMR (400 MHz, MeOH-*d*₄) δ 8.50 (brs, 1H), 7.32-7.26 (m, 2H), 7.21-7.15 (m, 2H), 7.11-7.04 (m, 3H), 6.93-6.88 (m, 2H), 6.46 (s, 1H), 5.20-5.15 (m, 1H), 4.85-4.75 (m, 2H), 4.23-4.12 (m, 6H), 3.18-2.99 (m, 11H), 2.66 (t, *J*=7.6 Hz, 4H), 2.44 (s, 6H), 2.30-2.23 (m, 1H), 2.16-2.10 (m, 1H), 1.65-1.55 (m, 4H), 1.49-1.38 (m, 7H), 0.97 (t, *J*=7.6 Hz, 6H).

### Example 156: Synthesis of Compound 356

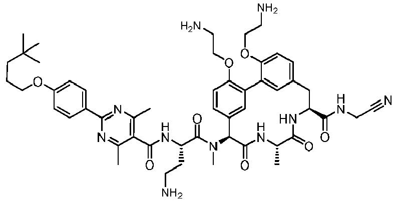

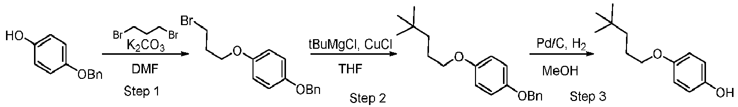

Step 1: Typical alkylation conditions (as described in Example 21) was applied to 4-(benzyloxy)phenol to give 1-(benzyloxy)-4-(3-bromopropoxy)benzene as a colorless oil.

Step 2: To a solution of 1-(benzyloxy)-4-(3-bromopropoxy)benzene (1.5 g, 4.67 mmol) and CuI (231 mg, 2.33 mmol) in THF (50 mL) was added t-BuMgCl (2N in Et₂O, 23.4 mL) and the mixture was stirred at 25 °C for 4h. The reaction was quenched with a saturated aqueous NH₄Cl solution (30 mL), and the resulting mixture was extracted by EtOAc (3 × 30 mL). The combined organic layers were washed with brine (2 × 100 mL), dried over Na₂SO₄, concentrated and the residue was purified by reverse-phase HPLC (acetonitrile 60-98%/0.225% formic acid in water) to give 1-(benzyloxy)-4-((4,4-dimethylpentyl)oxy)benzene as a white solid. ¹H NMR (400 MHz, CDCl₃) δ 7.35-7.21 (m, 5H), 6.90 (d, *J*=8.8 Hz, 2H), 6.83 (d, *J*=8.8 Hz, 2H), 5.01 (s, 2H), 3.88 (t, *J*=6.4 Hz, 2H), 1.76-1.70 (m, 2H), 1.30-1.20 (m, 2H), 0.91 (s, 9H).

Step 3: Typical hydrogenation conditions, as described in Example D, were applied to 1-(benzyloxy)-4-((4,4-dimethylpentyl)oxy)benzene to give 4-((4,4-dimethylpentyl)oxy)phenol as a colorless oil.

Compound 356 (formic acid salt) was prepared as a white solid from Compound **101-K** and 4-((4,4-dimethylpentyl)oxy)phenol by utilizing methods analogous to those described in Examples 10 and 53. LCMS (Method 5-95 AB, ESI): t_{R} = 0.766 min, [M + H]⁺ = 962.6; ¹H NMR (400 MHz, MeOH-*d*₄) δ 8.52 (brs, 1H), 8.23 (d, *J*=7.6 Hz, 2H), 7.26-7.21 (m, 2H), 7.12-7.08 (m, 2H), 6.96 (d, J=8.4 Hz, 2H), 6.86 (d, *J*= 2.4 Hz, 1H), 6.62 (brs, 1H), 6.54 (brs, 1H), 5.28-5.25 (m, 1H), 4.80-4.75 (m, 1H), 4.60-4.45 (m, 1H), 4.40-4.10 (m, 6H), 4.03 (t, *J*=6.4 Hz, 2H), 3.33-3.15 (m, 4H), 3.15-2.90 (m, 7H), 2.46 (s, 6H), 2.40-2.20 (m, 1H), 2.20-2.05 (m, 1H), 1.84-1.76 (m, 2H), 1.42-1.33 (m, 5H), 0.96 (s, 9H).

### Example 157: Synthesis of Compound 357

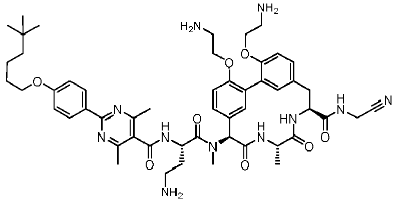

Compound 357 (formic acid salt) was prepared as a white solid from Compound **101-K** by utilizing methods analogous to those described in Example 156. LCMS (Method 5-95 AB, ESI): t_{R} = 0.780 min, [M + H]⁺ = 976.6; ¹H NMR (400 MHz, MeOH-*d*₄) δ 8.52 (brs, 1H), 8.27-8.22 (m, 2H), 7.25-7.18 (m, 2H), 7.14-7.05 (m, 2H), 7.04-6.95 (m, 2H), 6.86 (s, 1H), 6.65 (brs, 1H), 6.53 (brs, 1H), 5.27-5.24 (m, 1H), 4.85-4.75 (m, 2H), 4.35-4.20 (m, 6H), 4.07 (t, *J*=7.2 Hz, 2H), 3.20-2.90 (m, 11H), 2.47 (s, 6H), 2.25-2.15 (m, 1H), 2.14-2.05 (m, 1H), 1.81-1.70 (m, 2H), 1.50-1.40 (m, 2H), 1.36 (d, *J*=7.2 Hz, 3H), 1.30-1.20 (m, 2H), 0.94 (s, 9H).

### Example 158: Synthesis of Compound 358

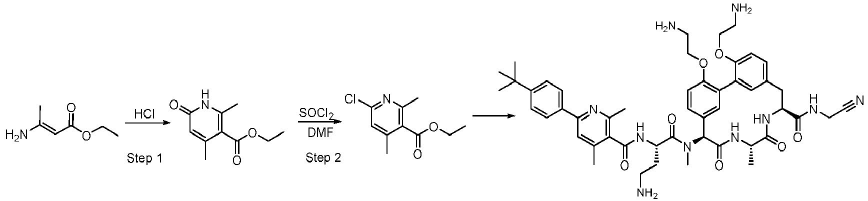

Step 1: To a solution of ethyl (*E*)-3-aminobut-2-enoate (17.0 g, 132 mmol) in toluene (100 mL) was added HCl (4N in dioxane, 66 mL) and the mixture was stirred at reflux for 16 h. After filtration, the filtrate was concentrated and the residue was purified by silica gel chromatography, eluting with 5% MeOH/EtOAc, to give ethyl 2,4-dimethyl-6-oxo-1,6-dihydropyridine-3-carboxylate (5.0 g, 20% yield) as a white solid. ¹H NMR (400 MHz, CDCl₃) δ 6.25 (s, 1H), 4.33 (q, *J*=7.2 Hz, 2H), 2.52 (s, 3H), 2.29 (s, 3H), 1.36 (t, *J*=7.2 Hz, 3H).

Step 2: To a solution of ethyl 2,4-dimethyl-6-oxo-1,6-dihydropyridine-3-carboxylate (2.0 g, 10.2 mmol) in toluene (100 mL) was added SOCl₂ (3.66 g, 30.7 mmol) and DMF (1.12 g, 15.4 mmol) and the mixture was stirred at 80 °C for 12 h. The reaction mixture was diluted with water (50 mL), which was extracted by EtOAc (3 × 50 mL). The combined organic portions were washed with brine (2 × 100 mL), dried over MgSO₄, concentrated and the residue was purified by silica gel chromatography, eluting with 10% EtOAc in petroleum ether, to give ethyl 6-chloro-2,4-dimethylnicotinate (2.0 g, 91% yield) as a brown oil. LCMS (Method 5-95 AB, ESI): t_{R} = 0.816 min, [M + H]⁺ = 213.8.

Compound 358 (formic acid salt) was prepared as a white solid from Compound **101-K** and ethyl 6-chloro-2,4-dimethylnicotinate by utilizing methods analogous to those described in Example 53. LCMS (Method 5-95 AB, ESI): t_{R} = 0.670 min, [M + H]⁺ = 903.8; ¹H NMR (400 MHz, MeOH-*d*₄) δ 8.46 (brs, 3H), 7.80-7.74 (m, 2H), 7.51-7.47 (m, 3H), 7.29-7.22 (m, 2H), 7.20-7.05 (m, 2H), 6.87 (s, 1H), 6.56 (brs, 2H), 5.30-5.26 (m, 1H), 4.86-4.77 (m, 1H), 4.45-4.15 (m, 7H), 3.34-3.10 (m, 8H), 3.03 (s, 3H), 2.50 (s, 3H), 2.40-2.10 (m, 5H), 1.37 (s, 9H), 1.35 (d, *J*=6.8 Hz, 3H).

### Example 159: Synthesis of Compound 359

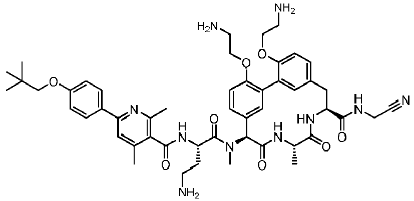

Compound 359 (formic acid salt) was prepared as a white solid from Compound **101-K** by utilizing methods analogous to those described in Example 158. LCMS (Method 5-95 AB, ESI): t_{R} = 0.705 min, [M + H]⁺ = 933.7; ¹H NMR (400 MHz, MeOH-*d*₄) δ 8.46 (brs, 2H), 7.60 (brs, 2H), 7.41 (brs, 1H), 7.29-7.20 (m, 2H), 7.15-7.01 (m, 2H), 6.94 (d, *J*=8.4 Hz, 2H), 6.81 (s, 1H), 6.75 (s, 1H), 6.37 (s, 1H), 5.34 (brs, 1H), 4.79-4.74 (m, 1H), 4.55-4.30 (m, 2H), 4.30-4.10 (m, 5H), 3.68 (s, 2H), 3.44-3.34 (m, 1H), 3.31-3.05 (m, 5H), 3.04 (s, 3H), 3.00-2.85 (m, 2H), 2.50-2.00 (m, 8H),1.37 (d, *J*=6.8 Hz, 3H), 1.07 (s, 9H).

### Example 160: Synthesis of Compound 360

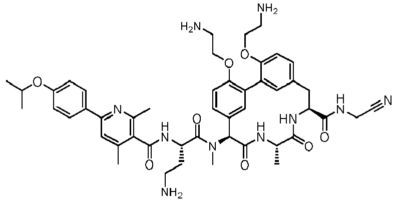

Compound 360 (formic acid salt) was prepared as a white solid from Compound **101-K** by utilizing methods analogous to those described in Example 158. LCMS (Method 5-95 AB, ESI): t_{R} = 0.515 min, [M + H]⁺ = 905.4; ¹H NMR (400 MHz, MeOH-*d*₄) δ 8.47 (brs, 3H), 7.61 (brs, 2H), 7.38 (brs, 1H), 7.27-7.23 (m, 2H), 7.11-7.05 (m, 2H), 6.91 (d, *J*=8.4 Hz, 2H), 6.82 (s, 1H), 6.69 (s, 1H), 6.37 (s, 1H), 5.40-5.30 (m, 1H), 4.76-4.65 (m, 2H), 4.55-4.31 (m, 2H), 4.30-4.10 (m, 5H), 3.34-3.11 (m, 6H), 3.03 (s, 3H), 3.01-2.85 (m, 2H), 2.50-2.00 (m, 8H), 1.35 (d, *J*= 6.0 Hz, 6H), 1.34 (d, *J*=6.4 Hz, 3H).

### Example 161: Synthesis of Compound 361

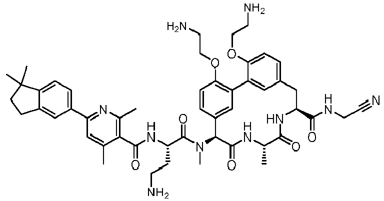

Compound 361 (formic acid salt) was prepared as a white solid from Compound **101-K** by utilizing methods analogous to those described in Example 158. LCMS (Method 5-95 AB, ESI): t_{R} = 0.688 min, [M + H]⁺ = 915.5; ¹H NMR (400 MHz, MeOH-*d*₄) δ 8.43 (brs, 2H), 7.63-7.15 (m, 6H), 7.12 (brs, 2H), 6.86 (s, 1H), 6.59 (brs, 1H), 5.33-5.27 (m, 1H), 4.79-4.76 (m, 2H), 4.44-4.20 (m, 6H), 3.31-3.12 (m, 8H), 3.04 (s, 3H), 2.95 (t, *J*=6.8 Hz, 2H), 2.48 (s, 3H), 2.30 (s, 3H), 2.28-2.14 (m, 2H), 1.99 (t, *J=*6.8 Hz, 2H), 1.37 (d, J=6.4 Hz, 3H), 1.36 (s, 6H).

### Example 162: Synthesis of Comopund 362

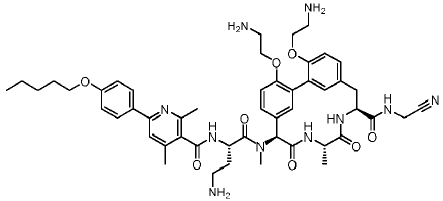

Compound 362 (formic acid salt) was prepared as a white solid from Compound **101-K** by utilizing methods analogous to those described in Example 158. LCMS (Method 5-95 AB, ESI): t_{R} = 0.700 min, [M + H]⁺ = 933.4; ¹H NMR (400 MHz, MeOH-*d*₄) δ 8.50 (brs, 2H), 7.94-7.86 (m, 2H), 7.50 (brs, 1H), 7.32-7.15 (m, 3H), 7.10 (d, *J*=8.4 Hz, 1H), 7.04-6.98 (m, 2H), 6.92 (d, *J*=2.0 Hz, 1H), 6.86-6.81 (m, 1H), 6.57 (s, 1H), 5.37-5.30 (m, 1H), 4.82-4.73 (m, 2H), 4.26-4.17 (m, 6H), 4.03 (t, *J*=6.4 Hz, 2H), 3.22-3.09 (m, 8H), 3.02 (s, 3H), 2.56 (s, 3H), 2.43 (s, 3H), 2.31-2.25 (m, 1H), 2.17-2.11 (m, 1H), 1.84-1.78 (m, 2H), 1.54-1.38 (m, 4H), 1.32 (d, *J*=6.8 Hz, 3H), 0.97 (t, *J*=6.8 Hz, 3H).

### Example 163: Synthesis of Compound 363

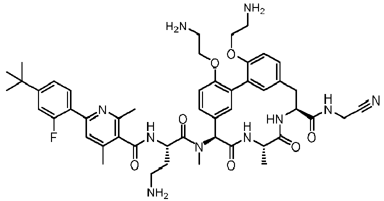

Compound 363 (formic acid salt) was prepared as a white solid from Compound **101-K** by utilizing methods analogous to those described in Example 158. LCMS (Method 5-95 AB, ESI): t_{R} = 0.700 min, [M + H]⁺ = 921.5; ¹H NMR (400 MHz, MeOH-*d*₄) δ 8.50 (s, 1H), 7.75-7.64 (m, 1H), 7.50 (s, 1H), 7.36-7.05 (m, 5H), 6.88 (s, 1H), 6.64 (brs, 1H), 6.54 (brs, 1H), 5.30-5.25 (m, 1H), 4.80-4.72 (m, 2H), 4.39-4.16 (m, 6H), 3.28-3.06 (m, 8H), 3.03 (s, 3H), 2.52 (s, 3H), 2.34 (s, 3H), 2.31-2.12 (m, 2H), 1.38 (s, 9H), 1.36 (d, *J*=6.8 Hz, 3H).

### Example 164: Synthesis of Compound 364

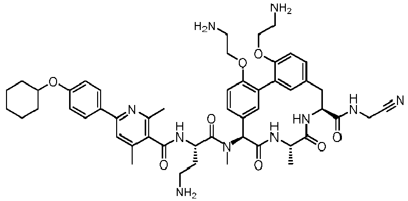

Compound 364 (formic acid salt) was prepared as a white solid from Compound **101-K** by utilizing methods analogous to those described in Example 158. LCMS (Method 5-95 AB, ESI): t_{R} = 0.690 min, [M + H]⁺ = 945.4; ¹H NMR (400 MHz, MeOH-*d*₄) δ 8.47 (brs, 3H), 7.78 (brs, 2H), 7.45 (brs, 1H), 7.27-7.08 (m, 4H), 6.98 (d, *J*=8.4 Hz, 2H), 6.87 (s, 1H), 6.65 (brs, 1H), 6.52 (brs, 1H), 5.30-5.20 (m, 1H), 4.85-4.75 (m, 2H), 4.50-4.15 (m, 7H), 3.25-3.10 (m, 8H), 3.02 (s, 3H), 2.51 (s, 3H), 2.40-2.05 (m, 7H), 2.05-1.95 (m, 2H), 1.90-1.75 (m, 2H), 1.70-1.40 (m, 6H), 1.36 (d, *J*=7.2 Hz, 3H).

### Example 165: Synthesis of Compound 365

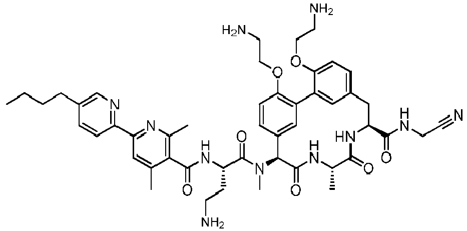

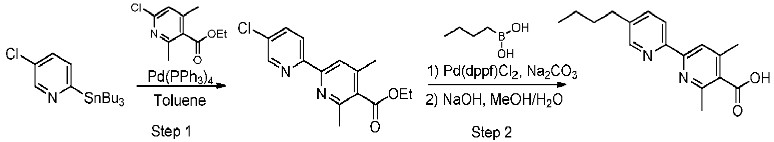

Step 1: A solution of 5-chloro-2-(tributylstannyl)pyridine (400 mg, 0.99 mmol), ethyl 6-chloro-2,4-dimethyl-pyridine-3-carboxylate (234 mg, 1.09 mmol) and Pd(PPh₃)₄ (115 mg, 0.10 mmol) in toluene (10 mL) was stirred at 110 °C for 8 h. After filtration, the volatiles were removed under reduced pressure and the residue was purified via silica gel chromatography, eluting with 0-5% EtOAc in petroleum ether, to give ethyl 6-(5-chloro-2-pyridyl)-2,4-dimethyl-pyridine-3-carboxylate (120 mg, 42% yield) as a white solid. ¹H NMR (400MHz, CDCl₃) δ 8.62 (s, 1H), 8.42 (d, *J*=8.6 Hz, 1H), 8.08 (s, 1H), 7.81-7.75 (m, 1H), 4.42 (q, *J*=7.2 Hz, 2H), 2.63 (s, 3H), 2.43 (s, 3H), 1.43 (t, *J*=7.2 Hz, 3H).

Step 2: Starting from ethyl 6-(5-chloro-2-pyridyl)-2,4-dimethyl-pyridine-3-carboxylate, typical Suzuki and ester hydrolysis conditions (as described in Example 10) were followed to give 5'-butyl-4,6-dimethyl-[2,2'-bipyridine]-5-carboxylic acid as a colorless oil.

Compound 365 (formic acid salt) was prepared as a white solid from Compound **101-K** and 5'-butyl-4,6-dimethyl-[2,2'-bipyridine]-5-carboxylic acid by utilizing methods analogous to those described in Example G. LCMS (Method 5-95 AB, ESI): t_{R} = 0.536 min, [M + H]⁺ = 904.3; ¹H NMR (400 MHz, MeOH-*d*₄) δ 8.60 (brs, 1H), 8.38 (brs, 1H), 8.12 (brs, 1H), 7.85-7.61 (m, 2H), 7.32-7.21 (m, 2H), 6.92 (brs, 1H), 6.80 (brs, 1H), 6.66 (brs, 1H), 6.25 (brs, 1H), 5.40-5.29 (m, 1H), 4.90-4.83 (m, 1H), 4.79-4.71 (m, 1H), 4.46 (brs, 2H), 4.29-4.20 (m, 4H), 3.48-3.39 (m, 1H), 3.33--3.10 (m, 7H), 3.03 (s, 3H), 2.74 (t, *J*=7.6 Hz, 2H), 2.51-2.38 (m, 2H), 2.34-2.09 (m, 4H), 1.75-1.67 (m, 2H), 1.49-1.42 (m, 2H), 1.36 (d, *J*=6.8 Hz, 3H), 1.02 (t, *J*=7.6 Hz, 3H).

### Example 166: Synthesis of Compound 366

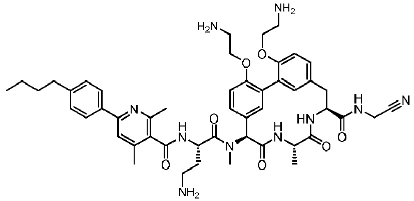

Compound 366 (trifluoroacetic acid salt) was prepared as a white solid from Compound **101-K** by utilizing methods analogous to those described in Example 158. LCMS (Method 5-95 AB, ESI): t_{R} = 0.704 min, [M + H]⁺ = 903.3; ¹H NMR (400 MHz, MeOH-*d*₄) δ 7.70-7.64 (m, 2H), 7.46-7.42 (m, 1H), 7.30-7.22 (m, 4H), 7.20-7.01 (m, 2H), 6.86 (s, 1H), 6.70 (brs, 1H), 6.48 (brs, 1H), 5.38-5.32 (m, 1H), 4.80-4.74 (m, 1H), 4.49-4.15 (m, 7H), 3.26-2.87 (m, 8H), 3.05 (s, 3H), 2.70 (t, *J*=7.6 Hz, 2H), 2.51 (s, 3H), 2.36-2.12 (m, 5H), 1.71-1.63 (m, 2H), 1.45-1.36 (m, 2H), 1.38 (d, *J*=6.8 Hz, 3H), 1.00 (t, *J*=7.2 Hz, 3H).

### Example 167: Synthesis of Compound 367

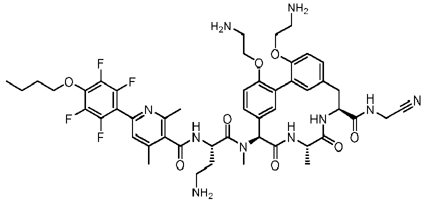

Compound 367 (formic acid salt) was prepared as a white solid from Compound **101-K** by utilizing methods analogous to those described in Example 151. LCMS (Method 5-95 AB, ESI): t_{R} = 0.740 min, [M + H]⁺ = 991.5; ¹H NMR (400 MHz, MeOH-*d*₄) δ 8.50 (brs, 1H), 7.41 (s, 1H), 7.34-7.22 (m, 2H), 7.19 (d, *J*=8.4 Hz, 1H), 7.10 (d, *J*=8.4 Hz, 1H), 6.92 (d, *J*=2.0 Hz, 1H), 6.83 (d, *J*=2.0 Hz, 1H), 6.41 (s, 1H), 5.23-5.20 (m, 1H), 4.85-4.77 (m, 2H), 4.34 (t, *J*=6.4 Hz, 2H), 4.28-4.16 (m, 6H), 3.23-3.09 (m, 8H), 3.01 (s, 3H), 2.60 (s, 3H), 2.45 (s, 3H), 2.33-2.23 (m, 1H), 2.21-2.11 (m, 1H), 1.84-1.76 (m, 2H), 1.61-1.51 (m, 2H), 1.36 (d, *J*=6.6 Hz, 3H), 1.02 (t, *J*=7.4 Hz, 3H).

### Example 168: Synthesis of Compound 368

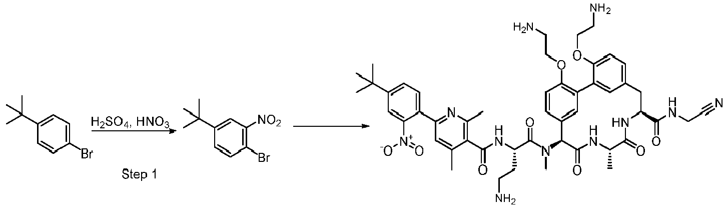

Step 1: To a stirred solution of 1-bromo-4-(t*e*rt-butyl)benzene (3.0 g, 14 mmol) in concentrated sulfuric acid (12 mL) was added HNO₃ (0.69 mL, 15.5 mmol) dropwise at 0 °C. The mixture was stirred at 0 °C for 1 h. The volatiles were removed under reduced pressure and the residue was taken up by EtOAc (50 mL), which was washed with a saturated aqueous Na₂CO₃ and brine (50 mL each). The organic layer was dried over MgSO₄, concentrated and the residue was purified by silica gel chromatography, eluting with petroleum ether, to give 1-bromo-4-(tert-butyl)-2-nitrobenzene (1.7 g, 47% yield) as a colorless oil.

Compound 368 (formic acid salt) was prepared as a white solid from Compound **101-K** and 1-bromo-4-(*tert*-butyl)-2-nitrobenzene by utilizing methods analogous to those described in Example 158. LCMS (Method 5-95 AB, ESI): t_{R} = 0.712 min, [M + H]⁺ = 948.4; ¹H NMR (400 MHz, MeOH-*d*₄) δ 8.48 (brs, 1H), 7.97 (d, *J*=2.0 Hz, 1H), 7.81 (d, *J*=8.0 Hz, 1H), 7.54 (d, *J*=8.0 Hz, 1H), 7.34 (s, 1H), 7.31-7.15 (m, 3H), 7.10 (d, *J*=8.4 Hz, 1H), 6.90 (d, *J*=2.4 Hz, 1H), 6.79 (s, 1H), 6.42 (s, 1H), 5.24-5.20 (m, 1H), 4.79-4.73 (m, 2H), 4.29-4.15 (m, 6H), 3.29-3.03 (m, 8H), 3.00 (s, 3H), 2.49 (s, 3H), 2.41 (s, 3H), 2.33-2.22 (m, 1H), 2.21-2.10 (m, 1H), 1.41 (s, 9H), 1.35 (d, *J*=6.8 Hz, 3H).

### Example 169: Synthesis of Compound 369

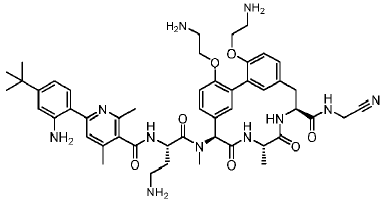

Compound 369 (formic acid salt) was prepared as a white solid from Compound **101-K** by utilizing methods analogous to those described in Example 168. LCMS (Method 5-95 AB, ESI): t_{R} = 0.664 min, [M + H]⁺ = 918.3; ¹H NMR (400 MHz, MeOH-*d*₄) δ 8.49 (brs, 1H), 7.41-7.15 (m, 5H), 7.09 (d, *J*=8.4 Hz, 1H), 6.89 (brs, 2H), 6.80 (brs, 1H), 6.78 (brs, 1H), 6.49(s, 1H), 5.29-5.24 (m, 1H), 4.84-4.75 (m, 2H), 4.37-4.17 (m, 6H), 3.31-3.11 (m, 8H), 3.01 (s, 3H), 2.54 (s, 3H), 2.31-2.23 (m, 4H), 2.18-2.13 (m, 1H), 1.36 (d, J=6.8 Hz, 3H), 1.33 (s, 9H).

### Example 170: Synthesis of Compound 370

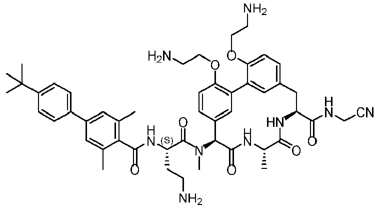

Compound 370 (formic acid salt) was prepared as a white solid from Compound **101-K** by utilizing methods analogous to those described in Example H. LCMS (Method 5-95 AB, ESI): t_{R} = 0.747 min, [M + H]⁺ = 902.6; ¹H NMR (400 MHz, MeOH-*d*₄) δ 8.53 (brs, 2H), 7.50-7.40 (m, 4H), 7.31-7.15 (m, 5H), 7.06 (d, J=8.4 Hz, 1H), 6.89 (d, *J*=2.0 Hz, 1H), 6.67 (s, 1H), 6.51 (s, 1H), 5.28-5.25 (m, 1H),4.80-4.78 (m, 2H), 4.26-4.17 (m, 6H), 3.22-3.07 (m, 8H), 3.02 (s, 3H), 2.35-2.25 (m, 1H), 2.31 (s, 6H), 2.20-2.11 (m, 1H), 1.37 (d, J=6.8 Hz, 3H), 1.36 (s, 9H).

### Example 171: Synthesis of Compound 371

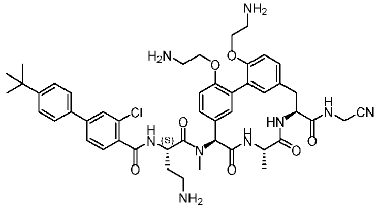

Compound 371 (formic acid salt) was prepared as a white solid from Compound **101-K** by utilizing methods analogous to those described in Example H. LCMS (Method 5-95 AB, ESI): t_{R} = 0.747 min, [M + H]⁺ = 908.3; ¹H NMR (400 MHz, MeOH-*d*₄) δ 8.50 (brs, 2H), 7.75 (s, 1H), 7.66-7.52 (m, 6H), 7.34-7.22 (m, 2H), 7.18 (d, *J*=8.4 Hz, 1H), 7.09 (d, *J*=8.4 Hz, 1H), 6.91 (s, 1H), 6.82 (s, 1H), 6.34 (s, 1H), 5.23-5.19 (m, 1H), 4.79-4.73 (m, 2H), 4.29-4.16 (m, 6H), 3.23-3.07 (m, 8H), 2.95 (s, 3H), 2.34-2.29 (m, 1H), 2.19-2.14 (m, 1H), 1.37 (d, J=6.8 Hz, 3H), 1.36 (s, 9H).

### Example 172: Synthesis of Compound 372

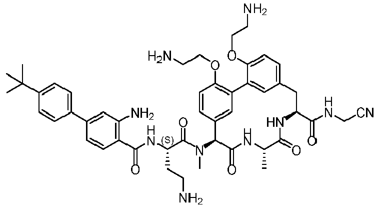

Compound 372 (formic acid salt) was prepared as a white solid from Compound **101-K** by utilizing methods analogous to those described in Example H. LCMS (Method 5-95 AB, ESI): t_{R} = 0.726 min, [M + H]⁺ = 889.4; ¹H NMR (400 MHz, MeOH-*d*₄) δ 8.50 (brs, 3H), 7.59-7.54 (m, 3H), 7.50-7.47 (m, 2H), 7.36-7.33 (m, 1H), 7.26-7.22 (m, 1H), 7.16 (d, J=8.6 Hz, 1H), 7.09 (d, *J*=8.6 Hz, 1H), 7.04 (d, *J*=2.0 Hz, 1H), 6.94-6.89 (m, 2H), 6.82 (d, J=2.0 Hz, 1H), 6.34 (s, 1H), 5.16-5.12 (m, 1H), 4.83-4.80 (m, 2H), 4.22-4.17 (m, 6H), 3.18-3.09 (m, 8H), 2.87 (s, 3H), 2.35-2.26 (m, 1H), 2.20-2.12 (m, 1H), 1.36 (s, 9H), 1.35 (d, J=6.8 Hz, 3H).

### Example 173: Synthesis of Compound 373

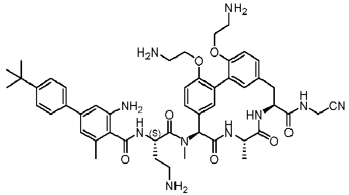

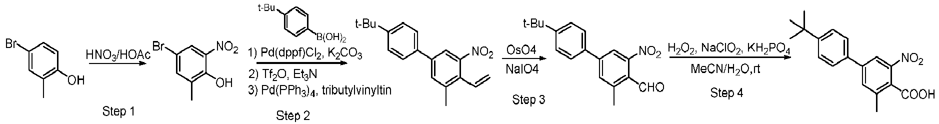

Step 1: To a stirred mixture of 4-bromo-2-methylphenol (4.0 g, 21.4 mmol) in acetic acid (22 mL) at 0 °C was added fuming HNO₃ (1.25 mL, 27.8 mmol) over 30 min and the mixture was stirred at 0 °C for another 15 min. The reaction was poured into ice water (80 mL) and the resulting precipitate was collected via filtration, redissolved by DCM (100 mL), which was dried over Na₂SO₄, concentrated and the residue was purified by silica gel chromatography, eluting with 0-1% EtOAc in petroleum ether, to give 4-bromo-2-methyl-6-nitrophenol (2.9 g, 58% yield) as a yellow solid.
Step 2: Starting from 4-bromo-2-methyl-6-nitrophenol, typical Suzuki and triflation (as described in Example 10) and Stille (as described in Example 165) conditions were followed to give 4'-(*tert*-butyl)-3-methyl-5-nitro-4-vinyl-1,1'-biphenyl as a yellow oil.

Step 3: A mixture of 4'-(*tert*-butyl)-3-methyl-5-nitro-4-vinyl-1,1'-biphenyl (100 mg, 0.34 mmol), OsO₄ (2.5 wt% in *t*-BuOH, 500 mg), NaIO₄ (362 mg, 1.7 mmol) and 0.2 M phosphate buffer (pH 7.2, 0.2 mL) in acetonitrile/H₂O (7.5 mL, v/v=2/1) was stirred at 25 °C for 24 h. The reaction was diluted with water (20 mL), which was extracted with EtOAc (2 × 20 mL). The combined organic layers were dried over Na₂SO₄, concentrated and the residue was purified by preparatory-TLC to give 4'-(*tert*-butyl)-3-methyl-5-nitro-[1,1'-biphenyl]-4-carbaldehyde (40 mg, 40% yield) as a white solid. ¹H NMR (400MHz, CDCl₃) δ 10.36 (s, 1H), 8.14 (s, 1H), 7.77 (s, 1H), 7.60-7.50 (m, 4H), 2.60 (s, 3H), 1.38 (s, 9H).

Step 4: A mixture of 4'-(*tert*-butyl)-3-methyl-5-nitro-[1,1'-biphenyl]-4-carbaldehyde (40 mg, 0.14 mmol), H₂O₂ (35% aqueous solution, 75 µL), NaClO₂ (25 mg, 0.27 mmol) and KH₂PO₄ (3 mg, 0.02 mmol) in acetonitrile/H₂O (6 mL, v/v=5/1) was stirred at 25 °C for 24 h. Na₂S₂O₃ (50 mg) was then added to quench the excess of H₂O₂. The mixture was partitioned between brine and EtOAc (each 20 mL) and the organic layer was dried over Na₂SO₄, and concentrated to give 4'-(*tert*-butyl)-3-methyl-5-nitro-[1,1'-biphenyl]-4-carboxylic acid (35 mg, 83% yield) as a white solid.

Compound 373 (formic acid salt) was prepared as a white solid from Compound **101-K** and 4'-(*tert*-butyl)-3-methyl-5-nitro-[1,1'-biphenyl]-4-carboxylic acid by utilizing methods analogous to those described in Example G. LCMS (Method 5-95 AB, ESI): t_{R} = 0.742 min, [M + H]⁺ = 903.4; ¹H NMR (400 MHz, MeOH-*d*₄) δ 8.51 (brs, 1H), 7.41 (brs, 4H), 7.31 (d, *J*=7.6 Hz, 1H), 7.24-7.12 (m, 2H), 7.08 (d, *J*=8.0 Hz, 1H), 6.89 (d, *J*=7.6 Hz, 2H), 6.73 (brs, 1H), 6.70 (brs, 1H), 6.53 (s, 1H), 5.20-5.17 (m, 1H), 4.80-4.78 (m, 1H), 4.67-4.61 (m, 1H), 4.30-4.15 (m, 6H), 3.23-3.11 (m, 8H), 3.00 (s, 3H), 2.33-2.05 (m, 2H), 2.21 (s, 3H), 1.36 (brs, 12H).

### Example 174: Synthesis of Compound 374

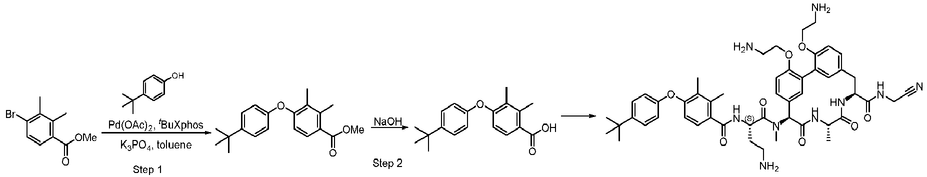

Step 1: A mixture of methyl 4-bromo-2,3-dimethylbenzoate (150 mg, 0.62 mmol), 4-(*tert-*butyl)phenol (139 mg, 0.93 mmol), Pd(OAc)₂ (7.0 mg, 0.03 mmol), 2-di-tert-butylphosphino-2',4',6'-triisopropylbiphenyl (26 mg, 0.06 mmol) and K₃PO₄ (330 mg, 1.85 mmol) in toluene (8 mL) was stirred at 100 °C for 16 h under N₂. The volatiles were removed under reduced pressure and the residue was purified by silica gel chromatography, eluting with 0-30% EtOAc in petroleum ether, to give methyl 4-(4-(*tert-*butyl)phenoxy)-2,3-dimethylbenzoate (180 mg, 93% yield) as a light yellow oil.
Step 2: Typical ester hydrolysis conditions (NaOH, MeOH/H₂O, as described in Example H) were applied to methyl 4-(4-(*tert*-butyl)phenoxy)-2,3-dimethylbenzoate to give 4-(4-(*tert*-butyl)phenoxy)-2,3-dimethylbenzoic acid as a white solid. LCMS (Method 5-95 AB, ESI): t_{R} = 0.990 min, [M + H]⁺ = 299.0.

Compound 374 (formic acid salt) was prepared as a white solid from Compound **101-K** and 4-(4-(*tert*-butyl)phenoxy)-2,3-dimethylbenzoic acid by utilizing methods analogous to those described in Example G. LCMS (Method 5-95 AB, ESI): t_{R} = 0.765 min, [M + H]⁺ = 918.5; ¹H NMR (400 MHz, MeOH-*d*₄) δ 8.50 (brs, 2H), 7.38 (d, *J*=8.0 Hz, 2H), 7.35-7.16 (m, 4H), 7.10 (d, *J*=8.0 Hz, 1H), 6.90 (d, *J*=8.0 Hz, 1H), 6.85-6.81 (m, 3H), 6.75 (d, *J*=8.0 Hz, 1H), 6.35 (s, 1H), 5.15-5.12 (m, 1H), 4.83-4.78 (m, 2H), 4.26-4.18 (m, 6H), 3.27-3.03 (m, 8H), 2.94 (s, 3H), 2.38 (s, 3H), 2.30-2.20 (m, 1H), 2.24 (s, 3H), 2.18-2.09 (m, 1H), 1.35 (d, *J*=7.2 Hz, 3H), 1.32 (s, 9H).

### Example 175: Synthesis of Compound 375

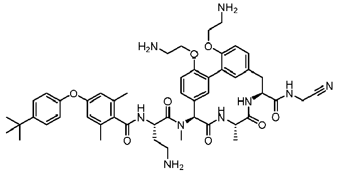

Compound 375 (formic acid salt) was prepared as a white solid from Compound **101-K** by utilizing methods analogous to those described in Example 174. LCMS (Method 5-95 AB, ESI): t_{R} = 0.621 min, [M + H]⁺ = 918.5; ¹H NMR (400 MHz, MeOH-*d*₄) δ 8.51 (brs, 1H), 7.43 (d, *J*=8.8 Hz, 2H), 7.31-7.25 (m, 2H), 7.19 (d, *J*=8.4 Hz, 1H), 7.12 (d, J=8.4 Hz, 1H), 6.96 - 6.91 (m, 3H), 6.84 (s, 1H), 6.70 (brs, 2H), 6.39 (s, 1H), 5.20-5.18 (m, 1H), 4.83-4.75 (m, 2H), 4.29-4.18 (m, 6H), 3.39-3.35 (m, 1H), 3.26-3.10 (m, 7H), 3.01 (s, 3H), 2.35-2.25 (m, 1H), 2.31 (s, 6H), 2.19-2.10 (m, 1H), 1.37 (d, J=6.8 Hz, 3H), 1.36 (s, 9H).

### Example 176: Synthesis of Compound 376

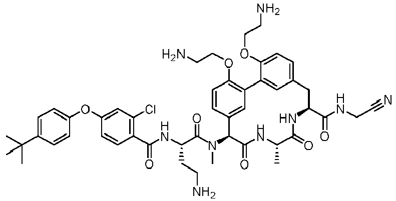

Compound 376 (formic acid salt) was prepared as a white solid from Compound **101-K** by utilizing methods analogous to those described in Example 174. LCMS (Method 5-95 AB, ESI): t_{R} = 0.621 min, [M + H]⁺ = 924.4; ¹H NMR (400 MHz, MeOH-*d*₄) δ 8.49 (brs, 2H), 7.50-7.46 (m, 3H), 7.29 (d, *J*=8.4 Hz, 1H), 7.23 (d, *J*=8.4 Hz, 1H), 7.16 (d, *J*=8.4 Hz, 1H), 7.09 (d, *J*=8.4 Hz, 1H), 7.02-6.95 (m, 4H), 6.89 (s, 1H), 6.81 (s, 1H), 6.31 (s, 1H), 5.19-5.13 (m, 1H), 4.80-4.73 (m, 2H), 4.25-4.15 (m, 6H), 3.36-3.32 (m, 1H), 3.21-3.07 (m, 7H), 2.91 (s, 3H), 2.34-2.23 (m, 1H), 2.17-2.08 (m, 1H), 1.35 (brs, 12H).

### Example 177: Synthesis of Compound 377

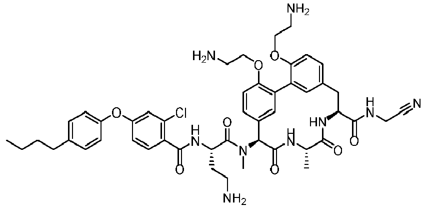

Compound 377 (formic acid salt) was prepared as a white solid from Compound **101-K** by utilizing methods analogous to those described in Example 174. LCMS (Method 5-95 AB, ESI): t_{R} = 0.760 min, [M + H]⁺ = 924.4; ¹H NMR (400 MHz, MeOH-*d*₄) δ 8.51 (brs, 1H), 7.48 (d, *J*=8.4 Hz, 1H), 7.30-7.22 (m, 3H), 7.15 (d, *J*=8.4 Hz, 1H), 7.10-6.94 (m, 6H), 6.81 (s, 1H), 6.38 (s, 1H), 6.31 (s, 1H), 5.18-5.13 (m, 1H), 4.79-4.73 (m, 2H), 4.24-4.16 (m, 6H), 3.45-3.40 (m, 1H), 3.18-3.06 (m, 7H), 2.93-2.88 (m, 3H), 2.62 (t, J=6.8 Hz, 2H), 2.33-2.22 (m, 1H), 2.17-2.07 (m, 1H), 1.61-1.58 (m, 2H), 1.43-1.33 (m, 5H), 0.96 (t, *J*=7.6 Hz, 3H).

### Example 178: Synthesis of Compound 378

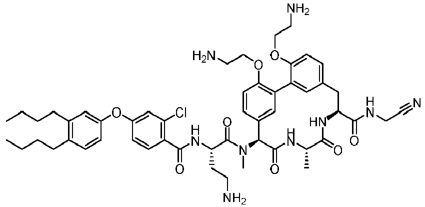

Compound 378 (formic acid salt) was prepared as a white solid from Compound **101-K** by utilizing methods analogous to those described in Example 174. LCMS (Method 5-95 AB, ESI): t_{R} = 0.825 min, [M + H]⁺ = 979.5; ¹H NMR (400 MHz, MeOH-*d*₄) δ 8.50 (brs, 3H), 7.49 (d, *J*=8.4 Hz, 1H), 7.30-6.80 (m, 11H), 6.32 (s, 1H), 5.20-5.15 (m, 1H), 4.85-4.75 (m, 2H), 4.25-4.15 (m, 4H), 4.20 (s, 2H), 3.26-3.00 (m, 8H), 2.92 (s, 3H), 2.65-2.55 (m, 4H), 2.32-2.20 (m, 1H), 2.20-2.09 (m, 1H), 1.60-1.50 (m, 4H), 1.50-1.25 (m, 7H), 1.00-0.90 (m, 6H).

### Example 179: Synthesis of Compound 379

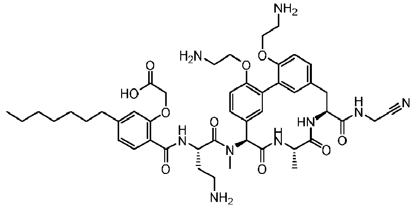

Compound 379 (formic acid salt) was prepared as a white solid from Compound **101-K** by utilizing methods analogous to those described in Example 53. LCMS (Method 5-95 AB, ESI): t_{R} = 0.730 min, [M + H]⁺ = 914.5; ¹H NMR (400 MHz, MeOH-*d*₄) δ 8.50 (brs, 2H), 7.78 (d, *J*=8.0 Hz, 1H), 7.39 (d, *J*=8.8 Hz, 1H), 7.23 (d, *J*=8.8 Hz, 1H), 7.15 (d, *J*=8.4 Hz, 1H), 7.08 (d, *J*=8.4 Hz, 1H), 6.90-6.80 (m, 4H), 6.38 (s, 1H), 5.11-5.09 (m, 2H), 4.51-4.47 (m, 2H), 4.38-4.34 (m, 1H), 4.28-4.24 (m, 4H), 4.20 (s, 2H), 3.40-3.35 (m, 1H), 3.22-3.09 (m, 7H), 2.87 (s, 3H), 2.67 (t, J=6.4 Hz, 2H), 2.32-2.26 (m, 2H), 1.68-1.58 (m, 3H), 1.39-1.26 (m, 10H), 0.90 (t, *J*=6.6 Hz, 3H).

### Example 180: Synthesis of Compound 380

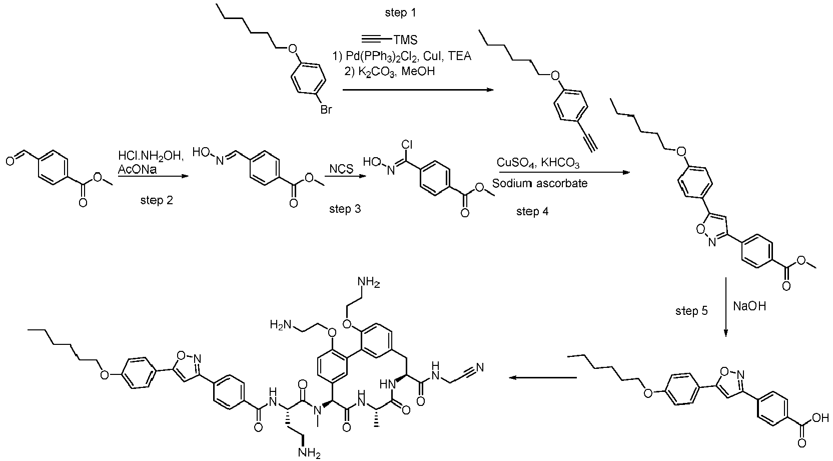

Step 1: Starting from 1-bromo-4-(hexyloxy)benzene, typical Sonogoshira and trimethylsilyl removal conditions (as described in Example 110) were followed to give 1-ethynyl-4-(hexyloxy)benzene as yellow oil.

Step 2: A mixture of methyl 4-formylbenzoate (4.0 g, 24.4 mmol), hydroxylamino hydrochloride (3.4 g, 48.8 mmol) and sodium acetate (4.0 g, 48.8 mmol) in MeOH/H₂O (21 mL, v/v=20/1) was stirred at 25 °C for 3 h. The volatiles were removed under reduced pressure and the residue was partitioned between H₂O (100 mL) and EtOAc (100 mL). The organic layer was dried over Na₂SO₄, concentrated and the residue was purified by silica gel chromatography, eluting with 0-30% EtOAc in petroleum ether, to give methyl (*E*)-4-((hydroxyimino)methyl)benzoate (2 g, 46% yield) as white solid.

Step 3: A mixture of methyl (*E*)-4-((hydroxyimino)methyl)benzoate (1.0 g, 5.6 mmol) and *N-*chlorosuccinimide (1.1 g, 8.4 mmol) in DMF (10 mL) was stirred at 25 °C for 4 h. The volatiles were removed and the residue was purified by silica gel chromatography, eluting with 0-20% EtOAc in petroleum ether, to give methyl (Z)-4-(chloro(hydroxyimino)methyl)benzoate (966 mg, 81% yield) as a white solid.

Step 4: A mixture of 1-ethynyl-4-(hexyloxy)benzene (from Step 1) (320 mg, 1.6 mmol), methyl (Z)-4-(chloro(hydroxyimino)methyl)benzoate (405 mg, 1.9 mmol), KHCO₃ (681 mg, 6.8 mmol), CuSO₄ (8 mg, 0.03 mmol) and sodium ascorbate (3.1 mg, 0.02 mmol) in 2-methyl-2-propanol (8 mL) was stirred at 45 °C for 1 h. The reaction mixture was diluted with H₂O (20 mL), which was extracted with EtOAc (2 × 30 mL). The combined organic layers were dried over Na₂SO₄, concentrated and the residue was purified by silica gel chromatography, eluting with 0-60% DCM in petroleum ether, to give methyl 4-(5-(4-(hexyloxy)phenyl)isoxazol-3-yl)benzoate (120 mg, 20% yield) as a white solid. ¹H NMR (400MHz, CDCl₃) δ 8.16 (d, *J*=8.4 Hz, 2H), 7.95 (d, *J*=8.4 Hz, 2H), 7.78 (d, *J*=8.4 Hz, 2H), 7.01 (d, *J*=8.4 Hz, 2H), 6.75 (s, 1H), 4.03 (t, *J*=6.4 Hz, 2H), 3.97 (s, 3H), 1.86-1.79 (m, 2H), 1.49-1.26 (m, 6H), 0.93 (t, *J*=6.4 Hz, 3H).

Step 5: Typical ester hydrolysis conditions (NaOH, MeOH/H₂O, described in Example H) were applied to methyl 4-(5-(4-(hexyloxy)phenyl)isoxazol-3-yl)benzoate to give 4-(5-(4-(hexyloxy)phenyl)isoxazol-3-yl)benzoic acid as a white solid.

Compound 380 (formic acid salt) was prepared as a white solid from Compound **101-K** and 4-(5-(4-(hexyloxy)phenyl)isoxazol-3-yl)benzoic acid by utilizing methods analogous to those described in Example G. LCMS (Method 5-95 AB, ESI): t_{R} = 0.793 min, [M + H]⁺ = 985.6; ¹H NMR (400 MHz, MeOH-*d*₄) δ 8.49 (brs, 2H), 8.10-7.98 (m, 4H), 7.85 (d, *J*=8.4 Hz, 2H), 7.33 (d, *J*=8.4 Hz, 1H), 7.23 (d, *J*=8.4 Hz, 1H), 7.16 (d, *J*=8.4 Hz, 2H), 7.10-7.07 (m, 3H), 6.89 (brs, 1H), 6.80 (s, 1H), 6.37 (s, 1H), 5.21-5.15 (m, 1H), 4.85-4.75 (m, 2H), 4.33-4.15 (m, 6H), 4.07 (t, *J*=6.0 Hz, 2H), 3.29-2.97 (m, 8H), 2.89 (s, 3H), 2.38-2.29 (m, 1H), 2.24-2.17 (m, 1H), 1.86-1.78 (m, 2H), 1.56-1.32 (m, 9H), 0.94 (t, *J*=6.4 Hz, 3H).

### Examples 181 and 182: Synthesis of Compounds 381 and 382

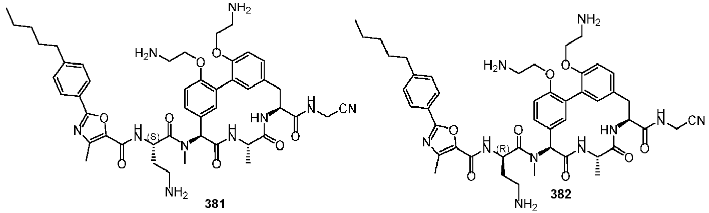

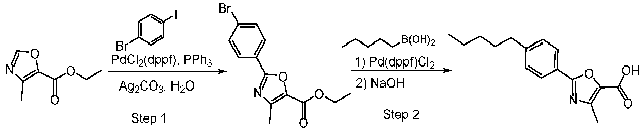

Step 1: A mixture of ethyl 4-methyloxazole-5-carboxylate (600 mg, 4.25 mmol), 1-bromo-4-iodobenzene (1.44 g, 5.10 mmol), Pd(dppf)Cl₂ (158 mg, 0.21 mmol), PPh₃ (112 mg, 0.43 mmol) and Ag₂CO₃ (2.34 g, 8.50 mmol) in H₂O (20 mL) was stirred at 70 °C for 16 h under N₂. The reaction mixture was extracted with DCM (3 x 20 mL). The combined organic layers were dried over Na₂SO₄, concentrated and the residue was purified by silica gel chromatography, eluting with 0-10% EtOAc in petroleum ether, to give ethyl 2-(4-bromophenyl)-4-methyloxazole-5-carboxylate (1.0 g, 80% yield) as a white solid. 1H NMR (400 MHz, CDCl₃) δ 7.99 (d, J=8.4 Hz, 2H), 7.62 (d, J=8.4 Hz, 2H), 4.42 (q, *J*=7.2 Hz, 2H), 2.54 (s, 3H), 1.42 (t, J=7.2 Hz, 3H).

Step 2: Starting from ethyl 2-(4-bromophenyl)-4-methyloxazole-5-carboxylate, typical Suzuki and ester hydrolysis conditions, as described in Example H, were followed to give 4-methyl-2-(4-pentylphenyl)oxazole-5-carboxylic acid as a white solid.

The title compounds (formic acid salts) were prepared as white solids using **101-K** and 4-methyl-2-(4-pentylphenyl)oxazole-5-carboxylic acid by utilizing methods analogous to those described in Example G, with the two epimers separated under achiral HPLC conditions.
Compound 381: LCMS (Method 5-95 AB, ESI): t_{R} = 0.738 min, [M + H]⁺ = 893.5; ¹H NMR (400 MHz, MeOH-*d*₄) δ 8.47 (brs, 3H), 8.09 (d, *J*=8.4 Hz, 2H), 7.40 (d, *J*=8.4 Hz, 2H), 7.31 (d, *J*=8.4 Hz, 1H), 7.24 (d, *J*=8.4 Hz, 1H), 7.13-7.07 (m, 2H), 6.89 (s, 1H), 6.81 (s, 1H), 6.31 (s, 1H), 5.17-5.11 (m, 2H), 4.54-4.50 (m, 1H), 4.28-4.14 (m, 4H), 4.19 (s, 2H), 3.26-3.04 (m, 8H), 2.88 (s, 3H), 2.71 (t, *J*=8.0 Hz, 2H), 2.54 (s, 3H), 2.37-2.31 (m, 1H), 2.21-2.16 (m, 1H), 1.73-1.63 (m, 3H), 1.40-1.29 (m, 6H), 0.92 (t, *J*=6.8 Hz, 3H).

Compound 382: LCMS (Method 5-95 AB, ESI): t_{R} = 0.747 min, [M + H]⁺ = 893.8; ¹H NMR (400 MHz, MeOH-*d*₄) δ 8.49 (brs, 2H), 8.08 (d, *J*=8.4 Hz, 2H), 7.37 (d, *J*=8.4 Hz, 2H), 7.30 (d, *J*=8.4 Hz, 1H), 7.23 (d, *J*=8.4 Hz, 1H), 7.18 (d, *J*=8.4 Hz, 1H), 7.08 (d, *J*=8.4 Hz, 1H), 6.89 (s, 1H), 6.80 (s, 1H), 6.30 (s, 1H), 5.28-5.22 (m, 2H), 4.59-4.57 (m, 1H), 4.28-4.15 (m, 4H), 4.18 (s, 2H), 3.27-3.05 (m, 8H), 2.88 (s, 3H), 2.74-2.69 (m, 2H), 2.51 (s, 3H), 2.30-2.28 (m, 1H), 2.18-2.17 (m, 1H), 1.71-1.63 (m, 3H), 1.43-1.34 (m, 6H), 0.91 (t, *J*=6.6 Hz, 3H).

### Example 183: Synthesis of Compound 383

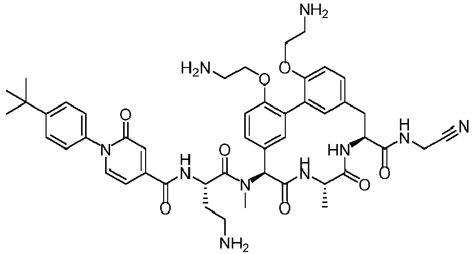

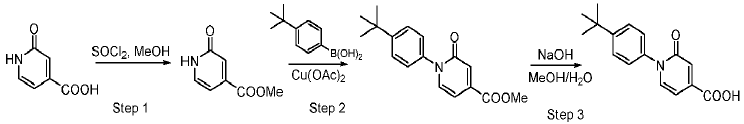

Step 1: A mixture of 2-oxo-1,2-dihydropyridine-4-carboxylic acid (1.5 g, 10.8 mmol) and SOCl₂ (3.1 mL, 43.2 mmol) in MeOH (30 mL) was stirred at 80 °C for 3 h. The volatiles were removed under reduced pressure and the residue was taken up by EtOAc (50 mL), which was washed with saturated aqueous Na₂CO₃ solution (2 x 30 mL). The organic layer was dried over Na₂SO₄ and concentrated to give methyl 2-oxo-1,2-dihydropyridine-4-carboxylate (540 mg, 33% yield) as a white solid.

Step 2: A mixture of methyl 2-oxo-1,2-dihydropyridine-4-carboxylate (540 mg, 3.5 mmol), Cu(OAc)2 (128 mg, 0.71 mmol) and (4-(*tert*-butyl)phenyl)boronic acid (816 mg, 4.6 mmol) in DCM (20 mL) and pyridine (2 mL) was stirred at 25 °C for 24 h. The volatiles were removed under reduced pressure and the residue was taken up by EtOAc (50 mL), which was washed with brine (2 x 50 mL). The organic layer was dried over Na₂SO₄, concentrated and the residue was purified by silica gel chromatography, eluting with 0-5% MeOH in DCM, to give methyl 1-(4-(*tert*-butyl)phenyl)-2-oxo-1,2-dihydropyridine-4-carboxylate (440 mg, 44% yield) as a brown solid. LCMS (Method 5-95 AB, ESI): t_{R} = 0.863 min, [M + H]⁺ = 285.9.

Step 3: Typical ester hydrolysis conditions (NaOH, MeOH/H₂O, as described in Example H) were applied to methyl 1-(4-(*tert*-butyl)phenyl)-2-oxo-1,2-dihydropyridine-4-carboxylate to give 1-(4-(*tert*-butyl)phenyl)-2-oxo-1,2-dihydropyridine-4-carboxylic acid as a yellow solid.

Compound 383 (formic acid salt) was prepared as a white solid from Compound **101-K** and 1-(4-(*tert*-butyl)phenyl)-2-oxo-1,2-dihydropyridine-4-carboxylic acid by utilizing methods analogous to those described in Example G. LCMS (Method 5-95 AB, ESI): t_{R} = 0.676 min, [M + H]⁺ = 891.8; ¹H NMR (400 MHz, MeOH-*d*₄) δ 8.49 (brs, 1H), 7.67 (d, *J*=8.4 Hz, 1H), 7.56 (d, *J*=8.4 Hz, 2H), 7.30-7.19 (m, 3H), 7.18-7.10 (m, 3H), 6.99 (brs, 1H), 6.85 (brs, 2H), 6.68 (brs, 1H), 5.11-5.02 (m, 1H), 4.85-4.78 (m, 2H), 4.37-4.18 (m, 6H), 3.29-3.08 (m, 8H), 2.92 (s, 3H), 2.34-2.19 (m, 2H), 1.38 (brs, 12H).

### Example 184: Synthesis of Compound 384

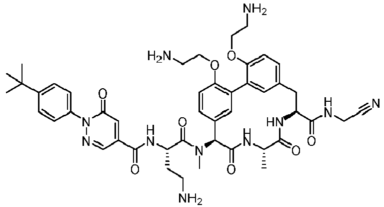

Compound 384 (formic acid salt) was prepared as a white solid from Compound **101-K** by utilizing methods analogous to those described in Example 183. LCMS (Method 5-95 AB, ESI): t_{R} = 0.698 min, [M + H]⁺ = 892.5; ¹H NMR (400 MHz, MeOH-*d*₄) δ 8.51 (brs, 2H), 8.27 (s, 1H), 7.55-7.48 (m, 3H), 7.35-7.19 (m, 5H), 7.16 (d, *J*=8.4 Hz, 1H), 6.86 (d, J=2.0 Hz, 1H), 6.72 (s, 1H), 6.62 (brs, 1H), 5.17-5.07 (m, 1H), 4.85-4.75 (m, 2H), 4.40-4.20 (m, 4H), 4.23 (s, 2H), 3.25-3.09 (m, 8H), 2.95 (s, 3H), 2.37-2.18 (m, 2H), 1.40 (brs, 12H).

### Example 185: Synthesis of Compound 385

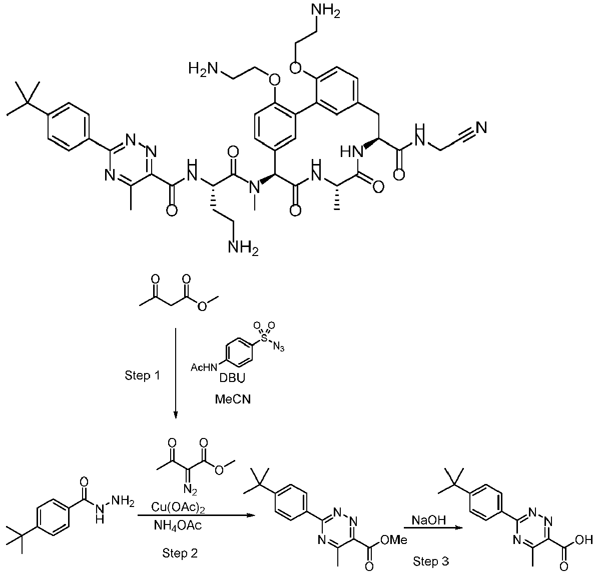

Step 1: To a solution of methyl 3-oxobutanoate (5.0 g, 43 mmol) and DBU (9.8 g, 65 mmol) in acetonitrile (40 mL) was added 4-acetamido benzenesulfonylazide (15.5 g, 65 mmol) at 0 °C and the mixture was stirred at 20 °C for 1 h. After filtration, the filtrate was concentrated and the residue was partitioned with saturated aqueous NaHCO₃ solution and EtOAc (50 mL each). The organic layer was dried over Na₂SO₄, concentrated and the residue was purified via silica gel chromatography, eluting with 4% EtOAc in petroleum ether, to give methyl 2-diazo-3-oxobutanoate (2.4 g, 39% yield) as a colorless oil.

Step 2: A sealed tube containing 4-(*tert*-butyl)benzohydrazide (425 mg, 2.2 mmol), Cu(OAc)₂ (128 mg, 0.70 mmol) and NH₄OAc (542 mg, 7.0 mmol) in 1,2-dichloroethane (2 mL) was heated under microwave irradiation at 80 °C for 10 min. The mixture was filtered through silica gel, washed with 50% EtOAc in petroleum ether, and concentrated. Methyl 2-diazo-3-oxobutanoate (200 mg, 1.4 mmol) and AcOH (2 mL) were added to the residue and the resulting mixture was heated under microwave irradiation at 110 °C for 5 min. The volatiles were removed under reduced pressure and the residue was re-dissolved with EtOAc (50 mL), which was washed with saturated aqueous NaHCO₃ and brine (50 mL each). The organic layer was dried over Na₂SO₄, concentrated and the residue was purified via silica gel chromatography, eluting with 5-20% EtOAc in petroleum ether, to give methyl 3-(4-(*tert*-butyl)phenyl)-5-methyl-1,2,4-triazine-6-carboxylate (20 mg, 5% yield) as a yellow solid. ¹H NMR (400 MHz, CDCl₃) δ 8.53 (d, *J* =8.4 Hz, 2H), 7.57 (d, *J* =8.4 Hz, 2H), 4.08 (s, 3H), 2.88 (s, 3H), 1.38 (s, 9H).
Step 3: Typical ester hydrolysisconditions (NaOH, MeOH/H₂O, described in Example H) was applied to methyl 3-(4-(*tert*-butyl)phenyl)-5-methyl-1,2,4-triazine-6-carboxylate to give 3-(4-(*tert*-butyl)phenyl)-5-methyl-1,2,4-triazine-6-carboxylic acid as a yellow solid. LCMS (Method 5-95 AB, ESI): t_{R} = 0.875 min, [M + H]⁺ = 271.9.

Compound 385 (formic acid salt) was prepared as a white solid from Compound **101-K** and 3-(4-(*tert*-butyl)phenyl)-5-methyl-1,2,4-triazine-6-carboxylic acid by utilizing methods analogous to those described in Example G. LCMS (Method 5-95 AB, ESI): t_{R} = 0.720 min, [M + H]⁺ = 891.5; ¹H NMR (400 MHz, MeOH-*d*₄) δ 8.52-8.44 (m, 4H), 7.63 (d, *J*=8.4 Hz, 2H), 7.32 (d, *J*=8.4 Hz, 1H), 7.23 (d, *J*=8.0 Hz, 1H), 7.15 (d, *J*=8.0 Hz, 1H), 7.08 (d, *J*=8.0 Hz, 1H), 6.89 (s, 1H), 6.79 (s, 1H), 6.37 (s, 1H), 5.25-5.21 (m, 1H), 4.82-4.75 (m, 2H), 4.22-4.15 (m, 6H), 3.20-3.09 (m, 7H), 2.95 (s, 3H), 2.89 (s, 3H), 2.37-2.17 (m, 2H), 1.39 (s, 9H), 1.36 (d, *J*=6.4 Hz, 3H).

### Example 186: Synthesis of Compound 386

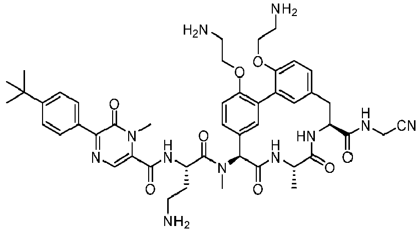

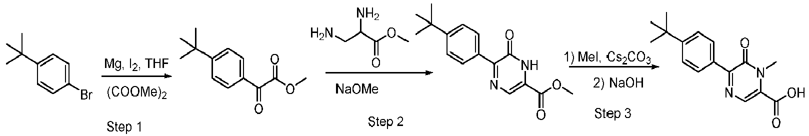

Step 1: To a solution of 1-bromo-4-(*tert*-butyl)benzene (3.0 g, 14.1 mmol) in THF (20 mL) at 0 °C was added Mg (741 mg, 28.2 mmol) and I₂ (357 mg, 1.41 mmol). The mixture was gradually warmed up to 75 °C while stirring and then stirred at that temperature for 3 h. The above mixture was then added dropwise to a solution of di-methyl oxalate in THF (20 mL) at -78 °C and the resulting mixture was stirred at the same temperature for 1 h, then gradually warmed up to 20 °C while stirring and stirred for another 12 h. The reaction was quenched with saturated aqueous NH₄Cl solution (20 mL), which was extracted with EtOAc (3 x 30 mL). The combined organic layers were washed with brine (30 mL), dried over Na₂SO₄, concentrated and the residue was purified by silica gel chromatography, eluting with 0-5% EtOAc in petroleum ether, to methyl 2-(4-(*tert*-butyl)phenyl)-2-oxoacetate (900 mg, 48% yield). ¹H NMR (400 MHz, CDCl₃) δ 7.96 (d, *J*=8.4 Hz, 2H), 7.54 (d, *J*=8.4 Hz, 2H), 3.98 (s, 3H), 1.36 (s, 9H).

Step 2: A mixture of methyl 2-(4-(*tert*-butyl)phenyl)-2-oxoacetate (900 mg, 4.1 mmol), methyl 2,3-diaminopropanoate (1.45 g, 12.3 mmol) and NaOMe (1.1 g, 20.5 mmol) in MeOH (30 mL) was stirred at 70 °C for 12 h. The volatiles were removed and the residue was purified by reverse-phase HPLC to give methyl 5-(4-(*tert*-butyl)phenyl)-6-oxo-1,6-dihydropyrazine-2-carboxylate (80 mg, 6.8% yield) as a yellow solid. ¹H NMR (400 MHz, CDCl₃) δ 8.28 (d, *J*=8.4 Hz, 2H), 8.13 (s, 1H), 7.50 (d, *J*=8.4 Hz, 2H), 3.97 (s, 3H), 1.36 (s, 9H).

Step 3: Starting from methyl 5-(4-(*tert*-butyl)phenyl)-6-oxo-1,6-dihydropyrazine-2-carboxylate, typical alkylation (as described in Example 21) and ester hydrolysis conditions (as described in Example H) were followed to give 5-(4-(*tert*-butyl)phenyl)-1-methyl-6-oxo-1,6-dihydropyrazine-2-carboxylic acid as a yellow solid.

Compound 386 (formic acid salt) was prepared as a white solid from Compound **101-K** and 5-(4-(tert-butyl)phenyl)-1-methyl-6-oxo-1,6-dihydropyrazine-2-carboxylic acid by utilizing methods analogous to those described in Example G. LCMS (Method 5-95 AB, ESI): t_{R} = 0.716 min, [M + H]⁺ = 906.6; ¹H NMR (400 MHz, MeOH-*d*₄) δ 8.49 (brs, 2H), 8.38-8.34 (m, 3H), 7.56-7.52 (m, 2H), 7.30-7.22 (m, 2H), 7.09-7.04 (m, 2H), 6.88 (s, 1H), 6.81 (s, 1H), 6.33 (s, 1H), 5.20-5.16 (m, 1H), 4.81-4.77 (m, 2H), 4.22-4.16 (m, 6H), 3.69 (s, 3H), 3.40-3.35 (m, 1H), 3.18-3.06 (m, 7H), 2.88 (s, 3H), 2.36-2.33 (m, 1H), 2.14-2.10 (m, 1H), 1.39 (s, 9H), 1.36 (d, *J*=6.8 Hz, 3H).

### Example 187: Synthesis of Compound 387

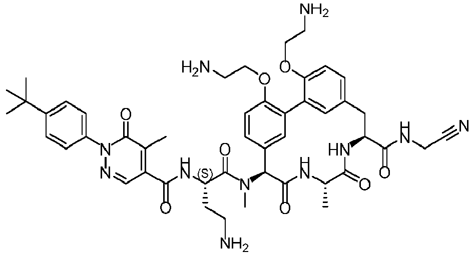

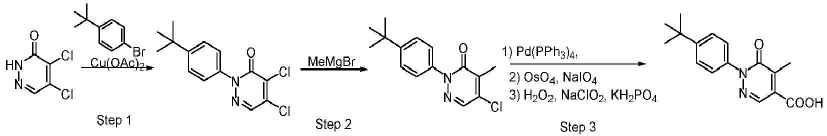

Step 1: Typical Chan-Lam conditions (as described in Example 183) were applied to 4,5-dichloropyridazin-3(2*H*)-one to give 2-(4-(*tert*-butyl)phenyl)-4,5-dichloropyridazin-3(2*H*)-one as a white solid.

Step 2: To a solution of 2-(4-(*tert*-butyl)phenyl)-4,5-dichloropyridazin-3(2*H*)-one (1.0 g, 3.4 mmol) in THF (30 mL) was added MeMgBr (3N in Et₂O, 9.0 mL) dropwise at 0 °C and the reaction was stirred at the same temperature for 2 h. The reaction was quenched with saturated aqueous NH₄Cl solution (40 mL), which was extracted with EtOAc (3 x 40 mL). The combined organic layers were washed with brine (100 mL), dried over Na₂SO₄, concentrated and the residue was purified by silica gel chromatography, eluting with 25% EtOAc in petroleum ether, to give 2-(4-(*tert*-butyl)phenyl)-5-chloro-4-methylpyridazin-3(2*H*)-one (350 mg, 38% yield) as a off-white solid. ¹H NMR (400 MHz, CDCl₃) δ 8.01 (s, 1H), 7.56 (d, *J*=7.6 Hz, 2H), 7.25 (d, *J*=7.6 Hz, 2H), 2.39 (s, 3H), 1.38 (s, 9H).
Step 3: 1-(4-(*tert*-Butyl)phenyl)-5-methyl-6-oxo-1,6-dihydropyridazine-4-carboxylic acid was prepared as a white solid from 2-(4-(*tert*-butyl)phenyl)-5-chloro-4-methylpyridazin-3(2*H*)-one by utilizing methods analogous to those described in Example 173. LCMS (Method 5-95 AB, ESI): t_{R} = 0.886, [M + H]⁺ = 286.9

Compound 387 (formic acid salt) was prepared as a white solid from Compound **101-K** and 1-(4-(*tert*-butyl)phenyl)-5-methyl-6-oxo-1,6-dihydropyridazine-4-carboxylic acid by utilizing methods analogous to those described in Example G. LCMS (Method 5-95 AB, ESI): t_{R} = 0.695 min, [M + H]⁺ = 906.6; ¹H NMR (400 MHz, MeOH-*d*₄) δ 8.46 (brs, 2H), 8.08 (s, 1H), 7.64 (d, *J*=8.4 Hz, 2H), 7.36 (d, *J*=8.4 Hz, 2H), 7.30-7.21 (m, 2H), 7.15-7.04 (m, 2H), 6.88 (s, 1H), 6.80 (s, 1H), 6.30 (s, 1H), 5.20-5.15 (m, 1H), 4.78-4.74 (m, 2H), 4.24-4.18 (m, 4H), 4.20 (s, 2H), 3.40-3.35 (m, 1H), 3.20-3.11 (m, 7H), 2.90 (s, 3H), 2.44 (s, 3H), 2.31-2.26 (m, 1H), 2.15-2.08 (m, 1H), 1.40 (s, 9H), 1.36 (d, *J*=6.4 Hz, 3H).

### Example 188: Synthesis of Compound 388

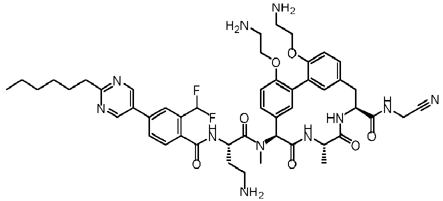

Compound 388 (formic acid salt) was prepared as a white solid from **101-K** by utilizing methods analogous to those described in Example L and Example 4. LCMS (Method 5-95 AB, ESI): t_{R} = 0.788 min, [M + H]⁺ = 955.7; ¹H NMR (400 MHz, MeOH-*d*₄) δ 9.04 (s, 2H), 8.50 (brs, 3H), 8.07 (s, 1H), 7.98 (d, *J*=8.0 Hz, 1H), 7.79 (d, *J*=8.0 Hz, 1H), 7.33-7.07 (m, 5H), 6.90 (s, 1H), 6.80 (s, 1H), 6.36 (s, 1H), 5.22-5.18 (m, 1H), 4.85-4.78 (m, 2H), 4.23-4.19 (m, 7H), 3.47-3.40 (m, 1H), 3.25-3.05 (m, 7H), 3.01 (t, *J*=7.6 Hz, 2H), 2.93 (s, 3H), 2.40-2.25 (m, 1H), 2.20-2.05 (m, 1H), 1.88-1.84 (m, 2H), 1.50-1.25 (m, 9H), 0.92 (t, *J*=6.4 Hz, 3H).

### Example 189: Synthesis of Compound 389

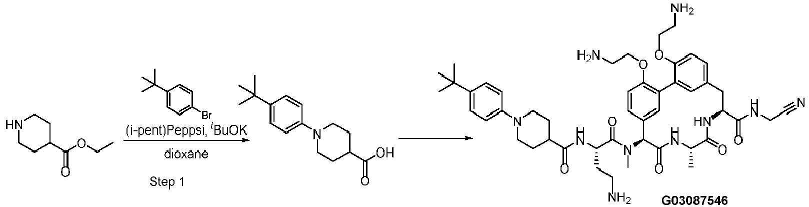

Step 1: A mixture of ethyl piperidine-4-carboxylate (200 mg, 1.27 mmol), 1-bromo-4-(*tert-*butyl)benzene (407 mg, 1.91 mmol), dichloro[1,3-bis(2,6-di-3-pentylphenyl)imidazol-2-ylidene](3-chloropyridyl)palladium(II) (50 mg, 0.06mmol) and *t*-BuOK (357 mg, 3.18 mmol) in 1,4-dioxane (5 mL) was stirred at 110 °C for 16 h under N₂. The volatiles were removed under reduced pressureand the residue was purified by reverse-phase HPLC, eluting with acetonitrile 17-47% / 0.225% formic acid in water, to afford 1-(4-(*tert*-butyl)phenyl)piperidine-4-carboxylic acid (15 mg, 4.5% yield) as a white solid. LCMS (Method 5-95 AB, ESI): t_{R} = 0.723 min, [M + H]⁺ = 262.2.

Compound 389 (formic acid salt) was prepared as a white solid from Compound **101-K** and 1-(4-(*tert*-butyl)phenyl)piperidine-4-carboxylic acid by utilizing methods analogous to those described in Example G. LCMS (Method 5-95 AB, ESI): t_{R} = 0.643 min, [M + H]⁺ = 881.5; ¹H NMR (400 MHz, MeOH-*d*₄) δ 8.37 (brs, 3H), 7.29-7.22 (m, 4H), 7.15-6.99 (m, 3H), 6.95-6.92 (m, 2H), 6.86-6.77 (m, 1H), 6.36-6.29 (m, 1H), 4.96-4.95 (m, 1H), 4.80-4.76 (m, 2H), 4.26-4.08 (m, 6H), 3.70-3.64 (m, 2H), 3.33-3.32 (m, 1H), 3.21-3.04 (m, 7H), 2.86-2.83 (m, 3H), 2.73-2.69 (m, 2H), 2.47-2.43 (m, 1H), 2.21-2.17 (m, 1H), 2.05-2.02 (m, 1H), 2.00-1.82 (m, 4H), 1.44-1.35 (m, 3H), 1.28 (s, 9H).

### Example 190: Synthesis of Compound 390

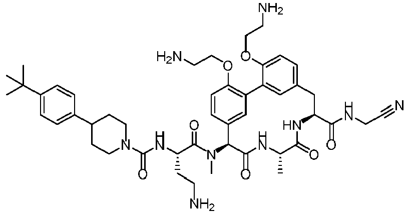

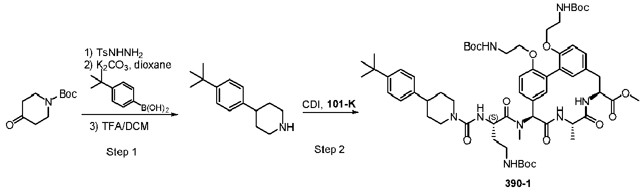

Step 1: 4-(4-(*tert*-Butyl)phenyl)piperidine was prepared as a white solid from *tert*-butyl 4-oxopiperidine-1-carboxylate by utilizing methods analogous to those described in Example 17. LCMS (Method 5-95 AB, ESI): t_{R} = 0.731, [M + H]⁺ = 218.2

Step 2: A mixture of Compound **101-K** (100 mg, 0.11 mmol) and CDI (19 mg, 0.11 mmol) in DCM (2 mL) was stirred at 20 °C for 16 h, followed by the addition of 4-(4-(*tert*-butyl)phenyl)piperidine (47 mg, 0.22 mmol). The resulting mixture was stirred for another 24 h. The reaction mixture was diluted with DCM (30 mL), which was washed with brine (2 x 30 mL). The organic layer was dried over Na₂SO₄, concentrated and the residue was purified by preparatory-TLC to give compound 390-1 (50 mg, 40% yield) as a white solid. LCMS (Method 5-95 AB, ESI): t_{R} = 1.061, [M + H]⁺ = 1157.9

Compound 390 (formic acid salt) was prepared as a white solid from compound 390-1 by utilizing methods analogous to those described in Example G. LCMS (Method 5-95 AB, ESI): t_{R} = 0.725 min, [M + H]⁺ = 881.6; ¹H NMR (400 MHz, MeOH-*d*₄) δ 7.37-7.33 (m, 3H), 7.19-7.15 (m, 3H), 7.11-7.06 (m, 2H), 6.89 (s, 1H), 6.82 (s, 1H), 6.31 (s, 1H), 4.82-4.75 (m, 1H), 4.23-4.19 (m, 8H), 3.26-3.17 (m, 4H), 3.14-3.05 (m, 4H), 2.98-2.91 (m, 4H), 2.88 (s, 3H), 2.77-2.71 (m, 2H), 2.18-2.16 (m, 1H), 1.89-1.86 (m, 2H), 1.66-1.60 (m, 2H), 1.43-1.34 (m, 3H), 1.30 (s, 9H).

### Examples 191 and 192: Synthesis of Compounds 391 and 392

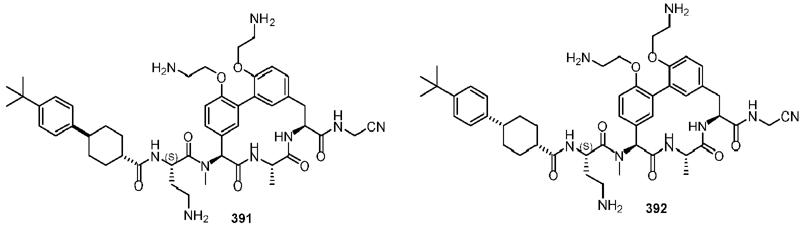

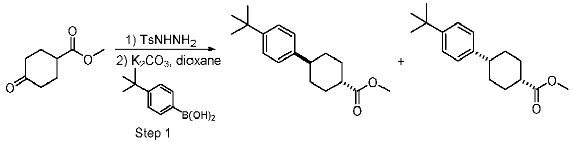

Step 1: Starting from methyl 4-oxocyclohexane-1-carboxylate, the methods analogous to those described in Example 17 were followed to give methyl (*trans*)-4-(4-(*tert*-butyl)phenyl)cyclohexane-1 - carboxylate and methyl (*cis*)-4-(4-(*tert*-butyl)phenyl)cyclohexane-1-carboxylate after preparatory-TLC separation.

Methyl (*trans*)-4-(4-(*tert*-butyl)phenyl)cyclohexane-1-carboxylate: ¹H NMR (400 MHz, CDCl₃) δ 7.33 (d, J=8.2 Hz, 2H), 7.15 (d, J=8.2 Hz, 2H), 3.70 (s, 3H), 2.54-2.48 (m, 1H), 2.40-2.34 (m, 1H), 2.14-2.08 (m, 2H), 2.03-1.96 (m, 2H), 1.66-1.57 (m, 2H), 1.54-1.43 (m, 2H), 1.32 (s, 9H). Methyl (*cis*)-4-(4-(*tert*-butyl)phenyl)cyclohexane-1-carboxylate: ¹H NMR (400 MHz, CDCl₃) δ 7.31 (d, J=8.4 Hz, 2H), 7.14 (d, J=8.4 Hz, 2H), 3.73 (s, 3H), 2.72 (brs, 1H), 2.58-2.49 (m, 1H), 2.29-2.22 (m, 2H), 1.84-1.76 (m, 2H), 1.70 - 1.62 (m, 4H), 1.32 (s, 9H).

Compound 391 (formic acid salt) and Compound 392 (formic acid salt) were each prepared as a white solid from **101-K** and methyl (*trans*)-4-(4-(*tert*-butyl)phenyl)cyclohexane-1-carboxylate or methyl (*cis*)-4-(4-(*tert*-butyl)phenyl)cyclohexane-1-carboxylate, respectively, by utilizing methods analogous to those described in Example G.

Compound 391: LCMS (Method 5-95 AB, ESI): t_{R} = 0.730 min, [M + H]⁺ = 880.5; ¹H NMR (400 MHz, MeOH-*d*₄) δ 7.32-7.28 (m, 3H), 7.27-7.22 (m, 1H), 7.21-7.16 (m, 1H), 7.16-7.10 (m, 3H), 6.90 (brs, 1H), 6.82 (brs, 1H), 6.32 (s, 1H), 4.85-4.75 (m, 3H), 4.33-4.12 (m, 6H), 3.26-2.96 (m, 8H), 2.83 (s, 3H), 2.54-2.35 (m, 3H), 2.22-2.16 (m, 1H), 2.08-2.01 (m, 1H), 1.98-1.90 (m, 4H), 1.73-1.49 (m, 5H), 1.36 (t, *J*=6.8 Hz, 2H), 1.30 (s, 9H).

Compound 392: LCMS (Method 5-95 AB, ESI): t_{R} = 0.738 min, [M + H]⁺ = 880.5; ¹H NMR (400 MHz, MeOH-*d*₄) δ 8.49 (brs, 2H), 7.34-7.28 (m, 3H), 7.27-7.21 (m, 1H), 7.19-7.13 (m, 3H), 7.09 (d, *J*=8.4 Hz, 1H), 6.89 (d, *J*=2.0 Hz, 1H), 6.82 (d, *J*=2.0 Hz, 1H), 6.29 (s, 1H), 4.98-4.95 (m, 1H), 4.81-4.76 (m, 2H), 4.28-4.16 (m, 6H), 3.30-3.09 (m, 6H), 3.07-2.98 (m, 2H), 2.85 (s, 3H), 2.67-2.61 (m, 2H), 2.29-2.13 (m, 1H), 2.10-1.88 (m, 5H), 1.76-1.73 (m, 4H), 1.35 (d, *J*=6.8 Hz, 3H), 1.29 (s, 9H).

### Example 193: Synthesis of Compound 393

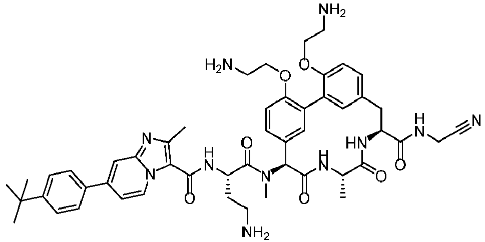

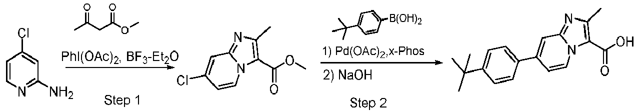

Step 1: A mixture of 4-chloropyridin-2-amine (500 mg, 3.9 mmol), methyl 3-oxobutanoate (542 mg, 4.7 mmol), PhI(OAc)₂ (1.5 g, 4.7 mmol) and BF₃·Et₂O (0.1 mL, 0.78mmol) in toluene (20 mL) was stirred at 110 °C for 24 h. The volatiles were removed under reduced pressure and the residue was partitioned between EtOAc and H₂O (50 mL each). The organic layer was dried over Na₂SO₄, concentrated and the residue was purified via silica gel chromatography, eluting with 20% EtOAc in petroleum ether, to give methyl 7-chloro-2-methylimidazo[1,2-*a*]pyridine-3-carboxylate (280 mg, 32% yield) as a white solid. ¹H NMR (400 MHz, CDCl₃) δ 9.22 (d, *J*=8.8 Hz, 1H), 7.59 (d, *J*=2.4 Hz, 1H), 6.95 (dd, *J*=8.8, 2.4 Hz, 1H), 3.95 (s, 1H), 2.69 (s, 1H).

Step 2: Starting from methyl 7-chloro-2-methylimidazo[1,2-*a*]pyridine-3-carboxylate, typical Suzuki and ester hydrolysis conditions, analogous to those described in Example H, were followed to give 7-(4-(*tert*-butyl)phenyl)-2-methylimidazo[1,2-*a*]pyridine-3-carboxylic acid as a white solid. LCMS (Method 5-95 AB, ESI): t_{R} = 0.786, [M + H]⁺ = 308.9

Compound 393 (formic acid salt) was prepared as a white solid from Compound **101-K** and 7-(4-(*tert*-butyl)phenyl)-2-methylimidazo[1,2-*a*]pyridine-3-carboxylic acid by utilizing methods analogous to those described in Example G. LCMS (Method 5-95 AB, ESI): t_{R} = 0.704 min, [M + H]⁺ = 928.7; ¹H NMR (400 MHz, MeOH-*d*₄) δ 9.19 (d, *J*=8.0 Hz, 1H), 7.77 (d, *J*=8.0 Hz, 1H), 7.72 (d, *J*=8.0 Hz, 2H), 7.56 (d, *J*=8.0 Hz, 2H), 7.43 (d, *J*=8.0 Hz, 1H), 7.31 (d, *J*=8.0 Hz, 1H), 7.24 (d, *J*=8.4 Hz, 1H), 7.16 (d, *J*=8.4 Hz, 1H), 7.08 (d, *J*=8.0 Hz, 1H), 6.90 (d, *J*=2.0 Hz, 1H), 6.81 (brs, 1H), 6.37 (s, 1H), 5.28-5.25 (m, 1H), 4.81-4.75 (m, 2H), 4.22-4.15 (m, 6H), 3.34-3.10 (m, 8H), 2.93 (s, 3H), 2.70 (s, 3H), 2.38-2.30 (m, 1H), 2.19-2.13 (m, 1H), 1.36 (brs, 12H).

### Example 194: Synthesis of Compound 394

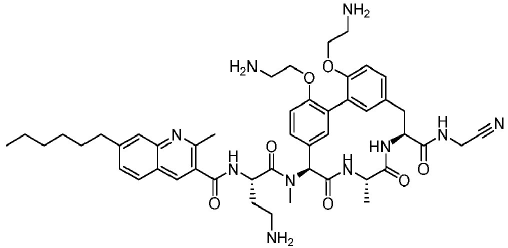

Compound 394 (formic acid salt) was prepared as a white solid from Compound **101-K** by utilizing methods analogous to those described in Example H. LCMS (Method 5-95 AB, ESI): t_{R} = 0.692 min, [M + H]⁺ = 891.5; ¹H NMR (400 MHz, MeOH-*d*₄) δ 8.48 (brs, 2H), 8.39 (s, 1H), 7.91 (d, *J*=8.4 Hz, 1H), 7.79 (s, 1H), 7.53 (d, *J*=8.4 Hz, 1H), 7.34 (d, *J*=8.4 Hz, 1H), 7.24 (d, *J*=8.4 Hz, 1H), 7.19 (d, *J*=8.4 Hz, 1H), 7.10 (d, *J*=8.4 Hz, 1H), 6.92 (d, *J*=2.2 Hz, 1H), 6.81 (s, 1H), 6.40 (s, 1H), 5.24-5.17 (m, 1H), 4.84-4.77 (m, 2H), 4.31-4.14 (m, 6H), 3.40-3.35 (m, 1H), 3.27-3.08 (m, 7H), 2.99 (s, 3H), 2.87 (t, *J*=7.5 Hz, 3H), 2.78 (s, 3H), 2.34-2.29 (m, 1H), 2.23-2.14 (m, 1H), 1.81-1.71 (m, 2H), 1.51-1.26 (m, 9H), 0.91 (t, *J*=6.8 Hz, 3H).

### Example 195: Synthesis of Compound 395

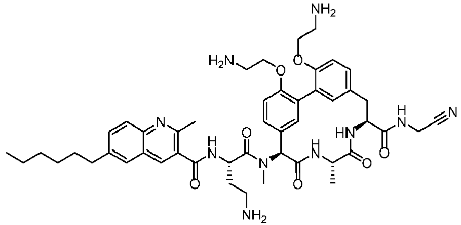

Compound 395 (formic acid salt) was prepared as a white solid from Compound **101-K** by utilizing methods analogous to those described in Example H. LCMS (Method 5-95 AB, ESI): t_{R} = 0.693 min, [M + H]⁺ = 891.8; ¹H NMR (400 MHz, MeOH-*d*₄) δ 8.49 (brs, 2H), 8.38 (s, 1H), 7.92 (d, *J*=8.4 Hz, 1H), 7.77 (s, 1H), 7.71 (d, *J*=8.8 Hz, 1H), 7.34 (d, *J*=8.4 Hz, 1H), 7.24 (d, *J*=8.4 Hz, 1H), 7.19 (d, *J*=8.4 Hz, 1H), 7.10 (d, *J*=8.8 Hz, 1H), 6.92 (d, *J*=2.0 Hz, 1H), 6.81 (s, 1H), 6.41 (s, 1H), 5.23-5.17 (m, 1H), 4.84-4.77 (m, 2H), 4.35-4.21 (m, 4H), 4.20 (s, 2H), 3.40-3.35 (m, 1H), 3.27-3.12 (m, 7H), 2.99 (s, 3H), 2.85 (t, *J*=7.5 Hz, 3H), 2.77 (s, 3H), 2.35-2.27 (m, 1H), 2.24-2.16 (m, 1H), 1.79-1.71 (m, 2H), 1.45-1.30 (m, 9H), 0.91 (t, *J*=6.8 Hz, 3H).

### Example 196: Synthesis of Compound 396

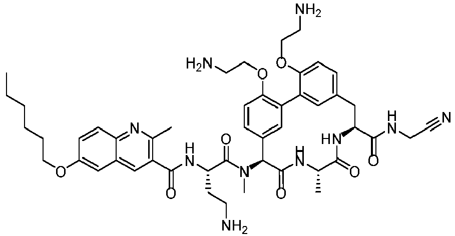

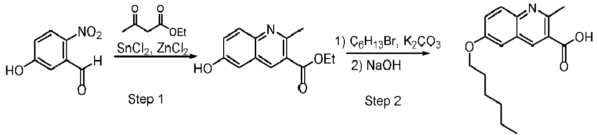

Step 1: A mixture of 5-hydroxy-2-nitrobenzaldehyde (1.5 g, 9.0 mmol), ethyl acetoacetate (1.17 g, 9.0 mmol), ZnCl₂ (6.1 g, 45 mmol) and SnCl₂ (8.5 g, 45 mmol) in EtOH (30 mL) was stirred at 70 °C for 3 h under N₂. The volatiles were removed under reduced pressure and the residue was taken up by EtOAc (50 mL), which was washed with brine (2 x 50 mL). The organic layer was dried over Na₂SO₄, concentrated and the residue was purified by silica gel chromatography, eluting with 0-10% MeOH in DCM, to give ethyl 6-hydroxy-2-methylquinoline-3-carboxylate (500 mg, 24% yield) as a brown solid. ¹H NMR (400 MHz, MeOH-*d*4) 9.44 (s, 1H), 8.12 (d, *J*=9.2 Hz, 1H), 7.80 (dd, *J*=9.2, 2.4 Hz, 1H), 7.60 (d, *J*=2.4 Hz, 1H), 4.53 (q, *J*=7.2 Hz, 2H), 3.19 (s, 3H), 1.50 (t, *J*=7.2 Hz, 3H).
Step 2: Starting from ethyl 6-hydroxy-2-methylquinoline-3-carboxylate, typical akylation (as described in Example 21) and ester hydrolysis (NaOH, MeOH/H₂O, described in Example H) conditions were followed to give 6-(hexyloxy)-2-methylquinoline-3-carboxylic acid as a white solid. LCMS (Method 5-95 AB, ESI): t_{R} = 0.798 min, [M + H]⁺ = 287.9.

Compound 396 (formic acid salt) was prepared as a white solid from Compound **101-K** and 6-(hexyloxy)-2-methylquinoline-3-carboxylic acid by utilizing methods analogous to those described in Example G. LCMS (Method 5-95 AB, ESI): t_{R} = 0.706 min, [M + H]⁺ = 907.4; ¹H NMR (400 MHz, MeOH-*d*₄) δ 8.45 (brs, 1H), 8.32 (s, 1H), 7.90 (d, *J*=8.4 Hz, 1H), 7.47 (d, *J*=8.4 Hz, 1H), 7.34-7.30 (m, 2H), 7.23 (d, *J*=8.0 Hz, 1H), 7.17 (d, *J*=8.0 Hz, 1H), 7.09 (d, *J*=8.0 Hz, 1H), 6.91 (s, 1H), 6.81 (s, 1H), 6.39 (s, 1H), 5.21-5.18 (m, 1H), 4.81-4.77 (m, 2H), 4.24-4.16 (m, 6H), 4.12 (t, *J*=6.4 Hz, 2H), 3.40-3.35 (m, 1H), 3.20-3.10 (m, 7H), 2.98 (s, 3H), 2.73 (s, 3H), 2.22-2.19 (m, 1H), 2.18-2.10 (m, 1H), 1.88-1.84 (m, 2H), 1.55-1.53 (m, 2H), 1.42-1.35 (m, 7H), 0.94 (t, *J*=6.8 Hz, 3H).

### Example 197: Synthesis of Compound 397

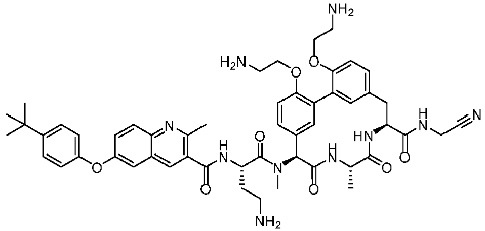

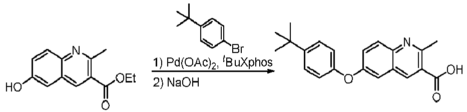

Compound 397 (formic acid salt) was prepared as a white solid from Compound **101-K** and ethyl 6-hydroxy-2-methylquinoline-3-carboxylate (described in Example 396) by utilizing methods analogous to those described in Example 174. LCMS (Method 5-95 AB, ESI): t_{R} = 0.733 min, [M + H]⁺ = 955.6; ¹H NMR (400 MHz, MeOH-*d*₄) δ 8.54 (brs, 2H), 8.29 (s, 1H), 7.99 (d, *J*=8.4 Hz, 1H), 7.61-7.55 (m, 1H), 7.49 (d, *J*=8.4 Hz, 2H), 7.32 (d, *J*=2.4 Hz, 2H), 7.24 (d, *J*=8.0 Hz, 1H), 7.16 (d, *J*=8.0 Hz, 1H), 7.12-7.04 (m, 3H), 6.90 (d, *J*=2.0 Hz, 1H), 6.80 (s, 1H), 6.38 (s, 1H), 5.21-5.15 (m, 1H), 4.80-4.78 (m, 2H), 4.28-4.16 (m, 6H), 3.29-3.10 (m, 8H), 2.97 (s, 3H), 2.76 (s, 3H), 2.34-2.10 (m, 2H), 1.36 (s, 9H), 1.34 (d, J=6.8 Hz, 3H).

### Example 198: Synthesis of Compound 398

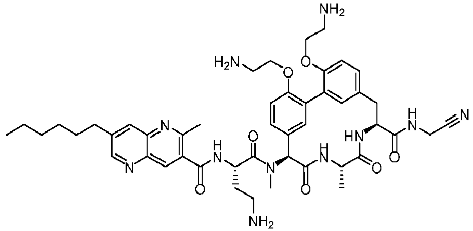

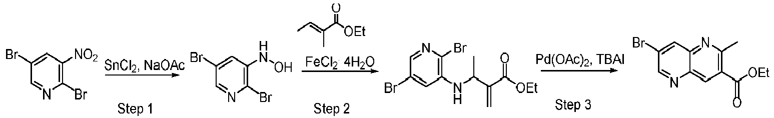

Step 1: A mixture of 2,5-dibromo-3-nitropyridine (3.0 g, 10.6 mmol), SnCl₂ (10.1 g, 53.2 mmol) and sodium acetate (8.7 g, 106 mmol) in MeOH/THF (130 mL, v/v=2/1) was stirred at 0 °C for 5 h. After filtration, the filtrate was concentrated and the residue was partitioned between EtOAc and saturated aqueous NaHCO₃ (100 mL each). The organic layer was washed with brine (2 x 100 mL), dried over Na₂SO₄, concnetrated to give *N*-(2,5-dibromopyridin-3-yl)hydroxylamine (2.7 g) as a yellow solid, which was used directly in the next step.

Step 2: A mixture of *N*-(2,5-dibromopyridin-3-yl)hydroxylamine (3.6 g, 13.4 mmol), ethyl (*E*)-2-methylbut-2-enoate (5.2 g, 40.2 mmol), and FeCl₂·4H₂O (0.27 g, 1.34 mmol) in 1,4-dioxane (20 mL) was stirred at 70 °C for 6 h. After filtration, the filtrate was concentrated to dryness and the residue was purified via silica gel chromatography, eluting with 0-10% EtOAc in petroleum ether, to give ethyl 3-((2,5-dibromopyridin-3-yl)amino)-2-methylenebutanoate (800 mg, 16% yield) as a pale yellow oil.

Step 3: A mixture of ethyl 3-((2,5-dibromopyridin-3-yl)amino)-2-methylenebutanoate (800 mg, 2.1 mmol), tetra-butyl ammonium iodide (782 mg, 2.1 mmol) and Pd(OAc)₂ (48 mg, 0.21 mmol) in DMF (10 mL), was stirred at 90 °C for 72 h under N₂. After filtration, the filtrate was concentrated and the residue was purified via silica gel chromatography, eluting with 0-5% EtOAc in petroleum ether, to give ethyl 7-bromo-2-methyl-1,5-naphthyridine-3-carboxylate (100 mg, 16% yield) as a white solid.

Compound 398 (formic acid salt) was prepared as a white solid from Compound **101-K** and ethyl 7-bromo-2-methyl-1,5-naphthyridine-3-carboxylate by utilizing methods analogous to those described in Example 194. LCMS (Method 5-95 AB, ESI): t_{R} = 0.710 min, [M + H]⁺ = 892.5; ¹H NMR (400 MHz, MeOH-*d*₄) δ 8.85 (s, 1H), 8.50 (brs, 3H), 8.39 (s, 1H), 8.11 (s, 1H), 7.32-7.01 (m, 4H), 6.81 (brs, 1H), 6.53 (brs, 2H), 5.25-5.19 (m, 1H), 4.81-4.75 (m, 2H), 4.39-4.20 (m, 6H), 3.30-3.06 (m, 8H), 3.00 (s, 3H), 2.90-2.87 (m, 2H), 2.78 (s, 3H), 2.34-2.22 (m, 2H), 1.77-1.75 (m, 2H), 1.50-1.31 (m, 9H), 0.93 (t, *J*=6.8 Hz, 3H).

### Example 199: Synthesis of Compound 399

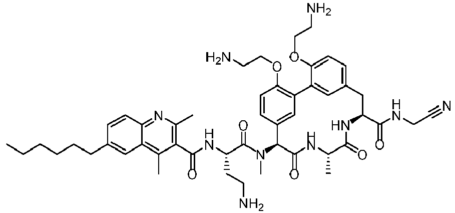

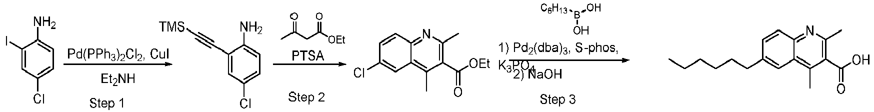

Step 1: Typical Sonogoshira conditions, as described in Example K, were applied to 4-chloro-2-iodoaniline to give 4-chloro-2-((trimethylsilyl)ethynyl)aniline as a brown oil.

Step 2: A mixture of 4-chloro-2-((trimethylsilyl)ethynyl)aniline (420 mg, 1.9 mmol), ethyl acetoacetate (0.36 mL, 2.8 mmol) and *p*-toluene sulfonic acid (357 mg, 1.9 mmol) in EtOH (16 mL) was stirred at 85 °C for 16 h. The volatiles were removed under reduced pressure and the residue was re-dissolved with EtOAc (20 mL), which was washed with saturated aqueous NaHCO₃ and brine (20 mL each). The organic layer was dried over Na₂SO₄, concentrated and the residue was purified via silica gel chromatography, eluting with 5% EtOAc in petroleum ether, to give ethyl 6-chloro-2,4-dimethylquinoline-3-carboxylate (90 mg, 18% yield) as a white solid. ¹H NMR (400 MHz, CDCl₃) δ 7.98 (d, *J*=2.0 Hz, 1H), 7.96 (d, *J*=9.2 Hz, 1H), 7.67 (dd, *J*=9.2, 2.0 Hz, 1H), 4.50 (q, *J*=7.2 Hz, 2H), 2.70 (s, 3H), 2.63 (s, 3H), 1.46 (t, *J*=7.2 Hz, 3H).

Step 3: Starting from ethyl 6-chloro-2,4-dimethylquinoline-3-carboxylate, typical Suzuki (Pd₂(dba)₃/S-phos coupling and ester hydrolysis (NaOH, MeOH/H₂O, as described in Example H) conditions were followed to give 6-hexyl-2,4-dimethylquinoline-3-carboxylic acid as a yellow solid. LCMS (Method 5-95 AB, ESI): t_{R} = 0.659 min, [M + H]⁺ = 285.9

Compound 399 (formic acid salt) was prepared as a white solid from Compound **101-K** and 6-hexyl-2,4-dimethylquinoline-3-carboxylic acid by utilizing methods analogous to those described in Example G. LCMS (Method 5-95 AB, ESI): t_{R} = 0.707 min, [M + H]⁺ = 905.6; ¹H NMR (400 MHz, MeOH-*d*₄) δ 8.50 (brs, 2H), 7.88-7.85 (m, 2H), 7.64 (d, *J*=8.4 Hz, 1H), 7.30 (d, *J*=8.4 Hz, 1H), 7.22-7.18 (m, 2H), 7.08 (d, *J*=8.4 Hz, 1H), 6.89 (brs, 1H), 6.74 (brs, 1H), 6.44 (s, 1H), 5.27-5.23 (m, 1H), 4.78-4.77 (m, 2H), 4.25-4.18 (m, 6H), 3.19-3.11 (m, 8H), 3.02 (s, 3H), 2.83 (t, J=7.6 Hz, 2H), 2.69 (s, 3H), 2.64 (s, 3H), 2.27-2.17 (m, 2H), 1.72-1.70 (m, 2H), 1.40-1.20 (m, 9H), 0.89 (t, *J*=6.8 Hz, 3H).

### Example 200: Synthesis of Compound 400

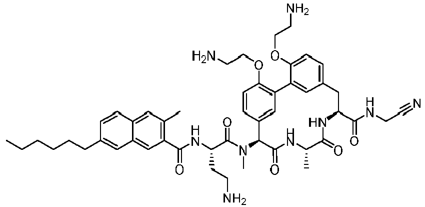

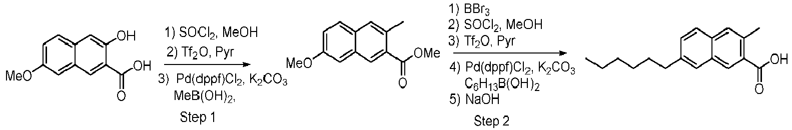

Step 1: Starting from 3-hydroxy-7-methoxy-2-naphthoic acid, typical methyl ester formation (described in Example M), triflation, Suzuki conditions (described in Example 10) were followed to give methyl 7-methoxy-3-methyl-2-naphthoate as a white solid. LCMS (Method 5-95 AB, ESI): t_{R} = 0.813 min, [M + H]⁺ = 230.9.

Step 2: Starting from methyl 7-methoxy-3-methyl-2-naphthoate, de-methylation, methyl ester formation (as described in Example M), triflation and Suzuki coupling (as described in Example10 ) and ester hydrolysis conditions (as described in Example H) were followed to give 7-hexyl-3-methyl-2-naphthoic acid as a white solid. LCMS (Method 5-95 AB, ESI): t_{R} = 0.944 min, [M + H]⁺ = 270.9.

Compound 400 (trifluoroacetic acid salt) was prepared as a white solid from Compound **101-K** and 7-hexyl-3-methyl-2-naphthoic acid by utilizing methods analogous to those described in Example G. LCMS (Method 5-95 AB, ESI): t_{R} = 0.693 min, [M + H]⁺ = 890.4; ¹H NMR (400 MHz, MeOH-*d*₄) δ 7.94 (s, 1H), 7.77 (d, *J*=8.4 Hz, 1H), 7.75-7.65 (m, 2H), 7.44 (d, *J*=8.4 Hz, 1H), 7.36 (d, *J*=8.4 Hz, 1H), 7.27 (d, *J*=8.4 Hz, 1H), 7.20 (d, *J*=8.4 Hz, 1H), 7.12 (d, *J*=8.4 Hz, 1H), 6.94 (d, *J*=2.0 Hz, 1H), 6.85 (s, 1H), 6.39 (s, 1H), 5.25-5.18 (m, 1H), 4.83-4.75 (m, 2H), 4.28-4.21 (m, 6H), 3.40-3.35 (m, 1H), 3.26-3.12 (m, 7H), 3.00 (s, 3H), 2.81 (t, *J*=8.0 Hz, 2H), 2.57 (s, 3H), 2.37-2.27 (m, 1H), 2.25-2.14 (m, 1H), 1.75-1.71 (m, 2H), 1.45-1.20 (m, 11H), 0.92 (t, J=7.2 Hz, 3H).

### Example 201: Synthesis of Compound 401

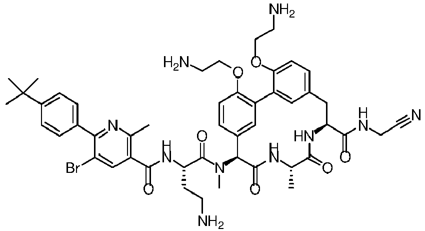

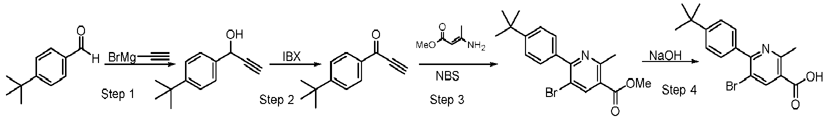

Step 1: To a solution of 4-(*tert*-butyl)benzaldehyde (5.0 g, 30.8 mmol) in THF (50 mL) was added ethynyl magnesium bromide (0.5 N in THF, 92.5 mL) dropwise at 0 °C and the mixture was stirred at 20 °C for 4 h. The reaction was quenched with saturated aqueous NH₄Cl (30 mL), which was extracted with EtOAc (3 x 30 mL). The combined organic layers were dried by Na₂SO₄, concentrated and the residue was purified by silica gel chromatography, eluting with 0-5% EtOAc in petroleum ether, to give 1-(4-(*tert*-butyl)phenyl)prop-2-yn-1-ol (4.5 g, 78% yield) as a colorless oil.

Step 2: A mixture of 1-(4-(*tert*-butyl)phenyl)prop-2-yn-1-ol (4.5 g, 24 mmol), 2-iodoxybenzoic acid (20 g, 72 mmol) in EtOAc (50 mL) was stirred at 80 °C for 4 h. After filtration, the filtrate was evaporated *in vacuo* to give 1-(4-(*tert*-butyl)phenyl)prop-2-yn-1-one (4.2 g) as a yellow oil, which was used directly in the next step.

Step 3: A mixture of 1-(4-(*tert*-butyl)phenyl)prop-2-yn-1-one (3.9 g, 20.8 mmol) and methyl (*E*)-3-aminobut-2-enoate (2.0 g, 17.4 mmol) in EtOH (30 mL) was stirred at 50 °C for 0.5 h. After cooling to 0 °C, *N*-bromosuccinimide (3.7 g, 20.8 mmol) was added to the above solution and the resulting mixture was stirred at 0 °C for 0.5 h. The volatiles were removed and the residue was re-dissolved by EtOAc (50 mL), which was washed with brine (2 x 50 mL). The organic layer was dried by Na₂SO₄, concentrated and the residue was purified by reverse-phase HPLC (solvent gradient: acetonitrile 45-95%/(0.225%formic acid)-water) to give methyl 5-bromo-6-(4-(*tert*-butyl)phenyl)-2-methylnicotinate (3.2 g, 51% yield) as a yellow oil. LCMS (Method 5-95 AB, ESI): t_{R} = 0.945 min, [M + H]⁺ = 361.9.

Step 4: Typical ester hydrolysis conditions, as described in Example H, were applied to methyl 5-bromo-6-(4-(*tert*-butyl)phenyl)-2-methylnicotinate to give 5-bromo-6-(4-(*tert*-butyl)phenyl)-2-methylnicotinic acid as a yellow solid.

Compound 401 (formic acid salt) was prepared as a white solid from Compound **101-K** and 5-bromo-6-(4-(*tert*-butyl)phenyl)-2-methylnicotinic acid by utilizing methods analogous to those described in Example G. LCMS (Method 5-95 AB, ESI): t_{R} = 0.743 min, [M + H]⁺ = 967.4; ¹H NMR (400 MHz, MeOH-*d*₄) δ 8.49 (brs, 3H), 8.16 (s, 1H), 7.65-7.50 (m, 4H), 7.32 (d, *J*=8.4 Hz, 1H), 7.25 (d, *J*=8.4 Hz, 1H), 7.19 (d, *J*=8.4 Hz, 1H), 7.10 (d, *J*=8.4 Hz, 1H), 6.91 (s, 1H), 6.83 (s, 1H), 6.39 (s, 1H), 5.20-5.10 (m, 1H), 4.85-4.75 (m, 2H), 4.25-4.15 (m, 6H), 3.40-3.35 (m, 1H), 3.20-3.10 (m, 7H), 2.95 (s, 3H), 2.62 (s, 3H), 2.33-2.25 (m, 1H), 2.20-2.10 (m, 1H), 1.39 (s, 9H), 1.36 (d, *J*=6.8 Hz, 3H).

### Example 202: Synthesis of Compound 402

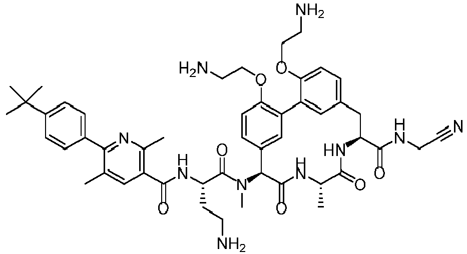

Step 1: Starting from methyl 5-bromo-6-(4-(*tert*-butyl)phenyl)-2-methylnicotinate (described in Example 201), typical Suzuki and ester hydrolysis conditions (as described in Example H) were followed to give 6-(4-(*tert*-butyl)phenyl)-2,5-dimethylnicotinic acid as a yellow solid.

Compound 402 (formic acid salt) was prepared as a white solid from Compound **101-K** and 6-(4-(*tert*-butyl)phenyl)-2,5-dimethylnicotinic acid by utilizing methods analogous to those described in Example G. LCMS (Method 5-95 AB, ESI): t_{R} = 0.688 min, [M + H]⁺ = 903.5; ¹H NMR (400 MHz, MeOH-*d*₄) δ 8.47 (brs, 2H), 7.77 (s, 1H), 7.56 (d, *J*=8.4 Hz, 2H), 7.43 (d, *J*=8.4 Hz, 2H), 7.31 (d, *J*=8.0 Hz, 1H), 7.24 (d, *J*=8.0 Hz, 1H), 7.18 (d, *J*=8.0 Hz, 1H), 7.10 (d, *J*=8.8 Hz, 1H), 6.92 (brs, 1H), 6.83 (s, 1H), 6.38 (s, 1H), 5.20-5.15 (m, 1H), 4.85-4.75 (m, 2H), 4.25-4.15 (m, 6H), 3.40-3.35 (m, 1H), 3.25-3.10 (m, 7H), 2.96 (s, 3H), 2.60 (s, 3H), 2.35 (s, 3H), 2.33-2.25 (m, 1H), 2.20-2.10 (m, 1H), 1.40 (s, 9H), 1.36 (d, J=7.2 Hz, 3H).

### Example 203: Synthesis of Compound 403

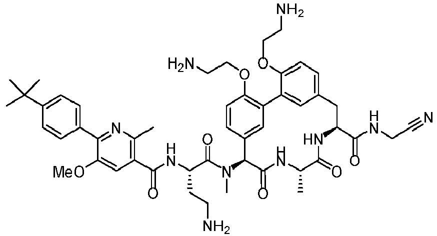

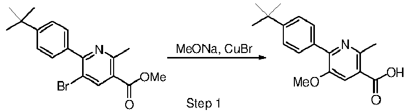

Step 1: A mixture of methyl 5-bromo-6-(4-(*tert*-butyl)phenyl)-2-methylnicotinate (described in Example 402) (400 mg, 1.1mmol), NaOMe (90 mg, 1.7 mmol) and CuBr (16 mg, 0.11 mmol) in NMP/MeOH (9 mL, v/v=8/1) was stirred at 110 °C for 20 h under N₂. The volatiles were removed under reduced pressure and the residue was purified by reverse-phase HPLC (acetonitrile 50-75% / 0.2% formic acid in water) to give 6-(4-(*tert*-butyl)phenyl)-5-methoxy-2-methylnicotinic acid (22 mg, 6% yield) as a white solid. LCMS (Method 5-95 AB, ESI): t_{R} = 0.833 min, [M + H]⁺ = 300.2.

Compound 403 (formic acid salt) was prepared as a white solid from Compound **101-K** and 6-(4-(*tert*-butyl)phenyl)-5-methoxy-2-methylnicotinic acid by utilizing methods analogous to those described in Example G. LCMS (Method 5-95 AB, ESI): t_{R} = 0.710 min, [M + H]⁺ = 919.6; ¹H NMR (400 MHz, MeOH-*d*₄) δ 8.48 (brs, 2H), 7.75 (d, *J*=8.4 Hz, 2H), 7.54 (s, 1H), 7.48 (d, *J*=8.4 Hz, 2H), 7.32 (d, *J*=8.0 Hz, 1H), 7.24 (d, *J*=8.0 Hz, 1H), 7.17 (d, *J*=8.0 Hz, 1H), 7.10 (d, *J*=8.0 Hz, 1H), 6.91 (d, *J*=2.4 Hz, 1H), 6.82 (s, 1H), 6.38 (s, 1H), 5.19-5.16 (m, 1H), 4.81-4.78 (m, 2H), 4.24-4.19 (m, 6H), 3.92 (s, 3H), 3.40-3.35 (m, 1H), 3.20-3.10 (m, 7H), 2.96 (s, 3H), 2.58 (s, 3H), 2.35-2.14 (m, 2H), 1.37 (s, 9H), 1.36 (d, J=6.0 Hz, 3H).

### Example 204: Synthesis of Compound 404

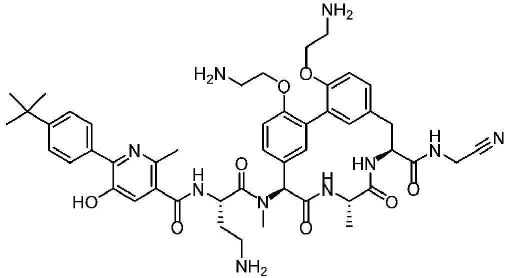

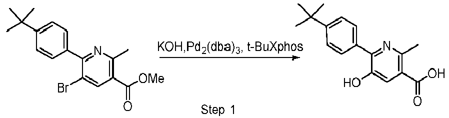

Step 1: A mixture of of methyl 5-bromo-6-(4-(*tert*-butyl)phenyl)-2-methylnicotinate (described in Example 402) (120 mg, 0.33 mmol), Pd₂(dba)₃ (6.1 mg, 0.99 mmol), t-BuXphos (11 mg, 0.03 mmol) and KOH (56 mg, 0.99 mmol) in 1,4-dioxane/H₂O (8 mL, v/v=4/1) was stirred at 100 °C for 12 h under N₂. The volatiles were removed under reduced pressure and the residue was purified by reverse-phase HPLC (acetonitrile 25-55% / 0.2% formic acid in water) to give 6-(4-(*tert*-butyl)phenyl)-5-hydroxy-2-methylnicotinic acid (10 mg, 11% yield) as a white solid. LCMS (Method 5-95 AB, ESI): t_{R} = 0.634 min, [M + H]⁺ = 286.4.

Compound 404 (formic acid salt) was prepared as a white solid from Compound **101-K** and 6-(4-(*tert*-butyl)phenyl)-5-hydroxy-2-methylnicotinic acid by utilizing methods analogous to those described in Example G. LCMS (Method 5-95 AB, ESI): t_{R} = 0.692 min, [M + H]⁺ = 905.9; ¹H NMR (400 MHz, MeOH-*d*₄) δ 8.49 (brs, 3H), 7.84 (d, *J*=8.4 Hz, 2H), 7.51 (d, *J*=8.4 Hz, 2H), 7.35-7.29 (m, 2H), 7.25 (d, *J*=8.0 Hz, 1H), 7.18 (d, *J*=8.4 Hz, 1H), 7.10 (d, *J*=8.4 Hz, 1H), 6.91 (d, *J*=2.8 Hz, 1H), 6.83 (s, 1H), 6.38 (s, 1H), 5.16-5.14 (m, 1H), 4.62-4.54 (m, 2H), 4.25-4.14 (m, 6H), 3.50-3.47 (m, 1H), 3.20-3.07 (m, 5H), 2.96 (s, 3H), 2.82-2.65 (m, 2H), 2.56 (s, 3H), 2.34-2.10 (m, 2H), 1.38 (s, 9H), 1.37 (d, *J*=7.2 Hz, 3H).

### Example 205: Synthesis of Compound 405

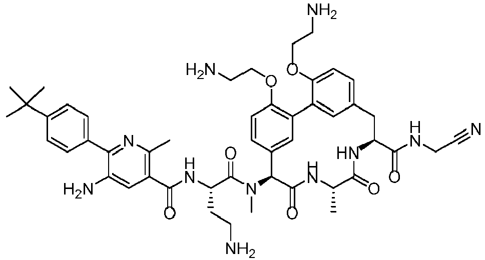

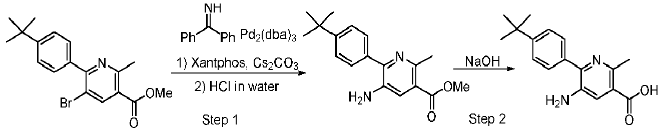

Step 1: A mixture of methyl 5-bromo-6-(4-(*tert*-butyl)phenyl)-2-methylnicotinate (described in Example 402) (200 mg, 0.53 mmol), benzophenone imine (145 mg, 0.80 mmol), Pd₂(dba)₃ (49 mg, 0.05 mmol), Xantphos (62 mg, 0.11 mmol) and Cs₂CO₃ (346 mg, 1.06 mmol) in toluene (2 mL) was stirred at 90 °C for 16 h under N₂. The reaction mixture was diluted with water (20 mL), which was extracted by EtOAc (3 x 20 mL). The combined organic layers were washed with brine (2 x 50 mL), dried over MgSO₄, concentrated and the residue was purified by preparatory-TLC (20% EtOAc in petroleum ether, R_{f}= 0.5). To the above isolated material was added HCl solution (2N, 3 mL) and the mixture was stirred at 20 °C for 3 h. The volatiles were removed and the residue was purified by prep-TLC to give methyl 5-amino-6-(4-(*tert*-butyl)phenyl)-2-methylnicotinate (100 mg, 78% yield) as a white solid. LCMS (Method 5-95 AB, ESI): t_{R} = 0.757 min, [M + H]⁺ = 298.9.

Step 2: Typical ester hydrolysis conditions (NaOH, MeOH/H₂O, described in Example H) were applied to methyl 5-amino-6-(4-(*tert*-butyl)phenyl)-2-methylnicotinate to give 5-amino-6-(4-(*tert-*butyl)phenyl)-2-methylnicotinic acid as a white solid.

Compound 405 (formic acid salt) was prepared as a white solid from Compound **101-K** and 5-amino-6-(4-(*tert*-butyl)phenyl)-2-methylnicotinic acid by utilizing methods analogous to those described in Example G. LCMS (Method 5-95 AB, ESI): t_{R} = 0.650 min, [M + H]⁺ = 904.5; ¹H NMR (400 MHz, MeOH-*d*₄) δ 8.45 (brs, 2H), 7.60-7.40 (m, 4H), 7.35-7.15 (m, 4H), 7.12 (d, *J*=8.4 Hz, 1H), 6.87 (s, 1H), 6.72 (s, 1H), 6.45 (s, 1H), 5.16-5.13 (m, 1H), 4.85-4.75 (m, 2H), 4.40-4.05 (m, 6H), 3.40-3.35 (m, 1H), 3.25-3.05 (m, 7H), 2.97 (s, 3H), 2.43 (s, 3H), 2.35-2.10 (m, 2H), 1.37 (s, 9H), 1.36 (d, *J*=6.4 Hz, 3H).

### Example 206: Synthesis of Compound 406

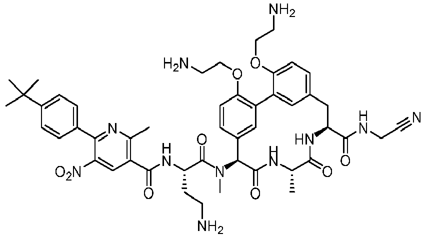

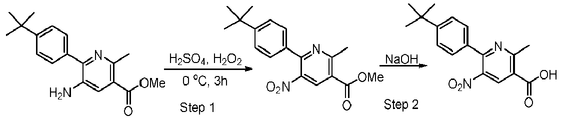

Step 1: A mixture of methyl 5-amino-6-(4-(*tert*-butyl)phenyl)-2-methylnicotinate (described in Example 205, 200 mg, 0.67 mmol) in 98% aqueous H₂SO₄ and 50% aqueous H₂O₂ (4 mL, v/v=1/1) was stirred at 0 °C for 16 h. The mixture was quenched with saturated Na₂CO₃ solution until pH=7, and then extracted by EtOAc (3 x 20 mL). The combined organic layers were washed with brine (50 mL), dried over MgSO₄, concentrated and the residue was purified by preparatory-TLC (10% EtOAc in petroleum ether, R_{f}=0.5) to give methyl 6-(4-(*tert*-butyl)phenyl)-2-methyl-5-nitronicotinate (60 mg, 27% yield) as a white solid. LCMS (Method 5-95 AB, ESI): t_{R} = 1.010 min, [M + H]⁺ = 329.1.

Step 2: Typical ester hydrolysis conditions (NaOH, MeOH/H₂O, as described in Example H) were applied to methyl 6-(4-(*tert*-butyl)phenyl)-2-methyl-5-nitronicotinate to give *6-(4-(tert-*butyl)phenyl)-2-methyl-5-nitronicotinic acid as a white solid.

Compound 406 (formic acid salt) was prepared as a white solid from Compound **101-K** and 6-(4-(*tert*-butyl)phenyl)-2-methyl-5-nitronicotinic acid by utilizing methods analogous to those described in Example G. LCMS (Method 5-95 AB, ESI): t_{R} = 0.730 min, [M + H]⁺ = 934.5; ¹H NMR (400 MHz, MeOH-*d*₄) δ 8.32 (s, 1H), 7.60-7.40 (m, 4H), 7.24 (d, *J*=8.0 Hz, 1H), 7.20 (d, *J*=8.0 Hz, 1H), 7.17 (d, *J*=8.0 Hz, 1H), 7.11 (d, *J*=8.4 Hz, 1H), 6.92 (d, *J*=2.0 Hz, 1H), 6.83 (s, 1H), 6.38 (s, 1H), 5.19-5.17 (m, 1H), 4.79-4.75 (m, 2H), 4.40-4.05 (m, 6H), 3.30-3.05 (m, 8H), 2.95 (s, 3H), 2.75 (s, 3H), 2.40-2.10 (m, 2H), 1.38 (s, 9H), 1.37 (d, J=7.2 Hz, 3H).

### Example 207: Synthesis of Compound 407

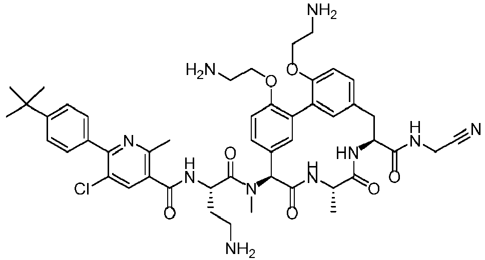

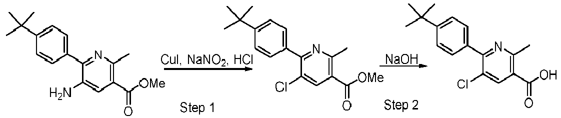

Step 1: To a solution of methyl 5-amino-6-(4-(*tert*-butyl)phenyl)-2-methylnicotinate (described in Example 205, 200 mg, 0.67 mmol) in 6M aqueous HCl (6 mL) was added a solution of NaNO₂ (139 mg, 2.0 mmol) in H₂O (2 mL) dropwsie at 0 °C and the mixture was stirred at 15 °C for 3 h, followed by the addition of CuCl (398 mg, 4.0 mmol). The resulting mixture was stirred at 15 °C for 16 h. The reaction was diluted with H₂O (20 mL), which was extracted by EtOAc (3 x 20 mL). The combined organic layers were washed with brine (2 x 50 mL), dried over MgSO₄, concentrated and the residue was purified by preparatory-TLC (30% EtOAc in petroleum ether) to give methyl 6-(4-(*tert*-butyl)phenyl)-5-chloro-2-methylnicotinate (50 mg, 24% yield) as a colorless oil. LCMS (Method 5-95 AB, ESI): t_{R} = 1.060 min, [M + H]⁺ = 317.9.

Step 2: Typical ester hydrolysis conditions (NaOH, MeOH/H₂O, as described in Example H) were applied to methyl 6-(4-(tert-butyl)phenyl)-5-chloro-2-methylnicotinate to give 6-(4-(*tert-*butyl)phenyl)-5-chloro-2-methylnicotinic acid as a white solid.

Compound 407 (formic acid salt) was prepared as a white solid from Compound **101-K** and 6-(4-(*tert*-butyl)phenyl)-5-chloro-2-methylnicotinic acid by utilizing methods analogous to those described in Example G. LCMS (Method 5-95 AB, ESI): t_{R} = 0.740 min, [M + H]⁺ = 923.5; ¹H NMR (400 MHz, MeOH-*d*₄) δ 8.44 (brs, 1H), 7.98 (s, 1H), 7.64 (d, *J*=8.4 Hz, 2H), 7.56 (d, *J*=8.4 Hz, 2H), 7.32 (d, *J*=8.0 Hz, 1H), 7.28-7.10 (m, 2H), 7.11 (d, *J*=8.4 Hz, 1H), 6.91 (d, *J*=2.4 Hz, 1H), 6.83 (s, 1H), 6.38 (s, 1H), 5.17-5.15 (m, 1H), 4.79-4.75 (m, 1H), 4.40-4.10 (m, 7H), 3.30-3.05 (m, 8H), 2.95 (s, 3H), 2.63 (s, 3H), 2.35-2.05 (m, 2H), 1.38 (s, 9H), 1.35 (d, *J*=6.4 Hz, 3H).

### Example 208: Synthesis of Compound 408

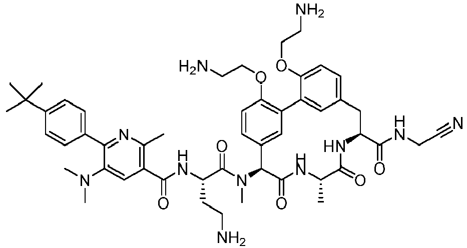

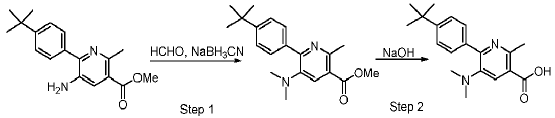

Step 1: A mixture of methyl 5-amino-6-(4-(*tert*-butyl)phenyl)-2-methylnicotinate (described in Example 205, 50 mg, 0.17 mmol), NaBH₃CN (53 mg, 0.84 mmol) and paraformaldehyde (50 mg, 1.7 mmol) in acetic acid (3 mL) was stirred at 20 °C for 10 h. The reaction was quenched with saturated aqueous Na₂CO₃ to adjust pH to 8, and then extracted with EtOAc (2 x 20 mL). The combined organic layers were dried over Na₂SO₄, concentrated and the residue was purified by preparatory-TLC (20% EtOAc in petroleum ether, R_{f}=0.5) to give methyl 6-(4-(*tert*-butyl)phenyl)-5-(dimethylamino)-2-methylnicotinate (50 mg, 91% yield) as a yellow solid.

Step 2: Typical ester hydrolysis conditions (NaOH, MeOH/H₂O, as described in Example H) were applied to methyl 6-(4-(*tert*-butyl)phenyl)-5-(dimethylamino)-2-methylnicotinate to give 6-(4-(*tert-*butyl)phenyl)-5-(dimethylamino)-2-methylnicotinic acid as a white solid. LCMS (Method 5-95 AB, ESI): t_{R} = 0.787 min, [M + H]⁺ = 313.4.

Compound 408 (formic acid salt) was prepared as a white solid from Compound **101-K** and 6-(4-(*tert*-butyl)phenyl)-5-(dimethylamino)-2-methylnicotinic acid by utilizing methods analogous to those described in Example G. LCMS (Method 5-95 AB, ESI): t_{R} = 0.711 min, [M + H]⁺ = 932.5; ¹H NMR (400 MHz, MeOH-*d*₄) δ 8.46 (brs, 3H), 7.71 (d, *J*=8.4 Hz, 2H), 7.54-7.48 (m,3H), 7.32 (d, *J*=8.0 Hz, 1H), 7.26-7.15 (m, 2H), 7.10 (d, *J*=8.4 Hz, 1H), 6.92 (d, *J*=2.4 Hz, 1H), 6.83 (s,1H), 6.34 (s,1H), 5.20-5.14 (m, 1H), 4.82-4.78 (m, 2H), 4.30-4.15 (m, 6H), 3.36-3.10 (m, 8H), 2.96 (s, 3H), 2.60 (s, 6H), 2.55 (s, 3H), 2.32-2.27 (m, 1H), 2.20-2.15 (m, 1H), 1.38 (s, 9H), 1.36 (d, *J*=6.0 Hz, 3H).

### Example 209: Synthesis of Compound 409

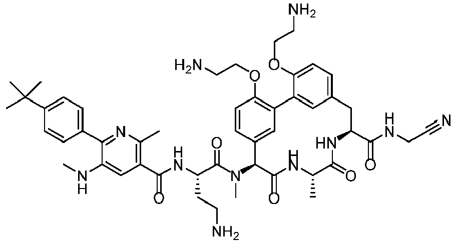

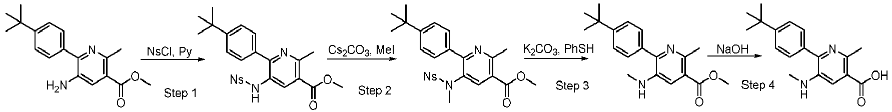

Step 1: To a solution of methyl 5-amino-6-(4-(*tert*-butyl)phenyl)-2-methylnicotinate (described in Example 205, 50 mg, 0.17 mmol) in DCM (2 mL) and pyridine (0.5 mL) was added 2-nitro benzenesulfonyl chloride (111 mg, 0.50 mmol) and the mixture was stirred at 15 °C for 16 h. The reaction mixture was diluted with water (20 mL), which was extracted by EtOAc (2 x 20 mL). The combined organic layers were washed with brine (30 mL), dried over MgSO₄, concentrated and the residue was purified by preparatory-TLC (20% EtOAc in petroleum ether, R_{f}=0.5) to give methyl 6-(4-(*tert-*butyl)phenyl)-2-methyl-5-((4-nitrophenyl)sulfonamido)nicotinate (50 mg, 62% yield) as a yellow solid. LCMS (Method 5-95 AB, ESI): t_{R} = 0.963 min, [M + H]⁺ = 484.1.

Step 2: Typical alkylation conditions (as described in Example 21) were applied to methyl 6-(4-(*tert*-butyl)phenyl)-2-methyl-5-((4-nitrophenyl)sulfonamido)nicotinate to give methyl 6-(4-(*tert-*butyl)phenyl)-2-methyl-5-((*N*-methyl-4-nitrophenyl)sulfonamido)nicotinate as a yellow solid.

Step 3: A mixture of methyl 6-(4-(*tert*-butyl)phenyl)-2-methyl-5-((*N*-methyl-4-nitrophenyl)sulfonamido)nicotinate (50 mg, 0.10 mmol) and K₂CO₃ (70 mg, 0.50 mmol) in DMF (3 mL) and thiophenol (4.2 mL) was stirred at 20 °C for 2 h. The reaction was partitioned between EtOAc and brine (30 mL each); the organic layer was dried over Na₂SO₄, concentrated and the residue was purified by silica gel chromatography, eluting with 30% EtOAc in petroleum ether, to give methyl 6-(4-(*tert-*butyl)phenyl)-2-methyl-5-(methylamino)nicotinate (25 mg, 80% yield) as a colorless oil. LCMS (Method 5-95 AB, ESI): t_{R} = 0.688 min, [M + H]⁺ = 313.4.

Step 4: Typical ester hydrolysis conditions (NaOH, MeOH/H₂O, as described in Example H) were applied to methyl 6-(4-(*tert*-butyl)phenyl)-2-methyl-5-(methylamino)nicotinate to give *6-(4-(tert-*butyl)phenyl)-2-methyl-5-(methylamino)nicotinic acid as a white solid.

Compound 409 (formic acid salt) was prepared as a white solid from Compound **101-K** and 6-(4-(*tert*-butyl)phenyl)-2-methyl-5-(methylamino)nicotinic acid by utilizing methods analogous to those described in Example G. LCMS (Method 5-95 AB, ESI): t_{R} = 0.650 min, [M + H]⁺ = 918.5; ¹H NMR (400 MHz, MeOH-*d*₄) δ 8.52 (brs, 1H), 7.58 (d, *J*=8.4 Hz, 2H), 7.48 (d, *J*=8.0 Hz, 2H), 7.35-7.16 (m, 3H), 7.13-7.05 (m, 2H), 6.91 (s, 1H), 6.82 (s, 1H), 6.36 (s, 1H), 5.20-5.10 (m, 1H), 4.79-4.75 (m, 1H), 4.40-4.10 (m, 7H), 3.40-3.35 (m, 1H), 3.25-3.05 (m, 7H), 2.95 (s, 3H), 2.80 (s, 3H), 2.47 (s, 3H), 2.35-2.10 (m, 2H), 1.42 (s, 9H), 1.36 (d, *J*=7.2 Hz, 3H).

### Example 210: Synthesis of Compound 410

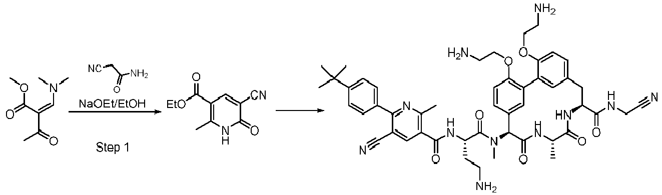

Step 1: A mixture of methyl (*Z*)-2-((dimethylamino)methylene)-3-oxobutanoate (6.7 g, 36 mmol), 2-cyanoacetamide (2.98 g, 35 mmol), acetic acid (5.3 g, 88 mmol), and sodium ethoxide (1.24 g, 3.8 mmol) in EtOH (60 mL) was stirred at 15 °C for 1 h. The volatiles were removed and 1N aqueous HCl (50 mL) was added. The precipitate was filtered, washed with H₂O and saturated NaHCO₃ solution and dried in an oven to yield ethyl 5-cyano-2-methyl-6-oxo-1,6-dihydropyridine-3-carboxylate (4.5 g, 62% yield) as a yellow solid. LCMS (Method 5-95 AB, ESI): t_{R} = 0.614 min, [M + H]⁺ = 206.8.
Compound 410 (formic acid salt) was prepared as a white solid from Compound **101-K** and ethyl 5-cyano-2-methyl-6-oxo-1,6-dihydropyridine-3-carboxylate by utilizing methods analogous to those described in Example 158. LCMS (Method 5-95 AB, ESI): t_{R} = 0.605 min, [M + H]⁺ = 914.6; ¹H NMR (400 MHz, MeOH-*d*₄) δ 8.48 (brs, 3H), 8.23 (s, 1H), 7.90 (d, *J*=8.4 Hz, 2H), 7.62 (d, *J*=8.4 Hz, 2H), 7.35 (d, *J*=8.4 Hz, 1H), 7.24-7.20 (m, 2H), 7.10 (d, *J*=8.4 Hz, 1H), 6.92 (d, *J*=2.4 Hz, 1H), 6.82 (s, 1H), 6.41 (s, 1H), 5.20-5.16 (m, 1H), 4.80-4.78 (m, 2H), 4.25-4.18 (m, 6H), 3.40-3.35 (m, 1H), 3.18-3.12 (m, 7H), 2.96 (s, 3H), 2.75 (s, 3H), 2.31-2.28 (m, 1H), 2.18-2.15 (m, 1H), 1.40 (s, 9H), 1.36 (t, J=6.8 Hz, 3H).

### Example 211: Synthesis of Compound 411

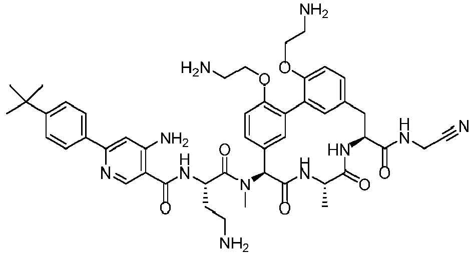

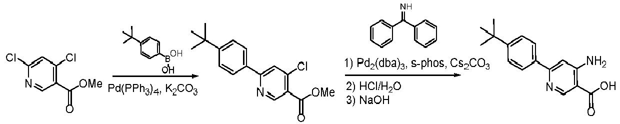

4-Amino-6-(4-(*tert*-butyl)phenyl)nicotinic acid was prepared as a white solid from methyl 4,6-dichloronicotinate by utilizing typical Suzuki procedure (as described in Example H) and the methods described in Example 205. LCMS (Method 5-95 AB, ESI): t_{R} = 0.744 min, [M + H]⁺ = 270.9. Compound 411 (formic acid salt) was prepared as a white solid from Compound **101-K** and 4-amino-6-(4-(*tert*-butyl)phenyl)nicotinic acid by utilizing methods analogous to those described in Example 158. LCMS (Method 5-95 AB, ESI): t_{R} = 0.570 min, [M + H]⁺ = 890.5; ¹H NMR (400 MHz, MeOH-*d*₄) δ 8.58 (brs, 1H), 7.79 (d, *J*=8.4 Hz, 2H), 7.55 (d, *J*=8.4 Hz, 2H), 7.34 (d, *J*=8.0 Hz, 1H), 7.30-7.05 (m, 5H), 6.89 (s, 1H), 6.81 (s, 1H), 6.34 (s, 1H), 5.20-5.10 (m, 1H), 4.79-4.75 (m, 1H), 4.40-4.10 (m, 7H), 3.25-3.05 (m, 8H), 2.87 (s, 3H), 2.35-2.20 (m, 1H), 2.15-2.05 (m, 1H), 1.37 (s, 9H), 1.36 (d, *J*=7.2 Hz, 3H).

### Example 212: Synthesis of Compound 412

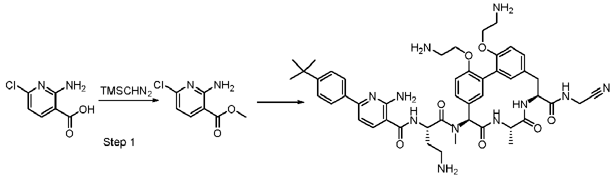

Step 1: A mixture of 2-amino-6-chloronicotinic acid (150 mg, 0.87 mmol) and TMSCHN₂ (2N solution in hexane, 0.87 mL) in toluene (10 mL) and methanol (2.5 mL) was stirred at 25 °C for 16 h. The volatiles were removed under reduced pressure and the residue was partitioned between EtOAc and brine (50 mL each). The organic layer was dried over Na₂SO₄ and concentrated to give methyl 2-amino-6-chloronicotinate (156 mg) as a white solid, which was used directly in the next step.
Compound 412 (formic acid salt) was prepared as a white solid from Compound **101-K** and methyl 2-amino-6-chloronicotinate by utilizing methods analogous to those described in Example 158. LCMS (Method 5-95 AB, ESI): t_{R} = 0.689 min, [M + H]⁺ = 890.5; ¹H NMR (400 MHz, MeOH-*d*₄) δ 8.46 (brs, 3H), 8.03-7.98 (m, 1H), 7.92-7.80 (m, 2H), 7.49 (d, *J* = 7.6 Hz, 2H), 7.33-7.21 (m, 2H), 7.16-7.05 (m, 3H), 6.86 (d, *J* = 1.6 Hz, 2H), 6.78 (s, 1H), 6.44 (s, 1H), 5.12-5.09 (m, 1H), 4.81-4.75 (m, 2H), 4.24-4.14 (m, 6H), 3.37-3.36 (m, 1H), 3.20-3.11 (m, 7H), 2.89 (s, 3H), 2.34-2.16 (m, 2H), 1.37 (s, 9H), 1.36 (d, *J*=7.2 Hz, 3H).

### Example 213: Synthesis of Compound 413

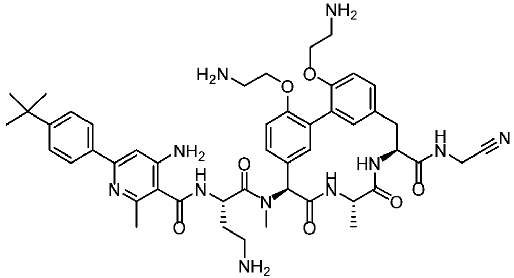

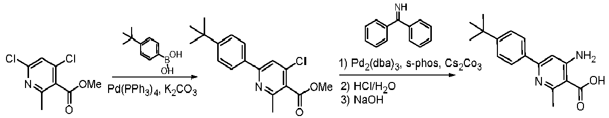

Compound 413 (formic acid salt) was prepared as a white solid from **101-K** by utilizing methods analogous to those described in Example 211. LCMS (Method 5-95 AB, ESI): t_{R} = 0.658 min, [M + H]⁺ = 904.4; ¹H NMR (400 MHz, MeOH-*d*₄) δ 8.42 (brs, 1H), 7.71 (d, *J*=8.0 Hz, 2H), 7.63 (d, *J*=8.0 Hz, 2H), 7.35 (d, *J*=8.4 Hz, 1H), 7.25 (d, *J*=8.4 Hz, 1H), 7.20 (d, *J*=8.4 Hz, 1H), 7.11 (d, *J*=8.4 Hz, 1H), 7.01 (s, 1H), 6.92 (d, *J*=2.0 Hz, 1H), 6.83 (s, 1H), 6.43 (s, 1H), 5.11-5.05 (m, 1H), 4.83-4.75 (m, 2H), 4.32-4.23 (m, 4H), 4.20 (s, 2H), 3.28-3.08 (m, 8H), 2.98 (s, 3H), 2.59 (s, 3H), 2.30-2.10 (m, 2H), 1.38 (s, 9H), 1.36 (d, *J*=7.2 Hz, 3H).

### Example 214: Synthesis of Compound 414

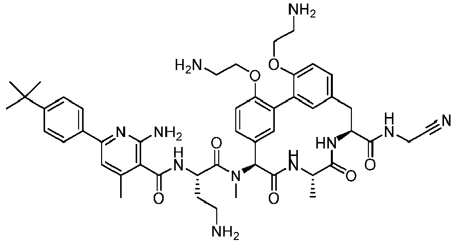

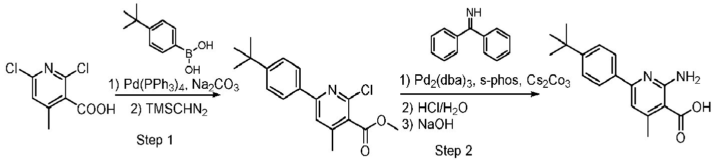

Steps 1-1: 2-Amino-6-(4-(*tert*-butyl)phenyl)-4-methylnicotinic acid was prepared as a white solid from 2,6-dichloro-4-methylnicotinic acid by utilizing typical Suzuki procedures (as described in Example H) and the methods described in Examples 212 and 205. LCMS (Method 5-95 AB, ESI): t_{R} = 0.665 min, [M + H]⁺ = 284.9.
Compound 414 (formic acid salt) was prepared as a white solid from Compound **101-K** and 2-amino-6-(4-(*tert*-butyl)phenyl)-4-methylnicotinic acid by utilizing methods analogous to those described in Example G. LCMS (Method 5-95 AB, ESI): t_{R} = 0.669 min, [M + H]⁺ = 904.5; ¹H NMR (400 MHz, MeOH-*d*₄) δ 8.31 (brs, 2H), 7.75 (d, *J*=8.4 Hz, 1H), 7.65 (d, *J*=8.4 Hz, 1H), 7.45-7.38 (m, 2H), 7.30-7.14 (m, 3H), 7.06-6.95 (m, 3H), 6.85 (s, 0.5H), 6.82 (s, 0.5H), 6.55 (s, 0.5H), 6.48 (s, 0.5H), 5.10-5.06 (m, 1H), 4.85-4.75 (m, 2H), 4.33-4.16 (m, 6H), 3.30-2.95 (m, 8H) 2.97 (s, 3H), 2.29-2.16 (m, 5H), 1.39 (s, 9H), 1.37 (d, *J*=7.2 Hz, 3H).

### Example 215: Synthesis of Compound 415

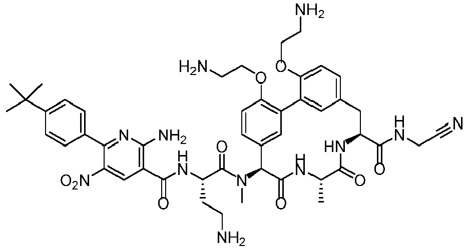

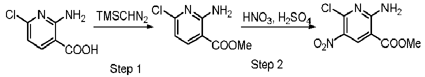

Step 1: Following a procedure analogous to that described in Example 212, Step 1, 2-amino-6-chloronicotinic acid was converted to methyl 2-amino-6-chloronicotinate as a white solid.

Step 2: A mixture of methyl 2-amino-6-chloronicotinate (300 mg, 1.6 mmol) and HNO₃ (80 µL, 1.9 mmol) in sulfuric acid (10 mL) was stirred at 0 °C for 1 h. The volatiles were removed and the residue was taken up in EtOAc (50 mL), which was washed with brine (2 x 50 mL). The organic layer was dried over MgSO₄, concentrated and the residue was purified by silica gel chromatography, eluting with 10% EtOAc in petroleum ether, to give methyl 2-amino-6-chloro-5-nitronicotinate (150 mg, 40% yield) as a colorless oil LCMS (ESI): [M + H]⁺ = 232.0.

Compound 415 (formic acid salt) was prepared as a white solid from Compound **101-K** and methyl 2-amino-6-chloro-5-nitronicotinate by utilizing methods analogous to those described in Example 158. LCMS (Method 5-95 AB, ESI): t_{R} = 0.728 min, [M + H]⁺ = 935.4; ¹H NMR (400 MHz, MeOH-*d*₄) δ 8.47 (brs, 2H), 7.54-7.47 (m, 3H), 7.43-7.36 (m, 2H), 7.31-7.17 (m, 3H), 7.12 (d, *J*=8.4 Hz, 1H), 6.94 (s, 1H), 6.80 (s, 1H), 6.39 (brs, 1H), 5.17-5.13 (m, 1H), 4.62-4.55 (m, 2H), 4.28-4.18 (m, 6H), 3.28-3.10 (m, 8H), 2.91 (s, 3H), 2.35-2.31 (m, 1H), 2.26-2.18 (m, 1H), 1.39 (s, 9H), 1.38 (t, *J*=7.2 Hz, 3H).

### Example 216: Synthesis of Compound 416

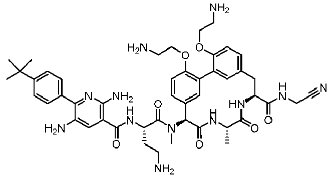

Compound 416 (formic acid salt) was prepared as a white solid by utilizing methods analogous to those described in Example 215. LCMS (Method 5-95 AB, ESI): t_{R} = 0.653 min, [M + H]⁺ = 905.4; ¹H NMR (400 MHz, MeOH-*d*₄) δ 8.40 (brs, 2H), 7.55-7.47 (m, 5H), 7.32-7.15 (m, 3H), 7.11 (d, *J*=8.8 Hz, 1H), 6.86 (s, 1H), 6.75 (s, 1H), 6.47 (brs, 1H), 5.08-5.03 (m, 1H), 4.79-4.73 (m, 2H), 4.35-4.14 (m, 6H), 3.40-3.35 (m, 1H), 3.23-3.00 (m, 7H), 2.91 (s, 3H), 2.36-2.13 (m, 2H), 1.38 (d, J=7.2 Hz, 3H), 1.37 (s, 9H).

### Example 217: Synthesis of Compound 417

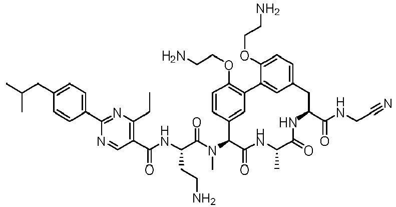

Compound 417 (formic acid salt) was prepared as a white solid from Compound **101-K** by utilizing methods analogous to those described in Example 26. LCMS (Method 5-95 AB, ESI): t_{R} = 0.749 min, [M + H]⁺ = 904.7; ¹H NMR (400 MHz, MeOH-*d*₄) δ 8.74 (s, 1H), 8.49 (brs, 1H), 8.34 (d, *J* = 8.0 Hz, 2H), 7.33-7.28 (m, 3H), 7.22-7.18 (m, 2H), 7.08 (d, *J* = 8.0 Hz, 1H), 6.89 (d, *J*=1.6 Hz, 1H), 6.77 (s, 1H), 6.42 (s, 1H), 5.19-5.16 (m,1H), 4.80-4.78 (m,2H), 4.26-4.19 (m, 6H), 3.26-2.95 (m, 13H), 2.57 (d, *J*=6.4 Hz, 2H), 2.34-2.25 (m, 1H), 2.20-2.13 (m, 1H), 1.97-1.90 (m, 1H), 1.39-1.34 (m, 6H), 0.94 (d, *J*=6.4 Hz, 6H).

### Example 218: Synthesis of Compound 418

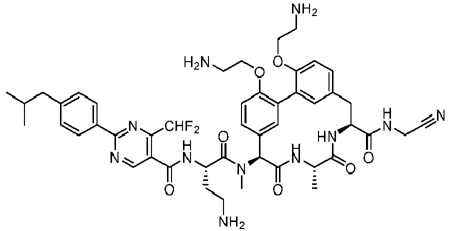

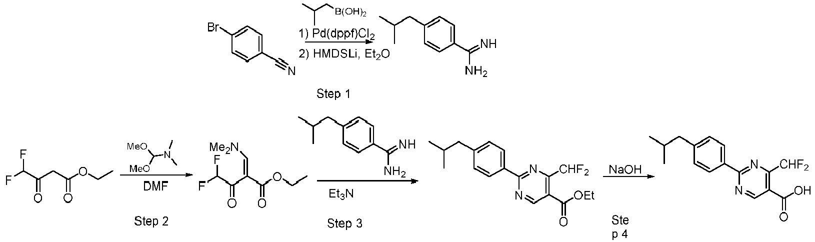

Step 1: Starting from 4-bromobenzonitrile, typical Suzuki and amidine formation conditions, analogous to those described in Example 35, were followed to give 4-isobutylbenzimidamide as a colorless oil. LCMS (Method 5-95 AB, ESI): t_{R} = 0.563 min, [M + H]⁺ = 177.0.

Step 2: Ethyl (*E*)-2-((dimethylamino)methylene)-4,4-difluoro-3-oxobutanoate was prepared as a yellow oil by utilizing methods analogous to those described in Example 26.

Step 3: A mixture of ethyl (*E*)-2-((dimethylamino)methylene)-4,4-difluoro-3-oxobutanoate (400 mg, 1.8 mmol), 4-isobutylbenzimidamide (478 mg, 2.7 mmol) and triethylamine (505 µL, 3.6 mmol) in toluene (15 mL) was stirred at 110 °C for 1 h. The volatiles were removed and the residue was purified via silica gel chromatography, eluting with 0-2% EtOAc in petroleum ether, to give ethyl 4-(difluoromethyl)-2-(4-isobutylphenyl)pyrimidine-5-carboxylate (500 mg, 83% yield) as a white solid. ¹H NMR (400 MHz, CDCl₃): δ 9.36 (s, 1H), 8.49 (d, *J*=8.4 Hz, 2H), 7.42 (t, J=54 Hz, 1H), 7.29 (d, *J*=8.4 Hz, 2H), 4.47 (q, *J*=6.8 Hz, 2H), 2.57 (d, *J*=7.2 Hz, 2H), 1.99-1.88 (m, 1H), 1.45 (t, *J*=6.8 Hz, 3H), 1.29 (d, *J*=6.4 Hz, 6H).

Step 4: Typical ester hydrolysis conditions (NaOH, MeOH/H₂O, as described in Example H) were applied to ethyl 4-(difluoromethyl)-2-(4-isobutylphenyl)pyrimidine-5-carboxylate to give 4-(difluoromethyl)-2-(4-isobutylphenyl)pyrimidine-5-carboxylic acid as a white solid.

Compound 418 (formic acid salt) was prepared as a white solid from Compound **101-K** and 4-(difluoromethyl)-2-(4-isobutylphenyl)pyrimidine-5-carboxylic acid by utilizing methods analogous to those described in Example G. LCMS (Method 5-95 AB, ESI): t_{R} = 0.762 min, [M + H]⁺ = 926.6; ¹H NMR (400 MHz, MeOH-*d*₄) δ 9.09 (s, 1H), 8.48-8.44 (m, 3H), 7.37-7.34 (m, 3H), 7.29-7.25 (m, 1H), 7.21 (d, *J*=8.0 Hz, 1H), 7.15-7.11 (m, 1H), 6.93 (d, *J*=2.4 Hz, 1H), 6.84 (s, 1H), 6.40 (s, 1H), 5.24-5.21 (m, 1H), 4.83-4.80 (m, 1H), 4.30-4.17 (m, 7H), 3.38-3.35 (m, 1H), 3.26-3.12 (m, 8H), 2.97 (s, 3H), 2.61 (d, *J*=8.0 Hz, 3H), 2.34-2.18 (m, 1H), 1.99-1.96 (m, 1H), 1.38 (d, *J*=6.8 Hz, 3H), 0.97 (d, J=6.8 Hz, 6H).

### Example 219: Synthesis of Compound 419

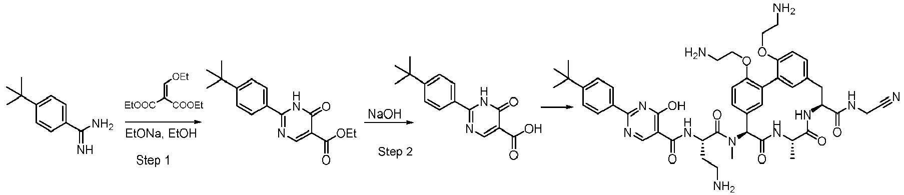

Step 1: A mixture of 4-(*tert*-butyl)benzimidamide (4.0 g, 22.7 mmol), diethyl 2-(ethoxymethylene)malonate (4.9 g, 22.7 mmol) and sodium ethoxide (1.7 g, 25.0 mmol) in EtOH (10 mL) was stirred at 60 °C for 1 h. The volatiles were removed and the residue was taken up in EtOAc (100 mL), which was washed with brine (100 mL). The organic layer was dried over MgSO₄, concentrated and the residue was purified by silica gel chromatography, eluting with 40% EtOAc in petroleum ether, to give ethyl 2-(4-(*tert*-butyl)phenyl)-6-oxo-1,6-dihydropyrimidine-5-carboxylate (800 mg, 12% yield) as a white solid. LCMS (Method 5-95 AB, ESI): t_{R} = 0.749 min, [M + H]⁺ = 300.9.

Step 2: Typical ester hydrolysis conditions (NaOH, MeOH/H₂O, as described in Example H) were applied to ethyl 2-(4-(tert-butyl)phenyl)-6-oxo-1,6-dihydropyrimidine-5-carboxylate to give 2-(4-(*tert*-butyl)phenyl)-6-oxo-1,6-dihydropyrimidine-5-carboxylic acid as a white solid.

Compound 419 (formic acid salt) was prepared as a white solid from Compound **101-K** and 2-(4-(*tert*-butyl)phenyl)-6-oxo-1,6-dihydropyrimidine-5-carboxylic acid by utilizing methods analogous to those described in Example G. LCMS (Method 5-95 AB, ESI): t_{R} = 0.693 min, [M + H]⁺ = 892.9; ¹H NMR (400 MHz, MeOH-*d*₄) δ 8.76 (s, 1H), 8.50 (brs, 1H), 8.07 (d, *J*=8.0 Hz, 2H), 7.53 (d, *J*=8.0 Hz, 2H), 7.30 (d, *J*=8.0 Hz, 1H), 7.19 (d, *J*=8.0 Hz, 1H), 7.14 (d, *J*=8.0 Hz, 1H), 7.05 (d, *J*=8.0 Hz, 1H), 6.86 (brs, 1H), 6.78 (s, 1H), 6.39 (s, 1H), 5.21-5.17 (m, 1H), 4.80-4.76 (m, 2H), 4.21-4.16 (m, 6H), 3.40-3.35 (m, 1H), 3.15-3.10 (m, 7H), 2.86 (s, 3H), 2.33-2.28 (m, 1H), 2.19-2.14 (m, 1H), 1.37 (s, 9H), 1.36 (d, *J*=7.2 Hz, 3H).

### Example 220: Synthesis of Compound 420

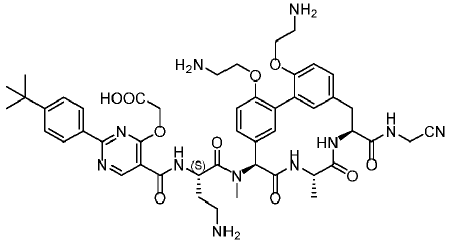

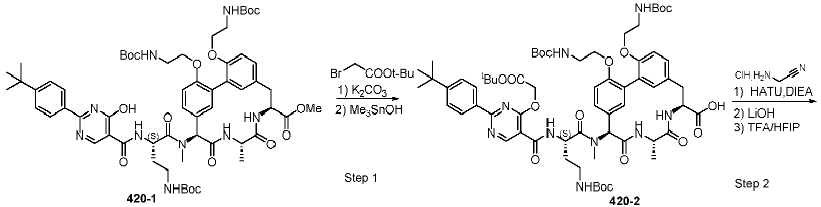

Step 1: Starting from compound 420-1 (prepared as described in Example 219), typical alkylation (as described in Example 21) and Me₃SnOH methyl ester hydrolysis (as described in Example N) conditions were followed to give compound 420-2 as a white solid.

Step 2: Starting from compound 420-2, typical amide coupling (HATU/DIEA), ester hydrolysis (LiOH, THF/H₂O) and Boc removal (TFA/HFIP) conditions, as described in Example G, were followed to give Compound 420 (formic acid salt) as a white solid. LCMS (Method 5-95 AB, ESI): t_{R} = 0.722, [M + H]⁺ = 950.5; ¹H NMR (400 MHz, MeOH-*d*₄) δ 8.97 (s, 1H), 8.43 (brs, 2H), 8.35 (d, *J*=8.0 Hz, 2H), 7.53 (d, *J*=8.0 Hz, 2H), 7.41 (d, *J*=8.0 Hz, 1H), 7.23 (d, *J*=8.4 Hz, 1H), 7.16 (d, *J*=8.0 Hz, 1H), 7.07 (d, *J*=8.4 Hz, 1H), 6.87 (s, 1H), 6.83 (s, 1H), 6.47(s, 1H), 5.17-5.13 (m, 1H), 4.82-4.71 (m, 4H), 4.35-4.16 (m, 6H), 3.29-3.08 (m, 8H), 2.88 (s, 3H), 2.45-2.30 (m, 1H), 2.26-2.10 (m, 1H), 1.36 (s, 9H), 1.35 (d, J=7.2 Hz, 3H).

### Example 221: Synthesis of Compound 421

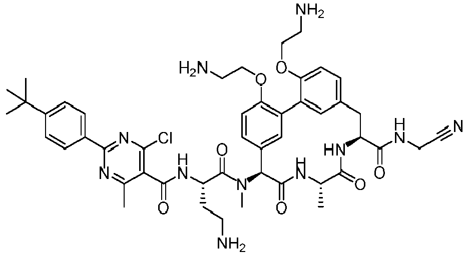

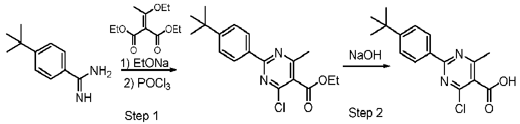

Step 1: Ethyl 2-(4-(*tert*-butyl)phenyl)-4-chloro-6-methylpyrimidine-5-carboxylate was prepared as a yellow solid by utilizing typical chlorination (POCl₃) conditions (as described in Example 53) and the methods described in Example 219. LCMS (Method 5-95 AB, ESI): t_{R} = 1.110 min, [M + H]⁺ = 332.9.

Step 2: A mixture of ethyl 2-(4-(*tert*-butyl)phenyl)-4-chloro-6-methylpyrimidine-5-carboxylate (100 mg, 0.30 mmol) and NaOH (60 mg, 1.5 mmol) in THF/H₂O (15 mL, v/v=2/1) was stirred at 20 °C for 1h. The reaction was adjusted to pH=4 using saturated aqueous KHSO₄, and then extracted with EtOAc (3 x 20 mL). The combined organic layers were washed with brine (50 mL), dried over Na₂SO₄, concentrated and the residue was purified by reverse-phase HPLC (acetonitrile 50-80% / 0.05% HCl in water) to give 2-(4-(*tert*-butyl)phenyl)-4-chloro-6-methylpyrimidine-5-carboxylic acid (60 mg, 66% yield) as a white solid. LCMS (Method 5-95 AB, ESI): t_{R} = 0.965 min, [M + H]⁺ = 304.9.

Compound 421 (trifluoroacetic acid salt) was prepared as a white solid from Compound **101-K** and 2-(4-(*tert*-butyl)phenyl)-4-chloro-6-methylpyrimidine-5-carboxylic acid by utilizing methods analogous to those described in Example G. LCMS (Method 5-95 AB, ESI): t_{R} = 0.727 min, [M + H]⁺ = 924.4; ¹H NMR (400 MHz, MeOH-*d*₄) δ 8.35 (d, *J*=8.4 Hz, 2H), 7.56 (d, *J*=8.4 Hz, 2H), 7.35-7.15 (m, 3H), 7.10 (d, *J*=8.0 Hz, 1H), 6.91 (s, 1H), 6.79 (s, 1H), 6.39 (s, 1H), 5.30-5.20 (m, 1H), 4.80-4.75 (m, 2H), 4.30-4.05 (m, 6H), 3.25-3.10 (m, 8H), 2.99 (s, 3H), 2.59 (s, 3H), 2.35-2.05 (m, 2H), 1.38 (s, 9H), 1.37 (d, J=6.8 Hz, 3H).

### Example 222: Synthesis of Compound 422

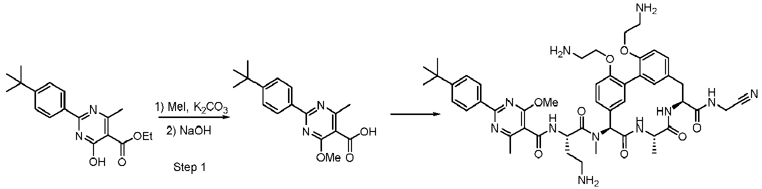

Step 1: Starting from ethyl 2-(4-(*tert*-butyl)phenyl)-4-chloro-6-methylpyrimidine-5-carboxylate (described in Example 221), typical alkylation (as described in Example 21) and ester hydrolysis conditions (NaOH, MeOH/H₂O, described in Example H) were followed to give 2-(4-(*tert*-butyl)phenyl)-4-methoxy-6-methylpyrimidine-5-carboxylic acid as a white solid. LCMS (Method 5-95 AB, ESI): t_{R} = 0.815 min, [M + H]⁺ = 300.9.

Compound 422 (formic acid salt) was prepared as a white solid from Compound **101-K** and 2-(4-(*tert*-butyl)phenyl)-4-methoxy-6-methylpyrimidine-5-carboxylic acid by utilizing methods analogous to those described in Example G. LCMS (Method 5-95 AB, ESI): t_{R} = 0.727 min, [M + H]⁺ = 920.6; ¹H NMR (400 MHz, MeOH-*d*₄) δ 8.50 (brs, 2H), 8.36 (d, *J*=8.4 Hz, 2H), 7.54 (d, *J*=8.4 Hz, 2H), 7.32-7.28 (m, 1H), 7.16-7.26 (m, 2H), 7.10 (d, *J*=8.4 Hz, 1H), 6.92 (d, *J*=2.4 Hz, 1H), 6.81 (s, 1H), 6.36 (s, 1H), 5.23-5.20 (m, 1H), 4.84-4.78 (m, 2H), 4.30-4.10 (m, 6H), 4.11 (s, 3H), 3.25-3.09 (m, 8H), 2.96 (s, 3H), 2.51 (s, 3H), 2.30-2.20 (m, 1H), 2.20-2.09 (m, 1H), 1.38 (s, 9H), 1.37 (d, J=6.8 Hz, 3H).

### Example 223: Synthesis of Compound 423

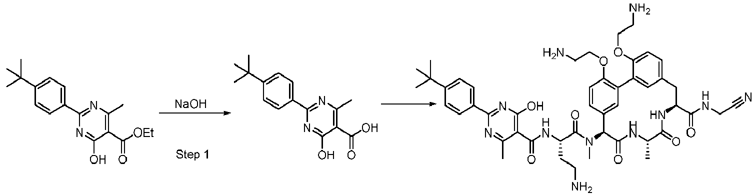

Step 1: Typical ester hydrolysis conditions (NaOH, MeOH/H₂O, as described in Example H) were applied to ethyl 2-(4-(*tert*-butyl)phenyl)-4-chloro-6-methylpyrimidine-5-carboxylate (described in Example 221) to give 2-(4-(*tert*-butyl)phenyl)-4-hydroxy-6-methylpyrimidine-5-carboxylic acid as a white solid. LCMS (Method 5-95 AB, ESI): t_{R} = 0.788 min, [M + H]⁺ = 286.9.

Compound 423 (formic acid salt) was prepared as a white solid from Compound **101-K** and 2-(4-(*tert*-butyl)phenyl)-4-hydroxy-6-methylpyrimidine-5-carboxylic acid by utilizing methods analogous to those described in Example G. LCMS (Method 5-95 AB, ESI): t_{R} = 0.690 min, [M + H]⁺ = 906.4; ¹H NMR (400 MHz, MeOH-*d*₄) δ 8.44 (brs, 1H), 7.98 (d, *J*=8.0 Hz, 2H), 7.59 (d, *J*=8.0 Hz, 2H), 7.31 (d, *J*=8.4 Hz, 1H), 7.18 (d, *J*=8.8 Hz, 2H), 7.08 (d, *J*=8.0 Hz, 1H), 6.86 (s, 1H), 6.75 (s, 1H), 6.38 (s, 1H), 5.20-5.10 (m, 1H), 4.79-4.75 (m, 1H), 4.40-4.10 (m, 7H), 3.25-3.05 (m, 8H), 2.91 (s, 3H), 2.57 (s, 3H), 2.35-2.20 (m, 1H), 2.15-2.05 (m, 1H), 1.38 (s, 9H), 1.37 (d, J=6.8 Hz, 3H).

### Example 224: Synthesis of Compound 424

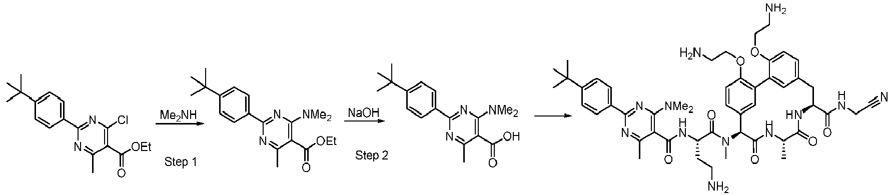

Step 1: A mixture of ethyl 2-(4-(*tert*-butyl)phenyl)-4-chloro-6-methylpyrimidine-5-carboxylate (described in Example 221, 40 mg, 0.12 mmol), Me₂NH (33 mg, 33% w/w in H₂O, 0.24 mmol) and DIEA (31 mg, 0.24 mmol) in DMF (5 mL) was stirred at 70 °C for 3 h. The volatiles were removed and the residue was taken up by EtOAc (20 mL), which was washed with brine (2 x 20 mL). The organic layer was dried over MgSO₄, concentrated and the residue was purified by preparatory-TLC (30% EtOAc in petroleum ether, R_{f} =0.5) to give ethyl 2-(4-(*tert*-butyl)phenyl)-4-(dimethylamino)-6-methylpyrimidine-5-carboxylate (30 mg, 73% yield) as a white solid. LCMS (Method 5-95 AB, ESI): t_{R} = 0.811 min, [M + H]⁺ = 342.0.

Step 2: Typical ester hydrolysis conditions (NaOH, MeOH/H₂O, as described in Example H) were applied to ethyl 2-(4-(*tert*-butyl)phenyl)-4-(dimethylamino)-6-methylpyrimidine-5-carboxylate to give 2-(4-(*tert*-butyl)phenyl)-4-(dimethylamino)-6-methylpyrimidine-5-carboxylic acid as a white solid. LCMS (Method 5-95 AB, ESI): t_{R} = 0.769 min, [M + H]⁺ = 313.9.

Compound 424 (formic acid salt) was prepared as a white solid from Compound **101-K** and 2-(4-(*tert*-butyl)phenyl)-4-(dimethylamino)-6-methylpyrimidine-5-carboxylic acid by utilizing methods analogous to those described in Example G. LCMS (Method 5-95 AB, ESI): t_{R} = 0.690 min, [M + H]⁺ = 933.6; ¹H NMR (400 MHz, MeOH-*d*₄) δ 8.50 (brs, 2H), 8.23 (d, *J*=8.4 Hz, 2H), 7.50 (d, *J*=8.4 Hz, 2H), 7.25-7.07 (m, 3H), 7.09 (d, *J*=8.4 Hz, 1H), 6.90 (d, *J*=2.4 Hz, 1H), 6.79 (s, 1H), 6.43 (s, 1H), 5.19-5.16 (m, 1H), 4.86-4.77 (m, 2H), 4.30-4.10 (m, 6H), 3.40-3.32 (m, 1H), 3.24 (s, 6H), 3.23-3.13 (m, 7H), 3.00 (s, 3H), 2.44 (s, 3H), 2.25-2.15 (m, 1H), 2.15-2.05 (m, 1H), 1.41 (s, 9H), 1.37 (d, J=7.2 Hz, 3H).

### Example 225: Synthesis of Compound 425

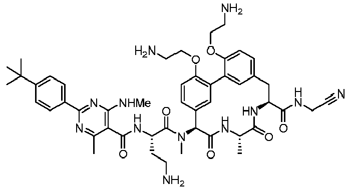

Compound 425 (formic acid salt) was prepared as a white solid from Compound **101-K** by utilizing methods analogous to those described in Example 224. LCMS (Method 5-95 AB, ESI): t_{R} = 0.690 min, [M + H]⁺ = 919.5; ¹H NMR (400 MHz, MeOH-*d*₄) δ 8.48 (brs, 2H), 8.27 (d, *J*=8.4 Hz, 2H), 7.52 (d, *J*=8.4 Hz, 2H), 7.33 (d, *J*=8.0 Hz, 1H), 7.19 (d, *J*=8.0 Hz, 1H), 7.10 (d, J=8.4 Hz, 1H), 6.91 (d, J=2.4 Hz, 1H), 6.83 (s, 1H), 6.45 (s, 1H), 5.09-5.07 (m, 1H), 4.85-4.75 (m, 2H), 4.30-4.05 (m, 6H), 3.30-3.05 (m, 11H), 2.97 (s, 3H), 2.43 (s, 3H), 2.30-2.05 (m, 2H), 1.37 (brs, 12H).

### Example 226: Synthesis of Compound 426

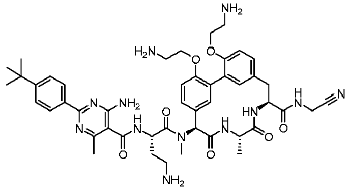

Compound 226 (trifluoroacetic acid salt) was prepared as a white solid from Compound **101-K** by utilizing the methods analogous to those described in Example 224. LCMS (Method 5-95 AB, ESI): t_{R} = 0.660 min, [M + H]⁺ = 905.5; ¹H NMR (400 MHz, MeOH-*d*₄) δ 8.20 (d, *J*=8.4 Hz, 2H), 7.50 (d, *J*=8.4 Hz, 2H), 7.33 (d, *J*=8.8 Hz, 1H), 7.25-7.15 (m, 2H), 7.09 (d, *J*=8.8 Hz, 1H), 6.90 (d, *J*=2.4 Hz, 1H), 6.81 (s, 1H), 6.44 (s, 1H), 5.15-5.10 (m, 1H), 4.85-4.75 (m, 2H), 4.30-4.10 (m, 6H), 3.40-3.35 (m, 1H), 3.25-3.05 (m, 7H), 2.97 (s, 3H), 2.45 (s, 3H), 2.25-2.05 (m, 2H), 1.41 (s, 9H), 1.36 (d, *J*=7.2 Hz, 3H).

### Example 227: Synthesis of Compound 427

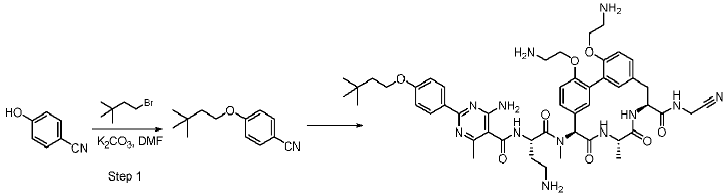

Step 1: Typical alkylation condition (as described in Example 21) was applied to 4-hydroxybenzonitrile to give 4-(3,3-dimethylbutoxy)benzonitrile as a colorless oil. ¹H NMR (400 MHz, CDCl₃) δ 7.55 (d, *J*=8.8 Hz, 2H), 6.93 (d, *J*=8.8 Hz, 2H), 4.06 (t, *J*=7.2 Hz, 2H), 1.74 (t, *J*=7.2 Hz, 2H), 0.99 (s, 3H).

Compound 427 (formic acid salt) was prepared as a white solid from Compound **101-K** and 4-(3,3-dimethylbutoxy)benzonitrile by utilizing methods analogous to those described in Examples 35, 221, and 226. LCMS (Method 5-95 AB, ESI): t_{R} = 0.711 min, [M + H]⁺ = 949.5; ¹H NMR (400 MHz, MeOH-*d*₄) δ 8.45 (brs, 1H), 8.24-8.13 (m, 2H), 7.35-7.29 (m, 1H), 7.24-7.17 (m, 2H), 7.10 (d, *J*=8.4 Hz, 1H), 7.00-6.93 (m, 2H), 6.89 (s, 1H), 6.76 (brs, 1H), 6.51 (br s, 1H), 5.10-5.05 (m, 1H), 4.86-4.78 (m, 2H), 4.30-4.16 (m, 6H), 4.12 (t, *J*=7.2 Hz, 2H), 3.28-3.24 (m, 4H), 3.17-3.00 (m, 4H), 2.95 (s, 3H), 2.45-2.37 (m, 3H), 2.29-2.10 (m, 2H), 1.75 (t, *J*=7.2 Hz, 2H), 1.47-1.32 (m, 3H), 1.03 (s, 9H).

### Example 228: Synthesis of Compound 428

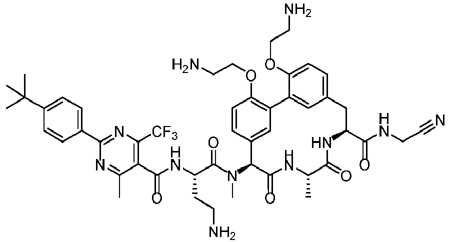

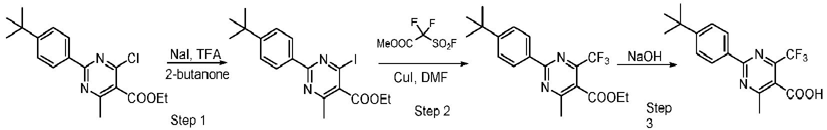

Step 1: A mixture of ethyl 2-(4-(*tert*-butyl)phenyl)-4-chloro-6-methylpyrimidine-5-carboxylate (described in Example 221, 500 mg, 1.5 mmol), NaI (4.5 g, 30 mmol), and TFA (856 mg, 7.5 mmol) in 2-butanone (3 mL) was stirred at 60 °C for 2h. The volatiles were removed and the residue was taken up in EtOAc (50 mL), which was washed with brine (2 x 50 mL). The organic layer was dried over MgSO₄, concentrated and the residue was purified by silica gel chromatography, eluting 20% EtOAc in petroleum ether, to give ethyl 2-(4-(*tert*-butyl)phenyl)-4-iodo-6-methylpyrimidine-5-carboxylate (400 mg, 63% yield) as a colorless oil. LCMS (Method 5-95 AB, ESI): t_{R} = 1.160 min, [M + H]⁺ = 424.9.

Step 2: A mixture of ethyl 2-(4-(*tert*-butyl)phenyl)-4-iodo-6-methylpyrimidine-5-carboxylate (50 mg, 0.12 mmol), methyl 2,2-difluoro-2-(fluorosulfonyl)acetate (57 mg, 0.30 mmol), and CuI (34 mg, 0.18 mmol) in DMF (2 mL) was stirred at 80 °C for 15 h. The volatiles were removed and the residue was taken up in EtOAc (20 mL), which was washed with brine (2 x 20 mL). The organic layer was dried over MgSO₄, concentrated and the residue was purified by preparatory-TLC (20% EtOAc in petroleum ether, R_{f} = 0.6) to give ethyl 2-(4-(*tert*-butyl)phenyl)-4-methyl-6-(trifluoromethyl)pyrimidine-5-carboxylate (25 mg, 58% yield) as a colorless oil. LCMS (Method 5-95 AB, ESI): t_{R} = 1.150 min, [M + H]⁺ = 367.0.

Step 3: Typical ester hydrolysis conditions (NaOH, MeOH/H₂O, described in Example H) were applied to ethyl 2-(4-(*tert*-butyl)phenyl)-4-methyl-6-(trifluoromethyl)pyrimidine-5-carboxylate to give 2-(4-(tert-butyl)phenyl)-4-methyl-6-(trifluoromethyl)pyrimidine-5-carboxylic acid as a white solid. LCMS (Method 5-95 AB, ESI): t_{R} = 1.020 min, [M + H]⁺ = 339.0.

Compound 428 (formic acid salt) was prepared as a white solid from Compound **101-K** and 2-(4-(*tert*-butyl)phenyl)-4-methyl-6-(trifluoromethyl)pyrimidine-5-carboxylic acid by utilizing methods analogous to those described in Example G. LCMS (Method 5-95 AB, ESI): t_{R} = 0.750 min, [M + H]⁺ = 958.5; ¹H NMR (400 MHz, MeOH-*d*₄) δ 8.42 (d, *J*=8.4 Hz, 2H), 7.58 (d, *J*=8.4 Hz, 2H), 7.30-7.19 (m, 3H), 7.10 (d, *J*=8.0 Hz, 1H), 6.90 (s, 1H), 6.79 (s, 1H), 6.40 (s, 1H), 5.26-5.23 (m, 1H), 4.80-4.75 (m, 1H), 4.35-4.10 (m, 7H), 3.33-3.09 (m, 8H), 3.00 (s, 3H), 2.40-2.20 (m, 1H), 2.20-2.05 (m, 1H), 1.39 (s, 9H), 1.35 (d, *J*=6.8 Hz, 3H).

### Example 229: Synthesis of Compound 429

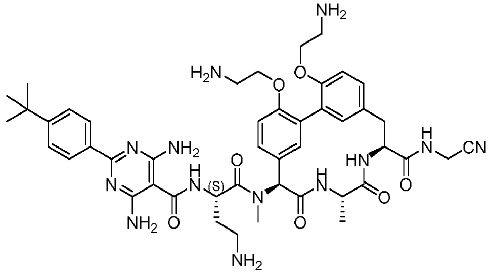

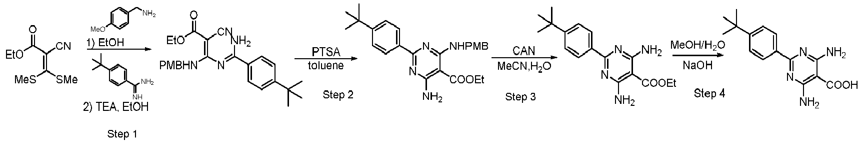

Step 1: A solution of ethyl 2-cyano-3,3-bis(methylthio)acrylate (900 mg, 4.1 mmol) and (4-methoxyphenyl)methanamine (682 mg, 5.0 mmol) in EtOH (16 mL) was stirred at 60 °C for 2 h. After cooling the mixture to 0 °C, 4-(*tert*-butyl)benzimidamide (725 mg, 4.1 mmol) and triethylamine (1.71 mL, 12.3 mmol) were added. The resulting mixture was stirred at 60 °C for 22 h. The volatiles were removed under reduced pressure and the residue was taken up by EtOAc (40 mL), which was washed with brine (40 mL). The organic layer was dried over Na₂SO₄ and the residue was purified by preparatory reverse-phase HPLC to give (*E*)-ethyl 3-((*Z*)-(amino(4-(*tert*-butyl)phenyl)methylene)amino)-2-cyano-3-((4-methoxybenzyl)amino)acrylate (220 mg, 17.5% yield) as a white solid.

Step 2: A solution of (*E*)-ethyl 3-((*Z*)-(amino(4-(*tert*-butyl)phenyl)methylene)amino)-2-cyano-3-((4-methoxybenzyl)amino)acrylate (250 mg, 0.58 mmol) and *p*-TsOH (5.0 mg, 0.03 mmol) in toluene (8 mL) was stirred at 110 °C for 3 days under N₂. The volatiles were removed under reduced pressure and the residue was taken up by EtOAc (40 mL), which was washed with brine (40 mL). The organic layer was dried over Na₂SO₄ and the residue was purified by preparatory-TLC (eluent: EtOAc: petroleum ether = 1:3, Rf =0.5) to give ethyl 4-amino-2-(4-(*tert*-butyl)phenyl)-6-((4-methoxybenzyl)amino)pyrimidine-5-carboxylate (130 mg, 52% yield) as a white solid. LCMS (Method 5-95 AB, ESI): t_{R} = 0.809 min, [M + H]⁺ = 435.1.

Step 3: A solution of ethyl 4-amino-2-(4-(*tert*-butyl)phenyl)-6-((4-methoxybenzyl)amino)pyrimidine-5-carboxylate (120 mg, 0.28 mmol) and (NH₄)₂Ce(NO₃)₆ (454 mg, 0.83 mmol) in MeCN/H₂O (0.4 mL, v/v=1/1) was stirred at 0 °C for 30 min. The reaction was washed with saturated Na₂CO₃ (15 mL), which was extracted with EtOAc (3 x 15 mL). The combined organic layers were concentrated and the residue was purified by preparatory-TLC (EtOAc: petroleum ether= 1:3) to afford ethyl 4,6-diamino-2-(4-(*tert*-butyl)phenyl)pyrimidine-5-carboxylate (15 mg, 17% yield). Step 4: Typical ester hydrolysis conditions (NaOH, MeOH/H₂O, described in Example H) were applied to ethyl 4,6-diamino-2-(4-(*tert*-butyl)phenyl)pyrimidine-5-carboxylate to give 4,6-diamino-2-(4-(*tert-*butyl)phenyl)pyrimidine-5-carboxylic acid as a white solid. LCMS (Method 5-95 AB, ESI): t_{R} = 0.902 min, [M + H]⁺ = 286.9.

Compound 429 (formic acid salt) was prepared as a white solid from Compound **101-K** and 2-(4-(*tert*-butyl)phenyl)-4-methyl-6-(trifluoromethyl)pyrimidine-5-carboxylic acid by utilizing methods analogous to those described in Example G. LCMS (Method 5-95 AB, ESI): t_{R} = 0.676 min, [M + H]⁺ = 906.6; ¹H NMR (400 MHz, MeOH-*d*₄) δ 8.34 (brs, 1H), 8.14 (d, *J*=8.8 Hz, 1H), 8.07 (d, *J*=8.0 Hz, 1H), 7.47 (t, *J*=8.8 Hz, 2H), 7.34-7.28 (m, 2H), 7.21-7.18 (m, 2H), 7.11-7.06 (m, 2H), 6.89-6.77 (m, 1H), 6.51-6.48 (m, 1H), 5.17-5.10 (m, 1H), 4.80-4.72 (m, 2H), 4.30-4.05 (m, 4H), 4.17 (s, 2H), 3.28-2.90 (m, 11H), 2.26-2.18 (m, 1H), 2.16-2.10 (m, 1H), 1.42 (s, 9H), 1.41 (d, J=6.8 Hz, 3H).

### Example 230: Synthesis of Compound 430

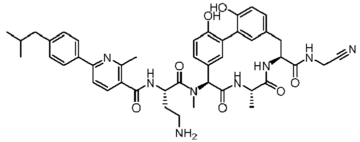

Compound 430 (trifluoroacetic acid salt) was prepared as a white solid by utilizing methods analogous to those described in Example 5 and Example O. LCMS (Method 5-95 AB, ESI): t_{R} = 0.791 min, [M + H]⁺ = 803.5; ¹H NMR (400 MHz, MeOH-*d*₄) δ 7.86 (d, *J*=8.0 Hz, 2H), 7.74 (d, J=8.0 Hz, 1H), 7.67 (d, *J*=8.0 Hz, 1H), 7.27 (d, *J*=8.0 Hz, 2H), 7.16 (d, *J*=8.0 Hz, 1H), 7.08-6.91 (m, 4H), 6.91 (s, 1H), 6.86 (s, 1H), 6.47 (s, 1H), 5.20-5.15 (m, 1H), 4.68-4.57 (m, 2H), 4.20 (s, 2H), 3.17-3.05 (m, 4H), 2.93 (s, 3H), 2.68 (s, 3H), 2.55 (d, *J*=6.8 Hz, 2H), 2.33-2.24 (m, 1H), 2.20-2.11 (m, 1H), 1.96-1.87 (m, 1H), 1.36 (d, *J*=6.4 Hz, 3H), 0.94 (d, *J*=6.4 Hz, 6H).

### Example 231: Synthesis of Compound 431

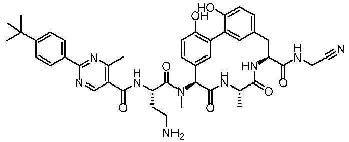

Compound 431 (formic acid salt) was prepared as a white solid by utilizing methods analogous to those described in Example J and Example O. LCMS (Method 5-95 AB, ESI): t_{R} = 0.814 min, [M + H]⁺ = 804.4; ¹H NMR (400 MHz, MeOH-*d*₄) δ 8.80 (s, 1H), 8.50 (br s, 1H), 8.23 (d, *J*=8.0 Hz, 2H), 8.51 (d, *J*=8.0 Hz, 2H), 7.20-7.02 (m, 3H), 6.99 (d, *J*=8.0 Hz, 1H), 6.93 (s, 1H), 6.86 (s, 1H), 6.75 (s, 1H), 5.21-5.15 (m, 1H), 4.83-4.77 (m, 1H), 4.60-4.54 (m, 1H), 4.27 (s, 2H), 3.20-2.85 (m, 4H), 2.96 (s, 3H), 2.55 (s, 3H), 2.32-2.19 (m, 2H), 1.45 (s, 9H), 1.39 (d, *J*=6.8 Hz, 3H).

### Example 232: Synthesis of Compound 432

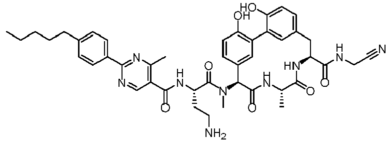

Compound 432 (trifluoroacetic acid salt) was prepared as a white solid by utilizing methods analogous to those described in Example O. LCMS (Method 5-95 AB, ESI): t_{R} = 0.833 min, [M + H]⁺ = 818.5; ¹H NMR (400 MHz, MeOH-*d*₄) δ 8.80 (s, 1H), 8.28 (d, *J*=7.6 Hz, 2H), 7.31 (d, *J*=7.6 Hz, 2H), 7.13 (d, *J*=7.6 Hz, 1H), 7.05-6.90 (m, 3H), 6.87-6.78 (m, 2H), 6.53 (s, 1H), 5.19-5.11 (m, 1H), 4.70-4.50 (m, 2H), 4.23 (s, 2H), 3.20-3.09 (m, 2H), 3.07-2.99 (m, 2H), 2.95 (s, 3H), 2.73-2.65 (m, 2H), 2.62 (s, 3H), 2.29-2.20 (m, 1H), 2.18-2.12 (m, 1H), 1.72-1.59 (m, 2H), 1.39-1.29 (m, 7H), 0.94 (br s, 3H).

### Example 233: Synthesis of Compound 433

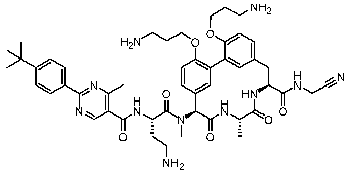

Compound 433 (formic acid salt) was prepared as a white solid by utilizing methods analogous to those described in Example J and Example O. LCMS (Method 5-95 AB, ESI): t_{R} = 0.731 min, [M + H]⁺ = 918.8; ¹H NMR (400 MHz, MeOH-*d*₄) δ 8.79 (s, 1H), 8.52 (br s, 2H), 8.37 (d, *J*=8.8 Hz, 2H), 7.55 (d, *J*=8.0 Hz, 2H), 7.31-7.28 (m, 1H), 7.20-7.17 (m, 2H), 7.13 (d, J=8.8 Hz, 1H), 7.02 (d, *J*=8.0 Hz, 1H), 6.84 (d, *J*=3.2 Hz, 1H), 6.73 (s, 1H), 6.38 (s, 1H), 5.19-5.16 (m, 1H), 4.82-4.75 (m, 2H), 4.26-4.02 (m, 4H), 4.20 (s, 2H), 3.40-3.30 (m, 1H), 3.14-3.06 (m, 3H), 2.99-2.93 (m, 4H), 2.97 (s, 3H), 2.69 (s, 3H), 2.31-2.00 (m, 4H), 1.38 (s, 9H), 1.35 (d, *J*=7.2 Hz, 3H).

### Example 234: Synthesis of Compound 434

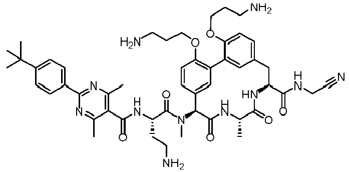

Compound 434 (formic acid salt) was prepared as a white solid by utilizing methods analogous to those described in Example 53 and Example O. LCMS (Method 5-95 AB, ESI): t_{R} = 0.602 min, [M + H]⁺ = 932.4; ¹H NMR (400 MHz, MeOH-*d*₄) δ 8.51 (br s, 2H), 8.27 (d, *J*=8.8 Hz, 2H), 7.50 (d, *J*=8.8 Hz, 2H), 7.26 (d, *J*=8.4 Hz, 1H), 7.15 (d, *J*=8.8 Hz, 2H), 7.02 (d, *J*=8.4 Hz, 1H), 6.82 (s, 1H), 6.63 (s, 1H), 6.48 (s, 1H), 5.27-5.23 (m, 1H), 4.77-4.74 (m, 2H), 4.28-4.04 (m, 4H), 4.22 (s, 2H), 3.12 (t, J=7.9 Hz, 2H), 3.08-2.97 (m, 6H), 3.03 (s, 3H), 2.52 (s, 6H), 2.32-2.24 (m, 1H), 2.19-2.03 (m, 4H), 1.38 (s, 9H), 1.34 (d, *J*=6.8 Hz, 3H).

### Example 235: Synthesis of Compound 435

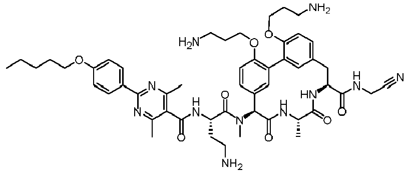

Compound 435 (formic acid salt) was prepared as a white solid by utilizing methods analogous to those described in Example 128 and Example O. LCMS (Method 5-95 AB, ESI): t_{R} = 0.626 min, [M + H]⁺ = 962.4; ¹H NMR (400 MHz, MeOH-*d*₄) δ 8.51 (br s, 2H), 8.25 (d, *J*=8.4 Hz, 2H), 7.28-7.24 (m, 1H), 7.16 (d, *J*=8.4 Hz, 1H), 7.10 (br s, 1H), 7.03 (d, *J*=8.4 Hz, 1H), 6.96 (d, *J*=8.4 Hz, 2H), 6.82 (s, 1H), 6.58 (s, 1H), 6.53 (s, 1H), 5.30-5.23 (m, 1H), 4.81-4.75 (m, 2H), 4.32-4.19 (m, 4H), 4.08-4.04 (m, 4H), 3.13 (t, *J*=7.6 Hz, 2H), 3.11-2.92 (m, 6H), 3.03 (s, 3H), 2.47 (s, 6H), 2.31-2.23 (m, 1H), 2.19-2.16 (m, 1H), 2.14-1.99 (m, 4H), 1.87-1.78 (m, 2H), 1.53-1.41 (m, 4H), 1.34 (d, *J*=6.8 Hz, 3H), 0.98 (t, *J*=7.2Hz, 3H).

### Example 236: Synthesis of Compound 436

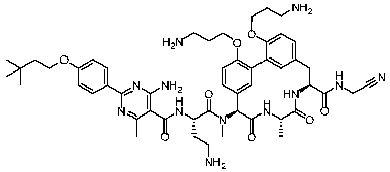

Compound 436 (formic acid salt) was prepared as a white solid by utilizing methods analogous to those described in Example 226 and Example O. LCMS (Method 5-95 AB, ESI): t_{R} = 0.722 min, [M + H]⁺ = 977.4; ¹H NMR (400 MHz, MeOH-*d*₄) δ 8.46 (br s, 1H), 8.24 (d, *J*=8.8 Hz, 2H), 7.32-7.29 (m, 1H), 7.21 (d, *J*=8.4 Hz, 1H), 7.12 (d, *J*=8.8 Hz, 1H), 7.03 (d, *J*=8.4 Hz, 1H), 6.98 (d, *J*=8.8 Hz, 2H), 6.85 (s, 1H), 6.76 (s, 1H), 6.42 (s, 1H), 5.12-5.05 (m, 1H), 4.79-4.75 (m, 2H), 4.29-4.03 (m, 8H), 3.40-3.30 (m, 2H), 3.15-2.85 (m, 6H), 2.94 (s, 3H), 2.44 (s, 3H), 2.30-1.98 (m, 2H), 1.75 (t, *J*=7.2 Hz, 2H), 1.40-1.30 (m, 4H), 1.02 (s, 9H).

### Example 237: Synthesis of Compound 437

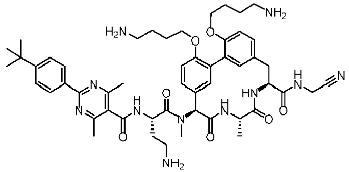

Compound 437 (formic acid salt) was prepared as a white solid by utilizing methods analogous to those described in Example 53 and Example O. LCMS (Method 5-95 AB, ESI): t_{R} = 0.724 min, [M + H]⁺ = 960.5; ¹H NMR (400 MHz, MeOH-*d*₄) δ 8.53 (br s, 2H), 8.23 (d, *J*=8.0 Hz, 2H), 7.47 (d, *J*=8.0 Hz, 2H), 7.23 (d, *J*=8.0 Hz, 1H), 7.13 (d, *J*=8.0 Hz, 1H), 7.00-6.97 (m, 2H), 6.79 (d, *J*=2.4 Hz, 1H), 6.56 (s, 1H), 6.51 (s, 1H), 5.30-5.27 (m, 1H), 4.79-4.74 (m, 2H), 4.21 (s, 2H), 4.19-4.00 (m, 4H), 3.11 (t, *J*=8.0 Hz, 2H), 3.02 (s, 3H), 2.97-2.84 (m, 6H), 2.47 (s, 6H), 2.32-2.23 (m, 1H), 2.18-2.09 (m, 1H), 1.88-1.72 (m, 8H), 1.39 (s, 9H), 1.34 (d, *J*=7.2 Hz, 3H).

### Example 238: Synthesis of Compound 438

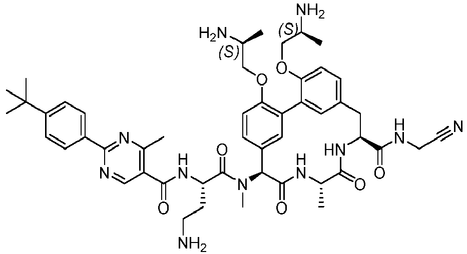

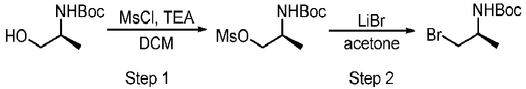

Step 1: To a solution of *tert*-butyl-(*S*)-2-hydroxy-1-methylethylcarbamate (3.9 g, 22.3 mmol) and triethylamine (5.6 g, 55.6 mmol) in DCM (30 mL) was dropwise added methanesulfonyl chloride (3.77 g, 32.9 mmol) at 0 °C. The resulting mixture was gradually warmed up while stirring and stirred at 20 °C for 6 h. The reaction was diluted with DCM (40 mL), which was washed with saturated aqueous NaHCO₃ and brine (each 40 mL). The organic layer was dried over Na₂SO₄, concentrated and the residue was purified on silica gel column, eluting with 0-20% EtOAc in petroleum ether, to give (S)-2-(*tert-*butoxycarbonylamino)propyl methanesulfonate (3.0 g, 53% yield) as a white solid.

Step 2: A solution of (*S*)-2-(*tert*-butoxycarbonylamino)propyl methanesulfonate (3.0 g, 11.8 mmol) and LiBr (4.1 g, 47.4 mmol) in acetone (10 mL) was stirred at 25 °C for 16 h. The reaction was diluted with EtOAc (40 mL) and washed with saturated aqueous NaHCO₃ and brine (each 40 mL). The organic layer was dried over Na₂SO₄, concentrated and the residue was purified on silica gel column, eluting with 0-20% EtOAc in petroleum ether, to give *tert*-butyl *N*-(*S*)-2-bromo-1-methyl-ethyl carbamate (1.8 g, 64% yield) as a white solid.

Compound 438 (formic acid salt) was prepared as a white solid from *tert*-butyl *N*-(*S*)-2-bromo-1-methyl-ethyl carbamate utilizing methods analogous to those described in Example J and Example O. LCMS (Method 5-95 AB, ESI): t_{R} = 0.733 min, [M + H]⁺ = 918.5; ¹H NMR (400 MHz, MeOH-*d*₄) δ 8.72 (s, 1H), 8.49 (br s, 3H), 8.24 (d, *J*=8.4 Hz, 2H), 7.52 (d, *J*=8.4 Hz, 2H), 7.32 (d, *J*=8.4 Hz, 1H), 7.24 (d, *J*=8.4 Hz, 1H), 7.15-7.05 (m, 2H), 6.84 (s, 1H), 6.58 (br s, 2H), 5.25-5.15 (m, 1H), 4.80-4.65 (m, 2H), 4.25-4.00 (m, 4H), 4.20 (s, 2H), 3.65-3.45 (m, 2H), 3.20-3.00 (m, 4H), 2.97 (s, 3H), 2.65 (s, 3H), 2.35-2.15 (m, 2H), 1.39 (s, 9H), 1.36 (d, *J*=7.2 Hz, 3H), 1.34 (d, *J*=7.2 Hz, 3H), 1.23 (d, *J*=6.4 Hz, 3H).

### Example 239: Synthesis of Compound 439

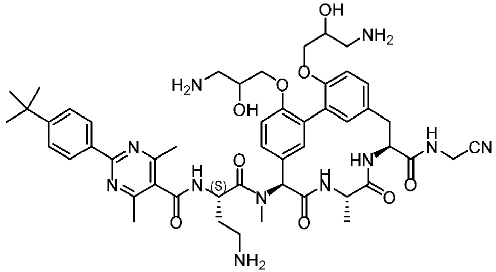

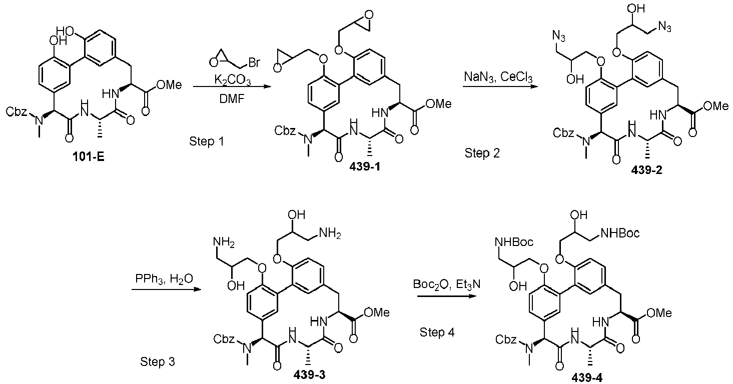

Step 1: A mixture of compound 101-E (300 mg, 0.53 mmol), 2-(bromomethyl)oxirane (732 mg, 5.3 mmol) and K₂CO₃ (738 mg, 5.3 mmol) in DMF (10 mL) was stirred at 50 °C for 4 h. The reaction was taken up by EtOAc (100 mL), which was washed with brine (3 x 100 mL). The organic layer was dried over Na₂SO₄, concentrated, and the residue was purified by preparatory reverse-phase HPLC (water (0.225% formic acid)-acetonitrile) to give compound 439-1 (200 mg, 56% yield) as a white solid. LCMS (Method 5-95 AB, ESI): t_{R} = 0.868 min, [M + H]⁺ = 674.3.

Step 2: A solution of compound 439-1 (300 mg, 0.45 mmol), sodium azide (675 mg, 10.3 mmol) and CeCl₃ (56 mg, 0.22 mmol) in acetonitrile (9 mL) was stirred at 75 °C for 12 h. The reaction mixture was taken up by EtOAc (50 ml), which was washed with saturated aqueous Na₂CO₃ solution (30 mL). The organic layer was dried over Na₂SO₄, concentrated and the residue was purified by preparatory-TLC (eluting with 10% MeOH in DCM, Rf=0.4) to give compound 439-2 (175 mg, 48% yield) as a white solid. LCMS (Method 5-95 AB, ESI): t_{R} = 0.883 min, [M + H]⁺ = 760.2.

Step 3: A solution of compound 439-2 (175 mg, 0.23 mmol) and PPh₃ (104 mg, 0.39 mmol) in THF/H₂O (11 mL, v/v=10/1) was stirred at 50 °C for 12 h. The reaction mixture was taken up by EtOAc (50 mL), which was washed with saturated aqueous Na₂CO₃ solution (30 mL). The organic layer was dried over Na₂SO₄, concentrated and the residue was purified by preparatory-TLC (eluting with 10% MeOH in DCM, Rf=0.5) to give compound 439-3 (165 mg, 99% yield) as a white solid. LCMS (Method 5-95 AB, ESI): t_{R} = 0.694 min, [M + H]⁺ = 708.3.

Step 4: A solution of compound 439-3 (165 mg, 0.23 mmol), Boc₂O (109 mg, 0.50 mmol) and triethylamine (60 mg, 0.59 mmol) in DCM (5 mL) was stirred at 20 °C for 12 h. The volatiles were removed under reduced pressure and the residue was taken up in EtOAc (30 mL), which was washed with brine (2 x 30 mL). The organic layer was dried over Na₂SO₄, concentrated and the residue was then purified by preparatory-TLC (eluting with 10% MeOH in DCM, Rf=0.3) to give compound 439-4 (150 mg, 70% yield) as a white solid. LCMS (Method 5-95 AB, ESI): t_{R} = 0.908 min, [M + Na]⁺ = 930.5.

Compound 439 (formic acid salt) was prepared as a white solid from compound 439-4 and utilizing methods analogous to those described in Example 53. LCMS (Method 5-95 AB, ESI): t_{R} = 0.720 min, [M + H]⁺ = 964.5; ¹H NMR (400 MHz, MeOH-*d*₄) δ 8.53 (br s, 2H), 8.20 (d, *J*=8.0 Hz, 2H), 7.47 (d, *J*=8.0 Hz, 2H), 7.28-7.20 (m, 2H), 7.02 (d, *J*=8.0 Hz, 2H), 6.80 (s, 1H), 6.61 (s, 1H), 6.49 (s, 1H), 5.36-5.20 (m, 1H), 4.82-4.60 (m, 2H), 4.30-3.90 (m, 6H), 4.20 (s, 2H), 3.20-2.60 (m, 11H), 2.46 (s, 6H), 2.35-2.23 (m, 1H), 2.18-2.10 (m, 1H), 1.39 (s, 9H), 1.34 (d, *J*=6.8 Hz, 3H).

### Example 240: Synthesis of Compound 440

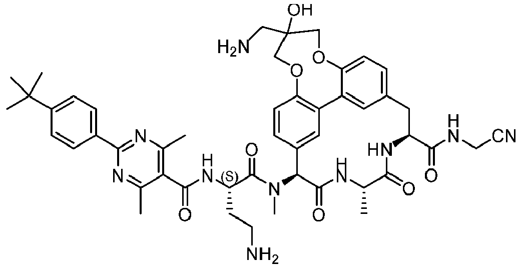

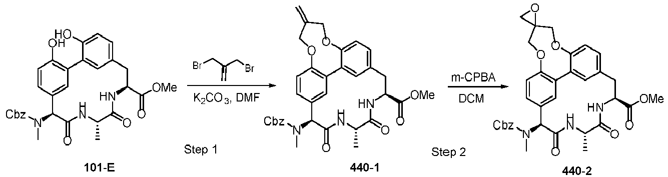

Step 1: A mixture of compound 101-E (300 mg, 0.53 mmol), 3-bromo-2-(bromoethyl)- 1-propene (0.31 mL, 2.7 mmol) and K₂CO₃ (369 mg, 2.7 mmol) in DMF (5 mL) was stirred at 25 °C for 3 h. The reaction was taken up by EtOAc (30 mL), which was washed with brine (2 x 30 mL). The organic layer was dried over Na₂SO₄, concentrated and the residue was purified by preparatory-TLC (eluting with 2% MeOH in DCM, Rf=0.3) to give compound 440-1 (300 mg, 92% yield) as a white solid. LCMS (Method 5-95 AB, ESI): t_{R} = 0.906 min, [M + H]⁺ = 614.1.

Step 2: A solution of compound 440-1 (150 mg, 0.24 mmol) and *m*-CPBA (422 mg, 2.4 mmol) in DCM (20 mL) was stirred at 0 °C for 30 min, then warmed up to 20 °C while stirring and stirred for 12 h at the same temperature. The mixture was washed with saturated aqueous NaHCO₃ and brine (each 30 mL). The organic layer was dried over Na₂SO₄, concentrated and the residue was purified by preparatory-TLC (eluting with 3% MeOH in DCM, Rf=0.3) to give compound 440-2 (120 mg, 78% yield) as a yellow solid. LCMS (Method 5-95 AB, ESI): t_{R} = 0.759 min, [M + Na]⁺ = 652.1.

Compound 440 (trifluoroacetic acid salt) was prepared as a white solid from compound 440-2 and utilizing methods analogous to those described in Example 239. LCMS (Method 5-95 AB, ESI): t_{R} = 0.751 min, [M + H]⁺ = 903.4; ¹H NMR (400 MHz, MeOH-*d*₄) δ 8.32-8.20 (m, 2H), 7.53-7.48 (m, 2H), 7.45-7.10 (m, 4H), 7.08 (br s, 1H), 6.66 (br s, 1H), 6.61 (br s, 1H), 5.31-5.28 (m, 1H), 4.85-4.75 (m, 2H), 4.65-4.55 (m, 2H), 4.49-4.45 (m, 3H), 4.31-4.24 (m, 3H), 3.15 (t, *J*=6.8 Hz, 2H), 3.06-2.89 (m, 2H), 2.99 (s, 3H), 2.51 (br s, 6H), 2.30-2.15 (m, 2H), 1.40 (s, 9H), 1.37 (d, *J*=6.8 Hz, 3H).

### Example 241: Synthesis of Compound 441

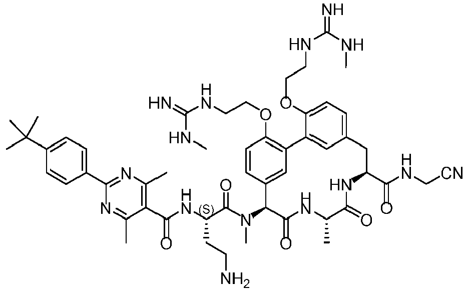

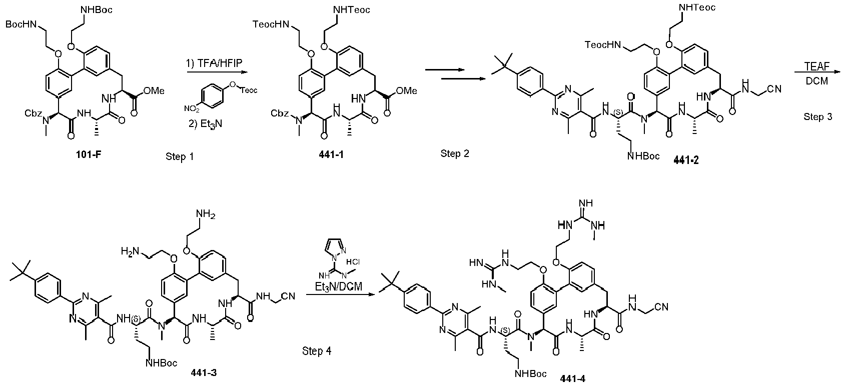

Step 1: A solution of compound 101-F (200 mg, 0.24 mmol) in 5% TFA in HFIP (5 mL) was stirred at 20 °C for 1 h. The volatiles were removed under reduced pressure and the residue was added to a solution of 4-nitrophenyl (2-(trimethylsilyl)ethyl) carbonate (230 mg, 0.81 mmol) and Et₃N (352 mg, 3.5 mmol) in DCM (20 mL). The resulting mixture was stirred at 20 °C for 16 h. The reaction was quenched with water (30 mL) and the organic layer was washed with brine (30 mL), dried over Na₂SO₄, concentrated and the residue was purified by preparatory TLC (eluting with 10% MeOH in DCM, Rf=0.3) to give compound 441-1 (200 mg, 92% yield) as a yellow solid. LCMS (Method 5-95 AB, ESI): t_{R} = 1.097 min, [M + Na]⁺ = 958.5.

Step 2: Compound 441-2 was prepared as a white solid from compound 441-1 and utilizing methods analogous to those described in Example 53. LCMS (Method 5-95 AB, ESI): t_{R} = 1.204 min, [M + H]⁺ = 1294.0.

Step 3: A solution of compound 441-2 (50 mg, 0.04 mmol) and tetrabutylammonium fluoride (69 mg, 0.31 mmol) in DMF (3 mL) was stirred at 50 °C for 1.5 h. The reaction was diluted with EtOAc (50 mL), which was washed with brine (2 x 50 mL). The organic layer was dried over Na₂SO₄ and evaporated in vacuo to give compound 441-3 (38 mg, 0.038 mmol, 98% yield) as a white solid, which was used directly in the next step. LCMS (Method 5-95 AB, ESI): t_{R} = 0.814 min, [M + H]⁺ = 1004.7.

Step 4: A solution of compound 441-3 (38 mg, 0.040 mmol), *N*-methylpyrazole-1-carboxamidine (19 mg, 0.15 mmol) and Et₃N (57 mg, 0.57 mmol) in DCM (2 mL) was stirred at 20 °C for 2 days. The reaction was quenched with water (10 mL), which was extracted with EtOAc (3 x 15 mL). The combined organic layers were washed with brine (50 mL), dried over Na₂SO₄, concentrated and the residue was purified by preparatory-TLC (eluting with 10% MeOH in DCM, Rf=0.1) to give compound 441-4 (20 mg, 45% yield) as a white solid. LCMS (Method 5-95 AB, ESI): t_{R} = 0.699 min, [M + H]⁺ = 1116.6.

Step 5: Typical Boc removal condition (TFA/HFIP, described in Example G) was applied to compound 441-4 to give compound 440 (formic acid salt) as a white solid. LCMS (Method 5-95 AB, ESI): t_{R} = 0.723 min, [M + H]⁺ = 1016.6; ¹H NMR (400 MHz, MeOH-*d*₄) δ 8.50 (br s, 1H), 8.34 (d, *J*=8.4 Hz, 2H), 7.55 (d, *J*=8.4 Hz, 2H), 7.37-7.00 (m, 4H), 6.89 (s, 1H), 6.77 (s, 1H), 6.43 (s, 1H), 5.23-5.19 (m, 1H), 4.85-4.76 (m, 2H), 4.25-4.00 (m, 4H), 4.21 (s, 2H), 3.73-3.42 (m, 4H), 3.27-2.95 (m, 7H), 2.67 (s, 3H), 2.62 (s, 6H), 2.60 (s, 3H), 2.34-2.10 (m, 2H), 1.38 (s, 9H), 1.31 (d, *J*=7.2 Hz, 3H).

### Example 242: Synthesis of Compound 442

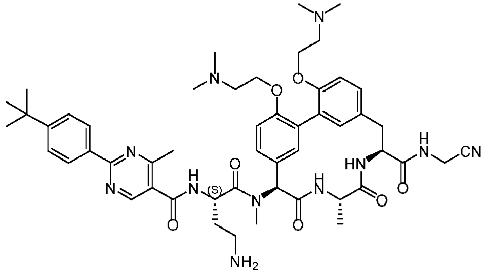

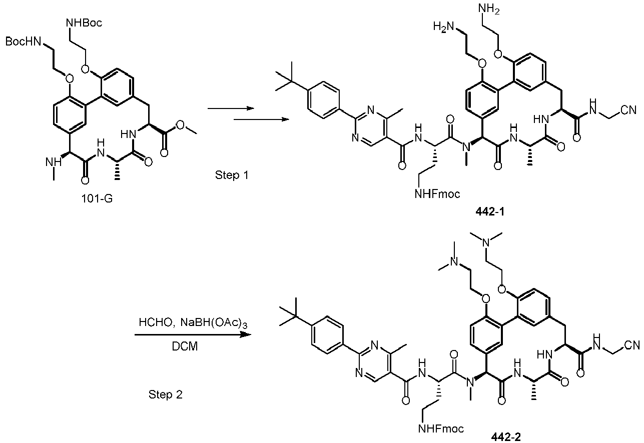

Step 1: Compound 442-1 was prepared as a white solid using by using compound 101-G and utilizing methods analogous to those described in Example J. LCMS (Method 5-95 AB, ESI): t_{R} = 0.865 min, [M + H]⁺ = 1113.9.

Step 2: A mixture of compound 442-1 (200 mg, 0.17 mmol) and (HCHO)ₙ (104 mg, 3.45 mmol), triethylamine (72 µL, 0.52 mmol), acetic acid (60 µL, 1.04 mmol) and NaBH₃CN (217 mg, 3.45mmol) in MeOH/H₂O (5.5 mL, v/v=10/1) was stirred at 15 °C for 32 h. The volatiles were removed under reduced pressure and the residue was purified by preparatory reverse-phase HPLC (acetonitrile 20-50%/0.225% formic acid in water) to afford compound 442-2 (60 mg, 30% yield) as a white solid. LCMS (Method 5-95 AB, ESI): t_{R} = 0.863 min, [M + H]⁺ = 1168.8.

Step 3: A solution of compound 442-2 (50 mg, 0.04 mmol) and quinuclidine (24 mg, 0.21 mmol) in DCM (5 mL) was stirred at 20 °C for 48 h. The volatiles were removed under reduced pressure and the residue was purified by reverse-phase HPLC (acetonitrile 0-40%/0.225% formic acid in water) to afford to give compound 442 (formic acid salt) (9.5 mg, 0.01 mmol, 23% yield) as a white solid. LCMS (Method 5-95 AB, ESI): t_{R} = 0.725 min, [M + H]⁺ = 946.6; ¹H NMR (400 MHz, MeOH-*d*₄) δ 8.76 (s, 1H), 8.52 (br s., 1H), 8.33 (d, *J*=8.4 Hz, 2H), 7.53 (d, *J*=8.4 Hz, 2H), 7.30-7.24 (m, 1H), 7.18-7.08 (m, 2H), 6.99 (d, *J*=8.4 Hz, 1H), 6.80 (d, *J*=2.4 Hz, 1H), 6.69 (s, 1H), 6.41 (s, 1H), 5.21-5.14 (m, 1H), 4.82-4.72 (m, 2H), 4.30-4.08 (m, 4H), 4.22 (s, 2H), 3.21-3.02 (m, 4H), 2.91 (s, 3H), 2.89-2.81 (m, 4H), 2.66 (s, 3H), 2.37 (s, 6H), 2.27 (s, 6H), 1.38 (s, 9H), 1.34 (d, J=6.4 Hz, 3H).

### Example 243: Synthesis of Compound 443

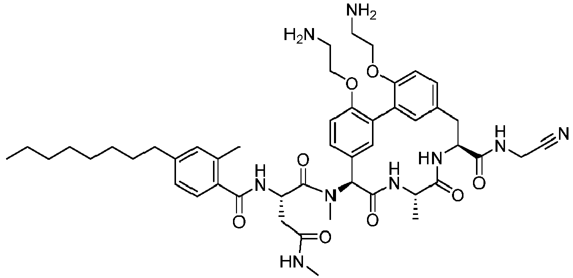

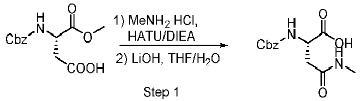

Step 1: Typical amide coupling (HATU/DIEA) and ester hydrolysis (LiOH, THF/H₂O) conditions, as described in Examples E and G, were applied to Cbz-Asp-OMe to give *N*²-((benzyloxy)carbonyl)-*N*⁴-methyl-*L*-asparagine as a white solid. LCMS (Method 5-95 AB, ESI): t_{R} = 0.567, [M + H]⁺ = 281.1

Compound 443 (trifluoroacetic acid salt) was prepared as a white solid from Compound **101-G** and *N*²-((benzyloxy)carbonyl)*-N*⁴-methyl-*L*-asparagine by utilizing methods analogous to those described in Example G. LCMS (Method 5-95 AB, ESI): t_{R} = 0.815 min, [M + H]⁺ = 896.6; ¹H NMR (400 MHz, MeOH-*d*₄) δ 7.25-7.01 (m, 7H), 6.87 (s, 1H), 6.80 (s, 1H), 6.28 (s, 1H), 4.95-4.75 (m, 3H), 4.35-4.20 (m, 4H), 4.18 (s, 2H), 3.30-3.00 (m, 8H), 2.79 (d, *J*=8.0 Hz, 3H), 2.69 (s, 3H), 2.62-2.59 (m, 2H), 2.41 (s, 3H), 1.60 (br s, 2H), 1.45-1.29 (m, 13H), 0.87 (t, *J*=6.8 Hz, 3H).

### Example 244: Synthesis of Compound 444

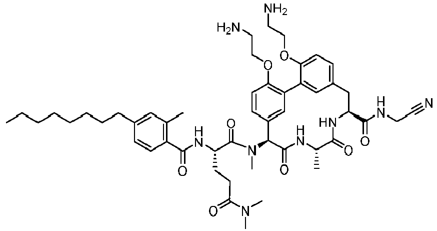

Compound 444 (formic acid salt) was prepared as a white solid from compound **101-G** by utilizing methods analogous to those described in Example 243. LCMS (Method 5-95 AB, ESI): t_{R} = 0.815 min, [M + H]⁺ = 896.6; ¹H NMR (400 MHz, MeOH-*d*₄) δ 8.54 (br s, 1H), 7.31-7.29 (m, 2H), 7.22 (d, *J*=8.0 Hz, 1H), 7.14 (d, *J*=8.0 Hz, 1H), 7.08-7.04 (m, 3H), 6.87 (d, *J*=2.4 Hz, 1H), 6.72 (s, 1H), 6.48 (s, 1H), 5.08-5.04 (m, 1H), 4.79-4.76 (m, 2H), 4.19-4.12 (m, 6H), 3.31-3.30 (m, 1H), 3.15-3.06 (m, 4H), 3.05(s, 3H), 3.01 (s, 3H), 2.92 (s, 3H), 2.65-2.51 (m, 3H), 2.37 (s, 3H), 2.24-2.17 (m, 1H), 2.03-1.98 (m, 1H), 1.60-1.58 (m, 2H), 1.35-1.29 (m, 15H), 0.89 (t, *J*=6.8 Hz, 3H).

### Example 245: Synthesis of Compound 445

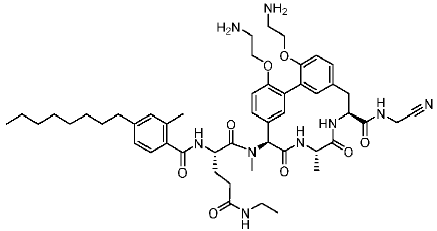

Compound 445 (formic acid salt) was prepared as a white solid from compound **101-G** by utilizing methods analogous to those described in Example 243. LCMS (Method 5-95 AB, ESI): t_{R} = 0.814 min, [M + H]⁺ = 924.7; ¹H NMR (400 MHz, MeOH-*d*₄) δ 8.51 (br s, 1H),7.37 (d, *J*=8.0 Hz, 1H), 7.31 (d, *J*=8.4 Hz, 1H),7.24 (d, *J*=8.0 Hz, 1H),7.16 (d, *J*=8.4 Hz, 1H),7.11-7.05 (m, 3H), 6.89 (s, 1H), 6.81 (s, 1H), 6.44 (s, 1H), 5.00-4.75 (m, 3H), 4.25-4.15 (m, 4H), 4.20 (s, 2H), 3.21-3.18 (m, 6H), 2.99 (s, 3H), 2.61 (t, *J*=7.6 Hz, 2H), 2.41-2.35 (m, 1H), 2.04-1.92 (m, 1H), 1.61 (br s, 2H), 1.39-1.25 (m, 14H), 1.09 (t, *J*=7.2 Hz, 3H), 0.90 (t, *J*=6.8 Hz, 3H).

### Example 246: Synthesis of Compound 446

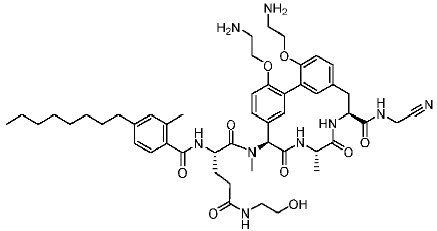

Compound 446 (formic acid salt) was prepared as a white solid from compound **101-G** by utilizing methods analogous to those described in Example 243. LCMS (Method 5-95 AB, ESI): t_{R} = 0.696 min, [M + H]⁺ = 940.6; ¹H NMR (400 MHz, MeOH-*d*₄) δ 8.48 (br s, 2H), 7.38 (d, *J*=7.5 Hz, 1H), 7.31 (d, *J*=8.0 Hz, 1H), 7.24 (d, *J*=7.5 Hz, 1H), 7.15 (d, *J*=8.0 Hz, 1H), 7.05-7.11 (m, 3H), 6.89 (br s, 1H), 6.81 (br s, 1H), 6.40 (s, 1H), 5.01-4.97 (m, 1H), 4.80-4.75 (m, 2H), 4.26-4.16 (m, 6H), 3.57 (t, *J*=6.4 Hz, 2H), 3.21-3.14 (m, 4H), 2.98 (s, 3H), 2.60 (t, *J*=6.4 Hz, 2H), 2.45-2.37 (s, 5H), 2.29-2.19 (m, 2H), 2.05-1.93 (m, 2H), 1.63-1.57 (m, 2H), 1.44-1.22 (m, 15H), 0.92-0.85 (m, 3H).

### Example 247: Synthesis of Compound 447

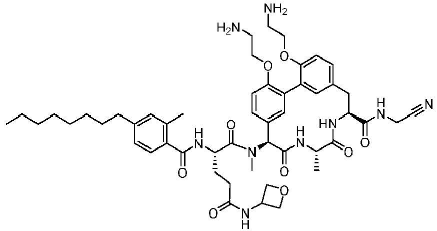

Compound 447 (trifluoroacetic acid salt) was prepared as a white solid from compound **101-G** by utilizing methods analogous to those described in Example 243. LCMS (Method 5-95 AB, ESI): t_{R} = 0.782 min, [M + Na]⁺ = 974.7; ¹H NMR (400 MHz, MeOH-*d*₄) δ 7.30-7.24 (m, 2H), 7.19 (d, *J*=8.0 Hz, 1H), 7.10 (d, *J*=8.0 Hz, 1H), 7.03-7.01 (m, 3H), 6.82 (s, 1H), 6.67 (br s, 1H), 6.50 (br s, 1H), 5.07-5.03 (m, 1H), 4.78-4.73 (m, 2H), 4.34-4.29 (m, 1H), 4.23-4.19 (m, 3H), 4.12-4.07 (m, 4H), 4.00-3.95 (m, 1H), 3.62-3.53 (m, 2H), 3.13-3.06 (m, 1H), 2.98-2.82 (m, 7H), 2.53-2.51 (m, 3H), 2.35 (br s, 3H), 2.28-2.24 (m, 1H), 2.15-2.00 (m, 1H), 1.63-1.48 (m, 2H), 1.34-1.23 (m, 15H), 0.90 (t, *J*=6.4 Hz, 3H).

### Example 248: Synthesis of Compound 448

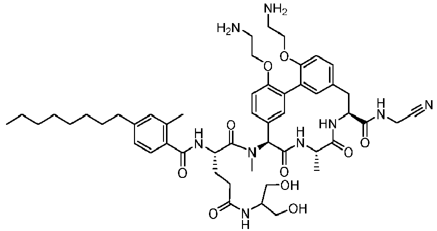

Compound 448 (trifluoroacetic acid salt) was prepared as a white solid from compound **101-G** by utilizing methods analogous to those described in Example 243. LCMS (Method 5-95 AB, ESI): t_{R} = 0.794 min, [M + H]⁺ = 971.2; ¹H NMR (400 MHz, MeOH-*d*₄) δ 7.33 (d, *J*=8.0 Hz, 1H), 7.26 (d, *J*=8.0 Hz, 1H), 7.20 (d, *J*=8.0 Hz, 1H), 7.11 (d, *J*=8.0 Hz, 1H), 7.04-7.02 (m, 3H), 6.84-6.83 (m, 1H), 6.69 (s, 1H), 6.48 (s, 1H), 5.04-5.01(m, 1H), 4.77-4.75 (m, 2H),4.19 (s, 2H), 4.12-3.90 (m, 5H), 3.63-3.59 (m, 3H), 3.25-3.27 (m, 1H) 3.14-3.07 (m, 1H), 2.98-2.89 (m, 6H), 2.55-2.41 (m, 4H), 2.36 (br s, 3H), 2.26-2.17 (m, 1H), 2.03-2.02 (m, 1H), 1.64-1.53 (m, 2H), 1.36-1.29 (m, 15H), 0.90 (t, *J*=6.8 Hz, 3H).

### Example 249: Synthesis of Compound 449

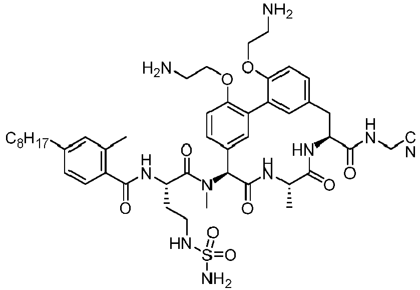

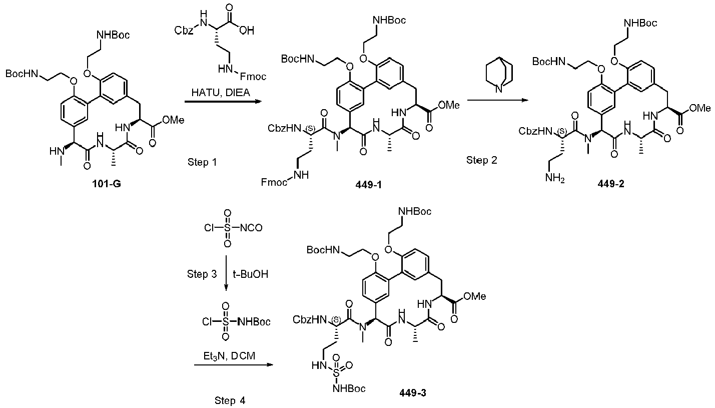

Step 1: Typical amide coupling condition (HATU/DIEA, described in Example E) was applied to compound **101-G** (500 mg, 0.70 mmol) and (*S*)-4-((((9*H*-fluoren-9-yl)methoxy)carbonyl)amino)-2-(((benzyloxy)carbonyl)amino)butanoic acid (332 mg, 0.70 mmol) to give compound **449-1** (400 mg, 49% yield) as a white solid. LCMS (Method 5-95 AB, ESI): t_{R} = 1.039 min, [M + H]⁺ = 1170.4.

Step 2: A solution of compound **449-1** (150 mg, 0.13 mmol) and quinuclidine (43 mg, 0.38 mmol) in DCM (8 mL) was stirred at 25 °C for 24 h. The volatiles were concentrated and the residue was purified by preparatory-TLC (eluting with 10% methanol in DCM, Rf = 0.2) to give compound **449-2** (90 mg, 74.1% yield) as a white solid.

Step 3: To a stirred solution of *t*-BuOH (1.86 mL, 19.5 mmol) in DCM (12 mL) at 0 °C was added sulfurisocyanatidic chloride (1.41 mL, 15.0 mmol) dropwise over 10 min. The reaction mixture was gradually warmed up to 25 °C while stirring and was stirred at the same temperature for 0.5 h. The solvent was concentrated *in vacuo* to one-third volume and the flask was placed back into the 0 °C bath, where the product started to precipitate out from the solution. After filtration, the product was washed with hexane and dried in an oven to give *tert*-butyl (chlorosulfonyl)carbamate (1.8 g, 55.6% yield) as a colorless solid. ¹H NMR (400MHz, CDCl₃) δ 8.45 (br s, 1H), 1.56 (s, 9H).

Step 4: A solution of compound **449-2** (90 mg, 0.09 mmol), *tert*-butyl (chlorosulfonyl)carbamate (82 mg, 0.38 mmol) and Et₃N (53 µL, 0.38 mmol) in DCM (5 mL) was stirred at 25 °C for 16 hr. The volatiles were removed under reduced pressure and the residue was purified by preparatory-TLC (eluting with 5% methanol in DCM, Rf=0.5) to afford compound **449-3** (70 mg, 65.4% yield) as a white solid. LCMS (Method 5-95 AB, ESI): t_{R} = 0.947 min, [M-Boc+ H]⁺ = 1027.1.

Compound 449 (formic acid salt) was prepared as a white solid from compound **449-3** by utilizing methods analogous to those described in Example G. LCMS (Method 5-95 AB, ESI): t_{R} = 0.710 min, [M + H]⁺ = 948.1; ¹H NMR (400 MHz, MeOH-*d*₄) δ 8.49 (br s, 3H), 7.36 (d, *J*=7.2 Hz, 1H), 7.36-7.28 (m, 1H), 7.28-7.20 (m, 1H), 7.16 (d, *J*=8.8 Hz, 1H), 7.12-7.02 (m, 3H), 6.89 (d, *J*=2.4 Hz, 1H), 6.80 (d, *J*=2.4 Hz, 1H), 6.43 (s, 1H), 5.14-5.07 (m, 1H), 4.79-4.73 (m, 2H), 4.29-4.11 (m, 6H), 3.38-3.32 (m, 2H), 3.26-3.08 (m, 6H), 2.99 (s, 3H), 2.64-2.55 (m, 2H), 2.41 (s, 3H), 2.22-2.10 (m, 2H), 2.04-1.90 (m, 2H), 1.65-1.56 (m, 2H), 1.45-1.22 (m, 11H), 0.90 (t, *J*=6.8 Hz, 3H).

### Example 250: Synthesis of Compound 450

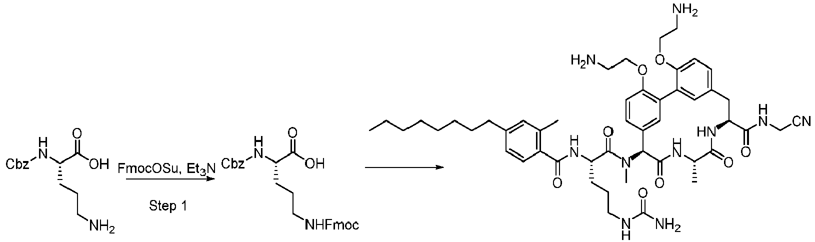

Step 1: A solution of (*S*)-5-amino-2-(((benzyloxy)carbonyl)amino)pentanoic acid (500 mg, 1.88 mmol), FmocOSu (950 mg, 2.82 mmol) and Et₃N (0.52 mL, 3.76 mmol) in DCM/MeOH (20 mL, v/v=1/1) was stirred at 25 °C for 18 h. After that, the reaction mixture was washed with a solution of 1N KHSO₄ and brine (each 20 mL) and the organic layer was dried over Na₂SO₄, concentrated *in vacuo* and the residue was purified via silica gel chromatography, eluting with 5% MeOH in DCM, to give (*S*)-5-((((9*H*-fluoren-9-yl)methoxy)carbonyl)amino)-2-(((benzyloxy)carbonyl)amino)pentanoic acid (700 mg, 76.3% yield) as a white solid. LCMS (Method 5-95 AB, ESI): t_{R} = 0.891 min, [M + H]⁺ = 489.3.

Compound 450 (formic acid salt) was prepared as a white solid from compound **101-G** and (S)-5-((((9*H*-fluoren-9-yl)methoxy)carbonyl)amino)-2-(((benzyloxy)carbonyl)amino)pentanoic acid by utilizing methods analogous to those described in Example P. LCMS (Method 5-95 AB, ESI): t_{R} = 0.705 min, [M + H]⁺ = 925.6; ¹H NMR (400 MHz, MeOH-*d*₄) δ 8.51 (br s, 2H), 7.35-7.29 (m, 2H), 7.28-7.20 (m, 2H), 7.16 (d, *J*=8.4 Hz, 1H), 7.11-7.04 (m, 3H), 6.88 (d, *J*=2.2 Hz, 1H), 6.80 (d, *J*=2.2 Hz, 1H), 6.44 (s, 1H), 4.79-4.73 (m, 3H), 4.25-4.13 (m, 6H), 3.26-3.08 (m, 8H), 2.97 (s, 3H), 2.60 (t, *J*=7.5 Hz, 2H), 2.40 (s, 3H), 1.98-1.88 (m, 2H), 1.83-1.67 (m, 4H), 1.63-1.58 (m, 2H), 1.37-1.24 (m, 11H), 0.90 (t, *J*=6.8 Hz, 3H).

### Example 251: Synthesis of Compound 451

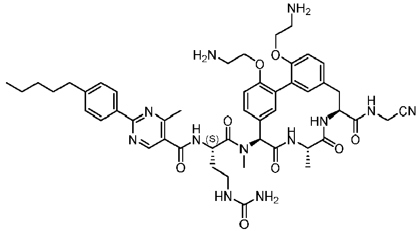

Compound 451 (formic acid salt) was prepared as a white solid from compound **101-G** by utilizing methods analogous to those described in Example P. LCMS (Method 5-95 AB, ESI): t_{R} = 0.776 min, [M + H]⁺ = 947.8; ¹H NMR (400 MHz, MeOH-*d*₄) δ 8.78 (s, 1H), 8.50 (br s, 2H), 8.22 (d, *J*=8.0 Hz, 2H), 7.37-7.26 (m, 3H), 7.20 (d, *J*=8.0 Hz, 1H), 7.15-7.02 (m, 2H), 6.85 (s, 1H), 6.62 (br s, 2H), 5.05-5.03 (m, 1H), 4.77-4.73 (m, 2H), 4.36-4.15 (m, 4H), 4.29 (s, 2H), 3.37-3.35 (m, 1H), 3.30-3.05 (m, 7H), 2.99 (s, 3H), 2.70 (t, J=7.2 Hz, 2H), 2.67 (s, 3H), 2.16-2.10 (m, 1H), 1.97-1.92 (m, 1H), 1.75-1.60 (m, 3H), 1.47-1.32 (m, 7H), 0.93 (t, *J*=6.8 Hz, 3H).

### Example 252: Synthesis of Compound 452

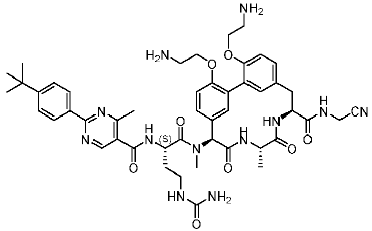

Compound 452 (formic acid salt) was prepared as a white solid from compound **101-G** by utilizing methods analogous to those described in Example P. LCMS (Method 5-95 AB, ESI): t_{R} = 0.750 min, [M + H]⁺ = 933.6; ¹H NMR (400 MHz, MeOH-*d*₄) δ 8.78 (s, 1H), 8.50 (br s, 2H), 8.26 (d, *J*=8.0 Hz, 2H), 7.52 (d, *J*=8.0 Hz, 2H), 7.31 (d, *J*=8.0 Hz, 1H), 7.21 (d, *J*=8.0 Hz, 1H), 7.13-7.00 (m, 2H), 6.85 (s, 1H), 6.60 (br s, 2H), 5.04-5.01 (m, 1H), 4.77-4.73 (m, 2H), 4.34-4.18 (m, 4H), 4.22 (s, 2H), 3.37-3.35 (m, 1H), 3.27-3.05 (m, 7H), 2.99 (s, 3H), 2.67 (s, 3H), 2.16-2.10 (m, 1H), 1.97-1.94 (m, 1H), 1.39 (s, 9H), 1.35 (d, *J*=6.8 Hz, 3H).

### Example 253: Synthesis of Compound 453

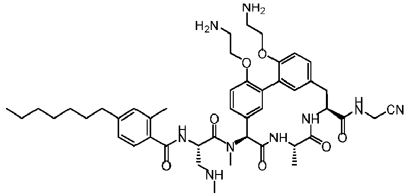

Compound 453 (formic acid salt) was prepared as a white solid from compound **101-G** by utilizing methods analogous to those described in Example 53 and Example Q. LCMS (Method 5-95 AB, ESI): t_{R} = 0.627 min, [M + H]⁺ = 854.6; ¹H NMR (400 MHz, MeOH-*d*₄) δ 8.45 (br s, 5H), 7.39 (d, *J*=8.0 Hz, 1H), 7.32 (d, *J*=8.0 Hz, 1H), 7.23 (d, *J*=8.0 Hz, 1H), 7.17 (d, *J*=8.0 Hz, 1H), 7.11 - 7.09 (m, 3H), 6.89 (s, 1H), 6.81 (s, 1H), 6.26 (s, 1H), 5.31-5.29 (m, 1H), 4.80-4.76 (m, 2H), 4.25- 4.20 (m, 4H), 4.19 (s, 2H), 3.61-3.47 (m, 2H), 3.25-3.17 (m, 6H), 2.88 (s, 3H), 2.75 (s, 3H), 2.64-2.60 (m, 2H), 2.42 (s, 3H), 1.64-1.60 (m, 2H), 1.34-1.29 (m, 11H), 0.90 (t, *J*=7.0 Hz, 3H).

### Example 254: Synthesis of Compound 454

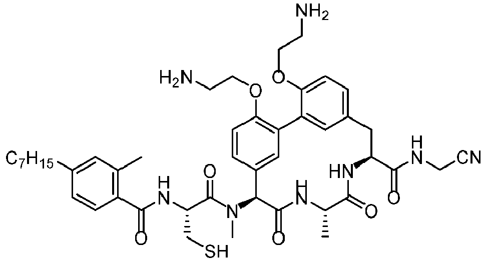

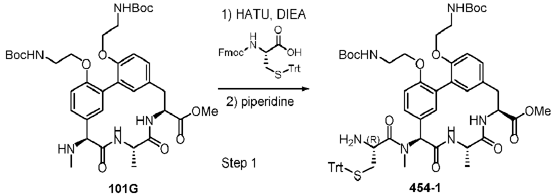

Step 1: Typical amide coupling (HATU/DIEA, described in Example E) with compound 101G and *N*-(((9*H*-fluoren-9-yl)methoxy)carbonyl)-*S*-trityl-*L*-cysteine, followed by Fmoc removal (with piperidine), were followed to give compound 454-1 as a white solid. LCMS (Method 5-95 AB, ESI): t_{R} = 0.793 min, [M + H]⁺ = 1059.6

Compound 454 (formic acid salt) was prepared as a white solid from compound 454-1 by utilizing methods analogous to those described in Example G. LCMS (Method 5-95 AB, ESI): t_{R} = 0.805 min, [M + H]⁺ = 857.6; ¹H NMR (400 MHz, MeOH-*d*₄) δ 8.49 (br s, 1H), 7.45-7.00 (m, 7H), 6.89 (s, 2H), 6.81 (s, 1H), 6.37 (s, 1H), 5.15-5.05 (m, 1H), 4.75-4.70 (m, 2H), 4.30-4.05 (m, 6H), 3.25-2.90 (m, 9H), 2.82-2.70 (m, 2H), 2.61 (t, *J*=6.4 Hz, 2H), 2.41 (s, 3H), 1.70-1.55 (m, 2H), 1.40-1.20 (m, 11H), 0.89 (t, J=6.8 Hz, 3H).

### Example 255: Synthesis of Compound 455

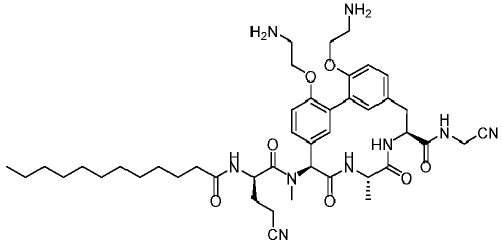

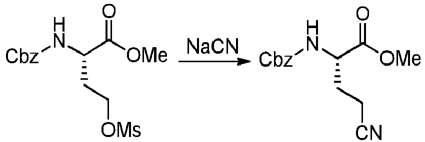

To a solution of (S)-methyl 2-(((benzyloxy)carbonyl)amino)-4-hydroxybutanoate (400 mg, 1.16 mmol) in DMF (5 mL) was added NaCN (62 mg, 1.27 mmol) at room temperature and the reaction mixture was warmed to 75 °C while stirring and stirred at the same temperature for 2 h. The volatiles were removed and the residue was taken by EtOAc (40 mL), which was washed by brine (40 mL). The organic layer was dried over Na₂SO₄, concentrated and the residue was purified by Prep-TLC to give (S)-methyl 2-(((benzyloxy)carbonyl)amino)-4-cyanobutanoate (210 mg, 65.6% yield) as colorless oil. LCMS (Method 5-95 AB, ESI): t_{R} = 0.779, M+Na⁺ = 298.9.

Compound 455-diastereomeric mixture) (formic acid salt) was prepared as a diautilizing the methods previously described. LCMS (Method 5-95 AB, ESI): t_{R} = 0.814, [M + H]⁺ = 830.4.

Compound 455 (formic acid salt) was separated as a single unknown stereoisomer. LCMS (Method 5-95 AB, ESI): t_{R} = 0.696 min, [M + H]⁺ = 830.3; ¹H NMR (400 MHz, MeOH-*d*₄) δ 8.45 (br s, 1H), 7.32-7.11(m, 4H), 6.91 (s, 1H), 6.83 (s, 1H), 6.27 (s, 1H), 5.13-4.78 (m, 3H), 4.27-4.20 (m, 4H), 4.21 (s, 2H), 3.23-3.19 (m, 4H), 2.92 (s, 3H), 2.70-2.53 (m, 3H), 2.34-1.99 (m, 4H), 1.70- 1.64 (m, 2H), 1.40-1.31 (m, 23H), 0.90 (t, J=6.8 Hz, 3H).

### Example 256: Synthesis of Compound 456

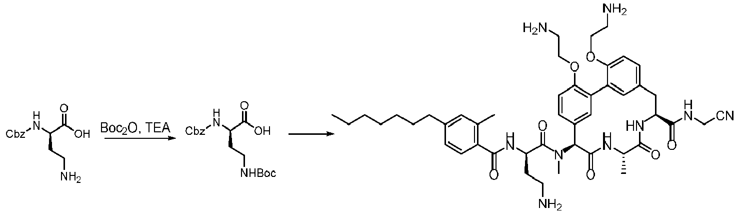

Compound 456 (trifluoroacetic acid salt) was prepared as a white solid from compound **101-G** by utilizing methods analogous to those described in Example G. LCMS (Method 5-95 AB, ESI): t_{R} = 0.760 min, [M + H]⁺ = 854.6; ¹H NMR (400 MHz, MeOH-*d*₄) δ 7.40 (d, *J*=7.2 Hz, 1H), 7.29-7.08 (m, 5H), 6.90-6.80 (m, 3H), 6.29 (s, 1H), 4.85-4.76 (m, 3H), 4.30-4.20 (m, 4H), 4.18 (s, 2H), 3.31-3.05 (m, 8H), 2.92 (s, 3H), 2.60 (d, *J*=7.6 Hz, 2H), 2.42 (s, 3H), 2.20-2.00 (m, 2H), 1.60 (t, *J*=6.4 Hz, 2H), 1.34-1.29 (m, 11H), 0.89 (t, *J*=7.6 Hz, 3H).

### Example 257: Synthesis of Compound 457

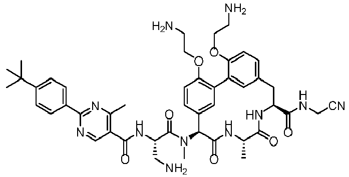

Compound 457 (formic acid salt) was prepared as a white solid from compound **101-L** by utilizing methods analogous to those described in Example G. LCMS (Method 5-95 AB, ESI): t_{R} = 0.601 min, [M + H]⁺ = 876.5; ¹H NMR (400 MHz, MeOH-*d*₄) δ 8.86 (s, 1H), 8.45 (br s, 2H), 8.37 (d, J=8.0 Hz, 2H), 7.56 (d, *J*=8.0 Hz, 2H), 7.33 (d, *J*=7.6 Hz, 1H), 7.24-7.18 (m, 2H), 7.09 (d, *J*=7.6 Hz, 1H), 6.91 (s,1H), 6.79 (s, 1H), 6.33 (s, 1H), 5.35-5.34 (m, 1H), 4.90-4.81 (m, 2H), 4.28-4.20 (m, 6H), 3.52-3.48 (m, 2H), 3.48-3.31 (m, 7H), 3.30-3.24 (m, 2H), 2.91 (s, 3H), 2.75 (s, 3H), 1.39 (s, 9H), 1.37 (d, *J*=6.8 Hz, 3H).

### Example 258: Synthesis of Compound 458

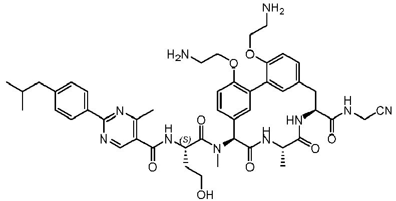

Compound 458 (formic acid salt) was prepared as a white solid from compound **101-G** by utilizing methods analogous to those described in Example R. LCMS (Method 5-95 AB, ESI): t_{R} = 0.765 min, [M + H]⁺ = 891.3; ¹H NMR (400 MHz, MeOH-*d*₄) δ 8.72 (s, 1H), 8.53 (br s, 1H), 8.18 (d, *J*=7.6 Hz, 2H), 7.33-7.18 (m, 4H), 7.05 (d, *J*=7.6 Hz, 2H), 6.83 (s, 1H), 6.74 (s, 1H), 6.51 (s, 1H), 5.23-5.18 (m, 1H), 4.82-4.73 (m, 2H), 4.35-4.18 (m, 6H), 3.76 (t, *J*=5.2 Hz, 2H), 3.25-3.00 (m, 6H), 3.03 (s, 3H), 2.63 (s, 3H), 2.57 (d, *J*=6.8 Hz, 2H), 2.22-2.10 (m, 1H), 2.02-1.89 (m, 2H), 1.35 (d, *J*=7.2 Hz, 3H), 0.96 (d, *J*=5.6 Hz, 6H).

### Example 259: Synthesis of Compound 459

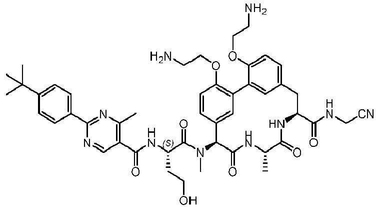

Compound 459 (formic acid salt) was prepared as a white solid from Compound **101-G** by utilizing methods analogous to those described in Example R. LCMS (Method 5-95 AB, ESI): t_{R} = 0.747 min, [M + H]⁺ = 891.4; ¹H NMR (400 MHz, MeOH-*d*₄) δ 8.71 (s, 1H), 8.53 (br s, 2H), 8.21 (d, *J*=8.0 Hz, 2H), 7.49 (d, *J*=8.0 Hz, 2H), 7.31 (d, *J*=8.0 Hz, 1H), 7.22 (d, *J*=8.0 Hz, 1H), 7.05 (d, *J*=8.0 Hz, 2H), 6.83 (s, 1H), 6.73 (s, 1H), 6.54 (s, 1H), 5.21-5.15 (m, 1H), 4.83-4.80 (m, 1H), 4.74-4.69 (m, 1H), 4.39-4.15 (m, 4H), 4.22 (s, 2H), 3.76 (t, *J*=5.6 Hz, 2H), 3.35-2.90 (m, 6H), 3.03 (s, 3H), 2.64 (s, 3H), 2.16-2.10 (m, 1H), 2.06-1.90 (m, 1H), 1.39 (s, 9H), 1.37 (d, *J*=6.4 Hz, 3H).

### Example 260: Synthesis of Compound 460

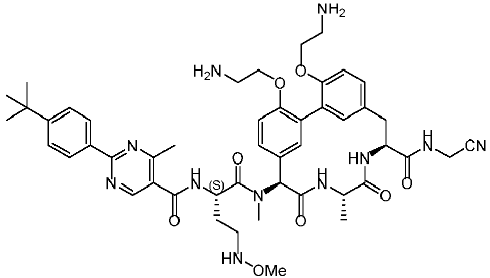

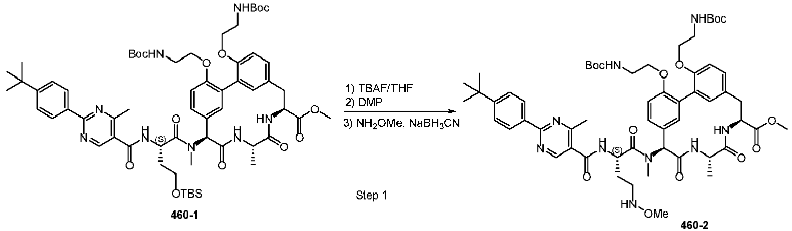

Step 1: Starting from compound 460-1 (described in Example 259), TBS deprotection, DMP oxidation and reductive amination were applied to give compound 460-2 as a white solid. LCMS (Method 5-95 AB, ESI): t_{R} = 0.872 min, [M + H]⁺ = 1096.6.

Compound 460 (formic acid salt) was prepared as a white solid from compound 460-2 by utilizing methods analogous to those described in Example G. LCMS (Method 5-95 AB, ESI): t_{R} = 0.633 min, [M + H]⁺ = 920.4; ¹H NMR (400 MHz, MeOH-*d*₄) δ 8.69 (s, 1H), 8.50 (brs, 3H), 8.18 (d, *J*=7.6 Hz, 2H), 7.50 (d, *J*=8.4 Hz, 2H), 7.31-7.23 (m, 2H), 7.04 (brs, 2H), 6.81 (s, 1H), 6.75 (s, 1H), 6.46 (s, 1H), 5.18-5.15 (m, 1H), 4.85-4.75 (m, 2H), 4.73-4.67 (m, 1H), 4.40-4.15 (m, 3H), 4.25 (s, 3H), 3.54-3.48 (m, 1H), 3.32-3.23 (m, 3H), 3.13-2.90 (m, 4H), 3.05 (s, 3H), 2.63 (s, 3H), 2.22-2.18 (m, 1H), 2.01-1.97 (m, 1H), 1.39 (s, 9H), 1.36 (d, *J*=6.0 Hz, 3H).

### Example 261: Synthesis of Compound 461

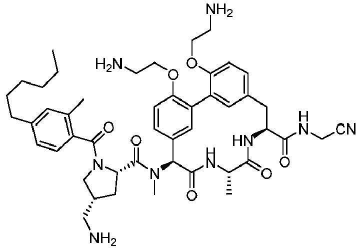

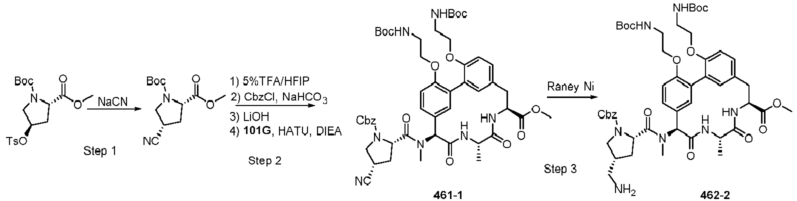

Step 1: A solution of 1-(*tert*-butyl) 2-methyl (2*S*,4*R*)-4-(tosyloxy)pyrrolidine-1,2-dicarboxylate (1.0 g, 2.5 mmol) and sodium cyanide (320 mg, 6.53 mmol) in DMSO (10 mL) was stirred at 80 °C for 5 h. The reaction was diluted with EtOAc (120 mL) and washed with brine (60 mL x 3). The organic layer was dried over Na₂SO₄, concentrated and the residue was purified by silica gel chromatography, eluting with 10% EtOAc in petroleum ether, to give 1-(*tert*-butyl) 2-methyl (2*S*,4*S*)-4-cyanopyrrolidine-1,2-dicarboxylate (300 mg, 47% yield) as a white solid.

Step 2: Starting from 1-(*tert*-butyl) 2-methyl (2*S*,4*S*)-4-cyanopyrrolidine-1,2-dicarboxylate, typical Boc removal, Cbz protection, ester hydrolysis and amide coupling with **101G** (using procedures analogous to those described in Examples 4 and 7) were applied to give compound 461-1 as a white solid. LCMS (Method 5-95 AB, ESI): t_{R} = 0.925 min, [M + H-Boc]⁺ = 870.5.

Step 3: To a solution of compound 461-1 (155 mg, 0.16 mmol) and nickel (66.6 mg, 1.13 mmol) in THF (20 mL) was added one drop ammonia and the mixture was stirred at 20 °C for 12 h under H₂ (15 psi). The reaction mixture was filtered and the filtrate was concentrated. The resulting residue was purified by preparatory-TLC (eluting with 10% MeOH in DCM, Rf = 0.15) to give compound 461-2 (120 mg, 77% yield) as a white solid. LCMS (Method 5-95 AB, ESI): t_{R} = 0.719 min, [M + H]⁺ = 974.8.

Compound 461 (formic acid salt) was prepared as a white solid from compound 461-2 by utilizing previously described methods. LCMS (Method 5-95 AB, ESI): t_{R} = 0.717 min, [M + H]⁺ = 866.7; ¹H NMR (400 MHz, MeOH-*d*₄) δ 8.45 (s, 2H), 7.38 (d, *J*=8.0 Hz, 1H), 7.27-7.08 (m, 6H), 6.89 (s, 1H), 6.84 (s, 1H), 6.40 (s, 1H), 5.11-5.08 (m, 1H), 4.80-4.70 (m, 2H), 4.23-4.15 (m, 4H), 4.19 (s, 2H), 3.62-3.58 (m, 1H), 3.47 (brs,1H), 3.19-3.14 (m, 3H), 3.05-2.98 (m, 4H), 2.95 (s, 3H), 2.74-2.68 (m, 2H), 2.63-2.59 (m, 2H), 2.37-2.32 (m, 1H), 2.35 (s, 3H), 1.81-1.78 (m, 1H), 1.67-1.54 (m, 2H), 1.36-1.33 (m, 9H), 0.90 (t, *J*=5.2 Hz, 3H).

### Example 262: Synthesis of Compound 462

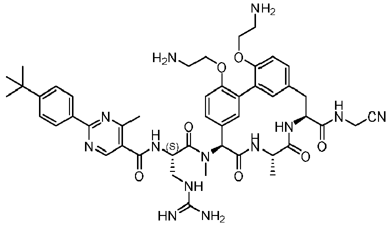

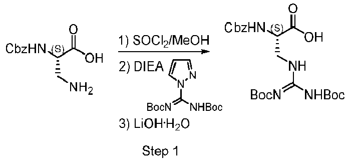

Step 1: Starting from (*S*)-3-amino-2-(((benzyloxy)carbonyl)amino)propanoic acid, methyl ester formation (as described in Example M), guanidine formation (as described in Example 241) and ester hydrolysis (as described in Example G), (*S*)-2-(((benzyloxy)carbonyl)amino)-3-(2,3-bis(*tert-*butoxycarbonyl) guanidino)propanoic acid was obtained as a white solid. LCMS (Method 5-95 AB, ESI): t_{R} = 0.907 min, [M + H]⁺ = 481.2.

Compound 462 (formic acid salt) was prepared as a white solid from compound **101G** and (S)-2-(((benzyloxy) carbonyl)amino)-3-(2,3-bis(tert-butoxycarbonyl)guanidino)propanoic acid by utilizing previously disclosed methods. LCMS (Method 5-95 AB, ESI): t_{R} = 0.602 min, [M + H]⁺ = 918.5; ¹H NMR (400 MHz, MeOH-*d*₄) δ 8.77 (s, 1H), 8.51 (br s, 1H), 8.35 (d, *J*=8.0 Hz, 1H), 7.55 (d, *J*=8.8 Hz, 1H), 7.30 (d, *J*=8.0 Hz, 1H), 7.22-7.16 (m, 2H), 7.08 (d, *J*=8.8 Hz, 1H), 6.89 (d, *J*=2.4 Hz, 1H), 6.76 (s, 1H), 6.40 (s, 1H), 5.28 (t, *J*=6.4 Hz, 1H), 4.82-4.75 (m, 2H), 4.25-4.18 (m, 4H), 4.20 (s, 2H), 3.79-3.74 (m, 1H), 3.66-3.61 (m, 1H), 3.16-3.08 (m, 5H), 2.93 (s, 3H), 2.76-2.65 (m, 1H), 2.68 (s, 3H), 1.38 (s, 9H), 1.37 (d, *J*=7.6 Hz, 3H).

### Example 263: Synthesis of Compound 463

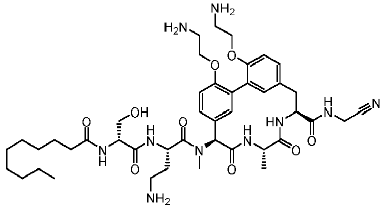

Compound 463 (formic acid salt) was prepared as a white solid utilizing methods analogous to those described in Example S and Example 7. LCMS (Method 5-95 AB, ESI): t_{R} = 0.576 min, [M + Na]⁺ = 902.4; ¹H NMR (400 MHz, MeOH-*d*₄) δ 8.52 (brs, 2H), 7.30-7.22 (m, 2H), 7.15 (d, *J*=8.4 Hz, 1H), 7.08 (d, *J*=8.4 Hz, 1H), 6.87 (s, 1H), 6.81 (s, 1H), 6.26 (s, 1H), 5.05-4.70 (m, 2H), 4.40-4.30 (m, 1H), 4.25-4.10 (m, 6H), 3.85-3.70 (m, 2H), 3.20-3.00 (m, 8H), 2.81 (s, 3H), 2.40-2.25 (m, 2H), 2.25-2.13 (m, 1H), 2.10-1.95 (m, 1H), 1.70-1.55 (m, 2H), 1.40-1.20 (m, 15H), 0.90 (t, *J*=6.4 Hz, 3H).

### Example 264: Synthesis of Compound 464

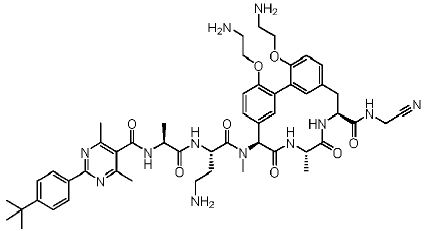

Compound 464 was prepared as a white solid utilizing methods analogous to those described in Example S and Example 7. LCMS (Method 5-95 AB, ESI): t_{R} = 0.724 min, [M + H]⁺ = 975.5; ¹H NMR (400 MHz, MeOH-*d*₄) δ 8.33 (d, *J*=8.4 Hz, 2H), 7.54 (d, *J*=8.4 Hz, 2H), 7.30 (d, *J*=7.2 Hz, 1H), 7.24 (d, *J*=7.2 Hz, 1H), 7.16 (d, *J*=8.4 Hz, 1H), 7.09 (d, *J*=8.4 Hz, 1H), 6.91 (s, 1H), 6.89 (s, 1H), 6.32 (s, 1H), 5.10-4.81 (m, 3H), 4.56 (m, 1H), 4.24-4.18 (brs, 4H), 4.20 (s, 2H), 3.35-3.30 (m, 1H), 3.21-3.07 (m, 7H), 2.86 (s, 3H), 2.58 (s, 6H), 2.29-2.17 (m, 1H), 2.13-2.01 (m, 1H), 1.43 (d, *J*=7.0 Hz, 3H), 1.44 (s, 9H), 1.37 (t, *J*=7.2 Hz, 3H).

### Example 265: Synthesis of Compound 465

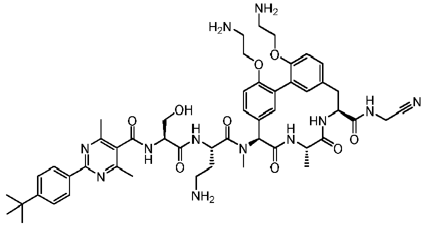

Compound 465 was prepared utilizing methods analogous to those described in Example S and Example 7. LCMS (Method 5-95 AB, ESI): t_{R} = 0.625 min, [M + H]⁺ = 991.8; ¹H NMR (400 MHz, MeOH-*d*₄) δ 8.34 (d, *J*=8.4Hz, 2H), 7.54 (d, *J*=8.8Hz, 2H), 7.34-7.22 (m, 2H), 7.15 (d, *J*=8.4Hz, 1H), 7.12-7.07 (m, 1H), 6.92 (s, 1H), 6.84 (s, 1H), 6.30 (s, 1H), 5.15-4.59 (m, 4H), 4.27-4.17 (m, 4H), 4.19 (s, 2H), 3.89 (d, *J*=6.0Hz, 2H), 3.26-3.01 (m, 8H), 2.87 (s, 3H), 2.60 (s, 6H), 2.28-2.19 (m, 1H), 2.14-2.05 (m, 1H), 1.37 (s, 9H), 1.36 (t, *J*=7.2 Hz, 3H).

### Example 266: Synthesis of Compound 466

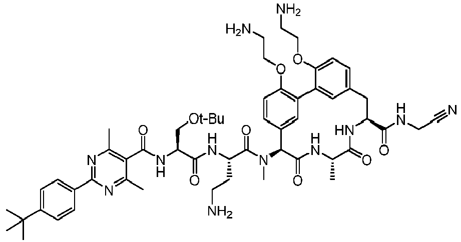

Compound 466 was prepared utilizing methods analogous to those described in Example S and Example 7. LCMS (Method 5-95 AB, ESI): t_{R} = 0.663 min, [M + H]⁺ = 1047.8; ¹H NMR (400 MHz, MeOH-*d*₄) δ 8.50 (brs, 2H), 8.33 (d, *J*=8.4Hz, 2H), 7.54 (d, *J*=8.4Hz, 2H), 7.32-7.19 (m, 2H), 7.19-7.03 (m, 2H), 6.90 (s, 1H), 6.81 (s, 1H), 6.32 (s, 1H), 5.28-4.70 (m, 4H), 4.25-4.08 (m, 4H), 4.19 (s, 2H), 3.75 (d, *J*=6.0Hz, 2H), 3.42-3.33 (m, 1H), 3.29-3.09 (m, 10H), 2.88 (s, 3H), 2.59 (s, 6H), 2.32-2.15 (m, 1H), 2.14-2.00 (m, 1H), 1.40 (s, 9H), 1.37 (t, *J*=7.2 Hz, 3H), 1.23(s, 9H).

### Example 267: Synthesis of Compound 467

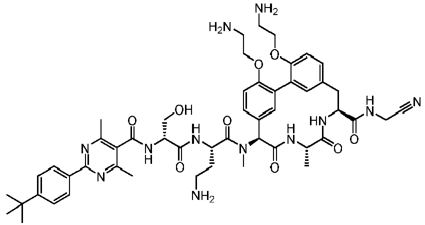

Compound 467 was prepared utilizing methods analogous to those described in Example S and Example 7. LCMS (Method 5-95 AB, ESI): t_{R} = 0.716 min, [M + H]⁺ = 991.8; ¹H NMR (400 MHz, MeOH-*d*₄) 8.54 (br s, 1H), 8.33 (d, *J*=8.4 Hz, 2H), 7.54 (d, *J*=8.4 Hz, 2H), 7.36-7.22 (m, 2H), 7.13-7.04 (m, 2H), 6.89 (s, 1H), 6.81 (s, 1H), 6.30 (s, 1H), 5.14-4.66 (m, 4H), 4.21-4.09 (m, 4H), 4.19 (s, 2H), 3.92 (d, *J*=6.0 Hz, 2H), 3.24-3.07 (m, 8H), 2.87 (s, 3H), 2.53 (s, 6H), 2.26-2.21 (m, 1H), 2.10-2.01 (m, 1H), 1.37-1.29 (m, 12H).

### Example 268: Synthesis of Compound 468

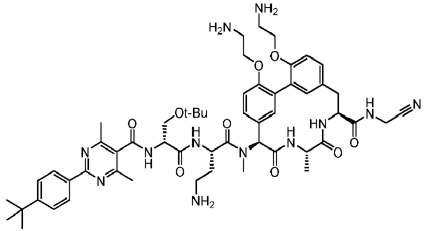

Compound 468 was prepared utilizing methods analogous to those described in Example S and Example 7. LCMS (Method 5-95 AB, ESI): t_{R} = 0.766 min, [M + H]⁺ = 1047.6; ¹H NMR (400 MHz, MeOH-*d*₄) δ 8.53 (br s, 1H), 8.33 (d, *J*=8.4 Hz, 2H), 7.52 (d, *J*=8.4 Hz, 2H), 7.31-7.01 (m, 5H), 6.85 (s, 1H), 6.37 (s, 1H), 5.10-4.71 (m, 4H), 4.31-4.12 (m, 6H), 3.80-3.74 (m, 2H), 3.10-2.90 (m, 8H), 2.86 (s, 3H), 2.53 (s, 6H), 2.23-2.18 (m, 1H), 2.04-2.02 (m, 1H), 1.42 (t, J=7.2 Hz, 3H), 1.38 (s, 9H), 1.24 (s, 9H).

### Example 269: Synthesis of Compound 469

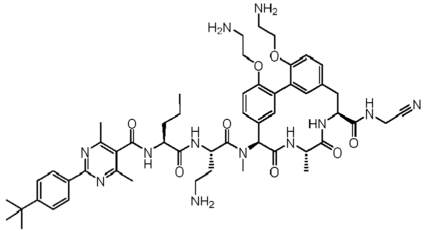

Compound 469 (formic acid salt) was prepared as a white solid utilizing methods analogous to those described in Example S and Example 7. LCMS (Method 5-95 AB, ESI): t_{R} = 0.744 min, [M + Na]⁺ = 1025.7; ¹H NMR (400 MHz, MeOH-*d*₄) δ 8.48 (br s, 1H), 8.33 (d, *J*=8.4Hz, 2H), 7.53 (d, *J*=8.4Hz, 2H), 7.32-6.82 (m, 6H), 6.30 (s, 1H), 5.10-4.79 (m, 4H), 4.25-4.18 (m, 6H), 3.17-3.16 (m, 9H), 2.93-2.87 (m, 2H), 2.58 (s, 6H), 2.24-2.00 (m, 2H), 1.78-1.76 (m, 2H), 1.52-1.37 (m, 14H), 1.03 (t, *J*=5.2 Hz, 3H).

### Example 270: Synthesis of Compound 470

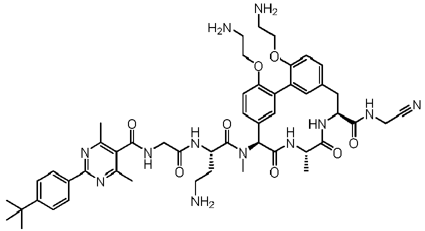

Compound 470 (formic acid salt) was prepared as a white solid utilizing methods analogous to those described in Example S and Example 7. LCMS (Method 5-95 AB, ESI): t_{R} = 0.625 min, [M + H]⁺ = 961.5; ¹H NMR (400 MHz, MeOH-*d*₄) δ 8.50 (br s, 2H), 8.34 (d, *J*=8.4Hz, 2H), 7.55 (d, *J*=8.4Hz, 2H), 7.35-7.21 (m, 2H), 7.17-7.06 (m, 2H), 6.94 (s, 1H), 6.75 (s, 1H), 6.32 (s, 1H), 5.11-4.80 (m, 4H), 4.22-4.10 (m, 7H), 3.51-3.32 (m, 1H), 3.21-3.00 (m, 7H), 2.88 (s, 3H), 2.61 (s, 6H), 2.32-2.18 (m, 1H), 2.12-2.00 (m, 1H), 1.42 (s, 9H), 1.36 (t, *J*=6.4 Hz, 3H).

### Example 271: Synthesis of Compound 471

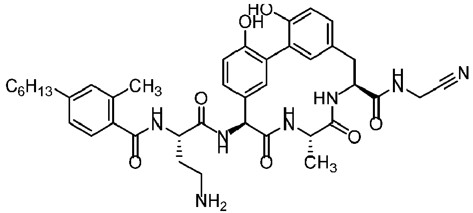

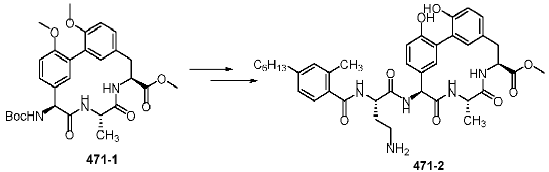

Compound 471-1 was prepared as a white solid utilizing methods analogous to those described in Example C, starting with (*S*)-2-((*tert*-butoxycarbonyl)amino)-2-(4-hydroxyphenyl)acetic acid instead of (*S*)-2-((*tert*-butoxycarbonyl)(methyl)amino)-2-(4-hydroxyphenyl)acetic acid. LCMS (Method 5-95 AB, ESI): t_{R} = 0.828 min, [M + Na]⁺ = 564.2.
Compound 471-2 was prepared as a white solid from compound 471-1 utilizing methods analogous to those described in Example O. LCMS (Method 5-95 AB, ESI): t_{R} = 0.699 min, [M + H]⁺ = 716.3. Starting from compound 471-2, typical Boc₂O protection (Boc₂O, Et₃N, as described in Example 6), ester hydrolysis (LiOH, THF/H₂O, as described in Example G), amide coupling (HATU/DIEA, as described in Example G) and Boc removal (TFA/HFIP, as described in Example G) conditions were applied to give compound 471 (formic acid salt) as a white solid. LCMS (Method 5-95 AB, ESI): t_{R} = 0.691 min, [M + H]⁺ = 741.1; ¹H NMR (400 MHz, DMSO-*d*₆) δ 9.09 (d, *J*=8.4 Hz, 1H), 8.85-8.70 (m, 2H), 8.55(d, *J*=7.2 Hz, 1H), 8.31 (s, 1H), 8.03 (d, *J*=8.0 Hz, 1H), 7.30 (d, *J*=8.0 Hz, 1H), 7.15-6.90 (m, 6H), 6.63 (t, *J*=8.4 Hz, 2H), 5.66 (d, *J*=8.0 Hz, 1H), 4.90-4.80 (m, 1H), 4.65-4.60 (m, 1H), 4.60-4.50 (m, 1H), 4.17 (d, *J*=5.2 Hz, 1H), 3.00-2.85 (m, 4H), 2.60-2.50 (m, 2H), 2.32 (s, 3H), 2.15-2.00 (m, 1H), 2.00-1.90 (m, 1H), 1.60-1.50 (m, 2H), 1.30-1.15 (m, 9H), 0.85 (t, *J*=6.8 Hz, 3H).

### Example 272: Synthesis of Compound 472

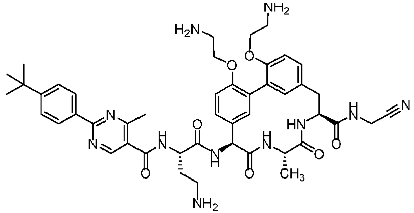

Compound 472 (formic acid salt) was prepared as a white solid from compound 471-1, by utilizing methods analogous to those described in Example D and Example G. LCMS (Method 5-95 AB, ESI): t_{R} = 0.719 min, [M + H]⁺ = 876.5; ¹H NMR (400 MHz, MeOH-*d*₄) δ 8.84 (s, 1H), 8.78 (s, 1H), 8.45 (br s, 1H), 8.38-8.36 (m, 2H), 7.57-7.49 (m, 3H), 7.31-7.06 (m, 2H), 7.04-7.02 (m, 2H), 6.83 (br s, 1H), 5.86 (s, 1H), 5.76 (s, 1H), 4.75-4.70 (m, 2H), 4.52-4.48 (m, 1H), 4.24-4.15 (m, 6H), 3.19-3.09 (m, 7H), 2.72 (s, 2H), 2.66 (s, 2H), 2.25-2.00 (m, 2H), 1.38 (br s, 12H).

### Example 273: Synthesis of Compound 473

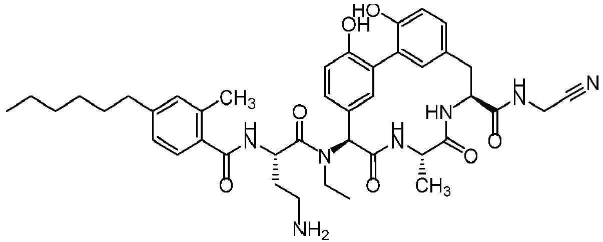

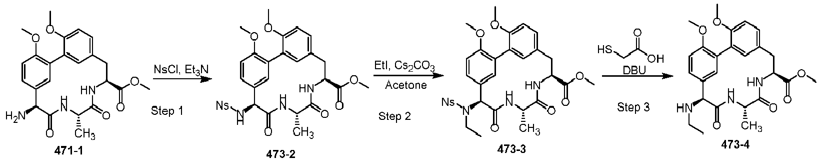

Step 1: To a solution of compound 471-1 (390 mg, 0.88 mmol) and 4-nitro-benzene sulfonyl chloride (255 mg, 1.15 mmol) in acetonitrile (5 mL) was added Et₃N (0.31 mL, 2.21 mmol) dropwise at 0 °C. The mixture was stirred at the same temperature for 3 h. The precipitate was collected, which was dried over oven to give compound 473-2 (520 mg, 94% yield) as a yellow solid.

Step 2: To a solution of compound 473-2 (520 mg, 0.83 mmol) and ethyl iodide (650 mg, 4.15 mmol) in acetone (10 mL) at 0 °C was added K₂CO₃ (573 mg, 4.15 mmol) and the mixture was gradually warmed up to 25 °C while stirring and stirred at the same temperature for 14 h. The volatiles were removed and the residue was re-dissolved with ethyl acetate (120 mL), which was washed with brine (3 x 50 mL). The organic layer was dried over Na₂SO₄, concentrated and the residue was purified by silica gel chromatography, eluting with 50% ethyl acetate in petroleum ether, to give compound 473-3 (400 mg, 73.6% yield) as a white solid.

Step 3: A solution of compound 473-3 (400 mg, 0.61 mmol), thioglycolic acid (0.28 mL, 4.03 mmol) and DBU (0.92 mL, 6.17 mmol) in acetonitrile (5 mL) was stirred at 25 °C for 4 h. The volatiles were removed under reduced pressure and the residue was re-dissolved with ethyl acetate (100 mL), which washed with brine (3 x 50 mL). The organic layer was dried over Na₂SO₄, concentrated and the residue was purified by silica gel chromatography, eluting with 67% ethyl acetate in petroleum ether, to give compound 473-4 (260 mg, 91% yield) as a white solid. LCMS (Method 5-95 AB, ESI): t_{R} = 0.698 min, [M + H]⁺ = 470.5.

Starting from compound 473-4, compound 473 (formic acid salt) was prepared as a white solid utilizing methods analogous to those described in Example 271. LCMS (Method 5-95 AB, ESI): t_{R} = 0.811 min, [M + H]⁺ = 768.5; ¹H NMR (400 MHz, MeOH-*d*₄) δ 8.50 (br s, 1H), 7.33 (d, *J*=8.0 Hz, 1H), 7.19 (d, *J*=8.0 Hz, 1H), 7.08-7.00 (m, 4H), 6.99 (br s, 1H), 6.91 (d, *J*=8.0 Hz, 1H), 6.84 (d, *J*=8.0 Hz, 1H), 6.28 (s, 1H), 5.14 (m, 1H), 4.85-4.77 (m, 1H), 4.60 (br s, 1H), 4.18 (s, 2H), 3.40-3.25 (m, 2H), 3.16-3.08 (m, 4H), 2.60 (t, *J*=7.6 Hz, 2H), 2.40 (s, 3H), 2.25-2.00 (m, 2H), 1.60-1.50 (m, 3H), 1.35-1.32 (m, 8H), 0.97 (t, *J*=7.2 Hz, 3H), 0.89 (t, *J*=6.8 Hz, 3H).

### Example 274: Synthesis of Compound 474

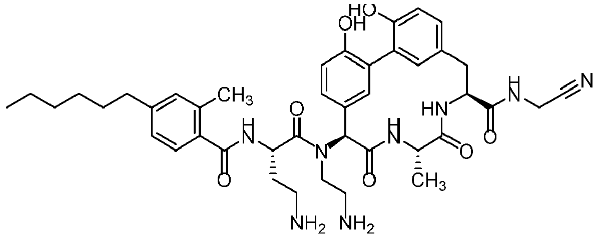

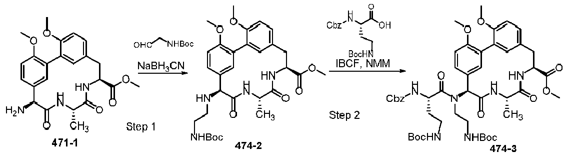

Step 1: To a solution of compound **471-1** (480 mg, 1.09 mmol), *tert*-butyl (2-oxoethyl)carbamate (173 mg, 1.2 mmol) and acetic acid (0.3 mL) in MeOH (10 mL) was added NaBH₃CN (75 mg, 1.2 mmol) and the resulting solution was stirred at 20 °C for 6 h. The volatiles were removed under reduced pressure and the residue was re-dissolved by EtOAc (40 mL), which was washed with brine (2 x 40 mL). The organic layer was dried over MgSO₄, concentrated and the residue was purified by preparatory-TLC (eluting with 50% EtOAc in petroleum ether, Rf=0.4) to afford compound 474-2 (440 mg, 69% yield) as a white solid. LCMS (Method 5-95 AB, ESI): t_{R} = 0.619 min, [M + H]⁺ = 585.1.

Step 2: A solution of (*S*)-2-(((benzyloxy)carbonyl)amino)-4-((*tert-*butoxycarbonyl)amino)butanoic acid (200 mg, 0.57 mmol), *N*-methyl morpholine (115 mg, 1.14mmol) and isobutyl chloroformate (62 mg, 0.45 mmol) in THF (10 mL) was stirred at -10 °C for 1 h, followed by the addition of compound 474-2 (232 mg, 0.40 mmol). The resulting mixture was stirred at the same temperature for another 1 h. The volatiles were removed under reduced pressure and the residue was re-dissolved by EtOAc (30 mL), which was washed with brine (2 x 30 mL). The organic layer was dried over MgSO₄, concentrated and the residue was purified by preparatory-TLC (eluting with 50% EtOAc in petroleum ether, Rf=0.3) to give compound 474-3 (130 mg, 25% yield) as a white solid. LCMS (Method 5-95 AB, ESI): t_{R} = 0.835 min, [M + H]⁺ = 919.6.

Starting from compound 474-3, compound 474 (formic acid salt) was prepared as a white solid utilizing methods analogous to those described in Example 271. LCMS (Method 5-95 AB, ESI): t_{R} = 0.661 min, [M + H]⁺ = 783.5; ¹H NMR (400 MHz, MeOH-*d*₄) δ 8.55 (br s, 2H), 7.44 (d, *J*=7.2 Hz, 1H), 7.33 (d, *J*=7.2 Hz, 1H), 7.17 (br s, 1H), 7.18-7.06 (m, 4H), 6.96-6.81 (m, 1H), 6.29 (s, 1H), 5.99 (s, 1H), 5.09 (br s,1H), 4.78-4.76 (m, 2H), 4.17 (d, *J*=8.0 Hz, 2H), 3.78-3.40 (m, 4H), 3.35-3.02 (m, 4H), 2.61 (t, *J*=7.2 Hz, 2H), 2.47 (s, 3H), 2.41-2.05 (m, 2H), 1.64-1.60 (m, 2H), 1.37-1.30 (m, 9H), 0.91 (t, *J*=6.8 Hz, 3H).

### Example 275: Synthesis of Compound 475

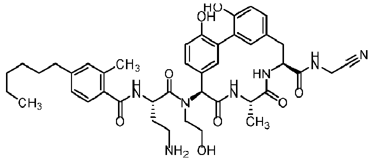

Compound 475 (formic acid salt) was prepared as a white solid utilizing methods analogous to those described in Example 274. LCMS (Method 5-95 AB, ESI): t_{R} = 0.814 min, [M + H]⁺ = 784.6; ¹H NMR (400 MHz, MeOH-*d*₄) δ 8.54 (br s, 3H), 7.33 (d, *J*=8.0 Hz, 1H), 7.18-7.01 (m, 6H), 6.87 (d, *J*=8.0 Hz, 1H), 6.80 (d, *J*=8.0 Hz, 1H), 6.15 (s, 1H), 5.00-4.75 (m, 3H), 4.18 (s, 2H), 3.80-3.70 (m, 2H), 3.30-3.10 (m, 3H), 3.07-3.01 (m, 3H), 2.63-2.59 (m, 2H), 2.40 (s, 3H), 2.30-2.15 (m, 1H), 2.13-2.02 (m, 1H), 1.70-1.50 (m, 3H), 1.37-1.27 (m, 8H), 0.90 (t, *J*=6.8 Hz, 3H).

### Example 276: Synthesis of Compound 476

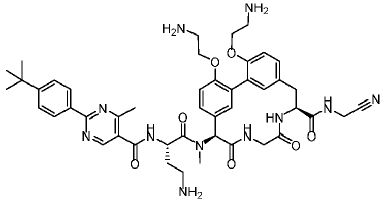

Compound 476 (formic acid salt) was prepared as a white solid utilizing methods analogous to those described in Example 7. LCMS (Method 5-95 AB, ESI): t_{R} = 0.718 min, [M + H]⁺ = 876.4; ¹H NMR (400 MHz, MeOH-*d*₄) δ 8.72 (s, 1H), 8.46 (br s, 2H), 8.28 (d, *J*=8.0 Hz, 1H), 7.52 (d, *J*=8.0 Hz, 1H), 7.35-7.30 (m, 1H), 7.25-7.20 (m, 1H), 7.12 (d, *J*=8.4 Hz, 1H), 7.06 (d, *J*=8.4 Hz, 1H), 6.86 (s, 1H), 6.64 (s, 1H), 6.56 (s, 1H), 5.22-5.18 (m, 1H), 4.80-4.70 (m, 2H), 4.67 (d, *J*=14.4 Hz, 1H), 4.34-4.22 (m, 4H), 4.20 (s, 2H), 3.59-3.52 (m, 1H), 3.31-3.25 (m, 4H), 3.18-3.12 (m, 2H), 3.00-2.95 (m, 1H), 2.92 (s, 3H), 2.66 (s, 3H), 2.28-2.17 (m, 1H), 2.15-2.01 (m, 1H), 1.39 (s, 9H).

### Example 277: Synthesis of Compound 477

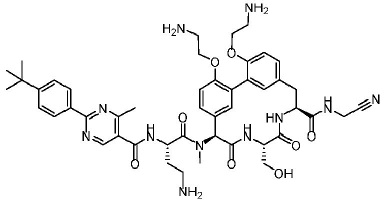

Compound 477 (formic acid salt) was prepared as a white solid utilizing methods analogous to those described in Example 7. LCMS (Method 5-95 AB, ESI): t_{R} = 0.722 min, [M + H]⁺ = 906.4; ¹H NMR (400 MHz, MeOH-*d*₄) δ 8.72 (s, 1H), 8.50 (br s, 3H), 8.30 (d, *J*=7.6 Hz, 2H), 7.53 (d, *J*=8.0 Hz, 2H), 7.31 (d, *J*=7.6 Hz, 1H), 7.22-7.14 (m, 2H), 7.06 (d, *J*=8.0 Hz, 1H), 6.79 (s, 1H), 6.66 (s, 1H), 6.50 (s, 1H), 5.20-5.15 (m, 1H), 4.81-4.75 (m, 2H), 4.28-4.14 (m, 4H), 4.19 (s, 2H), 3.72 (d, *J*=7.2 Hz, 2H), 3.43-3.38 (m, 2H), 3.22-3.13 (m, 5H), 3.05-3.01 (m, 1H), 2.95 (s, 3H), 2.67 (s, 3H), 2.27-2.66 (m, 1H), 2.18-2.16 (m, 1H), 1.38 (s, 9H).

### Example 278: Synthesis of Compound 478

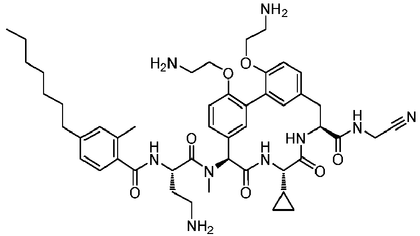

Compound 478 (formic acid salt) was prepared from Compound 105 (Example U) utilizing methods analogous to those described in Example G (Compound 101) to give a white solid. LCMS (ESI): [M+H]⁺ = 880; ¹H NMR (400 MHz, Methanol-*d*₄) δ 7.34-7.32 (m, 2H), 7.26 (dd, *J* = 8.6, 2.3 Hz, 1H), 7.19 (d, *J* = 8.6 Hz, 1H), 7.16 - 7.08 (m, 3H), 6.82 (dd, *J* = 9.8, 2.4 Hz, 2H), 6.39 (s, 1H), 5.15 (dd, *J* = 7.9, 5.5 Hz, 1H), 4.98 (dd, *J* = 11.5, 3.2 Hz, 1H), 4.29 - 4.19 (m, 7H), 3.30 (d, *J* = 3.3 Hz, 1H), 3.24-3.17 (m, 5H), 3.16 - 3.09 (m, 2H), 2.93 (s, 2H), 2.64 (t, *J* = 7.6 Hz, 2H), 2.44 (s, 3H), 2.34 - 2.25 (m, 1H), 2.19-2.10 (m, 1H), 1.63 (q, *J* = 7.3 Hz, 2H), 1.39 - 1.28 (m, 9H), 1.26-1.19 (m, 1H), 0.96 - 0.88 (m, 3H), 0.57 - 0.49 (m, 4H).

### Example 279: Synthesis of Compound 479

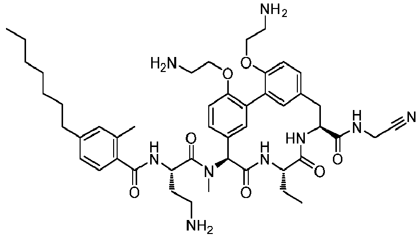

Compound 479 (formic acid salt) was prepared utilizing methods analogous to those described in Example G (Compound 101) to give a white solid. LCMS (ESI): [M+H]⁺ = 868; ¹H NMR (400 MHz, Methanol-*d*₄) δ 7.37 - 7.32 (m, 2H), 7.27 (dd, *J* = 8.6, 2.4 Hz, 1H), 7.20 (d, *J* = 8.6 Hz, 1H), 7.15 - 7.08 (m, 3H), 6.88 (dd, *J* = 17.2, 2.4 Hz, 2H), 6.34 (s, 1H), 5.15 (dd, *J* = 7.8, 5.5 Hz, 1H), 4.94 (dd, *J* = 11.5, 3.1 Hz, 1H), 4.62 (t, *J* = 7.4 Hz, 1H), 4.31 - 4.19 (m, 6H), 3.25 (q, *J* = 4.9 Hz, 4H), 3.16 - 3.09 (m, 2H), 2.94 (s, 3H), 2.64 (t, *J* = 7.6 Hz, 2H), 2.43 (s, 3H), 2.34-2.23 (m, 1H), 2.19 - 2.09 (m, 1H), 1.88-1.78 (m, 1H), 1.73-1.59 (m, 2H), 1.38 - 1.29 (m, 10H), 0.98 (t, *J* = 7.4 Hz, 3H), 0.95 - 0.89 (m, 2H).

### Example 280: Synthesis of Compound 480

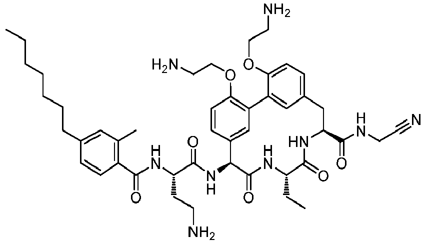

Compound 480 (formic acid salt) was prepared utilizing methods analogous to those described in Example 279 and Example G (Compound 101) to give a white solid. LCMS (ESI): [M+H]⁺ = 854; ¹H NMR (400 MHz, Methanol-*d*₄) δ 7.46 (dd, *J* = 18.2, 7.7 Hz, 1H), 7.36 (dd, *J* = 22.5, 7.7 Hz, 1H), 7.29 - 7.20 (m, 1H), 7.15 - 7.03 (m, 3H), 6.99 (d, *J* = 8.3 Hz, 1H), 6.84 - 6.79 (m, 1H), 5.87 (s, 1H), 4.99 - 4.91 (m, 2H), 4.69 - 4.61 (m, 1H), 4.52 (d, *J* = 11.0 Hz, 1H), 4.21 - 3.99 (m, 6H), 3.12 - 2.92 (m, 4H), 2.61 (td, *J* = 7.6, 3.9 Hz, 3H), 2.40 (d, *J* = 22.7 Hz, 3H), 1.80 (dt, *J* = 12.5, 6.5 Hz, 1H), 1.64 (d, *J* = 21.1 Hz, 3H), 1.30 (d, *J* = 13.8 Hz, 12H), 0.97 (t, *J* = 7.4 Hz, 3H), 0.92 - 0.87 (m, 3H).

### Example 281: Synthesis of Compound 481

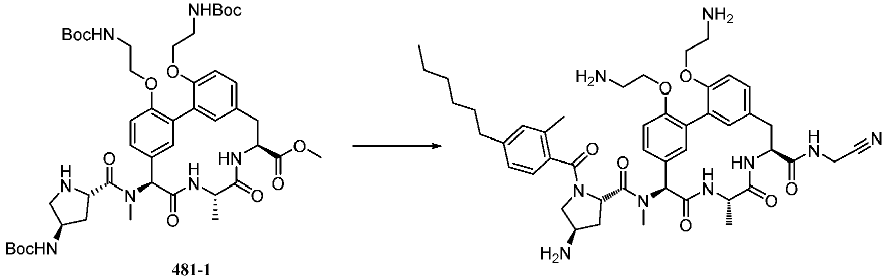

Compound 481-1 was prepared as an off-white solid following the method for compound 101-I using (2*S*,4*R*)-1-benzyloxycarbonyl-4-(*tert*-butoxycarbonylamino)pyrrolidine-2-carboxylic acid. LCMS (ESI): [M+H]⁺ = 926.

Compound 481 (formic acid salt) was prepared utilizing methods analogous to those described in Example G (Compound 101) to give a off white solid. LCMS (ESI): [M+H]⁺ = 852; ¹H NMR (400 MHz, Methanol-*d*₄) δ 7.42 - 7.36 (m, 1H), 7.29 - 7.04 (m, 8H), 6.87 (dd, *J* = 21.4, 2.5 Hz, 1H), 6.39 (d, *J* = 6.2 Hz, 1H), 5.21 (t, *J* = 7.2 Hz, 1H), 4.80 - 4.72 (m, 1H), 4.24 - 4.15 (m, 5H), 3.84 - 3.78 (m, 1H), 3.75 - 3.68 (m, 1H), 3.25 - 3.19 (m, 1H), 3.19 - 3.11 (m, 4H), 2.95 (d, *J* = 3.2 Hz, 3H), 2.62 (t, *J* = 7.6 Hz, 2H), 2.39 (s, 2H), 2.34 (q, *J* = 5.4, 4.6 Hz, 4H), 1.62 (dt, *J* = 14.9, 8.1 Hz, 3H), 1.38 - 1.28 (m, 10H), 0.93 - 0.88 (m, 3H).

### Example 282: Synthesis of Compound 482

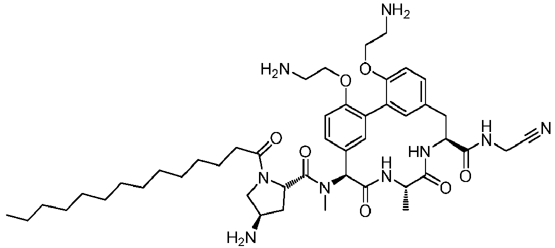

Compound 482 (formic acid salt) was prepared as an off-white solid utilizing methods analogous to those described in Example G (Compound 101). LCMS (Method A, ESI): t_{R} = 3.519 min, [M + H]⁺ = 860; ¹H NMR (400 MHz, Methanol-*d*₄) δ 7.40 - 7.34 (m, 1H), 7.23 (dd, *J* = 8.6, 2.4 Hz, 1H), 7.18 (d, *J* = 8.4 Hz, 1H), 7.09 (d, *J* = 8.6 Hz, 1H), 6.85 (dd, *J* = 18.5, 2.5 Hz, 2H), 6.30 (s, 1H), 5.02 (dd, *J* = 7.9, 5.8 Hz, 1H), 4.88 (d, *J* = 3.1 Hz, 1H), 4.80 - 4.74 (m, 1H), 4.26 - 4.18 (m, 6H), 4.00 - 3.88 (m, 2H), 3.58 (dd, *J* = 10.1, 4.1 Hz, 1H), 3.22 - 3.16 (m, 4H), 2.88 (s, 3H), 2.44 - 2.37 (m, 2H), 2.31 - 2.26 (m, 1H), 1.65 (q, *J =* 7.4 Hz, 2H), 1.42 - 1.27 (m, 26H), 0.93 - 0.87 (m, 3H).

### Example 283: Synthesis of Compound 483

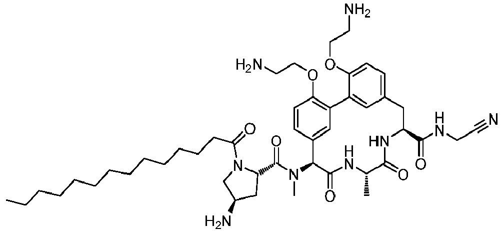

Compound 483 (formic acid salt) was prepared as an off-white solid utilizing methods analogous to those described in Example G (Compound 101). LC-MS: (Method A, ESI): t_{R} = 3.522 min, [M + H]⁺ = 860; ¹H NMR (400 MHz, Methanol-*d*₄) δ 7.31 (dd, *J* = 8.6, 2.5 Hz, 1H), 7.17 (dd, *J* = 8.5, 2.4 Hz, 1H), 7.11 (d, *J* = 8.6 Hz, 1H), 7.02 (d, *J* = 8.4 Hz, 1H), 6.78 (dd, *J* = 19.8, 2.4 Hz, 2H), 6.22 (s, 1H), 4.99 (dd, *J* = 8.3, 5.2 Hz, 1H), 4.81 (s, 1H), 4.69 (q, *J* = 6.7 Hz, 1H), 4.24 - 4.10 (m, 6H), 4.01 - 3.96 (m, 2H), 3.63 (q, *J* = 7.4 Hz, 1H), 3.22 - 3.15 (m, 4H), 3.11 - 3.01 (m, 1H), 2.81 (s, 3H), 2.38 - 2.26 (m, 4H), 1.58 (q, *J* = 7.3 Hz, 2H), 1.36 - 1.19 (m, 24H), 0.87 - 0.80 (m, 3H).

### Example 284: Synthesis of Compound 484

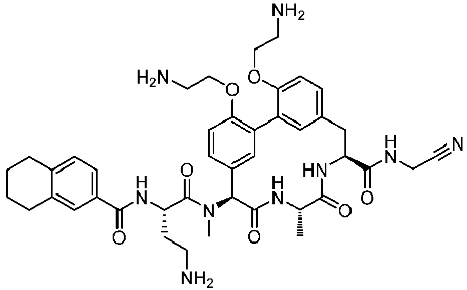

Compound 484 (formic acid salt) was prepared as an off-white solid utilizing methods analogous to those described in Example G (Compound 101). LC-MS: (Method A, ESI): t_{R} = 2.073 min, [M + H]⁺ = 796; ¹H NMR (400 MHz, DMSO-*d*₆) δ 9.04 (d, *J* = 7.8 Hz, 1H), 8.78 (t, *J* = 5.7 Hz, 1H), 8.73 (d, *J* = 7.7 Hz, 1H), 8.37 (d, *J* = 9.0 Hz, 1H), 7.66 - 7.58 (m, 2H), 7.22 - 7.13 (m, 2H), 7.06 (dd, *J* = 27.1, 8.6 Hz, 2H), 6.71 (d, *J* = 2.5 Hz, 2H), 6.28 (s, 1H), 4.97 - 4.90 (m, 1H), 4.80 - 4.67 (m, 2H), 4.17 (d, *J* = 5.8 Hz, 2H), 4.03 - 3.93 (m, 3H), 3.18 (d, *J* = 16.8 Hz, 2H), 3.04 - 2.97 (m, 1H), 2.88 - 2.72 (m, 10H), 2.05 - 1.91 (m, 2H), 1.80 - 1.71 (m, 4H), 1.19 (d, *J* = 6.7 Hz, 3H).

### Example 285: Synthesis of compound 485

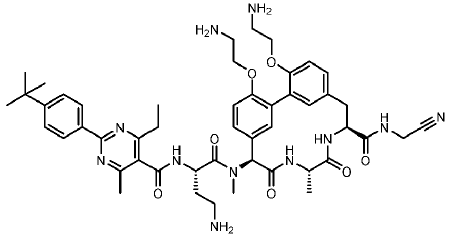

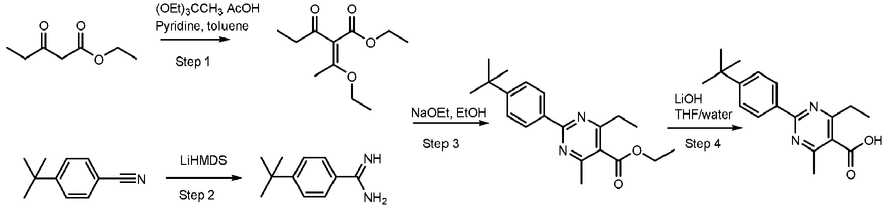

Step 1: A solution of ethyl 3-oxopentanoate (1.00 mL, 7.02 mmol), triethyl orthoacetate (2.0 mL, 11 mmol), pyridine (57 µL, 0.698 mmol), acetic acid (40 µL, 0.697 mmol) and toluene (6.0 mL, 56 mmol) was heated in a sealed vial at 120 °C for 23 h. The reaction mixture was evaporated under reduced pressure to yield the crude product as an orange oil. The crude product was purified via flash chromatography on silica gel (40 g silica, solvent gradient: 0-50% ethyl acetate in heptanes) to yield 254.2 mg (17%) of ethyl (*Z*)-2-(1-ethoxyethylidene)-3-oxopentanoate as an orange oil. LCMS (ESI): [M+H]⁺ = 215.0.

Step 2: To a solution of 4-tert-butylbenzonitrile (9.00 mL, 53 mmol) in diethyl ether (100 mL, 960 mmol) at 0 °C was added lithium bis(trimethylsilyl)amide (1 mol/L) in THF (110.0 mL, 110.0 mmol), dropwise over 30 minutes. The reaction was stirred at 0 °C for 2 h and then warmed to room temperature. After an additional 6 h, the reaction was cooled in an ice bath and quenched by careful addition of hydrogen chloride (12 mol/L in water) (20 mL, 240 mmol), diluted with water (50 mL) and then stirred for 10 minutes. The resulting mixture was extracted with water (4 x 50 mL). The combined aqueous extracts were adjusted to pH ~13 with aqueous sodium hydroxide (10 mol/L) in water (15 mL, 150 mmol), and then extracted with 10% isopropanol in dichloromethane (4 x 50 mL). The combined dichlormethane extracts were dried over magnesium sulfate, and filtered. The filtered solids were stirred with 1:1 DCM:EtOAc and re-filtered, the combined filtrates were evaporated in vacuo to yield 3.494 g (37%) of 4-(tert-butyl)benzimidamide. LCMS (ESI): [M+H]⁺ = 177.15; ¹H NMR (400 MHz, DMSO-d₆) δ 7.68 (d, *J* = 8.4 Hz, 2H), 7.43 (d, *J* = 8.2 Hz, 2H), 6.98 (br s, 3H), 1.29 (s, 9H).

Step 3: A mixture of ethyl (2Z)-2-(1-ethoxyethylidene)-3-oxo-pentanoate (254.2 mg, 1.186 mmol), 4-*tert*-butyl-benzamidine (197.0 mg, 1.118 mmol), sodium ethoxide (21 wt% solution in ethanol) (0.80 mL, 2.1 mmol), and ethanol (1.5 mL, 26 mmol) was heated in a sealed vial at 70 °C overnight. The reaction mixture was evaporated onto celite. The crude product was purified via flash chromatography on silica gel (12 g silica, solvent gradient: 0-50% ethyl acetate in heptanes) to yield 80.2 mg (22%) of ethyl 2-(4-(*tert*-butyl)phenyl)-4-ethyl-6-methylpyrimidine-5-carboxylate as a clear, colorless oil. LCMS (ESI): [M+H]⁺ = 327.

Step 4: To a solution of ethyl 2-(4-*tert*-butylphenyl)-4-ethyl-6-methyl-pyrimidine-5-carboxylate (97 mg, 0.2972 mmol) in tetrahydrofuran (2.0 mL, 25 mmol) was added lithium hydroxide (1.0 M in water) (0.30 mL, 0.30 mmol). The reaction mixture was stirred at room temperature for 3.5 h. Following the addition of additional lithium hydroxide (1.0 M in water) (0.90 mL, 0.90 mmol) and methanol (1 mL), the reaction mixture was heated 50 °C overnight. The reaction mixture was diluted with dichloromethane, neutralized with hydrochloric acid (1 mol/L) in water (1.2 mL), and washed with brine. The aqueous layer was extracted with an additional portion of dichloromethane, and the combined organic layers were dried over magnesium sulfate, filtered, and evaporated in vacuo to yield 88.4 mg (99.7%) of 2-(4-(*tert-*butyl)phenyl)-4-ethyl-6-methylpyrimidine-5-carboxylic acid, which was carried forward without purification. LCMS (ESI): [M+H]⁺ = 299.15.

Compound 485 (TFA salt) was prepared from 2-(4-(*tert*-butyl)phenyl)-4-ethyl-6-methylpyrimidine-5-carboxylic acid and Compound 101-K utilizing methods analogous to those described in Example G (Compound 101). LCMS (Method A, ESI): t_{R} = 2.971 min, [M+H]⁺ = 918.5; ¹H NMR (400 MHz, DMSO-*d*₆) δ 9.18 (d, *J* = 7.3 Hz, 1H), 8.96 (d, *J* = 7.9 Hz, 1H), 8.71 (t, *J* = 5.7 Hz, 1H), 8.37 - 8.29 (m, 3H), 7.99 - 7.72 (m, 6H), 7.56 (d, *J* = 8.5 Hz, 2H), 7.28 - 7.22 (m, 1H), 7.21 - 7.07 (m, 3H), 6.74 (s, 1H), 6.45 (s, 1H), 5.09 - 4.99 (m, 1H), 4.82 - 4.66 (m, 2H), 4.28 - 4.05 (m, 7H), 3.21 - 2.98 (m, 11H), 2.91 (s, 3H), 2.77 (q, *J* = 7.4 Hz, 2H), 2.02 (d, *J* = 39.8 Hz, 2H), 1.34 (s, 9H), 1.29 (t, *J* = 7.5 Hz, 3H), 1.21 (d, *J* = 6.7 Hz, 3H).

### Example 286: Synthesis of compound 486

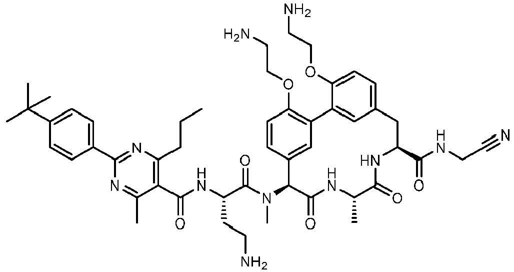

Compound 486 (TFA salt) was prepared from ethyl 3-oxohexanoate utilizing methods analogous to those described in Example 285. LCMS (Method A, ESI): t_{R} = 3.162 min, [M+H]⁺ = 932.5; ¹H NMR (400 MHz, DMSO-*d*₆) δ 9.18 (d, *J* = 7.3 Hz, 1H), 8.95 (d, *J* = 7.9 Hz, 1H), 8.71 (t, *J* = 5.7 Hz, 1H), 8.38 - 8.30 (m, 3H), 7.93 - 7.76 (m, 10H), 7.56 (d, *J* = 8.5 Hz, 2H), 7.26 - 7.08 (m, 3H), 6.74 (d, *J* = 2.4 Hz, 1H), 6.47 (s, 1H), 5.09 - 4.98 (m, 1H), 4.81 - 4.67 (m, 2H), 4.29 - 4.05 (m, 6H), 3.28 - 2.93 (m, 6H), 2.91 (s, 2H), 2.79 - 2.63 (m, 2H), 2.12 - 1.92 (m, 2H), 1.78 (q, *J* = 7.5 Hz, 2H), 1.34 (s, 9H), 1.21 (d, *J* = 6.7 Hz, 2H), 0.95 (t, *J* = 7.3 Hz, 2H).

### Example 287: Synthesis of compound 487

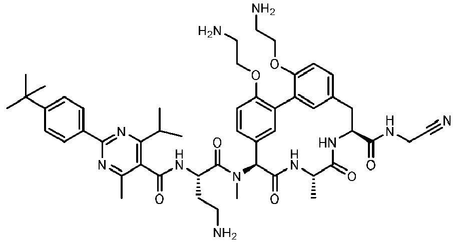

Compound 487 (TFA salt) was prepared from ethyl 4-methyl-3-oxopentanoate utilizing methods analogous to those described in Example 285. LCMS (Method A, ESI): t_{R} = 3.260 min, [M+H]⁺ = 932.5; ¹H NMR (400 MHz, DMSO-*d*₆) δ 9.20 (d, *J* = 7.3 Hz, 1H), 8.95 (d, *J* = 8.1 Hz, 1H), 8.69 (d, *J* = 5.8 Hz, 1H), 8.38 - 8.29 (m, 3H), 7.83 (s, 9H), 7.57 (d, *J* = 8.4 Hz, 2H), 7.24 (d, *J* = 8.6 Hz, 1H), 7.20 - 7.06 (m, 3H), 6.73 (s, 2H), 6.46 (s, 1H), 5.08 - 4.97 (m, 1H), 4.80 - 4.65 (m, 2H), 4.28 - 4.06 (m, 6H), 3.25 - 2.97 (m, 8H), 2.91 (s, 3H), 2.13 - 1.92 (m, 2H), 1.34 (s, 9H), 1.29 (d, *J* = 6.6 Hz, 6H), 1.21 (d, *J* = 6.7 Hz, 3H).

### Example 288: Synthesis of Compound 488

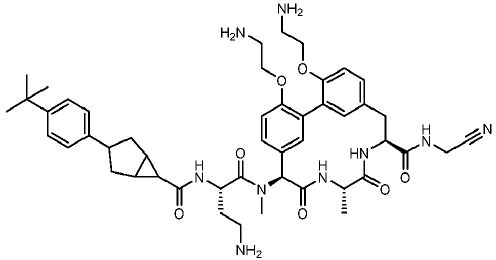

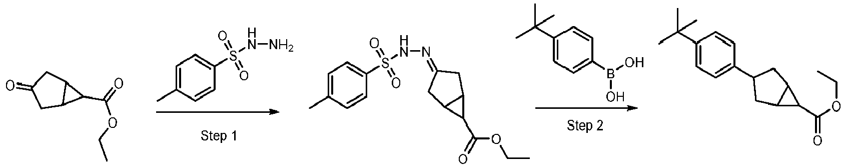

Step 1: To a solution of ethyl 3-oxobicyclo[3.1.0]hexane-6-carboxylate (519.9 mg, 3.091 mmol) in ethanol (8.0 mL, 140 mmol) was added 4-methylbenzenesulfonohydrazide (596.9 mg, 3.109 mmol). The reaction mixture was stirred at room temperature for 90 min, and then evaporated in vacuo to yield ethyl 3-(2-tosylhydrazono)bicyclo[3.1.0]hexane-6-carboxylate which was carried forward without purification. LCMS (ESI): [M+H]⁺ = 337.05.

Step 2: The product obtained in Step 1 was combined with (4-tert-butylphenyl)boronic acid (840.2 mg, 4.720 mmol), potassium carbonate (669 mg, 4.8405 mmol) and 1,4-dioxane (12 mL, 140 mmol) and the mixture was heated at 100 °C for 2 h. Additional (4-tert-butylphenyl)boronic acid (514.5 mg, 2.890 mmol) and 1,4-dioxane (10 mL) were added and the reaction mixture was heated at 110 °C overnight. The reaction mixture was evaporated *in vacuo* onto celite. The crude product was purified via flash chromatography on silica gel (40 g silica, solvent gradient: 0-20% ethyl acetate in heptanes) to provide 249.5 mg of ethyl 3-(4-(tert-butyl)phenyl)bicyclo[3.1.0]hexane-6-carboxylate as a yellow oil (28% yield over 2 steps).

Compound 488 (TFA salt) was prepared from of ethyl 3-(4-(tert-butyl)phenyl)bicyclo[3.1.0]hexane-6-carboxylate utilizing methods analogous to those described in Example 285. LCMS (Method A, ESI): t_{R} = 2.997 min, [M+H]⁺ = 879.5.

### Example 289: Synthesis of Compound 489

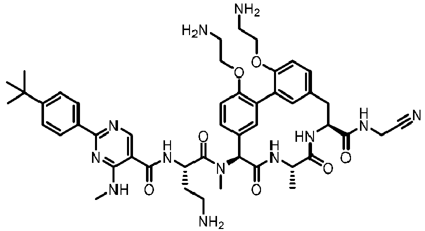

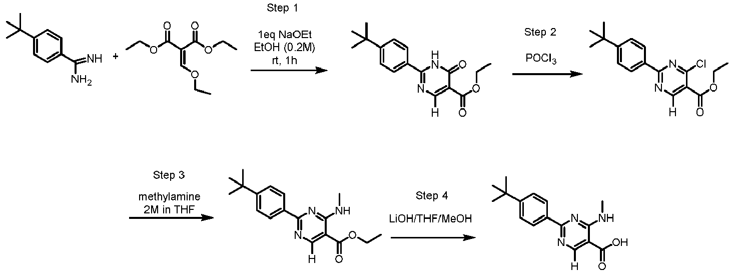

Step 1: To a solution of 4-*tert*-butylbenzamidine (1.02 g, 5.78 mmol) in ethanol (29 mL) was added diethyl 2-(ethoxymethylene)propanedioate (1.25 g, 5.78 mmol), followed by sodium ethoxide (2.6 M solution in ethanol, 2.4 mL, 5.78 mmol). The reaction was left to stir under nitrogen at room temperature. After 1.5 h the reaction was concentrated to give 2.29 g of the crude product, which was carried over without purification.

Step 2: To a vial containing ethyl 2-(4-*tert*-butylphenyl)-6-oxo-1*H*-pyrimidine-5-carboxylate (1.20 g, 4.0 mmol) was added phosphoryl chloride (1.9 mL, 20 mmol). The reaction was heated to 60 °C. After 1.5 h additional 3 equiv of phosphoryl chloride was added (1.2 mL, 12 mmol). After 3 h the reaction was evaporated *in vacuo* and used directly in the next step.

Step 3: To a solution of ethyl 2-(4-*tert*-butylphenyl)-4-chloro-pyrimidine-5-carboxylate (1.28 g, 4 mmol) in THF (8 mL) was added a solution of methylamine (2 M) in THF (8 mL, 16 mmol). After stirring for 2 h at room temperature the reaction was concentrated, treated with water, and extracted with DCM (3 times). Combined organic layers were washed with brine, dried over magnesium sulfate, filtered, and concentrated. Purification by flash chromatography on silica gel (solvent gradient: 0-40 % isopropyl acetate/heptane, then 100% isopropyl acetate) gave the desired product (0.754 g, 60%) as a white solid. LCMS (ESI): [M+1]⁺= 314.2; ¹H NMR (400 MHz, DMSO-*d*₆) δ 8.83 (s, 1H), 8.38 - 8.31 (m, 2H), 8.22 (d, *J* = 5.2 Hz, 1H), 7.54 (d, *J* = 8.5 Hz, 2H), 4.33 (q, *J* = 7.1 Hz, 2H), 3.11 (d, *J* = 4.8 Hz, 3H), 1.37 - 1.30 (m, 12H).

Step 4: To a solution of ethyl 2-(4-*tert*-butylphenyl)-4-(methylamino)pyrimidine-5-carboxylate (0.15 g, 0.50 mmol) in THF (2.7 mL) and methanol (0.8 mL) was added a solution of lithium hydroxide (1M) in water (1.5 mL, 1.5 mmol). The reaction was heated to 60 °C. After stirring overnight the reaction was evaporated *in vacuo,* treated with dilute HCl to reach a pH of ~ 5-6, and extracted with ethyl acetate (3 times). The combined organic layers were washed with brine, dried over magnesium sulfate, filtered, and evaporated *in vacuo.* The crude solid was dissolved in DCM and filtered through a pad of celite, washing with 5 % methanol/DCM. The filtrate was evaporated *in vacuo* to give the title compound (142 mg) as a white solid. LCMS (ESI): [M+1]⁺= 286; ¹H NMR (400 MHz, DMSO-*d*₆) δ 13.24 (s, 1H), 8.77 (s, 1H), 8.33 (d, *J* = 8.3 Hz, 2H), 7.52 (d, *J* = 8.3 Hz, 2H), 7.14 (s, 1H), 3.09 (d, *J* = 4.7 Hz, 3H), 1.32 (s, 9H).

Step 5: Compound **489** (TFA salt) was prepared from 2-(4-(*tert*-butyl)phenyl)-4-(methylamino)pyrimidine-5-carboxylic acid utilizing the methods described in Example G (Compound 101-K). LCMS (Method A, ESI): t_{R} = 2.82 min, [M + H]⁺ = 906.4; ¹H NMR (400 MHz, DMSO-*d*₆) δ 9.04 (d, *J* = 7.8 Hz, 1H), 8.95 (d, *J* = 7.8 Hz, 1H), 8.83 (s, 1H), 8.71 (t, *J* = 5.7 Hz, 1H), 8.56 (d, *J* = 5.5 Hz, 1H), 8.39 (d, *J* = 9.1 Hz, 1H), 8.36 - 8.24 (m, 2H), 7.94 - 7.68 (m, 10H), 7.59 - 7.48 (m, 2H), 7.30 - 7.20 (m, 2H), 7.15 (d, *J* = 8.7 Hz, 1H), 7.08 (t, *J* = 7.0 Hz, 1H), 6.76 - 6.68 (m, 2H), 6.32 (s, 1H), 5.04 - 4.90 (m, 1H), 4.83 - 4.64 (m, 2H), 4.23 - 4.07 (m, 6H), 3.17 - 2.99 (m, 10H), 2.73 (s, 3H), 2.11 (s, 2H), 1.40 - 1.27 (m, 11H), 1.22 (d, *J* = 6.8 Hz, 3H).

### Example 290: Synthesis of Compound 490

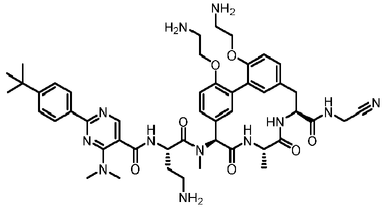

Compound 490 (TFA salt) was prepared as a white solid utilizing methods analogous to those described in Example 289. LCMS (Method A, ESI): t_{R} = 2.66 min, [M + H]⁺ = 920.5; ¹H NMR (400 MHz, DMSO-*d*₆) δ 9.12 (d, *J =* 7.3 Hz, 1H), 8.95 (d, *J* = 8.0 Hz, 1H), 8.71 (t, *J* = 5.6 Hz, 1H), 8.39 - 8.23 (m, 4H), 7.95 - 7.72 (m, 10H), 7.56 - 7.49 (m, 2H), 7.28 - 7.02 (m, 4H), 6.73 (d, *J* = 2.4 Hz, 2H), 6.40 (s, 1H), 4.91 (q, *J* = 7.4 Hz, 1H), 4.81 - 4.65 (m, 2H), 4.26 - 4.06 (m, 6H), 3.22 - 2.91 (m, 15H), 2.85 (s, 2H), 2.08 - 1.95 (m, 2H), 1.33 (s, 9H), 1.21 (d, *J* = 6.7 Hz, 3H).

### Example 291: Synthesis of Compound 491

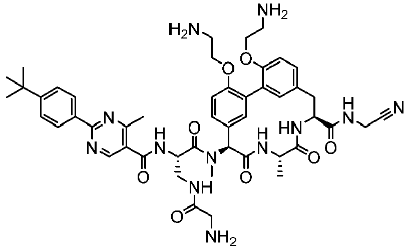

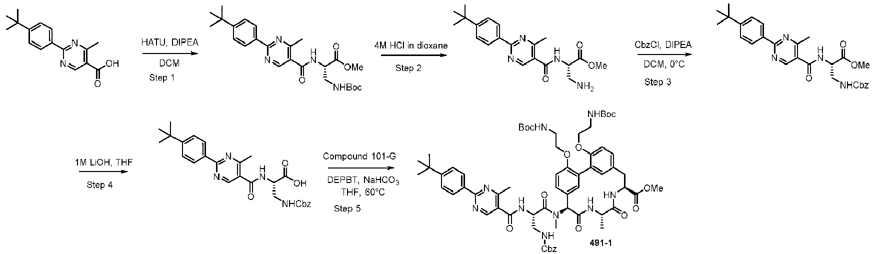

Step 1: To a mixture of 2-(4-*tert*-butylphenyl)-4-methyl-pyrimidine-5-carboxylic acid (prepared as described in Example 69) (811 mg, 3.0 mmol) and DCM (9 mL) was added DIPEA (1.57 mL, 9 mmol) then HATU (1.25 g, 3.3 mmol). The reaction mixture was stirred at 20 °C for 5 min then [(1*S*)-1-[(*tert-*butoxycarbonylamino)methyl]-2-methoxy-2-oxo-ethyl]ammonium chloride (917 mg, 3.6 mmol) was added. The mixture was stirred at 20 °C for 18 h and then diluted with DCM, washed with saturated aqueous ammonium chloride, brine and then dried over Na₂SO₄ and the solvent removed. The residue was purified by silica gel chromatography (eluting with 40% ethyl acetate/cyclohexane) to give methyl (2S)-3-(*tert*-butoxycarbonylamino)-2-[[2-(4-*tert*-butylphenyl)-4-methyl-pyrimidine-5-carbonyl]amino]propanoate (1.37 g, 97%) as a white foam solid. LCMS (ESI): [M + H]⁺ = 471.

Step 2: Methyl (2S)-3-(*tert*-butoxycarbonylamino)-2-[[2-(4-tert-butylphenyl)-4-methyl-pyrimidine-5-carbonyl]amino]propanoate (1.37 g, 2.91 mmol) was dissolved in HCl in dioxane (4.0 M, 5 mL, 20 mmol) and stirred at 20 °C for 5 h. The solvent was removed to give [(2*S*)-2-[[2-(4-*tert-*butylphenyl)-4-methyl-pyrimidine-5-carbonyl]amino]-3-methoxy-3-oxo-propyl]ammonium chloride 1.3 g (109%) as an off white solid. LCMS (ESI): [M + H]⁺ = 371.

Step 3: To a solution of [(2S)-2-[[2-(4-*tert*-butylphenyl)-4-methyl-pyrimidine-5-carbonyl]amino]-3-methoxy-3-oxo-propyl]ammonium chloride (407 mg, 1 mmol) in DCM (4 mL) under nitrogen at 0 °C was added DIPEA (0.52 mL, 3 mmol) followed by benzyl chloroformate (0.17 mL, 1.1 mmol) dropwise. The mixture was stirred at 0 °C for 2 h. The reaction was quenched with water, diluted with DCM and allowed to warm to 20 °C. The phases were separated, and the organic layer was washed with brine, dried over Na₂SO₄ and the solvent removed. The residue was purified by silica gel chromatography (eluting with 50% ethyl acetate/cyclohexane) to give methyl (2S)-3-(benzyloxycarbonylamino)-2-[[2-(4-*tert*-butylphenyl)-4-methyl-pyrimidine-5-carbonyl]amino]propanoate (426 mg, 84%) as a white foam. LCMS (ESI): [M + H]⁺ = 505.

Step 4: To methyl (2*S*)-3-(benzyloxycarbonylamino)-2-[[2-(4-*tert*-butylphenyl)-4-methyl-pyrimidine-5-carbonyl]amino]propanoate (423 mg, 0.84 mmol) in THF (4 mL) was added lithium hydroxide (1.0 M aqueous solution, 1.0 mL, 1.0 mmol). The mixture was stirred at 20 °C for 2 h. The solvent was removed, the residue dissolved in water and acidified to ca. pH 2 with 1M aqueous HCl. The mixture was extracted with ethyl acetate twice and the combined organic layers were washed with brine, dried over Na₂SO₄ and the solvent removed to give (2*S*)-3-(benzyloxycarbonylamino)-2-[[2-(4-*tert-*butylphenyl)-4-methyl-pyrimidine-5-carbonyl]amino]propanoic acid (416 mg, 101%) as a white foam. LCMS (ESI): [M + H]⁺ = 491.

Step 5: To a mixture of Compound 101-G (592 mg, 0.83 mmol) and (2*S*)-3-(benzyloxycarbonylamino)-2-[[2-(4-*tert*-butylphenyl)-4-methyl-pyrimidine-5-carbonyl]amino]propanoic acid (407 mg, 0.83 mmol) in THF (5.0 mL) under nitrogen was added sodium hydrogencarbonate (279 mg, 3.32 mmol) followed by DEPBT (745 mg, 2.49 mmol). The mixture was stirred at 60 °C for 18 h. The mixture was cooled to ambient temperature, diluted with ethyl acetate and the organic layer was washed successively with saturated aqueous sodium hydrogen carbonate and brine then dried over Na₂SO₄ and the solvent was removed. The residue was purified by silica gel chromatography (solvent gradient: 50%-100% ethyl acetate in cyclohexane) to give compound 491-1 (422 mg, 43%) as a white glassy solid. LCMS (ESI): [M + H]⁺ = 1186.

Starting from compound 494-1 and (*N*-*tert*-butoxycarbonyl)glycine, the typical amide coupling (HATU/DIEA), hydrolysis and global Boc removal (TFA/HFIP) procedures (as described in Examples 5 and 7) were followed to afford Compound **491** (trifluoroacetic acid salt) as a white solid. LCMS (ESI): t_{R} = 2.50 min, [M + H]⁺ = 933.7. ¹H NMR (400 MHz, DMSO-*d*₆) δ 9.18 (d, *J* = 8.2 Hz, 1H), 8.99 (d, *J* = 8.2 Hz, 1H), 8.85 (s, 1H), 8.76-8.70 (m, 1H), 8.66-8.60 (m, 1H), 8.40-8.31 (m, 3H), 8.10-7.84 (m, 9H), 7.58 (d, *J* = 8.9 Hz, 2H), 7.28-7.03 (m, 4H), 6.73 (s, 2H), 6.36 (s, 1H), 5.10-5.02 (m, 1H), 4.79-4.67 (m, 2H), 4.27-4.07 (m, 6H), 3.77-2.97 (m, 11H), 2.86 (s, 2H), 2.63 (s, 3H), 1.33 (s, 9H), 1.22 (d, *J* = 7.4 Hz, 3H).

### Example 292: Synthesis of Compound 492

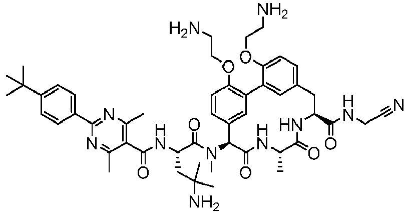

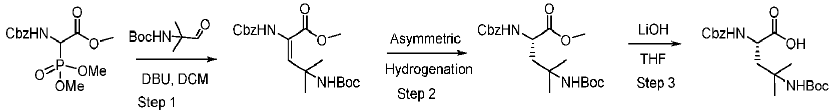

Step 1: To a mixture of methyl 2-(benzyloxycarbonylamino)-2-dimethoxyphosphoryl-acetate (994 mg, 3.0 mmol) and *tert*-butyl *N*-(1,1-dimethyl-2-oxo-ethyl)carbamate (562 mg, 3.0 mmol) in DCM (5.0 mL) was added 1,8-diazabicyclo[5.4.0]undecane (0.45 mL, 3 mmol). The mixture was stirred at 20 °C for 18 h. The reaction mixture was diluted with DCM, washed successively with 1M aqueous HCl, and brine, then dried over Na₂SO₄ and the solvent was removed. The residue was purified by silica gel chromatography (eluting with 30% ethyl acetate/cyclohexane) to give methyl (*E*)-2-(benzyloxycarbonylamino)-4-(*tert*-butoxycarbonylamino)-4-methyl-pent-2-enoate (746 mg, 63%) as a colorless oil. LCMS (ESI): [M + Na]⁺ = 415.

Step 2: A solution of methyl (*E*)-2-(benzyloxycarbonylamino)-4-(*tert*-butoxycarbonylamino)-4-methyl-pent-2-enoate (740 mg, 1.89 mmol) in methanol (10 mL) was added to 1,2-bis[(25,5S)-2,5-diethylphosphoplano]benzene(1,5-cyclooctadiene)rhodium(I) trifluoromethanesulfonate (136 mg, 0.19 mmol) in a glass lined steel bomb. The bomb was flushed with nitrogen four times and then flushed with hydrogen, the pressure increased to 4.5 atm and the mixture was stirred for 18 h. The pressure was released, the mixture was removed from the bomb and the solvent removed. The residue was purified by silica gel chromatography (eluting with 20% ethyl acetate/cyclohexane) to give methyl (2S)-2-(benzyloxycarbonylamino)-4-(*tert*-butoxycarbonylamino)-4-methyl-pentanoate (425 mg, 57%) as a white solid. LCMS (ESI): [M + H]⁺ = 395.

Step 3: To a solution of methyl (2S)-2-(benzyloxycarbonylamino)-4-(*tert-*butoxycarbonylamino)-4-methyl-pentanoate (412 mg, 1.04 mmol) in THF (5 mL) was added a solution of LiOH (1.0M, 1.1 mL, 1.1 mmol). The mixture was stirred at 20 °C for 5 h. The solvent was removed and the residue dissolved in water, acidified with 1.0 M aqueous HCl, and extracted twice with ethyl acetate. The combined organic extracts were washed with brine, dried over Na₂SO₄ and the solvent removed to give (2*S*)-2-(benzyloxycarbonylamino)-4-(tert-butoxycarbonylamino)-4-methyl-pentanoic acid (390 mg, 98%) as a white solid. LCMS (ESI): [M - H]⁺ = 379.

Compound 101-G was coupled to (2*S*)-2-(benzyloxycarbonylamino)-4-(*tert-*butoxycarbonylamino)-4-methyl-pentanoic acid and hydrogenated as described in Example E. The resulting compound was coupled with 2-(4-tert-butylphenyl)-4,6-dimethyl-pyrimidine-5-carboxylic acid

, followed by ester hydrolysis, coupling with aminoacetonitrile hydrochloride and global deprotection using the procedures described in Example G to afford Compound **492** (formic acid salt) as a white solid. LCMS (ESI): t_{R} = 2.78 min, [M + H]⁺ = 932.5. ¹H NMR (400 MHz, DMSO-*d*₆) δ 9.25 (d, *J* = 7.6 Hz, 1H), 8.96 (d, *J* = 7.9 Hz, 1H), 8.74-8.69 (m, 1H), 8.39-8.29 (m, 3H), 7.56 (d, *J* = 8.3 Hz, 2H), 7.30-7.06 (m, 5H), 6.78-6.71 (m, 2H), 6.46 (s, 1H), 5.11-5.03 (m, 1H), 4.79-4.64 (m, 2H), 4.22-4.05 (m, 7H), 3.25-3.01 (m, 6H), 2.95 (s, 3H), 2.25-2.17 (m, 1H), 2.00-1.91 (m, 1H), 1.33 (s, 15H), 1.19 (d, *J* = 6.9 Hz, 3H).

### Example 293: Synthesis of Compound 493

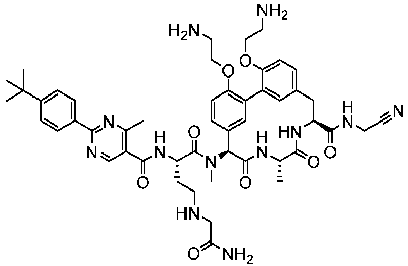

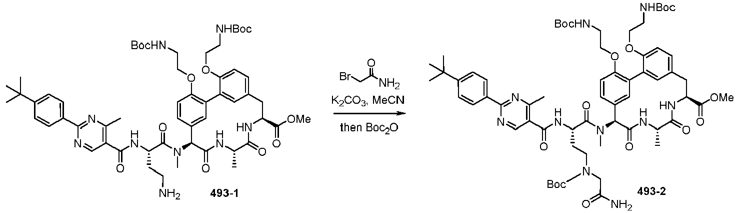

Compound 493-1 was prepared as a white solid (373 mg) following procedures analogous to those described in Example 291, using [(1*S*)-3-(*tert*-butoxycarbonylamino)-1-methoxycarbonylpropyl]ammonium chloride in place of [(1*S*)-1-[(*tert*-butoxycarbonylamino)methyl]-2-methoxy-2-oxoethyl]ammonium chloride. LCMS (ESI): [M + H]⁺ = 1066.

To Compound 493-1 (373 mg, 0.35 mmol) in acetonitrile (3.0 mL) was added potassium carbonate (58 mg, 0.42 mmol) and 2-bromoacetamide (46 mg, 0.33 mmol). DMF (0.5 mL) added to aid solubility. The mixture was stirred at 20 °C for 18 h then potassium carbonate (58 mg, 0.42 mmol) and di-tert-butyl dicarbonate (92 mg, 0.42 mmol) were added. The reaction was stirred at 20 °C for 3 h and the resulting mixture was then partitioned between ethyl acetate and water and the phases separated. The organic layer was washed with brine, dried over Na₂SO₄ and the solvent removed. The residue was purified by silica gel chromatography (eluting with 7% MeOH/DCM) to give Compound 493-2 (295 mg, 69%) as a glass. LCMS (ESI): [M + H]⁺ = 1247.

Starting from Compound 493-2, typical hydrolysis, amide coupling (HATU/DIEA) and global Boc removal (TFA/HFIP) procedures (as described in Example G) were followed to afford Compound 493 (formic acid salt) as a white solid. LCMS (ESI): t_{R} = 2.51 min, [M + H]⁺ = 947.5. ¹H NMR (400 MHz, DMSO-*d*₆) δ 9.15 (d, *J* = 7.2 Hz, 1H), 8.97 (d, *J* = 7.8 Hz, 2H), 8.82 (s, 2H), 8.73-8.67 (m, 2H), 8.44-8.32 (m, 3H), 8.02-7.66 (m, 4H), 7.63-7.55 (m, 3H), 7.30-7.00 (m, 5H), 6.73 (s, 2H), 6.38 (s, 1H), 5.05-4.93 (m, 1H), 4.80-4.67 (m, 2H), 4.30-4.02 (m, 6H), 3.21-2.94 (m, 8H), 2.84 (s, 3H), 2.65 (s, 3H), 2.54 (s, 2H), 1.34 (s, 9H), 1.22 (d, *J* = 7.0 Hz, 3H).

### Example 294: Synthesis of Compound 494

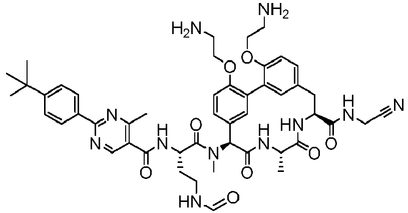

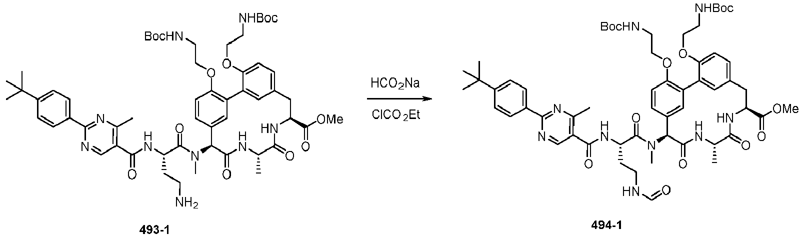

Step 1: A mixture of Compound 493-1 (340 mg, 0.32 mmol) and sodium formate (26 mg, 0.38 mmol) in ethyl formate (5 mL) was stirred at 50 °C for 5 h. The mixture was partitioned between ethyl acetate and water and the phases separated. The organic layer was washed with brine, dried over Na₂SO₄ and the solvent removed. The residue was purified via silica gel chromatography (solvent gradient: 0-10% MeOH/DCM) to give Compound 494-1 (273 mg, 78%) as a white solid. LCMS (ESI): [M + H]⁺ = 1095.

Starting from Compound 494-1, typical hydrolysis, amide coupling (HATU/DIEA) and global Boc removal (TFA/HFIP) procedures (as described in Examples E and G) were followed to afford Compound 494 (formic acid salt) as a white solid. LCMS (ESI): t_{R} = 2.94 min, [M + H]⁺ = 918.7. ¹H NMR (400 MHz, DMSO-*d*₆) δ 9.11-9.00 (m, 2H), 8.84-8.77 (m, 2H), 8.39-7.98 (m, 8H), 7.60-7.55 (m, 2H), 7.23-7.02 (m, 4H), 6.76-6.66 (m, 2H), 6.37 (s, 1H), 4.89-4.67 (m, 4H), 4.17 (d, *J* = 6.9 Hz, 2H), 4.10-4.01 (m, 4H), 3.30-3.19 (m, 2H), 3.17 (s, 1H), 3.05-2.87 (m, 6H), 2.83 (s, 2H), 2.65 (s, 3H), 2.03-1.74 (m, 2H), 1.37 (s, 9H), 1.19 (d, *J* = 8.4 Hz, 3H).

### Example 295: Synthesis of Compound 495

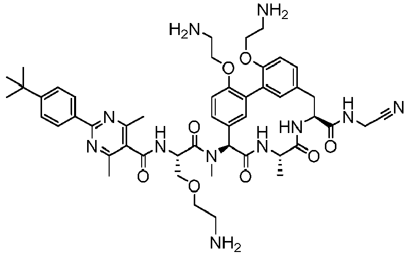

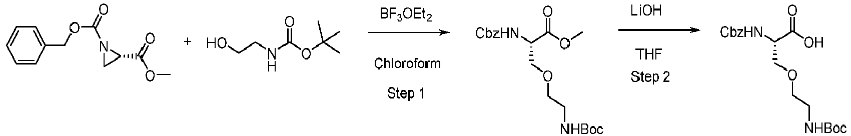

Step 1: To a solution of methyl (*S*)-*N*-*Z*-aziridine-2-carboxylate (1.26 mL, 6.38 mmol) and *tert-*butyl *N*-(2-hydroxyoxyethyl)carbamate (3.45 mL, 22.32 mmol) in chloroform (45 mL) at 0 °C, under argon, was added, dropwise, boron trifluoride diethyl etherate (0.81 mL, 6.38 mmol). The reaction mixture was allowed to reach ambient temperature over 30 min. The reaction was quenched with saturated aqueous NaHCO₃ (70 mL) and extracted with DCM. The organic extracts were dried over MgSO₄ and the solvent removed. The crude residue was purified by silica gel chromatography (eluting with 50% ethyl acetate/cyclohexane) to give methyl (2*S*)-2-(benzyloxycarbonylamino)-3-[2-(*tert-*butoxycarbonylamino)ethoxy]propanoate (1.30 g, 3.28 mmol) as a colorless oil. LCMS (ESI): [M + Na]⁺ = 419.

Step 2: To a solution of methyl (2*S*)-2-(benzyloxycarbonylamino)-3-[2-(*tert-*butoxycarbonylamino)ethoxy]propanoate (1.3 g, 3.28 mmol) in THF (30 mL) was added lithium hydroxide (1.0 M, 6.56 mL, 6.56 mmol) and the reaction mixture was stirred at 50 °C for 30 min. The reaction was quenched with 1N aqueous HCl (8.0 mL) followed by water (50 mL) and the resulting mixture was extracted with DCM. The combined organic extracts were dried over MgSO₄ and the solvent removed to give (2*S*)-2-(benzyloxycarbonylamino)-3-[2-(*tert*-butoxycarbonylamino)ethoxy]propanoic acid (1.20 g, 3.14 mmol) as a colorless oil. LCMS (ESI): [M + Na]⁺ = 405.

Compound 101-G was coupled to (2*S*)-2-(benzyloxycarbonylamino)-3-[2-(*tert-*butoxycarbonylamino)ethoxy]propanoic and hydrogenated as described in Example E. The resulting compound was coupled with 2-(4-tert-butylphenyl)-4,6-dimethyl-pyrimidine-5-carboxylic acid, followed by ester hydrolysis, coupling with aminoacetonitrile hydrochloride and global deprotection using the procedures described in Example G to afford Compound **495** (formic acid salt) as a white solid. LCMS (ESI): t_{R} = 2.48 min, [M + H]⁺ = 934.4. ¹H NMR (400 MHz, DMSO-*d*₆) δ 9.21-9.11 (m, 1H), 8.96 (d, *J* = 8.0 Hz, 1H), 8.72 (t, *J* = 5.4 Hz, 1H), 8.41-8.29 (m, 3H), 7.90-7.74 (m, 6H), 7.55 (d, *J* = 8.4 Hz, 2H), 7.28-7.06 (m, 4H), 6.73-6.69 (m, 1H), 6.43 (s, 1H), 5.20-5.12 (m, 1H), 4.78-4.56 (m, 1H), 4.28-4.07 (m, 6H), 3.86-3.78 (m, 2H), 3.74-3.61 (m, 6H), 3.25-2.96 (m, 9H), 2.91 (s, 3H), 2.65 (m, 2H), 2.46 (d, *J* = 5.2 Hz, 1H), 1.34 (s, 9H), 1.24-1.18 (m, 3H).

### Example 296: Synthesis of Compound 496

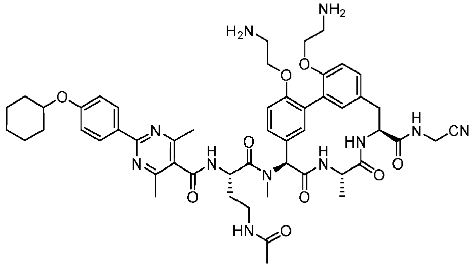

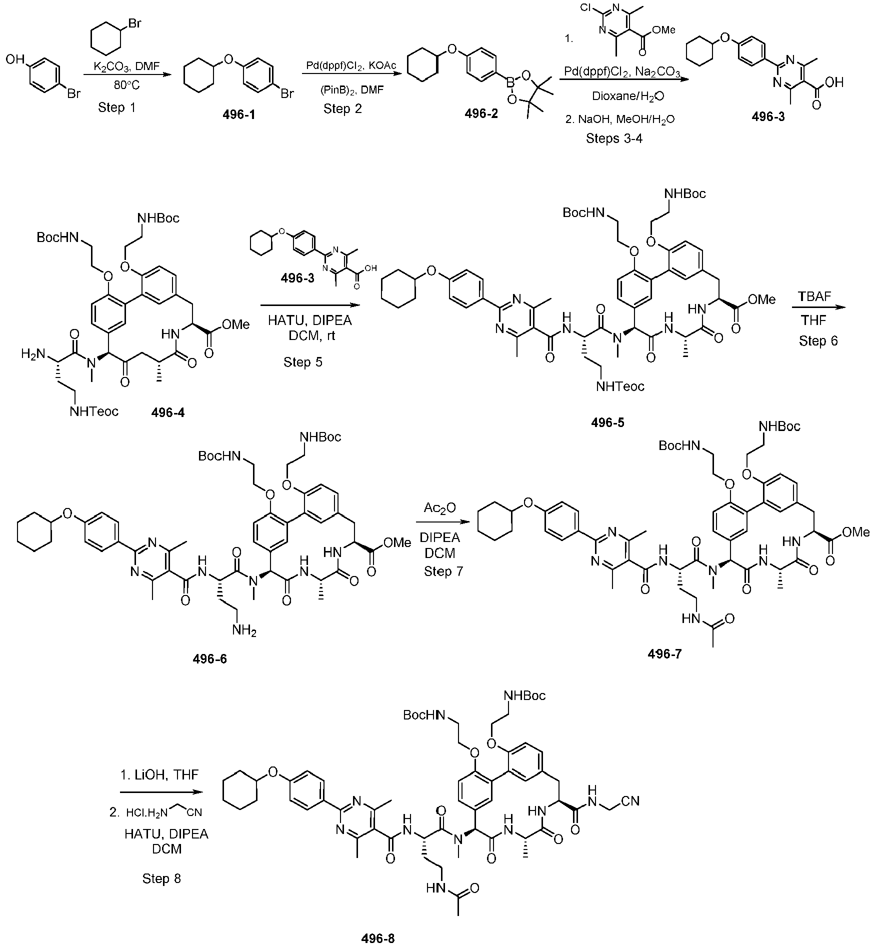

Step 1: To a solution of 4-bromophenol (1.0 g, 5.78 mmol) in DMF (10 mL) was added bromocyclohexane (1.8 g, 11.0 mmol) and K₂CO₃ (2.4 g, 17.4 mmol) and the reaction was stirred at 80 °C for 16 h. The reaction mixture was poured into water (20 mL), which was extracted with EtOAc (50 mL x 3). The combined organic layers were washed with brine (100 mL x 2), dried over Na₂SO₄ and concentrated. The residue was purified by silica gel chromatography, eluting with petroleum ether, to give compound 496-1 (170.0 mg, 11%) as a colorless oil.

Step 2: To a solution of compound 496-1 (170.0 mg, 0.67 mmol) in DMF (5 mL) was added bis(pinacolato)diboron (254 mg, 1.00 mmol), Pd(dppf)Cl₂ (24.4 mg, 0.03 mmol) and potassium acetate (196 mg, 2.00 mmol) and the resulting mixture was stirred at 80 °C under nitrogen for 3 h. The reaction was poured into water (20 mL), which was extracted with EtOAc (20 mL x 3). The combined organic layers were washed with brine (50 mL x 2), dried over Na₂SO₄ and concentrated. The residue was purified by silica gel chromatography (eluent: 8% EtOAc in petroleum ether) to give compound 496-2 (140 mg, 70% yield) as a yellow oil.

Step 3: A mixture of compound 496-2 (200 mg, 0.66 mmol) in 1,4-dioxane (5 mL) and H₂O (1 mL) was added methyl 2-chloro-4,6-dimethylpyrimidine-5-carboxylate (described in Example 53) (199 mg, 0.99 mmol) and K₂CO₃ (274 mg, 2.0 mmol) and Pd(dppf)Cl₂ (48.4 mg, 0.07 mmol) and the resulting mixture was stirred at 110 °C under nitrogen for 16 h. After filtration, the filtrate was diluted with H₂O (20 mL), which was extracted with EtOAc (20 mL x 2). The combined organic layers were washed with water and brine (30 mL each), dried over Na₂SO₄ and concentrated. The residue was purified by prep-TLC (eluent: 10% EtOAC in petroleum ether) to give methyl 2-(4-(cyclohexyloxy)phenyl)-4,6-dimethylpyrimidine-5-carboxylate (110 mg, 59% yield) as a white solid. LCMS (ESI): [M+H]⁺ = 341.0.

Step 4: To a solution of methyl 2-(4-(cyclohexyloxy)phenyl)-4,6-dimethylpyrimidine-5-carboxylate (110 mg, 0.32 mmol) in methanol (10 mL) was added 1.0 M aqueous NaOH (1.62 mL, 1.62 mmol) and the mixture was stirred at 80 °C for 4 h. The reaction mixture was adjusted to pH=4 using saturated aqueous KHSO₄, which was added with EtOAc (40 mL). The organic layer was washed with brine (30 mL x 2), dried over Na₂SO₄ and concentrated to afford compound 496-3 (100 mg, 95% yield) as a white solid, which was used directly in the next step.

Compound 496-4 was prepared from Compound 101-G and (*S*)-2-(((benzyloxy)carbonyl)amino)-4-(((2-(trimethylsilyl)ethoxy)carbonyl)amino)butanoic acid using conditions analogous to those described in Example E. LCMS (ESI): [M + H]⁺ = 958.

Step 5: Example E was applied to Compound 496-4 (3.37 g, 3.52 mmol) and Compound 496-3 (1.26 g, 3.87 mmol) to give Compound 496-5 (3.78 g, 85%) as a white solid. LCMS (ESI): [M + H]⁺ = 1266.

Step 6: To a solution of Compound 496-5 (3.05 g, 2.41 mmol) in THF (15 mL) was added a solution of tetrabutylammonium fluoride (1.0 M in THF, 3.6 mL, 3.6 mmol). The mixture was stirred at 50 °C for 18 h. The mixture was cooled to room temperature and the solvent removed. The residue was dissolved in ethyl acetate, washed with brine, dried over Na₂SO₄ and the solvent removed to give Compound 496-6 (3.20 g, 118%) as an off white solid. The residue used directly without further purification. LCMS (ESI): [M + H]⁺ = 1122.

Step 7: To Compound 496-6 (280 mg, 0.25 mmol) in DCM (3 mL) at 0 °C was added DIPEA (0.07 mL, 0.38 mmol) followed by acetic anhydride (28 mg, 0.28 mmol). The mixture was stirred at 0 °C for 2 h. The mixture was partitioned between DCM and water and the phases separated. The organic layer was washed with brine, dried over Na₂SO₄ and the solvent was removed. The residue was purified on silica gel column (8% MeOH/DCM) to give Compound 496-7 (195 mg, 67%) as a white solid. LCMS (ESI): [M + H]⁺ = 1164.

Steps 8-9: Starting from Compound 496-7, typical hydrolysis, amide coupling (HATU/DIEA) and global Boc removal (TFA/HFIP) procedures (Example G) were followed to afford Compound 496 (trifluoroacetic acid salt) as a white solid. LCMS (ESI): t_{R} = 3.18 min, [M + H]⁺ = 988.4. ¹H NMR (400 MHz, DMSO-*d*₆) δ 9.05-8.96 (m, 2H), 8.76 (t, *J* = 5.8 Hz, 1H), 8.37-8.28 (m, 5H), 7.94 (t, *J* = 5.8 Hz, 1H), 7.28-6.93 (m, 7H), 6.76-6.68 (m, 2H), 6.39 (s, 1H), 4.88-4.81 (m, 1H), 4.78-4.67 (m, 2H), 4.49-4.42 (m, 1H), 4.19-4.15 (m, 2H), 4.07-3.94 (m, 4H), 3.23-3.14 (m, 3H), 3.03-2.82 (m, 4H), 2.84 (s, 3H), 2.44 (s, 1H), 1.99-1.92 (m, 2H), 1.79 (s, 3H), 1.77-1.69 (m, 3H), 1.58-1.37 (m, 4H), 1.33-1.23 (m, 1H), 1.19 (d, *J* = 7.2 Hz, 3H).

### Example 297: Synthesis of Compound 497

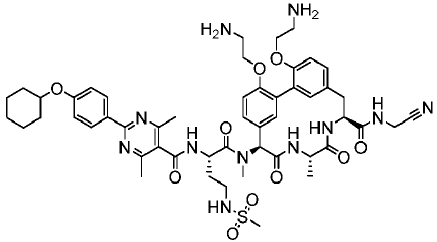

Compound **497** (trifluoroacetic acid salt) was prepared as a white solid utilizing methods analogous to those described in Example 296 using methanesulfonyl chloride in place of acetic anhydride in Step 7. LCMS (ESI): t_{R} = 3.30 min, [M + H]⁺ = 1024.4. ¹H NMR (400 MHz, DMSO-*d*₆) δ 9.01-8.86 (m, 2H), 8.67 (t, *J =* 7.4 Hz, 1H), 8.33-8.28 (m, 3H), 7.30-7.02 (m, 9H), 6.78-6.72 (m, 2H), 6.42 (s, 1H), 4.97-4.91 (m, 1H), 4.77-4.69 (m, 2H), 4.48-4.40 (m, 1H), 4.28-4.03 (m, 7H), 3.26-2.98 (m, 8H), 2.90 (s, 3H), 2.88 (s, 3H), 2.00-1.91 (m, 3H), 1.86-1.70 (m, 3H), 1.59-1.51 (m, 1H), 1.50-1.35 (m, 4H), 1.33-1.24 (m, 1H), 1.24 (d, *J*= 6.6 Hz, 3H).

### Example 298: Synthesis of Compound 498

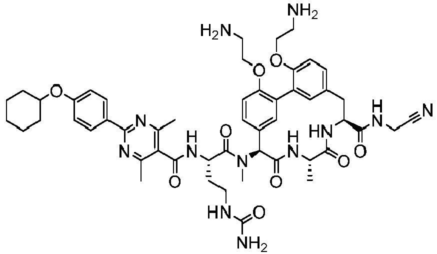

Compound 498 (formic acid salt) was prepared as a white solid utilizing the methods in Example 296 using trimethyl isocyanate in place of acetic anhydride in Step 7. LCMS (ESI): t_{R} = 3.06 min, [M + H]⁺ = 989.4. ¹H NMR (400 MHz, DMSO-*d*₆) δ 9.06-8.96 (m, 2H), 8.78 (t, *J* = 5.5 Hz, 1H), 8.33-8.29 (m, 5H), 7.21-7.18 (m, 1H), 7.16-7.10 (m, 2H), 7.07-7.02 (m, 4H), 6.76-6.68 (m, 2H), 6.39 (s, 1H), 6.16-6.09 (m, 1H), 5.51-5.39 (m, 2H), 4.89-4.81 (m, 1H), 4.78-4.67 (m, 2H), 4.48-4.41 (m, 1H), 4.22-4.14 (m, 2H), 4.08-3.95 (m, 4H), 3.21-2.85 (m, 7H), 2.84 (s, 3H), 2.45 (s, 1H), 2.00-1.92 (m, 2H), 1.92-1.84 (m, 1H), 1.76-1.69 (m, 2H), 1.58-1.51 (m, 1H), 1.48-1.37 (m, 3H), 1.33-1.24 (m, 1H), 1.24 (d, *J* = 6.4 Hz, 3H).

### Example 299: Synthesis of Compound 499

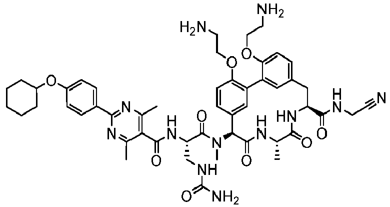

Compound **499** (trifluoroacetic acid salt) was prepared as a white solid utilizing the methods in Example 296 using trimethylsilyl isocyanate in place of acetic anhydride in Step 7. LCMS (ESI): t_{R} = 3.05 min, [M + H]⁺ = 975.3. ¹H NMR (400 MHz, DMSO-*d*₆) δ 9.01-8.95 (m, 2H), 8.70 (t, *J* = 5.3 Hz, 1H), 8.34-8.26 (m, 3H), 7.88 (br s, 6H), 7.28-7.03 (m, 6H), 6.77-6.70 (m, 2H), 6.41 (s, 1H), 6.14-6.07 (m, 1H), 5.78-5.64 (m, 2H), 5.01-4.94 (m, 1H), 4.79-4.66 (m, 2H), 4.49-4.41 (m, 1H), 4.28-4.08 (m, 7H), 3.51-3.43 (m, 2H), 3.24-3.00 (m, 8H), 2.94 (s, 2H), 2.47-2.44 (m, 1H), 2.00-1.92 (m, 2H), 1.77-1.69 (m, 2H), 1.59-1.24 (m, 7H), 1.21 (d, *J* = 7.0 Hz, 3H).

### Example 300: Synthesis of Compound 500

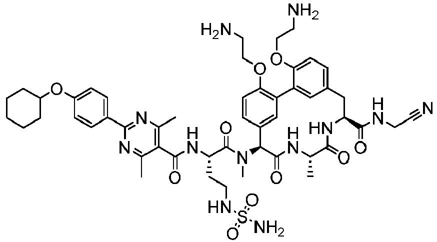

Compound 500 (trifluoroacetic acid salt) was prepared as a white solid utilizing the methods in Example 296 using *tert*-butyl (chlorosulfonyl)carbamate (formed by mixing equimolar amounts of chlorosulfonyl isocyanate and *tert*-butanol in DCM) in place of acetic anhydride in Step 7. LCMS (ESI): t_{R} = 3.22 min, [M + H]⁺ = 1025.4. ¹H NMR (400 MHz, DMSO-*d*₆) δ 9.01-8.95 (m, 2H), 8.72-8.67 (m, 1H), 8.34-8.25 (m, 3H), 7.92-7.81 (br s, 6H), 7.26-7.03 (m, 7H), 6.75-6.68 (m, 3H), 6.53 (s, 2H), 6.45 (s, 1H), 4.97-4.90 (m, 1H), 4.79-4.69 (m, 2H), 4.48-4.42 (m, 1H), 4.25-4.10 (m, 6H), 3.25-2.97 (m, 9H), 2.89 (s, 3H), 2.01-1.95 (m, 3H), 1.83-1.69 (m, 3H), 1.57-1.25 (m, 6H), 1.21 (d, *J* = 6.8 Hz, 3H).

### Example 301: Synthesis of Compound 501

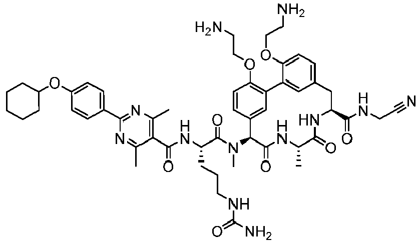

Compound **501** (formic acid salt) was prepared as a white solid utilizing the methods in Example 296 using trimethylsilyl isocyanate in place of acetic anhydride in Step 7. LCMS (ESI): t_{R} = 3.06 min, [M + H]⁺ = 1003.5. ¹H NMR (400 MHz, DMSO-*d*₆) δ 9.11-8.99 (m, 2H), 8.80 (t, *J* = 5.6 Hz, 1H), 8.35-8.28 (m, 4H), 7.19-7.02 (m, 7H), 6.76-6.70 (m, 2H), 6.41 (s, 1H), 6.05-5.96 (m, 1H), 5.38 (s, 2H), 4.86-4.68 (m, 4H), 4.48-4.42 (m, 2H), 4.21-4.13 (m, 2H), 4.08-4.00 (m, 6H), 3.22-3.07 (m, 2H), 3.05-2.95 (m, 3H), 2.86 (s, 6H), 2.68-2.63 (m, 1H), 2.48 (s, 4H), 2.01-1.93 (m, 3H), 1.80-1.70 (m, 3H), 1.63-1.24 (m, 9H), 1.20 (d, *J* = 6.8 Hz, 3H).

### Example 302: Synthesis of Compound 502

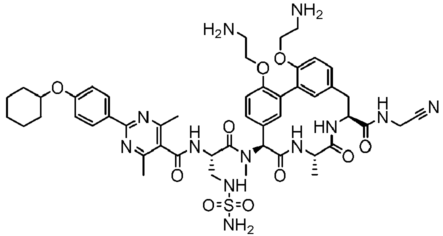

Compound **502** (trifluoroacetic acid salt) was prepared as a white solid utilizing methods analogous to those described in Example 296 using tert-butyl (chlorosulfonyl)carbamate (formed by mixing equimolar amounts of chlorosulfonyl isocyanate and tert-butanol in DCM) in place of acetic anhydride in Step 7. LCMS (ESI): t_{R} = 3.09 min, [M + H]⁺ = 1001.5. ¹H NMR (400 MHz, DMSO-*d*₆) δ 9.10-8.99 (m, 2H), 8.80-8.74 (m, 1H), 8.40 (d, *J* = 8.8 Hz, 1H), 8.35-8.29 (m, 4H), 7.24-7.03 (m, 7H), 6.74 (dd, *J* = 1.6, 13.2 Hz, 2H), 6.65 (s, 3H), 6.38 (s, 1H), 5.13 (dd, *J* = 7.6, 13.6 Hz, 1H), 4.77-4.70 (m, 2H), 4.48-4.42 (m, 1H), 4.21-4.15 (m, 2H), 4.14-4.01 (m, 6H), 3.18 (d, *J* = 16.0 Hz, 2H), 3.06-2.97 (m, 2H) 2.95-2.91 (m, 6H), 2.48 (s, 4H), 2.46-2.43 (m, 1H), 1.97 (d, *J* = 6.4 Hz, 3H), 1.78-1.73 (m, 3H), 1.57-1.23 (m, 7H), 1.20 (d, *J* = 6.8 Hz, 3H).

### Example 303: Synthesis of Compound 503

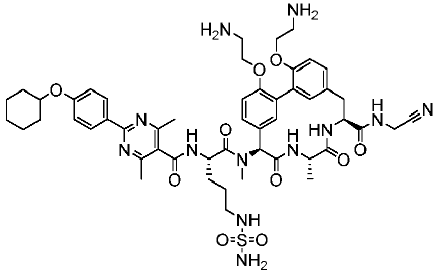

Compound **503** (trifluoroacetic acid salt) was prepared as a white solid utilizing the methods in Example 296 using *tert*-butyl (chlorosulfonyl)carbamate (formed by mixing equimolar amounts of chlorosulfonyl isocyanate and *tert*-butanol in DCM) in place of acetic anhydride in Step 7. LCMS (ESI): t_{R} = 3.15 min, [M + H]⁺ = 1039.6. ¹H NMR (400 MHz, DMSO-*d*₆) δ 8.97 (d, *J* = 6.8 Hz, 2H), 8.70 (t, *J* = 5.6 Hz, 1H), 8.34-8.25 (m, 3H), 7.94-7.88 (m, 6H), 7.25-7.03 (m, 7H), 6.73 (t, *J* = 2.4 Hz, 2H), 6.53-6.44 (m, 3H), 4.82-4.69 (m, 3H), 4.48-4.42 (m, 1H), 4.25-4.11 (m, 6H), 3.25-2.88 (m, 12H), 2.49-2.43 (m, 4H), 1.97-1.26 (m, 15H), 1.22 (d, *J* = 6.8 Hz, 3H).

### Example 304: Synthesis of Compound 504

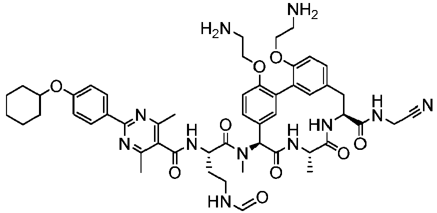

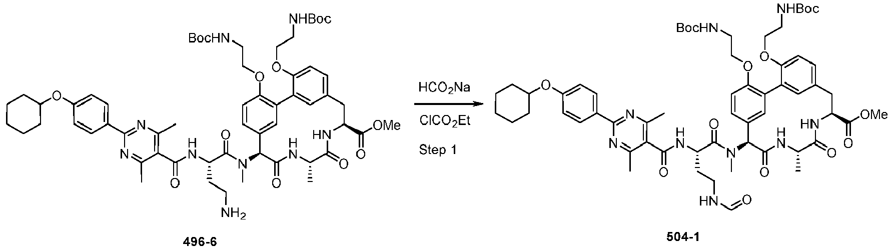

Step 1: A mixture of Compound 496-6 (described in Example 296) (280 mg, 0.25 mmol) and sodium formate (20.4 mg, 0.38 mmol) in ethyl formate (4 mL) was stirred at 50 °C for 18 h. The mixture was partitioned between ethyl acetate and water and the phases separated. The organic layer was washed with brine, dried over Na₂SO₄ and the solvent removed. The residue was purified by silica gel chromatography (eluting with 7% MeOH/DCM) to give Compound 504-1 (168 mg, 58%) as a white solid. LCMS (ESI): [M + H]⁺ = 1150.

Starting from Compound 504-1, typical hydrolysis, amide coupling (HATU/DIEA) and global Boc removal (TFA/HFIP) procedures (as described in Examples 5 and 7) were followed to afford Compound 504 (formic acid salt) as a white solid. LCMS (ESI): t_{R} = 3.70 min, [M + H]⁺ = 974.3. ¹H NMR (400 MHz, DMSO-*d*₆) δ 9.05-8.97 (m, 2H), 8.72-8.67 (m, 1H), 8.38-8.00 (m, 6H), 7.25-6.99 (m, 6H), 6.80-6.67 (m, 2H), 6.42 (s, 1H), 4.89-4.82 (m, 1H), 4.80-4.66 (m, 3H), 4.49-4.40 (m, 2H), 4.17 (d, *J* = 5.7 Hz, 3H), 4.13-4.00 (m, 6H), 3.30-3.19 (m, 4H), 3.05-2.90 (m, 6H), 2.85 (s, 3H), 2.68-2.63 (m, 1H), 2.00-1.87 (m, 3H), 1.82 -1.68 (m, 3H), 1.59-1.20 (m, 7H), 1.19 (d, *J* = 5.7 Hz, 3H).

### Example 305: Synthesis of Compound 505

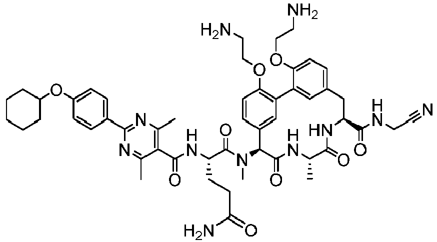

Compound 505 (formic acid salt) was prepared as a white solid from Compound 101-G and Compound **496-3** (described in Example 296), utilizing methods analogous to those described in Example 7. LCMS (ESI): t_{R} = 3.15 min, [M + H]⁺ = 974.4. ¹H NMR (400 MHz, DMSO-*d*₆) δ 9.06-8.87 (m, 2H), 8.74-8.63 (m, 1H), 8.37-8.28 (m, 2H), 8.25 (s, 1H), 7.45-6.91 (m, 6H), 6.88-6.66 (m, 2H), 6.42 (s, 1H), 4.96-4.58 (m, 3H), 4.51-4.40 (m, 1H), 4.17 (d, *J* = 5.8 Hz, 2H), 4.12-3.99 (m, 4H), 3.25-3.09 (m, 1H), 3.05-2.85 (m, 6H), 2.34-2.22 (m, 2H), 2.09-1.89 (m, 2H), 1.84-1.67 (m, 2H), 1.60-1.36 (m, 4H), 1.35-1.17 (m, 4H).

### Example 306: Synthesis of Compound 506

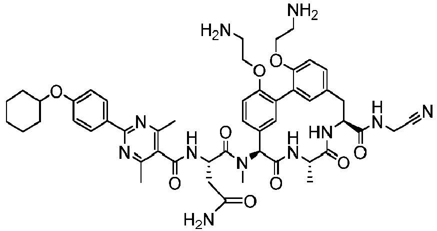

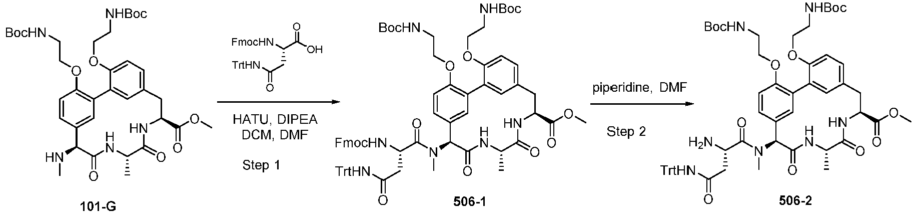

Step 1: Following a HATU Coupling with Compound 101-G (357 mg, 0.50 mmol) and Fmoc-L-Asn(Trt)-OH (448 mg, 0.75 mmol) afforded Compound 506-1 (561 mg, 87%). LCMS (ESI): [M + H]⁺ = 1292.

Step 2: To a solution of Compound 506-1 (503 mg, 0.389 mmol) in DMF (3.0 mL) was added piperidine (0.385 mL, 3.89 mmol). The mixture was stirred at ambient temperature for 18 h. The mixture was partitioned between ethyl acetate and water and the organic layer was washed with brine, dried over Na₂SO₄ and the solvent removed. The crude residue was purified via silica gel chromatography (solvent gradient: 0-10% MeOH/DCM) to give Compound 506-2 (300 mg, 72%) as an off-white solid. LCMS (ESI): [M + H]⁺ = 1070.

Compound 506 (trifluoroacetic acid salt) was obtained as a white solid from Compound 506-2, following typical amide coupling (HATU/DIEA), hydrolysis, amide coupling (HATU/DIEA) and global Boc/Trityl removal (TFA/HFIP) procedures (as described in Example G). LCMS (ESI): t_{R} = 3.10 min, [M + H]⁺ = 933.5. ¹H NMR (400 MHz, DMSO-*d*₆) δ 9.03 (d, *J* = 7.7 Hz, 1H), 8.50 (d, *J* = 8.8 Hz, 1H), 8.34-8.27 (m, 2H), 7.84-7.76 (m, 1H), 7.40 (s, 1H), 7.10 (s, 1H), 7.08-7.01 (m, 3H), 6.99-6.90 (m, 2H), 6.80-6.73 (m, 2H), 6.31 (s, 1H), 5.31-5.23 (m, 1H), 4.68-4.58 (m, 2H), 4.50-4.41 (m, 1H), 4.08-3.96 (m, 4H), 3.10-3.00 (m, 1H), 2.95-2.83 (m, 5H), 2.68-2.59 (m, 2H), 2.42-2.38 (m, 1H), 2.45 (s, 1H), 2.01-1.90 (m, 2H), 1.78-1.69 (m, 2H), 1.59-1.22 (m, 6H), 1.17 (d, *J* = 6.5 Hz, 3H).

### Example 307: Synthesis of Compound 507

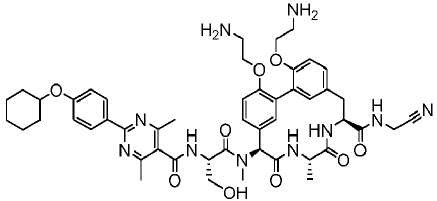

Compound 507 was prepared as a white solid utilizing methods analogous to those described in Example R using (*S*)-2-(((benzyloxy)carbonyl)amino)-3-((*tert*-butyldimethylsilyl)oxy)propanoic acid, 2-[4-(cyclohexoxy)phenyl]-4,6-dimethyl-pyrimidine-5-carboxylic acid and Compound 101-G and purified by chiral SFC. LCMS (ESI): t_{R} = 3.06 min, [M + H]⁺ = 960.3. ¹H NMR (400 MHz, DMSO-*d*₆) δ 9.08-8.93 (m, 2H), 8.76-8.66 (d, *J* = 7.1 Hz, 1H), 8.36-8.25 (m, 2H), 7.89 (br s, 6H), 7.39-7.01 (m, 7H), 6.78-6.68 (m, 2H), 6.45 (s, 1H), 5.03-4.93 (m, 1H), 4.80-4.67 (m, 2H), 4.50-4.40 (m, 1H), 4.33-4.07 (m, 6H), 3.82-3.74 (m, 1H), 3.64-3.54 (m, 1H), 3.24-2.97 (m, 6H), 2.92 (s, 3H), 2.69-2.62 (m, 1H), 2.47 (s, 1H), 2.43 (s, 1H), 2.02-1.91 (m, 2H), 1.79-1.68 (m, 2H), 1.61-1.15 (m, 9H).

### Example 308: Synthesis of Compound 508

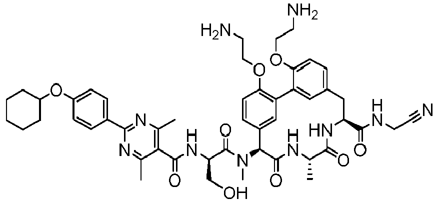

Compound 508 was isolated as a minor epimer from the synthesis of Compound 507, *via* chrial SFC. LCMS (ESI): t_{R} = 3.13 min, [M + H]⁺ = 933.5. ¹H NMR (400 MHz, DMSO-*d*₆) δ 9.06 (d, *J* = 7.1 Hz, 1H), 8.66 (t, *J* = 5.4 Hz, 1H), 8.53 (d, *J* = 9.0 Hz, 1H), 8.74-8.49 (m, 2H), 8.36-8.18 (m, 2H), 7.86 (br s, 6H), 7.32-7.22 (m, 1H), 7.20-7.01 (m, 5H), 6.94 (s, 1H), 6.42 (s, 1H), 5.04-4.93 (m, 1H), 4.65-4.54 (m, 1H), 4.50-4.40 (m, 1H), 4.28-4.11 (m, 6H), 3.97-3.86 (m, 1H), 3.84-3.75 (m, 1H), 3.65-3.56 (m, 1H), 3.23-3.05 (m, 6H), 3.01 (s, 3H), 2.74-2.64 (m, 1H), 2.41 (s, 1H), 2.02-1.90 (m, 2H), 1.80-1.68 (m, 2H), 1.61-1.14 (m, 9H).

### Example 309: Synthesis of Compound 509

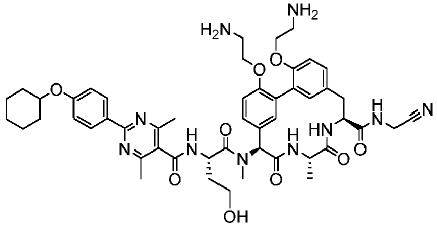

Compound **509** (trifluoroacetic acid salt) was prepared as a white solid utilizing methods analogous to those described in Example R from (*S*)-2-(((benzyloxy)carbonyl)amino)-4-((*tert*butyldimethylsilyl)oxy)butanoic acid, 2-[4-(cyclohexoxy)phenyl]-4,6-dimethyl-pyrimidine-5-carboxylic acid and Compound 101-G. LCMS (ESI): t_{R} = 3.14 min, [M + H]⁺ = 947.5. ¹H NMR (400 MHz, DMSO-*d*₆) δ ¹H NMR (400 MHz, DMSO-*d*₆) δ 8.98-8.95 (m, 2H), 8.69 (t, *J* = 4.5 Hz, 1H), 8.33-8.23 (m, 3H), 7.94-7.84 (br s, 6H), 7.26-7.04 (m, 7H), 6.76-6.70 (m, 2H), 6.45 (s, 1H), 5.18-4.97 (m, 2H), 4.79-4.67 (m, 2H), 4.49-4.42 (m, 2H), 4.29-4.07 (m, 6H), 3.56-3.50 (m, 2H), 3.22-2.97 (m, 7H), 2.89 (s, 3H), 2.00-1.83 (m, 3H), 1.79-1.69 (m, 3H), 1.99-1.51 (m, 1H), 1.50-1.35 (m, 4H), 1.33-1.25 (m, 2H), 1.21 (d, *J* = 7.0 Hz, 3H).

### Example 310: Synthesis of Compound 510

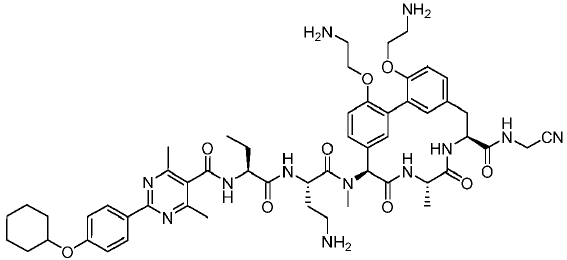

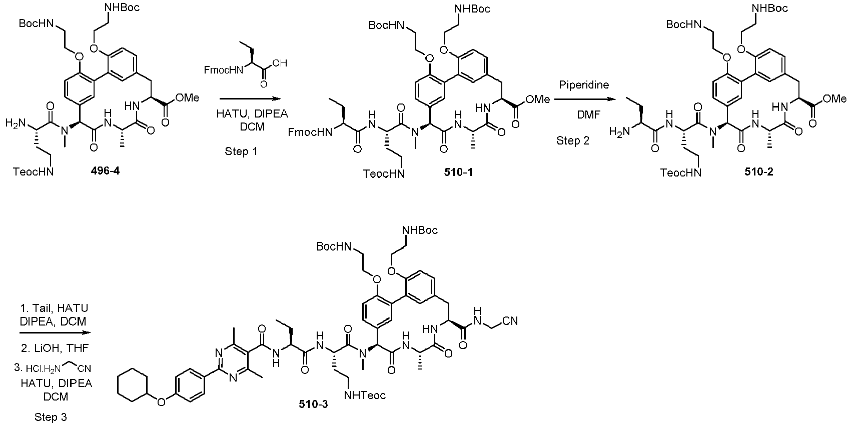

Step 1: To a mixture of Compound 496-4 (described in Example 296)(479 mg, 0.50 mmol) and (2*S*)-2-(9*H*-fluoren-9-ylmethoxycarbonylamino)butanoic acid (195 mg, 0.60 mmol) in DCM (7 mL) was added DIPEA (0.17 mL, 1 mmol) followed by HATU (247 mg, 0.65 mmol) portion wise. The mixture was stirred at 20 °C for 16 h. The mixture was diluted with DCM, washed with water, brine, then dried over Na₂SO₄ and the solvent removed. The crude product was purified via silica gel chromatography (solvent gradient: 70-80% ethyl acetate/cyclohexane) to give Compound 510-1 (435 mg, 69%) as a colorless oil. LCMS (ESI): [M + H]⁺ = 1265.

Step 2: To Compound 510-1 (432 mg, 0.34 mmol) in DMF (4 mL) was added piperidine (0.34 mL, 3.41 mmol) and the mixture stirred at 20 °C for 16 h. The mixture was diluted with water and extracted twice with ethyl acetate. The combined organic extracts were washed with brine, dried over Na₂SO₄ and the solvent removed. The residue was purified by silica gel chromatography (solvent gradient: 7-10% MeOH/DCM) to give Compound 510-2 (313 mg, 88%) as a white solid. LCMS (ESI): [M + H]⁺ =1044.

Step 3: The methods of Example G were applied to Compound 510-2 to afford Compound 510-3 (133 mg, 33% over 3 steps) as a colorless glass. LCMS (ESI): [M + H]⁺ = 1376.

Step 4: To a solution of Compound 510-3 (130 mg, 0.09 mmol) in THF (2 mL) was added a solution of tetrabutylammonium fluoride (1.0 M in THF, 0.14 mL, 0.14 mmol) and the mixture stirred at 60 °C for 24 hours. The mixture was allowed to cool to ambient temperature and the solvent was removed under reduced pressure. The residue was partitioned between ethyl acetate and brine, the phases separated. The organic layer was dried over Na₂SO₄ and the solvent removed. The general global Boc removal (TFA/HFIP) procedure of Example G was followed to give Compound 510 (trifluoroacetic acid salt) as a white solid. LCMS (ESI): t_{R} = 2.86 min, [M + H]⁺ = 1032.2. ¹H NMR (400 MHz, DMSO-*d*₆) δ 8.97 (d, *J* = 8.0 Hz, 1H), 8.79 (d, *J* = 6.4 Hz, 1H), 8.73 (t, *J* = 5.6 Hz, 1H), 8.59 (d, *J* = 8.4 Hz, 1H), 8.38 (d, *J* = 8.8 Hz, 1H), 8.31 (d, *J* = 8.8 Hz, 2H), 7.99-7.89 (m, 6H), 7.79 (s, 3H), 7.33-7.02 (m, 7H), 6.71 (s, 2H), 6.30 (s, 1H), 4.96-4.89 (m, 1H), 4.78-4.67 (m, 2H), 4.47-4.33 (m, 2H), 4.24-4.10 (m, 5H), 3.27-2.81 (m, 9H), 2.74 (s, 3H), 2.47 (s, 4H), 2.06-1.84 (m, 4H), 1.78-1.23 (m, 11H), 1.21 (d, *J* = 6.8 Hz, 3H), 0.97 (t, *J* = 7.2 Hz, 3H).

### Example 311: Synthesis of Compound 511

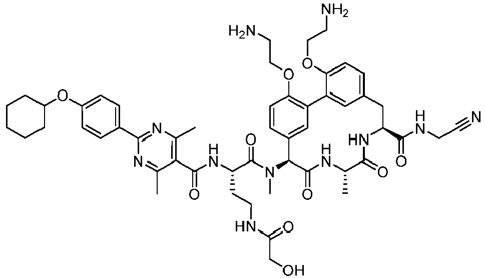

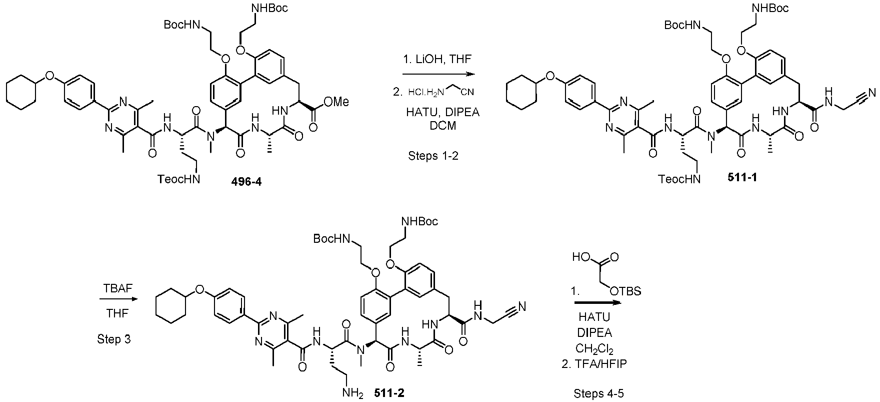

Steps 1 and 2: The methods of Example G were applied to Compound 496-4 (described in Example 296)(1.20 g, 0.95 mmol) to afford Compound 511-1 (898 mg, 73% over 2 steps) as a white foam. LCMS (ESI): [M + H]⁺ = 1266.

Step 3: To a solution of Compound 511-1 (895 mg, 0.69 mmol) in THF (5 mL) was added a solution of tetrabutylammonium fluoride (1.0 M in THF, 1 mL, 1 mmol). The mixture was stirred at 20 °C for 16 h, then the temperature raised to 50 °C and stirred for a further 6 h. The mixture was cooled to ambient temperature and the solvent removed under reduced pressure. The residue was partitioned between ethyl acetate and brine and the phases separated. The organic layer was collected and dried over Na₂SO₄ and solvent removed to yield Compound 511-2 (888 mg, 112%) as a white solid. This material was used directly in the next steps without further purification. LCMS (ESI): [M + H]⁺ = 1146.

Steps 4 and 5: Starting from Compound 511-2 and 2-[*tert*-butyl(dimethyl)silyl]oxyacetic acid, the amide coupling (HATU/DIEA) and global Boc removal (TFA/HFIP) procedures of Example G were followed to afford Compound 511 (trifluoroacetic acid salt) as a white solid. LCMS (ESI): t_{R} = 3.05 min, [M + H]⁺ = 1004.8. ¹H NMR (400 MHz, DMSO-*d*₆) δ 9.01 (d, *J* =7 Hz, 1H), 8.96 (d, *J* = 7.0 Hz, 1H), 8.69 (t, *J* = 5.5 Hz, 1H), 8.32 (d, *J* = 8.7 Hz, 2H), 8.27 (d, *J* = 8.7 Hz, 1H), 7.94-7.80 (br s, 6H), 7.28-7.02 (m, 7H), 6.73 (dd, *J* = 2.0, 10.7 Hz, 2H), 6.43 (s, 1H), 4.89-4.66 (m, 4H), 4.49-4.41 (m, 2H), 4.28-4.08 (m, 6H), 3.78 (s, 2H), 3.32-2.97 (m, 9H), 2.85 (s, 3H), 2.03-1.92 (m, 3H), 1.82-1.69 (m, 3H), 1.60-1.35 (m, 5H), 1.35-1.24 (m, 2H), 1.21 (d, *J* = 7.0 Hz, 3H).

### Example 312: Synthesis of Compound 512

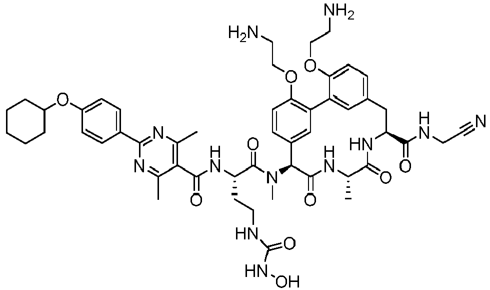

Step 1: To a solution of 1,1'-carbonylimidazole (55 mg, 0.34 mmol) in THF (2.0 mL) under nitrogen at 20 °C was added a solution of Compound 511-2 (195 mg, 0.17 mmol) in THF (2.0 mL) dropwise. The mixture was stirred at 20 °C for 1 hour, then O-(*tert*-butyldimethylsilyl)hydroxylamine (75 mg, 0.51 mmol) was added. The mixture was stirred at 20 °C for 18 h. The temperature was raised to 50 °C and stirred for a further 4 h. The mixture was cooled to ambient temperature, partitioned between ethyl acetate and water and the phases separated. The organic layer was washed with 0.1M aqueous HCl, brine, dried over Na₂SO₄ and the solvent removed under reduced pressure. The resulting residue was used directly in next step without further purification LCMS (ESI): [M + H]⁺ = 1206.

Step 2: The global Boc removal (TFA/HFIP) procedure of Example G was followed to give Compound 512 (trifluoroacetic acid salt) as a white solid. LCMS (ESI): t_{R} = 3.20 min, [M + H]⁺ = 1005.7. ¹H NMR (400 MHz, DMSO-*d*₆) δ 9.01-8.95 (m, 2H), 8.70 (t, *J* = 5.5 Hz, 1H), 8.60-8.51 (br s, 2H), 8.35-8.25 (m, 4H), 7.97-7.83 (br s, 6H), 7.28-7.01 (m, 7H), 6.87 (t, *J* = 5.5 Hz, 1H), 6.75-6.71 (br s, 2H), 6.44 (s, 1H), 4.87-4.79 (m, 2H), 4.79-4.67 (m, 2H), 4.50-4.41 (m, 2H), 4.29-4.08 (m, 6H), 3.26-2.96 (m, 9H), 2.86 (s, 3H), 2.01-1.93 (m, 3H), 1.80-1.69 (m, 3H), 1.60-1.50 (m, 1H), 1.50-1.35 (m, 4H), 1.35-1.25 (m, 2H), 1.21 (d, *J* = 7.0 Hz, 3H).

### Example 313: Synthesis of Compound 513

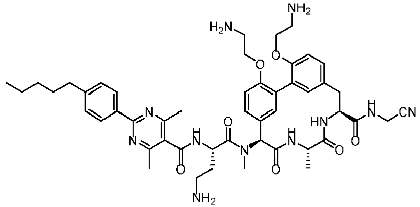

Compound 513 (formic acid salt) was prepared as a white solid from Compound **101-K** by utilizing methods analogous to those described in Example 53. LCMS (Method 5-95 AB, ESI): t_{R} = 0.754 min, [M + H]⁺ = 918.8; ¹H NMR (400 MHz, MeOH-*d*₄) δ 8.46 (br s, 3H), 8.28 (d, *J*=8.4 Hz, 2H), 7.32-7.26 (m, 3H), 7.20 (d, *J*=8.4 Hz, 2H), 7.09 (d, J=8.4 Hz, 1H), 6.90 (s, 1H), 6.75 (brs, 1H), 6.45 (s, 1H), 5.25-5.21 (m, 1H), 4.85-4.78 (m, 2H), 4.29-4.19 (m, 6H), 3.23-3.05 (m, 8H), 3.00 (s, 3H), 2.69 (t, *J*=7.6 Hz, 2H), 2.53 (s, 6H), 2.33-2.22 (m, 1H), 2.20-2.10 (m, 1H), 1.70-1.64 (m, 2H), 1.38-1.33 (m, 7H), 0.92 (t, *J*=6.8 Hz, 3H).

### Examples 314-358: Synthesis of compounds 514-558

The following compounds in table 3 were prepared by utilizing methods analogous to those previously described.

**Table 3**

| **Comp. #** | **Structure** |
|---|---|
| 514 | 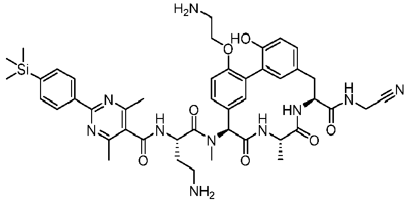 |
| 516 | 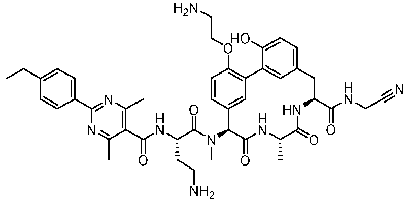 |
| 517 | 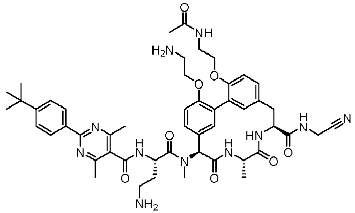 |
| 518 | 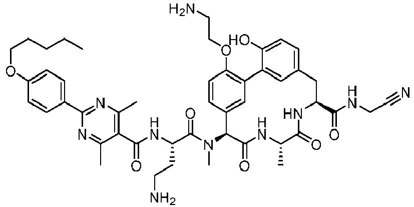 |
| 519 | 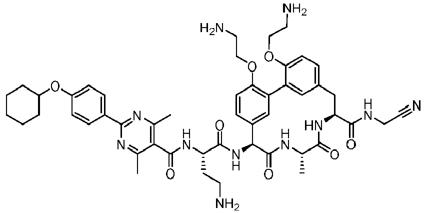 |
| 520 | 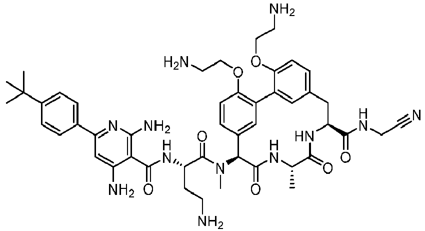 |
| 521 | 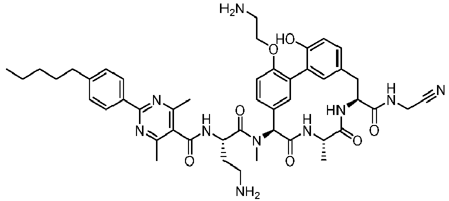 |
| 522 | 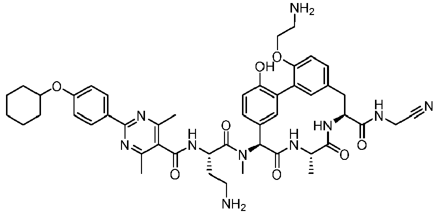 |
| 523 | 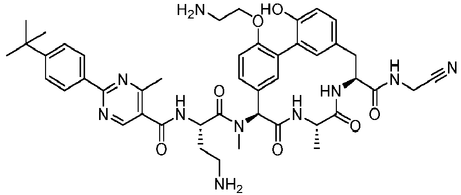 |
| 524 | 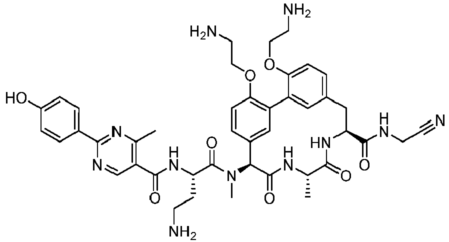 |
| 525 | 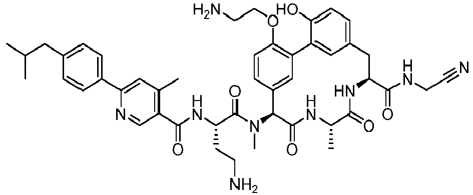 |
| 526 | 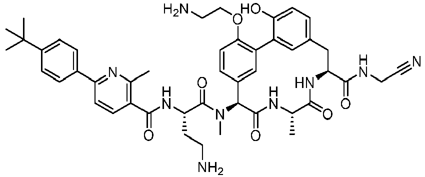 |
| 527 | 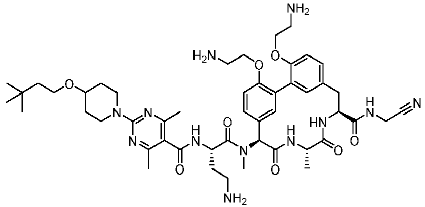 |
| 528 | 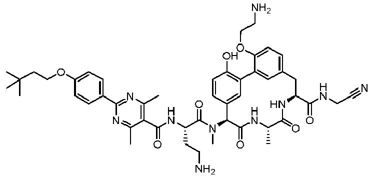 |
| 529 | 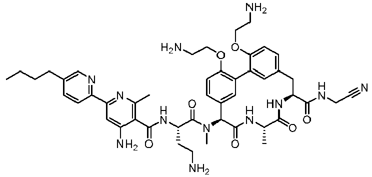 |
| 530 | 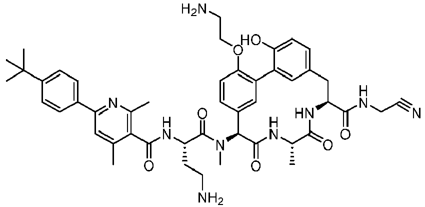 |
| 531 | 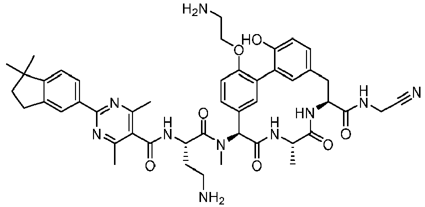 |
| 532 | 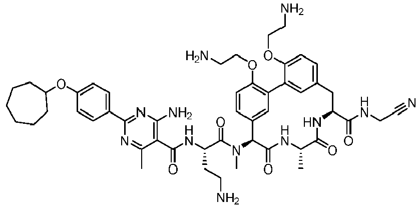 |
| 533 | 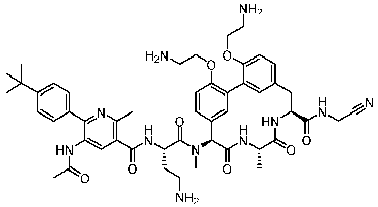 |
| 534 | 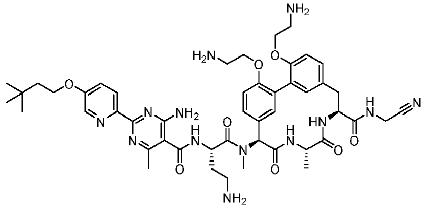 |
| 535 | 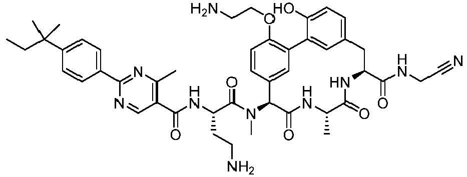 |
| 536 | 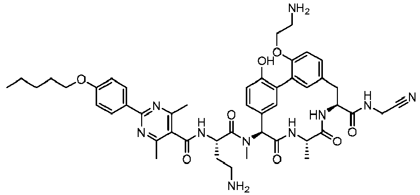 |
| 537 | 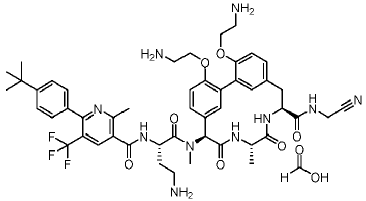 |
| 538 | 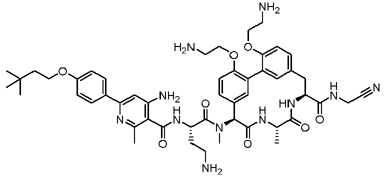 |
| 539 | 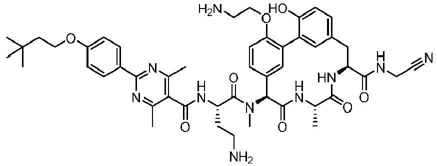 |
| 540 | 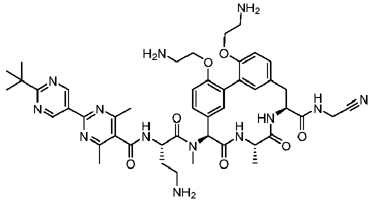 |
| 541 | 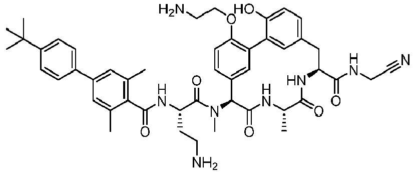 |
| 542 | 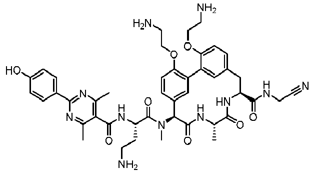 |
| 543 | 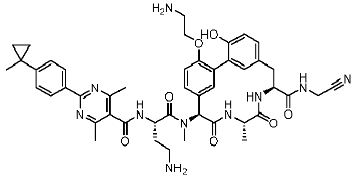 |
| 544 | 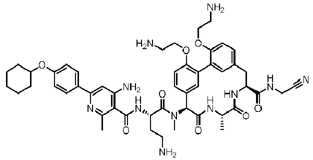 |
| 545 | 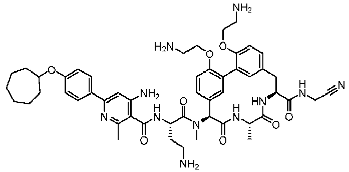 |
| 546 | 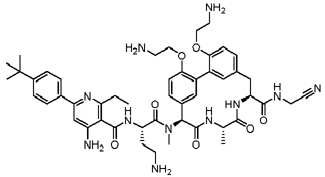 |
| 547 | 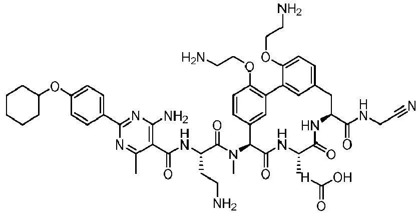 |
| 548 | 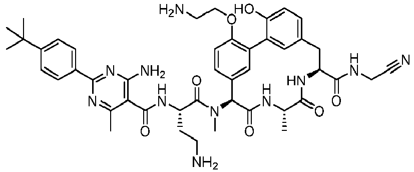 |
| 549 | 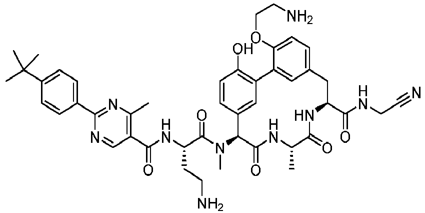 |
| 550 | 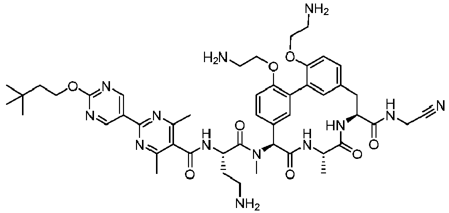 |
| 551 | 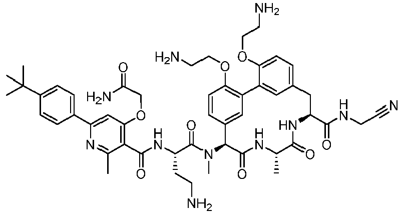 |
| 552 | 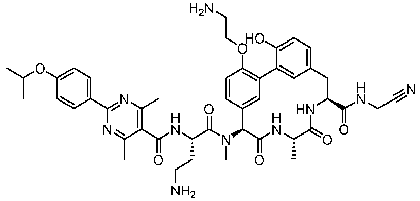 |
| 553 | 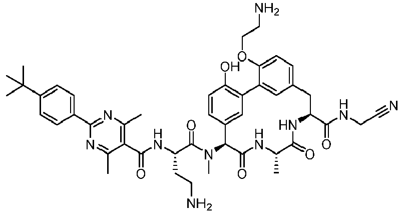 |
| 554 | 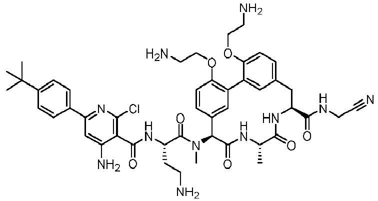 |
| 555 | 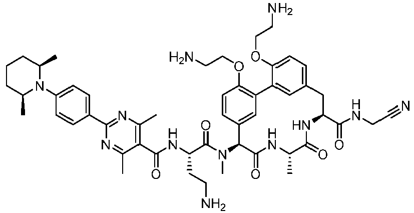 |
| 556 | 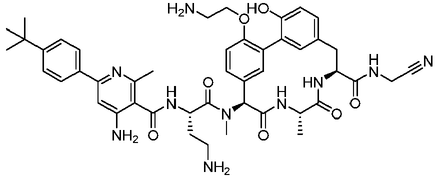 |
| 557 | 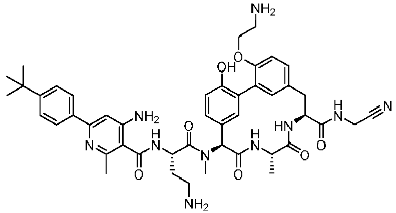 |
| 558 | 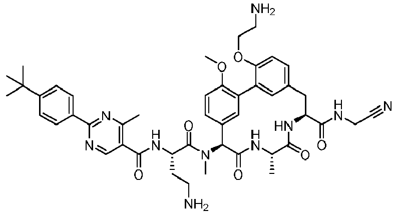 |

### Example 359. Synthesis of compound 559

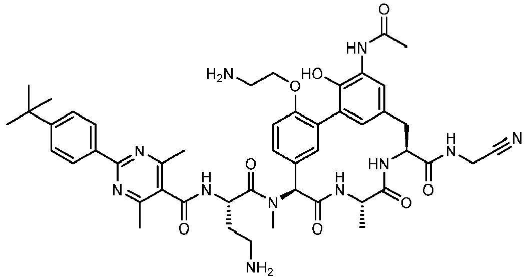

### Step 1. Methyl (4S,7S,10S)-10-((S)-4-((tert-butoxycarbonyl)afnino)-2-(2-(4-(tert-butyl)phenyl)-4,6-dimethylpyrimidine-5-carboxamido)-N-methylbutanamido)-1⁶-(2-((tert-butoxycarbonyl)amino)ethoxy)-2⁶-hydroxy-7-methyl-2⁵-nitro-6,9-dioxo-5,8-diaza-1,2(1,3)-dibenzenacyclodecaphane-4-carboxylate

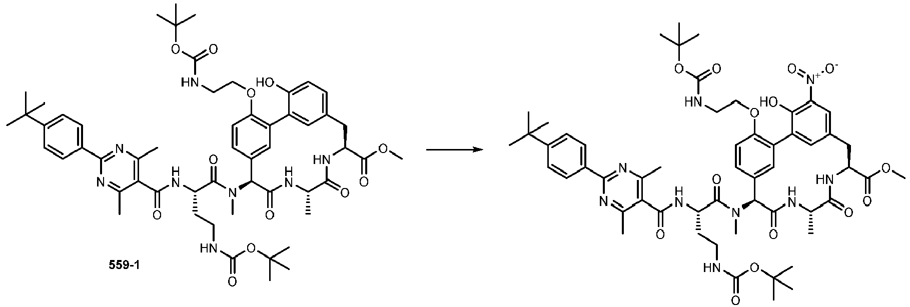

To a mixture of compound 559-1 (77.4 mg, 0.0746 mmol) and acetic acid (1.0 mL, 17 mmol) was added nitric acid (11 µL, 0.22 mmol). The reaction mixture was stirred at room temperature for 2 h. The reaction mixture was evaporated under reduced pressure, and the resulting residue was diluted with dichloromethane, washed with saturated aqueous sodium bicarbonate, dried over magnesium sulfate, filtered, and evaporated in vacuo. The crude product was purified via flash chromatography on silica gel (4 g silica, solvent gradient: 0-5% methanol in dichloromethane) to yield 63.1 mg (78%) of the title compound. LCMS (ESI): [M+H]⁺ = 1082.45.

### Step 2. methyl (4S,7S,10S)-2⁵-amino-10-((S)-4-((tert-butoxycarbonyl)amino)-2-(2-(4-(tert-butyl)phenyl)-4,6-dimethylpyrimidine-5-carboxamido)-N-methylbutanamido)-1⁶-(2-((tert-butoxycarbonyl)amino)ethoxy)-2⁶-hydroxy-7-methyl-6,9-dioxo-5,8-diaza-1,2(1,3)-dibenzenacyclodecaphane-4-carboxylate

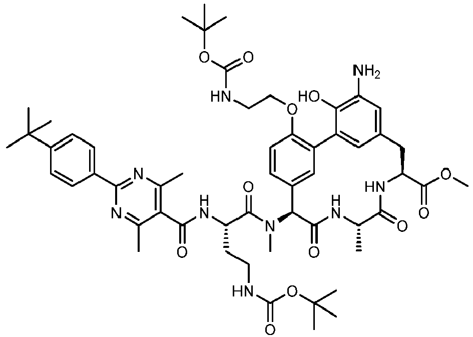

To a solution of methyl (45,75,10*S*)-10-((5)-4-((*tert*-butoxycarbonyl)amino)-2-(2-(4-(*tert-*butyl)phenyl)-4,6-dimethylpyrimidine-5-carboxamido)-*N*-methylbutanamido)-1⁶-(2-((*tert-*butoxycarbonyl)amino)ethoxy)-2⁶-hydroxy-7-methyl-2⁵-nitro-6,9-dioxo-5,8-diaza-l, 2(1,3)-dibenzenacyclodecaphane-4-carboxylate (63.1 mg, 0.0583 mmol) in ethanol (2.0 mL) was added palladium (10 wt. % on carbon) (6.5 mg, 0.0061 mmol). The reaction mixture was purged with nitrogen and then with hydrogen, and stirred at room temperature under a balloon of hydrogen for 24 h. The reaction mixture was filtered through celite, rinsing with methanol, and the filtrate was evaporated in vacuo to yield the title compound (57.7 mg, 94%). LCMS (ESI): [M+H]⁺ = 1052.4.

### Step 3. methyl (4S,7S,10S)-2⁵-acetamido-10-((S)-4-((tert-butoxycarbonyl)amino)-2-(2-(4-(tert-butyl)phenyl)-4,6-dimethylpyrimidine-5-carboxamido)-N-methylbutanamido)-1⁶-(2-((tert-butoxycarbonyl)amino)ethoxy)-2⁶-hydroxy-7-methyl-6,9-dioxo-5,8-diaza-1,2(1,3)-dibenzenacyclodecaphane-4-carboxylate

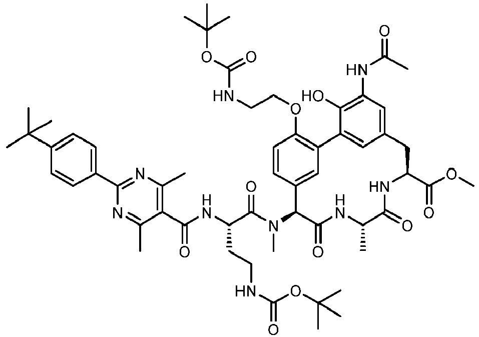

To a solution of methyl (4*S*,7*S*,10*S*)-2⁵-amino-10-((*S*)-4-((*tert*-butoxycarbonyl)amino)-2-(2-(4-(*tert*-butyl)phenyl)-4,6-dimethylpyrimidine-5-carboxamido)-*N*-methylbutanamido)-1⁶-(2-((*tert-*butoxycarbonyl)amino)ethoxy)-2⁶-hydroxy-7-methyl-6,9-dioxo-5,8-diaza-1,2(1,3)-dibenzenacyclodecaphane-4-carboxylate (57.7 mg, 0.0548 mmol) in dichloromethane (1.5 mL) was added acetic anhydride (16 µL, 0.169 mmol) and pyridine (27 µL, 0.330 mmol). The reaction mixture was stirred at room temperature for 2 h. The reaction mixture was evaporated onto celite, and the crude product was purified via flash chromatography on silica gel (4 g silica, solvent gradient: 0-5% methanol in DCM) to yield 60.0 mg (69%) of the title compound. LCMS (ESI): [M+H]⁺ = 1094.5.

### Step 4. (4S,7S,10S)-2⁵-acetamido-10-((S)-4-((tert-butoxycarbonyl)amino)-2-(2-(4-(tert-butyl)phenyl)-4,6-dimethylpyrimidine-5-carboxamido)-N-methylbutanamido)-1⁶-(2-((tert-butoxycarbonyl)amino)ethoxy)-2⁶-hydroxy-7-methyl-6,9-dioxo-5,8-diaza-1,2(1,3)-dibenzenacyclodecaphane-4-carboxylic acid

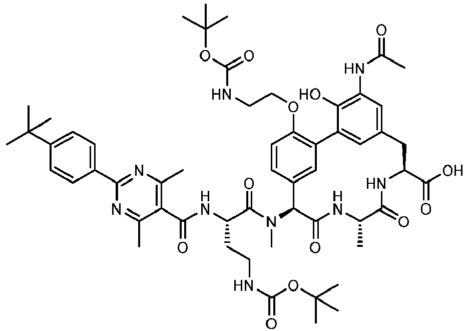

To a solution of methyl (4*S*,7*S*,10*S*)-2⁵-acetamido-10-((*S*)-4-((*tert*-butoxycarbonyl)amino)-2-(2-(4-(*tert*-butyl)phenyl)-4,6-dimethylpyrimidine-5-carboxamido)-*N*-methylbutanamido)-1⁶-(2-((*tert-*butoxycarbonyl)amino)ethoxy)-2⁶-hydroxy-7-methyl-6,9-dioxo-5,8-diaza-1,2(1,3)-dibenzenacyclodecaphane-4-carboxylate (41.3 mg, 0.0377 mmol) in tetrahydrofuran (1.0 mL) was added water (0.20 mL) and lithium hydroxide (1.0 M in water) (0.10 mL, 0.10 mmol). The reaction mixture was stirred at room temperature for 1 h. The reaction mixture was evaporated in vacuo, and carried forward without purification assuming quantitative yield. LCMS (ESI): [M+H]⁺ = 1081.25.

### Step 5. tert-butyl ((S)-4-(((3S,6S,9S)-1⁵-acetamido-2⁶-(2-((tert-butoxycarbonyl)amino)ethoxy)-9-((cyanomethyl)carbamoyl)-1⁶-hydroxy-6-methyl-4,7-dioxo-5,8-diaza-1,2(1,3)-dibenzenacyclodecaphane-3-yl)(methyl)amino)-3-(2-(4-(tert-butyl)phenyl)-4,6-dimethylpyrimidine-5-carboxamido)-4-oxobutyl)carbamate

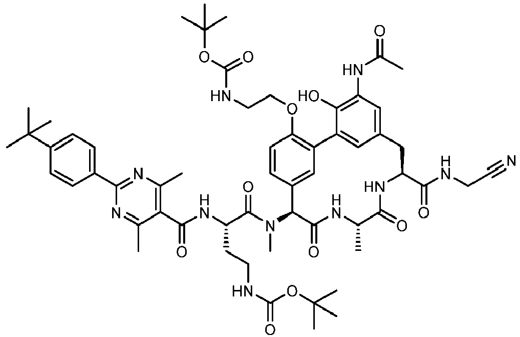

To a mixture of (4*S*,7*S*,10*S*)-2⁵-acetamido-10-((*S*)-4-((*tert*-butoxycarbonyl)amino)-2-(2-(4-(*tert-*butyl)phenyl)-4,6-dimethylpyrimidine-5-carboxamido)-*N*-methylbutanamido)-1⁶-(2-((*tert-*butoxycarbonyl)amino)ethoxy)-2⁶-hydroxy-7-methyl-6,9-dioxo-5,8-diaza-1,2(1,3)-dibenzenacyclodecaphane-4-carboxylic acid (0.0377 mmol, 0.0377 mmol), aminoacetonitrile hydrochloride (7.5 mg, 0.081 mmol), HATU (32.0 mg, 0.0825 mmol) and DMF (1.0 mL) was added N,N-diisopropylethylamine (33 µL, 0.189 mmol). The reaction mixture was stirred at room temperature for 3 h. To the reaction mixture was added 8.6 mg aminoacetonitrile hydrochloride, 50 uL N,N-diisopropylethylamine, and 31.6 mg HATU. The reaction mixture was stirred overnight at room temperature. The reaction mixture was diluted with ethyl acetate and washed with 2:1:1 water: brine: sodium bicarbonate, 10% aqueous citric acid, and brine, dried over magnesium sulfate, filtered, and evaporated in vacuo. The crude product was purified via flash chromatography on silica gel (4 g silica, solvent gradient: 0-10% methanol in dichloromethane) to yield 33.1 mg (42%) of the title compound. LCMS (ESI): [M+H]⁺ = 1118.

### Step 6. (4S,7S,10S)-2⁵-acetamido-10-((S)-4-amino-2-(2-(4-(tert-butyl)phenyl)-4,6-dimethylpyrimidine-5-carboxamido)-N-methylbutanamido)-1⁶-(2-aminoethoxy)-N-(cyanomethyl)-2⁶-hydroxy-7-methyl-6,9-dioxo-5,8-diaza-1,2(1,3)-dibenzenacyclodecaphane-4-carboxamide

To a solution of *tert*-butyl ((*S*)-4-(((3*S*,6*S*,9*S*)-1⁵-acetamido-2⁶-(2-((*tert-*butoxycarbonyl)amino)ethoxy)-9-((cyanomethyl)carbamoyl)-1⁶-hydroxy-6-methyl-4,7-dioxo-5,8-diaza-1,2(1,3)-dibenzenacyclodecaphane-3-yl)(methyl)amino)-3-(2-(4-(*tert*-butyl)phenyl)-4,6-dimethylpyrimidine-5-carboxamido)-4-oxobutyl)carbamate (33.1 mg, 0.0296 mmol) in 1,1,1,3,3,3-hexafluoro-2-propanol (1.0 mL) was added trifluoroacetic acid (0.1 mL, 1 mmol). The reaction mixture was stirred at room temperature for 2 h. The reaction mixture was evaporated in vacuo, azeotroping with diethyl ether (2 x 2 mL). The resulting residue was purified via reverse phase preparatory HPLC and lyophilized to yield 3.4 mg (12%) of the title compound. LCMS (Method A, ESI): t_{R} = 4.443 min, [M+H]⁺ = 918.4; ¹H NMR (400 MHz, DMSO-*d*₆) δ 9.92 (s, 1H), 9.20 - 9.07 (m, 2H), 9.00 (d, *J* = 7.9 Hz, 1H), 8.73 - 8.65 (m, 1H), 8.58 - 8.41 (m, 1H), 8.36 - 8.28 (m, 3H), 7.56 (d, *J* = 8.5 Hz, 3H), 7.22 - 7.12 (m, 3H), 6.86 - 6.47 (m, 3H), 6.43 (s, 1H), 5.04 (s, 1H), 4.84 - 4.63 (m, 2H), 4.37 - 4.12 (m, 5H), 3.20 (d, *J* = 4.9 Hz, 3H), 3.12 (d, *J* = 3.5 Hz, 3H), 2.95 (d, *J* = 15.8 Hz, 6H), 2.18 - 2.01 (m, 5H), 1.33 (d, *J* = 2.2 Hz, 12H), 1.21 (d, *J* = 6.9 Hz, 3H).

### Example 360. Synthesis of Compound 560

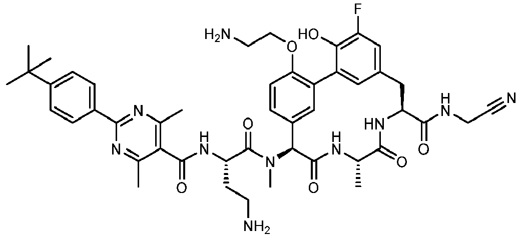

### Step 1. methyl (S)-2-((tert-butoxycarbonyl)amino)-3-(3-fluoro-4-hydroxy-5-iodophenyl)propanoate

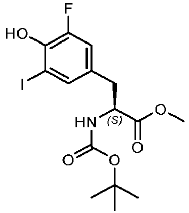

To a solution of methyl (2*S*)-2-(*tert*-butoxycarbonylamino)-3-(3-fluoro-4-hydroxyphenyl)propanoate (1.4814 g, 4.256 mmol) in methanol (10.0 mL) was sequentially added silver sulfate (1.418 g, 4.548 mmol) and iodine (1.123 g, 4.40 mmol). The reaction mixture was stirred at room temperature for 2 h. The reaction was quenched with aqueous sodium thiosulfate and extracted with ethyl acetate. The organic portion was dried over brine and magnesium sulfate, filtered, and evaporated in vacuo. The crude product was purified via flash chromatography on silica gel (40 g silica, solvent gradient: 0-100% ethyl acetate in dichloromethane) to yield 1.5246 g (81%) of the title compound. LCMS (ESI): [M+Na]⁺ = 461.95.

### Step 2. methyl (S)-3-(4-(benzyloxy)-3-fluoro-5-iodophenyl)-2-((tert-butoxycarbonyl)amino)propanoate

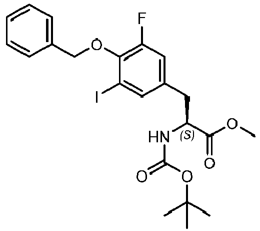

To a mixture of methyl (*S*)-2-((*tert*-butoxycarbonyl)amino)-3-(3-fluoro-4-hydroxy-5-iodophenyl)propanoate (1.5246 g, 3.471 mmol), potassium carbonate (962 mg, 6.9604 mmol) and N,N-dimethylformamide (12 mL) was added benzyl bromide (0.50 mL, 4.2 mmol). The reaction mixture was stirred at room temperature for 19 h. The reaction mixture was diluted with ethyl acetate, washed with water and brine, dried over magnesium sulfate, filtered, and evaporated in vacuo. The crude product was purified via flash chromatography on silica gel (40 g silica, solvent gradient: 0-50% ethyl acetate in heptanes) to yield 1.6527 g (95%) of the title compound as a clear colorless gum. LCMS (ESI): [M+Na]⁺ = 552.0.

### Step 3. methyl (S)-3-(4-(benzyloxy)-3-fluoro-5-(4,4,5,5-tetramethyl-1,3,2-dioxaborolan-2-yl)phenyl)-2-((tert-butoxycarbonyl)amino)propanoate

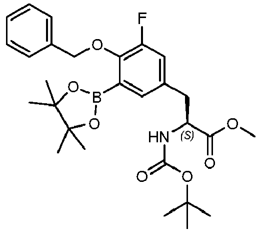

A mixture of methyl (*S*)-3-(4-(benzyloxy)-3-fluoro-5-iodophenyl)-2-((*tert-*butoxycarbonyl)amino)propanoate (1.6527 g, 3.122 mmol), bis(pinacolato)diboron (1.3990 g, 5.455 mmol), potassium acetate (0.985 g, 9.94 mmol), [1,1'-bis(diphenylphosphino)ferrocene]palladium(II) chloride, complex with dichloromethane (1:1) (0.289 g, 0.354 mmol), and dimethylsulfoxide (10.0 mL) was heated at 70 °C under nitrogen for 16 h. The reaction mixture was diluted with ethyl acetate, washed with water (2x) and brine, dried over magnesium sulfate, filtered, and evaporated in vacuo. The crude product was purified via flash chromatography on silica gel (40 g silica, solvent gradient: 0-100% ethyl acetate in dichloromethane) to yield 0.9468 g (57%) of the title compound. LCMS (ESI): [M+H-Boc]⁺ = 430.15, [M+NH₄]⁺ = 547.25. ¹H NMR (400 MHz, DMSO-d₆) δ 7.54 - 7.49 (m, 2H), 7.41 - 7.22 (m, 6H), 4.96 (s, 2H), 4.15 (td, J = 9.4, 4.7 Hz, 1H), 3.62 (s, 3H), 2.99 (dd, J = 13.7, 4.7 Hz, 1H), 2.82 (dd, J = 13.8, 10.4 Hz, 1H), 1.32 (s, 9H), 1.29 (s, 12H).

### Step 4. methyl (S)-2-amino-3-(4-(benzyloxy)-3-fluoro-5-(4,4,5,5-tetramethyl-1,3,2-dioxaborolan-2-yl)phenyl)propanoate

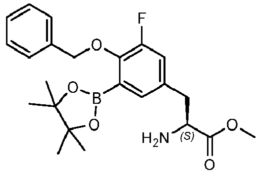

To a solution of methyl (*S*)-3-(4-(benzyloxy)-3-fluoro-5-(4,4,5,5-tetramethyl-1,3,2-dioxaborolan-2-yl)phenyl)-2-((*tert*-butoxycarbonyl)amino)propanoate (945 mg, 1.785 mmol) in dichloromethane (8 mL) was added trifluoroacetic acid (1.0 mL, 13 mmol). The reaction mixture was stirred at room temperature. After 7 h, the reaction mixture was evaporated in vacuo to afford the title compound as a TFA salt, which was carried forward without purification. LCMS (ESI): [M+H]⁺ = 430.

### Step 5. methyl (5S,8S,11S)-11-(4-(benzyloxy)-3-fluoro-5-(4,4,5,5-tetramethyl-1,3,2-dioxaborolan-2-yl)benzyl)-5-(4-(2-((tert-butoxycarbonyl)amino)ethoxy)-3-iodophenyl)-4,8-dimethyl-3,6,9-trioxo-1-phenyl-2-oxa-4,7,10-triazadodecan-12-oate

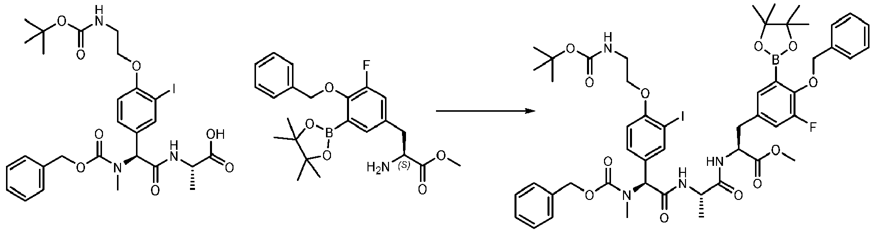

To a solution of (2*S*)-2-[[(2*S*)-2-[benzyloxycarbonyl(methyl)amino]-2-[4-[2-(*tert-*butoxycarbonylamino)ethoxy]-3-iodo-phenyl]acetyl]amino]propanoic acid (1.011 g, 1.542 mmol) in THF (5 mL) was added 2-chloro-4,6-dimethoxy-1,3,5-triazine (365.9 mg, 2.084 mmol) and 4-methylmorpholine (0.90 mL, 8.2 mmol). After 20 minutes a solution of methyl (*S*)-2-amino-3-(4-(benzyloxy)-3-fluoro-5-(4,4,5,5-tetramethyl-1,3,2-dioxaborolan-2-yl)phenyl)propanoate (1.785 mmol, 1.785 mmol) in THF (5 mL) was added. The reaction mixture was stirred at room temperature for 16 h. The reaction mixture was diluted with ethyl acetate, washed with water (2x) and brine, dried over magnesium sulfate, filtered, and evaporated in vacuo. The crude product was purified via flash chromatography on silica gel (40 g silica, solvent gradient: 0-5% methanol in dichloromethane) to yield 1.412 g (86%) of the title compound. LCMS (ESI): [M+H-tBu]⁺ = 1011.20, [M+NH₄]⁺ = 1084.30.

### Step 6. methyl (4S,7S,10S)-2⁶-(benzyloxy)-10-(((benzyloxy)carbonyl)(methyl)amino)-1⁶-(2-((tert-butoxycarbonyl)amino)ethoxy)-2⁵-fluoro-7-methyl-6,9-dioxo-5,8-diaza-1,2(1,3)-dibenzenacyclodecaphane-4-carboxylate

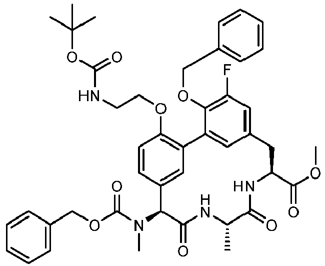

To a mixture of methyl (5*S*,8*S*,11*S*)-11-(4-(benzyloxy)-3-fluoro-5-(4,4,5,5-tetramethyl-1,3,2-dioxaborolan-2-yl)benzyl)-5-(4-(2-((*tert*-butoxycarbonyl)amino)ethoxy)-3-iodophenyl)-4,8-dimethyl-3,6,9-trioxo-1-phenyl-2-oxa-4,7,10-triazadodecan-12-oate (1.412 g, 1.324 mmol) and potassium phosphate tribasic (883 mg, 4.0766 mmol) in acetonitrile (60 mL) was added water (6.0 mL), followed by [1,1'-bis(diphenylphosphino)ferrocene]palladium(II) chloride, complex with dichloromethane (1:1) (169.0 mg, 0.2070 mmol). The reaction mixture was heated under a nitrogen balloon at 70 °C for 90 min. The reaction mixture was evaporated under reduced pressure to remove the majority of acetonitrile. The residual material was diluted with ethyl acetate, washed with water and brine, dried over magnesium sulfate, filtered, and evaporated in vacuo. The crude product was purified via flash chromatography on silica gel (40 g silica, solvent gradient: 0-5% methanol in dichloromethane) to yield 335.0 mg (31%) of the title compound. LCMS (ESI): [M+H]⁺ = 813.3.

### Step 7. methyl (4S,7S,10S)-1⁶-(2-((tert-butoxycarbonyl)amino)ethoxy)-2⁵-fluoro-2⁶-hydroxy-7-methyl-10-(methylamino)-6,9-dioxo-5,8-diaza-1,2(1,3)-dibenzenacyclodecaphane-4-carboxylate

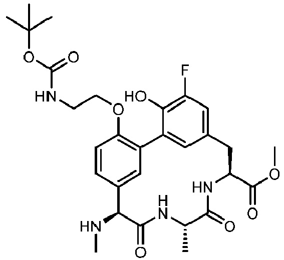

A suspension of methyl (4*S*,7*S*,10*S*)-2⁶-(benzyloxy)-10-(((benzyloxy)carbonyl)(methyl)amino)-1⁶-(2-((*tert*-butoxycarbonyl)amino)ethoxy)-2⁵-fluoro-7-methyl-6,9-dioxo-5,8-diaza-1,2(1,3)-dibenzenacyclodecaphane-4-carboxylate (335 mg, 335 mg, 0.4121 mmol) in ethanol (3 mL) was purged with nitrogen. Ammonium formate (411.1 mg, 6.520 mmol) and palladium hydroxide (20 wt. % on carbon) (139.7 mg, 0.1990 mmol) were added and the reaction mixture heated under microwave irradiation at 100 °C for 20 minutes. 72.7 mg palladium hydroxide (20 wt. % on carbon) and 220.3 mg ammonium formate were added and the mixture heated under microwave irradiation at 100 °C for 45 minutes. The reaction mixture was filtered through celite, rinsing with dichloromethane. The filtrate was diluted further with dichloromethane and washed with aqueous sodium bicarbonate to remove formate salt. The aqueous layer was extracted with an additional portion of DCM, and the combined DCM portions were dried over brine and magnesium sulfate, filtered, and evaporated in vacuo to yield 261.8 mg (quantitative) of the title compound, which was carried forward without purification. LCMS (ESI): [M+H]⁺ = 589.25.

### Step 8. methyl (4S,7S,10S)-10-((S)-2-((((9H-fluoren-9-yl)methoxy)carbonyl)amino)-4-((tert-butoxycarbonyl)amino)-N-methylbutanamido)-1⁶-(2-((tert-butoxycarbonyl)amino)ethoxy)-2⁵-fluoro-2⁶-hydroxy-7-methyl-6,9-dioxo-5,8-diaza-1,2(1,3)-dibenzenacyclodecaphane-4-carboxylate

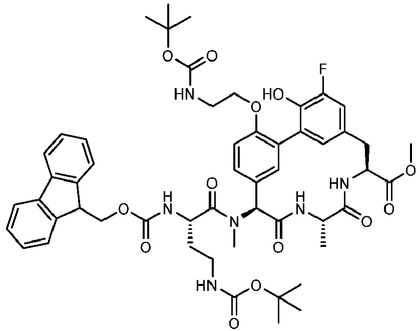

To a solution of (2*S*)-4-(*tert*-butoxycarbonylamino)-2-(9*H*-fluoren-9-ylmethoxycarbonylamino)butanoic acid (196.0 mg, 0.4449 mmol) in THF (1.0 mL) was added 2-chloro-4,6-dimethoxy-1,3,5-triazine (117.1 mg, 0.6670 mmol) and 4-methylmorpholine (0.20 mL, 1.8 mmol) After 20 minutes a solution of methyl (4*S*,7*S*,10*S*)-1⁶-(2-((*tert*-butoxycarbonyl)amino)ethoxy)-2⁵-fluoro-2⁶-hydroxy-7-methyl-10-(methylamino)-6,9-dioxo-5,8-diaza-1,2(1,3)-dibenzenacyclodecaphane-4-carboxylate (261.8 mg, 0.4003 mmol) in THF (2 mL) was added. The reaction mixture was stirred at room temperature for 2 h. The reaction mixture was diluted with ethyl acetate, washed with water and brine, dried over magnesium sulfate, filtered, and evaporated in vacuo. The crude product was purified via flash chromatography on silica gel (12 g silica, solvent gradient: 0-100% ethyl acetate in dichloromethane) to yield 273.4 mg (68%) of the title compound. LCMS (ESI): [M+H]⁺ = 1011.40.

### Step 9. methyl (4S,7S,10S)-10-((S)-2-amino-4-((tert-butoxycarbonyl)amino)-N-methylbutanamido)-1⁶-(2-((tert-butoxycarbonyl)amino)ethoxy)-2⁵-fluoro-2⁶-hydroxy-7-methyl-6,9-dioxo-5,8-diaza-1,2(1,3)-dibenzenacyclodecaphane-4-carboxylate

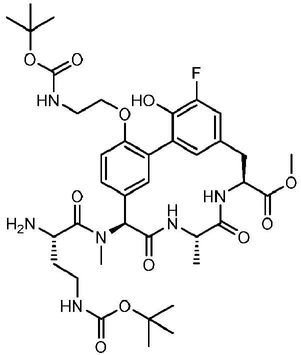

To a solution of methyl (4*S*,7*S*,10*S*)-10-((*S*)-2-((((9*H*-fluoren-9-yl)methoxy)carbonyl)amino)-4-((*tert*-butoxycarbonyl)amino)-*N*-methylbutanamido)-1⁶-(2-((*tert*-butoxycarbonyl)amino)ethoxy)-2⁵-fluoro-2⁶-hydroxy-7-methyl-6,9-dioxo-5,8-diaza-1,2(1,3)-dibenzenacyclodecaphane-4-carboxylate (273.4 mg, 0.2704 mmol) in THF (3.0 mL) was added tetrabutylammonium fluoride (1 mol/L in THF, 0.55 mL, 0.55 mmol). The reaction mixture was stirred at room temperature. After 4 h, the reaction mixture was partitioned between heptanes (50 mL) and water (50 mL) with 5 mL of 10% aqueous citric acid added. The organic layer was extracted again with 10% aqueous citric acid. The combined aqueous portions were adjusted to pH ~8 with solid sodium bicarbonate and extracted with DCM (3 x 50 mL). The combined dichloromethane portions were dried over magnesium sulfate, filtered, and evaporated in vacuo to yield the title compound in quantitative yield, which was carried forward without purification. LCMS (ESI): [M+H]⁺ = 789.40.

### Step 10. methyl (4S,7S,10S)-10-((S)-4-((tert-butoxycarbonyl)afnino)-2-(2-(4-(tert-butyl)phenyl)-4,6-dimethylpyrimidine-5-carboxamido)-N-methylbutanamido)-1⁶-(2-((tert-butoxycarbonyl)amino)ethoxy)-2⁵-fluoro-2⁶-hydroxy-7-methyl-6,9-dioxo-5,8-diaza-1,2(1,3)-dibenzenacyclodecaphane-4-carboxylate

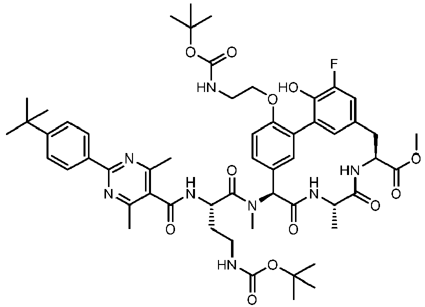

To a solution of 2-(4-*tert*-butylphenyl)-4,6-dimethyl-pyrimidine-5-carboxylic acid (91.5 mg, 0.322 mmol) in tetrahydrofuran (2 mL) was added triethylamine (0.20 mL, 1.4 mmol) followed by thionyl chloride (42 µL, 0.577 mmol). The reaction mixture was stirred at room temperature for 10 minutes, and then evaporated in vacuo. The resulting residue was suspended in 2 mL THF. Half of this THF solution (approximately 0.161 mmol) was added to a 0 °C cooled solution of methyl (45,75,105)-10-((5)-2-amino-4-((*tert*-butoxycarbonyl)amino)-*N*-methylbutanamido)-1⁶-(2-((*tert*-butoxycarbonyl)amino)ethoxy)-2⁵-fluoro-2⁶-hydroxy-7-methyl-6,9-dioxo-5,8-diaza-1,2(1,3)-dibenzenacyclodecaphane-4-carboxylate (0.135 mmol, 0.135 mmol) and triethylamine (60.0 µL, 0.426 mmol) in tetrahydrofuran (2 mL). The reaction mixture was stirred at 0 °C, allowing the ice bath to slowly warm to room temperature. After 3 h, the reaction mixture was evaporated onto celite, and the crude product was purified via flash chromatography on silica gel (12 g silica, solvent gradient: 0-5% methanol in dichloromethane) to yield 99.6 mg (70%) of the title compound. LCMS (ESI): [M+H]⁺ = 1055.50

### Step 11. (4S,7S,10S)-10-((S)-4-amino-2-(2-(4-(tert-butyl)phenyl)-4,6-dimethylpyrimidine-5-carboxamido)-N-methylbutanamido)-1⁶-(2-aminoethoxy)-N-(cyanomethyl)-2⁵-fluoro-2⁶-hydroxy-7-methyl-6,9-dioxo-5,8-diaza-1,2(1,3)-dibenzenacyclodecaphane-4-carboxamide

The title compound was prepared as a TFA salt from methyl (4*S*,7*S*,10*S*)-10-((*S*)-4-((*tert-*butoxycarbonyl)amino)-2-(2-(4-(*tert*-butyl)phenyl)-4,6-dimethylpyrimidine-5-carboxamido)-*N-*methylbutanamido)-1⁶-(2-((*tert*-butoxycarbonyl)amino)ethoxy)-2⁵-fluoro-2⁶-hydroxy-7-methyl-6,9-dioxo-5,8-diaza-1,2(1,3)-dibenzenacyclodecaphane-4-carboxylate following procedures analogous to those described for example 559, steps 4-6. LCMS (Method A, ESI): t_{R} = 4.499 min, [M+H]⁺ = 879.4; ¹H NMR (400 MHz, DMSO-*d*₆) δ 9.17 (d, J = 7.3 Hz, 1H), 8.99 (d, J = 7.9 Hz, 1H), 8.70 (t, J = 5.6 Hz, 1H), 8.45 (d, J = 9.0 Hz, 1H), 8.37 - 8.30 (m, 2H), 7.56 (d, J = 8.6 Hz, 2H), 7.22 - 7.07 (m, 3H), 6.81 (s, 1H), 6.52 (s, 1H), 6.43 (s, 1H), 5.04 (q, J = 7.7 Hz, 1H), 4.80-4.64 (m, 2H), 4.33-4.21 (m, 2H), 4.19 - 4.15 (m, 2H), 3.46-3.38 (m, 2H), 3.24-3.18 (m, 3H), 3.17 - 3.07 (m, 1H), 3.03 - 2.93 (m, 3H), 2.91 (s, 3H), 2.54 (s, 1H), 2.47-2.44 (m, 1H), 2.14 - 1.89 (m, 2H), 1.34 (s, 11H), 1.21 (d, J = 6.7 Hz, 3H).

### Example 361: Synthesis of Compound 561

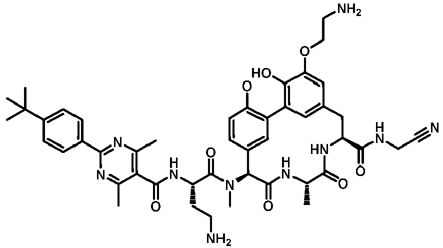

### Step 1: methyl ((S)-2-((tert-butoxycarbonyl)(methyl)amino)-2-(4-methoxyphenyl)acetyl)-L-alaninate

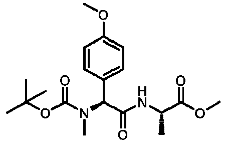

To a solution of methyl ((S)-2-((tert-butoxycarbonyl)(methyl)amino)-2-(4-hydroxyphenyl)acetyl)-L-alaninate (13.8 mmol, 5.54 g) in acetone (10 mL) was added potassium carbonate (14.25 mmol, 1.97 g) and iodomethane (14.25 mmol, 2.02 g) and the reaction was stirred at room temperature overnight. The solvent was evaporated. EtOAc was added and the mixture was filtered. The filtrate was washed with water and brine, dried over Na₂SO₄ and concentrated to give **compound 561-1** (1.355 g, 100%) as an oil, which was used as is. (MS+1) m/z 381.1

### Step 2: methyl ((S)-2-((tert-butoxycarbonyl)(methyl)amino)-2-(3-iodo-4-methoxyphenyl)acetyl)-L-alaninate

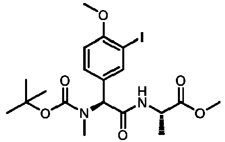

To a solution of **compound 561-1** (3.562 mmol, 1.355 g) in methanol (50 mL) at 0°C was added sequentially silver sulfate (3.740 mmol, 1.166 g) and iodine (3.740 mmol, 949 mg). The reaction mixture was stirred at room temperature for 1 h. A solution of 10% (w/w) sodium thiosulfate was added till the reaction turned pale yellow. Most of methanol was evaporated by rotary evaporation then saturated sodium bicarbonate and ethyl acetate were added. The aqueous layer was extracted twice with ethyl acetate. The combined organic layers were washed with brine, dried over sodium sulfate and concentrated. The residue was purified on silica, eluting with 0 to 3% MeOH in DCM to afford **compound 561-2** (1.393 g, 77.2%). (MS+1) m/z 507.1

### Step 3: ((S)-2-((tert-butoxycarbonyl)(methyl)amino)-2-(3-iodo-4-methoxyphenyl)acetyl)-L-alanine

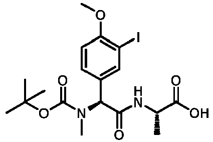

To a solution of **compound 561-2** (2.751 mmol, 1.393 g) in THF (16mL) at 0°C was added lithium hydroxide, 0.5M in water (2.751 mmol, 5.50 mL). The reaction was stirred at room temperature for 2 h. The reaction mixture was cooled to 0°C and adjusted to pH2-3 by addition of 0.5M HCl. Extracted with EtOAc, washed with brine, dried with MgSO4, concentrated in vacuo to afford crude **compound 561-3** (1.353 g, 99.9%) as a light tan solid, which was used without further purification. (MS+1) m/z 493.0

### Step 4: methyl (S)-2-((tert-butoxycarbonyl)amino)-3-(3,5-diiodo-4-methoxyphenyl)propanoate

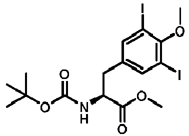

To a solution of methyl (S)-2-((tert-butoxycarbonyl)amino)-3-(4-hydroxy-3,5-diiodophenyl)propanoate (1.10 mmol, 5.471g) in acetone (50 mL) was added potassium carbonate (4.40 mmol, 5.528 g) and iodomethane (4.40 mmol, 5.677 g, 2.49 mL) and the reaction was stirred at room temperature overnight. The solvent was evaporated. EtOAc was added and the mixture was filtered. The filtrate was washed by water and brine, dried over Na₂SO₄ and concentrated. The residue was purified on silica eluted with 0 to 40% EtOAc in heptanes to give **compound 561-4** (95.321 g, 94.82%) as a white foam. (MS+1) m/z 561.8

### Step 5: methyl (S)-2-((tert-butoxycarbonyl)amino)-3-(3-hydroxy-5-iodo-4-methoxyphenyl)propanoate

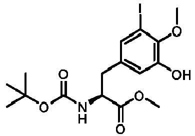

A mixture of **compound 561-4** (16.7 mmol, 9.40 g) and trimethyl borate (83.7 mmol, 8.7 g, 9.53 mL) in diethyl ether (100 mL) was cooled to -70°C. n-butyllithium (2.5 mol/L) in hexanes (41.9 mmol, 11.00 g, 17 mL) was added drop-wise while keeping internal temperature below -65°C. The reaction mixture was allowed warming to room temperature and stirred overnight.

The reaction mixture was cooled to 0°C, and treated with acetic acid (167 mmol, 10.0 g, 9.6 mL) and hydrogen peroxide (30%w/w) in water (17.1 mL). The reaction mixture was stirred overnight allowing warm to room temperature. The reaction was quenched with saturated NH4Cl (aq.) and extracted with isopropyl acetate. The organic layer was washed with brine, dried with MgSO4, and concentrated. The residue was purified on silica eluted with 0 to 50% EtOAc in heptanes afforded **compound 561-5** (2.45 g, 32.5%) as a white foam. (MS+1) m/z 452.3

### Step 6: methyl (S)-2-((tert-butoxycarbonyl)amino)-3-(3-hydroxy-4-methoxy-5-(4,4,5,5-tetramethyl-1,3,2-dioxaborolan-2-yl)phenyl)propanoate

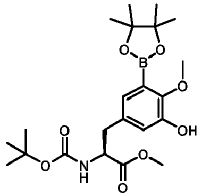

**compound 561-5** (4.63 mmol, 2.09 g), bis(pinacolato)diboron (10.2 mmol, 2.59 g), potassium acetate (18.5 mmol, 1.82 g), and 1,1'-bis(diphenylphosphino)ferrocene-palladium(II)dichloride dichloromethane complex (0.463 mmol, 386 mg) were suspended in dry dimethyl sulfoxide (50 mL) (degassed by sparging with nitrogen), and heated to 85 °C under nitrogen overnight. The reaction mixture was cooled to room temperature, and then filtered through Celite. The filtrate was diluted with 1:1 isopropyl acetate and water, and filtered through Celite. The layers were separated, and the aqueous layer was extracted 3x with isopropyl acetate. The combined organic layer was dried with MgSO4, and concentrated in vacuo. The dark brown residue was purification by flash chromatography (silica, 20 to 60% EtOAc in heptanes) provided **compound 561-6** (1.743 g, 83.4%) as a white solid. (MS+1) m/z 452.1

### Step 7: methyl (S)-2-amino-3-(3-hydroxy-4-methoxy-5-(4,4,5,5-tetramethyl-1,3,2-dioxaborolan-2-yl)phenyl)propanoate

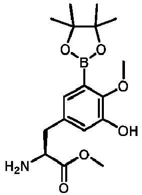

**compound 561-6** (1.405 mmol, 634 mg) in dichloromethane (4 mL) was cooled to 0°C and treated with trifluoroacetic acid (1 mL). The reaction mixture was stirred at room temperature for 2 h. The mixture was concentrated. To the residue was added Et₂O, and then concentrated. Repeat the process 2 more times. The residue was dried on high vacuum to afford **compound 561-7** (493.4 mg, 100.0%), which was used as is. (MS+1) m/z 352.1

### Step 8: methyl (6S,9S,12S)-12-(3-hydroxy-4-methoxy-5-(4,4,5,5-tetramethyl-1,3,2-dioxaborolan-2-yl)benzyl)-6-(3-iodo-4-methoxyphenyl)-2,2,5,9-tetramethyl-4,7,10-trioxo-3-oxa-5,8,11-triazatridecan-13-oate

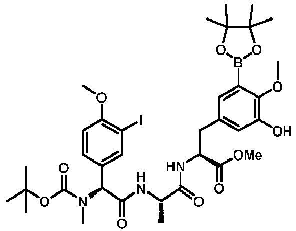

To a solution of **compound 561-7** (1.405 mmol, 493.4 mg) in acetonitrile (5 mL) and N,N-dimethylformamide (3 mL) was added 1-hydroxybenzotriazole hydrate (1.532 mmol, 234.6 mg), **compound 561-3** (1.277 mmol, 672 mg), TEA (0.38 mL, 0.38 mmol) and 1-(3-dimethylaminopropyl)-3-ethylcarbodiimide hydrochloride (3.192 mmol, 611.9 mg) at 0 °C. The resulting mixture was allowed to warm to room temperature and stirred overnight. The reaction mixture was partitioned between EtOAc and water. The aqueous layer was extracted twice with EtOAc. The combined organic layers were washed with water, saturated NaHCO₃, and brine, dried with MgSO₄ and concentrated under reduced pressure to afford crude **compound 561-8** as an off-white solid, which was used without further purification. (MS+1) m/z 826.1

### Step 9: methyl (4S,7S,10S)-10-((tert-butoxycarbonyl)(methyl)amino)-25-hydroxy-16,26-dimethoxy-7-methyl-6,9-dioxo-5,8-diaza-1,2(1,3)-dibenzenacyclodecaphane-4-carboxylate

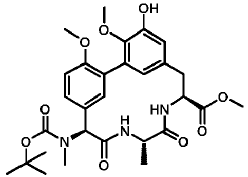

A mixture of **compound 561-8** (2.000 mmol, 1.651 g), potassium phosphate tribasic (7.000 mmol, 1.53g), and 1,1'-bis(diphenylphosphino)ferrocene-palladium(II)dichloride dichloromethane complex (0.2000 mmol, 165 mg) in degassed acetonitrile under nitrogen was heated at 65 °C for 2 h. The reaction mixture was cooled to room temperature and partitioned between EtOAc and a dilute aqueous NH4Cl solution. The aqueous phase was extracted twice with EtOAc. The combined organic layers were washed with water and brine, dried and concentrated under reduced pressure. Purification by flash column chromatography (SiO2; 0-100% EtOAc in DCM), afforded **compound 561-9** (276 mg, 17.14%) as an off-white solid. (MS+1) m/z 572.1

### Step 10: methyl (4S,7S,10S)-25-(2-(((benzyloxy)carbonyl)amino)ethoxy)-10-((tert-butoxycarbonyl)(methyl)amino)-16,26-dimethoxy-7-methyl-6,9-dioxo-5,8-diaza-1,2(1,3)-dibenzenacyclodecaphane-4-carboxylate

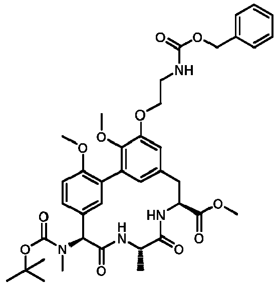

To a solution of **compound 561-9** (0.249 mmol, 142 mg) and benzyl (2-bromoethyl)carbamate (0.746 mmol, 203 mg) in N,N-dimethylformamide (5 mL) was added cesium carbonate (1.243 mmol, 405 mg). The reaction mixture was stirred at room temperature overnight. The reaction was diluted with EtOAc and washed with water. The organic layer was washed with brine and concentrated. The residue was purified on silica eluted with 0 to 2% MeOH in DCM to afford **compound 561-10** (124 mg, 47.96%). (MS+1) m/z 749.3

### Step 11: methyl (4S,7S,10S)-25-(2-(((benzyloxy)carbonyl)amino)ethoxy)-16,26-dimethoxy-7-methyl-10-(methylamino)-6,9-dioxo-5,8-diaza-1,2(1,3)-dibenzenacyclodecaphane-4-carboxylate

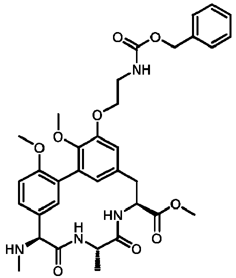

**10** (0.1416 mmol, 106 mg) in dichloromethane (8 mL) was cooled to 0°C and treated with trifluoroacetic acid (2 mL). The reaction mixture was stirred at room temperature for 1 h. The mixture was concentrated. To the residue was added Et₂O, and then concentrated again. Repeat the process 2 more times. The residue was dried on high vacuum to afford crude **compound 561-11** (91.8 mg, 100%), which was carried forward without purification. (MS+1) m/z 649.1

### Step 12: methyl (4S,7S,10S)-10-((S)-4-(((benzyloxy)carbonyl)amino)-2-((tert-butoxycarbonyl)amino)-N-methylbutanamido)-25-(2-(((benzyloxy)carbonyl)amino)ethoxy)-16,26-dimethoxy-7-methyl-6,9-dioxo-5,8-diaza-1,2(1,3)-dibenzenacyclodecaphane-4-carboxylate

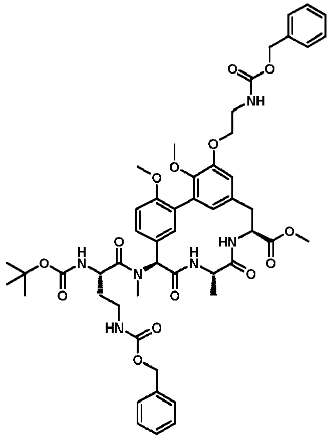

To a mixture of (2S)-4-(benzyloxycarbonylamino)-2-(tert-butoxycarbonylamino)butanoic acid (0.2127 mmol, 74.97 mg) and **compound 561-11** (0.1418 mmol, 92 mg) in N,N-dimethylformamide (5 mL) was added N,N-diisopropylethylamine (1.418 mmol, 183 mg, 0.247 mL) and HATU (0.284 mmol, 110 mg). After stirred at room temperature for 15 min, the reaction mixture was partitioned between EtOAc and water. The organic layer was washed with brine, dried with MgSO4, and concentrated. The residue was purified on silica eluted with 0-5% MeOH in DCM to afford **compound 561-12** (128 mg, 85.4%). (MS+1) m/z 983.4

### Step 13: methyl (4S,7S,10S)-10-((S)-2-amino-4-(((benzyloxy)carbonyl)amino)-N-methylbutanamido)-25-(2-(((benzyloxy)carbonyl)amino)ethoxy)-16,26-dimethoxy-7-methyl-6,9-dioxo-5,8-diaza-1,2(1,3)-dibenzenacyclodecaphane-4-carboxylate

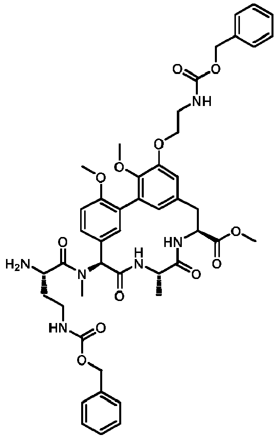

**compound 561-12** (0.2360 mmol, 232 mg) in dichloromethane (10 mL) was cooled to 0°C and treated with trifluoroacetic acid (4 mL). The reaction mixture was stirred at room temperature for 0.5 h, and then concentrated. To the residue was added Et₂O, and re-concentrated. Repeat the process 2 more times. The residue was dried under high vacuum to afford **compound 561-13,** which was carried forward without purification. (MS+1) m/z 883.3

### Step 14: methyl (4S,7S,10S)-10-((S)-4-(((benzyloxy)carbonyl)amino)-2-(2-(4-(tert-butyl)phenyl)-4,6-dimethylpyrimidine-5-carboxamido)-N-methylbutanamido)-25-(2-(((benzyloxy)carbonyl)amino)ethoxy)-16,26-dimethoxy-7-methyl-6,9-dioxo-5,8-diaza-1,2(1,3)-dibenzenacyclodecaphane-4-carboxylate

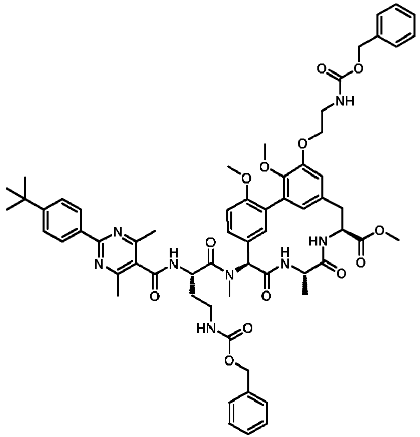

To a mixture of 2-(4-tert-butylphenyl)-4,6-dimethyl-pyrimidine-5-carboxylic acid (0.355 mmol, 101 mg) and **compound 561-13** (0.237 mmol, 209 mg) in N,N-dimethylformamide (8 mL) was added N,N-diisopropylethylamine (2.367 mmol, 306 mg, 0.413 mL) and HATU (0.473 mmol, 1834 mg). After stirring at room temperature for 15 min, the reaction mixture was partitioned between EtOAc and water. The organic layer was washed with brine, dried with MgSO4, and concentrated. The residue was purified on silica eluted with 0-10% MeOH in DCM to afford **compound 561-14** (138 mg, 51%). (MS+1) m/z 1149.5

### Step 15: (4S,7S,10S)-10-((S)-4-amino-2-(2-(4-(tert-butyl)phenyl)-4,6-dimethylpyrimidine-5-carboxamido)-N-methylbutanamido)-25-(2-aminoethoxy)-16,26-dihydroxy-7-methyl-6,9-dioxo-5,8-diaza-1,2(1,3)-dibenzenacyclodecaphane-4-carboxylic acid

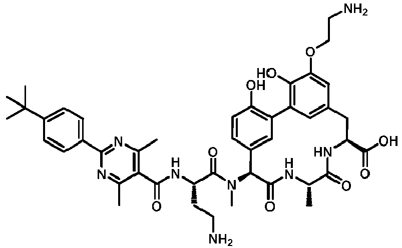

Aluminum chloride (7.233 mmol, 965 mg) and 1-dodecanethiol (7.233 mmol, 1.464 mg, 1.73 mL) in dichloromethane (15 mL) was stirred at room temperature for 0.5 h. 14 (0.120 mmol, 156 mg) in dichloromethane (15 mL) was added gradually. The resulting mixture was stirred at room temperature overnight. The reaction mixture was concentrated, and the residue dried on high vacuum to afford crude **compound 561-15** which was carried forward without purification. (MS+1) m/z 839.4

### Step 16: methyl (4S,7S,10S)-10-((S)-4-amino-2-(2-(4-(tert-butyl)phenyl)-4,6-dimethylpyrimidine-5-carboxamido)-N-methylbutanamido)-25-(2-aminoethoxy)-16,26-dihydroxy-7-methyl-6,9-dioxo-5,8-diaza-1,2(1,3)-dibenzenacyclodecaphane-4-carboxylate

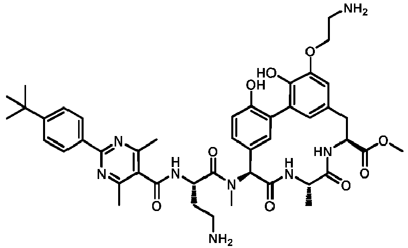

To **compound 561-15** (0.1204 mmol, 101 mg) in anhydrous methanol (15 mL) was added thionyl chloride (1 mL). The reaction mixture was stirred at room temperature for 1 h. The mixture was concentrated to provide crude **compound 561-16,** which was carried forward without purification.

### Step 17: methyl (4S,7S,10S)-10-((S)-4-((tert-butoxycarbonyl)amino)-2-(2-(4-(tert-butyl)phenyl)-4,6-dimethylpyrimidine-5-carboxamido)-N-methylbutanamido)-25-(2-((tert-butoxycarbonyl)amino)ethoxy)-16,26-dihydroxy-7-methyl-6,9-dioxo-5,8-diaza-1,2(1,3)-dibenzenacyclodecaphane-4-carboxylate

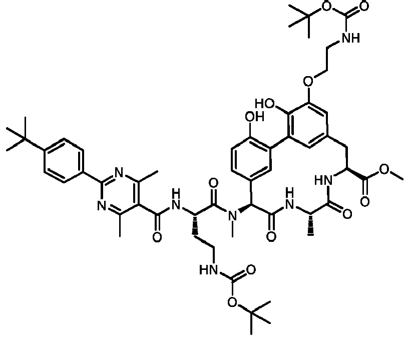

**16** (0.1184 mmol, 101 mg) in acetone (15 mL) and water (5 mL) was treated with saturated K2CO3 in water to pH 9-10. Di-tert-butyl dicarbonate (1.184 mmol, 266.4 mg) was then added and the reaction mixture was stirred at room temperature for 1h. The reaction mixture was extracted with EtOAc and the organic layer was concentrated. The residue was purified on silica eluted with 0 to 10% MeOH in DCM provided **compound 561-17** (124.7 mg, 100.0%). (MS+1) m/z 1053.6

### Step 18: methyl (4S,7S,10S)-10-((S)-4-((tert-butoxycarbonyl)amino)-2-(2-(4-(tert-butyl)phenyl)-4,6-dimethylpyrimidine-5-carboxamido)-N-methylbutanamido)-25-(2-((tert-butoxycarbonyl)amino)ethoxy)-16-((tert-butoxycarbonyl)oxy)-26-hydroxy-7-methyl-6,9-dioxo-5,8-diaza-1,2(1,3)-dibenzenacyclodecaphane-4-carboxylate

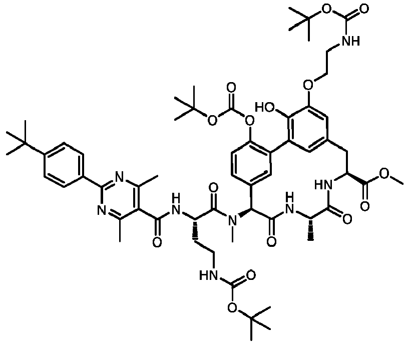

A mixture of **compound 561-17** (0.1184 mmol, 124.7 mg), N,N-diisopropylethylamine (0.5920 mmol, 76 mg, 0.103 mL), and di-tert-butyl dicarbonate (0.5920 mmol, 133 mg) in dichloromethane (20 mL) was stirred at room temperature overnight. The reaction mixture was concentrated and the residue purified on silica eluted with 0 to 10% MeOH in DCM afforded **compound 561-18** (117 mg, 86%). (MS+1) m/z 1154.2

### Step 19: (4S,7S,10S)-10-((S)-4-((tert-butoxycarbonyl)amino)-2-(2-(4-(tert-butyl)phenyl)-4,6-dimethylpyrimidine-5-carboxamido)-N-methylbutanamido)-25-(2-((tert-butoxycarbonyl)amino)ethoxy)-16-((tert-butoxycarbonyl)oxy)-26-hydroxy-7-methyl-6,9-dioxo-5,8-diaza-1,2(1,3)-dibenzenacyclodecaphane-4-carboxylic acid

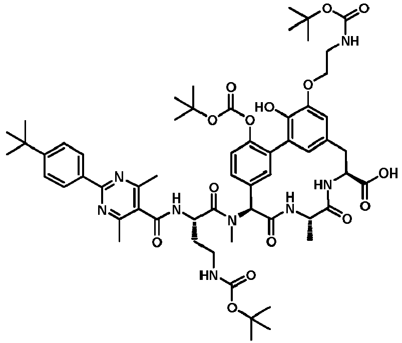

**compound 561-18** (0.101 mmol, 116 mg) was dissolved in 1,4-dioxane (6 mL) and water (3 mL). Lithium hydroxide (0.5 M, 0.304 mmol, 0.61 mL) was added at 0°C drop-wise, and the reaction mixture was stirred at room temperature for 1h. Water (6 mL) was added and the reaction mixture, and the mixture was acidified with 0.5 M HCl to pH~3. The resultant white precipitate was extracted with EtOAc, dried with MgSO4, filtered, and concentrated to afford crude **compound 561-19** (106.8 mg, 92.64%) as a white solid, which was used as is. (MS+1) m/z 1140.4

### Step 20: tert-butyl (2-(((3S,6S,9S)-3-((S)-4-((tert-butoxycarbonyl)amino)-2-(2-(4-(tert-butyl)phenyl)-4,6-dimethylpyrimidine-5-carboxamido)-N-methylbutanamido)-26-((tert-butoxycarbonyl)oxy)-9-((cyanomethyl)carbamoyl)-16-hydroxy-6-methyl-4,7-dioxo-5,8-diaza-1,2(1,3)-dibenzenacyclodecaphane-15-yl)oxy)ethyl)carbamate

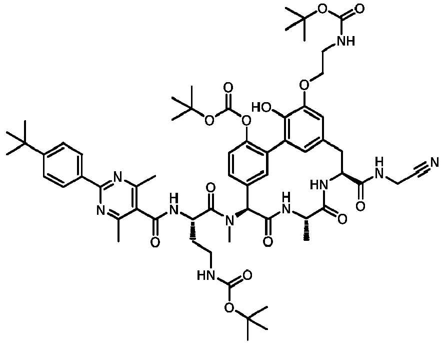

To a mixture of **compound 561-19** (0.035 mmol, 40 mg), aminoacetonitrile hydrochloride (0.35 mmol, 33mg) in N,N-dimethylformamide (5 mL) was added N,N-diisopropylethylamine (0.526 mmol, 68 mg, 0.092 mL) and HATU (0.0526 mmol, 21 mg). The reaction was stirred at room temperature for 1h. The reaction mixture was diluted with EtOAc, and washed with water. The organic layer was dried with Na₂SO₄ and concentrated. The residue was purified on silica eluted with 0 to 10% MeOH in DCM provided **compound 561-20** (17.1 mg, 41.4%). (MS+1) m/z 1177.8

### Step 21: (4S,7S,10S)-10-((S)-4-amino-2-(2-(4-(tert-butyl)phenyl)-4,6-dimethylpyrimidine-5-carboxamido)-N-methylbutanamido)-25-(2-aminoethoxy)-N-(cyanomethyl)-16,26-dihydroxy-7-methyl-6,9-dioxo-5,8-diaza-1,2(1,3)-dibenzenacyclodecaphane-4-carboxamide

**compound 561-20** (0.0145 mmol, 17.1 mg) in trifluoroacetic Acid (0.25 mL) and 1,1,1,3,3,3-hexafluoro-2-propanol (4.75 mL) was stirred at room temperature for 1h. The reaction mixture was concentrated. To the residue was added Et₂O, and re-concentrated; repeated the process 2 more times. The residue was dried on high vacuum and then purified by reverse phase HPLC provided Compound 561 (6 mg, 47.1%) as a white powder. (MS+1) m/z 877.4 ¹H NMR (400 MHz, Methanol-d4) δ 8.34 (dd, J = 8.7, 2.2 Hz, 2H), 7.59 - 7.52 (m, 2H), 7.18 (dd, J = 8.2, 2.3 Hz, 1H), 7.07 (d, J = 2.4 Hz, 1H), 7.01 (d, J = 8.5 Hz, 1H), 6.90 (d, J = 2.0 Hz, 1H), 6.43 (s, 1H), 5.26 - 5.19 (m, 2H), 4.28 (td, J = 8.7, 7.5, 4.2 Hz, 2H), 4.20 (d, J = 7.3 Hz, 1H), 3.46 (s, 1H), 3.41 (t, J = 5.1 Hz, 3H), 3.15 (d, J = 7.5 Hz, 3H), 3.04 (s, 1H), 2.99 (s, 3H), 2.30 (dq, J = 14.2, 7.2 Hz, 1H), 2.16 (dq, J = 14.3, 7.5 Hz, 1H), 1.38 - 1.33 (m, 9H).

### Example 362: Synthesis of Compound 562

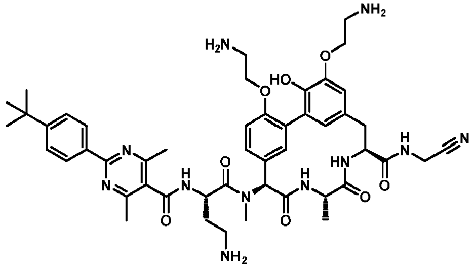

### Step 1: methyl (S)-2-amino-3-(3-bromo-4-hydroxyphenyl)propanoate hydrochloride

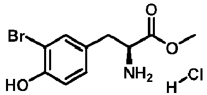

To a solution of (2S)-2-amino-3-(3-bromo-4-hydroxy-phenyl)propanoic acid (39.32 mmol, 10.3 g) in methanol (100 mL) was slowly added thionyl chloride (86.5 mmol, 10,29 g, 6.30 mL) at 0 °C. The resulting mixture was stirred at room temperature. The reaction mixture was concentrated under reduced pressure, and the residue dried on high vacuum to afford the title compound (12.21 g, 100%), which was used without further purification. (MS+1) m/z 272.0

### Step 2: methyl (S)-3-(3-bromo-4-hydroxyphenyl)-2-((tert-butoxycarbonyl)amino)propanoate

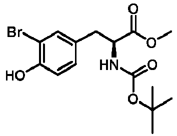

To a mixture of methyl (S)-2-amino-3-(3-bromo-4-hydroxyphenyl)propanoate hydrochloride (39.32 mmol, 12.21 g) in 1:1 acetone/water (100mL) was added sodium bicarbonate (118 mmol, 9.91 g) and di-tert-butyl dicarbonate (58.98 mmol, 13.27 g). The resulting reaction mixture was stirred at room temperature overnight. The reaction mixture was quenched by addition of 1 N HCl to pH 3-4. The aqueous layer was extracted twice with EtOAc (2x100mL) and the combined organic layers were washed with brine, dried over Na₂SO₄ and concentrated to afford crude methyl (S)-3-(3-bromo-4-hydroxyphenyl)-2-((tert-butoxycarbonyl)amino)propanoate, which was carried forward without further purification. (MS+1) m/z 372.0

### Step 3: methyl (S)-3-(3-bromo-4-hydroxy-5-iodophenyl)-2-((tert-butoxycarbonyl)amino)propanoate

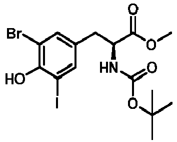

To a solution of (S)-3-(3-bromo-4-hydroxyphenyl)-2-((tert-butoxycarbonyl)amino)propanoate (18.95 mmol, 7.09 g) in N,N-dimethylformamide (50 mL) was added N-iodosuccinimide (22.74 mmol, 5.22 g). The reaction was stirred at room temperature for 1 h. The reaction mixture was partitioned between EtOAc and water. The organic layer was washed with saturated NaHCO₃ and brine, dried with Na₂SO₄, and concentrated. The residue was purified on silica eluted with 15 to 30% EtOAc in heptanes provided the title compound (6.86 g, 72.4%) as a white solid. (MS+1) m/z 499.9

### Step 4: methyl (S)-3-(3-bromo-5-iodo-4-methoxyphenyl)-2-((tert-butoxycarbonyl)amino)propanoate

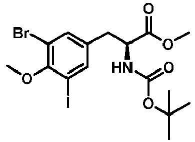

Methyl (S)-3-(3-bromo-4-hydroxy-5-iodophenyl)-2-((tert-butoxycarbonyl)amino)propanoate (6.24 mmol, 3.12 g) was dissolved in acetone (50 mL) and treated sequentially with potassium carbonate (31.2 mmol, 4.31 g) and iodomethane (7.49 mmol, 1.06 g, 0.466 mL). The solution was stirred at room temperature overnight. The solvent was evaporated under reduced pressure and the residue was dissolved in EtOAc, which was washed with water and brine, dried over Na₂SO₄ and concentrated. The residue was purified by flash chromatography (0 to 40% EtOAc in heptanes) provided the title compound (2.28 g, 71.1%) as a white foam. (MS+1) m/z 513.9

### Step 5: methyl (S)-3-(3-bromo-5-hydroxy-4-methoxyphenyl)-2-((tert-butoxycarbonyl)amino)propanoate

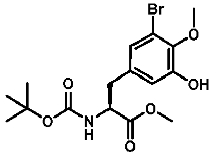

Bis(pinacolato)diboron (8.046 mmol, 2.04 g), methyl (S)-3-(3-bromo-5-iodo-4-methoxyphenyl)-2-((tert-butoxycarbonyl)amino)propanoate (5.364 mmol, 2.76 g), potassium acetate (13.41 mmol, 1.32 g), and 1,1'-bis(diphenylphosphino)ferrocene-palladium(II)dichloride dichloromethane complex (0.2682 mmol, 223 mg) were suspended in dry dimethyl sulfoxide (50 mL) (degassed by sparging with nitrogen), and heated to 65 °C under nitrogen overnight. The mixture was cooled to room temperature, and filtered through Celite. The filtrate was diluted with 1:1 EtOAc and water, and filtered through Celite. The layers were separated, and the aqueous layer was extracted 3x with EtOAc. The combined organic layer was dried with MgSO4, and concentrated in vacuo to afford crude methyl (S)-3-(3-bromo-4-methoxy-5-(4,4,5,5-tetramethyl-1,3,2-dioxaborolan-2-yl)phenyl)-2-((tert-butoxycarbonyl)amino)propanoate as a dark brown gum, which was carried forward without further purification. (MS+1) m/z 514.1

The above methyl (S)-3-(3-bromo-4-methoxy-5-(4,4,5,5-tetramethyl-1,3,2-dioxaborolan-2-yl)phenyl)-2-((tert-butoxycarbonyl)amino)propanoate (5.364 mmol, 2.76 g) was dissolved in methanol (24 mL) and hydrogen peroxide (30% in water) (8 mL) and was stirred at room temperature for 1 h. MeOH was removed under reduced pressure. To the residue was added EtOAc and brine. The organic layer was separated, dried with Na₂SO₄, filtered, and concentrated. The residue was purified on silica eluted with 0 to 50% EtOAc in heptanes provided the title compound (1.15 g, 53% over 2 steps). (MS+1) m/z 404.0

### Step 6: tert-butyl ((S)-2-(((benzyloxy)carbonyl)(methyl)amino)-2-(4-hydroxy-3-(4,4,5,5-tetramethyl-1,3,2-dioxaborolan-2-yl)phenyl)acetyl)-L-alaninate

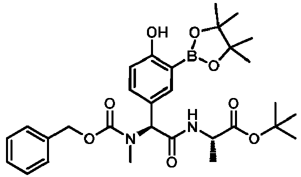

Bis(pinacolato)diboron (19.22 mmol, 4.88 tert-butyl (2S)-2-[[(2S)-2-[benzyloxycarbonyl(methyl)amino] -2-(4-hydroxy-3 -iodo-phenyl)acetyl] amino]propanoate (12.01 mmol, 6.827 g), potassium acetate (36.03 mmol, 3.536 g), and 1,1'-bis(diphenylphosphino)ferrocene-palladium(II)dichloride dichloromethane complex (0.6005 mmol, 500 mg) were suspended in dry dimethyl sulfoxide (120 mL) (degassed by sparging with nitrogen), and heated to 85 °C under nitrogen overnight. The mixture was cooled to room temperature, and then filtered through Celite. The filtrate was diluted with 1:1 EtOAc and water, and filtered through Celite. The layers were separated, and the aqueous layer was extracted 3 times with EtOAc. The combined organic layer was dried with MgSO4, and concentrated in vacuum and the residue was run through a quick silica column eluted with 20 to 80% EtOAc in heptanes provided the title compound (3.85 g, 56.4%). (MS+1) m/z 569.2

### Step 7: methyl (S)-2-((tert-butoxycarbonyl)amino)-3-(2',5-dihydroxy-6-methoxy-5'-((SS,8S)-4,8,11,11-tetramethyl-3,6,9-trioxo-1-phenyl-2,10-dioxa-4,7-diazadodecan-5-yl)-[1,1'-biphenyl]-3-yl)propanoate

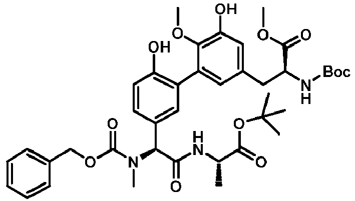

A mixture of tert-butyl ((S)-2-(((benzyloxy)carbonyl)(methyl)amino)-2-(4-hydroxy-3-(4,4,5,5-tetramethyl-1,3,2-dioxaborolan-2-yl)phenyl)acetyl)-L-alaninate (3.42 mmol, 1.94 g), methyl (S)-3-(3-bromo-5-hydroxy-4-methoxyphenyl)-2-((tert-butoxycarbonyl)amino)propanoate (2.85 mmol, 1.92 g), potassium phosphate tribasic (11.4 mmol, 2.49 g and chloro(crotyl)(tri-tert-butylphosphine)palladium(II) (0.285 mmol, 120 mg) in degassed acetonitrile (60 mL) and water (4 mL) under nitrogen was heated at 85 °C for 2 h. The reaction mixture was concentrated under reduced pressure, and the residue was purified by silica gel column chromatography (0-50% EtOAc in DCM) provided the title compound (2.64 g, 60.5%). (MS+1) m/z 766.3

### Step 8: ((S)-2-(5'-((S)-2-amino-3-methoxy-3-oxopropyl)-3',6-dihydroxy-2'-methoxy-[1,1'-biphenyl]-3-yl)-2-(((benzyloxy)carbonyl)(methyl)amino)acetyl)-L-alanine

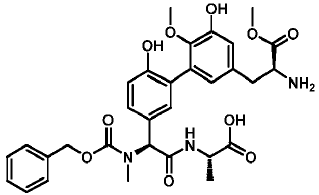

methyl (S)-2-((tert-butoxycarbonyl)amino)-3-(2',5-dihydroxy-6-methoxy-5'-((5S,8S)-4,8,11,11-tetramethyl-3,6,9-trioxo-1-phenyl-2,10-dioxa-4,7-diazadodecan-5-yl)-[1,1'-biphenyl]-3-yl)propanoate (1.72 mmol, 2.64 g) in TFA (8mL) and DCM (32mL) was stirred at room temperature overnight. The reaction mixture was concentrated. To the residue was added Et₂O and re-concentrated and the process was repeated two more times. The residue was dried on high vacuum to afford the crude title compound (1.05 g, 100%), which was carried forward without further purification. (MS+1) m/z 610.2

### Step 9: methyl (4S,7S,10S)-10-(((benzyloxy)carbonyl)(methyl)amino)-16,25-dihydroxy-26-methoxy-7-methyl-6,9-dioxo-5,8-diaza-1,2(1,3)-dibenzenacyclodecaphane-4-carboxylate

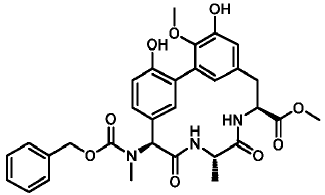

To a solution of 2,4,6-tripropyl-1,3,5,2,4,6-trioxatriphosphorinane-2,4,6-trioxide, 50% in DMF (3.79 mmol, 2.41 g, 2.21 mL) and N,N-diisopropylethylamine (8.61 mmol, 1.11 g, 1.50 mL) in N,N-dimethylformamide (42 mL) at room temperature was added a solution of ((S)-2-(5'-((S)-2-amino-3-methoxy-3-oxopropyl)-3',6-dihydroxy-2'-methoxy-[1,1'-biphenyl]-3-yl)-2-(((benzyloxy)carbonyl)(methyl)amino)acetyl)-L-alanine (1.72 mmol, 1.05 g) in N,N-dimethylformamide (43 mL). The resulting reaction mixture was stirred at room temperature for 1 h. The reaction mixture was diluted with EtOAc, washed with saturated NaHCO₃ (aq.) and brine. The organic layer was dried with MgSO4, and concentrated. The residue was purified on silica eluted with 0 to 5% MeOH in DCM to provide the title compound (747 mg, 49.1%). (MS+1) m/z 592.2

### Step 10: methyl (4S,7S,10S)-10-(((benzyloxy)carbonyl)(methyl)amino)-16,25-bis(2-((tert-butoxycarbonyl)amino)ethoxy)-26-methoxy-7-methyl-6,9-dioxo-5,8-diaza-1,2(1,3)-dibenzenacyclodecaphane-4-carboxylate

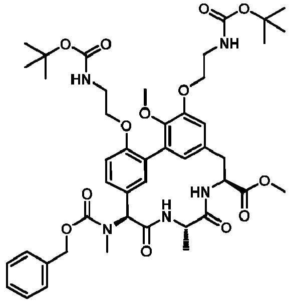

To a solution of methyl (4S,7S,10S)-10-(((benzyloxy)carbonyl)(methyl)amino)-16,25-dihydroxy-26-methoxy-7-methyl-6,9-dioxo-5,8-diaza-1,2(1,3)-dibenzenacyclodecaphane-4-carboxylate (0.3222 mmol, 373.7 mg) and tert-butyl n-(2-bromoethyl)carbamate (1.611 mmol, 380 mg) in N,N-dimethylformamide (10 mL) was added cesium carbonate (1.611 mmol, 524.8 mg). The reaction mixture was stirred at room temperature overnight. The reaction mixture was diluted with EtOAc, and washed with water. The organic layer was washed with brine and concentrated. The residue was purified on silica eluted with 0 to 5% MeOH in DCM to afford the title compound (266 mg, 48%). (MS+1) m/z 878.0

### Step 11: methyl (4S,7S,10S)-16,25-bis(2-((tert-butoxycarbonyl)amino)ethoxy)-26-methoxy-7-methyl-10-(methylamino)-6,9-dioxo-5,8-diaza-1,2(1,3)-dibenzenacyclodecaphane-4-carboxylate

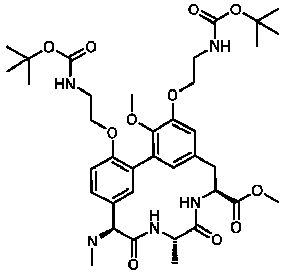

To a solution of methyl (4S,7S,10S)-10-(((benzyloxy)carbonyl)(methyl)amino)-16,25-bis(2-((tert-butoxycarbonyl)amino)ethoxy)-26-methoxy-7-methyl-6,9-dioxo-5,8-diaza-1,2(1,3)-dibenzenacyclodecaphane-4-carboxylate (0.1545 mmol, 266 mg) in isopropyl alcohol (50mL) was added 20% Pd(OH)₂ (50mg) and the reaction was placed under an atmosphere of hydrogen. The reaction mixture was stirred at room temperature overnight. The reaction mixture was filtered through Celite and the filtrate concentrated to afford crude methyl (4S,7S,10S)-16,25-bis(2-((tert-butoxycarbonyl)amino)ethoxy)-26-methoxy-7-methyl-10-(methylamino)-6,9-dioxo-5,8-diaza-1,2(1,3)-dibenzenacyclodecaphane-4-carboxylate (114.9 mg, 100%), which was carried forward without further purification. (MS+1) m/z 744.0

### Step 12: methyl (4S,7S,10S)-10-((S)-2-((((9H-fluoren-9-yl)methoxy)carbonyl)amino)-4-((tert-butoxycarbonyl)amino)-N-methylbutanamido)-16,25-bis(2-((tert-butoxycarbonyl)amino)ethoxy)-26-methoxy-7-methyl-6,9-dioxo-5 ,8-diaza-1,2(1,3)-dibenzenacyclodecaphane-4-carboxylate

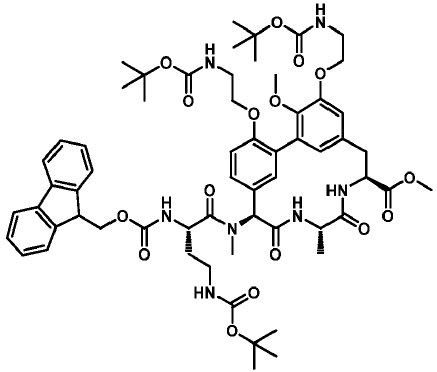

To a mixture of (2S)-4-(tert-butoxycarbonylamino)-2-(9H-fluoren-9-ylmethoxycarbonylamino)butanoic acid (0.232 mmol, 102 mg) and methyl (4S,7S,10S)-16,25-bis(2-((tert-butoxycarbonyl)amino)ethoxy)-26-methoxy-7-methyl-10-(methylamino)-6,9-dioxo-5,8-diaza-1,2(1,3)-dibenzenacyclodecaphane-4-carboxylate (0.154 mmol, 114 mg) in N,N-dimethylformamide (5 mL) was added N,N-diisopropylethylamine (0.7724 mmol, 99.8 mg, 0.135 mL) and HATU (0.3862 mmol, 149.8 mg). After stirring at room temperature for 0.5 h, the reaction mixture was partitioned between EtOAc and water, washed with brine, and the organic layer was concentrated. The residue was purified on silica eluted with 0 to 10% MeOH in DCM to provide the title compound (286 mg, 95%). (MS+1) m/z 1167.4

### Step 13: methyl (4S,7S,10S)-10-((S)-2-amino-4-((tert-butoxycarbonyl)amino)-N-methylbutanamido)-16,25-bis(2-((tert-butoxycarbonyl)amino)ethoxy)-26-methoxy-7-methyl-6,9-dioxo-5,8-diaza-1,2(1,3)-dibenzenacyclodecaphane-4-carboxylate

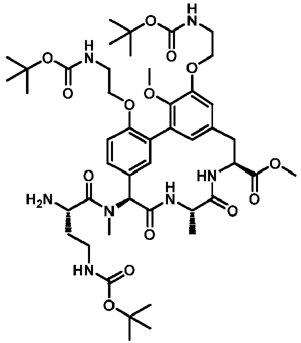

A mixture of methyl (4S,7S,10S)-10-((S)-2-((((9H-fluoren-9-yl)methoxy)carbonyl)amino)-4-((tert-butoxycarbonyl)amino)-N-methylbutanamido)-16,25-bis(2-((tert-butoxycarbonyl)amino)ethoxy)-26-methoxy-7-methyl-6,9-dioxo-5,8-diaza-1,2(1,3)-dibenzenacyclodecaphane-4-carboxylate (0.1471 mmol, 286 mg) in 10% piperidine in DMF (10mL) was stirred at room temperature for 10 min, then concentrated and dried under high vacuum to provide the crude title compound (138.9 mg, 100%), which was carried forward without further purification. (MS+1) m/z 944.6

### Step 14: methyl (4S,7S,10S)-10-((S)-4-((tert-butoxycarbonyl)amino)-2-(2-(4-(tert-butyl)phenyl)-4,6-dimethylpyrimidine-5-carboxamido)-N-methylbutanamido)-16,25-bis(2-((tert-butoxycarbonyl)amino)ethoxy)-26-methoxy-7-methyl-6,9-dioxo-5,8-diaza-1,2(1,3)-dibenzenacyclodecaphane-4-carboxylate

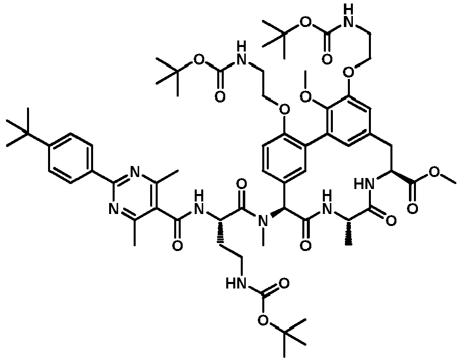

To a solution of methyl (4S,7S,10S)-10-((S)-2-amino-4-((tert-butoxycarbonyl)amino)-N-methylbutanamido)-16,25-bis(2-((tert-butoxycarbonyl)amino)ethoxy)-26-methoxy-7-methyl-6,9-dioxo-5,8-diaza-1,2(1,3)-dibenzenacyclodecaphane-4-carboxylate (0.1471 mmol, 138.9 mg) in N,N-dimethylformamide (5 mL) was added 2-(4-tert-butylphenyl)-4,6-dimethyl-pyrimidine-5-carboxylic acid (0.1765 mmol, 50.2 mg) , N,N-diisopropylethylamine (0.7356 mmol, 95 mg, 0.128 mL), and HATU (0.2207 mmol, 85.6 mg). After stirring at room temperature for 0.5 h, the reaction mixture was diluted with EtOAc, washed 3 times with water. The combined organic layers were concentrated and the residue was purified using silica gel chromatography, eluting with 0 to 10% MeOH in DCM to provide the title compound (93 mg, 52%). (MS+1) m/z 1211.5

### Step 15: (4S,7S,10S)-10-((S)-4-amino-2-(2-(4-(tert-butyl)phenyl)-4,6-dimethylpyrimidine-5-carboxamido)-N-methylbutanamido)-16,25-bis(2-aminoethoxy)-26-hydroxy-7-methyl-6,9-dioxo-5,8-diaza-1,2(1,3)-dibenzenacyclodecaphane-4-carboxylic acid

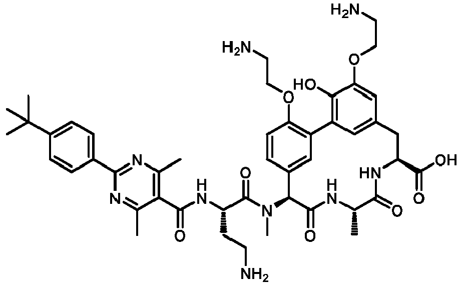

Aluminum chloride (12.0 mmol, 1.60 g) and 1-dodecanethiol (12.0 mmol, 2.43 g, 2.88 mL) in dichloromethane (10 mL) was stirred at room temperature for 0.5h, then added to a solution of methyl (4S,7S,10S)-10-((S)-4-((tert-butoxycarbonyl)amino)-2-(2-(4-(tert-butyl)phenyl)-4,6-dimethylpyrimidine-5-carboxamido)-N-methylbutanamido)-16,25-bis(2-((tert-butoxycarbonyl)amino)ethoxy)-26-methoxy-7-methyl-6,9-dioxo-5,8-diaza-1,2(1,3)-dibenzenacyclodecaphane-4-carboxylate (0.077032 mmol, 148 mg) in dichloromethane (10 mL). The resulting mixture was stirred at room temperature overnight. The mixture was concentrated, and the residue dried under high vacuum provided the crude title compound, which was carried forward without further purification. (MS+1) m/z 883.4

### Step 16: methyl (4S,7S,10S)-10-((S)-4-amino-2-(2-(4-(tert-butyl)phenyl)-4,6-dimethylpyrimidine-5-carboxamido)-N-methylbutanamido)-16,25-bis(2-aminoethoxy)-26-hydroxy-7-methyl-6,9-dioxo-5,8-diaza-1,2(1,3)-dibenzenacyclodecaphane-4-carboxylate

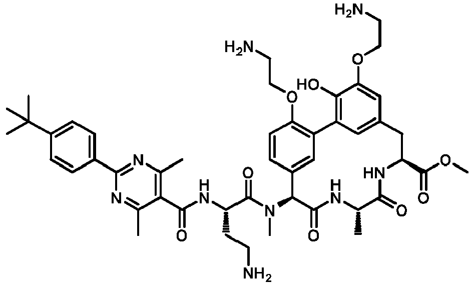

To a solution of (4S,7S,10S)-10-((S)-4-amino-2-2-4-(tert-butyl)phenyl)-4,6-dimethylpyrimidine-5-carboxamido)-N-methylbutanamido)-16,25-bis(2-aminoethoxy)-26-hydroxy-7-methyl-6,9-dioxo-5,8-diaza-1,2(1,3)-dibenzenacyclodecaphane-4-carboxylic acid (0.07596 mmol, 67 mg) in anhydrous methanol (10 mL) was added thionyl chloride (15 mmol, 1.80 g, 1.1 mL). The reaction mixture was stirred at room temperature for 1 h. The mixture was concentrated to provide the crude title compound, which was used immediately in the next step without further purification. (MS+1) m/z 896.8 **Step 17:** methyl (4S,7S,10S)-10-((S)-4-((tert-butoxycarbonyl)amino)-2-(2-(4-(tert-butyl)phenyl)-4,6-dimethylpyrimidine-5-carboxamido)-N-methylbutanamido)-16,25-bis(2-((tert-butoxycarbonyl)amino)ethoxy)-26-hydroxy-7-methyl-6,9-dioxo-5,8-diaza-1,2(1,3)-dibenzenacyclodecaphane-4-carboxylate

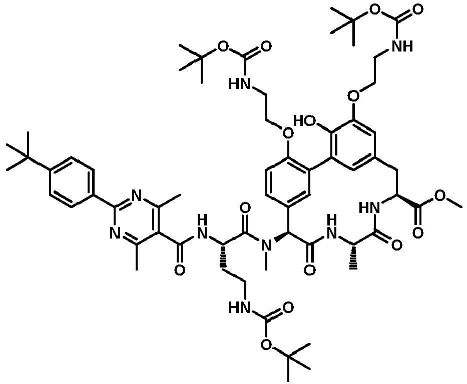

A mixture of methyl (4S,7S,10S)-10-((S)-4-amino-2-(2-(4-(tert-butyl)phenyl)-4,6-dimethylpyrimidine-5-carboxamido)-N-methylbutanamido)-16,25-bis(2-aminoethoxy)-26-hydroxy-7-methyl-6,9-dioxo-5,8-diaza-1,2(1,3)-dibenzenacyclodecaphane-4-carboxylate (0.07589 mmol, 68 mg) suspended in acetone (10 mL) and water (5 mL) was treated with saturated NaHCO₃ in water to pH 9-10. A large excess di-tert-butyl dicarbonate (1.518 mmol, 341.5 mg) was then added and the reaction mixture was stirred at room for 0.5 h until complete conversion was observed. The reaction mixture was diluted with EtOAc and filtered through Celite. The organic layer was concentrated. The residue was purified on silica eluted with 0 to 10% MeOH in DCM to provide the title compound (51.3 mg, 56.5%). (MS+1) m/z 1196.8

### Step 18: (4S,7S,10S)-10-((S)-4-((tert-butoxycarbonyl)amino)-2-(2-(4-(tert-butyl)phenyl)-4,6-dimethylpyrimidine-5-carboxamido)-N-methylbutanamido)-16,25-bis(2-((tert-butoxycarbonyl)amino)ethoxy)-26-hydroxy-7-methyl-6,9-dioxo-5,8-diaza-1,2(1,3)-dibenzenacyclodecaphane-4-carboxylic acid

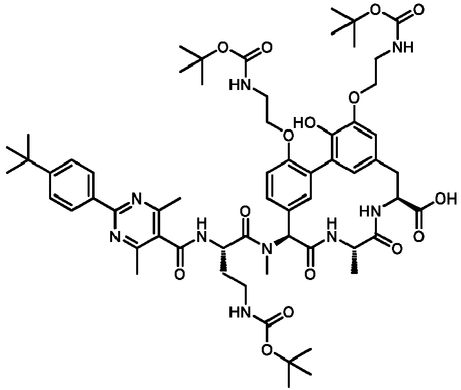

methyl (4S,7S,10S)-10-((S)-4-((tert-butoxycarbonyl)amino)-2-(2-(4-(tert-butyl)phenyl)-4,6-dimethylpyrimidine-5-carboxamido)-N-methylbutanamido)-16,25-bis(2-((tert-butoxycarbonyl)amino)ethoxy)-26-hydroxy-7-methyl-6,9-dioxo-5,8-diaza-1,2(1,3)-dibenzenacyclodecaphane-4-carboxylate (0.0524 mmol, 114 mg) was dissolved in 1,4-dioxane (3 mL) and water (1 mL), and lithium hydroxide (0.5 M, 0.26204 mmol, 0.524 mL) was added dropwise at 0°C and the reaction mixture was stirred at room temperature for 0.5 h. Water (3 mL) was added and the reaction mixture was acidified with 0.5 M HCl to pH 3-4. The resultant white precipitate was extracted with EtOAc, dried with MgSO4, filtered, and concentrated to afford the crude title compound (105 mg, 93%) as a white solid, which was used in the next step without further purification. (MS+1) m/z 1183.5

### Step 19: tert-butyl ((S)-4-(((3S,6S,9S)-15,26-bis(2-((tert-butoxycarbonyl)amino)ethoxy)-9-((cyanomethyl)carbamoyl)-16-hydroxy-6-methyl-4,7-dioxo-5,8-diaza-1,2(1,3)-dibenzenacyclodecaphane-3-yl)(methyl)amino)-3-(2-(4-(tert-butyl)phenyl)-4,6-dimethylpyrimidine-5-carboxamido)-4-oxobutyl)carbamate

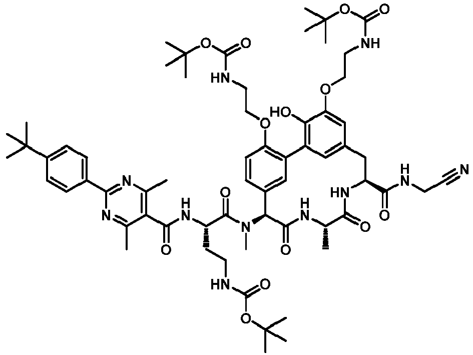

To a mixture of (4S,7S,10S)-10-((S)-4-((tert-butoxycarbonyl)amino)-2-(2-(4-(tert-butyl)phenyl)-4,6-dimethylpyrimidine-5-carboxamido)-N-methylbutanamido)-16,25-bis(2-((tert-butoxycarbonyl)amino)ethoxy)-26-hydroxy-7-methyl-6,9-dioxo-5,8-diaza-1,2(1,3)-dibenzenacyclodecaphane-4-carboxylic acid (0.03721 mmol, 80 mg) and aminoacetonitrile hydrochloride (0.1861 mmol, 17.2 mg) in N,N-dimethylformamide (5 mL) was added N,N-diisopropylethylamine (0.3721 mmol, 48.1 mg, 0.0649 mL) and HATU (0.05582 mmol, 21.7 mg). The reaction was stirred at room temperature for 0.5 h. The reaction mixture was diluted with EtOAc, and washed with water. The organic layer was dried with Na₂SO₄ and concentrated. The residue was purified using silica gel chromatography, eluting with 0 to 10% MeOH in DCM to provide the title compound (36 mg, 63.4%). (MS+1) m/z 1221.8

### Step 20: (4S,7S,10S)-10-((S)-4-amino-2-(2-(4-(tert-butyl)phenyl)-4,6-dimethylpyrimidine-5-carboxamido)-N-methylbutanamido)-16,25-bis(2-aminoethoxy)-N-(cyanomethyl)-26-hydroxy-7-methyl-6,9-dioxo-5,8-diaza-1,2(1,3)-dibenzenacyclodecaphane-4-carboxamide

tert-butyl ((S)-4-(((3S,6S,9S)-15,26-bis(2-((tert-butoxycarbonyl)amino)ethoxy)-9-((cyanomethyl)carbamoyl)-16-hydroxy-6-methyl-4,7-dioxo-5,8-diaza-1,2(1,3)-dibenzenacyclodecaphane-3-yl)(methyl)amino)-3-(2-(4-(tert-butyl)phenyl)-4,6-dimethylpyrimidine-5-carboxamido)-4-oxobutyl)carbamate (0.0236 mmol, 36 mg) was dissolved in TFA (0.1 mL) and 1,1,1,3,3,3-hexafluoro-2-propanol (4.9 mL) and stirred at room temperature for 1.5 h. The reaction mixture was concentrated. To the residue was added Et₂O and re-concentrated. This process was repeated twice more, and the residue was purified by reverse phase HPLC to provide the title compound (23.4 mg, 92.1%) as a white powder. (MS+1) m/z 921.4
¹H NMR (400 MHz, Methanol-d4) δ 8.39 - 8.30 (m, 2H), 7.59 - 7.49 (m, 2H), 7.30 (dd, J = 8.8, 2.4 Hz, 1H), 7.17 (d, J = 8.6 Hz, 1H), 6.96 (d, J = 2.4 Hz, 1H), 6.87 (d, J = 2.0 Hz, 1H), 6.47 (s, 1H), 6.42 (s, 1H), 5.22 (dd, J = 7.5, 6.0 Hz, 1H), 4.83 (d, J = 19.6 Hz, 3H), 4.45 - 4.32 (m, 2H), 4.36 - 4.22 (m, 2H), 4.26 - 4.09 (m, 2H), 3.47 - 3.22 (m, 6H), 3.19 - 3.04 (m, 2H), 2.99 (s, 3H), 2.65 (s, 1H), 2.57(s, 6H), 2.30 (dq, J = 14.1, 7.3 Hz, 1H), 2.16 (dq, J = 14.7, 7.6 Hz, 1H), 1.38 (s, 12H).

### Example 363: Synthesis of Compound 563

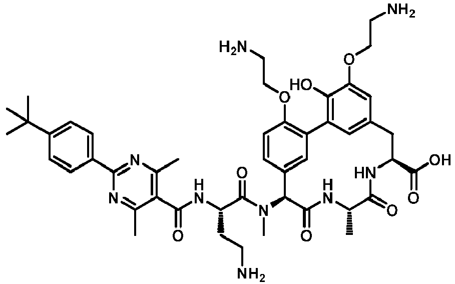

(4S,7S,10S)-10-((S)-4-((tert-butoxycarbonyl)amino)-2-(2-(4-(tert-butyl)phenyl)-4,6-dimethylpyrimidine-5-carboxamido)-N-methylbutanamido)-16,25-bis(2-((tert-butoxycarbonyl)amino)ethoxy)-26-hydroxy-7-methyl-6,9-dioxo-5,8-diaza-1,2(1,3)-dibenzenacyclodecaphane-4-carboxylic acid (0.01269 mmol, 25 mg) was dissolved in TFA (0.1 mL) and 1,1,1,3,3,3-hexafluoro-2-propanol (4.9 mL) and stirred at room temperature for 4 h. The reaction mixture was concentrated and the residue purified by reverse phase HPLC to provide the title compound (12.4 mg, 98.1%) as a white powder. m/z 883.4
¹H NMR (400 MHz, Methanol-d4) δ 8.39 - 8.30 (m, 2H), 7.59 - 7.49 (m, 2H), 7.29 (dd, J = 8.9, 2.5 Hz, 1H), 7.17 (d, J = 8.6 Hz, 1H), 6.95 (s, 1H), 6.50 (s, 1H), 6.43 (s, 1H), 5.22 (t, J = 6.8 Hz, 1H), 4.86 (s, 2H), 4.43 - 4.21 (m, 3H), 3.48 - 3.29 (m, 5H), 3.28 (dd, J = 3.4, 1.7 Hz, 2H), 3.20 - 3.03 (m, 2H), 3.00 (s, 3H), 2.65 (s, 1H), 2.57 (s, 5H), 2.30 (dq, J = 14.1, 7.5, 7.1 Hz, 1H), 2.16 (dq, J = 14.6, 7.6 Hz, 1H), 1.38 (s, 9H), 1.43 - 1.26 (m, 4H).

### Example 364: Synthesis of Compound 564

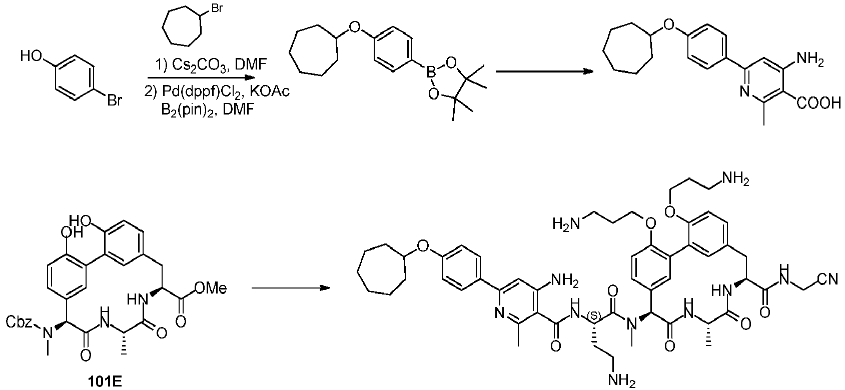

**Step 1:** Starting from 4-bromophenol and bromocycloheptane, S_{N}2 reaction conditions (described in example 44) and Suzuki borylation (described in example 10) were applied to give 2-(4-(cycloheptyloxy)phenyl)-4,4,5,5-tetramethyl-1,3,2-dioxaborolane as colorless oil. ¹H NMR (400 MHz, CDCl₃) δ 7.73(d, J=8.8 Hz, 2H), 6.86(d, J=8.8 Hz, 2H), 4.51-4.45 (m, 1H), 2.05-1.98 (m, 2H), 1.82-1.72 (m, 4H), 1.60-1.57 (m, 4H), 1.51-1.44 (m, 2H), 1.34 (s, 12H).
tert-Butyl (3-bromopropyl)carbamate and 4-amino-6-(4-(cycloheptyloxy)phenyl)-2-methyl nicotinic acid was prepared as a white solid by utilizing methods analogous to those described in example 213. LCMS (Method 5-95 AB, ESI): t_{R} = 0.791 min, [M + H]⁺ = 341.0

**Compound 564** (FA salt) was prepared as a white solid from 101E, tert-butyl (3-bromo propyl)carbamate and 4-amino-6-(4-(cycloheptyloxy)phenyl)-2-methylnicotinic acid by utilizing methods analogous to those described in example 53. LCMS (Method 5-95 AB, ESI): t_{R} = 0.703 min, [M/2 + H]⁺ = 495.1; ¹H NMR (400 MHz, MeOH-*d*₄) δ 8.45 (br s, 1H), 7.68-7.65 (m, 2H), 7.28-7.16 (m, 2H), 7.10-7.07 (m, 1H), 7.03-6.95 (m, 4H), 6.89-6.87 (m, 1H), 6.81-6.72 (m, 1H), 6.43-6.37 (m, 1H), 5.07-5.02 (m, 1H), 4.74-4.71 (m, 1H), 4.59-4.53 (m, 1H), 4.20-4.15 (m, 4H), 4.08-4.02 (m, 2H), 3.20-3.01 (m, 4H), 2.98-2.93 (m, 7H), 2.51 (d, *J* = 3.2 Hz, 3H), 2.21-2.09 (m, 2H), 2.04-1.99 (m, 6H), 1.82-1.70 (m, 4H), 1.62-1.60 (m, 4H), 1.52-1.51 (m, 2H), 1.39-1.31 (m, 3H).

### Example 365: Synthesis of Compound 565

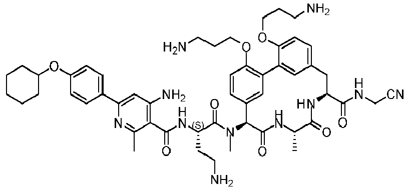

**Compound 565** (FA salt) was prepared as a white solid from **101E** and bromocyclohexane utilizing methods analogous to those described in example 364. LCMS (Method 5-95 AB, ESI): t_{R} = 0.692 min, [M + H]⁺ = 974.5; ¹H NMR (400 MHz, MeOH-*d*₄) δ 8.40 (br s, 1H), 7.64 (d, *J*=8.0 Hz, 2H), 7.26-7.14 (m, 2H), 7.07-6.93 (m, 5H), 6.89 (br s, 1H), 6.78-6.70 (m, 1H), 6.41-6.33 (m, 1H), 5.02-4.99 (m, 1H), 4.72-4.69 (m, 2H), 4.41-4.36 (m, 1H), 4.19-4.13 (m, 4H), 4.04-4.00 (m, 2H), 3.17-3.05 (m, 3H), 2.99-2.90 (m, 7H), 2.51 (br s, 3H), 2.20 (br s, 1H), 2.14-2.11 (m, 1H), 1.98 (br s, 6H), 1.75 (br s, 2H), 1.54-1.20 (m, 9H).

### Example 366: Synthesis of Compound 566

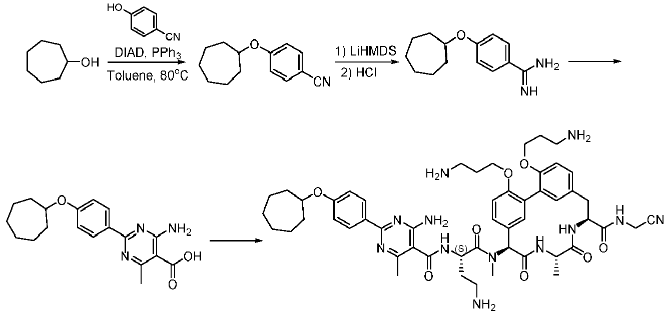

**Step 1:** To a solution of 4-hydroxybenzonitrile (5.00 g, 43.8 mmol), cycloheptanol (11.7 g, 98.5 mmol) and triphenylphosphine (27.6 g, 105 mmol) in toluene (50 mL) was added DIAD (21.3 g, 105 mmol) at 25^{°}C under N₂. The reaction was stirred at 80^{°}C for 2 h. The volatiles were removed and the residue was purified by chromatography on silica, eluting with 0-5% EtOAc in petroleum ether, to afford 4-(cycloheptyloxy)benzonitrile (6.1 g, 65% yield) as a white solid.

**Step 2:** To a solution of 4-(cycloheptyloxy)benzonitrile (3.8 g, 17.6 mmol) in THF (40 mL) was added LiHMDS (1 M in THF, 70.6 mL) at 0°C. The reaction was stirred at 25^{°}C for 40 h. After that, HCl (4 M in H₂O, 60 mL) was added and the mixture was stirred for 10 min, which was then adjusted to pH = 12 using NaOH and extracted with DCM (100 mL). The organic layer was dried over Na₂SO₄ and concentrated to afford 4-(cycloheptyloxy) benzimidamide (2.6 g, 63% yield) as brown oil. 4-Amino-2-(4-(cycloheptyloxy)phenyl)-6-methylpyrimidine-5-carboxylic acid was prepared as a white solid by utilizing methods analogous to those described in example 221 and example 213. LCMS (Method 5-95 AB, ESI): t_{R} = 0.671 min, [M + H]⁺ = 342.5

**Compound 566** (FA salt) was prepared as a white solid from **101E** and 4-Amino-2-(4-(cyclo heptyloxy)phenyl)-6-methylpyrimidine-5-carboxylic acid utilizing methods analogous to those described in example 364. LCMS (Method 5-95 AB, ESI): t_{R} = 0.589 min, [M + H]⁺ = 989.6; ¹H NMR (400 MHz, MeOH-*d*₄) δ 8.53 (br s, 1H), 8.11 (d, *J*=8.0 Hz, 2H), 7.28-7.06 (m, 4H), 6.89 (d, *J* =8.0 Hz, 2H), 6.83 (s, 1H), 6.64-6.61 (m, 2H), 4.79-4.67 (m, 3H), 4.61-4.60 (m, 1H), 4.39-4.09 (m, 6H), 3.28-3.01 (m, 10H), 2.31 (s, 3H), 2.23-2.08 (m, 7H), 1.88-1.80 (m, 5H), 1.75-1.60 (m, 4H), 1.58 (s, 2H), 1.43-1.32 (m, 4H).

### Example 367: Synthesis of Compound 567

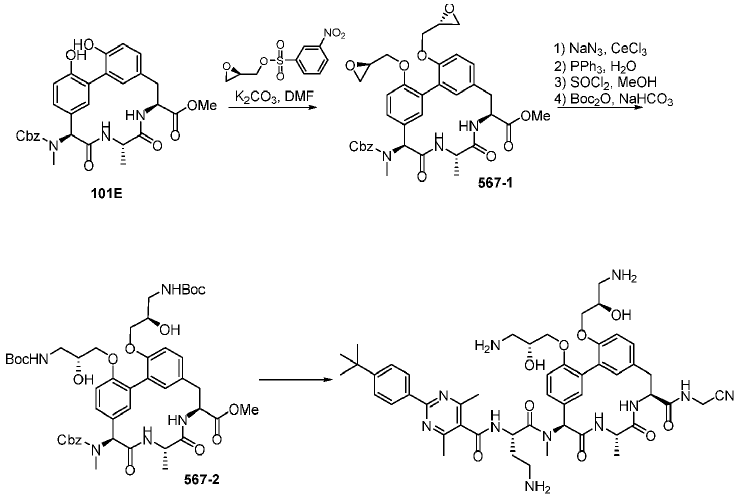

**Step 1:** A solution of **101E** (1.0 g, 1.78 mmol), K₂CO₃ (4.4 g, 32 mmol) and [(2*R*)-oxiran-2- yl] methyl 3-nitrobenzenesulfonate (6.9 g, 26.7 mmol) in DMF (10 mL) was stirred at 50^{°}C for 24 h. The reaction mixture was added with EtOAc (150 mL), which was washed with brine (150 mL x 3), dried over Na₂SO₄, concentrated and the residue was purified by column (2% MeOH in DCM) to give **compound** 567-1 (1.0 g, 83% yield) as a white solid. LCMS (10-80AB_7min, ESI): t_{R} = 3.611 min, [M + Na]⁺ = 696.1

**Step 2:** A solution of **compound 567-1** (1.0 g, 1.48 mmol) in acetonitrile (20 mL) and Water (5 mL) was added CeCl₃ (183 mg, 0.74 mmol) and NaN₃ (1.45 g, 22 mmol) and the mixture was stirred at 75^{°}C for 24 h. Water (100 mL) was added to the above solution and the mixture was stirred for 5 min. The resulting precipitate was collected for next step. The filtrate was treated with aq HCl (1 mol/L) until pH=6, which was extracted with EtOAc (50 mL x 2). The organic layers were combined, dried over Na₂SO₄ and concentrated. The resulting residue together with aforementioned precipitate was treated with PPh₃ (2.0 g, 7.9 mmol), H₂O (0.3 mL) in THF (20 mL) at 50°C for 15 h. The volatiles were removed and the residue was treated with SOCl₂ (230 mg, 2 mmol) in MeOH (6 mL) at 75^{°}C for 1 h. After concentration under vacuum, the residue was dissolved in THF/H2O (20 mL, v/v=7:1), to which NaHCO₃ (415 mg, 4.9 mmol) and Boc₂O (925 mg, 4.2 mmol) were added. The mixture was stirred at 30^{°}C for 2 h. The reaction mixture was added with water (50 mL), which was extracted with EtOAc (100 mL x 2). The combined organic layers were dried over Na₂SO₄, concentrated and the residue was purified by column, eluting with 30% acetone in petroleum ether to remove PPh₃O, then 70% EtOAc in petroleum ether) to give **compound 567-2** (860 mg, 67% yield) as a white solid. LCMS (Method 5-95 AB, ESI): t_{R} = 0.878 min, [M + Na]⁺ = 930.2

**Compound 567** (FA salt) was prepared as a white solid from **Compound 567-2** by utilizing methods analogous to those described in example 53. LCMS (Method 5-95 AB, ESI): t_{R} = 0.708 min, [M + H]⁺ = 964.8; ¹H NMR (400 MHz, MeOH-*d*₄) δ 8.51 (br s, 1H), 8.20 (d, *J*=8.0 Hz, 2H), 7.47 (d, *J*=8.0 Hz, 2H), 7.29-7.15 (m, 2H), 7.02 (s, 1H), 6.80 (s, 1H), 6.59 (s, 1H), 6.50 (s, 1H), 5.36-5.23 (m, 1H), 4.81-4.70 (m, 2H), 4.32-4.04 (m, 7H), 4.02-3.90 (m, 1H), 3.20-3.04 (m, 4H), 3.03-2.87 (m, 6H), 2.46 (s, 6H), 2.31-2.10 (m, 2H), 1.38 (s, 9H), 1.33 (d, J=6.4 Hz, 3H).

### Example 368: Synthesis of Compound 568

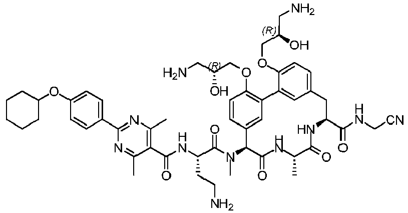

**Compound 568** (FA salt) was prepared as a white solid from 2-(4-(cyclohexyloxy)phenyl) -4,6-dimethylpyrimidine-5-carboxylic acid (described in example 137) by utilizing methods analogous to those described in example 367. LCMS (Method 5-95 AB, ESI): t_{R} = 0.742 min, [M/2 + H]⁺ = 504.0; ¹H NMR (400 MHz, MeOH-*d*₄) δ 8.54 (br s,1H), 8.19-8.01 (m, 2H), 7.22 (br s, 2H), 7.08-6.85 (m, 4H), 6.77 (s, 1H), 6.71 (s, 1H), 6.36 (br s, 1H), 5.40-5.30 (m, 1H), 4.82-4.68 (m, 2H), 4.51-3.86 (m, 10H), 3.28-2.64 (m, 10H), 2.58-2.22 (m, 7H), 2.21-2.08 (m, 1H), 2.07-1.98 (m, 2H), 1.90-1.79 (m, 2H), 1.68-1.26 (m, 9H).

### Example 369: Synthesis of Compound 569

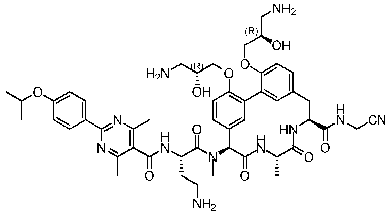

**Compound 569** (FA salt) was prepared as a white solid from 2-(4-isopropoxyphenyl)-4,6-dimethylpyrimidine-5-carboxylic acid by utilizing methods analogous to those described in example 367. LCMS (Method 5-95 AB, ESI): t_{R} = 0.679 min, [M + H]⁺ = 966.5; ¹H NMR (400 MHz, MeOH-*d*₄) δ 8.54 (br s, 1H), 8.10-8.04 (m, 2H), 7.24 (br s, 2H), 7.02 (d, *J*=8.8 Hz, 1H), 6.94-6.87 (m, 3H), 6.76 (s, 2H), 6.30 (s, 1H), 5.38-5.36 (m, 1H), 4.77-4.69 (m, 2H), 4.34-4.16 (m, 7H), 3.98-3.95 (m, 1H), 3.31-3.26 (m, 1H), 3.16-3.06 (m, 4H), 3.01 (s, 3H), 2.95-2.92 (m, 2H), 2.76-2.48 (m, 2H), 2.35 (s, 6H), 2.30-2.12 (m, 2H), 1.38 (d, *J*=6.0 Hz, 6H), 1.33 (d, *J*=6.8 Hz, 3H).

### Example 370: Synthesis of Compound 570

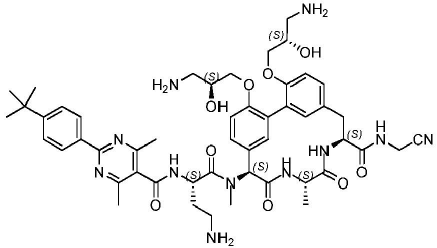

**Compound 570** (FA salt) was prepared as a white solid from 101E and [(2*S*)-oxiran-2- yl] methyl 3-nitrobenzenesulfonate by utilizing methods analogous to those described in example 367. LCMS (Method 5-95 AB, ESI): t_{R} = 0.720 min, [M + H]⁺ = 964.6; ¹H NMR (400 MHz, MeOH-*d*₄) δ 8.53 (br s, 1H), 8.14 (d, *J*=8.0 Hz, 2H), 7.45 (d, *J*=8.0 Hz, 2H), 7.27-7.16 (m, 2H), 7.06-6.92 (m, 2H), 6.78 (s, 1H), 6.67 (s, 1H), 6.41 (s, 1H), 5.41-5.18 (m, 1H), 4.83-4.70 (m, 2H), 4.41-3.90 (m, 9H), 3.23-2.82 (m, 10H), 2.43 (s, 6H), 2.33-2.08 (m, 2H), 1.39 (s, 9H), 1.33 (d, *J*=6.4 Hz, 3H).

### Example 371: Synthesis of Compound 571

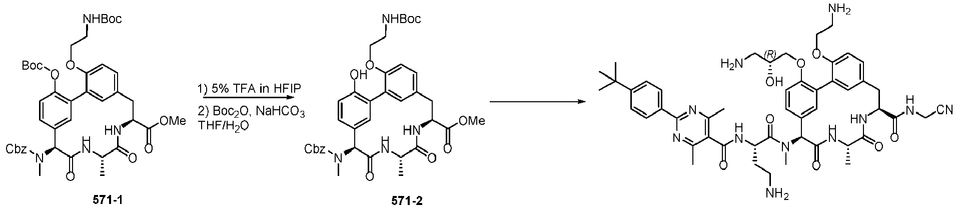

Step 1: A solution of **compound 571-1** (from example V (compound 106-B1), 300 mg, 0.37 mmol) in 10% TFA in DCM (30 mL) was stirred at 25^{°}C for 5 h. The volatiles were removed and the residue was re-dissolved in THF (12 mL), to which Boc₂O (93 mg, 0.43 mmol) was added. The resulting mixture was added with sat. NaHCO₃ solution until pH=8 and the reaction was stirred at 25^{°}C for 2 h. The reaction was added with water (30 mL), which was extracted with EtOAc (30 mL x 3). The combined organic layers were washed with brine (50 mL x 2), dried over Na₂SO₄, concentrated and the residue was purified by prep-TLC (10% MeOH in DCM, Rf =0.4) to get **compound 571-2** (251 mg, 96% yield) as a white solid. LCMS (Method 5-95 AB, ESI): t_{R} = 0.907 min, [M + Na]⁺ = 727.5

**Compound 571** (FA salt) was prepared as a white solid from **compound 571-2** by utilizing methods analogous to those described in example 367. LCMS (Method 10-80 AB, ESI, 7 min): t_{R} = 1.756 min, [M + H]⁺ = 935.6; ¹H NMR (400 MHz, MeOH-*d*₄) δ 8.52 (br s, 2H), 8.28-8.14 (m, 2H), 7.50-7.45 (m, 2H), 7.32-7.18 (m, 2H), 7.10-6.96 (m, 2H), 6.83 (s, 1H), 6.65-6.40 (m, 2H), 5.30 (m, 1H), 4.85-4.77 (m, 2H), 4.46-4.29 (m, 3H), 4.25-4.00 (m, 4H), 3.30-3.26 (m, 2H), 3.14 (br s, 3H), 3.07-3.01 (m, 3H), 3.00-2.86 (m, 3H), 2.45 (s, 6H), 2.35-2.23 (m, 1H), 2.22-2.09 (m, 1H), 1.39 (s, 9H), 1.34 (d, *J*=6.0 Hz, 3H).

### Example 372: Synthesis of Compound 572

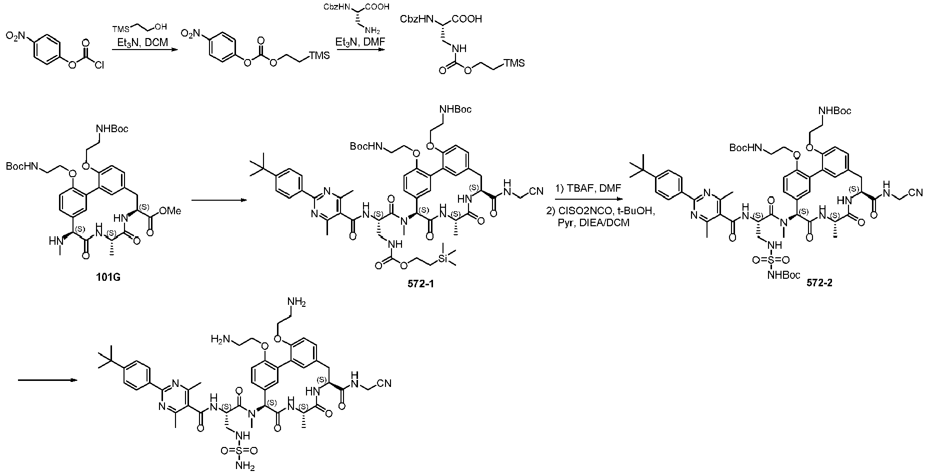

**Step 1:** To a solution of 2-(trimethylsilyl)ethanol (7.0 g, 60 mmol) and Et₃N (10 g, 100 mmol) in DCM (15 mL) was added 4-nitro-phenylchloroformate (10 g, 50 mmol) and the mixture was stirred at 30^{°}C for 1 h. The reaction was quenched with water (50 mL), and then extracted with DCM (50 mL x 3). The combined organic layers were dried with Na₂SO₄, concentrated and the residue was purified by column, eluting with 2% EtOAc in petroleum ether, to give (4-nitrophenyl) 2-trimethylsilylethyl carbonate (10 g, 71% yield) as colorless oil.

**Step 2:** To a solution of (4-nitrophenyl) 2-trimethylsilylethyl carbonate (5.0 g, 17.6 mmol) in DMF (45 mL) was added (2*S*)-3-amino-2-(benzyloxycarbonylamino)propanoic acid (3.5 g, 14.7 mmol) and Et₃N (3.0 g, 29.4 mmol) at 25^{°}C and the mixture was stirred at the same temperature for 3 h. The volatiles were removed and the residue was partitioned between DCM and H₂O (200 mL each). The organic layer was washed with brine (100 mL x 2), dried over Na₂SO₄, concentrated and the residue was purified by silica-gel column, eluting with 10%-30% MeOH in DCM to give (2S)-2-(benzyloxycarbonylamino)-3-(2-trimethylsilyl ethoxycarbonylamino)propanoic acid (3.4 g, 61% yield) as a white solid.

**Compound 572-1** was prepared as a white solid by utilizing methods analogous to those described in example 53. LCMS (Method 5-95 AB, ESI): t_{R} = 1.146 min, [M + H]⁺ = 1235.4.

**Step 3:** To a solution of **compound 572-1** (120 mg, 0.097 mmol) in DMF (2 mL) was added TBAF (1 M in THF, 40 µL) at 20^{°}C and the reaction was stirred at 50^{°}C for 2h. The mixture was diluted with water (20 mL), which was extracted with EtOAc (20 mL x 2). The combined organic layers were washed with brine (40 mL), dried over Na₂SO₄ and concentrated to have INT-A.

Another solution of chlorosulfonyl isocyanate (17 mg, 0.11 mmol) in DCM (2 mL) at 0^{°}C was added t-BuOH (13 µL, 0.11 mmol) and the mixture was stirred for 30 min, followed by the addition of pyridine (26 µL, 0.22 mmol). The resulting mixture was stirred for 40min during which time precipitate formed, which was added via pipette to a mixture of INT-A and DIEA (40 mg, 0.30 mmol) in DCM (2 mL) at 0^{°}C. The reaction was stirred at 0^{°}C for 0.5 h. After that, the mixture was diluted with DCM (10 mL), which was washed with brine (10 mL). The organic layer was dried over Na₂SO₄, concentrated and the residue was purified by prep-TLC (10% MeOH in DCM) to get **compound 572-2** (40 mg, 33% yield)

**Compound 572** (FA salt) was prepared as a white solid from **compound 572-2** by utilizing methods analogous to those described in example 53. LCMS (Method 5-95 AB, ESI): t_{R} = 0.756 min, [M + H]⁺ = 969.7; ¹H NMR (400 MHz, MeOH-*d*₄) δ 8.54 (br s, 2H), 8.01 (d, *J*=8.0 Hz, 2H), 7.37 (d, *J*=8.0 Hz, 2H), 7.28-7.21 (m, 2H), 7.05 (d, J*=*8.0 Hz, 1H), 6.90-9.84 (m, 2H), 6.76 (s, 1H), 6.27 (s, 1H), 5.51-5.47 (m, 1H), 4.76-4.71 (m, 1H), 4.43 (s, 1H), 4.29-4.20 (m, 5H), 4.09 (s, 1H), 3.56-3.51 (m, 1H), 3.40-3.34 (m, 2H), 3.21 (s, 2H), 3.09 (s, 3H), 2.87-2.77 (m, 1H), 2.61-2.53 (m, 1H), 2.35 (s, 6H), 1.39 (s, 9H), 1.33 (d, *J*=6.4 Hz, 3H).

### Example 373: Synthesis of Compound 573

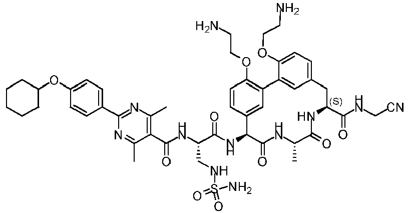

**Compound 573** (FA salt) was prepared as a white solid from 2-(4-(cyclohexyloxy)phenyl) -4,6-dimethylpyrimidine-5-carboxylic acid (described in example 137) by utilizing methods analogous to those described in example 372. LCMS (Method 5-95 AB, ESI): t_{R} = 0.801 min, [M + H]⁺ = 997.4; ¹H NMR (400 MHz, DMSO-*d*₆) δ 8.40 (br s, 3H), 8.12 (d, *J*=8.0 Hz, 1H), 7.28-7.15 (m, 2H), 7.06-7.04 (m, 3H), 7.00-6.94 (m, 2H), 6.74-6.64 (m, 3H), 5.76-5.72 (m, 1H), 4.93-4.60 (m, 2H), 4.45-4.35 (m, 2H), 4.20-4.16 (m, 2H), 4.05 (s, 5H), 3.77 (s, 12H), 2.97 (m, 4H), 2.42 (s, 2H),1.94 (s, 2H), 1.74-1.72 (m, 2H), 1.56-1.36 (m, 6H), 1.25-1.21 (m, 3H).

### Example 374: Synthesis of Compound 574

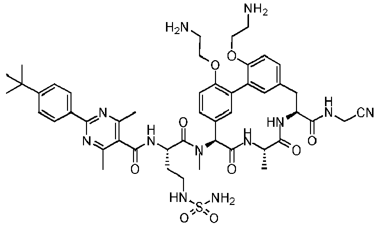

**Compound 574** (FA salt) was prepared as a white solid from (*S*)-4-amino-2-(((benzyloxy) carbonyl) amino) butanoic acid by utilizing methods analogous to those described in example 372. LCMS (Method 5-95 AB, ESI): t_{R} = 0.782 min, [M + H]⁺ = 983.7; ¹H NMR (400 MHz, MeOH-*d*₄) δ 8.51 (br s, 1H), 8.12-8.06 (m, 2H), 7.43 (d, *J*=8.4 Hz, 2H), 7.27-7.23 (m, 2H), 7.07 (d, *J*=8.4 Hz, 1H), 7.00-6.96 (m, 1H), 6.83 (s, 1H), 6.81 (s, 1H), 6.40 (s, 1H), 5.33-5.32 (m, 1H), 4.90-4.85 (m, 1H), 4.78-4.73 (m, 1H), 4.45-4.41 (m, 2H), 4.32-4.22 (m, 4H), 3.35 (m, 3H), 3.27-3.17 (m, 4H), 3.07 (m, 3H), 2.91 (m, 1H), 2.43 (s, 6H), 2.04-1.98 (m, 1H), 2.07-1.95 (m, 1H), 1.39 (s, 9H), 1.34 (d, *J*=6.8 Hz, 3H).

### Example 375: Synthesis of Compound 575

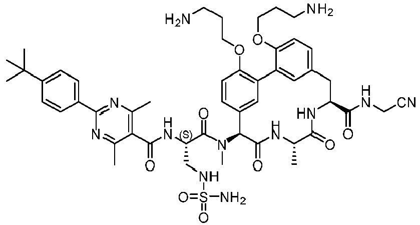

**Compound 575** (FA salt) was prepared as a white solid from **101E** and tert-butyl (3-bromo propyl)carbamate by utilizing methods analogous to those described in example 372. LCMS (Method 5-95 AB, ESI): t_{R} = 0.772 min, [M + H]⁺ = 997.6; ¹H NMR (400 MHz, MeOH-*d*₄) δ 8.42 (br s, 1H), 7.99 (d, *J*=8.0Hz, 2H), 7.35 (d, *J*=8.4Hz, 2H), 7.27-7.12 (m, 2H), 7.07-6.93 (m, 2H), 6.87-6.75 (m, 1H), 6.69 (s, 1H), 6.19 (br s, 1H), 5.58-5.48 (m, 1H), 4.78-4.62 (m, 1H), 4.50-4.15(m, 5H), 4.13-3.77 (m, 2H), 3.60-3.47 (m, 1H), 3.41-3.32 (m, 1H), 3.28-3.16 (m, 2H), 3.15-2.93 (m, 5H), 2.83-2.68 (m, 1H), 2.60-2.46 (m, 1H), 2.31 (s, 6H), 2.20-1.90 (m, 4H), 1.39 (s, 9H), 1.34 (d, *J*=6.8 Hz, 3H).

### Example 376: Synthesis of Compound 576

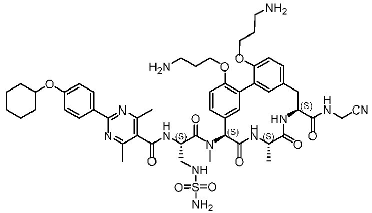

**Compound 576** (FA salt) was prepared as a white solid from **101E,** tert-butyl (3-bromo propyl)carbamate and 2-(4-(cyclohexyloxy)phenyl) -4,6-dimethylpyrimidine-5-carboxylic acid (described in example 137) by utilizing methods analogous to those described in example 372. LCMS (Method 5-95 AB, ESI): t_{R} = 0.771 min, [M + H]⁺ = 1039.9; ¹H NMR (400 MHz, MeOH-*d*₄) δ 8.41 (br s, 3H), 8.11 (d, *J*=6.4 Hz, 2H), 7.22 (br s, 2H), 7.11-6.95 (m, 3H), 6.90 (d, *J*=8.4 Hz, 2H), 6.77 (s, 1H), 6.40 (br s, 1H), 5.52-5.45 (m, 1H), 4.84-4.74 (m, 2H), 4.48-4.24 (m, 6H), 4.13-4.05 (m, 1H), 3.61-3.49 (m, 1H), 3.42-3.35 (m, 1H), 3.22-2.97 (m, 8H), 2.96-2.85 (m, 1H), 2.39 (s, 6H), 2.22 (br s, 2H), 2.09-2.02 (m, 4H), 1.89-1.80 (m, 2H), 1.69-1.44 (m, 6H), 1.38 (d, *J*=6.8 Hz, 3H).

### Example 377: Synthesis of Compound 577

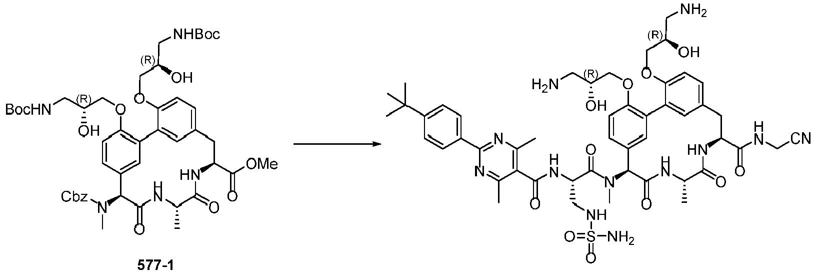

**Compound 577** (FA salt) was prepared as a white solid from **compound 577-1** (synthesis described in example 367) by utilizing methods analogous to those described in example 372. LCMS (Method 10-80 AB, ESI, 7 min): t_{R} = 2.329 min, [M + H]⁺ = 1029.4; ¹H NMR (400 MHz, MeOH-*d*₄) δ 8.54 (br s, 1H), 7.98 (d, *J*=8.4 Hz, 2H), 7.34 (d, *J* =8.4 Hz, 2H), 7.27-7.09 (m, 2H), 6.99 (d, *J* =8.8 Hz, 2H), 6.87-6.75 (m, 1H), 6.69 (s, 1H), 6.16 (br s, 1H), 5.52-5.95 (m, 1H), 4.55-4.52 (m, 1H), 4.45-4.10 (m, 8H), 4.00-3.70 (m, 2H), 3.60-3.45 (m, 1H), 3.40-3.25 (m, 5H), 3.17-3.08 (m, 2H), 3.00-2.90 (m, 1H), 2.81-2.65 (m, 1H), 2.30 (s, 6H), 1.40 (s, 9H), 1.34 (d, *J*=6.4 Hz, 3H).

### Example 378: Synthesis of Compound 578

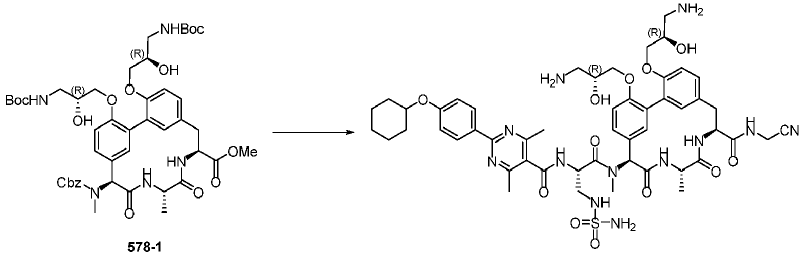

**Compound 578** (FA salt) was prepared as a white solid from **compound 578-1** (synthesis described in example 367) and 2-(4-(cyclohexyloxy)phenyl) -4,6-dimethylpyrimidine-5- carboxylic acid (described in example 137) by utilizing methods analogous to those described in example 372. LCMS (Method 5-95 AB, ESI): t_{R} = 0.776 min, [M + H]⁺ = 1072.0; ¹H NMR (400 MHz, MeOH-*d*₄) δ 8.54 (br s, 1H), 8.08-7.88 (m, 2H), 7.29-7.11 (m, 2H), 7.08-6.80 (m, 5H), 6.72 (s, 1H), 6.24 (br s, 1H), 5.55-5.46 (m, 1H), 4.83-4.62 (m, 2H), 4.49-3.83 (m, 10H), 3.59-3.48 (m, 1H), 3.41-3.34 (m, 1H), 3.30-3.18 (m, 1H), 3.16-2.71 (m, 7H), 2.54-2.15 (m, 6H), 2.09-1.98 (m, 2H), 1.90-1.80 (m, 2H), 1.70-1.31 (m, 9H).

### Example 379: Synthesis of Compound 579

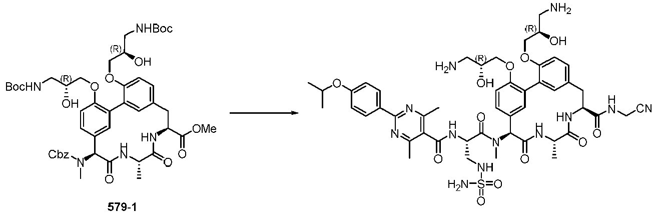

**Compound 579** (FA salt) was prepared as a white solid from **compound 579-1** (synthesis described in example 367) and 2-(4-isopropoxyphenyl)-4,6- dimethylpyrimidine-5-carboxylic acid by utilizing methods analogous to those described in example 372. LCMS (Method 5-95 AB, ESI): t_{R} = 0.582 min, [M + H]⁺ = 1031.4; ¹H NMR (400 MHz, MeOH-*d*₄) δ 8.54 (br s, 1H), 8.14-7.94 (m, 2H), 7.26-7.16 (m, 2H), 7.06-7.01 (m, 1H), 7.01-6.90 (m, 2H), 6.86 (d, *J*=8.4 Hz, 2H), 6.75 (br s, 1H), 6.34 (s, 1H), 5.54-5.45 (m, 1H), 4.78-4.67 (m, 2H), 4.38-4.08 (m, 8H), 4.00-3.92 (m, 1H), 3.58-3.50 (m, 1H), 3.41-3.33 (m, 2H), 3.30-3.22 (m, 1H), 3.14-2.99 (m, 5H), 2.95-2.81 (m, 2H), 2.34 (s, 6H), 1.39 (d, *J*=5.6 Hz, 6H), 1.37 (d, *J*=6.4 Hz, 3H)

### Example 380: Synthesis of Compound 580

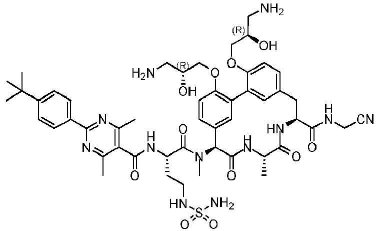

**Compound 580** (FA salt) was prepared as a white solid from (*S*)-4-amino-2-(((benzyloxy) carbonyl) amino) butanoic acid by utilizing methods analogous to those described in example 377. LCMS (Method 5-95 AB, ESI): t_{R} = 0.776 min, [M + H]⁺ = 1043.8; ¹H NMR (400 MHz, MeOH-*d*₄) δ 8.55 (s, 1H), 8.08 (d, *J*=6.8 Hz, 2H), 7.40 (d, *J*=8.0 Hz, 2H), 7.26-7.14 (m, 2H), 7.05-6.82 (m, 3H), 6.75 (s, 1H), 6.28 (br s, 1H), 5.41-5.32 (m, 1H), 4.77-4.67 (m, 1H), 4.42-3.88 (m, 10H), 3.28-3.15 (m, 3H), 3.12-2.98 (m, 5H), 2.95-2.74 (m, 2H), 2.38 (s, 6H), 2.26-2.15 (m, 1H), 2.08-1.95 (m, 1H), 1.39 (s, 9H), 1.32 (d, *J*=6.8 Hz, 3H).

### Example 381: Synthesis of Compound 581

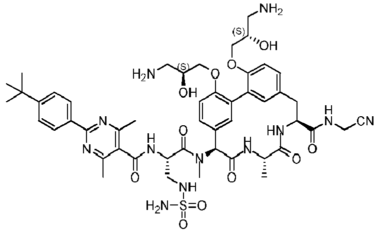

**Compound 581** (TFA salt) was prepared as a white solid from [(2*S*)-oxiran-2- yl] methyl 3-nitrobenzenesulfonate by utilizing methods analogous to those described in example 377. LCMS (Method 5-95 AB, ESI): t_{R} = 0.772 min, [M + H]⁺ = 1029.3; ¹H NMR (400 MHz, MeOH-*d*₄) δ 7.98 (d, *J*=8.0 Hz, 2H), 7.36 (d, *J=*8.0 Hz, 2H), 7.25 (d, *J*=8.8 Hz, 1H), 7.16 (d, *J*=8.8 Hz, 1H), 7.05-6.95 (m, 2H), 6.89-6.79 (m, 1H), 6.70 (br s, 1H), 6.19 (br s, 1H), 5.58-5.48 (m, 1H), 4.83-4.61 (m, 2H), 4.44-3.99 (m, 8H), 3.59-3.48 (m, 1H), 3.41-3.31 (m, 2H), 3.29-3.14 (m, 2H), 3.10 (s, 3H), 3.08-3.02 (m, 1H), 3.00-2.92 (m, 1H), 2.82-2.69 (m, 1H), 2.32 (s, 6H), 1.40 (s, 9H), 1.36(d, *J*=7.2 Hz, 3H).

### Example 382: Synthesis of Compound 582

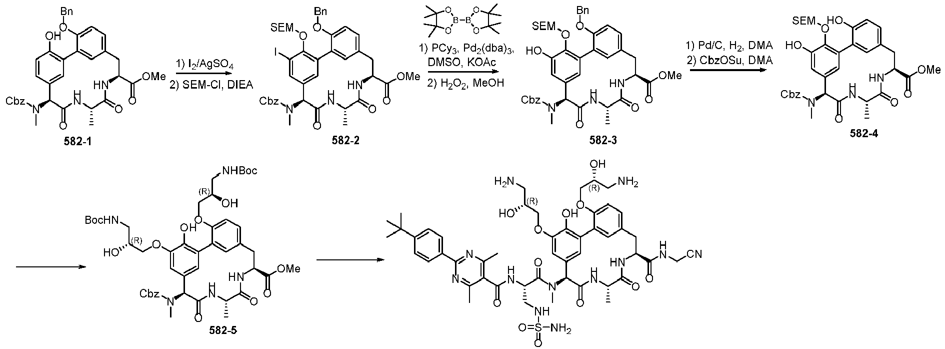

Step 1: To a solution of **compound 582-1** (from example V (compound 106-A2), 13.0 g, 20 mmol) in MeOH (100 mL), AgSO₄ (4.35 g, 14.0 mmol) and I₂ (5.57 g, 22 mmol) was added at 20°C and the reaction mixture was stirred for at 20^{°}C for 2 h. After filtration, the filtrate was concentrated and the residue was purified by silica-gel column, eluting with 5% MeOH in DCM. The resulting material was dissolved in DCM (130 mL), to which DIEA (6.5 g, 50 mmol) and SEM-Cl (4.45 mL, 25 mmol) was added. The reaction was stirred for 4h at 25^{°}C. The mixture was added with DCM (300 mL), which was washed with saturated NH₄Cl solution and brine (250 mL each). The organic layer was dried over Na₂SO₄, concentrated and the residue was purified by silica-gel column to give **compound 582-2** (15 g, 83% yield) as a off-white solid. LCMS (Method 5-95 AB, ESI): t_{R} = 1.088 min, [M + Na]⁺ = 930.0

Step 2: A mixture of KOAc (7.57 g, 77 mmol), **compound 582-2** (10.0 g, 11 mmol), Pin₂B₂ (14.0 g, 55 mmol), Pd₂(dba)₃ (504 mg, 0.55 mmol) and PCy₃ (309 mg, 1.1 mmol) in DMSO (150 mL) was stirred at 80^{°}C overnight under N₂. The mixture was diluted with EtOAc (500 mL), which was washed with saturated NaHCO₃ solution and brine (each 500 mL). The organic layer was dried over Na₂SO₄, concentrated and the residue was purified by silica-gel column, eluting with 5% MeOH in DCM. The resulting material was re-dissolved in MeOH (100 mL), to which was added H₂O₂ (30% w/w, 20 mL). The reaction was stirred for 6 h at 30^{°}C. The mixture was diluted with EtOAc (200 mL), which was washed with water and brine (100 mL each). The organic layer was dried over Na₂SO₄, concentrated and the residue was purified by silica-gel column, eluting with 5% MeOH in DCM, to obtain the desire product **compound 582-3** (7.2 g, 9.0 mmol, 82% yield) as a white solid. LCMS (Method 5-95 AB, ESI): t_{R} = 0.996 min, [M + Na]⁺ = 820.0

Step 3: A solution of **compound 582-3** (2.4 g, 3.0 mmol) and 10% Pd/C (1.6 g, 1.5 mmol) in DMA (20 mL) was stirred under H₂ (50 psi) at 50^{°}C for 20 h. After filtration, the filtrate was added with CbzOSu (749 mg, 3.0 mmol) and the resulting mixture was stirred at 25^{°}C for 1 h. After that, the mixture was added with EtOAc (120 mL), which was washed with brine (100 mL x 3). The organic layer was dried over Na₂SO₄, concentrated and the residue was purified by silica-gel column, eluting with 50% EtOAc in petroleum ether, to give **compound 582-4** (1.9 g, 90% yield) as a white solid. LCMS (Method 5-95 AB, ESI): t_{R} = 0.904 min, [M + Na]⁺ = 730.2

**Compound 582-4** was prepared as a white solid by utilizing methods analogous to those described in example 367 (SEM was removed under methyl ester formation condition). LCMS (Method 5-95 AB, ESI): t_{R} = 0.863 min, [M + Na]⁺ = 946.3

**Compound 582** (TFA salt) was prepared by utilizing methods analogous to those described in example 377 (over-acylation on un-protected phenol occurred during each amide coupling step, which can be converted back to the desired product by treating with 3% ammonia in MeOH for 1 h). LCMS (Method 10-80 AB, ESI, 7 min): t_{R} = 2.331 min, [M + H]⁺= 1045.6; 1H NMR (400MHz, DMSO-*d6*, T=80^{°}C) δ 8.84-8.58 (m, 2H), 8.52 (br s, 1H), 8.32 (d, *J*=7.6 Hz, 2H), 8.07 (d, *J*=8.4 Hz, 1H), 7.54 (d, *J*=7.6 Hz, 2H), 7.18 (d, *J*=8.4 Hz, 1H), 7.02 (d, *J*=8.4 Hz, 1H), 6.83 (br s, 1H), 6.77 (br s, 1H), 6.46 (br s, 1H), 6.34 (s, 1H), 5.20-5.09 (m, 1H), 4.81-4.68 (m, 2H), 4.22-4.06 (m, 8H), 3.45-3.35 (m, 1H), 3.26-3.15 (m, 2H), 3.10-2.92 (m, 6H), 2.88-2.72 (m, 2H), 2.53 (s, 6H), 1.35 (s, 9H), 1.24 (d, *J*=6.0 Hz, 3H)

### Example 383: Synthesis of Compound 583

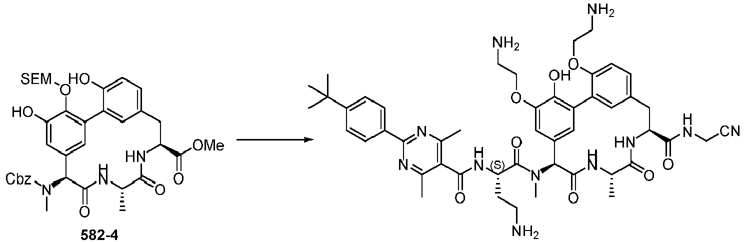

**Compound 583** (TFA salt) was prepared from **compound 582-4** (described in example 382) by utilizing methods analogous to those described in example 53. LCMS (Method 5-95 AB, ESI): t_{R} = 0.706 min, [M + H]⁺ = 920.4; ¹H NMR (400 MHz, MeOH-*d*₄) δ 8.35 (d, *J*=8.4 Hz, 2H), 7.55 (d, *J*=8.4 Hz, 2H), 7.23 (d, *J*=7.6 Hz, 1H), 7.08 (d, *J*=8.0 Hz, 1H), 6.91 (s, 1H), 6.84 (s, 1H), 6.58 (s, 1H), 6.42 (s, 1H), 5.25-5.15 (m, 1H), 4.85-4.80 (m, 2H), 4.48-4.15 (m, 6H), 3.45-3.35 (m, 4H), 3.27-3.07 (m, 4H), 3.07-3.00 (m, 3H), 2.59 (s, 6H), 2.35-2.25 (m, 1H), 2.25-2.15 (m, 1H), 1.40 (s, 9H), 1.38 (d, *J*=7.2 Hz, 3H).

### Example 384: Synthesis of Compound 584

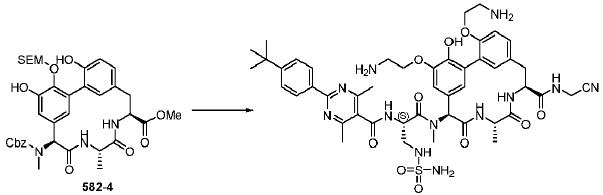

**Compound 584** (TFA salt) was prepared from **compound 582-4** (described in example 382) by utilizing methods analogous to those described in example 372. LCMS (Method 5-95 AB, ESI): t_{R} = 0.767 min, [M + H]⁺ = 985.6; ¹H NMR (400 MHz, MeOH-*d*₄) δ 8.34-8.16 (m, 2H), 7.54-7.26 (m, 2H), 7.12-7.05 (m, 2H), 6.85 (s, 1H), 6.71-6.37 (m, 3H), 5.33 (m, 1H), 4.80-4.78 (m, 2H), 4.38-4.20 (m, 6H), 3.61-3.38 (m, 6H), 3.20-2.90 (m, 2H), 3.02 (s, 3H), 2.53 (s, 6H), 1.36 (s, 9H), 1.27 (d, *J*=6.8 Hz, 3H).

### Example 385: Synthesis of Compound 585

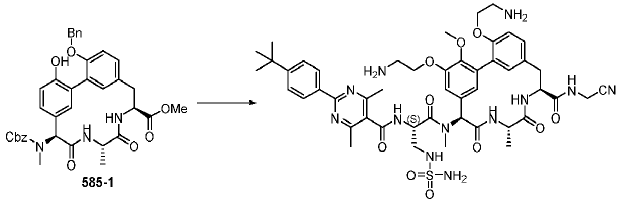

**Compound 585** (FA salt) was prepared from **compound 585-1** (from example V (compound 106-A2), iodomethane, and tert-butyl (2-bromoethyl)carbamate, by utilizing methods analogous to those described in example 382. LCMS (Method 5-95 AB, ESI): t_{R} = 0.758 min, [M + Na]⁺ = 1021.1; ¹H NMR (400 MHz, DMSO-*d*₄) δ 8.51 (s, 2H), 8.32 (d, *J*=8.4 Hz, 2H), 7.54 (d, *J*=8.4 Hz, 2H), 7.09-7.00 (m, 3H), 6.54 (br s, 1H), 6.52 (br s, 1H), 6.39 (s, 1H), 5.24-5.08 (m, 1H), 4.88-4.67 (m, 2H), 4.39-4.20 (m, 7H), 3.91 (s, 3H), 3.68-3.31 (m, 6H), 3.19 (s, 3H), 2.94-2.79 (m, 2H), 2.45 (s, 6H), 1.36 (s, 9H), 1.23 (d, *J*=6.8 Hz, 3H).

### Example 386: Synthesis of Compound 586

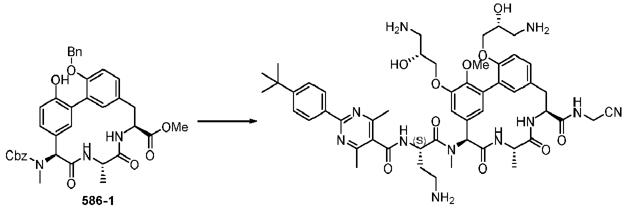

**Compound 586** (FA salt) was prepared from **compound 586-1** (from example V (compound 106-A2), iodomethane, and (S)-3-(((benzyloxy)carbonyl)amino)-5-((tert-butoxycarbonyl) amino)-2-oxopentanoic acid, by utilizing methods analogous to those described in example 382. LCMS (Method 5-95 AB, ESI): t_{R} = 0.749 min, [M + Na]⁺ = 1016.3; ¹H NMR (400 MHz, MeOH-*d*₄) δ 8.50 (br s, 2H), 8.29 (d, *J*=8.8 Hz, 2H), 7.51 (d, *J*=8.8 Hz, 2H), 7.06 (d, *J*=8.4 Hz, 2H), 6.75 (s, 1H), 6.58 (s, 1H), 6.56 (s, 1H), 5.23-5.21(m, 1H), 4.82-4.77 (m, 1H), 4.26-4.04 (m, 8H), 3.80 (s, 3H), 3.29-3.00 (m, 8H), 3.07 (s, 3H), 2.55 (s, 6H), 2.33-2.17 (m, 2H), 1.40 (s, 9H), 1.38 (d, *J*=7.2 Hz, 3H).

### Example 387: Synthesis of Compound 587

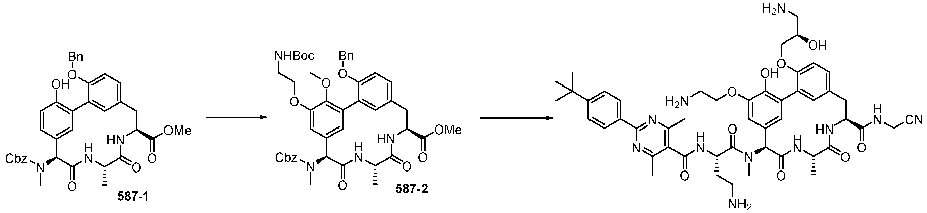

**Compound 587-2** was prepared from **compound 587-1** ((from example V (compound 106-A2)), iodomethane and tert-butyl (2-bromoethyl)carbamate, by utilizing methods analogous to those described in example 382. LCMS (Method 5-95 AB, ESI): t_{R} = 0.854 min, [M + Na]⁺ = 847.2
**Compound 587** (FA salt) was prepared from **compound 587-2** and (S)-3-(((benzyloxy) carbonyl)amino)-5-((tert-butoxycarbonyl)amino)-2-oxopentanoic acid, by utilizing methods analogous to those described in example 382. LCMS (Method 5-95 AB, ESI): t_{R} = 0.706 min, [M + H]⁺ = 964.3; ¹H NMR (400 MHz, MeOH-*d*₄) δ 8.49 (br s, 2H), 8.35 (d, *J*=8.4 Hz, 2H), 7.54 (d, *J*=8.4 Hz, 2H), 7.28-7.21 (m, 1H), 7.08-6.96 (m, 2H), 6.86 (s, 1H), 6.62-6.55 (m, 1H), 6.43 (s, 1H), 5.20-5.09 (m, 1H), 4.84-4.72 (m, 2H), 4.38-4.02 (m, 7H), 3.66 (s, 3H), 3.37-3.31 (m, 3H), 3.21-2.95 (m, 8H), 2.59 (s, 6H), 2.33-2.07 (m, 2H), 1.38 (s, 9H), 1.36 (d, *J*=6.8 Hz, 3H).

### Example 388: Synthesis of Compound 588

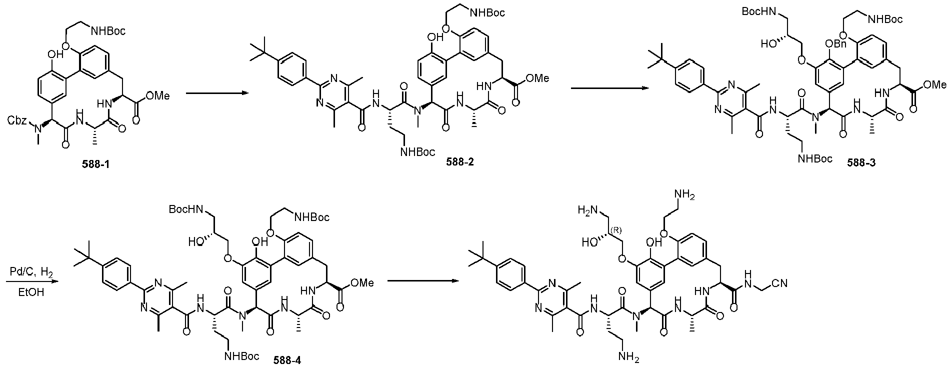

**Compound 588-2** was prepared from **compound 588-1** ((from example V (compound 106-B1)), by utilizing methods analogous to those described in example 54. LCMS (Method 5-95 AB, ESI): t_{R}= 1.042 min, [M + Na]⁺ = 1059.1

**Compound 588-3** was prepared from **compound 588-2** and benzyl bromide (synthesis described in example 391), by utilizing methods analogous to those described in example 382. LCMS (Method 5-95 AB, ESI): t_{R} = 1.083 min, [M + Na]⁺ = 1338.6
**Compound 588-4** was prepared hydrogenation of **compound 588-3** (described in example 414). LCMS (Method 5-95 AB, ESI): t_{R} = 1.017 min, [M + Na]⁺ = 1249.6

**Compound 588** (FA salt) was prepared by utilizing methods analogous to those described in example 382. LCMS (Method 5-95 AB, ESI): t_{R} = 0.704 min, [M + H]⁺ = 950.6; ¹H NMR (400 MHz, MeOH-*d*₄) δ 8.50 (br s, 2H), 8.31 (d, *J*=7.6 Hz, 2H), 7.53 (d, *J*=8.0 Hz, 2H), 7.16 (s, 1H), 7.07 (d, *J*=8.8 Hz, 1H), 6.83 (s, 1H), 6.77 (s, 1H), 6.54 (s, 2H), 5.22-5.21 (t, *J*=6.4 Hz, 1H), 4.81 (q, *J*=6.4 Hz, 1H), 4.41-4.34 (m, 2H), 4.25 (s, 3H), 4.07-4.05 (m, 2H), 3.43-3.34 (m, 2H), 3.22-3.05 (m, 9H), 2.56 (s, 6H), 2.32-2.17 (m, 2H), 1.40 (s, 9H), 1.38 (d, *J*=6.8 Hz, 3H).

### Example 389: Synthesis of Compound 589

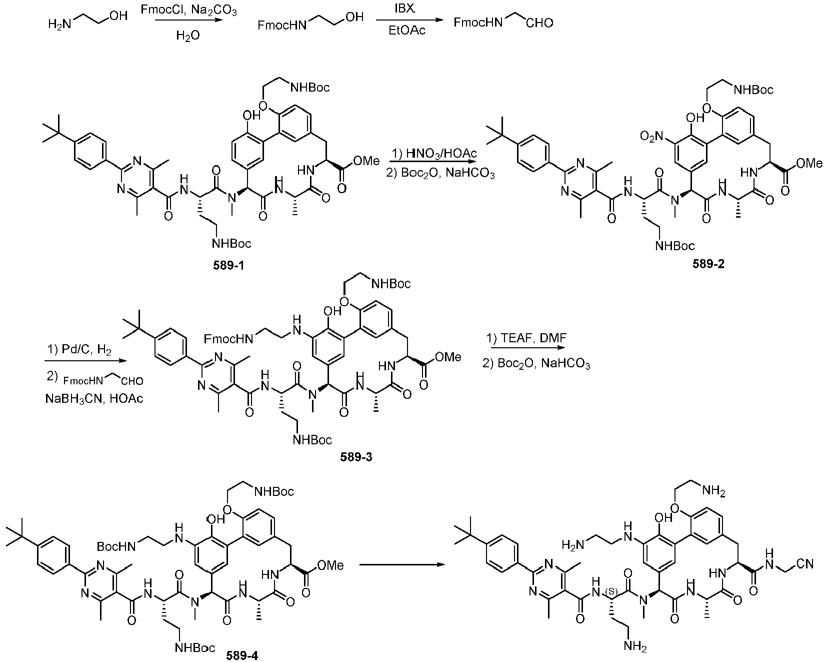

**Step 1**: To a solution of 2-aminoethanol (2.0 g, 32.7 mmol) and Na₂CO₃ (10.4 g, 98.2 mmol) in H₂O (35 mL) was added FmocCl (9.3 g, 36.0 mmol) at 20 C and the mixture was stirred at the same temperature for 2 h. The mixture was extracted with DCM (100 mL × 3). The combined organic layers were dried over Na₂SO₄, concentrated and the residue was purified by silica-gel column, eluting with 50% EtOAc in petroleum ether, to give (9H-fluoren-9-yl) methyl (2-hydroxyethyl)carbamate (5.6 g, 60.4% yield) as a white solid.

**Step 2:** A solution of (9H-fluoren-9-yl) methyl (2-hydroxyethyl)carbamate (1.0 g, 3.5 mmol) and 2-iodoxybenzoic acid (3.0 g, 10.5 mmol) in EtOAc (100mL) was stirred at 80°C under N₂ for 16 h. After filtration, the filtrate was concentrated to afford (9H-fluoren-9-yl)methyl (2-oxoethyl)carbamate (1.0 g, quantitative yield) as a white solid. ¹H NMR (400 MHz, CDCl₃) δ 9.67 (s, 1H), 7.78 (d, *J*=8.0 Hz, 2H), 7.60 (d, *J*=8.0 Hz, 2H), 7.43-7.39 (m, 2H), 7.34-7.31 (m, 2H), 5.50-5.44 (m, 1H), 4.43 (d, *J*=6.8 Hz, 2H), 4.24 (t, *J*=6.8 Hz, 1H), 4.20-4.00 (m, 2H).

Step 3: A solution of compound **589-1** (synthesis described in example 388, 200 mg, 0.19 mmol) in HOAc (2 mL) was added fume nitric acid (30 µL) at 0°C. The mixture was gradually warmed up to 25°C while stirring and stirred at the same temperature for 3.5 h. The above mixture was added with saturated NaHCO₃ solution (40 mL), Boc₂O (84 mg, 0.39 mmol) and THF (12 mL) and the resulting mixture was stirred at 25 C for 1.5 h. The mixture was extracted with EtOAc (30 mL × 3). The combined organic layers were washed with brine (50 mL × 2), dried over Na₂SO₄, concentrated and the residue was purified on silica-gel column, eluting with 5% MeOH in DCM, to afford **compound 589-2** (140 mg, 67% yield) as a yellow solid. LCMS (Method 5-95 AB, ESI): t_{R} = 1.131 min, [M + H]⁺ = 1082.7

Step 4: A solution of **compound 589-2** (140 mg, 0.13 mmol) and 10% Pd/C (134 mg, 0.13 mmol) in Ethanol (10mL) was stirred at 30°C under H₂ (15 psi) for 1 h. After filtration, the filtrate was concentrated and the residue was re-dissolved in MeOH (4 mL), to which (9H-fluoren-9-yl)methyl (2-oxoethyl)carbamate (43 mg, 0.15 mmol) and HOAc (50 µL) were added. The resulting mixture was stirred at 25°C for 6 h. After that, the mixture was added with EtOAc (40 mL), which was washed with brine (50 mL), dried over Na₂SO₄ and concentrated. The residue was purified by pre-TLC, eluting with 7%MeOH in DCM, to give **compound 589-3** (140 mg, 83% yield) as a white solid. LCMS (Method 5-95 AB, ESI): t_{R} = 1.136 min, [M + H]⁺ = 1318.2

Step 5: A solution of **compound 589-3** (140 mg, 0.11 mmol) and TEAF·4H₂O (47 mg, 0.21 mmol) in DMF (2 mL) was stirred at 30°C for 30 min. The mixture was added with EtOAc (30 mL), which was washed with brine (30 mL), dried over Na₂SO₄ and concentrated. The resulting residue was re-disssolved in THF (3 mL), to which Boc2O (24 mg, 0.12 mmol) and saturated NaHCO₃ (2 mL) was added. The resulting mixture was stirred at 25°C for 1 h. The reaction was partitioned between EtOAc and water (50 mL each) and the organic layer was washed with brine (40 mL), dried over Na₂SO₄ and concentrated. The residue was purified by pre-TLC, eluting with 5% MeOH in DCM, to afford **compound 589-4** (50 mg, 38% yield) as a white solid. LCMS (Method 5-95 AB, ESI): t_{R} = 1.052 min, [M + H]⁺ = 1195.4

**Compound 589** (TFA salt) was prepared by utilizing methods analogous to those described in example 382. LCMS (Method 5-95 AB, ESI): t_{R} = 0.733 min, [M + H]⁺ = 919.6; ¹H NMR (400 MHz, MeOH-*d*₄) δ 8.29 (d, *J*=8.0 Hz, 2H), 7.51 (d, *J*=8.8 Hz, 2H), 7.18 (d, *J*=8.8 Hz, 1H), 7.04 (d, *J*=8.8 Hz, 1H), 6.91 (s, 1H), 6.52 (s, 1H), 6.41 (s, 1H), 6.21 (s, 1H), 5.15-5.12 (m, 1H), 4.80-4.76 (m, 2H), 4.31-4.26 (m, 2H), 4.19 (s, 2H), 3.45-3.43 (m, 2H), 3.30-3.28 (m, 3H), 3.15-3.09 (m, 5H), 3.02 (s, 3H), 2.55 (s, 6H), 2.26-2.12 (m, 2H), 1.36 (s, 9H), 1.34 (d, *J*=6.8 Hz, 3H).

### Example 390: Synthesis of Compound 590

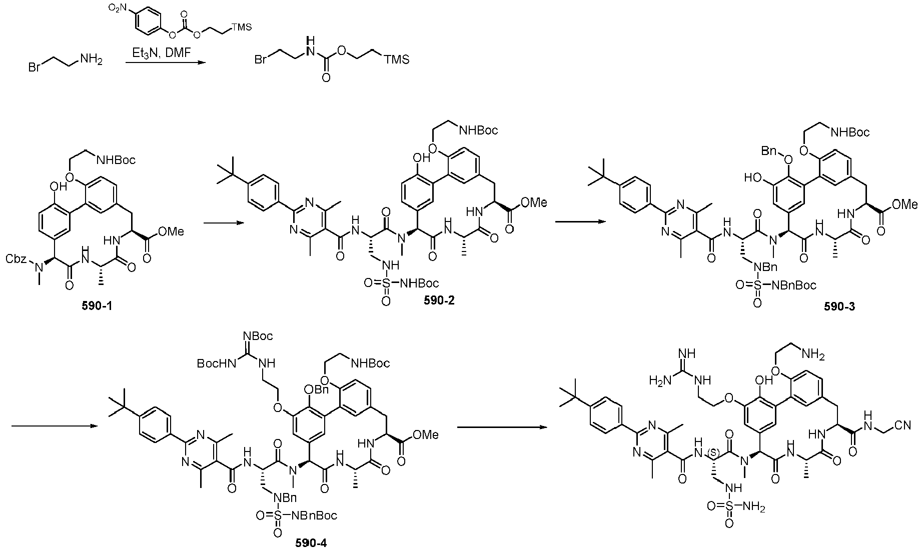

2-(trimethylsilyl)ethyl (2-bromoethyl)carbamate was prepared from 2-bromoethanamine and 4-nitrophenyl (2-(trimethylsilyl)ethyl) carbonate (synthesis described in example 372) as pale-yellow oil by applying the same method described in example 372.
**Compound 590-2** was prepared from **compound 590-1** ((from example V (compound 106-B1)) as a white solid by utilizing methods analogous to those described in example 372. LCMS (Method 5-95 AB, ESI): t_{R} = 0.973 min, [M + H]⁺ = 1124.1

**Compound 590-3** was prepared from **compound 590-2** and benzyl bromide (synthesis described in example 391) as a white solid by utilizing methods analogous to those described in example 382. LCMS (Method 5-95 AB, ESI): t_{R} = 1.224 min, [M + H]⁺ = 1388.8

**Compound 590-4** was prepared from **compound 590-3** and 2-(trimethylsilyl)ethyl (2-bromoethyl)carbamate as a white solid by utilizing methods analogous to those described in example 395. LCMS (Method 5-95 AB, ESI): t_{R} = 1.252 min, [M/2 + H]⁺ = 837.9

**Compound 590** (TFA salt) was prepared by utilizing methods analogous to those described in example 388. LCMS (Method 5-95 AB, ESI): t_{R} = 0.769 min, [M + H]⁺ = 1027.7; ¹H NMR (400 MHz, MeOH-*d*₄) δ 8.38-8.18 (m, 2H), 7.57-7.43 (m, 2H), 7.28-6.99 (m, 3H), 6.86 (br s, 1H), 6.71 (br s, 1H), 6.52 (s, 1H), 5.37-5.26 (m, 1H), 4.83-4.56 (m, 2H), 4.37 (s, 2H), 4.30-4.02 (m, 3H), 3.70-3.45 (m, 3H), 3.43-3.32 (m, 2H), 3.18-2.96 (m, 1H), 3.08 (s, 3H), 2.52 (s, 6H), 1.40 (s, 9H), 1.38 (d, *J*=6.8 Hz, 3H).

### Example 391: Synthesis of Compound 591

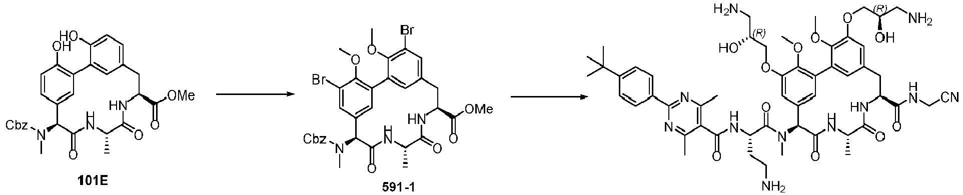

Step 1: A solution of NBS (634 mg, 3.56 mmol) and **101E** (1.0 g, 1.78 mmol) in DCM (5 mL) was stirred at 30°C for 3h. The volatiles were removed and the residue was purified by silica-gel column, eluting with 5% MeOH in DCM. The resulting residue was re-dissolved in DMF (6 mL), to which K₂CO₃ (1.15 g, 8.34 mmol) and iodomethane (0.7 mL, 11.3 mmol) were added. The reaction was stirred at 25°C for 16 h. The mixture was added with EtOAc (60 mL), followed by the filtration. The filtrate was washed with brine (50 mL × 3), dried over Na₂SO₄, concentrated and the residue was purified by silica-gel column, eluting with 40% EtOAc in petroleum ether, to give **compound 591-1** (1.2 g, 96% yield) as a white solid. LCMS (Method 5-95 AB, ESI): t_{R} = 1.023 min, [M + H]⁺ = 746.0.

**Compound 591** (TFA salt) was prepared from **compound 591-1** and (S)-3-(((benzyloxy) carbonyl)amino)-5-((tert-butoxycarbonyl)amino)-2-oxopentanoic acid, by utilizing methods analogous to those described in example 382. LCMS (Method 5-95 AB, ESI): t_{R} = 0.696 min, [M + H]⁺ = 1024.8; ¹H NMR (400 MHz, MeOH-*d*₄) δ 8.31 (d, *J*=8.4 Hz, 2H), 7.51 (d, *J*=8.4 Hz, 2H), 6.92 (d, *J*=7.6 Hz, 2H), 6.52 (s, 1H), 6.42 (s, 2H), 5.20-5.10 (m, 1H), 4.78-4.72 (m, 2H), 4.28-4.16 (m, 8H), 3.78-3.66 (m, 3H), 3.30-3.20 (m, 3H), 3.19-3.00 (m, 11H), 2.56 (s, 6H), 2.25-2.15 (m, 1H), 2.15-2.05 (m, 1H), 1.36 (s, 9H), 1.33 (d, *J*=6.8 Hz, 3H).

### Example 392: Synthesis of Compound 592

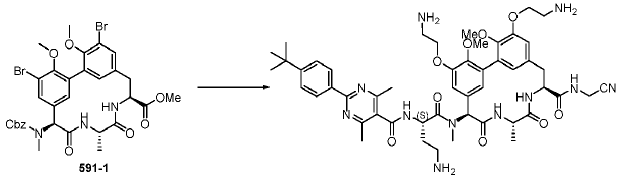

**Compound 592** (FA salt) was prepared from **compound 591-1** (synthesis described in example 391) and tert-butyl (2-bromoethyl)carbamate, by utilizing methods analogous to those described in example 391. LCMS (Method 5-95 AB, ESI): t_{R} = 0.703 min, [M + Na]⁺ = 986.6; ¹H NMR (400 MHz, MeOH-*d*₄) δ 8.50 (br s, 3H), 8.33 (d, *J*=8.8 Hz, 2H), 7.52 (d, *J*=8.8 Hz, 2H), 7.00 (s, 1H), 6.96 (s, 1H), 6.57 (br s, 1H), 6.49 (br s, 1H), 6.41 (s, 1H), 5.14-5.11 (m, 1H), 4.77-4.75 (m, 2H), 4.32-4.19 (m, 6H), 3.73 (s, 3H), 3.69 (s, 3H), 3.39-3.34 (m, 6H), 3.14-3.05 (m, 2H), 3.01 (s, 3H), 2.57 (s, 6H), 2.26-2.12 (m, 2H), 1.36 (s, 9H), 1.33 (d, *J*=6.8 Hz, 3H).

### Example 393: Synthesis of Compound 593

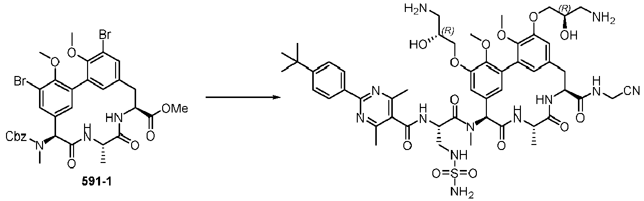

**Compound 593** (FA salt) was prepared from **compound 591-1** (synthesis described in example 391) by utilizing methods analogous to those described in example 391. LCMS (Method 5-95 AB, ESI): t_{R} = 0.736 min, [M + H]⁺ = 1089.6; ¹H NMR (400 MHz, MeOH-*d*₄) δ 8.51 (br s, 1H), 8.18-8.12 (m, 2H), 7.44-7.38 (m, 2H), 6.86 (br s, 1H), 6.71 (br s, 2H), 6.44 (s, 1H), 6.14 (s, 1H), 5.39-5.30 (m, 1H), 4.79-4.67 (m, 2H), 4.25-4.03 (m, 8H), 4.02-3.88 (m, 4H), 3.76 (s, 3H), 3.59-3.50 (m, 1H), 3.39-3.30 (m, 2H), 3.29-3.20 (m, 2H), 3.11-3.00 (m, 2H), 3.05 (s, 3H), 2.41 (s, 6H), 1.36 (s, 9H), 1.35 (d, *J*=6.4 Hz, 3H)

### Example 394: Synthesis of Compound 594

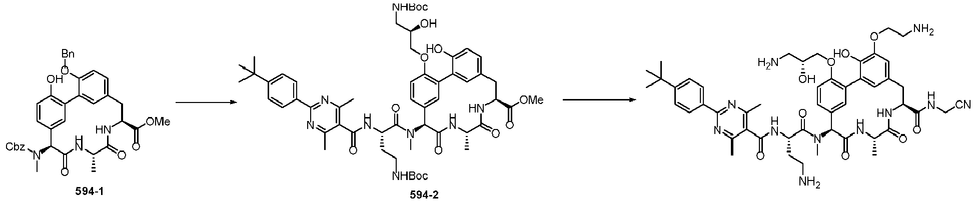

**Compound 594-2** was prepared from **compound 594-1** (from example V (compound 106-A2)) by utilizing methods analogous to those described in example 395. LCMS (Method 5-95 AB, ESI): t_{R} = 0.950 min, [M + H]⁺ = 1067.5

**Compound 594** (FA salt) was prepared from **compound 594-2** and tert-butyl (2-bromoethyl) carbamate by utilizing methods analogous to those described in example 382. LCMS (Method 5-95 AB, ESI): t_{R} = 0.729 min, [M + H]⁺ = 950.4; ¹H NMR (400 MHz, MeOH-*d*₄) δ 8.56 (br s, 2H), 8.35 (d, *J*=8.0 Hz, 2H), 7.56 (d, *J*=8.0 Hz, 2H), 7.29 (d, *J*=8.4 Hz, 1H), 7.16 (d, *J*=8.4 Hz, 1H), 6.95 (d, *J*=1.2 Hz, 1H), 6.85 (s, 1H), 6.46 (s, 1H), 6.41 (s, 2H), 5.26-5.22 (m, 1H), 4.84-4.77 (m, 2H), 4.30-4.10 (m, 7H), 3.50 - 3.40 (m, 2H), 3.25-3.05 (m, 6H), 3.01 (s, 3H), 2.58 (s, 6H), 2.30-2.27 (m, 1H), 2.18-2.14 (m, 1H), 1.36 (s, 9H), 1.34 (d, *J*=6.8 Hz, 3H).

### Example 395: Synthesis of Compound 595

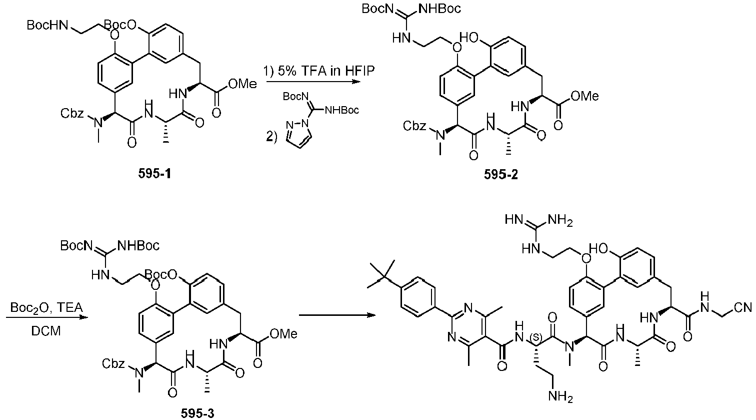

Step 1: A solution of **compound 595-1** (from example V (compound 106-B2), 300 mg, 0.37 mmol) in 5% TFA in HFIP (15 mL) was stirred at room temperature for 1 h. The mixture was concentrated and the residue was re-dissolved in THF (8 mL), which was added tert-butyl (((tert-butoxycarbonyl)amino) (1H-pyrazol-1 -yl)methylene)carbamate (158 mg, 0.51 mmol). The resulting mixture was stirred at room temperature for another 3 h. The reaction was added with water (15 mL), which was extracted with EtOAc (15 mL × 3). The combined organic layers were washed with brine (30 mL), dried with Na₂SO₄, concentrated and purified by column (5% MeOH/DCM) to give **Compound 595-2** (280 mg, 97.5% yield over two steps) as a white solid. LCMS (Method 5-95 AB, ESI): t_{R} = 0.825 min, [M + H]⁺ = 847.1

Step 2: To a solution of **Compound 595-2** (280 mg, 0.33 mmol) in DCM (15 mL), Et₃N (100 mg, 0.99 mmol) and Boc₂O (2.16 g, 9.92 mmol) was added at room temperature and the reaction was stirred for 72 hours at the same temperature. The reaction was added with water (15 mL), which was extracted with EtOAc (15 mL × 3). The combined organic layers were washed with brine (30 mL), dried with Na₂SO₄, concentrated and purified by column (5% MeOH/DCM) to give **Compound 595-3** (300 mg, 96% yield) as a white solid. LCMS (Method 5-95 AB, ESI): t_{R} = 0.875 min, [M + H]⁺ = 947.4

**Compound 595** (TFA salt) was prepared as a white solid from **Compound 595-3** by utilizing methods analogous to those described in example 54. LCMS (Method 5-95 AB, ESI): t_{R} = 0.749 min, [M + H]⁺ = 903.6; ¹H NMR (400 MHz, MeOH-*d4*) δ 8.30 (d, *J*=8.4 Hz, 2H), 7.51 (d, *J*=8.0 Hz, 2H), 7.27 (d, *J*=8.4 Hz, 1H), 7.17 (d, *J*=8.4 Hz, 1H), 6.92-6.86 (m, 2H), 6.85 (d, *J*=8.4 Hz, 1H), 6.62 (br s, 1H), 6.41 (s, 1H), 5.35-5.26 (m, 1H), 4.83-4.78 (m, 1H), 4.62 (br s, 1H), 4.42-4.32 (m, 2H), 4.30-4.23 (m, 2H), 3.74-3.61 (m, 2H), 3.14 (t, *J*=8.0 Hz, 2H), 3.05-2.72 (m, 5H), 2.44 (s, 6H), 2.29-2.10 (m, 2H), 1.38 (s, 9H), 1.34(d, *J*=6.4Hz, 3H).

### Example 396: Synthesis of Compound 596

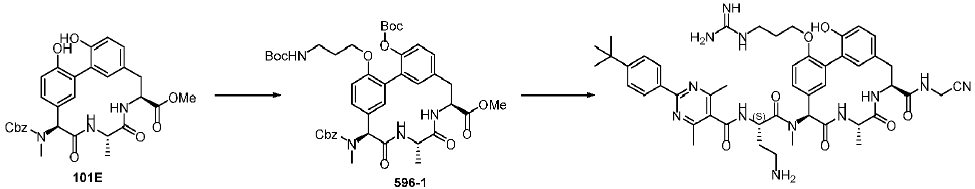

**Compound 596-1** was prepared as a white solid from **101E** and tert-butyl (3-bromopropyl) carbamateby utilizing methods analogous to those described in example 54.

**Compound 596** (FA salt) was prepared as a white solid from **Compound 596-1** by utilizing methods analogous to those described in example 395. LCMS (Method 5-95 AB, ESI): t_{R} = 0.634 min, [M + H]⁺ = 917.5; ¹H NMR (400MHz,MeOH-*d4*) δ 8.52 (br s, 1H), 8.37-8.23 (m, 2H), 7.57-7.46 (m, 2H), 8.33-8.22 (m, 1H), 7.20-6.89 (m, 3H), 6.87-6.75 (m, 1H), 6.62 (br s, 1H), 6.50 (s, 1H), 5.30-5.18 (m, 1H), 4.83-4.79 (m, 2H), 4.33-4.01 (m, 4H), 3.54-3.35 (m, 2H), 3.26-2.91 (m, 7H), 2.56-2.49 (s, 6H), 2.35-1.95 (m, 4H), 1.42 (s, 9H), 1.38 (d, *J*=6.8 Hz, 3H).

### Example 397: Synthesis of Compound 597

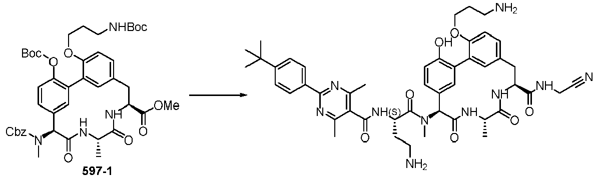

**Compound 597** (FA salt) was prepared as a white solid from **Compound 597-1** (side product during the synthesis of **compound 596-1** in example 396 synthesis) by utilizing methods analogous to those described in example 54. LCMS (Method 5-95 AB, ESI): t_{R} = 0.758min, [M + H]⁺ = 875.4; ¹H NMR (400 MHz, MeOH-*d4*) δ 8.50 (br s, 2H), 8.19 (d, *J*=8.0 Hz, 2H), 7.47 (d, *J*=8.0 Hz, 2H), 7.11-7.06 (m, 1H), 7.05-6.95 (m, 3H), 6.79 (br s, 1H), 6.64 (br s, 1H), 6.54 (s, 1H), 5.35-5.29 (m, 1H), 4.82-4.75 (m, 2H), 4.35-4.25 (m, 3H), 4.20-4.10 (m, 1H), 3.27-3.10 (m, 5H), 3.11(s, 3H), 3.05-2.90 (m, 1H), 2.45 (s, 6H), 2.35-2.25 (m, 1H), 2.20-2.10 (m, 3H), 1.39 (s, 9H), 1.35 (d, *J*=7.2Hz, 3H).

### Example 398: Synthesis of Compound 598

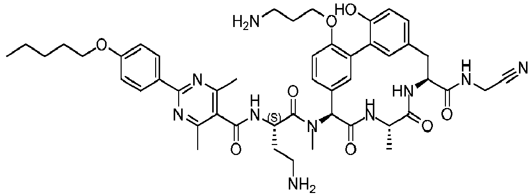

**Compound 598** (FA salt) was prepared as a white solid from 4,6-dimethyl-2-(4-(pentyloxy) phenyl)pyrimidine-5-carboxylic acid (described in example 128) by utilizing methods analogous to those described in example 396 and example 54. LCMS (Method 5-95 AB, ESI): t_{R} = 0.777min, [M + H]⁺ = 905.8; ¹H NMR (400 MHz, MeOH-*d4*) δ 8.52 (br s, 2H), 8.28 (d, *J*=8.4 Hz, 2H), 7.60-7.25 (m, 2H), 7.05-7.00 (m, 1H), 6.98 (d, *J*=8.4Hz, 2H), 6.91 (d, *J*=2.4 Hz, 1H), 6.83 (br s, 1H), 6.62 (br s, 1H), 6.51 (s, 1H), 5.30-5.20 (m, 1H), 4.82-4.75 (m, 2H), 4.41-4.29 (m, 2H), 4.22 (s, 2H), 4.06 (t, *J*= 6.4 Hz, 2H), 3.27-2.95 (m, 9H), 2.49 (s, 6H), 2.35-2.24 (m, 1H), 2.20-2.10 (m, 3H), 1.90-1.80 (m, 2H), 1.55-1.40 (m, 4H), 1.35 (d, *J*=6.4 Hz, 3H), 0.98 (t, *J*=6.8 Hz, 3H).

### Example 399: Synthesis of Compound 599

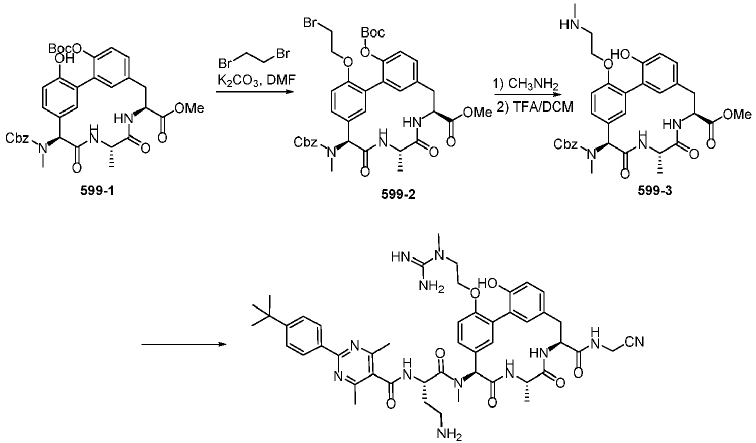

Step 1: A solution of **compound 599-1** (from example V (compound 106-A2), 300 mg, 0.45 mmol), 1,2-dibromoethane (852 mg, 4.5 mmol) and K₂CO₃ (627 mg, 4.5 mmol) in DMF (10 mL) was stirred at 25°C for 12 h. The reaction was taken up in EtOAc (100 mL), which was washed brine (50 mL x3), dried over Na2SO4, concentrated and the residue was purified by prep-TLC (10% MeOH in DCM) to give **compound 599-2** (275 mg, 79% yield) as a white solid.

Step 2: A solution of **compound 599-2** (275 mg, 0.36 mmol), methylamine (2M in THF, 1.8 mL), K₂CO₃ (494 mg, 3.6 mmol) in DMF (10 mL) was stirred at 25°C for 12 h. The reaction mixture was taken up in EtOAc (100 ml), which was washed with brine (50 mL × 2), dried over Na2SO4, concentrated. The residue was treated with 10% TFA/DCM (10 mL) for 1 h. The volatiles were removed and the residue was purified by prep-TLC to give compound **599-3** (160 mg, 72% yield) as a off-white solid. LCMS (Method 5-95 AB, ESI): t_{R} = 0.745 min, [M + H]⁺ = 619.6

The title compound (FA salt) was prepared as white solids from **compound 599-3** by utilizing methods analogous to those described in example 395. LCMS (Method 5-95 AB, ESI): t_{R} = 0.758 min, [M + H]⁺ = 917.4; ¹H NMR (400 MHz, MeOH-d*4*) δ 8.54 (br s, 1H), 8.22 (d, *J*=8.0Hz, 2H), 7.50 (d, *J*=8.0Hz, 2H), 7.29-7.15 (m, 2H), 6.96-6.80 (m, 3H), 6.57 (s, 1H), 6.53 (s, 1H), 5.30-5.26 (m, 1H), 4.81-4.60 (m, 2H), 4.46-4.24 (m, 2H), 4.24 (s, 2H), 3.87-3.74(m, 2H), 3.15-2.89 (m, 10H), 2.49 (s, 6H), 2.30-2.12 (m, 2H), 1.39 (s, 9H), 1.36-1.20 (m, 3H).

### Example 400: Synthesis of Compound 600

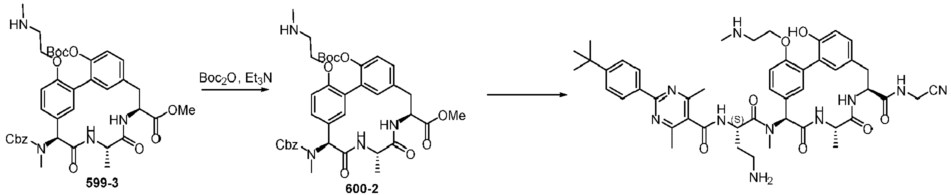

Step 1: A solution of **compound 599-3** (synthesis described in example 399, 210 mg, 0.3 mmol), Boc₂O (273 mg, 1.25 mmol) and Et₃N (127 mg, 1.25 mmol) in DCM (20 mL) was stirred at 25°C for 12 h. The volatiles were removed and the residue was taken up in EtOAc (30 mL), which was washed with brine (30 mL × 2). The organic layer was dried over Na₂SO₄, concentrated and the residue was purified by prep-TLC (10% MeOH in DCM) to give **compound 600-2** (143 mg, 70% yield) as a white solid.

**Compound 600** (FA salt) was prepared as a white solid from **compound 600-2** by utilizing methods analogous to those described in example 54. LCMS (Method 5-95 AB, ESI): t_{R} = 0.740min, [M + H]⁺ = 875.4; ¹H NMR (400 MHz,MeOH-*d*4) δ 8.52 (br s, 1H), 8.10 (d, *J*=7.6Hz, 2H), 7.43 (d, *J*=7.6 Hz, 2H), 7.30-7.19 (m, 2H), 7.06-6.74 (m, 3H), 6.73 (br s, 1H), 6.32 (s, 1H), 5.40-5.31 (m, 1H), 4.82-4.65 (m, 2H), 4.53 (s, 2H), 4.35-4.18 (m, 2H), 3.52-3.44 (m, 2H), 3.14 (t, *J*=7.6 Hz, 2H), 3.01 (s, 3H), 2.95-2.84 (m, 1H), 2.80 (s, 3H), 2.66-2.52 (m, 1H), 2.41 (s, 6H), 2.31-2.13 (m, 1H), 1.39 (s, 9H), 1.34 (d, *J*=6.4Hz, 3H).

### Example 401: Synthesis of Compound 601

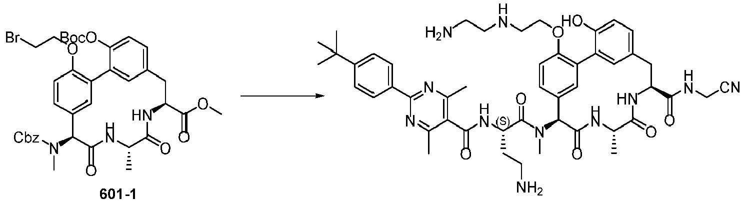

**Compound 601** (FA salt) was prepared as a white solid from **compound 601-1** (described in example 399) and tert-butyl (2-aminoethyl)carbamate by utilizing methods analogous to those described in example 399 and example 600. LCMS (Method 5-95 AB, ESI): t_{R} = 0.716min, [M + H]⁺ = 904.5; ¹H NMR (400 MHz, MeOH-*d4*) δ 8.49 (br s, 1H), 8.24 (d, *J*=8.0 Hz, 1H), 8.10 (d, *J*=8.0 Hz, 1H), 7.53-7.41 (m, 2H), 7.26-7.15 (m, 2H), 7.10-6.99 (m, 1H), 6.85-6.74 (m, 2H), 6.67 (br s, 1H), 6.34 (s, 1H), 5.33-5.23 (m, 1H), 4.79-4.76 (m, 2H), 4.38-4.30 (m, 2H), 4.26-4.18 (m, 2H), 3.17-3.03 (m, 5H), 2.99-2.88 (m, 6H), 2.51 (s, 3H), 2.39 (s, 3H), 2.30-2.13 (m, 2H), 1.40 (s, 9H), 1.37 (d, J=6.4 Hz, 3H).

### Example 402: Synthesis of Compound 602

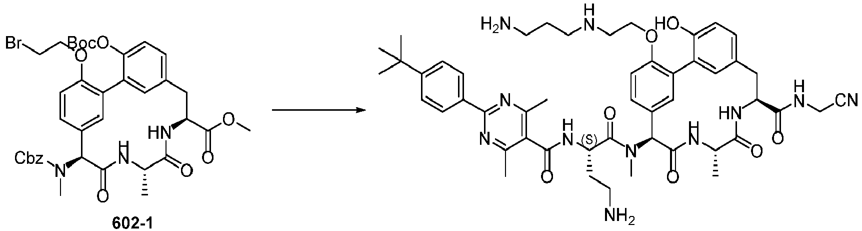

**Compound 602** (FA salt) was prepared as a white solid from **compound 602-1** (described in example 399) and tert-butyl (3-aminopropyl)carbamate by utilizing methods analogous to those described in example 399 and example 600. LCMS (Method 10-80 AB_7min, ESI): t_{R} = 2.087min, [M + H]⁺ = 918.6; ¹H NMR (400 MHz,MeOH-*d4*) 8.57 (br s, 2H), 8.13 (d, *J*=8.0 Hz, 2H), 7.44 (d, *J*=8.0 Hz, 2H), 7.28-7.19 (m, 2H), 6.91-6.78 (m, 3H), 6.64 (br s, 1H), 6.38 (s, 1H), 5.33-5.18 (m, 1H), 4.82-4.50 (m, 2H), 4.46 (s, 2H), 4.31-4.17 (m, 2H), 3.61-3.55 (m, 4H), 3.26-3.08 (m, 4H), 3.02 (s, 3H), 2.92-2.51 (m, 2H), 2.41 (s, 6H), 2.33-2.05 (m, 2H), 2.02-1.87 (m, 2H), 1.39 (s, 9H), 1.35 (d, *J*=6.8Hz, 3H).

### Example 403: Synthesis of Compound 603

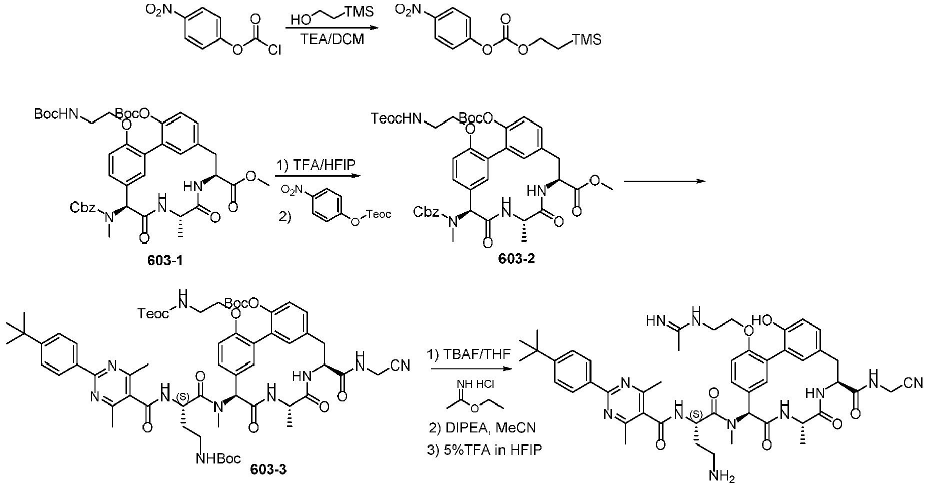

**Step 1**: To a solution of 4-nitro-phenyl-chloroformate (3.0 g, 14.9 mmol) and 2-(trimethylsilyl) ethanol (2.1 g, 17.9 mmol) in DCM (15 mL) was added Et₃N (3.0 g, 29.8 mmol) and the mixture was stirred at 30 C for 1 h. The reaction was quenched with water (15 mL), which was extracted with DCM (30 mL × 3). The combined organic layers were washed with brine (50 mL × 2), dried with Na₂SO₄, concentrated and the residue was purified by silica-gel column, eluting with 5% EtOAc in petroleum ether, to give (4-nitrophenyl) 2-trimethylsilylethyl carbonate (2.8 g, 68% yield) as colorless oil.

**Step 2**: A solution of **compound 603-1** (from example V (compound 106-B2), 400 mg, 0.50 mmol) in 5% TFA in HFIP (15 mL) was stirred at 30 C for 1 h. The reaction was concentrated and the residue was re-dissolved in DMF (15 mL), to which DIEA (577 mg, 4.5 mmol) and (4-nitrophenyl) 2-trimethyl silylethyl carbonate (253 mg, 0.89 mmol) was added sequentially at 0 C. The resulting mixture was stirred for 16 h at 30 C. The reaction was quenched with water (30 mL), which was extracted with EtOAc (30 mL × 3). The combined organic layers were washed with brine (50 mL × 2), concentrated and the residue was purified by silica-gel column, eluting with 0-5% MeOH in DCM, to obtain **compound 603-2** (310 mg, 93% yield) as a white solid. LCMS (Method 5-95 AB, ESI): t_{R} = 0.964 min, [M + Na]⁺ = 771.3

**Step 3: compound 603-3** (130 mg) was prepared as white solids from **compound 603-2** by utilizing methods analogous to those described in example 54. LCMS (Method 5-95 AB, ESI): t_{R} = 1.054 min, [M + Na]⁺ = 1227.6

Step 4: To a solution of **compound 603-3** (110 mg, 0.09 mmol) in DMF (3 mL) was added TBAF (95 mg, 0.36 mmol) and the mixture was stirred at 50 C for 3 h. The reaction was added with water (15 mL), which was exacted with EtOAc (15 mL × 3). The combined organic layers were washed with brine (50 mL × 2), dried over Na₂SO₄ and concentrated. The residue was re-dissolved with acetonitrile (5 mL), to which ethyl ethanimidate (24 mg, 0.27 mmol) and DIEA (58 mg, 0.45 mmol) was added. The mixture was stirred at 25°C for 2 h. The reaction was concentrated to dryness and the residue was taken up in EtOAc (30 mL), which was washed with brine (30 mL). The organic layer was dried over Na₂SO₄ and concentrated and the resulting residue was treated with 5% TFA/HFIP (5 mL) at 25 C for 3 h. The reaction was concentrated and the residue was purified by prep-HPLC (acetonitrile 10-35/0.225%FA in water) to afford the title compound (TFA salt, 13.6 mg, 16% yield over three steps) as a white solid. LCMS (Method 5-95 AB, ESI): t_{R} = 0.764 min, [M + H]⁺ = 902.4; ¹HNMR (400 MHz, MeOH-*d4*) δ 8.24 (d, *J*=8.0Hz, 1H), 7.49 (d, *J*=8.0 Hz, 2H), 7.33-7.15 (m, 2H), 7.03-6.81 (m, 3H), 6.60-6.50 (m, 2H), 5.29-5.21 (m, 1H), 4.82-4.75 (m, 2H), 4.44-4.30 (m, 2H), 4.23 (s, 2H), 3.75-3.63(m, 2H), 3.21-2.82 (m, 7H), 2.50 (s, 6H), 2.31-2.05 (m, 2H), 2.21 (s, 3H), 1.38 (s, 9H), 1.35 (d, *J*=6.8Hz, 3H).

### Examples 404 and 405: Synthesis of Compound 604 and 605

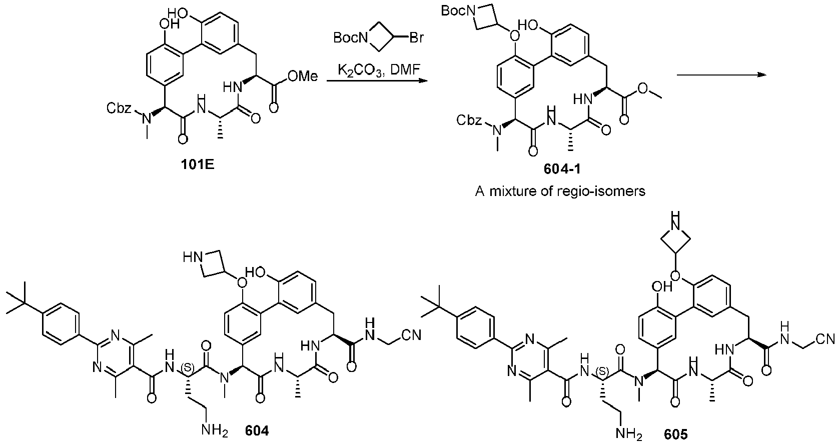

Step 1: A mixture of **101E** (320 mg, 0.58 mmol), K₂CO₃ (394 mg, 2.85 mmol) and tert-butyl 3-bromoazetidine-1-carboxylate (672 mg, 2.85 mmol) in DMF (5 mL) was stirred at 50°C for 5 days. The reaction mixture was taken up in EtOAc (50 mL), which was washed with saturated brine solution (30 mL × 2), dried over Na₂SO₄, concentrated and the residue was purified by HPLC(water(0.225%FA)-ACN) to give compound **604-1** (120 mg, 29% yield) as a white solid. LCMS (Method 5-95 AB, ESI): t_{R} = 0.895 min, [M + Na]⁺ = 739.5

Both title compounds (TFA salt) were prepared as white solids from **Compound 604-1** by utilizing methods analogous to those described in example 395 and they were separated at the final step by HPLC.
**Compound 604:** LCMS (Method 5-95 AB, ESI): t_{R} = 0.753 min, [M + H]⁺ = 873.9; ¹H NMR (400 MHz,Methanol-*d4*) δ 8.25 (d, *J*=8.4 Hz, 2H),7.49 (d, *J*=8.4Hz, 2H), 7.13-6.96 (m, 3H), 6.80 (br s, 1H), 6.74 (d, *J*=8.4 Hz, 2H), 6.54 (br s, 1H), 5.31-5.10 (m, 2H), 4.80-4.78 (m, 2H), 4.50-4.35 (m, 2H), 4.26-4.18 (m, 3H), 4.08-4.00 (m, 1H), 3.35-3.33 (m, 1H), 3.11 (t, *J*=7.2Hz, 2H), 3.05-2.98 (m, 4H), 2.49 (s, 6H), 2.30-2.11 (m, 2H), 1.39 (s, 9H), 1.35 (d, *J*=6.8Hz, 3H). **Compound 605:** LCMS (Method 5-95 AB, ESI): t_{R} = 0.740 min, [M + H]⁺ = 873.4; ¹H NMR (400 MHz,MeOH-*d*4) δ 8.55 (br s, 1H), 8.26 (d, *J*=8.0 Hz, 2H), 7.49 (d, *J*=8.0 Hz, 2H), 7.25-7.22 (m, 1H), 6.93-6.81 (m, 4H), 6.54 (br s, 2H), 5.32-5.24 (m, 2H), 4.82-4.78 (m, 2H), 4.47-4.44 (m, 2H), 4.30-4.10 (m, 2H), 4.24 (s, 2H), 3.22-3.09 (m, 4H), 3.00 (s, 3H), 2.49 (s, 6H), 2.34-2.10 (m, 2H), 1.39 (s, 9H), 1.35 (d, *J*=7.2Hz, 3H).

### Examples 406 and 407: Synthesis of Compound 606 and 607

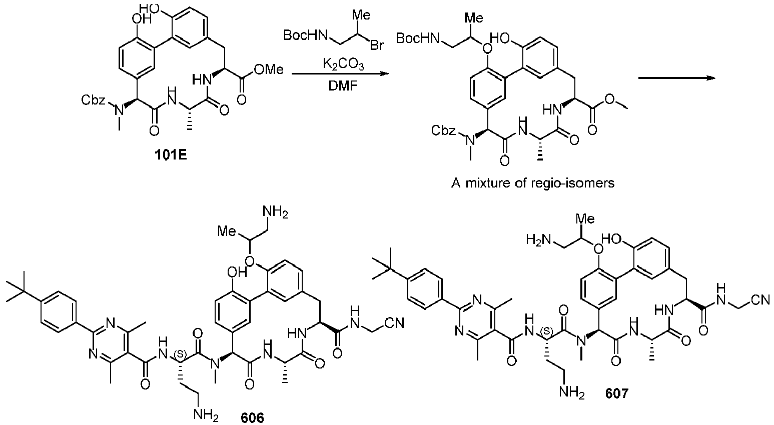

Both title compounds (FA salt) were prepared as white solids from **101E** and tert-butyl (2-bromopropyl)carbamate by utilizing methods analogous to those described in examples 404/405 and they were separated at the final step by HPLC.

**Compound 606:** LCMS (Method 5-95 AB, ESI): t_{R} = 0.751 min, [M + H]⁺ = 875.5; ¹H NMR (400 MHz,MeOH-*d*4) δ 8.54 (br s, 1H), 8.12-8.06 (m, 2H), 7.46-7.40 (m, 2H), 7.25-7.20 (m, 1H), 7.06-6.84 (m, 1H), 6.79-6.74 (m, 3H), 6.43 (br s, 1H), 5.36-5.32 (m, 1H), 4.80-4.75 (m, 4H), 4.32-4.24 (m, 2H),3.28-3.00 (m, 8H), 2.42-1.93 (m, 8H), 1.52-1.26 (m, 15H).

**Compound 607**: LCMS (Method 5-95 AB, ESI): t_{R} = 0.753 min, [M + H]⁺ = 875.5; ¹H NMR (400 MHz,MeOH-*d*4) δ 8.52 (br s, 1H), 8.21-8.14 (m, 2H), 7.46-7.40 (m, 2H), 7.25-7.21(m, 1H), 7.06-7.01 (m, 1H), 6.85-6.61 (m, 3H), 6.43 (br s, 1H), 5.35-5.28 (m, 1H), 4.81-4.76 (m, 4H), 4.30-4.24 (m, 2H), 3.35-3.30 (m, 1H), 3.18-2.93 (m, 7H), 2.45-1.93 (m, 8H), 1.48-1.32 (m, 15H).

### Examples 408 and 409: Synthesis of Compound 608 and 609

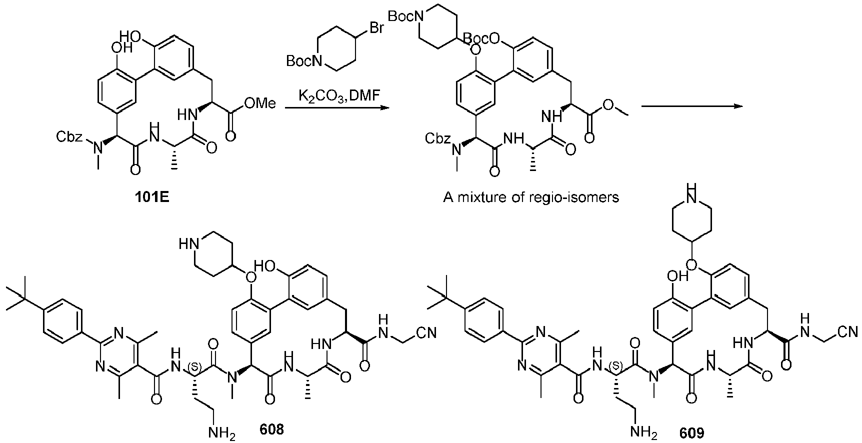

Both title compounds (FA salt) were prepared as white solids from **101E** and tert-butyl 4-bromopiperidine-1-carboxylate by utilizing methods analogous to those described in examples 404/405 and they were separated at the final step by HPLC.
**Compound 608:** LCMS (Method 5-95 AB, ESI): t_{R} = 0.751 min, [M + H]⁺ = 901.5; ¹H NMR (400 MHz,MeOH-*d*4) δ 8.50 (br s,2H), 8.13 (d, *J*=8.0 Hz, 2H), 7.42 (d, *J*=8.0 Hz, 2H), 7.10-7.03 (m, 3H), 6.91 (br s, 1H), 6.80 (s, 1H), 6.78 (s, 1H), 6.43 (br s, 1H), 5.38-5.33 (m, 1H), 4.81-4.60 (m, 1H), 4.34-4.12 (m, 4H), 3.57-3.50 (m, 2H), 3.28-3.07 (m, 10H), 2.69-2.10 (m, 4H), 2.41 (s, 6H), 2.00-1.93 (m, 1H), 1.81-1.75 (m, 1H), 1.80 (s, 9H), 1.35 (d, *J*=6.8Hz, 3H).
**Compound 609:** LCMS (Method 5-95 AB, ESI): t_{R} = 0.748min, [M + H]⁺ = 901.5; ¹H NMR (400 MHz,MeOH-*d4*) 8.54 (br s, 1H), 8.15 (d, *J*=8.0 Hz, 2H), 7.42 (d, *J*=8.0 Hz, 2H), 7.28-7.19 (m, 2H), 6.89 (br s, 1H), 6.86-6.73 (m, 2H), 6.64 (br s, 1H), 6.38 (s, 1H), 5.33-5.18 (m, 1H), 4.82-4.50 (m, 2H), 4.32-4.15 (m, 3H), 3.61-3.55 (m, 1H), 3.28-2.86 (m, 10H), 2.41 (s, 6H), 2.48-2.09 (m, 6H), 1.38 (s, 9H), 1.34 (d, *J*=6.8Hz, 3H).

### Examples 410 and 411: Synthesis of Compound 610 and 611

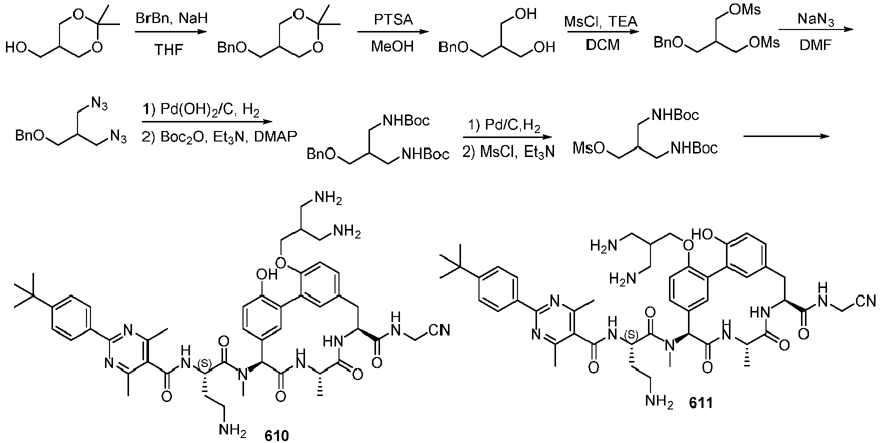

**Step 1**: To a solution of (2,2-dimethyl-1,3-dioxan-5-yl)methanol (1.0 g, 6.84 mmol) and NaH (60% in oil, 0.41 g, 10.3 mmol) in THF (30 mL) was added BnBr (1.62 mL, 13.7 mmol). The reaction was stirred at 20°C for 16 h. The reaction was taken up in EtOAc (50 mL), which was washed with brine (30 mL × 2), dried over Na₂SO₄, concentrated and the residue was purified by silica gel column, eluting with 10% EtOAc in petroleum ether, to obtain 5-(benzyloxy methyl)-2,2-dimethyl-1,3-dioxane (1.6 g, 99% yield) as colorless oil.

**Step 2**: To a solution of 5-(benzyloxymethyl)-2,2-dimethyl-1,3-dioxane (1.5 g, 6.35 mmol) in MeOH (20 mL) was added TsOH (109 mg, 0.63 mmol). The reaction mixture was stirred at 20°C for 1 h. The reaction was taken up in EtOAc (50 mL), which was washed with brine (20 mL × 3), dried over Na₂SO₄, concentrated and the crude was purified by silica gel column, eluting with 50% EtOAc in petroleum ether, to obtain 2-(benzyloxymethyl)propane-1,3-diol (1.0 g, 80% yield) as colorless oil.

**Step 3**: A solution of 2-(benzyloxymethyl)propane-1,3-diol (1.0 g, 5.1 mmol) and Et₃N (2.86 mL, 20.4 mmol) in DCM (20 mL) was stirred at 0°C for 30 min, followed by the slow addition of MsCl (1.22 mL, 15.7 mmol). The mixture was stirred for another 3 h at 0°C. After filtration, the filtrate was concentrated and the residue was purified by silica gel column, eluting with 10% EtOAc in petroleum ether, to obtain [2-(benzyloxymethyl)-3-methyl sulfonyloxy-propyl] methanesulfonate (1.5 g, 84% yield) as colorless oil. ¹H NMR (400 MHz, CDCl₃) δ 7.35-7.27 (m, 5H), 4.50 (s, 2H), 4.36-4.29 (m, 4H), 3.55 (d, *J*=6.0 Hz, 2H), 2.99 (s, 6H), 2.55-2.46 (m, 1H).

325] **Step 4**: A solution of [2-(benzyloxymethyl)-3-methylsulfonyloxy-propyl] methanesulfonate (1.5 g, 4.3 mmol) and NaN₃ (2.84 g, 43.7 mmol) in DMF (20 mL) was stirred at 80°C for 16 h. After filtration, the filtrate was taken up in EtOAc (200 mL), which was washed with brine (100 mL × 3 ), dried over Na₂SO₄, concentrated and the residue was purified by silica-gel column, eluting with 7% MeOH in DCM, to give [3-azido-2-(azidomethyl)propoxy]methyl benzene (1.0 g, 95.4% yield) as colorless oil.

**Step 5:** To a solution of [3-azido-2-(azidomethyl)propoxy]methylbenzene (1.0 g, 4.1 mmol) in MeOH (20 mL) was added 10% Pd(OH)₂/C (432 mg, 0.41 mmol). The reaction mixture was stirred at 15°C for 5 h under H₂ (15 psi). The mixture was filtered and the filtrate was concentrated. The residue was re-dissolved in THF (10 mL), to which was added Boc₂O (4.04 g, 18.5 mmol), Et₃N (2.81 g, 27.8 mmol) and DMAP (142 mg, 1.2 mmol). The reaction mixture was stirred at 20°C for 16 h. After that, the reaction was taken up in EtOAc (200 mL), which was washed with brine (100 mL × 3), dried over Na₂SO₄, concentrated and the residue was purified by silica gel column, eluting with 20% EtOAc in petroleum ether, to give tert-butyl N-[2-(benzyloxymethyl)-3-(tert-butoxycarbonylamino)propyl]carbamate (1.3 g, 71% yield) as colorless oil.

**Step 6**: To a solution of tert-butyl N-[2-(benzyloxymethyl)-3-(tert-butoxycarbonylamino) propyl]carbamate (1.3 g, 3.3 mmol) in MeOH (20 mL) was added 10% Pd/C (351 mg, 0.33 mmol) and the reaction mixture was stirred at 60°C for 5 h under H₂ (40 psi). The mixture was filtered and the filtrate was concentrated. The residue was re-dissolved in DCM, to which Et₃N (740 µL, 5.3 mmol) was added at 0°C for 30 min. MsCl (150 µL, 2.0 mmol) in DCM (2 mL) was then added dropwise to the above solution and the resulting mixture was stirred for another 3 h at 0°C. After filtration, the filtrate was concentrated to obtain [3-(tert-butoxy carbonylamino)-2-[(tert-butoxycarbonylamino)methyl]propyl] methanesulfonate (500 mg, quantitative yield) as colorless oil, which was used directly without further purification.

Both title compounds (FA salt) were prepared as white solids from **101E** and [3-(tert-butoxy carbonylamino)-2-[(tert-butoxycarbonylamino)methyl]propyl] methanesulfonate by utilizing methods analogous to those described in examples 404/405 and they were separated at the final step by HPLC.

**Compound 610:** LCMS (Method 5-95 AB, ESI): t_{R} = 0.743 min, [M + H]⁺ = 904.4; ¹H NMR (400 MHz, MeOH-*d4*) δ 8.48 (br s, 2H), 8.35 (d, *J*=8.0 Hz, 2H), 7.56 (d, *J*=8.0 Hz, 2H), 7.35-6.90 (m, 4H), 6.89-6.75 (m, 2H), 6.43 (s, 1H), 5.26-5.25 (m, 1H), 4.80-4.70 (m, 2H), 4.60-4.25 (m, 2H), 4.22 (s, 2H), 3.50 -3.40 (m, 1H), 3.25-2.95 (m, 10H), 2.57 (s, 6H), 2.45-2.35 (m, 1H), 2.30-2.20 (m, 1H), 2.19-2.10 (m, 1H), 1.40 (s, 9H), 1.37 (d, *J*=6.8 Hz, 3H).

**Compound 611:** LCMS (Method 5-95 AB, ESI): t_{R} = 0.740min, [M + H]⁺ = 905.0; ¹H NMR (400 MHz, MeOH-*d4*) δ 8.57 (br s, 1H), 8.40 - 8.20 (m, 2H), 7.56-7.54 (m, 2H), 7.35-7.00 (m, 3H), 6.98-6.75 (m, 2H), 6.69 (br s, 1H), 6.47 (s, 1H), 5.30-5.15 (m, 1H), 4.80 - 4.70 (m, 2H), 4.41-4.31 (m, 2H), 4.22 (s, 2H), 3.50-3.40 (m, 1H), 3.25-3.04 (m, 7H), 3.05 (s, 3H), 2.57 (s, 6H), 2.45-2.35 (m, 1H), 2.30-2.20 (m, 1H), 2.19-2.10 (m, 1H), 1.40 (s, 9H), 1.38-1.25 (m, 3H).

### Example 412: Synthesis of Compound 612

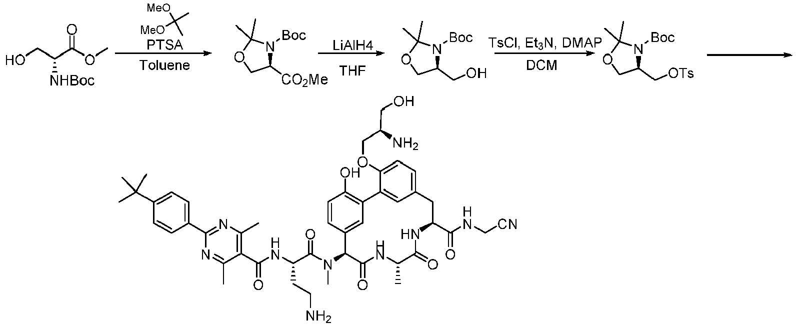

**Step 1:** A solution of (*R*)-methyl 2-((tert-butoxycarbonyl)amino)-3-hydroxypropanoate (7.77 g, 35.4 mmol) in toluene (80 mL) was added 2,2-dimethoxypropane (7.38 g, 70.9 mmol) and TsOH (610 mg, 3.54 mmol) was stirred at 110°C for 0.5 h. The volatiles were distilled under 1 atm and the residue was re-dissolved with EtOAc (100 mL), which was washed with saturated NaHCO₃ and brine (100 mL each). The organic layer was dried over Na₂SO₄, concentrated and the residue was purified by chromatography on silica, eluting with 10% EtOAc in petroleum ether, to afford (*R*)-3-tert-butyl 4-methyl 2,2-dimethyloxazolidine- 3,4- dicarboxylate (7.60 g, 83% yield) as yellow oil.

**Step 2:** To a suspension of lithium aluminium hydride (3.34 g, 87.9 mmol) in tetrahydrofuran (80 mL) was added a solution of (*R*)-3-tert-butyl 4-methyl 2,2-dimethyloxazolidine- 3,4- dicarboxylate (7.60 g, 29.3 mmol) in tetrahydrofuran (5 mL) dropwise at 0°C under N₂. The reaction was then gradually warmed up to 25°C while stirring and stirred at the same temperature for 2 h. The reaction was quenched with 10% NaOH solution (3.5 mL). After filtration, the filtrate was concentrated and the residue was partitioned between EtOAc and water (300 mL each). The organic layer was washed with brine (300 mL), dried over Na₂SO₄, concentrated to give (S)-tert-butyl 4-(hydroxymethyl)-2,2-dimethyloxazolidine-3-carboxylate (5.51 g, 81% yield) as colorless oil.

**Step 3:** To a solution of (*S*)-tert-butyl 4-(hydroxymethyl)-2,2-dimethyloxazolidine-3-carboxylate (4.90 g, 21.2 mmol) and TsCl (6.06 g, 31.8 mmol) in dichloromethane (50 mL) was added Et₃N (5.91 mL, 42.4 mmol) and DMAP (259 mg, 2.12 mmol) and the reaction was stirred at 25°C for 16 h. The reaction mixture was added with water and DCM (100 mL each). The organic layer was washed with brine (100 mL × 2), concentrated and the residue was purified by silica-gel column, eluting with 20-50% EtOAc in petroleum ether, to afford (*R*)-tert-butyl 2,2-dimethyl-4-((tosyloxy)methyl)oxazolidine-3-carboxylate (4.73 g, 58% yield) as a white solid.

**Compound 612** (FA salt) was prepared as a white solid from **101E** and (*R*)-tert-butyl 2,2-dimethyl-4-((tosyloxy)methyl)oxazolidine-3-carboxylate by utilizing methods analogous to those described in example 404. LCMS (Method 5-95 AB, ESI): t_{R} = 0.611 min, [M + H]⁺ = 891.4; ¹H NMR (400 MHz, MeOH-*d4*) δ 8.60 (br s, 1H), 8.28-8.12 (m, 2H), 7.54-7.48 (m, 2H), 7.29-7.02 (m, 3H), 6.88-6.81 (m, 2H), 6.65 (br s, 1H), 6.54 (s, 1H), 5.37-5.30 (m, 1H), 4.84-4.80 (m, 2H), 4.73-4.20 (m, 4H), 4.25 (s, 2H), 4.28-4.23 (m, 3H), 3.92-3.77 (m, 2H), 3.64-3.60 (m, 1H), 3.15-3.11 (m, 2H), 3.03 (s, 3H), 2.48 (s, 6H), 2.33-2.29 (m, 1H), 2.21-2.14 (m, 1H), 1.41 (s, 9H), 1.37 (d, *J*= 6.4 Hz, 3H).

### Example 413: Synthesis of Compound 613

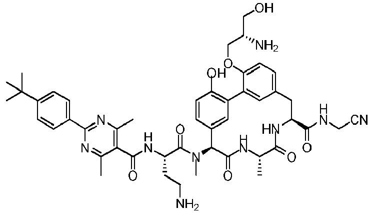

**Compound 613** (TFA salt) was prepared as a white solid from (S)-methyl 2-((tert-butoxy carbonyl)amino)-3-hydroxypropanoate by utilizing methods analogous to those described in example 412. LCMS (Method 5-95 AB, ESI): t_{R} = 0.609 min, [M + H]⁺ = 891.4; ¹H NMR (400 MHz, MeOH-d4) δ 8.36 (d, *J*=8.4 Hz, 2H), 7.56 (d, *J*=8.4 Hz, 2H), 7.33-7.30 (m, 1H), 7.20-7.15 (m, 1H), 7.12-7.05 (m, 1H), 6.72-6.93 (m, 2H), 6.91-6.85 (m, 1H), 6.78 (br s, 1H), 6.46 (s, 1H), 5.26-5.22 (m, 2H), 4.43-4.29 (m, 3H), 4.22 (s, 2H), 4.07-4.02 (m, 2H), 3.85-3.73 (m, 2H), 3.03 (s, 3H), 2.52 (s, 6H), 2.30-2.00 (m, 2H), 1.42 (s, 9H), 1.37 (d, *J* = 6.8 Hz, 3H).

### Example 414: Synthesis of Compound 614

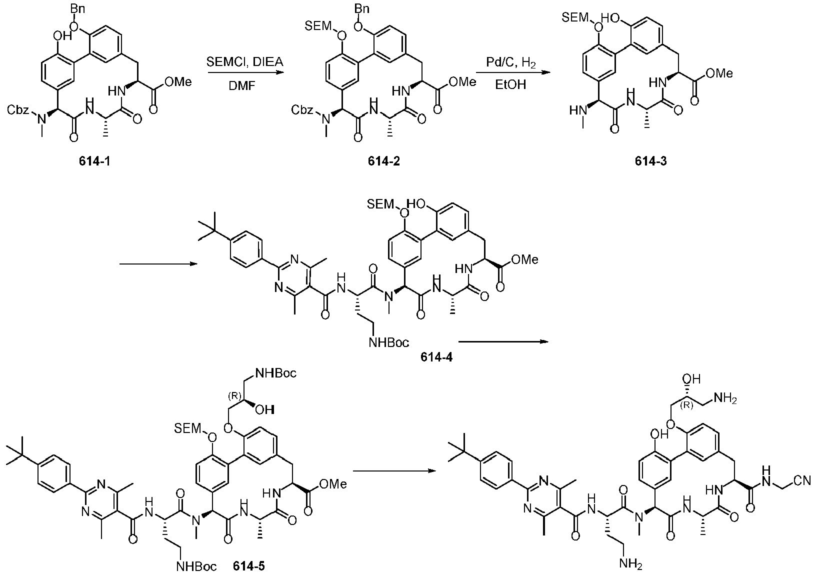

**Step** 1: To a solution of **compound 614-1** (from example V (compound 106-A2)) 200 mg, 0.31 mmol) and DIEA (507 µL, 3.1 mmol) in DMF (5 mL) was added SEM-Cl (543 µL, 3.1 mmol) and the reaction was stirred at 50°C for 16 h. The mixture was partitioned between EtOAc and H₂O (each 100 mL). The organic layer was washed with brine, dried over Na₂SO₄, concentrated and the residue was purified by silica-gel column, eluting with 10% MeOH in DCM, to afford **compound 614-2** (200 mg, 83% yield) as yellow oil. LCMS (Method 5-95 AB, ESI): t_{R} = 1.038 min, [M + Na]⁺ = 804.0
**Step 2:** To a solution of **compound 614-2** (200 mg, 0.26 mmol) in EtOH (20 mL) was added 10% Pd/C (272 mg, 0.26 mmol) and a drop of ammonia and the mixture was stirred at 30°C under H₂ (15 psi) for 3 h. After filtration, the volatiles were concentrate to afford **compound 614-3** (96 mg, 67% yield) as a white solid. LCMS (Method 5-95 AB, ESI): t_{R} = 0.758 min, [M + Na]⁺ = 580.0

**Compound 614-4** was prepared as a white solid from **compound 614-3** by utilizing methods analogous to those described in example 54. LCMS (Method 5-95 AB, ESI): t_{R} = 1.125 min, [M + Na]⁺ = 1046.3
**Compound 614-5** was prepared from compound **614-4** as a white solid by utilizing methods analogous to those described in example 367. LCMS (Method 5-95 AB, ESI): t_{R} = 1.117 min, [M + Na]⁺ = 1219.2

**Compound 614** (FA salt) was prepared as a white solid from **compound 614-5** by utilizing methods analogous to those described in example 54. LCMS (Method 5-95 AB, ESI): t_{R} = 0.728min, [M + H]⁺ = 891.4; ¹H NMR (400 MHz, MeOH-*d*4) δ 8.55 (br s, 1H), 8.27 (d, *J* = 8.0 Hz, 2H), 7.54 (d, *J* = 8.0 Hz, 2H), 7.14-6.99 (m, 4H), 6.85 (br s, 1H), 6.69 (br s, 1H), 6.58 (s, 1H), 5.35-5.25 (m, 1H), 5.35-5.25 (m, 2H), 4.71-4.40 (m, 2H), 4.31-4.06 (m, 3H), 3.21-3.17 (m, 1H), 3.15-3.07 (m, 5H), 3.03 (s, 3H), 2.51 (s, 6H), 2.33-2.24 (m, 1H), 2.20-2.11 (m, 1H), 1.41 (s, 9H), 1.37 (d, *J* = 6.4 Hz, 3H).

### Example 415: Synthesis of Compound 615

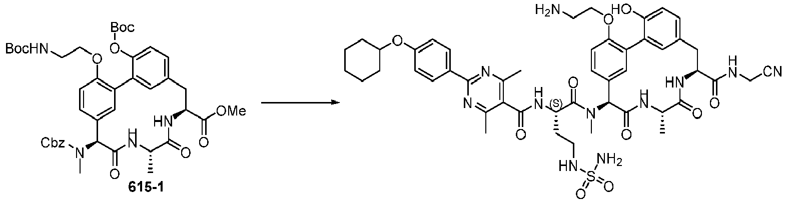

**Compound 615** (TFA salt) was prepared as a white solid from **compound 615-1** (from example V (compound 106-B2)) and 2-(4-(cyclohexyloxy)phenyl)-4,6-dimethylpyrimidine-5-carboxylic acid (described in example 137) by utilizing methods analogous to those described in example 374. LCMS (Method 5-95 AB, ESI): t_{R} = 0.702 min, [M + H]⁺ = 982.4; ¹H NMR (400 MHz, MeOH-*d4*) δ 8.24-8.08 (m, 2H), 7.27-7.14 (m, 2H), 6.97-6.69 (m, 6H), 6.45 (s, 1H), 5.33-5.24 (m, 1H), 4.81-4.74 (m, 2H), 4.57-4.37 (m, 4H), 4.28-4.20 (m, 1H), 3.42-3.35 (m, 2H), 3.29-3.19 (m, 2H), 3.05 (s,3H), 2.99-2.74 (m, 2H), 2.44 (s, 6H), 2.24-2.14 (m, 1H), 2.09-1.97 (m, 3H), 1.89-1.79 (m, 2H), 1.66-1.41 (m, 6H), 1.35 (d, *J*=6.8Hz, 3H).

### Example 416: Synthesis of Compound 616

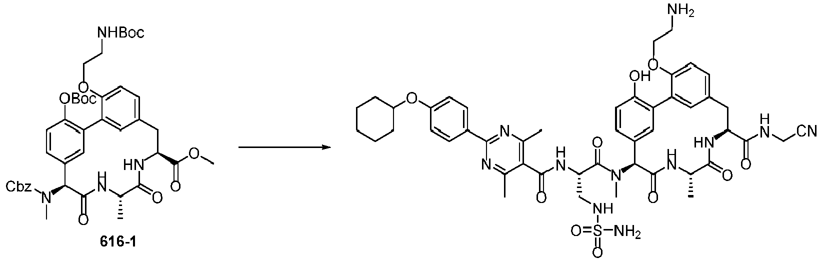

**Compound 616** (TFA salt) was prepared as a white solid from **compound 616-1** (from example V (compound 106-B1)) and 2-(4-(cyclohexyloxy)phenyl)-4,6-dimethylpyrimidine -5-carboxylic acid (described in example 137) by utilizing methods analogous to those described in example 374. LCMS (Method 5-95 AB, ESI): t_{R} = 0.831 min, [M + H]⁺ = 968.4; ¹H NMR (400 MHz, MeOH-*d4*) δ 8.10-7.93 (m, 2H), 7.17-6.97 (m, 4H), 6.97-6.81 (m, 3H), 6.72 (br s, 1H), 6.26 (br s, 1H), 5.54-5.50 (m, 1H), 4.83-4.72 (m, 2H), 4.51-4.34 (m, 3H), 4.29 (s, 2H), 4.02-3.74 (m, 1H), 3.58-3.37 (m, 4H), 3.12 (s, 3H), 2.85-2.75 (m, 1H), 2.33 (s, 6H), 2.09-2.02 (m, 2H), 1.89-1.85 (m, 2H), 1.67-1.38 (m, 9H).

### Example 417: Synthesis of Compound 617

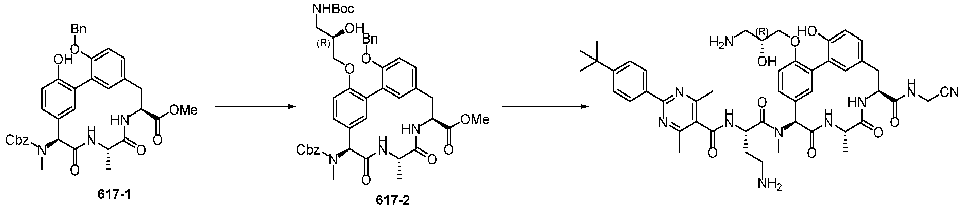

**Compound 617-2** was prepared as a white solid from **compound 617-1** (from example V (compound 106-A2)) by utilizing methods analogous to those described in example 367. LCMS (Method 5-95 AB, ESI): t_{R} = 0.817 min, [M + Na]⁺ = 847.2

**Compound 617** (FA salt) was prepared as a white solid from **compound 617-2** and (S)-2-(((benzyloxy)carbonyl)amino)-4-((tert-butoxycarbonyl)amino)butanoic acid by utilizing methods analogous to those described in example 414 and example 382. LCMS (Method 5-95 AB, ESI): t_{R} = 0.617 min, [M + H]⁺ = 891.3; ¹H NMR (400MHz, MeOH-d4) δ 8.45 (br s, 1H), 8.21 (d, *J*=7.6Hz, 2H), 7.48 (d, *J*=7.6 Hz, 2H), 7.31-7.15 (m, 2H), 6.93-6.86 (m, 2H), 6.85-6.81 (m, 1H), 6.59 (br s, 1H), 6.48 (s, 1H), 5.34-5.26 (m, 1H), 4.85-4.76 (m, 2H), 4.30-4.19 (m, 3H), 4.21 (s, 2H), 3.28-3.08 (m, 4H), 3.00 (s, 3H), 2.97-2.70 (m, 2H), 2.47 (s, 6H), 2.36-2.25 (m, 1H), 2.21-2.11 (m, 1H), 1.39 (s, 9H), 1.34 (d, *J*=6.8Hz, 3H).

### Example 418: Synthesis of Compound 618

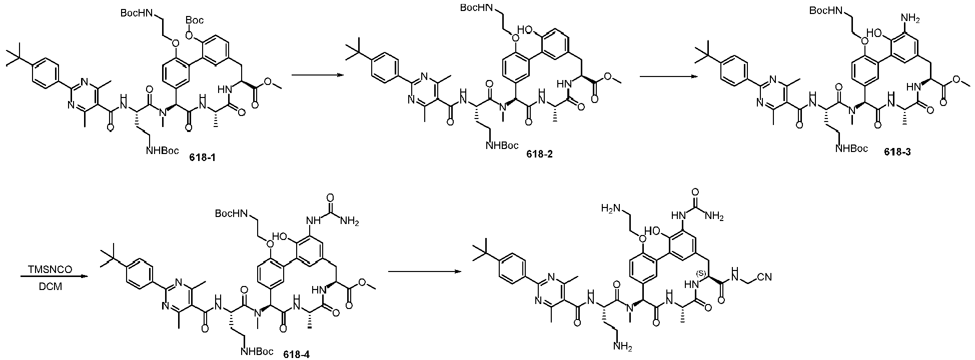

**Compound 618-2** was prepared as a white solid from **compound 618-1** (from example V (compound 106-B2)) by utilizing methods analogous to those described in example 371. LCMS (Method 5-95 AB, ESI): t_{R} = 0.944 min, [M + H]⁺ = 1038.9

**Compound 618-3** was prepared as a white solid from **compound 618-2** by utilizing methods analogous to those described in example 389. LCMS (Method 5-95 AB, ESI): t_{R} = 0.795 min, [M + H]⁺ = 1052.8

A solution of **compound 618-3** (70 mg, 0.07 mmol), trimethylsilyl isocyanate (153 mg, 1.3 mmol) in DCM (5 mL) was stirred for 5 h at 25°C. The reaction was added with DCM (30 mL), which was washed with 0.1 M HCl solution and brine (30 mL each). The organic layer was dried over Na₂SO₄, concentrated and the residue was purified by prep-TLC, eluting with 10% MeOH in DCM, to give **compound 618-4** (20 mg, 27% yield) as a white solid. LCMS (Method 5-95 AB, ESI): t_{R} = 0.989 min, [M + H]⁺ = 1095.6

**Compound 618** (TFA salt) was prepared as a white solid by utilizing methods analogous to those described in example 382. LCMS (Method 5-95 AB, ESI): t_{R} = 0.715 min, [M + H]⁺ = 919.8; ¹H NMR (400 MHz, MeOH-*d*₄) δ 8.21 (d, *J*=7.6 Hz, 2H), 7.51 (d, *J*=7.6 Hz, 2H), 7.29 (d, *J*=7.2 Hz, 1H), 7.22 (d, *J*=7.2 Hz, 1H), 7.03 (br s, 1H), 6.95 (br s, 1H), 6.61 (br s, 1H), 6.34 (s, 1H), 5.35-5.27 (m, 1H), 4.63-4.33 (m, 4H), 4.26 (s, 2H), 3.43-3.31 (m, 3H), 3.20-3.08 (m, 2H), 3.03 (s, 3H), 2.99-2.92 (m, 1H), 2.49 (s, 6H), 2.40-2.28 (m, 1H), 2.21-2.10 (m, 1H), 1.40 (s, 9H), 1.37 (d, *J*=6.8 Hz, 3H).

### Example 419: Synthesis of Compound 619

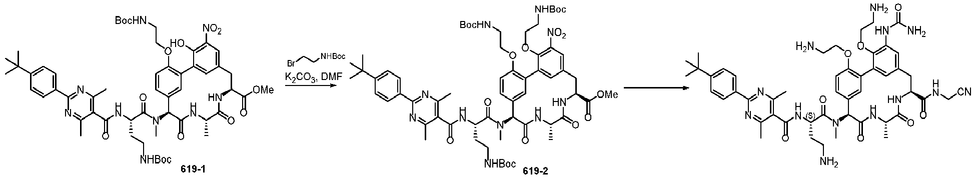

Step 1: A solution of **compound 619-1** (134 mg, 0.12 mmol), K₂CO₃ (86 mg, 0.62 mmol) and tert-butyl (2-bromoethyl)carbamate (139 mg, 0.62 mmol) in DMF (5 mL) was stirred at 50°C for 48 h while four portions of K₂CO₃ (86 mg, 0.62 mmol) and tert-butyl (2-bromoethyl) carbamate (139 mg, 0.62 mmol) were added every 12 h. After filtration, the filtrate was partitioned between EtOAc and water (40 mL each). The organic layer was washed with brine (30 mL × 2), dried over Na₂SO₄, concentrated and the residue was purified by prep-TLC, eluting with 5% MeOH in DCM, to afford **compound 619-2** (131 mg, 86% yield) as a white solid. LCMS (Method 5-95 AB, ESI): t_{R} = 1.214 min, [M + H]⁺ = 1225.8

**Compound 619** (FA salt) was prepared as a white solid by utilizing methods analogous to those described in example 418. LCMS (Method 10-80 AB, ESI, 7 min): t_{R} = 1.749 min, [M + H]⁺ = 962.5; ¹H NMR (400 MHz, MeOH-*d*₄) δ 8.53 (br s, 1H), 8.05 (s, 2H), 7.45 (d, *J*=8.4 Hz, 2H), 7.34-7.23 (m, 2H), 7.20-7.08 (m, 1H), 6.91 (s, 1H), 6.76 (br s, 1H), 6.09 (br s, 1H), 5.42-5.27 (m, 1H), 4.87-4.72 (m, 2H), 4.59-4.40 (m, 2H), 4.23 (s, 2H), 3.74 (t, *J*=5.2 Hz, 2H), 3.49-3.45 (m, 2H), 3.32-3.31 (m, 1H), 3.14 (t, *J*=8.4 Hz, 2H), 3.07-3.03 (m, 2H), 3.02 (s, 3H), 2.92-2.77 (m, 1H), 2.38 (s, 6H), 2.30-2.24 (m, 1H), 2.21-2.07 (m, 1H), 1.38 (s, 9H), 1.35 (d, *J*=6.8 Hz, 3H).

### Example 420: Synthesis of Compound 620

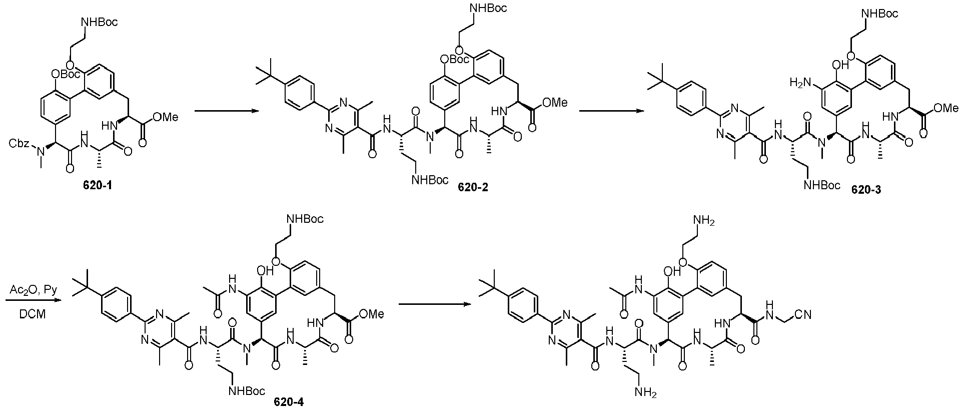

**Compound 620-2** was prepared as a white solid from **compound 620-1** (from example V (compound 106-B1)) by utilizing methods analogous to those described in example 54. LCMS (Method 5-95 AB, ESI): t_{R} = 1.161 min, [M + H]⁺ = 1138.1

**Compound 620-3** was prepared as a white solid by utilizing methods analogous to those described in example 418. LCMS (Method 5-95 AB, ESI): t_{R} = 0.986 min, [M + Na]⁺ = 1074.7

A solution of **compound 620-3** (100 mg, 0.10 mmol), Ac₂O (30 µL, 0.29 mmol) and pyridine (50 µL, 0.57 mmol) in DCM (2 mL) was stirred for 2 h at 25°C. The mixture was added EtOAc (50 mL), which washed with brine (50 mL), dried over Na₂SO₄ and concentrated. The residue was purified by pre-TLC, eluting with 5% MeOH in DCM, to afford **compound 620-4** (108 mg, 97% yield) as a white solid. LCMS (Method 5-95 AB, ESI): t_{R} = 1.059 min, [M + H]⁺ = 1094.8

**Compound 620** (FA salt) was prepared as a white solid by utilizing methods analogous to those described in example 382. LCMS (Method 20-80 AB, ESI, 7 min): t_{R} = 2.366 min, [M/2 + H]⁺ = 459.9; ¹H NMR (400 MHz, MeOH-*d*₄) δ 8.55 (br s, 1H), 8.21 (d, *J*=8.0 Hz, 2H), 7.54 (s, 1H), 7.46 (d, *J*=8.0 Hz, 2H), 7.00-6.90 (m, 2H), 6.66 (s, 1H), 6.48 (s, 1H), 6.31 (s, 1H), 5.34-5.31 (m, 1H), 4.86-4.74 (m, 2H), 4.40-4.28 (m, 4H), 3.42-3.32 (m, 2H), 3.31-3.26 (m, 1H), 3.12 (t, *J*=7.8 Hz, 2H), 2.98 (s, 3H), 2.95-2.89 (m, 1H), 2.43 (s, 6H), 2.36-2.27 (m, 1H), 2.15 (m, 1H), 2.17-2.11 (s, 3H), 1.39 (s , 9H), 1.36 (d, *J*=6.8 Hz, 3H).

### Example 421: Synthesis of Compound 621

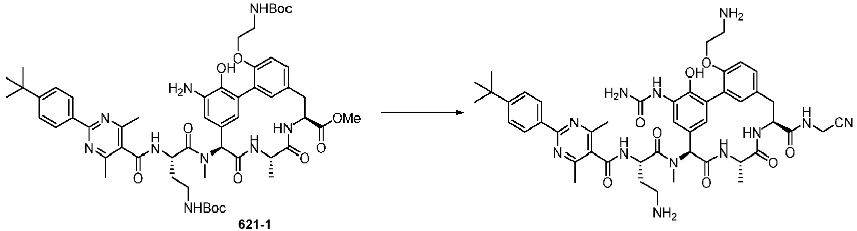

**Compound 621** (FA salt) was prepared from **compound 621-1** (synthesis described in example 418) as a white solid by utilizing methods analogous to those described in example 418. LCMS (Method 20-80 AB, ESI, 7 min): t_{R} = 2.372 min, [M + H]⁺ = 919.5; ¹H NMR (400 MHz, MeOH-*d*₄) δ 8.70 (br s, 1H), 8.10 (d, *J*=7.6 Hz, 2H), 7.55-7.40 (m, 3H), 6.99 (d, *J*=8.4 Hz, 1H), 6.90 (br s, 1H), 6.62 (s, 1H), 6.44 (s, 1H), 6.33 (s, 1H), 5.43-5.32 (m, 1H), 4.87-4.70 (m, 2H), 4.44-4.27 (m, 4H), 3.45-3.34 (m, 2H), 3.21-3.04 (m, 2H), 2.98 (s, 3H), 2.93-2.79 (m, 1H), 2.62-2.51 (m, 1H), 2.41 (s, 6H), 2.36-2.28 (m, 1H), 2.22-2.09 (m, 1H), 1.41 (s, 9H), 1.30 (d, *J*=6.4 Hz, 3H).

### Example 422: Synthesis of Compound 622

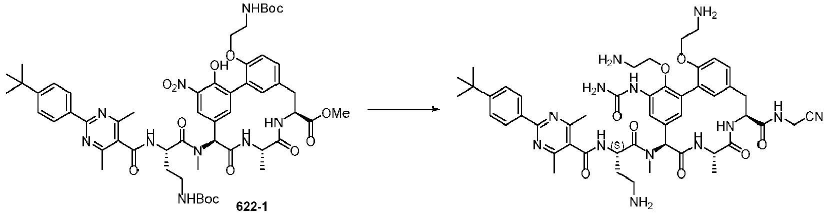

**Compound 622** (FA salt) was prepared from **compound 622-1** (synthesis described in example 418) as a white solid by utilizing methods analogous to those described in example 419. LCMS (Method 20-80 AB, ESI, 7 min): t_{R} = 1.930 min, [M + H]⁺ = 962.6; ¹H NMR (400 MHz, MeOH-*d*₄) δ 8.49 (br s, 1H), 8.26 (d, *J*=8.0 Hz, 2H), 7.68 (s, 1H), 7.50 (d, *J*=8.0 Hz, 2H), 7.20 (d, *J*=8.4 Hz, 1H), 7.08 (d, *J*=8.4 Hz, 1H), 6.85 (s, 1H), 6.64 (s, 1H), 6.47 (s, 1H), 5.43-5.32 (m, 1H), 4.87-4.70 (m, 2H), 4.44-4.27 (m, 2H), 4.22 (s, 2H), 4.00-3.80 (m, 2H), 3.45-3.34 (m, 2H), 3.21-3.04 (m, 2H), 2.98 (s, 3H), 2.62-2.40 (m, 2H), 2.51 (s, 6H), 2.36-2.28 (m, 1H), 2.22-2.09 (m, 1H), 1.38 (s, 9H), 1.36 (d, *J*=6.8 Hz, 3H).

### Example 423: Synthesis of Compound 623

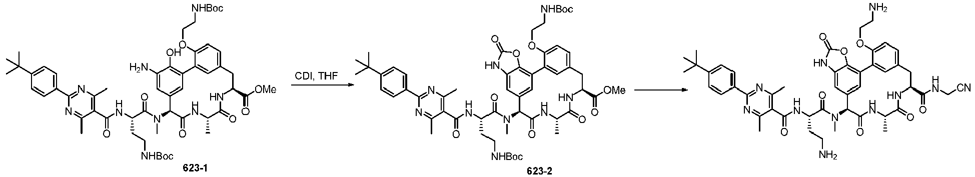

Step 1: A solution of **compound 623-1** (synthesis described in example 418, 110 mg, 0.10 mmol), 1,1'-carbonyldiimidazole (339 mg, 2.1 mmol) in anhydrous THF (6 mL) was stirred at 25 C for 16 h. The mixture was partitioned between EtOAc and water (40 mL each) and the organic layer was washed with brine (30 mL × 2), dried over Na₂SO₄ and concentrated. The residue was purified by pre-TLC, eluting with 5% MeOH in DCM, to obtain **compound 623-2** (91 mg, 81% yield) as a white solid. LCMS (Method 5-95 AB, ESI): t_{R} = 1.047 min, [M + H]⁺ = 1078.7

**Compound 623** (FA salt) was prepared from **compound 623-2** as a white solid by utilizing methods analogous to those described in example 54. LCMS (Method 10-80 AB, ESI, 7 min): t_{R} = 2.188 min, [M + H]⁺ = 902.5; ¹H NMR (400 MHz, MeOH-*d*₄) δ 8.52 (br s, 1H), 8.34 (d, *J*=8.4Hz, 2H), 7.53 (d, *J*=8.4Hz, 2H), 7.29-7.19 (m, 1H), 7.14 (d, *J*=8.4Hz, 1H), 6.96 (s, 1H), 6.81 (br s, 1H), 6.75 (s, 1H), 6.55 (s, 1H), 5.22-5.18 (m, 1H), 4.73-4.56 (m, 2H), 4.51-4.40 (m, 1H), 3.39-4.27 (m, 1H), 4.22 (s, 2H), 3.47-3.38 (m, 2H), 3.38-3.34 (m, 2H), 3.15-3.11 (m, 2H), 3.00 (s, 3H), 2.57 (s, 6H), 2.33-2.22 (m, 2H), 1.41 (s, 9H), 1.37 (d, *J*=6.0 Hz, 3H).

### Example 424: Synthesis of Compound 624

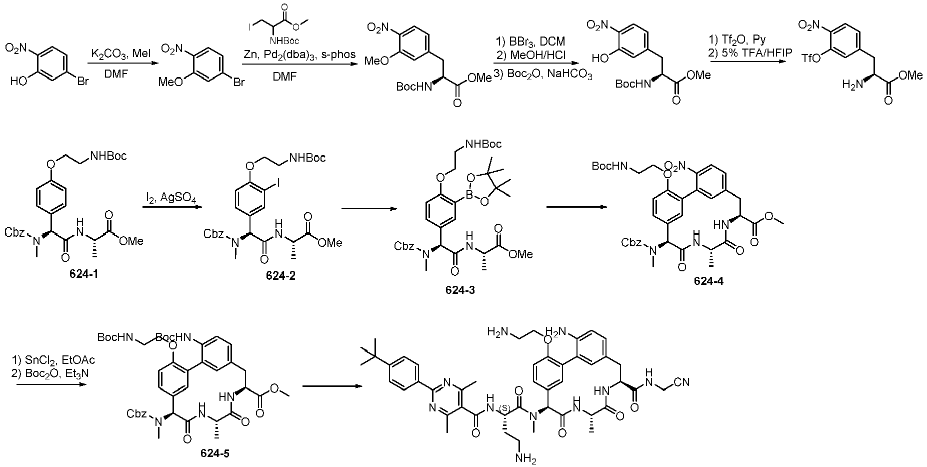

**Step 1:** 4-bromo-2-methoxy-1-nitrobenzene was prepared as a yellow solid from 5-bromo-2-nitrophenol by utilizing methylation procedure described in example 391.

**Step 2:** A solution of methyl 2-((tert-butoxycarbonyl)amino)-3-iodopropanoate (4.26 g, 12.9 mmol), zinc (1.69 g, 25.8 mmol) and I₂ (100 mg) in DMF (10mL) was stirred at 20°C under N₂ for 30 min, followed by the addition of 4-bromo-2-methoxy-1-nitrobenzene (2.0 g, 8.6 mmol), sphos (354 mg, 0.86 mmol) and Pd₂(dba)₃ (395 mg, 0.43 mmol) under N₂. The mixture was then warmed to 60°C while stirring and stirred for at 60°C for 3 h. The mixture was taken up in EtOAc (200 mL), which was washed with brine (150 mL × 2), dried over MgSO₄ and concentrated. The residue was purified by silica-gel column, eluting with 0-20% EtOAc in petroleum ether, to give (S)-methyl 2-((tert-butoxycarbonyl)amino)-3-(3-methoxy -4-nitrophenyl)propanoate (2.0 g, 66% yield) as yellow oil.

**Step 3:** A solution of (S)-methyl 2-((tert-butoxycarbonyl)amino)-3-(3-methoxy-4-nitrophenyl) propanoate (2.0 g, 5.6 mmol) in DCM (10 mL), BBr₃ (3.2 mL, 33.9 mmol) was added at 0°C. The mixture was warmed to 25°C slowly while stirring and stirred at the same temperature for 16 h. The reaction was added into MeOH (20mL) slowly and the resulting mixture was concentrated. The residue was re-dissolved in saturated HCl/MeOH (50 mL) and the mixture was stirred at 25°C for 5 h. The volatiles were removed and the resulting residue was re-dissolved in THF (15 mL), to which Boc₂O (1.44 mL, 6.3 mmol) and 15 mL saturated NaHCO₃ solution were added. The reaction mixture was stirred at 25°C for 16 h. After that, the mixture was diluted with EtOAc (100mL), which was washed with brine (100 mL), dried over Na₂SO₄ and concentrated. The residue was purified by silica-gel column, eluting with 0-30% EtOAc in petroleum ether, to obtain (S)-methyl 2-((tert-butoxycarbonyl)amino)-3- (3-hydroxy-4-nitrophenyl)propanoate (1.44 g, 74% yield) as a yellow solid. NMR (400 MHz, CDCl₃): δ 10.59 (s, 1H), 8.04 (d, *J*=8.0 Hz, 1H), 6.94 (s, 1H), 6.78 (d, *J*=8.0 Hz, 1H), 5.07 (d, *J*=7.6 Hz, 1H, NH), 4.62 (br s, 1H, phenol-OH), 3.76 (s, 3H), 3.27-3.05 (m, 2H), 1.43 (s, 9H).

**Step 4:** (S)-methyl 2-amino-3-(4-nitro-3-(((trifluoromethyl)sulfonyl)oxy)phenyl)propanoate was prepared as a white solid from (S)-methyl 2-((tert-butoxycarbonyl)amino)-3-(3-hydroxy -4-nitrophenyl)propanoate by utilizing triflate formation procedure (described in example 10) and Boc removal procedure (described in example 53).

**Step 5:** To a solution of **compound 624-1** (10.0 g, 18.4 mmol) in DCM/MeOH (150 mL, v/v=1:2) was added Ag₂SO₄ (4.0 g, 12.9 mmol) and iodine (5.1 g, 20.2 mmol). The mixture was stirred at 25 °C for 3 h. The mixture was partitioned betwen EtOAc and saturated NaHCO₃ solution (300 mL each) and the organic layer was washed by 5% Na2S2O3 and brine (300 mL each), dried over Na₂SO₄ and concentrated. The residue was purified by silica-gel column, eluting with 40-60% EtOAc in petroleum ether, to give **compound 624-2** (12.0 g, 97% yield) as a yellow solid. LCMS (Method 5-95 AB, ESI): t_{R} = 0.959 min, [M + Na]⁺ = 692.1

**Step 6:** A solution of **compound 624-2** (12.0 g, 18 mmol), Pd(PPh3)2Cl2 (1.26 g, 1.8 mmol), bis(pinacolato)diboron (22.7 g, 90 mmol), KOAc (12.3 g, 126 mmol) in DMSO (40 mL) was stirred at 60°C for 3 h under N₂. The mixture was taken up in EtOAc (500 mL), which was washed with brine (500 mL × 3), dried over Na₂SO₄, concentrated and the residue was purified by silica-gel column, eluting 40-60% EtOAc in petroleum ether, to give **compound 624-3** (9.0 g, 75% yield) as a white solid.
**Step 7: Compound 624-4** was prepared from **compound 624-3** and (S)-methyl-2-amino-3-(4- nitro-3-(((trifluoromethyl)sulfonyl)oxy)phenyl)propanoate by utilizing methods analogous to those described in **101B.** LCMS (Method 5-95 AB, ESI): t_{R} = 0.927 min, [M + Na]⁺ = 756.3

**Step 8:** A solution of **compound 624-4** (100 mg, 0.14mmol) and SnCl₂·2H₂O (308 mg, 1.4 mmol) in EtOAc (10 mL) was stirred at 75°C for 2 h. The mixture was added with EtOAc (30 mL), which was washed with saturated Na₂CO₃ solution and brine (40 mL each), dried over MgSO₄ and concentrated. The resulting residue was re-dissolved in DCM (10 mL), to which Boc₂O (152 µL, 0.66 mmol) and Et₃N (147µL, 1.1 mmol) were added. The mixture was stirred at 25°C for 32 h. The volatiles were removed and the residue was purified by prep-TLC, eluting with 10% MeOH in DCM, to give **compound 624-5** (100 mg, 94% yield) as a white solid. LCMS (Method 5-95 AB, ESI): t_{R} = 0.994 min, [M + Na]⁺ = 826.5

**Compound 624** (FA salt) was prepared from **compound 624-5** as a white solid by utilizing methods analogous to those described in example 54. LCMS (Method 5-95 AB, ESI): t_{R} = 0.730 min, [M + H]⁺ = 860.4; ¹H NMR (400 MHz, MeOH-*d*₄) δ 8.55 (br s, 3H), 8.14 (d, *J*=8.0 Hz, 2H), 7.44 (d, *J*=8.0 Hz, 2H), 7.30-7.22 (m, 2H), 6.89-6.71 (m, 4H), 6.33 (s, 1H), 5.30 (m, 1H), 4.60-4.50 (m, 2H), 4.39-4.20 (m, 2H), 4.26 (s, 2H), 3.08-2.54 (m, 8H), 3.01 (s, 3H), 2.42 (s, 6H), 2.24-2.11 (m, 2H), 1.38 (s, 9H), 1.35 (d, *J*=6.4 Hz, 3H).

### Example 425: Synthesis of Compound 625

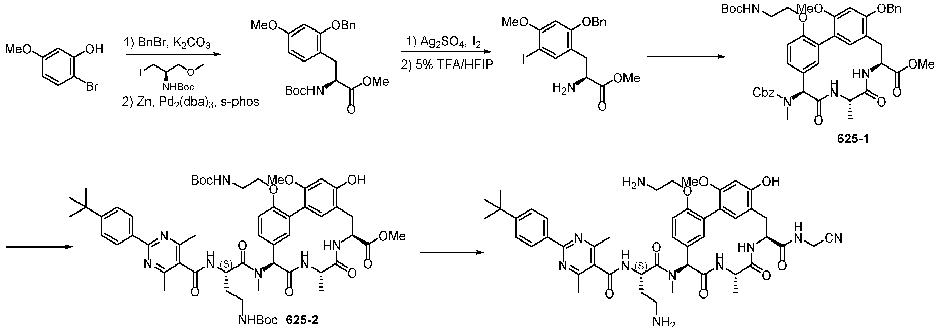

**Step 1**: (S)-methyl 3-(2-(benzyloxy)-4-methoxyphenyl)-2-((tert-butoxycarbonyl)amino) propanoate was prepared as a white solid from 2-bromo-5-methoxyphenol and benzyl bromide by utilizing methods analogous to those described in example 424. ¹H NMR (400 MHz, CDCl₃) δ 7.47-7.31 (m, 5H), 7.02 (d, *J*=8.4 Hz, 1H), 6.51 (d, *J*=2.0 Hz, 1H), 6.44 (dd, *J*=8.4, 2.0 Hz, 1H), 5.12-5.03 (m, 2H), 4.50-4.45 (m, 1H), 3.77(s, 3H), 3.61 (s, 3H), 3.12-2.98 (m, 2H), 1.39 (s, 9H).

Step 2: **Compound 625-1** was prepared as a white solid by utilizing methods analogous to those described in example 424. LCMS (Method 5-95 AB, ESI): t_{R} = 0.991 min, [M + Na]⁺ = 847.5

**Step 3: Compound 625-2** was prepared as a white solid from (S)-2-(((benzyloxy)carbonyl) amino)-4-((tert-butoxycarbonyl)amino)butanoic acid by utilizing methods analogous to those described in example 382. LCMS (Method 5-95 AB, ESI): t_{R} = 0.896 min, [M + H]⁺ = 1068.3

**Compound 625** (FA salt) was prepared as a white solid by utilizing methods analogous to those described in example 382. LCMS (Method 5-95 AB, ESI): t_{R} = 0.743 min, [M + Na]⁺ = 913.5; ¹H NMR (400 MHz, MeOH-*d*₄) δ 8.56 (br s, 2H), 8.08 (d, *J*=7.6 Hz, 2H), 7.43(d, *J*=7.6 Hz, 2H), 7.18 (br s, 2H), 6.77 (br s, 1H), 6.73 (s, 1H), 6.52 (s, 1H), 6.17 (s, 1H), 5.37-5.35 (m, 1H), 4.79-4.74 (m, 2H), 4.39-4.25 (m, 2H), 4.31 (s, 2H), 3.83 (s, 3H), 3.27-3.15 (m, 2H), 3.10-2.95 (m, 2H), 3.02 (s, 3H), 2.79-2.71 (m, 2H), 2.38 (s, 6H), 2.28-2.08 (m, 2H), 1.40 (s, 9H), 1.34 (d, *J*=6.8 Hz, 2H).

### Example 426: Synthesis of Compound 626

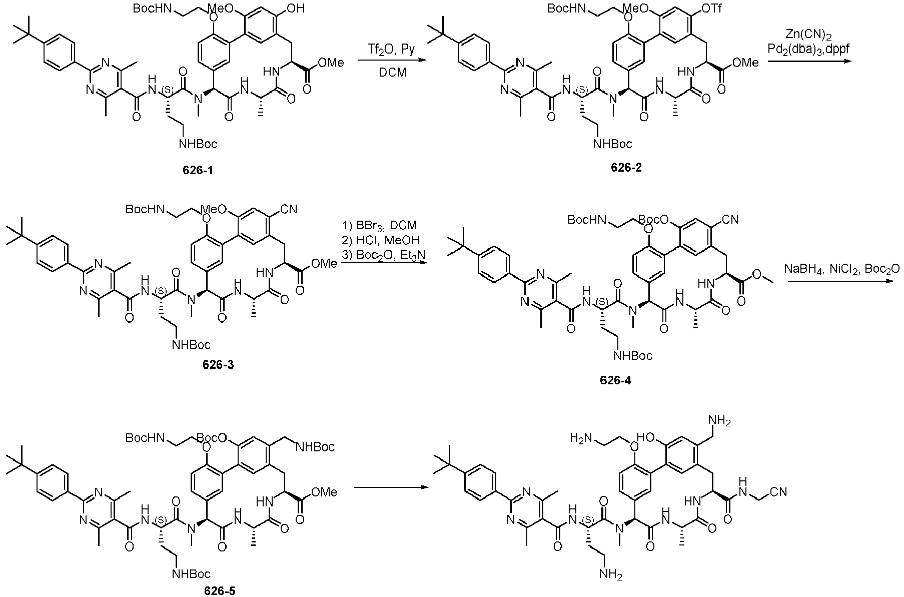

Step 1: **Compound 626-2** was prepared as a white solid from **compound 626-1** (synthesis described in example 425) by utilizing triflate formation procedure described in example 10). LCMS (Method 5-95 AB, ESI): t_{R} = 1.054 min, [M + H]⁺ = 1199.6

Step 2: A solution of **compound 626-2** (240 mg, 0.20 mmol), Zn(CN)₂ (48 mg, 0.40 mmol), dppf (44 mg, 0.08 mmol) and Pd₂(dba)₃ (17 mg, 0.04 mmol) in DMF (8 mL) was stirred at 100°C in 16 h under N₂. The mixture was added with EtOAc (80 mL), which was washed with brine (50 mL × 2), dried over Na₂SO₄ and concentrated. The residue was purified by prep-TLC, eluting with 10% MeOH in DCM, to give **compound 626-3** (160 mg, 74% yield) as a white solid. LCMS (Method 5-95 AB, ESI): t_{R} = 1.148 min, [M + H]⁺ = 1077.0

Step 3: **Compound 626-4** was prepared as a white solid from **compound 626-3** by utilizing the methods described in example 424 except Boc addition procedure where Boc₂O, Et₃N were used (described in example 395). LCMS (Method 5-95 AB, ESI): t_{R} = 1.174 min, [M + Na]⁺ = 1184.3

Step 4: To a solution of **compound 626-4** (50 mg, 0.04 mmol), Boc₂O (57 mg, 0.26 mmol) and NiCl₂·6H₂O (15 mg, 0.06 mmol) in MeOH (4 mL) was added NaBH₄ (11 mg, 0.30 mmol) slowly at 0°C. The reaction was gradually warmed to 25°C while stirring and stirred at the same temperature overnight. The mixture was added with EtOAc (40 mL), which was washed with brine (40 mL), dried over Na₂SO₄ and concentrated. The residue was purified by prep-TLC, eluting with 10% MeOH in DCM, to give **compound 626-5** (30 mg, 55% yield) as a white solid. LCMS (Method 5-95 AB, ESI): t_{R} = 1.133 min, [M + Na]⁺ = 1288.3

**Compound 626** (FA salt) was prepared from **compound 626-5** as a white solid by utilizing methods analogous to those described in example 54. LCMS (Method 5-95 AB, ESI): t_{R} = 0.760 min, [M + H]⁺ = 891.0; ¹H NMR (400 MHz, MeOH-*d*₄) δ 8.49 (br s, 2H), 8.32 (d, *J*=8.4 Hz, 2H), 7.53 (d, *J*=8.4 Hz, 1H), 7.30 (d, *J*=8.4 Hz, 1H), 7.18 (d, *J*=8.4 Hz, 1H), 7.01 (s, 1H), 6.92 (s, 1H), 6.81 (s, 1H), 6.45 (s, 1H), 5.24-5.21 (m, 2H), 4.85-4.80 (m, 1H), 4.40-4.32 (m, 2H), 4.22 (s, 2H), 4.10-4.04 (m, 2H), 3.35-3.11 (m, 6H), 2.98 (s, 3H), 2.55 (s, 6H), 2.29-2.13 (m, 2H), 1.38 (s, 9H), 1.35 (d, *J*=6.8 Hz, 3H).

### Example 427: Synthesis of Compound 627

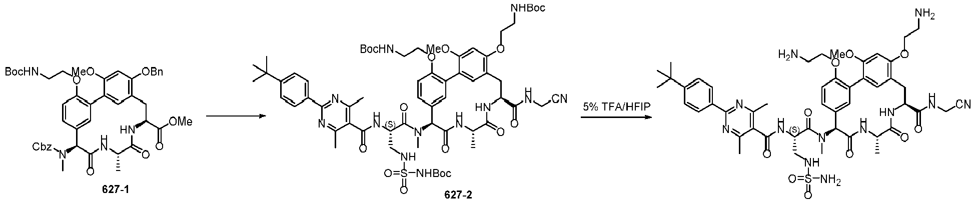

**Compound 627-2** was prepared from **compound 627-1** (described in example 425) and tert-butyl (2-bromoethyl)carbamate, by utilizing methods analogous to those described in example 382. LCMS (Method 5-95 AB, ESI): t_{R} = 1.011 min, [M + H]⁺ = 1299.6

**Compound 631** (FA salt) was prepared from **compound 627-2** as a white solid by utilizing Boc removal condition described in example 53. LCMS (Method 5-95 AB, ESI): t_{R} = 0.785 min, [M + H]⁺= 999.6; ¹H NMR (400 MHz, MeOH-*d*₄) δ 8.53 (br s, 3H), 8.19 (d, *J*=7.6 Hz, 2H), 7.47 (d, *J*=7.6 Hz, 2H), 7.24-7.16 (m, 3H), 6.77 (s, 1H), 6.72 (s, 1H), 6.62 (br s, 1H), 6.52 (s, 1H), 5.42-5.40 (m, 1H), 4.78-4.62 (m, 2H), 4.27-4.22 (m, 6H), 3.88 (s, 3H), 3.58-3.53 (m, 2H), 3.40-3.38 (m, 1H), 3.05 (s, 3H), 2.99-2.96 (m, 2H), 2.90-2.75 (m, 3H), 2.49 (s, 6H), 1.38 (s, 9H), 1.37 (d, *J*=6.4 Hz, 3H).

### Example 428: Synthesis of Compound 628

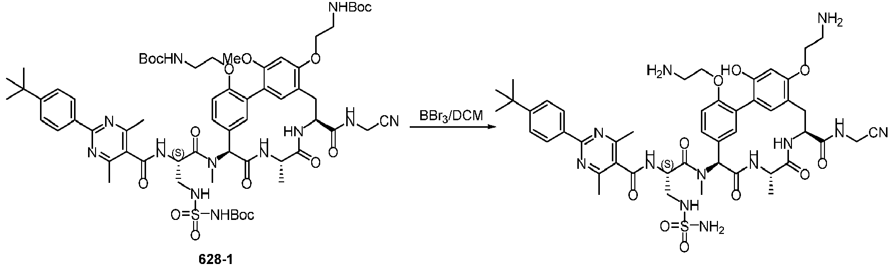

To a solution of **compound 628-1** (synthesis described in example 427, 50 mg, 0.04 mmol) in DCM (4 mL) was added BBr₃ (36 µL, 0.38 mmol) at 0°C. The reaction was gradually warmed to 25°C while stirring and stirred at the same temperature for 16 h. The reaction was quenched by water and the resulting mixture was lyophilized immediately. The residue was purified by Prep-HPLC (acetonitrile 23-33/0.225%FA in water) to afford **compound 628** (FA salt) as a white solid (2.3 mg, 5.9% yield). LCMS (Method 5-95 AB, ESI): t_{R} = 0.793 min, [M + Na]⁺ = 1007.8; ¹H NMR (400 MHz, MeOH-*d*₄) δ 8.53 (br s, 3H), 8.22 (d, *J*=7.6 Hz, 2H), 7.48 (d, *J*=7.6 Hz, 2H), 7.21 (d, *J*=8.0 Hz, 1H), 7.16 (d, *J*=8.0 Hz, 1H), 6.82 (s, 1H), 6.63 (br s, 1H), 6.53 (s, 1H), 6.50 (br s, 1H), 5.39-5.35 (m, 2H), 4.81-4.77 (m, 1H), 4.65-4.15 (m, 6H), 4.21 (s, 2H), 3.58-3.53 (m, 2H), 3.39-3.33 (m, 4H), 3.10 (s, 3H), 3.05-2.98 (m, 2H), 2.50 (s, 6H), 1.36 (s, 9H), 1.35 (d, *J*=6.4 Hz, 3H).

### Example 429: Synthesis of Compound 629

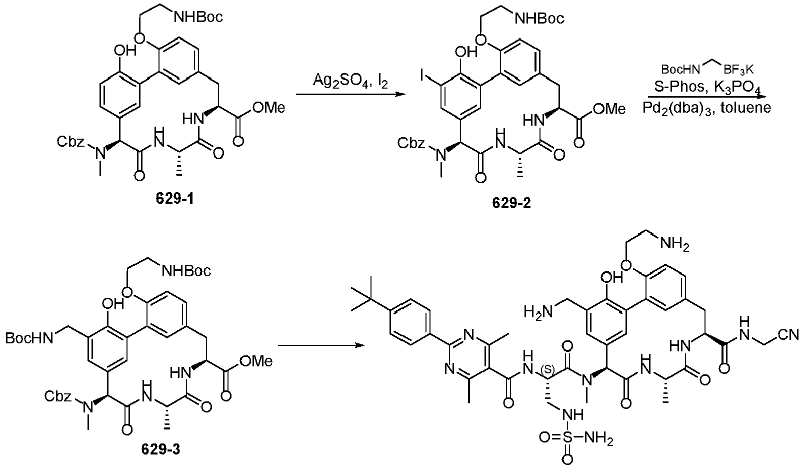

**Step 1: Compound 629-2** was prepared from **compound 629-1** (synthesis described in example 371), by utilizing iodination condition described in example 382. LCMS (Method 5-95 AB, ESI): t_{R}= 0.925 min, [M + Na]⁺ = 853.1

**Step 2:** A solution of **compound 629-2** (200 mg, 0.24 mmol), Pd₂dba₃ (11 mg, 0.01 mmol), SPhos (10 mg, 0.02 mmol), potassium tert-butyl N-(difluoroboranylmethyl)carbamate fluoride (63 mg, 0.26 mmol) and K₃PO₄ (153 mg, 0.72 mmol) in toluene (2 mL) and H₂O (0.10 mL) at 85°C for 16 h under N₂. The volatiles were removed and the residue was added with EtOAc (30 mL). After filtration, the filtrate was washed with brine (35 mL × 3), dried over Na₂SO₄, concentrated and the residue was purified by prep-TLC, eluting with 10% MeOH in DCM, to give **compound 629-3** (110 mg, 55% yield) as a yellow solid. LCMS (Method 5-95 AB, ESI): t_{R} = 0.926 min, [M + Na]⁺ = 856.1

**Compound 629** (FA salt) was prepared as a white solid by utilizing methods analogous to those described in example 382. LCMS (Method 5-95 AB, ESI): t_{R} = 0.785 min, [M + H]⁺ = 955.5; ¹H NMR (400 MHz, MeOH-*d*₄) δ 8.44 (brs, 1H), 8.32 (d, *J*=8.0 Hz, 1H), 7.53 (d, *J*=8.4 Hz, 2H), 7.23 (d, *J*=8.0 Hz, 1H), 7.15 (s, 1H), 7.07 (d, *J*=8.0 Hz, 1H), 6.93 (brs, 1H), 6.86 (s, 1H), 6.79 (s, 1H), 6.48 (s, 1H), 5.30-5.27 (m, 1H), 4.90-4.81 (m, 2H), 4.35 (s, 2H), 4.21 (s, 2H), 4.10-4.00 (m, 2H), 3.60-3.55 (m, 2H), 3.25-3.10 (m, 4H), 3.06 (s, 3H), 2.59 (s, 6H), 1.38 (s, 9H), 1.36 (d, *J*=6.0 Hz, 3H).

### Examples 430-437: Synthesis of compounds 630-637

The following compounds in table 4 were prepared by utilizing methods analogous to those previously described.

**Table 4**

| **Comp. #** | **Structure** |
|---|---|
| 630 | 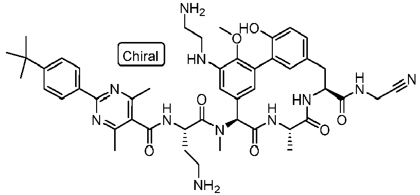 |
| 631 | 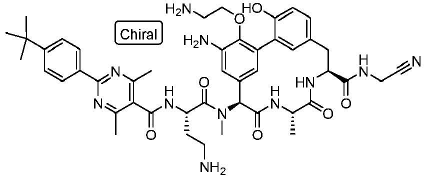 |
| 632 | 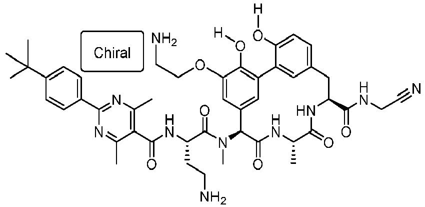 |
| 633 | 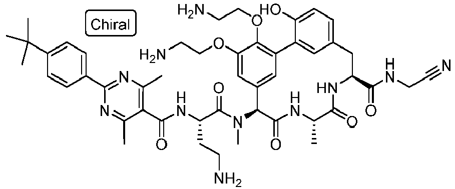 |
| 634 | 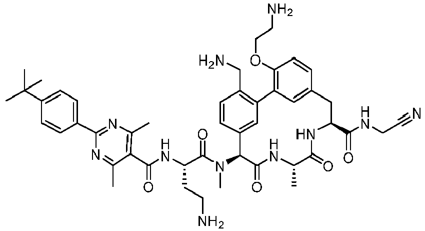 |
| 635 | 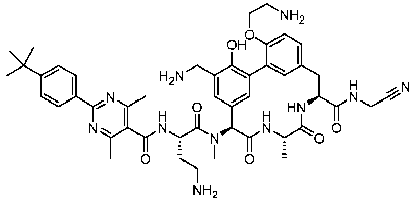 |
| 636 | 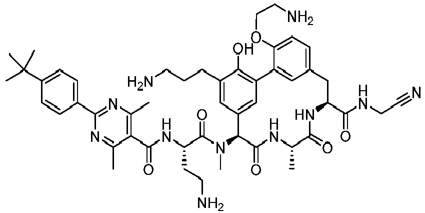 |
| 637 | 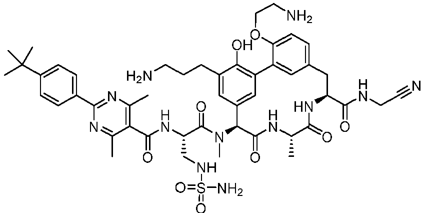 |

### Example 438-449: Synthesis of compounds 638-649

The following compounds in table 5 were prepared by utilizing methods analogous to those previously described.

**Table 5**

| **Comp. #** | **Structure** |
|---|---|
| **638** | 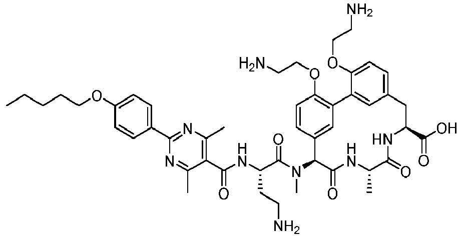 |
| **639** | 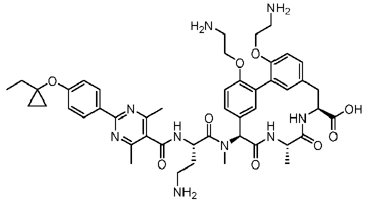 |
| **640** | 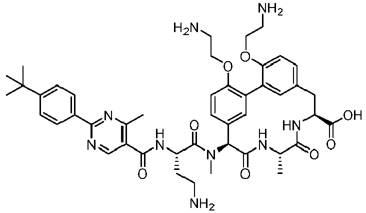 |
| **641** | 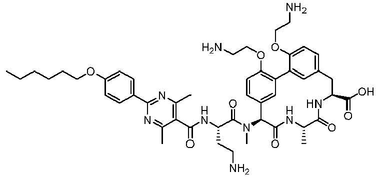 |
| **642** | 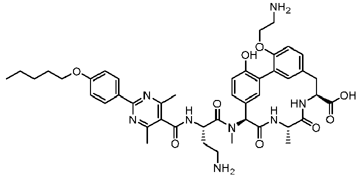 |
| **643** | 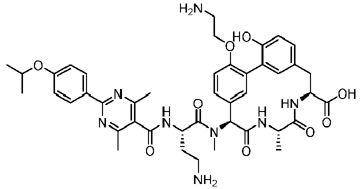 |
| **644** | 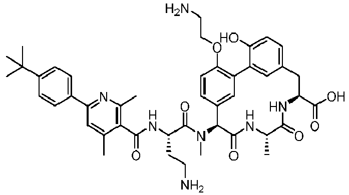 |
| **645** | 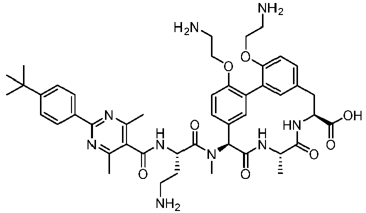 |
| **646** | 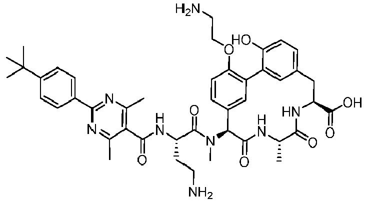 |
| **647** | 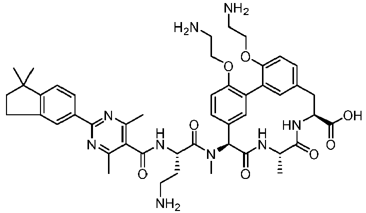 |
| **648** | 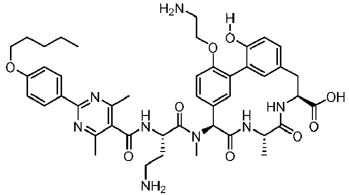 |
| **649** | 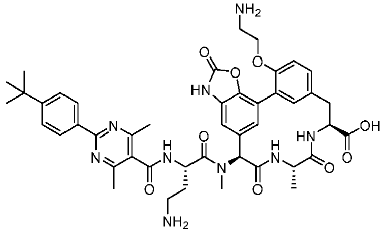 |

### Biological Assays

### Example B1: Determination of Minimum Inhibitory Concentration

In vitro antimicrobial activity of each compound was determined by measuring minimal inhibitor concentrations (MICs) using the broth micro-dilution technique as approved by the Clinical and Laboratory Standards Institute (CLSI) (Methods for Dilution Antimicrobial Susceptibility Tests for Bacteria that Grow Aerobically; Approved Standard - Eighth Edition. CLSI document M07-A8. Wayne, PA: Clinical and Laboratroy Standards; 2009). Antibacterial activity was measure against two strains of bacteria: a Methicillin Resistant *Staphylococcus aureus* strain USA 200, (*S. aureus*) and *Escherichia coli* ATCC 25922 (*E. coli*)*,* a clinically relevant Gram-negative strain. Cells were inoculated onto plates of Trypyticase Soy Agar or Luria Agar respectively and grown at 35°C for 20 hours. Inocula suspensions were prepared by scraping cells into 1 mL of testing media (cation adjusted Mueller Hinton Broth supplemented with 0.002% v/v Tween-80) and diluting to a final OD₆₀₀ₙₘ of 0.01.

Test compounds were prepared in DMSO at a concentration of 10 mg/mL. The compounds were tested under several different dilution formats and the data are reported in Tables 6, 7 and 8. In protocol 1, the compound stocks were diluted into testing media at a concentration of 64 µg/ml and serial 2-fold dilutions were made in the same media, in 96-well U bottom microtiter dishes, for a total of 10 compound concentrations. In protocol 2, the compound stocks were diluted into testing media at a concentration of 4 µg/mL and serial 2-fold dilutions were made in the same media, in 96-well U bottom microtiter dishes, for a total of 10 compound concentrations. In protocol 3, compound stocks were diluted into testing media at a concentration of 0.5 µg/mL, with serial 2-fold dilutions conducted as described above. In protocol 4, compound stocks were diluted into testing media at a concentration of 0.13 µg/mL, with serial 2-fold dilutions conducted as described above. Inocula suspensions were added to the 2-fold serial dilutions of test compounds to a final density of OD OD₆₀₀ₙₘ of 0.0005 and incubated at 35°C for 22 hours. After incubation the plates were examined visually and the lowest concentration of test compound that completely prevented bacterial growth were recorded as the MICs. The results are listed in Table 6, 7, and 8.

**Table 6**

| **Comp. #** | **MIC *S*. *aureus* µM** | **MIC *E*. *coli* µM** | **Comp. #** | **MIC *S. aureus* µM** | **MIC *E*. *coli* µM** |
|---|---|---|---|---|---|
| **201** | 0.062 | 0.42 | **381** | 0.067 | 4.3 |
| **202** | 0.023 | 0.37 | **382** | 0.4 | 4.3 |
| **203** | 0.031 | 0.13 | **383** | 0.1 | 1.1 |
| **204** | 0.032 | 2.1 | **384** | 0.033 | 0.4 |
| **205** | 0.012 | 0.066 | **385** | 0.017 | 0.27 |
| **206** | 0.025 | 0.13 | **386** | 0.75 | 8 |
| **207** | 0.017 | 0.1 | **387** | 0.25 | 8 |
| **208** | 0.017 | 0.14 | **388** | 0.031 | 1 |
| **209** | 0.26 | 2.1 | **389** | 0.13 | 4 |
| **210** | 0.022 | 0.35 | **390** | 0.094 | 8 |
| **211** | 0.026 | 0.17 | **391** | 0.031 | 0.25 |
| **212** | 0.017 | 0.067 | **392** | 0.063 | 3 |
| **213** | 0.28 | 1.1 | **393** | 0.021 | 8.2 |
| **214** | 0.034 | 0.41 | **394** | 0.0083 | 0.2 |
| **215** | 0.017 | 0.13 | **395** | 0.013 | 0.27 |
| **216** | 0.0084 | 0.2 | **396** | 0.016 | 0.5 |
| **217** | 0.0082 | 0.099 | **397** | 0.023 | 0.38 |
| **218** | 0.012 | 0.097 | **398** | 0.025 | 0.27 |
| **219** | 0.012 | 0.13 | **399** | 0.0078 | 0.5 |
| **220** | 0.0062 | 0.099 | **400** | 0.047 | 0.25 |
| **221** | 0.024 | 1 | **401** | 0.016 | 0.25 |
| **222** | 0.033 | 0.53 | **402** | 0.031 | 0.38 |
| **223** | 0.016 | 0.26 | **403** | 0.016 | 0.5 |
| **224** | 0.017 | 0.13 | **404** | 0.094 | 0.38 |
| **225** | 0.016 | 0.19 | **405** | 0.063 | 0.38 |
| **226** | 0.0082 | 0.099 | **406** | 0.023 | 0.25 |
| **227** | 0.017 | 0.077 | **407** | 0.016 | 0.13 |
| **228** | 0.57 | 2.3 | **408** | 0.023 | 0.38 |
| **229** | 0.57 | 4.6 | **409** | 0.13 | 0.75 |
| **230** | 0.012 | 0.066 | **410** | 0.016 | 0.3 |
| **231** | 0.35 | 1.4 | **411** | 0.016 | 0.25 |
| **232** | 0.015 | 0.16 | **412** | 0.023 | 0.5 |
| **233** | 0.016 | 0.13 | **413** | 0.016 | 0.13 |
| **234** | 0.0082 | 0.066 | **414** | 0.016 | 0.25 |
| **235** | 0.016 | 0.26 | **415** | 0.063 | 1.5 |
| **236** | 0.016 | 0.13 | **416** | 0.094 | 3 |
| **237** | 0.0083 | 0.27 | **417** | 0.0062 | 0.13 |
| **238** | 0.53 | 2.1 | **418** | 0.008 | 0.064 |
| **239** | 0.015 | 0.12 | **419** | 0.063 | 8 |
| **240** | 0.012 | 0.13 | **420** | 0.75 | 3 |
| **241** | 0.0081 | 0.097 | **421** | 0.031 | 0.19 |
| **242** | 0.016 | 0.39 | **422** | 0.031 | 0.38 |
| **243** | 0.008 | 0.13 | **423** | 0.13 | 2 |
| **244** | 0.012 | 0.13 | **424** | 0.023 | 0.19 |
| **245** | 0.0081 | 0.097 | **425** | 0.016 | 0.19 |
| **246** | 0.067 | 0.4 | **426** | 0.023 | 0.12 |
| **247** | 0.016 | 0.19 | **427** | NT | 0.094 |
| **248** | 0.016 | 0.13 | **428** | NT | 0.5 |
| **249** | 0.043 | 0.35 | **429** | 0.023 | 1 |
| **250** | 0.066 | 0.35 | **430** | 0.15 | 4.7 |
| **251** | 0.067 | 1.1 | **431** | 0.18 | 3.9 |
| **252** | 0.0081 | 0.097 | **432** | 0.045 | 1.4 |
| **253** | 0.012 | 0.14 | **433** | 0.016 | 0.096 |
| **254** | 0.061 | 0.24 | **434** | 0.023 | 0.21 |
| **255** | 0.0082 | 0.066 | **435** | 0.012 | 0.094 |
| **256** | 0.0078 | 0.1 | **436** | NT | 0.063 |
| **257** | 0.016 | 0.13 | **437** | 0.031 | 0.38 |
| **258** | 0.012 | 0.13 | **438** | 0.012 | 0.16 |
| **259** | 0.025 | 0.26 | **439** | 0.016 | 0.076 |
| **260** | 0.079 | 0.45 | **440** | NT | 1 |
| **261** | 0.014 | 0.1 | **441** | NT | 0.38 |
| **262** | 0.0081 | 0.097 | **442** | 0.042 | 0.91 |
| **263** | 0.016 | 0.53 | **443** | 0.27 | 4.2 |
| **264** | 0.52 | 1.6 | **444** | 0.064 | 4.1 |
| **265** | 0.068 | 0.41 | **445** | 0.13 | 4.1 |
| **266** | 0.033 | 0.2 | **446** | 0.032 | 4.1 |
| **267** | 0.39 | 1 | **447** | 0.033 | 4.2 |
| **268** | 0.016 | 0.12 | **448** | 0.13 | 4.1 |
| **269** | 0.016 | 0.094 | **449** | 0.016 | 0.5 |
| **270** | 0.012 | 0.064 | **450** | 0.016 | 2.1 |
| **271** | 0.032 | 0.26 | **451** | 0.047 | 0.76 |
| **272** | 0.012 | 0.065 | **452** | 0.13 | 1.3 |
| **273** | 0.0082 | 0.099 | **453** | 0.026 | 1.1 |
| **274** | 0.023 | 0.063 | **454** | 0.55 | 4.4 |
| **275** | 0.012 | 0.094 | **455** | NT | NT |
| **276** | 0.016 | 0.063 | **456** | 0.56 | 3.3 |
| **277** | 0.016 | 0.13 | **457** | 0.025 | 0.27 |
| **278** | 0.0078 | 0.094 | **458** | 0.033 | 1.1 |
| **279** | 0.023 | 0.13 | **459** | 0.083 | 0.71 |
| **280** | 0.031 | 0.13 | **460** | 0.13 | 4.1 |
| **281** | 0.016 | 0.065 | **461** | 0.051 | 1.1 |
| **282** | 0.016 | 0.094 | **462** | 0.027 | 5.4 |
| **283** | 0.012 | 0.094 | **463** | NT | NT |
| **284** | 0.016 | 0.13 | **464** | 0.063 | 4 |
| **285** | 0.047 | 0.19 | **465** | 0.047 | 4 |
| **286** | 0.016 | 0.13 | **466** | 0.047 | 1.5 |
| **287** | 0.065 | 1 | **467** | 0.19 | 8 |
| **288** | 0.023 | 1.5 | **468** | 0.063 | 1 |
| **289** | 0.016 | 0.75 | **469** | 0.13 | 6 |
| **290** | 0.19 | 0.75 | **470** | 0.047 | 4 |
| **291** | 0.094 | 0.25 | **471** | 0.04 | 5.1 |
| **292** | 0.13 | 0.75 | **472** | 0.0085 | 0.11 |
| **293** | 0.13 | 1.5 | **473** | 0.31 | 4.9 |
| **294** | 0.031 | 0.19 | **474** | 0.11 | 4.8 |
| **295** | 0.0078 | 0.063 | **475** | 0.6 | 4.8 |
| **296** | 0.016 | 0.38 | **476** | 0.025 | 1.1 |
| **297** | 0.016 | 0.75 | **477** | 0.26 | 3.2 |
| **298** | 0.016 | 0.13 | **478** | 0.071 | 1.1 |
| **299** | 0.047 | 0.38 | **479** | 0.027 | 0.77 |
| **300** | 0.031 | 0.25 | **480** | 0.061 | 1.6 |
| **301** | 0.016 | 0.094 | **481** | 0.037 | 2.3 |
| **302** | NT | 0.5 | **482** | 0.036 | 0.24 |
| **303** | NT | 0.13 | **483** | 0.036 | 0.29 |
| **304** | NT | 0.13 | **484** | NT | NT |
| **305** | NT | 0.13 | **485** | 0.023 | 0.5 |
| **306** | 0.84 | 14 | **486** | 0.023 | 0.38 |
| **307** | 0.033 | 0.2 | **487** | 0.016 | 0.38 |
| **308** | 0.012 | 0.096 | **488** | NT | 0.5 |
| **309** | 0.27 | 1.1 | **489** | 0.016 | 0.38 |
| **310** | 0.066 | 0.53 | **490** | 0.023 | 1 |
| **311** | 1 | 8.2 | **491** | 0.049 | 3.1 |
| **312** | 0.024 | 0.063 | **492** | 0.016 | 2 |
| **313** | 0.016 | 0.13 | **493** | 0.047 | 1 |
| **314** | 0.033 | 0.2 | **494** | 0.068 | 1.1 |
| **315** | 0.016 | 0.25 | **495** | 0.031 | 1 |
| **316** | 0.38 | 1.5 | **496** | 0.094 | 1 |
| **317** | 0.047 | 0.5 | **497** | 0.047 | 0.75 |
| **318** | 0.016 | 0.38 | **498** | 0.063 | 1 |
| **319** | 1 | 8 | **499** | 0.063 | 1.5 |
| **320** | 0.016 | 0.19 | **500** | 0.031 | 0.48 |
| **321** | 0.047 | 0.38 | **501** | 0.063 | 1 |
| **322** | 0.15 | 0.6 | **502** | 0.078 | 0.24 |
| **323** | 0.38 | 1 | **503** | 0.047 | 1 |
| **324** | 0.047 | 0.13 | **504** | 0.063 | 0.75 |
| **325** | 0.016 | 0.13 | **505** | 0.094 | 1.5 |
| **326** | 0.031 | 0.13 | **506** | 0.094 | 0.83 |
| **327** | NT | 0.13 | **507** | 0.063 | 0.38 |
| **328** | 0.0093 | 0.07 | **508** | 1 | 6 |
| **329** | 0.011 | 0.11 | **509** | 0.047 | 0.5 |
| **330** | 0.033 | 0.26 | **510** | 0.023 | 1.5 |
| **331** | 0.0081 | 0.13 | **511** | NT | 2 |
| **332** | 0.0078 | 0.063 | **512** | NT | 1 |
| **333** | 1 | 2 | **513** | 0.0061 | 0.097 |
| **334** | 0.5 | 4 | **514** | 0.031 | 0.19 |
| **335** | 0.032 | 0.19 | **516** | 0.19 | 2 |
| **336** | 0.016 | 0.15 | **517** | 0.19 | 0.75 |
| **337** | 0.012 | 0.11 | **518** | 0.028 | 0.16 |
| **338** | 0.012 | 0.13 | **519** | NT | 0.13 |
| **339** | 0.047 | 0.25 | **520** | NT | 1 |
| **340** | 0.023 | 0.063 | **521** | 0.047 | 0.2 |
| **341** | 0.016 | 0.13 | **522** | NT | 0.13 |
| **342** | 0.097 | 0.39 | **523** | 0.044 | 0.28 |
| **343** | 0.13 | 0.75 | **524** | 0.56 | 4.5 |
| **344** | 0.063 | 0.38 | **525** | 0.053 | 0.56 |
| **345** | 0.023 | 0.25 | **526** | 0.047 | 0.33 |
| **346** | 0.016 | 0.13 | **527** | NT | 0.38 |
| **347** | 0.047 | 0.25 | **528** | NT | 0.063 |
| **348** | 0.023 | 0.19 | **529** | NT | 4 |
| **349** | 0.023 | 0.094 | **530** | 0.063 | 0.35 |
| **350** | 0.016 | 0.094 | **531** | 0.047 | 0.25 |
| **351** | NT | 0.25 | **532** | NT | 0.063 |
| **352** | 0.023 | 0.094 | **533** | NT | 1.5 |
| **353** | 0.047 | 2 | **534** | NT | 0.5 |
| **354** | 0.016 | 1.5 | **535** | 0.029 | 0.23 |
| **355** | 0.094 | 4 | **536** | 0.016 | 0.13 |
| **356** | NT | 0.13 | **537** | NT | 0.25 |
| **357** | NT | 0.19 | **538** | NT | 0.063 |
| **358** | 0.033 | 0.2 | **539** | 0.023 | 0.15 |
| **359** | 0.008 | 0.064 | **540** | NT | 1.5 |
| **360** | 0.066 | 0.26 | **541** | NT | 0.75 |
| **361** | 0.016 | 0.13 | **542** | 0.55 | 4.4 |
| **362** | 0.063 | 0.25 | **543** | 0.063 | 0.35 |
| **363** | 0.016 | 0.19 | **544** | NT | 0.13 |
| **364** | 0.016 | 0.25 | **545** | NT | 1 |
| **365** | 0.063 | 0.31 | **546** | NT | 0.38 |
| **366** | 0.016 | 0.13 | **547** | NT | 0.094 |
| **367** | NT | 0.5 | **548** | 0.047 | 0.46 |
| **368** | 0.047 | 0.5 | **549** | 0.035 | 0.21 |
| **369** | 0.047 | 0.75 | **550** | NT | 2 |
| **370** | 0.031 | 0.19 | **551** | NT | 1 |
| **371** | 0.031 | 0.19 | **552** | 0.25 | 1.4 |
| **372** | 0.023 | 0.38 | **553** | 0.055 | 0.21 |
| **373** | NT | 0.13 | **554** | NT | 0.094 |
| **374** | 0.016 | 0.25 | **555** | NT | 0.19 |
| **375** | 0.031 | 0.5 | **556** | NT | 0.19 |
| **376** | 0.016 | 0.25 | **557** | NT | 0.25 |
| **377** | 0.031 | 0.5 | **558** | 0.046 | 0.73 |
| **378** | 0.031 | 0.38 | | | |
| **379** | 0.26 | 1 | | | |
| **380** | 0.045 | 0.48 | | | |

**Table 7**

| **Cp#** | **MIC *S*. *aureus* µM** | **MIC *E*. *coli* µM** | **Cp#** | **MIC *S*. *aureus* µM** | **MIC *E*. *coli* µM** |
|---|---|---|---|---|---|
| **559** | NT | 0.38 | **597** | NT | 0.26 |
| **560** | NT | 0.25 | **598** | NT | 0.094 |
| **561** | NT | 0.75 | **599** | NT | 0.58 |
| **562** | NT | 0.094 | **600** | NT | 0.38 |
| **563** | NT | 0.25 | **601** | NT | 0.50 |
| **564** | NT | 0.063 | **602** | NT | 0.063 |
| **565** | NT | 0.13 | **603** | NT | 0.44 |
| **566** | NT | 0.094 | **604** | NT | 0.75 |
| **567** | NT | 0.094 | **605** | NT | 0.75 |
| **568** | NT | 0.063 | **606** | NT | 0.50 |
| **569** | NT | 0.50 | **607** | NT | 0.50 |
| **570** | NT | 0.063 | **608** | NT | 0.38 |
| **571** | NT | 0.094 | **609** | NT | 0.50 |
| **572** | NT | 0.28 | **610** | NT | 0.19 |
| **573** | NT | 0.50 | **611** | NT | 0.19 |
| **574** | NT | 0.50 | **612** | NT | 0.25 |
| **575** | NT | 0.31 | **613** | NT | 0.75 |
| **576** | NT | 0.50 | **614** | NT | 0.19 |
| **577** | NT | 0.21 | **615** | NT | 1.0 |
| **578** | NT | 0.19 | **616** | NT | 0.50 |
| **579** | NT | 0.75 | **617** | NT | 0.19 |
| **580** | NT | 0.58 | **618** | NT | 0.19 |
| **581** | NT | 0.25 | **619** | NT | 0.13 |
| **582** | NT | 0.75 | **620** | NT | 0.50 |
| **583** | NT | 0.17 | **621** | NT | 0.25 |
| **584** | NT | 0.50 | **622** | NT | 0.50 |
| **585** | NT | 0.38 | **623** | NT | 0.50 |
| **586** | NT | 0.19 | **624** | NT | 0.38 |
| **587** | NT | 0.094 | **625** | NT | 0.38 |
| **588** | NT | 0.094 | **626** | NT | 0.25 |
| **589** | NT | 1.0 | **627** | NT | 0.50 |
| **590** | NT | 0.38 | **628** | NT | 0.44 |
| **591** | NT | 0.25 | **630** | NT | 0.25 |
| **592** | NT | 0.19 | **631** | NT | 0.19 |
| **593** | NT | 1.5 | **632** | NT | 0.50 |
| **594** | NT | 0.13 | **633** | NT | 0.047 |
| **595** | NT | 0.33 | **634** | NT | 0.25 |
| **596** | NT | 0.50 | | | |

**Table 8**

| **Comp. #** | **MIC *S*. *aureus* µM** | **MIC *E*. *coli* µM** |
|---|---|---|
| **638** | 0.19 | 0.22 |
| **639** | 0.75 | 0.38 |
| **640** | NT | 0.25 |
| **641** | 0.047 | 0.094 |
| **642** | 0.21 | 0.23 |
| **643** | 1.1 | 2.3 |
| **644** | 1.2 | 0.58 |
| **645** | 0.52 | 0.4 |
| **646** | 1.1 | 0.37 |
| **647** | 0.19 | 0.25 |
| **648** | 0.28 | 0.1 |

| | | |
|---|---|---|
| NT = not tested | | |

### Example B2: Whole-cell SpsB Biochemical Screening Assay

A kinetic fluorogenic enzyme activity assay is used to assess inhibition of SpsB (*Staphylococcus aureus* signal peptidase) activity and IC₅₀s are determined. This assay uses a suspension of *Staphylococcus aureus* cells as a source of SpsB instead of recombinant SpsB protein.

Cell preparation: Luria broth (LB) is inoculated with *S*. *aureus* (USA300 background, overexpressing SpsB) and shaken at 37°C until an OD₆₀₀ₙₘ of 1.5-2.0 is reached (~4 hr). The culture is then diluted to an OD₆₀₀ₙₘ of 1.0 with LB, aliquoted and centrifuged at 10,000x g for 2 mins. The supernatant is removed and the pellet is resuspended in phosphate buffer (1x PBS, 12.5 mg/L MgCl₂, 25 mg/L CaCl₂, 0.1% Tween-80) to an OD₆₀₀ₙₘ of 0.5, then centrifuged again at 10,000x g for 2 mins. The supernatant is removed and the pellets are frozen at -20°C.

Test compounds are prepared in DMSO at a concentration of 10 mg/mL. These compound stocks are diluted into DMSO to a concentration of 25 µg/mL and serial 3-fold dilutions are made in DMSO, for a total of 11 compound concentrations. 20 nL of each compound solution is pre-spotted into a white 384-well plate (50 µL/well polypropylene, Nunc) using acoustic fluid transfer (Echo).

Frozen S. aureus pellets are resuspended in assay buffer (1x PBS, 12.5 mg/L MgCl2, 25 mg/L CaCl2, 0.1% Tween-80) to an OD600nm of 0.05, then mixed 1:1 (v/v) with 20 µM substrate ((Dabcyl)βAla-KPAKAAE(Edans)) in assay buffer, and this solution is added (20 µL/well) to the 384-well plate that has been pre-spotted with compound. Fluorescence intensity is then immediately read kinetically for 30 minutes with 2 minute read intervals to monitor cleavage of the internally quenched peptide substrate (excitation wavelength = 340 nm, emission wavelength = 490nm, Molecular Devices Spectramax M5). Reaction rate (slope) is plotted against inhibitor concentration to derive the IC₅₀.

### Example B3: Activity in a neutropenic thigh infection model

The ability of a compound to inhibit an infection of a bacterial pathogen can be measured using a murine neutropenic thigh infection model. The reduction of bacterial burden is a measure of antibacterial activity *in vivo.*

Jugular vein cannulated CD-1 mice are subjected to induced neutropenia (<100 cells/mm³) by injecting 150 mg/kg and 100 mg/kg cyclophosphamide at day -5 and day -2 respectively. At day -1, saline is infused at 20 µL/hour for 12 hours using Harvard Apparatus PHD 2000 Infusion pumps. At day 0, mice are infected in the thigh muscle with with 1×10⁵ CFU/ 50 µL of *Escherichia coli* strain ATCC 25922. There are four test groups and one vehicle group that begin dosing at 1 hour post infection:
Group 1 - vehicle control (3% HP-beta-cyclodextrin in PBS)
Group 2 - Compound disclosed herein group dosed at a concentration of 0.62 mg/mL solution,
infused at 80µL/hour for 23 hours, with a target steady-state concentration (Css) of 13 µg/mL.
Group 3 - Compound disclosed herein group dosed at a concentration of 0.21 mg/mL solution,
infused at 80µL/hour for 23 hours to achieve a steady state concentration (Css) of 3.4 µg/mL.
Group 4 - Compound disclosed herein group dosed at 0.07 mg/mL solution (Css 1.2 ug/mL) infused at 80µL/hour for 23 hours.
Group 5 - Compound disclosed herein group dosed at 0.02 mg/mL solution (Css 0.31 ug/mL) infused at 80µL/hour for 23 hours.

At 24 hours post infection, bacterial burden in the thigh muscle is determined by plating the tissue homogenate in serial dilutions on blood agar plates.

### Example B4: Clinical Trial of the Safety and Efficacy of Compounds of Formula (I), (Ia)-(If), (II), (IIa)-(IIe), (III), (IIIa)-(IIIc), (IV), (IVa)-(IVc), (V), or (Va)-(Vc) in Patients with C. Difficile-Associated Diarrhea

**Purpose**: This study aims to determine the safety and efficacy of compounds presented herein for the treatment of symptoms of C. difficile-associated diarrhea and lowering the risk of repeat episodes of diarrhea. The compounds are evaluated in comparison to current standard antibiotic treatment, so all patients will receive active medication. All study-related care is provided including doctor visits, physical exams, laboratory tests and study medication. Total length of participation is approximately 10 weeks.

**Patients:** Eligible subjects will be men and women 18 years and older.

### Criteria:

### Inclusion Criteria:

Be at least 18 years old;
Have active mild to moderate C. difficile- Associated Diarrhea (CDAD);
Be able to tolerate oral medication;
Not be pregnant or breast-feeding; and
Sign and date an informed consent form.

**Study Design:** This is a randomized, double-blind, active control study of the efficacy, safety, and tolerability of a compound of Formula (I), (Ia)-(If), (II), (IIa)-(IIe), (III), (IIIa)-(IIIc), (IV), (IVa)-(IVc), (V), or (Va)-(Vc) in patients with C. difficile-associated diarrhea.

### Example B5: Clinical Trial Comparing a Compound of Formula (I), (Ia)-(If), (II), (IIa)-(IIe), (III), (IIIa)-(IIIc), (IV), (IVa)-(IVc), (V), or (Va)-(Vc) with Vancomycin for the Treatment of MRSA Osteomyleitis

**Purpose:** This study aims to determine the efficacy of compounds presented herein as compared to vancomycin for the treatment of methicillin-resistant Staphylococcus aureus (MRSA) osteomyelitis.

**Patients:** Eligible subjects will be men and women 18 years and older.

### Criteria:

### Inclusion Criteria:

Culture-proven MRSA, obtained in operating room or sterile biopsy procedure from bone site. The infection and sampling site is either within the bone or a deep soft-tissue site that is contiguous with bone;
OR radiographic abnormality consistent with osteomyelitis in conjunction with a positive blood culture for MRSA;
Surgical debridement of infection site, as needed;
Subject is capable of providing written informed consent; and
Subject capable of receiving outpatient parenteral therapy for 12 weeks.

### Exclusion Criteria:

Hypersensitivity to a compound of Formula (I), (1a)-(If), (II), (IIa)-(IIe), (III), (IIIa)-(IIIc), (IV), (IVa)-(IVc), (V), or (Va)-(Vc) or vancomycin;
S. aureus resistant to a compound of Formula (I), (1a)-(If), (II), (IIa)-(IIe), (III), (IIIa)-(IIIc), (IV), (IVa)-(IVc), (V), or (Va)-(Vc) or vancomycin;
Osteomyelitis that develops directly from a chronic, open wound;
Polymicrobial culture (the only exception is if coagulase-negative staphylococcus is present in the culture and the clinical assessment is that it is a contaminant);
Subject has a positive pregnancy test at study enrollment;
Baseline renal or hepatic insufficiency that would preclude administration of study drugs;
Active injection drug use without safe conditions to administer intravenous antibiotics for 3 months; and
Anticipated use of antibiotics for greater than 14 days for an infection other than osteomyelitis.

**Study Design:** This is a randomized, open-label, active control, efficacy trial comparing vancomycin with a compound of Formula (I), (Ia)-(If), (II), (IIa)-(IIe), (III), (IIIa)-(IIIc), (IV), (IVa)-(IVc), (V), or (Va)-(Vc) for the treatment of MRSA Osteomyelitis.

### Example B5: Clinical Trial Evaluating a Compound of Formula (I), (Ia)-(If), (II), (IIa)-(IIe), (III), (IIIa)-(IIIc), (IV), (IVa)-(IVc), (V), or (Va)-(Vc) in Selected Serious Infections Caused by Vancomycin-Resistant Enterococcus (VRE)

**Purpose:** This study aims to determine the safety and efficacy of a compound of Formula (I), (Ia)-(If), (II), (IIa)-(IIe), (III), (IIIa)-(IIIc), (IV), (IVa)-(IVc), (V), or (Va)-(Vc) in the treatment of selected serious infections caused by VRE.

**Patients:** Eligible subjects will be men and women 18 years and older.

### Criteria:

### Inclusion Criteria:

Isolation of one of the following multi-antibiotic resistant bacteria: vancomycin-resistant Enterococcus faecium, vancomycin-resistant Enterococcus faecalis alone or as part of a polymicrobial infection; and
Have a confirmed diagnosis of a serious infection (eg, bacteremia [unless due to an excluded infection], complicated intra-abdominal infection, complicated skin and skin structure infection, or pneumonia) requiring administration of intravenous (IV) antibiotic therapy.

### Exclusion Criteria:

Subjects with any concomitant condition or taking any concomitant medication that, in the opinion of the investigator, could preclude an evaluation of a response or make it unlikely that the contemplated course of therapy or follow-up assessment will be completed or that will substantially increase the risk associated with the subject's participation in this study.

Anticipated length of antibiotic therapy less than 7 days.

**Study Design:** This is a randomized, double-blind, safety and efficacy study of a compound of Formula (I), (Ia)-(If), (II), (IIa)-(IIe), (III), (IIIa)-(IIIc), (IV), (IVa)-(IVc), (V), or (Va)-(Vc) in the treatment of selected serious infections caused by VRE.

### Pharmaceutical Compositions

### Example C1: Parenteral Composition

To prepare a parenteral pharmaceutical composition suitable for administration by injection, 100 mg of a compound of Formula (I), (Ia)-(If), (II), (IIa)-(IIe), (III), (IIIa)-(IIIc), (IV), (IVa)-(IVc), (V), or (Va)-(Vc) is dissolved in DMSO and then mixed with 10 mL of 0.9% sterile saline. The mixture is incorporated into a dosage unit form suitable for administration by injection.

In another embodiment, the following ingredients are mixed to form an injectable formulation:

| **Ingredient** | **Amount** |
|---|---|
| Compound of Formula (I), (Ia)-(If), (II), (IIa)-(IIe), (III), (IIIa)-(IIIc), (IV), (IVa)-(IVc), (V), or (Va)-(Vc) | 1.2 g |

| | |
|---|---|
| sodium acetate buffer solution (0.4 M) | 2.0 mL |
| HCl (1 N) or NaOH (1 M) | q.s. to suitable pH |
| water (distilled, sterile) | q.s.to 20 mL |

All of the above ingredients, except water, are combined and stirred and if necessary, with slight heating if necessary. A sufficient quantity of water is then added.

### Example C2: Oral Composition

To prepare a pharmaceutical composition for oral delivery, 100 mg of a compound of Formula (I), (Ia)-(If), (II), (IIa)-(IIe), (III), (IIIa)-(IIIc), (IV), (IVa)-(IVc), (V), or (Va)-(Vc) is mixed with 750 mg of starch. The mixture is incorporated into an oral dosage unit, such as a hard gelatin capsule, which is suitable for oral administration.

In another embodiment, the following ingredients are mixed intimately and pressed into single scored tablets.

| Ingredient | Quantity per tablet, mg |
|---|---|
| compound of Formula (I), (Ia)-(If), (II), (IIa)-(IIe), (III), (IIIa)-(IIIc), (IV), (IVa)-(IVc), (V), or (Va)-(Vc) | 200 |
| Cornstarch | 50 |
| croscarmellose sodium | 25 |
| Lactose | 120 |
| magnesium stearate | 5 |

In yet another embodiment, the following ingredients are mixed intimately and loaded into a hard-shell gelatin capsule.

| Ingredient | Quantity per tablet, mg |
|---|---|
| compound of Formula (I), (Ia)-(If), (II), (IIa)-(IIe), (III), (IIIa)-(IIIc), (IV), (IVa)-(IVc), (V), or (Va)-(Vc) | 200 |
| lactose, spray-dried | 148 |
| magnesium stearate | 2 |

In yet another embodiment, the following ingredients are mixed to form a solution/suspension for oral administration:

| **Ingredient** | **Amount** |
|---|---|
| Compound of Formula (I), (Ia)-(If), (II), (IIa)-(IIe), (III), (IIIa)-(IIIc), (IV), (IVa)-(IVc), (V), or (Va)-(Vc) | 1 g |
| | 0.1 g |
| Anhydrous Sodium Carbonate | |
| Ethanol (200 proof), USP | 10 mL |
| Purified Water, USP | 90 mL |
| Aspartame | 0.003g |

### Example C3: Topical Gel Composition

To prepare a pharmaceutical topical gel composition, 100 mg of a compound of Formula (I), (1a)-(If), (II), (IIa)-(IIe), (III), (IIIa)-(IIIc), (IV), (IVa)-(IVc), (V), or (Va)-(Vc) is mixed with 1.75 g of hydroxypropyl cellulose, 10 mL of propylene glycol, 10 mL of isopropyl myristate and 100 mL of purified alcohol USP. The resulting gel mixture is then incorporated into containers, such as tubes, which are suitable for topical administration.

While preferred embodiments of the present disclosure have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments described herein may be employed in practicing the invention. It is intended that the following claims define the scope of the invention and that methods and structures within the scope of these claims and their equivalents be covered thereby.

The following numbered clauses, describing aspects of the invention, are part of the description.
1. A compound of Formula (I): 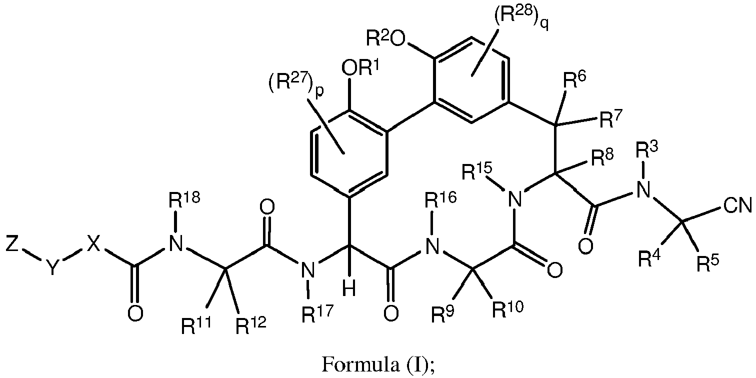 wherein:
   R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
   or R¹ and R² and the atoms to which they are attached form an optionally substituted heterocycloalkyl ring;
   R³ is H or -(C₁-C₆)alkyl;
   R⁴ is H, -(C₁-C₆)alkyl, -(C₁-C₆)alkyl-OH, -(C₃-C₆)cycloalkyl, or -C(O)NH₂;
   or R³ and R⁴ are combined to form a heterocycloalkyl ring;
   R⁵ is H or -(C₁-C₆)alkyl;
   or R⁴ and R⁵ and the carbon atom to which they are attached form a cyclopropyl ring;
   R⁶, R⁷, and R⁸ are each independently H, fluoro, hydroxyl, amino, optionally substituted alkyl, optionally substituted heteroalkyl, or -(C₁-C₆)alkyl;
   R⁹ is H, -(C₁-C₆)alkyl, -(C₁-C₆)haloalkyl, or -(C₃-C₆)cycloalkyl;
   R¹⁰ is H, -(C₁-C₆)alkyl, -(C₁-C₆)haloalkyl, or -(C₃-C₆)cycloalkyl;
   or R⁹ and R¹⁰ are combined to form a heterocycloalkyl or cycloalkyl ring
   R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
   or R¹¹ and R¹⁸ are combined to form an optionally substituted heterocycloalkyl ring; and R¹² is H;
   R¹⁵, R¹⁶, R¹⁷, and R¹⁸ are each independently H, -(C₁-C₆)alkyl, -(C₃-C₆)cycloalkyl, -(C₁-C₆)alkyl-OR²³, - (C₁-C₆)alkyl-C(O)OR²³, or -(C₁-C₆)alkyl-NR²¹R²²;
   X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
   Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -Oaryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
   Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
   each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)O(C₁-C₆)alkyl, -C(O)O(C₁-C₆)haloalkyl, -C(=NH)(C₁-C₆)alkyl, -C(=NH)N(R³¹)₂, - C(O)N(R³¹)₂, or -SO₂N(R³¹)₂; or R²¹ and R²² and the nitrogen atom to which they are attached form a heterocycloalkyl ring;
   each R³¹ is independently H or -(C₁-C₆)alkyl; or two R³¹ and the nitrogen atom to which they are attached form a heterocycloalkyl ring;
   each R²³ is independently H or -(C₁-C₆)alkyl;
   each R²⁴ is independently H or -(C₁-C₆)alkyl;
   each R²⁵ and R²⁶ is independently H or optionally substituted -(C₁-C₆)alkyl;
   or R²⁵ and R²⁶ and the nitrogen atom to which they are attached form a heterocycloalkyl ring;
   each R²⁷ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
   or R¹ and R²⁷ and the atoms to which they are attached form an optionally substituted 5- or 6-membered heterocycloalkyl ring;
   each R²⁸ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
   or R² and R²⁸ and the atoms to which they are attached form an optionally substituted 5- or 6-membered heterocycloalkyl ring;
   p is 0, 1, or 2; and
   q is 0, 1, or 2;
   or a pharmaceutically acceptable salt, solvate, or prodrug thereof.
2. The compound of clause 1 having the structure of Formula (Ia): 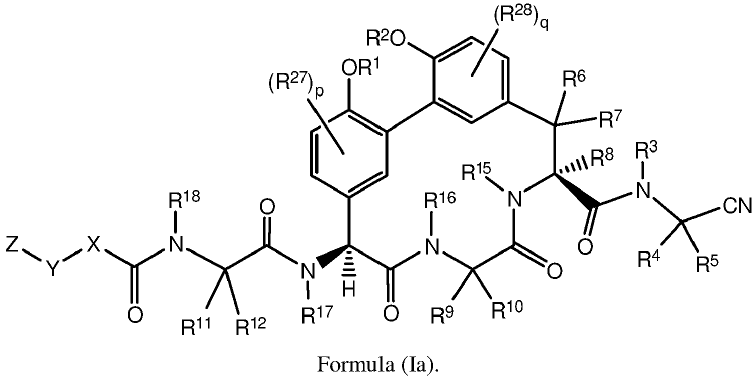
3. The compound of clause 1 or 2, wherein R⁶, R⁷, and R⁸ are H.
4. The compound of any one of clauses 1-3, wherein R¹⁵ and R¹⁶ are H.
5. The compound of any one of clauses 1-4 having the structure of Formula (Ib): 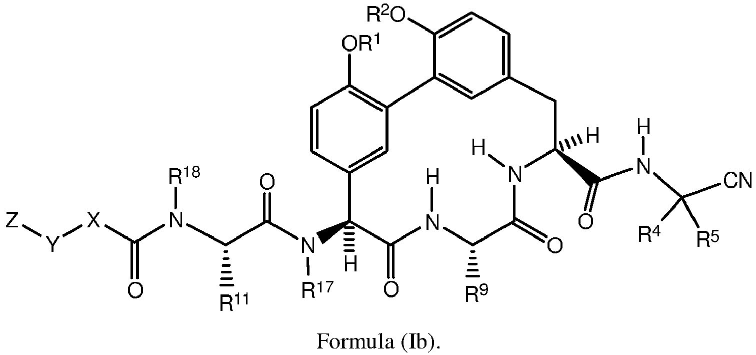
6. The compound of any one of clauses 1-5, wherein R¹⁷ is -CH₃.
7. The compound of any one of clauses 1-6, wherein R¹⁸ is H.
8. The compound of any one of clauses 1-7, wherein R⁵ is H.
9. The compound of any one of clauses 1-8, wherein R⁴ is H.
10. The compound of any one of clauses 1-8, wherein R⁴ is -(C₁-C₆)alkyl.
11. The compound of any one of clauses 1-8, wherein R⁴ is -(C₃-C₆)cycloalkyl.
12. The compound of any one of clauses 1-8, wherein R⁴ and R⁵ and the carbon atom to which they are attached form a cyclopropyl ring.
13. The compound of any one of clauses 1-12, wherein R⁹ is -(C₁-C₆)alkyl.
14. The compound of clause 13, wherein R⁹ is -CH₃.
15. The compound of clause 5 having the structure of Formula (Ic): 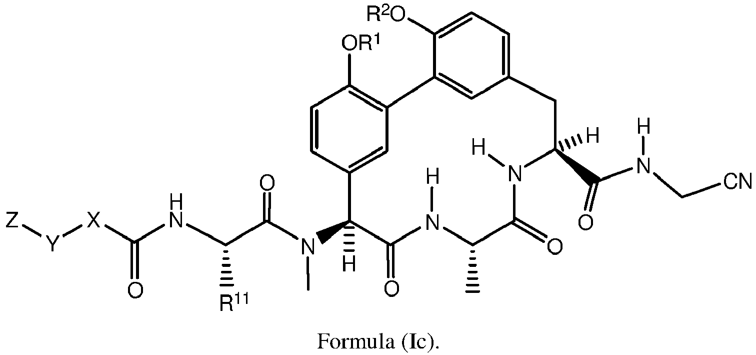
16. The compound of any one of clauses 1-15, wherein R¹¹ is -(C₁-C₆)alkyl-OR²³.
17. The compound of clause 16, wherein R¹¹ is -CH₂CH₂OH.
18. The compound of any one of clauses 1-15, wherein R¹¹ is -(C₁-C₆)alkyl.
19. The compound of any one of clauses 1-15, wherein R¹¹ is -(C₁-C₆)alkyl-NR²¹R²².
20. The compound of any one of clauses 1-15, wherein R¹¹ is -(C₁-C₆)alkyl-NH₂.
21. The compound of clause 20, wherein R¹¹ is -CH₂NH₂.
22. The compound of clause 20, wherein R¹¹ is -CH₂CH₂NH₂.
23. The compound of clause 20, wherein R¹¹ is -CH₂CH₂CH₂NH₂.
24. The compound of clause 20, wherein R¹¹ is -CH₂CH₂CH₂CH₂NH₂.
25. The compound of any one of clauses 1-24, wherein R¹ and R² are each independently H or -(C₁-C₆)alkyl-NR²¹R²².
26. The compound of any one of clauses 1-24, wherein R¹ and R² are each independently -(C₁-C₆)alkyl-NR²¹R²².
27. The compound of any one of clauses 1-24, wherein R¹ and R² are each -CH₂CH₂NH₂.
28. The compound of any one of clauses 1-24, wherein R¹ is -(C₁-C₆)alkyl-NR²¹R²² and R² is H.
29. The compound of any one of clauses 1-24, wherein R¹ is -CH₂CH₂NH₂ and R² is H.
30. The compound of any one of clauses 1-24, wherein R¹ is H and R² is -(C₁-C₆)alkyl-NR²¹R²².
31. The compound of any one of clauses 1-24, wherein R¹ is H and R² is -CH₂CH₂NH₂.
32. The compound of any one of clauses 1-24, wherein R¹ is H and R² is H.
33. The compound of clause 15 having the structure of Formula (Id): 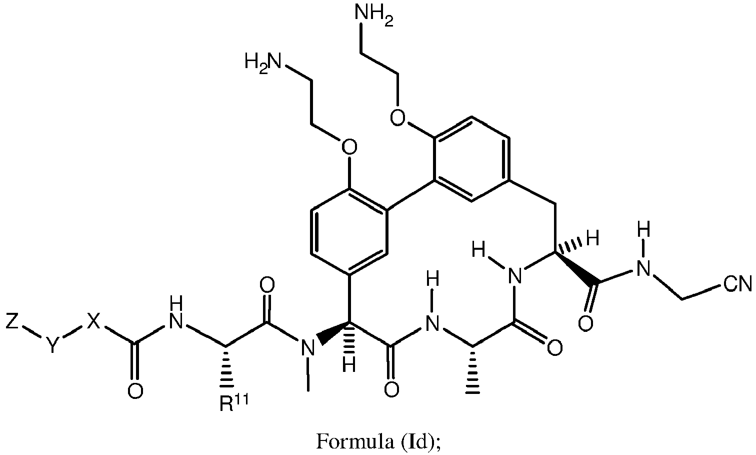 wherein R¹¹ is -CH₂CH₂NH₂ or -CH₂CH₂CH₂NH₂.
34. A compound of Formula (II): 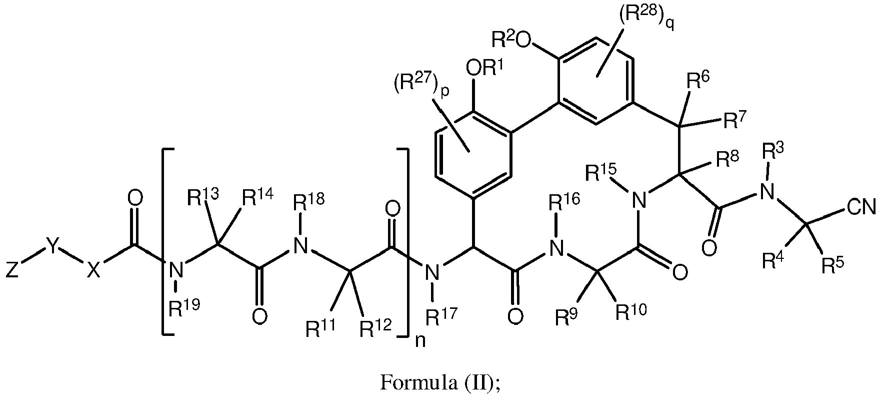 wherein:
   R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, - (C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²²,-(C₁-C₆)alkyl-N(R²³)C(O)(C₁-C₆)alkyl, -(C₁-C₆)alkyl-C(O)N(R²³)(C₁-C₆)alkyl, -(C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
   or R¹ and R² and the atoms to which they are attached form an optionally substituted heterocycloalkyl ring;
   R³ is H or -(C₁-C₆)alkyl;
   R⁴ is H, -(C₁-C₆)alkyl, -(C₁-C₆)alkyl-OH, -(C₃-C₆)cycloalkyl, or -C(O)NH₂;
   or R³ and R⁴ are combined to form a heterocycloalkyl ring;
   R⁵ is H or -(C₁-C₆)alkyl;
   or R⁴ and R⁵ and the carbon atom to which they are attached form a cyclopropyl ring;
   R⁶, R⁷, and R⁸ are each independently H or -(C₁-C₆)alkyl;
   R⁹ is H, -(C₁-C₆)alkyl, -(C₁-C₆)haloalkyl, or -(C₃-C₆)cycloalkyl;
   R¹⁰ is H or -(C₁-C₆)alkyl;
   R¹¹ and R¹² are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, -(C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
   R¹³ and R¹⁴ are each independently H, -NH₂, -(C₁-C₆)alkyl, -(C₁-C₆)alkyl-OR²³, -(C₁-C₆)alkyl-SR²³, -(C₁-C₆)alkyl-C(O)OR²³, -(C₁-C₆)alkyl-NR²¹R²², -(C₁-C₆)alkyl-CN, -(C₁-C₆)alkyl-C(O)NR²⁵R²⁶, -(C₁-C₆)alkyl-N(H)C(NH)NH₂, -(C₁-C₆)alkyl-heterocycloalkyl, or -(C₁-C₆)alkyl-heteroaryl;
   or R¹³ and R¹⁹ are combined to form an optionally substituted heterocycloalkyl ring; and R¹⁴ is H;
   R¹⁵, R¹⁶, R¹⁷, R¹⁸, and R¹⁹ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
   X is optionally substituted -(C₁-C₆)alkyl-, -(C₂-C₆)alkenyl-, -(C₂-C₆)alkynyl, optionally substituted -(C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
   Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -O(C₆-C₁₀)aryl-, -N(R²⁴)(C₁-C₆)alkyl-, - N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, -C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
   Z is H, halogen, -NH₂, -CN, -CF₃, -CO₂H, -(C₁-C₁₂)alkyl, -(C₂-C₁₂)alkenyl, -CH=((C₃-C₇)cycloalkyl), - (C₂-C₁₂)alkynyl, -C(O)NR²⁵R²⁶, -O-(C₁-C₁₂)alkyl, -S-(C₁-C₁₂)alkyl, -O-(C₃-C₁₀)[optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, - N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
   each R²¹ and R²² is independently H, -(C₁-C₆)alkyl, -(C₁-C₆)heteroalkyl, -(C₁-C₆)alkyl-CO₂H, -C(O)(C₁-C₆)alkyl, -C(O)N(R³¹)₂, or -SO₂N(R³¹)₂; or R²¹ and R²² and the nitrogen atom to which they are attached form a heterocycloalkyl ring;
   each R³¹ is independently H or -(C₁-C₆)alkyl; or two R³¹ and the nitrogen atom to which they are attached form a heterocycloalkyl ring;
   each R²³ is independently H or -(C₁-C₆)alkyl;
   each R²⁴ is independently H or -(C₁-C₆)alkyl;
   each R²⁵ and R²⁶ is independently H or optionally substituted -(C₁-C₆)alkyl;
   or R²⁵ and R²⁶ and the nitrogen atom to which they are attached form a heterocycloalkyl ring;
   each R²⁷ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
   or R¹ and R²⁷ and the atoms to which they are attached form an optionally substituted 5- or 6-membered heterocycloalkyl ring;
   each R²⁸ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
   or R² and R²⁸ and the atoms to which they are attached form an optionally substituted 5- or 6-membered heterocycloalkyl ring;
   n is 0 or 1;
   p is 0, 1, or 2; and
   q is 0, 1, or 2;
   or a pharmaceutically acceptable salt, solvate, or prodrug thereof.
35. The compound of clause 34 having the structure of Formula (IIa): 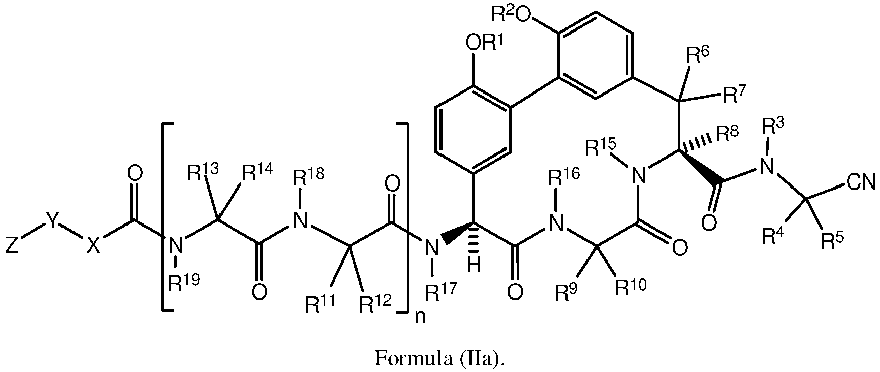
36. The compound of clause 34 or 35, wherein R⁶, R⁷, and R⁸ are H.
37. The compound of any one of clauses 35-36, wherein R¹⁵ and R¹⁶ are H.
38. The compound of any one of clauses 35 having the structure of Formula (IIb): 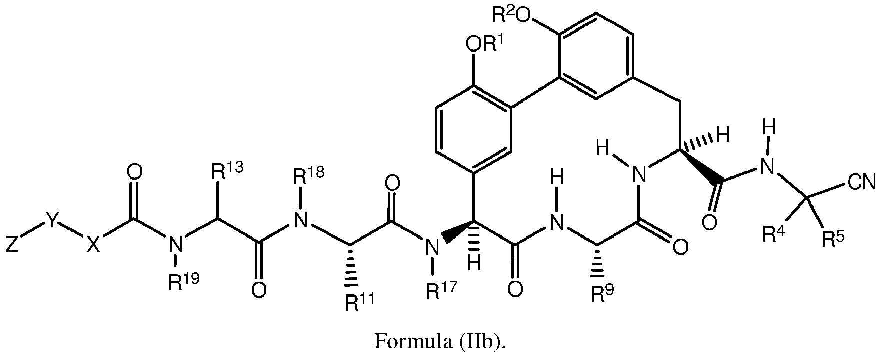
39. The compound of any one of clauses 34-38, wherein R¹⁸ is H.
40. The compound of any one of clauses 34-39, wherein R¹⁹ is H.
41. The compound of any one of clauses 34-40, wherein R¹⁷ is -CH₃.
42. The compound of any one of clauses 34-41, wherein R⁵ is H.
43. The compound of any one of clauses 34-42, wherein R⁴ is H.
44. The compound of any one of clauses 34-42, wherein R⁴ is -(C₁-C₆)alkyl.
45. The compound of any one of clauses 34-42, wherein R⁴ is -(C₃-C₆)cycloalkyl.
46. The compound of any one of clauses 34-41, wherein R⁴ and R⁵ and the carbon atom to which they are attached form a cyclopropyl ring.
47. The compound of any one of clauses 34-46, wherein R⁹ is -(C₁-C₆)alkyl.
48. The compound of clause 47, wherein R⁹ is -CH₃.
49. The compound of clause 38 having the structure of Formula (IIc): 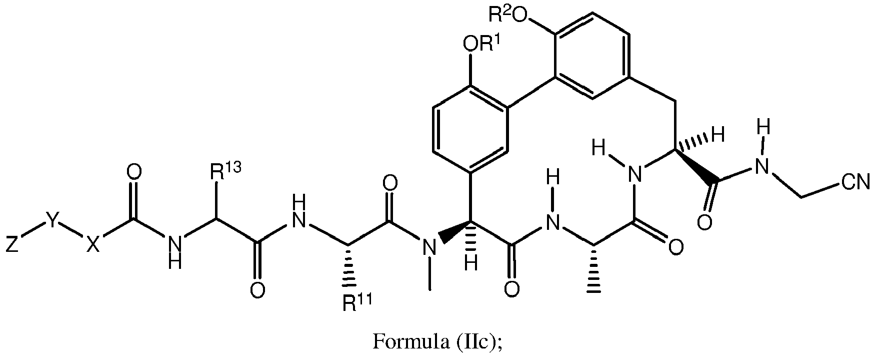 wherein R¹ and R² are each independently H or -CH₂CH₂NH₂.
50. The compound of any one of clauses 34-49, wherein R¹¹ is -(C₁-C₆)alkyl-OR²³.
51. The compound of clause 50, wherein R¹¹ is -CH₂CH₂OH.
52. The compound of any one of clauses 34-49, wherein R¹¹ is -(C₁-C₆)alkyl.
53. The compound of any one of clauses 34-49, wherein R¹¹ is -(C₁-C₆)alkyl-NR²¹R²².
54. The compound of any one of clauses 34-49, wherein R¹¹ is -(C₁-C₆)alkyl-NH₂.
55. The compound of clause 54, wherein R¹¹ is -CH₂NH₂.
56. The compound of clause 54, wherein R¹¹ is -CH₂CH₂NH₂.
57. The compound of clause 54, wherein R¹¹ is -CH₂CH₂CH₂NH₂.
58. The compound of clause 54, wherein R¹¹ is -CH₂CH₂CH₂CH₂NH₂.
59. The compound of any one of clauses 34-58, wherein R¹³ is -(C₁-C₆)alkyl-OR²³.
60. The compound of clause 59, wherein R¹³ is -CH₂OH.
61. The compound of clause 59, wherein R¹³ is -CH₂CH₂OH.
62. The compound of clause 34 or 35 having the structure of Formula (IId): 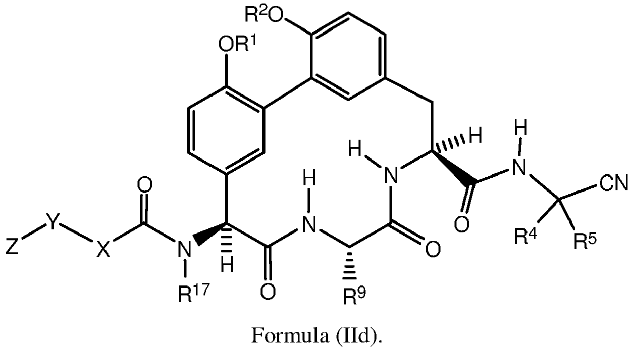
63. The compound of clause 62, wherein R¹⁷ is -CH₃.
64. The compound of clause 62 or 63, wherein R⁵ is H.
65. The compound of any one of clauses 62-64, wherein R⁴ is H.
66. The compound of any one of clauses 62-64, wherein R⁴ is -(C₁-C₆)alkyl.
67. The compound of any one of clauses 62-64, wherein R⁴ is -(C₃-C₆)cycloalkyl.
68. The compound of clause 62 or 63, wherein R⁴ and R⁵ and the carbon atom to which they are attached form a cyclopropyl ring.
69. The compound of any one of clauses 62-68, wherein R⁹ is -(C₁-C₆)alkyl.
70. The compound of clause 69, wherein R⁹ is -CH₃.
71. The compound of any one of clauses 34-70, wherein R¹ and R² are each independently H or -(C₁-C₆)alkyl-NR²¹R²².
72. The compound of any one of clauses 34-70, wherein R¹ and R² are each independently -(C₁-C₆)alkyl-NR²¹R²².
73. The compound of any one of clauses 34-70, wherein R¹ and R² are each -CH₂CH₂NH₂.
74. The compound of any one of clauses 34-70, wherein R¹ is -(C₁-C₆)alkyl-NR²¹R²² and R² is H.
75. The compound of any one of clauses 34-70, wherein R¹ is -CH₂CH₂NH₂ and R² is H.
76. The compound of any one of clauses 34-70, wherein R¹ is H and R² is -(C₁-C₆)alkyl-NR²¹R²².
77. The compound of any one of clauses 34-70, wherein R¹ is H and R² is -CH₂CH₂NH₂.
78. The compound any one of clause 62 having the structure of Formula (IIe): 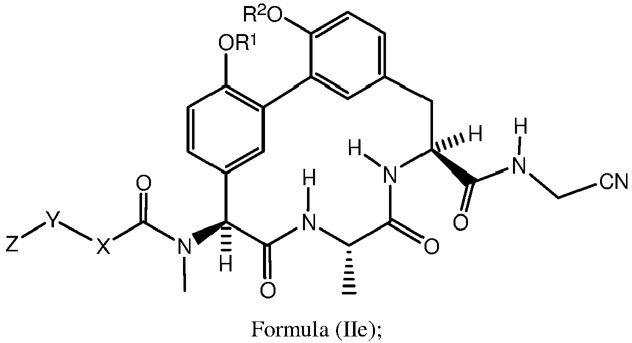 wherein R¹ and R² are each independently H or -CH₂CH₂NH₂.
79. The compound of any one of clauses 1-78, wherein X is optionally substituted aryl.
80. The compound of any one of clauses 1-78, wherein X is optionally substituted phenyl.
81. The compound of any one of clauses 1-78, wherein X is optionally substituted heteroaryl.
82. The compound of clause 81, wherein X is disubstituted heteroaryl.
83. The compound of clause 81, wherein X is heteroaryl disubstituted with substituents each independently selected from halogen, -CN, optionally substituted -(C₁-C₆)alkyl, optionally substituted -O-(C₁-C₆)alkyl, OR²³, -NR²⁵R²⁶, and -NO₂.
84. The compound of clause 81, wherein X is heteroaryl disubstituted with substituents each independently selected from -(C₁-C₆)alkyl.
85. The compound of clause 81, wherein X is heteroaryl disubstituted with methyl.
86. The compound of clause 81, wherein X is pyridinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂.
87. The compound of clause 81, wherein X is pyridinyl disubstituted with substituents each independently selected from -(C₁-C₆)alkyl.
88. The compound of clause 81, wherein X is pyridinyl disubstituted with methyl.
89. The compound of clause 81, wherein X is pyrimidinyl disubstituted with substituents each independently selected from halogen, -CN, optionally substituted -(C₁-C₆)alkyl, optionally substituted -O-(C₁-C₆)alkyl, OR²³, -NR²⁵R²⁶, or -NO₂.
90. The compound of clause 81, wherein X is pyrimidinyl disubstituted with substituents each independently selected from -(C₁-C₆)alkyl.
91. The compound of clause 81, wherein X is pyrimidinyl disubstituted with methyl.
92. The compound of any one of clauses 1-78, wherein X is optionally substituted -(C₁-C₆)alkyl-.
93. The compound of any one of clauses 1-91, wherein Y is optionally substituted aryl.
94. The compound of any one of clauses 92, wherein Y is optionally substituted phenyl.
95. The compound of any one of clauses 1-91, wherein Y is optionally substituted heteroaryl.
96. The compound of any one of clauses 1-91, wherein Y is optionally substituted -(C₁-C₆)alkyl-.
97. The compound of any one of clauses 1-91, wherein Y is optionally substituted (C₃-C₇)cycloalkyl-.
98. The compound of any one of clauses 1-91, wherein Y is optionally substituted heterocycloalkyl.
99. The compound of any one of clauses 1-91, wherein Y is -O-.
100. The compound of any one of clauses 1-91, wherein Y is -(C₂-C₆)alkynyl.
101. The compound of any one of clauses 1-91, wherein Y is -O-(C₁-C₆)alkyl-.
102. The compound of any one of clauses 1-91, wherein Y is a bond.
103. The compound of any one of clauses 1-101, wherein Z is -(C₁-C₁₂)alkyl.
104. The compound of any one of clauses 1-101, wherein Z is n-butyl, isobutyl, or tert-butyl.
105. The compound of any one of clauses 1-101, wherein Z is -O-(C₁-C₁₂)alkyl.
106. The compound of any one of clauses 1-101, wherein Z is -O-(C₃-C₇)cycloalkyl.
107. The compound of any one of clauses 1-101, wherein Z is -(C₂-C₁₂)alkenyl.
108. The compound of any one of clauses 1-101, wherein Z is optionally substituted aryl.
109. The compound of any one of clauses 1-101, wherein Z is optionally substituted phenyl.
110. The compound of any one of clauses 1-101, wherein Z is phenyl monsubstituted or disubstituted with a substituent independently selected from -(C₁-C₈)alkyl.
111. The compound of any one of clauses 1-101, wherein Z is phenyl monosubstituted with n-butyl, isobutyl, or tert-butyl.
112. The compound of any one of clauses 1-101, wherein Z is phenyl monosubstituted with n-butyl.
113. The compound of any one of clauses 1-101, wherein Z is phenyl monosubstituted with isobutyl.
114. The compound of any one of clauses 1-101, wherein Z is phenyl monosubstituted with tert-butyl.
115. The compound of any one of clauses 1-101, wherein Z is optionally substituted heteroaryl.
116. The compound of any one of clauses 1-101, wherein Z is optionally substituted -(C₃-C₇)cycloalkyl.
117. The compound of any one of clauses 1-101, wherein Z is optionally substituted heterocycloalkyl.
118. The compound of any one of clauses 1-101, wherein Z is halogen.
119. The compound of any one of clauses 1-101, wherein Z-Y-X- is not 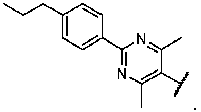
120. The compound of any one of clauses 1-119, wherein the compound is selected from any of the compounds in table 1 or a pharmaceutically acceptable salt, solvate, or prodrug thereof.
121. A pharmaceutical composition comprising the compound of any one of clauses 1-120, or a pharmaceutically acceptable salt, pharmaceutically acceptable solvate, or pharmaceutically acceptable prodrug thereof, and a pharmaceutically acceptable excipient.
122. Use of a compound of any one of clauses 1-120, or a pharmaceutically acceptable salt, pharmaceutically acceptable solvate, or pharmaceutically acceptable prodrug thereof, for preparation of a medicament for treatment of a bacterial infection in a patient.
123. A method of treatment of a bacterial infection in a mammal, comprising administering to the mammal an effective amount of a compound of any one of clauses 1-120, or a pharmaceutically acceptable salt, pharmaceutically acceptable solvate, or pharmaceutically acceptable prodrug thereof, to the mammal at a frequency and for a duration sufficient to provide a beneficial effect to the mammal.
124. A method of treatment of a lepB-mediated infection in a mammal, comprising administering to the mammal an effective amount of a compound of any one of clauses 1-120, or a pharmaceutically acceptable salt, pharmaceutically acceptable solvate, or pharmaceutically acceptable prodrug thereof, to the mammal at a frequency and for a duration sufficient to provide a beneficial effect to the mammal.
125. The method of clause 123 or 124, wherein the bacterial infection is an infection involving Pseudomonas aeruginosa, Pseudomonas fluorescens, Pseudomonas acidovorans, Pseudomonas alcaligenes, Pseudomonas putida, Stenotrophomonas maltophilia, Burkholderia cepacia, Aeromonas hydrophilia, Escherichia coli, Citrobacter freundii, Salmonella typhimurium, Salmonella typhi, Salmonella paratyphi, Salmonella enteritidis, Shigella dysenteriae, Shigella flexneri, Shigella sonnei, Enterobacter cloacae, Enterobacter aerogenes, Klebsiella pneumoniae, Klebsiella oxytoca, Serratia marcescens, Francisella tularensis, Morganella morganii, Proteus mirabilis, Proteus vulgaris, Providencia alcalifaciens, Providencia rettgeri, Providencia stuartii, Acinetobacter baumannii, Acinetobacter calcoaceticus, Acinetobacter haemolyticus, Yersinia enterocolitica, Yersinia pestis, Yersinia pseudotuberculosis, Yersinia intermedia, Bordetella pertussis, Bordetella parapertussis, Bordetella bronchiseptica, Haemophilus influenzae, Haemophilus parainfluenzae, Haemophilus haemolyticus, Haemophilus parahaemolyticus, Haemophilus ducreyi, Pasteurella multocida, Pasteurella haemolytica, Branhamella catarrhalis, Helicobacter pylori, Campylobacter fetus, Campylobacter jejuni, Campylobacter coli, Borrelia burgdorferi, Vibrio cholerae, Vibrio parahaemolyticus, Legionella pneumophila, Listeria monocytogenes, Neisseria gonorrhoeae, Neisseria meningitidis, Kingella, Moraxella, Gardnerella vaginalis, Bacteroides fragilis, Bacteroides distasonis, Bacteroides 3452A homology group, Bacteroides vulgatus, Bacteroides ovalus, Bacteroides thetaiotaomicron, Bacteroides uniformis, Bacteroides eggerthii, Bacteroides splanchnicus, Clostridium difficile, Mycobacterium tuberculosis, Mycobacterium avium, Mycobacterium intracellulare, Mycobacterium leprae, Corynebacterium diphtheriae, Corynebacterium ulcerans, Streptococcus pneumoniae, Streptococcus agalactiae, Streptococcus pyogenes, Enterococcus faecalis, Enterococcus faecium, Staphylococcus aureus, Staphylococcus epidermidis, Staphylococcus saprophyticus, Staphylococcus intermedius, Staphylococcus hyicus subsp. hyicus, Staphylococcus haemolyticus, Staphylococcus hominis, or Staphylococcus saccharolyticus.
126. The method of any one of clauses 123-125, wherein the bacterial infection is an infection involving a Gram-negative bacteria.
127. The method of any one of clauses 123-126, wherein administering comprises topical administration.
128. The method of any one of clauses 123-127 further comprising administering a second therapeutic agent.
129. The method of clause 128, wherein the second therapeutic agent is not an SpsB inhibitor.
130. The method of clause 128, wherein the second therapeutic agent is an aminoglycoside antibiotic, fluoroquinolone antibiotic, β-lactam antibiotic, macrolide antibiotic, glycopeptide antibiotic, rifampicin, chloramphenicol, fluoramphenicol, colistin, mupirocin, bacitracin, daptomycin, or linezolid.
131. The method of clause 130, wherein the second therapeutic agent is a β-lactam antibiotic.
132. The method of clause 131, wherein the β-lactam antibiotic is selected from penicillins, monobactams, cephalosporins, cephamycins, and carbapenems.
133. The method of clause 131, wherein the β-lactam antibiotic is selected from Azlocillin, Amoxicillin, Ampicillin, Doripenem, Meropenem, Biapenem, Cefamandole, Imipenem, Mezlocillin, Cefmetazole, Cefprozil, Piperacillin/tazobactam, Carbenicillin, Cefaclor, Cephalothin, Ertapenem, Cefazolin, Cefepime, Cefonicid, Cefoxitin, Ceftazidime, Oxacillin, Cefdinir, Cefixime, Cefotaxime, Cefotetan, Cefpodoxime, Ceftizoxime, Ceftriaxone, Faropenem, Mecillinam, Methicillin, Moxalactam, Ticarcillin, Tomopenem, Ceftobiprole, Ceftaroline, Flomoxef, Cefiprome, and Cefozopran.
134. The method of any one of clauses 131-133, further comprising administering a β-lactamase inhibitor.

## Claims

1. A compound of Formula (I): 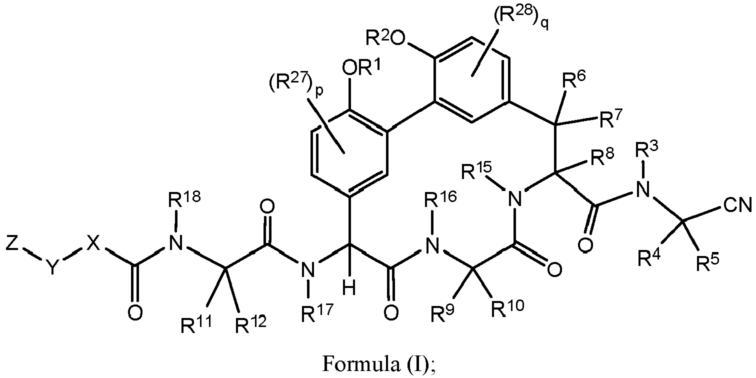 wherein:
R¹ and R² are each independently H, -(C₁-C₆)alkyl, -(C₁-C₆)alkyl-OR²³, -CH₂CH(OH)CH₂NH₂, - CH₂CH(heterocycloalkyl)CH₂NH₂, -CH₂C(O)NH₂, -CH₂C(O)N(H)CH₂CN, -(C₁-C₆)alkyl-C(O)OR²³, -(C₁-C₆)alkyl-NR²¹R²², -(C₁-C₆)alkyl-C(O)NR²⁵R²⁶, -(C₁-C₆)alkyl-N(R²³)C(O)(C₁-C₆)alkylNR²¹R²², -(C₁-C₆)alkyl-N(R²³)C(O)(C₁-C₆)alkyl, -(C₁-C₆)alkyl-C(O)N(R²³)(C₁-C₆)alkyl, - (C₁-C₆)alkyl-C(O)N(R²³)(C₁-C₆)alkyl-heterocycloalkyl, -(C₁-C₆)alkyl-NR²³C(=NH)NR²¹R²², -(C₁-C₆)alkyl-NR²³C(=NH)R²³, -(C₁-C₆)alkyl-[(C₁-C₆)alkyl-NR²¹R²²]₂, -(C₁-C₆)heteroalkyl, or optionally substituted heterocycloalkyl;
or R¹ and R² and the atoms to which they are attached form an optionally substituted heterocycloalkyl ring;
R³ is H or -(C₁-C₆)alkyl;
R⁴ is H, -(C₁-C₆)alkyl, -(C₁-C₆)alkyl-OH, -(C₃-C₆)cycloalkyl, or -C(O)NH₂;
or R³ and R⁴ are combined to form a heterocycloalkyl ring;
R⁵ is H or -(C₁-C₆)alkyl;
or R⁴ and R⁵ and the carbon atom to which they are attached form a cyclopropyl ring;
R⁶, R⁷, and R⁸ are each independently H, fluoro, hydroxyl, amino, optionally substituted alkyl, optionally substituted heteroalkyl, or -(C₁-C₆)alkyl;
R⁹ is H, -(C₁-C₆)alkyl, -(C₁-C₆)haloalkyl, or -(C₃-C₆)cycloalkyl;
R¹⁰ is H, -(C₁-C₆)alkyl, -(C₁-C₆)haloalkyl, or -(C₃-C₆)cycloalkyl;
or R⁹ and R¹⁰ are combined to form a heterocycloalkyl or cycloalkyl ring
R¹¹ and R¹² are each independently H, -NH₂, -(C₁-C₆)alkyl, -(C₁-C₆)alkyl-OR²³, -(C₁-C₆)alkyl-SR²³, - (C₁-C₆)alkyl-C(O)OR²³, -(C₁-C₆)alkyl-NR²¹R²², -(C₁-C₆)alkyl-NR²³OR²³, -(C₁-C₆)alkyl-NHC(O)NR²³OR²³, -(C₁-C₆)alkyl-O-(C₁-C₆)alkyl-NR²⁵R²⁶, -(C₁-C₆)alkyl-CN, -(C₁-C₆)alkyl-NR²³C(O)R²³, -(C₁-C₆)alkyl-C(O)NR²⁵R²⁶, -(C₁-C₆)heteroalkyl-CO₂H, -(C₁-C₆)alkyl-S(O)(C₁-C₆)alkyl, -(C₁-C₆)alkyl-N(H)CH=NH, -(C₁-C₆)alkyl-C(NH₂)=NH, -(C₁-C₆)alkyl-N(H)C(=NH)NH₂, -(C₁-C₆)alkyl-N(H)S(O)₂NR²⁵R²⁶, -(C₁-C₆)alkyl-N(H)S(O)₂(C₁-C₆)alkyl, -(C₁-C₆)alkyl-N(H)-C(O)NR²⁵R²⁶, -(C₁-C₆)alkylC(O)N(H)[optionally substituted(C₂-C₆)alkyl]-OR²³, - (C₁-C₆)alkylN(H)C(O)(C₁-C₆)alkyl-OR²³, -(C₁-C₆)alkylC(O)N(H)heterocycloalkyl, -(C₁-C₆)alkylC(O)NR²⁵R²⁶, -(C₁-C₆)alkyl-N(H)-C(O)-(C₁-C₆)alkyl-NR²⁵R²⁶, -(C₁-C₆)alkyl-N(H)-(C₁-C₆)alkylC(O)NR²⁵R²⁶, -(C₁-C₆)alkyl-heterocycloalkyl, optionally substituted -(C₁-C₆)alkyl-N(H)heterocycloalkyl, or -(C₁-C₆)alkyl-heteroaryl;
or R¹¹ and R¹⁸ are combined to form an optionally substituted heterocycloalkyl ring; and R¹² is H;
R¹⁵, R¹⁶, R¹⁷, and R¹⁸ are each independently H, -(C₁-C₆)alkyl, -(C₃-C₆)cycloalkyl, -(C₁-C₆)alkyl-OR²³, -(C₁-C₆)alkyl-C(O)OR²³, or -(C₁-C₆)alkyl-NR²¹R²²;
X is optionally substituted -(C₁-C₆)alkyl-, -(C₂-C₆)alkenyl-, -(C₂-C₆)alkynyl, optionally substituted - (C₃-C₇)cycloalkyl-, optionally substituted heterocycloalkyl, optionally substituted aryl, optionally substituted heteroaryl, -O-(C₁-C₆)alkyl-, -N(R²⁴)(C₁-C₆)alkyl-, -N(R²⁴)(C₆-C₁₀)aryl-, or -SO₂(C₁-C₆)alkyl-;
Y is a bond, -O-, -S-, optionally substituted -(C₁-C₆)alkyl-, -(C₂-C₆)alkenyl-, -(C₂-C₆)alkynyl, -(C₁-C₆)alkyl-N(R²⁴)(C₁-C₆)alkyl-, -O-(C₁-C₆)alkyl-, -Oaryl-, -N(R²⁴)(C₁-C₆)alkyl-, -N(R²⁴)SO₂(C₁-C₆)alkyl-, -N(R²⁴)C(O)(C₁-C₆)alkyl-, -C(O)(C₁-C₆)alkyl-, -S(C₁-C₆)alkyl-, -SO₂(C₁-C₆)alkyl-, - C(O)NH(C₁-C₆)alkyl-, optionally substituted -(C₃-C₇)cycloalkyl-, optionally substituted - C(O)N(R²⁴)aryl-, optionally substituted -N(R²⁴)C(O)aryl-, optionally substituted -N(R²⁴)SO₂aryl-, optionally substituted heterocycloalkyl, optionally substituted aryl, or optionally substituted heteroaryl;
Z is H, halogen, -NH₂, -CN, -CF₃, -CO₂H, -(C₁-C₁₂)alkyl, -(C₂-C₁₂)alkenyl, -CH=((C₃-C₇)cycloalkyl), -(C₂-C₁₂)alkynyl, -C(O)NR²⁵R²⁶, -O-(C₁-C₁₂)alkyl, -S-(C₁-C₁₂)alkyl, -O- [optionally substituted (C₃-C₇)cycloalkyl], -O-(C₁-C₆)alkyl-OR²³, -(C₁-C₁₂)alkyl-OR²³, -(C₁-C₁₂)alkyl-CN, -S-(C₁-C₁₂)alkyl, -N(R²⁴)(C₁-C₁₂)alkyl, -N(R²⁴)C(O)(C₁-C₁₂)alkyl, optionally substituted -(C₃-C₇)cycloalkyl, -(C₁-C₆)alkyl-(C₃-C₇)cycloalkyl, -(C₁-C₆)alkyl-heterocycloalkyl, optionally substituted heterocycloalkyl, optionally substituted aryl, or optionally substituted heteroaryl;
each R²¹ and R²² is independently H, -(C₁-C₆)alkyl, -(C₁-C₆)heteroalkyl, -(C₁-C₆)alkyl-CO₂H, - C(O)(C₁-C₆)alkyl, -C(O)O(C₁-C₆)alkyl, -C(O)O(C₁-C₆)haloalkyl, -C(=NH)(C₁-C₆)alkyl, - C(=NH)N(R³¹)₂, -C(O)N(R³¹)₂, or -SO₂N(R³¹)₂; or R²¹ and R²² and the nitrogen atom to which they are attached form a heterocycloalkyl ring;
each R³¹ is independently H or -(C₁-C₆)alkyl; or two R³¹ and the nitrogen atom to which they are attached form a heterocycloalkyl ring;
each R²³ is independently H or -(C₁-C₆)alkyl;
each R²⁴ is independently H or -(C₁-C₆)alkyl;
each R²⁵ and R²⁶ is independently H or optionally substituted -(C₁-C₆)alkyl;
or R²⁵ and R²⁶ and the nitrogen atom to which they are attached form a heterocycloalkyl ring;
each R²⁷ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R¹ and R²⁷ and the atoms to which they are attached form an optionally substituted 5- or 6-membered heterocycloalkyl ring;
each R²⁸ is independently halogen, -NR²³R²⁴, -NHC(O)R²³, -NHC(O)NR²³R²⁴, nitro, hydroxyl, optionally substituted -(C₁-C₆)alkyl, optionally substituted -(C₁-C₆)heteroalkyl, optionally substituted -(C₁-C₆)heteroalkyloxy, optionally substituted -(C₁-C₆)heteroalkylamino, -(C₁-C₆)alkoxy, -C(O)(C₁-C₆)alkyl, or -S(O)₂(C₁-C₆)alkyl;
or R² and R²⁸ and the atoms to which they are attached form an optionally substituted 5- or 6-membered heterocycloalkyl ring;
p is 1 or 2 and q is 0, 1, or 2;or q is 1 or 2 and p is 0, 1, or 2;
or a pharmaceutically acceptable salt or solvate thereof.

2. The compound of claim 1, or a pharmaceutically acceptable salt or solvate thereof, having the structure of Formula (Ia):

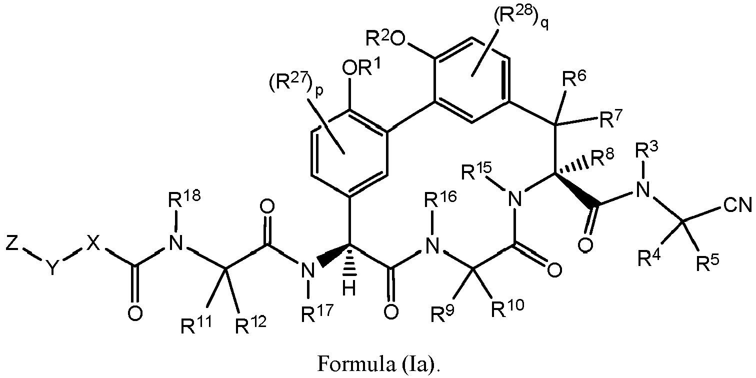

3. The compound of claim 1 or 2, or a pharmaceutically acceptable salt or solvate thereof, wherein R⁶, R⁷, and R⁸ are H.

4. The compound of any one of claims 1-3, or a pharmaceutically acceptable salt or solvate thereof, wherein R¹⁵ and R¹⁶ are H.

5. The compound of any one of claims 1-4, or a pharmaceutically acceptable salt or solvate thereof, wherein R¹⁷ is -CH₃.

6. The compound of any one of claims 1-5, or a pharmaceutically acceptable salt or solvate thereof, wherein R¹⁸ is H.

7. The compound of any one of claims 1-6, or a pharmaceutically acceptable salt or solvate thereof, wherein R⁵ is H.

8. The compound of any one of claims 1-7, or a pharmaceutically acceptable salt or solvate thereof, wherein R⁴ is H.

9. The compound of any one of claims 1-7, or a pharmaceutically acceptable salt or solvate thereof, wherein R⁴ is -(C₁-C₆)alkyl or -(C₃-C₆)cycloalkyl.

10. The compound of any one of claims 1-6, or a pharmaceutically acceptable salt or solvate thereof, wherein R⁴ and R⁵ and the carbon atom to which they are attached form a cyclopropyl ring.

11. The compound of any one of claims 1-10, or a pharmaceutically acceptable salt or solvate thereof, wherein R⁹ is -(C₁-C₆)alkyl.

12. The compound of claim 11, or a pharmaceutically acceptable salt or solvate thereof, wherein R⁹ is -CH₃.

13. The compound of any one of claims 1-12, or a pharmaceutically acceptable salt or solvate thereof, wherein R¹¹ is -(C₁-C₆)alkyl-OR²³, -(C₁-C₆)alkyl, or -(C₁-C₆)alkyl-NR²¹R²².

14. The compound of claim 13, or a pharmaceutically acceptable salt or solvate thereof, wherein R¹¹ is -CH₂CH₂OH.

15. The compound of any one of claims 1-13, or a pharmaceutically acceptable salt or solvate thereof, wherein R¹¹ is -(C₁-C₆)alkyl-NH₂.

16. The compound of claim 15, or a pharmaceutically acceptable salt or solvate thereof, wherein R¹¹ is -CH₂NH₂, -CH₂CH₂NH₂, -CH₂CH₂CH₂NH₂, or -CH₂CH₂CH₂CH₂NH₂.

17. The compound of any one of claims 1-16, or a pharmaceutically acceptable salt or solvate thereof, wherein R¹ and R² are each independently H or -(C₁-C₆)alkyl-NR²¹R²².

18. The compound of any one of claims 1-16, or a pharmaceutically acceptable salt or solvate thereof, wherein R¹ and R² are each independently -(C₁-C₆)alkyl-NR²¹R²².

19. The compound of any one of claims 1-16, or a pharmaceutically acceptable salt or solvate thereof, wherein R¹ is -(C₁-C₆)alkyl-NR²¹R²² and R² is H; or, wherein R¹ is H and R² is -(C₁-C₆)alkyl-NR²¹R²².

20. The compound of any one of claims 1-16, or a pharmaceutically acceptable salt or solvate thereof, wherein: R¹ is -CH₂CH₂NH₂ and R² is H; R¹ is H and R² is -CH₂CH₂NH₂; R¹ and R² are each - CH₂CH₂NH₂; or, R¹ is H and R² is H.

21. A compound or a pharmaceutically acceptable salt or solvate thereof, selected from the group consisting of
| Cp. # | Structure |
|---|---|
| 201 | 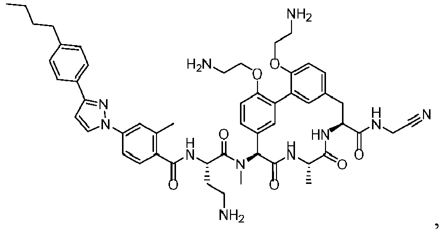 |
| 202 | 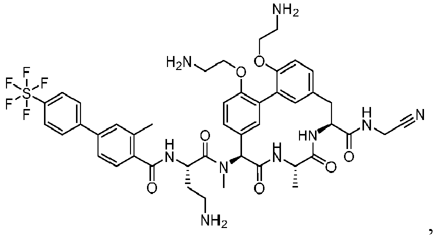 |
| 203 | 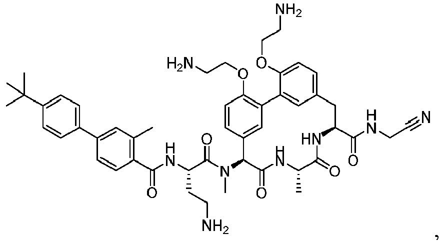 |
| 204 | 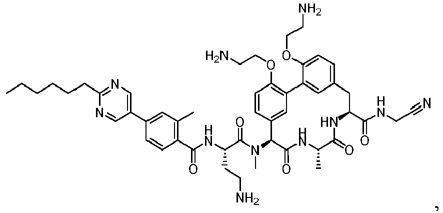 |
| 205 | 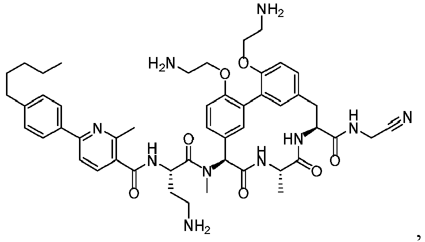 |
| 206 | 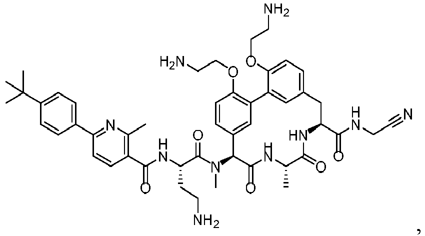 |
| 207 | 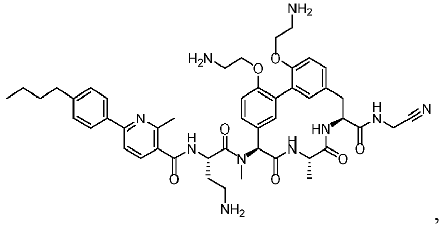 |
| 208 | 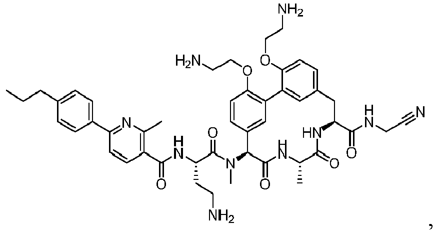 |
| 209 | 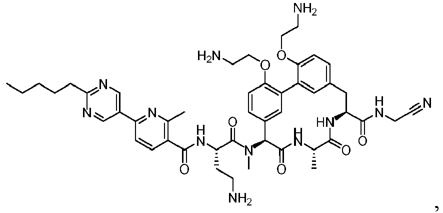 |
| 210 | 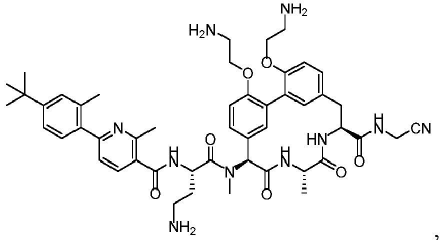 |
| 211 | 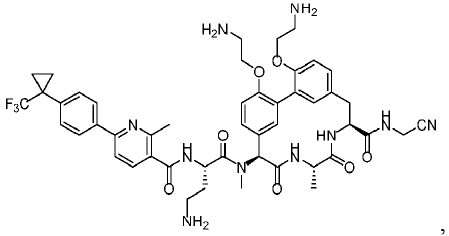 |
| 212 | 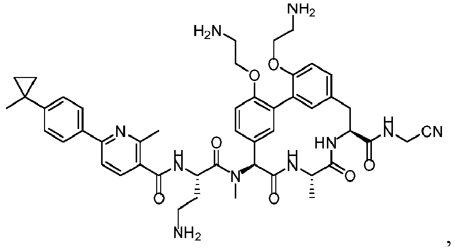 |
| 213 | 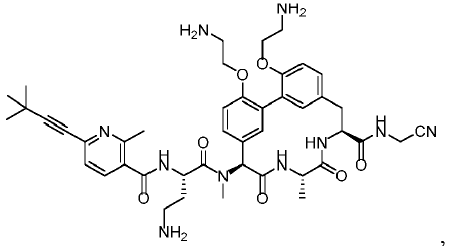 |
| 214 | 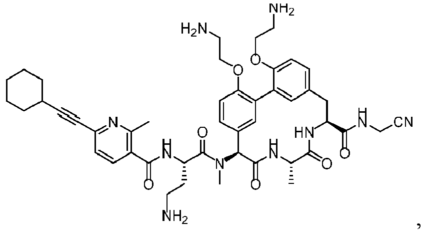 |
| 215 | 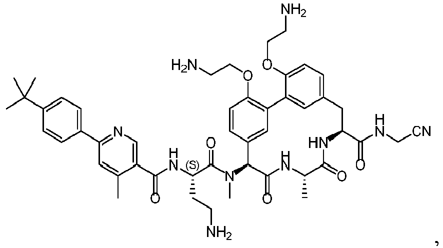 |
| 216 | 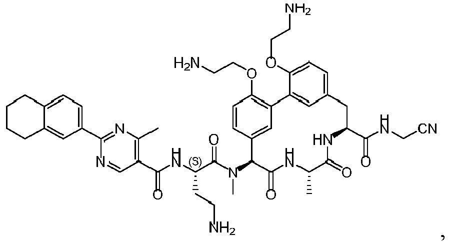 |
| 217 | 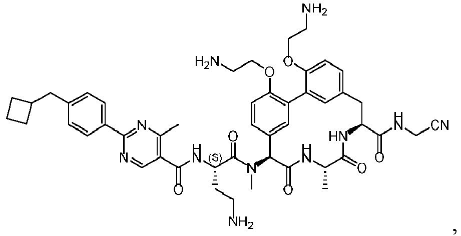 |
| 218 | 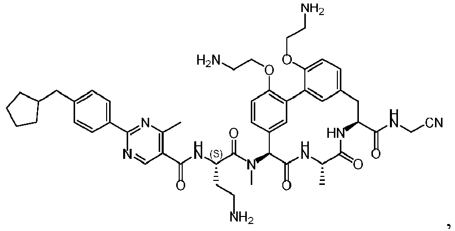 |
| 219 | 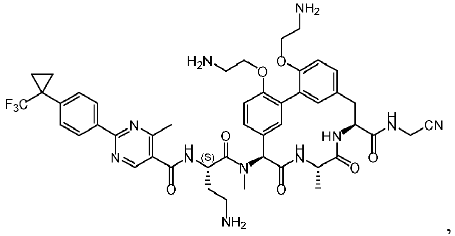 |
| 220 | 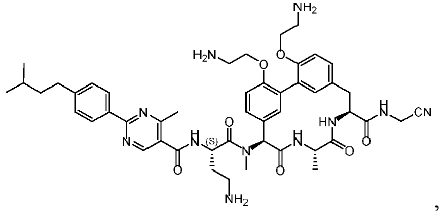 |
| 221 | 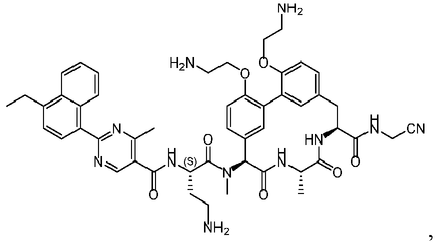 |
| 222 | 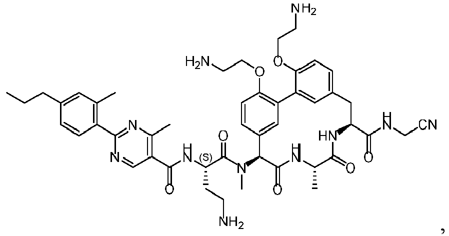 |
| 223 | 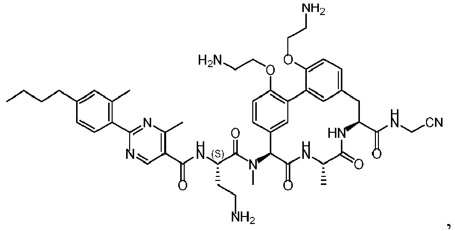 |
| 224 | 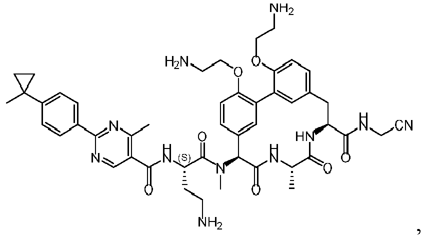 |
| 225 | 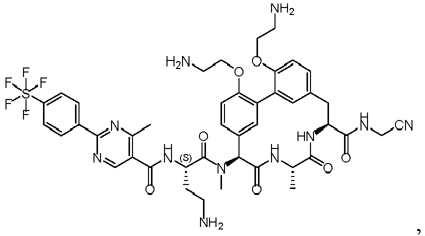 |
| 226 | |
| | 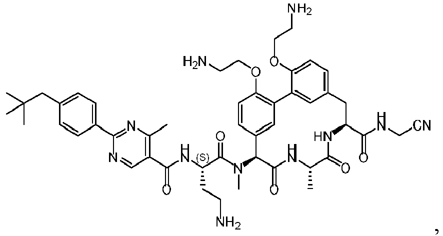 |
| 227 | 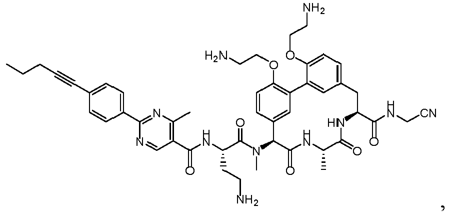 |
| 228 | 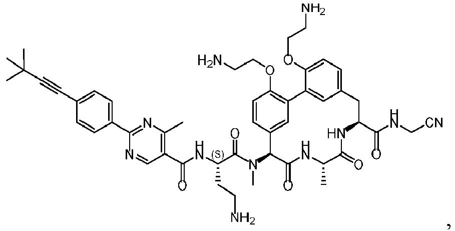 |
| 229 | 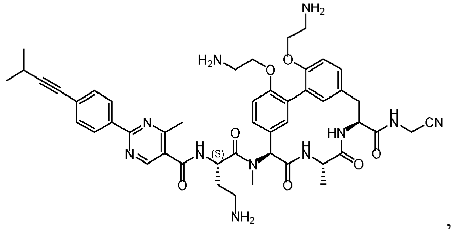 |
| 230 | 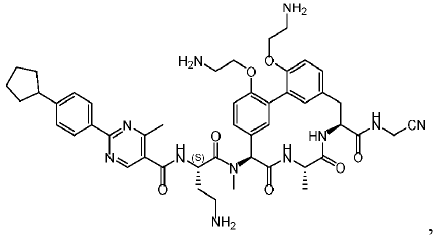 |
| 231 | 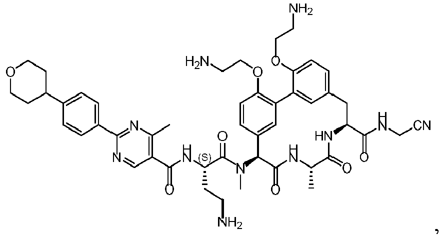 |
| 232 | 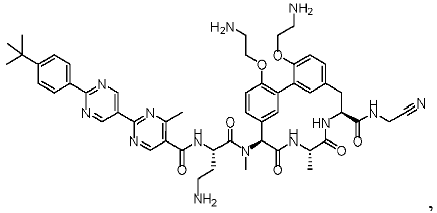 |
| 233 | 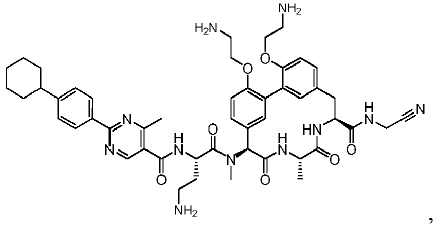 |
| 234 | 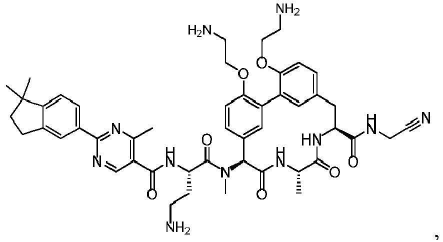 |
| 235 | 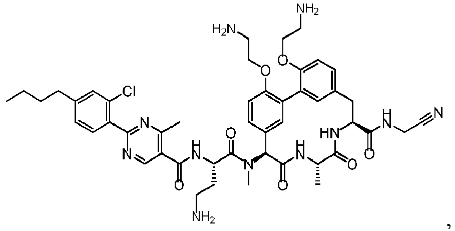 |
| 236 | 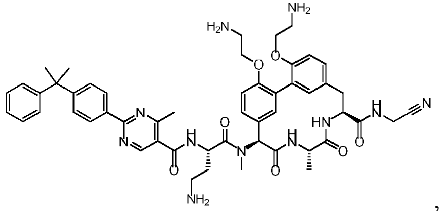 |
| 237 | 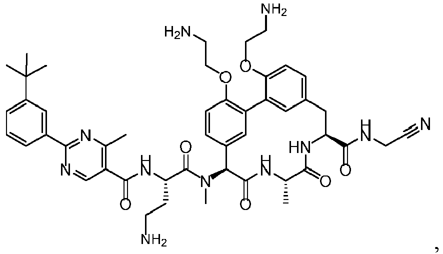 |
| 238 | 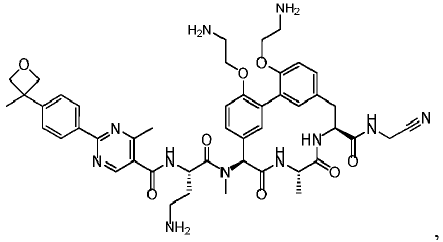 |
| 239 | 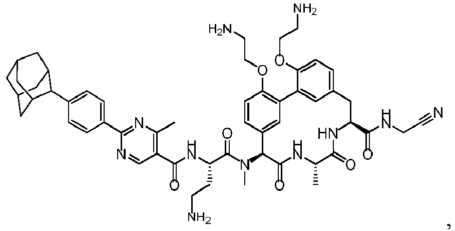 |
| 240 | 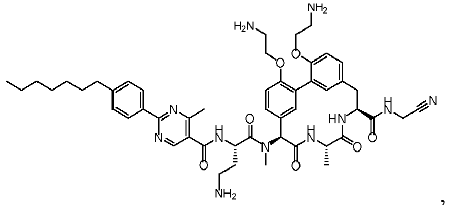 |
| 241 | 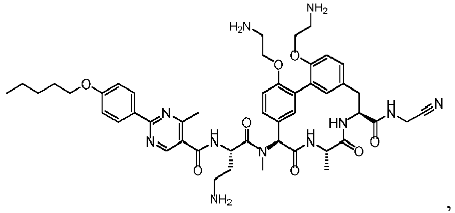 |
| 242 | 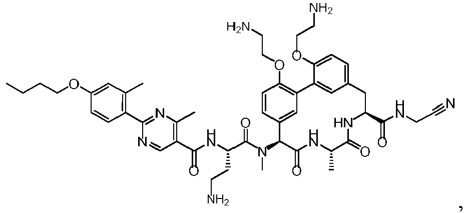 |
| 243 | 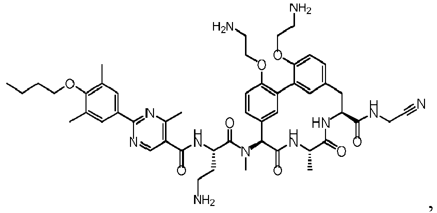 |
| 244 | 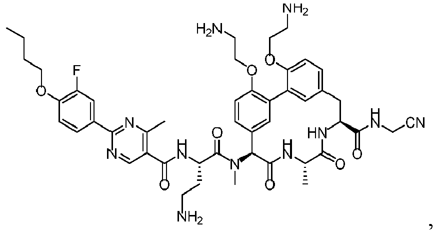 |
| 245 | 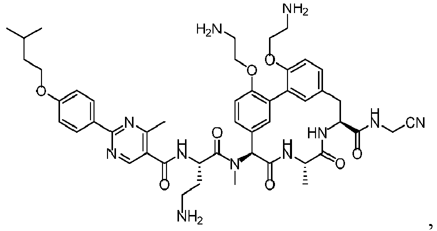 |
| 246 | 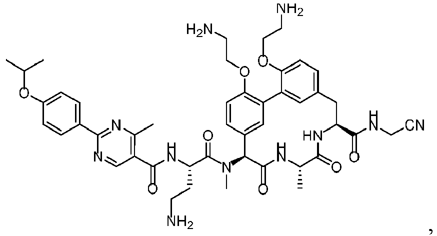 |
| 247 | 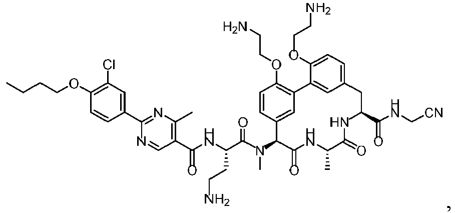 |
| 248 | 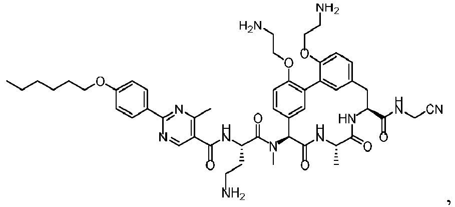 |
| 249 | 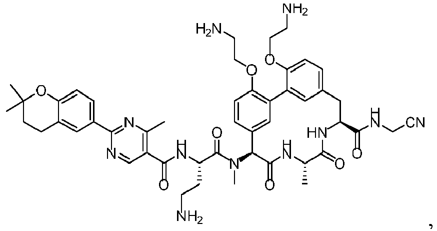 |
| 250 | 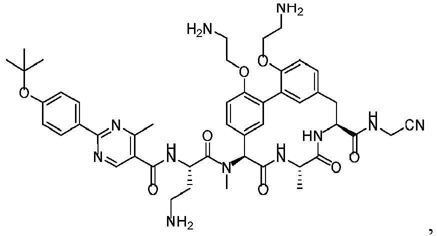 |
| 251 | 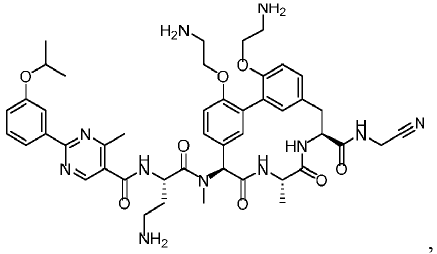 |
| 252 | 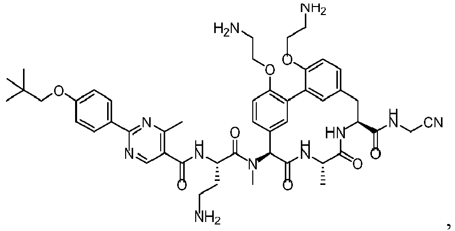 |
| 253 | 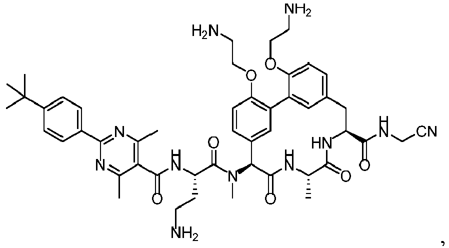 |
| 254 | 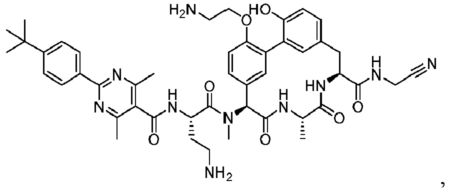 |
| 255 | 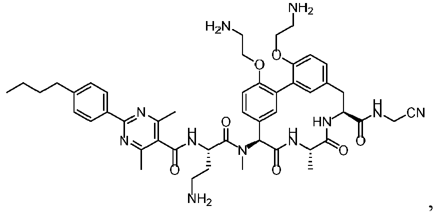 |
| 256 | 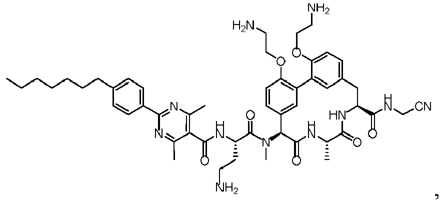 |
| 257 | 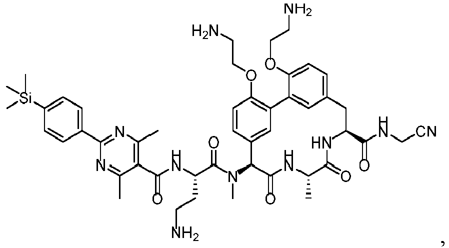 |
| 258 | 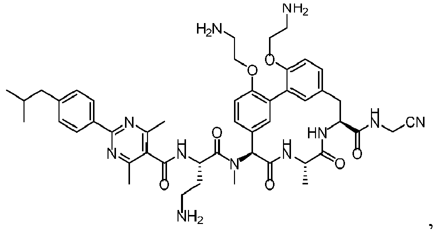 |
| 259 | 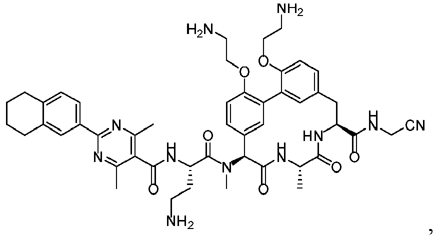 |
| 260 | 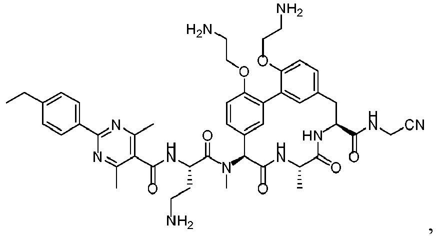 |
| 261 | 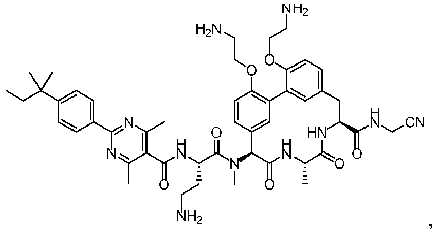 |
| 262 | 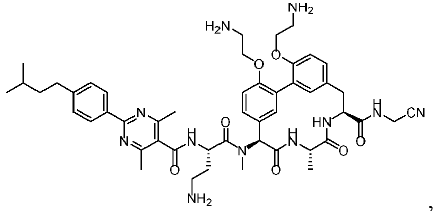 |
| 263 | 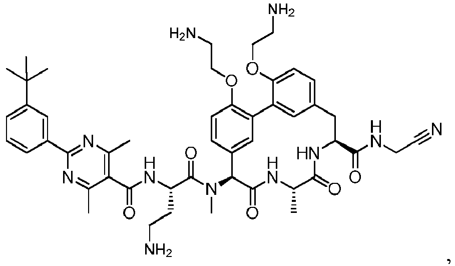 |
| 264 | 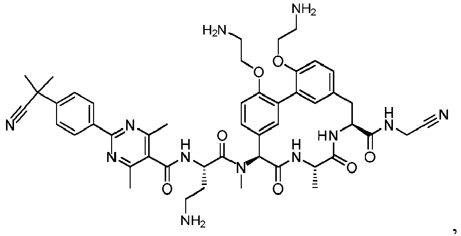 |
| 265 | 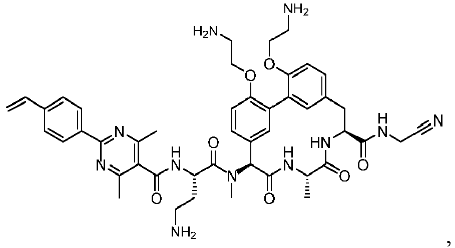 |
| 266 | 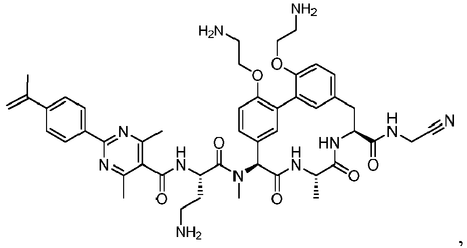 |
| 267 | 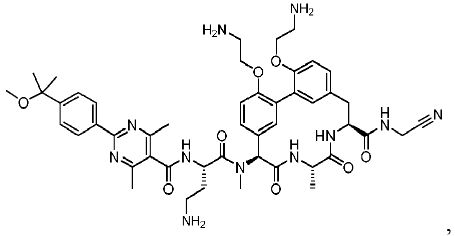 |
| 268 | 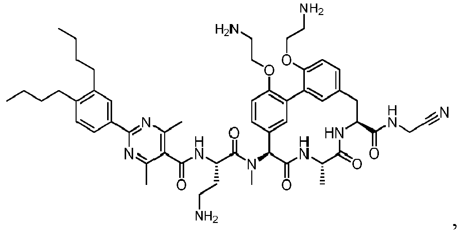 |
| 269 | 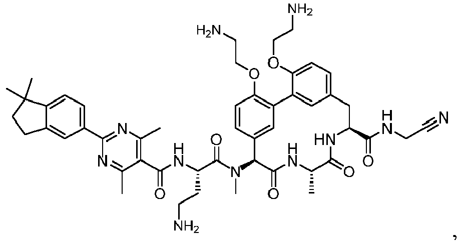 |
| 270 | 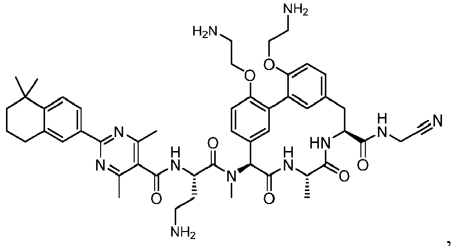 |
| 271 | 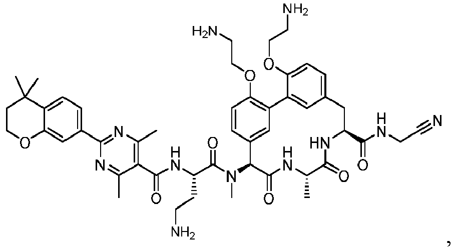 |
| 272 | 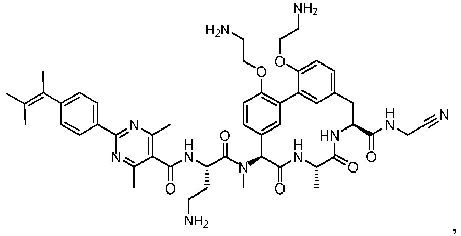 |
| 273 | 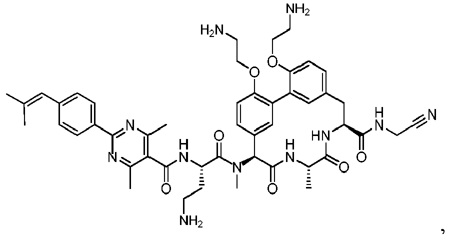 |
| 274 | 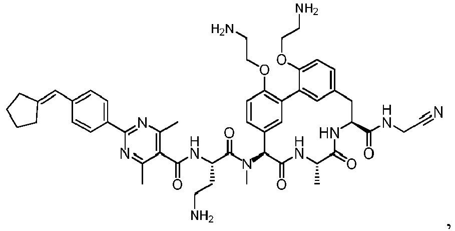 |
| 275 | 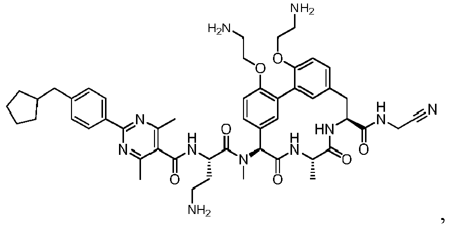 |
| 276 | 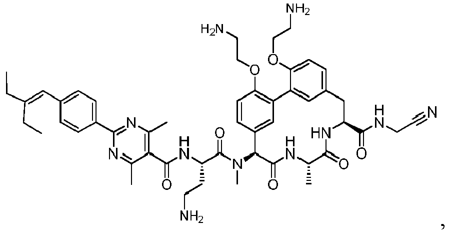 |
| 277 | 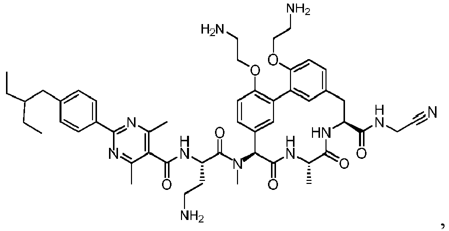 |
| 278 | 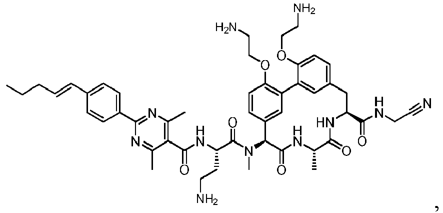 |
| 279 | 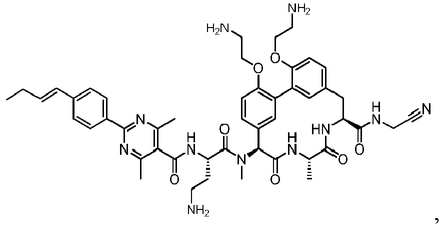 |
| 280 | 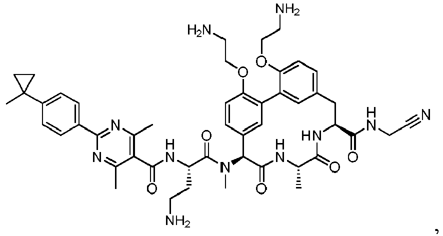 |
| 281 | 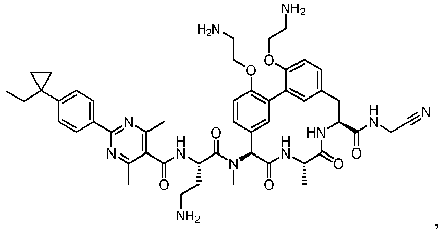 |
| 282 | 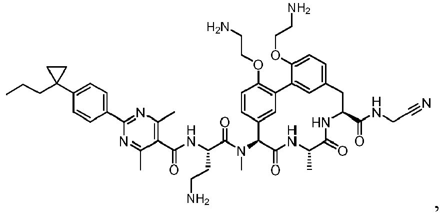 |
| 283 | 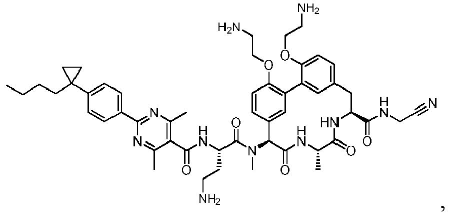 |
| 284 | 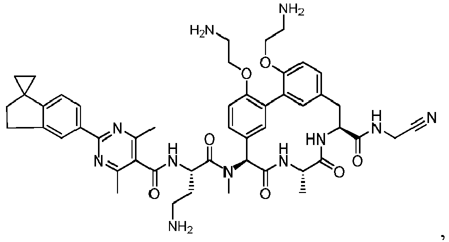 |
| 285 | 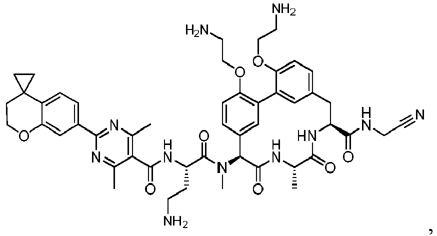 |
| 286 | 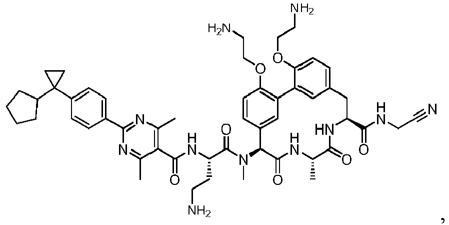 |
| 287 | 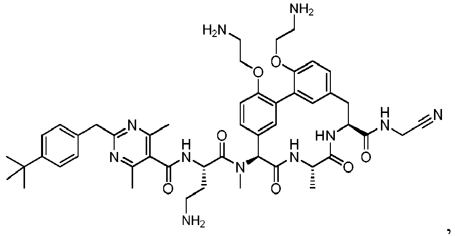 |
| 288 | 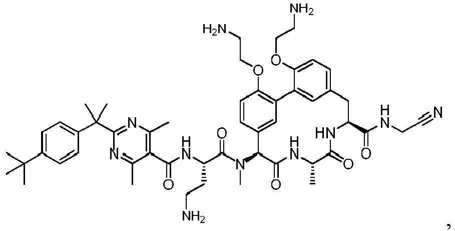 |
| 289 | 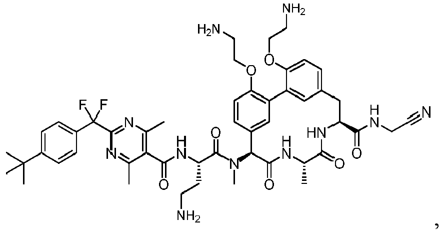 |
| 290 | 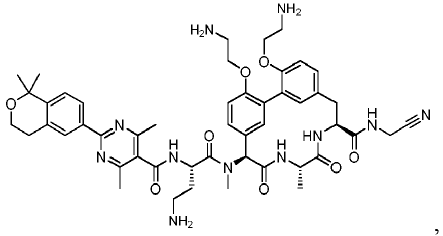 |
| 291 | 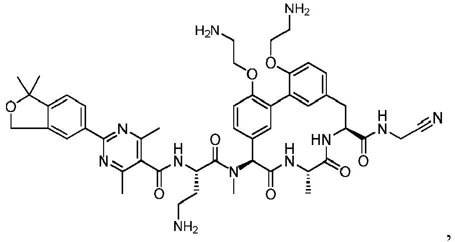 |
| 292 | 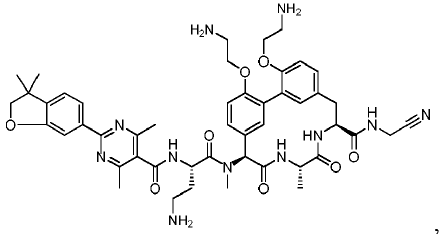 |
| 293 | 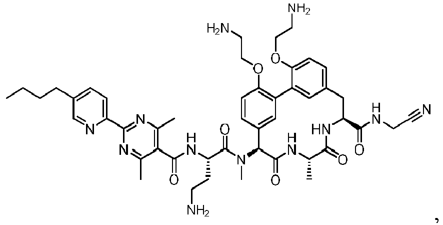 |
| 294 | 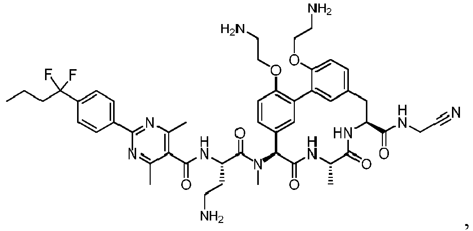 |
| 295 | 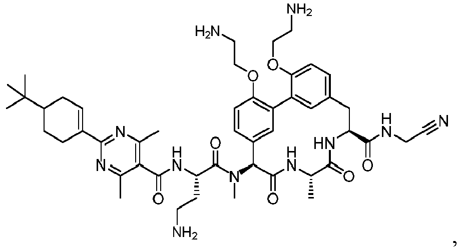 |
| 296 | 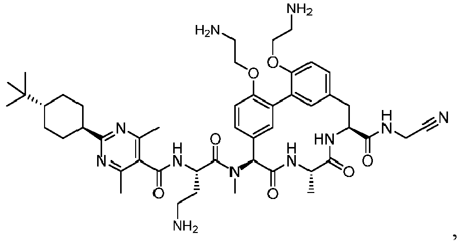 |
| 297 | 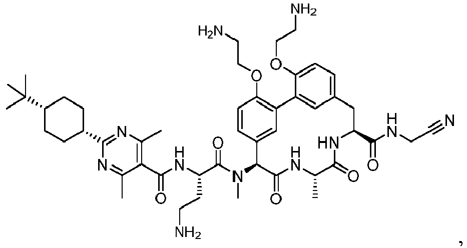 |
| 298 | 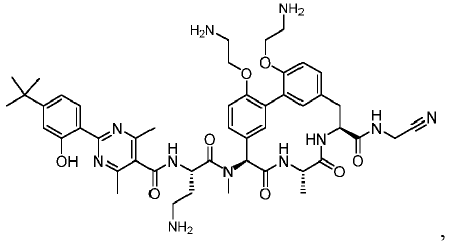 |
| 299 | 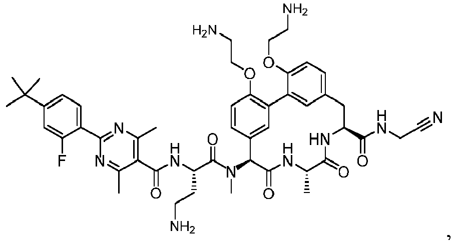 |
| 300 | 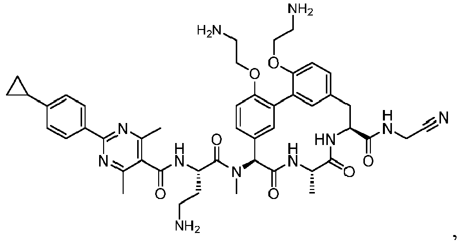 |
| 301 | 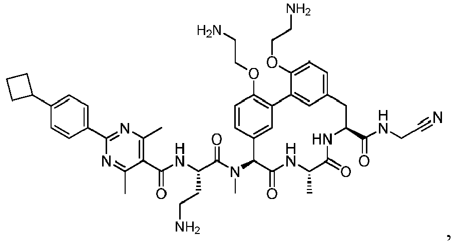 |
| 302 | 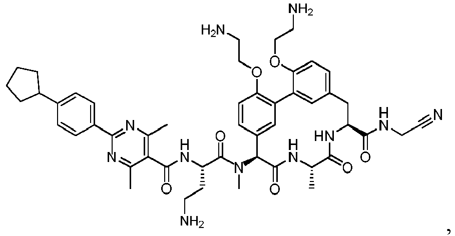 |
| 303 | 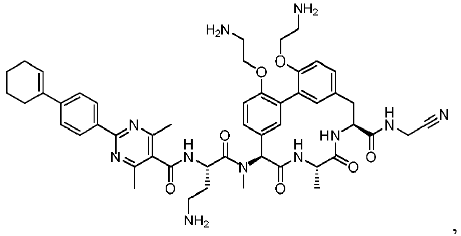 |
| 304 | 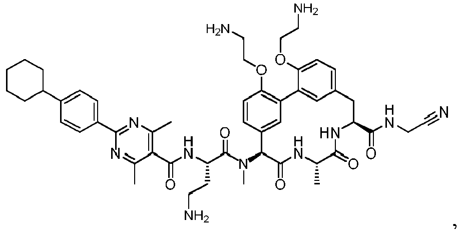 |
| 305 | 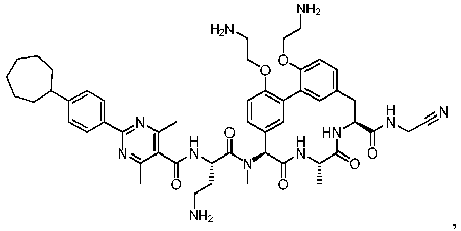 |
| 306 | 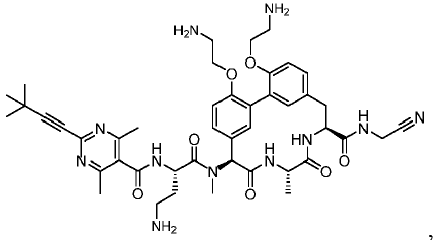 |
| 307 | 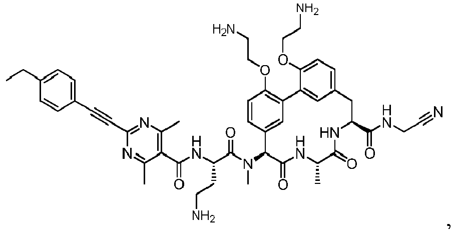 |
| 308 | 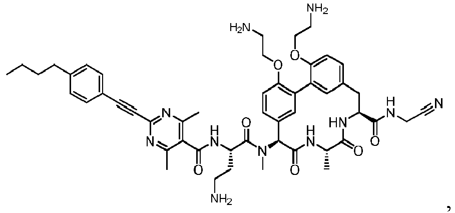 |
| 309 | 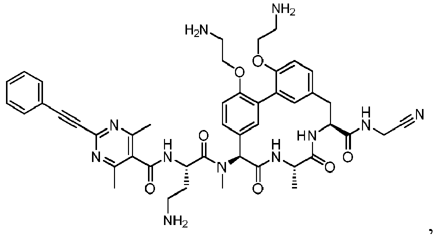 |
| 310 | 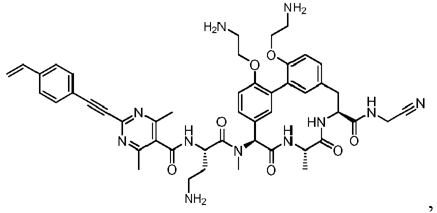 |
| 311 | 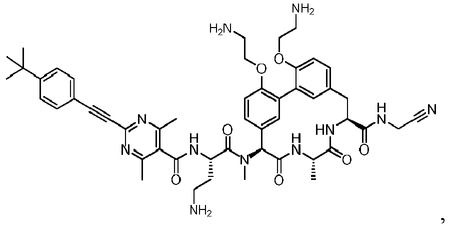 |
| 312 | 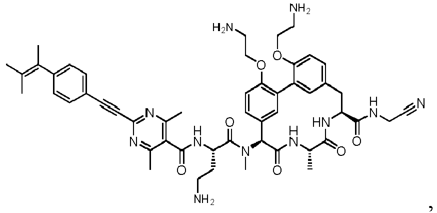 |
| 313 | 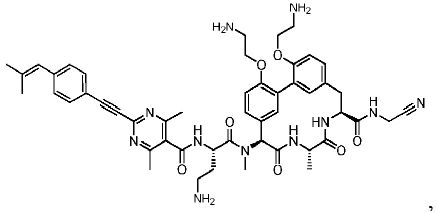 |
| 314 | 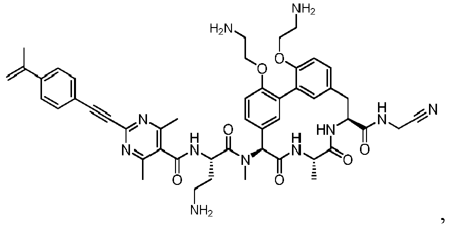 |
| 315 | 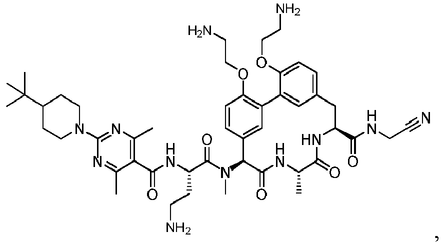 |
| 316 | 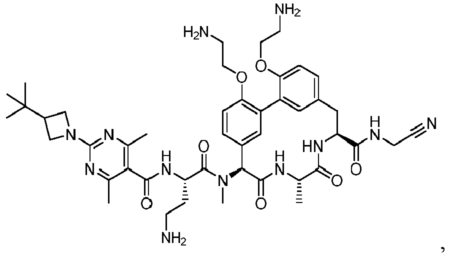 |
| 317 | 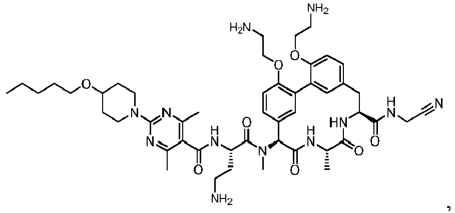 |
| 318 | 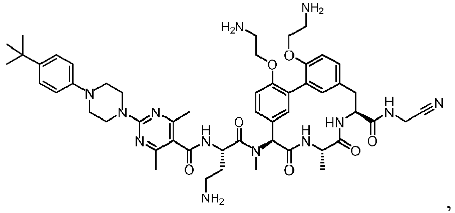 |
| 319 | 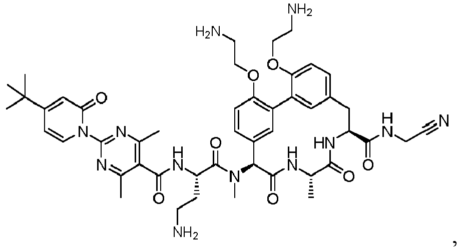 |
| 320 | 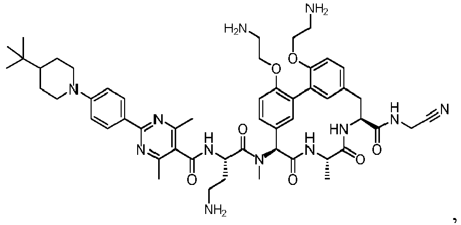 |
| 321 | 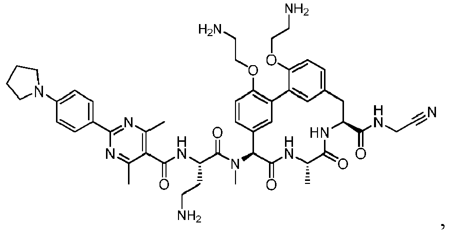 |
| 322 | 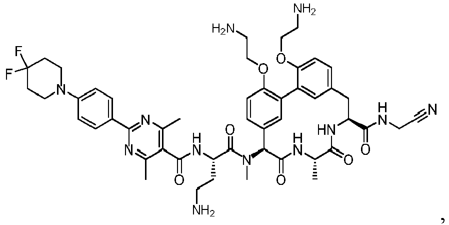 |
| 323 | 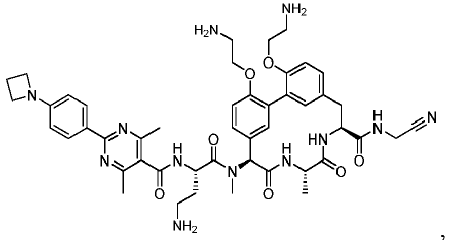 |
| 324 | 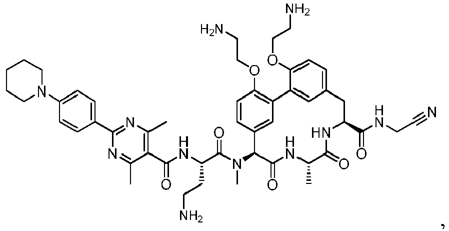 |
| 325 | 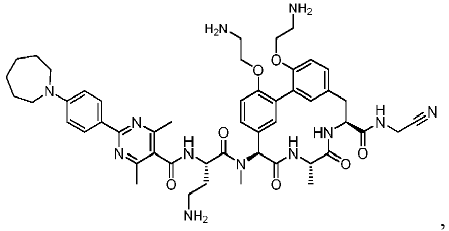 |
| 326 | 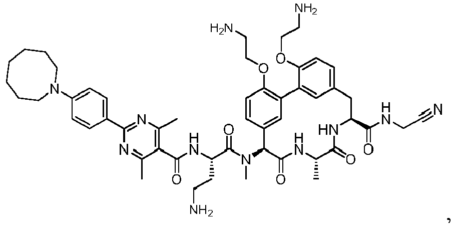 |
| 327 | 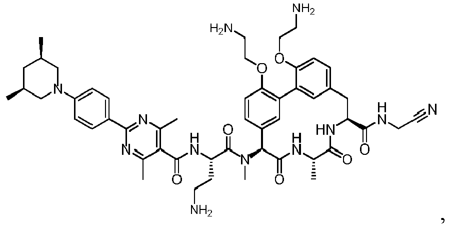 |
| 328 | 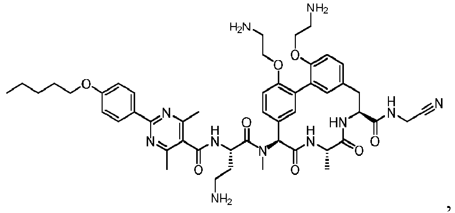 |
| 329 | 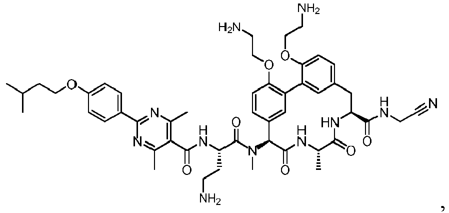 |
| 330 | 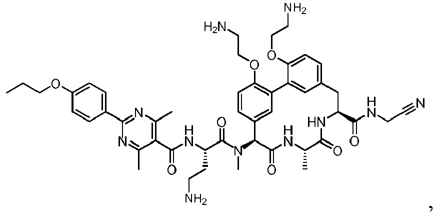 |
| 331 | 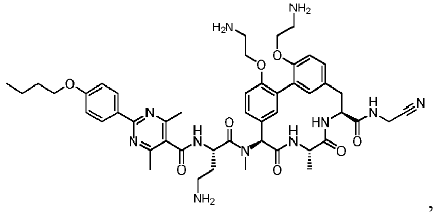 |
| 332 | 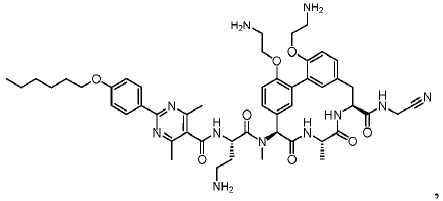 |
| 333 | 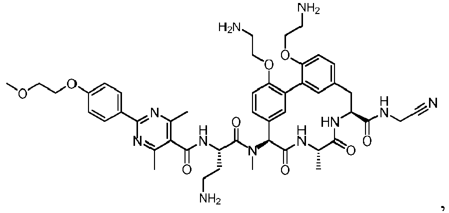 |
| 334 | 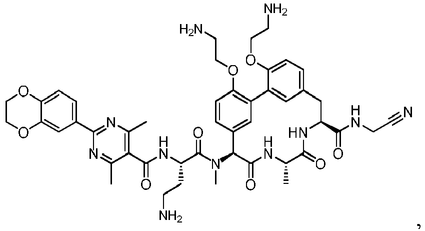 |
| 335 | 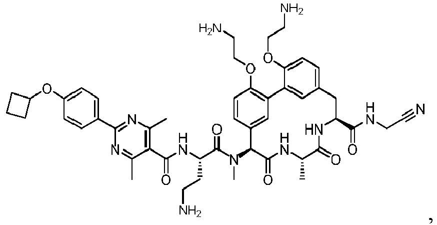 |
| 336 | 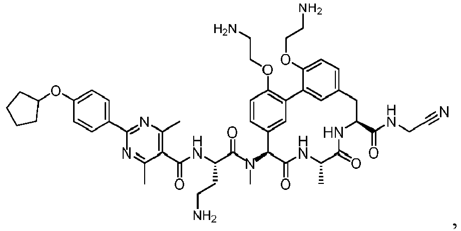 |
| 337 | 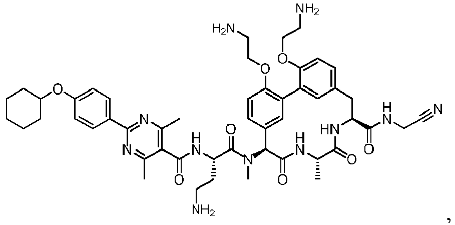 |
| 338 | 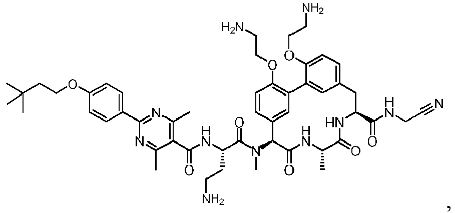 |
| 339 | 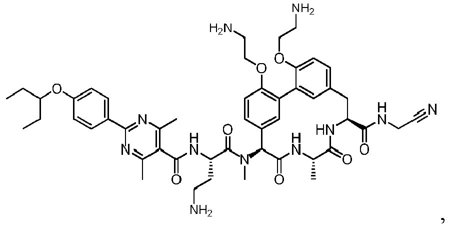 |
| 340 | 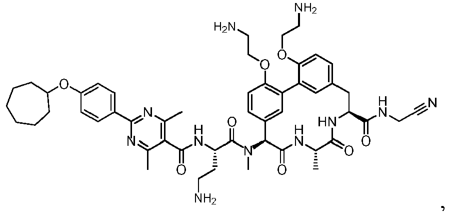 |
| 341 | 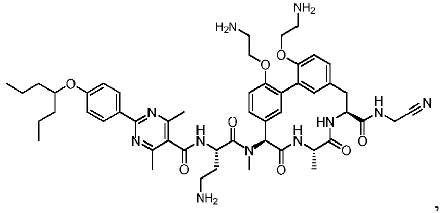 |
| 342 | 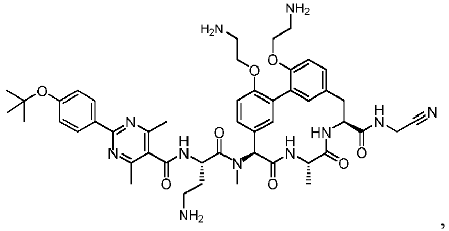 |
| 343 | 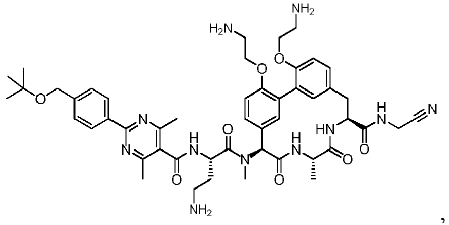 |
| 344 | 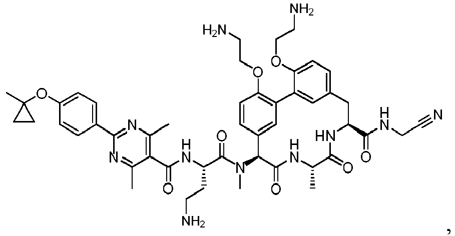 |
| 345 | 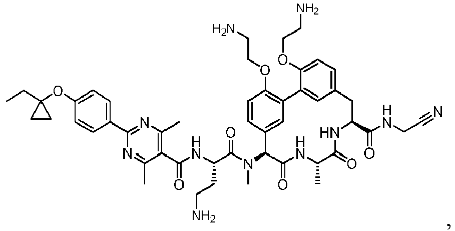 |
| 346 | 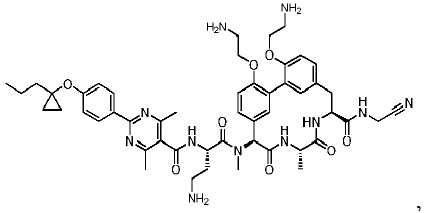 |
| 347 | 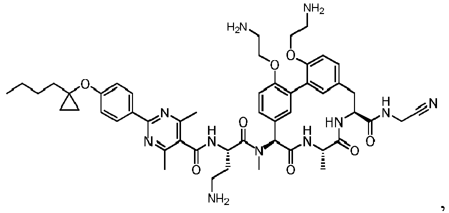 |
| 348 | 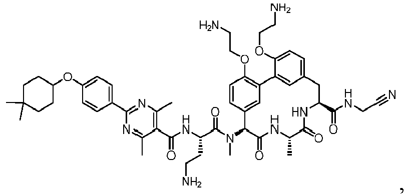 |
| 349 | 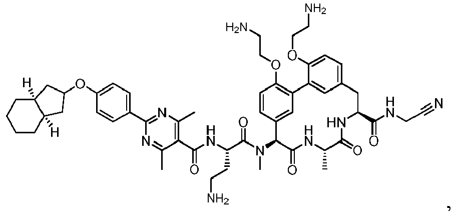 |
| 350 | 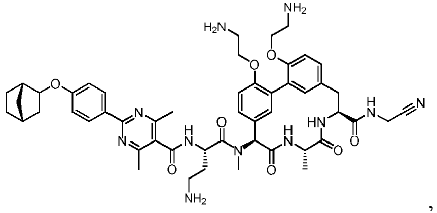 |
| 351 | 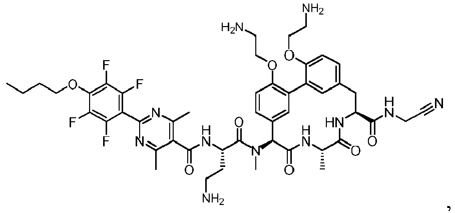 |
| 352 | 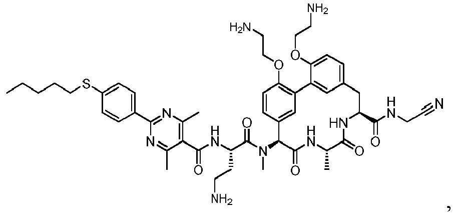 |
| 353 | 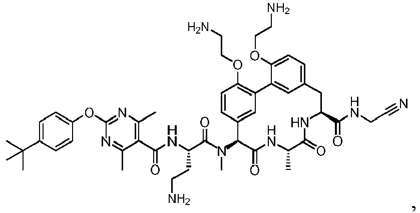 |
| 354 | 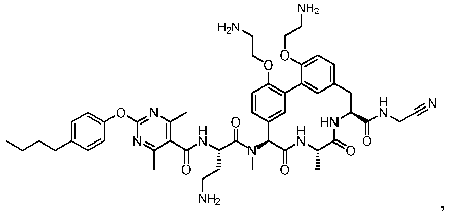 |
| 355 | 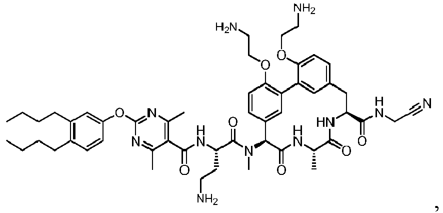 |
| 356 | 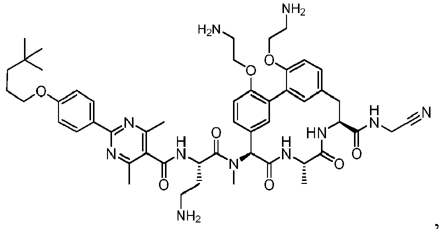 |
| 357 | 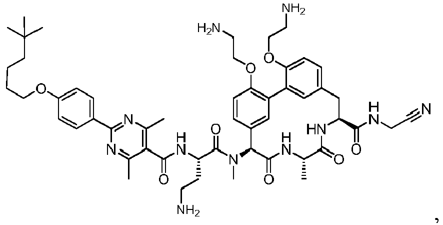 |
| 358 | 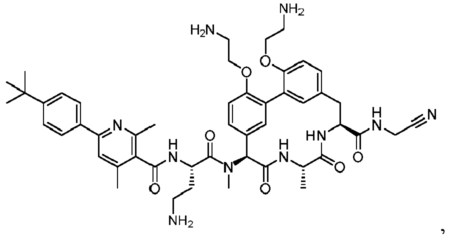 |
| 359 | 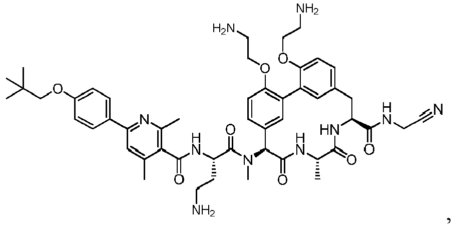 |
| 360 | 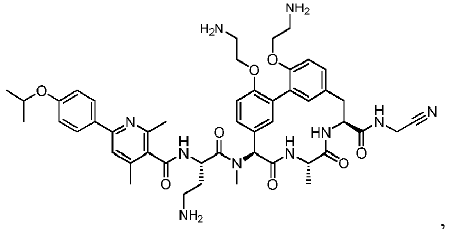 |
| 361 | 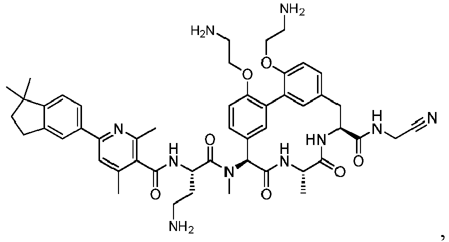 |
| 362 | 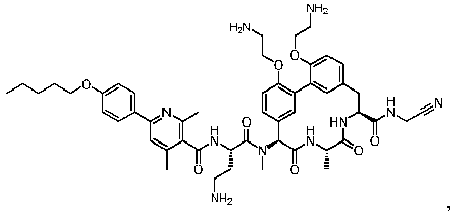 |
| 363 | 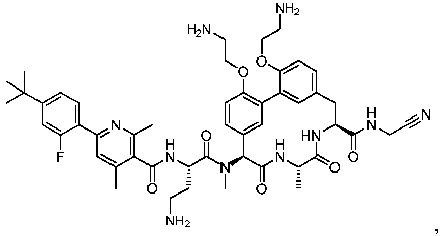 |
| 364 | 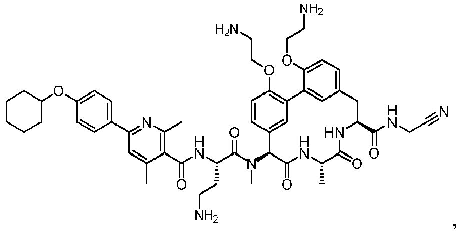 |
| 365 | 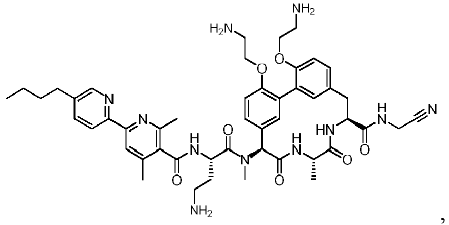 |
| 366 | 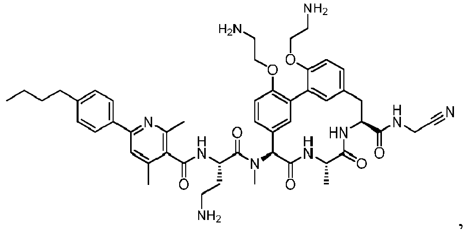 |
| 367 | 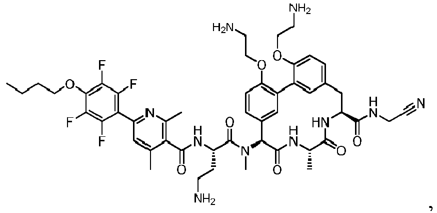 |
| 368 | 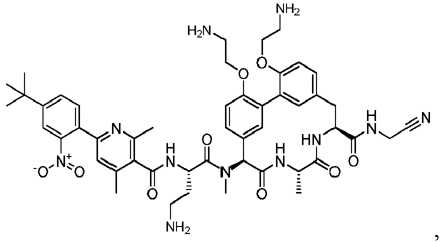 |
| 369 | 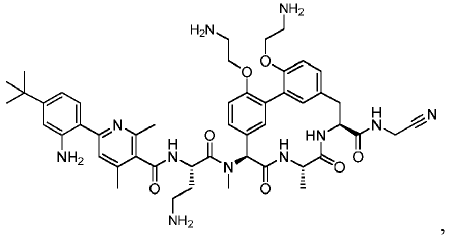 |
| 370 | 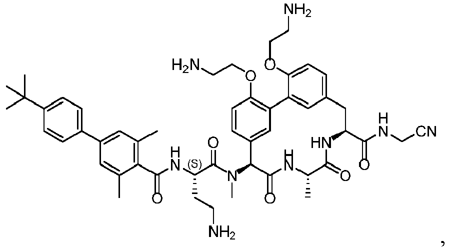 |
| 371 | 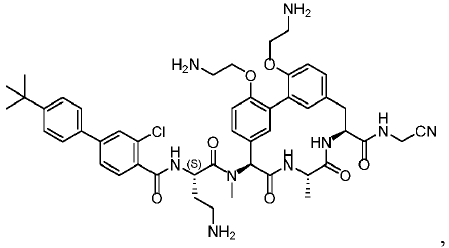 |
| 372 | 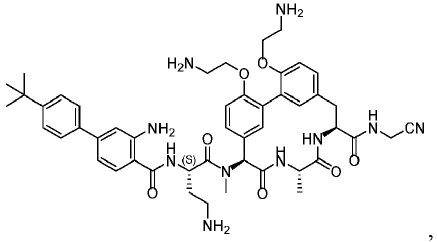 |
| 373 | 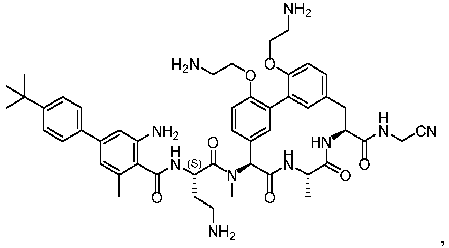 |
| 374 | 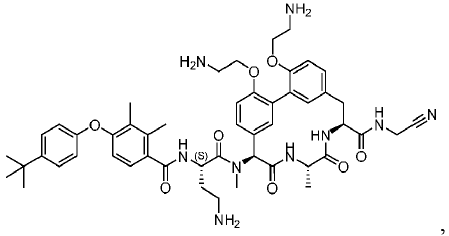 |
| 375 | 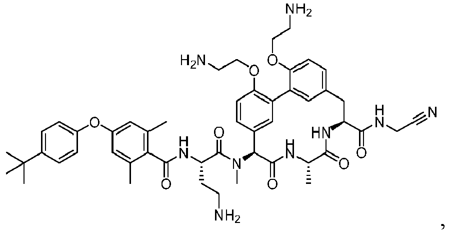 |
| 376 | 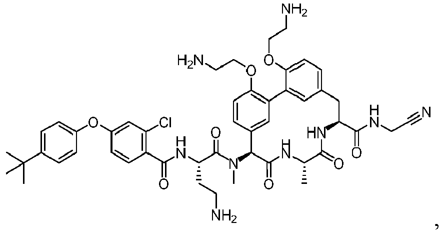 |
| 377 | 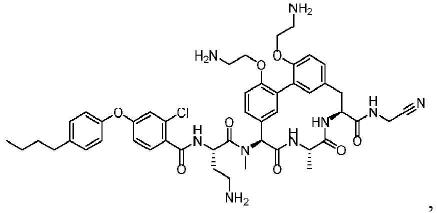 |
| 378 | 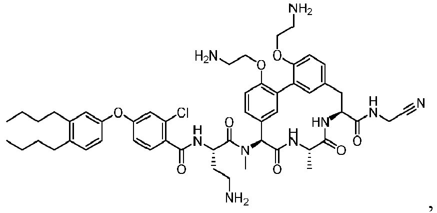 |
| 379 | 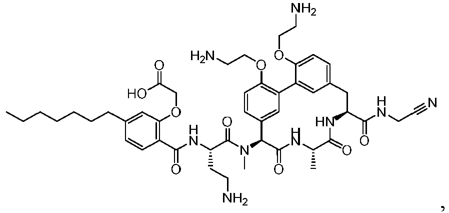 |
| 380 | 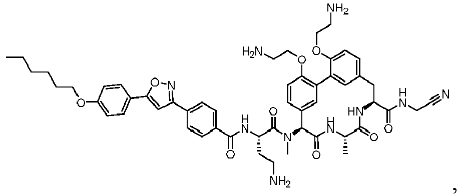 |
| 381 | 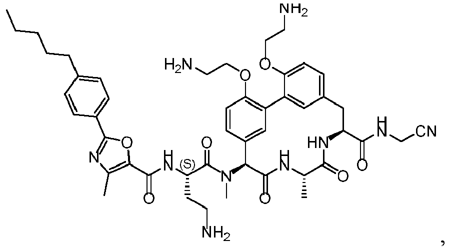 |
| 382 | 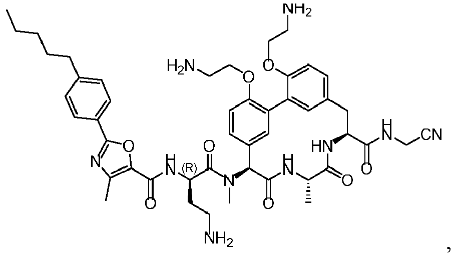 |
| 383 | 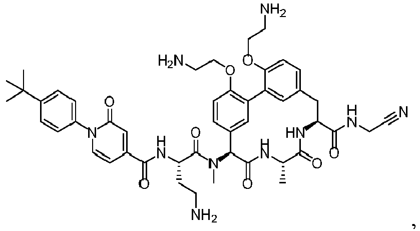 |
| 384 | 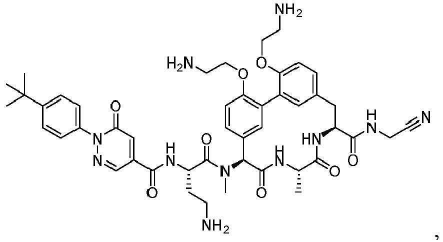 |
| 385 | 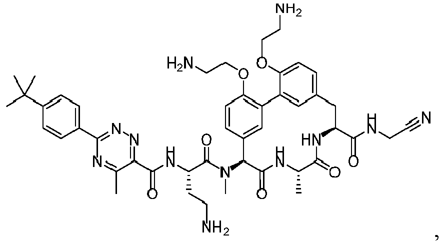 |
| 386 | 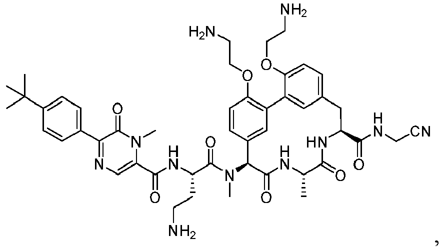 |
| 387 | 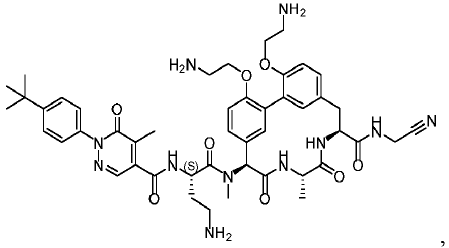 |
| 388 | 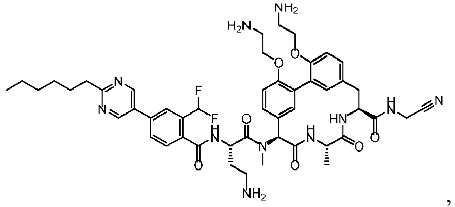 |
| 389 | 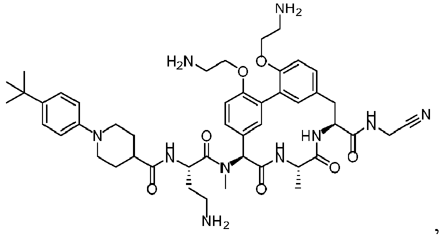 |
| 390 | 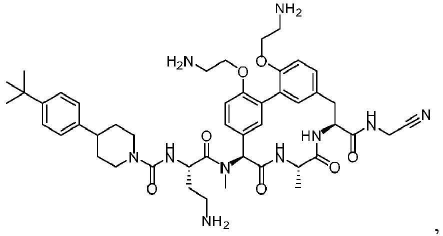 |
| 391 | 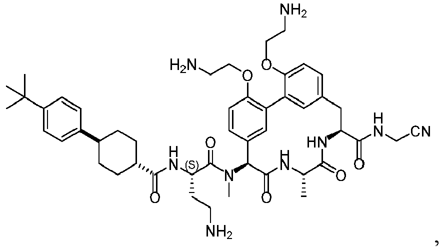 |
| 392 | 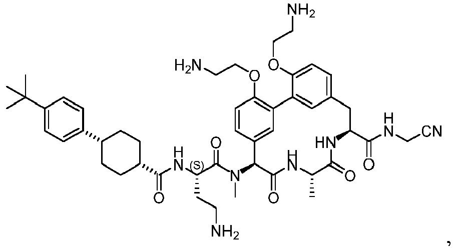 |
| 393 | 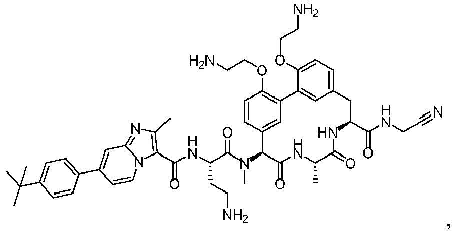 |
| 394 | 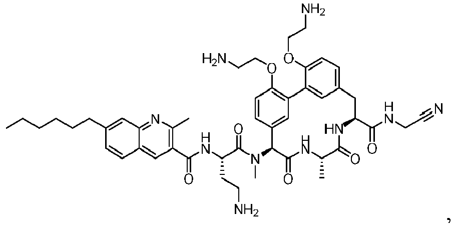 |
| 395 | 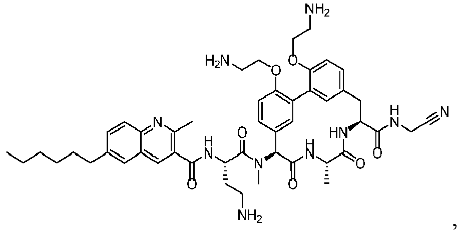 |
| 396 | 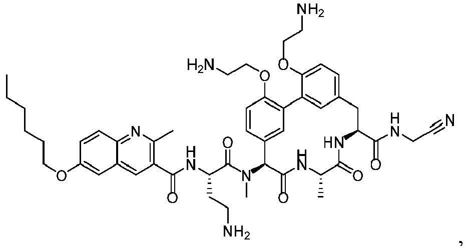 |
| 397 | 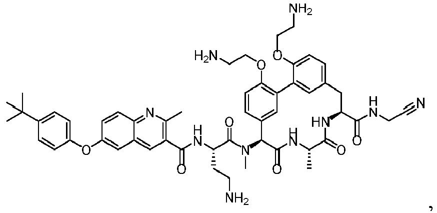 |
| 398 | 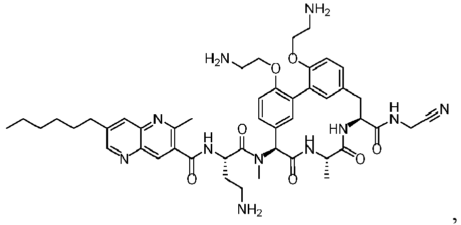 |
| 399 | 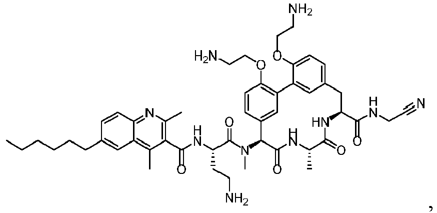 |
| 400 | 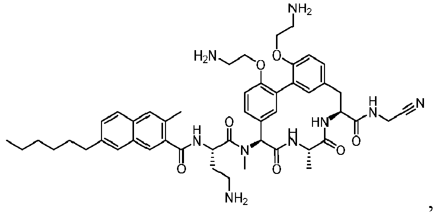 |
| 401 | 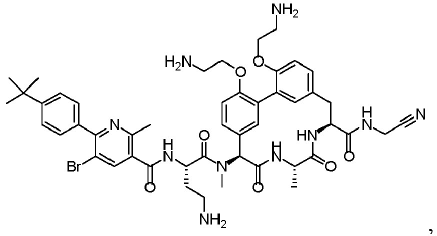 |
| 402 | 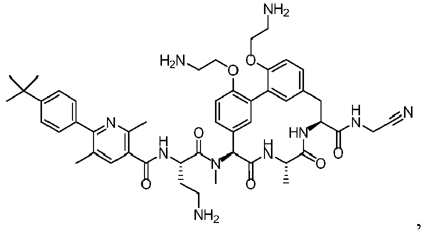 |
| 403 | 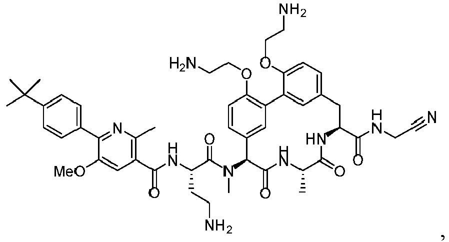 |
| 404 | 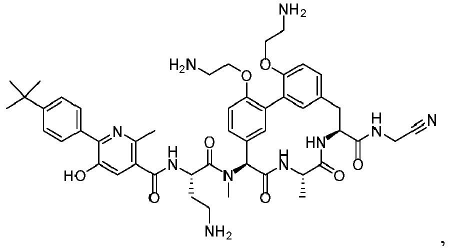 |
| 405 | 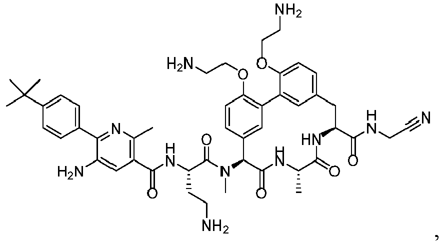 |
| 406 | 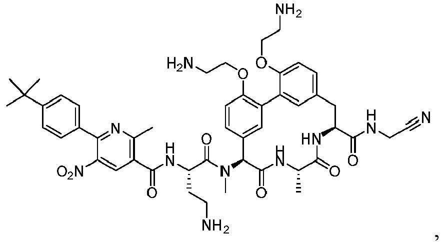 |
| 407 | 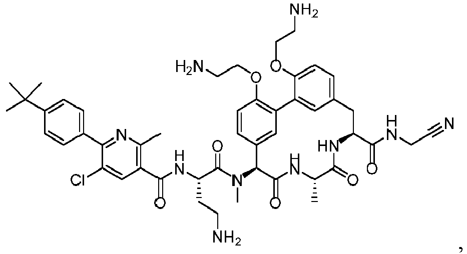 |
| 408 | 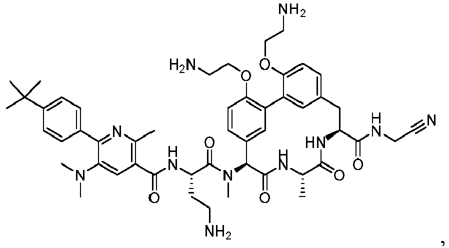 |
| 409 | 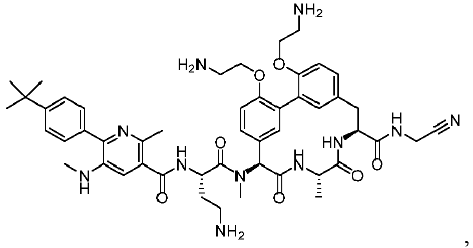 |
| 410 | 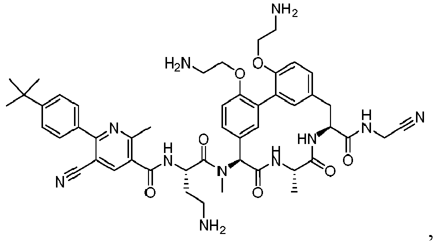 |
| 411 | 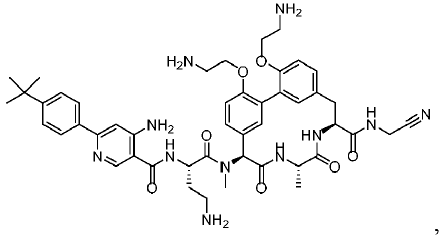 |
| 412 | 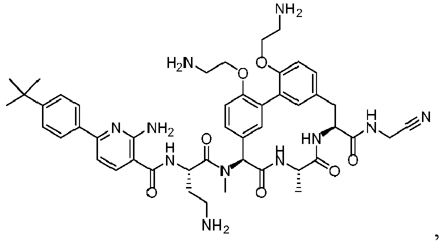 |
| 413 | 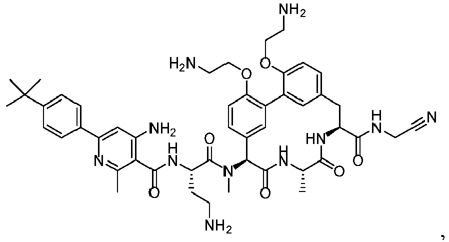 |
| 414 | 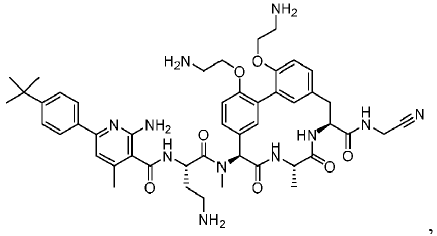 |
| 415 | 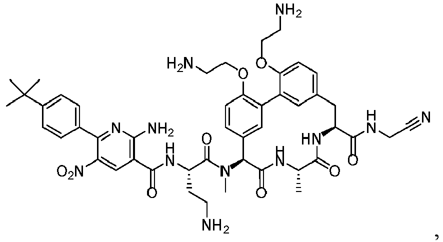 |
| 416 | 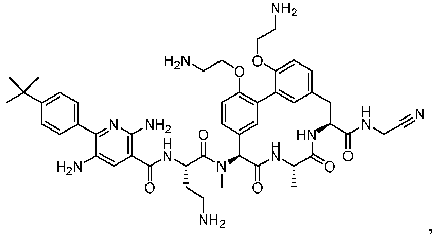 |
| 417 | 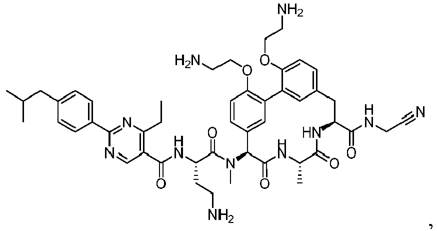 |
| 418 | 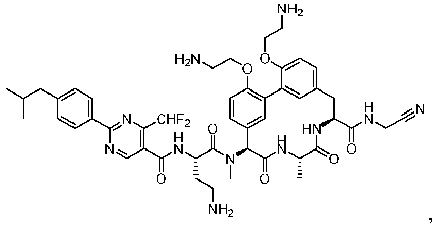 |
| 419 | 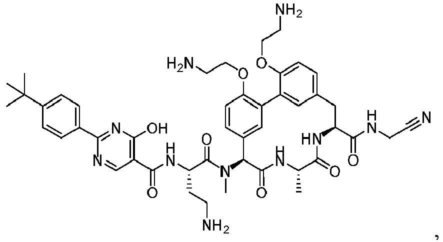 |
| 420 | 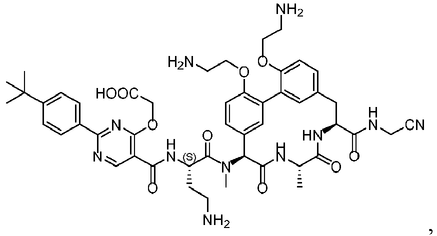 |
| 421 | 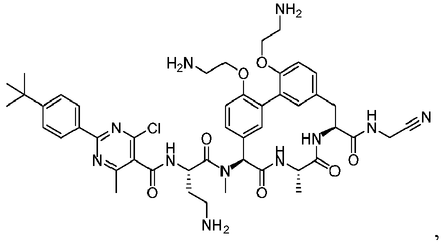 |
| 422 | 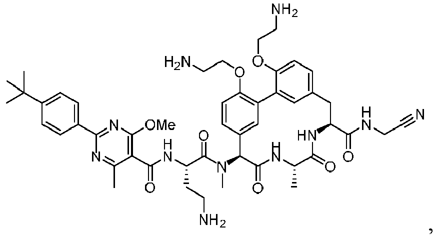 |
| 423 | 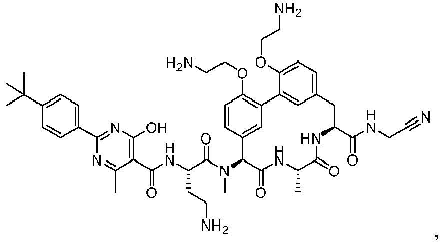 |
| 424 | 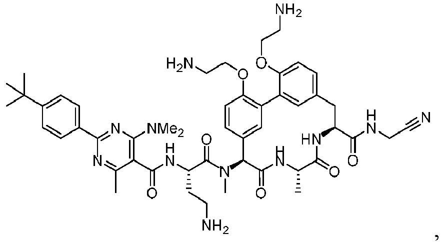 |
| 425 | 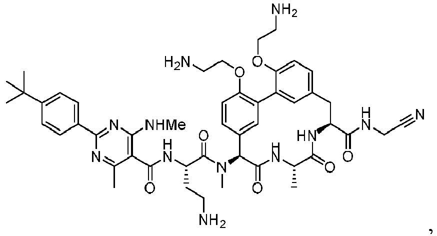 |
| 426 | 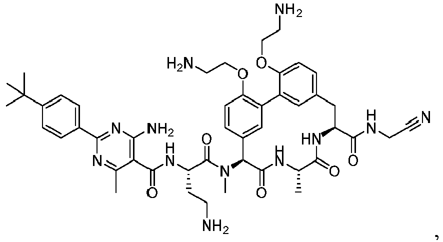 |
| 427 | 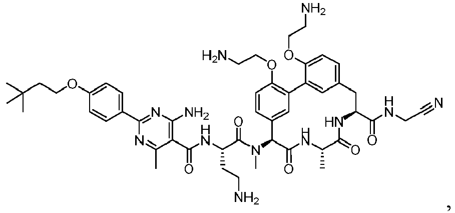 |
| 428 | 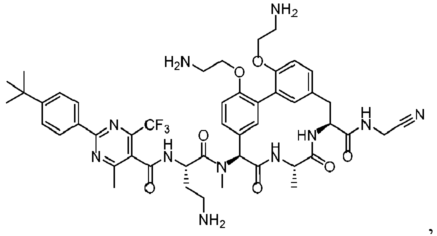 |
| 429 | 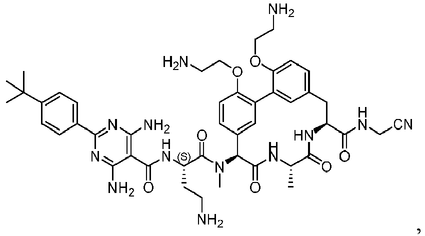 |
| 430 | 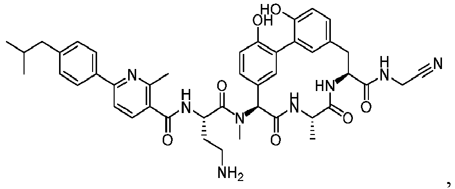 |
| 431 | 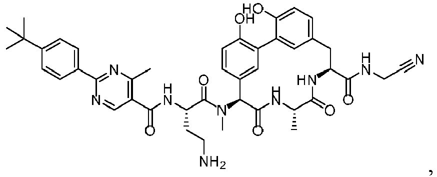 |
| 432 | 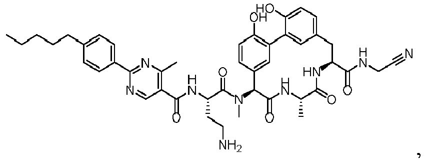 |
| 433 | 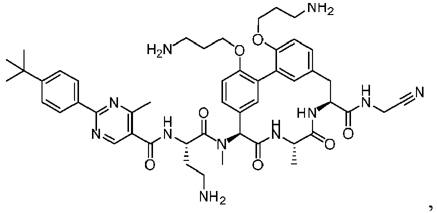 |
| 434 | 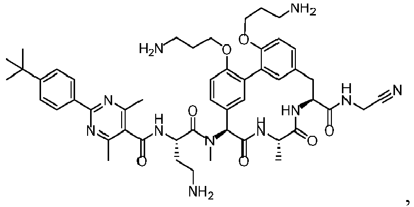 |
| 435 | 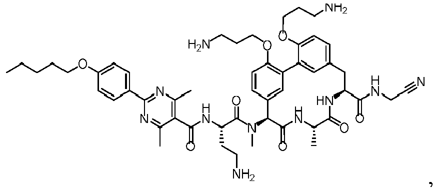 |
| 436 | 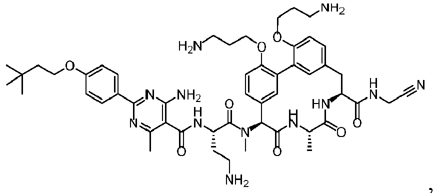 |
| 437 | 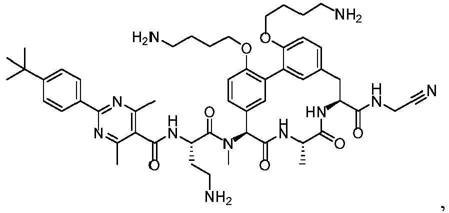 |
| 438 | 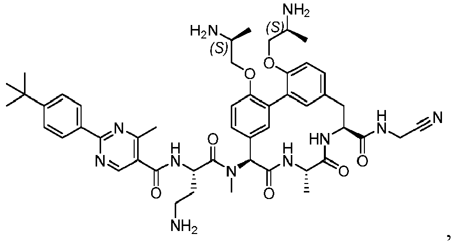 |
| 439 | 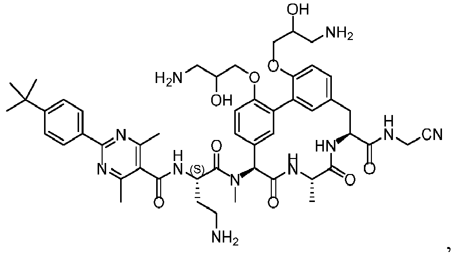 |
| 440 | 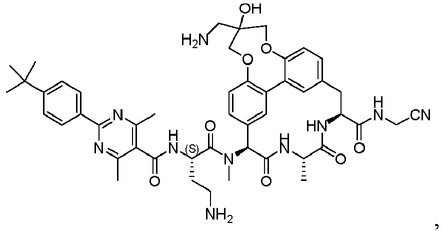 |
| 441 | 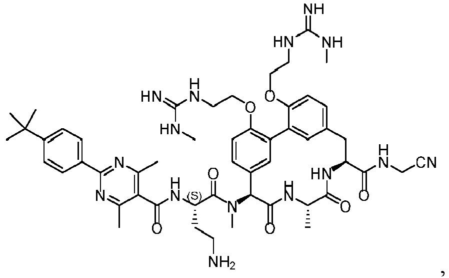 |
| 442 | 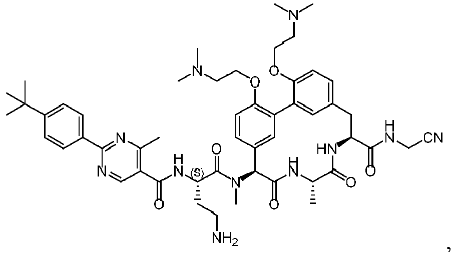 |
| 443 | 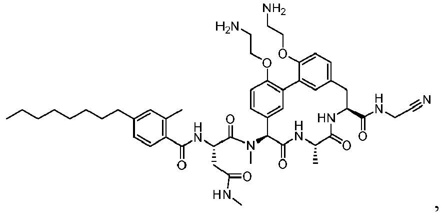 |
| 444 | 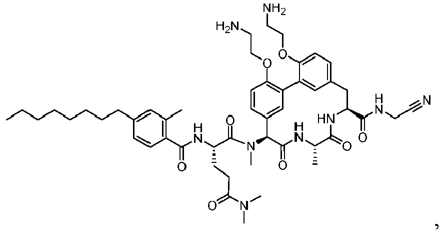 |
| 445 | 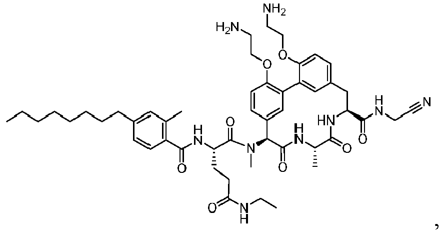 |
| 446 | 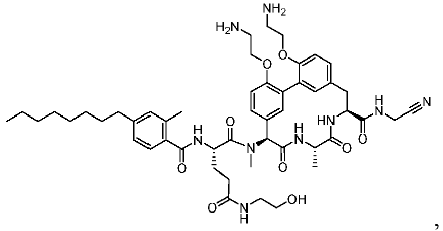 |
| 447 | 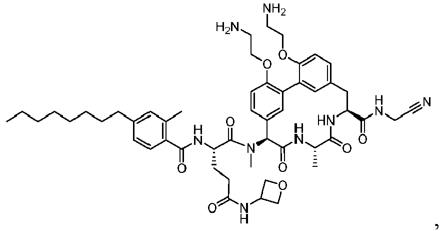 |
| 448 | 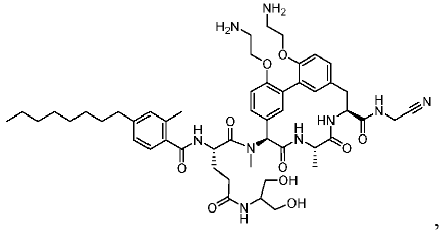 |
| 449 | 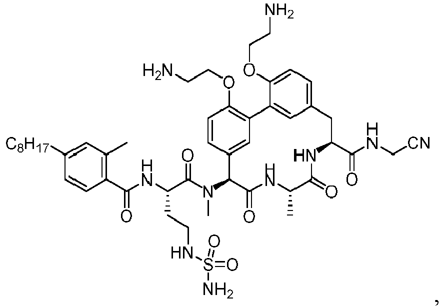 |
| 450 | 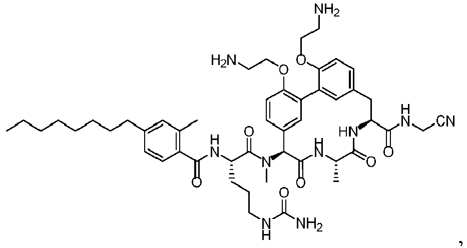 |
| 451 | 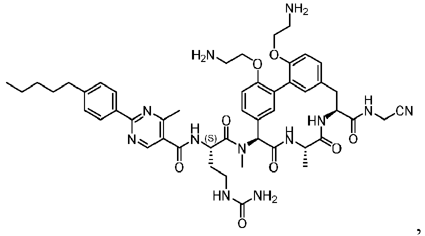 |
| 452 | 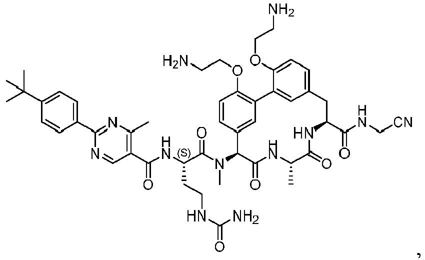 |
| 453 | 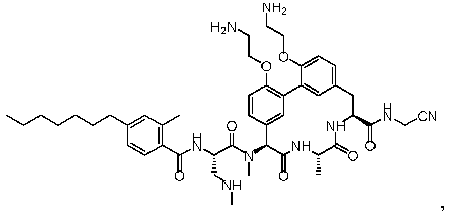 |
| 454 | 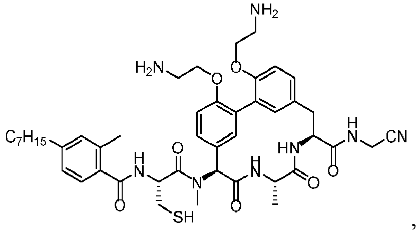 |
| 455 | 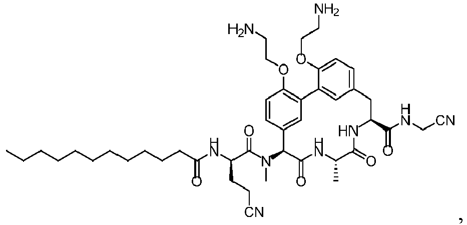 |
| 456 | 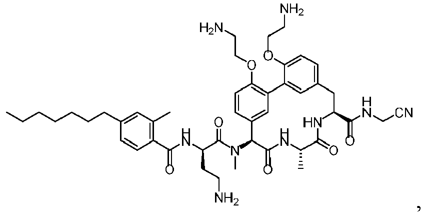 |
| 457 | 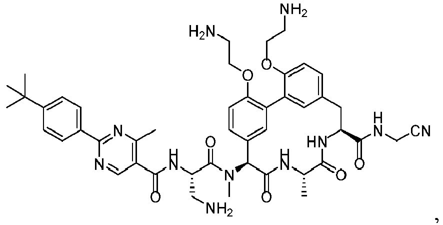 |
| 458 | 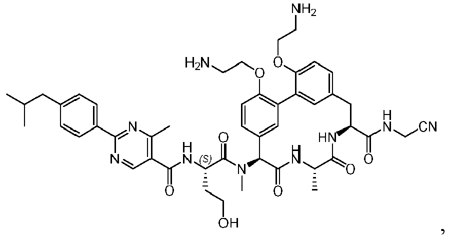 |
| 459 | 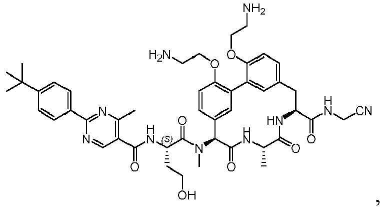 |
| 460 | 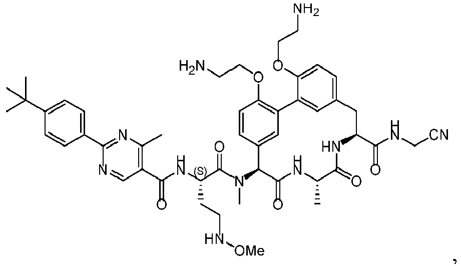 |
| 461 | 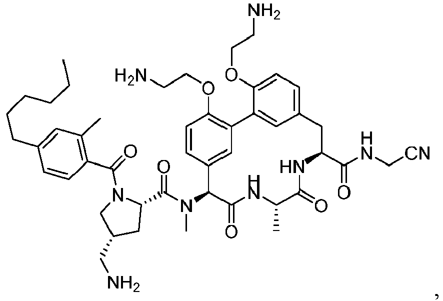 |
| 462 | 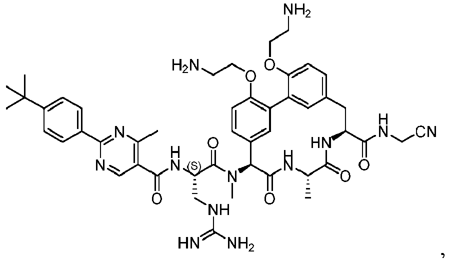 |
| 463 | 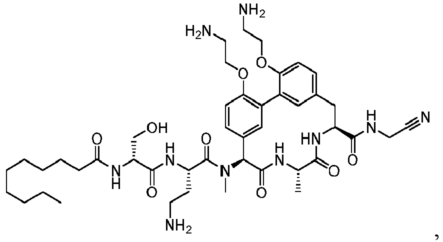 |
| 464 | 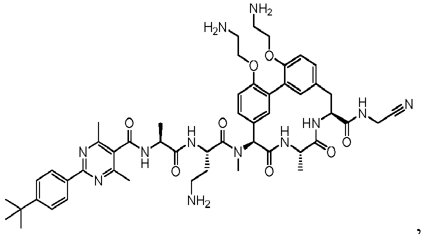 |
| 465 | 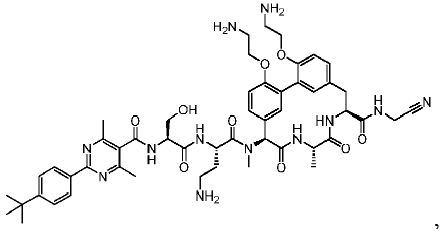 |
| 466 | 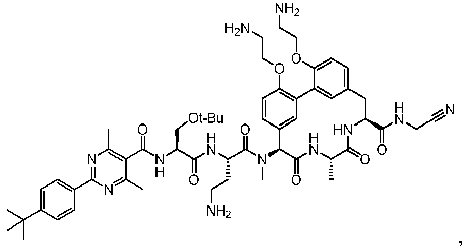 |
| 467 | 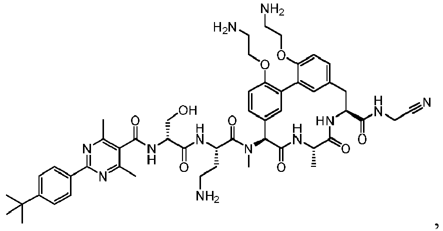 |
| 468 | 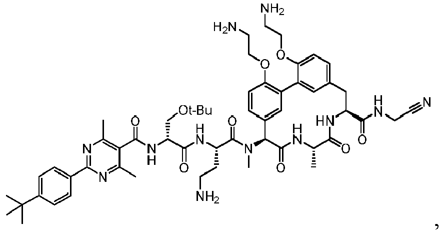 |
| 469 | 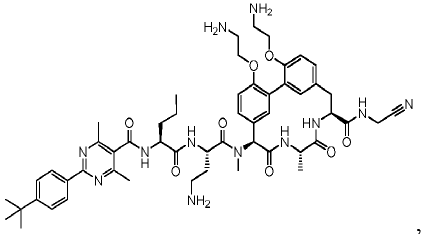 |
| 470 | 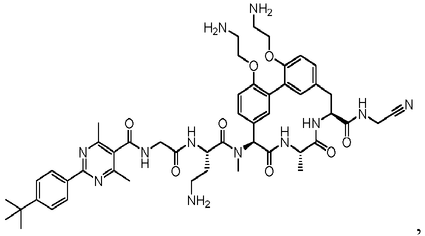 |
| 471 | 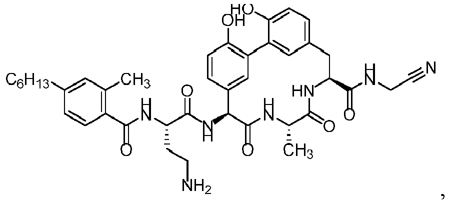 |
| 472 | 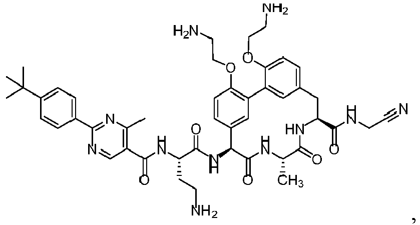 |
| 473 | 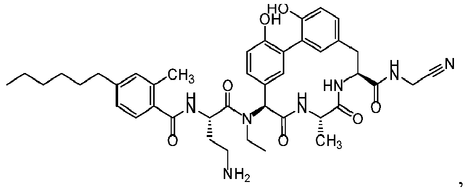 |
| 474 | 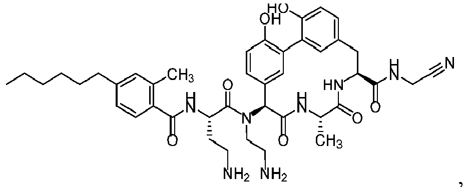 |
| 475 | 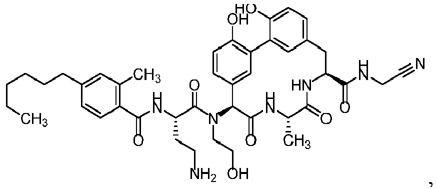 |
| 476 | 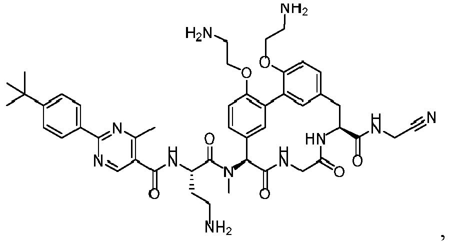 |
| 477 | 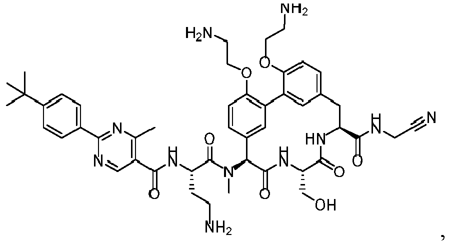 |
| 478 | 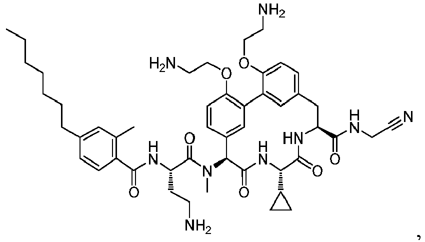 |
| 479 | 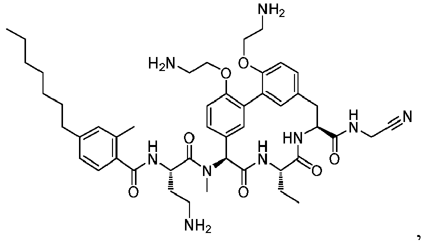 |
| 480 | 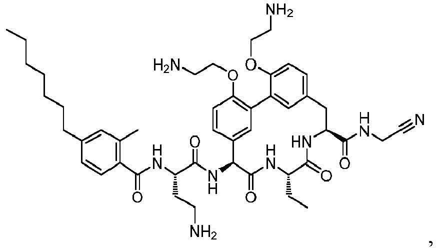 |
| 481 | 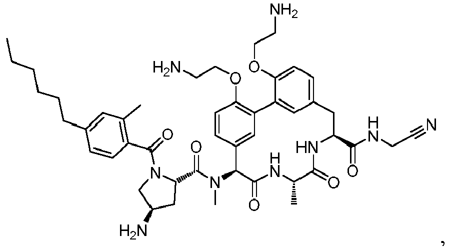 |
| 482 | 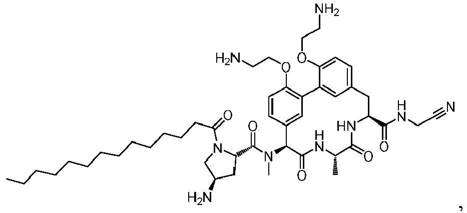 |
| 483 | 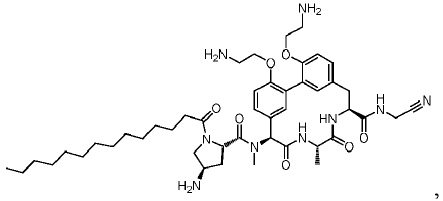 |
| 484 | 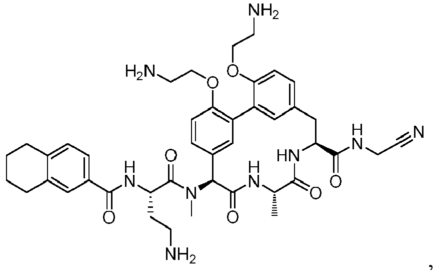 |
| 485 | 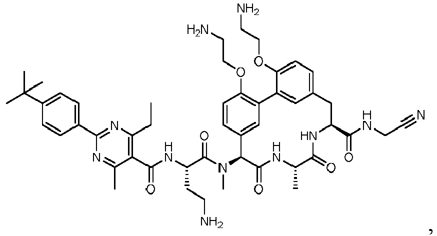 |
| 486 | 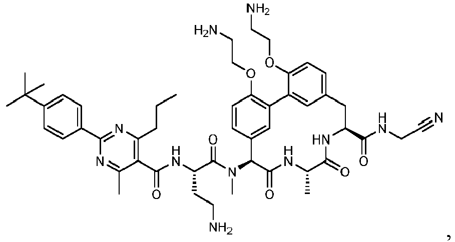 |
| 487 | 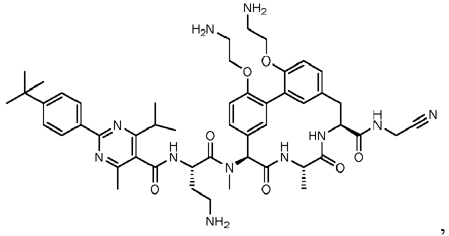 |
| 488 | 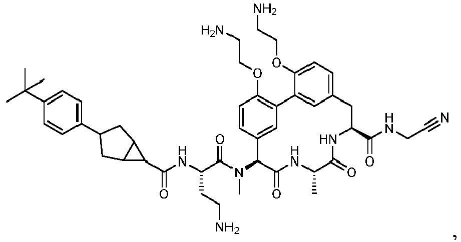 |
| 489 | 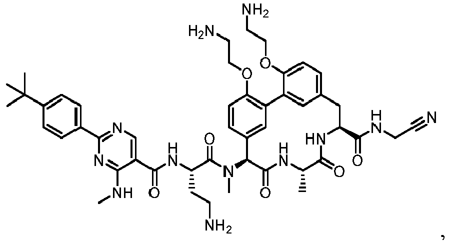 |
| 490 | 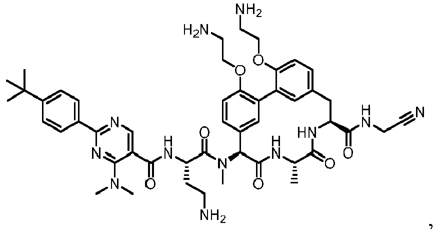 |
| 491 | 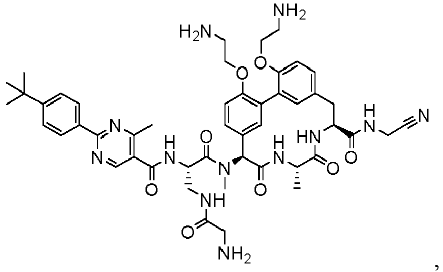 |
| 492 | 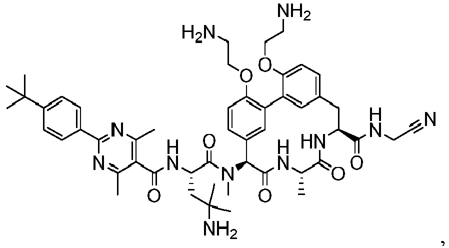 |
| 493 | 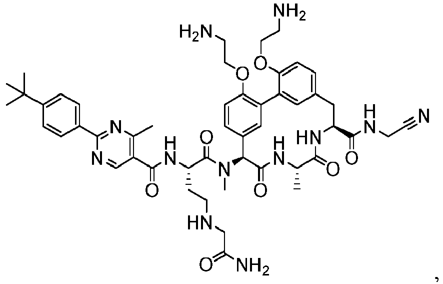 |
| 494 | 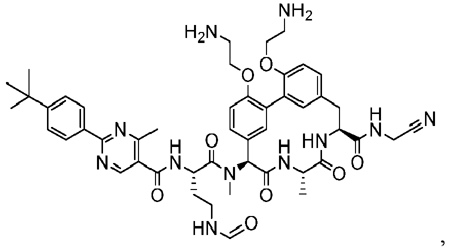 |
| 495 | 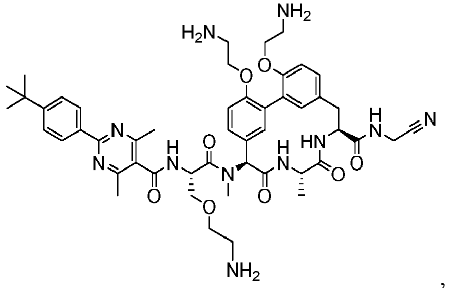 |
| 496 | 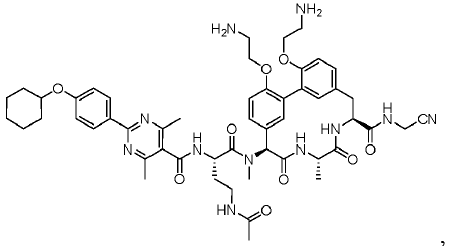 |
| 497 | 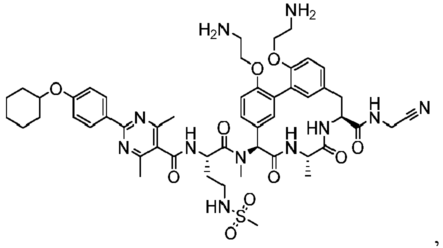 |
| 498 | 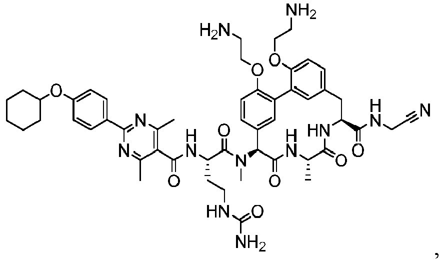 |
| 499 | 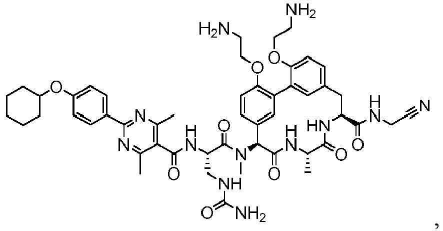 |
| 500 | 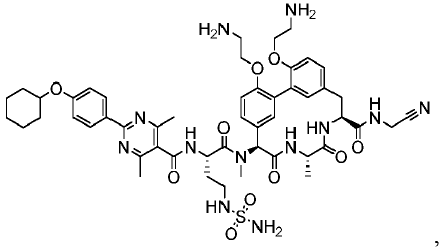 |
| 501 | 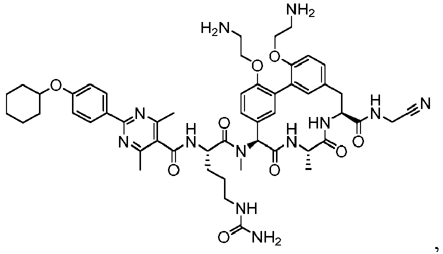 |
| 502 | 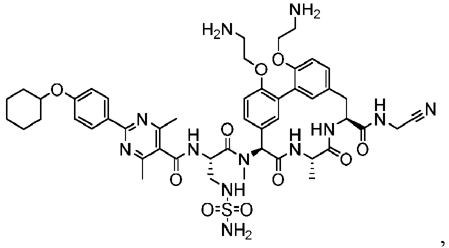 |
| 503 | 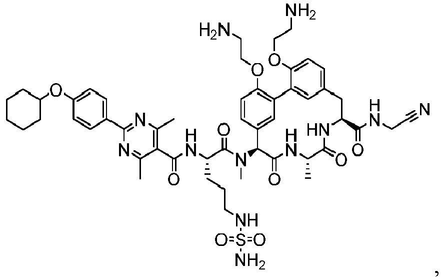 |
| 504 | 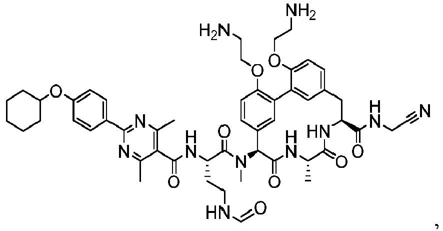 |
| 505 | 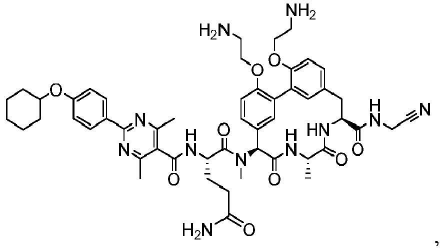 |
| 506 | 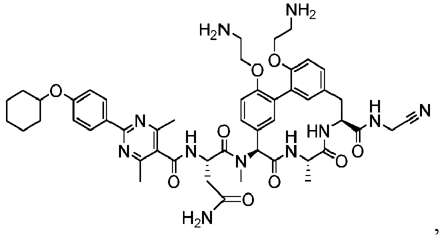 |
| 507 | 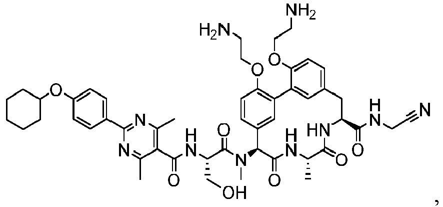 |
| 508 | 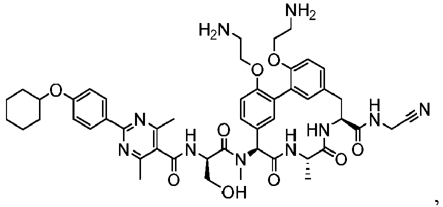 |
| 509 | 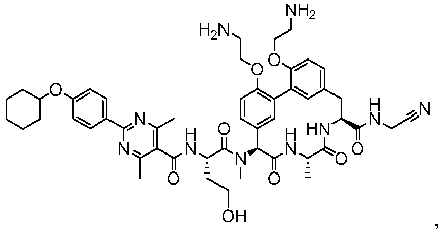 |
| 510 | 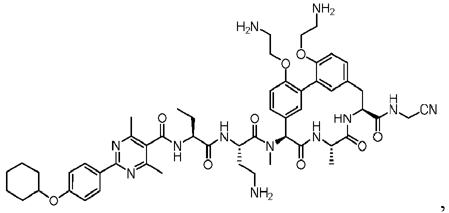 |
| 511 | 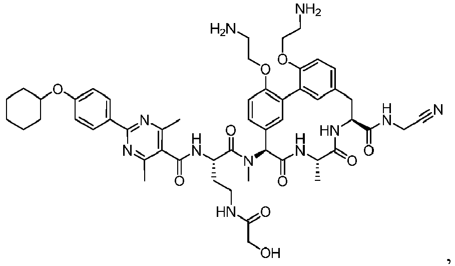 |
| 512 | 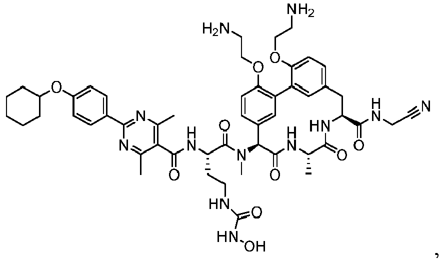 |
| 513 | 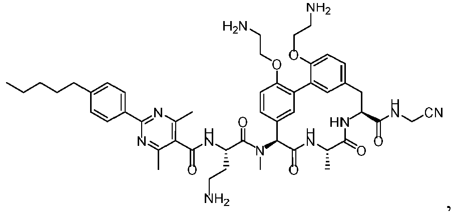 |
| 514 | 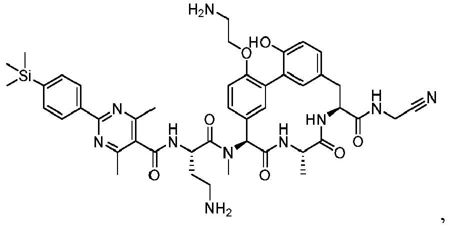 |
| 516 | 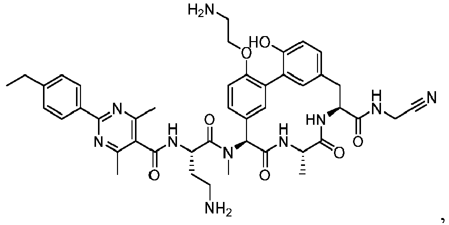 |
| 517 | 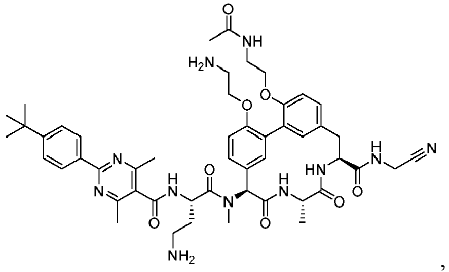 |
| 518 | 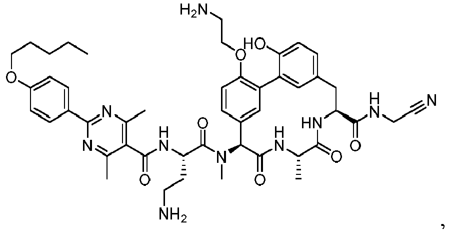 |
| 519 | 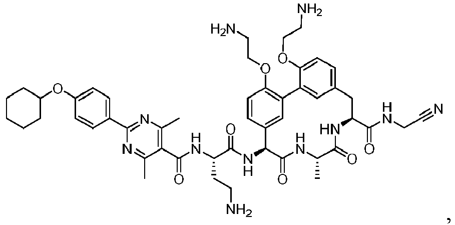 |
| 520 | 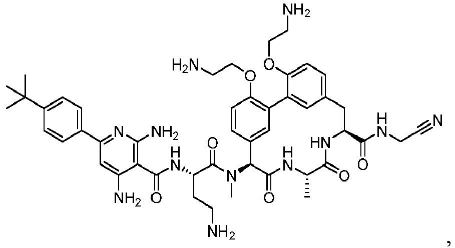 |
| 521 | 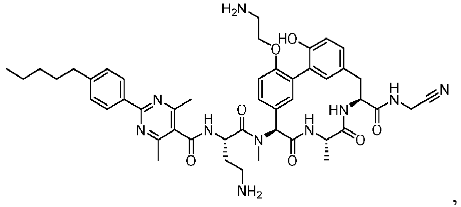 |
| 522 | 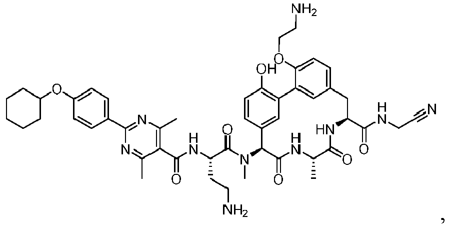 |
| 523 | 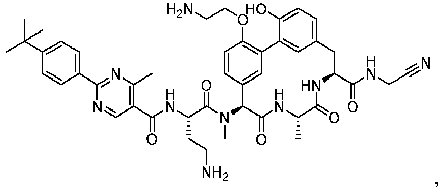 |
| 524 | 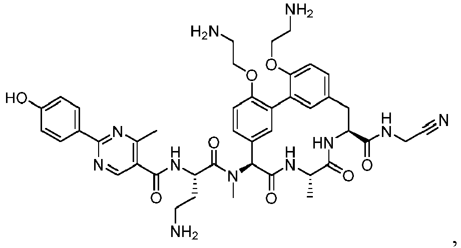 |
| 525 | 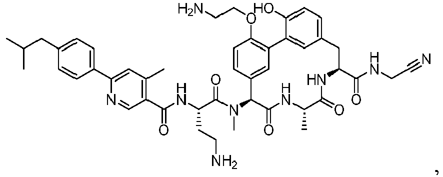 |
| 526 | 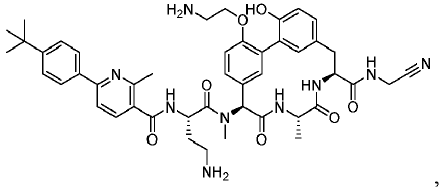 |
| 527 | 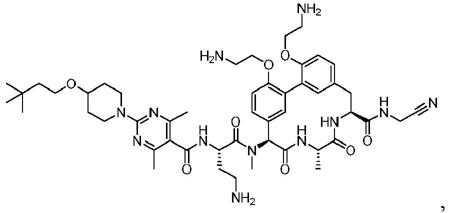 |
| 528 | 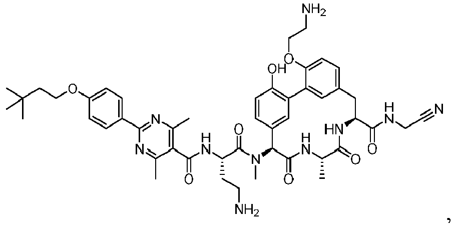 |
| 529 | 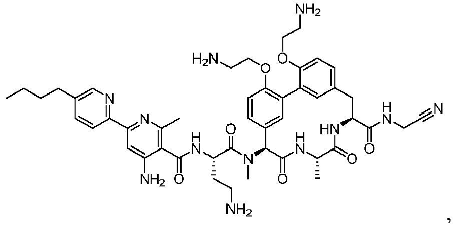 |
| 530 | 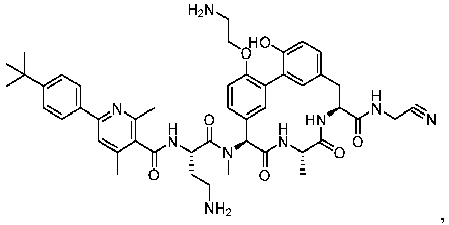 |
| 531 | 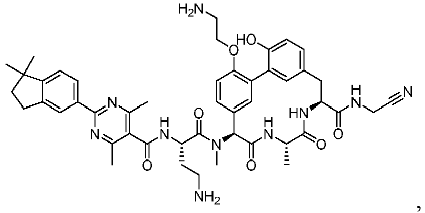 |
| 532 | 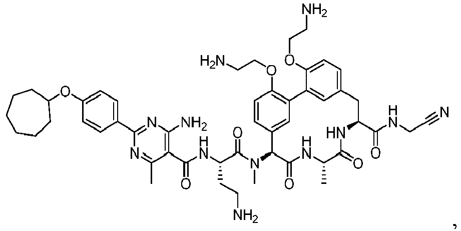 |
| 533 | 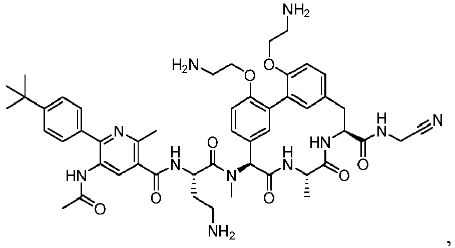 |
| 534 | 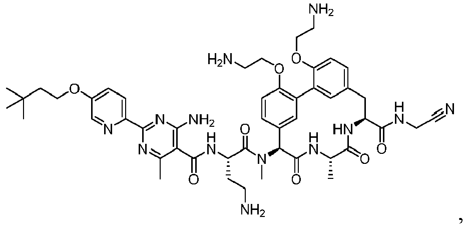 |
| 535 | 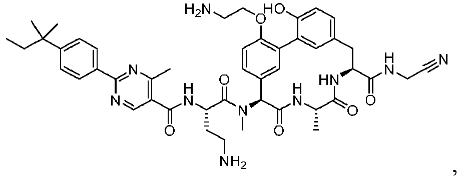 |
| 536 | 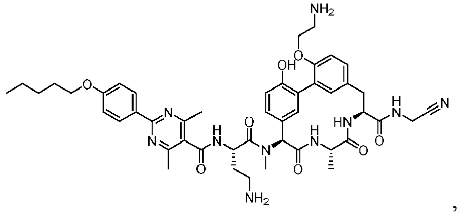 |
| 537 | 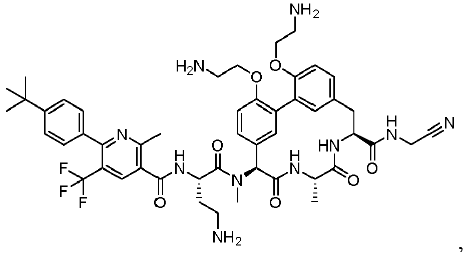 |
| 538 | 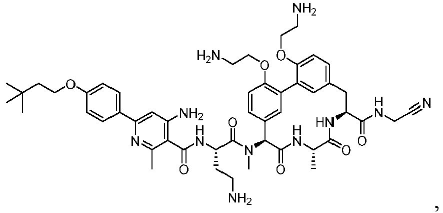 |
| 539 | 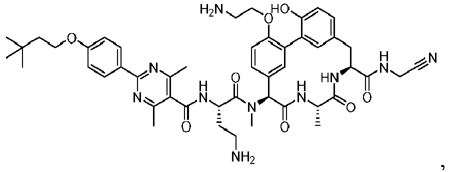 |
| 540 | 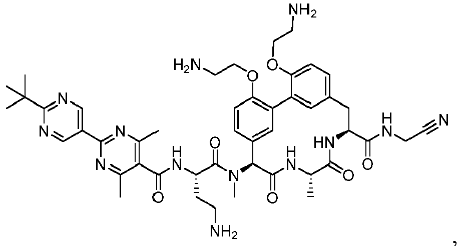 |
| 541 | 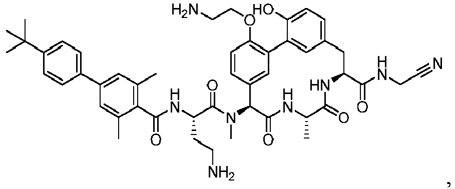 |
| 542 | 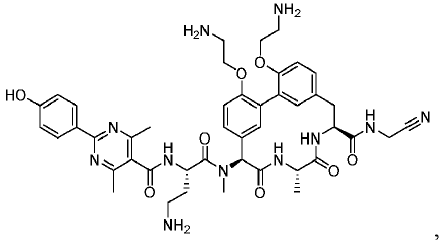 |
| 543 | 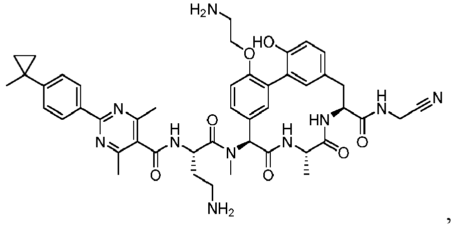 |
| 544 | 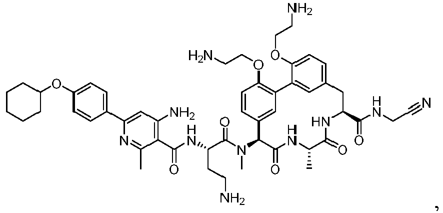 |
| 545 | 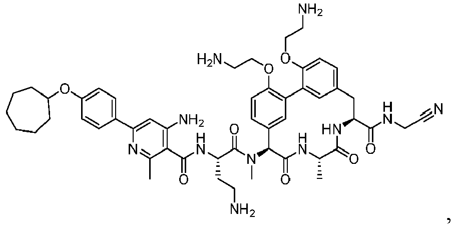 |
| 546 | 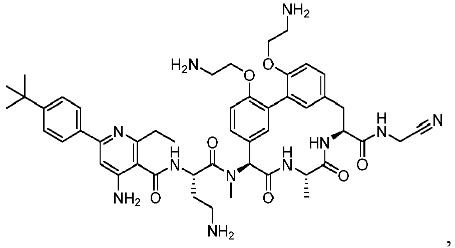 |
| 547 | 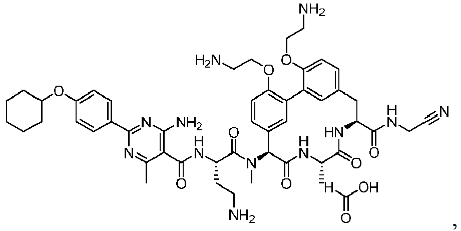 |
| 548 | 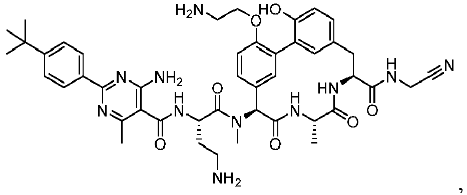 |
| 549 | 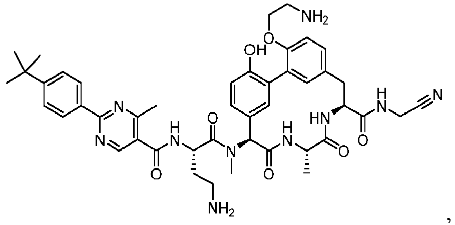 |
| 550 | 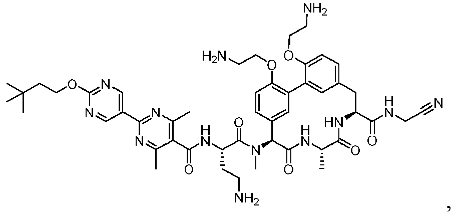 |
| 551 | 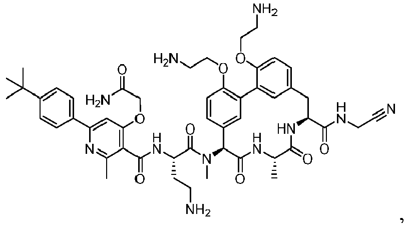 |
| 552 | 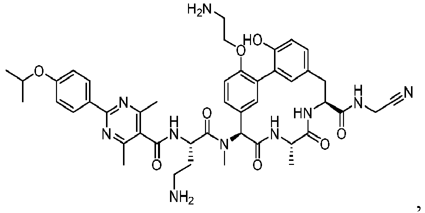 |
| 553 | 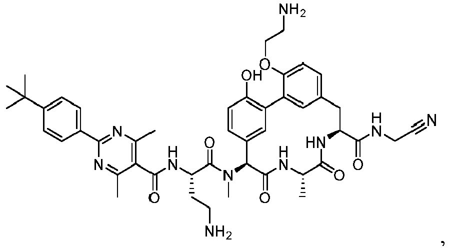 |
| 554 | 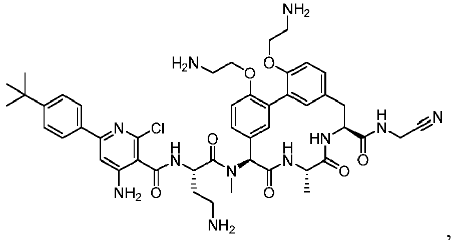 |
| 555 | 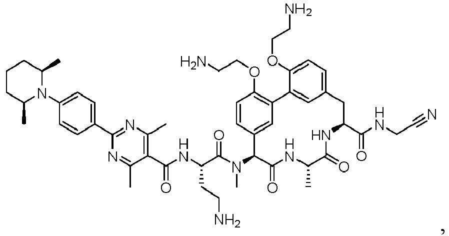 |
| 556 | 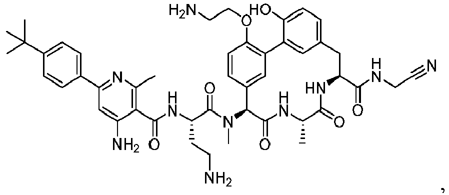 |
| 557 | 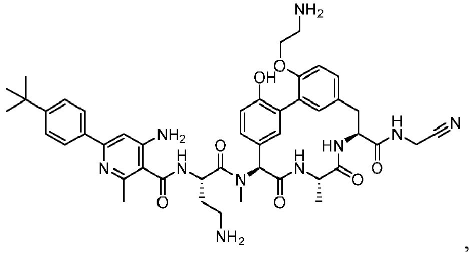 |
| 558 | 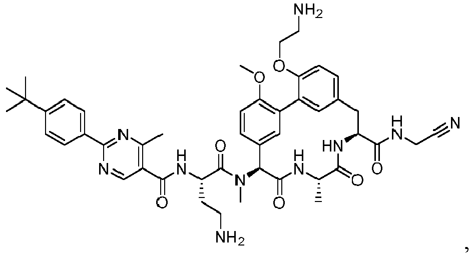 |
| 559 | 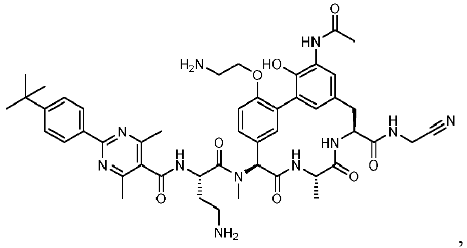 |
| 560 | 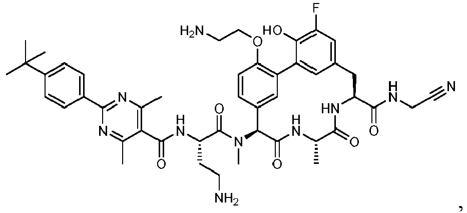 |
| 561 | 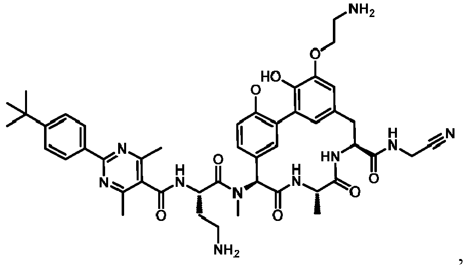 |
| 562 | 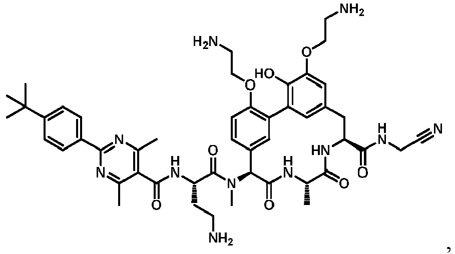 |
| 564 | 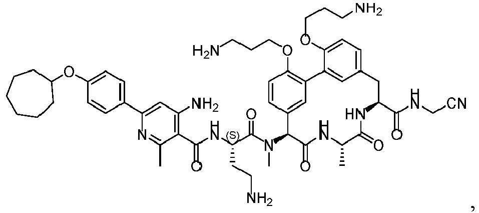 |
| 565 | 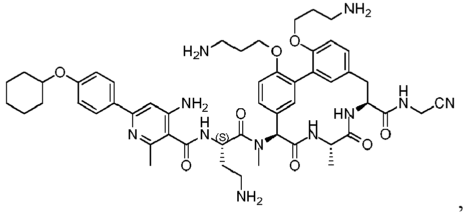 |
| 566 | 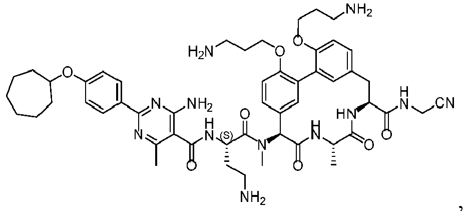 |
| 567 | 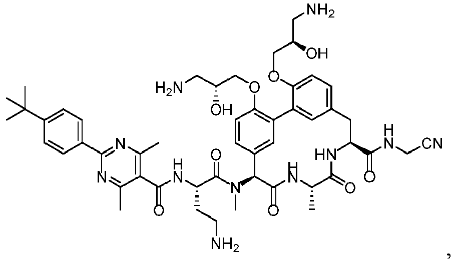 |
| 568 | 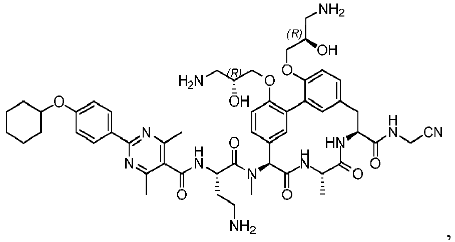 |
| 569 | 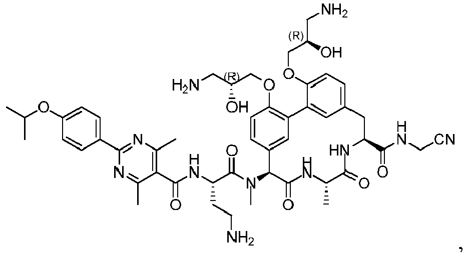 |
| 570 | 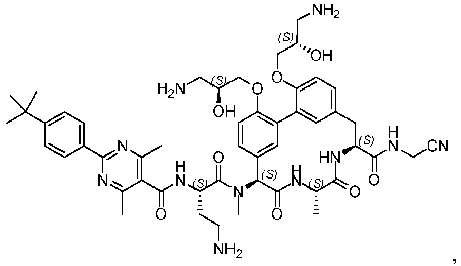 |
| 571 | 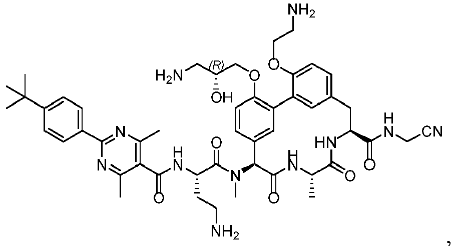 |
| 572 | 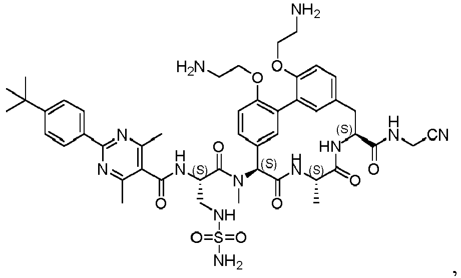 |
| 573 | 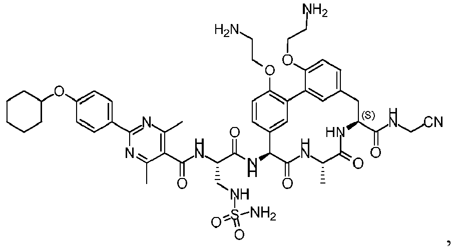 |
| 574 | 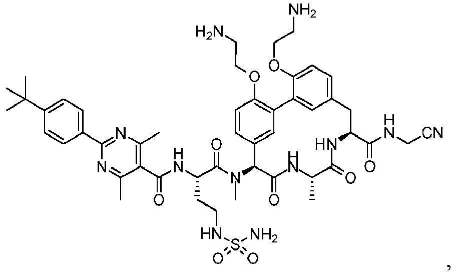 |
| 575 | 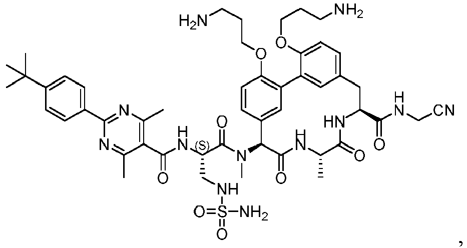 |
| 576 | 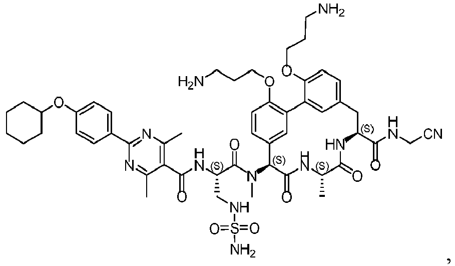 |
| 577 | 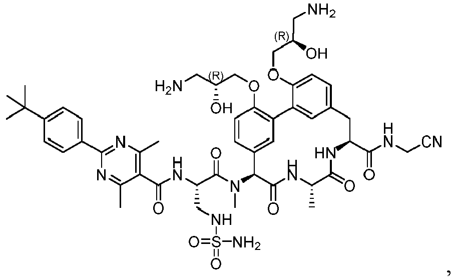 |
| 578 | 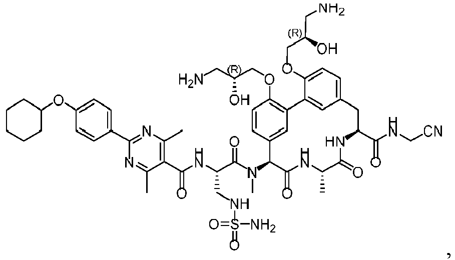 |
| 579 | 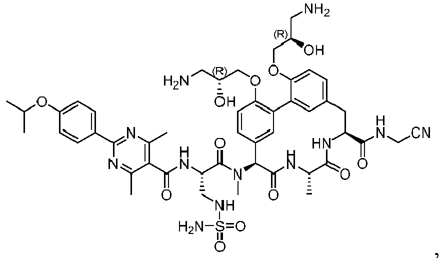 |
| 580 | 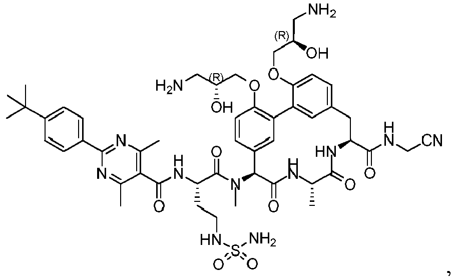 |
| 581 | 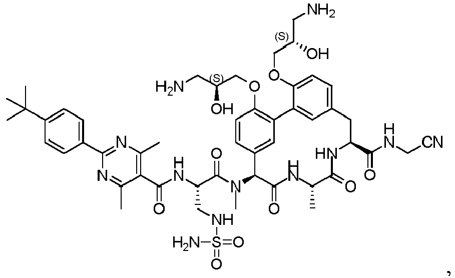 |
| 582 | 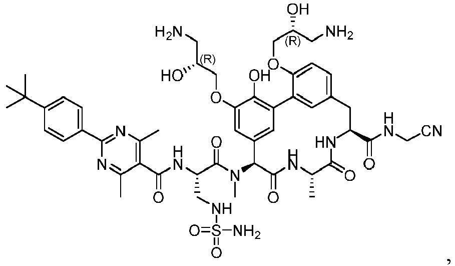 |
| 583 | 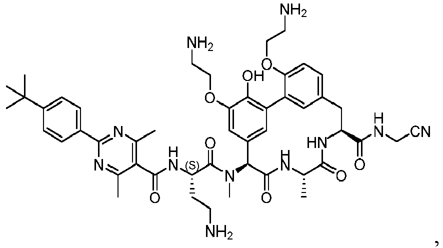 |
| 584 | 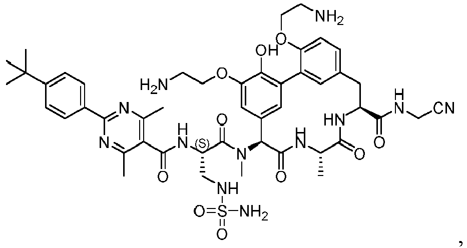 |
| 585 | 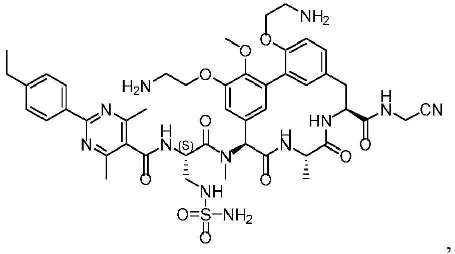 |
| 586 | 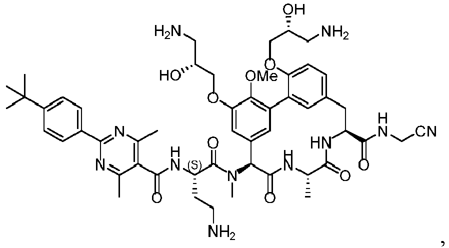 |
| 587 | 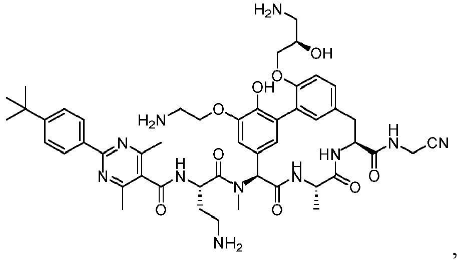 |
| 588 | 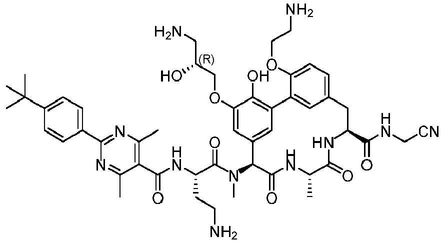 |
| 589 | 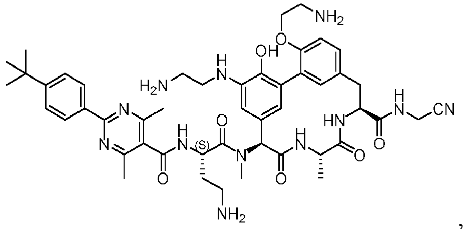 |
| 590 | 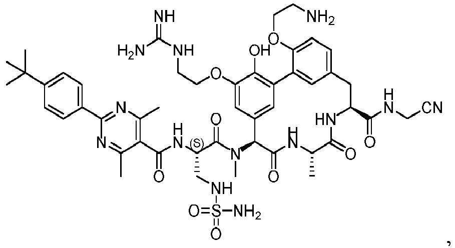 |
| 591 | 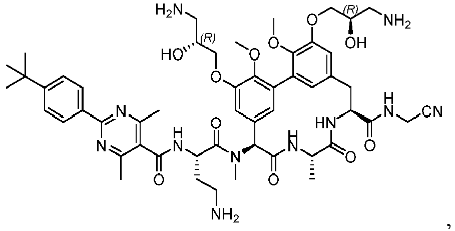 |
| 592 | 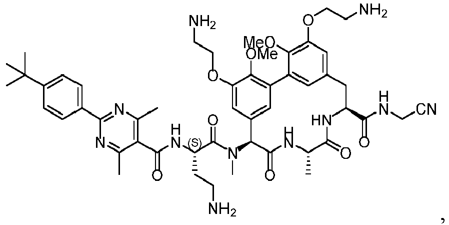 |
| 593 | 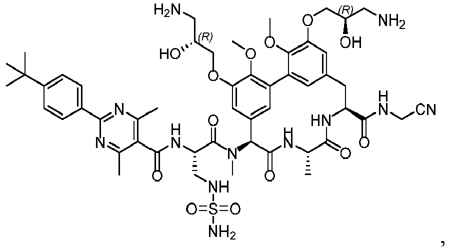 |
| 594 | 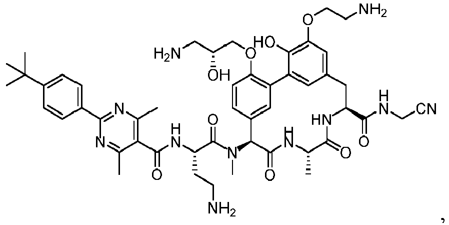 |
| 595 | 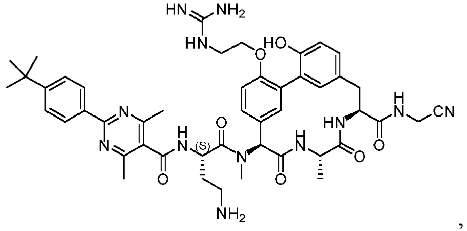 |
| 596 | 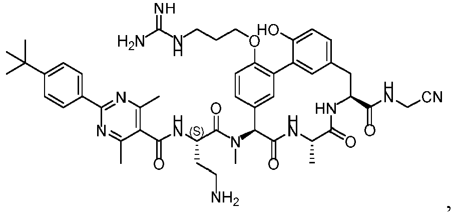 |
| 597 | 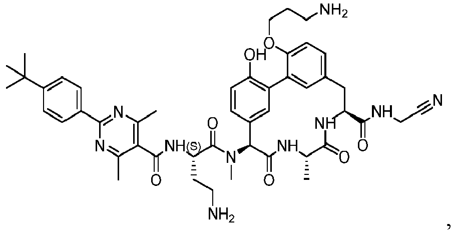 |
| 598 | 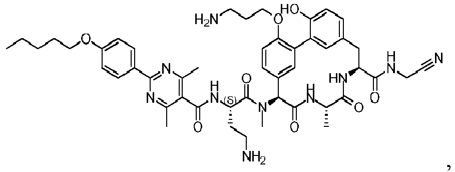 |
| 599 | 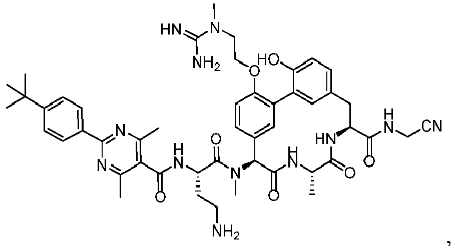 |
| 600 | 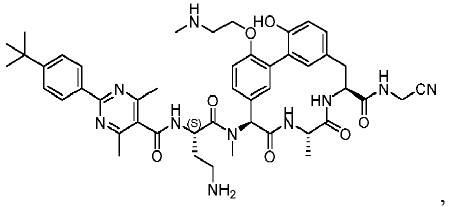 |
| 601 | 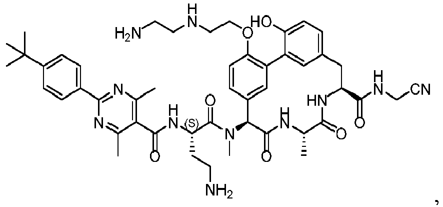 |
| 602 | 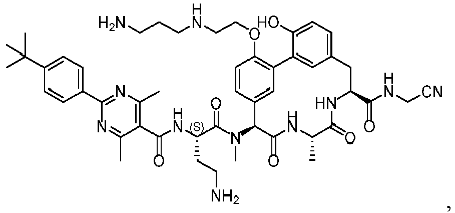 |
| 603 | 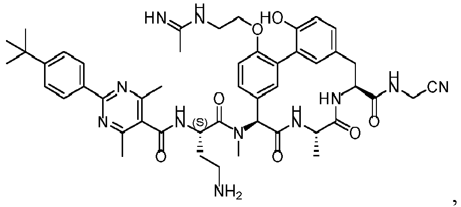 |
| 604 | 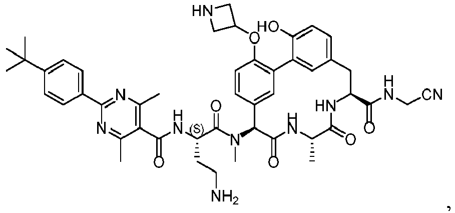 |
| 605 | 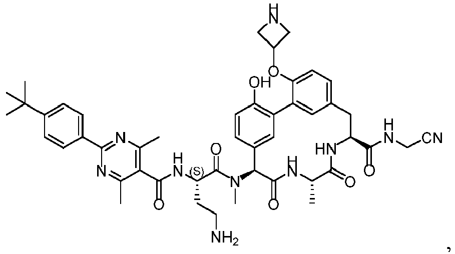 |
| 606 | 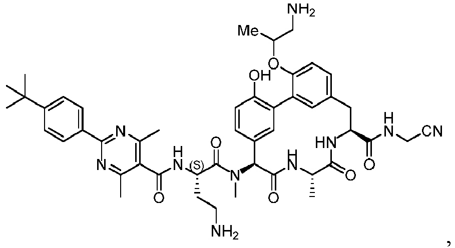 |
| 607 | 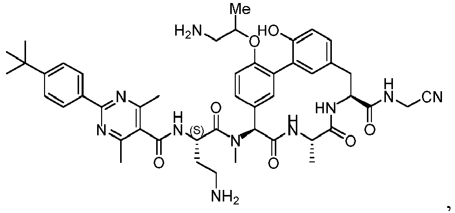 |
| 608 | 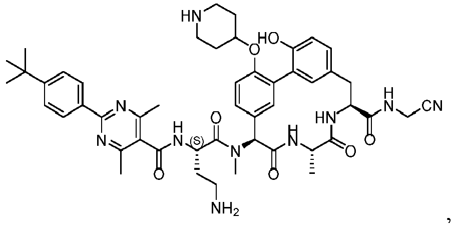 |
| 609 | 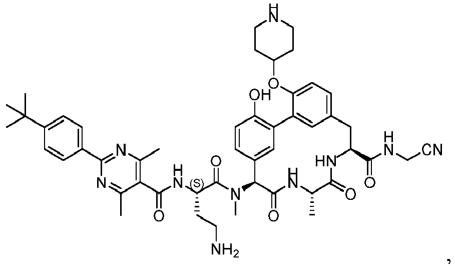 |
| 610 | 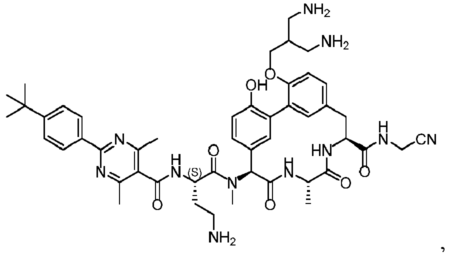 |
| 611 | 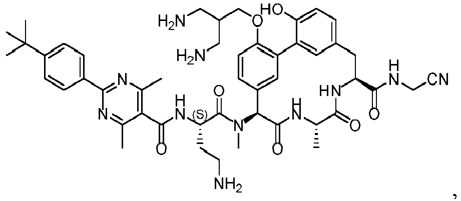 |
| 612 | 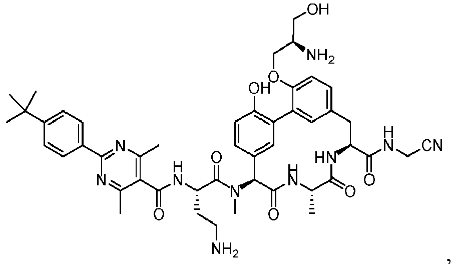 |
| 613 | 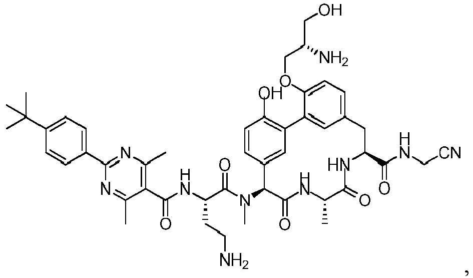 |
| 614 | 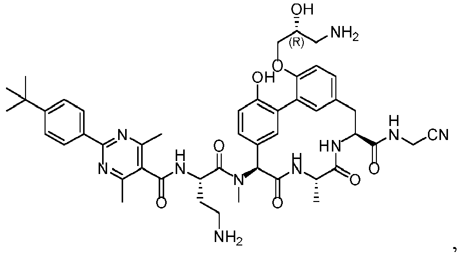 |
| 615 | 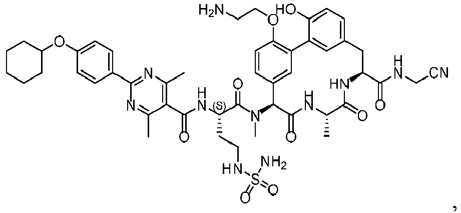 |
| 616 | 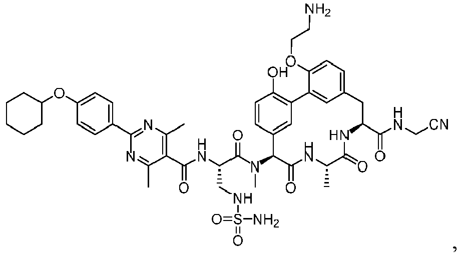 |
| 617 | 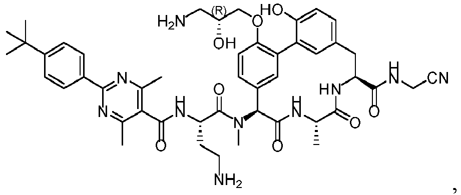 |
| 618 | 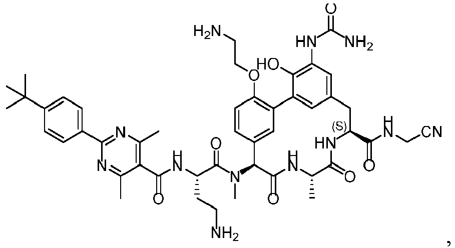 |
| 619 | 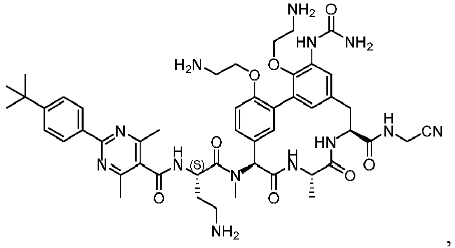 |
| 620 | 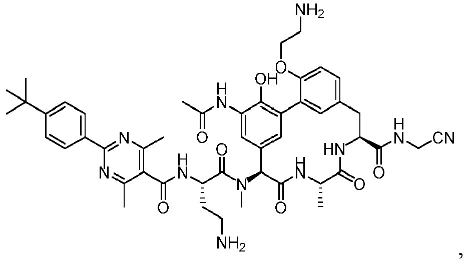 |
| 621 | 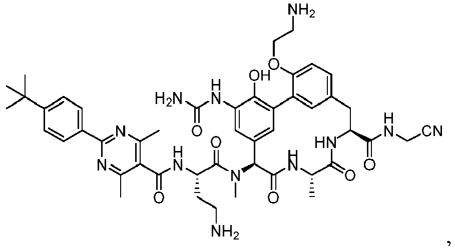 |
| 622 | 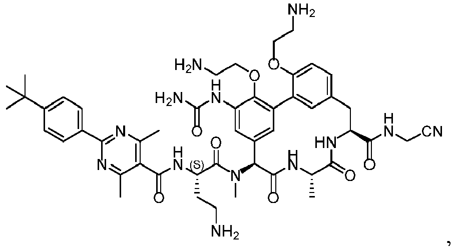 |
| 623 | 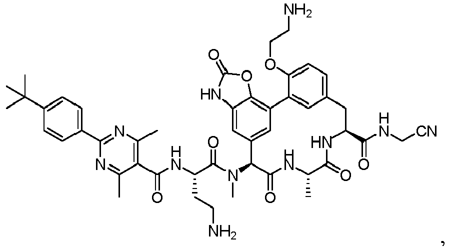 |
| 624 | 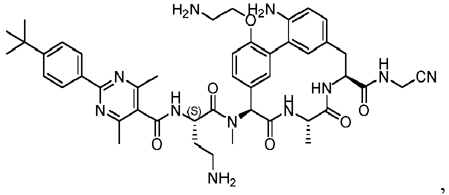 |
| 625 | 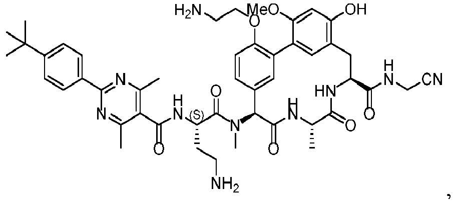 |
| 626 | 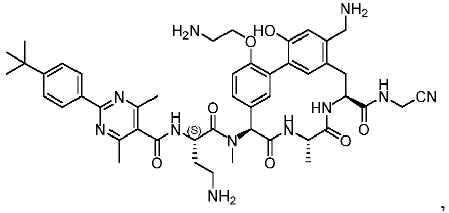 |
| 627 | 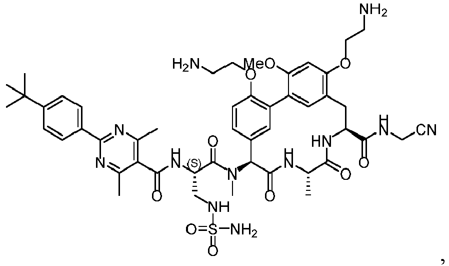 |
| 628 | 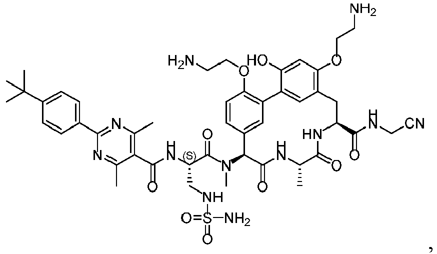 |
| 629 | 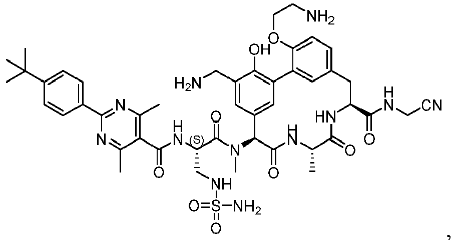 |
| 630 | 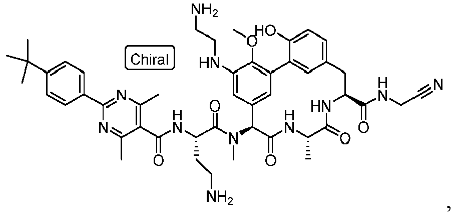 |
| 631 | 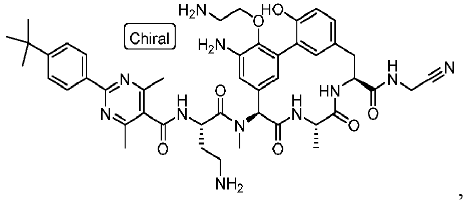 |
| 632 | 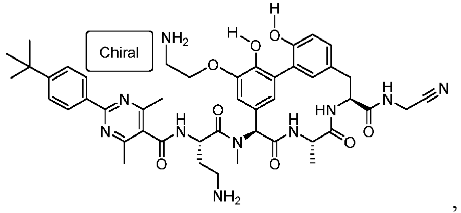 |
| 633 | 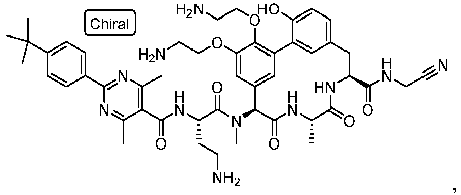 |
| 634 | 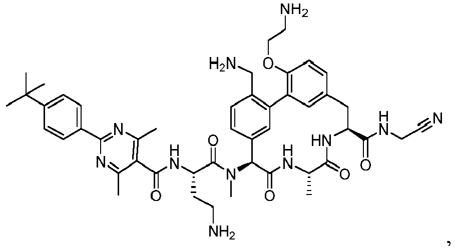 |
| 635 | 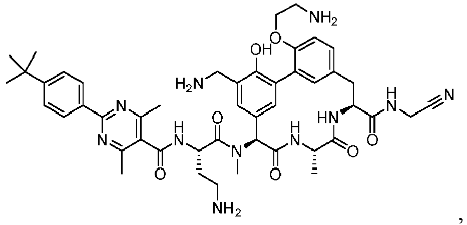 |
| 636 | 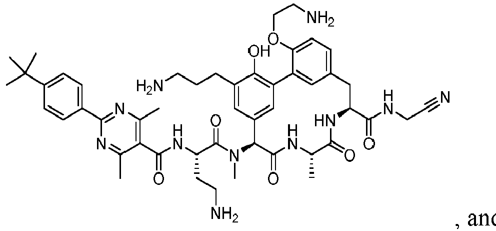 |
| 637 | 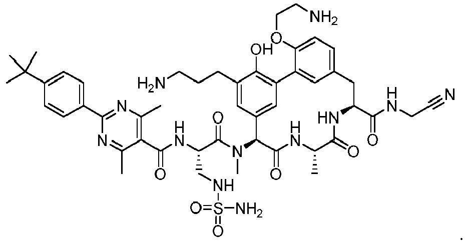 |

22. A pharmaceutical composition comprising the compound of any one of claims 1-21, or a pharmaceutically acceptable salt or solvate thereof, and a pharmaceutically acceptable excipient.

23. A compound of any one of claims 1-21, or a pharmaceutically acceptable salt or solvate thereof, or the pharmaceutical composition of claim 22, for use in a method of treatment of a bacterial infection in a patient.
